# EUROPEAN PATENT APPLICATION

(11) **EP 1 428 833 A2**
(43) Date of publication of application: **16.06.2004**
(21) Application number: 04001119.9
(22) Date of filing: 04.06.1998
(51) Int. Cl.: C07K 14/435, C12N 1/20, C12N 15/00, C12N 15/09, C12N 15/63, G01N 33/53, A61K 38/17, A61K 39/395, A61K 48/00

(54) **207 human secreted proteins**

(30) Priority: 06.06.1997 US 48915 P; 06.06.1997 US 48882 P; 06.06.1997 US 48892 P; 06.06.1997 US 48901 P; 06.06.1997 US 48900 P; 06.06.1997 US 48893 P; 06.06.1997 US 48964 P; 06.06.1997 US 48884 P; 06.06.1997 US 48894 P; 06.06.1997 US 48971 P; 06.06.1997 US 48885 P; 06.06.1997 US 49375 P; 06.06.1997 US 48881 P; 06.06.1997 US 48880 P; 06.06.1997 US 48896 P; 06.06.1997 US 49020 P; 06.06.1997 US 48876 P; 06.06.1997 US 48895 P; 06.06.1997 US 49019 P; 06.06.1997 US 48916 P; 06.06.1997 US 48970 P; 06.06.1997 US 48972 P; 06.06.1997 US 48949 P; 06.06.1997 US 48974 P; 06.06.1997 US 48883 P; 06.06.1997 US 48897 P; 06.06.1997 US 48898 P; 06.06.1997 US 49373 P; 06.06.1997 US 48917 P; 06.06.1997 US 48962 P; 06.06.1997 US 48963 P; 06.06.1997 US 48877 P; 06.06.1997 US 48878 P; 06.06.1997 US 49374 P; 06.06.1997 US 48875 P; 05.09.1997 US 57651 P; 05.09.1997 US 57654 P; 05.09.1997 US 57646 P; 05.09.1997 US 57662 P; 05.09.1997 US 57650 P; 05.09.1997 US 57661 P; 05.09.1997 US 57647 P; 05.09.1997 US 57584 P; 05.09.1997 US 57763 P; 05.09.1997 US 57769 P; 05.09.1997 US 57643 P; 05.09.1997 US 57645 P; 05.09.1997 US 57668 P; 05.09.1997 US 57635 P; 05.09.1997 US 57627 P; 05.09.1997 US 57667 P; 05.09.1997 US 57666 P; 05.09.1997 US 57764 P; 05.09.1997 US 57644 P; 05.09.1997 US 57765 P; 05.09.1997 US 57762 P; 05.09.1997 US 57775 P; 05.09.1997 US 57634 P; 05.09.1997 US 57777 P; 05.09.1997 US 57628 P; 05.09.1997 US 57778 P; 05.09.1997 US 57776 P; 05.09.1997 US 57760 P; 05.09.1997 US 57761 P; 05.09.1997 US 57771 P; 05.09.1997 US 57770 P; 05.09.1997 US 57649 P; 05.09.1997 US 57774 P; 05.09.1997 US 57648 P; 05.09.1997 US 57642 P; 05.09.1997 US 57629 P; 06.06.1997 US 48899 P; 18.12.1997 US 70923 P
(62) Divisional of application: 98926237.3
(71) Applicant: Human Genome Sciences, Inc., Rockville, MD 20850 (US)
(72) Inventor: Young, Paul, Gaithersburg, Maryland 20878 (US); Greene, John M., Gaithersburg, Maryland 20878 (US); Ferrie, Ann M., Germantown, Maryland 20874 (US); Ruben, Steven M., Olney, Maryland 20832 (US); Rosen, Craig A., Laytonsville, Maryland 20882 (US); Hu, Jing-Shan, Sunnyvale, California 94086 (US); Olsen, Henrik S., Gaithersburg, Maryland 20878 (US); Ebner, Reinhard, Gaithersburg, Maryland 20878 (US); Brewer, Laurie A., Poolesville, Maryland 20837 (US); More, Paul A., McLean, Virginia 22102 (US); Shi, Yanggu, Gaithersburg, Maryland 20878 (US); Florence, Charles, Rockville, Maryland 20851 (US); Florence, Kimberly, Rockville, Maryland 20851 (US); LaFleur, David W., Washington, DC 20009 (US); Ni, Jian, Rockville, Maryland 20853 (US); Fan, Ping, Gaithersburg, Maryland 20878 (US); Wei, Ying-Fei, Darnestown, Maryland 20878 (US); Fischer, Carrie L., Burke, Virginia 22015 (US); Soppet, Daniel R., Centreville, Virginia 22020 (US); Li, Yi, Sunnyvale, California 94086 (US); Zeng, Zhizhen, Gaithersburg, Maryland 20878 (US); Kyaw, Hla, Frederick, Maryland 21703 (US); Yu, Guo-Liang, Darnestown, Maryland 20878 (US); Feng, Ping, Gaithersburg, Maryland 20878 (US); Dillon, Patrick J., Carlsbad, California 92009 (US); Endress, Gregory A., Potomac, Maryland 20854 (US); Carter, Kenneth C., North Potomac, Maryland 20878 (US)
(74) Representative: VOSSIUS & PARTNER

(57) **Abstract**

The present invention relates to novel human secreted proteins and isolated nucleic acids containing the coding regionsof the genes encoding such proteins. Also provided are vectors, host cells, antibodies, and recombinant methods for producing human secreted proteins. The invention further relates to diagnostic and therapeutic methods useful for diagnosing and treating diseases, disorders, and/or conditions related to these novel human secreted proteins.

## Description

### Field of the Invention

This invention relates to newly identified polynucleotides and the polypeptides encoded by these polynucleotides, uses of such polynucleotides and polypeptides, and their production.

### Background of the Invention

Unlike bacterium, which exist as a single compartment surrounded by a membrane, human cells and other eucaryotes are subdivided by membranes into many functionally distinct compartments. Each membrane-bounded compartment, or organelle, contains different proteins essential for the function of the organelle. The cell uses "sorting signals," which are amino acid motifs located within the protein, to target proteins to particular cellular organelles.

One type of sorting signal, called a signal sequence, a signal peptide, or a leader sequence, directs a class of proteins to an organelle called the endoplasmic reticulum (ER). The ER separates the membrane-bounded proteins from all other types of proteins. Once localized to the ER, both groups of proteins can be further directed to another organelle called the Golgi apparatus. Here, the Golgi distributes the proteins to vesicles, including secretory vesicles, the cell membrane, lysosomes, and the other organelles.

Proteins targeted to the ER by a signal sequence can be released into the extracellular space as a secreted protein. For example, vesicles containing secreted proteins can fuse with the cell membrane and release their contents into the extracellular space - a process called exocytosis. Exocytosis can occur constitutively or after receipt of a triggering signal. In the latter case, the proteins are stored in secretory vesicles (or secretory granules) until exocytosis is triggered. Similarly, proteins residing on the cell membrane can also be secreted into the extracellular space by proteolytic cleavage of a "linker" holding the protein to the membrane.

Despite the great progress made in recent years, only a small number of genes encoding human secreted proteins have been identified. These secreted proteins include the commercially valuable human insulin, interferon, Factor VIII, human growth hormone, tissue plasminogen activator, and erythropoeitin. Thus, in light of the pervasive role of secreted proteins in human physiology, a need exists for identifying and characterizing novel human secreted proteins and the genes that encode them. This knowledge will allow one to detect, to treat, and to prevent medical disorders by using secreted proteins or the genes that encode them.

### Summary of the Invention

The present invention relates to novel polynucleotides and the encoded polypeptides. Moreover, the present invention relates to vectors, host cells, antibodies, and recombinant methods for producing the polypeptides and polynucleotides. Also provided are diagnostic methods for detecting disorders related to the polypeptides, and therapeutic methods for treating such disorders. The invention further relates to screening methods for identifying binding partners of the polypeptides.

### Detailed Description

### Definitions

The following definitions are provided to facilitate understanding of certain terms used throughout this specification.

In the present invention, "isolated" refers to material removed from its original environment (e.g., the natural environment if it is naturally occurring), and thus is altered "by the hand of man" from its natural state. For example, an isolated polynucleotide could be part of a vector or a composition of matter, or could be contained within a cell, and still be "isolated" because that vector, composition of matter, or particular cell is not the original environment of the polynucleotide.

In the present invention, a "secreted" protein refers to those proteins capable of being directed to the ER, secretory vesicles, or the extracellular space as a result of a signal sequence, as well as those proteins released into the extracellular space without necessarily containing a signal sequence. If the secreted protein is released into the extracellular space, the secreted protein can undergo extracellular processing to produce a "mature" protein. Release into the extracellular space can occur by many mechanisms, including exocytosis and proteolytic cleavage.

As used herein, a "polynucleotide" refers to a molecule having a nucleic acid sequence contained in SEQ ID NO:X or the cDNA contained within the clone deposited with the ATCC. For example, the polynucleotide can contain the nucleotide sequence of the full length cDNA sequence, including the 5' and 3' untranslated sequences, the coding region, with or without the signal sequence, the secreted protein coding region, as well as fragments, epitopes, domains, and variants of the nucleic acid sequence. Moreover, as used herein, a "polypeptide" refers to a molecule having the translated amino acid sequence generated from the polynucleotide as broadly defined.

In the present invention, the full length sequence identified as SEQ ID NO:X was often generated by overlapping sequences contained in multiple clones (contig analysis). A representative clone containing all or most of the sequence for SEQ ID NO:X was deposited with the American Type Culture Collection ("ATCC"). As shown in Table 1, each clone is identified by a cDNA Clone ID (Identifier) and the ATCC Deposit Number. The ATCC is located at 10801 University Boulevard, Manassas, Virginia 20110-2209, USA. The ATCC deposit was made pursuant to the terms of the Budapest Treaty on the international recognition of the deposit of microorganisms for purposes of patent procedure.

A "polynucleotide" of the present invention also includes those polynucleotides capable of hybridizing, under stringent hybridization conditions, to sequences contained in SEQ ID NO:X, the complement thereof, or the cDNA within the clone deposited with the ATCC. "Stringent hybridization conditions" refers to an overnight incubation at 42° C in a solution comprising 50% formamide, 5x SSC (750 mM NaCl, 75 mM sodium citrate), 50 mM sodium phosphate (pH 7.6), 5x Denhardt's solution, 10% dextran sulfate, and 20 µg/ml denatured, sheared salmon sperm DNA, followed by washing the filters in 0.1x SSC at about 65°C.

Also contemplated are nucleic acid molecules that hybridize to the polynucleotides of the present invention at lower stringency hybridization conditions. Changes in the stringency of hybridization and signal detection are primarily accomplished through the manipulation of formamide concentration (lower percentages of formamide result in lowered stringency); salt conditions, or temperature. For example, lower stringency conditions include an overnight incubation at 37°C in a solution comprising 6X SSPE (20X SSPE = 3M NaCl; 0.2M NaH₂PO₄; 0.02M EDTA, pH 7.4), 0.5% SDS, 30% formamide, 100 ug/ml salmon sperm blocking DNA; followed by washes at 50°C with 1XSSPE, 0.1% SDS. In addition, to achieve even lower stringency, washes performed following stringent hybridization can be done at higher salt concentrations (e.g. 5X SSC).

Note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility.

Of course, a polynucleotide which hybridizes only to polyA+ sequences (such as any 3' terminal polyA+ tract of a cDNA shown in the sequence listing), or to a complementary stretch of T (or U) residues, would not be included in the definition of "polynucleotide," since such a polynucleotide would hybridize to any nucleic acid molecule containing a poly (A) stretch or the complement thereof (e.g., practically any double-stranded cDNA clone).

The polynucleotide of the present invention can be composed of any polyribonucleotide or polydeoxribonucleotide, which may be unmodified RNA or DNA or modified RNA or DNA. For example, polynucleotides can be composed of single-and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, the polynucleotide can be composed of triple-stranded regions comprising RNA or DNA or both RNA and DNA. A polynucleotide may also contain one or more modified bases or DNA or RNA backbones modified for stability or for other reasons. "Modified" bases include, for example, tritylated bases and unusual bases such as inosine. A variety of modifications can be made to DNA and RNA; thus, "polynucleotide" embraces chemically, enzymatically, or metabolically modified forms.

The polypeptide of the present invention can be composed of amino acids joined to each other by peptide bonds or modified peptide bonds, i.e., peptide.isosteres, and may contain amino acids other than the 20 gene-encoded amino acids. The polypeptides may be modified by either natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched, for example, as a result of ubiquitination, and they may be cyclic, with or without branching. Cyclic, branched, and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination. (See, for instance, PROTEINS - STRUCTURE AND MOLECULAR PROPERTIES, 2nd Ed., T. E. Creighton, W. H. Freeman and Company, New York (1993); POSTTRANSLATIONAL COVALENT MODIFICATION OF PROTEINS, B. C. Johnson, Ed., Academic Press, New York, pgs. 1-12 (1983); Seifter et al., Meth Enzymol 182:626-646 (1990); Rattan et al., Ann NY Acad Sci 663:48-62 (1992).)

"SEQ ID NO:X" refers to a polynucleotide sequence while "SEQ ID NO:Y" refers to a polypeptide sequence, both sequences identified by an integer specified in Table 1.

"A polypeptide having biological activity" refers to polypeptides exhibiting activity similar, but not necessarily identical to, an activity of a polypeptide of the present invention, including mature forms, as measured in a particular biological assay, with or without dose dependency. In the case where dose dependency does exist, it need not be identical to that of the polypeptide, but rather substantially similar to the dose-dependence in a given activity as compared to the polypeptide of the present invention (i.e., the candidate polypeptide will exhibit greater activity or not more than about 25-fold less and, preferably, not more than about tenfold less activity, and most preferably, not more than about three-fold less activity relative to the polypeptide of the present invention.)

### Polynucleotides and Polypeptides of the Invention

### FEATURES OF PROTEIN ENCODED BY GENE NO: 1

This gene is expressed primarily in melanocytes and, to a lesser extent, in testes, ovary, kidney and other tissues.

Therefore, polynucleotides and polypeptides of the invention are useful as reagents for differential identification of the tissue(s) or cell type(s) present in a biological sample and for diagnosis of diseases and conditions, which include, but are not limited to, cancer, disorders of neural crest derived cells including pigmentation defects, melanoma, reproductive organ defects, and defects of the kidney. Similarly, polypeptides and antibodies directed to these polypeptides are useful in providing immunological probes for differential identification of the tissue(s) or cell type(s). For a number of disorders of the above tissues or cells, particularly of the skin, reproductive, and renal systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treating disorders that arise from alterations in the number or fate of neural crest derived cells including cancers such as melarioma and defects of the developing reproductive system.

### FEATURES OF PROTEIN ENCODED BY GENE NO: 2

This gene is expressed primarily in infant brain and fetal lung.

Therefore, polynucleotides and polypeptides of the invention are useful as reagents for differential identification of the tissue(s) or cell type(s) present in a biological sample and for diagnosis of diseases and conditions, which include, but are not limited to, developmental disorders of the brain or lung. Similarly, polypeptides and antibodies directed to these polypeptides are useful in providing immunological probes for differential identification of the tissue(s) or cell type(s). For a number of disorders of the above tissues or cells, particularly of the central nervous and pulmonary systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treating or diagnosing disorders associated with abnormal proliferation of cells in the Central nervous system and developing lung.

### FEATURES OF PROTEIN ENCODED BY GENE NO: 3

This gene is expressed primarily in breast lymph node and to a lesser extent in ovarian cancer and chondrosarcoma.

Therefore, polynucleotides and polypeptides of the invention are useful as reagents for differential identification of the tissue(s) or cell type(s) present in a biological sample and for diagnosis of diseases and conditions, which include, but are not limited to, immune responses such as inflammation or immune surveillance for tumors. This gene may be important for inflammatory responses associated with tumors. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NQ: 236 as residues: Lys-45 to Val-50, Lys-69 to Arg-76.

The tissue distribution indicates that polynucleotides and polypeptides corresponding to this gene are useful for treatment or diagnosis of immune responses including those associated with tumor-induced inflammation.

### FEATURES OF PROTEIN ENCODED BY GENE NO: 4

This gene is expressed primarily in T-cells and T-cell lymphomas.

Therefore, polynucleotides and polypeptides of the invention are useful as reagents for differential identification of the tissue(s) or cell type(s) present in a biological sample and for diagnosis of diseases and conditions, which include, but are not limited to, immunilogical diseases involving T-cells such as inflammation, autoimmunity, and cancers including T-cell lymphomas. Similarly, polypeptides and antibodies directed to these polypeptides are useful in providing immunological probes for differential identification of the tissue(s) or cell type(s). For a number of disorders of the above tissues or cells, particularly of T-cells and other cells of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosing and treating T-cell based disorders such as inflammatory diseases, autoimmune disease and tumors including T-cell lymphomas.

### FEATURES OF PROTEIN ENCODED BY GENE NO: 5

This gene is expressed primarily in activated monocytes.

Therefore, polynucleotides and polypeptides of the invention are useful as reagents for differential identification of the tissue(s) or cell type(s) present in a biological sample and for diagnosis of diseases and conditions, which include, but are not limited to, inflammation, autoimmunity, infection, or disorders involving activation of monocytes. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 238 as residues: Asp-19 to Arg-31.

The tissue distribution indicates that polynucleotides and polypeptides corresponding to this gene are useful for diagnosing or treating diseases that result in activation of monocytes including infections, inflammatory responses or autoimmune diseases.

### FEATURES OF PROTEIN ENCODED BY GENE NO: 6

The translation product of this gene shares sequence homology with terminal deoxynucleotidyltransferase which is thought to be important in catalyzing the elongation of oligo- or polydeoxynucleotide chains.

This gene is expressed primarily in activated human neutrophils.

Therefore, polynucleotides and polypeptides of the invention are useful as reagents for differential identification of the tissue(s) or cell type(s) present in a biological sample and for diagnosis of diseases and conditions which include, but are not limited to, cancer, particularly those of the blood such as leukemia and deficiencies in neutrophils such as neutropenia. Similarly, polypeptides and antibodies directed to these polypeptides are useful in providing immunological probes for differential identification of the tissue(s) or cell type(s). For a number of disorders of the above tissues or cells, particularly of the cardiovascular system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to terminal deoxynucleotidyltransferase indicates that polynucleotides and polypeptides corresponding to this gene are useful for treatment and differential diagnosis of acute leukemia's. Alternatively, this gene may function in the proliferation of neutrophils and be useful as a treatment for neutropenia, for example, following neutropenia as a result of chemotherapy:

### FEATURES OF PROTEIN ENCODED BY GENE NO: 7

The contig exhibits a reasonable homology to the human chorionic gonadotropic (HCG) analogue-GT beta-subunit as disclosed in U.S. Patent No. 5,508,261 and PCT Publication No. WO 92/22568. There is a high degree of conservation of the structurally important cysteine residues in these identities.

This gene is expressed primarily in IL-1 and LPS induced neutrophils.

Therefore, polynucleotides and polypeptides of the invention are useful as reagents for differential identification of the tissue(s) or cell type(s) present in a biological sample and for diagnosis of diseases and conditions, which include, but are not limited to, diseases of the immune system, including inflammatory diseases and allergies. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment/diagnosis of diseases of the immune system since expression is primarily in neutrophils, and may be useful as a growth factor for the differentiation or proliferation of neutrophils for the treatment of neutropenia following chemotherapy.

### FEATURES OF PROTEIN ENCODED BY GENE NO: 8

This gene is expressed primarily in IL-1- and LPS-induced neutrophils.

Therefore, polynucleotides and polypeptides of the invention are useful as reagents for differential identification of the tissue(s) or cell type(s) present in a biological sample and for diagnosis of diseases and conditions, which include, but are not limited to, diseases of the immune system, including inflammatory diseases and allergies. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 241 as residues: Ser-14 to Pro-22, Leu-43 to Val-53.

The tissue distribution indicates that polynucleotides and polypeptides corresponding to this gene are useful for treatment and diagnosis of diseases of the immune system since expression is primarily in neutrophils, and may be useful as a growth factor for the differentiation or proliferation of neutrophils for the treatment of neutropenia following chemotherapy.

### FEATURES OF PROTEIN ENCODED BY GENE NO: 9

This gene is expressed primarily in IL-1 and LPS induced neutrophils.

Therefore, polynucleotides and polypeptides of the invention are useful as reagents for differential identification of the tissue(s) or cell type(s) present in a biological sample and for diagnosis of diseases and conditions, which include, but are not limited to, diseases of the immune system, including inflammatory diseases and allergies. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 242 as residues: Tyr-22 to His-35.

The tissue distribution indicates that polynucleotides and polypeptides corresponding to this gene are useful for treatment/diagnosis of diseases of the immune system since expression is primarily in neutrophils, and may be useful as a growth factor for the differentiation or proliferation of neutrophils for the treatment of neutropenia following chemotherapy.

### FEATURES OF PROTEIN ENCODED BY GENE NO: 10

This gene is expressed primarily in activated T-cells and to a lesser extent in endothelial cells.

Therefore, polynucleotides and polypeptides of the invention are useful as reagents for differential identification of the tissue(s) or cell type(s) present in a biological sample and for diagnosis of diseases and conditions, which include, but are not limited to, immune dysfunctions including cancer of the T lymphocytes and autoimmune disorders and inflammation. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment and diagnosis of immune disorders particularly of T-cell origin and may act as a growth factor for particular subsets of T-cells such as CD4 positive cells which would make this a useful therapeutic for the treatment of HIV and other immune compromising illnesses.

### FEATURES OF PROTEIN ENCODED BY GENE NO: 11

This gene is expressed primarily in fetal tissue.

Therefore, polynucleotides and polypeptides of the invention are useful as reagents for differential identification of the tissue(s) or cell type(s) present in a biological sample and for diagnosis of many developmental abnormalities. Similarly, polypeptides and antibodies directed to these polypeptides are useful in providing immunological probes for differential identification of the tissue(s) or cell type(s). For a number of disorders of the above tissues or cells, particularly of the developing fetus, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful as a growth factor or differentiation factor for particular cell types in the developing fetus and may be useful in replacement or other types of therapy in cases where the gene is expressed aberrantly.

### FEATURES OF PROTEIN ENCODED BY GENE NO: 12

This gene is expressed primarily in T-cells and to a lesser extent in tumor tissue including glioblastoma, meningioma, and Wilm's tumor.

Therefore, polynucleotides and polypeptides of the invention are useful as reagents for differential identification of the tissue(s) or cell type(s) present in a biological sample and for diagnosis of diseases and conditions, which include, but are not limited to, diseases of the immune system including autoimmune conditions such as rheumatoid arthritis, inflammatory disorders and cancer. Similarly, polypeptides and antibodies directed to these polypeptides are useful in providing immunological probes for differential identification of the tissue(s) or cell type(s). For a number of disorders of the above tissues or cells, particularly of the immune, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 245 as residues: Thr-9 to Ser-14.

The tissue distribution indicates that polynucleotides and polypeptides corresponding to this gene are useful for diagnosis/ modulation of immune function disorders, including rheumatoid arthritis and inflammatory responses.

### FEATURES OF PROTEIN ENCODED BY GENE NO: 13

This gene is expressed primarily in placenta and to a lesser extent in fetal liver and bone marrow.

Therefore, polynucleotides and polypeptides of the invention are useful as reagents for differential identification of the tissue(s) or cell type(s) present in a biological sample and for diagnosis of hematological disorders. Similarly, polypeptides and antibodies directed to these polypeptides are useful in providing immunological probes for differential identification of the tissue(s) or cell type(s). For a number of disorders of the above tissues or cells, particularly of the hematological and immune systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful as a growth factor for hematapoietic stem cells or progenitor cells in the treatment of chemotherapy patients or kidney disease.

### FEATURES OF PROTEIN ENCODED BY GENE NO: 14

This gene is expressed primarily in stromal cells.

Therefore, polynucleotides and polypeptides of the invention are useful as reagents for differential identification of the tissue(s) or cell type(s) present in a biological sample and for diagnosis of hematapoietic disorders including cancer, neutropenia, anemia, and thrombocytopenia. Similarly, polypeptides and antibodies directed to these polypeptides are useful in providing immunological probes for differential identification of the tissue(s) or cell type(s). For a number of disorders of the above tissues or cells, particularly of the hematapoietic and immune, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful as a growth factor for hematapoietic stem cells or progenitor cells, in particular following chemotherapy treatment.

### FEATURES OF PROTEIN ENCODED BY GENE NO: 15

The translation product of this gene shares sequence homology with epsilon-COP from Bos taurus which is thought to be important as a component of coatomer, a complex of seven proteins, that is the major component of the non-clathrin membrane coat. Preferred polypeptides encoded by this gene comprise the following amino acid sequences:
MAPPAPGPASGGSGEVDELFDVKNAFYIGSYQQCINEAXXVKLSSPERDVERD VFLYRAYLAQRKFGVVLDEIKPSSAPELQAVRMFADYLAHESRRDSIVAELDRE MSRSXDVTNTTFLLMAASIYLHDQNPDAALRALHQGDSLECTAMTVQILLKLD RLDLARKELKRMQDLDEDATLTQLATAWVSLATGGEKLQDAYYIFQEMADKCS PTLLLLNGQAACHMAQGRWEAAEGLLQEALDKDSGYPETLVNLIVLSQHLGKP PEVTNRYLSQLKDAHRSHPFIKEYQAKENDFDRLVLQYAPSAEAGPELSGP (SEQ ID NO:458); or RDVERDVFLYRAYLAQRKFGVVLDEIKPSSAPELQAVRMF ADYLAHESRRDSIVAELDREMSRSXDVTNTTFLLMAASIYLHDQNPDAALRALH QGDSLECTAMTVQILLKLDRLDLARKELKRMQDLDEDATLTQLATAWVSLATG GEKLQDAYYIFQEMADKCSPTLLLLNGQAACHMAQGRWEAAEGLLQEALDKD SGYPETLVNLIVLSQHLGKPPEVTNRYLSQLKDAHRSHPFIKEYQAKENDFDRL VLQYAPSA (SEQ ID NO:459).

This gene is expressed primarily in activated monocytes and T-cells, and to a lesser extent in multiple other tissues.

Therefore, polynucleotides and polypeptides of the invention are useful as reagents for differential identification of the tissue(s) or cell type(s) present in a biological sample and for diagnosis of diseases and conditions, which include, but are not limited to, immunomodulation, specifically relating to transport problems in these cells. Similarly, polypeptides and antibodies directed to these polypeptides are useful in providing immunological probes for differential identification of the tissue(s) or cell type(s). For a number of disorders of the above tissues or cells, particularly of the immune, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to epsilon-COP indicates that polynucleotides and polypeptides corresponding to this gene are useful for treating /diagnosing problems with the cellular transport of proteins that may result in immunologic dysfunction.

### FEATURES OF PROTEIN ENCODED BY GENE NO: 16

The translation product of this gene shares sequence homology with an RNA helicase which is thought to be important in polynucleotide metabolism. The translation product of this contig exhibits good homology to the LbeIF4A antigen of Leishmania braziliensis. The LbeIF4A antigen, or immunogenic portions of it, can be used to induce protective immunity against leishmaniasis, specifically L. donovani, L. chagasi, L. infantum, L. major, L. braziliensis, L. panamensis, L. tropica and L. guyanensis. It can also be used diagnostically to detect Leishmania infection or to stimulate a cellular and/or humoral immune response or to stimulate the production of interleukin-12.

This gene is expressed primarily in colon cancer and to a lesser extent in pituitary.

Therefore, polynucleotides and polypeptides of the invention are useful as reagents for differential identification of the tissue(s) or cell type(s) present in a biological sample and for diagnosis of cancers particularly of the colon. Similarly, polypeptides and antibodies directed to these polypeptides are useful in providing immunological probes for differential identification of the tissue(s) or cell type(s). For a number of disorders of the above tissues or cells, particularly of the gastrointestinal system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 249 as residues: Glu-93 to Ala-98, Gln-150 to Leu-156, Leu-220 to Leu-231, Leu-268 to Arg-273, Val-324 to Pro-341, Arg-372 to Asn-380, Ser-405 to Gly-410, Phe-426 to Ala-433, Glu-458 to Asp-470, Arg-506 to Ser-547.

The tissue distribution and homology to RNA helicase indicates that polynucleotides and polypeptides corresponding to this gene are useful for development of diagnostic tests for colon cancer.

### FEATURES OF PROTEIN ENCODED BY GENE NO: 17

The translation product of this contig has sequence homology to a cytoplasmic protein that binds specifically to JNK designated the JNK interacting protein-1 or JIP-1 in mice. JIP-1 caused cytoplasmic retention of JNK and inhibition of JNK-regulated gene expression.

This gene is expressed primarily in brain including pituitary cerebellum frontal cortex, fetal brain and to a lesser extent in the kidney cortex.

Therefore, polynucleotides and polypeptides of the invention are useful as reagents for differential identification of the tissue(s) or cell type(s) present in a biological sample and for diagnosis of the central nervous system disorders including ischemia, epilepsy, Parkinson's disease, and schizophrenia. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Furthermore, the translation product of this contig may suppress the effects of the JNK signaling pathway on cellular proliferation, including transformation by the Bcr-Abl oncogene. Preferred epitopes include those comprising a sequence shown in SEQ ID NO: 250 as residues: Pro-6 to Ser-26, Ala-30 to Asp-41, Gly-55 to Ser-61, Gly-74 to Thr-80, Tyr-117 to Ala-123, Tyr-167 to Asp-172, Ala-212 to Cys-223, Pro-239 to Tyr-244.

The tissue distribution indicates that polynucleotides and polypeptides corresponding to this gene are useful for enhanced survival and/or differentiation of neurons as a treatment for neurodegenerative disease.

### FEATURES OF PROTEIN ENCODED BY GENE NO: 18

The translation product of this gene shares sequence homology with a liver stage antigen from a protozoan parasite.

This gene is expressed primarily in fetal tissue and to a lesser extent in activated T-cells and other immune cells.

Therefore, polynucleotides and polypeptides of the invention are useful as reagents for differential identification of the tissue(s) or cell type(s) present in a biological sample and for diagnosis of diseases and conditions, which include, but are not limited to, developmental abnormalities and diseases of immune function. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to a protozoan antigen indicates that polynucleotides and polypeptides corresponding to this gene are useful for treatment/immune modulation of parasitic infections.

### FEATURES OF PROTEIN ENCODED BY GENE NO: 19

Preferred polypeptide encoded by this gene comprise the following polypeptide sequences:
MKAIGIEPSLATYHHIIRLFDQPGDPLKRSSFIIYDIMNELMGKRFSPKD PDDDKFFQSAMSICSSLRDLELAYQVHGLLKTGDNWKFIGPDQHRNFYYSKFF DLICLMEQIDVTLKWYEDLIPSAYFPHSQTMIHLLQALDVANRLEVIPKIWER (SEQ ID NO:460); and/or KDSKEYGHTFRSDLREEILMLMARDKHPPELQVAF ADCAADIKSAYESQPIRQTAQDWPATSLNCIAILFLRAGRTQEAWKMLGLFRKH NKIPRSELLNELMDSAKVSNSPSQAIEVVELASAFSLPICEGLTQRVMSDFAINQ EQKEALSNLTALTSDSDTDSSSDSDSDTSEGK (SEQ ID NO:461). Polynucleotides encoding such polypeptides are also provided.

This gene is expressed primarily in stromal and CD34 depleted bone marrow cells and to a lesser extent in tissues of embryonic origin.

Therefore, polynucleotides and polypeptides of the invention are useful as reagents for differential identification of the tissue(s) or cell type(s) present in a biological sample and for diagnosis of diseases and conditions, which include, but are not limited to, diseases of hematologic origin including cancers and immune dysfunction. Similarly, polypeptides and antibodies directed to these polypeptides are useful in providing immunological probes for differential identification of the tissue(s) or cell type(s). For a number of disorders of the above tissues or cells, particularly of the hematapoietic and immune, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 252 as residues: Ser-28 to Gln-34.

The tissue distribution indicates that polynucleotides and polypeptides corresponding to this gene are useful as a growth factor for hematopoietic stem cells or progenitor cells which may be useful in the treatment of chemotherapy patients suffering from neutropenia.

### FEATURES OF PROTEIN ENCODED BY GENE NO: 20

Preferred polypeptide fragments can be found in an alternative open reading frame. These preferred polypeptides comprise the amino acid sequence:
MSSDNESDIEDEDLKLELRRLRDKHLKEIQDLQSRQKHEIESLYTKLGKVPPAVI IPPAAPLSGRRRRPTKSKGSKSSRSSSLGNKSPQLSGNLSGQSAASVLHPQQTL HPPGNIPESGQNQLLQPLKPSPSSDNLYSAFTSDGAISVPSLSAPGQGTSSTNTV GATVNSQAAQAQPPAMTSSRKGTFTDDLHKLVDNWARDAMNLSGRRGSKGH MNYEGPGMARKFSAPGQLCISMTSNLGGSAPISAASATSLGHFTKSMCPPQQY GFPATPFGAQWSGTGGPAPQPLGQFQPVGTASLQNFNISNLQKSISNPPGSNL RTT (SEQ ID NO:462); IQDLQSRQKHEIESLYTKLGKVPPAVITPPAAPLSGRRRR PTKSKGSKSSRSSSLGNKSPQLSGNLSGQSAASVLHPQQTLHPPGNIPESGQN QLLQPLKPSPSSDNLYSAFTSDGAISVPSLSAPGQGTSST (SEQ ID NO:463); TSDGAISVPSLSAPGQGTSSTNTVGATVNSQAAQAQPPAMTSSRKGTFTDDLH (SEQ ID NO:464); KGHMNYEGPGMARKFSAPGQLCISMTSNLGGSAPISAAS ATSLGHFTK (SEQ ID NO:465); QPLKPSPSSDNLYSAFTSDGAISVPSLSAPG (SEQ ID NO:466). Also preferred are polynucleotide fragments encoding these polypeptide fragments.

This gene is expressed in fetal liver and tissues associated with the CNS.

Therefore, polynucleotides and polypeptides of the invention are useful as reagents for differential identification of the tissue(s) or cell type(s) present in a biological sample and for diagnosis of diseases and conditions, which include, but are not limited to, liver and CNS diseases. Similarly, polypeptides and antibodies directed to these polypeptides are useful in providing immunological probes for differential identification of the tissue(s) or cell type(s). For a number of disorders of the above tissues or cells, particularly of the liver and CNS, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 253 as residues: Gln-26 to Lys-34.

The tissue distribution indicates that polynucleotides and polypeptides corresponding to this gene are useful for diagnosis and treatment for liver diseases such as hepatocellular carcinomas and diseases of the CNS.

### FEATURES OF PROTEIN ENCODED BY GENE NO: 21

In an alternative reading frame, this gene shows sequence homology to two recently cloned genes, karyopherin beta 3 and Ran_GTP binding protein 5. (See Accession Nos. gi|2102696 and gnl|PID|e328731.) The Ran_GTP binding protein is related to importin-beta, the key mediator of nuclear localization signal (NLS)-dependent nuclear transport. Based on homology, it is likely that this gene may activity similar to the RAN_GTP binding protein. Preferred polypeptide fragments comprise the amino acid sequence: VRVAAAESMXLLLECAXVRGPEYLTQMWHFMCDALIKA IGTEPDSDVLSEIMHSFAK (SEQ ID NO:467). Also preferred are polynucleotide fragments encoding these polypeptide fragments.

- This gene is expressed-in thymus tissue.

Therefore, polynucleotides and polypeptides of the invention are useful as reagents for differential identification of the tissue(s) or cell type(s) present in a biological sample and for diagnosis of diseases and conditions, which include, but are not limited to, immune disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment for immune disorders.

### FEATURES OF PROTEIN ENCODED BY GENE NO: 22

This gene is expressed primarily in prostate and osteoclastoma tissues. Preferred polypeptide fragments also comprise the amino acid sequence:
MEINNQNCFIVIDLVRTVMENGVEGLLIFGAFLPESWLIGVRCSSEPPKALLLIL AHSQKRRLDGWSHRHLRVHYCVSLTIHFS (SEQ ID NO:468). Also preferred are polynucleotide sequences encoding this polypeptide fragment.

Therefore, polynucleotides and polypeptides of the invention are useful as reagents for differential identification of the tissue(s) or cell type(s) present in a biological sample and for diagnosis of diseases and conditions, which include, but are not limited to, bone and prostate diseases, and cancers, particularly of the bone and prostate. Similarly, polypeptides and antibodies directed to these polypeptides are useful in providing immunological probes for differential identification of the tissue(s) or cell type(s). For a number of disorders of the above tissues or cells, particularly of the bone and prostate systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 255 as residues: Met-1 to Ser-11.

The tissue distribution indicates that polynucleotides and polypeptides - corresponding to this gene are useful for diagnosis and treatment for bone and prostate disorders, especially cancers of those systems.

### FEATURES OF PROTEIN ENCODED BY GENE NO: 23

This gene shares sequence homology with the FK506-binding protein (FKBP-13) family, a known cytosolic receptor for the immunosuppressants. Recently, another group has cloned a very similar gene, recognizing the homology to FK506-binding protein family, calling their gene FKBP23. (See Accession No. 2827255.)

This gene is expressed primarily in lymphoid tissues.

Therefore, polynucleotides and polypeptides of the invention are useful as reagents for differential identification of the tissue(s) or cell type(s) present in a biological sample, especially for those susceptible to immune suppressant therapies and for diagnosis of diseases and conditions, which include, but are not limited to, immune suppressant disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 256 as residues: Ala-19 to Val-31, Arg-38 to Gly-49, Ala-61 to Lys-66, Tyr-68 to Pro-78, Gly-116 to Ala-121, Asp-154 to Ser-162, Glu-173 to Gln-186, Phe-194 to Gly-203, Pro-207 to Val-212.

The tissue distribution and homology to FKBP-12 and -13 indicates that polynncleotides and polypeptides corresponding to this gene are useful for diagnosis and treatment for immune suppressant disorders.

### FEATURES OF PROTEIN ENCODED BY GENE NO: 24

This gene is expressed primarily in the brain and in the retina. This gene maps to chromosome 8, and therefore can be used in linkage analysis as a marker for chromosome 8.

Therefore, polynucleotides and polypeptides of the invention are useful as reagents for differential identification of the tissue(s) or cell type(s) present in a biological sample and for diagnosis of diseases and conditions, which include, but are . not limited to, neurological and ocular associated disease states. Similarly, polypeptides and antibodies directed to these polypeptides are useful in providing immunological probes for differential identification of the tissue(s) or cell type(s). For a number of disorders of the above tissues or cells, particularly of the disorders of the central nervous system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 257 as residues: Cys-34 to Asp-40.

The tissue distribution in retina indicates that polynucleotides and polypeptides corresponding to this gene are useful for the treatment and/or detection of eye disorders including blindness, color blindness, impaired vision, short and long sightedness, retinitis pigmentosa, retinitis proliferans, and retinoblastoma. Expression in the brain indicates a role in the is useful for the detection/treatment of neurodegenerative disease states and behavioral disorders such as Alzheimer's Disease, Parkinson's Disease, Huntington's Disease, schizophrenia, mania, dementia, paranoia, obsessive compulsive disorder and panic disorder.

### FEATURES OF PROTEIN ENCODED BY GENE NO: 25

This gene shows sequence homology to a newly identified class of proteins expressed in the nervous system, called stathmin family. (See Accession No. 2585991; see also Eur. J. Biochem. 248 (3), 794-806 (1997).) The stathmin family appears to be an ubiquitous phosphoprotein involved as a relay integrating various intracellular signaling pathways. These pathways affect cell proliferation and differentiation. Preferred polypeptide fragments comprise the amino acid sequence:
QDKHAEEVRKNKELKEEASR (SEQ ID NO:469); QQDLSPWAAPVGCPLXXASX TCHXLPLSGCLRRQSXSLPVVAXLCFWFSCPLASLFVPGQPCVTCPFPSLPFQD KHAEEVRKNKELKEEASR (SEQ ID NO:470). Also preferred are the polynucleotide fragments encoding these polypeptide fragments.

This gene is expressed primarily in brain.

Therefore, polynucleotides and polypeptides of the invention are useful as reagents for differential identification of the tissue(s) or cell type(s) present in a biological sample and for diagnosis of diseases and conditions, which include, but are not limited to, neurological disorders. Similarly, polypeptides and antibodies directed to these polypeptides are useful m providing immunological probes for differential identification of the tissue(s) or cell type(s). For a number of disorders of the above tissues or cells, particularly of the central nervous system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the detection/treatment of neurodegenerative disease states and behavioral disorders such as Alzheimer's Disease, Parkinson's Disease, Huntintons Disease, schizophrenia, mania, dementia, paranoia, obsessive compulsive disorder and panic disorder.

### FEATURES OF PROTEIN ENCODED BY GENE NO: 26

The polynucleotide sequence of this gene contains a domain similar to a Flt3 ligand peptide. Preferred polypeptide fragments comprise the amino acid sequence: PTRCCTTQPCRSSARRPCWVPMVPSPEGREXQPTCPS (SEQ ID NO:471). Thus, this gene may have activity as binding to Flt3 receptors, a process known to promote angiogenesis and/or lymphangiogenesis.

This gene is expressed in human tonsil, and to a lesser extent in teratocarcinoma, placenta, colon carcinoma, and fetal kidney.

Therefore, polynucleotides and polypeptides of the invention are useful as reagents for identification of the tissue(s) or cell type(s) present in a biological sample and for diagnosis of diseases and conditions, which include, but are not limited to, diseases of the tonsil, as well as cancers, such as colon, reproductive, and kidney cancers. Similarly, polypeptides and antibodies directed to these polypeptides are useful in providing immunological probes for differential identification of the tissue(s) or cell type(s). For a number of disorders of the above tissues or cells, particularly of the tonsils, colon, reproductive organs, and kidneys, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 259 as residues: Pro-22 to Glu-33.

The tissue distribution-in tonsil and several cancers and fetal tissues indicates that polynucleotides and polypeptides corresponding to this gene are useful for diagnosis and treatment of diseases of the tonsil or colon, such as tonsillitis, inflammatory diseases involving nose and paranasal sinuses, especially during the infection of influenza, adenoviruses, parainfluenza, rhinoviruses. The gene may also be useful in the diagnosis and treatment of neoplasms of nasopharynx or colon origins.

### FEATURES OF PROTEIN ENCODED BY GENE NO: 27

In an alternative reading frame exists a large open reading frame that encodes a preferred polypeptide. Preferred polypeptide fragments comprise the amino acid sequence:
MKRSLNENSARSTAGCLPVPLFNQKKRNRQPLTSNPLKDDSGISTPSDNYDFP PLPTDWAWEAVNPEXAPVMKTVDTGQIPHSVSRPLRSQDSVFNSIQSNTGRSQ GGWSYRDGNKNTSLKTWXKNDFKPQCKRTNLVANDGKNSCPMSSGAQQQK QLRTPEPPNLSRNKETELLRQTHSSKISGCTMRGLDKNSALQTLKPNFQQNQY KXQMLDDIPEDNTLKETSLYQLQFKEKASSLRIISAVIESMKYWREHAQKTVLL FEVLAVLDSAVTPGPYYSKTFLMRDGKNTLPCVFYEIDRELPRLIRGRVHRCVG NYDQKKNIFQCVSVRPASVSEQKTFQAFVKIADVEMQYYINVMNET (SEQ ID NO:472); SQDSVFNSIQSNTGRSQGGWSYRDGNKNTSLKTWXKNDFKPQCKR (SEQ ID NO:473); NKETELLRQTHSSKISGCTMRGLDKNSALQTLKPNF (SEQ ID NO:474);SSLRIISAVIESMKYWREHAQKTVLLFEVLAVLDSAVTPGPYYSKTFLM (SEQ ID NO:475); and PRLIRGRVHRCVGNYDQKKNIFQCVSVRPASVSEQKT FQAFV (SEQ ID NO:476).

This gene is expressed primarily in human testes.

Therefore, polynucleotides and polypeptides of the invention are useful as reagents for differential identification of the tissue(s) or cell type(s) present in a biological sample and for diagnosis of diseases and conditions, which include, but are not limited to, male reproductive disorders, including cancer. Similarly, polypeptides and antibodies directed to these polypeptides are useful in providing immunological probes for differential identification of the tissue(s) or cell type(s). For a number of disorders of the above tissues or cells, particularly of the male reproductive system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful as a hormone with reproductive or other systemic functions; contraceptive development; male infertility of testicular causes, such as Kleinfelterís syndrome, varicocele, orchitis; male sexual dysfunctions; testicular neoplasms; and inflammatory disorders such as epididymitis.

### FEATURES OF PROTEIN ENCODED BY GENE NO: 28

This gene is expressed primarily in apoptotic T-cell.

Therefore, polynucleotides and polypeptides of the invention are useful as reagents for differential identification of the tissue(s) or cell type(s) present in a biological sample and for diagnosis of diseases and conditions, which include, but are not limited to, diseases relating to T cells, as well as cancer in general. Similarly, polypeptides and antibodies directed to these polypeptides are useful in providing immunological probes for differential identification of the tissue(s) or cell type(s). For a number of disorders of the above tissues or cells, particularly of the disorders of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for immune disorders. Moreover, since the gene was isolated from an apoptotic cell and based on the understanding of the relationship of apoptosis and cancer, it is likely that this gene may play a role in the genesis of cancer.

### FEATURES OF PROTEIN ENCODED BY GENE NO: 29

This gene is expressed primarily in human tonsils.

Therefore, polynucleotides and polypeptides of the invention are useful as reagents for differential identification of the tissue(s) or cell type(s) present in a biological sample and for diagnosis of diseases and conditions, which include, but are not limited to, gastrointestinal disorders. Similarly, polypeptides and antibodies directed to these polypeptides are useful in providing immunological probes for differential identification of the tissue(s) or cell type(s). For a number of disorders of the above tissues or cells, particularly of the gastrointestinal system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level; i.e., the expression level in healthy tissue or bodily fluid from an individual not having the disorder.

The tissue distribution indicates that polynucleotides and polypeptides corresponding to this gene are useful for the diagnosis and treatment of gastrointestinal diseases.

### FEATURES OF PROTEIN ENCODED BY GENE NO: 30

The translation product of this gene shares sequence homology with C44C1.2 gene product of Caenorhabditis elegans with unknown function. Preferred polypeptide fragments comprise the amino acid sequence:
GVFRPCVCGRPASLTCSPLDPEVGPYCDTPTMRTLFNLLWLALACSPVHTTLSK SDAKKAASKTLLEKSQFSDKPVQDRGLVVTDLKAESVVLEHRSYCSAKARDRH FAGDVLGYVTPWNSHGYDVTKVFGSKFTQISPVWLQLKRRGREMFEVTGLHD VDQGWMRAVRKHAKGLHIVPRLLFEDWTYDDFRNVLDSEDEIEELSKTWQVA KNQHFDGFVVEVWNQLLSQKRVGLIHMLTHLAEALHQARLLALLVIPPAITPGT DQLGMFTHKEFEQLAPVLDGFSLMTYDYSTAHQPGPNAPLSWVRACVQVLDP KXKWRTKSSWGSTSMXWTXRXPXDARXPVVGXRXIQXLKDHXPRMVLDSK PQ (SEQ ID NO:477); TCSPLDPEVGPYCDTPTMRTLFNLLWLALACSPVHTTLS (SEQ ID NO:478); LVVTDLKAESVVLEHRSYCSAKARDRHFAGDVLGYVTPW NSHGYDVTKVFGSKF (SEQ ID NO:479); REMFEVTGLHDVDQGWMRAVRK HAKGLHIVPRLLFEDWTYDDFRNVLDSEDE (SEQ ID NO:480); HFDGFVVEVW NQLLSQKRVGLIHMLTHLAEALHQARLLALLVIPPAITPGTDQLGM (SEQ ID NO:481); DGFSLMTYDYSTAHQPGPNAPLSWVRACVQVLDPKXKWRTKSSW GST (SEQ ID NO:482). Also preferred are polynucleotide fragments encoding these polypeptide fragments. This gene maps to human chromosome 11, and therefore is useful in linkage analysis as a marker for chromosome 11.

This gene is expressed primarily in human T cells and to a lesser extent in human colon carcinoma.

Therefore, polynucleotides and polypeptides of the invention are useful as reagents for differential identification of the tissue(s) or cell type(s) present in a biological sample and for diagnosis of diseases and conditions, which include, but are not limited to, immune disorders and cancer. Similarly, polypeptides and antibodies directed to these polypeptides are useful in providing immunological probes for differential identification of the tissue(s) or cell type(s). For a number of disorders of the above tissues or cells, partieularly of the immune and gastrointestinal systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 263 as residues: Leu-21 to Ala-30, Ser-38 to Asp-47, Pro-87 to Asp-94, Leu-197 to Thr-204, Pro-256 to Ser-262, Thr-277 to Arg-282, Thr-293 to Trp-303.

The tissue distribution indicates that polynucleotides and polypeptides corresponding to this gene are useful for the diagnosis and treatment of immune disorders and gastrointestinal diseases.

### FEATURES OF PROTEIN ENCODED BY GENE NO: 31

The translation product of this gene shares sequence homology with Ribosomal protein L11 of Caenorhabditis elegans. (See Accession No. 156201.) Preferred polypeptide fragments comprise the amino acid sequence:
ERGVSINQFCKEFNERTKDIKEGIPLPTKILVKPDRTFEIKIGQPTVSYFLKAAAG IEKGARQTGKEVAGLVTLKHVYEIARIKAQDEAFALQDVPLSSWRSIIGSARSL GIRWKDLSSEELAAF QKERAIFLAAQKEADLAAQEEAAKK (SEQ ID NO:483). Also preferred are polynucleotide fragments encoding these polypeptide fragments.

This gene is expressed in human embryo tissue and to a lesser extent in human epithelioid sarcoma and other tissues.

Therefore, polynucleotides and polypeptides of the invention are useful as reagents for identification of the tissue(s) or cell type(s) present in a biological sample and for diagnosis of diseases and conditions, which include, but are not limited to, development disorders and epithelial cell cancer. Similarly, polypeptides and antibodies directed to these polypeptides are useful in providing immunological probes for differential identification of the tissue(s) or cell type(s). For a number of disorders of the above tissues or cells, particularly of the embryonic and epithelial cell systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 264 as residues: Lys-34 to Gly-40.

The tissue distribution indicates that polynucleotides and polypeptides corresponding to this gene are useful for the diagnosis and treatment of developmental disorders and epithelial cancer.

### FEATURES OF PROTEIN ENCODED BY GENE NO: 32

This gene is expressed primarily in resting T cells.

Therefore, polynucleotides and polypeptides of the invention are useful as reagents for differential identification of the tissue(s) or cell type(s) present in a biological sample and for diagnosis of diseases and conditions, which include, but are not limited to, inflammatory and general immune disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis and treatment of disorders of immune system.

### FEATURES OF PROTEIN ENCODED BY GENE NO: 33

This gene is believed to reside on chromosome 1. Accordingly, polynucleotides derived from this gene are useful in linkage analysis as chromosome 1 markers.

This gene is expressed primarily in prostate and to a lesser extent in soares adult brain, human umbilical vein endothelial cells, and amniotic cells.

Therefore, polynucleotides and polypeptides of the invention are useful as reagents for differential identification of the tissue(s) or cell type(s) present in a biological sample and for diagnosis of diseases and conditions, which include, but are not limited to, prostate-related disorders. Similarly, polypeptides and antibodies directed to these polypeptides are useful in providing immunological probes for differential identification of the tissue(s) or cell type(s). For a number of disorders of the above tissues or cells, particularly of the urinary system and nervous system expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that the protein products of this gene are useful for the diagnosis and treatment of disorders of the urinary and nervous systems.

### FEATURES OF PROTEIN ENCODED BY GENE NO: 34

This gene shares sequence homology with R05G6.4 gene product. (See Accession No. gil1326338.) This gene also shares sequence homology with the cyclophilin-like protein CyP-60. (See Accession No. 1199598, see also Biochem. J. 314 (1), 313-319 (1996).) Preferred polypeptide fragments comprise the amino acid sequence:
AVYTYHEKKKDTAASGYGTQNIRLSRDAVKDFDCCCLSLQPCHDPWTPDGYL YEREAILEYILHQKKEIARQMKAYEKQRGTRREEQKELQRAASQDHVRGFLEKE SAIVSRP LNPFTAKALSGTSPDDVQPGPSVGPPSKDKDKVLPSFWIPSLTPEAK ATKLEKPSRTVTCPMSGKPLRMSDLTPVHFTPLDSS VDRVGLITRSERYVCAVT RDSLSNATPCAVLRPSGAWTLECVEKLIRKDMVDPVTGDKLTDRDIIVLQRGT (SEQ ID NO:484); YLYEREAILEYILHQKKEIARQMKAYEKQRGTRREEQKELQ RAASQDHVRGFLE (SEQ ID NO:485); and FTAKALSGTSPDDVQPGPSVGPP SKDKDKVLPSFWIPSLTPEAKATKLEKPSRTVTCPMSGKPL (SEQ ID NO:486). Also preferred are polynucleotide fragments that encode these polypeptide fragments.

This gene is expressed primarily in human testis and to a lesser extent in other tissues.

Therefore, polynucleotides and polypeptides of the invention are useful as reagents for differential identification of the tissue(s) or cell type(s) present in a biological sample and for diagnosis of diseases and conditions, which include, but are not limited to, male reproductive disorders and in particular testicular cancer. Similarly, polypeptides and antibodies directed to these polypeptides are useful in providing immunological probes for differential identification of the tissue(s) or cell type(s). For a number of disorders of the above tissues or cells, particularly of the immune system. Expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of disorders of the male reproductive system and in particular of testicular cancer.

### FEATURES OF PROTEIN ENCODED BY GENE NO: 35

The translation product of this gene shares sequence homology with Lpe5p of Saccharomyces cerevisiae which is thought to be important in the metabolism of phospholipids.

This gene is expressed primarily in liver and brain.

Therefore, polynucleotides and polypeptides of the invention are useful as reagents for differential identification of the tissue(s) or cell type(s) present in a biological sample and for diagnosis of diseases and conditions, which include, but are not limited to, metabolic disorders. Similarly, polypeptides and antibodies directed to these polypeptides are useful in providing immunological probes for differential identification of the tissue(s) or cell type(s). For a number of disorders of the above tissues or cells, particularly of the metabolic and nervous systems expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 268 as residues: Pro-14 to Leu-20, Lys-28 to Asn-38, Arg-109 to Arg-114, Lys-119 to Asn-124, Glu-152 to Leu-157, Pro-172 to Val-180.

The tissue distribution and homology to Lpe5p of Saccharomyces cerevisiae indicates that polynucleotides and polypeptides corresponding to this gene are useful for the diagnosis and treatment of metabolic and nervous disorders.

### FEATURES OF PROTEIN ENCODED BY GENE NO: 36

This gene shares sequence homology with the nuclear ribonucleoprotein U (HNRNP U), encoded by *C. elegans* (See Accession gi|1703576.) Preferred polypeptide fragments comprise the amino acid sequence:
MDTSENRPENDVPEPPMPIADQVSNDDRPEGSVEDEEKKESSLPKSFKRKISVV SATKGVPAGNSDTEGGQPGRKRRWGASTATTQKKPSISTTTESLKSLIPDIKPL AGQEAVVDLHADDSRISEDETERNGDDGTHDKGLKICRTVTQVVPAEGQENGQ REEEEEEKEPEAEPPVPPQVSVEVALPPPAEHEVKKVTLGDTLTRRSISQQKSGV SITIDDPVRTAQVPSPPRGKISNIVHISNLVRPFTLGQLKELLGRTGTLVEEAFWI DKIKSHCFVTYSTVEEAVATRTALHGVKWPQSNPKFLCADYAEQDELDYHRGL - LVDRPSETKTEEQGIPRPLHPPPPPPVQPPQHPRAEQREQERAVREQWAERERE MERRERTRSEREWDRDKVREGPRSRSRSRXRRRKERAKSKEKKSEKKEKAQE EPPAKLLDDLFRKTKAAPCIYWLPLTDSQIVQKEAERAERAKEREKRRKEQEEE EQKEREKEAERERNRQLEREKRREHSRERDRERERERERDRGDRDRDRERDRE RGRERDRRDTKRHSRSRSRSTPVRDRGGR (SEQ ID NO:488). Also preferred are the polynucleotide fragments encoding this polypeptide fragments.

This gene is expressed primarily in epididymus.

Therefore, polynucleotides and polypeptides of the invention are useful as reagents for differential identification of the tissue(s) or cell type(s) present in a biological sample and for diagnosis of diseases and conditions, which include, but are not limited to, diseases of the male reproductive system. Similarly, polypeptides and antibodies directed to these polypeptides are useful in providing immunological probes for differential identification of the tissue(s) or cell type(s). For a number of disorders of the above tissues or cells, particularly of the male reproductive system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis and treatment of male reproductive disorders.

### FEATURES OF PROTEIN ENCODED BY GENE NO: 37

This gene is expressed primarily in amygdala.

Therefore, polynucleotides and polypeptides of the invention are useful as reagents for differential identification of the tissue(s) or cell type(s) present in a biological sample and for diagnosis of diseases and conditions, which include, but are not limited to, inflammatory diseases and reproductive disorders. Similarly, polypeptides and antibodies directed to these polypeptides are useful in providing immunological probes for differential identification of the tissue(s) or cell type(s). For a number of disorders of the above tissues or cells, particularly of the amygdala, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of inflammatory diseases and reproductive disorders.

### FEATURES OF PROTEIN ENCODED BY GENE NO: 38

This gene shares sequence homology with human opsonin protein P35 fragment. (See Accession No. R94181.) The opsonin protein activates the phagocytosis of pathogenic microbes by phagocytic cells. Preferred polypeptide fragments comprise the amino acid sequence: GCDSCPPHLPREAFAQDTQAEGECSSRAERADMCPDAP PSQEVPEGPGAAP (SEQ ID NO:489). Also preferred are polynucleotide fragments encoding these polypeptide fragments.

This gene is expressed in immune-related tissues such as thymus, macrophage, T cells and to a lesser extent in many other tissues.

Therefore, polynucleotides and polypeptides of the invention are useful as reagents for differential identification of the tissue(s) or cell type(s) present in a biological sample and for diagnosis of diseases and conditions, which include, but are not limited to, immune disorders and infectious disease. Similarly, polypeptides and antibodies directed to these polypeptides are useful in providing immunological probes for differential identification of the tissue(s) or cell type(s). For a number of disorders of the above tissues or cells, particularly of the immune system and infectious disease, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 271 as residues: Lys-9 to Arg-14, Met-38 to Asp-51.

The tissue distribution indicates that polynucleotides and polypeptides corresponding to this gene are useful for diagnosis and treatment of immune disorders, as well as the treatment and/or diagnosis of infectious disease.

### FEATURES OF PROTEIN ENCODED BY GENE NO: 39

The translation product of this gene shares sequence homology with alpha-2 type I collagen which is thought to be important in tissue repair. (See, e.g., 211607.) Preferred polypeptide fragments comprise the amino acid sequence: PQLPSCGRPW PGTASVFQSHTQGPREDPDPCRAQGSAGTHCPISLSPPRQ (SEQ ID NO:490). Also preferred are the polynucleotide sequences encoding these polypeptide sequences.

This gene is expressed primarily in the brain and to a lesser extent in the kidney and thymus

Therefore, polynucleotides and polypeptides of the invention are useful as reagents for differential identification of the tissue(s) or cell type(s) present in a biological sample and for diagnosis of diseases and conditions, which include, but are not limited to, brain, kidney, and immune disorders. Similarly, polypeptides and antibodies directed to these polypeptides are useful in providing immunological probes for differential identification of the tissue(s) or cell type(s). For a number of disorders of the above tissues or cells, particularly of the brain, kidney, and immune disorders, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to alpha-2 type I collagen indicates that polynucleotides and polypeptides corresponding to this gene are useful for diagnosis and treatment of tissue repair, and brain, kidney, immune disorders.

### FEATURES OF PROTEIN ENCODED BY GENE NO: 40

The translation product of this gene shares sequence homology with mini-collagen which is thought to be important in tissue repair tumor metastasis. (See Accession No. gnl|PID|d1006976.) Preferred polypeptide fragments comprise the amino acid sequence: PGFRGPSGSLGCSFFPRSLGRVLPPGCQRPGAHAD SSPPPTP (SEQ ID NO:491). Also preferred are polynucleotides encoding this polypeptide fragment.

This gene is expressed in ovarian cancer and to a lesser extent in dedritic cells and smooth muscle.

Therefore, polynucleotides and polypeptides of the invention are useful as reagents for differential identification of the tissue(s) or cell type(s) present in a biological sample and for diagnosis of diseases and conditions, which include, but are not limited to, tumor metastasis and tissue repair. Similarly, polypeptides and antibodies directed to these polypeptides are useful in providing immunological probes for differential identification of the tissue(s) or cell type(s). For a number of disorders of the above tissues or cells, particularly of the tumor metastasis and tissue repair, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 273 as residues: Asn-2 to His-11.

The tissue distribution and homology to mini-collegen gene indicates that polynucleotides and polypeptides corresponding to this gene are useful for diagnosis and treatment of tumor metastasis and tissue repair.

### FEATURES OF PROTEIN ENCODED BY GENE NO: 41

This gene shares sequence homology with the HIV TAT protein. (See Accession No. 328416.) Preferred polypeptide fragments comprise the amino acid sequence: EDLKKPDPASLRAASCGEGKKRKACKNCTCGLAEELEKEK SREQMSSQPKSACGNCYLGDAFRCASCPYLGMPAFKPGEKVLLS (SEQ ID NO:492); EDLKKPDPASLRAASCGEGKKRKACKNCTCGLAEELEKEK SREQMSSQPKSACGNCYLGDAFRCASCPYLGMPAFKPGEKVLLSDSNLHD (SEQ ID NO:493); CGNCYLGDAFRCASCPYLGMPAFKPGEKVLLSDS (SEQ ID NO:494); SCGEGKKRKACKNCTCGLAEELEKE (SEQ ID NO:495); SQPKSAC GNCYLGDAFRCASC (SEQ ID NO:496); and REAGQNSERQYVS LSRD (SEQ ID NO:497). Also preferred are polynucleotide fragments encoding these polypeptide fragments.

This gene is expressed primarily in the infant brain and to a lesser extent in the breast and testes.

Therefore, polynucleotides and polypeptides of the invention are useful as reagents for differential identification of the tissue(s) or cell type(s) present in a biological sample and for diagnosis of diseases and conditions, which include, but are not limited to, brain, testes and breast disorders. Similarly, polypeptides and antibodies directed to these polypeptides are useful in providing immunological probes for differential identification of the tissue(s) or cell type(s). For a number of disorders of the above tissues or cells, particularly of the brain, testes and breast disorders, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 274 as residues: Pro-7 to Val-15.

The tissue distribution indicates that polynucleotides and polypeptides corresponding to this gene are useful for diagnosis and treatment of brain, testes and breast, and other related disorders.

### FEATURES OF PROTEIN ENCODED BY GENE NO: 42

This gene is expressed primarily in the infant brain, human cerebellum, and to a lesser extent in medulloblastoma.

Therefore, polynucleotides and polypeptides of the invention are useful as reagents for differential identification of the tissue(s) or cell type(s) present in a biological sample and for diagnosis of diseases and conditions, which include, but are not limited to, brain related disorders and medulloblastoma and other brain cancers. Similarly, polypeptides and antibodies directed to these polypeptides are useful in providing immunological probes for differential identification of the tissue(s) or cell type(s). For a number of disorders of the above tissues or cells, particularly of the brain related disorders and brain cancers, including medulloblastoma, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 275 as residues: Thr-41 to Glu-47.

The tissue distribution indicates that polynucleotides and polypeptides corresponding to this gene are useful for diagnosis and treatment of human brain related disorders, brain cancers, and medulloblastoma.

### FEATURES OF PROTEIN ENCODED BY GENE NO: 43

The translation product of this gene shares sequence homology with a phosphotyrosine-independent ligand for the lck SH2 domain which is thought to be important in signal transduction related to phosphotyrosine-independent ligand for the lck SH2 domain. (See Accession No. gi|1184951.) Preferred polypeptide fragments comprise the amino acid sequence: ESSGQARTLADPGPGWPRQQGMCFGSLT GLSTTPHGFLTVSAEADPRLESLSQMLSMGFSDEGGWLTRLLQTKNYDIGAAL DTIQYSKH (SEQ ID NO:498). Also preferred are polynucleotide fragments encoding this polypeptide fragment. It is likely that this gene is a new member of a family of phosphotyrosine-independent ligands for the lck SH2 domains.

This gene is expressed primarily in the placenta and to a lesser extent in endothelial cells and neutrophil.

Therefore, polynucleotides and polypeptides of the invention are useful as reagents for differential identification of the tissue(s) or cell type(s) present in a biological sample and for diagnosis of diseases and conditions, which include, but are not limited to, reproductive, cardiovascular, immune, and infectious diseases. Similarly, polypeptides and antibodies directed to these polypeptides are useful in providing immunological probes for differential identification of the tissue(s) or cell type(s). For a number of disorders of the above tissues or cells, particularly of the cardiovascular, reproductive, and immune system, and infectious diseases, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to a phosphotyrosine-independent ligand for the lck SH2 domain indicates that polynucleotides and polypeptides corresponding to this gene are useful for diagnosis and treatment of cardiovascular, reproductive, and immune system diseases, as well as infectious diseases.

### FEATURES OF PROTEIN ENCODED BY GENE NO: 44

This gene is expressed primarily in the fetal brain, cerebellum and to a lesser extent in the placenta.

Therefore, polynucleotides and polypeptides of the invention are useful as reagents for differential identification of the tissue(s) or cell type(s) present in a biological sample and for diagnosis of diseases and conditions, which include, but are not limited to, neuronal cell related disorders. Similarly, polypeptides and antibodies directed to these polypeptides are useful in providing immunological probes for differential identification of the tissue(s) or cell type(s). For a number of disorders of the above tissues or cells, particularly of the neuronal cell related disorders, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 277 as residues: Thr-20 to Gly-28.

The tissue distribution and homology to proline-rich protein genes indicates that polynucleotides and polypeptides corresponding to this gene are useful for diagnosis and treatment of neuronal cell related disorders.

### FEATURES OF PROTEIN ENCODED BY GENE NO: 45

The translation product of this gene shares sequence homology with precerebellin of human, which is thought to be important in synaptic physiology. (See Accession No. gil180251.) It has been observed that cerebellin-like immunoreactivity is associated with Purkinje cell postsynaptic structures. Thus, it is likely that this gene also have synaptic activity. Preferred polypeptide fragments comprise the amino acid sequence: QEGSEPVLLEGECLVVCEPGRAAAGGPGGAALGEAPPGRVAFXAV RSHHHEPAGETGNGTSGAIYFDQVLVNEGGGFDRASGSFVAPVRGVYSFRFH VVKVYNRQTVQVSLMLNTWPVISAFANDPDVTREAATSSVLLPLDPGDRVSLR LRRGXSTGW (SEQ ID NO:499). Also preferred are polynucleotide fragments encoding these polypeptide fragments.

This gene is expressed primarily in cerebellum and infant brain. By Northern analysis, a single transcript of 2.4 kb was observed in brain tissues.

Therefore, polynucleotides and polypeptides of the invention are useful as reagents for differential identification of the tissue(s) or cell type(s) present in a biological sample and for diagnosis of diseases and conditions, which include, but are not limited to, neuronal cell signal transduction and synaptic physiology. Similarly, polypeptides and antibodies directed to these polypeptides are useful in providing immunological probes for differential identification of the tissue(s) or cell type(s). For a number of disorders of the above tissues or cells, particularly of the neuronal cell signal transduction and synaptic physiology expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to gene or gene family indicates that polynucleotides and polypeptides corresponding to this gene are useful for diagnosis and treatment of neuronal cell related disorders.

### FEATURES OF PROTEIN ENCODED BY GENE NO: 46

This gene is expressed in fetal liver and spleen, and to a lesser extent in bone marrow, umbilical vein, and T cells.

Therefore, polynucleotides and polypeptides of the invention are useful as reagents for differential identification of the tissue(s) or cell type(s) present in a biological sample and for diagnosis of diseases and conditions, which include, but are not limited to, disorders of the immune system, particularly hematopoiesis. Similarly, polypeptides and antibodies directed to these polypeptides are useful in providing immunological probes for differential identification of the tissue(s) or cell type(s). For a number of disorders of the above tissues or cells, particularly of the hematopoiesis and immune disorders, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 279 as residues: Asp-30 to Glu-57.

The tissue distribution indicates that polynucleotides and polypeptides corresponding to this gene are useful for diagnosis and treatment of hematopieotic and immune disorders.

### FEATURES OF PROTEIN ENCODED BY GENE NO: 47

The translation product of this gene shares sequence homology with a 12 kD nucleic acid binding protein of Feline calcivirus which is thought to be important in viral replication. (See Accession No. 59264)

This gene is expressed primarily in human cardiomyopathy and to a lesser extent in T helper cells, fetal brain and synovial sarcoma.

Therefore, polynucleotides and polypeptides of the invention are useful as reagents for differential identification of the tissue(s) or cell type(s) present in a biological sample and for diagnosis of diseases and conditions, which include, but are not limited to, cardiomyopathy as well as viral infection. Similarly, polypeptides and antibodies directed to these polypeptides are useful in providing immunological probes for differential identification of the tissue(s) or cell type(s). For a number of disorders of the above tissues or cells, particularly of the cardiovascular system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 280 as residues: Trp-20 to Cys-26.

The tissue distribution in cardiomyopathy and homology to viral 12 kD nucleic acid binding protein indicates that polynucleotides and polypeptides corresponding to this gene are useful for diagnosis and intervention of cardiomyopathy, including those caused by ischemic, hypertensive, congenital, valvular, or pericardial abnormalities. The gene expression pattern may be the consequence or the cause for these conditions.

### FEATURES OF PROTEIN ENCODED BY GENE NO: 48

The translation product of this gene shares sequence homology with tumor necrosis factor related gene product which is thought to be important in tumor necrosis, bacterial and viral infection, immune diseases and immunoreactions.

This gene is expressed primarily in colon and to a lesser extent in ovarian and breast cancers.

Therefore, polynucleotides and polypeptides of the invention are useful as reagents for differential identification of the tissue(s) or cell type(s) present in a biological sample and for diagnosis of diseases and conditions, which include, but are not limited to, tumors of colon, ovary or breast origins. Similarly, polypeptides and antibodies directed to these polypeptides are useful in providing immunological probes for differential identification of the tissue(s) or cell type(s). For a number of disorders of the above tissues or cells, particularly of the colon, ovary and breast, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to Tumor necrosis factors indicates that polynucleotides and polypeptides corresponding to this gene are useful for intervention of cancers of colon, ovary and breast origins, because TNF family members are known to be involved in the tumor development.

### FEATURES OF PROTEIN ENCODED BY GENE NO: 49

The translation product of this gene shares sequence homology with mucins, such as epithelial mucin, which is thought to be important in extracellular matrix functions such as protection, lubrication and cell adhesion (See for example Accession No. R68002). Preferred polypeptide fragments comprise the following amino acid sequence: PRSRPALRPGRQRPPSHSATSGVLRPRKKPDP (SEQ ID NO:500). Also preferred are polynucleotide fragments encoding these polypeptide fragments. Moreover, this gene maps to chromosome 22q11.2-qter, and therefore, can be used as a marker in linkage analysis for chromosome 22.

This gene is expressed primarily in corpus colosum.

Therefore, polynucleotides and polypeptides of the invention are useful as reagents for differential identification of the tissue(s) or cell type(s) present in a biological sample and for diagnosis of diseases and conditions, which include, but are not limited to, tumors, especially of corpus colosum, as well as metastatic lesions. Similarly, polypeptides and antibodies directed to these polypeptides are useful in providing immunological probes for differential identification of the tissue(s) or cell type(s). For a number of disorders of the above tissues or cells, particularly of the corpus colosum and other solid tissues, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to mucins indicates that polynucleotides and polypeptides corresponding to this gene are useful for serum tumor markers or immunotherapy targets because tumor cells have greatly elevated level of mucin expression and shed the molecules into the epithelial tissues.

### FEATURES OF PROTEIN ENCODED BY GENE NO: 50

This gene is expressed primarily in CD34 depleted buffy coat cord blood and primary dendritic cells.

Therefore, polynucleotides and polypeptides of the invention are useful as reagents for differential identification of the tissue(s) or cell type(s) present in a biological sample and for diagnosis of diseases and conditions, which include, but are not limited to, hematopoietic disorders and immunological disorders. Similarly, polypeptides and antibodies directed to these polypeptides are useful in providing immunological probes for differential identification of the tissue(s) or cell type(s). For a number of disorders of the above tissues or cells, particularly of the hematopoietic and immune systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution in CD34 depleted buffy coat cord blood and primary dendritic cells indicates that polynucleotides and polypeptides corresponding to this gene are useful for diagnosis and treatment of hematopoietic and immune disorders. Secreted or cell surface proteins in the above tissue distribution often are involved in cell activation (e.g. cytokines) or molecules involved in cell surface activation.

### FEATURES OF PROTEIN ENCODED BY GENE NO: 51

The translation product of this gene shares sequence homology with Interferon induced 1-8 gene encoded polypeptide which is thought to be important in binding to retroviral rev responsive element. Preferred polypeptide fragment comprise the following amino acid sequences: MTLITPSXKLTFXKGNKSWSSRACSSTLVDP (SEQ ID NO:501). Also preferred are polynucleotide fragments encoding these polypeptide fragments.

This gene is expressed primarily in CD34 positive cells and neutrophils.

Therefore, polynucleotides and polypeptides of the invention are useful as reagents for differential identification of the tissue(s) or cell type(s) present in a biological sample and for diagnosis of diseases and conditions, which include, but are not limited to, retroviral infection, such as AIDS, and other immune disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 284 as residues: Gln-51 to Trp-62.

The tissue distribution and homology to interferon induced gene 1-8 indicates that polynucleotides and polypeptides corresponding to this gene are useful for intervention of retroviral infection including HIV. The factor may be involved in viral stability or viral entry into the cells. Alternatively, the virus/factor complex may elicit the cellular immune reaction.

### FEATURES OF PROTEIN ENCODED BY GENE NO: 52

This gene shares sequence homology to immunoglobulin lambda chain (See Accession No. 2865484). Therefore it is likely that this gene has activity similar to an immunoglobulin lambda chain. Preferred polypeptide fragments comprise the following amino acid sequence: GHPSPALSIAPSDGSQLPCDEVPYGEAHVTRYCKKPLTNS HLETEAQSSSL (SEQ ID NO:502). Also preferred are polynucleotide fragments encoding these polypeptide fragments.

This gene is expressed primarily in Hodgkin's lymphoma.

Therefore, polynucleotides and polypeptides of the invention are useful as reagents for differential identification of the tissue(s) or cell type(s) present in a biological sample and for diagnosis of diseases and conditions, which include, but are not limited to, Hodgkin's lymphoma and other immune disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 285 as residues: Pro-27 to Thr-32.

The tissue distribution in Hodgkin's lymphoma and the sequence homology indicates that polynucleotides and polypeptides corresponding to this gene are useful for diagnosis of Hodgkin's lymphoma, since the elevated expression and secretion by the tumor mass may be indicative of tumors of this type. Additionally the gene product may be used as a target in the immunotherapy of the cancer.Because the gene is expressed in cells of lymphoid origin, the natural gene product may be involved in immune functions. Therefore it may be also used as an agent for immunological disorders including arthritis, asthma, immune deficiency diseases such as AIDS, and leukemia.

### FEATURES OF PROTEIN ENCODED BY GENE NO: 53

This gene has extensive homology to cDNA for Homo sapiens mRNA for the ISLR gene(See Accession No. AB003184). This protein is considered to be a new member of the Ig superfamily and contains a leucine-rich repeat (LRR) with conserved flanking sequences and a C2-type immunoglobulin (Ig)-like domain. These domains are important for protein-protein interaction or cell adhesion, and therefore it is possible that the novel protein ISLR may also interact with other proteins or cells. The ISLR gene was mapped on human chromosome 15q23-q24 by fluorescence in situ hybridization (See Medline Article No. 97468140). Homology to the ISLR gene has been confirmed by another independent group as well (See Accession No. Hs.102171)

This gene is expressed in a number of tissues including human retina, heart, skeletal muscle, prostate, ovary, small intestine, thyroid, adrenal cortex, testis, stomach, spinal cord, fetal lung and fetal kidney tissues, colon, tonsil and stomach cancer, and to a lesser extent in endometrial stromal cells treated with estradiol, breast tissue, synovium, lymphoma, and number of other tumors.

Therefore, polynucleotides and polypeptides of the invention are useful as reagents for differential identification of the tissue(s) or cell type(s) present in a biological sample and for diagnosis of diseases and conditions, which include, but are not limited to, tumors of colon, ovary and breast origins. However, due to the wide range of expression in various tissues, protein may play a vital role in the development of cancer in other tissues as well, not just those mentioned above. Similarly, polypeptides and antibodies directed to these polypeptides are useful in providing immunological probes for differential identification of the tissue(s) or cell type(s). For a number of disorders of the above tissues or cells, particularly of the colon, ovary and breast, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Additionally, this gene maps to chromosome 15q23-q24, and therefore, can be used as a marker in linkage analysis for chromosome 15.

The tissue distribution in tumors of colon, ovary, and breast origins indicates that polynucleotides and polypeptides corresponding to this gene are useful for diagnosis and intervention of these tumors, in addition to other tumors where expression has been indicated. Protein, as well as, antibodies directed against the protein may show utility as a tumor marker and/or immunotherapy targets for the above listed tumors and tissues.

### FEATURES OF PROTEIN ENCODED BY GENE NO: 54

Gene has homology to multidrug resistance gene 1 (See Accession No. P06795). Preferred polynucleotide fragments comprise the following sequence:
GCTTCGTGTCCAACCCTCTTGCCCTTCGCCTGTGTGCCTGGAGCCAGTCCCA CCACGCTCGCGTTTCCTCCTGTAGTGCTCACAGGTCCCAGCACCGATGGCA TTCCCTTTGCCCTGAGTCTGCAGCGGGTCCCTTTTGTGCTTCCTTCCCCTCA GGTAGCCTCTCTCCCCCTGGGCCACTCCCGGGGGTGAGGGGGTTACCCCTT CCCAGTGTTTTTTATTCCTGTGGGGCTCACCCCAAAGTATTAAAAGTAGCTTT GTAA (SEQ ID NO:503). Also preferred are polypeptide fragments encoded by these polynucleotide fragments.

This gene is expressed primarily in lung, esophagus, leukemia (Jurkat cells) and breast cancers and to a lesser extent in macrophages treated with GM-CSF fetal tissues and wide range of tissues.

Therefore, polynucleotides and polypeptides of the invention are useful as reagents for differential identification of the tissue(s) or cell type(s) present in a biological sample and for diagnosis of diseases and conditions which include, but are not limited to, cancer of wide range of origins. Similarly, polypeptides and antibodies directed to these polypeptides are useful in providing immunological probes for differential identification of the tissue(s) or cell type(s). For a number of disorders of the above tissues or cells, particularly of the solid tumors, lung and leukemia, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Furthermore, due to the high expression level in lung tissue and the proposed function of the multidrug resistance protein 1 gene as the efflux pump responsible for low-drug accumulation in multidrug-resistant cells, protein as well mutants thereof, may also be beneficial as a target for gene therapy, particularly for the chronic patient. Preferred epitopes include those comprising a sequence shown in SEQ ID NO: 287 as residues: Met-1 to Lys-16.

The tissue distribution in wide range of cancers and fetal tissues indicates that polynucleotides and polypeptides corresponding to this gene are useful for detection of cells in active proliferation, such as cancers. The gene products may be used for cancer markers or immunotherapy target.

### FEATURES OF PROTEIN ENCODED BY GENE NO: 55

This gene maps to the X chromosome.

This gene is expressed primarily in the brain and to a lesser extent in the developing embryo.

Therefore, polynucleotides and polypeptides of the invention are useful as reagents for differential identification of the tissue(s) or cell type(s) present in a biological sample and for diagnosis of diseases and conditions, which include, but are not limited to, neurodegenerative disease states and developmental disorders. Similarly, polypeptides and antibodies directed to these polypeptides are useful in providing immunological probes for differential identification of the tissue(s) or cell type(s). For a number of disorders, including sex-linked disorders, of the above tissues or cells, particularly of the neurological, developmental systems, and cardiovascular system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Moreover, this gene maps to the X chromosome, and therefore, may be used as a marker in linkage analysis for this chromosome.

The tissue distribution indicates that polynucleotides and polypeptides corresponding to this gene are useful for the detection/treatment of neurodegenerative disease states and behavioral disorders such as Alzheimer's Disease, Parkinson's Disease, Huntington's Disease, Klinefelter's, schizophrenia, mania, dementia, paranoia, obsessive compulsive disorder and panic disorder. In addition, the gene or gene product may also play a role in the treatment and/or detection of developmental disorders associated with the developing embryo, sexually-linked disorders, or disorders of the cardiovascular system.

### FEATURES OF PROTEIN ENCODED BY GENE NO: 56

The translation product of this gene shares sequence homology with paxillin which is thought to be important in mediating signal transduction from growth factor receptors to the cytoskeleton. Preferred polynucleotide fragments comprise the following sequence: TGGCTCACTGTCTTACAATCACTGCTGTGGAATCATGA TACCACITITAGCTCTTTGCATCTTCCTTCAGTGTATITITGTIZTTCAAGAGG AAGTAGATTTTAACTGGACAACTTTGAGTACTGACATCATTGATAAATAAACT GGCTTGTGGTTTCAA (SEQ ID NO:506). Also preferred are polypeptide fragments encoded by these polynucleotide fragments. More preferably, polypeptide fragments comprise the amino acid sequence: LDELMAHLTEMQAKVAVRAD AGKKHLPDKQDHKASLDSMLGGLEQELQDLGIATVPKGHCASCQKPIAGKVI HALGQSWHPEHFVCTHCKEEIGSSPFFERSGLXYCPNDYHQLFSPRCAYCAAP ILDKVLTAMNQTWHPEHFFCSHCGEVFGAEGFHEKDKKPYCRKDFLAMFSPK CGGCNRPVLENYLSAMDTVWHPECFVCGDCFTSFSTGSFFELDGRPFCELHYH HRRGTLCHGCGQPITGRCISAMGYKFHPEHFVCAFCLTQLSKGIFREQNDKTY CQPCFNKLF (SEQ ID NO:507); KASLDSMLGGLEQELQDLGIATVPKGHC ASCQKPIAGKVIHAL (SEQ ID NO:508); CPNDYHQLFSPRCAYCAAPILDKVL TAMNQTWHPEHFFCSHCGEVFGAEG (SEQ ID NO:509); DKKPYCRKDFLAM FSPKCGGCNRPVLENYLSAMDTVWHPECFVCGDCFTSFSTGSFFELDGRPFCE L (SEQ ID NO:510); CGQPITGRCISAMGYKFHPEHFVCAFCLTQLSKGIFRE QNDKTYCQ (SEQ ID NO:511 ). Polynucleotide fragments encoding these preferred polypeptide fragments are also contemplated.

This gene is expressed primarily in brain, and to a lesser extent in the developing embryo.

Therefore, polynucleotides and polypeptides of the invention are useful as reagents for differential identification of the tissue(s) or cell type(s) present in a biological sample and for diagnosis of diseases and conditions, which include, but are not limited to, neurological disease states and developmental abnormalities. Similarly, polypeptides and antibodies directed to these polypeptides are useful in providing immunological probes for differential identification of the tissue(s) or cell type(s). For a number of disorders of the above tissues or cells, particularly of the immune and nervous systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Moreover, since this gene shares homology with a gene that maps to chromosome 11, (See Accession No.T87404), gene as well as its translated product may be used for linkage analysis on chromosome 11.

The tissue distribution and homology to paxillin indicates that polynucleotides and polypeptides corresponding to this gene are useful for the treatment and or detection of disease states associated with abnormal signal transduction in brain and/or the developing embryo. This would include treatment or detection of neurodegenerative disease states and behavioral disorders such as Alzheimer's Disease, Parkinson's Disease, Huntingtons Disease; schizophrenia, mania, dementia, paranoia, obsessive compulsive disorder and panic disorder and also in the treatment and or detection of embryonic development defects.

### FEATURES OF PROTEIN ENCODED BY GENE NO: 57

This gene is expressed primarily in fetal spleen, brain, and to a lesser extent in six week old embryo.

Therefore, polynucleotides and polypeptides of the invention are useful as reagents for differential identification of the tissue(s) or cell type(s) present in a biological sample and for diagnosis of diseases and conditions, which include, but are not limited to, immune disorders, neurological disorders, and developmental abnormalities. Similarly, polypeptides and antibodies directed to these polypeptides are useful in providing immunological probes for differential identification of the tissue(s) or cell type(s). For a number of disorders of the above tissues or cells, particularly of the immune and developmental systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 290 as residues: Arg-28 to Gly-34.

The expression of this gene in fetal spleen indicates that polynucleotides and polypeptides corresponding to this gene are useful for treatment/detection of immune disorders such as arthritis, asthma, immune deficiency diseases such as AIDS, and leukemia. In addition the expression of this gene in the early embryo, indicates a key role in embryo development and hence the gene or gene product could be used in the treatment and or detection of embryonic development defects. This would include treatment or detection of neurodegenerative disease states and behavioral disorders such as Alzheimer's Disease, Parkinson's Disease, Huntintons Disease, schizophrenia, mania, dementia, paranoia, obsessive compulsive disorder and panic disorder and also in the treatment and or detection of embryonic development defects.

### FEATURES OF PROTEIN ENCODED BY GENE NO: 58

The translation product of this gene shares sequence homology with the gene disrupted in the neurodegenerative disease dentatorubal-pallidoluysian atrophy. Moreover a long open reading fame exists in an alternative frame. Preferred polypeptide fragments comprise the following:
MGSSQSVEIPGGGTEGYHVLRVQENSPGHRAGLEPFFDFIVSINGSRLNKDND TLKDLLKXNVEKPVKMLIYSSKTLELRETSVTPSNLWGGQGLLGVSIRFCSFD GANENVWHVLEVESNSPAALAGLRPHSDYIIGADTVMNESEDLFSLIETHEAKP LKLYVYNTDTDNCREVIITPNSAWGGEGSLGCGIGYGYLHRIPTRPFEEGKKIS LPGQMAGTPITPLKDGFTEVQLSSVNPPSLSPPGTTGIEQSLTGLSISSTPPAVSS VLSTGVPTVPLLPPQVNQSLTSVPPMNPATTLPGLMPLPAGLPNLPNLNLNLPA PHIMPGVGLPELVNPGLPPLPSMPPRNLPGIAPLPLPSEFLPSFPLVPESSSAASS GELLSSLPPTSNAPSDPATTTAKADAASSLTVDVTPPTAKAPTTVEDRVGDSTPV SEKPVSAAVDANASESP (SEQ ID NO:512); SVEIPGGGTEGYHVLRVQENSPGH RAGLEPFFDFIVSINGSRLNKDNDTLKDLLKXNVEKPVKMLIYSSKTLELRETS VTPSNLWGGQGLLGVSIRFCSFDGANENVWH (SEQ ID NO:513); ESNSPAA LAGLRPHSDYIIGADTVMNESEDLFSLIETHEAKPLKLYVYNTDTDNCREVIITP NSAWGGEGSLGCGIGYGYLHRIPTRPFEEGKKISLPGQMAGTPITPLKDGFTEV QLSSVNPPSLSPPGTTGIEQSLTG LSISS (SEQ ID NO:514); RIPTRPFEEGKKI SLPGQMAGTPITPLKDGFTEVQLSSVNPPSLSPPGTTGIEQSLTGLSISSTPPAVS SVLSTGVPTVPLLPPQVNQSLTSVPPMNPATTLPGLMPLPAGLPNLPNLNLNLP APHIMPGVGLPELVNPGLPPLPSMPPRN (SEQ ID NO:516); PGLPPLPSMPPRN LPGIAPLPLPSEFLPSFPLVPESSSAASSGELLSSLPPTSNAPSDPATTTAKADAA SSLTVDVTPPTAKAPTTVEDRVGDSTPVSEKPVSAAVDAN (SEQ ID NO:517).

This gene is expressed primarily in prostate cancer, and to a lesser extent in the pineal glands and in fetal lung.

Therefore, polynucleotides and polypeptides of the invention are useful as reagents for differential identification of the tissue(s) or cell type(s) present in a biological sample and for diagnosis of diseases and conditions, which include, but are not limited to, neurological conditions and pulmonary disorders. Similarly, polypeptides and antibodies directed to these polypeptides are useful in providing immunological probes for differential identification of the tissue(s) or cell type(s). For a number of disorders of the above tissues or cells, particularly of the nervous, pulmonary, and endocrine systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 291 as residues: Asn-9 to Leu-14.

The abundance of this gene in the pineal gland and its homology to a gene disrupted in the neurodegenerative disease state Dentatorubral-pallidoluysian atrophy indicates that this gene may be-useful in the treatment and/or detection of other neurodegenerative disease states and behavioral disorders such as Alzheimer's Disease, Parkinson's Disease, Huntingtons Disease, schizophrenia, mania, dementia, paranoia, obsessive compulsive disorder and panic disorder. The abundance of this gene in fetal lung would suggest that misregulation of the expression of this protein product in the adult could lead to lymphoma or sarcoma formation, particularly in the lung; that it may also be involved in predisposition to certain pulmonary defects such as pulmonary edema and embolism, bronchitis and cystic fibrosis; and thus the gen or the gene protein encoded by the gene could be used in the detection and/or treatment of these pulmonary disorders.

### FEATURES OF PROTEIN ENCODED BY GENE NO: 59

This gene is expressed primarily in the developing embryo.

Therefore, polynucleotides and polypeptides of the invention are useful as reagents for differential identification of the tissue(s) or cell type(s) present in a biological sample and for diagnosis of diseases and conditions, which include, but are not limited to, developmental abnormalities. Similarly, polypeptides and antibodies directed to these polypeptides are useful in providing immunological probes for differential identification of the tissue(s) or cell type(s). For a number of disorders of the above tissues or cells, particularly of the developmental system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The expression of this gene primarily in the embryo, indicates the gene plays a key role in embryo development and that the gene or the protein encoded by the gene could be used in the treatment and or detection of developmental defects in the embryo or in infants.

### FEATURES OF PROTEIN ENCODED BY GENE NO: 60

This gene displays homology to nestin, an intermediate filament protein, the expression of which correlates with the proliferation of Central Nervous System progenitor cells and that is useful in the identification of brain tumors. This gene maps to chromosome 1, and therefore, may be used as a marker in linkage analysis for chromosome 1 (See Accession No. AA527348).

This gene is expressed primarily in kidney and to a lesser extent in brain.

Therefore, polynucleotides and polypeptides of the invention are useful as reagents for differential identification of the tissue(s) or cell type(s) present in a biological sample and for diagnosis of diseases and conditions, which include, but are not limited to, renal disorders and neurodegenerative conditions. Similarly, polypeptides and antibodies directed to these polypeptides are useful in providing immunological probes for differential identification of the tissue(s) or cell type(s). For a number of disorders of the above tissues or cells, particularly of the excretory and nervous systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 293 as residues: Thr-128 to Asn-135.

The tissue distribution and homology to nestin indicates that polynucleotides and polypeptides corresponding to this gene are useful for detection and/or treatment of neurodegenerative disease states and behavioral disorders such as Alzheimer's Disease, Parkinson's Disease, Huntingtons Disease, schizophrenia, mania, dementia, paranoia, obsessive compulsive disorder and panic disorder. In addition, its abundance in kidney indicates that it is useful in the treatment and detection of acute renal failure and other disease states associated with the kidney.

### FEATURES OF PROTEIN ENCODED BY GENE NO: 61

Gene shares homology with the latrophilin-related protein 1 precursor as well as the calcium-independent alpha-latrotoxin receptor. Preferred polypeptide fragments comprise the following amino acid sequence:
IYKVFRHTAGLKPEVSCFENIRSCARXXXXXXXXXXXXWIFGVLHVVHASVV TAYLFTVSNAFQGMFIFLFLCVLSRKIQEEYYRLFKNVPCC (SEQ ID NO:518); WIFGVLHVVHASVVTAYLFTVSNAFQGMFIFLFLCVLSRKIQEEYYRLFKNVPC C (SEQ ID NO:519). Also preferred are polynucleotide fragments encoding these polypeptide fragments. (See Accession No. 2213659) The translation product of this gene shares sequence homology with CD 97, a seven transmembrane bound receptor.

This gene is expressed primarily in infant brain and in endothelial cells.

Therefore, polynucleotides and polypeptides of the invention are useful as reagents for differential identification of the tissue(s) or cell type(s) present in a biological sample and for diagnosis of diseases and conditions, which include, but are not limited to, neurological disorders and hematopoeitic disorders. Similarly, polypeptides and antibodies directed to these polypeptides are useful in providing immunological probes for differential identification of the tissue(s) or cell type(s). For a number of disorders of the above tissues or cells, particularly of the neurological and hematopoeitic systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 294 as residues: Lys-13 to Leu-21.

The tissue distribution of this gene suggest that it may be useful in the detection and/or treatment of neurodegenerative disease states and behavioral disorders such as Alzheimer's Disease, Parkinson's Disease, Huntingtons Disease, schizophrenia, mania, dementia, paranoia, obsessive compulsive disorder and panic disorder, while its expression in hematopoietic cell types indicates that the gene could be important for the treatment or detection of immune or hematopoietic disorders including arthritis, asthma and immunodeficiency diseases.

### FEATURES OF PROTEIN ENCODED BY GENE NO: 62

This gene is expressed primarily in fetal liver and fetal spleen.

Therefore, polynucleotides and polypeptides of the invention are useful as reagents for differential identification of the tissue(s) or cell type(s) present in a biological sample and for diagnosis of diseases and conditions, which include, but are not limited to, hematological and immunological disorders. Similarly, polypeptides and antibodies directed to these polypeptides are useful in providing immunological probes for differential identification of the tissue(s) or cell type(s). For a number of disorders of the above tissues or cells, particularly of the immune and hematopoetic systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 295 as residues: Ser-91 to Lys-98.

The tissue distribution of this gene fetal liver and spleen indicates that the gene could be important for the treatment or detection of immune or hematopoietic disorders including arthritis, leukemia, asthma and immunodeficiency diseases.

### FEATURES OF PROTEIN ENCODED BY GENE NO: 63

Gene shares homology with human serum amyloid protein. Preferred polypeptide fragments comprise the following amino acid sequence:
ALTRIPPGDWVINVTAVSFAGKTTARFFHSSPPSLGDQARTDPGHQRRD (SEQ ID NO:520) (See Accession No. W13671). Also preferred are polynucleotide fragments encoding these polypeptide fragments This gene maps to chromosome 9, and therefore, may be used as a marker in linkage analysis for chromosome 9 (See Accession No. AA004342).

This gene is expressed primarily in fetal liver and spleen.

Therefore, polynucleotides and polypeptides of the invention are useful as reagents for differential identification of the tissue(s) or cell type(s) present in a biological sample and for diagnosis of diseases and conditions, which include, but are not limited to, hematopoietic and immune disorders. Similarly, polypeptides and antibodies directed to these polypeptides are useful in providing immunological probes for differential identification of the tissue(s) or cell type(s). For a number of disorders of the above tissues or cells, particularly of the hematopoietic and immune systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution of this gene in fetal liver-spleen indicates that the gene could be important for the treatment or detection of immune or hematopoietic disorders including arthritis, leukemia, asthma, and immunodeficiency diseases.

### FEATURES OF PROTEIN ENCODED BY GENE NO: 64

This gene maps to chromosome 3, and therefore, may be used as a marker in linkage analysis for chromosome 3 (See Accession No. AA219669).

This gene is expressed specifically in the brain.

Therefore, polynucleotides and polypeptides of the invention are useful as reagents for differential identification of the tissue(s) or cell type(s) present in a biological sample and for diagnosis of diseases and conditions, which include, but are . not limited to, neurodegenerative disease states. Similarly, polypeptides and antibodies directed to these polypeptides are useful in providing immunological probes for differential identification of the tissue(s) or cell type(s). For a number of disorders of the above tissues or cells, particularly of the neurological systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the detection/treatment of neurodegenerative disease states and behavioral disorders such as Alzheimer's Disease, Parkinson's Disease, Huntintons Disease, schizophrenia, mania, dementia, paranoia, obsessive compulsive disorder and panic disorder.

### FEATURES OF PROTEIN ENCODED BY GENE NO: 65

Gene shares homology with a yeast protein. Preferred polypeptide fragments comprise the following amino acid sequence: LQEVNITLPENSVWYERYKFDIP VFHL (SEQ ID NO:521). Also preferred are polynucleotide fragments encoding these polypeptide fragments. (See Accession No. 1332638)

This gene is expressed primarily in fetal tissue (fetus and fetal liver).

Therefore, polynucleotides and polypeptides of the invention are useful as reagents for differential identification of the tissue(s) or cell type(s) present in a biological sample and for diagnosis of diseases and conditions, which include, but are not limited to, liver disorders and cancers (e.g. hepatoblastoma). Similarly, polypeptides and antibodies directed to these polypeptides are useful in providing immunological probes for differential identification of the tissue(s) or cell type(s). For a number of disorders of the above tissues or cells, particularly of the hepatic system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 298 as residues: Asn-59 to Glu-64.

The tissue distribution indicates that polynucleotides and polypeptides corresponding to this gene are useful for the detection and treatment of liver disorders and cancers (e.g. hepatoblastoma, jaundice, hepatitis, liver metabolic diseases and conditions that are attributable to the differentiation of hepatocyte progenitor cells). In addition the expression in fetus would suggest a useful role for the protein product in developmental abnormalities, fetal deficiencies, pre-natal disorders and various would-healing models and/or tissue trauma.

### FEATURES OF PROTEIN ENCODED BY GENE NO: 66

Gene has homology with a B-cell surface antigen which may indicate gene plays a role in the immune response, including, but not limited to disorders and infections of the immune system. Preferred polynucleotide fragments comprise the following sequence: TAGCATGTAGCCAGTCGAATAACNTATAAGGACAAAGTGGAGTC CACGCGTGCGGCCGTCTAGACTAGTGGATCCCCCGGCTGCAGGATTCGGC ACGAG (SEQ ID NO:523). Also preferred are polypeptide fragments encoded by these polynucleotide fragments (See Accession No.T94535). Additionally, this gene shares homology with an interferon-gamma receptor. Preferred polypeptide fragments also comprise the following amino acid sequence: MQGSGSQFRACLLCLCFSCPC SPGGPRWNSRQGGRRFPKTCRAISQNLVFKYKTFCPVRYMQPHRSSLCLHFTS YVFILSTWGSLRTYSTDLKKKKKNSRGGPVPIRPKS (SEQ ID NO:522); MQGSGSQFRACLLCLCFSCPCSPGGPRWNSRQGGRRFPKTCRAISQNLVFK (SEQ ID NO:524); PVRYMQPHRSSLCLHFTSYVFILSTWGSLRTYSTDLKKKKK NSRGGPVPIRPKS (SEQ ID NO:525); and GEEQRDCSLGWRGVGMRATHCQAA RMFVLFSLPKYAGL (SEQ ID NO:526). Also preferred are polynucleotide fragments encoding these polypeptide fragments

This gene is expressed primarily in T-cells and gall bladder.

Therefore, polynucleotides and polypeptides of the invention are useful as reagents for differential identification of the tissue(s) or cell type(s) present in a biological sample and for diagnosis of diseases and conditions, which include, but are not limited to, immunological disorders and conditions (immunodeficiencies, cancer, leukemia, hematopoeisis). Similarly, polypeptides and antibodies directed to these polypeptides are useful in providing immunological probes for differential identification of the tissue(s) or cell type(s). For a number of disorders of the above tissues or cells, particularly of the immune and digestive systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 299 as residues: Thr-41 to Gly-52.

The tissue distribution indicates that polynucleotides and polypeptides corresponding to this gene are useful for the treatment and diagnosis of immune disorders including: leukemias, lymphomas, auto-immune disorders, immuno-supressive (transplantation) and immunodeficiencies (e.g. AIDS), inflammation and hematopoeitic disorders. The expression of this gene in gall bladder would suggest a possible role for this gene product in digestive disorders, particularly of the pancreas.

### FEATURES OF PROTEIN ENCODED BY GENE NO: 67

This gene maps to chromosome 11, and therefore, may be used as a marker in linkage analysis for chromosome 11 (See Accession No. AA011622).

This gene is expressed primarily in a variety of fetal and developmental tissues (e.g. fetal spleen, infant brain).

Therefore, polynucleotides and polypeptides of the invention are useful as reagents for differential identification of the tissue(s) or cell type(s) present in a biological sample and for diagnosis of diseases and conditions, which include, but are not limited to, developmental, immune or neurological abnormalities. Similarly, polypeptides and antibodies directed to these polypeptides are useful in providing immunological probes for differential identification of the tissue(s) or cell type(s). For a number of disorders of the above tissues or cells, particularly of the developing immune and central nervous systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 300 as residues: Ser-38 to Ser-43.

The tissue distribution indicates that polynucleotides and polypeptides corresponding to this gene are useful for developmental abnormalities or fetal deficiencies. The detection in infant brain would suggest a role in neurological disorders (both developmental and neurodegenerative conditions of the brain and nervous system, behavioral disorders, depression, schizophrenia, Alzheimer's disease, Parkinson's disease, Huntington's disease, mania, dementia). In addition, the detection in spleen would similarly suggest a role in detection and treatment of immunologically mediated disorders (e.g. immunodeficiency, inflammation, cancer, wound healing, tissue repair, hematopoeisis).

### FEATURES OF PROTEIN ENCODED BY GENE NO: 68

This gene is expressed primarily in spleen, T-cells, and fetal heart.

Therefore, polynucleotides and polypeptides of the invention are useful as reagents for differential identification of the tissue(s) or cell type(s) present in a biological sample and for diagnosis of diseases and conditions, which include, but are not limited to, immunological deficiencies, including AIDSand cardiovascular disorders. Similarly, polypeptides and antibodies directed to these polypeptides are useful in providing immunological probes for differential identification of the tissue(s) or cell type(s). For a number of disorders of the above tissues or cells, particularly of the immune and cardiovascular systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis and treatment of immune disorders including: leukemias, lymphomas, autoimmune disorders, immunodeficiencies (e.g. AIDS), immune-suppressive conditions (transplantation) and hematopoeitic disorders. The expression in fetal heart indicates that polynucleotides and polypeptides corresponding to this gene are useful for the treatment and diagnosis of cadiovascular disorders (e.g. heart disease, restenosis, atherosclerosis, stoke, angina, thrombosis).

### FEATURES OF PROTEIN ENCODED BY GENE NO: 69

Gene shares homology with a human collagen protein. Preferred polypeptide fragments comprise the following amino acid sequence:
MPRKTSKCRQLLCSGASRNADTAARQSTCSSHRPPGKIPSLGPRRXPGCXSVP SSRGEQSTGSPAAPRCGRRDAHRGLPGGAAMTPGDTWASFNPRAGHSKSQGE GQESSGASRQDRHPVSHWVERQREAWGAPRSSSAGGVKVAATTEREPEFKIK TGKA (SEQ ID NO:527); CSGASRNADTAARQSTCSSHRPPGKIPSLGPRRXPG CXSVPSSRGEQSTGSPAAPRCGRRDAHRGLPGGAAMTPGDTWASFNPRAGHS (SEQ ID NO:528); QGEGQESSGASRQDRHPVSHWVERQREAWGAPRSSSAGG VKVAATTEREPEFKIKTGKA (SEQ ID NO:529) (See Accession No. 124886). Also preferred are polynucleotide fragments encoding these polypeptide fragments.

This gene is expressed primarily in fetal heart.

Therefore, polynucleotides and polypeptides of the invention are useful as reagents for differential identification of the tissue(s) or cell type(s) present in a biological sample and for diagnosis of diseases and conditions, which include, but are not limited to, cardiovascular disorders. Similarly, polypeptides and antibodies directed to these polypeptides are useful in providing immunological probes for differential identification of the tissue(s) or cell type(s). For a number of disorders of the above tissues or cells, particularly of the cardiovascular system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 302 as residues: Pro-32 to Ser-39.

The tissue distribution indicates that polynucleotides and polypeptides corresponding to this gene are useful for the treatment and diagnosis of cadiovascular disorders (e.g. heart disease; restenosis, atherosclerosis, stroke, angina, thrombosis).

### FEATURES OF PROTEIN ENCODED BY GENE NO: 70

The translation product of this gene shares sequence homology with a chicken single-strand DNA-binding protein. Preferred polypeptide fragments comprise the following amino acid sequence:
MSPRYPGGPRPPLRIPNQALGGVPGSQPLLPSGMDPTRQQGHPNMGGPMQRM TPPRGMVPLGPQNYGGAMRPPLNALGGPGMPGMNMGPGGGRPWPNPTNAN SIPYSSASPGNYVGPPGGGGPPGTPIMPSPADSTNSGDNMYTLMNAVPPGPNR PNFPMGPGSDGPMGGLGGMESHHMNGSLGSGDMDSISKNSPNNMSLSNQP GTPRDDGEMGGNFLNPFQSESYSPSMTMSV (SEQ ID NO:530); MSPRYPGG PRPPLRIPNQALGGVPGSQPLLPSGMDPTRQQGHPNMGGPMQRMTPPRGMVP LGPQNYGGAMRPPLNALGGPGMPGMNMGPGGGRPWPNPTNANSIPYSSASP GNY (SEQ ID. NO:531); LNALGGPGMPGMNMGPGGGRPWPNPTNANSIPYSS ASPGNYVGPPGGGGPPGTPIMPSPADSTNSGDNMYTLMNAVPPGPN (SEQ ID NO:532); GPMGGLGGMESHHMNGSLGSGDMDSISKNSPNNMSLSNQPGTPR DDGEMGGNFLNPFQSESYSPSMTMSV (SEQ ID NO:533); TCEHSSEAKAFHDY (SEQ ID NO:534). Also preferred are polynucleotide fragments encoding these polypeptide fragments. (See Accession No. 1562534)

This gene is expressed primarily in placenta and to a lesser extent in the fetal heart and a variety of other tissues and cell types.

Therefore, polynucleotides and polypeptides of the invention are useful as reagents for differential identification of the tissue(s) or cell type(s) present in a biological sample and for diagnosis of diseases and conditions, which include, but are not limited to, developmental abnormalities, fetal deficiencies, and particularly of the cardiovascular system. Similarly, polypeptides and antibodies directed to these polypeptides are useful in providing immunological probes for differential identification of the tissue(s) or cell type(s). For a number of disorders of the above tissues or cells, particularly of the reproductive system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the detection and treatment of developmental abnormalities or fetal deficiencies, ovarian and other endometrial cancers, reproductive dysfunction, cardiovascular disorders, and pre-natal disorders.

### FEATURES OF PROTEIN ENCODED BY GENE NO: 71

This gene is expressed primarily in fetal liver and to a lesser extent in the breast and testes.

Therefore, polynucleotides and polypeptides of the invention are useful as reagents for differential identification of the tissue(s) or cell type(s) present in a biological sample and for diagnosis of diseases and conditions, which include, but are not limited to, liver disorders (including hepatoblastomas) and reproductive disorders. Similarly, polypeptides and antibodies directed to these polypeptides are useful in providing immunological probes for differential identification of the tissue(s) or cell type(s). For a number of disorders of the above tissues or cells, particularly of the hepatic and reproductive systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for detection and treatment of liver disorders and cancers (e.g. hepatoblastoma, jaundice, hepatitis, liver metabolic diseases and conditions that are attributable to the differentiation of hepatocyte progenitor cells). The expression in testes and breast indicates that polynucleotides and polypeptides corresponding to this gene are useful for the detection and treatment of endocrine and reproductive disorders (e.g. sperm maturation, milk production, testicular and breast cancers).

### FEATURES OF PROTEIN ENCODED BY GENE NO: 72

This gene maps to chromosome 1, and therefore, may be used as a marker in linkage analysis for chromosome 1 (See Accession No. W93595).

This gene is expressed primarily in smooth muscle and to a lesser extent in brain.

Therefore, polynucleotides and polypeptides of the invention are useful as reagents for differential identification of the tissue(s) or cell type(s) present in a biological sample and for diagnosis of diseases and conditions, which include, but are not limited to, cardiovascular and neurological disorders. Similarly, polypeptides and antibodies directed to these polypeptides are useful in providing immunological probes for differential identification of the tissue(s) or cell type(s). For a number of disorders of the above tissues or cells, particularly of the cardiovascular and central nervous systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the detection and treatment of restenosis, atherosclerosis, stroke, angina, thrombosis, wound healing and other conditions of heart disease. In addition, the expression in brain would suggest that polynucleotides and polypeptides corresponding to this gene are useful for the detection and treatment of developmental, degenerative and behavioral conditions of the brain and nervous system (e.g. schizophrenia, depression, Alzheimer's disease, Parkinson's disease, Huntington's disease, mania, dementia, paranoia, addictive behavior and sleep disorders).

### FEATURES OF PROTEIN ENCODED BY GENE NO: 73

Gene shares homology with human stromalin-2. Preferred polypeptide fragments comprise the following amino acid sequence:
QAFVLLSDLLLIFSPQMIVGGRDFLRPLVFFPEATLQSELASFLMDHVFIQPGDL GSGA (SEQ ID NO:535); ACSYLLCNPEFTFFSRADFARSQLVDLLTDRFQQE LEELLQVG (SEQ ID NO:536),QKQLSSLRDRMVAFCELCQSCLSDVDTEIQEQV ST (SEQ ID NO:537); QVILPALTLVYFSILWTLTHISKSDAS (SEQ ID NO:538); STHDLTRWELYEPCCQLLQKAVDTGXVPHQV (SEQ ID NO:539). Also preferred are polynucleotide fragments encoding these polypeptide fragments (See Accession No.R65208 ) This gene maps to chromosome 7, and therefore, may be used as a marker in linkage analysis for chromosome 7 (See Accession No. D52585).

This gene is expressed primarily in the brain (infant brain, adult brain, pituitary, cerebellum, hippocampus, schizophrenic hypothalmus, amygdala).

Therefore, polynucleotides and polypeptides of the invention are useful as reagents for differential identification of the tissue(s) or cell type(s) present in a biological sample and for diagnosis of diseases and conditions, which include, but are not limited to, developmental and neurodegenerative diseases of the brain and nervous system. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 306 as residues: Thr-25 to Lys-36, Lys-55 to Ser-63.

The tissue distribution indicates that polynucleotides and polypeptides corresponding to this gene are useful for detection and treatment of developmental, degenerative and behavioral conditions of the brain and nervous system (e.g. schizophrenia, depression, Alzheimer's disease, Parkinson's disease, Huntington's disease, mania, dementia, paranoia, addictive behavior and sleep disorders).

### FEATURES OF PROTEIN ENCODED BY GENE NO: 74

This gene is expressed primarily in the hypothalamus of a human suffering from schizophrenia.

Therefore, polynucleotides and polypeptides of the invention are useful as reagents for differential identification of the tissue(s) or cell type(s) present in a biological sample and for diagnosis of diseases and conditions, which include, but are not limited to, disorders of the CNS particularly schizophrenia. Similarly, polypeptides and antibodies directed to these polypeptides are useful in providing immunological probes for differential identification of the tissue(s) or cell type(s). For a number of disorders of the above tissues or cells, particularly of the CNS, such as schizophrenia expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 307 as residues: Gly-38 to Ala-44.

The tissue distribution indicates that the protein products of this gene are useful for the study, diagnosis and treatment of schizophrenia and other disorders involving the CNS.

### FEATURES OF PROTEIN ENCODED BY GENE NO: 75

Preferred polypeptides of the invention comprise the following amino acid sequence encoded by this gene:
LAVSTSFICCADISTALPLGSSRPAPAPRHREHEHGHQARPPRLLXTSLMPLSTP AAAQLLWTQLTPMGGRPGGRHSPPTLHTGPRALPPGPPHPSLHVAALSLLR (SEQ ID NO:540). Polynucleotides encoding such polypeptides are also provided.

This gene is expressed primarily in endometrial tumor and to a lesser extent in amniotic cells.

Therefore, polynucleotides and polypeptides of the invention are useful as reagents for differential identification of the tissue(s) or cell type(s) present in a biological sample and for diagnosis of diseases and conditions, which include, but are not limited to, reproductive and immune disorders particularly cancers of those systems. Similarly, polypeptides and antibodies directed to these polypeptides are useful in providing immunological probes for differential identification of the tissue(s) or cell type(s). For a number of disorders of the above tissues or cells, particularly of the reproductive and immune systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 308 as residues: Ser-3 to Arg-9.

The tissue distribution indicates that the protein products of this gene are useful for study and treatment of immune and reproductive disorders particularly cancers of those systems.

### FEATURES OF PROTEIN ENCODED BY GENE NO: 76

This gene is expressed primarily in kidney cortex and to a lesser extent in early stage human brain.

Therefore, polynucleotides and polypeptides of the invention are useful as reagents for differential identification of the tissue(s) or cell type(s) present in a biological sample and for diagnosis of diseases and conditions, which include, but are not limited to, renal disorders such as renal cancer. Similarly, polypeptides and antibodies directed to these polypeptides are useful in providing immunological probes for differential identification of the tissue(s) or cell type(s). For a number of disorders of the above tissues or cells, particularly of the kidney expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 309 as residues: Gly-38 to Gly-45, Gly-47 to Gly-52, Pro-92 to Lys-110.

The tissue distribution indicates that the protein products of this gene are useful for study, treatment and diagnosis of renal diseases such as cancer of the kidney.

### FEATURES OF PROTEIN ENCODED BY GENE NO: 77

This gene is expressed primarily in kidney medulla.

Therefore, polynucleotides and polypeptides of the invention are useful as reagents for differential identification of the tissue(s) or cell type(s) present in a biological sample and for diagnosis of diseases and conditions, which include, but are not limited to, metabolic and renal disorders. Similarly, polypeptides and antibodies directed to these polypeptides are useful in providing immunological probes for differential identification of the tissue(s) or cell type(s). For a number of disorders of the above tissues or cells, particularly of the metabolic and renal systems, expression of this gene at significantly higher or lower levels maybe routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that the protein products of this gene are useful for study, treatment and diagnosis of metabolic and renal diseases and disorders.

### FEATURES OF PROTEIN ENCODED BY GENE NO: 78

This gene is expressed in chronic synovitis and microvascular endothelium.

Therefore, polynucleotides and polypeptides of the invention are useful as reagents for differential identification of the tissue(s) or cell type(s) present in a biological sample and for diagnosis of diseases and conditions, which include, but are not limited to, arthritis and atherosclerosis. Similarly, polypeptides and antibodies directed to these polypeptides are useful in providing immunological probes for differential identification of the tissue(s) or cell type(s). For a number of disorders of the above tissues or cells, particularly of the vascular and skeletal systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that the protein products of this gene are useful for study, diagnosis and treatment of arthritic and other inflammatory diseases as well as cardiovascular diseases.

### FEATURES OF PROTEIN ENCODED BY GENE NO: 79

This gene is expressed in resting T-cells and activated monocytes.

Therefore, polynucleotides and polypeptides of the invention are useful as reagents for differential identification of the tissue(s) or cell type(s) present in a biological sample and for diagnosis of diseases and conditions, which include, but are not limited to, immune disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that the protein products of this gene are useful for the study and treatment of immune diseases such as inflammatory conditions.

### FEATURES OF PROTEIN ENCODED BY GENE NO: 80

This gene is expressed in a variety of immune system tissues, e.g., neutrophils, T-cells, and TNF induced epithelial and endothelial cells.

Therefore, polynucleotides and polypeptides of the invention are useful as reagents for differential identification of the tissue(s) or cell type(s) present in a biological sample and for diagnosis of diseases and conditions, which include, but are not limited to, infectious and immune disorders. Similarly, polypeptides and antibodies directed to these polypeptides are useful in providing immunological probes for differential identification of the tissue(s) or cell type(s). For a number of disorders of the above tissues or cells, particularly of the immune and vascular systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 313 as residues: Met-1 to Trp-6.

The tissue distribution indicates that the protein products of this gene are useful for study and treatment of infectious diseases, immune and vascular disorders.

### FEATURES OF PROTEIN ENCODED BY GENE NO: 81

This gene is expressed in activated neutrophils.

Therefore, polynucleotides and polypeptides of the invention are useful as reagents for differential identification of the tissue(s) or cell type(s) present in a biological sample and for diagnosis of diseases and conditions, which include, but are not limited to, inflammation and other immune conditions. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower-levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that the protein products of this gene are useful for study and treatment of immune disorders.

### FEATURES OF PROTEIN ENCODED BY GENE NO: 82

This gene is expressed in neutrophils.

Therefore, polynucleotides and polypeptides of the invention are useful as reagents for differential identification of the tissue(s) or cell type(s) present in a biological sample and for diagnosis of diseases and conditions, which include, but are not limited to, inflammatory and other immune conditions. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 315 as residues: Ala-83 to Thr-91.

The tissue distribution indicates that the protein products of this gene are useful for study and treatment of immune disorders.

### FEATURES OF PROTEIN ENCODED BY GENE NO: 83

This gene is expressed in human neutrophils.

Therefore, polynucleotides and polypeptides of the invention are useful as reagents for differential identification of the tissue(s) or cell type(s) present in a biological sample and for diagnosis of diseases and conditions, which include, but are not limited to, inflammation and immune disorders. Similarly, polypeptides and antibodies directed to these polypeptides are useful in providing immunological probes for differential identification of the tissue(s) or cell type(s). For a number of disorders of the above tissues or cells, particularly of the immune and inflammatory system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that the protein products of this gene are useful for diagnosis and treatment of disorders of the inflammatory and immune systems.

### FEATURES OF PROTEIN ENCODED BY GENE NO: 84

This gene is expressed in human neutrophils.

Therefore, polynucleotides and polypeptides of the invention are useful as reagents for differential identification of the tissue(s) or cell type(s) present in a biological sample and for diagnosis of diseases and conditions, which include, but are not limited to, disorders of the inflammatory and immune systems. Similarly, polypeptides and antibodies directed to these polypeptides are useful in providing immunological probes for differential identification of the tissue(s) or cell type(s). For a number of disorders of the above tissues or cells, particularly of the inflammatory and immune systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that the protein products of this gene are useful for diagnosis and treatment of disorders of the immune and inflammatory systems.

### FEATURES OF PROTEIN ENCODED BY GENE NO: 85

This gene is expressed in activated neutrophils.

Therefore, polynucleotides and polypeptides of the invention are useful as reagents for differential identification of the tissue(s) or cell type(s) present in a biological sample and for diagnosis of diseases and conditions, which include, but are not limited to, inflammation and immune system diseases. Similarly, polypeptides and antibodies directed to these polypeptides are useful in providing immunological probes for differential identification of the tissue(s) or cell type(s). For a number of disorders of the above tissues or cells, particularly of the immune system and inflammatory system, expression of this gene at significantly higher or lower levels may be routinely detected in certain tissues (e.g., cancerous and wounded tissues) or bodily fluids (e.g., serum, plasma; urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that the protein products of this gene are useful for diagnosis and treatment of diseases of the inflammatory and immune systems.

### FEATURES OF PROTEIN ENCODED BY GENE NO: 86

This gene is expressed in activated neutrophils.

Therefore, polynucleotides and polypeptides of the invention are useful as reagents for differential identification of the tissue(s) or cell type(s) present in a biological sample and for diagnosis of diseases and conditions, which include, but are not limited to, inflammation and immune system disorders. Similarly, polypeptides and antibodies directed to these polypeptides are useful in providing immunological probes for differential identification of the tissue(s) or cell type(s). For a number of disorders of the above tissues or cells, particularly of the inflammatory and immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 319 as residues: Met-1 to Gly-6, Gly-32 to Pro-43, Leu-55 to Gln-60.

The tissue distribution indicates that the protein products of this gene are useful for diagnosis and treatment of disorders of the immune and inflammatory system.

### FEATURES OF PROTEIN ENCODED BY GENE NO: 87

In specific embodiments, polypeptides of the invention comprise the sequence: EQVLALLWPRFELILEMNVQSVRSTDPQRLGGLDTRPHYITRRYAEFSSALVSIN QTIPNERTMQLLGQLQVEVENFVLRVAAEFSSRKEQLVFLINNYDMMLGVLME RAADDSKEVESFQQLLNARTQEFIEELLSPPFGGLVAFVKEAEALIERGQAERLR GEEARVTQLIRGFGSSWKSSVESLSQDVMRSFTNFRNGTSIIQG (SEQ ID NO:541),ALLKYRFFYQFLLGNERATAKEIRDEYVETLSKIYLSYYRSYLGRLMK VQYEEVAEKDDLMGVEDTAKKGFXSKPSRSRNTIFTLGTRGSVISPTELEAPILV PHTAQR (SEQ ID NO: 542); EQRYPFEALFRSQHYXLLDNSCREYLFICEFFVVS GPXAHDLFHAVMGRTLSMTLKHLDSYLADGYDAIAVFLCIHIVLRFRNIAAKRD VPALDRYW (SEQ ID NO:543),GGLDTKPHYITRRYAEFSSALVSINQ (SEQ ID NO:544); SRKEQLVFLINNYDMMLGVL (SEQ ID NO: 545) and/or ALLKYRFFY QFLLGNERATAKEIRDEYVETLSKIYLSYYRSYLGRLMKVQYEEVAEKDDLMG VEDTAKKGFXSKPSLRSRNTIFTLGTRGSVISPTELEAPILVPHTAQRXEQRYPF EALFRSQHYXLLDNSCREYLFICEFFVVSGPXAHDLFHAVMGRTLSMTLKHLD SYLADCYDAIAVFLCIHIVLRFRNIAAKRDVPALDRYWEQVLALLWPRFELILEM NVQSVRSTDPQRLGGLDTRPHYTTRRYAEFSSALVSINQTIPIVERTMQLLGQLQV EVENFVLRVAAEFSSRKEQLVFLINNYDMMLGVLMERAADDSKEVESFQQLLN ARTQEFIEELLSPPFGGLVAFVKEAEALIERGQAERLRGEEARVTQLIRGFGSSW KSSVESLSQDVMRSFTNFRNGTS (SEQ ID NO:546). Polynucleotides encoding these polypeptides are also encompassed by the invention. The translation product of this gene shares sequence homology with suppressor of actin mutation which is thought to be important in mutation suppression.

This gene is expressed primarily in fetal liver and to a lesser extent in a variety of other tissues.

Therefore, polynucleotides and polypeptides of the invention are useful as reagents for differential identification of the tissue(s) or cell type(s) present in a biological sample and for diagnosis of diseases and conditions, which include, but are not limited to, liver and mutations. Similarly, polypeptides and antibodies directed to these polypeptides are useful in providing immunological probes for differential identification of the tissue(s) or cell type(s). For a number of disorders of the above tissues or cells, particularly of the liver or cancer, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 320 as residues: Val-53 to Arg-60, Thr-88 to Thr-94, Ala-142 to Ser-150, Gly-188 to Glu-196, Gly-208 to Ser-214, Thr-227 to Gly-232, Lys-279 to Phe-285.

The tissue distribution and homology to suppressor of actin mutation suggest that polynucleotides and polypeptides corresponding to this gene are useful for diagnosis and of liver disorder or cancer.

### FEATURES OF PROTEIN ENCODED BY GENE NO: 88

This gene maps to chromosome 9, and therefore can be used in linkage analysis as a marker for chromosome 9. In specific embodiments, polypeptides of the invention comprise the sequence:
YEGKEFDYVFSIDVNEGGPSYKLPYNTSDDPWLTAYNFLQKNDLNPMFLDQVA KFIIDNTKGQMLGLGNPSFSDPFTGGGRYVPGSSGSSNTLPTADPFTGAGRYV PGSASMGTTMAGVDPFTGNSAYRSAASKTMNIYFPKKEAVTFDQANPTQILGK LKELNGTAPEEKKLTEDDLILLEKILSLICNSSSEKPTVQQLQILWKAINCPEDIV FPALDILRLSIKHPSVNENFCNEKEGAQFSSHLINLLNPKGKPANQLLALRTFC NCFVGQAGQKLMMSQRESLMSHAIELKSGSNKNI (SEQ ID NO: 547); HIALATLALNYSVCFHKD (SEQ ID NO: 548); HNIEGKAQCLSLISTILEVVQ DLEATFRLLVALGTLISDDSNAVQLAKS (SEQ ID NO:549); LGVDSQIKKYSS VSEPAKVSECCRFILNLL (SEQ ID NO:550); and/or YEGKEFDYVFSIDVNEGGPS YKLPYNTSDDPWLTAYNFLQKNDLNPMFLDQVAKFIIDNTKGQMLGLGNPSFS DPFTGGGRYVPGSSGSSNTLPTADPFTGAGRYVPGSASMGTTMAGVDPFTGN SAYRSAASKTMNIYFPKKEAVTFDQANPTQILGKLKELNGTAPEEKKLTEDDLI LLEKILSLICNSSSEKPTVQQLQILWKAINCPEDIVFPALDILRLSIKHPSVNENFC NEKEGAQFSSHLINLLNPKGKPANQLLALRTFCNCFVGQAGQKLMMSQRESL MSHAIELKSGSNKNIHIALATLALNYSVCFHKDHNIEGKAQCLSLISTILEVVQD LEATFRLLVALGTLISDDSNAVQLAKSLGVDSQIKKYSSVSEPAKVSECCRFILN LL (SEQ ID NO:551). Polynucleotides encoding these polypeptides are also encompassed by the invention. These polypeptides share significant homology with phospholipase A2 activating protein which is thought to be important in signal transduction (see, e.g., Wang et al., Gene 161(2):237-241 (1995)).

This gene is expressed primarily in endothelial cells, to a less extent in placenta, endometrial stromal cells, osteosarcoma, testis tumor, muscle, and infant brain that are likely to be rich in blood vessles.

Therefore, polynucleotides and polypeptides of the invention are useful as reagents for differential identification of the tissue(s) or cell type(s) present in a biological sample and for diagnosis of diseases and conditions, which include, but are not limited to, disorders in vascular system, aberrent angiogenesis, tumor angiogenesis. Similarly, polypeptides and antibodies directed to these polypeptides are useful in providing immunological probes for differential identification of the tissue(s) or cell type(s). For a number of disorders of the above tissues or cells, particularly of the vascular system or tumors, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily - fluid from an individual not having the disorder.

The tissue distribution of this gene in endothelial cells and several potential highly vascularized tissues and its homology to phospholipase A2 activating protein suggest that this gene may be involved in transducing signals for endothelial cells in angiogenesis or vasculogenesis.

### FEATURES OF PROTEIN ENCODED BY GENE NO: 89

In specific embodiments, polypeptides of the invention comprise the sequence: YPNQDGDILRDQVLHEHIQRLSKVVTANHRALQIPEVYLREAPWPSAQSEIRTIS AYKTPRDKVQCILRMCSTIMNLLSLANEDSVPGADDFVPVLVFVLIKANPPCLL STVQYISSFYASCLSGEESYWWMQFTAAVE (SEQ ID NO:552); YPNQDGDILR DQVLHEHIQRLSKVVTANHRALQIPEVYLREAPWPSAQSEIRTISAYKTPRDKVQ CILRMCSTIMNLLSLANEDSVPGADDFVPVLVFVLIKANPPCLLSTVQYISSFYA SCLSGEESYWWMQFTAAVEFIKTI (SEQ ID NO:553); YPNQDGDILRDQVL (SEQ ID NO:554); EAPWPSAQSEI (SEQ ID NO:555); PVLVFVLIKANP (SEQ ID NO:560); SGEESYWWMQFTAAVEFIKTI (SEQ ID NO:556); ADDFVPVLVF VLIKANPP (SEQ ID NO:557); YKTPRDKVQCIL (SEQ ID NO:558); and/or GADDFVPVLVFVLIK (SEQ ID NO:559). The translation product of this gene shares sequence homology with human ras inhibitor and yeast VPS9p which is thought to be important in golgi vacuole transport.

This gene is expressed primarily in T cells and melanocytes and to a lesser extent in a variety of other tissues.

Therefore, polynucleotides and polypeptides of the invention are useful as reagents for differential identification of the tissue(s) or cell type(s) present in a biological sample and for diagnosis of diseases and conditions, which include, but are not limited to, dysfunction and disorders involving T cells and melanocytes. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to ras inhibitor indicates that polynucleotides and polypeptides corresponding to this gene are useful for regulating signal transduction; diagnosis and treatment of disorders involving T cells and melanocytes.

### FEATURES OF PROTEIN ENCODED BY GENE NO: 90

This gene maps to chromosome 9 and therefore polypeptides of the invention can be used in linkage analysis as a marker for chromosome 9. The translation product of this gene shares sequence homology with neuronal olfactomedin-related ER localized protein which is thought to be important in influence the maintenance, growth, or differentiation of chemosensory cilia on the apical dendrites of olfactory neurons. In specific embodiments, polypeptides of the invention comprise the sequence:
SARASTQPPAGQHPGPC (SEQ ID NO:561); MPGRWRWQRDMHPARKLLSLL FLILMGTELTQD (SEQ ID NO:562); SAAPDSLLRSSKGSTRGSL (SEQ ID NO:563); AAIVIWRGKSESRIAKTPGI (SEQ ID NO:564); FRGGGTLVLPPTHT PEWLIL (SEQ ID NO:567); PLGITLPLGAPETGGGD (SEQ ID NO:565); and/or CAAETWKGSQRAGQLCALLA (SEQ ID NO:566).

This gene is expressed in pineal gland.

Therefore, polynucleotides and polypeptides of the invention are useful as reagents for differential identification of the tissue(s) or cell type(s) present in a biological sample and for diagnosis of diseases and conditions, which include, but are not limited to, neurological and endocrinological disorders. Similarly, polypeptides and antibodies directed to these polypeptides are useful in providing immunological probes for differential identification of the tissue(s) or cell type(s). For a number of disorders of the above tissues or cells, particularly of the neurological or endocrine systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 323 as residues: Leu-20 to Ala-26, Arg-32 to Arg-39, Thr-104 to Gly-112.

The tissue distribution and homology to olfactomedin-related protein indicates that polynucleotides and polypeptides corresponding to this gene are useful for maintenance, growth, or differentiation of neuron cells in pineal gland, therefore, may be useful for diagnosis and treatment of neurological disorders in pineal gland.

### FEATURES OF PROTEIN ENCODED BY GENE NO: 91

This gene is expressed primarily in prostate and apoptotic T cells.

Therefore, polynucleotides and polypeptides of the invention are useful as reagents for differential identification of the tissue(s) or cell type(s) present in a biological sample and for diagnosis of diseases and conditions, which include, but are not limited to, prostate disease and T cell dysfunction. Similarly, polypeptides and antibodies directed to these polypeptides are useful in providing immunological probes for differential identification of the tissue(s) or cell type(s). For a number of disorders of the above tissues or cells, particularly of the prostate cancer, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for detect abnormal activity in prostate and T cells or probably treatment of this abnormality.

### FEATURES OF PROTEIN ENCODED BY GENE NO: 92

This gene is expressed primarily in prostate and to a lesser extent in smooth muscle cells, fibroblasts, and placenta.

Therefore, polynucleotides and polypeptides of the invention are useful as reagents for differential identification of the tissue(s) or cell type(s) present in a biological sample and for diagnosis of diseases and conditions, which include, but are not limited to, disorders in prostate or vascular system. Similarly, polypeptides and antibodies directed to these polypeptides are useful in providing immunological probes for differential identification of the tissue(s) or cell type(s). For a number of disorders of the above tissues or cells, particularly of the prosate or vascular system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for regulating function of prostate or highly vascularized tissues, e.g. placenta.

### FEATURES OF PROTEIN ENCODED BY GENE NO: 93

This gene is expressed-primarily in embryos and fetal tissues stage human and to a lesser extent in a wide variety of other proliferative tissues.

Therefore, polynucleotides and polypeptides of the invention are useful as reagents for differential identification of the tissue(s) or cell type(s) present in a biological sample and for diagnosis of diseases and conditions, which include, but are not limited to, disorders in embryonic development and cell proliferation. Similarly, polypeptides and antibodies directed to these polypeptides are useful in providing immunological probes for differential identification of the tissue(s) or cell type(s). For a number of disorders of the above tissues or cells, particularly of the embryonic tissues and proliferative cells, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis or treatment of abnormalities in developing and proliferative cells and organs.

### FEATURES OF PROTEIN ENCODED BY GENE NO: 94

The translation product of this gene shares sequence homology with transformation related protein which is thought to be important in transformation.

This gene is expressed primarily in female reproductive tissues, i.e., breast cancer cells, placenta, and ovary and to a lesser extent in fetal lung.

Therefore, polynucleotides and polypeptides of the invention are useful as reagents for differential identification of the tissue(s) or cell type(s) present in a biological sample and for diagnosis of diseases and conditions, which include, but are not limited to, cancer or dysfunction of reproductive tissues. Similarly, polypeptides and antibodies directed to these polypeptides are useful in providing immunological probes for differential identification of the tissue(s) or cell type(s). For a number of disorders of the above tissues or cells, particularly of the reproduction system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 327 as residues: Ser-50 to Pro-61.

The tissue distribution and homology to transformation related protein indicates that polynucleotides and polypeptides corresponding to this gene are useful for diagnosis and treatment of conditions caused by transformation, i.e. tumorigenesis in reproductive organs, e.g. breast, placenta, and ovary.

### FEATURES OF PROTEIN ENCODED BY GENE NO: 95

This gene is expressed primarily in testes, rhabdomyosarcoma, infant brain and to a lesser extent in some tumors and highly vascularized tissues.

Therefore, polynucleotides and polypeptides of the invention are useful as reagents for differential identification of the tissue(s) or cell type(s) present in a biological sample and for diagnosis of diseases and conditions, which include, but are not limited to, tumorigenesis, abnormal angiogenesis, and/or neurological disorders. , Similarly, polypeptides and antibodies directed to these polypeptides are useful in providing immunological probes for differential identification of the tissue(s) or cell type(s). For a number of disorders of the above tissues or cells, particularly of the tumor tissues or vascular tissues, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 328 as residues: Arg-46 to Trp-54, Pro-60 to Ile-69, Asn-116 to Ala-122, Arg-147 to Lys-153, Ser-158 to Glu-170, Ile-399 to Ser-405, Pro-486 to Met-499, Pro-502 to Asp-508.

The tissue distribution indicates that polynucleotides and polypeptides corresponding to this gene are useful for a range of disease states including treatment of tumor or vascular disorders and the treatment of neurological disorders such as Alzheimer's Disease, Parkinson's Disease, Huntingtons Disease, schizophrenia, mania, dementia; paranoia, obsessive compulsive disorder and panic disorder.

### FEATURES OF PROTEIN ENCODED BY GENE NO: 96

This gene maps to chromosome 7 and therefore polynucleotides of the present invention can be used in linkage analysis as a marker for chromosome 7. The translation product of this gene is homologous to the Clostridium perfringens enterotoxin (CPE) receptor gene product and shares sequence homology with a human ORF specific to prostate and a glycoprotein specific to oligodendrocytes both of which are tissue specific proteins.(See e.g., Katahira et al., J Cell Biol. 136(6):1239-1247 (1997). PMID: 9087440; UI: 97242441.

This gene is expressed primarily in pancreas tumor and ulcerative colitis and to a lesser extent in several tumors and normal tissues.

Therefore, polynucleotides and polypeptides of the invention are useful as reagents for differential identification of the tissue(s) or cell type(s) present in a biological sample and for diagnosis of diseases and conditions, which include, but are not limited to, pancreatic disorder, ulcerative colitis, tumors and food poisoning. Similarly, polypeptides and antibodies directed to these polypeptides are useful in providing immunological probes for differential identification of the tissue(s) or cell type(s). For a number of disorders of the above tissues or cells, particularly of the digestive system or tumorigenic system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 329 as residues: Gly-147 to Met-152, Cys-177 to Lys-188.

The tissue distribution and homology to prostate and oligodendrocyte-specific protein indicates that polynucleotides and polypeptides corresponding to this gene are useful for marker of diagnosis or treatment of disorder in pancreas, ulcerative colitis, and tumors. Furthermore, identity to the human receptor for Clostridium perfringenes entertoxin indicates that the soluble portion of this receptor could be used in the treatment of food poisoning associated with Clostridia perfringens by blocking the activity of perfringens enterotoxin.

### FEATURES OF PROTEIN ENCODED BY GENE NO: 97

The translation product of this gene shares sequence homology with ATPase which is thought to be important in metabolism.

This gene is expressed primarily in testes and several hematopoietic cells and to a lesser extent in other tissues.

Therefore, polynucleotides and polypeptides of the invention are useful as reagents for differential identification of the tissue(s) or cell type(s) present in a biological sample and for diagnosis of diseases and conditions, which include, but are not limited to, leukemia and hematopoietic disorders. Similarly, polypeptides and antibodies directed to these polypeptides are useful in providing immunological probes for differential identification of the tissue(s) or cell type(s). For a number of disorders of the above tissues or cells, particularly of the hematopoietic system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 330 as residues: Leu-37 to Ala-42.

The tissue distribution and homology to ATPase indicates that polynucleotides and polypeptides corresponding to this gene are useful for marker of diagnosis and treatment of leukemia and other hematopoietic disorders.

### FEATURES OF PROTEIN ENCODED BY GENE NO: 98

In specific embodiments, polypeptides of the invention comprise the sequence:
MRSARPSLGCLPSWAFSQALNI (SEQ ID NO:568); LLGLKGLAPAEISAVCE KGNFN (SEQ ID NO:569); VAHGLAWSYYIGYLRLILPELQARIR (SEQ ID NO:570); TYNQHYNNLLRGAVSQRC (SEQ ID NO:571); ILLPLDCGVPDNLSM ADPNIRFLDKLPQQTGDRAGIKDRVYSN (SEQ ID NO:572); SIYELLENGQRAGT CVLEYATPLQTLFAMSQYSQAGFSGEDRLEQ (SEQ ID NO:573); AKLFCRTLE DILADAPESQNNCRLIAYQEPADDSSFSLSQEVLRHLRQEEKEEVTVGSLKTSAV PSTSTMSQEPELLISGMEKPLPLRTDFS (SEQ ID NO:574); and/or LLGLKGLA PAEISAVCEKGNFNVAHGLAWSYYIGYLRLILPEL (SEQ ID NO:575). Polynucleotides encoding these polypeptides are also encompassed by the invention.

This gene is expressed primarily in prostate BPH and to a lesser extent in bone marrow.

Therefore, polynucleotides and polypeptides of the invention are useful as reagents for differential identification of the tissue(s) or cell type(s) present in a biological sample and for diagnosis of diseases and conditions, which include, but are not limited to, benign prostatic hypertrophy or prostate cancer. Similarly, polypeptides and antibodies directed to these polypeptides are useful in providing immunological probes for differential identification of the tissue(s) or cell type(s). For a number of disorders of the above tissues or cells, particularly of the male urinary system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 331 as residues: Ile-60 to Asn-69, Leu-106 to Asp-112, Glu-130 to Gly-136, Phe-160 to Glu-167, Pro-184 to Cys-190, Glu-197 to Ser-202, Arg-215 to Glu-221, Thr-237 to Pro-242.

The tissue distribution indicates that polynucleotides and polypeptides corresponding to this gene are useful for diagnosis or treatment of benign prostatic hypertrophy or prostate cancer.

### FEATURES OF PROTEIN ENCODED BY GENE NO: 99

This gene is expressed primarily in salivary gland.

Therefore, polynucleotides and polypeptides of the invention are useful as reagents for differential identification of the tissue(s) or cell type(s) present in a biological sample and for diagnosis of diseases and conditions, which include, but are not limited to, disorders or injuries of the salivary gland. Similarly, polypeptides and antibodies directed to these polypeptides are useful in providing immunological probes for differential identification of the tissue(s) or cell type(s). For a number of disorders of the above tissues or cells, particularly of glandular tissues, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides, corresponding to this gene are useful for treatment of disorders of, or injuries to the salivary gland or other glandular tissue.

### FEATURES OF PROTEIN ENCODED BY GENE NO: 100

This gene maps to chromosome 15, accordingly, polynucleotides of the invention can be used in linkage analysis as a marker for chromosome 15. The translation product of this gene shares sequence homology with a *C.elegans* gene of unknown function. In specific embodiments, polypeptides of the invention comprise the sequence: DPRVRLNSLTCKHIFISLTQ (SEQ ID NO:583); TMKLLKLRRNIV KLSLYRHFTN (SEQ ID NO:576); TLILAVAASIVFIIWTTMKFRI (SEQ ID NO:577); VTCQSDWRELWVDDAIWRLLFSMILFVI (SEQ ID NO:578); MVLWR PSANNQRFAFSPLSEEEEEDEQ (SEQ ID NO:580); KEPMLKESFEGMKMRS TKQEPNGNSKVNKAQEDDL (SEQ ID NO:584) and/or KWVEENVPSSVTDVALP ALLDSDEERMITHFERSKME (SEQ ID NO:582). Polynucleotides encoding these polypeptides are also encompassed by the invention.

This gene is expressed primarily in thyroid and to a lesser extent in osteoclastoma, kidney medulla, and lung.

Therefore, polynucleotides and polypeptides of the invention are useful as reagents for differential identification of the tissue(s) or cell type(s) present in a biological sample and for diagnosis of diseases and conditions, which include, but are not limited to, thyroid dysfunction or cancer. Similarly, polypeptides and antibodies directed to these polypeptides are useful in providing immunological probes for differential identification of the tissue(s) or cell type(s). For a number of disorders of the above tissues or cells, particularly of the endocrine system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 333 as residues: Lys-107 to Leu-124, Glu-150 to Thr-159, Pro-173 to Asp-179, Ser-192 to Ser-201.

The tissue distribution indicates that polynucleotides and polypeptides corresponding to this gene are useful for diagnosis and treatment of thyroid dysfunction or cancer.

### FEATURES OF PROTEIN ENCODED BY GENE NO: 101

This gene maps to chromosome 16, therefore polynucleotides of the invention can be used in linkage analysis as a marker for chromosome 16. In specific embodiments, polypeptides of the invention comprise the sequence:
IRHELTVLRDTRPACA (SEQ ID NO:585); and/or MDFXMALIYD (SEQ ID NO:586). Polynucleotides encoding these polypeptides are also encompassed by the invention.

This gene is expressed primarily in kidney cortex and to a lesser extent in adult brain, corpus colosum, hippocampus, and frontal cortex.

Therefore, polynucleotides and polypeptides of the invention are useful as reagents for differential identification of the tissue(s) or cell type(s) present in a biological sample and for diagnosis of diseases and conditions, which include, but are not limited to, neurological disorders. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment or diagnosis of neurological disorders.

### FEATURES OF PROTEIN ENCODED BY GENE NO: 102

In specific embodiments, polypeptides of the invention comprise the sequence:
MQEMMRNQDRALSNLESIPGGYNA (SEQ ID NO:587); LRRMYTDIQEPMLSA AQEQF GGNPF (SEQ ID NO:588); ASLVSNTSSGEGSQPSRTENRDPLPNPWAP QT (SEQ ID NO:589); SQSSSASSGTASTVGGTTGSTASGTSGQSTTAPNLVPGV GASMFNTPG MQSLLQQITENPQLMQNMLSAPY (SEQ ID NO:590); MRSMMQSLSQNPDLAAQMMLNNPLFAGNPQLQEQMRQQLPTFLQQ (SEQ ID NO:591); MQNPDTLSAMSNPRAMQALLQIQQGLQTLATEAPGLIPGFTPGLG ALGSTGGSSGTNGSNATPSENTSPTAGT (SEQ ID NO:592); TEPGHQQFI QQMLQALAGVNPQLQNPEVRFQQQLEQLSAMGFLNREANLQALIATGGDINAA IERLLGSQPS (SEQ ID NO:593); RNPAMMQEMMRNQDRALSNLESIPGGY NALRRMYTDIQEPMLSAA (SEQ ID NO:594); GNPFASLVSNTSS (SEQ ID NO:595); ENRDPLPNPWA (SEQ ID NO:595); GKILKDQDTLSQHGIHD (SEQ ID NO:597); GLTVHLVIKTQNRP (SEQ ID NO:598); SELQSQMQRQLLSNPEMM (SEQ ID NO:599); PEISHMLNNPDIMR (SEQ ID NO:600); and/or RQLIMANPQMQQLIQRNP (SEQ ID NO:601). Polynucleotides encoding these polypeptides are also encompassed by the invention.

This gene is expressed primarily in breast.

Therefore, polynucleotides and polypeptides of the invention are useful as reagents for differential identification of the tissue(s) or cell type(s) present in a biological sample and for diagnosis of diseases and conditions, which include, but are not limited to, breast cancer. Similarly, polypeptides and antibodies directed to these polypeptides are useful in providing immunological probes for differential identification of the tissue(s) or cell type(s). For a number of disorders of the above tissues or cells, particularly of tumor systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment and diagnosis of some types of breast cancer.

### FEATURES OF PROTEIN ENCODED BY GENE NO: 103

The translation product of this gene shares sequence homology with secreted serine proteases and lysozyme C precursor, which is thought to be important in bacteriolytic function. In specific embodiments, polypeptides of the invention comprise the sequence: NLCHVDCQDLLNPNLLAGIHCAKRIVS (SEQ ID NO:602); LDGFEGYSLSDWLCLAFVESKFN (SEQ ID NO:603); NENADGSFDYGLFQINSHYWCN (SEQ ID NO:604); and/or NLCHVDCQDLLNPNLLAGIHCAKRIVS (SEQ ID NO:605). Polynucleotides encoding these polypeptides are also encompassed by the invention.

This gene is expressed primarily in testes.

Therefore, polynucleotides and polypeptides of the invention are useful as reagents for differential identification of the tissue(s) or cell type(s) present in a biological sample and for diagnosis of diseases and conditions, which include, but are not limited to, infection. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 336 as residues: Ile-62 to Phe-70, Asn-78 to Asn-84.

The tissue distribution and homology to lysozyme C precursor indicates that polynucleotides and polypeptides corresponding to this gene are useful for boosting the moncyte-macrophage system and enhance the activity of immunoagents.

### FEATURES OF PROTEIN ENCODED BY GENE NO: 104

- This gene is expressed-primarily in apoptotic T-cell.

Therefore, polynucleotides and polypeptides of the invention are useful as reagents for differential identification of the tissue(s) or cell type(s) present in a biological sample and for diagnosis of diseases and conditions, which include, but are not limited to, immune disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment and diagnosis of some immune disorders.

### FEATURES OF PROTEIN ENCODED BY GENE NO: 105

The translation product of this gene shares sequence homology with ARI protein of Drosophila (accession 2058299; EMBL: locus DMARIADNE, accession X98309), which is thought to be important in axonal path-finding in the central nervous system. In specific embodiments, polypeptides of the invention comprise the sequence
IREVNEVIQNPAT (SEQ ID NO:606); ITRILLSHFNWDKEKLMERYF DGNLEKLFA (SEQ ID NO:607); NTRSSAQDMPCQICYLNYPNSYF (SEQ ID NO:608); TGLECGHKFCMQCWSEYLTTKIMEEGMGQTISCPAHG (SEQ ID NO:614); CDILVDDNTVMRLITDSKVKLKYQHLITNSFVECNRLLKWCPAPD CHHVVKVQYPDAKPV (SEQ ID NO:609); CDILVDDNTVMRLITDSK VKLKYQHLITNSFVECNRLLKWCPAPDCHHVVKV (SEQ ID NO:610); GCNHMVCRNQNCKAEFCWVCLGPWEPHGSAWYNCNRYNEDDAKAARDAQE RSRAALQRYL (SEQ ID NO:611); FYCNRYMNHMQSLRFEHKLYAQVKQ KMEEMQQHNMSWIEVQFLKKAVDVLCQCRATLMYT (SEQ ID NO: 612); YVFAFYLKKNNQSIIFENNQADLENATEVLSGYLERDISQDSLQDIKQKVQDKY RYCESR (SEQ ID NO:613) Polynucleotides encoding these polypeptides are also encompassed by the invention.

This gene is expressed primarily in adult brain, and to a lesser extent in endometrial tumor, melanocytes, and infant brain.

Therefore, polynucleotides and polypeptides of the invention are useful as reagents for differential identification of the tissue(s) or cell type(s) present in a biological sample and for diagnosis of diseases and conditions, which include, but are not limited to, diseases or injuries involving axonal path development. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to ARI protein indicates that polynucleotides and polypeptides corresponding to this gene are useful for treatment of disease states or injuries involving axonal path development, including neurodegenerative diseases and nerve injury.

### FEATURES OF PROTEIN ENCODED BY GENE NO: 106

The translation product of this gene shares sequence homology with cytochrome b561 [Sus scrofa] which is thought to be an integral membrane protein of neuroendocrine storage vesicles of neurotransmitters and peptide hormones.

This gene is expressed primarily in frontal cortex and to a lesser extent in rhabdomyosarcoma.

Therefore, polynucleotides and polypeptides of the invention are useful as reagents for differential identification of the tissue(s) or cell type(s) present in a biological sample and for diagnosis of diseases and conditions, which include, but are not limited to, neurological disorders. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 339 as residues: Ser-18 to Pro-24.

The tissue distribution and homology to cytochrome b561 [Sus scrofa] indicates that polynucleotides and polypeptides corresponding to this gene are useful for treatment and diagnosis of neurological disorders. This gene may also be important in regulation of some types of cancers.

### FEATURES OF PROTEIN ENCODED BY GENE NO: 107

In specific embodiments, polypeptides of the invention comprise the sequence:
MWGYLFVDAAWNFLGCLICGW (SEQ ID NO:615); MHFISSGNVSAIRSSILLL RXSLSYLGNCLRVSAIFVYFLLFLLLS (SEQ ID NO:616); and/or MDQALRGSPSE GFSTDPSPPQVGRQIPSFPPWRRLVLPKASGCFLEREWWLCVFKLRTRPGAEA HAYNSSILGGRGKGIT (SEQ ID NO:617). Polynucleotides encoding these polypeptides are also encompassed by the invention.

This gene is expressed primarily in pancreas tumor and to a lesser extent in cerebellum.

Therefore, polynucleotides and polypeptides of the invention are useful as reagents for differential identification of the tissue(s) or cell type(s) present in a biological sample and for diagnosis of diseases and conditions, which include, but are not limited to, pancreatic tumors. Similarly, polypeptides and antibodies directed to these polypeptides are useful in providing immunological probes for differential identification of the tissue(s) or cell type(s). For a number of disorders of the above tissues or cells, particularly of the endocrine system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 340 as residues: Pro-22 to Phe-33.

The tissue distribution indicates that polynucleotides and polypeptides corresponding to this gene are useful for diagnosis and treatment of pancreatic tumors.

### FEATURES OF PROTEIN ENCODED BY GENE NO: 108

This gene maps to chromosome 17 and therefore polynucleotides of the invention can be used in linkage analysis as a marker for chromosome 17. In specific embodiments, polypeptides of the invention comprise the sequence:
MLPALASCCHFSPPEQAARLKKLQEQEKQQKVEFRKRMEKEVSDFIQDSGQIK KKFQPMNKIERSILHDVVEVAGLTSFSFGEDDDCRYVMIFKKEFAPSDEELDSY RRGEEWDPQKAEEKRNXKELAQRQ (SEQ ID NO:618); EEEAAQQGPVVV SPASDYKDKYSHLIGKGAAKDAAHMLQANKTYGCXPVANKRDTRSIEEAMNE IRAKKRLRQSGE (SEQ ID NO:619); PPRRPAQLPLTPGAGQGAGRDKAAAIRA HPGAPPLNHLLP (SEQ IDNO:620); AVPQAGGKQVFDLSPLELGYVRGMCVCV (SEQ ID NO:621) and/or MLPALASCCHFSPPEQAARLKKLQEQEKQQKVEFRK RMEKEVSDFIQDSGQIKKKFQPMNKIERSILHDVVEVAGLTSFSFGEDDDCRYV MIFKKEFAPSDEELDSYRRGEEWDPQKAEEKRNXKELAQRQEEEAAQQGPVVV SPASDYKDKYSHLIGKGAAKDAAHMLQANKTYGCXPVANKRDTRSIEEAMNE IRAKKRLRQSGE (SEQ ID NO:622). Polynucleotides encoding these polypeptides are also encompassed by the invention. The translation product of this gene shares sequence homology with FSA-1 which may play a role as a structural protein component of the acrosome.

This gene is expressed primarily in fetal kidney and sperm.

Therefore, polynucleotides and polypeptides of the invention are useful as reagents for differential identification of the tissue(s) or cell type(s) present in a biological sample and for diagnosis of diseases and conditions, which include, but are not limited to, male reproductive disorders, especially involving acrosomal disfunction. Similarly, polypeptides and antibodies directed to these polypeptides are useful in providing immunological probes for differential identification of the tissue(s) or cell type(s). For a number of disorders of the above tissues or cells, particularly of the male reproductive system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 341 as residues: Glu-8 to Asn-35.

The tissue distribution and homology to FSA-1 indicates that polynucleotides and polypeptides corresponding to this gene are useful for treatment of infertility due to acrosomal disfunction of sperm.

### FEATURES OF PROTEIN ENCODED BY GENE NO: 109

This gene is expressed primarily in pituitary and to a lesser extent in epididymus.

Therefore, polynucleotides and polypeptides of the invention are useful as reagents for differential identification of the tissue(s) or cell type(s) present in a biological sample and for diagnosis of diseases and conditions, which include, but are not limited to, male reproductive disorders. Similarly, polypeptides and antibodies directed to these polypeptides are useful in providing immunological probes for differential identification of the tissue(s) or cell type(s). For a number of disorders of the above tissues or cells, particularly of the male reproductive system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 342 as residues: Met-1 to Trp-6.

Because the gene is found in both pituitary and epididymus, this indicates that polynucleotides and polypeptides corresponding to this gene are useful for treatment and diagnosis of male reproductive disorders. This may involve a secreted peptide produced in the pituitary targeting the epididymus.

### FEATURES OF PROTEIN ENCODED BY GENE NO: 110

In specific embodiments, polypeptides of the invention comprise the sequence:
LLCPVLNSGXSWNFPHPSQPEYSFHGFHSTRLWI (SEQ ID NO:623); and/or PSTPWFLFLLGLTCPFSTSHPRWDSIPP (SEQ ID NO:624). Polynucleotides encoding these polypeptides are also encompassed by the invention.

This gene is expressed primarily in resting T-cells.

Therefore, polynucleotides and polypeptides of the invention are useful as reagents for differential identification of the tissue(s) or cell type(s) present in a biological sample and for diagnosis of diseases and conditions, which include, but are not limited to, T-cell disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment and diagnosis of certain immune disorders, especially those involving T-cells.

### FEATURES OF PROTEIN ENCODED BY GENE NO: 111

This gene is expressed primarily in cerebellum and whole brain and to a lesser extent in infant brain and fetal kidney.

Therefore, polynucleotides and polypeptides of the invention are useful as reagents for differential identification of the tissue(s) or cell type(s) present in a biological sample and for diagnosis of diseases and conditions, which include, but are not limited to, neurological disorders. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 344 as residues: Asp-48 to Gly-55.

The tissue distribution indicates that polynucleotides and polypeptides corresponding to this gene are useful for diagnosis and treatment of neurological disorders.

### FEATURES OF PROTEIN ENCODED BY GENE NO: 112

The translation product of this gene shares sequence homology with yeast mitochondrial ribosomal protein homologous to ribosomal protein s15 of E.coli which is thought to be important in the early assembly of ribosomes (See Accession No. M380.16). This gene maps to chromosome 1, and therefore, may be used as a marker in linkage analysis for chromosome 1.

This gene is expressed primarily in developmental tissues.

Therefore, polynucleotides and polypeptides of the invention are useful as reagents for differential identification of the tissue(s) or cell type(s) present in a biological sample and for diagnosis of diseases and conditions, which include, but are not limited to, development of cancers and tumors in addition to healing wounds. Similarly, polypeptides and antibodies directed to these polypeptides are useful in providing immunological probes for differential identification of the tissue(s) or cell type(s). For a number of disorders of the above tissues or cells, particularly of the immune and developmental expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to ribosomalprotein s15 of E. coli indicates that polynucleotides and polypeptides corresponding to this gene are useful for diseases related to the assembly of ribosomes in the mitochondria which is important in the translation of RNA into protein. Therefore, this indicates that polynucleotides and polypeptides corresponding to this gene are useful for diagnosis and intervention of multiple tumors as well as in healing wounds which are thought to be under similar regulation as developmental tissues. Protein, as well as, antibodies directed against the protein have utility as tumor markers, in addition to immunotherapy targets, for the above listed tumors and tissues.

### FEATURES OF PROTEIN ENCODED BY GENE NO: 113

The translation product of this gene shares sequence homology with human poliovirus receptor precursors which are thought to be important in viral binding and uptake. Preferred polypeptide fragments comprise the following amino acid sequence:
ELSISISNVALADEGEYTCSIFTMPVRTAKSLVTVLGIPQKPIITGYKSSLREKDT ATLNCQSSGSKPAARLTWRKGDQELHGEPTRIQEDPNGKTFTVSSSVTFQVTR EDDGASIVCSVNHESLKGADRSTSQRIEVLYTPTAMIRPDPPHPREGQKLLLHC EGRGNPVPQQYLWEKEGSVPPLKMTQESALIFPFLNKSDSGTYGCTATSNMGS YKAYYTLNVND (SEQ ID NO:625). Also preferred are polynucleotide fragments encoding these polypeptide fragments (See Accession No. gnllPIDld1002627).

This gene is expressed almost exclusively in human brain tissue.

Therefore, polynucleotides and polypeptides of the invention are useful as reagents for differential identification of the tissue(s) or cell type(s) present in a biological sample and for diagnosis of diseases and conditions, which include, but are not limited to, susceptibility to viral disease and diseases of the CNS especially cancers of that system. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 346 as residues: Leu-26 to Asp-37, Lys-53 to Ser-59.

The tissue distribution and homology to poliovirus receptor precursors indicates that polynucleotides and polypeptides corresponding to this gene are useful for the treatment and prevention of diseases that involve the binding and uptake of virus particles for infection. It might also be helpful in genetic therapy where the goal is to insert foreign DNA into infected cells. With the help of this protein, the binding and uptake of this foreign DNA might be aided. In addition, it is expected that over expression of this gene will indicate abnormalities involving the CNS, particularly cancers of that system.

### FEATURES OF PROTEIN ENCODED BY GENE NO: 114

The translation product of this gene shares sequence homology with YO87_CAEEL hypothetical 28.5 KD protein ZK1236.7 in chromosome III of Caenorhabditis elegans in addition to alpha-1 collagen type III (See Accession No. gil537432). One embodiment for this gene is the polypeptide fragment(s) comprising the following amino acid sequence: VPELPDRVHQLHQAVQGCALGRPGFPGGPTH SGHHKSHPGPAGGDYNRCDRPGQVHLHNPRGTGRRGQLHPTAGPGVHRRA CPSQQLPHRLGPGVPCPSPSLTPVLPSWTQSWCG LPGYTSSS (SEQ ID NO:630). An additional embodiment is the polynucleotide fragment(s) encoding these polypeptide fragments

This gene is expressed primarily in brain cells and to a lesser extent in activated B and T cells.

Therefore, polynucleotides and polypeptides of the invention are useful as reagents for differential identification of the tissue(s) or cell type(s) present in a biological sample and for diagnosis of diseases and conditions, which include, but are not limited to, neurodegeneration and imunological disorders. Similarly, polypeptides and antibodies directed to these polypeptides are useful in providing immunological probes for differential identification of the tissue(s) or cell type(s). For a number of disorders of the above tissues or cells, particularly of the neural and immune systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e.,. the expression level in healthy tissue or bodily fluid from an individual not having the disorder. Preferred epitopes include those comprising a sequence shown in SEQ ID --NO: 347 as residues: Glu-34 to Glu-39, Gly-51 to Ser-72, Ala-88 to Glu-93, Gln-100 to Val-105.

The tissue distribution and homology to YO87_CAEEL hypothetical 28.5 KD protein ZK1236.7 in chromosome III of Caenorhabditis elegans as well as to a conserved alpha-1 collagen type III protein indicates that polynucleotides and polypeptides corresponding to this gene are useful for the detection and treatment of neurodegenerative disease states and behavioral disorders such as Alzheimer's Disease, Parkinson's Disease, Huntingtons' Disease, schizophrenia, mania, dementia, paranoia, obsessive compulsive disorder and panic disorders. Because the gene is expressed in cells of lymphoid origin, the natural gene product may be involved in immune functions. Therefore it may be also used as an agent for immunological disorders including arthritis, asthma, immune deficiency diseases such as AIDS, and leukemia.

### FEATURES OF PROTEIN ENCODED BY GENE NO: 115

The translation product of this gene shares sequence homology with alpha 3 type IX collagen which is thought to be important in hyaline cartilage formation via its ability to uptake inorganic sulfate by cells (See Accession No. gil975657). One embodiment of this gene is the polypeptide fragment comprising the following amino acid sequence: SLRRPRSAAXQTLTTFLSSVSSASSSALPGSREPCDPRAPPPPR SGSAASCCSCCCSCPRRRAPLRSPRGSKRRIRQREVVDLYNGMCLQGPAGVPG RDGSPGANGIPGTPGIPGRDGFKGEKGECLRESFEESWTPNYKQCSWSSLNY GIDLGKIAECTFTKMRSNSALRVLFSGSLRLKCRNACCQRWYFTFNGAECSGP LPIEAIIYLDQGSPEMNSTINIHRTSSVEGLCEGIGAGLVDVAIWVGTCSDYPKG DASTGWNSVSRIIIEELPK (SEQ ID NO:634). An additional embodiment are the polynucleotide fragments encoding this polypeptide fragment.

This gene is expressed primarily in smooth muscle and to a lesser extent in synovial tissue.

Therefore, polynucleotides and polypeptides of the invention are useful as reagents for differential identification of the tissue(s) or cell type(s) present in a biological sample and for diagnosis of diseases and conditions, which include, but are not limited to, dwarfism, spinal deformation, and specific joint abnormalities as well as chondrodysplasias i.e., spondyloepiphyseal dysplasia congenita, familial osteoarthritis, Atelosteogenesis type II, metaphyseal chondrodysplasia type Schmid and autoimmune disorders. Similarly, polypeptides and antibodies directed to these polypeptides are useful in providing immunological probes for differential identification of the tissue(s) or cell type(s). For a number of disorders of the above tissues or cells, particularly of the skeletal system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to alpha 3 type IX collagen indicates that polynucleotides and polypeptides corresponding to this gene are useful for the treatment and diagnosis of diseases associated with the mutation in this gene which leads to the many different types of chondrodysplasias. By the use of this product, the abnormal growth and development of bones of the limbs and spine could be routinely detected or treated in utero since the protein or muteins thereof could affect epithelial cells early in development and later the chondrocytes of the developing craniofacial structure.

### FEATURES OF PROTEIN ENCODED BY GENE NO: 116

The translation product of this gene shares sequence homology with retrovirus-related reverse transcriptase which is thought to be important in viral replication. One embodiment for this gene is the polypeptide fragments comprising the following amino acid sequence: TKKENCRPASLMNIDTKILNKILMNQ (SEQ ID NO:640). An additional embodiment is the polynucleotide fragments encoding these polypeptide fragments (See Accession No. pirIA253131GNHUL1).

This gene is expressed primarily in human meningima.

Therefore, polynucleotides and polypeptides of the invention are useful as reagents for differential identification of the tissue(s) or cell type(s) present in a biological sample and for diagnosis of diseases and conditions, which include, but are not limited to, retroviral diseases such as AIDS, and possibly certain cancers due to transactivation of latent cell division genes. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to retrovirus-related reverse transcriptase indicates that polynucleotides and polypeptides corresponding to this gene are useful for the detection and treatment of diseases and maladies associated with retroviral infection since a functional reverse transcriptase (RT) or RT-like molecule is an integral component of the retroviral life cycle.

### FEATURES OF PROTEIN ENCODED BY GENE NO: 117

The translation product of this gene shares sequence homology with an unknown gene from *C. elegans*, as well as weak homolog with mammalian metaxin, a gene contiguous to both thrombospondin 3 and glucocerebrosidase, is known to be required for embryonic development. Preferred polypeptide fragments comprise the following amino acid sequence: MCNLPIKVVCRANAEYMSPSGKVPXXHVGNQ WSELGPIVQFVKAKGHSLSDGLEEVQKAEMKAYMELVNNMLLTAELYLQWC DEATVGXITHXRYGSPYPWPLXHILAYQKQWEVKRKXKAIGWGKKTLDQVLE DVDQCCQALSQRLGTQPYFFNKQPTELDALVFGHLYTILTTQLTNDELSEKVKN YSNLLAFCRRI EQHYFEDRGKGRLS (SEQ ID NO:641); MCNLPIKVVCRANAE YMSPSGKVPXXHVGNQVVSELGPIVQFVK (SEQ ID NO:642),. Also preferred are polynucleotide fragments encoding these polypeptide fragments (See Accession No. gi|1326108).

This gene is expressed primarily in fetal tissues and to a lesser extent in hematopoietic cells and tissues, including spleen, monocytes, and T cells.

Therefore, polynucleotides and polypeptides of the invention are useful as reagents for differential identification of the tissue(s) or cell type(s) present in a biological sample and for diagnosis of diseases and conditions; which include, but are not limited to, cancer; lymphoproliferative disorders; inflammation; chondrosarcoma, and Gaucher disease. Similarly, polypeptides and antibodies directed to these polypeptides are useful in providing immunological probes for differential identification of the tissue(s) or cell type(s). For a number of disorders of the above tissues or cells, particularly of the hematopoietic and embryonic systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis and treatment of cancer and other proliferative disorders. Expression in embryonic tissue and other cellular sources marked by proliferating cells indicates that this protein may play a role in the regulation or cellular division. Additionally, the expression in hematopoietic cells and tissues indicates that this protein may play a role in the proliferation, differentiation, and survival of hematopoietic cell lineages. Thus, this gene may be useful in the treatment of lymphoproliferative disorders, and in the maintenance and differentiation of various hematopoietic lineages from early hematopoietic stem and committed progenitor cells.

### FEATURES OF PROTEIN ENCODED BY GENE NO: 118

The translation product of this gene shares sequence homology with reverse transcriptase which is important in the synthesis of a cDNA chain from an RNA molecule, and is a method whereby the infecting RNA chains of retroviruses are transcribed into their DNA complements. One embodiment for this gene is the polypeptide fragment comprising the following amino acid sequence:
MXXXNSHITIFTLNVNGLNAPNERHRLANWIQSQDQVCCIQETHLTGRDTHRL KIKGWRKIYQANGKQKK (SEQ ID NO:647). An additional embodiment is the polynucleotide fragments comprising polynucleotides encoding these polypeptide fragments (See Accession No. gi|2072964).

This gene is expressed primarily in skin and to a lesser extent in neutrophils.

Therefore, polynucleotides and polypeptides of the invention are useful as reagents for differential identification of the tissue(s) or cell type(s) present in a biological sample and for diagnosis of diseases and conditions which include, but are not limited to, cancer, hematopoietic disorders; inflammation; disorders of immune surveillance. Similarly, polypeptides and antibodies directed to these polypeptides are useful in providing immunological probes for differential identification of the tissue(s) or cell type(s). For a number of disorders of the above tissues or cells, particularly of the epidermis and/or hematopoietic system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to reverse transcriptase indicates that polynucleotides and polypeptides corresponding to this gene are useful for cancer therapy. Expression in the skin also indicates that this gene is useful in wound healing and fibrosis. Expression by neutrophils also indicates that this gene product plays a role in inflammation and the control of immune surveillance (i.e. recognition of viral pathogens). Reverse transcriptase family members are also useful in the detection and treatment of AIDS.

### FEATURES OF PROTEIN ENCODED BY GENE NO: 119

The translation product of this gene shares sequence homology with reverse transcriptase which is important in the synthesis of a cDNA copy of an RNA molecule, and is a method whereby a retrovirus reverse-transcribes its genome into an inheritable DNA copy.

This gene is expressed primarily in the frontal cortex of brain.

Therefore, polynucleotides and polypeptides of the invention are useful as reagents for differential identification of the tissue(s) or cell type(s) present in a biological sample and for diagnosis of diseases and conditions, which include, but are not limited to, cancer and neurodegenerative disorders. Similarly, polypeptides and antibodies directed to these polypeptides are useful in providing immunological probes for differential identification of the tissue(s) or cell type(s). For a number of disorders of the above tissues or cells, particularly of the CNS and peripheral nervous system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to reverse transcriptase suggest that this is useful in the treatment of cancer and AIDS. The expression in brain indicates that it plays a role in neurodegenerative disorders and in neural degeneration.

### FEATURES OF PROTEIN ENCODED BY GENE NO: 120

One embodiment of this gene has homology to a hypothetical protein in Schizosaccharomyces pombe (See Accession No. 2281980). Another embodiment for this gene is the polypeptide fragments comprising the following amino acid sequence: IYHLHSWIFFHFKRAFCMCFITMKVIHAHCSKLRKCXNAQISVFCTTLTASYPT (SEQ ID NO:651). An additional embodiment is the polynucleotide fragments encoding these polypeptide fragments. This gene maps to chromosome 18, and therefore, may be used as a marker in linkage analysis for chromosome 18.

This gene is expressed primarily in adult hypothalamus and to a lesser extent in infant brain.

Therefore, polynucleotides and polypeptides of the invention are useful as reagents for differential identification of the tissue(s) or cell type(s) present in a biological sample and for diagnosis of diseases and conditions, which include, but are not limited to, neurodegenerative disorders; endocrine function; and vertigo. Similarly, polypeptides and antibodies directed to these polypeptides are useful in providing immunological probes for differential identification of the tissue(s) or cell type(s). For a number of disorders of the above tissues or cells, particularly of the brain, CNS and peripheral nervous system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the treatment and diagnosis of neurodegenerative disorders; diagnosis of tumors of a brain or neuronal origin; treatments involving hormonal control of the entire body and of homeostasis, behavioral disorders, such as Alzheimer's Disease, Parkinson's Disease, Huntington's Disease, schizophrenia, mania, dementia, paranoia, obsessive compulsive disorder and panic disorder. In addition, the gene or gene product may also play a role in the treatment and/or detection of developmental disorders associated with the developing embryo.

### FEATURES OF PROTEIN ENCODED BY GENE NO: 121

The translation product of this gene shares sequence homology with the human IRLB protein which is thought to be important in binding to a c-myc promoter element and thus regulating its transcription (See Accession No. gi|33969). This gene maps to chromosome 1, and therefore, may be used as a marker in linkage analysis for chromosome 1.

This gene is expressed primarily in brain and breast and to a lesser extent in a variety of hematopoietic tissues and cells.

Therefore, polynucleotides and polypeptides of the invention are useful as reagents for differential identification of the tissue(s) or cell type(s) present in a biological sample and for diagnosis of diseases and conditions, which include, but are not limited to, cancer of the brain and breast; lymphoproliferative disorders; neurodegenerative diseases. Similarly, polypeptides and antibodies directed to these polypeptides are useful in providing immunological probes for differential identification of the tissue(s) or cell type(s). For a number of disorders of the above tissues or cells, particularly of the CNS, breast, and immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the treatment and diagnosis of cancer of the brain, breast, and hematopoietic system. In addition, it may be useful for the treatment of neurodegenerative disorders, as well as disorders of the hematopoietic system, including defects in immune competency and inflammation. Protein, as well as, antibodies directed against the protein may show utility as a tumor marker and immunotherapy targets for the above listed tumors and tissues.

### FEATURES OF PROTEIN ENCODED BY GENE NO: 122

The translation product of this gene shares sequence homology with an ATP synthase, a key component of the proton channel that is thought to be important in the translocation of protons across the membrane.

This gene is expressed primarily in T cell lymphoma.

Therefore, polynucleotides and polypeptides of the invention are useful as reagents for differential identification of the tissue(s) or cell type(s) present in a biological sample and for diagnosis of diseases and conditions, which include, but are not limited to, T cell lymphoma. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to ATP synthase indicates that polynucleotides and polypeptides corresponding to this gene are useful for the treatment of defects in proton transport, homeostasis, and metabolism, as well as the diagnosis and treatment of lymphoma. Because the gene is expressed in cells of lymphoid origin, the natural gene product may be involved in immune functions. Therefore it may be also used as an agent for immunological disorders including arthritis, asthma, immune deficiency diseases such as AIDS, and leukemia

### FEATURES OF PROTEIN ENCODED BY GENE NO: 123

This gene maps to chromosome 15, and therefore, may be used as a marker in linkage analysis for chromosome 15.

This gene is expressed primarily in a variety of fetal tissues, including fetal liver, lung, and spleen, and to a lesser extent in a variety of blood cells, including eosinophils and T cells.

Therefore, polynucleotides and polypeptides of the invention are useful as reagents for differential identification of the tissue(s) or cell type(s) present in a biological sample and for diagnosis of diseases and conditions, which include, but are not limited to, cancer (abnormal cell proliferation); T cell lymphomas; and hematopoietic disorders. Similarly, polypeptides and antibodies directed to these polypeptides are useful in providing immunological probes for differential identification of the tissue(s) or cell type(s). For a number of disorders of the above tissues or cells, particularly of the fetus and immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the treatment and diagnosis of conditions involving cell proliferation. Expression of this gene in fetal tissues, as well as in a variety of blood cell lineages indicates that it may play a role in either cellular proliferation; apoptosis; or cell survival. Thus it may be useful in the management and treatment of a variety of cancers and malignancies. In addition, its expression in blood cells suggest that it may play additional roles in hematopoietic disorders and conditions, and could be useful in treating diseases involving autoimmunity, immune modulation, immune surveillance, and inflammation..

### FEATURES OF PROTEIN ENCODED BY GENE NO: 124

This gene is expressed primarily in placenta and to a lesser extent in pineal gland and rhabdomyosarcoma.

Therefore, polynucleotides and polypeptides of the invention are useful as reagents for differential identification of the tissue(s) or cell type(s) present in a biological sample and for diagnosis of diseases and conditions, which include, but are not limited to, developmental, endocrine, and female reproductive disorders. Similarly, polypeptides and antibodies directed to these polypeptides are useful in providing immunological probes for differential identification of the tissue(s) or cell type(s). For a number of disorders of the above tissues or cells, particularly of the [insert system where a related disease state is likely, e.g., immune], expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 357 as residues: Leu-69 to Val-76.

The tissue distribution indicates that polynucleotides and polypeptides corresponding to this gene are useful for diagnosis and treatment of disorders in development. Protein, as well as, antibodies directed against the protein may show utility as a tumor marker and immunotherapy targets for the above listed tumors and tissues.

### FEATURES OF PROTEIN ENCODED BY GENE NO: 125

This gene is expressed primarily in benign prostatic hyperplasia.

Therefore, polynucleotides and polypeptides of the invention are useful as reagents for differential identification of the tissue(s) or cell type(s) present in a biological sample and for diagnosis of benign prostatic hyperplasia. Similarly, polypeptides and antibodies directed to these polypeptides are useful in providing immunological probes for differential identification of the tissue(s) or cell type(s). For a number of disorders of the above tissues or cells, particularly of the reproductive system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment and diagnosis of benign prostatic hyperplasia. Protein, as well as, antibodies directed against the protein may show utility as a tumor marker and immunotherapy targets for the above listed tumors and tissues.

### FEATURES OF PROTEIN ENCODED BY GENE NO: 126

This gene is expressed primarily in apoptotic T-cells and to a lesser extent in suppressor T cells and ulcerative colitis.

Therefore, polynucleotides and polypeptides of the invention are useful as reagents for differential identification of the tissue(s) or cell type(s) present in a biological sample and for diagnosis of diseases and conditions, which include, but are not limited to, diseases involving premature apoptosis, and immunological and gastrointestinal disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment and diagnosis of disorders involving inappropriate levels of apoptosis, especially in immune cell lineages. Because the gene is expressed in cells of lymphoid origin, the natural gene product may be involved in immune functions. Therefore it may be also used as an agent for immunological disorders including arthritis, asthma, immune deficiency diseases (such as AIDS), and leukemia.

### FEATURES OF PROTEIN ENCODED BY GENE NO: 127

This gene is expressed primarily in Raji cells.

Therefore, polynucleotides and polypeptides of the invention are useful as reagents for differential identification of the tissue(s) or cell type(s) present in a biological sample and for diagnosis of diseases and conditions, which include, but are not limited to, inflammation and T cell autoimmune disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 360 as residues: Asp-23 to Gly-29.

The tissue distribution indicates that polynucleotides and polypeptides corresponding to this gene are useful for treatment and diagnosis of inflammation and T cell autoimmune disorders. Because the gene is expressed in cells of lymphoid origin, the natural gene product may be involved in immune functions. Therefore it may be also used as an agent for immunological disorders including arthritis, asthma, immune deficiency diseases (such as AIDS), and leukemia.

### FEATURES OF PROTEIN ENCODED BY GENE NO: 128

The translation product of this gene shares sequence homology with an *C*. *elegans* coding region C47D12.2 of unknown function (See Accession No. gnllPIDle348986). One embodiment for this gene is the polypeptide fragments comprising the following amino acid sequence: EDDGFNRSIHEVILKNITWY SERVLTEISLGSLLILWIRTIQYNMTRTRDKYLHTNCLAALANMSAQFRSLHQY AAQRIISLFSLLSKKHNKVLEQATQSLRGSLSSNDVPLPDYAQDLNVIEEVIRMM LEIINSCLTNSLHHNPNLVALLYKRDLFEQFRTHPSFQDIMQNIDLVISFFSSRLL QAGS (SEQ ID NO:657); EDDGFNRSIHEVILKNTTWYSERVLTEISLGSLLILVV (SEQ ID NO:658); RTIQYNMTRTRDKYLHTNCLAALANMSAQFRSLHQYAAQ RIISLFSLLSKKHN (SEQ ID NO:659); KKHNKVLEQATQSLRGSLSSNDVPLPDY AQD (SEQ ID NO:661); SCLTNSLHHNPNLVYALLYKRDLFEQFRTHPSFQD IMQNIDLVISFFSSRLLQAGS (SEQ ID NO:660). An additional embodiment is the polynucleotide fragments encoding these polypeptide fragments. This gene maps to chromosome 18, and therefore, may be used as a marker in linkage analysis for chromosome 18.

This gene is expressed primarily in smooth muscle and to a lesser extent in fetal liver.

Therefore, polynucleotides and polypeptides of the invention are useful as reagents for differential identification of the tissue(s) or cell type(s) present in a biological sample and for diagnosis of diseases and conditions, which include, but are not limited to, atherosclerosis and other cardiovascular and hepatic disorders. Similarly, polypeptides and antibodies directed to these polypeptides are useful in providing immunological probes for differential identification of the tissue(s) or cell type(s). For a number of disorders of the above tissues or cells, particularly of the circulatory system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of circulatory system disorders such as atherosclerosis, hypertension, and thrombosis. In addition, the tissue distribution indicates that polynucleotides and polypeptides corresponding to this gene are useful for the detection and treatment of liver disorders and cancers (e.g. hepatoblastoma, jaundice, hepatitis, liver metabolic diseases and conditions that are attributable to the differentiation of hepatocyte progenitor cells). In addition the expression in fetus would suggest a useful role for the protein product in developmental abnormalities, fetal deficiencies, pre-natal disorders and various would-healing models and/or tissue trauma.

### FEATURES OF PROTEIN ENCODED BY GENE NO: 129

The translation product of this gene shares sequence homology with a ribosomal protein which is thought to be important in cellular metabolism, in addition to the *C.elegans* protein F40F11.1 which does not have a known function at the current time (See Accession No. gnIlPIDle244552). Preferred polypeptide fragments comprise the following amino acid sequence:
MADIQTERAYQKQPTIFQNKKRVLLGETGKEKLPRVTNKNIGLGFKDT PRRLLRGTYIDKKCPFTGNVSIRGRILSGVVTQDEDAEDHCHPPRLSALHPQVQ PLREAPQEHVCTPVPL LQGRPDR (SEQ ID NO:662); MKMQRTIVIRRDYLH YIRKYNRFEKRHKNMSVHLSPCFRDVQIGDIVTVGECRPLSKTVRFNVLKVTK AAGTKKQFQKF (SEQ ID NO:663); MADIQTERAYQKQPTIFQNKKRVLLGET GK (SEQ ID NO:664); HCHPPRLSALHPQVQPLREAPQEHVCTPVPLLQGRPDR (SEQ ID NO:666); NIGLGFKDTPRRLLRGTYIDKKCPFTGNVSIRGRILSGVVTQ (SEQ ID NO:669); MKMQRTIVIRRDYLHYIRKYNRFEKRHKNMSVHLSP (SEQ ID NO:667); CFRDVQIGDIVTVGECRPLSKTVRFNVLKVTKAAGTKKQFQKF (SEQ ID NO:668). Also preferred are polynucleotide fragments encoding these polypeptide fragments.

This gene is expressed primarily in Wilm's tumor and to a lesser extent in thymus and stromal cells.

Therefore, polynucleotides and polypeptides of the invention are useful as reagents for differential identification of the tissue(s) or cell type(s) present in a biological sample and for diagnosis of diseases and conditions, which include, but are not limited to, diseases affecting RNA translation. Similarly, polypeptides and antibodies directed to these polypeptides are useful in providing immunological probes for differential identification of the tissue(s) or cell type(s). For a number of disorders of the above tissues or cells, particularly of the Wilm's tumors, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 362 as residues: Thr-11 to Asp-20.

The tissue distribution and homology to a ribosomal protein indicates that polynucleotides and polypeptides corresponding to this gene are useful for diseases affecting RNA translation.

### FEATURES OF PROTEIN ENCODED BY GENE NO: 130

The translation product of this gene shares sequence homology with a yeast DNA helicase which is thought to be important in global transcriptional regulation (See Accession No. gnIlPIDle243594). One embodiment for this gene is the polypeptide fragments comprising the following amino acid sequence: IFYDSDWNPTVDQQA
MDRAHRLGQTKQVTVYRLICKGTIEERILQRAKEKSEIQRMVISG (SEQ ID NO:670); TRMIDLLEEYMVYRKHTYXRLDGSSKISERRDMVADFQNRNDI FVFLLSTRAGGLGINLTAXDTVHF (SEQ ID NO:671); TRMIDLLEEYMVYRK HTYXRLDGSSKISERRDM (SEQ ID NO:674); RRDMVADFQNRNDIFVFLL STRAGGLGINLTAXDTVHF (SEQ ID NO:675), IFYDSDWNPTVDQQAMD RAHRLGQTKQVTVYRLICKG (SEQ ID NO:676); RLICKGTIEERILQRAK EKSEIQRMVISG (SEQ ID NO:678). An additional embodiment is the polynucleotide fragments encoding these polypeptide fragments.

This gene is expressed primarily in amygdala.

Therefore, polynucleotides and polypeptides of the invention are useful as reagents for differential identification of the tissue(s) or cell type(s) present in a biological sample and for diagnosis of diseases and conditions, which include, but are not limited to, diseases and disorders of the brain. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to a DNA helicase indicates that polynucleotides and polypeptides corresponding to this gene are useful for diseases affecting RNA transcription, particularly developmental disorders and healing wounds since the later are though to approximate developmental transcriptional regulation.

### FEATURES OF PROTEIN ENCODED BY GENE NO: 131

This gene is expressed primarily in prostate and to a lesser extent in amygdala and pancreatic tumors.

Therefore, polynucleotides and polypeptides of the invention are useful as reagents for differential identification of the tissue(s) or cell type(s) present in a biological sample and for diagnosis of diseases and conditions, which include, but are not limited to, prostate enlargement and gastrointestinal disorders, particularly of the pancreas and gall bladder. Similarly, polypeptides and antibodies directed to these polypeptides are useful in providing immunological probes for differential identification of the tissue(s) or cell type(s). For a number of disorders of the above tissues or cells, particularly of the reproductive system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment and diagnosis of prostate diseases, including benign prostatic hyperplasia and prostate cancer. In addition, the tissue distribution in tumors of the pancreas indicates that polynucleotides and polypeptides corresponding to this gene are useful for diagnosis and intervention of these tumors, in addition to other tissues where expression has been indicated. Protein; as well as, antibodies directed against the protein may show utility as a tumor marker and/or immunotherapy targets for the above listed tumors and tissues.

### FEATURES OF PROTEIN ENCODED BY GENE NO: 132

This gene is expressed primarily in adult lung and to a lesser extent in hypothalamus.

Therefore, polynucleotides and polypeptides of the invention are useful as reagents for differential identification of the tissue(s) or cell type(s) present in a biological sample and for diagnosis of diseases and conditions, which include, but are not limited to, pulmonary diseases and neurological disorders. Similarly, polypeptides and antibodies directed to these polypeptides are useful in providing immunological probes for differential identification of the tissue(s) or cell type(s). For a number of disorders of the above tissues or cells, particularly of the pulmonary and respiratory systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of pulmonary and respiratory disorders such as emphysema, pneumonia, and pulmonary edema and emboli. In addition, the tissue distribution indicates that polynucleotides and polypeptides corresponding to this gene are useful for the detection/treatment of neurodegenerative disease states and behavioral disorders such as Alzheimer's Disease, Parkinson's Disease, Huntington's Disease, schizophrenia, mania, dementia, paranoia, obsessive compulsive disorder and panic disorder. In addition, the gene or gene product may also play a role in the treatment and/or detection of developmental disorders associated with the developing embryo, sexually-linked disorders, or disorders of the cardiovascular system.

### FEATURES OF PROTEIN ENCODED BY GENE NO: 133

This gene is expressed primarily in human liver.

Therefore, polynucleotides and polypeptides of the invention are useful as reagents for differential identification of the tissue(s) or cell type(s) present in a biological sample and for diagnosis of diseases and conditions, which include, but are not limited to, cirrhosis of the liver and other hepatic disorders. Similarly, polypeptides and antibodies directed to these polypeptides are useful in providing immunological probes for differential identification of the tissue(s) or cell type(s). For a number of disorders of the above tissues or cells, particularly of the digestive system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of liver disorders such as cirrhosis, jaundice, and Hepatitus. Protein, as well as, antibodies directed against the protein may show utility as a tumor marker and/or immunotherapy targets for the above listed tissues.

### FEATURES OF PROTEIN ENCODED BY GENE NO: 134

This gene is expressed primarily in fetal kidney and to a lesser extent in fetal liver and spleen.

Therefore, polynucleotides and polypeptides of the invention are useful as reagents for differential identification of the tissue(s) or cell type(s) present in a biological sample and for diagnosis of diseases and conditions, which include, but are not limited to, development and regeneration of liver and kidney and immunological disorders. Similarly, polypeptides and antibodies directed to these polypeptides are useful in providing immunological probes for differential identification of the tissue(s) or cell type(s). For a number of disorders of the above tissues or cells, particularly of the digestive and excretory systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 367 as residues: Pro-70 to Arg-77, Tyr-102 to Thr-107.

The tissue distribution indicates that polynucleotides and polypeptides corresponding to this gene are useful for diagnosis and treatment of diseases of the kidney and liver, such as cirrhosis, kidney failure, kidney stones, and liver failure, hepatoblastoma, jaundice, hepatitis, liver metabolic diseases and conditions that are attributable to the differentiation of hepatocyte progenitor cells. In addition the expression in fetus would suggest a useful role for the protein product in developmental abnormalities, fetal deficiencies, pre-natal disorders and various would-healing models and/or tissue trauma.

### FEATURES OF PROTEIN ENCODED BY GENE NO: 135

This gene is expressed primarily in brain, bone marrow, and to a lesser extent in placenta, T cell, testis and neutrophils.

Therefore, polynucleotides and polypeptides of the invention are useful as reagents for differential identification of the tissue(s) or cell type(s) present in a biological sample and for diagnosis of diseases and conditions, which include, but are not limited to, neurodegenerative and immunological diseases and cancer. Similarly, polypeptides and antibodies directed to these polypeptides are useful in providing immunological probes for differential identification of the tissue(s) or cell type(s). For a number of disorders of the above tissues or cells, particularly of the nervous and immune systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 368 as residues: Met-1 to His-6.

The tissue distribution indicates that polynucleotides and polypeptides corresponding to this gene are useful for the detection/treatment of neurodegenerative disease states and behavioral disorders such as Alzheimer's Disease, Parkinson's Disease, Huntingtons Disease, schizophrenia, mania, dementia, paranoia, obsessive compulsive disorder and panic disorder. In addition, the gene or gene product may also play a role in the treatment and/or detection of developmental disorders associated with the developing embryo, or sexually-linked disorders.

### FEATURES OF PROTEIN ENCODED BY GENE NO: 136

Translatation product of this gene is homologous to the human WD repeat protein HAN11. Preferred polypeptide fragments comprise the following amino acid sequence:
MSLHGKRKEIYKYEAPWTVYAMNWSVRPDKRFRLALGSFVEEYNNKVQLVG LDEESSEFICRNTFDHPYPTTKLMWIPDTKGVYPDLLATSGDYLRVWRVGETET RLECLLNNNKNSDFCAPLTSFDWNEVDPYLLGTSSIDTTCTIWGLETGQVLGRV NLVSGHVKTQLIAHDKEVYDIAFSRAGGGRDMFASVGADGSVRMFDLRHLEH STIIYEDPQHHPLLRLCWNKQDPNYLATMAMDGMEVVILDVRVPAHLXPGTTIE HVSMALLGPHIHPATSALQRMTTRLSSGTSSKCPEPLRTLSWPTQLXGEINNVQ WASTQPELSPSATTTAWRYSECSVGGAVPTRQGLLYFLPLPHPQS (SEQ ID NO:679); MSLHGKRKEIYKYEAPWTVYAMNWSVRPDKRFRLALGSFV EEYNNKVQLVGLDEESSEFICRNTFDHPYPTTKLMWIPDTKGVYPDLLATSGDY LRVWRVGETETRLECLLNNNKNSDFCAPLTSFDWNEVDPYLL (SEQ ID NO:680); SFDWNEVDPYLLGTSSIDTTCTIWGLETGQVLGRVNLVSGHVK TQLIAHDKEVYDIAFSRAGGGRDMFASVGADGSVRMFDLRHLEHSTIIYEDPQH HPLLRLCWNKQDPNYLATMAMDGMEVVILDVRVPAHLXPGTTI (SEQ ID NO:681); VGADGSVRMFDLRHLEHSTIIYEDPQHHPLLRLCWNKQDPNYLA TMAMDGMEVVILDVRVPAHLXPGTTIEHVSMALLGPHIHPATSALQRMTTRLS SGTSSKCPEPLRTLSWPTQLXGEINNVQWASTQPELSPSATTTAWRYSECSVG GAVPTRQGLLYFLPLPHPQS (SEQ ID NO:682). Also preferred are polynucleotide fragments encoding these polypeptide fragments.

This gene is expressed primarily in placenta, embryo, T cell and fetal lung and to a lesser extent in endothelial, tonsil and bone marrow.

Therefore, polynucleotides and polypeptides of the invention are useful as reagents for differential identification of the tissue(s) or cell type(s) present in a biological sample and for diagnosis of diseases and conditions, which include, but are not limited to, immunological and developmental diseases in addition to cancers. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 369 as residues: Gly-19 to Gln-28, Pro-36 to Phe-42.

The tissue distribution in tumors of colon, ovary, and breast origins indicates that polynucleotides and polypeptides corresponding to this gene are useful for diagnosis and intervention of these tumors, in addition to other tumors where expression has been indicated. Protein, as well as, antibodies directed against the protein may show utility as a tumor marker and/or immunotherapy targets for the above listed tumors and tissues. Because the gene is expressed in cells of lymphoid origin, the natural gene product may be involved in immune functions. Therefore it may be also used as an agent for immunological disorders including arthritis, asthma, immune deficiency diseases such as AIDS, and leukemia.

### FEATURES OF PROTEIN ENCODED BY GENE NO: 137

This gene is expressed primarily in TNF and INF induced epithelial cells, T cells and kidney.

Therefore, polynucleotides and polypeptides of the invention are useful as reagents for differential identification of the tissue(s) or cell type(s) present in a biological sample and for diagnosis of diseases and conditions, which include, but are not limited to, inflammatory conditions particularly inflammatory reactions in the kidney. Similarly, polypeptides and antibodies directed to these polypeptides are useful in providing immunological probes for differential identification of the tissue(s) or cell type(s). For a number of disorders of the above tissues or cells, particularly of renal system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 370 as residues: Thr-67 to Gly-72, Gln-132 to Ala-145, Arg-150 to Pro-157.

The tissue distribution indicates that the protein products of this gene are useful for treating the damage caused by inflammation of the kidney.

### FEATURES OF PROTEIN ENCODED BY GENE NO: 138

This gene maps to chromosome 1, and therefore, may be used as a marker in linkage analysis for chromosome 1 (See Accession No. D63485).

This gene is expressed primarily in breast cancer and colon cancer and to a lesser extent in thymus and fetal spleen.

Therefore, polynucleotides and polypeptides of the invention are useful as reagents for differential identification of the tissue(s) or cell type(s) present in a biological sample and for diagnosis of diseases and conditions, which include, but are not limited to, cancers, especially of the breast and colon tissues. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution in tumors of colon and breast origins indicates that polynucleotides and polypeptides corresponding to this gene are useful for diagnosis and intervention of these tumors, in addition to other tumors where expression has been indicated. Protein, as well as, antibodies directed against the protein may show utility as a tumor marker and/or immunotherapy targets for the above listed tumors and tissues.

### FEATURES OF PROTEIN ENCODED BY GENE NO: 139

This gene maps to chromosome 17, and therefore, can be used as a marker for linkage analysis from chromosome 17.

This gene is expressed primarily in CD34 positive cells, and to lesser extent in activated T-cells and neutrophils.

Therefore, polynucleotides and polypeptides of the invention are useful as reagents for differential identification of the tissue(s) or cell type(s) present in a biological sample and for diagnosis of diseases and conditions, which include, but are not limited to, immunologically related diseases and hematopoietic disorders. Similarly, polypeptides and antibodies directed to these polypeptides are useful in providing immunological probes for differential identification of the tissue(s) or cell type(s). For a number of disorders of the above tissues or cells, particularly of the immune system and hematopoietic system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution in CD34, T-cell and neutrophils indicates that polynucleotides and polypeptides corresponding to this gene are useful for treatment and diagnosis of hematopoietic disorders and immunologically related diseases, such as anemia, leukemia, inflammation, infection, allergy, immunodeficiency disorders, arthritis, asthma, immune deficiency diseases such as AIDS.

### FEATURES OF PROTEIN ENCODED BY GENE NO: 140

This gene was recently cloned by another group, who called the gene KIAA0313 gene. (See Accession No. d1021609.) Preferred polypeptide fragments comprise the amino acid sequence:
LYATATVISSPSTEXLSQDQGDRASLDAADSGRGSWTSCSSGSHDNIQTIQ HQRSWETLPFGHTHFDYSGDPAGLWASSSHMDQIMFSDHSTKYNRQNQSRES LEQAQSRASWASSTGYWGEDSEGDTGTIKRRGGKDVSIEAESSSLTSVTTEETK PVPMPAHIAVASSTTKGLIARKEGRYREPPPTPPGYIGIPITDFPEGHSHPARKP PDYNVALQRSRMVARSSDTAGPSSVQQPHGHPTSSRPVNKPQWHKXNESDPR LAPYQSQGFSTEEDEDEQVSAV (SEQ ID NO:683); HMDQIMFSDHSTKYNRQ NQSRESLEQAQSRASWASSTGYWGE (SEQ ID NO:684); SVTTEETKPVPMP AHIAVASSTTKGLIARKEGRYREPPPTPPGYIGIPITD (SEQ ID NO:685); and VALQRSRMVARSSDTAGPSSVQQPHGHPTSSRPVNKPQW HKXNESDPRLAPYQSQGF (SEQ ID NO:686). Also preferred are polynucleotide fragments encoding these polypeptide fragments. This gene maps to chromosome 4, and therefore, may be used as a marker in linkage analysis for chromosome 4 (See Accession No. AB002311).

This gene is expressed primarily in ovarian cancer, tumors of the Testis, brain, and colon.

Therefore, polynucleotides and polypeptides of the invention are useful as reagents for differential identification of the tissue(s) or cell type(s) present in a biological sample and for diagnosis of diseases and conditions, which include, but are not limited to, ovarian, testicle, brain and colon cancers. Similarly, polypeptides and antibodies directed to these polypeptides are useful in providing immunological probes for differential identification of the tissue(s) or cell type(s). For a number of disorders of the above tissues or cells, particularly of the male and female reproductive systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution in tumors of colon, ovary, testis, and brain origins indicates that polynucleotides and polypeptides corresponding to this gene are useful for diagnosis and intervention of these tumors, in addition to other tumors where expression has been indicated. Protein, as well as, antibodies directed against the protein may show utility as a tumor marker and/or immunotherapy targets for the above listed tumors and tissues.

### FEATURES OF PROTEIN ENCODED BY GENE NO: 141

This gene is expressed primarily in spleen and colon cancer.

Therefore, polynucleotides and polypeptides of the invention are useful as reagents for differential identification of the tissue(s) or cell type(s) present in a biological sample and for diagnosis of diseases and conditions, which include, but are not limited to, colon cancer and immunological disorders. Similarly, polypeptides and antibodies directed to these polypeptides are useful in providing immunological probes for differential identification of the tissue(s) or cell type(s). For a number of disorders of the above tissues or cells, particularly of the gastrointestinal trace and immune systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution in tumors of colon, ovary, and breast origins indicates that polynucleotides and polypeptides corresponding to this gene are useful for diagnosis and intervention of these tumors, in addition to other tumors where expression has been indicated. Protein, as well as, antibodies directed against the protein may show utility as a tumor marker and/or immunotherapy targets for the above listed tumors and tissues.

### FEATURES OF PROTEIN ENCODED BY GENE NO: 142

Translation product is homologous to T cell translocation protein, a putative zinc finger factor (See Accession No. 340454); as well as to the G-protein coupled receptor TM5 consensus polypeptide (See Accession No. R50734). Preferred polypeptide fragments comprise the following amino acid sequence:
CLLFVFVSLGMRCLFWTIVYNVLYLKHKCNTVLLCYHLCSI (SEQ ID NO:687); ACSKLIPAFEMVMRAKDNVYHLDCFACQLCNQRXCVGDKFFLKNNXXLCQT DYEEGLMKEGYAPXVR (SEQ ID NO:688). Also preferred are polynucleotide fragments encoding these polypeptide fragments.

This gene is expressed primarily in fetal brain.

Therefore, polynucleotides and polypeptides of the invention are useful as reagents for differential identification of the tissue(s) or cell type(s) present in a biological sample and for diagnosis of diseases and conditions, which include, but are not limited to, neurological disorders including brain cancer. Similarly, polypeptides and antibodies directed to these polypeptides are useful in providing immunological probes for differential identification of the tissue(s) or cell type(s). For a number of disorders of the above tissues or cells, particularly of the Central Nervous System, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the detection/treatment of neurodegenerative disease states and behavioral disorders such as Alzheimer's Disease, Parkinson's Disease, Huntingtons Disease, schizophrenia, mania, dementia, paranoia, obsessive compulsive disorder and panic disorder. In addition, the gene or gene product may also play a role in the treatment and/or detection of developmental disorders associated with the developing embryo.

### FEATURES OF PROTEIN ENCODED BY GENE NO: 143

Translation product for this gene has significant homology to the Fas ligand, which is a cysteine-rich type II transmembrane protein/tumor necrosis factor receptor homolog. Mutations within this protein have been shown to result in generalized lymphoproliferative disease leading to the development of lymphadenopathy and autoimmune disease (See Medline Article No. 94185175). Preferred polypeptide fragments comprise the following amino acid sequence:
SALSEPGAPDRRRPCPESVPRRPDDEQWPPPTALCLDVAPLPPSS (SEQ ID NO:689). Also preferred are polynucleotide fragments encoding these polypeptide fragments (See Accession No. 473565).

This gene is expressed primarily in osteoblasts, lung, and brain.

Therefore, polynucleotides and polypeptides of the invention are useful as reagents for differential identification of the tissue(s) or cell type(s) present in a biological sample and for diagnosis of diseases and conditions, which include, but are not limited to, osteoblast-related, pulmonary, neurological, and immunological diseases. Similarly, polypeptides and antibodies directed to these polypeptides are useful in providing immunological probes for differential identification of the tissue(s) or cell type(s). For a number of disorders of the above tissues or cells, particularly of the skeletal and nervous systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 376 as residues: Trp-33 to Thr-40, Lys-45 to Ile-63.

The tissue distribution in osteoblasts, lung, and brain combined with its homology to the Fas ligand indicates that polynucleotides and polypeptides corresponding to this gene are useful for diagnosis and intervention of these tumors, in addition to other tumors where expression has been indicated. Protein, as well as, antibodies directed against the protein may show utility as a tumor marker and/or immunotherapy targets for the above listed tumors and tissues. Because the Fas ligand gene is known to be expressed in cells of lymphoid origin, the natural gene product may be involved in immune functions. Therefore it may be also used as an agent for immunological disorders including asthma, immune deficiency diseases such as AIDS and leukemia, and various autoimmune disorders including lupus and arthritis.

### FEATURES OF PROTEIN ENCODED BY GENE NO: 144

This gene shares sequence homology with a 21.5 KD transmembrane protein in the SEC15-SAP4 intergenic region of yeast. (See Accession No. 1723971.) Preferred polypeptide fragments comprise the amino acid sequence:
AHASESGERWWACCGVRFGLRSIEAIGRSCCHDGPGGLVANRGRRFKWAIEL SGPGGGSRGRSDRGSGQGDSLYPVGYLDKQVPDTSVQETDRILVEKRCWDIAL GPLKQIPMNLFIMYMAGNTISIFPTMMVCMMAWRPIQALMAISATFKMLESSSQ KFLQGLVYLIGNLMGLALAVYKCQSMGLLPTHASDWLAFIEPPERMEFSGG GLLL (SEQ ID NO:691); PVGYLDKQVPDTSVQETDRILVEKRCWDIALGPLKQ IPMNLFI (SEQ ID NO:693); and ATFKMLESSSQKFLQGLVYLIGNLMGLALAV YKCQSMGLLPTHASD (SEQ ID NO:692). Also preferred are polynucleotide fragments encoding these polypeptide fragments.

This gene is expressed primarily in osteoclastoma, hemangiopericytoma, liver, lung.

Therefore, polynucleotides and polypeptides of the invention are useful as reagents for differential identification of the tissue(s) or cell type(s) present in a biological sample and for diagnosis of diseases and conditions, which include, but are not limited to, osteoclastoma, hemangiopericytoma, liver and lung tumors. Similarly, polypeptides and antibodies directed to these polypeptides are useful in providing immunological probes for differential identification of the above tissue(s) or cell type(s). For a number of disorders of the above tissues or cells, particularly of the lung and liver systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosing osteoclastoma, hemangiopericytoma, liver and lung tumors.

### FEATURES OF PROTEIN ENCODED BY GENE NO: 145

Translation product of this gene shares homology with the glucagon-69 gene which may indicate this gene plays a role in regulating metabolism. (See Accession No. A60318) One embodiment for this gene is the polypeptide fragments comprising the following amino acid sequence:
PTTKLDIMEKKKHIQIRFPSFYHKLVDSGRMRSKRETRREDSDTKHNL (SEQ ID NO:694). An additional embodiment is the polynucleotide fragments encoding these polypeptide fragments.

This gene is expressed primarily in brain, kidney, colon, and testis.

Therefore, polynucleotides and polypeptides of the invention are useful as reagents for differential identification of the tissue(s) or cell type(s) present in a biological sample and for diagnosis of diseases and conditions, which include, but are not limited to, brain, kidney, colon, and testicular cancer. Similarly, polypeptides and antibodies directed to these polypeptides are useful in providing immunological probes for differential identification of the tissue(s) or cell type(s). For a number of disorders of the above tissues or cells, particularly of the male reproductive system, neurological, circulatory, and gastrointestinal systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution in tumors of brain, kidney, colon, and testis origins, indicates that polynucleotides and polypeptides corresponding to this gene are useful for diagnosis and intervention of these tumors, in addition to other tumors where expression has been indicated. Protein, as well as, antibodies directed against the protein may show utility as a tumor marker and/or immunotherapy targets for the above listed tumors and tissues. The tissue distribution indicates that polynucleotides and polypeptides corresponding to this gene are useful for the detection/treatment of neurodegenerative disease states and behavioral disorders such as Alzheimer's Disease, Parkinson's Disease, Huntingtons Disease, schizophrenia, mania, dementia, paranoia, obsessive compulsive disorder and panic disorder. In addition, the gene or gene product may also play a role in the treatment and/or detection of developmental disorders associated with the developing embryo, sexually-linked disorders, or disorders of the cardiovascular system.

### FEATURES OF PROTEIN ENCODED BY GENE NO: 146

The translation product of this gene shares sequence homology with goliath protein which is thought to be important in the regulation of gene expression during development. Protein may serve as a transcription factor. One embodiment for this gene is the polypeptide fragments comprising the following amino acid sequence:
TEHIIAVMITELRGKDILSYLEKNISVQMTIAVGTRMPPKNFSRGSLVFVSISFIV LMIISSAWLIFYFIQKIRYTNARDRNQRRLGDAAKKAISKLTTRTVKKGDKETD PDFDHCAVCIESYKQNDVVRILPCKHVFHKSCVDPWLSEHCTCPMCKLNILKA LGIV (SEQ ID NO:695); TEHIIAVMITELRGKDILSYLEKNISVQMTIAVGTRMP PKNFSRGSLVFVSISFIVLM IISSAWLIFYF (SEQ ID NO:697); SISFIVLMIISSA WLIFYFIQKIRYTNARDRNQRRLGDAAKKAISKLTTRTVKKGDKE (SEQ ID NO:698); VKKGDKETDPDFDHCAVCIESYKQNDVVRILPCKHVFHKSCVDP WLSEHCTCPMCKLNILKALGIV (SEQ ID NO:699). An additional embodiment is the polynucleotide fragments encoding these polypeptide fragments (See Accession No. 157535). Moreover, another embodiment is the polynucleotide fragments encoding these polypeptide fragments:
MTHPGTEHIIAVMITELRGKDILSYLEKNISVQMTIAVGTRMPPKNFSRGS LVFVSISFIVLMIISSAWLIFYWQKIRYTNARDRNQRRLGDAAKKAISKLTTRTV KKGDKETDPDFDHCAVCIESYKQNDWRILPCKHVFHKSCVDPWLSEHCTCP MCKLNILKALGIVPNLPCTDNVAFDMERLTRTQAVNRRSALGDLAGDNSLGLE PLRTSGISPLPQDGELTPRTGEINIAVTKEWFIIASFGLLSALTLCYMIIRATASLN ANEVEWF (SEQ ID NO:696);MTHPGTEHIIAVMITELRGKDILSYLEKNISVQM TIAVGTRMPPKNFSRGSLVFVSISFIVLMIISSAWLIFYFIQKIRYTNARDRNQRR LGDAAKKAISKLTTRT (SEQ ID NO:700); AAKKAISKLTTRTVKKGDKE TDPDFDHCAVCIESYKQNDVVRILPCKHVFHKSCVDPWLSEHCTCPMCKLNIL KALGIVPNLPC (SEQ ID NO:701); TQAVNRRSALGDLAGDNSLGLEPLRTSGI SPLPQDGELTPRTGEINIAVTKEWFIIASFCLLSALTLCYMIIRATASLNANEVEW F (SEQ ID NO:702); PLHGVADHLGCDPQTRFFVPPNIKQWIALLQRGNCTF KEKISRAAFHNAVAWIYNNKSKEEPVTMTHPGTEHIIAVMITELRGKDILSYLE KNISVQMTIAVGTRMPPKNFSRGSLVFVSISFIVLMIISSAWLIFYFIQKIRYTNA RDRNQRRLGDAAKKAISKLTTRTVKKGDKETDPDFDHCAVCIESYKQNDVVRI LPCKHVFHKSCVDPWLSEHCTCPMCKLNILKALGIVPNLPCTDNVAFDMERLT RTQAVNRRSALGDLAGDNSLGLEPLRTSGISPLPQDGELTPRTGEINIAVTKEW FIIASFGLLSALTLCYMIIRATASLNANEVEWF(SEQ ID NO:703); and HGVADHLGCDPQTRFFVPPNIKQWIALLQRGNCTFKEKISRAAFHNAVAVVIY NNKSKEE (SEQ ID NO:704). An additional embodiment is the polynucleotide fragments encoding these polypeptide fragments. When tested against Jurkat cell lines, supernatants removed from cells containing this gene activated the GAS pathway. Thus, it is likely that this gene activates immune cells through the JAKS/STAT signal transduction pathway.

This gene is expressed primarily in macrophage, breast, kidney and to a lesser extent in synovium, hypothalamus and rhabdomyosarcoma.

Therefore, polynucleotides and polypeptides of the invention are useful as reagents for differential identification of the tissue(s) or cell type(s) present in a biological sample and for diagnosis of diseases and conditions, which include, but are not limited to, schizophrenia and cancer. Similarly, polypeptides and antibodies directed to these polypeptides are useful in providing immunological probes for differential identification of the tissue(s) or cell type(s). For a number of disorders of the above tissues or cells, particularly of the immune and neural system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to zinc finger protein indicates that polynucleotides and polypeptides corresponding to this gene are useful for the treatment of schizophrenia, kidney disease and other cancers. The tissue distribution in macrophage, breast, and kidney origins indicates that polynucleotides and polypeptides corresponding to this gene are useful for diagnosis and intervention of tumors within these tissues, in addition to other tumors where expression has been indicated. Protein, as well as, antibodies directed against the protein may show utility as a tumor marker and/or immunotherapy targets for the above listed tumors and tissues. Because the gene is expressed in cells of lymphoid origin, the natural gene product may be involved in immune functions. Therefore it may be also used as an agent for immunological disorders including arthritis, asthma, immune deficiency diseases such as AIDS, and leukemia.

### FEATURES OF PROTEIN ENCODED BY GENE NO: 147

The translation product of this gene shares sequence homology with HNP36 protein, an equilibrative nucleoside transporter, which is thought to be important in gene transcription as well as serving as an important component of the nucleoside transport apparatus (See Accession No. 1845345). One embodiment for this gene is the polypeptide fragments comprising the following amino acid sequence:
MSGQGLAGFFASVAMICAIASGSELSESAFGYFITACAVIILTIICYLGLPRLEFYR YYQQLKLEGPGEQETKLDLISKGEEPRAGKEESGVSVSNSQPTNESHSIKAILK NISVLAFSVCFIFTITIGMFPAVTVEVKSSIAGSSTWERYFIPVSCFLTFNIFDWLG RSLTAVFMWPGKDSRWLPSWXLARLVFVPLLLLCNIKPRRYLTVVFEHDAWFI FFMAAFAFSNGYLASLCMCFGPKKVKPAEAETAEPSWPSSCVWVWHWGLFS PSCSGQLCDKGWTEGLPASLPVCLLPLPSARGDPEWSGGFFF (SEQ ID NO:705); MSGQGLAGFFASVAMICAIASGSELSESAFGYFITACAVIILTIIC YLGLPRLEFYRYYQQLKLE GPGEQETKLDLISKGEEPRAGKEESGVSVSNSQ PTNESHSI (SEQ ID NO:706); SGVSVSNSQPTNESHSIKAILKNISVLAFSVCFI FTITIGMFPAVTVEVKSSIAGSSTWERYFIPVSCFLTFNIFDWLGRS (SEQ ID NO:707),TIGMFPAVTVEVKSSIAGSSTWERYFIPVSCFLTFNIFDWLGRSLTAVF MWPGKDSRWLPSWXLARLVFVPLLLLCNIK PRRYLTVVFEHDA (SEQ ID NO:708); FGPKKVKPAEAETAEPSWPSSCVWVWHWGLFSPSCSGQLCDK GWTEGLPASLPVCLLPLPSARGDPEWSGGFFF (SEQ ID NO:709). An additional embodiment is the polynucleotide fragments encoding these polypeptide fragments.

This gene is expressed primarily in eosinophils and aortic endothelium and to a lesser extent in umbilical vein endothelial cell and thymus.

Therefore, polynucleotides and polypeptides of the invention are useful as reagents for differential identification of the tissue(s) or cell type(s) present in a biological sample and for diagnosis of diseases and conditions, which include, but are not limited to, hematopoietic disease. Similarly, polypeptides and antibodies directed to these polypeptides are useful in providing immunological probes for differential identification of the tissue(s) or cell type(s). For a number of disorders of the above tissues or cells, particularly of the circular system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to HNP36 protein indicates that polynucleotides and polypeptides corresponding to this gene are useful for the treatment of blood neoplasias and other hematopoietic disease.

### FEATURES OF PROTEIN ENCODED BY GENE NO: 148

This gene is expressed primarily in breast cancer cell lines, thymus stromal cells, and ovary.

Therefore, polynucleotides and polypeptides of the invention are useful as reagents for differential identification of the tissue(s) or cell type(s) present in a biological sample and for diagnosis of diseases and conditions, which include, but are not limited to, endocrine and female reproductive system diseases including breast cancer. Similarly, polypeptides and antibodies directed to these polypeptides are useful in providing immunological probes for differential identification of the tissue(s) or cell type(s). For a number of disorders of the above tissues or cells, particularly of the endocrine system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis and treatment of endocrine disorders. In addition, the tissue distribution in tumors of thymus, ovary, and breast origins indicates that polynucleotides and polypeptides corresponding to this gene are useful for diagnosis and intervention of these tumors, in addition to other tumors where expression has been indicated. Protein, as well as, antibodies directed against the protein may show utility as a tumor marker and/or immunotherapy targets for the above listed tumors and tissues

### FEATURES OF PROTEIN ENCODED BY GENE NO: 149

Translation product of this gene has homology to pmt1 and pmt 2, two conserved schizosaccharomyces pombe genes. One embodiment for this gene is the polypeptide fragments comprising the following amino acid sequence:
DDDGFEIVPIEDPAKHRILDPEGLALGAVIASSKKAKRDLIDNSFNRYTFNEDEG ELPEWFVQEEKQHRIRQLPVGKKEVEHYRKRWREINARPIXXXXXXXXXXX XXXXXXLEQTRKKAEAVVNTVDIXRTRES (SEQ ID NO:710); DDDGFEIVPIEDPAKHRILDPEGLALGAVIASSKKAKRDLIDNSFNRYTF (SEQ ID NO:711); KRWREINARPIXXXXXXXXXXXXXXXXXLEQTRKKAE AVVNTVDIXRTRES (SEQ ID NO:712). An additional embodiment is the polynucleotide fragments encoding these polypeptide fragments (See Accession No. e1216734).

This gene is expressed primarily in retina and ovary and to a lesser extent in brreast cancer cell, epididymus and osteosarcoma.

Therefore, polynucleotides and polypeptides of the invention are useful as reagents for differential identification of the tissue(s) or cell type(s) present in a biological sample and for diagnosis of diseases and conditions, which include, but are not limited to, neuronal growth disorders, cancer and reproductive system disorders. Similarly, polypeptides and antibodies directed to these polypeptides are useful in providing immunological probes for differential identification of the tissue(s) or cell type(s). For a number of disorders of the above tissues or cells, particularly of the neural and reproductive system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 382 as residues: Met-1 to Gly-7.

The tissue distribution indicates that polynucleotides and polypeptides corresponding to this gene are useful for the diagnosis or treatment of reproductive system disease and cancers.

### FEATURES OF PROTEIN ENCODED BY GENE NO: 150

One embodiment for this gene is the polypeptide fragments comprising the following amino acid sequence:
MIKDKGRARTALTSSQPAHLCPENPLLHLKAAVKEKKRNKKKKTIGSPKRIQS PLNNKLLNSPAKTLPGACGSPQKLIDGFLKHEGPPAEKPLEELSASTSGVPGLS SLQSDPAGCVRPPAPNLAGAVEFNDVKTLLREWITTISDPMEEDILQVVKYCTD LIEEKDLEKLDLVIKYMKRLMQQSVESVWNMAFDFILDNVQVVLQQTYGSTLK VT (SEQ ID NO:713); MIKDKGRARTALTSSQPAHLCPENPLLHLKAAVKE KKRNKKKKTIGSPKRIQ (SEQ ID NO:714); KRIQSPLNNKLLNSPAKT LPGACGSPQKLIDGFLKHEGPPAEKPLEELSASTSGVPGLSSLQSDPAGCVRPP APNLAGAVEFNDVKTLLREWITTISDPM (SEQ ID NO:715); TISDPMEEDILQVVKYCTDLIEEKDLEKLDLVIKYMKRLMQQSVE SVWNMAFDFILDNVQVVLQQTYGSTLKVT (SEQ ID NO:716). An additional embodiment is the polynucleotide fragments encoding these polypeptide fragments.

This gene is expressed primarily in 12 week embryo and to a lesser extent in hemangiopericytoma and frontal cortex.

Therefore, polynucleotides and polypeptides of the invention are useful as reagents for differential identification of the tissue(s) or cell type(s) present in a biological sample and for diagnosis of diseases and conditions, which include, but are not limited to, growth disorders and hemangiopericytoma. Similarly, polypeptides and antibodies directed to these polypeptides are useful in providing immunological probes for differential identification of the tissue(s) or cell type(s). For a number of disorders of the above tissues or cells, particularly of the circular and neural system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 383 as residues: Leu-4 to Lys-11.

The tissue distribution indicates that polynucleotides and polypeptides corresponding to this gene are useful for the treatment of growth disorders, hemangiopericytoma and other soft tissue tumors.

### FEATURES OF PROTEIN ENCODED BY GENE NO: 151

The translation product of this gene has been found to have homology to a human DNA mismatch repair protein PMS3. Preferred polypeptide fragments comprise the following amino acid sequence: FCHDCKFPEASPAMNCEP (SEQ ID NO:717). Also preferred are polynucleotide fragments encoding these polypeptide fragments (See Accession No. R95250).

This gene is expressed primarily in neutrophils.

Therefore, polynucleotides and polypeptides of the invention are useful as reagents for differential identification of the tissue(s) or cell type(s) present in a biological sample and for diagnosis of diseases and conditions, which include, but are not limited to, lymphoma, immunodeficiency diseases, and cancers resulting from genetic instability. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 384 as residues: Met-1 to Lys-6.

The tissue distribution in neutrophils and the sequence homology indicates that polynucleotides and polypeptides corresponding to this gene are useful for diagnosis of Hodgkin's lymphoma, since the elevated expression and secretion by the tumor mass may be indicative of tumors of this type. Additionally the gene product may be used as a target in the immunotherapy of the cancer. Because the gene is expressed in cells of lymphoid origin, the natural gene product may be involved in immune functions. Therefore it may be also used as an agent for immunological disorders including arthritis, asthma, immune deficiency diseases such as AIDS, and leukemia. Furthermore, its homology to a known DNA repair protein would suggest gene may be useful in establishing cancer predisposition and prevention in gene therapy applications.

### FEATURES OF PROTEIN ENCODED BY GENE NO: 152

This gene is expressed primarily in neutrophils.

Therefore, polynucleotides and polypeptides of the invention are useful as reagents for differential identification of the tissue(s) or cell type(s) present in a biological sample and for diagnosis of diseases and conditions, which include, but are not limited to, infectious diseases and lymphoma. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the treatment of inflammation and infectious diseases.

### FEATURES OF PROTEIN ENCODED BY GENE NO: 153

One embodiment for this gene is the polypeptide fragments comprising the following amino acid sequence:
MASSVPAGGHTRAGGIFLIGKLDLEASLFKSFQWLPFVLRKKC NFFCWDSSAHSLPLHPLSASCSAPACHASDTHLLYPSTRALCPSIFAWLVAPHS VFRTNAPGPTPSSQSSPVFPVFPVSFMALIVCXLVCC (SEQ ID NO:720); MASSVPAGGHTRAGGIFLIGKLDLEASLFKSFQWLPFVLRKKCNFFCWDSSAH SLPLHPLSASCSAPACHA (SEQ ID NO:721);FAWLVAPHSVFRTNAPGPTPS SQSSPVFPVFPVSFMALIVCXLVCC (SEQ ID NO:722). An additional embodiment is the polynucleotide fragments encoding these polypeptide fragments.

This gene is expressed primarily in neutrophils.

Therefore, polynucleotides and polypeptides of the invention are useful as reagents for differential identification of the tissue(s) or cell type(s) present in a biological sample and for diagnosis of diseases and conditions, which include, but are not limited to, inflammation and infectious disease. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 386 as residues: Ser-11 to Pro-17.

The tissue distribution indicates that polynucleotides and polypeptides corresponding to this gene are useful for the treatment of infectious diseases and inflammation.

### FEATURES OF PROTEIN ENCODED BY GENE NO: 154

This gene is expressed in multiple tissues including ovary, uterus, adipose tissue, brain, and the liver.

Therefore, polynucleotides and polypeptides of the invention are useful as reagents for differential identification of the tissue(s) or cell type(s) present in a biological sample and for diagnosis of diseases and conditions, which include, but are not limited to, uterine, ovarian, brain, and liver cancer. Similarly, polypeptides and antibodies directed to these polypeptides are useful in providing immunological probes for differential identification of the tissue(s) or cell type(s). For a number of disorders of the above tissues or cells, particularly of the female reproductive system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution of this gene indicates that polynucleotides and polypeptides corresponding to this gene are useful for diagnostic or therapeutic uses in the treatment of the female reproductive system, obesity, and liver disorders, particularly cancer in the above tissues.

### FEATURES OF PROTEIN ENCODED BY GENE NO: 155

This gene maps to chromosome 3, and therefore, may be used as a marker in linkage analysis for chromosome 3 (See Accession No. D87452).

This gene is expressed in multiple tissues including brain, aortic endothelial cells, smooth muscle, pituitary, testis, melancytes, spleen, nertrophils, and placenta.

Therefore, polynucleotides and polypeptides of the invention are useful as reagents for differential identification of the tissue(s) or cell type(s) present in a biological sample and for diagnosis of diseases and conditions, which include, but are not limited to, immunological disorders including immunodeficiencies, cancers of the brain and the female reproductive system, as well as cardiovascular disorders, such as atherosclerosis and stroke. Similarly, polypeptides and antibodies directed to these polypeptides are useful in providing immunological probes for differential identification of the tissue(s) or cell type(s). For a number of disorders of the above tissues or cells, particularly of the central nervous and immune systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution suggest that polynucleotides and polypeptides corresponding to this gene are useful in treatment/detection of disorders in the nervous system, including schizophrenia, neurodegeneration, neoplasia, brain cancer as well as cardiovascular and female reproductive disorders including cancer within the above tissues.

### FEATURES OF PROTEIN ENCODED BY GENE NO: 156

The translation product of this gene shares sequence homology with the human gene encoding cytochrome b561 (See Accession No. P10897). Cytochrome b561 is a transmembrane electron transport protein that is specific to a subset of secretory vesicles containing catecholamines and amidated peptides. This protein is thought to supply reducing equivalents to the intravesicular enzymes dopamine-beta-hydroxylase and alpha-peptide amidase. Preferred polypeptides of the invention comprise the amino acid sequence:
MAMEGYWRFLALLGSALLVGFLSVIFALVWVLHYREGLGWDGSALEFNWHP VLMVTGFVFIQGIAIIVYRLPWTWKCSKLLMKSIHAGLNAVAAILAIISVVAVFE NHNVNNIANMYSLHSWVGLIAVICYLLQLLSGFSVFLLPWAPLSLRAFLMPIHV YSGIVIFGTVIATALMGLTEKLIFSLRDPAYSTFPPEGVFVNTLGLLILVFGALIF WIVTRPQWKRPKEPNSTILHPNGGTEQGARGSMPAYSGNNMDKSDSEL NSEVAARKRNLALDEAGQRSTM (SEQ ID NO:724); as well as antigenic fragments of at least 20 amino acids of this gene and/or biologically active fragments. Also preferred are polynucleotide fragments encoding these polypeptide fragments.

This gene is expressed primarily in anergic T-cells.

Therefore, polynucleotides and polypeptides of the invention are useful as reagents for differential identification of the tissue(s) or cell type(s) present in a biological sample and for diagnosis of diseases and conditions, which include, but are not limited to, immune system and metabolism related diseases. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that the protein product or RNA of this gene is useful for treatment or diagnosis of immune system and metabolic diseases or conditions including Tay-Sachs disease, phenylketonuria, galactosemia, various porphyrias, and Hurler's syndrome.

### FEATURES OF PROTEIN ENCODED BY GENE NO: 157

The translation product of this gene shares sequence homology with collagen which is important in mammalian development. This gene also shows sequence homology with bcl-2. (See Accession No. P80988.) Preferred polypeptide fragments comprise the amino acid sequence: PGRAGPSPGLSLQLPAEPGHPAGNLAPL TSRPQPLCRIPAVPG (SEQ ID NO:725). Also preferred are polynucleotide sequences encoding this polypeptide fragment.

This gene is expressed primarily in HL-60 tissue culture cells and to a lesser extent in liver, breast, and uterus.

Therefore, polynucleotides and polypeptides of the invention are useful as reagents for differential identification of the tissue(s) or cell type(s) present in a biological sample and for diagnosis of diseases and conditions, which include, but are not limited to, immunological diseases, hereditary disorders involving the MHC class of immune molecules, as well as developmental disorders and reproductive disorders. Similarly, polypeptides and antibodies directed to these polypeptides are useful in providing immunological probes for differential identification of the tissue(s) or cell type(s). For a number of disorders of the above tissues or cells, particularly of the immune and reproductive system expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 390 as residues: Ser-39 to Gly-46, Leu-49 to Ala-62.

The tissue distribution and homology to collagen indicates that polynucleotides and polypeptides corresponding to this gene are useful for diagnosis and treatment of hereditary MHC disorders and particularly autoimmune disorders including rheumatoid arthritis, lupus, scleroderma, and dermatomyositis, as well as many reproductive disorders, including cancer of the uterus, and breast tissues.

### FEATURES OF PROTEIN ENCODED BY GENE NO: 158

This gene is expressed primarily in the amygdala region of the brain.

Therefore, polynucleotides and polypeptides of the invention are useful as reagents for differential identification of the tissue(s) or cell type(s) present in a biological sample and for diagnosis of diseases and conditions, which include, but are not limited to, a variety of brain disorders, particularly those effecting mood and personality. Similarly, polypeptides and antibodies directed to these polypeptides are useful in providing immunological probes for differential identification of the tissue(s) or cell type(s). For a number of disorders of the above tissues or cells, particularly of the brain and central nervous system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment and/or diagnosis of a variety of brain disorders, particularly bipolar disorder, unipolar depression, and dementia.

### FEATURES OF PROTEIN ENCODED BY GENE NO: 159

This gene is expressed in a variety of tissues and cell types including brain, smooth muscle, kidney, salivary gland and T-cells.

Therefore, polynucleotides and polypeptides of the invention are useful as reagents for differential identification of the tissue(s) or cell type(s) present in a biological sample and for diagnosis of diseases and conditions, which include, but are not limited to, cancers of a variety of organs including brain, smooth muscle, kidney, salivary gland and T-cells and cardiovascular disorders. Similarly, polypeptides and antibodies directed to these polypeptides are useful in providing immunological probes for differential identification of the tissue(s) or cell type(s). For a number of disorders of the above tissues or cells, particularly of the central nervous, urinary, salivary, digestive, and immune systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution in brain, smooth muscle, and T-cells indicates that polynucleotides and polypeptides corresponding to this gene are useful for diagnosis of various neurological, and cardiovascular disorders, but not limited to cancer within the above tissues. Additionally the gene product may be used as a target in the immunotherapy of the cancer. Because the gene is expressed in cells of lymphoid origin, the natural gene product may be involved in immune functions. Therefore it may be also used as an agent for immunological disorders including arthritis, asthma, immune deficiency diseases such as AIDS, and leukemia.

### FEATURES OF PROTEIN ENCODED BY GENE NO: 160

The translation product of this gene shares sequence homology with collagen which is thought to be important in cellular interactions, extracellular matrix formation, and has been found to be an identifying determinant in autoimmune disorders. Moreover, this gene shows sequence homology with the yeast protein, Sls1p, an endoplasmic reticulum component, involved in the protein translocation process in Yeast Yarrowia lipolytica. (See Accession No. 1052828; see also J. Biol. Chem. 271, 11668-11675 (1996).) With mouse, this same region shows sequence homology with the heavy chain of kinesin. (See Accession No. 2062607.) Recently, suppression of the heavy chain of kinesin was shown to inhibits insulin secretion from primary cultures of mouse beta-cells. (See Endocrinology 138 (5), 1979-1987 (1997).) Moreover, kinesin was found associated with drug resistance and cell immortalization. (See 468355.) Thus, it is likely that this gene also act as a genetic suppressor elements.

This gene is expressed primarily in the greater omentum and to a lesser extent in a variety of organs and cell types including gall bladder, stromal bone marrow cells, lymph node, liver, testes, pituitary, and thymus.

Therefore, polynucleotides and polypeptides of the invention are useful as reagents for differential identification of the tissue(s) or cell type(s) present in a biological sample and for diagnosis of diseases and conditions, which include, but are not limited to, disorders of the endocrine, gastrointestinal, and immunological systems, including autoimmune disorders and cancers in a variety of organs and cell types. Similarly, polypeptides and antibodies directed to these polypeptides are useful in providing immunological probes for differential identification of the tissue(s) or cell type(s). For a number of disorders of the above tissues or cells, particularly of the immune and gastrointestinal systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 393 as residues: Asn-27 to Leu-47, Gln-81 to Lys-88, Asp-93 to Lys-102, Asn-107 to Leu-116, Met-129 to Glu-141, Glu-150 to Asp-157, Lys-176 to Glu-185, Glu-333 to Tyr-349, Cys-393 to Leu-403, Gln-423 to Gly-429.

The tissue distribution in within various endocrine and immunological tissues combined with the sequence homology to a conserved collagen motif indicates that polynucleotides and polypeptides corresponding to this gene are useful for the diagnosis of various autoimmune disorders including, but not limited to, rheumatoid arthritis, lupus erthyematosus, scleroderma, dermatomyositis Because the gene is expressed in cells of lymphoid origin, the natural gene product may be involved in immune functions. Therefore it may be also used as an agent for immunological disorders including arthritis, asthma, immune deficiency diseases such as AIDS, and leukemia.

### FEATURES OF PROTEIN ENCODED BY GENE NO: 161

This gene has homology to the tissue inhibitor of metalloproteinase 2. Such inhibitors are vital to proper regulation of metalloproteins such as collagenases (See Accession No. P16368). In addition, this gene maps to chromosome 17, and therefore, may be used as a marker in linkage analysis for chromosome 17 (See Accession No. P16368).

This gene is expressed primarily in several types of cancer including osteoclastoma, chondrosarcoma, and rhabdomyosarcoma and to a lesser extent in several non-malignant tissues including synovium, amygdala, testes, placenta.

Therefore, polynucleotides and polypeptides of the invention are useful as reagents for differential identification of the tissue(s) or cell type(s) present in a biological sample and for diagnosis of diseases and conditions, which include, but are not limited to, various types of cancer, particularly cancers of bone and cartilage, as well as various autoimmune disorders. Similarly, polypeptides and antibodies directed to these polypeptides are useful in providing immunological probes for differential identification of the tissue(s) or cell type(s). For a number of disorders of the above tissues or cells, particularly of the musculoskeletal system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution in various cancers and the sequence homology to a collagenase inhibitor indicates that polynucleotides and polypeptides corresponding to this gene are useful for detection of various autoimmune disorders such as rheumatoid arthritis, lupus, scleroderma, and dermatomyositis. Therefore it may be also used as an agent for immunological disorders including arthritis, asthma, immune deficiency diseases such as AIDS, and leukemia.

### FEATURES OF PROTEIN ENCODED BY GENE NO: 162

This gene is homologous to the mitochondrial ATP6 gene and therefore is likely a homolog of this gene family (See Accession No. X76197).

This gene is expressed primarily in brain tissue.

Therefore, polynucleotides and polypeptides of the invention are useful as reagents for differential identification of the tissue(s) or cell type(s) present in a biological sample and for diagnosis of diseases and conditions, which include, but are not limited to, a variety of brain disorders, including Down's syndrome, depression, Schizophrenia, and epilepsy. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution in brain tissue indicates this gene is useful for diagnosis of various neurological disorders including, but not limited to, brain cancer. Additionally the gene product may be used as a target in the immunotherapy of cancer in the brain as well as for the diagnosis of metabolic disorders such as obesity Tay-Sachs disease, phenylketonuria and Hurler's Syndrome.

### FEATURES OF PROTEIN ENCODED BY GENE NO: 163

This gene is expressed primarily in placenta, neutrophils, and microvascular endothelial cells and to a lesser extent in multiple tissues including brain, prostate, spleen, thymus, and bone.

Therefore, polynucleotides and polypeptides of the invention are useful as reagents for differential identification of the tissue(s) or cell type(s) present in a biological sample and for diagnosis of diseases and conditions, which include, but are not limited to, neutropenea and other diseases of the immune system. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution in placenta indicates that polynucleotides and polypeptides corresponding to this gene are useful for diagnosis various female reproductive disorders. Additionally the gene product may be used as a target in the immunotherapy of various cancers. Because the gene is expressed in some cells of lymphoid and endocrine origin, the natural gene product may be involved in immune functions and metabolism regulation, respectively. Therefore it may be also used as an agent for immunological disorders including arthritis, asthma, immune deficiency diseases such as AIDS, and leukemia.

### FEATURES OF PROTEIN ENCODED BY GENE NO: 164

This gene is expressed primarily in neutrophils, monocytes, bone marrow, and fetal liver.

Therefore, polynucleotides and polypeptides of the invention are useful as reagents for differential identification of the tissue(s) or cell type(s) present in a biological sample and for diagnosis of diseases and conditions, which include, but are not limited to, immune system disorders including, but not limited to, autoimmune disorders such as lupus, and immunodeficiency disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution in various immune system tissue indicates that polynucleotides and polypeptides corresponding to this gene are useful for the diagnosis of various immunological disorders such as Hodgkin's lymphoma, arthritis, asthma, immune deficiency diseases such as AIDS, and leukemia.

### FEATURES OF PROTEIN ENCODED BY GENE NO: 165

The translation product of this gene shares sequence homology with dystrophin which is thought to be defective in both Duchene and Becker Muscular Dystrophy. Preferred polypeptide fragments comprise the following amino acid sequence:
MKLLGECSSSIDSVKRLEHKLKEEEESLPGFVNLHSTETQTAGVIDRWELLQAQ ALSKELRMKQNLQKWQQFNSDLNSIWAWLGDTEEELEQLQRLELSTDIQTIELQ IKKLKELQKAVDHRKAIILSINLCSPEFTQADSKESRDLQDRLXQMNGRWDRV CSLLEEWRGLLQDALMQCQGFHEMSHGLLLMLENIDRRKNEIVPIDSNLDAEIL QDHHKQLMQIKHELLESQLRVASLQDMSCQLLVNAEGTDCLEAKEKVHVIGNR LKLLLKEVSRHIKELEKLLDVSSSQQDLSSWSSADELDTSGSVSPXSGRSTPNR QKTPRGKCSLSQPGPSVSSPHSRSTKGGSDSSLSEPXPGRSGRGFLFRVLRAA LPLQLLLLLLIGLACLVPMSEEDYSCALSNNFARSFHPMLRYTNGPPPL (SEQ ID NO:726); MKLLGECSSSIDSVKRLEHKLKEEEESLPGFVNLHSTETQTAGVIDR WELLQAQALSKELRMKQNLQKWQQFNSDLNSIWAWLGDTEEELEQLQRLELS TDIQTIELQIK (SEQ ID NO:727); KLKELQKAVDHRKAIILSINLCSPEFTQADSK ESRDLQDRLXQMNGRWDRVCSLLEEWRGLLQDALMQCQGFHEMSHGLLLML ENIDRRKNEIVPIDSNLDAEILQDHHKQLMQIKHELLESQLRVASLQDMSCQL (SEQ ID NO:728); QDMSCQLLVNAEGTDCLEAKEKVHVIGNRLKLLLKEVS RHIKELEKLLDVSSSQQDLSSWSSADELDTSGSVSPXSGRSTPNRQKTPRGKCS LSQPGPSVSSPHS (SEQ ID NO:729); DSSLSEPXPGRSGRGFLFRVLRAAL PLQLLLLLLIGLACLVPMSEEDYSCALSNNFARSFHPMLRYTNGPPPL (SEQ ID NO:730). Also preferred are polynucleotide fragments encoding these polypeptide fragments. Furthermore, this gene maps to chromosome 6, and therefore, may be used as a marker in linkage analysis for chromosome 6 (See Accession No. N62896).

This gene is expressed in numerous tissues including the heart, kidney, and brain.

Therefore, polynucleotides and polypeptides of the invention are useful as reagents for differential identification of the tissue(s) or cell type(s) present in a biological sample and for diagnosis of diseases and conditions, which include, but are not limited to, musculoskeletal disorders including Muscular Dystrophy and cardiovascular diseases. Similarly, polypeptides and antibodies directed to these polypeptides are useful in providing immunological probes for differential identification of the tissue(s) or cell type(s). For a number of disorders of the above tissues or cells, particularly of the muscle tissues, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to dystrophin indicates that polynucleotides and polypeptides corresponding to this gene are useful for the diagnosis and treatment of Muscular Dystrophy and other muscle disorders.

### FEATURES OF PROTEIN ENCODED BY GENE NO: 166

This gene is expressed primarily in human cerebellum.

Therefore, polynucleotides and polypeptides of the invention are useful as reagents for differential identification of the tissue(s) or cell type(s) present in a biological sample and for diagnosis of diseases and conditions, which include, but are not limited to, diseases of the central nervous system, including Alzheimer's Disease, Parkinson's Disease, ALS, and mental illnesses. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 399 as residues: Pro-20 to Gly-26, Leu-37 to Pro-42, His-57 to Gly-63.

The tissue distribution indicates that the protein products of this gene are useful for treatment/diagnosis of diseases of the central nervous system and may protect or enhance survival of neuronal cells by slowing progression of neurodegenerative diseases.

### FEATURES OF PROTEIN ENCODED BY GENE NO: 167

Preferred polypeptides encoded by this gene comprise the following amino acid sequence:
MKLLICGNYLAPSHSESSRRCCLLCFYPLCLEINFGMKVFLSMPFLVLFQ SLIQED (SEQ ID NO:731). Polynucleotides encoding such polypeptides are also provided. This gene is believed to reside on chromosome 15. Therefore polynucleotides derived from this gene are useful in linkage analysis as chromosome 15 markers.

This gene is expressed primarily in human testes tumor and to a lesser extent in normal human testes.

Therefore, polynucleotides and polypeptides of the invention are useful as reagents for differential identification of the tissue(s) or cell type(s) present in a biological sample and for diagnosis of diseases and conditions, which include, but are not limited to, diseases of the testes, particularly cancer, and other reproductive disorders. Similarly, polypeptides and antibodies directed to these polypeptides are useful in providing immunological probes for differential identification of the tissue(s) or cell type(s). For a number of disorders of the above tissues or cells, particularly of the male reproductive tissues, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that the protein products of this gene are useful for treatment/diagnosis of testicular diseases including cancers.

### FEATURES OF PROTEIN ENCODED BY GENE NO: 168

This gene is expressed primarily in fetal liver.

Therefore, polynucleotides and polypeptides of the invention are useful as reagents for differential identification of the tissue(s) or cell type(s) present in a biological sample and for diagnosis of diseases and conditions, which include, but are not limited to, conditions affecting hematopoietic development and metabolic diseases. Similarly, polypeptides and antibodies directed to these polypeptides are useful in providing immunological probes for differential identification of the tissue(s) or cell type(s). For a number of disorders of the above tissues or cells, particularly of the hepatic system, and fetal hematopoietic system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 401 as residues: His-7 to Trp-17, Leu-19 to Lys-27, Pro-33 to Gly-44, Lys-68 to Gly-74, Lys-85 to Cys-95.

The tissue distribution indicates that the protein products of this gene are useful for treatment/diagnosis of diseases of the developing liver and hematopoietic system, and act as a growth differentiation factor for hematopoietic stem cells.

### FEATURES OF PROTEIN ENCODED BY GENE NO: 169

The polypeptide encoded by this gene is believed to be a membrane bound receptor. The extracellular domain of which is expected to consist of the following amino acid sequence:
RILLVKYSANEENKYDYLPTTVNVCSELVKLVFCVLVSFCVIKKDHQSRNLKY ASWKEFSDFMKWSIPAFLYFLDNLIVFYVLSYLQPAMAVIFSNFSIITTALLFRIV LKXRLNWIQWASLLTLFLSIVALTAGTKTLQHNLAGRGFHHDAFFSPSNSCLL FRNECPRKDNCTAKEWTFPEAKWNTTARVFSHIRLGMGHVLIIVQCFISSMANI YNEKILKEGNQLTEXIFIQNSKLYFFGILFNGLTLGLQRSNRDQIKNCGFFYGH S (SEQ ID NO:732). Thus, preferred polypeptides encoded by this gene comprise the extracellular domain as shown above. It will be recognized, however, that deletions of either end of the extracellular domain up to the first cysteine from the N-terminus and the first cysteine of the C-terminus, is expected to retain the biological functions of the full-length extracellular domain because the cysteines are thought to be responsible for providing secondary structure to the molecule. Thus, deletions of one or more amino acids from either end (or both ends) of the extracellular domain are contemplated. Of course, further deletions including the cysteines are also contemplated as useful as such polypeptides is expected to have immunological properties such as the ability to evoke and immune response. Polynucleotides encoding all of the foregoing polypeptides are provided.

This gene is expressed primarily in human osteoclastoma and to a lesser extent in hippocampus and chondrosarcoma.

Therefore, polynucleotides and polypeptides of the invention are useful as reagents for differential identification of the tissue(s) or cell type(s) present in a biological sample and for diagnosis of diseases and conditions, which include, but are not limited to, cancers, particularly those of the bone and connective tissues. Similarly, polypeptides and antibodies directed to these polypeptides are useful in providing immunological probes for differential identification of the tissue(s) or cell type(s). For a number of disorders of the above tissues or cells, particularly of the skeletal system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 402 as residues: Met-1 to Cys-6, Ala-41 to Tyr-49, Lys-76 to Lys-84.

The tissue distribution indicates that the protein products of this gene are useful for diagnosis of cancers of the bone and connective tissues, and may act as growth factors for cells involved in bone or connective tissue growth.

### FEATURES OF PROTEIN ENCODED BY GENE NO: 170

Preferred polypeptides encoded by this gene comprising the following amino acid sequence:
NSVPNLQTLAVLTEAIGPEPAIPRXPREPPVATSTPATPSAGPQPLPTGTV LVPGGPAPPCLGEAWALLLPPCRPSLTSCFWSPRPSPWKETGV (SEQ ID NO:733). Polynucleotides encoding such polypeptides are also provided herein.

This gene is expressed primarily in hematopoietic progenitor cells.

Therefore, polynucleotides and polypeptides of the invention are useful as reagents for differential identification of the tissue(s) or cell type(s) present in a biological sample and for diagnosis of diseases and conditions, which include, but are not limited to, diseases of the blood including cancer and autoimmune disorders. Similarly, polypeptides and antibodies directed to these polypeptides are useful in providing immunological probes for differential identification of the tissue(s) or cell type(s). For a number of disorders of the above tissues or cells, particularly of the blood/circulatory system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 403 as residues: Gln-4 to His-10, Pro-25 to His-32.

The tissue distribution indicates that the protein products of this gene are useful for diagnosis of diseases involving growth differentiation of hematopoietic cells.

### FEATURES OF PROTEIN ENCODED BY GENE NO: 171

Preferred polypeptides encoded by this gene comprise the following amino acid sequences: ALQLAFYPDAVEEWLEENVHPSLQRLQXLLQDLSEVSAPP (SEQ ID NO:734); and/or CHPPALAGTLLRTPEGRAHARGLLLEAGGA (SEQ ID NO:735). Polynucleotides encoding such polypeptides are also provided. The protein product of this gene shares sequence homology with metallothionines. Thus, polypeptide encoded by this gene are expected to have metallothionine activity, such activities are known in the art and described elsewhere herein.

This gene is expressed primarily in kidney cortex.

Therefore, polynucleotides and polypeptides of the invention are useful as reagents for differential identification of the tissue(s) or cell type(s) present in a biological sample and for diagnosis of diseases and conditions, which include, but are not limited to, diseases of the kidney including cancer and renal dysfunction. Similarly, polypeptides and antibodies directed to these polypeptides are useful in providing immunological probes for differential identification of the tissue(s) or cell type(s). For a number of disorders of the above tissues or cells, particularly of the renal system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 404 as residues: Ser-47 to Gln-52.

The tissue distribution indicates that polynucleotides and polypeptides corresponding to this gene are useful for treatment/diagnosis of diseases of the kidney including kidney failure.

### FEATURES OF PROTEIN ENCODED BY GENE NO: 172

This gene is expressed primarily in 12 week old early stage human.

Therefore, polynucleotides and polypeptides of the invention are useful as reagents for differential identification of the tissue(s) or cell type(s) present in a biological sample and for diagnosis of diseases and conditions, which include, but are not limited to, developmental abnormalities. Similarly, polypeptides and antibodies directed to these polypeptides are useful in providing immunological probes for differential identification of the tissue(s) or cell type(s). For a number of disorders of the above tissues or cells, particularly of the developing embryo, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 405 as residues: Gln-31 to Thr-43, Gly-51 to Ser-58, Pro-65 to Pro-72.

The tissue distribution indicates that polynucleotides and polypeptides corresponding to this gene are useful for treatment/diagnosis of developmental problems with fetal tissue. The gene may be involved in vital organ development in the early stage, especially hematopoiesis, cardiovascular system, and neural development.

### FEATURES OF PROTEIN ENCODED BY GENE NO: 173

The translation product of this gene shares sequence homology with TGN38, an integral membrane protein previously shown to be predominantly localized to the trans-Golgi network (TGN) of cells.

This gene is expressed primarily in developing embryo and to a lesser extent in cancer tissues including lymphoma, endometrial, protate and colon.

Therefore, polynucleotides and polypeptides of the invention are useful as reagents for differential identification of the tissue(s) or cell type(s) present in a biological sample and for diagnosis of diseases and conditions, which include, but are not limited to, developmental abnormalities and cancer. Similarly, polypeptides and antibodies directed to these polypeptides are useful in providing immunological probes for differential identification of the tissue(s) or cell type(s). For a number of disorders of the above tissues or cells, particularly of the developing fetus, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 406 as residues: His-65 to Ser-72, Pro-82 to Gly-91, Pro-98 to Glu-118, Ser-126 to Gly-166, Pro-180 to Asp-188, Tyr-209 to Lys-214, Gln-220 to Leu-228.

The tissue distribution and homology to an integral membrane protein indicates that polynucleotides and polypeptides corresponding to this gene are useful for diagnosis of cancers and developmental abnormalities where aberrant expression relates to an abnormality.

### FEATURES OF PROTEIN ENCODED BY GENE NO: 174

The translation product of this gene shares sequence homology with a dnaJ heat shock protein from E. coli which is allelic to sec63, a gene that affects transit of nascent secretory proteins across the endoplasmic reticulum in yeast.

This gene is expressed primarily in Hodgkin's lymphoma and to a lesser extent in testes.

Therefore, polynucleotides and polypeptides of the invention are useful as reagents for differential identification of the tissue(s) or cell type(s) present in a biological sample and for diagnosis of diseases and conditions, which include, but are not limited to, cancer. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 407 as residues: Thr-13 to Trp-21, Arg-74 to Asp-81.

The tissue distribution and homology to dnaJ indicates that polynucleotides and polypeptides corresponding to this gene are useful as a diagnostic for cancer including Hodgkin's lymphoma.

### FEATURES OF PROTEIN ENCODED BY GENE NO: 175

This gene is expressed primarily in endothelial cells and to a lesser extent in bone marrow stromal cells.

Therefore, polynucleotides and polypeptides of the invention are useful as reagents for differential identification of the tissue(s) or cell type(s) present in a biological sample and for diagnosis of diseases and conditions, which include, but are not limited to, diseases involving angiogenic abnormalities including diabetic retinopathy, macular degeneration, and other diseases including arteriosclerosis and cancer. Similarly, polypeptides and antibodies directed to these polypeptides are useful in providing immunological probes for differential identification of the tissue(s) or cell type(s). For a number of disorders of the above tissues or cells, particularly of the vascular system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that the protein products of this gene are useful for treating diseases where an increase or decrease in angiogenesis is indicated and as a factor in the wound healing process.

### FEATURES OF PROTEIN ENCODED BY GENE NO: 176

The translation product of this gene shares sequence homology with MAT8 (mouse) which is thought to be important in regulating chloride conductance in cells (particularly in the breast) by modulating the response mediated by CAMP and protein kinase C to extracellular signals.

This gene is expressed primarily in amniotic cells and hematopoeitic cells including macrophages, Neutrophils, T cells, TNF induced aortic endothelium and to a lesser extent in testes, TNF induced epithelial cells, and smooth muscle.

Therefore, polynucleotides and polypeptides of the invention are useful as reagents for differential identification of the tissue(s) or cell type(s) present in a biological sample and for diagnosis of diseases and conditions, which include, but are not limited to, inflammatory responses mediated by T cells, macrophages, and/or neutrophils particularly those involving TNF, and also cancer. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 409 as residues: Thr-19 to Ala-33, Leu-54 to Asp-82, Pro-89 to Ala-97, Pro-100 to Lys-125, Ser-127 to Phe-135, Gly-164 to Leu-169, Cys-173 to Arg-178.

The tissue distribution and homology to mat-8 indicates that polynucleotides and polypeptides corresponding to this gene are useful for modifying inflammatory responses to cytokines such as TNF and thus modifying the duration and/or severity of inflammation. Polynucleotides and polypeptides derived from this gene are thought to be useful in the diagnosis and treatment of cancer.

### FEATURES OF PROTEIN ENCODED BY GENE NO: 177

This gene is expressed primarily in endothelial cells.

Therefore, polynucleotides and polypeptides of the invention are useful as reagents for differential identification of the tissue(s) or cell type(s) present in a biological sample and for diagnosis of diseases and conditions, which include, but are not limited to, vascular restenosis. Similarly, polypeptides and antibodies directed to these polypeptides are useful in providing immunological probes for differential identification of the tissue(s) or cell type(s). For a number of disorders of the above tissues or cells, particularly of the vascular system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treating diseases associated with vascular response to injury such as vascular restenosis following angioplasty.

### FEATURES OF PROTEIN ENCODED BY GENE NO: 178

One embodiment of the claimed invention comprises:
MRPDWKAGAGPGGPPQKPAPSSQRKPPARPSAAAAAIAVAAAEEERRLRQRN RLRLEEDKPAVERCLEELVFGDVENDEDALLRRLRGPRVQEHEDSGDSEVENEA KGNFPPQKKPVWVDEEDEDEEMVDMMNNRFRKDMMKNASESKLSKDNLKK RLKEEFQHAMGGVPAWAETTKRKTSSDDESEEDEDDLLQRTGNFISTSTSLPRG ILKMKNCQHANAERPTVARISICAVPSRCTDCDGCWD (SEQ ID NO:737); or CLEELVFGDVENDEDALLRRLRGPRVQEHEDSGDSEVENEAKGNFPPQKKPV WVDEEDEDEEMVDMMNNRFRKDMMKNASESKLSKDNLKKRLKEEFQHAMG GVPAWAETTKRKTSSDDESEEDEDDLLQRTGNFISTSTSLPRGILKMKNCQHA NAERPTVARISICAVPSRCTDCDGC (SEQ ID NO: 738). LKEKIVRSFEVSPDGS FLLINGIAGYLHLLAMKTKELIGSMKINGRVAASTFSSDSKKVYASSGDGEVYV WDVNSRKCLNRFVDEGSLYGLSIATSRNGQYVACGSNCGVVNIYNQDSCLQE TNPKPIKAIMNLVTGVTSLTFNPTTEILAIASEKMKEAVRLVHLPSCTVFSNFPVI KNKNISHVHTMDFSPRSGYFALGNEKGKALMYRLHHYSDF (SEQ ID NO-739); and/or KINGRVAASTFSSDSKKVYASSGDGEVYVWDVNSRKCLNRFVDEGSL YGLSIATSRNGQYVACGSNCGVVNIYNQDSCLQETNPKPIKAIMNLVTGVTSLT FNPTTEILAIASEKMKEAVRLVHLPSCTVFSNFPVIKNKNISHVHTMDFSPRSG YFALGNEKGKAL (SEQ ID NO:740).

This gene is expressed primarily in epidydimus and endometrial tumors and to a lesser extent in T cell lymphoma and cell lines derived from colon cancer.

Therefore, polynucleotides and polypeptides of the invention are useful as reagents for differential identification of the tissue(s) or cell type(s) present in a biological sample and for diagnosis of diseases and conditions, which include, but are not limited to, tumors of the reproductive organs including testis and endometrial cells. Similarly, polypeptides and antibodies directed to these polypeptides are useful in providing immunological probes for differential identification of the tissue(s) or cell type(s). For a number of disorders of the above tissues or cells, particularly of the reproductive system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 411 as residues: Ser-67 to Lys-72, Val-87 to Leu-93, Tyr-128 to Pro-141, Asp-204 to Gly-210.

The tissue distribution indicates that the protein products of this gene are useful for treating tumors of the endometrium or epithelial tumors of the reproductive system.

### FEATURES OF PROTEIN ENCODED BY GENE NO: 179

Preferred polypeptides encoded by this gene comprise the following amino acid sequence:
MRILQLILLALATGLVGGETRIIKGFECKLHSQPWQAALFEKTRLLCGATLIAPR WLLTAAHCLKPRYIVHLGQHNLQKEEGCEQTRTATESFPHPGFNNSLPNKDH RNDIMLVKMASPVSITWAVRPLTLSSRCVTAGTSCSFPAGAARPDPSYACLTPC DAPTSPSLSTRSVRTPTPATSQTPWCVPACRKGARTPARVTPGALWSVTSLFKA LSPGARIRVRSPESLVSTRKSANMWTGSRRR (SEQ ID NO:741); ETRIIKGFEC KLHSQPWQAALFEKTRLLCGATLIAPRWLLTAAHCLKPRYIVHLGQHNLQKEE GCEQTRTATESFPHPGFNNSLPNKDHRNDIMLVKMASPVSITWAVRPLTLSSR CVTAGTSCSFPAGAARPDPSYACLTPCDAPTSPSLSTRSVRTPTPATSQTPWCVP ACRKGARTPARVTPGALWSVTSLFKALSPGARIRVRSPESLVSTRKSANMWTG SRRR (SEQ ID NO:742); or CKLHSQPWQAALFEKTRLLCGATLIAPRWLLT AAHCLKPRYIVHLGQHNLQKEEGCEQTRTATESFPHPGFNS (SEQ ID NO:743). The translation product of this gene shares sequence homology with neuropsin a novel serine protease which is thought to be important in modulating extracellular signaling pathways in the brain. Owing to the structural similarity to other serine proteases the protein products of this gene are expected to have serine protease activity which may be assayed by methods known in the art and described elsewhere herein.

This gene is expressed primarily in endometrial tumor and to a lesser extent in colon cancer, benign hypertrophic prostate, and thymus.

Therefore, polynucleotides and polypeptides of the invention are useful as reagents for differential identification of the tissue(s) or cell type(s) present in a biological sample and for diagnosis of diseases and conditions, which include, but are not limited to, cancers of the endometrium or colon and benign hypertrophy of the prostate. Similarly, polypeptides and antibodies directed to these polypeptides are useful in providing immunological probes for differential identification of the tissue(s) or cell type(s). For a number of disorders of the above tissues or cells, particularly of the urogenital or reproductive systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 412 as residues: Gly-12 to Ser-22, Pro-34 to Ser-53.

The tissue distribution and homology to serine proteases indicates that polynucleotides and polypeptides corresponding to this gene are useful for diagnosing or treating hperproliferative disorders such as cancer of the endometrium or colon and hyperplasia of the prostate.

### FEATURES OF PROTEIN ENCODED BY GENE NO: 180

Preferred polypeptide encoded by this gene comprise the following amino acid sequence: VLQGRYFSPILEMRRLRPEGXXNLPGGSRAQKEPRQDLTLVLWPHC PHFAMTRSYVPTKQCMVQGSFYCIFIFKGPVQNWC (SEQ ID NO:744). Polynucleotides encoding such polypeptide are also provided.

This gene is expressed primarily in fetal brain

Therefore, polynucleotides and polypeptides of the invention are useful as reagents for differential identification of the tissue(s) or cell type(s) present in a biological sample and for diagnosis of diseases and conditions, which include, but are not limited to, identifying and expanding stem cells in the CNS. Similarly, polypeptides and antibodies directed to these polypeptides are useful in providing immunological probes for differential identification of the tissue(s) or cell type(s). For a number of disorders of the above tissues or cells, particularly of the nervous system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that the protein products of this gene are useful for detecting and expanding stem cell populations in the (or of the) central nervous system.

### FEATURES OF PROTEIN ENCODED BY GENE NO: 181

This gene is expressed primarily in early stage human brain and a stromal cell line.

Therefore, polynucleotides and polypeptides of the invention are useful as reagents for differential identification of the tissue(s) or cell type(s) present in a biological sample and for diagnosis of diseases and conditions, which include, but are not limited to, developmental abnormalities of the CNS. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 414 as residues: Gln-42 to Gln-47, Gln-54 to Pro-60.

The tissue distribution indicates that the protein products of this gene play a role in the development of the central nervous system. Therefore this gene and its products are useful for diagnosing or treating developmental abnormalities of the central nervous system.

### FEATURES OF PROTEIN ENCODED BY GENE NO: 182

Preferred polypeptides encoded by this gene comprise the following amino acid sequence:
MPIIDQVNPELHDFMQSAEVGTIFALSWLITWFGHVLSDFRHVVRLYDF FLACHPLMPIYFAAVIVLYREQEVLDCDCDMASVHHLLSQIPQDLPYETLISRXE TFLFSFPHPNLLGRPLPNSKLRGRQPLLSKTLSWHQPSRGLIWCCGSGXRGLL RPEDRTKDVLTKPRTNRFVKLAVMGLTVALGAAALAWKSALEWAPKFQLQL FP (SEQ ID NO:745); or CPEFFIPATLPCPFVFAFTSEASSRAYLTQRGPGGLAQ NLMPLPVGFWMGSLPPPWCWRKWVSEACSCFC (SEQ ID NO:746) These polypeptides are structurally similar to various TGF-beta family members. Thus, this polypeptide is expected to have a variety of activities in the modulation of cell growth and proliferation.

This gene is expressed primarily in osteoclastoma, microvascular endothelium, and bone marrow derived cell lines.

Therefore, polynucleotides and polypeptides of the invention are useful as reagents for differential identification of the tissue(s) or cell type(s) present in a biological sample and for diagnosis of diseases and conditions, which include, but are not limited to, hematological diseases particularly involving aberrant proliferation of stem cells. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 415 as residues: Ser-33 to Ala-39.

The tissue distribution indicates that the protein products of this gene is useful for treating disorders of the progenitors of the immune system. Applications include in vivo expansion of progenitor cells, ex vivo expansion of progenitor cells, or the treatment of tumors of the circulatory system, such as lymphomas.

### FEATURES OF PROTEIN ENCODED BY GENE NO: 183

This gene maps to chromosome 17 and therefore, polynucleotides of the invention can be used in linkage analysis as a marker for chromosome 17. In specific embodiments, polypeptides of the invention comprise the sequence:
GFGSVSAAGRRSGGTWQPVQ (SEQ ID NO:747); PGGLAVGSRWWSRSLT (SEQ ID NO:748); LEPSRQRRPRRRGGTSRPETDQRAKCWRQL (SEQ ID NO:749); and/or VCLRCQNRMEN (SEQ ID NO:750). In further specific embodiments, polypeptides of the invention comprise the sequence: MAACTARRPGR GQPLVVPVADXGPVAKAALCAAXAGAFSPASTTTTRRHLSSRNRPEGKVLETV GVFEVPKQNGKYETGQLFLHSIFGYRGVVLFPWQARLXDRDVASAAPEKAEN PAGHGSKEVKGKTHTYYQVLIDARDCPHISQRSQTEAVTFLANHDDSRALYAIP GLDYVSHEDILPYTSTDQVPIQHELFERFLLYDQTKAPPFVARETLRAWQEKNH PWLELSDVHRETTENIRVTVIPFYMGMREAQNSHVYWWRYCIRLENLDSDVVQ LRERHWRIFSLSGTLETVRGRGVVGREPVLSKEQPAFQYSSHVSLQASSGHMW GTFRFERPDGSHFDVRIPPFSLESNKDEKTPPSGLHW (SEQ ID NO:751); MAACTARRPGRGQPLVVPVADXGPVAKAALCAA (SEQ ID NO:752); VLETVGVFEVPKQNGKYETGQLFLHSIFGYRGVVL (SEQ ID NO:757); GLDYVSHEDILPYTST (SEQ ID NO:758); DVHRETTENIRVTVIPFYM (SEQ ID NO:759); WWRYCIRLENLDSDVVQLRER (SEQ ID NO:760); and/or PAFQYSS HVSLQASSGHMWGTFRFER (SEQ ID NO:761). Polynucleotides encoding these polypeptides are also encompassed by the invention.

This gene is expressed primarily in gall bladder, prostate, and fetal brain, and to a lesser extent in a few tumor and fetal tissues.

Therefore, polynucleotides and polypeptides of the invention are useful as reagents for differential identification of the tissue(s) or cell type(s) present in a biological sample and for diagnosis of diseases and conditions, which include, but are not limited to, growth related disorders such as cancers. Similarly, polypeptides and antibodies directed to these polypeptides are useful in providing immunological probes for differential identification of the tissue(s) or cell type(s). For a number of disorders of the above tissues or cells, particularly of the prostate, gall bladder, and fetal brain, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of growth-related disorders, such cancers.

### FEATURES OF PROTEIN ENCODED BY GENE NO: 184

In specific embodiments, polypeptides of the invention comprise the sequence:SLCCPEGAEGC (SEQ ID NO:762) and/or QLKKTHYDRPCP (SEQ ID NO:763). Polynucleotides encoding these polypeptides are also encompassed by the invention.

This gene is expressed primarily in stromal cell, tonsil, and glioblastoma and to a lesser extent in some other tissues.

Therefore, polynucleotides and polypeptides of the invention are useful as reagents for differential identification of the tissue(s) or cell type(s) present in a biological sample and for diagnosis of diseases and conditions, which include, but are not limited to, immune and inflammatory disorders and glioblastoma. Similarly, polypeptides and antibodies directed to these polypeptides are useful in providing immunological probes for differential identification of the tissue(s) or cell type(s). For a number of disorders of the above tissues or cells, particularly of the stromal cells, tonsil, and glioblastoma expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Additionally, it is believed that the product of this gene regulates pancreatic cell differentiation into beta cells. Accordingly, polynucleotides and polypeptides of the invention are useful in the treatment of insulin-dependent diabetes mellitus and associated conditions e.g. pancreatic hypofunction and the prevention, as well as the treatment of undifferentiated type pancreatic cancers. Preferred epitopes include those comprising a sequence shown in SEQ ID NO: 417 as residues: Pro-27 to Ala-32.

The tissue distribution indicates that polynucleotides and polypeptides corresponding to this gene are useful for diagnosis and treatment of immune and inflammatory disorders and glioblastoma.

### FEATURES OF PROTEIN ENCODED BY GENE NO: 185

This gene is expressed primarily in hepatocellular carcinoma and to a lesser extent in other tissues.

Therefore, polynucleotides and polypeptides of the invention are useful as reagents for differential identification of the tissue(s) or cell type(s) present in a biological sample and for diagnosis of diseases and conditions, which include, but are not limited to, liver diseases. Similarly, polypeptides and antibodies directed to these polypeptides are useful in providing immunological probes for differential identification of the tissue(s) or cell type(s). For a number of disorders of the above tissues or cells, particularly of the liver, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 418 as residues: Gly-32 to Lys-39.

The tissue distribution indicates that polynucleotides and polypeptides corresponding to this gene are useful for diagnosis and treatment of liver diseases.

### FEATURES OF PROTEIN ENCODED BY GENE NO: 186

This gene is expressed primarily in hippocampus and to a lesser extent in other tissues.

Therefore, polynucleotides and polypeptides of the invention are useful as reagents for differential identification of the tissue(s) or cell type(s) present in a biological sample and for diagnosis of diseases and conditions, which include, but are not limited to, neutronal disorders. Similarly, polypeptides and antibodies directed to these polypeptides are useful in providing immunological probes for differential identification of the tissue(s) or cell type(s). For a number of disorders of the above tissues or cells, particularly of the hippocampus, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of neuronal disorders.

### FEATURES OF PROTEIN ENCODED BY GENE NO: 187

This gene is expressed primarily in bone cancer and hippocampus and to a lesser extent in osteoclastoma and other tissues.

Therefore, polynucleotides and polypeptides of the invention are useful as reagents for differential identification of the tissue(s) or cell type(s) present in a biological sample and for diagnosis of diseases and conditions, which include, but are not limited to, bone-related disorders and neuronal diseases. Similarly, polypeptides and antibodies directed to these polypeptides are useful in providing immunological probes for differential identification of the tissue(s) or cell type(s). For a number of disorders of the above tissues or cells, particularly of the bone, ostoeclast, and hippocampus, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of bone-related disorders and neuronal diseases.

### FEATURES OF PROTEIN ENCODED BY GENE NO: 188

This gene maps to chromosome 4 and therefore polynucleotides of the invention can be used in linkage analysis as a marker for chromosome 4.

This gene is expressed primarily in neuronal tissues such as hippocampus, spinal cord, and hypothalamus and to a lesser extent in a few other tissues such as ovary.

Therefore, polynucleotides and polypeptides of the invention are useful as reagents for differential identification of the tissue(s) or cell type(s) present in a biological sample and for diagnosis of diseases and conditions, which include, but are not limited to, neuronal diseases. Similarly, polypeptides and antibodies directed to these polypeptides are useful in providing immunological probes for differential identification of the tissue(s) or cell type(s). For a number of disorders of the above tissues or cells, particularly of the neuronal tissues, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of neuronal disorders.

### FEATURES OF PROTEIN ENCODED BY GENE NO: 189

This gene maps to chromosome 10, therefore, polynucleotides of the invention can be used in linkage analysis as a marker for chromosome 10.

This gene is expressed primarily in neuronal tissues and immune tissues, and to a lesser extent in a few other tissues such as skin tumor, lung etc.

Therefore, polynucleotides and polypeptides of the invention are useful as reagents for differential identification of the tissue(s) or cell type(s) present in a biological sample and for diagnosis of diseases and conditions, which include, but are not limited to, neuronal and immune-related disorders. Similarly, polypeptides and antibodies directed to these polypeptides are useful in providing immunological probes for differential identification of the tissue(s) or cell type(s). For a number of disorders of the above tissues or cells, particularly of the neuronal and immune-related tissues, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 422 as residues: Pro-19 to Asp-25.

The tissue distribution indicates that polynucleotides and polypeptides corresponding to this gene are useful for diagnosis and treatment of neuronal and immune-related disorders.

### FEATURES OF PROTEIN ENCODED BY GENE NO: 190

The translation product of this gene shares sequence homology with human N33, a gene located in a homozygously deleted region of human metastatic prostate cancer which is thought to be important in prevention of prostate cancer. In specific embodiments, polypeptides of the invention comprise the sequence:
AQRKKEMVLSEKVSQLMEWTNKRPVIRMNGDKFRRLVKAPPRNYSVIVMFTA LQLHRQCVVCKQADEEFQILANSWRYSSAFTNRIFFAMVDFDEGSDVFQMLNM NSAPTFINFPAKGKPKRGDTYELQVRGFSAEQIARWIADRTDVNIRVIRPPNMA ARWRFWCVSVT (SEQ ID NO:765); MVVALLIVCDVPSAS (SEQ ID NO:766); AQRKKEMVLSEKVSQL (SEQ ID NO:767); MEWTNKRPVIRMNGDKF (SEQ ID:768); RRLVKAPPRNYSVIVMFTALQLHRQCVVCKQADEEFQILANSWRY SSAFTNRIFFA (SEQ ID NO:769); MVDFDEGSDVFQMLNMNSAPTFINFPAK GKP (SEQ ID NO:770); KRGDTYELQVRGFSAEQIARWIADRTDVNIRVIRPPN (SEQ ID NO:771); and/or YAGPLMLGLLLAVIGGLVYLRRVIWNFSLIKLDGLLQL CVLCLL (SEQ ID NO:772). Polynucleotides encoding these polypeptides are also encompassed by the invention.

This gene is expressed primarily in infant adrenal gland prostate cell line and to a lesser extent in a few other tissues like liver, smooth muscle etc.

Therefore, polynucleotides and polypeptides of the invention are useful as reagents for differential identification of the tissue(s) or cell type(s) present in a biological sample and for diagnosis of diseases and conditions, which include, but are not limited to, prostate cancer and endocrine disorders. Similarly, polypeptides and antibodies directed to these polypeptides are useful in providing immunological probes for differential identification of the tissue(s) or cell type(s). For a number of disorders of the above tissues or cells, particularly of the prostate and adrenal gland, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 423 as residues: Pro-34 to Gly-43, Arg-113 to Pro-120.

The tissue distribution and homology to N33 indicates that polynucleotides and polypeptides corresponding to this gene are useful for diagnosis and treatment for prostate cancer and endocrine disorders.

### FEATURES OF PROTEIN ENCODED BY GENE NO: 191

This gene is expressed primarily in T cell and to a lesser extent in fetal lung.

Therefore, polynucleotides and polypeptides of the invention are useful as reagents for differential identification of the tissue(s) or cell type(s) present in a biological sample and for diagnosis of diseases and conditions, which include, but are not limited to, immune disorders. Similarly, polypeptides and antibodies directed to these polypeptides are useful in providing immunological probes for differential identification of the tissue(s) or cell type(s). For a number of disorders of the above tissues or cells, particularly of the immune, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 424 as residues: Trp-3 to Phe-9.

The tissue distribution indicates that polynucleotides and polypeptides corresponding to this gene are useful for diagnosis and treatment of immune disorders.

### FEATURES OF PROTEIN ENCODED BY GENE NO: 192

This gene maps to chromosome 6, therefore, polynucleotides of the invention can be used in linkage analysis as a marker for chromosome 6. Neural activity and neurotrophins induce synaptic remodeling in part by altering gene expression. This gene is believed to be a glycosylphoshatidylinositol-anchored protein encoded by a hippocampal gene and to possess neural activity. This molecule is believed to be expressed in postmitotic-differentiating neurons of the developing nervous system and neuronal structures associated with plasticity in the adult. Message of this gene is believed to be induced by neuronal activity and by the activity-regulated neurotrophins BDNF and NT-3. The product of this gene is believed to stimulate neurite outgrowth and arborization in primary embryonic hippocampal and cortical cultures and to act as a downstream effector of activity-induced neurite outgrowth. In specific embodiments, polypeptides of the invention comprise the sequence: DAVFKGFSDCLLKLGDS (SEQ ID NO:773); CQEGAKDMWDKLRKESKNLN (SEQ ID NO:774); VLLVSLSAALATWLSF (SEQ ID NO:775); MGLKLNGRYISLILAVQIAYLVQAVR AAGKCDAVFKGFSDCLLKLGDS (SEQ ID NO:776); PAAWDDKTNIKTVCTYW EDFHSCTVTALTDCQEGAKDMWDKLRKESKNLNIQGSLFELCGSGNGAAGSL LPAFPVLLVSLSAALATWLSF (SEQ ID NO:777); and/or MGLKLNGRYISLILA VQIAYLVQAVRAAGKCDAVFKGFSDCLLKLGDSXXXXXPAAWDDKTNIKTVC TYWEDFHSCTVTALTDCQEGAKDMWDKLRKESKNLNIQGSLFELCGSGNGAA GSLLPAFPVLLVSLSAALATWLSF (SEQ ID NO:778). Polynucleotides encoding this polypeptide are also encompassed by the invention.

This gene is expressed primarily in human placenta, endometrial tumor and tissues of the central nervous system (CNS).

Therefore, polynucleotides and polypeptides of the invention are useful as reagents for differential identification of the tissue(s) or cell type(s) present in a biological sample and for diagnosis of diseases and conditions, which include, but are not limited to, relating to reproductive disorders, cancers and neurological diseases. Similarly, polypeptides and antibodies directed to these polypeptides are useful in providing immunological probes for differential identification of the tissue(s) or cell type(s). For a number of disorders of the above tissues or cells, particularly of the reproductive and neurological disorders, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 425 as residues: Asp-47 to Asp-63, His-75 to Tyr-80, Pro-83 to Tyr-89.

The tissue distribution indicates that polynucleotides and polypeptides corresponding to this gene are useful for diagnosis and treatment of reproductive disorders such as endometrial tumors. Expression of this gene in tissues of the CNS and its strong homology to Neuritin suggest that the protein product from this gene may also be used in the treatment and diagnosis of neurological disorders and in the regeneration of neural tissues, e.g., following injury.

### FEATURES OF PROTEIN ENCODED BY GENE NO: 193

The translation product of this gene shares sequence homology with tenascin which is thought to be important in development. The translation product of this gene is believed to be a ligand of the fibroblast growth factor family. FGF ligand activity is known in the art and can be assayed by methods known in the art and disclosed elsewhere herein.

This gene is expressed primarily in endometrial tumors, and other types of tumors.

Therefore, polynucleotides and polypeptides of the invention are useful as reagents for differential identification of the tissue(s) or cell type(s) present in a biological sample and for diagnosis of diseases and conditions, which include, but are not limited to, cancers. Similarly, polypeptides and antibodies directed to these polypeptides are useful in providing immunological probes for differential identification of the tissue(s) or cell type(s). For a number of disorders of the above tissues or cells, particularly of the cancer tissues, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 426 as residues: Gly-29 to Glu-34, Arg-71 to Arg-76, Thr-176 to Cys-182, Gly-184 to Glu-199.

The tissue distribution and homology to tenascin indicates that polynucleotides and polypeptides corresponding to this gene are useful for diagnosis and treatment of cancers.

### FEATURES OF PROTEIN ENCODED BY GENE NO: 194

In specific embodiments, polypeptides of the invention comprise the sequence: MNSAAGFSHLDRRERVLKLGESFEKQPRCASTLC (SEQ ID NO:779). Polynucleotides encoding these polypeptides are also encompassed by the invention.

This gene is expressed primarily in fetal human lung and neutrophils.

Therefore, polynucleotides and polypeptides of the invention are useful as reagents for differential identification of the tissue(s) or cell type(s) present in a biological sample and for diagnosis of diseases and conditions, which include, but are not limited to, lung development and respiratory disorders. Similarly, polypeptides and antibodies directed to these polypeptides are useful in providing immunological probes for differential identification of the tissue(s) or cell type(s). For a number of disorders of the above tissues or cells, particularly of the respiratory system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution in fetal lung and neutrophils indicates that polynucleotides and polypeptides corresponding to this gene are useful for diagnosis and treatment of lung and immunity related diseases, for example, lung cancer, viral, fungal or bacterial infections (e.g. lesions caused by tuberculosis), inflammation (e.g. pneumonia), metabolic lesions etc.

### FEATURES OF PROTEIN ENCODED BY GENE NO: 195

This gene is expressed primarily in breast lymph node.

Therefore, polynucleotides and polypeptides of the invention are useful as reagents for differential identification of the tissue(s) or cell type(s) present in a biological sample and for diagnosis of diseases and conditions, which include, but are not limited to, immunal disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis and treatment of immunal disorders.

### FEATURES OF PROTEIN ENCODED BY GENE NO: 196

This gene maps to chromosome 5 and accordingly, polynucleotides of the invention can be used in linkage analysis as a marker for chromosome 5. The translation product of this gene shares sequence homology with human M-phase phosphoprotein 4 which is thought to be important in phosphorylation and signal transduction processes. In specific embodiments, polypeptides of the invention comprise the sequence:
TIYPTEEELQAVQKIVSITERALKLVSD (SEQ ID NO:780); RALKGVLRV GVLAKGLLLRGDRNVNLVLLC (SEQ ID NO:781); ALAALRHAKWFQARAN GLQSCVIIIRILRDLCQRVPTWS (SEQ ID NO:782); GDALRRVFECISSGIIL (SEQ ID NO:783); LAFRQIHKVLGMDPLP (SEQ ID NO:784); and/or TIYPTEEELQAVQ KIVSITERALKLVSDSLSEHEKNKNKEGDDKKEGGKDRALKGVLRVGVLAKG LLLRGDRNVNLVLLCSEKPSKTLLSRIAENLPKQLAVISPEKYDIKCAVSEAAIIL NSCVEPKMQVTITLTSPIIREENMREGDVTSGMVKDPPDVLDRQKCLDALAALR HAKWFQARANGLQSCVIIIRILRDLCQRVPTWSDFPSWAMELLVEKAISSASSP QSPGDALRRVFECISSGIILKGSPGLLDPCEKDPFDTLATMTDQQREDITSSAQFA LRLLAFRQIHKVLGMDPLPQMSQRFNIHNNRKRRRDSDGVDGFEAEGKKDKK DYDNF (SEQ ID NO:785). Polynucleotides encoding these polypeptides are also encompassed by the invention.

This gene is expressed primarily in Human Hippocampus and to a lesser extent in Prostate, Human Frontal Cortex.

Therefore, polynucleotides and polypeptides of the invention are useful as reagents for differential identification of the tissue(s) or cell type(s) present in a biological sample and for diagnosis of diseases and conditions, which include, but are not limited to, disorders related to reproductive system and nervous system. Similarly, polypeptides and antibodies directed to these polypeptides are useful in providing immunological probes for differential identification of the tissue(s) or cell type(s). For a number of disorders of the above tissues or cells, particularly of the reproductive system and nervous system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to human M-phase phosphoprotein 4 indicates that polynucleotides and polypeptides corresponding to this gene are useful for the diagnosis and treatment of reproductive and nervous system disorders.

### FEATURES OF PROTEIN ENCODED BY GENE NO: 197

In specific embodiments, polypeptides of the invention comprise the sequence:
MGSQHSAAARPSSCRRKQEDDRDG (SEQ ID NO:786);
LLAEREQEEAIAQFPYVEFTGRDSITCLTC (SEQ ID NO:787); and/or
QGTGYIPTEQVNELVALIPHSDQRLRPQRTKQYV (SEQ ID NO:788).
Polynucleotides encoding these polypeptides are also encompassed by the invention.

This gene is expressed primarily in Human Primary Breast Cancer and to a lesser extent in Human Adult Spleen, Hodgkin's Lymphoma I, Salivary Gland.

Therefore, polynucleotides and polypeptides of the invention are useful as reagents for differential identification of the tissue(s) or cell type(s) present in a biological sample and for diagnosis of diseases and conditions, which include, but are not limited to, cancer and immunal disorders. Similarly, polypeptides and antibodies directed to these polypeptides are useful in providing immunological probes for differential identification of the tissue(s) or cell type(s). For a number of disorders of the above tissues or cells, particularly of the cancer and immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 430 as residues: Ser-126 to Gly-138.

The tissue distribution indicates that polynucleotides and polypeptides corresponding to this gene are useful for the diagnosis and treatment of cancer and immunal disorders.

### FEATURES OF PROTEIN ENCODED BY GENE NO: 198

This gene is expressed primarily in monocytes.

Therefore, polynucleotides and polypeptides of the invention are useful as reagents for differential identification of the tissue(s) or cell type(s) present in a biological sample and for diagnosis of diseases and conditions, which include, but are not limited to, blood cell disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis and treatment of blood cell disorders.

### FEATURES OF PROTEIN ENCODED BY GENE NO: 199

This gene is expressed primarily in Human Ovary and Synovia and to a lesser extent in Human 8 Week Whole Embryo.

Therefore, polynucleotides and polypeptides of the invention are useful as reagents for differential identification of the tissue(s) or cell type(s) present in a biological sample and for diagnosis of diseases and conditions, which include, but are not limited to, reproductive and developmental disorders. Similarly, polypeptides and antibodies directed to these polypeptides are useful in providing immunological probes for differential identification of the tissue(s) or cell type(s). For a number of disorders of the above tissues or cells, particularly of the reproductive and developmental system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis and treatment of reproductive and developmental disorders.

### FEATURES OF PROTEIN ENCODED BY GENE NO: 200

This gene maps to chromosome 8 and therefore polynucleotides of the invention can be used in linkage analysis as a marker for chromosome 8. The translation product of this gene shares limited sequence homology with collagen proline rich domain.

This gene is expressed primarily in CNS.

Therefore, polynucleotides and polypeptides of the invention are useful as reagents for differential identification of the tissue(s) or cell type(s) present in a biological sample and for diagnosis of diseases and conditions, which include, but are not limited to, neurological diseases. Similarly, polypeptides and antibodies directed to these polypeptides are useful in providing immunological probes for differential identification of the tissue(s) or cell type(s). For a number of disorders of the above tissues or cells, particularly of the nervous system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 433 as residues: Pro-35 to Asp-41.

The tissue distribution indicates that polynucleotides and polypeptides corresponding to this gene are useful for diagnosis and treatment of neurological diseases.

### FEATURES OF PROTEIN ENCODED BY GENE NO: 201

Translation product of this gene shares homology with a mammalian histone H 1a protein. One embodiment for this gene is the polypeptide fragments comprising the following amino acid sequence: ARLNVGRESLKREMLKSQGVKVSESPMGAR HSSWPEGAAFCKKVQGAQMQFPPRR (SEQ ID NO:789); ARLNVGRESLKR EML (SEQ ID NO:790); LKSQGVKVSESPMGARHSSW (SEQ ID NO:791); AFCKKVQGAQMQFPPRR (SEQ ID NO:792). An additional embodiment is the polynucleotide fragments encoding these polypeptide (See Accession No. pir|S24178) fragments.

This gene is expressed primarily in neutrophils.

Therefore, polynucleotides and polypeptides of the invention are useful as reagents for differential identification of the tissue(s) or cell type(s) present in a biological sample and for diagnosis of diseases and conditions, which include, but are not limited to, immune disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of immune disorders. Since the gene is expressed in cells of lymphoid origin, the natural gene product may be involved in vital immune functions. Therefore it may be also used as an agent for immunological disorders including arthritis, asthma, immune deficiency diseases such as AIDS, and leukemia.

### FEATURES OF PROTEIN ENCODED BY GENE NO: 202

This gene is expressed primarily in neutrophils.

Therefore, polynucleotides and polypeptides of the invention are useful as reagents for differential identification of the tissue(s) or cell type(s) present in a biological sample and for diagnosis of diseases and conditions, which include, but are not limited to, immune disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of immune disorders. Since the gene is expressed in cells of lymphoid origin, the natural gene product may be involved in immune functions. Therefore it may be also used as an agent for immunological disorders including arthritis, asthma, immune deficiency diseases such as AIDS, and leukemia.

### FEATURES OF PROTEIN ENCODED BY GENE NO: 203

This gene is expressed primarily in Neutrophils.

Therefore, polynucleotides and polypeptides of the invention are useful as reagents for differential identification of the tissue(s) or cell type(s) present in a biological sample and for diagnosis of diseases and conditions, which include, but are not limited to, infectious disorders, immune disorders, and cancers. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 436 as residues: Thr-31 to Lys-36.

The tissue distribution indicates that polynucleotides and polypeptides corresponding to this gene are useful for diagnosis and treatment of infectious disorders, immune disorders, and cancers. Since the gene is expressed in cells of lymphoid origin, the natural gene product may be involved in immune functions. Therefore it may be also used as an agent for immunological disorders including arthritis, asthma, immune deficiency diseases such as AIDS, and leukemia. Protein, as well as, antibodies directed against the protein may show utility as a tumor marker and/or immunotherapy targets for the above listed tumors and tissues.

### FEATURES OF PROTEIN ENCODED BY GENE NO: 204

This gene maps to chromosome 16 and therefore polynucleotides of the invention can be used in linkage analysis as markers for chromosome 16. The translation product of this gene shares sequence homology with lactate dehydrogenase which is thought to be important in lactate metabolism.

This gene is expressed primarily in human tonsils and to a lesser extent in Spleen, and Neutrophils.

Therefore, polynucleotides and polypeptides of the invention are useful as reagents for differential identification of the tissue(s) or cell type(s) present in a biological sample and for diagnosis of diseases and conditions, which include, but are not limited to, immune disorders, infectious disorders, and cancers. Similarly, polypeptides and antibodies directed to these polypeptides are useful in providing immunological probes for differential identification of the tissue(s) or cell type(s). For a number of disorders of the above tissues or cells, particularly of the immune disorders, infectious disorders, and cancers, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 437 as residues: Gly-7 to Ser-12.

The tissue distribution and homology to lactate dehydrogenase gene indicates that polynucleotides and polypeptides corresponding to this gene are useful for diagnosis and treatment of immune disorders, infectious disorders, and cancers.

### FEATURES OF PROTEIN ENCODED BY GENE NO: 205

The translation product of this gene shares sequence homology with Gcap1 protein which is developmentally regulated in brain.

This gene is expressed primarily in placenta and endometrial tumor and to a lesser extent in several other tumors.

Therefore, polynucleotides and polypeptides of the invention are useful as reagents for differential identification of the tissue(s) or cell type(s) present in a biological sample and for diagnosis of diseases and conditions, which include, but are not limited to, vasculogenesis/angiogenesis and tumorigenesis. Similarly, polypeptides and antibodies directed to these polypeptides are useful in providing immunological probes for differential identification of the tissue(s) or cell type(s). For a number of disorders of the above tissues or cells, particularly of the vascular system and tumors, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution and homology to Gcap1 protein indicates that polynucleotides and polypeptides corresponding to this gene are useful for diagnosis and treatment of disorder or dysfunction of vascular system of tumorigenesis.

### FEATURES OF PROTEIN ENCODED BY GENE NO: 206

In specific embodiments, polypeptides of the invention comprise the sequence
MPYAQWLAENDRFEEAQKAFHKAGRQREA (SEQ ID NO:799); VQVLEQLTNNAVAESRFNDAAYYYWMLSMQCLDIAQD (SEQ ID NO:794); PAQKDTMLGKFYHFQRLAELYHGYHAIHRHTEDP (SEQ ID NO: 795); FSVHRPETLFNISRFLLHSLPKDTPSGISKVKILFT (SEQ ID NO:800); LAKQSKALGAYRLARHAYDKLRGLYIP (SEQ ID NO:796); ARFQKSIELG TLTIRAKPFHDSEELVPLCYRCSTNN (SEQ ID NO: 797); and/or PLLNNLGNVC INCRQPFIFSASSYDVLHLVEFYLEEGITDEEAISLIDLEVLRPKRDDRQLEICKQQ LPDSCG (SEQ ID NO:798). Polynucleotides encoding these polypeptides are also encompassed by the invention.

This gene is expressed primarily in testes.

Therefore, polynucleotides and polypeptides of the invention are useful as reagents for differential identification of the tissue(s) or cell type(s) present in a biological sample and for diagnosis of diseases and conditions, which include, but are not limited to, male reproductive and endocrine disorders. Similarly, polypeptides and antibodies directed to these polypeptides are useful in providing immunological probes for differential identification of the tissue(s) or cell type(s). For a number of disorders of the above tissues or cells, particularly of the reproductive and endocrine systems, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for treatment of male reproductive and endocrine disorders.

### FEATURES OF PROTEIN ENCODED BY GENE NO: 207

This gene is expressed in fetal lung.

Therefore, polynucleotides and polypeptides of the invention are useful as reagents for differential identification of the tissue(s) or cell type(s) present in a biological sample and for diagnosis of diseases and conditions, which include, but are not limited to, lung diseases such as cystic fibrosis. Similarly, polypeptides and antibodies directed to these polypeptides are useful in providing immunological probes for differential identification of the tissue(s) or cell type(s). For a number of disorders of the above tissues or cells, particularly of the respiratory system, expression of this gene at significantly higher or lower levels may be routinely detected in certain tissues (e.g., cancerous and wounded tissues) or bodily fluids (e.g., serum, plasma, urine, synovial fluid or spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 440 as residues: Tyr-49 to Cys-54.

The tissue distribution indicates that polynucleotides and polypeptides corresponding to this gene are useful for detection and treatment of disorders associated with developing lungs particularly in premature infants where the lungs are the last tissues to develop. The tissue distribution indicates that polynucleotides and polypeptides corresponding to this gene are useful for diagnosis and intervention of lung tumors since the gene may be involved in the regulation of cell division, particularly since it is expressed in fetal tissue. Protein, as well as, antibodies directed against the protein may show utility as a tumor marker and immunotherapy targets for the above listed tumors and tissues.

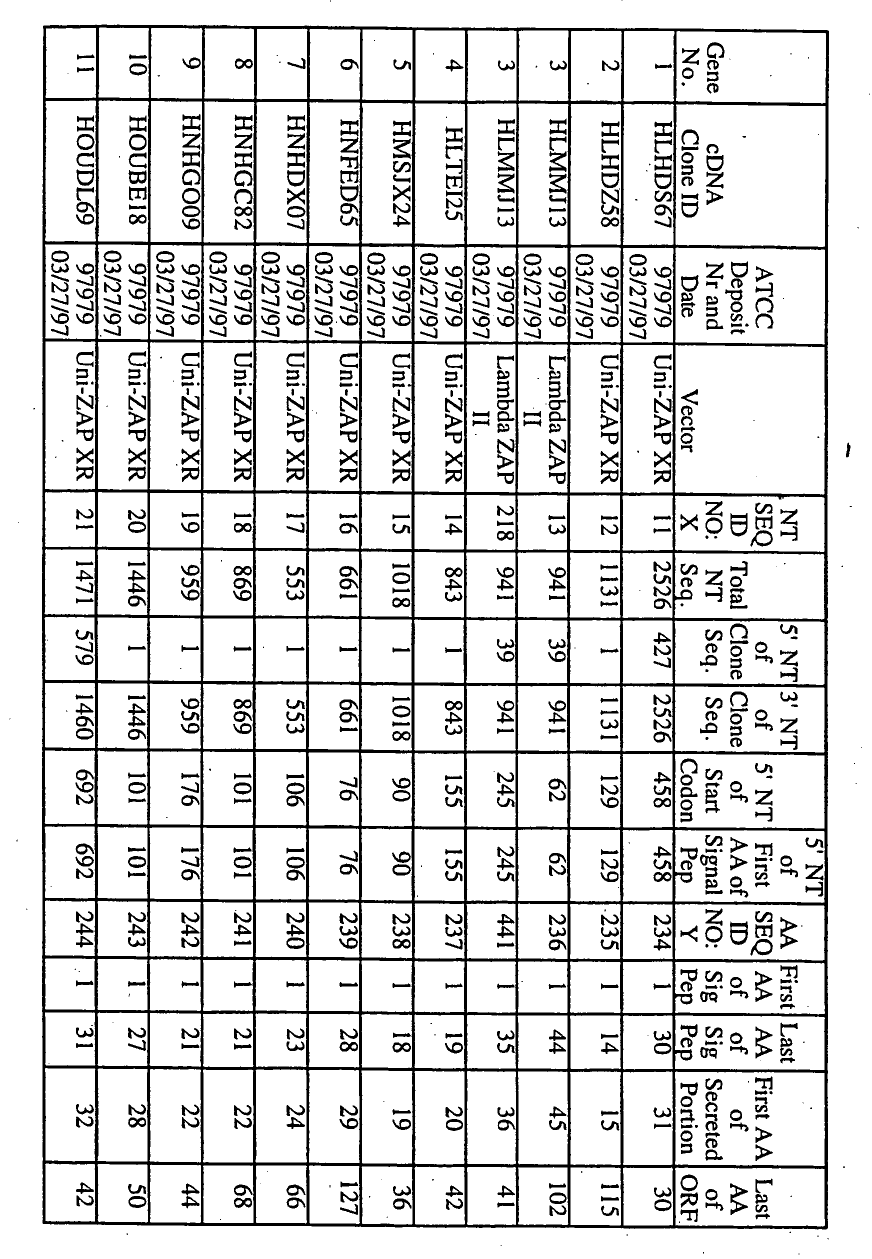

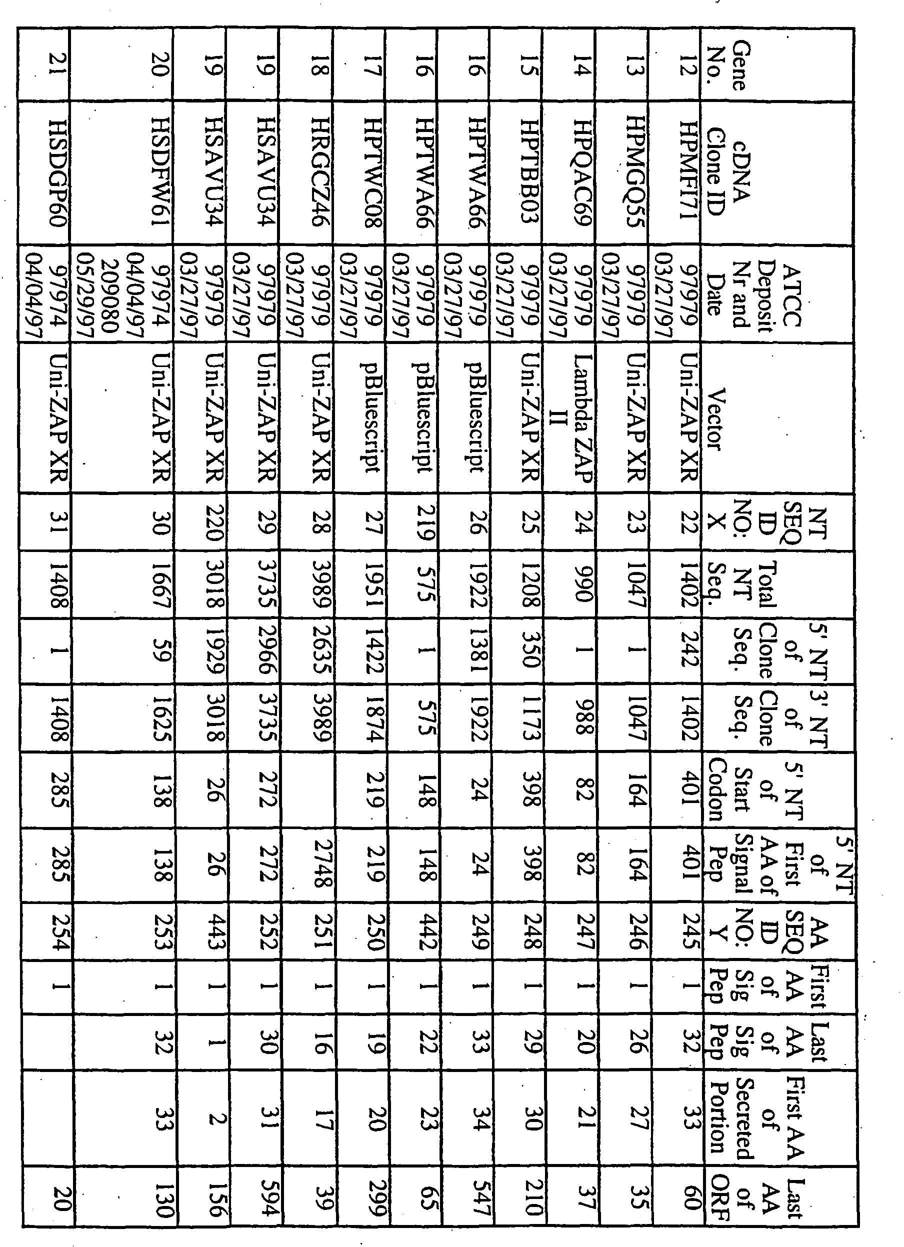

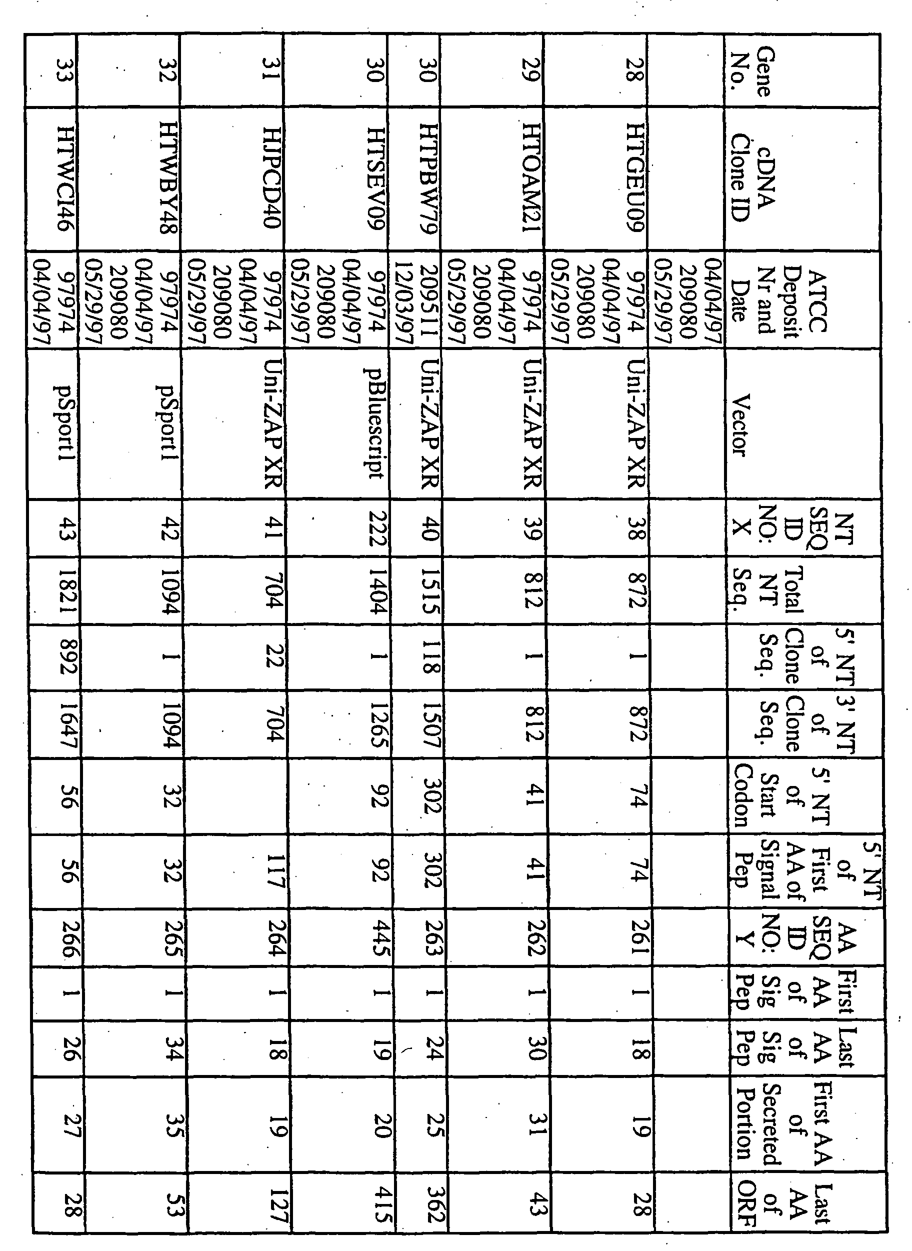

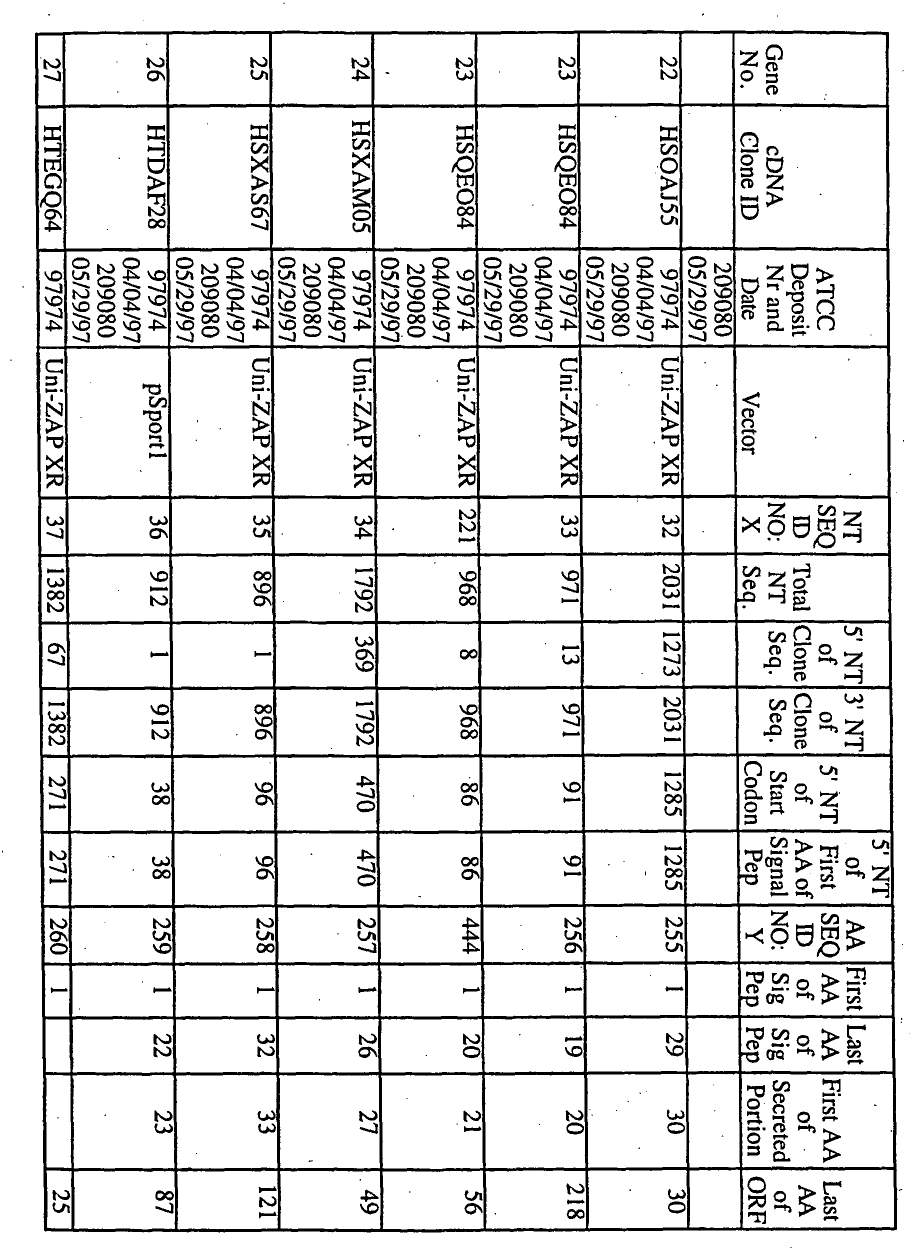

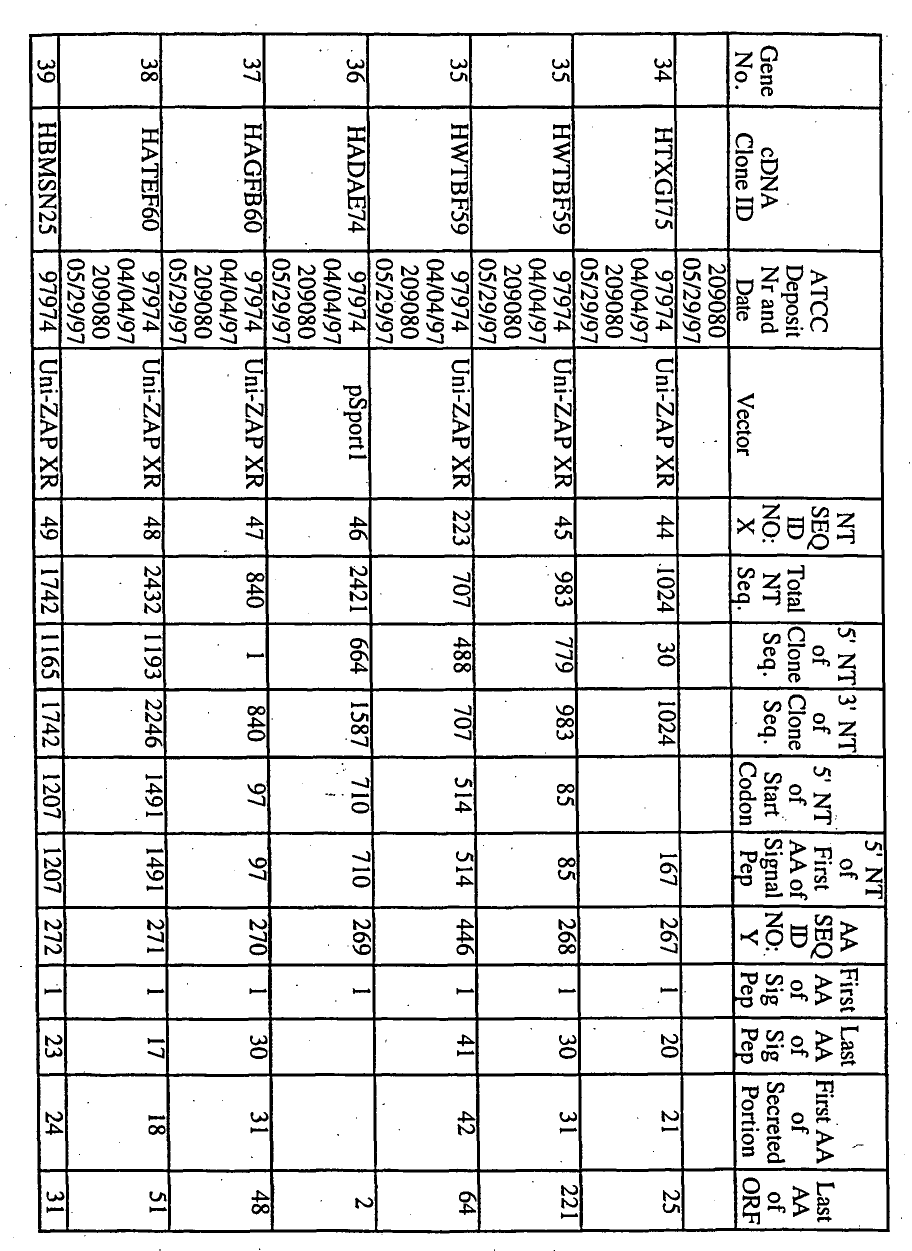

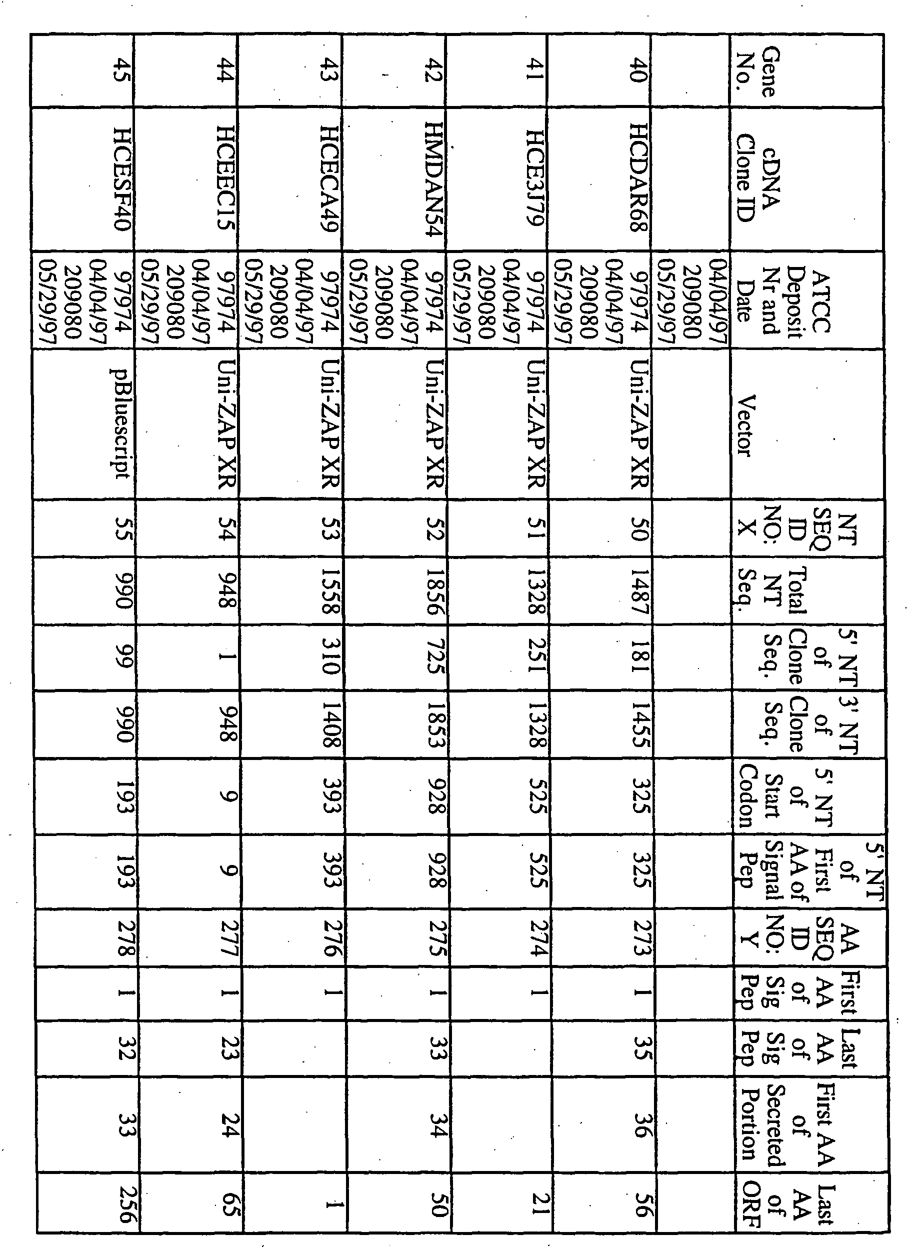

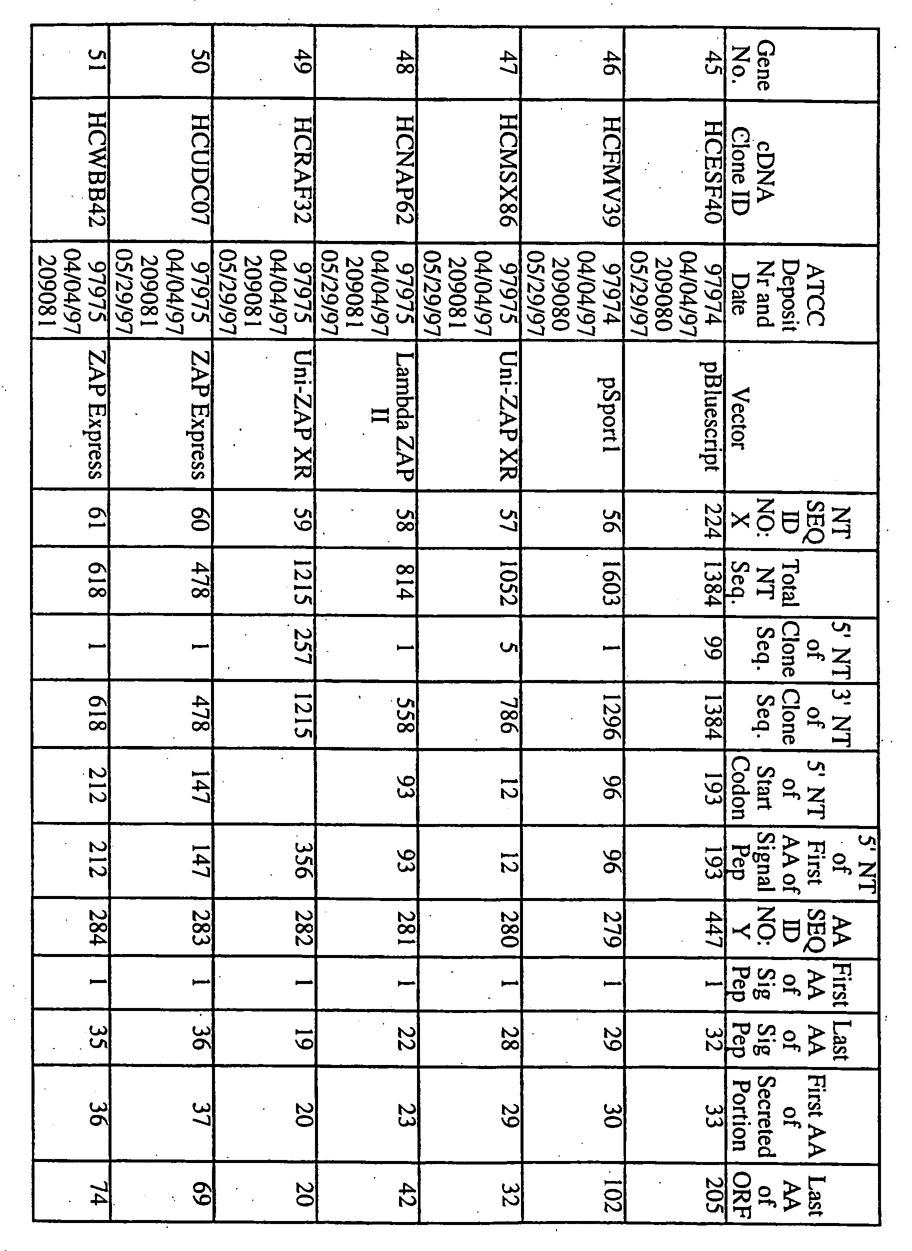

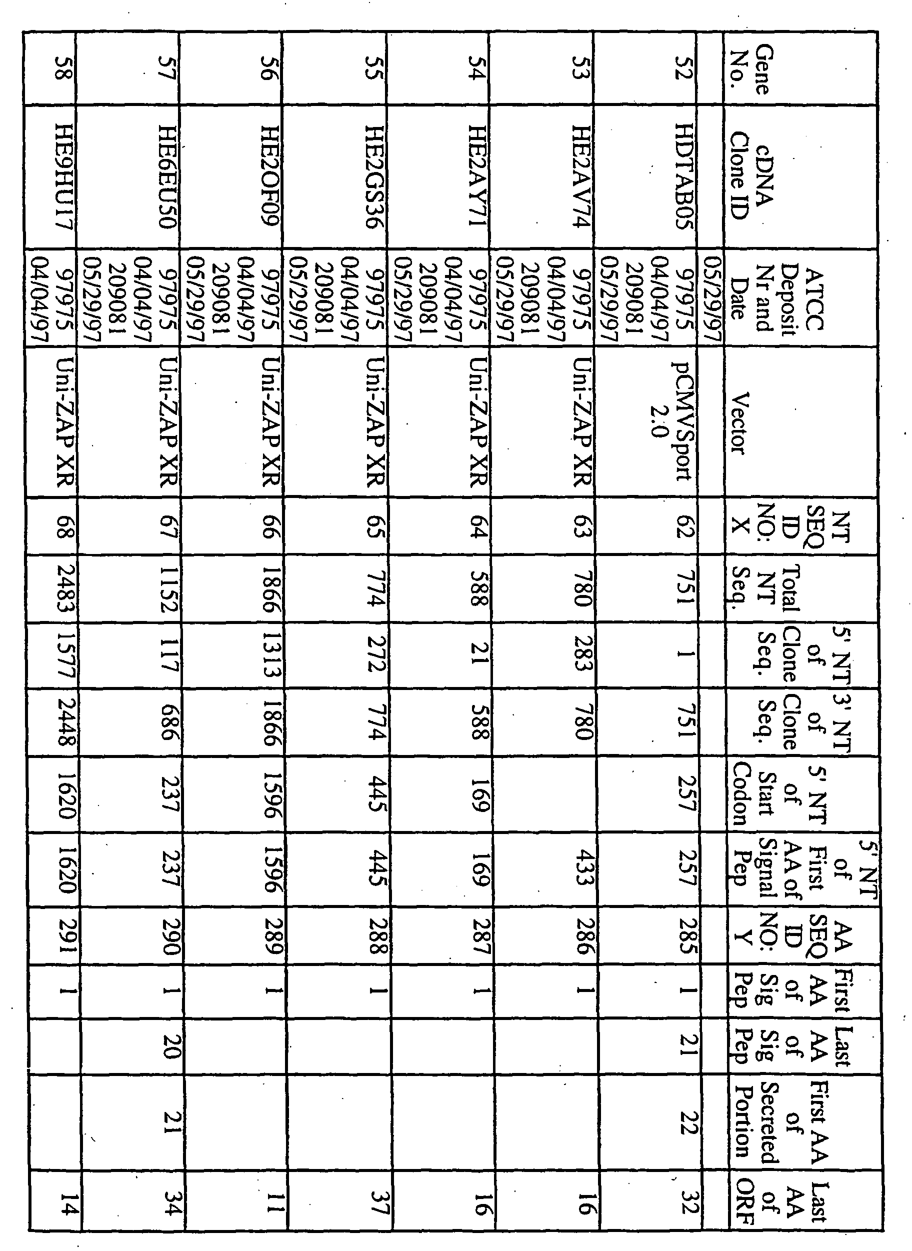

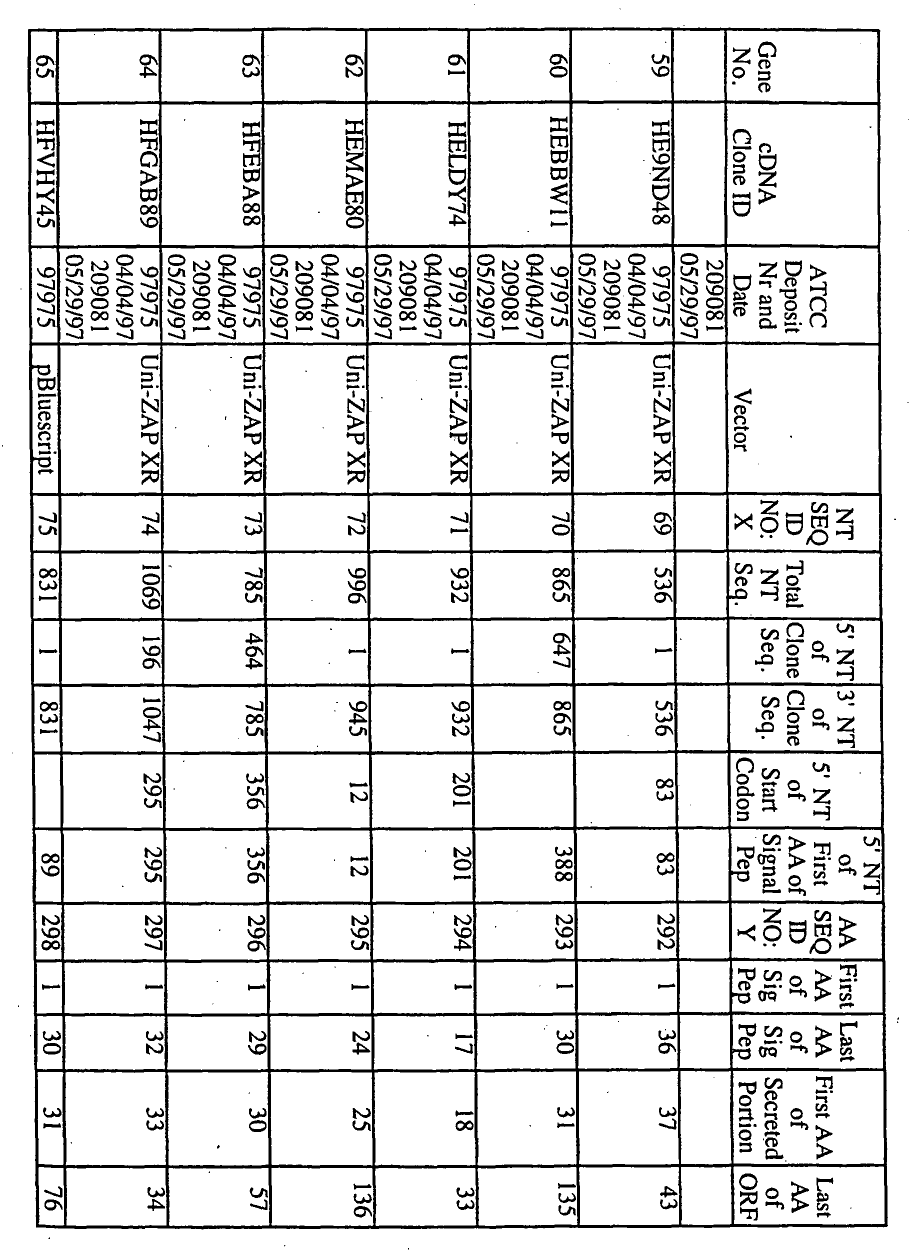

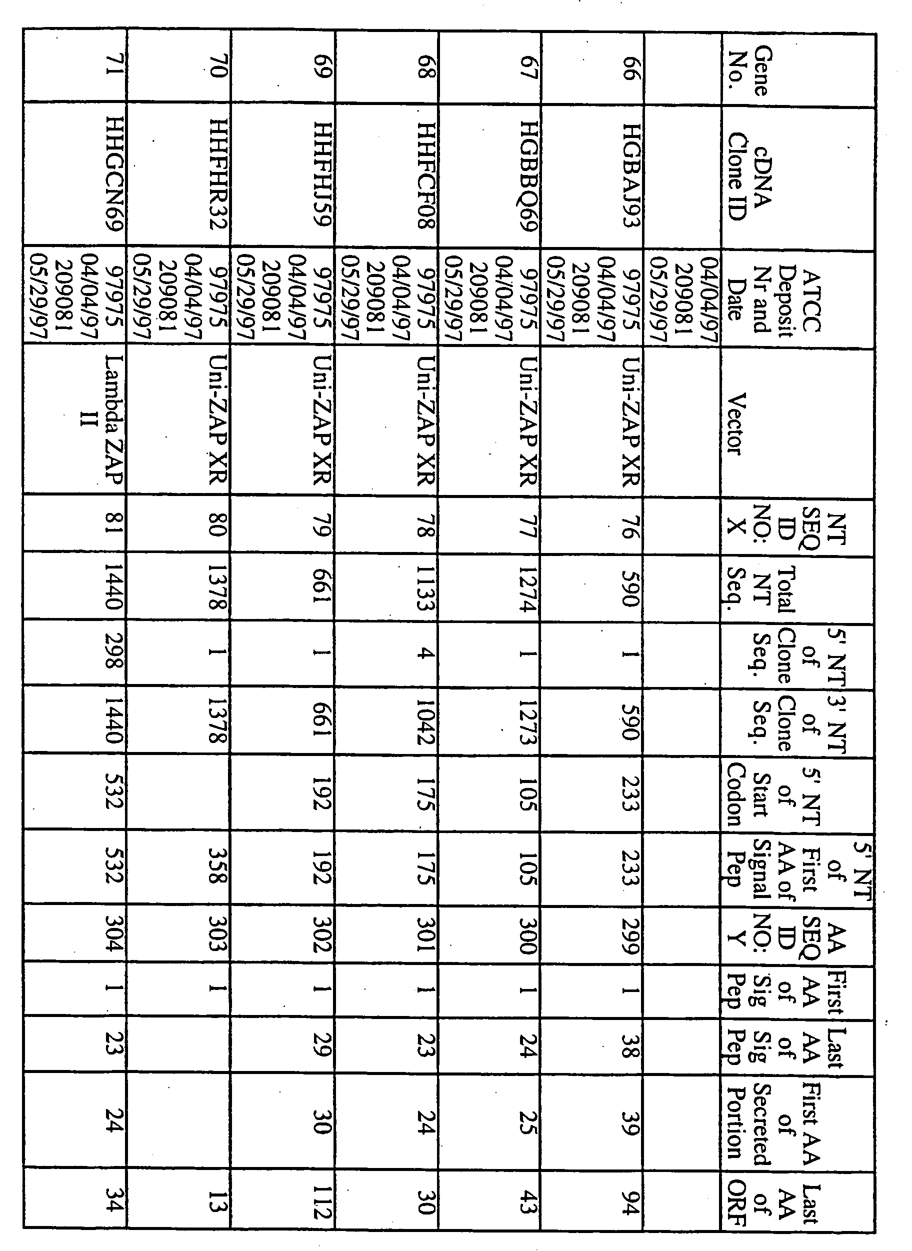

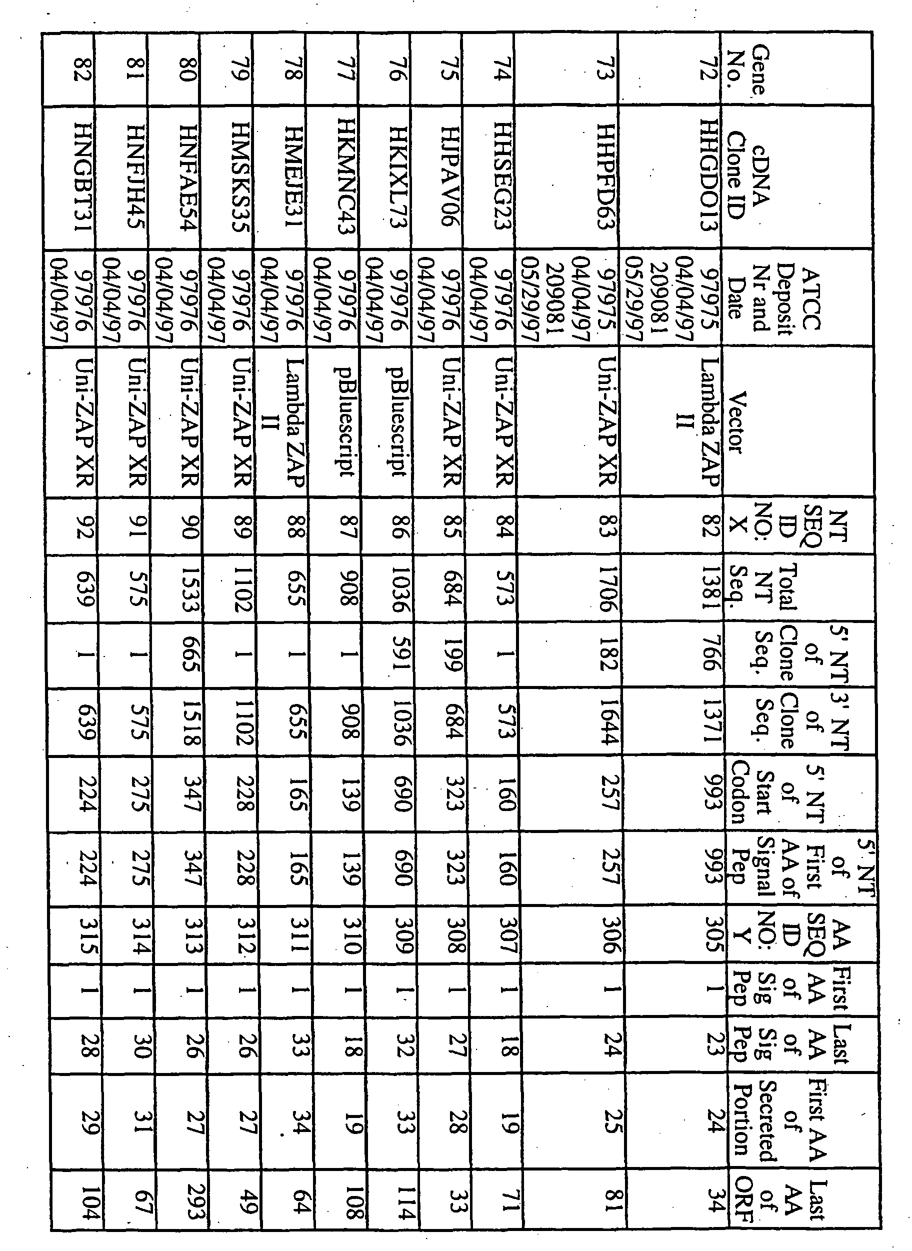

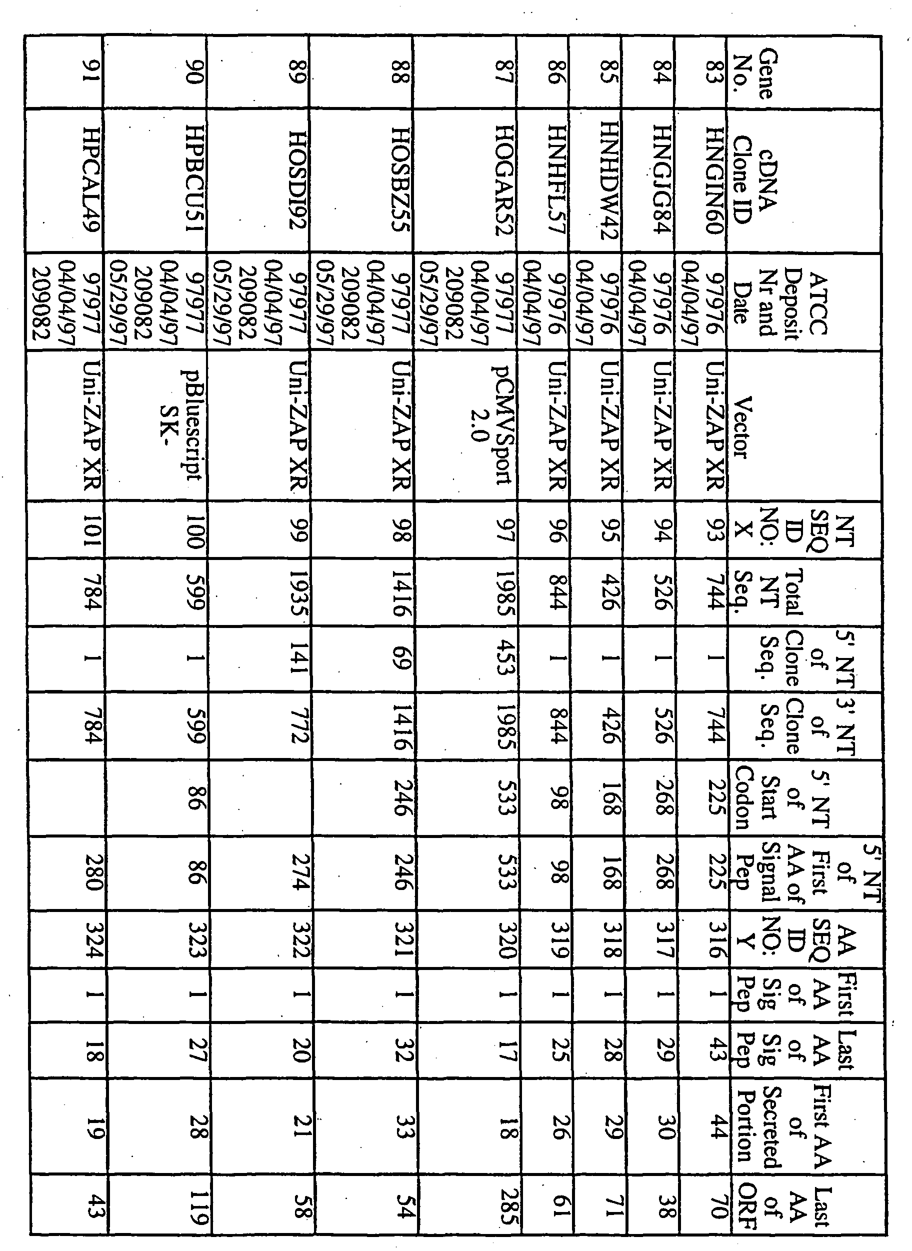

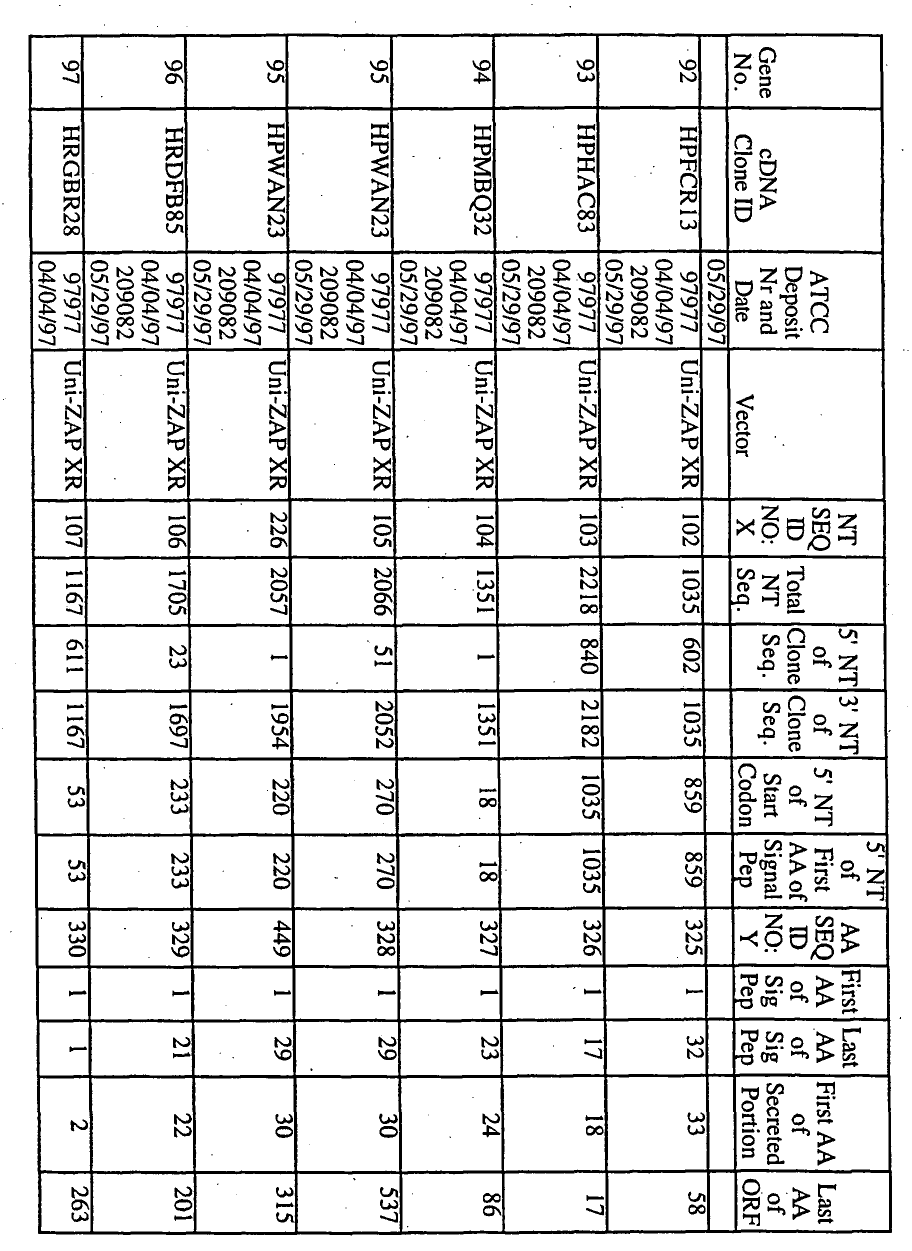

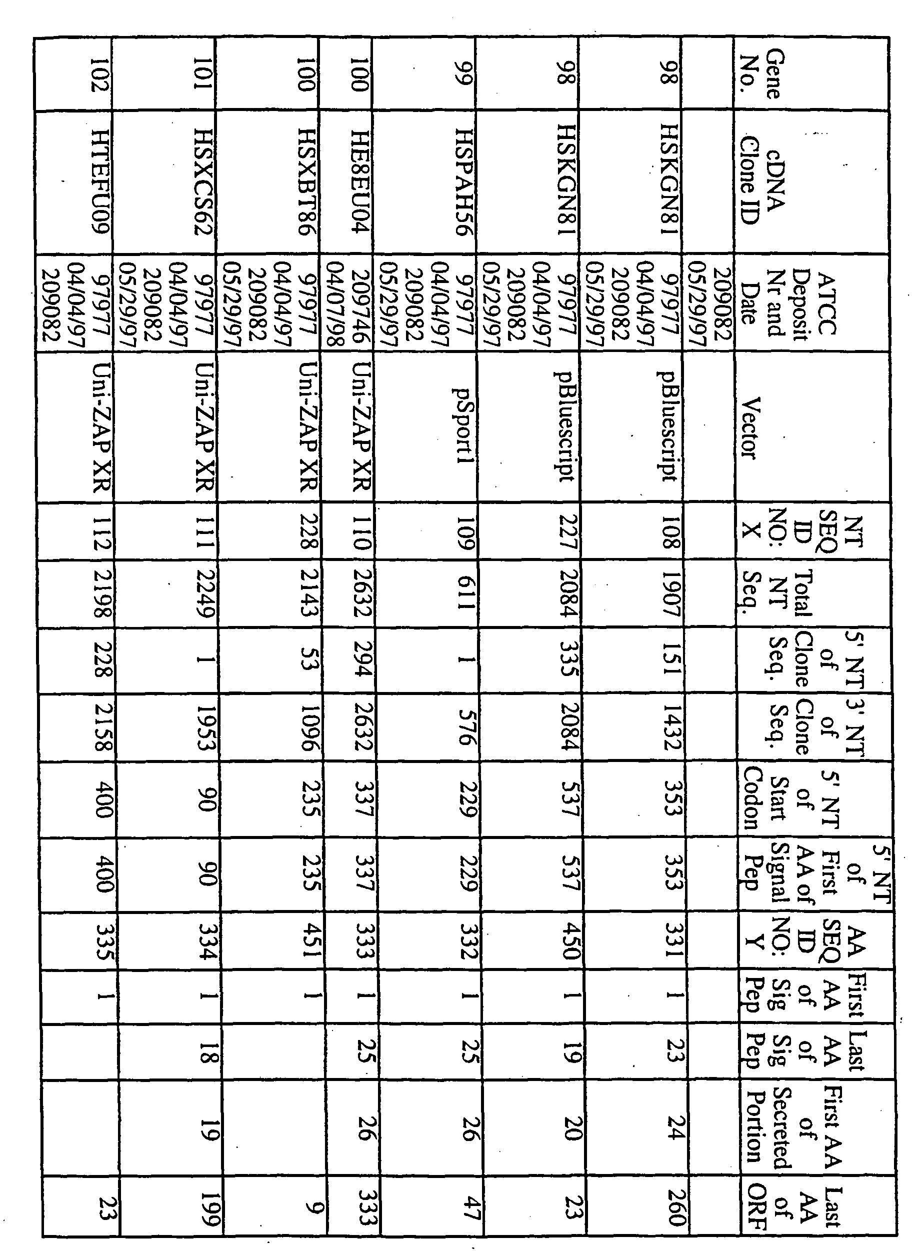

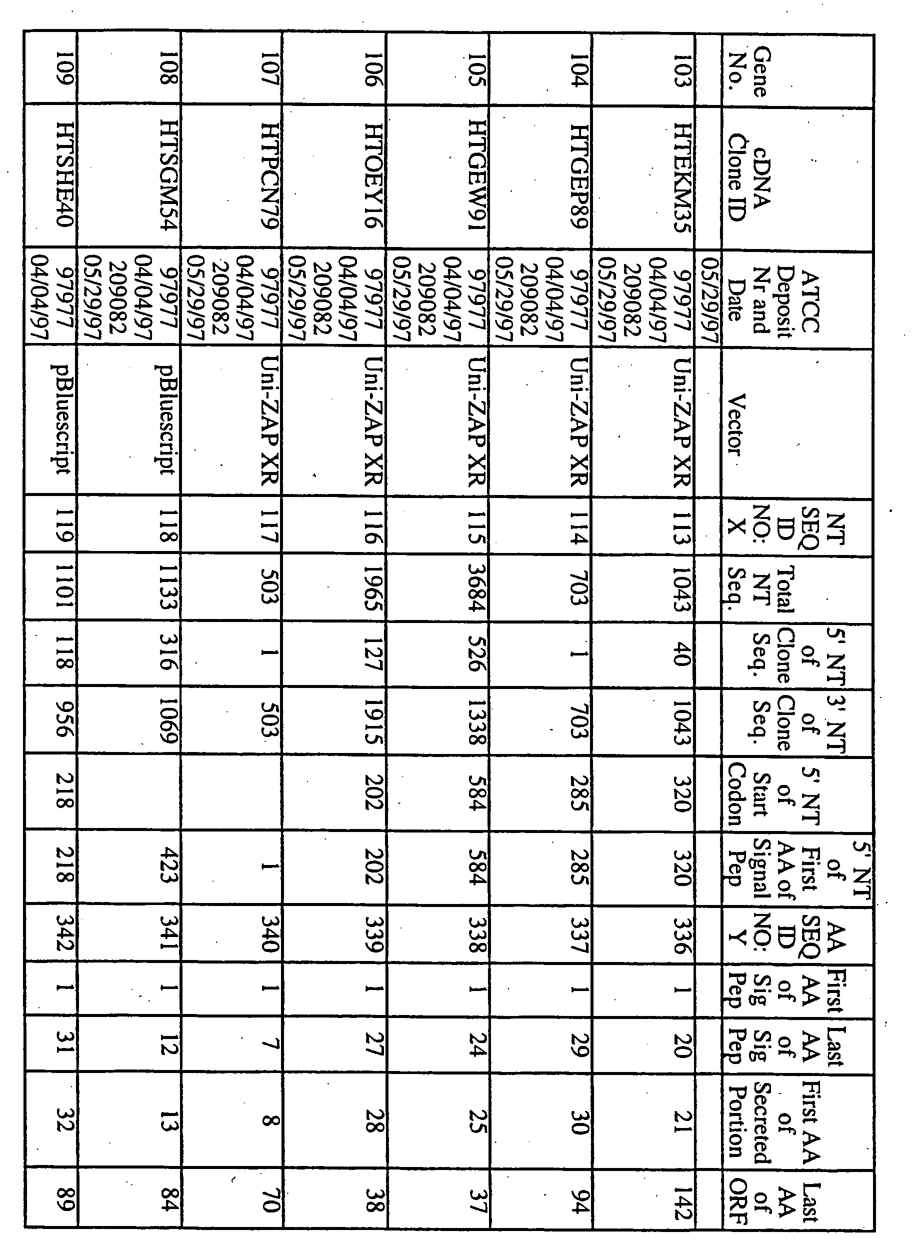

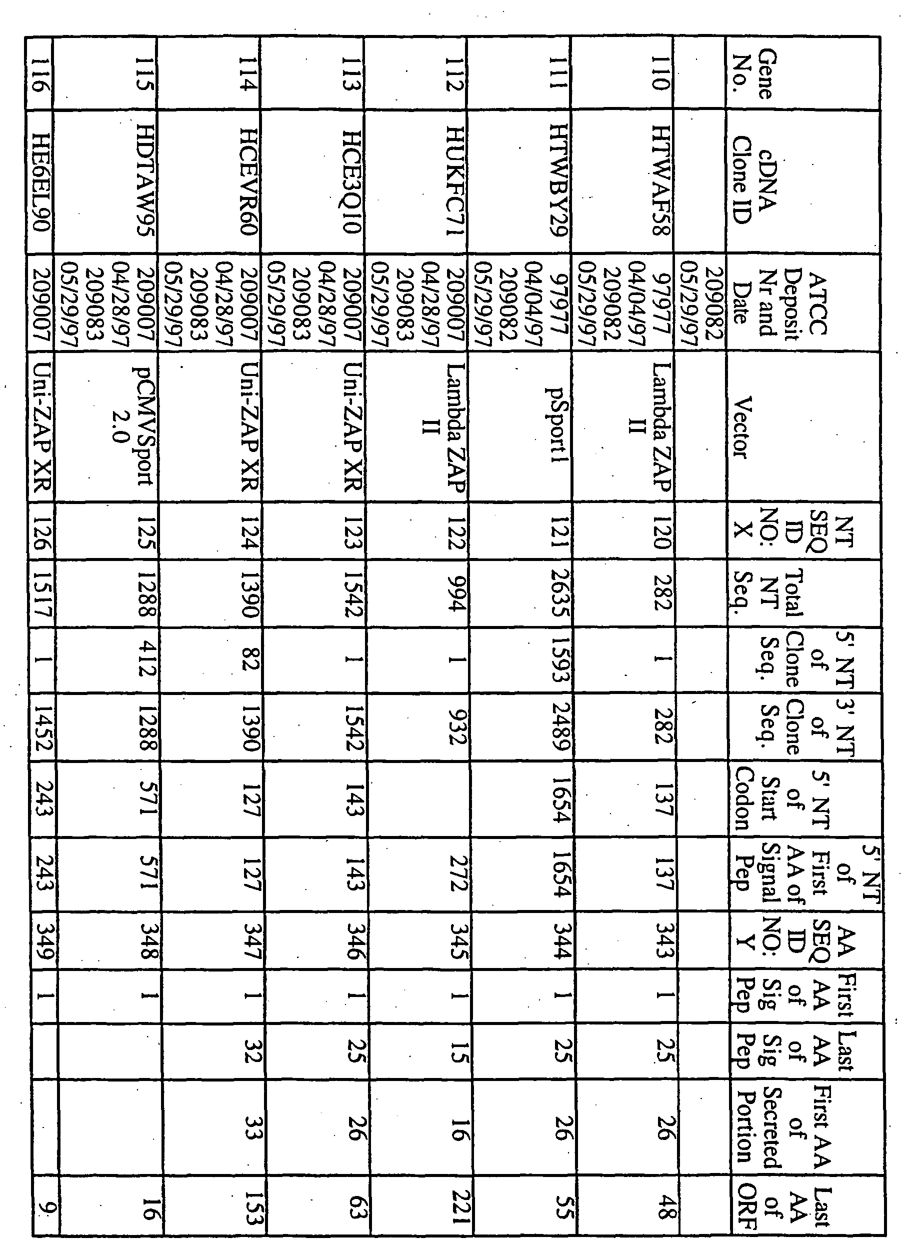

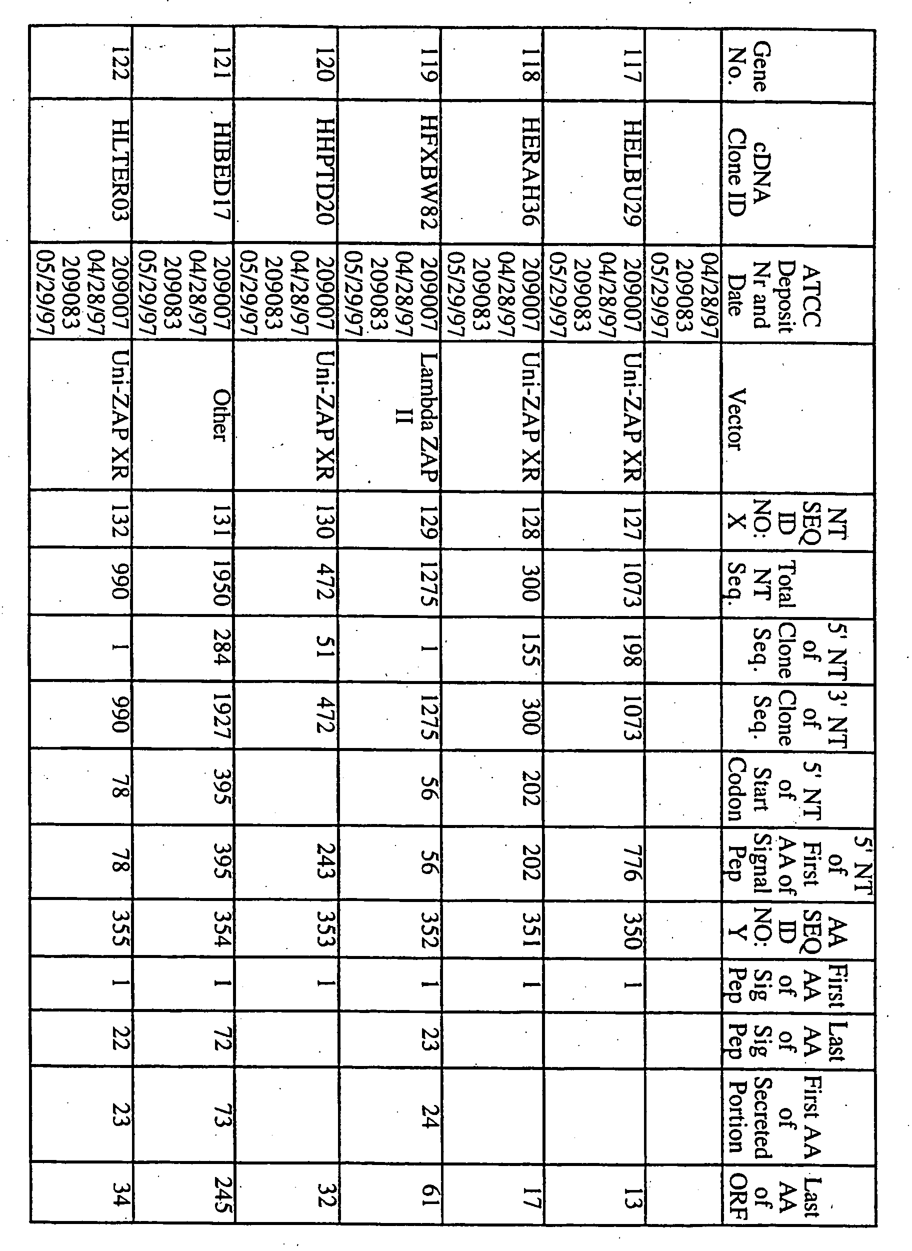

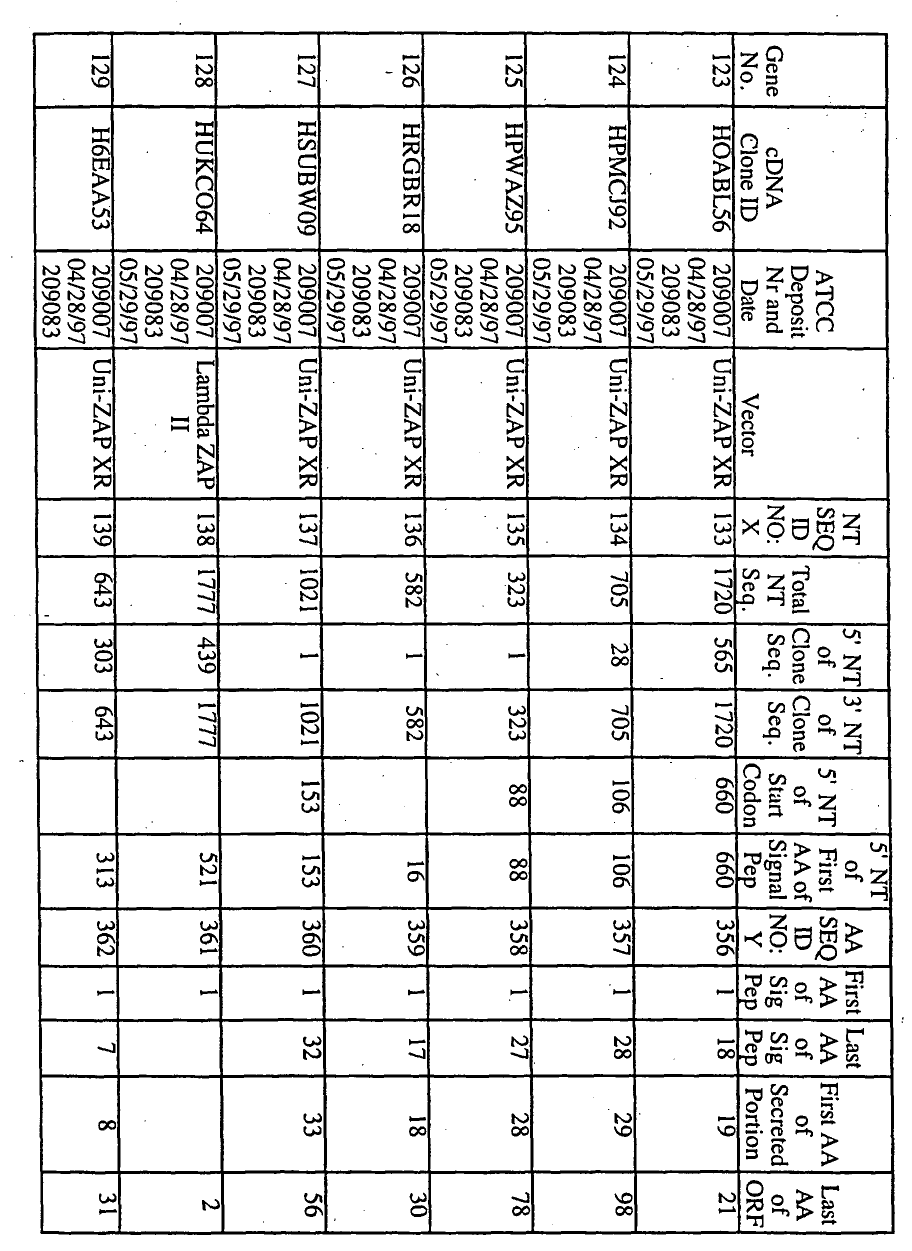

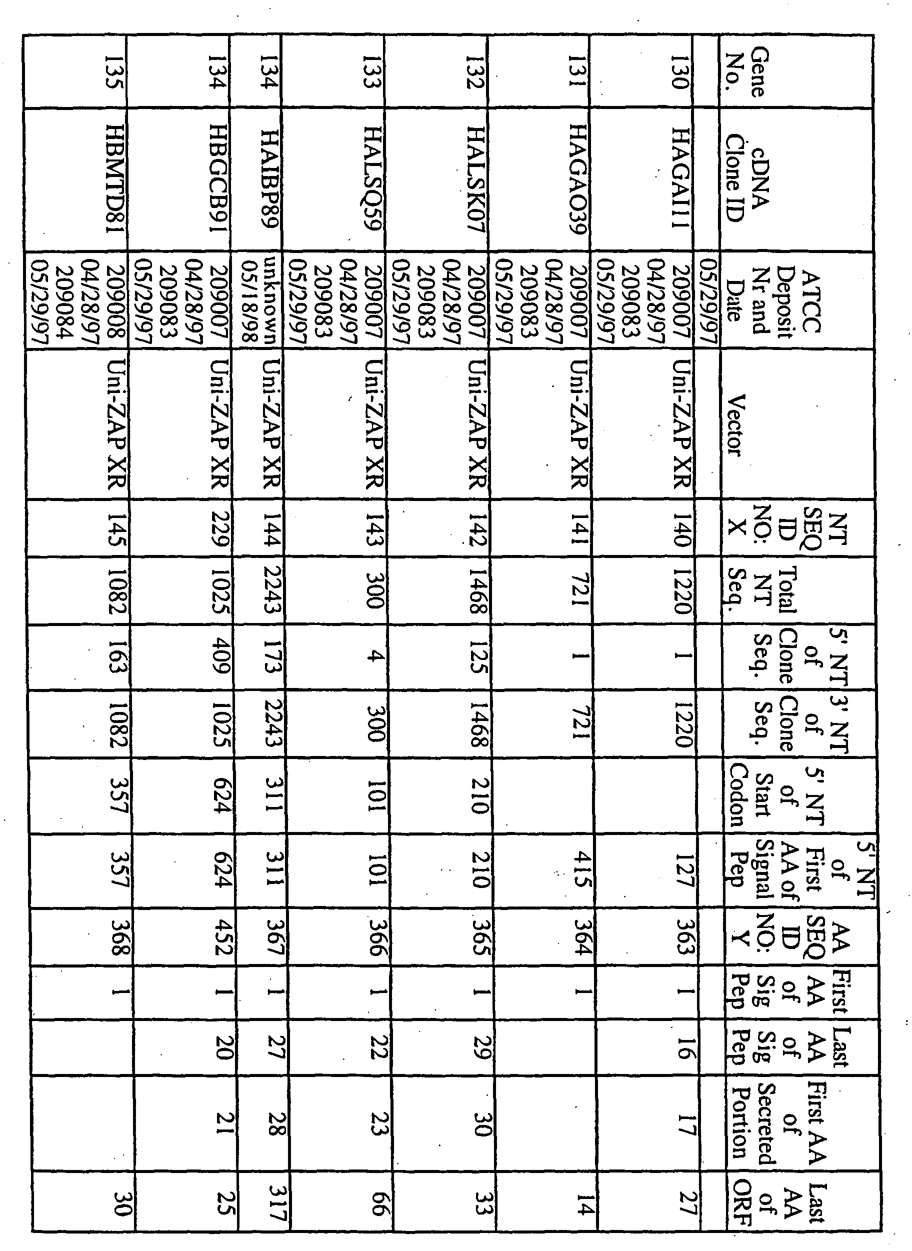

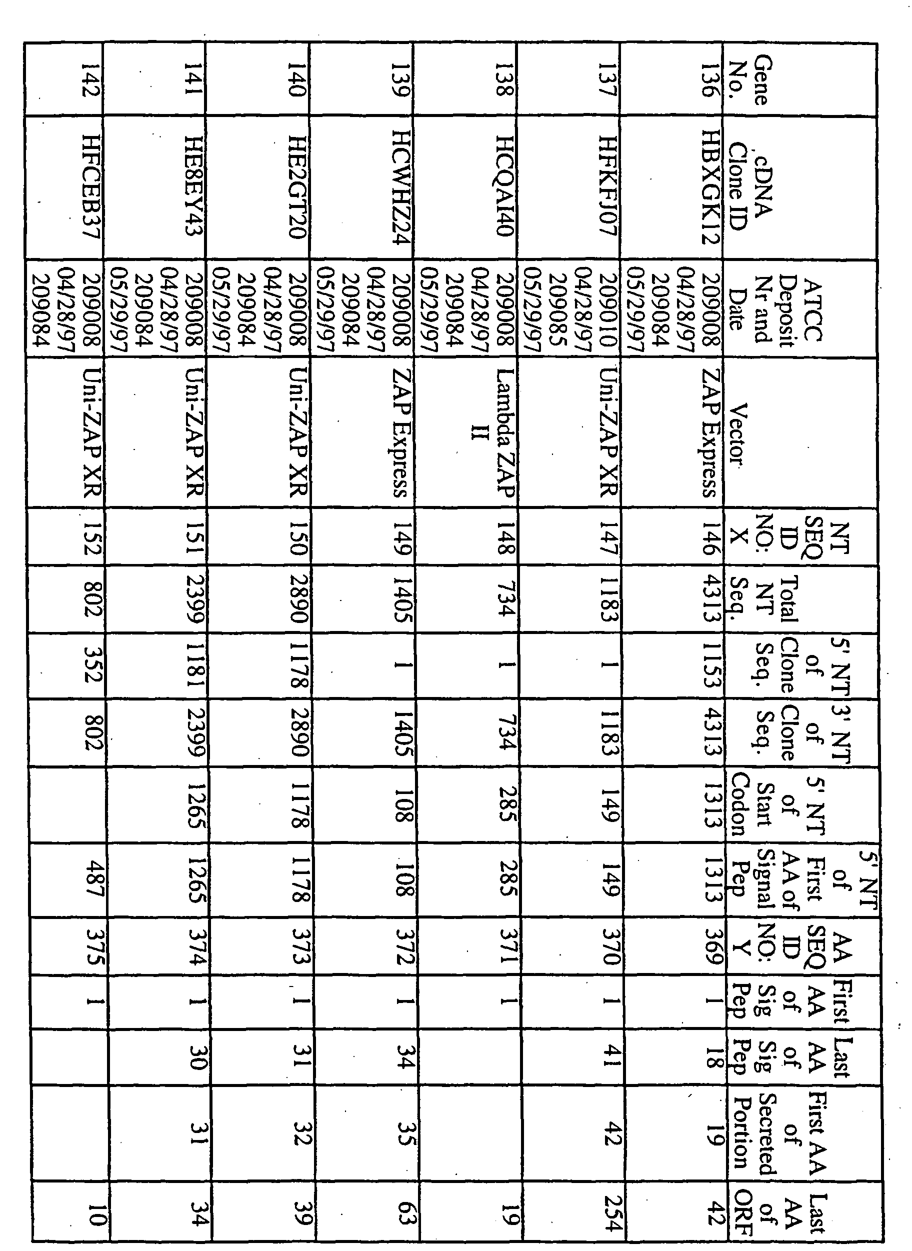

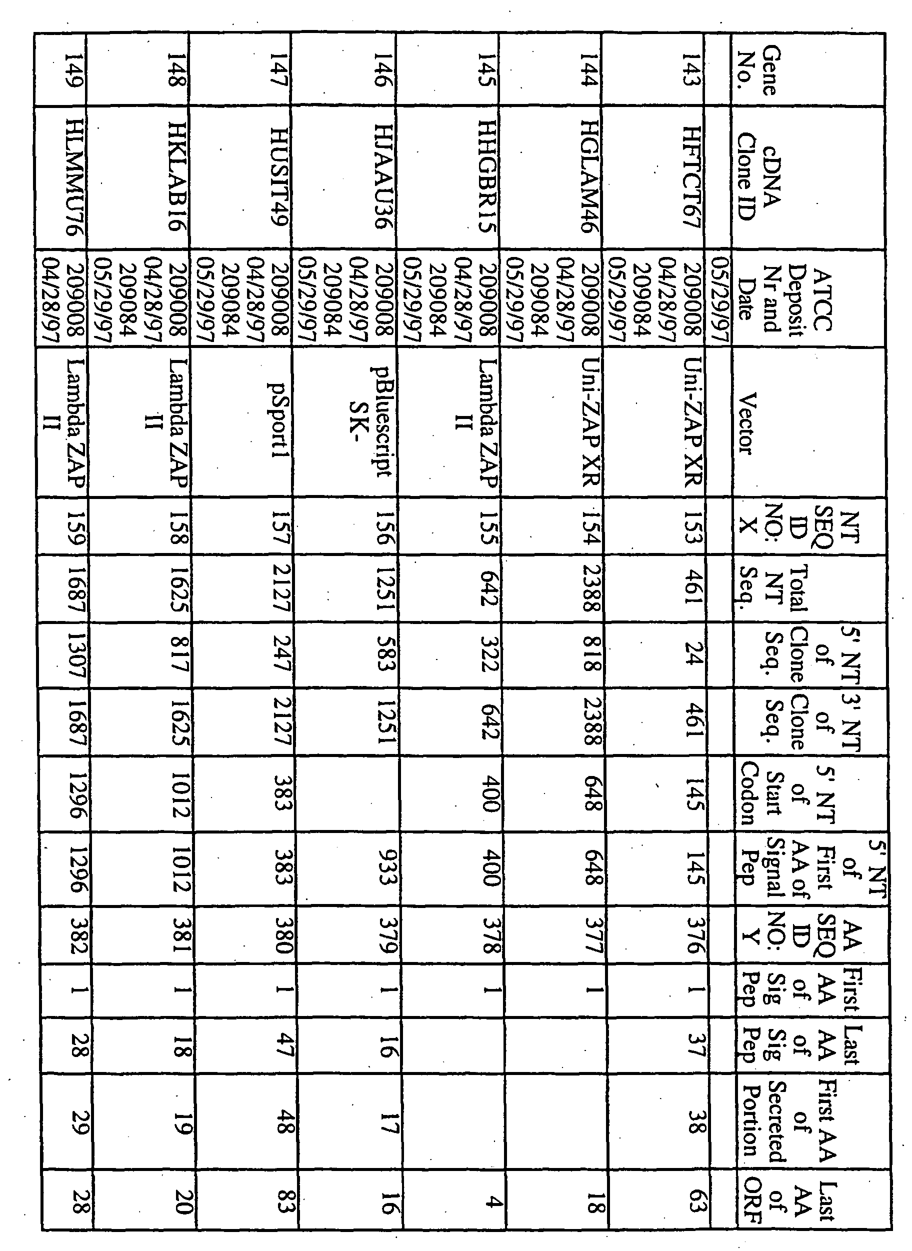

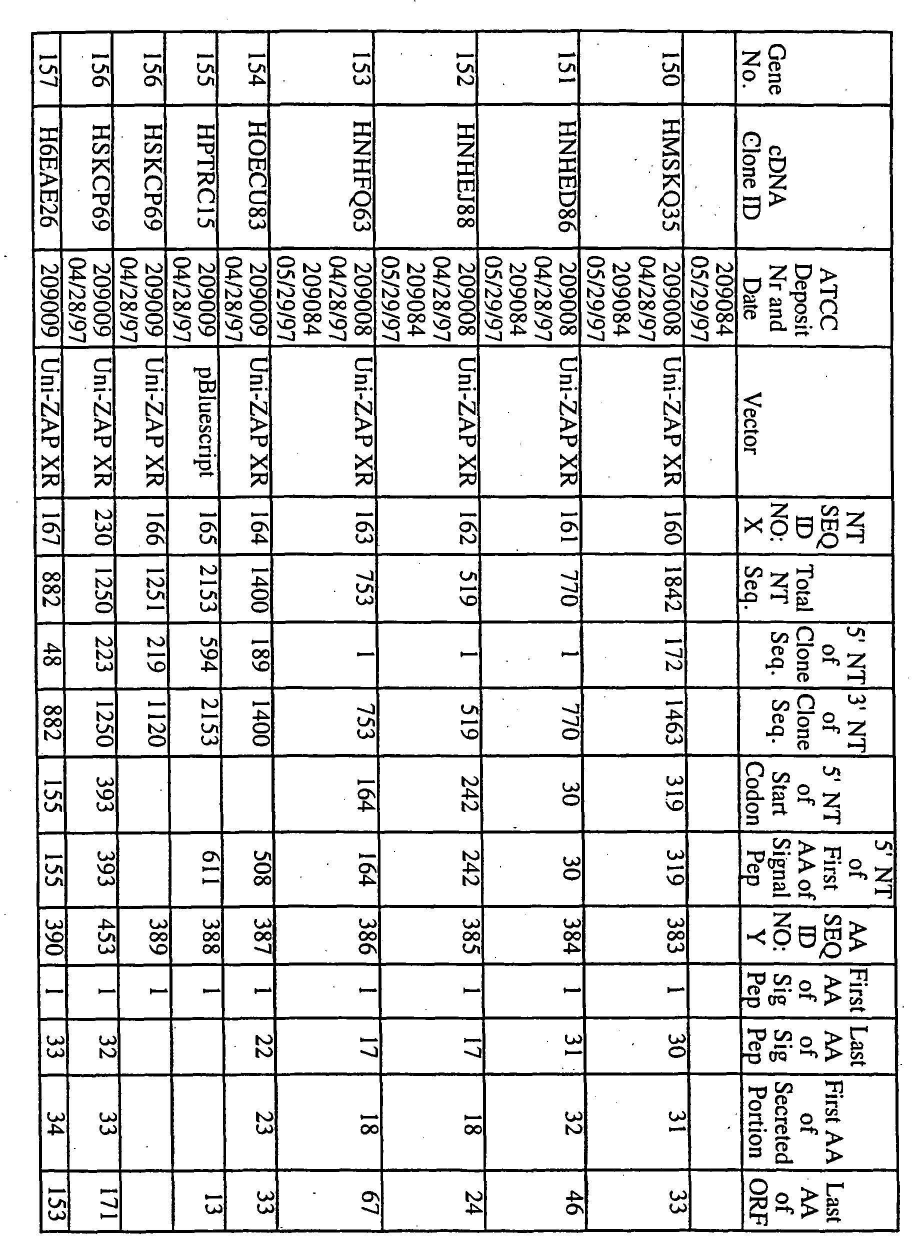

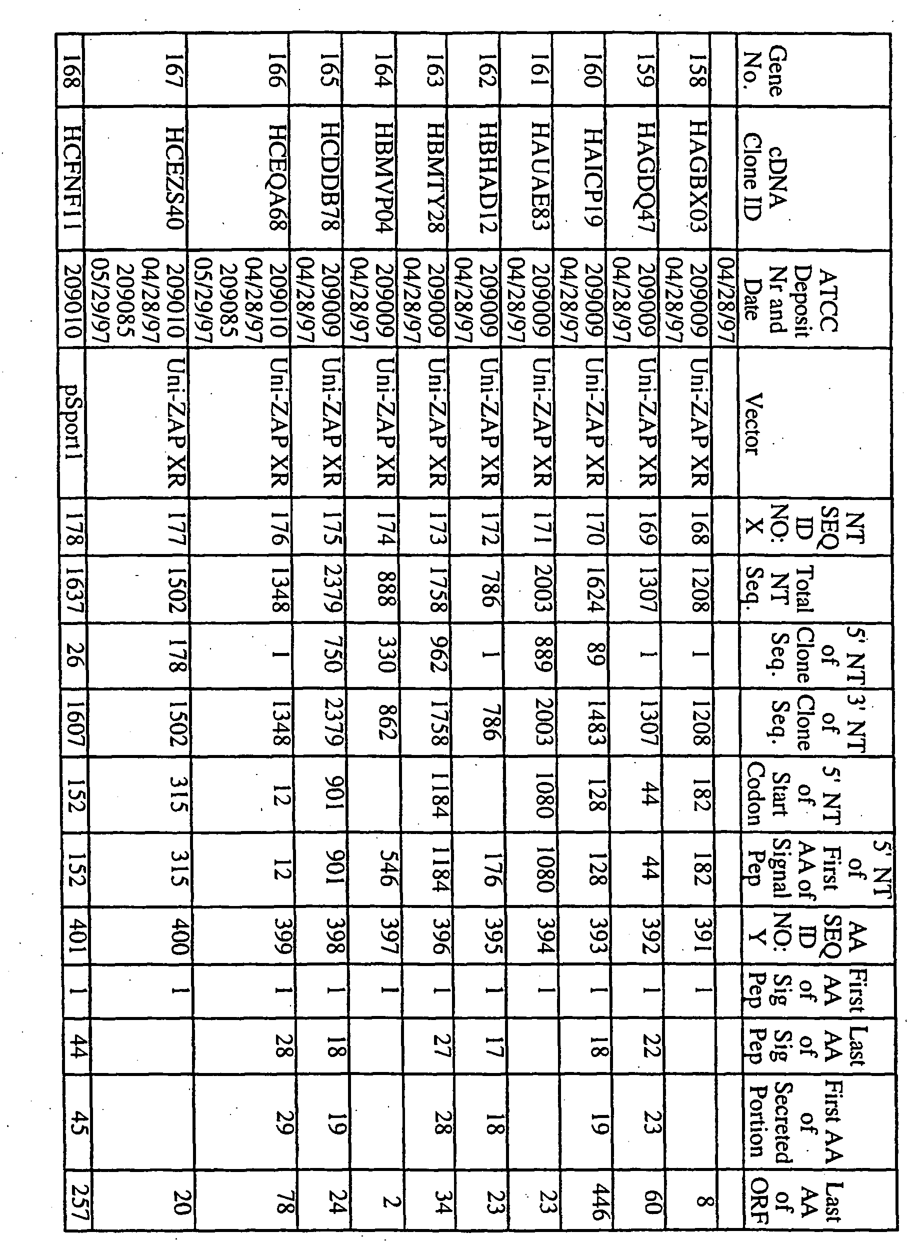

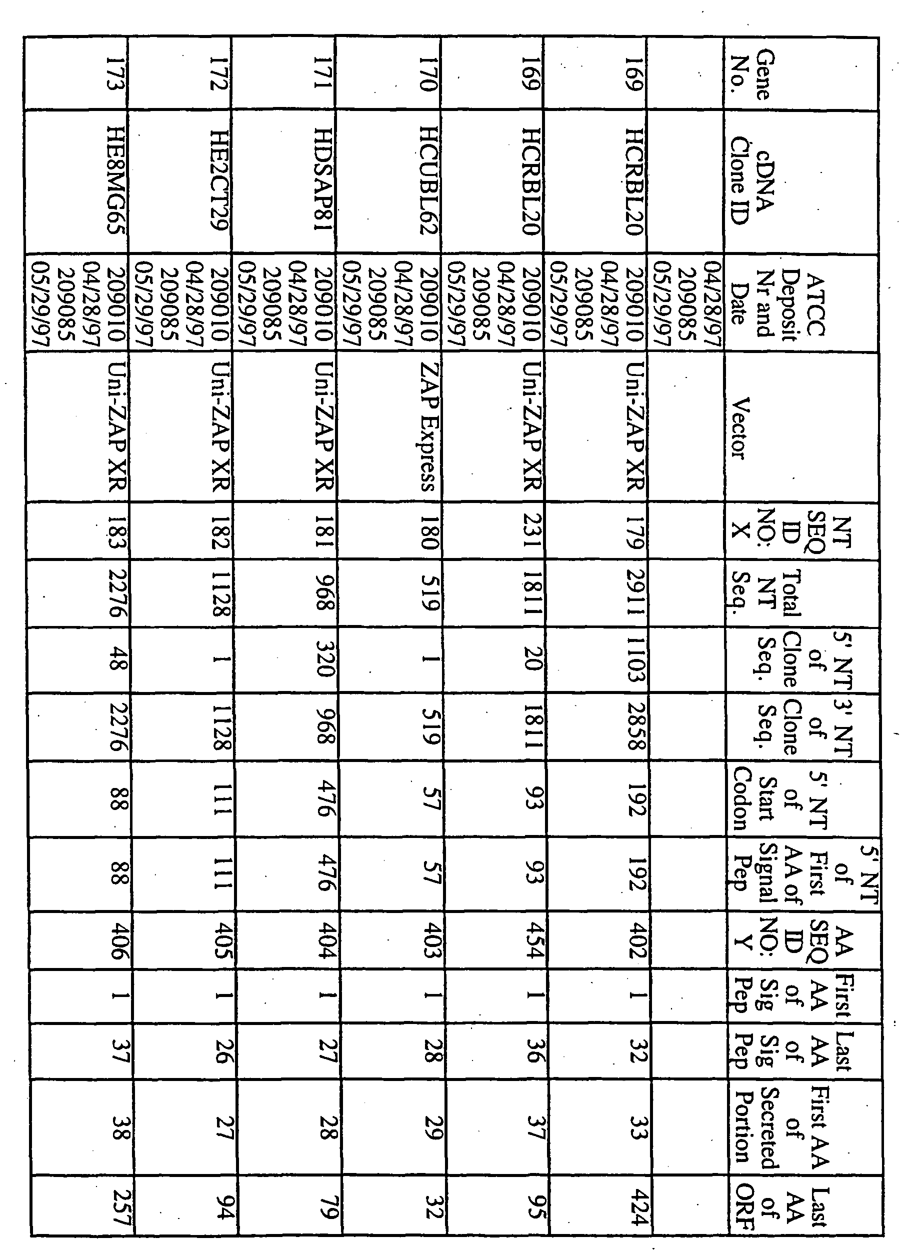

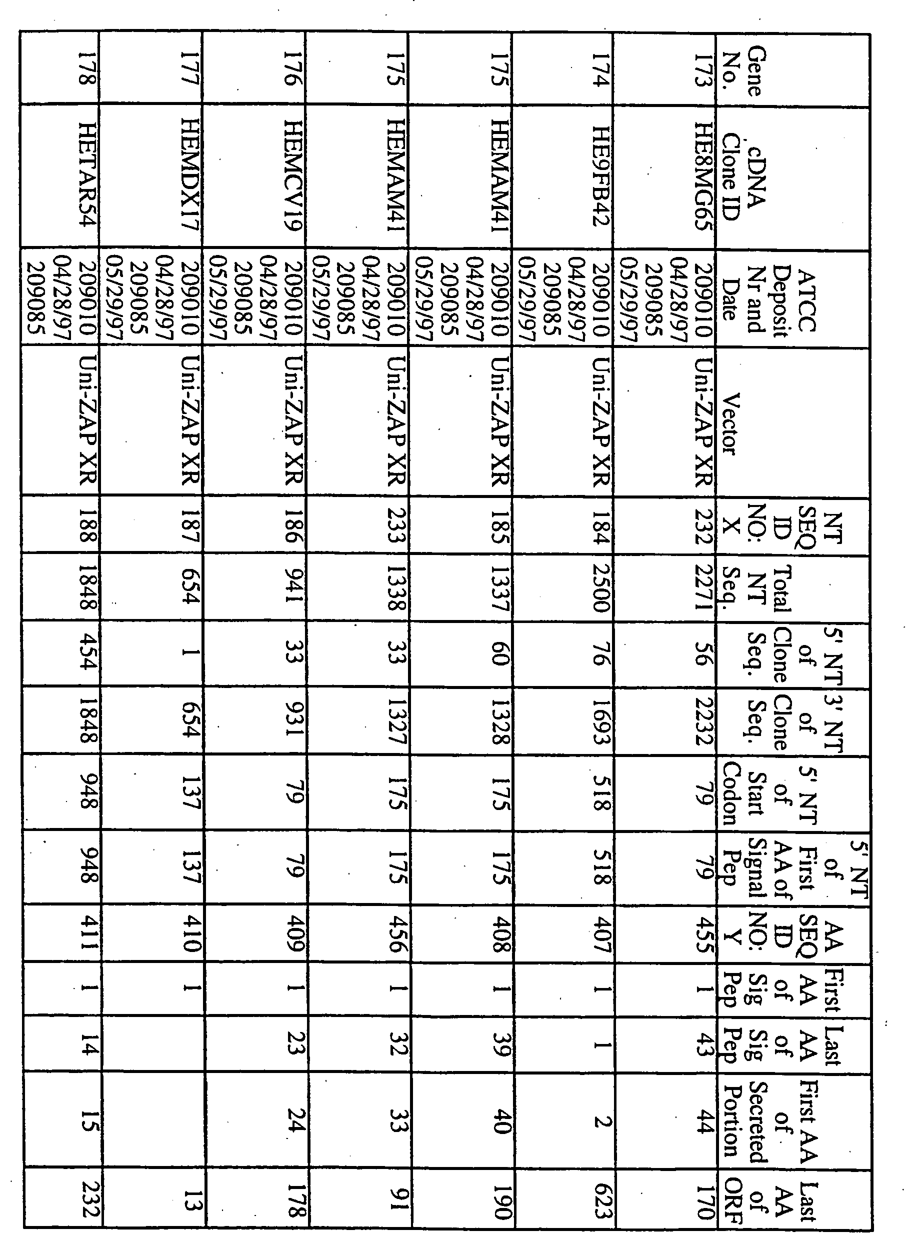

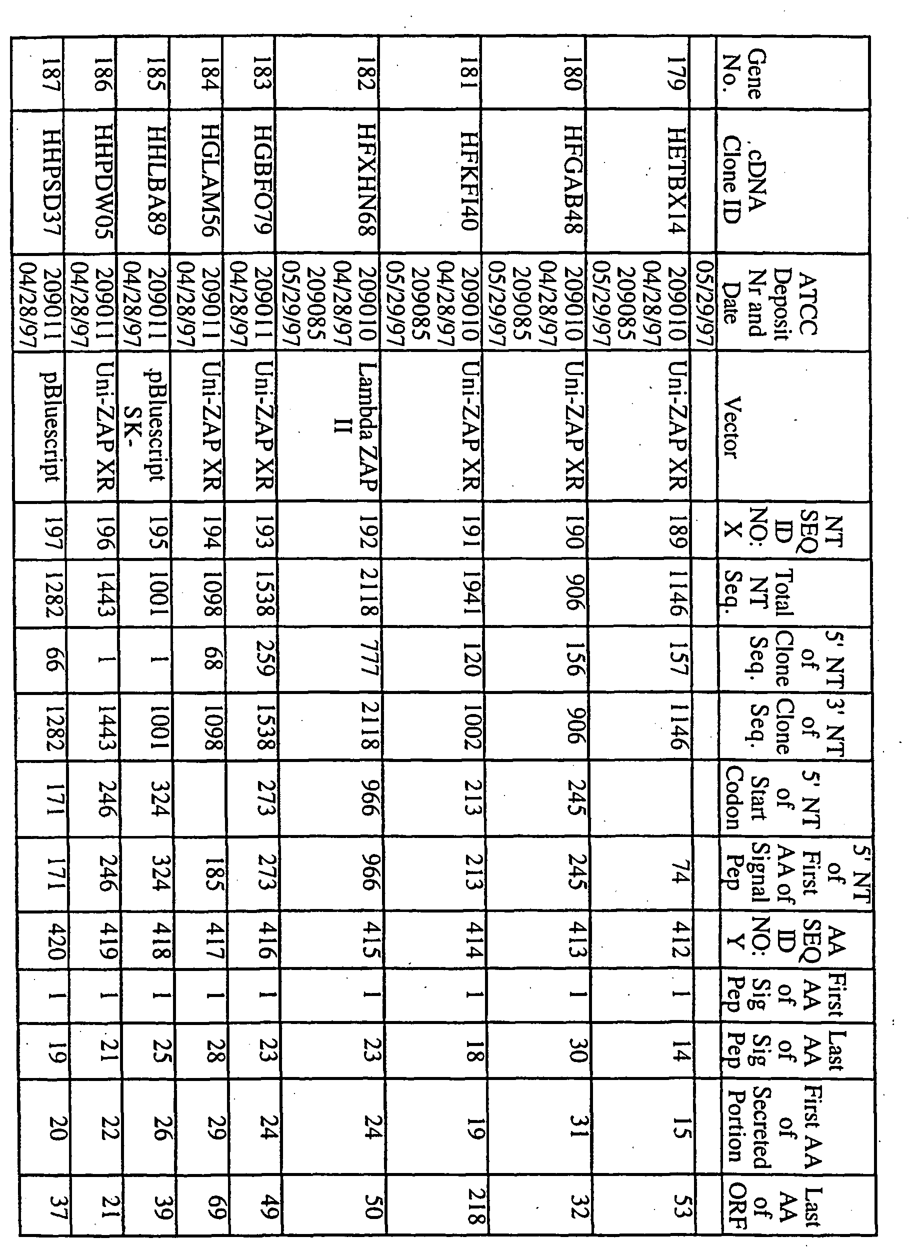

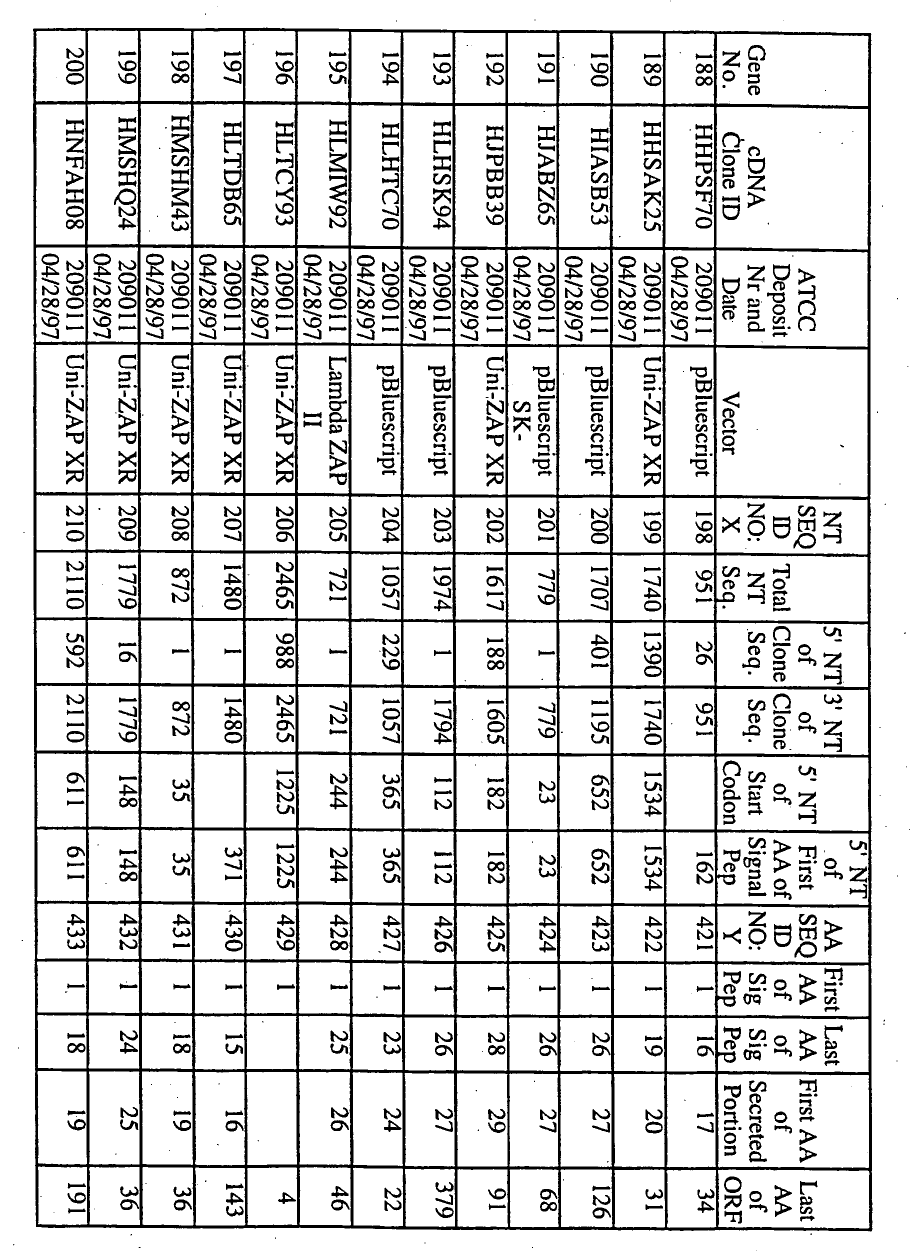

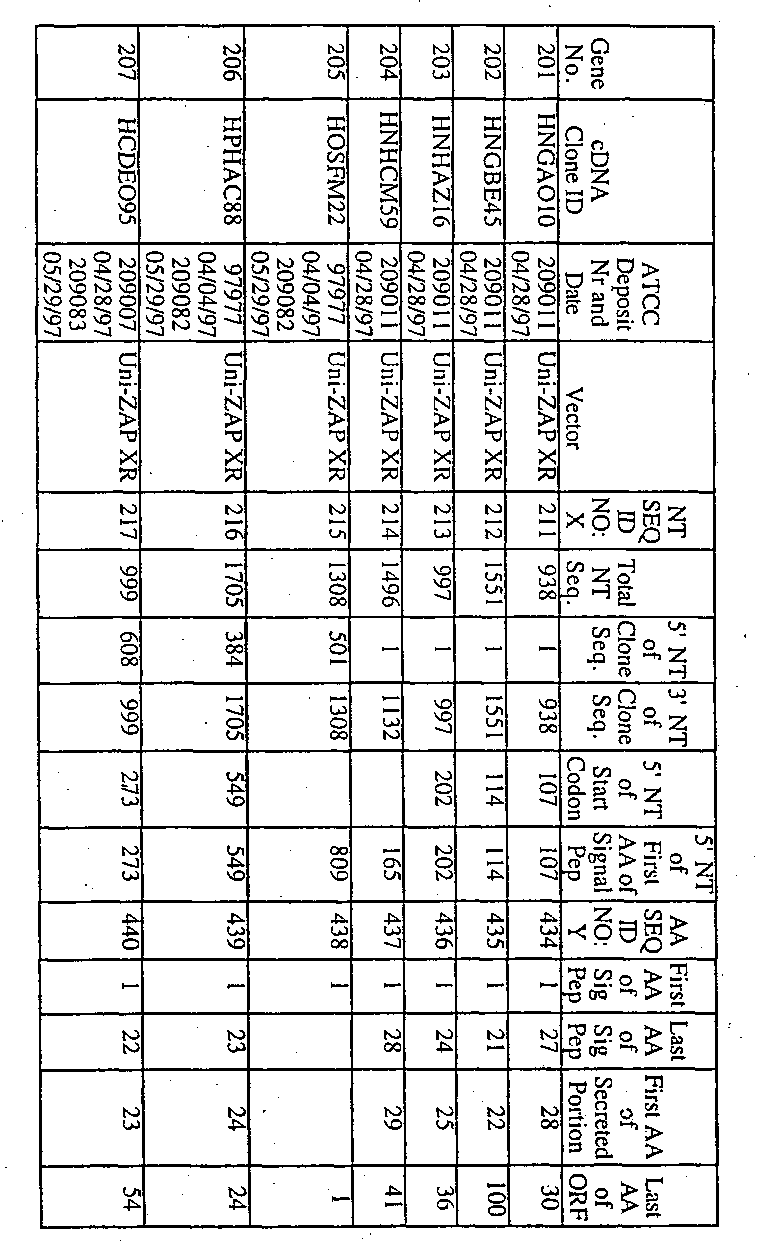

Table 1 summarizes the information corresponding to each "Gene No." described above. The nucleotide sequence identified as "NT SEQ ID NO:X" was assembled from partially homologous ("overlapping") sequences obtained from the "cDNA clone ID" identified in Table 1 and, in some cases, from additional related DNA clones. The overlapping sequences were assembled into a single contiguous sequence of high redundancy (usually three to five overlapping sequences at each nucleotide position), resulting in a final sequence identified as SEQ ID NO:X.

The cDNA Clone ID was deposited on the date and given the corresponding deposit number listed in "ATCC Deposit No:Z and Date." Some of the deposits contain multiple different clones corresponding to the same gene. "Vector" refers to the type of vector contained in the cDNA Clone ID.

"Total NT Seq." refers to the total number of nucleotides in the contig identified by "Gene No." The deposited clone may contain all or most of these sequences, reflected by the nucleotide position indicated as "5' NT of Clone Seq." and the "3' NT of Clone Seq." of SEQ ID NO:X. The nucleotide position of SEQ ID NO:X of the putative start codon (methionine) is identified as "5' NT of Start Codon." Similarly, the nucleotide position of SEQ ID NO:X of the predicted signal sequence is identified as "5' NT of First AA of Signal Pep."

The translated amino acid sequence, beginning with the methionine, is identified as "AA SEQ ID NO:Y," although other reading frames can also be easily translated using known molecular biology techniques. The polypeptides produced by these alternative open reading frames are specifically contemplated by the present invention.

The first and last amino acid position of SEQ ID NO:Y of the predicted signal peptide is identified as "First AA of Sig Pep" and "Last AA of Sig Pep." The predicted first amino acid position of SEQ ID NO:Y of the secreted portion is identified as "Predicted First AA of Secreted Portion." Finally, the amino acid position of SEQ ID NO:Y of the last amino acid in the open reading frame is identified as "Last AA of ORF."

SEQ ID NO:X and the translated SEQ ID NO:Y are sufficiently accurate and otherwise suitable for a variety of uses well known in the art and described further below. For instance, SEQ ID NO:X is useful for designing nucleic acid hybridization probes that will detect nucleic acid sequences contained in SEQ ID NO:X or the cDNA contained in the deposited clone. These probes will also hybridize to nucleic acid molecules in biological samples, thereby enabling a variety of forensic and diagnostic methods of the invention. Similarly, polypeptides identified from SEQ ID NO:Y may be used to generate antibodies which bind specifically to the secreted proteins encoded by the cDNA clones identified in Table 1.

Nevertheless, DNA sequences generated by sequencing reactions can contain sequencing errors. The errors exist as misidentified nucleotides, or as insertions or deletions of nucleotides in the generated DNA sequence. The erroneously inserted or deleted nucleotides cause frame shifts in the reading frames of the predicted amino acid sequence. In these cases, the predicted amino acid sequence diverges from the actual amino acid sequence, even though the generated DNA sequence may be greater than 99.9% identical to the actual DNA sequence (for example, one base insertion or deletion in an open reading frame of over 1000 bases).

Accordingly, for those applications requiring precision in the nucleotide sequence or the amino acid sequence, the present invention provides not only the generated nucleotide sequence identified as SEQ ID NO:X and the predicted translated amino acid sequence identified as SEQ ID NO:Y, but also a sample of plasmid DNA containing a human cDNA of the invention deposited with the ATCC, as set forth in Table 1. The nucleotide sequence of each deposited clone can readily be determined by sequencing the deposited clone in accordance with known methods. The predicted amino acid sequence can then be verified from such deposits. Moreover, the amino acid sequence of the protein encoded by a particular clone can also be directly determined by peptide sequencing or by expressing the protein in a suitable host cell containing the deposited human cDNA, collecting the protein, and determining its sequence.

The present invention also relates to the genes corresponding to SEQ ID NO:X, SEQ ID NO:Y, or the deposited clone. The corresponding gene can be isolated in accordance with known methods using the sequence information disclosed herein. Such methods include preparing probes or primers from the disclosed sequence and identifying or amplifying the corresponding gene from appropriate sources of genomic material.

Also provided in the present invention are species homologs. Species homologs may be isolated and identified by making suitable probes or primers from the sequences provided herein and screening a suitable nucleic acid source for the desired homologue.

The polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

The polypeptides may be in the form of the secreted protein, including the mature form, or may be a part of a larger protein, such as a fusion protein (see below). It is often advantageous to include an additional amino acid sequence which contains secretory or leader sequences, pro-sequences, sequences which aid in purification , such as multiple histidine residues, or an additional sequence for stability during recombinant production.

The polypeptides of the present invention are preferably provided in an isolated form, and preferably are substantially purified. A recombinantly produced version of a polypeptide, including the secreted polypeptide, can be substantially purified by the one-step method described in Smith and Johnson, Gene 67:31-40 (1988). Polypeptides of the invention also can be purified from natural or recombinant sources using antibodies of the invention raised against the secreted protein in methods which are well known in the art.

### Signal Sequences

Methods for predicting whether a protein has a signal sequence, as well as the cleavage point for that sequence, are available. For instance, the method of McGeoch, Virus Res. 3:271-286 (1985), uses the information from a short N-terminal charged region and a subsequent uncharged region of the complete (uncleaved) protein. The method of von Heinje, Nucleic Acids Res. 14:4683-4690 (1986) uses the information from the residues surrounding the cleavage site, typically residues -13 to +2, where +1 indicates the amino terminus of the secreted protein. The accuracy of predicting the cleavage points of known mammalian secretory proteins for each of these methods is in the range of 75-80%. (von Heinje, supra.) However, the two methods do not always produce the same predicted cleavage point(s) for a given protein.

In the present case, the deduced amino acid sequence of the secreted polypeptide was analyzed by a computer program called SignalP (Henrik Nielsen et al., Protein Engineering 10:1-6 (1997)), which predicts the cellular location of a protein based on the amino acid sequence. As part of this computational prediction of localization, the methods of McGeoch and von Heinje are incorporated. The analysis of the amino acid sequences of the secreted proteins described herein by this program provided the results shown in Table 1.

As one of ordinary skill would appreciate, however, cleavage sites sometimes vary from organism to organism and cannot be predicted with absolute certainty. Accordingly, the present invention provides secreted polypeptides having a sequence shown in SEQ ID NO:Y which have an N-terminus beginning within 5 residues (i.e., + or - 5 residues) of the predicted cleavage point. Similarly, it is also recognized that in some cases, cleavage of the signal sequence from a secreted protein is not entirely uniform, resulting in more than one secreted species. These polypeptides, and the polynucleotides encoding such polypeptides, are contemplated by the present invention.

Moreover, the signal sequence identified by the above analysis may not necessarily predict the naturally occurring signal sequence. For example, the naturally occurring signal sequence may be further upstream from the predicted signal sequence. However, it is likely that the predicted signal sequence will be capable of directing the secreted protein to the ER. These polypeptides, and the polynucleotides encoding such polypeptides, are contemplated by the present invention.

### Polynucleotide and Polypeptide Variants

"Variant" refers to a polynucleotide or polypeptide differing from the polynucleotide or polypeptide of the present invention, but retaining essential properties thereof. Generally, variants are overall closely similar, and, in many regions, identical to the polynucleotide or polypeptide of the present invention.

By a polynucleotide having a nucleotide sequence at least, for example, 95% "identical" to a reference nucleotide sequence of the present invention, it is intended that the nucleotide sequence of the polynucleotide is identical to the reference sequence except that the polynucleotide sequence may include up to five point mutations per each 100 nucleotides of the reference nucleotide sequence encoding the polypeptide. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. The query sequence may be an entire sequence shown inTable 1, the ORF (open reading frame), or any fragement specified as described herein.

As a practical matter, whether any particular nucleic acid molecule or polypeptide is at least 90%, 95%, 96%, 97%, 98% or 99% identical to a nucleotide sequence of the presence invention can be determined conventionally using known computer programs. A preferred method for determing the best overall match between a query sequence (a sequence of the present invention) and a subject sequence, also referred to as a global sequence alignment, can be determined using the FASTDB computer program based on the algorithm of Brutlag et al. (Comp. App. Biosci. (1990) 6:237-245). In a sequence alignment the query and subject sequences are both DNA sequences. An RNA sequence can be compared by converting U's to T's. The result of said global sequence alignment is in percent identity. Preferred parameters used in a FASTDB alignment of DNA sequences to calculate percent identiy are:
Matrix=Unitary, k-tuple=4, Mismatch Penalty=1, Joining Penalty=30, Randomization Group Length=0, Cutoff Score=1, Gap Penalty=5, Gap Size Penalty 0.05, Window Size=500 or the lenght of the subject nucleotide sequence, whichever is shorter.

If the subject sequence is shorter than the query sequence because of 5' or 3' deletions, not because of internal deletions, a manual correction must be made to the results. This is becuase the FASTDB program does not account for 5' and 3' truncations of the subject sequence when calculating percent identity. For subject sequences truncated at the 5' or 3' ends, relative to the the query sequence, the percent identity is corrected by calculating the number of bases of the query sequence that are 5' and 3' of the subject sequence, which are not matched/aligned, as a percent of the total bases of the query sequence. Whether a nucleotide is matched/aligned is determined by results of the FASTDB sequence alignment. This percentage is then subtracted from the percent identity, calculated by the above FASTDB program using the specified parameters, to arrive at a final percent identity score. This corrected score is what is used for the purposes of the present invention. Only bases outside the 5' and 3' bases of the subject sequence, as displayed by the FASTDB alignment, which are not matched/aligned with the query sequence, are calculated for the purposes of manually adjusting the percent identity score.

For example, a 90 base subject sequence is aligned to a 100 base query sequence to determine percent identity. The deletions occur at the 5' end of the subject sequence and therefore, the FASTDB alignment does not show a matched/alignement of the first 10 bases at 5' end. The 10 unpaired bases represent 10% of the sequence (number of bases at the 5' and 3' ends not matched/total number of bases in the query sequence) so 10% is subtracted from the percent identity score calculated by the FASTDB program. If the remaining 90 bases were perfectly matched the final percent identity would be 90%. In another example, a 90 base subject sequence is compared with a 100 base query sequence. This time the deletions are internal deletions so that there are no bases on the 5' or 3' of the subject sequence which are not matched/aligned with the query. In this case the percent identity calculated by FASTDB is not manually corrected. Once again, only bases 5' and 3' of the subject sequence which are not matched/aligned with the query sequnce are manually corrected for. No other manual corrections are to made for the purposes of the present invention.

By a polypeptide having an amino acid sequence at least, for example, 95% "identical" to a query amino acid sequence of the present invention, it is intended that the amino acid sequence of the subject polypeptide is identical to the query sequence except that the subject polypeptide sequence may include up to five amino acid alterations per each 100 amino acids of the query amino acid sequence. In other words, to obtain a polypeptide having an amino acid sequence at least 95% identical to a query amino acid sequence, up to 5% of the amino acid residues in the subject sequence may be inserted, deleted, (indels) or substituted with another amino acid. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

As a practical matter, whether any particular polypeptide is at least 90%, 95%, 96%, 97%, 98% or 99% identical to, for instance, the amino acid sequences shown in Table 1 or to the amino acid sequence encoded by deposited DNA clone can be determined conventionally using known computer programs. A preferred method for determing the best overall match between a query sequence (a sequence of the present invention) and a subject sequence, also referred to as a global sequence alignment, can be determined using the FASTDB computer program based on the algorithm of Brutlag et al. (Comp. App. Biosci. (1990) 6:237-245). In a sequence alignment the query and subject sequences are either both nucleotide sequences or both amino acid sequences. The result of said global sequence alignment is in percent identity. Preferred parameters used in a FASTDB amino acid alignment are: Matrix=PAM 0, k-tuple=2, Mismatch Penalty= 1, Joining Penalty=20, Randomization Group Length=0, Cutoff Score=1, Window Size=sequence length, Gap Penalty=5, Gap Size Penalty=0.05, Window Size=500 or the length of the subject amino acid sequence, whichever is shorter.

If the subject sequence is shorter than the query sequence due to N- or C-terminal deletions, not because of internal deletions, a manual correction must be made to the results. This is becuase the FASTDB program does not account for N- and C-terminal truncations of the subject sequence when calculating global percent identity. For subject sequences truncated at the N- and C-termini, relative to the the query sequence, the percent identity is corrected by calculating the number of residues of the query sequence that are N- and C-terminal of the subject sequence, which are not matched/aligned with a corresponding subject residue, as a percent of the total bases of the query sequence. Whether a residue is matched/aligned is determined by results of the FASTDB sequence alignment. This percentage is then subtracted from the percent identity, calculated by the above FASTDB program using the specified parameters, to arrive at a final percent identity score. This final percent identity score is what is used for the purposes of the present invention. Only residues to the N- and C-termini of the subject sequence, which are not matched/aligned with the query sequence, are considered for the purposes of manually adjusting the percent identity score. That is, only query residue positions outside the farthest N- and C-terminal residues of the subject sequence.

For example, a 90 amino acid residue subject sequence is aligned with a 100 residue query sequence to determine percent identity. The deletion occurs at the N-terminus of the subject sequence and therefore, the FASTDB alignment does not show a matching/alignment of the first 10 residues at the N-terminus. The 10 unpaired residues represent 10% of the sequence (number of residues at the N- and C- termini not matched/total number of residues in the query sequence) so 10% is subtracted from the percent identity score calculated by the FASTDB program. If the remaining 90 residues were perfectly matched the final percent identity would be 90%. In another example, a 90 residue subject sequence is compared with a 100 residue query sequence. This time the deletions are internal deletions so there are no residues at the N- or C-termini of the subject sequence which are not matched/aligned with the query. In this case the percent identity calculated by FASTDB is not manually corrected. Once again, only residue positions outside the N- and C-terminal ends of the subject sequence, as displayed in the FASTDB alignment, which are not matched/aligned with the query sequnce are manually corrected for. No other manual corrections are to made for the purposes of the present invention.

The variants may contain alterations in the coding regions, non-coding regions, or both. Especially preferred are polynucleotide variants containing alterations which produce silent substitutions, additions, or deletions, but do not alter the properties or activities of the encoded polypeptide. Nucleotide variants produced by silent substitutions due to the degeneracy of the genetic code are preferred. Moreover, variants in which 5-10, 1-5, or 1-2 amino acids are substituted, deleted, or added in any combination are also preferred. Polynucleotide variants can be produced for a variety of reasons, e.g., to optimize codon expression for a particular host (change codons in the human mRNA to those preferred by a bacterial host such as E. coli).

Naturally occurring variants are called "allelic variants," and refer to one of several alternate forms of a gene occupying a given locus on a chromosome of an organism. (Genes II, Lewin, B., ed., John Wiley & Sons, New York (1985).) These allelic variants can vary at either the polynucleotide and/or polypeptide level. Alternatively, non-naturally occurring variants may be produced by mutagenesis techniques or by direct synthesis.

Using known methods of protein engineering and recombinant DNA technology, variants may be generated to improve or alter the characteristics of the polypeptides of the present invention. For instance, one or more amino acids can be deleted from the N-terminus or C-terminus of the secreted protein without substantial loss of biological function. The authors of Ron et al., J. Biol. Chem. 268: 2984-2988 (1993), reported variant KGF proteins having heparin binding activity even after deleting 3, 8, or 27 amino-terminal amino acid residues. Similarly, Interferon gamma exhibited up to ten times higher activity after deleting 8-10 amino acid residues from the carboxy terminus of this protein. (Dobeli et al., J. Biotechnology 7:199-216 (1988).)

Moreover, ample evidence demonstrates that variants often retain a biological activity similar to that of the naturally occurring protein. For example, Gayle and coworkers (J. Biol. Chem 268:22105-22111 (1993)) conducted extensive mutational analysis of human cytokine IL-1a. They used random mutagenesis to generate over 3,500 individual IL-1a mutants that averaged 2.5 amino acid changes per variant over the entire length of the molecule. Multiple mutations were examined at every possible amino acid position. The investigators found that "[m]ost of the molecule could be altered with little effect on either [binding or biological activity]." (See, Abstract.) In fact, only 23 unique amino acid sequences, out of more than 3,500 nucleotide sequences examined, produced a protein that significantly differed in activity from wild-type.

Furthermore, even if deleting one or more amino acids from the N-terminus or C-terminus of a polypeptide results in modification or loss of one or more biological functions, other biological activities may still be retained. For example, the ability of a deletion variant to induce and/or to bind antibodies which recognize the secreted form will likely be retained when less than the majority of the residues of the secreted form are removed from the N-terminus or C-terminus. Whether a particular polypeptide lacking N- or C-terminal residues of a protein retains such immunogenic activities can readily be determined by routine methods described herein and otherwise known in the art.

Thus, the invention further includes polypeptide variants which show substantial biological activity. Such variants include deletions, insertions, inversions, repeats, and substitutions selected according to general rules known in the art so as have little effect on activity. For example, guidance concerning how to make phenotypically silent amino acid substitutions is provided in Bowie, J. U. et al., Science 247:1306-1310 (1990), wherein the authors indicate that there are two main strategies for studying the tolerance of an amino acid sequence to change.

The first strategy exploits the tolerance of amino acid substitutions by natural selection during the process of evolution. By comparing amino acid sequences in different species, conserved amino acids can be identified. These conserved amino acids are likely important for protein function. In contrast, the amino acid positions where substitutions have been tolerated by natural selection indicates that these positions are not critical for protein function. Thus, positions tolerating amino acid substitution could be modified while still maintaining biological activity of the protein.

The second strategy uses genetic engineering to introduce amino acid changes at specific positions of a cloned gene to identify regions critical for protein function. For example, site directed mutagenesis or alanine-scanning mutagenesis (introduction of single alanine mutations at every residue in the molecule) can be used. (Cunningham and Wells, Science 244:1081-1085 (1989).) The resulting mutant molecules can then be tested for biological activity.

As the authors state, these two strategies have revealed that proteins are surprisingly tolerant of amino acid substitutions. The authors further indicate which amino acid changes are likely to be permissive at certain amino acid positions in the protein. For example, most buried (within the tertiary structure of the protein) amino acid residues require nonpolar side chains, whereas few features of surface side chains are generally conserved. Moreover, tolerated conservative amino acid substitutions involve replacement of the aliphatic or hydrophobic amino acids Ala, Val, Leu and Ile; replacement of the hydroxyl residues Ser and Thr; replacement of the acidic residues Asp and Glu; replacement of the amide residues Asn and Gln, replacement of the basic residues Lys, Arg, and His; replacement of the aromatic residues Phe, Tyr, and Trp, and replacement of the small-sized amino acids Ala, Ser, Thr, Met, and Gly.

Besides conservative amino acid substitution, variants of the present invention include (i) substitutions with one or more of the non-conserved amino acid residues, where the substituted amino acid residues may or may not be one encoded by the genetic code, or (ii) substitution with one or more of amino acid residues having a substituent group, or (iii) fusion of the mature polypeptide with another compound, such as a compound to increase the stability and/or solubility of the polypeptide (for example, polyethylene glycol), or (iv) fusion of the polypeptide with additional amino acids, such as an IgG Fc fusion region peptide, or leader or secretory sequence, or a sequence facilitating purification. Such variant polypeptides are deemed to be within the scope of those skilled in the art from the teachings herein.

For example, polypeptide variants containing amino acid substitutions of charged amino acids with other charged or neutral amino acids may produce proteins with improved characteristics, such as less aggregation. Aggregation of pharmaceutical formulations both reduces activity and increases clearance due to the aggregate's immunogenic activity. (Pinckard et al., Clin. Exp. Immunol. 2:331-340 (1967); Robbins et al., Diabetes 36: 838-845 (1987); Cleland et al., Crit. Rev. Therapeutic Drug Carrier Systems 10:307-377 (1993).)

### Polynucleotide and Polypeptide Fragments

In the present invention, a "polynucleotide fragment" refers to a short polynucleotide having a nucleic acid sequence contained in the deposited clone or shown in SEQ ID NO:X. The short nucleotide fragments are preferably at least about 15 nt, and more preferably at least about 20 nt, still more preferably at least about 30 nt, and even more preferably, at least about 40 nt in length. A fragment "at feast 20 nt in length," for example, is intended to include 20 or more contiguous bases from the cDNA sequence contained in the deposited clone or the nucleotide sequence shown in SEQ ID NO:X. These nucleotide fragments are useful as diagnostic probes and primers as discussed herein. Of course, larger fragments (e.g., 50, 150, 500, 600, 2000 nucleotides) are preferred.

Moreover, representative examples of polynucleotide fragments of the invention, include, for example, fragments having a sequence from about nucleotide number 1-50, 51-100, 101-150, 151-200, 201-250, 251-300, 301-350, 351-400, 401-450, 451-500, 501-550, 551-600, 651-700, 701-750, 751-800, 800-850, 851-900, 901-950, 951-1000, 1001-1050, 1051-1100, 1101-1150, 1151-1200, 1201-1250, 1251-1300, 1301-1350, 1351-1400, 1401-1450, 1451-1500, 1501-1550, 1551-1600, 1601-1650, 1651-1700, 1701-1750, 1751-1800, 1801-1850, 1851-1900, 1901-1950, 1951-2000, or 2001 to the end of SEQ ID NO:X or the cDNA contained in the deposited clone. In this context "about" includes the particularly recited ranges, larger or smaller by several (5, 4, 3, 2, or 1) nucleotides, at either terminus or at both termini. Preferably, these fragments encode a polypeptide which has biological activity. More preferably, these polynucleotides can be used as probes or primers as discussed herein.

In the present invention, a "polypeptide fragment" refers to a short amino acid sequence contained in SEQ ID NO:Y or encoded by the cDNA contained in the deposited clone. Protein fragments may be "free-standing," or comprised within a larger polypeptide of which the fragment forms a part or region, most preferably as a single continuous region. Representative examples of polypeptide fragments of the invention, include, for example, fragments from about amino acid number 1-20, 21-40, 41-60, 61-80, 81-100, 102-120, 121-140, 141-160, or 161 to the end of the coding region. Moreover, polypeptide fragments can be about 20, 30, 40, 50, 60, 70, 80, 90, 100, 110, 120, 130, 140, or 150 amino acids in length. In this context "about" includes the particularly recited ranges, larger or smaller by several (5, 4, 3, 2, or 1) amino acids, at either extreme or at both extremes.

Preferred polypeptide fragments include the secreted protein as well as the mature form. Further preferred polypeptide fragments include the secreted protein or the mature form having a continuous series of deleted residues from the amino or the carboxy terminus, or both. For example, any number of amino acids, ranging from 1-60, can be deleted from the amino terminus of either the secreted polypeptide or the mature form. Similarly, any number of amino acids, ranging from 1-30, can be deleted from the carboxy terminus of the secreted protein or mature form. Furthermore, any combination of the above amino and carboxy terminus deletions are preferred. Similarly, polynucleotide fragments encoding these polypeptide fragments are also preferred.

Particularly, N-terminal deletions of the polypeptide of the present invention can be described by the general formula m-p, where p is the total number of amino acids in the polypeptide and m is an integer from 2 to (p-1), and where both of these integers (m & p) correspond to the position of the amino acid residue identified in SEQ ID NO:Y.

Moreover, C-terminal deletions of the polypeptide of the present invention can also be described by the general formula 1-n, where n is an integer from 2 to (p-1), and again where these integers (n & p) correspond to the position of the amino acid residue identified in SEQ ID NO:Y.

The invention also provides polypeptides having one or more amino acids deleted from both the amino and the carboxyl termini, which may be described generally as having residues m-n of SEQ ID NO:Y, where m and n are integers as described above.

Also preferred are polypeptide and polynucleotide fragments characterized by structural or functional domains, such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Polypeptide fragments of SEQ ID NO:Y falling within conserved domains are specifically contemplated by the present invention. Moreover, polynucleotide fragments encoding these domains are also contemplated.

Other preferred fragments are biologically active fragments. Biologically active fragments are those exhibiting activity similar, but not necessarily identical, to an activity of the polypeptide of the present invention. The biological activity of the fragments may include an improved desired activity, or a decreased undesirable activity.

### Epitopes & Antibodies

In the present invention, "epitopes" refer to polypeptide fragments having antigenic or immunogenic activity in an animal, especially in a human. A preferred embodiment of the present invention relates to a polypeptide fragment comprising an epitope, as well as the polynucleotide encoding this fragment. A region of a protein molecule to which an antibody can bind is defined as an "antigenic epitope." In contrast, an "immunogenic epitope" is defined as a part of a protein that elicits an antibody response. (See, for instance, Geysen et al., Proc. Natl. Acad. Sci. USA 81:3998- 4002 (1983).)

Fragments which function as epitopes may be produced by any conventional means. (See, e.g., Houghten, R. A., Proc. Natl. Acad. Sci. USA 82:5131-5135 (1985) further described in U.S. Patent No. 4,631,211.)

In the present invention, antigenic epitopes preferably contain a sequence of at least seven, more preferably at least nine, and most preferably between about 15 to about 30 amino acids. Antigenic epitopes are useful to raise antibodies, including monoclonal antibodies, that specifically bind the epitope. (See, for instance, Wilson et al., Cell 37:767-778 (1984); Sutcliffe, J. G. et al., Science 219:660-666 (1983).)

Similarly, immunogenic epitopes can be used to induce antibodies according to methods well known in the art. (See, for instance, Sutcliffe et al., supra; Wilson et al., supra; Chow, M. et al., Proc. Natl. Acad. Sci. USA 82:910-914; and Bittle, F. J. et al., J. Gen. Virol. 66:2347-2354 (1985).) A preferred immunogenic epitope includes the secreted protein. The immunogenic epitopes may be presented together with a carrier protein, such as an albumin, to an animal system (such as rabbit or mouse) or, if it is long enough (at least about 25 amino acids), without a carrier. However, immunogenic epitopes comprising as few as 8 to 10 amino acids have been shown to be sufficient to raise antibodies capable of binding to, at the very least, linear epitopes in a denatured polypeptide (e.g., in Western blotting.)

As used herein, the term "antibody" (Ab) or "monoclonal antibody" (Mab) is meant to include intact molecules as well as antibody fragments (such as, for example, Fab and F(ab')2 fragments) which are capable of specifically binding to protein. Fab and F(ab')2 fragments lack the Fc fragment of intact antibody, clear more rapidly from the circulation, and may have less non-specific tissue binding than an intact antibody. (Wahl et al., J. Nucl. Med. 24:316-325 (1983).) Thus, these fragments are preferred, as well as the products of a FAB or other immunoglobulin expression library. Moreover, antibodies of the present invention include chimeric, single chain, and humanized antibodies.

### Fusion Proteins

Any polypeptide of the present invention can be used to generate fusion proteins. For example, the polypeptide of the present invention, when fused to a second protein, can be used as an antigenic tag. Antibodies raised against the polypeptide of the present invention can be used to indirectly detect the second protein by binding to the polypeptide. Moreover, because secreted proteins target cellular locations based on trafficking signals, the polypeptides of the present invention can be used as targeting molecules once fused to other proteins.

Examples of domains that can be fused to polypeptides of the present invention include not only heterologous signal sequences, but also other heterologous functional regions. The fusion does not necessarily need to be direct, but may occur through linker sequences.

Moreover, fusion proteins may also be engineered to improve characteristics of the polypeptide of the present invention. For instance, a region of additional amino acids, particularly charged amino acids, may be added to the N-terminus of the polypeptide to improve stability and persistence during purification from the host cell or subsequent handling and storage. Also, peptide moieties may be added to the polypeptide to facilitate purification. Such regions may be removed prior to final preparation of the polypeptide. The addition of peptide moieties to facilitate handling of polypeptides are familiar and routine techniques in the art.

Moreover, polypeptides of the present invention, including fragments, and specifically epitopes, can be combined with parts of the constant domain of immunoglobulins (IgG), resulting in chimeric polypeptides. These fusion proteins facilitate purification and show an increased half-life in vivo. One reported example describes chimeric proteins consisting of the first two domains of the human CD4-polypeptide and various domains of the constant regions of the heavy or light chains of mammalian immunoglobulins. (EP A 394,827; Traunecker et al., Nature 331:84-86 (1988).) Fusion proteins having disulfide-linked dimeric structures (due to the IgG) can also be more efficient in binding and neutralizing other molecules, than the monomeric secreted protein or protein fragment alone. (Fountoulakis et al., J. Biochem. 270:3958-3964 (1995).)

Similarly, EP-A-O 464 533 (Canadian counterpart 2045869) discloses fusion proteins comprising various portions of constant region of immunoglobulin molecules together with another human protein or part thereof. In many cases, the Fc part in a fusion protein is beneficial in therapy and diagnosis, and thus can result in, for example, improved pharmacokinetic properties. (EP-A 0232 262.) Alternatively, deleting the Fc part after the fusion protein has been expressed, detected, and purified, would be desired. For example, the Fc portion may hinder therapy and diagnosis if the fusion protein is used as an antigen for immunizations. In drug discovery, for example, human proteins, such as hIL-5, have been fused with Fc portions for the purpose of high-throughput screening assays to identify antagonists of hIL-5. (See, D. Bennett et al., J. Molecular Recognition 8:52-58 (1995); K. Johanson et al., J. Biol. Chem. 270:9459-9471 (1995).)

Moreover, the polypeptides of the present invention can be fused to marker sequences, such as a peptide which facilitates purification of the fused polypeptide. In preferred embodiments, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described in Gentz et al., Proc. Natl. Acad. Sci. USA 86:821-824 (1989), for instance, hexa-histidine provides for convenient purification of the fusion protein. Another peptide tag useful for purification, the "HA" tag, corresponds to an epitope derived from the influenza hemagglutinin protein. (Wilson et al., Cell 37:767 (1984).)

Thus, any of these above fusions can be engineered using the polynucleotides or the polypeptides of the present invention.

### Vectors, Host Cells, and Protein Production

The present invention also relates to vectors containing the polynucleotide of the present invention, host cells, and the production of polypeptides by recombinant techniques. The vector may be, for example, a phage, plasmid, viral, or retroviral vector. Retroviral vectors may be replication competent or replication defective. In the latter case, viral propagation generally will occur only in complementing host cells.

The polynucleotides may be joined to a vector containing a selectable marker for propagation in a host. Generally, a plasmid vector is introduced in a precipitate, such as a calcium phosphate precipitate, or in a complex with a charged lipid. If the vector is a virus, it may be packaged in vitro using an appropriate packaging cell line and then transduced into host cells.

The polynucleotide insert should be operatively linked to an appropriate promoter, such as the phage lambda PL promoter, the E. coli lac, trp, phoA and tac promoters, the SV40 early and late promoters and promoters of retroviral LTRs, to name a few. Other suitable promoters will be known to the skilled artisan. The expression constructs will further contain sites for transcription initiation, termination, and, in the transcribed region, a ribosome binding site for translation. The coding portion of the transcripts expressed by the constructs will preferably include a translation initiating codon at the beginning and a termination codon (UAA, UGA or UAG) appropriately positioned at the end of the polypeptide to be translated.

As indicated, the expression vectors will preferably include at least one selectable marker. Such markers include dihydrofolate reductase, G418 or neomycin resistance for eukaryotic cell culture and tetracycline, kanamycin or ampicillin resistance genes for culturing in E. coli and other bacteria. Representative examples of appropriate hosts include, but are not limited to, bacterial cells, such as E. coli, Streptomyces and Salmonella typhimurium cells; fungal cells, such as yeast cells; insect cells such as Drosophila S2 and Spodoptera Sf9 cells; animal cells such as CHO, COS, 293, and Bowes melanoma cells; and plant cells. Appropriate culture mediums and conditions for the above-described host cells are known in the art.

Among vectors preferred for use in bacteria include pQE70, pQE60 and pQE-9, available from QIAGEN, Inc.; pBluescript vectors, Phagescript vectors, pNH8A, pNH16a, pNH18A, pNH46A, available from Stratagene Cloning Systems, Inc.; and ptrc99a, pKK223-3, pKK233-3, pDR540, pRIT5 available from Pharmacia Biotech, Inc. Among preferred eukaryotic vectors are pWLNEO, pSV2CAT, pOG44, pXT1 and pSG available from Stratagene; and pSVK3, pBPV, pMSG and pSVL available from Pharmacia. Other suitable vectors will be readily apparent to the skilled artisan.

Introduction of the construct into the host cell can be effected by calcium phosphate transfection, DEAE-dextran mediated transfection, cationic lipid-mediated transfection, electroporation, transduction, infection, or other methods. Such methods are described in many standard laboratory manuals, such as Davis et al., Basic Methods In Molecular Biology (1986). It is specifically contemplated that the polypeptides of the present invention may in fact be expressed by a host cell lacking a recombinant vector.

A polypeptide of this invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification.

Polypeptides of the present invention, and preferably the secreted form, can also be recovered from: products purified from natural sources, including bodily fluids, tissues and cells, whether directly isolated or cultured; products of chemical synthetic procedures; and products produced by recombinant techniques from a prokaryotic or eukaryotic host, including, for example, bacterial, yeast, higher plant, insect, and mammalian cells. Depending upon the host employed in a recombinant production procedure, the polypeptides of the present invention may be glycosylated or may be non-glycosylated. In addition, polypeptides of the invention may also include an initial modified methionine residue, in some cases as a result of host-mediated processes. Thus, it is well known in the art that the N-terminal methionine encoded by the translation initiation codon generally is removed with high efficiency from any protein after translation in all eukaryotic cells. While the N-terminal methionine on most proteins also is efficiently removed in most prokaryotes, for some proteins, this prokaryotic removal process is inefficient, depending on the nature of the amino acid to which the N-terminal methionine is covalently linked.

### Uses of the Polynucleotides

Each of the polynucleotides identified herein can be used in numerous ways as reagents. The following description should be considered exemplary and utilizes known techniques.

The polynucleotides of the present invention are useful for chromosome identification. There exists an ongoing need to identify new chromosome markers, since few chromosome marking reagents, based on actual sequence data (repeat polymorphisms), are presently available. Each polynucleotide of the present invention can be used as a chromosome marker.

Briefly, sequences can be mapped to chromosomes by preparing PCR primers (preferably 15-25 bp) from the sequences shown in SEQ ID NO:X. Primers can be selected using computer analysis so that primers do not span more than one predicted exon in the genomic DNA. These primers are then used for PCR screening of somatic cell hybrids containing individual human chromosomes. Only those hybrids containing the human gene corresponding to the SEQ ID NO:X will yield an amplified fragment.

Similarly, somatic hybrids provide a rapid method of PCR mapping the polynucleotides to particular chromosomes. Three or more clones can be assigned per day using a single thermal cycler. Moreover, sublocalization of the polynucleotides can be achieved with panels of specific chromosome fragments. Other gene mapping strategies that can be used include in situ hybridization, prescreening with labeled flow-sorted chromosomes, and preselection by hybridization to construct chromosome specific-cDNA libraries.

Precise chromosomal location of the polynucleotides can also be achieved using fluorescence in situ hybridization (FISH) of a metaphase chromosomal spread. This technique uses polynucleotides as short as 500 or 600 bases; however, polynucleotides 2,000-4,000 bp are preferred. For a review of this technique, see Verma et al., "Human Chromosomes: a Manual of Basic Techniques," Pergamon Press, New York (1988).

For chromosome mapping, the polynucleotides can be used individually (to mark a single chromosome or a single site on that chromosome) or in panels (for marking multiple sites and/or multiple chromosomes). Preferred polynucleotides correspond to the noncoding regions of the cDNAs because the coding sequences are more likely conserved within gene families, thus increasing the chance of cross hybridization during chromosomal mapping.

Once a polynucleotide has been mapped to a precise chromosomal location, the physical position of the polynucleotide can be used in linkage analysis. Linkage analysis establishes coinheritance between a chromosomal location and presentation of a particular disease. (Disease mapping data are found, for example, in V. McKusick, Mendelian Inheritance in Man (available on line through Johns Hopkins University Welch Medical Library).) Assuming 1 megabase mapping resolution and one gene per 20 kb, a cDNA precisely localized to a chromosomal region associated with the disease could be one of 50-500 potential causative genes.

Thus, once coinheritance is established, differences in the polynucleotide and the corresponding gene between affected and unaffected individuals can be examined. First, visible structural alterations in the chromosomes, such as deletions or translocations, are examined in chromosome spreads or by PCR. If no structural alterations exist, the presence of point mutations are ascertained. Mutations observed in some or all affected individuals, but not in normal individuals, indicates that the mutation may cause the disease. However, complete sequencing of the polypeptide and the corresponding gene from several normal individuals is required to distinguish the mutation from a polymorphism. If a new polymorphism is identified, this polymorphic polypeptide can be used for further linkage analysis.

Furthermore, increased or decreased expression of the gene in affected individuals as compared to unaffected individuals can be assessed using polynucleotides of the present invention. Any of these alterations (altered expression, chromosomal rearrangement, or mutation) can be used as a diagnostic or prognostic marker.

In addition to the foregoing, a polynucleotide can be used to control gene expression through triple helix formation or antisense DNA or RNA. Both methods rely on binding of the polynucleotide to DNA or RNA. For these techniques, preferred polynucleotides are usually 20 to 40 bases in length and complementary to either the region of the gene involved in transcription (triple helix - see Lee et al., Nucl. Acids Res. 6:3073 (1979); Cooney et al., Science 241:456 (1988); and Dervan et al., Science 251:1360 (1991)) or to the mRNA itself (antisense - Okano, J. Neurochem. 56:560 (1991); Oligodeoxy-nucleotides as Antisense Inhibitors of Gene Expression, CRC Press, Boca Raton, FL (1988).) Triple helix formation optimally results in a shut-off of RNA transcription from DNA, while antisense RNA hybridization blocks translation of an mRNA molecule into polypeptide. Both techniques are effective in model systems, and the information disclosed herein can be used to design antisense or triple helix polynucleotides in an effort to treat disease.

Polynucleotides of the present invention are also useful in gene therapy. One goal of gene therapy is to insert a normal gene into an organism having a defective gene, in an effort to correct the genetic defect. The polynucleotides disclosed in the present invention offer a means of targeting such genetic defects in a highly accurate manner. Another goal is to insert a new gene that was not present in the host genome, thereby producing a new trait in the host cell.

The polynucleotides are also useful for identifying individuals from minute biological samples. The United States military, for example, is considering the use of restriction fragment length polymorphism (RFLP) for identification of its personnel. In this technique, an individual's genomic DNA is digested with one or more restriction enzymes, and probed on a Southern blot to yield unique bands for identifying personnel. This method does not suffer from the current limitations of "Dog Tags" which can be lost, switched, or stolen, making positive identification difficult. The polynucleotides of the present invention can be used as additional DNA markers for RFLP.

The polynucleotides of the present invention can also be used as an alternative to RFLP, by determining the actual base-by-base DNA sequence of selected portions of an individual's genome. These sequences can be used to prepare PCR primers for amplifying and isolating such selected DNA, which can then be sequenced. Using this technique, individuals can be identified because each individual will have a unique set of DNA sequences. Once an unique ID database is established for an individual, positive identification of that individual, living or dead, can be made from extremely small tissue samples.

Forensic biology also benefits from using DNA-based identification techniques as disclosed herein. DNA sequences taken from very small biological samples such as tissues, e.g., hair or skin, or body fluids, e.g., blood, saliva, semen, etc., can be amplified using PCR. In one prior art technique, gene sequences amplified from polymorphic loci, such as DQa class II HLA gene, are used in forensic biology to identify individuals. (Erlich, H., PCR Technology, Freeman and Co. (1992).) Once these specific polymorphic loci are amplified, they are digested with one or more restriction enzymes, yielding an identifying set of bands on a Southern blot probed with DNA corresponding to the DQa class II HLA gene. Similarly, polynucleotides of the present invention can be used as polymorphic markers for forensic purposes.

There is also a need for reagents capable of identifying the source of a particular tissue. Such need arises, for example, in forensics when presented with tissue of unknown origin. Appropriate reagents can comprise, for example, DNA probes or primers specific to particular tissue prepared from the sequences of the present invention. Panels of such reagents can identify tissue by species and/or by organ type. In a similar fashion, these reagents can be used to screen tissue cultures for contamination.

In the very least, the polynucleotides of the present invention can be used as molecular weight markers on Southern gels, as diagnostic probes for the presence of a specific mRNA in a particular cell type, as a probe to "subtract-out" known sequences in the process of discovering novel polynucleotides, for selecting and making oligomers for attachment to a "gene chip" or other support, to raise anti-DNA antibodies using DNA immunization techniques, and as an antigen to elicit an immune response.

### Uses of the Polypeptides

Each of the polypeptides identified herein can be used in numerous ways. The following description should be considered exemplary and utilizes known techniques.

A polypeptide of the present invention can be used to assay protein levels in a biological sample using antibody-based techniques. For example, protein expression in tissues can be studied with classical immunohistological methods. (Jalkanen, M., et al., J. Cell. Biol. 101:976-985 (1985); Jalkanen, M., et al., J. Cell. Biol. 105:3087-3096 (1987).) Other antibody-based methods useful for detecting protein gene expression include immunoassays, such as the enzyme linked immunosorbent assay (ELISA) and the radioimmunoassay (RIA). Suitable antibody assay labels are known in the art and include enzyme labels, such as, glucose oxidase, and radioisotopes, such as iodine (125I, 121I), carbon (14C), sulfur (35S), tritium (3H), indium (112In), and technetium (99mTc), and fluorescent labels, such as fluorescein and rhodamine, and biotin.

In addition to assaying secreted protein levels in a biological sample, proteins can also be detected in vivo by imaging. Antibody labels or markers for in vivo imaging of protein include those detectable by X-radiography, NMR or ESR. For X-radiography, suitable labels include radioisotopes such as barium or cesium, which emit detectable radiation but are not overtly harmful to the subject. Suitable markers for NMR and ESR include those with a detectable characteristic spin, such as deuterium, which may be incorporated into the antibody by labeling of nutrients for the relevant hybridoma.

A protein-specific antibody or antibody fragment which has been labeled with an appropriate detectable imaging moiety, such as a radioisotope (for example, 131I, 112In, 99mTc), a radio-opaque substance, or a material detectable by nuclear magnetic resonance, is introduced (for example, parenterally, subcutaneously, or intraperitoneally) into the mammal. It will be understood in the art that the size of the subject and the imaging system used will determine the quantity of imaging moiety needed to produce diagnostic images. In the case of a radioisotope moiety, for a human subject, the quantity of radioactivity injected will normally range from about 5 to 20 millicuries of 99mTc. The labeled antibody or antibody fragment will then preferentially accumulate at the location of cells which contain the specific protein. In vivo tumor imaging is described in S.W. Burchiel et al., "Immunopharmacokinetics of Radiolabeled Antibodies and Their Fragments." (Chapter 13 in Tumor Imaging: The Radiochemical Detection of Cancer, S.W. Burchiel and B. A. Rhodes, eds., Masson Publishing Inc. (1982).)

Thus, the invention provides a diagnostic method of a disorder, which involves (a) assaying the expression of a polypeptide of the present invention in cells or body fluid of an individual; (b) comparing the level of gene expression with a standard gene expression level, whereby an increase or decrease in the assayed polypeptide gene expression level compared to the standard expression level is indicative of a disorder.

Moreover, polypeptides of the present invention can be used to treat disease. For example, patients can be administered a polypeptide of the present invention in an effort to replace absent or decreased levels of the polypeptide (e.g., insulin), to supplement absent or decreased levels of a different polypeptide (e.g., hemoglobin S for hemoglobin B), to inhibit the activity of a polypeptide (e.g., an oncogene), to activate the activity of a polypeptide (e.g., by binding to a receptor), to reduce the activity of a membrane bound receptor by competing with it for free ligand (e.g., soluble TNF receptors used in reducing inflammation), or to bring about a desired response (e.g., blood vessel growth).

Similarly, antibodies directed to a polypeptide of the present invention can also be used to treat disease. For example, administration of an antibody directed to a polypeptide of the present invention can bind and reduce overproduction of the polypeptide. Similarly, administration of an antibody can activate the polypeptide, such as by binding to a polypeptide bound to a membrane (receptor).

At the very least, the polypeptides of the present invention can be used as molecular weight markers on SDS-PAGE gels or on molecular sieve gel filtration columns using methods well known to those of skill in the art. Polypeptides can also be used to raise antibodies, which in turn are used to measure protein expression from a recombinant cell, as a way of assessing transformation of the host cell. Moreover, the polypeptides of the present invention can be used to test the following biological activities.

### Biological Activities

The polynucleotides and polypeptides of the present invention can be used in assays to test for one or more biological activities. If these polynucleotides and polypeptides do exhibit activity in a particular assay, it is likely that these molecules may be involved in the diseases associated with the biological activity. Thus, the polynucleotides and polypeptides could be used to treat the associated disease.

### Immune Activity

A polypeptide or polynucleotide of the present invention may be useful in treating deficiencies or disorders of the immune system, by activating or inhibiting the proliferation, differentiation, or mobilization (chemotaxis) of immune cells. Immune cells develop through a process called hematopoiesis, producing myeloid (platelets, red blood cells, neutrophils, and macrophages) and lymphoid (B and T lymphocytes) cells from pluripotent stem cells. The etiology of these immune deficiencies or disorders may be genetic, somatic, such as cancer or some autoimmune disorders, acquired (e.g., by chemotherapy or toxins), or infectious. Moreover, a polynucleotide or polypeptide of the present invention can be used as a marker or detector of a particular immune system disease or disorder.

A polynucleotide or polypeptide of the present invention may be useful in treating or detecting deficiencies or disorders of hematopoietic cells. A polypeptide or polynucleotide of the present invention could be used to increase differentiation and proliferation of hematopoietic cells, including the pluripotent stem cells, in an effort to treat those disorders associated with a decrease in certain (or many) types hematopoietic cells. Examples of immunologic deficiency syndromes include, but are not limited to: blood protein disorders (e.g. agammaglobulinemia, dysgammaglobulinemia), ataxia telangiectasia, common variable immunodeficiency, Digeorge Syndrome, HIV infection, HTLV-BLV infection, leukocyte adhesion deficiency syndrome, lymphopenia, phagocyte bactericidal dysfunction, severe combined immunodeficiency (SCIDs), Wiskott-Aldrich Disorder, anemia, thrombocytopenia, or hemoglobinuria.

Moreover, a polypeptide or polynucleotide of the present invention could also be used to modulate hemostatic (the stopping of bleeding) or thrombolytic activity (clot formation). For example, by increasing hemostatic or thrombolytic activity, a polynucleotide or polypeptide of the present invention could be used to treat blood coagulation disorders (e.g., afibrinogenemia, factor deficiencies), blood platelet disorders (e.g. thrombocytopenia), or wounds resulting from trauma, surgery, or other causes. Alternatively, a polynucleotide or polypeptide of the present invention that can decrease hemostatic or thrombolytic activity could be used to inhibit or dissolve clotting. These molecules could be important in the treatment of heart attacks (infarction), strokes, or scarring.

A polynucleotide or polypeptide of the present invention may also be useful in treating or detecting autoimmune disorders. Many autoimmune disorders result from inappropriate recognition of self as foreign material by immune cells. This inappropriate recognition results in an immune response leading to the destruction of the host tissue. Therefore, the administration of a polypeptide or polynucleotide of the present invention that inhibits an immune response, particularly the proliferation, differentiation, or chemotaxis of T-cells, may be an effective therapy in preventing autoimmune disorders.

Examples of autoimmune disorders that can be treated or detected by the present invention include, but are not limited to: Addison's Disease, hemolytic anemia, antiphospholipid syndrome, rheumatoid arthritis, dermatitis, allergic encephalomyelitis, glomerulonephritis, Goodpasture's Syndrome, Graves' Disease, Multiple Sclerosis, Myasthenia Gravis, Neuritis, Ophthalmia, Bullous Pemphigoid, Pemphigus, Polyendocrinopathies, Purpura, Reiter's Disease, Stiff-Man Syndrome, Autoimmune Thyroiditis, Systemic Lupus Erythematosus, Autoimmune Pulmonary Inflammation, Guillain-Barre Syndrome, insulin dependent diabetes mellitis, and autoimmune inflammatory eye disease.

Similarly, allergic reactions and conditions, such as asthma (particularly allergic asthma) or other respiratory problems, may also be treated by a polypeptide or polynucleotide of the present invention. Moreover, these molecules can be used to treat anaphylaxis, hypersensitivity to an antigenic molecule, or blood group incompatibility.

A polynucleotide or polypeptide of the present invention may also be used to treat and/or prevent organ rejection or graft-versus-host disease (GVHD). Organ rejection occurs by host immune cell destruction of the transplanted tissue through an immune response. Similarly, an immune response is also involved in GVHD, but, in this case, the foreign transplanted immune cells destroy the host tissues. The administration of a polypeptide or polynucleotide of the present invention that inhibits an immune response, particularly the proliferation, differentiation, or chemotaxis of T-cells, may be an effective therapy in preventing organ rejection or GVHD.

Similarly, a polypeptide or polynucleotide of the present invention may also be used to modulate inflammation. For example, the polypeptide or polynucleotide may inhibit the proliferation and differentiation of cells involved in an inflammatory response. These molecules can be used to treat inflammatory conditions, both chronic and acute conditions, including inflammation associated with infection (e.g., septic shock, sepsis, or systemic inflammatory response syndrome (SIRS)), ischemia-reperfusion injury, endotoxin lethality, arthritis, complement-mediated hyperacute rejection, nephritis, cytokine or chemokine induced lung injury, inflammatory bowel disease, Crohn's disease, or resulting from over production of cytokines (e.g., TNF or IL-1.)

### Hyperproliferative Disorders

A polypeptide or polynucleotide can be used to treat or detect hyperproliferative disorders, including neoplasms. A polypeptide or polynucleotide of the present invention may inhibit the proliferation of the disorder through direct or indirect interactions. Alternatively, a polypeptide or polynucleotide of the present invention may proliferate other cells which can inhibit the hyperproliferative disorder.

For example, by increasing an immune response, particularly increasing antigenic qualities of the hyperproliferative disorder or by proliferating, differentiating, or mobilizing T-cells, hyperproliferative disorders can be treated. This immune response may be increased by either enhancing an existing immune response, or by initiating a new immune response. Alternatively, decreasing an immune response may also be a method of treating hyperproliferative disorders, such as a chemotherapeutic agent.

Examples of hyperproliferative disorders that can be treated or detected by a polynucleotide or polypeptide of the present invention include, but are not limited to neoplasms located in the: abdomen, bone, breast, digestive system, liver, pancreas, peritoneum, endocrine glands (adrenal, parathyroid, pituitary, testicles, ovary, thymus, thyroid), eye, head and neck, nervous (central and peripheral), lymphatic system, pelvic, skin, soft tissue, spleen, thoracic, and urogenital.

Similarly, other hyperproliferative disorders can also be treated or detected by a polynucleotide or polypeptide of the present invention. Examples of such hyperproliferative disorders include, but are not limited to: hypergammaglobulinemia, lymphoproliferative disorders, paraproteinemias, purpura, sarcoidosis, Sezary Syndrome, Waldenstron's Macroglobulinemia, Gaucher's Disease, histiocytosis, and any other hyperproliferative disease, besides neoplasia, located in an organ system listed above.

### Infectious Disease

A polypeptide or polynucleotide of the present invention can be used to treat or detect infectious agents. For example, by increasing the immune response, particularly increasing the proliferation and differentiation of B and/or T cells, infectious diseases may be treated. The immune response may be increased by either enhancing an existing immune response, or by initiating a new immune response. Alternatively, the polypeptide or polynucleotide of the present invention may also directly inhibit the infectious agent, without necessarily eliciting an immune response.

Viruses are one example of an infectious agent that can cause disease or symptoms that can be treated or detected by a polynucleotide or polypeptide of the present invention. Examples of viruses, include, but are not limited to the following DNA and RNA viral families: Arbovirus, Adenoviridae, Arenaviridae, Arterivirus, Birnaviridae, Bunyaviridae, Caliciviridae, Circoviridae, Coronaviridae, Flaviviridae, Hepadnaviridae (Hepatitis), Herpesviridae (such as, Cytomegalovirus, Herpes Simplex, Herpes Zoster), Mononegavirus (e.g., Paramyxoviridae, Morbillivirus, Rhabdoviridae), Orthomyxoviridae (e.g., Influenza), Papovaviridae, Parvoviridae, Picornaviridae, Poxviridae (such as Smallpox or Vaccinia), Reoviridae (e.g., Rotavirus), Retroviridae (HTLV-I, HTLV-II, Lentivirus), and Togaviridae (e.g., Rubivirus). Viruses falling within these families can cause a variety of diseases or symptoms, including, but not limited to: arthritis, bronchiollitis, encephalitis, eye infections (e.g., conjunctivitis, keratitis), chronic fatigue syndrome, hepatitis (A, B, C, E, Chronic Active, Delta), meningitis, opportunistic infections (e.g., AIDS), pneumonia, Burkitt's Lymphoma, chickenpox , hemorrhagic fever, Measles, Mumps, Parainfluenza, Rabies, the common cold, Polio, leukemia, Rubella, sexually transmitted diseases, skin diseases (e.g., Kaposi's, warts), and viremia. A polypeptide or polynucleotide of the present invention can be used to treat or detect any of these symptoms or diseases.

Similarly, bacterial or fungal agents that can cause disease or symptoms and that can be treated or detected by a polynucleotide or polypeptide of the present invention include, but not limited to, the following Gram-Negative and Gram-positive bacterial families and fungi: Actinomycetales (e.g., Corynebacterium, Mycobacterium, Norcardia), Aspergillosis, Bacillaceae (e.g., Anthrax, Clostridium), Bacteroidaceae, Blastomycosis, Bordetella, Borrelia, Brucellosis, Candidiasis, Campylobacter, Coccidioidomycosis, Cryptococcosis, Dermatocycoses, Enterobacteriaceae (Klebsiella, Salmonella, Serratia, Yersinia), Erysipelothrix, Helicobacter, Legionellosis, Leptospirosis, Listeria, Mycoplasmatales, Neisseriaceae (e.g., Acinetobacter, Gonorrhea, Menigococcal), Pasteurellacea Infections (e.g., Actinobacillus, Heamophilus, Pasteurella), Pseudomonas, Rickettsiaceae, Chlamydiaceae, Syphilis, and Staphylococcal. These bacterial or fungal families can cause the following diseases or symptoms, including, but not limited to: bacteremia, endocarditis, eye infections (conjunctivitis, tuberculosis, uveitis), gingivitis, opportunistic infections (e.g., AIDS related infections), paronychia, prosthesis-related infections, Reiter's Disease, respiratory tract infections, such as Whooping Cough or Empyema, sepsis, Lyme Disease, Cat-Scratch Disease, Dysentery, Paratyphoid Fever, food poisoning, Typhoid, pneumonia, Gonorrhea, meningitis, Chlamydia, Syphilis, Diphtheria, Leprosy, Paratuberculosis, Tuberculosis, Lupus, Botulism, gangrene, tetanus, impetigo, Rheumatic Fever, Scarlet Fever, sexually transmitted diseases; skin diseases (e.g., cellulitis, dermatocycoses), toxemia, urinary tract infections, wound infections. A polypeptide or polynucleotide of the present invention can be used to treat or detect any of these symptoms or diseases.

Moreover, parasitic agents causing disease or symptoms that can be treated or detected by a polynucleotide or polypeptide of the present invention include, but not limited to, the following families: Amebiasis, Babesiosis, Coccidiosis, Cryptosporidiosis, Dientamoebiasis, Dourine, Ectoparasitic, Giardiasis, Helminthiasis, Leishmaniasis, Theileriasis, Toxoplasmosis, Trypanosomiasis, and Trichomonas. These parasites can cause a variety of diseases or symptoms, including, but not limited to: Scabies, Trombiculiasis, eye infections, intestinal disease (e.g., dysentery, giardiasis), liver disease, lung disease, opportunistic infections (e.g., AIDS related), Malaria, pregnancy complications, and toxoplasmosis. A polypeptide or polynucleotide of the present invention can be used to treat or detect any of these symptoms or diseases.

Preferably, treatment using a polypeptide or polynucleotide of the present invention could either be by administering an effective amount of a polypeptide to the patient, or by removing cells from the patient, supplying the cells with a polynucleotide of the present invention, and returning the engineered cells to the patient (ex vivo therapy). Moreover, the polypeptide or polynucleotide of the present invention can be used as an antigen in a vaccine to raise an immune response against infectious disease.

### Regeneration

A polynucleotide or polypeptide of the present invention can be used to differentiate, proliferate, and attract cells, leading to the regeneration of tissues. (See, Science 276:59-87 (1997).) The regeneration of tissues could be used to repair, replace, or protect tissue damaged by congenital defects, trauma (wounds, burns, incisions, or ulcers), age, disease (e.g. osteoporosis, osteocarthritis, periodontal disease, liver failure), surgery, including cosmetic plastic surgery, fibrosis, reperfusion injury, or systemic cytokine damage.

Tissues that could be regenerated using the present invention include organs (e.g., pancreas, liver, intestine, kidney, skin, endothelium), muscle (smooth, skeletal or cardiac), vascular (including vascular endothelium), nervous, hematopoietic, and skeletal (bone, cartilage, tendon, and ligament) tissue. Preferably, regeneration occurs without or decreased scarring. Regeneration also may include angiogenesis.

Moreover, a polynucleotide or polypeptide of the present invention may increase regeneration of tissues difficult to heal. For example, increased tendon/ligament regeneration would quicken recovery time after damage. A polynucleotide or polypeptide of the present invention could also be used prophylactically in an effort to avoid damage. Specific diseases that could be treated include of tendinitis, carpal tunnel syndrome, and other tendon or ligament defects. A further example of tissue regeneration of non-healing wounds includes pressure ulcers, ulcers associated with vascular insufficiency, surgical, and traumatic wounds.

Similarly, nerve and brain tissue could also be regenerated by using a polynucleotide or polypeptide of the present invention to proliferate and differentiate nerve cells. Diseases that could be treated using this method include central and peripheral nervous system diseases, neuropathies, or mechanical and traumatic disorders (e.g., spinal cord disorders, head trauma, cerebrovascular disease, and stoke). Specifically, diseases associated with peripheral nerve injuries, peripheral neuropathy (e.g., resulting from chemotherapy or other medical therapies), localized neuropathies, and central nervous system diseases (e.g., Alzheimer's disease, Parkinson's disease, Huntington's disease, amyotrophic lateral sclerosis, and Shy-Drager syndrome), could all be treated using the polynucleotide or polypeptide of the present invention.

### Chemotaxis

A polynucleotide or polypeptide of the present invention may have chemotaxis activity. A chemotaxic molecule attracts or mobilizes cells (e.g., monocytes, fibroblasts, neutrophils, T-cells, mast cells, eosinophils, epithelial and/or endothelial cells) to a particular site in the body, such as inflammation, infection, or site of hyperproliferation. The mobilized cells can then fight off and/or heal the particular trauma or abnormality.

A polynucleotide or polypeptide of the present invention may increase chemotaxic activity of particular cells. These chemotactic molecules can then be used to treat inflammation, infection, hyperproliferative disorders, or any immune system disorder by increasing the number of cells targeted to a particular location in the body. For example, chemotaxic molecules can be used to treat wounds and other trauma to tissues by attracting immune cells to the injured location. Chemotactic molecules of the present invention can also attract fibroblasts, which can be used to treat wounds.

It is also contemplated that a polynucleotide or polypeptide of the present invention may inhibit chemotactic activity. These molecules could also be used to treat disorders. Thus, a polynucleotide or polypeptide of the present invention could be used as an inhibitor of chemotaxis.

### Binding Activity

A polypeptide of the present invention may be used to screen for molecules that bind to the polypeptide or for molecules to which the polypeptide binds. The binding of the polypeptide and the molecule may activate (agonist), increase, inhibit (antagonist), or decrease activity of the polypeptide or the molecule bound. Examples of such molecules include antibodies, oligonucleotides, proteins (e.g., receptors),or small molecules.

Preferably, the molecule is closely related to the natural ligand of the polypeptide, e.g., a fragment of the ligand, or a natural substrate, a ligand, a structural or functional mimetic. (See, Coligan et al., Current Protocols in Immunology 1(2):Chapter 5 (1991).) Similarly, the molecule can be closely related to the natural receptor to which the polypeptide binds, or at least, a fragment of the receptor capable of being bound by the polypeptide (e.g., active site). In either case, the molecule can be rationally designed using known techniques.

Preferably, the screening for these molecules involves producing appropriate cells which express the polypeptide, either as a secreted protein or on the cell membrane. Preferred cells include cells from mammals, yeast, Drosophila, or *E*. *coli*. Cells expressing the polypeptide (or cell membrane containing the expressed polypeptide) are then preferably contacted with a test compound potentially containing the molecule to observe binding, stimulation, or inhibition of activity of either the polypeptide or the molecule.

The assay may simply test binding of a candidate compound to the polypeptide, wherein binding is detected by a label, or in an assay involving competition with a labeled competitor. Further, the assay may test whether the candidate compound results in a signal generated by binding to the polypeptide.

Alternatively, the assay can be carried out using cell-free preparations, polypeptide/molecule affixed to a solid support, chemical libraries, or natural product mixtures. The assay may also simply comprise the steps of mixing a candidate compound with a solution containing a polypeptide, measuring polypeptide/molecule activity or binding, and comparing the polypeptide/molecule activity or binding to a standard.

Preferably, an ELISA assay can measure polypeptide level or activity in a sample (e.g., biological sample) using a monoclonal or polyclonal antibody. The antibody can measure polypeptide level or activity by either binding, directly or indirectly, to the polypeptide or by competing with the polypeptide for a substrate.

All of these above assays can be used as diagnostic or prognostic markers. The molecules discovered using these assays can be used to treat disease or to bring about a particular result in a patient (e.g., blood vessel growth) by activating or inhibiting the polypeptide/molecule. Moreover, the assays can discover agents which may inhibit or enhance the production of the polypeptide from suitably manipulated cells or tissues.

Therefore, the invention includes a method of identifying compounds which bind to a polypeptide of the invention comprising the steps of: (a) incubating a candidate binding compound with a polypeptide of the invention; and (b) determining if binding has occurred. Moreover, the invention includes a method of identifying agonists/antagonists comprising the steps of: (a) incubating a candidate compound with a polypeptide of the invention, (b) assaying a biological activity , and (b) determining if a biological activity of the polypeptide has been altered.

### Other Activities

A polypeptide or polynucleotide of the present invention may also increase or decrease the differentiation or proliferation of embryonic stem cells, besides, as discussed above, hematopoietic lineage.

A polypeptide or polynucleotide of the present invention may also be used to modulate mammalian characteristics, such as body height, weight, hair color, eye color, skin, percentage of adipose tissue, pigmentation, size, and shape (e.g., cosmetic surgery). Similarly, a polypeptide or polynucleotide of the present invention may be used to modulate mammalian metabolism affecting catabolism, anabolism, processing, utilization, and storage of energy.

A polypeptide or polynucleotide of the present invention may be used to change a mammal's mental state or physical state by influencing biorhythms, caricadic rhythms, depression (including depressive disorders), tendency for violence, tolerance for pain, reproductive capabilities (preferably by Activin or Inhibin-like activity), hormonal or endocrine levels, appetite, libido, memory, stress, or other cognitive qualities.

A polypeptide or polynucleotide of the present invention may also be used as a food additive or preservative, such as to increase or decrease storage capabilities, fat content, lipid, protein, carbohydrate, vitamins, minerals, cofactors or other nutritional components.

### Other Preferred Embodiments

Other preferred embodiments of the claimed invention include an isolated nucleic acid molecule comprising a nucleotide sequence which is at least 95% identical to a sequence of at least about 50 contiguous nucleotides in the nucleotide sequence of SEQ ID NO:X wherein X is any integer as defined in Table 1.

Also preferred is a nucleic acid molecule wherein said sequence of contiguous nucleotides is included in the nucleotide sequence of SEQ ID NO:X in the range of positions beginning with the nucleotide at about the position of the 5' Nucleotide of the Clone Sequence and ending with the nucleotide at about the position of the 3' Nucleotide of the Clone Sequence as defined for SEQ ID NO:X in Table 1.

Also preferred is a nucleic acid molecule wherein said sequence of contiguous nucleotides is included in the nucleotide sequence of SEQ ID NO:X in the range of positions beginning with the nucleotide at about the position of the 5' Nucleotide of the Start Codon and ending with the nucleotide at about the position of the 3' Nucleotide of the Clone Sequence as defined for SEQ ID NO:X in Table 1.

Similarly preferred is a nucleic acid molecule wherein said sequence of contiguous nucleotides is included in the nucleotide sequence of SEQ ID NO:X in the range of positions beginning with the nucleotide at about the position of the 5' Nucleotide of the First Amino Acid of the Signal Peptide and ending with the nucleotide at about the position of the 3' Nucleotide of the Clone Sequence as defined for SEQ ID NO:X in Table 1.

Also preferred is an isolated nucleic acid molecule comprising a nucleotide sequence which is at least 95% identical to a sequence of at least about 150 contiguous nucleotides in the nucleotide sequence of SEQ ID NO:X.

Further preferred is an isolated nucleic acid molecule comprising a nucleotide sequence which is at least 95% identical to a sequence of at least about 500 contiguous nucleotides in the nucleotide sequence of SEQ ID NO:X.

A further preferred embodiment is a nucleic acid molecule comprising a nucleotide sequence which is at least 95% identical to the nucleotide sequence of SEQ ID NO:X beginning with the nucleotide at about the position of the 5' Nucleotide of the First Amino Acid of the Signal Peptide and ending with the nucleotide at about the position of the 3' Nucleotide of the Clone Sequence as defined for SEQ ID NO:X in Table 1.

A further preferred embodiment is an isolated nucleic acid molecule comprising a nucleotide sequence which is at least 95% identical to the complete nucleotide sequence of SEQ ID NO:X.

Also preferred is an isolated nucleic acid molecule which hybridizes under stringent hybridization conditions to a nucleic acid molecule, wherein said nucleic acid molecule which hybridizes does not hybridize under stringent hybridization conditions to a nucleic acid molecule having a nucleotide sequence consisting of only A residues or of only T residues.

Also preferred is a composition of matter comprising a DNA molecule which comprises a human cDNA clone identified by a cDNA Clone Identifier in Table 1, which DNA molecule is contained in the material deposited with the American Type Culture Collection and given the ATCC Deposit Number shown in Table 1 for said cDNA Clone Identifier.

Also preferred is an isolated nucleic acid molecule comprising a nucleotide sequence which is at least 95% identical to a sequence of at least 50 contiguous nucleotides in the nucleotide sequence of a human cDNA clone identified by a cDNA Clone Identifier in Table 1, which DNA molecule is contained in the deposit given the ATCC Deposit Number shown in Table 1.

Also preferred is an isolated nucleic acid molecule, wherein said sequence of at least 50 contiguous nucleotides is included in the nucleotide sequence of the complete open reading frame sequence encoded by said human cDNA clone.

Also preferred is an isolated nucleic acid molecule comprising a nucleotide sequence which is at least 95% identical to sequence of at least 150 contiguous nucleotides in the nucleotide sequence encoded by said human cDNA clone.

A further preferred embodiment is an isolated nucleic acid molecule comprising a nucleotide sequence which is at least 95% identical to sequence of at least 500 contiguous nucleotides in the nucleotide sequence encoded by said human cDNA clone.

A further preferred embodiment is an isolated nucleic acid molecule comprising a nucleotide sequence which is at least 95% identical to the complete nucleotide sequence encoded by said human cDNA clone.

A further preferred embodiment is a method for detecting in a biological sample a nucleic acid molecule comprising a nucleotide sequence which is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1; which method comprises a step of comparing a nucleotide sequence of at least one nucleic acid molecule in said sample with a sequence selected from said group and determining whether the sequence of said nucleic acid molecule in said sample is at least 95% identical to said selected sequence.

Also preferred is the above method wherein said step of comparing sequences comprises determining the extent of nucleic acid hybridization between nucleic acid molecules in said sample and a nucleic acid molecule comprising said sequence selected from said group. Similarly, also preferred is the above method wherein said step of comparing sequences is performed by comparing the nucleotide sequence determined from a nucleic acid molecule in said sample with said sequence selected from said group. The nucleic acid molecules can comprise DNA molecules or RNA molecules.

A further preferred embodiment is a method for identifying the species, tissue or cell type of a biological sample which method comprises a step of detecting nucleic acid molecules in said sample, if any, comprising a nucleotide sequence that is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1.

The method for identifying the species, tissue or cell type of a biological sample can comprise a step of detecting nucleic acid molecules comprising a nucleotide sequence in a panel of at least two nucleotide sequences, wherein at least one sequence in said panel is at least 95% identical to a sequence of at least 50 contiguous nucleotides in a sequence selected from said group.

Also preferred is a method for diagnosing in a subject a pathological condition associated with abnormal structure or expression of a gene encoding a secreted protein identified in Table 1, which method comprises a step of detecting in a biological sample obtained from said subject nucleic acid molecules, if any, comprising a nucleotide sequence that is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1.

The method for diagnosing a pathological condition can comprise a step of detecting nucleic acid molecules comprising a nucleotide sequence in a panel of at least two nucleotide sequences, wherein at least one sequence in said panel is at least 95% identical to a sequence of at least 50 contiguous nucleotides in a sequence selected from said group.

Also preferred is a composition of matter comprising isolated nucleic acid molecules wherein the nucleotide sequences of said nucleic acid molecules comprise a panel of at least two nucleotide sequences, wherein at least one sequence in said panel is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1. The nucleic acid molecules can comprise DNA molecules or RNA molecules.

Also preferred is an isolated polypeptide comprising an amino acid sequence at least 90% identical to a sequence of at least about 10 contiguous amino acids in the amino acid sequence of SEQ ID NO:Y wherein Y is any integer as defined in Table 1.

Also preferred is a polypeptide, wherein said sequence of contiguous amino acids is included in the amino acid sequence of SEQ ID NO:Y in the range of positions beginning with the residue at about the position of the First Amino Acid of the Secreted Portion and ending with the residue at about the Last Amino Acid of the Open Reading Frame as set forth for SEQ ID NO:Y in Table 1.

Also preferred is an isolated polypeptide comprising an amino acid sequence at least 95% identical to a sequence of at least about 30 contiguous amino acids in the amino acid sequence of SEQ ID NO:Y.

Further preferred is an isolated polypeptide comprising an amino acid sequence at least 95% identical to a sequence of at least about 100 contiguous amino acids in the amino acid sequence of SEQ ID NO:Y.

Further preferred is an isolated polypeptide comprising an amino acid sequence at least 95% identical to the complete amino acid sequence of SEQ ID NO:Y.

Further preferred is an isolated polypeptide comprising an amino acid sequence at least 90% identical to a sequence of at least about 10 contiguous amino acids in the complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is a polypeptide wherein said sequence of contiguous amino acids is included in the amino acid sequence of a secreted portion of the secreted protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to a sequence of at least about 30 contiguous amino acids in the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to a sequence of at least about 100 contiguous amino acids in the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Further preferred is an isolated antibody which binds specifically to a polypeptide comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1.

Further preferred is a method for detecting in a biological sample a polypeptide comprising an amino acid sequence which is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1; which method comprises a step of comparing an amino acid sequence of at least one polypeptide molecule in said sample with a sequence selected from said group and determining whether the sequence of said polypeptide molecule in said sample is at least 90% identical to said sequence of at least 10 contiguous amino acids.

Also preferred is the above method wherein said step of comparing an amino acid sequence of at least one polypeptide molecule in said sample with a sequence selected from said group comprises determining the extent of specific binding of polypeptides in said sample to an antibody which binds specifically to a polypeptide comprising an amino acid sequence that is at least 90% identical to a sequence of at least 10 contiguous amino acids in a sequence selected from the group consisting of: an amino acid sequence of SEQ ID NO: Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1.

Also preferred is the above method wherein said step of comparing sequences is performed by comparing the amino acid sequence determined from a polypeptide molecule in said sample with said sequence selected from said group.

Also preferred is a method for identifying the species, tissue or cell type of a biological sample which method comprises a step of detecting polypeptide molecules in said sample, if any, comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is the above method for identifying the species, tissue or cell type of a biological sample, which method comprises a step of detecting polypeptide molecules comprising an amino acid sequence in a panel of at least two amino acid sequences, wherein at least one sequence in said panel is at least 90% identical to a sequence of at least 10 contiguous amino acids in a sequence selected from the above group.

Also preferred is a method for diagnosing in a subject a pathological condition associated with abnormal structure or expression of a gene encoding a secreted protein identified in Table 1, which method comprises a step of detecting in a biological sample obtained from said subject polypeptide molecules comprising an amino acid sequence in a panel of at least two amino acid sequences, wherein at least one sequence in said panel is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

In any of these methods, the step of detecting said polypeptide molecules includes using an antibody.

Also preferred is an isolated nucleic acid molecule comprising a nucleotide sequence which is at least 95% identical to a nucleotide sequence encoding a polypeptide wherein said polypeptide comprises an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is an isolated nucleic acid molecule, wherein said nucleotide sequence encoding a polypeptide has been optimized for expression of said polypeptide in a prokaryotic host.

Also preferred is an isolated nucleic acid molecule, wherein said polypeptide comprises an amino acid sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Further preferred is a method of making a recombinant vector comprising inserting any of the above isolated nucleic acid molecule into a vector. Also preferred is the recombinant vector produced by this method. Also preferred is a method of making a recombinant host cell comprising introducing the vector into a host cell, as well as the recombinant host cell produced by this method.

Also preferred is a method of making an isolated polypeptide comprising culturing this recombinant host cell under conditions such that said polypeptide is expressed and recovering said polypeptide. Also preferred is this method of making an isolated polypeptide, wherein said recombinant host cell is a eukaryotic cell and said polypeptide is a secreted portion of a human secreted protein comprising an amino acid sequence selected from the group consisting of: an amino acid sequence of SEQ ID NO:Y beginning with the residue at the position of the First Amino Acid of the Secreted Portion of SEQ ID NO:Y wherein Y is an integer set forth in Table 1 and said position of the First Amino Acid of the Secreted Portion of SEQ ID NO:Y is defined in Table 1; and an amino acid sequence of a secreted portion of a protein encoded by a human cDNA clone identified by a cDNA Clone Identifier in Table 1 and contained in the deposit with the ATCC Deposit Number shown for said cDNA clone in Table 1. The isolated polypeptide produced by this method is also preferred.

Also preferred is a method of treatment of an individual in need of an increased level of a secreted protein activity, which method comprises administering to such an individual a pharmaceutical composition comprising an amount of an isolated polypeptide, polynucleotide, or antibody of the claimed invention effective to increase the level of said protein activity in said individual.

Having generally described the invention, the same will be more readily understood by reference to the following examples, which are provided by way of illustration and are not intended as limiting.

### Examples

### Example 1: Isolation of a Selected cDNA Clone From the Deposited Sample

Each cDNA clone in a cited ATCC deposit is contained in a plasmid vector. Table 1 identifies the vectors used to construct the cDNA library from which each clone was isolated. In many cases, the vector used to construct the library is a phage vector from which a plasmid has been excised. The table immediately below correlates the related plasmid for each phage vector used in constructing the cDNA library. For example, where a particular clone is identified in Table 1 as being isolated in the vector "Lambda Zap," the corresponding deposited clone is in "pBluescript."

| Vector Used to Construct Library | Corresponding Deposited Plasmid |
|---|---|
| Lambda Zap | pBluescript (pBS) |
| Uni-Zap XR | pBluescript (pBS) |
| Zap Express | pBK |
| lafmid BA | plafmid BA |
| pSport1 | pSport1 |
| pCMVSport 2.0 | pCMVSport 2.0 |
| pCMVSport 3.0 | pCMVSport 3.0 |
| pCR®2.1 | pCR®2.1 |

Vectors Lambda Zap (U.S. Patent Nos. 5,128,256 and 5,286,636), Uni-Zap XR (U.S. Patent Nos. 5,128, 256 and 5,286,636), Zap Express (U.S. Patent Nos. 5,128,256 and 5,286,636), pBluescript (pBS) (Short, J. M. et al., Nucleic Acids Res. 16:7583-7600 (1988); Alting-Mees, M. A. and Short, J. M., Nucleic Acids Res. 17:9494 (1989)) and pBK (Alting-Mees, M. A. et al., Strategies 5:58-61 (1992)) are commercially available from Stratagene Cloning Systems, Inc., 11011 N. Torrey Pines Road, La Jolla, CA, 92037. pBS contains an ampicillin resistance gene and pBK contains a neomycin resistance gene. Both can be transformed into E. coli strain XL-1 Blue, also available from Stratagene. pBS comes in 4 forms SK+, SK-, KS+ and KS. The S and K refers to the orientation of the polylinker to the T7 and T3 primer sequences which flank the polylinker region ("S" is for SacI and "K" is for KpnI which are the first sites on each respective end of the linker). "+" or "-" refer to the orientation of the f1 origin of replication ("ori"), such that in one orientation, single stranded rescue initiated from the f1 ori generates sense strand DNA and in the other, antisense.

Vectors pSport1, pCMVSport 2.0 and pCMVSport 3.0, were obtained from Life Technologies, Inc., P. O. Box 6009, Gaithersburg, MD 20897. All Sport vectors contain an ampicillin resistance gene and may be transformed into E. coli strain DH10B, also available from Life Technologies. (See, for instance, Gruber, C. E., et al., Focus 15:59 (1993).) Vector lafmid BA (Bento Scares, Columbia University, NY) contains an ampicillin resistance gene and can be transformed into E. coli strain XL-1 Blue. Vector pCR®2.1, which is available from Invitrogen, 1600 Faraday Avenue, Carlsbad, CA 92008, contains an ampicillin resistance gene and may be transformed into E. coli strain DH10B, available from Life Technologies. (See, for instance, Clark, J. M., Nuc. Acids Res. 16:9677-9686 (1988) and Mead, D. et al., Bio/Technology 9: (1991).) Preferably, a polynucleotide of the present invention does not comprise the phage vector sequences identified for the particular clone in Table 1, as well as the corresponding plasmid vector sequences designated above.

The deposited material in the sample assigned the ATCC Deposit Number cited in Table 1 for any given cDNA clone also may contain one or more additional plasmids, each comprising a cDNA clone different from that given clone. Thus, deposits sharing the same ATCC Deposit Number contain at least a plasmid for each cDNA clone identified in Table 1. Typically, each ATCC deposit sample cited in Table 1 comprises a mixture of approximately equal amounts (by weight) of about 50 plasmid DNAs, each containing a different cDNA clone; but such a deposit sample may include plasmids for more or less than 50 cDNA clones, up to about 500 cDNA clones.

Two approaches can be used to isolate a particular clone from the deposited sample of plasmid DNAs cited for that clone in Table 1. First, a plasmid is directly isolated by screening the clones using a polynucleotide probe corresponding to SEQ ID NO:X.

Particularly, a specific polynucleotide with 30-40 nucleotides is synthesized using an Applied Biosystems DNA synthesizer according to the sequence reported. The oligonucleotide is labeled, for instance, with ³²P-γ-ATP using T4 polynucleotide kinase and purified according to routine methods. (E.g., Maniatis et al., Molecular Cloning: A Laboratory Manual, Cold Spring Harbor Press, Cold Spring, NY (1982).) The plasmid mixture is transformed into a suitable host, as indicated above (such as XL-1 Blue (Stratagene)) using techniques known to those of skill in the art, such as those provided by the vector supplier or in related publications or patents cited above. The transformants are plated on 1.5% agar plates (containing the appropriate selection agent, e.g., ampicillin) to a density of about 150 transformants (colonies) per plate. These plates are screened using Nylon membranes according to routine methods for bacterial colony screening (e.g., Sambrook et al., Molecular Cloning: A Laboratory Manual, 2nd Edit., (1989), Cold Spring Harbor Laboratory Press, pages 1.93 to 1.104), or other techniques known to those of skill in the art.

Alternatively, two primers of 17-20 nucleotides derived from both ends of the SEQ ID NO:X (i.e., within the region of SEQ ID NO:X bounded by the 5' NT and the 3' NT of the clone defined in Table 1) are synthesized and used to amplify the desired cDNA using the deposited cDNA plasmid as a template. The polymerase chain reaction is carried out under routine conditions, for instance, in 25 µl of reaction mixture with 0.5 ug of the above cDNA template. A convenient reaction mixture is 1.5-5 mM MgCl₂, 0.01% (w/v) gelatin, 20µM each of dATP, dCTP, dGTP, dTTP, 25 pmol of each primer and 0.25 Unit of Taq polymerase. Thirty five cycles of PCR (denaturation at 94°C for 1 min; annealing at 55°C for 1 min; elongation at 72°C for 1 min) are performed with a Perkin-Elmer Cetus automated thermal cycler. The amplified product is analyzed by agarose gel electrophoresis and the DNA band with expected molecular weight is excised and purified. The PCR product is verified to be the selected sequence by subcloning and sequencing the DNA product.

Several methods are available for the identification of the 5' or 3' non-coding portions of a gene which may not be present in the deposited clone. These methods include but are not limited to, filter probing, clone enrichment using specific probes, and protocols similar or identical to 5' and 3' "RACE" protocols which are well known in the art. For instance, a method similar to 5' RACE is available for generating the missing 5' end of a desired full-length transcript. (Fromont-Racine et al., Nucleic Acids Res. 21(7):1683-1684 (1993).)

Briefly, a specific RNA oligonucleotide is ligated to the 5' ends of a population of RNA presumably containing full-length gene RNA transcripts. A primer set containing a primer specific to the ligated RNA oligonucleotide and a primer specific to a known sequence of the gene of interest is used to PCR amplify the 5' portion of the desired full-length gene. This amplified product may then be sequenced and used to generate the full length gene.

This above method starts with total RNA isolated from the desired source, although poly-A+ RNA can be used. The RNA preparation can then be treated with phosphatase if necessary to eliminate 5' phosphate groups on degraded or damaged RNA which may interfere with the later RNA ligase step. The phosphatase should then be inactivated and the RNA treated with tobacco acid pyrophosphatase in order to remove the cap structure present at the 5' ends of messenger RNAs. This reaction leaves a 5' phosphate group at the 5' end of the cap cleaved RNA which can then be ligated to an RNA oligonucleotide using T4 RNA ligase.

This modified RNA preparation is used as a template for first strand cDNA synthesis using a gene specific oligonucleotide. The first strand synthesis reaction is used as a template for PCR amplification of the desired 5' end using a primer specific to the ligated RNA oligonucleotide and a primer specific to the known sequence of the gene of interest. The resultant product is then sequenced and analyzed to confirm that the 5' end sequence belongs to the desired gene.

### Example 2: Isolation of Genomic Clones Corresponding to a Polynucleotide

A human genomic P1 library (Genomic Systems, Inc.) is screened by PCR using primers selected for the cDNA sequence corresponding to SEQ ID NO:X., according to the method described in Example 1. (See also, Sambrook.)

### Example 3: Tissue Distribution of Polypeptide

Tissue distribution of mRNA expression of polynucleotides of the present invention is determined using protocols for Northern blot analysis, described by, among others, Sambrook et al. For example, a cDNA probe produced by the method described in Example 1 is labeled with P³² using the rediprime™ DNA labeling system (Amersham Life Science), according to manufacturer's instructions. After labeling, the probe is purified using CHROMA SPIN-100™ column (Clontech Laboratories, Inc.), according to manufacturer's protocol number PT1200-1. The purified labeled probe is then used to examine various human tissues for mRNA expression.

Multiple Tissue Northern (MTN) blots containing various human tissues (H) or human immune system tissues (IM) (Clontech) are examined with the labeled probe using ExpressHyb™ hybridization solution (Clontech) according to manufacturer's protocol number PT1190-1. Following hybridization and washing, the blots are mounted and exposed to film at -70°C overnight, and the films developed according to standard procedures.

### Example 4: Chromosomal Mapping of the Polynucleotides

An oligonucleotide primer set is designed according to the sequence at the 5' end of SEQ ID NO:X. This primer preferably spans about 100 nucleotides. This primer set is then used in a polymerase chain reaction under the following set of conditions : 30 seconds, 95°C; 1 minute, 56°C; 1 minute, 70°C. This cycle is repeated 32 times followed by one 5 minute cycle at 70°C. Human, mouse, and hamster DNA is used as template in addition to a somatic cell hybrid panel containing individual chromosomes or chromosome fragments (Bios, Inc). The reactions is analyzed on either 8% polyacrylamide gels or 3.5 % agarose gels. Chromosome mapping is determined by the presence of an approximately 100 bp PCR fragment in the particular somatic cell hybrid.

### Example 5: Bacterial Expression of a Polypeptide

A polynucleotide encoding a polypeptide of the present invention is amplified using PCR oligonucleotide primers corresponding to the 5' and 3' ends of the DNA sequence, as outlined in Example 1, to synthesize insertion fragments. The primers used to amplify the cDNA insert should preferably contain restriction sites, such as BamHI and XbaI, at the 5' end of the primers in order to clone the amplified product into the expression vector. For example, BamHI and XbaI correspond to the restriction enzyme sites on the bacterial expression vector pQE-9. (Qiagen, Inc., Chatsworth, CA). This plasmid vector encodes antibiotic resistance (Amp^{r}), a bacterial origin of replication (ori), an IPTG-regulatable promoter/operator (P/O), a ribosome binding site (RBS), a 6-histidine tag (6-His), and restriction enzyme cloning sites.

The pQE-9 vector is digested with BamHI and Xbal and the amplified fragment is ligated into the pQE-9 vector maintaining the reading frame initiated at the bacterial RBS. The ligation mixture is then used to transform the E. coli strain M 15/rep4 (Qiagen, Inc.) which contains multiple copies of the plasmid pREP4, which expresses the lacI repressor and also confers kanamycin resistance (Kan^{r}). Transformants are identified by their ability to grow on LB plates and ampicillin/kanamycin resistant colonies are selected. Plasmid DNA is isolated and confirmed by restriction analysis.

Clones containing the desired constructs are grown overnight (O/N) in liquid culture in LB media supplemented with both Amp (100 ug/ml) and Kan (25 ug/ml). The O/N culture is used to inoculate a large culture at a ratio of 1:100 to 1:250. The cells are grown to an optical density 600 (O.D.⁶⁰⁰) of between 0.4 and 0.6. IPTG (Isopropyl-B-D-thiogalacto pyranoside) is then added to a final concentration of 1 mM. IPTG induces by inactivating the lacI repressor, clearing the P/O leading to increased gene expression.

Cells are grown for an extra 3 to 4 hours. Cells are then harvested by centrifugation (20 mins at 6000Xg). The cell pellet is solubilized in the chaotropic agent 6 Molar Guanidine HCl by stirring for 3-4 hours at 4°C. The cell debris is removed by centrifugation, and the supernatant containing the polypeptide is loaded onto a nickel-nitrilo-tri-acetic acid ("Ni-NTA") affinity resin column (available from QIAGEN, Inc., *supra*). Proteins with a 6 x His tag bind to the Ni-NTA resin with high affinity and can be purified in a simple one-step procedure (for details see: The QIAexpressionist (1995) QIAGEN, Inc., *supra*).

Briefly, the supernatant is loaded onto the column in 6 M guanidine-HCl, pH 8, the column is first washed with 10 volumes of 6 M guanidine-HCl, pH 8, then washed with 10 volumes of 6 M guanidine-HCl pH 6, and finally the polypeptide is eluted with 6 M guanidine-HCl, pH 5.

The purified protein is then renatured by dialyzing it against phosphate-buffered saline (PBS) or 50 mM Na-acetate, pH 6 buffer plus 200 mM NaCl. Alternatively, the protein can be successfully refolded while immobilized on the Ni-NTA column. The recommended conditions are as follows: renature using a linear 6M-1M urea gradient in 500 mM NaCl, 20% glycerol, 20 mM Tris/HCl pH 7.4, containing protease inhibitors. The renaturation should be performed over a period of 1.5 hours or more. After renaturation the proteins are eluted by the addition of 250 mM immidazole. Immidazole is removed by a final dialyzing step against PBS or 50 mM sodium acetate pH 6 buffer plus 200 mM NaCl. The purified protein is stored at 4° C or frozen at -80° C.

In addition to the above expression vector, the present invention further includes an expression vector comprising phage operator and promoter elements operatively linked to a polynucleotide of the present invention, called pHE4a. (ATCC Accession Number 209645, deposited on February 25, 1998.) This vector contains: 1) a neomycinphosphotransferase gene as a selection marker, 2) an E. coli origin of replication, 3) a T5 phage promoter sequence, 4) two lac operator sequences, 5) a Shine-Delgarno sequence, and 6) the lactose operon repressor gene (lacIq). The origin of replication (oriC) is derived from pUC19 (LTI, Gaithersburg, MD). The promoter sequence and operator sequences are made synthetically.

DNA can be inserted into the pHEa by restricting the vector with NdeI and XbaI, BamHI, XhoI, or Asp718, running the restricted product on a gel, and isolating the larger fragment (the stuffer fragment should be about 310 base pairs). The DNA insert is generated according to the PCR protocol described in Example 1, using PCR primers having restriction sites for NdeI (5' primer) and XbaI, BamHI, XhoI, or Asp718 (3' primer). The PCR insert is gel purified and restricted with compatible enzymes. The insert and vector are ligated according to standard protocols.

The engineered vector could easily be substituted in the above protocol to express protein in a bacterial system.

### Example 6: Purification of a Polypeptide from an Inclusion Body

The following alternative method can be used to purify a polypeptide expressed in *E coli* when it is present in the form of inclusion bodies. Unless otherwise specified, all of the following steps are conducted at 4-10°C.

Upon completion of the production phase of the *E*. *coli* fermentation, the cell culture is cooled to 4-10°C and the cells harvested by continuous centrifugation at 15,000 rpm (Heraeus Sepatech). On the basis of the expected yield of protein per unit weight of cell paste and the amount of purified protein required, an appropriate amount of cell paste, by weight, is suspended in a buffer solution containing 100 mM Tris, 50 mM EDTA, pH 7.4. The cells are dispersed to a homogeneous suspension using a high shear mixer.

The cells are then lysed by passing the solution through a microfluidizer (Microfuidics, Corp. or APV Gaulin, Inc.) twice at 4000-6000 psi. The homogenate is then mixed with NaCl solution to a final concentration of 0.5 M NaCl, followed by centrifugation at 7000 xg for 15 min. The resultant pellet is washed again using 0.5M NaCl, 100 mM Tris, 50 mM EDTA, pH 7.4.

The resulting washed inclusion bodies are solubilized with 1.5 M guanidine hydrochloride (GuHCl) for 2-4 hours. After 7000 xg centrifugation for 15 min., the pellet is discarded and the polypeptide containing supernatant is incubated at 4°C overnight to allow further GuHCl extraction.

Following high speed centrifugation (30,000 xg) to remove insoluble particles, the GuHCl solubilized protein is refolded by quickly mixing the GuHCl extract with 20 volumes of buffer containing 50 mM sodium, pH 4.5, 150 mM NaCl, 2 mM EDTA by vigorous stirring. The refolded diluted protein solution is kept at 4°C without mixing for 12 hours prior to further purification steps.

To clarify the refolded polypeptide solution, a previously prepared tangential filtration unit equipped with 0.16 µm membrane filter with appropriate surface area (e.g., Filtron), equilibrated with 40 mM sodium acetate, pH 6.0 is employed. The filtered sample is loaded onto a cation exchange resin (e.g., Poros HS-50, Perseptive Biosystems). The column is washed with 40 mM sodium acetate, pH 6.0 and eluted with 250 mM, 500 mM, 1000 mM, and 1500 mM NaCl in the same buffer, in a stepwise manner. The absorbance at 280 nm of the effluent is continuously monitored. Fractions are collected and further analyzed by SDS-PAGE.

Fractions containing the polypeptide are then pooled and mixed with 4 volumes of water. The diluted sample is then loaded onto a previously prepared set of tandem columns of strong anion (Poros HQ-50, Perseptive Biosystems) and weak anion (Poros CM-20, Perseptive Biosystems) exchange resins. The columns are equilibrated with 40 mM sodium acetate, pH 6.0. Both columns are washed with 40 mM sodium acetate, pH 6.0, 200 mM NaCl. The CM-20 column is then eluted using a 10 column volume linear gradient ranging from 0.2 M NaCl, 50 mM sodium acetate, pH 6.0 to 1.0 M NaCl, 50 mM sodium acetate, pH 6.5. Fractions are collected under constant A₂₈₀ monitoring of the effluent. Fractions containing the polypeptide (determined, for instance, by 16% SDS-PAGE) are then pooled.

The resultant polypeptide should exhibit greater than 95% purity after the above refolding and purification steps. No major contaminant bands should be observed from Commassie blue stained 16% SDS-PAGE gel when 5 µg of purified protein is loaded. The purified protein can also be tested for endotoxin/LPS contamination, and typically the LPS content is less than 0.1 ng/ml according to LAL assays.

### Example 7: Cloning and Expression of a Polypeptide in a Baculovirus Expression System

In this example, the plasmid shuttle vector pA2 is used to insert a polynucleotide into a baculovirus to express a polypeptide. This expression vector contains the strong polyhedrin promoter of the *Autographa californica* nuclear polyhedrosis virus (AcMNPV) followed by convenient restriction sites such as BamHI, Xba I and Asp718. The polyadenylation site of the simian virus 40 ("SV40") is used for efficient polyadenylation. For easy selection of recombinant virus, the plasmid contains the beta-galactosidase gene from *E. coli* under control of a weak Drosophila promoter in the same orientation, followed by the polyadenylation signal of the polyhedrin gene. The inserted genes are flanked on both sides by viral sequences for cell-mediated homologous recombination with wild-type viral DNA to generate a viable virus that express the cloned polynucleotide.

Many other baculovirus vectors can be used in place of the vector above, such as pAc373, pVL941, and pAcIM1, as one skilled in the art would readily appreciate, as long as the construct provides appropriately located signals for transcription, translation, secretion and the like, including a signal peptide and an in-frame AUG as required. Such vectors are described, for instance, in Luckow et al., Virology 170:31-39(1989).

Specifically, the cDNA sequence contained in the deposited clone, including the AUG initiation codon and the naturally associated leader sequence identified in Table 1, is amplified using the PCR protocol described in Example 1. If the naturally occurring signal sequence is used to produce the secreted protein, the pA2 vector does not need a second signal peptide. Alternatively, the vector can be modified (pA2 GP) to include a baculovirus leader sequence, using the standard methods described in Summers et al., "A Manual of Methods for Baculovirus Vectors and Insect Cell Culture Procedures," Texas Agricultural Experimental Station Bulletin No. 1555 (1987).

The amplified fragment is isolated from a 1% agarose gel using a commercially available kit ("Geneclean," BIO 101 Inc., La Jolla, Ca.). The fragment then is digested with appropriate restriction enzymes and again purified on a 1% agarose gel.

The plasmid is digested with the corresponding restriction enzymes and optionally, can be dephosphorylated using calf intestinal phosphatase, using routine procedures known in the art. The DNA is then isolated from a 1% agarose gel using a commercially available kit ("Geneclean" BIO 101 Inc., La Jolla, Ca.).

The fragment and the dephosphorylated plasmid are ligated together with T4 DNA ligase. *E. coli* HB101 or other suitable *E. coli* hosts such as XL-1 Blue (Stratagene Cloning Systems, La Jolla, CA) cells are transformed with the ligation mixture and spread on culture plates. Bacteria containing the plasmid are identified by digesting DNA from individual colonies and analyzing the digestion product by gel electrophoresis. The sequence of the cloned fragment is confirmed by DNA sequencing.

Five µg of a plasmid containing the polynucleotide is co-transfected with 1.0 µg of a commercially available linearized baculovirus DNA ("BaculoGold™ baculovirus DNA", Pharmingen, San Diego, CA), using the lipofection method described by Felgner et al., Proc. Natl. Acad. Sci. USA 84:7413-7417 (1987). One µg of BaculoGold™ virus DNA and 5 µg of the plasmid are mixed in a sterile well of a microtiter plate containing 50 µl of serum-free Grace's medium (Life Technologies Inc., Gaithersburg, MD). Afterwards, 10 µl Lipofectin plus 90 µl Grace's medium are added, mixed and incubated for 15 minutes at room temperature. Then the transfection mixture is added drop-wise to Sf9 insect cells (ATCC CRL 1711) seeded in a 35 mm tissue culture plate with 1 ml Grace's medium without serum. The plate is then incubated for 5 hours at 27° C. The transfection solution is then removed from the plate and 1 ml of Grace's insect medium supplemented with 10% fetal calf serum is added. Cultivation is then continued at 27° C for four days.

After four days the supernatant is collected and a plaque assay is performed, as described by Summers and Smith, *supra*. An agarose gel with "Blue Gal" (Life Technologies Inc., Gaithersburg) is used to allow easy identification and isolation of gal-expressing clones, which produce blue-stained plaques. (A detailed description of a "plaque assay" of this type can also be found in the user's guide for insect cell culture and baculovirology distributed by Life Technologies Inc., Gaithersburg, page 9-10.) After appropriate incubation, blue stained plaques are picked with the tip of a micropipettor (e.g., Eppendorf). The agar containing the recombinant viruses is then resuspended in a microcentrifuge tube containing 200 µl of Grace's medium and the suspension containing the recombinant baculovirus is used to infect Sf9 cells seeded in 35 mm dishes. Four days later the supernatants of these culture dishes are harvested and then they are stored at 4° C.

To verify the expression of the polypeptide, Sf9 cells are grown in Grace's medium supplemented with 10% heat-inactivated FBS. The cells are infected with the recombinant baculovirus containing the polynucleotide at a multiplicity of infection ("MOI") of about 2. If radiolabeled proteins are desired, 6 hours later the medium is removed and is replaced with SF900 II medium minus methionine and cysteine (available from Life Technologies Inc., Rockville, MD). After 42 hours, 5 µCi of ³⁵S-methionine and 5 µCi ³⁵S-cysteine (available from Amersham) are added. The cells are further incubated for 16 hours and then are harvested by centrifugation. The proteins in the supernatant as well as the intracellular proteins are analyzed by SDS-PAGE followed by autoradiography (if radiolabeled).

Microsequencing of the amino acid sequence of the amino terminus of purified protein may be used to determine the amino terminal sequence of the produced protein.

### Example 8: Expression of a Polypeptide in Mammalian Cells

The polypeptide of the present invention can be expressed in a mammalian cell. A typical mammalian expression vector contains a promoter element, which mediates the initiation of transcription of mRNA, a protein coding sequence, and signals required for the termination of transcription and polyadenylation of the transcript. Additional elements include enhancers, Kozak sequences and intervening sequences flanked by donor and acceptor sites for RNA splicing. Highly efficient transcription is achieved with the early and late promoters from SV40, the long terminal repeats (LTRs) from Retroviruses, e.g., RSV, HTLVI, HIVI and the early promoter of the cytomegalovirus (CMV). However, cellular elements can also be used (e.g., the human actin promoter).

Suitable expression vectors for use in practicing the present invention include, for example, vectors such as pSVL and pMSG (Pharmacia, Uppsala, Sweden), pRSVcat (ATCC 37152), pSV2dhfr (ATCC 37146), pBC12MI (ATCC 67109), pCMVSport 2.0, and pCMVSport 3.0. Mammalian host cells that could be used include, human Hela, 293, H9 and Jurkat cells, mouse NIH3T3 and C127 cells, Cos 1, Cos 7 and CV1, quail QC1-3 cells, mouse L cells and Chinese hamster ovary (CHO) cells.

Alternatively, the polypeptide can be expressed in stable cell lines containing the polynucleotide integrated into a chromosome. The co-transfection with a selectable marker such as dhfr, gpt, neomycin, hygromycin allows the identification and isolation of the transfected cells.

The transfected gene can also be amplified to express large amounts of the encoded protein. The DHFR (dihydrofolate reductase) marker is useful in developing cell lines that carry several hundred or even several thousand copies of the gene of interest. (See, e.g., Alt, F. W., et al., J. Biol. Chem. 253:1357-1370 (1978); Hamlin, J. L. and Ma, C., Biochem. et Biophys. Acta, 1097:107-143 (1990); Page, M. J. and Sydenham, M. A., Biotechnology 9:64-68 (1991).) Another useful selection marker is the enzyme glutamine synthase (GS) (Murphy et al., Biochem J. 227:277-279 (1991); Bebbington et al., Bio/Technology 10:169-175 (1992). Using these markers, the mammalian cells are grown in selective medium and the cells with the highest resistance are selected. These cell lines contain the amplified gene(s) integrated into a chromosome. Chinese hamster ovary (CHO) and NSO cells are often used for the production of proteins.

Derivatives of the plasmid pSV2-dhfr (ATCC Accession No. 37146), the expression vectors pC4 (ATCC Accession No. 209646) and pC6 (ATCC Accession No.209647) contain the strong promoter (LTR) of the Rous Sarcoma Virus (Cullen et al., Molecular and Cellular Biology, 438-447 (March, 1985)) plus a fragment of the CMV-enhancer (Boshart et al., Cell 41:521-530 (1985).) Multiple cloning sites, e.g., with the restriction enzyme cleavage sites BamHI, XbaI and Asp718, facilitate the cloning of the gene of interest. The vectors also contain the 3' intron, the polyadenylation and termination signal of the rat preproinsulin gene, and the mouse DHFR gene under control of the SV40 early promoter.

Specifically, the plasmid pC6, for example, is digested with appropriate restriction enzymes and then dephosphorylated using calf intestinal phosphates by procedures known in the art. The vector is then isolated from a 1% agarose gel.

A polynucleotide of the present invention is amplified according to the protocol outlined in Example 1. If the naturally occurring signal sequence is used to produce the secreted protein, the vector does not need a second signal peptide. Alternatively, if the naturally occurring signal sequence is not used, the vector can be modified to include a heterologous signal sequence. (See, e.g., WO 96/34891.)

The amplified fragment is isolated from a 1% agarose gel using a commercially available kit ("Geneclean," BIO 101 Inc., La Jolla, Ca.). The fragment then is digested with appropriate restriction enzymes and again purified on a 1% agarose gel.

The amplified fragment is then digested with the same restriction enzyme and purified on a 1% agarose gel. The isolated fragment and the dephosphorylated vector are then ligated with T4 DNA ligase. *E. coli* HB101 or XL-1 Blue cells are then transformed and bacteria are identified that contain the fragment inserted into plasmid pC6 using, for instance, restriction enzyme analysis.

Chinese hamster ovary cells lacking an active DHFR gene is used for transfection. Five µg of the expression plasmid pC6 is cotransfected with 0.5 µg of the plasmid pSVneo using lipofectin (Felgner et al., *supra*). The plasmid pSV2-neo contains a dominant selectable marker, the *neo* gene from Tn5 encoding an enzyme that confers resistance to a group of antibiotics including G418. The cells are seeded in alpha minus MEM supplemented with 1 mg/ml G418. After 2 days, the cells are trypsinized and seeded in hybridoma cloning plates (Greiner, Germany) in alpha minus MEM supplemented with 10, 25, or 50 ng/ml of metothrexate plus 1 mg/ml G418. After about 10-14 days single clones are trypsinized and then seeded in 6-well petri dishes or 10 ml flasks using different concentrations of methotrexate (50 nM, 100 nM, 200 nM, 400 nM, 800 nM). Clones growing at the highest concentrations of methotrexate are then transferred to new 6-well plates containing even higher concentrations of methotrexate (1 µM, 2 µM, 5 µM, 10 mM, 20 mM). The same procedure is repeated until clones are obtained which grow at a concentration of 100 - 200 µM. Expression of the desired gene product is analyzed, for instance, by SDS-PAGE and Western blot or by reversed phase HPLC analysis.

### Example 9: Protein Fusions

The polypeptides of the present invention are preferably fused to other proteins. These fusion proteins can be used for a variety of applications. For example, fusion of the present polypeptides to His-tag, HA-tag, protein A, IgG domains, and maltose binding protein facilitates purification. (See Example 5; see also EP A 394,827; Traunecker, et al., Nature 331:84-86 (1988).) Similarly, fusion to IgG-1, IgG-3, and albumin increases the halflife time in vivo. Nuclear localization signals fused to the polypeptides of the present invention can target the protein to a specific subcellular localization, while covalent heterodimer or homodimers can increase or decrease the activity of a fusion protein. Fusion proteins can also create chimeric molecules having more than one function. Finally, fusion proteins can increase solubility and/or stability of the fused protein compared to the non-fused protein. All of the types of fusion proteins described above can be made by modifying the following protocol, which outlines the fusion of a polypeptide to an IgG molecule, or the protocol described in Example 5.

Briefly, the human Fc portion of the IgG molecule can be PCR amplified, using primers that span the 5' and 3' ends of the sequence described below. These primers also should have convenient restriction enzyme sites that will facilitate cloning into an expression vector, preferably a mammalian expression vector.

For example, if pC4 (Accession No. 209646) is used, the human Fc portion can be ligated into the BamHI cloning site. Note that the 3' BamHI site should be destroyed. Next, the vector containing the human Fc portion is re-restricted with BamHI, linearizing the vector, and a polynucleotide of the present invention, isolated by the PCR protocol described in Example 1, is ligated into this BamHI site. Note that the polynucleotide is cloned without a stop codon, otherwise a fusion protein will not be produced.

If the naturally occurring signal sequence is used to produce the secreted protein, pC4 does not need a second signal peptide. Alternatively, if the naturally occurring signal sequence is not used, the vector can be modified to include a heterologous signal sequence. (See, e.g., WO 96/34891.)

### Human IgG Fc region:

GGGATCCGGAGCCCAAATCTTCTGACAAAACTCACACATGCCCACCGTGCC
CAGCACCTGAATTCGAGGGTGCACCGTCAGTCTTCCTCTTCCCCCCAAAACC
CAAGGACACCCTCATGATCTCCCGGACTCCTGAGGTCACATGCGTGGTGGT
GGACGTAAGCCACGAAGACCCTGAGGTCAAGTTCAACTGGTACGTGGACG
GCGTGGAGGTGCATAATGCCAAGACAAAGCCGCGGGAGGAGCAGTACAAC

AGCACGTACCGTGTGGTCAGCGTCCTCACCGTCCTGCACCAGGACTGGCTG
AATGGCAAGGAGTACAAGTGCAAGGTCTCCAACAAAGCCCTCCCAACCCCC
ATCGAGAAAACCATCTCCAAAGCCAAAGGGCAGCCCCGAGAACCACAGGT
GTACACCCTGCCCCCATCCCGGGATGAGCTGACCAAGAACCAGGTCAGCCT
GACCTGCCTGGTCAAAGGCTTCTATCCAAGCGACATCGCCGTGGAGTGGGA
GAGCAATGGGCAGCCGGAGAACAACTACAAGACCACGCCTCCCGTGCTGG
ACTCCGACGGCTCCTTCTTCCTCTACAGCAAGCTCACCGTGGACAAGAGCA
GGTGGCAGCAGGGGAACGTCTTCTCATGCTCCGTGATGCATGAGGCTCTGC
ACAACCACTACACGCAGAAGAGCCTCTCCCTGTCTCCGGGTAAATGAGTGC
GACGGCCGCGACTCTAGAGGAT (SEQ ID NO:1)

### Example 10: Production of an Antibody from a Polypeptide

The antibodies of the present invention can be prepared by a variety of methods. (See, Current Protocols, Chapter 2.) For example, cells expressing a polypeptide of the present invention is administered to an animal to induce the production of sera containing polyclonal antibodies. In a preferred method, a preparation of the secreted protein is prepared and purified to render it substantially free of natural contaminants. Such a preparation is then introduced into an animal in order to produce polyclonal antisera of greater specific activity.

In the most preferred method, the antibodies of the present invention are monoclonal antibodies (or protein binding fragments thereof). Such monoclonal antibodies can be prepared using hybridoma technology. (Köhler et al., Nature 256:495 (1975); Köhler et al., Eur. J. Immunol. 6:511 (1976); Köhler et al., Eur. J. Immunol. 6:292 (1976); Hammerling et al., in: Monoclonal Antibodies and T-Cell Hybridomas, Elsevier, N.Y., pp. 563-681 (1981).) In general, such procedures involve immunizing an animal (preferably a mouse) with polypeptide or, more preferably, with a secreted polypeptide-expressing cell. Such cells may be cultured in any suitable tissue culture medium; however, it is preferable to culture cells in Earle's modified Eagle's medium supplemented with 10% fetal bovine serum (inactivated at about 56°C), and supplemented with about 10 g/l of nonessential amino acids, about 1,000 U/ml of penicillin, and about 100 µg/ml of streptomycin.

The splenocytes of such mice are extracted and fused with a suitable myeloma cell line. Any suitable myeloma cell line may be employed in accordance with the present invention; however, it is preferable to employ the parent myeloma cell line (SP2O), available from the ATCC. After fusion, the resulting hybridoma cells are selectively maintained in HAT medium, and then cloned by limiting dilution as described by Wands et al. (Gastroenterology 80:225-232 (1981).) The hybridoma cells obtained through such a selection are then assayed to identify clones which secrete antibodies capable of binding the polypeptide.

Alternatively, additional antibodies capable of binding to the polypeptide can be produced in a two-step procedure using anti-idiotypic antibodies. Such a method makes use of the fact that antibodies are themselves antigens, and therefore, it is possible to obtain an antibody which binds to a second antibody. In accordance with this method, protein specific antibodies are used to immunize an animal, preferably a mouse. The splenocytes of such an animal are then used to produce hybridoma cells, and the hybridoma cells are screened to identify clones which produce an antibody whose ability to bind to the protein-specific antibody can be blocked by the polypeptide. Such antibodies comprise anti-idiotypic antibodies to the protein-specific antibody and can be used to immunize an animal to induce formation of further protein-specific antibodies.

It will be appreciated that Fab and F(ab')2 and other fragments of the antibodies of the present invention may be used according to the methods disclosed herein. Such fragments are typically produced by proteolytic cleavage, using enzymes such as papain (to produce Fab fragments) or pepsin (to produce F(ab')2 fragments). Alternatively, secreted protein-binding fragments can be produced through the application of recombinant DNA technology or through synthetic chemistry.

For in vivo use of antibodies in humans, it may be preferable to use "humanized" chimeric monoclonal antibodies. Such antibodies can be produced using genetic constructs derived from hybridoma cells producing the monoclonal antibodies described above. Methods for producing chimeric antibodies are known in the art. (See, for review, Morrison, Science 229:1202 (1985); Oi et al., BioTechniques 4:214 (1986); Cabilly et al., U.S. Patent No. 4,816,567; Taniguchi et al., EP 171496; Morrison et al., EP 173494; Neuberger et al., WO 8601533; Robinson et al., WO 8702671; Boulianne et al., Nature 312:643 (1984); Neuberger et al., Nature 314:268 ( 1985).)

### Example 11: Production Of Secreted Protein For High-Throughput Screening Assays

The following protocol produces a supernatant containing a polypeptide to be tested. This supernatant can then be used in the Screening Assays described in Examples 13-20.

First, dilute Poly-D-Lysine (644 587 Boehringer-Mannheim) stock solution (1mg/ml in PBS) 1:20 in PBS (w/o calcium or magnesium 17-516F Biowhittaker) for a working solution of 50ug/ml. Add 200 ul of this solution to each well (24 well plates) and incubate at RT for 20 minutes. Be sure to distribute the solution over each well (note: a 12-channel pipetter may be used with tips on every other channel). Aspirate off the Poly-D-Lysine solution and rinse with 1ml PBS (Phosphate Buffered Saline). The PBS should remain in the well until just prior to plating the cells and plates may be poly-lysine coated in advance for up to two weeks.

Plate 293T cells (do not carry cells past P+20) at 2 x 10⁵ cells/well in .5ml DMEM(Dulbecco's Modified Eagle Medium)(with 4.5 G/L glucose and L-glutamine (12-604F Biowhittaker))/10% heat inactivated FBS(14-503F Biowhittaker)/1x Penstrep( 17-602E Biowhittaker). Let the cells grow overnight.

The next day, mix together in a sterile solution basin: 300 ul Lipofectamine (18324-012 Gibco/BRL) and 5ml Optimem I (31985070 Gibco/BRL)/96-well plate. With a small volume multi-channel pipetter, aliquot approximately 2ug of an expression vector containing a polynucleotide insert, produced by the methods described in Examples 8 or 9, into an appropriately labeled 96-well round bottom plate. With a multi-channel pipetter, add 50ul of the Lipofectamine/Optimem I mixture to each well. Pipette up and down gently to mix. Incubate at RT 15-45 minutes. After about 20 minutes, use a multi-channel pipetter to add 150ul Optimem I to each well. As a control, one plate of vector DNA lacking an insert should be transfected with each set of transfections.

Preferably, the transfection should be performed by tag-teaming the following tasks. By tag-teaming, hands on time is cut in half, and the cells do not spend too much time on PBS. First, person A aspirates off the media from four 24-well plates of cells, and then person B rinses each well with .5-1 ml PBS. Person A then aspirates off PBS rinse, and person B, using a12-channel pipetter with tips on every other channel, adds the 200ul of DNA/Lipofectamine/Optimem I complex to the odd wells first, then to the even wells, to each row on the 24-well plates. Incubate at 37°C for 6 hours.

While cells are incubating, prepare appropriate media, either 1%BSA in DMEM with 1x penstrep, or CHO-5 media (116.6 mg/L of CaCl2 (anhyd); 0.00130 mg/L CuSO₄-5H₂O; 0.050 mg/L of Fe(NO₃)₃-9H₂O; 0.417 mg/L of FeSO₄-7H₂O; 311.80 mg/L of Kcl; 28.64 mg/L of MgCl₂; 48.84 mg/L of MgSO₄; 6995.50 mg/L of NaCl; 2400.0 mg/L of NaHCO₃; 62.50 mg/L of NaH₂PO₄-H₂O; 71.02 mg/L of Na₂HPO4; .4320 mg/L of ZnSO₄-7H₂O; .002 mg/L of Arachidonic Acid ; 1.022 mg/L of Cholesterol; .070 mg/L of DL-alpha-Tocopherol-Acetate; 0.0520 mg/L of Linoleic Acid; 0.010 mg/L of Linolenic Acid; 0.010 mg/L of Myristic Acid; 0.010 mg/L of Oleic Acid; 0.010 mg/L of Palmitric Acid; 0.010 mg/L of Palmitic Acid; 100 mg/L of Pluronic F-68; 0.010 mg/L of Stearic Acid; 2.20 mg/L of Tween 80; 4551 mg/L of D-Glucose; 130.85 mg/ml of L- Alanine; 147.50 mg/ml of L-Arginine-HCL; 7.50 mg/ml of L-Asparagine-H₂O; 6.65 mg/ml of L-Aspartic Acid; 29.56 mg/ml of L-Cystine-2HCL-H₂O; 31.29 mg/ml of L-Cystine-2HCL; 7.35 mg/ml of L-Glutamic Acid; 365.0 mg/ml of L-Glutamine; 18.75 mg/ml of Glycine; 52.48 mg/ml of L-Histidine-HCL-H₂O; 106.97 mg/ml of L-Isoleucine; 111.45 mg/ml of L-Leucine; 163.75 mg/ml of L-Lysine HCL; 32.34 mg/ml of L-Methionine; 68.48 mg/ml of L-Phenylalainine; 40.0 mg/ml of L-Proline; 26.25 mg/ml of L-Serine; 101.05 mg/ml of L-Threonine; 19.22 mg/ml of L-Tryptophan; 91.79 mg/ml of L-Tryrosine-2Na-2H₂O; 99.65 mg/ml of L-Valine; 0.0035 mg/L of Biotin; 3.24 mg/L of D-Ca Pantothenate; 11.78 mg/L of Choline Chloride; 4.65 mg/L of Folic Acid; 15.60 mg/L of i-Inositol; 3.02 mg/L of Niacinamide; 3.00 mg/L of Pyridoxal HCL; 0.031 mg/L of Pyridoxine HCL; 0.319 mg/L of Riboflavin; 3.17 mg/L of Thiamine HCL; 0.365 mg/L of Thymidine; and 0.680 mg/L of Vitamin B₁₂; 25 mM of HEPES Buffer; 2.39 mg/L of Na Hypoxanthine; 0.105 mg/L of Lipoic Acid; 0.081 mg/L of Sodium Putrescine-2HCL; 55.0 mg/L of Sodium Pyruvate; 0.0067 mg/L of Sodium Selenite; 20uM of Ethanolamine; 0.122 mg/L of Ferric Citrate; 41.70 mg/L of Methyl-B-Cyclodextrin complexed with Linoleic Acid; 33.33 mg/L of Methyl-B-Cyclodextrin complexed with Oleic Acid; and 10 mg/L of Methyl-B-Cyclodextrin complexed with Retinal) with 2mm glutamine and 1x penstrep. (BSA (81-068-3 Bayer) 100gm dissolved in 1L DMEM for a 10% BSA stock solution). Filter the media and collect 50 ul for endotoxin assay in 15ml polystyrene conical.

The transfection reaction is terminated, preferably by tag-teaming, at the end of the incubation period. Person A aspirates off the transfection media, while person B adds 1.5ml appropriate media to each well. Incubate at 37°C for 45 or 72 hours depending on the media used: 1%BSA for 45 hours or CHO-5 for 72 hours.

On day four, using a 300ul multichannel pipetter, aliquot 600ul in one 1ml deep well plate and the remaining supernatant into a 2ml deep well. The supernatants from each well can then be used in the assays described in Examples 13-20.

It is specifically understood that when activity is obtained in any of the assays described below using a supernatant, the activity originates from either the polypeptide directly (e.g., as a secreted protein) or by the polypeptide inducing expression of other proteins, which are then secreted into the supernatant. Thus, the invention further provides a method of identifying the protein in the supernatant characterized by an activity in a particular assay.

### Example 12: Construction of GAS Reporter Construct

One signal transduction pathway involved in the differentiation and proliferation of cells is called the Jaks-STATs pathway. Activated proteins in the Jaks-STATs pathway bind to gamma activation site "GAS" elements or interferon-sensitive responsive element ("ISRE"), located in the promoter of many genes. The binding of a protein to these elements alter the expression of the associated gene.

GAS and ISRE elements are recognized by a class of transcription factors called Signal Transducers and Activators of Transcription, or "STATs." There are six members of the STATs family. Stat1 and Stat3 are present in many cell types, as is Stat2 (as response to IFN-alpha is widespread). Stat4 is more restricted and is not in many cell types though it has been found in T helper class I, cells after treatment with IL-12. Stat5 was originally called mammary growth factor, but has been found at higher concentrations in other cells including myeloid cells. It can be activated in tissue culture cells by many cytokines.

The STATs are activated to translocate from the cytoplasm to the nucleus upon tyrosine phosphorylation by a set of kinases known as the Janus Kinase ("Jaks") family. Jaks represent a distinct family of soluble tyrosine kinases and include Tyk2, Jak1, Jak2, and Jak3. These kinases display significant sequence similarity and are generally catalytically inactive in resting cells.

The Jaks are activated by a wide range of receptors summarized in the Table below. (Adapted from review by Schidler and Darnell, Ann. Rev. Biochem. 64:621-51 (1995).) A cytokine receptor family, capable of activating Jaks, is divided into two groups: (a) Class 1 includes receptors for IL-2, IL-3, IL-4, IL-6, IL-7, IL-9, IL-11, IL-12, IL-15, Epo, PRL, GH, G-CSF, GM-CSF, LIF, CNTF, and thrombopoietin; and (b) Class 2 includes IFN-a, IFN-g, and IL-10. The Class 1 receptors share a conserved cysteine motif (a set of four conserved cysteines and one tryptophan) and a WSXWS motif (a membrane proxial region encoding Trp-Ser-Xxx-Trp-Ser (SEQ ID NO:2)).

Thus, on binding of a ligand to a receptor, Jaks are activated, which in turn activate STATs, which then translocate and bind to GAS elements. This entire process is encompassed in the Jaks-STATs signal transduction pathway.

Therefore, activation of the Jaks-STATs pathway, reflected by the binding of the GAS or the ISRE element, can be used to indicate proteins involved in the proliferation and differentiation of cells. For example, growth factors and cytokines are known to activate the Jaks-STATs pathway. (See Table below.) Thus, by using GAS elements linked to reporter molecules, activators of the Jaks-STATs pathway can be identified.

| | JAKs | | | | STATS | GAS(elements) or ISRE |
|---|---|---|---|---|---|---|
| Ligand | tyk2 | Jak1 | Jak2 | Jak3 | | |
| IFN family | | | | | | |
| IFN-a/B | + | + | - | - | 1,2,3 | ISRE |
| IFN-g | | + | + | - | 1 | GAS (IRF1>Lys6>IFP) |
| Il-10 | + | ? | ? | - | 1,3 | |

| gp130 family | | | | | | |
|---|---|---|---|---|---|---|
| IL-6 (Pleiotrohic) | + | + | + | ? | 1,3 | GAS (IRF1>Lys6>IFP) |
| Il-11(Pleiotrohic) | ? | + | ? | ? | 1,3 | |
| OnM(Pleiotrohic) | ? | + | + | ? | 1,3 | |
| LIF(Pleiotrohic) | ? | + | + | ? | 1,3 | |
| CNTF(Pleiotrohic) | -/+ | + | + | ? | 1,3 | |
| G-CSF(Pleiotrohic) | ? | + | ? | ? | 1,3 | |
| IL-12(Pleiotrohic) | + | - | + | + | 1,3 | |

| g-C family | | | | | | |
|---|---|---|---|---|---|---|
| IL-2 (lymphocytes) | - | + | - | + | 1,3,5 | GAS |
| IL-4 (lymph/myeloid) | - | + | - | + | 6 | GAS (IRF1 = IFP >>Ly6)(IgH) |
| IL-7 (lymphocytes) | - | + | - | + | 5 | GAS |
| IL-9 (lymphocytes) | - | + | - | + | 5 | GAS |
| IL-13 (lymphocyte) | - | + | ? | ? | 6 | GAS |
| IL-15 | ? | + | ? | + | 5 | GAS |

| gp140 family | | | | | | |
|---|---|---|---|---|---|---|
| IL-3 (myeloid) | - | - | + | - | 5 | GAS (IRF1>IFP>>Ly6) |
| IL-5 (myeloid) | | - | + | - | 5 | GAS |
| GM-CSF (myeloid) | - | - | + | - | 5 | GAS |

| Growth hormone family | | | | | | |
|---|---|---|---|---|---|---|
| GH | ? | - | + | - | 5 | |
| PRL | ? | +/- | + | - | 1,3,5 | |
| EPO | ? | - | + | - | 5 | GAS(B-CAS>IRF1=IFP>>Ly6) |

| Receptor Tyrosine Kinases | | | | | | |
|---|---|---|---|---|---|---|
| EGF | ? | + | + | - | 1,3 | GAS (IRF1) |
| PDGF | ? | + | + | - | 1,3 | |
| CSF-1 | ? | + | + | - | 1,3 | GAS (not IRF1) |

To construct a synthetic GAS containing promoter element, which is used in the Biological Assays described in Examples 13-14, a PCR based strategy is employed to generate a GAS-SV40 promoter sequence. The 5' primer contains four tandem copies of the GAS binding site found in the IRF1 promoter and previously demonstrated to bind STATs upon induction with a range of cytokines (Rothman et al., Immunity 1:457-468 (1994).), although other GAS or ISRE elements can be used instead. The 5' primer also contains 18bp of sequence complementary to the SV40 early promoter sequence and is flanked with an XhoI site. The sequence of the 5' primer is:

5':GCGCCTCGAGATTTCCCCGAAATCTAGATTTCCCCGAAATGATTTCCCCG
AAATGATTTCCCCGAAATATCTGCCATCTCAATTAG:3' (SEQ ID NO:3)

The downstream primer is complementary to the SV40 promoter and is flanked with a Hind III site: 5':GCGGCAAGCTTTTTGCAAAGCCTAGGC:3' (SEQ ID NO:4)

PCR amplification is performed using the SV40 promoter template present in the B-gal:promoter plasmid obtained from Clontech. The resulting PCR fragment is digested with XhoI/Hind III and subcloned into BLSK2-. (Stratagene.) Sequencing with forward and reverse primers confirms that the insert contains the following sequence:

5':CTCGAGATTTCCCCGAAATCTAGATTTCCCCGAAATGATTTCCCCGAAATG
ATTTCCCCGAAATATCTGCCATCTCAATTAGTCAGCAACCATAGTCCCGCCC
CTAACTCCGCCCATCCCGCCCCTAACTCCGCCCAGTTCCGCCCATTCTCCGC
CCCATGGCTGACTAATTTTTTTTATTTATGCAGAGGCCGAGGCCGCCTCGGC
CTCTGAGCTATTCCAGAAGTAGTGAGGAGGCTTTTTTGGAGGCCTAGGCTTT
TGCAAAAAGCTT:3' (SEQ ID NO:5)

With this GAS promoter element linked to the SV40 promoter, a GAS:SEAP2 reporter construct is next engineered. Here, the reporter molecule is a secreted alkaline phosphatase, or "SEAP." Clearly, however, any reporter molecule can be instead of SEAP, in this or in any of the other Examples. Well known reporter molecules that can be used instead of SEAP include chloramphenicol acetyltransferase (CAT), luciferase, alkaline phosphatase, B-galactosidase, green fluorescent protein (GFP), or any protein detectable by an antibody.

The above sequence confirmed synthetic GAS-SV40 promoter element is subcloned into the pSEAP-Promoter vector obtained from Clontech using HindIII and XhoI, effectively replacing the SV40 promoter with the amplified GAS:SV40 promoter element, to create the GAS-SEAP vector. However, this vector does not contain a neomycin resistance gene, and therefore, is not preferred for mammalian expression systems.

Thus, in order to generate mammalian stable cell lines expressing the GAS-SEAP reporter, the GAS-SEAP cassette is removed from the GAS-SEAP vector using SalI and NotI, and inserted into a backbone vector containing the neomycin resistance gene, such as pGFP-1 (Clontech), using these restriction sites in the multiple cloning site, to create the GAS-SEAP/Neo vector. Once this vector is transfected into mammalian cells, this vector can then be used as a reporter molecule for GAS binding as described in Examples 13-14.

Other constructs can be made using the above description and replacing GAS with a different promoter sequence. For example, construction of reporter molecules containing NFK-B and EGR promoter sequences are described in Examples 15 and 16. However, many other promoters can be substituted using the protocols described in these Examples. For instance, SRE, IL-2, NFAT, or Osteocalcin promoters can be substituted, alone or in combination (e.g., GAS/NF-KB/EGR, GAS/NF-KB, Il-2/NFAT, or NF-KB/GAS). Similarly, other cell lines can be used to test reporter construct activity, such as HELA (epithelial), HUVEC (endothelial), Reh (B-cell), Saos-2 (osteoblast), HUVAC (aortic), or Cardiomyocyte.

### Example 13: High-Throughput Screening Assay for T-cell Activity.

The following protocol is used to assess T-cell activity by identifying factors, such as growth factors and cytokines, that may proliferate or differentiate T-cells. T-cell activity is assessed using the GAS/SEAP/Neo construct produced in Example 12. Thus, factors that increase SEAP activity indicate the ability to activate the Jaks-STATS signal transduction pathway. The T-cell used in this assay is Jurkat T-cells (ATCC Accession No. TIB-152), although Molt-3 cells (ATCC Accession No. CRL-1552) and Molt-4 cells (ATCC Accession No. CRL-1582) cells can also be used.

Jurkat T-cells are lymphoblastic CD4+ Th1 helper cells. In order to generate stable cell lines, approximately 2 million Jurkat cells are transfected with the GAS-SEAP/neo vector using DMRIE-C (Life Technologies)(transfection procedure described below). The transfected cells are seeded to a density of approximately 20,000 cells per well and transfectants resistant to 1 mg/ml genticin selected. Resistant colonies are expanded and then tested for their response to increasing concentrations of interferon gamma. The dose response of a selected clone is demonstrated.

Specifically, the following protocol will yield sufficient cells for 75 wells containing 200 ul of cells. Thus, it is either scaled up, or performed in multiple to generate sufficient cells for multiple 96 well plates. Jurkat cells are maintained in RPMI + 10% serum with 1%Pen-Strep. Combine 2.5 mls of OPTI-MEM (Life Technologies) with 10 ug of plasmid DNA in a T25 flask. Add 2.5 ml OPTI-MEM containing 50 ul of DMRIE-C and incubate at room temperature for 15-45 mins.

During the incubation period, count cell concentration, spin down the required number of cells (10⁷ per transfection), and resuspend in OPTI-MEM to a final concentration of 10⁷ cells/ml. Then add 1ml of 1 x 10⁷ cells in OPTI-MEM to T25 flask and incubate at 37°C for 6 hrs. After the incubation, add 10 ml of RPMI + 15% serum.

The Jurkat:GAS-SEAP stable reporter lines are maintained in RPMI + 10% serum, 1 mg/ml Genticin, and 1% Pen-Strep. These cells are treated with supernatants containing a polypeptide as produced by the protocol described in Example 11.

On the day of treatment with the supernatant, the cells should be washed and resuspended in fresh RPMI + 10% serum to a density of 500,000 cells per ml. The exact number of cells required will depend on the number of supernatants being screened. For one 96 well plate, approximately 10 million cells (for 10 plates, 100 million cells) are required.

Transfer the cells to a triangular reservoir boat, in order to dispense the cells into a 96 well dish, using a 12 channel pipette. Using a 12 channel pipette, transfer 200 ul of cells into each well (therefore adding 100, 000 cells per well).

After all the plates have been seeded, 50 ul of the supernatants are transferred directly from the 96 well plate containing the supernatants into each well using a 12 channel pipette. In addition, a dose of exogenous interferon gamma (0.1, 1.0, 10 ng) is added to wells H9, H10, and H11 to serve as additional positive controls for the assay.

The 96 well dishes containing Jurkat cells treated with supernatants are placed in an incubator for 48 hrs (note: this time is variable between 48-72 hrs). 35 ul samples from each well are then transferred to an opaque 96 well plate using a 12 channel pipette. The opaque plates should be covered (using sellophene covers) and stored at -20°C until SEAP assays are performed according to Example 17. The plates containing the remaining treated cells are placed at 4°C and serve as a source of material for repeating the assay on a specific well if desired.

As a positive control, 100 Unit/ml interferon gamma can be used which is known to activate Jurkat T cells. Over 30 fold induction is typically observed in the positive control wells.

### Example 14: High-Throughput Screening Assay Identifying Myeloid Activity

The following protocol is used to assess myeloid activity by identifying factors, such as growth factors and cytokines, that may proliferate or differentiate myeloid cells. Myeloid cell activity is assessed using the GAS/SEAP/Neo construct produced in Example 12. Thus, factors that increase SEAP activity indicate the ability to activate the Jaks-STATS signal transduction pathway. The myeloid cell used in this assay is U937, a pre-monocyte cell line, although TF-1, HL60, or KG1 can be used.

To transiently transfect U937 cells with the GAS/SEAP/Neo construct produced in Example 12, a DEAE-Dextran method (Kharbanda et. al., 1994, Cell Growth & Differentiation, 5:259-265) is used. First, harvest 2x10e⁷ U937 cells and wash with PBS. The U937 cells are usually grown in RPMI 1640 medium containing 10% heat-inactivated fetal bovine serum (FBS) supplemented with 100 units/ml penicillin and 100 mg/ml streptomycin.

Next, suspend the cells in 1 ml of 20 mM Tris-HCl (pH 7.4) buffer containing 0.5 mg/ml DEAE-Dextran, 8 ug GAS-SEAP2 plasmid DNA, 140 mM NaCl, 5 mM KCl, 375 uM Na₂HPO₄.7H₂O, 1 mM MgCl₂, and 675 uM CaCl₂. Incubate at 37°C for 45 min.

Wash the cells with RPMI 1640 medium containing 10% FBS and then resuspend in 10 ml complete medium and incubate at 37°C for 36 hr.

The GAS-SEAP/U937 stable cells are obtained by growing the cells in 400 ug/ml G418. The G418-free medium is used for routine growth but every one to two months, the cells should be re-grown in 400 ug/ml G418 for couple of passages.

These cells are tested by harvesting 1x10⁸ cells (this is enough for ten 96-well plates assay) and wash with PBS. Suspend the cells in 200 ml above described growth medium, with a final density of 5x10⁵ cells/ml. Plate 200 ul cells per well in the 96-well plate (or 1x10⁵ cells/well).

Add 50 ul of the supernatant prepared by the protocol described in Example 11. Incubate at 37°C for 48 to 72 hr. As a positive control, 100 Unit/ml interferon gamma can be used which is known to activate U937 cells. Over 30 fold induction is typically observed in the positive control wells. SEAP assay the supernatant according to the protocol described in Example 17.

### Example 15: High-Throughput Screening Assay Identifying Neuronal Activity.

When cells undergo differentiation and proliferation, a group of genes are activated through many different signal transduction pathways. One of these genes, EGR1 (early growth response gene 1), is induced in various tissues and cell types upon activation. The promoter of EGR1 is responsible for such induction. Using the EGR1 promoter linked to reporter molecules, activation of cells can be assessed.

Particularly, the following protocol is used to assess neuronal activity in PC12 cell lines. PC12 cells (rat phenochromocytoma cells) are known to proliferate and/or differentiate by activation with a number of mitogens, such as TPA (tetradecanoyl phorbol acetate), NGF (nerve growth factor), and EGF (epidermal growth factor). The EGR1 gene expression is activated during this treatment. Thus, by stably transfecting PC12 cells with a construct containing an EGR promoter linked to SEAP reporter, activation of PC12 cells can be assessed.

The EGR/SEAP reporter construct can be assembled by the following protocol. The EGR-1 promoter sequence (-633 to +1)(Sakamoto K et al., Oncogene 6:867-871 (1991)) can be PCR amplified from human genomic DNA using the following primers:

5' GCGCTCGAGGGATGACAGCGATAGAACCCCGG -3' (SEQ ID NO:6)

5' GCGAAGCTTCGCGACTCCCCGGATCCGCCTC-3' (SEQ ID NO:7)

Using the GAS:SEAP/Neo vector produced in Example 12, EGR1 amplified product can then be inserted into this vector. Linearize the GAS:SEAP/Neo vector using restriction enzymes XhoI/HindIII, removing the GAS/SV40 stuffer. Restrict the EGR 1 amplified product with these same enzymes. Ligate the vector and the EGR 1 promoter.

To prepare 96 well-plates for cell culture, two mls of a coating solution (1:30 dilution of collagen type I (Upstate Biotech Inc. Cat#08-115) in 30% ethanol (filter sterilized)) is added per one 10 cm plate or 50 ml per well of the 96-well plate, and allowed to air dry for 2 hr.

PC12 cells are routinely grown in RPMI-1640 medium (Bio Whittaker) containing 10% horse serum (JRH BIOSCIENCES, Cat. # 12449-78P), 5% heat-inactivated fetal bovine serum (FBS) supplemented with 100 units/ml penicillin and 100 ug/ml streptomycin on a precoated 10 cm tissue culture dish. One to four split is done every three to four days. Cells are removed from the plates by scraping and resuspended with pipetting up and down for more than 15 times.

Transfect the EGR/SEAP/Neo construct into PC12 using the Lipofectamine protocol described in Example 11. EGR-SEAP/PC12 stable cells are obtained by growing the cells in 300 ug/ml G418. The G418-free medium is used for routine growth but every one to two months, the cells should be re-grown in 300 ug/ml G418 for couple of passages.

To assay for neuronal activity, a 10 cm plate with cells around 70 to 80% confluent is screened by removing the old medium. Wash the cells once with PBS (Phosphate buffered saline). Then starve the cells in low serum medium (RPMI-1640 containing 1% horse serum and 0.5% FBS with antibiotics) overnight.

The next morning, remove the medium and wash the cells with PBS. Scrape off the cells from the plate, suspend the cells well in 2 ml low serum medium. Count the cell number and add more low serum medium to reach final cell density as 5x10⁵ cells/ml.

Add 200 ul of the cell suspension to each well of 96-well plate (equivalent to 1x10⁵ cells/well). Add 50 ul supernatant produced by Example 11, 37°C for 48 to 72 hr. As a positive control, a growth factor known to activate PC12 cells through EGR can be used, such as 50 ng/ul of Neuronal Growth Factor (NGF). Over fifty-fold induction of SEAP is typically seen in the positive control wells. SEAP assay the supernatant according to Example 17.

### Example 16: High-Throughput Screening Assay for T-cell Activity

NF-κB (Nuclear Factor κB) is a transcription factor activated by a wide variety of agents including the inflammatory cytokines IL-1 and TNF, CD30 and CD40, lymphotoxin-alpha and lymphotoxin-beta, by exposure to LPS or thrombin, and by expression of certain viral gene products. As a transcription factor, NF-κB regulates the expression of genes involved in immune cell activation, control of apoptosis (NF-κB appears to shield cells from apoptosis), B and T-cell development, anti-viral and antimicrobial responses, and multiple stress responses.

In non-stimulated conditions, NF- κB is retained in the cytoplasm with I-κB (Inhibitor κB). However, upon stimulation, I- κB is phosphorylated and degraded, causing NF- κB to shuttle to the nucleus, thereby activating transcription of target genes. Target genes activated by NF- κB include IL-2, IL-6, GM-CSF, ICAM-1 and class 1 MHC.

Due to its central role and ability to respond to a range of stimuli, reporter constructs utilizing the NF-κB promoter element are used to screen the supernatants produced in Example 11. Activators or inhibitors of NF-kB would be useful in treating diseases. For example, inhibitors of NF-κB could be used to treat those diseases related to the acute or chronic activation ofNF-kB, such as rheumatoid arthritis.

To construct a vector containing the NF-κB promoter element, a PCR based strategy is employed. The upstream primer contains four tandem copies of the NF-κB binding site (GGGGACTTTCCC) (SEQ ID NO:8), 18 bp of sequence complementary to the 5' end of the SV40 early promoter sequence, and is flanked with an XhoI site:

5':GCGGCCTCGAGGGGACTTTCCCGGGGACTTTCCGGGGACTTTCCGGGAC
TTTCCATCCTGCCATCTCAATTAG:3' (SEQ ID NO:9)

The downstream primer is complementary to the 3' end of the SV40 promoter and is flanked with a Hind III site:

5':GCGGCAAGCTTTTGCAAAGCCTAGGC:3' (SEQ ID NO:4)

PCR amplification is performed using the SV40 promoter template present in the pB-gal:promoter plasmid obtained from Clontech. The resulting PCR fragment is digested with XhoI and Hind III and subcloned into BLSK2-. (Stratagene) Sequencing with the T7 and T3 primers confirms the insert contains the following sequence:

5':CTCGAGGGGACTTTCCCGGGGACTTTCCGGGACTTTCCGGGACTTTCC
ATCTGCCATCTCAATTAGTCAGCAACCATAGTCCCGCCCCTAACTCCGCCCA
TCCCGCCCCTAACTCCGCCCAGTTCCGCCCATTCTCCGCCCCATGGCTGACT
AATTTTTTTTATTTATGCAGAGGCCGAGGCCGCCTCGGCCTCTGAGCTATTC
CAGAAGTAGTGAGGAGGCTTTTTTGGAGGCCTAGGCTTTTGCAAAAAGCTT:
3' (SEQ ID NO:10)

Next, replace the SV40 minimal promoter element present in the pSEAP2-promoter plasmid (Clontech) with this NF-κB/SV40 fragment using XhoI and HindIII. However, this vector does not contain a neomycin resistance gene, and therefore, is not preferred for mammalian expression systems.

In order to generate stable mammalian cell lines, the NF-κB/SV40/SEAP cassette is removed from the above NF-κB/SEAP vector using restriction enzymes SalI and NotI, and inserted into a vector containing neomycin resistance. Particularly, the NF-κB/SV40/SEAP cassette was inserted into pGFP-1 (Clontech), replacing the GFP gene, after restricting pGFP-1 with SalI and NotI.

Once NF-κB/SV40/SEAP/Neo vector is created, stable Jurkat T-cells are created and maintained according to the protocol described in Example 13. Similarly, the method for assaying supernatants with these stable Jurkat T-cells is also described in Example 13. As a positive control, exogenous TNF alpha (0.1,1, 10 ng) is added to wells H9, H10, and H11, with a 5-10 fold activation typically observed.

### Example 17: Assay for SEAP Activity

As a reporter molecule for the assays described in Examples 13-16, SEAP activity is assayed using the Tropix Phospho-light Kit (Cat. BP-400) according to the following general procedure. The Tropix Phospho-light Kit supplies the Dilution, Assay, and Reaction Buffers used below.

Prime a dispenser with the 2.5x Dilution Buffer and dispense 15 µl of 2.5x dilution buffer into Optiplates containing 35 µl of a supernatant. Seal the plates with a plastic sealer and incubate at 65°C for 30 min. Separate the Optiplates to avoid uneven heating.

Cool the samples to room temperature for 15 minutes. Empty the dispenser and prime with the Assay Buffer. Add 50 µl Assay Buffer and incubate at room temperature 5 min. Empty the dispenser and prime with the Reaction Buffer (see the table below). Add 50 µl Reaction Buffer and incubate at room temperature for 20 minutes. Since the intensity of the chemiluminescent signal is time dependent, and it takes about 10 minutes to read 5 plates on luminometer, one should treat 5 plates at each time and start the second set 10 minutes later.

Read the relative light unit in the luminometer. Set H12 as blank, and print the results. An increase in chemiluminescence indicates reporter activity.

| **Reaction Buffer Formulation**: | | |
|---|---|---|
| # of plates | Rxn buffer diluent (ml) | CSPD (ml) |
| 10 | 60 | 3 |
| 11 | 65 | 3.25 |
| 12 | 70 | 3.5 |
| 13 | 75 | 3.75 |
| 14 | 80 | 4 |
| 15 | 85 | 4.25 |
| 16 | 90 | 4.5 |
| 17 | 95 | 4.75 |
| 18 | 100 | 5 |
| 19 | 105 | 5.25 |
| 20 | 110 | 5.5 |
| 21 | 115 | 5.75 |
| 22 | 120 | 6 |
| 23 | 125 | 6.25 |
| 24 | 130 | 6.5 |
| 25 | 135 | 6.75 |
| 26 | 140 | 7 |
| 27 | 145 | 7.25 |
| 28 | 150 | 7.5 |
| 29 | 155 | 7.75 |
| 30 | 160 | 8 |
| 31 | 165 | 8.25 |
| 32 | 170 | 8.5 |
| 33 | 175 | 8.75 |
| 34 | 180 | 9 |
| 35 | 185 | 9.25 |
| 36 | 190 | 9.5 |
| 37 | 195 | 9.75 |
| 38 | 200 | 10 |
| 39 | 205 | 10.25 |
| 40 | 210 | 10.5 |
| 41 | 215 | 10.75 |
| 42 | 220 | 11 |
| 43 | 225 | 11.25 |
| 44 | 230 | 11.5 |
| 45 | 235 | 11.75 |
| 46 | 240 | 12 |
| 47 | 245 | 12.25 |
| 48 | 250 | 12.5 |
| 49 | 255 | 12.75 |
| 50 | 260 | 13 |

### Example 18: High-Throughput Screening Assay Identifying Changes in Small Molecule Concentration and Membrane Permeability

Binding of a ligand to a receptor is known to alter intracellular levels of small molecules, such as calcium, potassium, sodium, and pH, as well as alter membrane potential. These alterations can be measured in an assay to identify supernatants which bind to receptors of a particular cell. Although the following protocol describes an assay for calcium, this protocol can easily be modified to detect changes in potassium, sodium, pH, membrane potential, or any other small molecule which is detectable by a fluorescent probe.

The following assay uses Fluorometric Imaging Plate Reader ("FLIPR") to measure changes in fluorescent molecules (Molecular Probes) that bind small molecules. Clearly, any fluorescent molecule detecting a small molecule can be used instead of the calcium fluorescent molecule, fluo-3, used here.

For adherent cells, seed the cells at 10,000 -20,000 cells/well in a Co-star black 96-well plate with clear bottom. The plate is incubated in a CO₂ incubator for 20 hours. The adherent cells are washed two times in Biotek washer with 200 ul of HBSS (Hank's Balanced Salt Solution) leaving 100 ul of buffer after the final wash.

A stock solution of 1 mg/ml fluo-3 is made in 10% pluronic acid DMSO. To load the cells with fluo-3, 50 ul of 12 ug/ml fluo-3 is added to each well. The plate is incubated at 37°C in a CO₂ incubator for 60 min. The plate is washed four times in the Biotek washer with HBSS leaving 100 ul of buffer.

For non-adherent cells, the cells are spun down from culture media. Cells are re-suspended to 2-5x10⁶ cells/ml with HBSS in a 50-ml conical tube. 4 ul of 1 mg/ml fluo-3 solution in 10% pluronic acid DMSO is added to each ml of cell suspension. The tube is then placed in a 37°C water bath for 30-60 min. The cells are washed twice with HBSS, resuspended to 1x10⁶ cells/ml, and dispensed into a microplate, 100 ul/well. The plate is centrifuged at 1000 rpm for 5 min. The plate is then washed once in Denley CellWash with 200 ul, followed by an aspiration step to 100 ul final volume.

For a non-cell based assay, each well contains a fluorescent molecule, such as fluo-3. The supernatant is added to the well, and a change in fluorescence is detected.

To measure the fluorescence of intracellular calcium, the FLIPR is set for the following parameters: (1) System gain is 300-800 mW; (2) Exposure time is 0.4 second; (3) Camera F/stop is F/2; (4) Excitation is 488 nm; (5) Emission is 530 nm; and (6) Sample addition is 50 ul. Increased emission at 530 nm indicates an extracellular signaling event which has resulted in an increase in the intracellular Ca⁺⁺ concentration.

### Example 19: High-Throughput Screening Assay Identifying Tyrosine Kinase Activity

The Protein Tyrosine Kinases (PTK) represent a diverse group of transmembrane and cytoplasmic kinases. Within the Receptor Protein Tyrosine Kinase RPTK) group are receptors for a range of mitogenic and metabolic growth factors including the PDGF, FGF, EGF, NGF, HGF and Insulin receptor subfamilies. In addition there are a large family of RPTKs for which the corresponding ligand is unknown. Ligands for RPTKs include mainly secreted small proteins, but also membrane-bound and extracellular matrix proteins.

Activation of RPTK by ligands involves ligand-mediated receptor dimerization, resulting in transphosphorylation of the receptor subunits and activation of the cytoplasmic tyrosine kinases. The cytoplasmic tyrosine kinases include receptor associated tyrosine kinases of the src-family (e.g., src, yes, Ick, lyn, fyn) and non-receptor linked and cytosolic protein tyrosine kinases, such as the Jak family, members of which mediate signal transduction triggered by the cytokine superfamily of receptors (e.g., the Interleukins, Interferons, GM-CSF, and Leptin).

Because of the wide range of known factors capable of stimulating tyrosine kinase activity, the identification of novel human secreted proteins capable of activating tyrosine kinase signal transduction pathways are of interest. Therefore, the following protocol is designed to identify those novel human secreted proteins capable of activating the tyrosine kinase signal transduction pathways.

Seed target cells (e.g., primary keratinocytes) at a density of approximately 25,000 cells per well in a 96 well Loprodyne Silent Screen Plates purchased from Nalge Nunc (Naperville, IL). The plates are sterilized with two 30 minute rinses with 100% ethanol, rinsed with water and dried overnight. Some plates are coated for 2 hr with 100 ml of cell culture grade type I collagen (50 mg/ml), gelatin (2%) or polylysine (50 mg/ml), all of which can be purchased from Sigma Chemicals (St. Louis, MO) or 10% Matrigel purchased from Becton Dickinson (Bedford,MA), or calf serum, rinsed with PBS and stored at 4°C. Cell growth on these plates is assayed by seeding 5,000 cells/well in growth medium and indirect quantitation of cell number through use of alamarBlue as described by the manufacturer Alamar Biosciences, Inc. (Sacramento, CA) after 48 hr. Falcon plate covers #3071 from Becton Dickinson (Bedford,MA) are used to cover the Loprodyne Silent Screen Plates. Falcon Microtest III cell culture plates can also be used in some proliferation experiments.

To prepare extracts, A431 cells are seeded onto the nylon membranes of Loprodyne plates (20,000/200ml/well) and cultured overnight in complete medium. Cells are quiesced by incubation in serum-free basal medium for 24 hr. After 5-20 minutes treatment with EGF (60ng/ml) or 50 ul of the supernatant produced in Example 11, the medium was removed and 100 ml of extraction buffer ((20 mM HEPES pH 7.5, 0.15 M NaCl, 1% Triton X-100, 0.1% SDS, 2 mM Na3VO4, 2 mM Na4P2O7 and a cocktail of protease inhibitors (# 1836170) obtained from Boeheringer Mannheim (Indianapolis, IN) is added to each well and the plate is shaken on a rotating shaker for 5 minutes at 4°C. The plate is then placed in a vacuum transfer manifold and the extract filtered through the 0.45 mm membrane bottoms of each well using house vacuum. Extracts are collected in a 96-well catch/assay plate in the bottom of the vacuum manifold and immediately placed on ice. To obtain extracts clarified by centrifugation, the content of each well, after detergent solubilization for 5 minutes, is removed and centrifuged for 15 minutes at 4°C at 16,000 x g.

Test the filtered extracts for levels of tyrosine kinase activity. Although many methods of detecting tyrosine kinase activity are known, one method is described here.

Generally, the tyrosine kinase activity of a supernatant is evaluated by determining its ability to phosphorylate a tyrosine residue on a specific substrate (a biotinylated peptide). Biotinylated peptides that can be used for this purpose include PSK1 (corresponding to amino acids 6-20 of the cell division kinase cdc2-p34) and PSK2 (corresponding to amino acids 1-17 of gastrin). Both peptides are substrates for a range of tyrosine kinases and are available from Boehringer Mannheim.

The tyrosine kinase reaction is set up by adding the following components in order. First, add 10ul of 5uM Biotinylated Peptide, then 10ul ATP/Mg₂₊ (5mM ATP/50mM MgCl₂), then 10ul of 5x Assay Buffer (40mM imidazole hydrochloride, pH7.3, 40 mM beta-glycerophosphate, 1mM EGTA, 100mM MgCl₂, 5 mM MnCl₂, 0.5 mg/ml BSA), then 5ul of Sodium Vanadate(1mM), and then 5ul of water. Mix the components gently and preincubate the reaction mix at 30°C for 2 min. Initial the reaction by adding 10ul of the control enzyme or the filtered supernatant.

The tyrosine kinase assay reaction is then terminated by adding 10 ul of 120mm EDTA and place the reactions on ice.

Tyrosine kinase activity is determined by transferring 50 ul aliquot of reaction mixture to a microtiter plate (MTP) module and incubating at 37°C for 20 min. This allows the streptavadin coated 96 well plate to associate with the biotinylated peptide. Wash the MTP module with 300ul/well of PBS four times. Next add 75 ul of anti-phospotyrosine antibody conjugated to horse radish peroxidase(anti-P-Tyr-POD(0.5u/ml)) to each well and incubate at 37°C for one hour. Wash the well as above.

Next add 100ul of peroxidase substrate solution (Boehringer Mannheim) and incubate at room temperature for at least 5 mins (up to 30 min). Measure the absorbance of the sample at 405 nm by using ELISA reader. The level of bound peroxidase activity is quantitated using an ELISA reader and reflects the level of tyrosine kinase activity.

### Example 20: High-Throughput Screening Assay Identifying Phosphorylation Activity

As a potential alternative and/or compliment to the assay of protein tyrosine kinase activity described in Example 19, an assay which detects activation (phosphorylation) of major intracellular signal transduction intermediates can also be used. For example, as described below one particular assay can detect tyrosine phosphorylation of the Erk-1 and Erk-2 kinases. However, phosphorylation of other molecules, such as Raf, JNK, p38 MAP, Map kinase kinase (MEK), MEK kinase, Src, Muscle specific kinase (MuSK), IRAK, Tec, and Janus, as well as any other phosphoserine, phosphotyrosine, or phosphothreonine molecule, can be detected by substituting these molecules for Erk-1 or Erk-2 in the following assay.

Specifically, assay plates are made by coating the wells of a 96-well ELISA plate with 0.1ml of protein G (1ug/ml) for 2 hr at room temp, (RT). The plates are then rinsed with PBS and blocked with 3% BSA/PBS for 1 hr at RT. The protein G plates are then treated with 2 commercial monoclonal antibodies (100ng/well) against Erk-1 and Erk-2 (1 hr at RT) (Santa Cruz Biotechnology). (To detect other molecules, this step can easily be modified by substituting a monoclonal antibody detecting any of the above described molecules.) After 3-5 rinses with PBS, the plates are stored at 4°C until use.

A431 cells are seeded at 20,000/well in a 96-well Loprodyne filterplate and cultured overnight in growth medium. The cells are then starved for 48 hr in basal medium (DMEM) and then treated with EGF (6ng/well) or 50 ul of the supernatants obtained in Example 11 for 5-20 minutes. The cells are then solubilized and extracts filtered directly into the assay plate.

After incubation with the extract for 1 hr at RT, the wells are again rinsed. As a positive control, a commercial preparation of MAP kinase (10ng/well) is used in place of A431 extract. Plates are then treated with a commercial polyclonal (rabbit) antibody (1ug/ml) which specifically recognizes the phosphorylated epitope of the Erk-1 and Erk-2 kinases (1 hr at RT). This antibody is biotinylated by standard procedures. The bound polyclonal antibody is then quantitated by successive incubations with Europium-streptavidin and Europium fluorescence enhancing reagent in the Wallac DELFIA instrument (time-resolved fluorescence). An increased fluorescent signal over background indicates a phosphorylation.

### Example 21: Method of Determining Alterations in a Gene Corresponding to a Polynucleotide

RNA isolated from entire families or individual patients presenting with a phenotype of interest (such as a disease) is be isolated. cDNA is then generated from these RNA samples using protocols known in the art. (See, Sambrook.) The cDNA is then used as a template for PCR, employing primers surrounding regions of interest in SEQ ID NO:X. Suggested PCR conditions consist of 35 cycles at 95°C for 30 seconds; 60-120 seconds at 52-58°C; and 60-120 seconds at 70°C, using buffer solutions described in Sidransky, D., et al., Science 252:706 (1991).

PCR products are then sequenced using primers labeled at their 5' end with T4 polynucleotide kinase, employing SequiTherm Polymerase. (Epicentre Technologies). The intron-exon borders of selected exons is also determined and genomic PCR products analyzed to confirm the results. PCR products harboring suspected mutations is then cloned and sequenced to validate the results of the direct sequencing.

PCR products is cloned into T-tailed vectors as described in Holton, T.A. and Graham, M.W., Nucleic Acids Research, 19:1156 (1991) and sequenced with T7 polymerase (United States Biochemical). Affected individuals are identified by mutations not present in unaffected individuals.

Genomic rearrangements are also observed as a method of determining alterations in a gene corresponding to a polynucleotide. Genomic clones isolated according to Example 2 are nick-translated with digoxigenindeoxy-uridine 5'-triphosphate (Boehringer Manheim), and FISH performed as described in Johnson, Cg. et al., Methods Cell Biol. 35:73-99 (1991). Hybridization with the labeled probe is carried out using a vast excess of human cot-1 DNA for specific hybridization to the corresponding genomic locus.

Chromosomes are counterstained with 4,6-diamino-2-phenylidole and propidium iodide, producing a combination of C- and R-bands. Aligned images for precise mapping are obtained using a triple-band filter set (Chroma Technology, Brattleboro, VT) in combination with a cooled charge-coupled device camera (Photometrics, Tucson, AZ) and variable excitation wavelength filters. (Johnson, Cv. et al., Genet. Anal. Tech. Appl., 8:75 (1991).) Image collection, analysis and chromosomal fractional length measurements are performed using the ISee Graphical Program System. (Inovision Corporation, Durham, NC.) Chromosome alterations of the genomic region hybridized by the probe are identified as insertions, deletions, and translocations. These alterations are used as a diagnostic marker for an associated disease.

### Example 22: Method of Detecting Abnormal Levels of a Polypeptide in a Biological Sample

A polypeptide of the present invention can be detected in a biological sample, and if an increased or decreased level of the polypeptide is detected, this polypeptide is a marker for a particular phenotype. Methods of detection are numerous, and thus, it is understood that one skilled in the art can modify the following assay to fit their particular needs.

For example, antibody-sandwich ELISAs are used to detect polypeptides in a sample, preferably a biological sample. Wells of a microtiter plate are coated with specific antibodies, at a final concentration of 0.2 to 10 ug/ml. The antibodies are either monoclonal or polyclonal and are produced by the method described in Example 10. The wells are blocked so that non-specific binding of the polypeptide to the well is reduced.

The coated wells are then incubated for > 2 hours at RT with a sample containing the polypeptide. Preferably, serial dilutions of the sample should be used to validate results. The plates are then washed three times with deionized or distilled water to remove unbounded polypeptide.

Next, 50 ul of specific antibody-alkaline phosphatase conjugate, at a concentration of 25-400 ng, is added and incubated for 2 hours at room temperature. The plates are again washed three times with deionized or distilled water to remove unbounded conjugate.

Add 75 ul of 4-methylumbelliferyl phosphate (MUP) or p-nitrophenyl phosphate (NPP) substrate solution to each well and incubate 1 hour at room temperature. Measure the reaction by a microtiter plate reader. Prepare a standard curve, using serial dilutions of a control sample, and plot polypeptide concentration on the X-axis (log scale) and fluorescence or absorbance of the Y-axis (linear scale). Interpolate the concentration of the polypeptide in the sample using the standard curve.

### Example 23: Formulating a Polypeptide

The secreted polypeptide composition will be formulated and dosed in a fashion consistent with good medical practice, taking into account the clinical condition of the individual patient (especially the side effects of treatment with the secreted polypeptide alone), the site of delivery, the method of administration, the scheduling of administration, and other factors known to practitioners. The "effective amount" for purposes herein is thus determined by such considerations.

As a general proposition, the total pharmaceutically effective amount of secreted polypeptide administered parenterally per dose will be in the range of about 1 µg/kg/day to 10 mg/kg/day of patient body weight, although, as noted above, this will be subject to therapeutic discretion. More preferably, this dose is at least 0.01 mg/kg/day, and most preferably for humans between about 0.01 and 1 mg/kg/day for the hormone. If given continuously, the secreted polypeptide is typically administered at a dose rate of about 1 µg/kg/hour to about 50 µg/kg/hour, either by 1-4 injections per day or by continuous subcutaneous infusions, for example, using a mini-pump. An intravenous bag solution may also be employed. The length of treatment needed to observe changes and the interval following treatment for responses to occur appears to vary depending on the desired effect.

Pharmaceutical compositions containing the secreted protein of the invention are administered orally, rectally, parenterally, intracistemally, intravaginally, intraperitoneally, topically (as by powders, ointments, gels, drops or transdermal patch), bucally, or as an oral or nasal spray. "Pharmaceutically acceptable carrier" refers to a non-toxic solid, semisolid or liquid filler, diluent, encapsulating material or formulation auxiliary of any type. The term "parenteral" as used herein refers to modes of administration which include intravenous, intramuscular, intraperitoneal, intrasternal, subcutaneous and intraarticular injection and infusion.

The secreted polypeptide is also suitably administered by sustained-release systems. Suitable examples of sustained-release compositions include semi-permeable polymer matrices in the form of shaped articles, e.g., films, or mirocapsules. Sustained-release matrices include polylactides (U.S. Pat. No. 3,773,919, EP 58,481), copolymers of L-glutamic acid and gamma-ethyl-L-glutamate (Sidman, U. et al., Biopolymers 22:547-556 (1983)), poly (2- hydroxyethyl methacrylate) (R. Langer et al., J. Biomed. Mater. Res. 15:167-277 (1981), and R. Langer, Chem. Tech. 12:98-105 (1982)), ethylene vinyl acetate (R. Langer et al.) or poly-D- (-)-3-hydroxybutyric acid (EP 133,988). Sustained-release compositions also include liposomally entrapped polypeptides. Liposomes containing the secreted polypeptide are prepared by methods known per se: DE 3,218,121; Epstein et al., Proc. Natl. Acad. Sci. USA 82:3688-3692 (1985); Hwang et al., Proc. Natl. Acad. Sci. USA 77:4030-4034 (1980); EP 52,322; EP 36,676; EP 88,046; EP 143,949; EP 142,641; Japanese Pat. Appl. 83-118008; U.S. Pat. Nos. 4,485,045 and 4,544,545; and EP 102,324. Ordinarily, the liposomes are of the small (about 200-800 Angstroms) unilamellar type in which the lipid content is greater than about 30 mol. percent cholesterol, the selected proportion being adjusted for the optimal secreted polypeptide therapy.

For parenteral administration, in one embodiment, the secreted polypeptide is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation. For example, the formulation preferably does not include oxidizing agents and other compounds that are known to be deleterious to polypeptides.

Generally, the formulations are prepared by contacting the polypeptide uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non-aqueous vehicles such as fixed oils and ethyl oleate are also useful herein, as well as liposomes.

The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) polypeptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, manose, or dextrins; chelating agents such as EDTA; sugar alcohols such as mannitol or sorbitol; counterions such as sodium; and/or nonionic surfactants such as polysorbates, poloxamers, or PEG.

The secreted polypeptide is typically formulated in such vehicles at a concentration of about 0.1 mg/ml to 100 mg/ml, preferably 1-10 mg/ml, at a pH of about 3 to 8. It will be understood that the use of certain of the foregoing excipients, carriers, or stabilizers will result in the formation of polypeptide salts.

Any polypeptide to be used for therapeutic administration can be sterile. Sterility is readily accomplished by filtration through sterile filtration membranes (e.g., 0.2 micron membranes). Therapeutic polypeptide compositions generally are placed into a container having a sterile access port, for example, an intravenous solution bag or vial having a stopper pierceable by a hypodermic injection needle.

Polypeptides ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous polypeptide solution, and the resulting mixture is lyophilized. The infusion solution is prepared by reconstituting the lyophilized polypeptide using bacteriostatic Water-for-Injection.

The invention also provides a pharmaceutical pack or kit comprising one or more containers filled with one or more of the ingredients of the pharmaceutical compositions of the invention. Associated with such container(s) can be a notice in the form prescribed by a governmental agency regulating the manufacture, use or sale of pharmaceuticals or biological products, which notice reflects approval by the agency of manufacture, use or sale for human administration. In addition, the polypeptides of the present invention may be employed in conjunction with other therapeutic compounds.

### Example 24: Method of Treating Decreased Levels of the Polypeptide

It will be appreciated that conditions caused by a decrease in the standard or normal expression level of a secreted protein in an individual can be treated by administering the polypeptide of the present invention, preferably in the secreted form. Thus, the invention also provides a method of treatment of an individual in need of an increased level of the polypeptide comprising administering to such an individual a pharmaceutical composition comprising an amount of the polypeptide to increase the activity level of the polypeptide in such an individual.

For example, a patient with decreased levels of a polypeptide receives a daily dose 0.1-100 ug/kg of the polypeptide for six consecutive days. Preferably, the polypeptide is in the secreted form. The exact details of the dosing scheme, based on administration and formulation, are provided in Example 23.

### Example 25: Method of Treating Increased Levels of the Polypeptide

Antisense technology is used to inhibit production of a polypeptide of the present invention. This technology is one example of a method of decreasing levels of a polypeptide, preferably a secreted form, due to a variety of etiologies, such as cancer.

For example, a patient diagnosed with abnormally increased levels of a polypeptide is administered intravenously antisense polynucleotides at 0.5, 1.0, 1.5, 2.0 and 3.0 mg/kg day for 21 days. This treatment is repeated after a 7-day rest period if the treatment was well tolerated. The formulation of the antisense polynucleotide is provided in Example 23.

### Example 26: Method of Treatment Using Gene Therapy

One method of gene therapy transplants fibroblasts, which are capable of expressing a polypeptide, onto a patient. Generally, fibroblasts are obtained from a subject by skin biopsy. The resulting tissue is placed in tissue-culture medium and separated into small pieces. Small chunks of the tissue are placed on a wet surface of a tissue culture flask, approximately ten pieces are placed in each flask. The flask is turned upside down, closed tight and left at room temperature over night. After 24 hours at room temperature, the flask is inverted and the chunks of tissue remain fixed to the bottom of the flask and fresh media (e.g., Ham's F12 media, with 10% FBS, penicillin and streptomycin) is added. The flasks are then incubated at 37°C for approximately one week.

At this time, fresh media is added and subsequently changed every several days. After an additional two weeks in culture, a monolayer of fibroblasts emerge. The monolayer is trypsinized and scaled into larger flasks.

pMV-7 (Kirschmeier, P.T. et al., DNA, 7:219-25 (1988)), flanked by the long terminal repeats of the Moloney murine sarcoma virus, is digested with EcoRI and HindIII and subsequently treated with calf intestinal phosphatase. The linear vector is fractionated on agarose gel and purified, using glass beads.

The cDNA encoding a polypeptide of the present invention can be amplified using PCR primers which correspond to the 5' and 3' end sequences respectively as set forth in Example 1. Preferably, the 5' primer contains an EcoRI site and the 3' primer includes a HindIII site. Equal quantities of the Moloney murine sarcoma virus linear backbone and the amplified EcoRI and HindIII fragment are added together, in the presence of T4 DNA ligase. The resulting mixture is maintained under conditions appropriate for ligation of the two fragments. The ligation mixture is then used to transform bacteria HB 101, which are then plated onto agar containing kanamycin for the purpose of confirming that the vector has the gene of interest properly inserted.

The amphotropic pA317 or GP+am12 packaging cells are grown in tissue culture to confluent density in Dulbecco's Modified Eagles Medium (DMEM) with 10% calf serum (CS), penicillin and streptomycin. The MSV vector containing the gene is then added to the media and the packaging cells transduced with the vector. The packaging cells now produce infectious viral particles containing the gene (the packaging cells are now referred to as producer cells).

Fresh media is added to the transduced producer cells, and subsequently, the media is harvested from a 10 cm plate of confluent producer cells. The spent media, containing the infectious viral particles, is filtered through a millipore filter to remove detached producer cells and this media is then used to infect fibroblast cells. Media is removed from a sub-confluent plate of fibroblasts and quickly replaced with the media from the producer cells. This media is removed and replaced with fresh media. If the titer of virus is high, then virtually all fibroblasts will be infected and no selection is required. If the titer is very low, then it is necessary to use a retroviral vector that has a selectable marker, such as neo or his. Once the fibroblasts have been efficiently infected, the fibroblasts are analyzed to determine whether protein is produced.

The engineered fibroblasts are then transplanted onto the host, either alone or after having been grown to confluence on cytodex 3 microcarrier beads.

### Example 27: Method of Treatment Using Gene Therapy - In Vivo

Another aspect of the present invention is using *in vivo* gene therapy methods to treat disorders, diseases and conditions. The gene therapy method relates to the introduction of naked nucleic acid (DNA, RNA, and antisense DNA or RNA) sequences into an animal to increase or decrease the expression of the polypeptide of the present invention. A polynucleotide of the present invention may be operatively linked to a promoter or any other genetic elements necessary for the expression of the encoded polypeptide by the target tissue. Such gene therapy and delivery techniques and methods are known in the art, see, for example, W090/11092, W098/11779; U.S. Patent NO. 5693622, 5705151, 5580859; Tabata H. et al. (1997) Cardiovasc. Res. 35(3):470-479, Chao J et al. (1997) Pharmacol. Res. 35(6):517-522, Wolff J.A. (1997) Neuromuscul. Disord. 7(5):314-318, Schwartz B. et al. (1996) Gene Ther. 3(5):405-411, Tsurumi Y. et al. (1996) Circulation 94(12):3281-3290 (incorporated herein by reference).

The polynucleotide constructs of the present invention may be delivered by any method that delivers injectable materials to the cells of an animal, such as, injection into the interstitial space of tissues (heart, muscle, skin, lung, liver, intestine and the like). These polynucleotide constructs can be delivered in a pharmaceutically acceptable liquid or aqueous carrier.

The term "naked" polynucleotide, DNA or RNA, refers to sequences that are free from any delivery vehicle that acts to assist, promote, or facilitate entry into the cell, including viral sequences, viral particles, liposome formulations, lipofectin or precipitating agents and the like. However, the polynucleotides may also be delivered in liposome formulations (such as those taught in Felgner P.L. et al. (1995) Ann. NY Acad. Sci. 772:126-139 and Abdallah B. et al. (1995) Biol. Cell 85(1):1-7) which can be prepared by methods well known to those skilled in the art.

The polynucleotide vector constructs of the present invention used in the gene therapy method are preferably constructs that will not integrate into the host genome nor will they contain sequences that allow for replication. Any strong promoter known to those skilled in the art can be used for driving the expression of DNA. Unlike other gene therapies techniques, one major advantage of introducing naked nucleic acid sequences into target cells is the transitory nature of the polynucleotide synthesis in the cells. Studies have shown that non-replicating DNA sequences can be introduced into cells to provide production of the desired polypeptide for periods of up to six months.

The polynucleotide construct of the present invention can be delivered to the interstitial space of tissues within the an animal, including of muscle, skin, brain, lung, liver, spleen, bone marrow, thymus, heart, lymph, blood, bone, cartilage, pancreas, kidney, gall bladder, stomach, intestine, testis, ovary, uterus, rectum, nervous system, eye, gland, and connective tissue. Interstitial space of the tissues comprises the intercellular fluid, mucopolysaccharide matrix among the reticular fibers of organ tissues, elastic fibers in the walls of vessels or chambers, collagen fibers of fibrous tissues, or that same matrix within connective tissue ensheathing muscle cells or in the lacunae of bone. It is similarly the space occupied by the plasma of the circulation and the lymph fluid of the lymphatic channels. Delivery to the interstitial space of muscle tissue is preferred for the reasons discussed below. They may be conveniently delivered by injection into the tissues comprising these cells. They are preferably delivered to and expressed in persistent, non-dividing cells which are differentiated, although delivery and expression may be achieved in non-differentiated or less completely differentiated cells, such as, for example, stem cells of blood or skin fibroblasts. *In vivo* muscle cells are particularly competent in their ability to take up and express polynucleotides.

For the naked polynucleotide injection, an effective dosage amount of DNA or RNA will be in the range of from about 0.05 g/kg body weight to about 50 mg/kg body weight. Preferably the dosage will be from about 0.005 mg/kg to about 20 mg/kg and more preferably from about 0.05 mg/kg to about 5 mg/kg. Of course, as the artisan of ordinary skill will appreciate, this dosage will vary according to the tissue site of injection. The appropriate and effective dosage of nucleic acid sequence can readily be determined by those of ordinary skill in the art and may depend on the condition being treated and the route of administration. The preferred route of administration is by the parenteral route of injection into the interstitial space of tissues. However, other parenteral routes may also be used, such as, inhalation of an aerosol formulation particularly for delivery to lungs or bronchial tissues, throat or mucous membranes of the nose. In addition, naked polynucleotide constructs can be delivered to arteries during angioplasty by the catheter used in the procedure.

The dose response effects of injected polynucleotide in *muscle in vivo* is determined as follows. Suitable template DNA for production of mRNA coding for the polypeptide of the present invention is prepared in accordance with a standard recombinant DNA methodology. The template DNA, which may be either circular or linear, is either used as naked DNA or complexed with liposomes. The quadriceps muscles of mice are then injected with various amounts of the template DNA.

Five to six week old female and male Balb/C mice are anesthetized by intraperitoneal injection with 0.3 ml of 2.5% Avertin. A 1.5 cm incision is made on the anterior thigh, and the quadriceps muscle is directly visualized. The template DNA is injected in 0.1 ml of carrier in a 1 cc syringe through a 27 gauge needle over one minute, approximately 0.5 cm from the distal insertion site of the muscle into the knee and about 0.2 cm deep. A suture is placed over the injection site for future localization, and the skin is closed with stainless steel clips.

After an appropriate incubation time (e.g., 7 days) muscle extracts are prepared by excising the entire quadriceps. Every fifth 15 um cross-section of the individual quadriceps muscles is histochemically stained for protein expression. A time course for protein expression may be done in a similar fashion except that quadriceps from different mice are harvested at different times. Persistence of DNA in muscle following injection may be determined by Southern blot analysis after preparing total cellular DNA and HIRT supernatants from injected and control mice. The results of the above experimentation in mice can be use to extrapolate proper dosages and other treatment parameters in humans and other animals using naked DNA of the present invention.

It will be clear that the invention may be practiced otherwise than as particularly described in the foregoing description and examples. Numerous modifications and variations of the present invention are possible in light of the above teachings and; therefore, are within the scope of the appended claims.

The entire disclosure of each document cited (including patents, patent applications, journal articles, abstracts, laboratory manuals, books, or other disclosures) in the Background of the Invention, Detailed Description, and Examples is hereby incorporated herein by reference.

Sequence Listing

(1) GENERAL INFORMATION:

(i) APPLICANT: Human Genome Sciences, Inc., et al.

(ii) TITLE OF INVENTION: 207 Human Secreted Proteins

(iii) NUMBER OF SEQUENCES: 800

(iv) CORRESPONDENCE ADDRESS:

(A) ADDRESSEE: Human Genome Sciences, Inc.

(B) STREET: 9410 Key West Avenue

(C) CITY: Rockville

(D) STATE: Maryland

(E) COUNTRY: USA

(F) ZIP: 20850

(v) COMPUTER READABLE FORM:

(A) MEDIUM TYPE: Diskette, 3.50 inch, 1.4Mb storage

(B) COMPUTER: HP Vectra 486/33

(C) OPERATING SYSTEM: MSDOS version 6.2

(D) SOFTWARE: ASCII Text

(vi) CURRENT APPLICATION DATA:

(A) APPLICATION NUMBER:

(B) FILING DATE:

(C) CLASSIFICATION:

(vii) PRIOR APPLICATION DATA:

(A) APPLICATION NUMBER:

(B) FILING DATE:

(viii) ATTORNEY/AGENT INFORMATION:

(A) NAME: Kenley K. Hoover

(B) REGISTRATION NUMBER: 40,302

(C) REFERENCE/DOCKET NUMBER: PZ007PCT

(vi) TELECOMMUNICATION INFORMATION:

(A) TELEPHONE: (301) 309-8504

(B) TELEFAX: (301) 309-8439

(2) INFORMATION FOR SEQ ID NO: 1:

(i) SEQUENCE CHARACTERISTICS:
(A) LENGTH: 733 base pairs
(B) TYPE: nucleic acid
(C) STRANDEDNESS: double
(D) TOPOLOGY: linear

(xi) SEQUENCE DESCRIPTION: SEQ ID NO: 1:

GGGATCCGGA GCCCAAATCT TCTGACAAAA CTCACACATG CCCACCGTGC CCAGCACCTG 60

AATTCGAGGG TGCACCGTCA GTCTTCCTCT TCCCCCCAAA ACCCAAGGAC ACCCTCATGA 120

TCTCCCGGAC TCCTGAGGTC ACATGCGTGG TGGTGGACGT AAGCCACGAA GACCCTGAGG 180

TCAAGTTCAA CTGGTACGTG GACGGCGTGG AGGTGCATAA TGCCAAGACA AAGCCGCGGG 240

AGGAGCAGTA CAACAGCACG TACCGTGTGG TCAGCGTCCT CACCGTCCTG CACCAGGACT 300

GGCTGAATGG CAAGGAGTAC AAGTGCAAGG TCTCCAACAA AGCCCTCCCA ACCCCCATCG 360

AGAAAACCAT CTCCAAAGCC AAAGGGCAGC CCCGAGAACC ACAGGTGTAC ACCCTGCCCC 420

CATCCCGGGA TGAGCTGACC AAGAACCAGG TCAGCCTGAC CTGCCTGGTC AAAGGCTTCT 480

ATCCAAGCGA CATCGCCGTG GAGTGGGAGA GCAATGGGCA GCCGGAGAAC AACTACAAGA 540

CCACGCCTCC CGTGCTGGAC TCCGACGGCT CCTTCTTCCT CTACAGCAAG CTCACCGTGG 600

ACAAGAGCAG GTGGCAGCAG GGGAACGTCT TCTCATGCTC CGTGATGCAT GAGGCTCTGC 660

ACAACCACTA CACGCAGAAG AGCCTCTCCC TGTCTCCGGG TAAATGAGTG CGACGGCCGC 720

GACTCTAGAG GAT 733

(2) INFORMATION FOR SEQ ID NO: 2:

(i) SEQUENCE CHARACTERISTICS:
(A) LENGTH: 5 amino acids
(B) TYPE: amino acid
(D) TOPOLOGY: linear

(xi) SEQUENCE DESCRIPTION: SEQ ID NO: 2:

Trp Ser Xaa Trp Ser
1               5

(2) INFORMATION FOR SEQ ID NO: 3:

(i) SEQUENCE CHARACTERISTICS:
(A) LENGTH: 86 base pairs
(B) TYPE: nucleic acid
(C) STRANDEDNESS: double
(D) TOPOLOGY: linear

(xi) SEQUENCE DESCRIPTION: SEQ ID NO: 3:

GCGCCTCGAG ATTTCCCCGA AATCTAGATT TCCCCGAAAT GATTTCCCCG AAATGATTTC 60

CCCGAAATAT CTGCCATCTC AATTAG 86

(2) INFORMATION FOR SEQ ID NO: 4:

(i) SEQUENCE CHARACTERISTICS:
(A) LENGTH: 27 base pairs
(B) TYPE: nucleic acid
(C) STRANDEDNESS: double
(D) TOPOLOGY: linear

(xi) SEQUENCE DESCRIPTION: SEQ ID NO: 4:

GCGGCAAGCT TTTTGCAAAG CCTAGGC 27

(2) INFORMATION FOR SEQ ID NO: 5:

(i) SEQUENCE CHARACTERISTICS:
(A) LENGTH: 271 base pairs
(B) TYPE: nucleic acid
(C) STRANDEDNESS: double
(D) TOPOLOGY: linear

(xi) SEQUENCE DESCRIPTION: SEQ ID NO: 5:

CTCGAGATTT CCCCGAAATC TAGATTTCCC CGAAATGATT TCCCCGAAAT GATTTCCCCG 60

AAATATCTGC CATCTCAATT AGTCAGCAAC CATAGTCCCG CCCCTAACTC CGCCCATCCC 120

GCCCCTAACT CCGCCCAGTT CCGCCCATTC TCCGCCCCAT GGCTGACTAA TTTTTTTTAT 180

TTATGCAGAG GCCGAGGCCG CCTCGGCCTC TGAGCTATTC CAGAAGTAGT GAGGAGGCTT 240

TTTTGGAGGC CTAGGCTTTT GCAAAAAGCT T 271

(2) INFORMATION FOR SEQ ID NO: 6:

(i) SEQUENCE CHARACTERISTICS:
(A) LENGTH: 32 base pairs
(B) TYPE: nucleic acid
(C) STRANDEDNESS: double
(D) TOPOLOGY: linear

(xi) SEQUENCE DESCRIPTION: SEQ ID NO: 6:

GCGCTCGAGG GATGACAGCG ATAGAACCCC GG 32

(2) INFORMATION FOR SEQ ID NO: 7:

(i) SEQUENCE CHARACTERISTICS:
(A) LENGTH: 31 base pairs
(B) TYPE: nucleic acid
(C) STRANDEDNESS: double
(D) TOPOLOGY: linear

(xi) SEQUENCE DESCRIPTION: SEQ ID NO: 7:

GCGAAGCTTC GCGACTCCCC GGATCCGCCT C 31

(2) INFORMATION FOR SEQ ID NO: 8:

(i) SEQUENCE CHARACTERISTICS:
(A) LENGTH: 12 base pairs
(B) TYPE: nucleic acid
(C) STRANDEDNESS: double
(D) TOPOLOGY: linear

(xi) SEQUENCE DESCRIPTION: SEQ ID NO: 8:

GGGGACTTTC CC 12

(2) INFORMATION FOR SEQ ID NO: 9:

(i) SEQUENCE CHARACTERISTICS:
(A) LENGTH: 73 base pairs
(B) TYPE: nucleic acid

(C) STRANDEDNESS: double
(D) TOPOLOGY: linear

(xi) SEQUENCE DESCRIPTION: SEQ ID NO: 9:

GCGGCCTCGA GGGGACTTTC CCGGGGACTT TCCGGGGACT TTCCGGGACT TTCCATCCTG 60

CCATCTCAAT TAG 73

(2) INFORMATION FOR SEQ ID NO: 10:

(i) SEQUENCE CHARACTERISTICS:
(A) LENGTH: 256 base pairs
(B) TYPE: nucleic acid
(C) STRANDEDNESS: double
(D) TOPOLOGY: linear

(xi) SEQUENCE DESCRIPTION: SEQ ID NO: 10:

CTCGAGGGGA CTTTCCCGGG GACTTTCCGG GGACTTTCCG GGACTTTCCA TCTGCCATCT 60

CAATTAGTCA GCAACCATAG TCCCGCCCCT AACTCCGCCC ATCCCGCCCC TAACTCCGCC 120

CAGTTCCGCC CATTCTCCGC CCCATGGCTG ACTAATTTTT TTTATTTATG CAGAGGCCGA 180

GGCCGCCTCG GCCTCTGAGC TATTCCAGAA GTAGTGAGGA GGCTTTTTTG GAGGCCTAGG 240

CTTTTGCAAA AAGCTT 256

(2) INFORMATION FOR SEQ ID NO: 11:

(i) SEQUENCE CHARACTERISTICS:
(A) LENGTH: 2526 base pairs
(B) TYPE: nucleic acid
(C) STRANDEDNESS: double
(D) TOPOLOGY: linear

(xi) SEQUENCE DESCRIPTION: SEQ ID NO: 11:

GACAGGCTAT CCGAGAATCT GAGAGCTGGG CCCGGCAATT CCTCCAGYTA CCCTTGTGAC 60

CTAAGTCCAG TCACACATTT CCCAAAGTTT CTCTTTGTCA TAACCCTGGT CTGGCTGGTT 120

TTGRGGRCTT GAGAATGGGT CAGGGACTCC AGGCCAAGTC CAACAGAGAC CCCAAACCCA 180

CCACACACCA GCAGCCACAA CCTCACCACC AACAAAGAGG ACTTTTGTGG GGCCACAAGT 240

AAGAGGTCAT TTCTGGAATG GACTCAGACC TTTAAACAGG AGAGTTGAGC ACTTCCAGKS 300

AGTTTTTAAG CAAGGCATGG GGAACAGGGA ATAGAACCTT TCAAAGAGGT TGCCCAGAGA 360

AAAGCTGGGC CTCTTGCATT CGGCTTCCTT GGAGCAGCCT CTTCTGGCAG AAAGCCATCA 420

GGTGCTCAAT CATCTTCTCC TGGCCAAGGC TCTGACCATG CTTAGTACTG GAATAGAGGT 480

GGCCAGGCCC CCAGCGACTC TTCTTGGCCT GATGTTTGTC CTCACAGGCA TGCCACGTGG 540

CCTGAGATGA TTCAGAACAA ATCATGCTAA CTTTGAATCC ATCCAGCCAC TTGCAAATGA 600

TAATCAGAAG TCAGCTTGTT CACTGTTAGA AAGAAACTAA CAAAAGAGAA CCCAGAGCAA 660

TCTAGAATCT TTGAGTGCTT GGCTTTCCAA GGATACTGCG GAGACTCTGG CCAAGCTGAT 720

GAMCTTCTGA ARTGTCACTG GCACCATATG CAACAAGAAC CACCATTCAC TGAGTAGCTA 780

ATGGGTTTGG GGCCTGGGAC ATTCCATCTG AGGTCCTTCC TGAACATGTC ACTCCACAGC 840

AGAGGACCGG TTGCAGCTTA CCCAGAACCA CTCCTCCAGG AGAGCTGGAT GTTTTGCGTG 900

CAACACCTTG AGCACTGACT GCTATTGTTC AAAAAAAGCC TTTGCTGCAT TCGGAGGACT 960

GCCCCGTGCC CTGAGGTGAC TTCCTAACTA TGTGGTTTCA TTAGCGAATT TATTTTTTGT 1020

GCTGGGTGGA CATTTGTATT TTGTTAGGTT GCTGTTTAAG CTCAAGTTTG CTGTGCTCTC 1080

TGCAGCTACA AAACATCTTG GCATATTTAA GAKTGGCTTT TATAAATAGC TTTATTCTGA 1140

TATTAATCAG ATTCCCAACT TTACTGAGAA TTAAGGACTG GGGTACTTTA AAGAAATGCA 1200

AATAGCAATT GAAGAACCAC TGCTGCAGGT GGTAGCCCTG GCTAGACTGA ATTACACTAG 1260

AAATCAGCCA GAAGGAAGCG TCCTTGGGAT CCCAGATCAC TCTTTTTTTT TTTTTTTTTA 1320

AAAGGGGCAG CCCCTTGATG GCTCATCTCT CTGAATAACA GTTACGTCTT CATATCGATA 1380

CCAGATGCCT TCTTCATCAT GCCACTGAAG CCACTCACCA CCTTCAAGAA CATGCCAACC 1440

TCTGTCAGAT TCACTTACCC ACAAACAAGG AGGCACGTTT GGCACAAAGT GTTGTCCTCC 1500

AGGTCCAAGT GGACTCTACA GAGTGCTTGA CCTCAACACA CTGGATTCCA GGTGGACTGG 1560

ACCAAGAGCA GGCAAAGACA CGGGAACTGA AAAACTCCAC AGGGTTTGGA GAATAGAAAT 1620

GAAAAGCCAC GTCATATAAC TCAAGAATAA ATGGTGTTTT GGAAATTTTA AAATTATCAT 1680

CGAAGGTGGT GAAACTATTT CAGGCCCAAA TGAAAGGAAA TCGCCAGTTG GGGATGAAAT 1740

CACAGAGCCT GTGTTTTATG ATATGGTTGG ATGTCCACTG ATGAAATTTT AAAGGAGTTT 1800

CATTTTTAAA AGTGCGCATG ATTCTACATA TGAGAATTCT TTAGGCCAAG AAACTGTCCT 1860

TGGCTCAGAG GTGTTGGGAA TTAAAGCAGA GAGAAGCCAT TCGTGATGCT TAGAACCAAG 1920

GATGGTCATG TACACAAAGA CCATCGAGAC GGCCATTCTT GTTTACAAAA CACTTACCAA 1980

GAAAGCACTT TGTAGGGGAA CTTTAGTAAG TTCTTCTCAT TTCATTATGT TTCTTCCAAG 2040

GAAACAGGAG AGACTGAATT AATAATTCTC TCTTTCCTCT TAAGCACTTT TAAAATAATA 2100

AAGTACATCT TGAAATTTGG GGGGGCATCT CTGATTTAAA AAAAGAAAAA GGCTGCTTGA 2160

TGTATGTTAT GCAGAGACAC TCTGCCTCTG GTGGCTGCAG AGCAATACCC AAGCCTCATT 2220

TGGAAGGCTC AACATTTGGA ATTGCACTTT AATTGATTAA TCCTCAATTC ATGTGGCCTT 2280

ACGGGATGGT GGGTCTGGGA CCCCAATTCA TTCTTATCTG CCAAAGAATT ATCTAGAAGC 2340

ACATCAAATA CCAGCACCCC ACCTGCACAA TGGGGGTGGA AAACTTTTGT ATCCCTAAGC 2400

ATATTATTTT ATAGTGTCTG CCATGCCATG TGGAAATACT TTATTTTTAA CCTCAGGATT 2460

TAAATAAAGT AAACACTATG ACATTTAAAA AAAAAAAAAA AAAACTCGAG GGGGGCCCGG 2520

TACCCA 2526

(2) INFORMATION FOR SEQ ID NO: 12:

(i) SEQUENCE CHARACTERISTICS:
(A) LENGTH: 1131 base pairs
(B) TYPE: nucleic acid
(C) STRANDEDNESS: double
(D) TOPOLOGY: linear

(xi) SEQUENCE DESCRIPTION: SEQ ID NO: 12:

CACTGCACCA GCTTTGTTAT CTGTAAAATG ATGATAATAC CAACACCTTC TTCTTGGGGT 60

ACTGAAGATG AGAGAACATG ATATGTGTAA AGTGCCTTCC ACAATACCCA GAACATAGCA 120

AACATGTAAT GAATGTAGTA ATAGTAATTA TTTTATTTTC TTTTGATTCA GTTGGGACTA 180

TGTTCAGCTG TAACAGAATA CCCAAAATAA CTGTTTTAAA CAAATTAAAG TTTWGTTGTG 240

AAGTTTTGTT ACGAATTCAG ACAATCCAGG GCTTTTATAG ATGCACCAGG ATCAGCAGGT 300

ACAAAGGCAT CTTTCCTGAT TTCTGCCAGT CTCAATGCAT GGGTTGCAAT CCAGARTCCA 360

RGATGGCAGT TCCAGCCCTG GTTACGCCCA TATTAGCACA CAGAAAGAAA GAGAAAGGGA 420

TGTGCCTCTT CACTTTAATC ATAGCTCCCA CTAGATGCAC CCACTACTTC TGCTGATACT 480

CCATTAGCTA ATGCTTGCTT ACATGGTCAC ACTTAGTTTC CAGAGAGACA TGTCTGGACA 540

GTCATGTGCT CAATTAATAT CCAAGTGTCC AATTACTGAG AAAAAAAGAA ACTAGCACCT 600

TTGCTTGGTT GCATTCCTCT TAGCATAAGC CACATTCTTT TTATGAAGTT GTCCTCAGTT 660

ACTTGGATGC CTCAGTTGTC CTTTCAWTTA GAAAWGCYCC TKGGACAYCC TGAAWCTGAC 720

TTCTTTTGTC ATCAGCACCA TCACTACCAC TGCCYTCTTC AAAGCCACCA CGTTCTGTCC 780

CCAGGATGGT TGCAACAACC ACCATAGGGA CTTTTTGCCT TCTACTTCCA CACAATAGNC 840

CAGAGTAAGC TTTTGAAAAT GTAGGTCAGA TCATGTCTCT CTCTTCCTCT TCAAAACCCT 900

CCCGATGGCT TTTCATATTA CTCAAAAGAA AACCTAAAAC TTTGCTGTGA GATCTATGTG 960

ACCCGGCTTA TTCTTCCTCT TACTTTATCT CTGTATTGCT CTTCCTCACT CTACTCCAGC 1020

CATCCCACCT CCTTGCTGCT TGTCCTATAC TCCTAAAAGA AGTTCAGTCT TCCCTTATGA 1080

TATTTGCACT TAAAATAGAA AAAAAAAAAA AAAAAAAACT CGAGGGGGGC C 1131

(2) INFORMATION FOR SEQ ID NO: 13:

(i) SEQUENCE CHARACTERISTICS:
(A) LENGTH: 941 base pairs
(B) TYPE: nucleic acid
(C) STRANDEDNESS: double
(D) TOPOLOGY: linear

(xi) SEQUENCE DESCRIPTION: SEQ ID NO: 13:

GGCACGAGTA GCATTTCATT TAATCTGCAG GTATATTCTC CCAACAGTTT ATTGTCATGT 60

GATGTCCTCA GCCAAGATTG TRAGGCAGAG AGGAGCTGTC CCAACCTACT ATACCACCGA 120

GGCTGGAGAG ATCATATTTT TGGTATTAAA CTGGAGTCTC TCCATCCTTC ACATTGTTGA 180

TGTCCTCTGT AGCAAACCGG AAAAGTCAGT GACAGAAGAT GCCGCTAGCG GTTTGAGCCA 240

GAGAATGACA GCTCTGGTTT GGAGAAAAGG GCCGGATGGT GGCTCTAGAA AGCCCATCCT 300

TCTGCTCTTC TTTTTTCTCC CCCTTATATT GTGCTTTCAT TCATTCATTC ATTCATCAAA 360

CATTTGTTGA GCACCTATTA TGTGTCAAGC TCTGTGCTAG CCTCTGGAAA ACCTGCCCTC 420

ATGTAGCTCA CTGTGGAGTA GGAGAAACAA TGACTACACT ATGATAAGCA CGGGTTGTCA 480

GGGTCTCACA GAGCAGTGGC CCCTCATCCA GACCGATGAG GTCAAAGAAG GCATCCAGGC 540

GAGGATGGTG TCAGAGCTAA CTGAAGAATG AGAGGGAGCT GCACCASCAG GGGTTGGAAC 600

TGAAGGTGGC AGTGCCTGGA GTCTTGATTC CAGCAGAGGG AGAGCAGTCT GTGAAAAGGC 660

ACCAAGGGTG GGAGAGGGCA GAGCACATGG AGGAACTTCA GGTAGTTCTG GATGGCSCTG 720

GGGCAAAGCT AGAGAGGTAA GAAGAATCTA CAAATGTTCC TCGAGTTACA TGAACTTCCA 780

TCCCAATAAA CCCATTGGAA ACGAAAAATT TAAGTCAGAA GTGCATTTAA GGCTGGTCCG 840

AGTAGAATGA TTTTTACAAC GAATTGATCA CAACCAGTTA CAGATGTCTT TGTTCCTTCT 900

CCACTCCCAC TGCTTCACCT GACTAGCCTT TAAAAAAAAA A 941

(2) INFORMATION FOR SEQ ID NO: 14:

(i) SEQUENCE CHARACTERISTICS:
(A) LENGTH: 843 base pairs
(B) TYPE: nucleic acid
(C) STRANDEDNESS: double
(D) TOPOLOGY: linear

(xi) SEQUENCE DESCRIPTION: SEQ ID NO: 14:

CNAGGGATAA CCCCAAAGNT GGGAAATAAA CCCTCAATTA AAGGGGGAAC CAAAAAGCTG 60

GGAAGTTCCC CCCCGCGGTG GCGGCCNGNT CTAGGAACTA GTGGAATCCC CCGGGGCTGC 120

AGGGAATTCG GCACGGAGTG GGAATGTTGT TTGTATGATA CTATTTCCAC AAWATGCATT 180

GAGACTTGGT KTGTGGCCTA GGACATGGTC AATTCTTTYT AAATATTCCG TGAATTTCTT 240

TAGTGCATAT TCTCCGATGG GGGCTGTGGG GACAGAGTTC TAAATATGCC CATTAGATTA 300

AATCTCTTCA TTCTGTTGCT CACATCTTCT ATATCCTTAT TAATCTGTCA ATCTCTTCAA 360

GAGAGGTGTT ATTAAAATCT CTCACTGTAT GTGTCACTTT GCCCTTAAAA TTCTGATGAT 420

TTGCTTTATA AATGGTTATA ACCATTTTCC AGGAAGAACA TTAAAGAACT TTCCATTGGC 480

ATTATCCAGT TTCCCTCAAA ATACTGGTTT TTTTTATTTT GGCTNCTAAG CAGCTATGAA 540

TCCAGTTTCT CAGAAGCCCT TGTCTCAAGG CATTTGTTTC CAGATTACCT TGTTAGCATC 600

CACACTATGG GCTATTTTAG AAAAACAAAA AAAGTATCAA AATCATATAG CTATGATTTT 660

CCTGTGCTTG AAGGAGCCTT AAAGCTCATC TAGTCCAGCC AGTATTTGTT CATCCAAATT 720

CTGCCAAGAA ATCTCTATTG TCAAGATATT CTTTACCATC TTTGGGACAT TCTCATTATT 780

AGAAACAAAT CCTAAGAAGA AATTCTGCCA TAKACAACCC ATCCGTTCTT TAAAAAAAAA 840

AAA 843

(2) INFORMATION FOR SEQ ID NO: 15:

(i) SEQUENCE CHARACTERISTICS:
(A) LENGTH: 1018 base pairs
(B) TYPE: nucleic acid
(C) STRANDEDNESS: double
(D) TOPOLOGY: linear

(xi) SEQUENCE DESCRIPTION: SEQ ID NO: 15:

CTGTAATTTT TAATTTTCAT ATACCGTGCT TTGATTCTAA TTTTATTTTT TGAGTTCTCT 60

GAAGGTTACA TATACAGAGT GCTTCAGGAA TGATCATTTT GTTATTATTC ATGCTTCTTA 120

ACAATGTTGT TTTAGTCCAA GAAGATAATT GCCAGAGAAA GAATACAGTG CAGGAAAGAA 180

GARGCTGGAG CCAGTGGTGA AGARGGATTG AGARGACAGA CATTGTGGGA ATGAAATCAT 240

GAATAATCGT GTTTTTGAAT TGTCCAAAAA CTTCTACAAA CCATGAAATG TTGGAGTTTA 300

AATCTAATTG TTGAAAAATT CCCCACATTC CTTGTATCCC TTAGGTTGAG CATAATTCCA 360

CATCCGTGGA CTGATGCACT TCCCAAGAGG GGGCCTCATT AACTCTTCCG AGGCAGCAGC 420

AGCAAGGGCA CCCCCTCCTT TCCCCCCACA CCCCAYTTCT CATGGCTCTT CTTTCTCTCA 480

TCTCATGCTT AGGTTAGAAA AGGGCACAAG GTAAGGAAGC CCTTGGGAAT AGGCTGAATC 540

TGGCTATCTA ATTTGGTGCC AAATACTTAA TGTGCTTGAA TTTAAAAACA GCAAACATGT 600

AGAAAGGTAA TTATAATTAT GAGGCCAGTT CTTTAAGCTA GCTTTTTTTC CCCTCTCAAA 660

CAGCATATTG GCTTGGATGT CAGCAGGAGA AAGTGTTTTT TGCAATACAC ATAATGCATA 720

TATGGTCCTG TTAGCAATCT ATAGAAAATA GATATTGCTC ATTAAGGTAA ATATTTTTGT 780

TGATGAATGA TCTGGAATGG TCTGGACTTG TTGTGTGAAC AGGAAATTGC TCTGTAGGCT 840

TTGACTTGTG AGGTAAAGAG TGAGGCTGGT AAGATTAATT AAAGTAAATA CTGTGACAAT 900

AGGATGTCAA AACCAAAAAC GTGTTTCTGA AACTCAAGGA ATTAATGACA CATAGGGAAG 960

TTTTTGCCAT ATTAAGCATA GAGTAGGAGA GGCAAGTCAA GAATAAAAAA AAAAAAAA 1018

(2) INFORMATION FOR SEQ ID NO: 16:

(i) SEQUENCE CHARACTERISTICS:
(A) LENGTH: 661 base pairs
(B) TYPE: nucleic acid
(C) STRANDEDNESS: double
(D) TOPOLOGY: linear

(xi) SEQUENCE DESCRIPTION: SEQ ID NO: 16:

TTTAAGAAAT TAGTGAATCC CCGGNTGCAG GGAATTCGGC ACGAGGAGGA GGCCGTCAGC 60

TGGCAGGAGC GCAGGATGGC AGCTGYTCCC CCGGGTTGCA CCCCCCCAGY TCTGCTGGAC 120

ATAAGYTGGT TAACAGAGAG CCTGGGAGCT GGGCAGCCTG TACCTGTGGA GTGCCGGCAC 180

CGCCTGGAGG TGGCTGGGCC AAGGAAGGGG CCTCTGAGCC CAGCATGGAT GCCTGCCTAT 240

GCCTGCCAGC GCCCTACGCC CCTCACACAC CACAACACTG GCCTMTCCGA GCTGCTGGAG 300

CATGGAGTGT GTGAGGAGGT GGAGAGAGTT CGGCGCTCAG AGAGGTACCA GACCATGAAG 360

GTGCGCAGGG CAGGGCTCGG ACCTACCCCA GGAATGTCCT GCCCTGGGAA TGACAACACA 420

GTCCACACCA TGCACGGGGA GGCAAACAGG GGCAGCTGAC CCAGCCCAGG GGTCAGANGA 480

GGTCTTGCCG AGGAAGTGGC AGCTAAGCTG ATACCTGATA TGCACWAGKC AGCCARGYGG 540

AGACAGGCAA GGAAGAAGCT TGTTTTGAGG ACAGAATTTT CTAGATCACT CAGCACCATC 600

TGGCTTTTGG GGCTTTTTGT TTTATTTTGT TTTTGAGACG GGGTCTCGCT CTGTCGCCCA 660

N 661

(2) INFORMATION FOR SEQ ID NO: 17:

(i) SEQUENCE CHARACTERISTICS:

(A) LENGTH: 553 base pairs
(B) TYPE: nucleic acid
(C) STRANDEDNESS: double
(D) TOPOLOGY: linear

(xi) SEQUENCE DESCRIPTION: SEQ ID NO: 17:

GGCACAGGGC TATTTGCCCC TCTCTCCACA TGACAGAACT GCTCTAAGTT TCTTTGCTGC 60

TCTTCTCAGC TGTCAGACGG CTTGCTGCTT GTTTTCCACA CCACCATGTC TATTCTTTGC 120

TGTCCTTWAC TCTGCCTGTT TTTTTCCTTT TGTATTTCTT CTGGCTCTTG TCCCTTTTCC 180

CACGTGTCWC AGCTTTCCTT TATTGCCACT TTCAGTCAGA GCAGTCCTGT GCTTCTGGTG 240

CCGGCATACA ATACTTACTT GAGTTTCTTG GCTTTTCTTG ACTGTGCATC TCTTACTTCA 300

ACATAGGAAT AGCCTGTCAT AGAATTTCTC CAGTTCCAGG GCTCAAGAGG GAGAGTGCCA 360

GAAAATTGAG ACTGTTTTCC CTGTCTTGGA TTGAATTCAT AAAGCAAAAC CAGTGTTTGT 420

GTGAGGGTTT GCTGTGTCAT GCCTATAGGT TGTTTGGGTG CAAACCTATA GAATCCAGCC 480

TGCGAAAAGA AAGRAACCAG AGAATANCAG CATCAGAACA ATGCTTGACA TCATTTCTCA 540

ATCAAGCAGT CCA 553

(2) INFORMATION FOR SEQ ID NO: 18:

(i) SEQUENCE CHARACTERISTICS:
(A) LENGTH: 869 base pairs
(B) TYPE: nucleic acid
(C) STRANDEDNESS: double
(D) TOPOLOGY: linear

(xi) SEQUENCE DESCRIPTION: SEQ ID NO: 18:

GGCACGAGCT GCCAACACTG AGGTCTTCGT GGCTTCTCAC ATCTAGATGT ATCCCTCTCA 60

AATCTATCCT CTATCCAGGC ACCAGATTGA GGTATCTAAA ATGTCAACTT TCCAGTTACT 120

CCTTCTTATA CTAGCCCAAT CAACTTACAA GATAAAGTCC AAGCCCCTTC ATATGACAAA 180

CCACACCCTG CTTAACTCTC CAGGTTTGAA TCCTTCATCT CCTACTTTAA ACTTTAAAAC 240

CCAGCAGCAC GAAAGTGTCT CCTATGCATG TTGCCATATG CGTTCTCTCC ATCATGCATT 300

TGCCTGAGCA AGATGTCTTG AGTTAACATC TTATTCTTTA AGACTCATTG TGGTGGTAGA 360

CAGCCTTTAA TAACGGATCC TTGGCCAGGC ACAGTGACTC ACACCTGTAA TCCCAGAACT 420

TTGAAAGGCC AAAGAAGGAA GAAAGCTTGA GGCCAGTAGT TTGAGACCAG CCTGGGAAAC 480

AGAGAGATAT CCCATCTGTA CCAAAAATTT AAAAAAATAT TAGCAGGGAG TAGTGGCATG 540

CACAAGTGGT CCCAGCTCCA TGGGAGASTG AGGTAGGAAC ATCACTTGAG CCCAGGAAGT 600

CAAGGCTGCA GTGAACCATG ATCAGAACAT TGCANTCCAG CTTGGGTAAC AGAGTGAGAC 660

CTTAGGTCAG AAAAATGAAT AAATAAGCAT AAAATTTTAA AAACTTAGCC AGGCATGGTG 720

GCACACATCT GTGGTCCCTG CTACTTAGGA GGCTGAGGTG AGAGGATCCT TGAGCCCAGG 780

AGGTCAACAC TACAGTGAGC TATGATTGTG CCACTAAACT CCAACCTGGG TGAAAAAGCA 840

AAACCCTGCC AAAAAAAAAA AAAAAAACT 869

(2) INFORMATION FOR SEQ ID NO: 19:

(i) SEQUENCE CHARACTERISTICS:
(A) LENGTH: 959 base pairs
(B) TYPE: nucleic acid
(C) STRANDEDNESS: double
(D) TOPOLOGY: linear

(xi) SEQUENCE DESCRIPTION: SEQ ID NO: 19:

GGCGAGCCGA GATCGTGCCA TTGCACTCCA GCCTGGGCAA CAAGAGTGAA ACTCTGTCTC 60

AAAAAAAAAA AATTATAATA CTATATGCCA TAAAATGACA TTTCATATTT AAAGAGTTTT 120

TTAAAACTCT TGTATTCACA TGCCATAATT TGAAACCCTA TTTCACTGAA TGAGAATGGT 180

ATCTGTTGTC CTCATTTTTT CATTTTTATC CTTAACAATT TCCACCACAG CCAGTGCATA 240

TAATGGCAAT GACACCCAGG GATGGAATGA TAAGTTCCAT CRCMGCTCAG TCAAGACGCA 300

GACTTGATGT GGCCCCAACA ACAGTCAATA ATGGAGTCTC CAAAATAAAG CTCTATAGGA 360

AAGGTAAATA CCCGCTGCAC AAGAAACCAC AGCATCTAGG TTCTAACCCC ATCTCTATGA 420

AGAGCTTGCT GGGAGAGTTT TGACATTWAA CAATCTGTCT GATKGCCAAT TTTYTTCTTC 480

TATAAAATGA TAATGTTKGA YTCAAAGATC CAAAGTCAAT TCATGGTCTA AAACTTAATG 540

ATTTTTTTAG GTTTTGKGAC ATTTCACTGT ACACTGTAGT AATTTATATC TTATTTTCCC 600

ACTAATTTAG AAAAATATYT AAATGATCCT TAATTGGCAA TGGGTCCTAA GAATTTTGTT 660

TTAAATCCCT GTTACCCAAA AGAGCCCTTT TTTGTATCTC GCAGTAGTTA CAAGGATCTT 720

TCTAAATCTT AAAAAAAAAA AAAAAAGAAA GAAAGAAAAG AAAAGAAAAA AAGTCAGCCG 780

GGCGTGGTGG CTCATGCCTG TAATCCCAGC ACTTTGGGAC CAAGGTGGAC AGATCACGAG 840

GTCAGGAGAT GGAGACCATC CCGGCCAACA TGGAGAAACC CTGTCTCTAC TAAAAAAAAA 900

AAAAACTCGA GGGGGGCCCG GTACCCAATN CGCCGGCTAG TGGTCGTAAA ACAATCAAA 959

(2) INFORMATION FOR SEQ ID NO: 20:

(i) SEQUENCE CHARACTERISTICS:
(A) LENGTH: 1446 base pairs
(B) TYPE: nucleic acid
(C) STRANDEDNESS: double
(D) TOPOLOGY: linear

(xi) SEQUENCE DESCRIPTION: SEQ ID NO: 20:

CGGGGCAGGG CTGTGTGGCA CCGCCAGGGA GCGGGCCCAC CTGAGTCACT TTATTGGGTT 60

CAGTCAACAC TTTCTTGCTC CCTGTTTTCT CTTCTGTGGG ATGATCTCAG ATGCAGGGGC 120

TGGTTTTGGG GTTTTCCTGC TTGTGCCAAG GGCTGGACAC TGCTGGGGGG CTGGAAAGCC 180

CCTCCCTTCC TGTCCTTCTG TGGCCTCCAT CCCCTCATGG GTGCTGCCAT CCTTCCTGGA 240

GAGAGGGAGG TGAAAGCTGG TGTGAGCCCA GTGGGTTCCC GCCCACTCAC CCAGGAGCTG 300

GCTGGGCCAG GACCGGGAGA GGGAGCACTG CTGCCCTCCT GGCCCTGCTC CTTCCGCAGT 360

TAGGGGTGGA CCGAGCCTCG CTTTCCCCAC TGTTCTGGAG GGAAGGGGAA GGAGGGGGTC 420

TTCAGGCTGG AGCCAGGCTG GGGGTGCTGG GTGGAGAGAT GAGATTTAGG GGGTGCCTCA 480

TGGGGTGGGC AGGCCTGGGG TGAAATRAGA AAGGCCCAGA ACGTGCAGGT CTGCGGAGGG 540

GAAGTGTCCT GAGTGAAGGA GGGGACCCCC ATCCTGGGGG ATGCTGGGAG TGAGTGAGTG 600

AGATGGCTGA GTGAGGGTTA TGGGGAGCCT GAGGTTTTAT GGGCCTGTGT ATCCCCTTCT 660

CCCGGCCCCA GCCTGCCTCC CTCCTGCCCG CCTGGCCCAC AGGTCTCCCT CTGGTCCCTG 720

TCCCTCTGGT GGTTGGGGAT GGAGCGGCAG CAAGGGGTGT AATGGGGCTG GGTTCTGTCT 780

TCTACAGGCC ACCCCGAGGT CCTCAGTGGT TGCCTGGGGA GCCGGACGGG GCTCCTGAGG 840

GGTACAGGTT GGGTGGGCCC TCCCTGAGGG TCTGGGGTCA GGCTTTGGCT CTGCTGCCTC 900

TCAGTCACCA AGTCACCTCC CTCTGAAAAT CCAGTCCCTT CTTTGGATGT CCTTGTGAGT 960

CACTCTGGGC CTGGCTGTCG TCCCTCCTCA GCTTCTTGTT CCTGGGACAA GGGTCAAGCC 1020

AGGATGGGCC CAGGCCTGGG ATCCCCCACC CCAGGACCCC CAGGCCCCCT CCCCTGCTGC 1080

TTTGCGGGGG GCAGGGCAGA AATGGACTCC TTTTGGGTCC CCGAGGTGGG GTCCCCTCCC 1140

AGCCCTGCAT CCTCCGTGCC STAGACCTGC TCCCCAGAGG AGGGGCCTTG ACCCACAGGA 1200

CGTGTGGTGG CGCCTGGCAC TCAGGGACCC CCAGCTGCCC CAGCCCTGGT CTCTGGCGCA 1260

TCTCTTCCCT CTTGTCCCGA AGATCTGCGC CTCTAGTGCC TTTTGAGGGG TTCCCATCAT 1320

CCCTCCCTGA TATTGTATTG AAAATATTAT GCACACTGTT CATGCTTCTA CTAATCAATA 1380

AACGCTTTAT TTAAAGCCAA AAAAAAAAAA AAAAAACTCG AGGGGGGGCC CGTACCCAAT 1440

TCGCCA 1446

(2) INFORMATION FOR SEQ ID NO: 21:

(i) SEQUENCE CHARACTERISTICS:
(A) LENGTH: 1471 base pairs
(B) TYPE: nucleic acid
(C) STRANDEDNESS: double
(D) TOPOLOGY: linear

(xi) SEQUENCE DESCRIPTION: SEQ ID NO: 21:

CAAAAAATAA TAATGATAAT TTAAAATAAA TAAGTAACTA ATAAAAAGAT TTTATATCCC 60

AGTCTTATGA TGTTGGTTGG CAAGGCTAGA TAAAAAGATG TTAGAATGAA AGAACATATT 120

TTTAGTGATA TGTAAATGAA GGATTCTACA ATAGTCATAT ATTTTTATAT GAATGAATGT 180

TGGGTTGGGC TGGAGAGGTA TGTGTGTGTA AATATAAAGG TCTCACATTC AGAGTATAGC 240

TCTGAAATAA TGGAACTCAT GTCTACAATT CAACATGCAT CTGTATAGTT ACATCTCATG 300

TAAATATACA CAGACATATT TTGCAGCCAG TAATTGACAG TTAATGTCCA AAACAGGTGA 360

TTGATAGGTA ACAGAAATTA GATAACCACC AATTTTGCCC AAGAGAAAGA CTAGAAGGAC 420

TAAAAGCAGT TGAATGTATG GTACTGACAT TGTCATAAGC AGTCTGATAA CCAGTTTATT 480

GAAACGTGTG CATTAACAGA GAATTTAATT TTAAACCCAT AATTTCTCCT ATCCATTAAA 540

ATATTATAAT TGTTAGTAGT ATGAAACCAA CAGGAAATGT TTTTTAATCA TTTAGTGAGG 600

TGATTCATTT GTTTCATGGG CAAACACTAT CCAGGAAAAG CCTTGCTTGC CTGTTTCCCA 660

AAGAGCTCTA AGAAATAGAA TCAAGTGTAA AATGGTTCAG ACCATTCAGG ATTTCTTGTC 720

ACTCTTCTCA ACCCCGATCT TCCTGTTATT ACTGATGTTT GAAACCCTGT CATTAGCCCC 780

GGCCTGGTTA AAGCCCCTCA GAGTCACCTC TCATTCATAG CAATAGAATT CAACCCCAAG 840

TGGTTGATGG TGTCCCCAGC ACAGCCGAGA GACCTGATCT CTGGATTCAG TGCTTTTAGC 900

TCTTCGAGTT TACCCTAAGA TACCTTCGGG CAATATTTTT AACCAACCCA AAAGCTCTTC 960

AGGTCATTTC TGAAGAGGAC AAGGTGAATC TTGGCTTGGA ACACCATTTT TGGGCTCTTG 1020

CTACTGAATG AATCAGAAAG GAATTTTTTC TGAAGAGCAT TAGAAAGTAA AGGAGATGTT 1080

AAAATAAGTT CTTGAAGTAT GTTTTATATT TATCTAAAAC ACTGATTTTA AAAGTTTACA 1140

TTCAAATGTG TATTCAAAAG AAGTACTGAT TTGTAATTAT TATAGTTTGT GTGTATCATC 1200

CCCTTTTAAC CGTGCCTAAC AACTGTACTT AAATTTTGTT TTCCTAGTGT AACAAATGTT 1260

TCCCATAAGA TTTTCTAGAG CCAAATAATG GGAGTGAAAA ATTCCTTAAG TGTTATATAA 1320

GAAAATATAT TAGAAAATCA GCTTTGGATT ATACGATTTC TAAAATATAC TAATACAGAA 1380

TCCTCAGTAA TATGTTTTGA ATTGGATTTT TTCTCAGAAC TGTTACATAA TAAATAATAC 1440

ATCAACCAGA AAAAAAAAAA AAAAAAATTN C 1471

(2) INFORMATION FOR SEQ ID NO: 22:

(i) SEQUENCE CHARACTERISTICS:
(A) LENGTH: 1402 base pairs
(B) TYPE: nucleic acid
(C) STRANDEDNESS: double
(D) TOPOLOGY: linear

(xi) SEQUENCE DESCRIPTION: SEQ ID NO: 22:

AGGGACGTCT TGCCTGAGGA GATGCCCATT TCTGTCCTGG RTTACCCTCA CTGCGTGGTG 60

CATGAGCTGC CAGAGCTGAC GGCGGAGAGT TTGGAAGCAG GTGACAGTAA CCAATTTTGC 120

TGGAGGAACC TCTTTTCTTG TATCAATCTG CTTCGGATCT TGAACAAGCT GACAAAGTGG 180

AAGCATTCAA GGACAATGAT GCTGGTGGTG TTCAAGTCAG CCCCCATCTT GAAGCGGGCC 240

CTAAAGGTGA AACAAGCCAT GATGCAGCTC TATGTGCTGA AGCTGCTCAA GGTACAGACC 300

AAATACTTGG GGCGGCAGTG GCGAAAGAGC AACATGAAGA CCATGTCTGC CATCTACCAG 360

AAGGTGCGGC ATCGGCTGAA CGACGACTGG GCATACGGCA ATGATCTTGA TGCCCGGCCT 420

TGGGACTTCC AGGCAGAGGA GTGTGCCCTT CGTGCCAACA TTGAACGCTT CAACGCCCGG 480

CGCTATGACC GGGCCCACAG CAACCCTGAC TTCCTGCCAG TGGACAACTG CCTGCAGAGT 540

GTCCTGGGCC AACGGGTGGA CCTCCCTGAG GACTTTCAGA TGAACTATGA CCTCTGGTTA 600

GAAAGGGAGG TCTTCTCCAA GCCCATTTCC TGGGAAGAGC TGCTGCAGTG AGGCTGTTGG 660

TTAGGGGACT GAAATGGAGA GAAAAGATGA TCTGAAGGTA CCTGTGGGAC TGTCCTAGTT 720

CATTGCTGCA GTGCTCCCAT CCCCCACCAG GTGGCAGCAC AGCCCCACTG TGTCTTCCGC 780

AGTCTGTCCT GGGCTTGGGT GAGCCCAGCT TGACCTCCCC TTGGTTCCCA GGGTCCTGCT 840

CCGAAGCAGT CATCTCTGCC TGAGATCCAT TCTTCCTTTA MTTCCCCCAM CCTCCTCTCT 900

TGGATATGGT TGGTTTTGGC TCATTTCACA ATCAGCCCAA GGYTGGGAAA GCTGGAATGG 960

GATGGGAACC CCTCCGCCGT GCATCTRAAT TTCAGGGGTC ATGCTGATGC CTCTCGAGAC 1020

ATACAAATCC TTGCCTTTGT CAGCTTGCAA AGGAGGAGAG TTTAGGATTA GGGCCAGGGC 1080

CAGAAAGTCG GTATCTTGGT TGTGCTCTGG GGTGGGGGTG GGGTGTTTCT GATGTTATTC 1140

CAGCCTCCTG CTACATTATA TCCAGAAGTA ATTGCGGAGG CTCCTTCAGC TGCCTCAGCA 1200

CTTTGATTTT GGACAGGGAC AAGGTAGGAA GAGAAGCTTC CCTTAACCAG AGGGGCCATT 1260

TTTCCTTTTG GCTTTCGAGG GCCTGTAAAT ATCTATATAT AATTCTGTGT GTATTCTGTG 1320

TCATGTTGGG GTTTTTAATG TGATTGTGTA TTCTGTTTAC ATTAAAAAGA AGCAAAAATA 1380

ATAAAAAAAA AAAAAAAAAA CT 1402

(2) INFORMATION FOR SEQ ID NO: 23:

(i) SEQUENCE CHARACTERISTICS:
(A) LENGTH: 1047 base pairs
(B) TYPE: nucleic acid
(C) STRANDEDNESS: double
(D) TOPOLOGY: linear

(xi) SEQUENCE DESCRIPTION: SEQ ID NO: 23:

GGCACAGGGG ACTACAGGCA CCCACGACCA TACCCAGCTA ATTTTTGTAT TTTTTTGTAG 60

AGATGGGGTT TCACGATGTC GCCCAGGCTG GTCTTGAACT CCTGGGCTTG AGCGATCTTC 120

CCATCTTTCC ATCTTGGCCT CCTAAAGTGC TGGGACTGCA GGCATGAGCC ACCATGCCCA 180

GCCAAGATTC TTATTGATTA CCATGTTGCT TCAAGAAGCC AAGCCAGTTT CCAATATTCC 240

CCATTTGCTG GAGTCTTGGT ACTTTGGGTA GAAGCAACTG GTAAATTGTT AATTGGAACA 300

NTTGGTGGTG TAGATAACCA CGTATGGCCA AACCTAGAGC ATCTAGGCTC ACAATTACTA 360

TCCTGACTTG ATAACAAGTG TTCTGATATT AACCTGAAAA TGGGAATAAT GCCAAATCTG 420

TGTAACTTAA CATCTATATA CACAGTGGGG AGAACTGAAG TTATTAAACC TGGAATCTCT 480

GTGATCAAGG CTAACAGTAG TTATCTAAGA AGCAAAGGAC CTACAATTCT TAGACTTGGA 540

GTCATATTCT TTAAGGACGT GTTCTGAAAC TATATCAAGC ATCTGGTTTC CACGTATTTC 600

TCCCTCAGAA ATTATGAAGT ACAAGTAAAA ATGAAGGTAC AGGGTAAGAC ACATGCTGCT 660

TTCTTGCTCT TGAGTGGAGA CAGTTTTCCA GCCATCTTAA CCCCTTWACA CAAAACAATT 720

TGTGTTTTAT AGCAAATAAG TGACTCAACA TAATTTCAAT ATGATGTTTA TCCACCAGTA 780

CTTTCCTTTC AGCTTCTAGT CCCATAARTG GTTTGTGAAG TCATCGGTTA CATTAGCCAA 840

GATAGGCCTA GACTTGAAGT CTAGAATGTT TTTCCCACTA TATGCCAAAG TAGAATGTGG 900

GTATCTCAGG GTCATTTTTG TTGTTCAATT TCCCACCTGT ACAGTTGTTA TGATTCACTT 960

TCCTTATGTG TCTAATAAAT CTTGTTCCAT GAAATGATCA AAAAAAAAAA AAAAAAAACT 1020

CGAGGGGGGG CCCGGTACCC AAATCGC 1047

(2) INFORMATION FOR SEQ ID NO: 24:

(i) SEQUENCE CHARACTERISTICS:
(A) LENGTH: 990 base pairs
(B) TYPE: nucleic acid
(C) STRANDEDNESS: double

(D) TOPOLOGY: linear

(xi) SEQUENCE DESCRIPTION: SEQ ID NO: 24:

TTGGAAAGGG TCTAGCTCTT TCTCATTCAC CAACTATATT AGAAGCACTT GAGGGAAATT 60

TACCACTCCA AATCCAAAGC AATGAACAGT CTTTTCTGGA TGATTTTATT GCCTGTGTCC 120

CAGGATCAAG TGGTGGAAGG CTTGCAAGGT GGCTTCAGCC AGATTCATAT GCGGATCCTC 180

AGAAAACATC TTTGATCCTG GAATAAGGAT GATATTCGTT GTGGTTGGCC TACCACCATA 240

ACTGTTCAAA CAAAAGACCA GTATGGGGAT GTGGTACATG TTCCCAATAT GAAGGTAATT 300

ATAACTGGAT TAAATTAGCA GACATCTATA TACTGGCTGC AATGACTGAT AAAATTTTAG 360

AAATGCCAAG TGCTGAGRGT CCATTTGTTC TACCCTCTTT ATATAAAGGG TGATGCTGAA 420

AGTTTGTTTA AATGACTTGT TTATATTAAT TAGTCCCCAA GTGTCCAAGT TACACCTGTT 480

TTTTTTGTGA GTTTGTTCTT TACATTTTGC TACCTGTTAC GGGGACTCAA AGGAGGGATA 540

AGAAAGTATC CATCTAAAGA GTGCTAGACA CATACAGTGA AGCCCCTCAA TATGTATTGA 600

TTGAATAAAT GCATGAAAGA ATACATTTTT AAATTTTGTG TATAGTTTTG AAAGACTCAA 660

GTACGTTCTG TGTTTGGTAT TACTGAAACC ACATTTTAAA AATAACACTC ATTAAGTTAG 720

AAATATATGA GTTTAGATTG TAAAAGAATG AGGAATTGAA ATAGTTGTAT ACCATATTGA 780

TGAATATAGA GTTTTTAGGA TACCTCTTAC CTGAAATATT AATAATAATG TTTNCAGAGC 840

ATATTATACA TAATTATTTG TGATTTAATC TGTTAATATG AATATCTCAT TTAAAACTTT 900

TATTTCTGAA AAAATTATAT TGAATAAAAT TTTATATAGG CAGTCCCCAG CCCTTTCCTC 960

CTTCAAAGTT GTCTTATAGA GTGATTGGTT 990

(2) INFORMATION FOR SEQ ID NO: 25:

(i) SEQUENCE CHARACTERISTICS:
(A) LENGTH: 1208 base pairs
(B) TYPE: nucleic acid
(C) STRANDEDNESS: double
(D) TOPOLOGY: linear

(xi) SEQUENCE DESCRIPTION: SEQ ID NO: 25:

TAATCGCTAC TATAGGGAAA GCTGGTCGCT GCAGGTACCG GTCCGGAATT CCGGGTCGAC 60

CCACGCGTCC GAGCGAAATG GCGCCTCCGG CCCCCGGCCC GGCCTCCGGC GGCTCCGGGG 120

AGGTAGACGA GCTGTTCGAC GTAAAGAACG CCTTCTACAT CGGCAGCTAC CAGCAGTGCA 180

TAAACGAGGC GCASGGGTGA AGCTRTCAAG CCCAGAGAGA GACGTGGAGA GGGACGTCTT 240

CCTGTATAGA GCGTACCTGG CGCAGAGGAA GTTCGGTGTG GTCCTGGATG AGATCAAGCC 300

CTCCTCGGCC CCTGAGCTCC AGGCCGTGCG CATGTTTGCT GACTACCTCG CCCACGAGAG 360

TCGGAGGGAC AGCATCGTGG CCGAGCTGGA CCGAGAGATG AGCAGGAGCK TGGACGTGAC 420

CAACACCACC TTCCTGCTCA TGGCCGCCTC CATCTATCTC CACGACCAGA ACCCGGATGC 480

CGCCCTGCGT GCGCTGCACC AGGGGGACAG CCTGGAGTGC ACAGCCATGA CAGTGCAGAT 540

CCTGCTGAAG CTGGACCGCC TGGACCTCGC CCGGAAGGAG CTGAAGAGAA TGCAGGACCT 600

GGACGAGGAT GCCACCCTCA CCCAGCTCGC CACTGCCTGG GTCAGCCTGG CCACGGGTGG 660

TGAGAAGCTG CAGGATGCCT ACTACATCTT CCAGGAGATG GCTGACAAGT GCTCGCCCAC 720

CCTGCTGCTG CTCAATGGGC AGGCGGCCTG CCACATGGCC CAGGGCCGCT GGGAGGCCGC 780

TGAGGGCCTG CTGCAGGAGG CGCTAGACAA GGATAGTGGC TACCCRGAGA CGCTGGTCAA 840

CCTCATCGTC CTGTCCCAGC ACCTKGGCAA GCCCCCTGAG GTGACAAACC GATACCTGTC 900

CCAGCTGAAG GATGCCCACA GGTCCCATCC CTTCATCAAG GAGTACCAGG CCAAGGAGAA 960

CGACTTTGAC AGGCTGGTGC TACAGTACGC TCCCAGCGCT GAGGCTGGCC CAGAGCTGTC 1020

AGGACCATGA AGCCAGGACA GAGGCCAGGA GCCAGCCCTG CAGCCCTCCC CACCCGGCAT 1080

CCACCTGCAT CCCTCTGGGG CAGGAGCCCA CCCCCAGCAC CCCCATCTGT TAATAAATAT 1140

CTCAACTCCA RGGTGTTCCA CCTGAAAAAA AAAAAAAAAA AAAAAAAAAA AAAAAAAAAA 1200

AAAAAAAA 1208

(2) INFORMATION FOR SEQ ID NO: 26:

(i) SEQUENCE CHARACTERISTICS:
(A) LENGTH: 1922 base pairs
(B) TYPE: nucleic acid
(C) STRANDEDNESS: double
(D) TOPOLOGY: linear

(xi) SEQUENCE DESCRIPTION: SEQ ID NO: 26:

GTGCTGCGCT ACTGAGCAGC GCCATGGAGG ACTCTGAAGC ACTGGGCTTC GAACACATGG 60

GCCTCGATCC CCGGCTCCTT CAGGCTGTCA CCGATCTGGG CTGGTCGCGA CCTACGCTGA 120

TCCAGGAGAA GGCCATCCCA CTGGCCCTAG AAGGGAAGGA CCTCCTGGCT CGGGCCCGCA 180

CGGGCTCCGG GAAGACGGCC GCTTATGCTA TTCCGATGCT GCAGCTGTTG CTCCATAGGA 240

AGGCGACAGG TCCGGTGGTA GAACAGGCAG TGAGAGGCCT TGTTCTTGTT CCTACCAAGG 300

AGCTGGCACG GCAAGCACAG TCCATGATTC AGCAGCTGGC TACCTACTGT GCTCGGGATG 360

TCCGAGTGGC CAATGTCTCA GCTGCTGAAG ACTCAGTCTC TCAGAGAGCT GTGCTGATGG 420

AGAAGCCAGA TGTGGTAGTA GGGACCCCAT CTCGCATATT AAGCCACTTG CAGCAAGACA 480

GCCTGAAACT TCGTGACTCC CTGGAGCTTT TGGTGGTGGA CGAAGCTGAC CTTCTTTTTT 540

CCTTTGGCTT TGAAGAAGAG CTCAAGAGTC TCCTCTGTCA CTTGCCCCGG ATTTACCAGG 600

CTTTTCTCAT GTCAGCTACT TTTAACGAGG ACGTACAAGC ACTCAAGGAG CTGATATTAC 660

ATAACCCGGT TACCCTTAAG TTACAGGAGT CCCAGCTGCC TGGGCCAGAC CAGTTACAGC 720

AGTTTCAGGT GGTCTGTGAG ACTGAGGAAG ACAAATTCCT CCTGCTGTAT GCCCTGCTCA 780

AGCTGTCATT GATTCGGGGC AAGTCTCTGC TCTTTGTCAA CACTCTAGAA CGGAGTTACC 840

GGCTACGCCT GTTCTTGGAA CAGTTCAGCA TCCCCACCTG TGTGCTCAAT GGAGAGCTTC 900

CACTGCGCTC CAGGTGCCAC ATCATCTCAC AGTTCAACCA AGGCTTCTAC GACTGTGTCA 960

TAGCAACTGA TGCTGAAGTC CTGGGGGCCC CAGTCAAGGG CAAGCGTCGG GGCCGAGGGC 1020

CNAAAGGGGA CAAGGCCTCT GATCCGGAAG CAGGTGTGGC CCGGGGCATA GACTTCCACC 1080

ATGTGTCTGC TGTGCTCAAC TTTGATCTTC CCCCAACCCC TGAGGCCTAC ATCCATCGAG 1140

CTGGCAGGAC AGCACGCGCT AACAACCCAG GCATAGTCTT AACCTTTGTG CTTCCCACGG 1200

AGCAGTTCCA CTTAGGCAAG ATTGAGGAGC TTCTCAGTGG AGAGAACAGG GGCCCCATTC 1260

TGCTCCCCTA CCAGTTCCGG ATGGAGGAGA TCGAGGGCTT CCGCTATCGC TGCAGGGATG 1320

CCATGCGCTC AGTGACTAAG CAGGCCATTC GGGAGGCAAG ATTGAAGGAG ATCAAGGAAG 1380

AGCTTCTGCA TTCTGAGAAG CTTAAGACAT ACTTTGAAGA CAACCCTAGG GACCTCCAGC 1440

TGCTGCGGCA TGACCTACCT TTGCACCCCG CAGTGGTGAA GCCCCACCTG GGCCATGTTC 1500

CTGACTACCT GGTTCCTCCT GCTCTCCGTG GCCTGGTRCG CCCTCACAAG AAGCGGAAGA 1560

AGCTGTCTTC CTCTTGTAGG AAGGCCAAGA GAGCAAAGTC CCAGAACCCA CTGCGCAGCT 1620

TCAAGCACAA AGGAAAGAAA TTCAGACCCA CAGCCAAGCC CTCCTGAGGT TGTTGGGCCT 1680

CTCTGGAGCT GAGCACATTG TGGAGCACAG GCTTACACCC TTCGTGGACA GGCGAGGCTC 1740

TGGTGCTTAC TGCACAGCCT GAACAGACAG TTCTGGGGCC GGCAGTGCTG GGCCCTTTAG 1800

CTCCTTGGCA CTTCCAAGCT GGCATCTTGC CCCTTGACAA CAGAATAAAA ATTTTAGCTG 1860

CCCCAAAAAA AAAAAAAAAA AAAAAAACTC GAGGGGGGGC CCGTACCCAA TTCGCCCTAT 1920

AA 1922

(2) INFORMATION FOR SEQ ID NO: 27:

(i) SEQUENCE CHARACTERISTICS:
(A) LENGTH: 1951 base pairs
(B) TYPE: nucleic acid

(C) STRANDEDNESS: double
(D) TOPOLOGY: linear

(xi) SEQUENCE DESCRIPTION: SEQ ID NO: 27:

TCGTCCCCAG AGCGGGCTGA GCCCCAGGCG SAGGGTGGCG GGGGAGCCTG GGGGAGCCGC 60

CGCCACCTCC ACGGGCCTCT CTGAGCTCGG ACACCAGCGC CCTGTCCTAT GACTCTGTCA 120

AGTACACGCT GGTGGTAGAT GAGCATGCAC AGCTGGAGCT GGTGAGCCTG CGCCGTGCTT 180

CGGAGACTAC AGTGACGAGA GTGACTCTGC CACCGTCTAT GACAACTGTG CCTCCGTCTC 240

CTCGCCCTAT GAGTCGGCCA TCGGAGAGGA ATATGAGGAG GCCCCGCGGC CCCAGCCCCC 300

TGCCTGCCTC TCCGAGGAAC TCCACGCCTG ATGAACCCGA CGTCCATTTC TCCAAGAAAT 360

TCCTGAACGT YTTCATGAGT GGCCGCTCCC GCTCCTCCAG TGCTGAGTCC TTCGGGCTGT 420

TCTCCTGCAT CATCAACGGG GAGGAGCAGG AGCAGACCCA CCGGGCCATA TTCAGGTTTG 480

TGCCTCGACA CGAAGACGAA CTTGAGCTGG AAGTGGATGA CCCTCTGCTA GTGGAGCTCC 540

AGGCTGAAGA CTACTGGTAC GAGGCCTACA ACATGCGCAC TGGTGCCCGG GGTGTCTTTC 600

CTGCCTATTA CGCCATCGAG GTCACCAAGG AGCCCGAGCA CATGGCAGCC CTGGCCAAAA 660

ACAGTGACTG GGTGGACCAG TTCCGGGTGA AGTTCCTGGG CTCAGTCCAG GTTCCCTATC 720

ACAAGGGCAA TGACGTCCTC TGTGCTGCTA TGCAAAAGAT TGCCACCACC CGCCGGCTCA 780

CCGTGCACTT TAACCCGCCC TCCAGCTGTG TCCTGGAGAT CAGCGTGCGG GGTGTGAAGA 840

TAGGCGTCAA GGCCGATGAC TCCCAGGAGG CCAAGGGGAA TAAATGTAGC CACTTTTTCC 900

AGTTAAAAAA CATCTCTTTC TGCGGATATC ATCCAAAGAA CAACAAGTAC TTTGGGTTCA 960

TCACCAAGCA CCCCGCCGAC CACCGGTTTG CCTGCCACGT CTTTGTGTCT GAAGACTCCA 1020

CCAAAGCCCT GGCAGAGTCC GTGGGGAGAG CATTCCAGCA GTTCTACAAG CAGTTTGTGG 1080

AGTACACCTG CCCCACAGAA GATATCTACC TGGAGTAGCT GTGCAGCCCC GCCCTCTGCG 1140

TCCCCCAGCC CTCAGGCCAG TGCCAGGACA GCTGGCTGCT GACAGGATGT GGCACTGCTT 1200

GAGGAGGGGC ACCTGCCACC GCCAGAGGAC AAGGAAGTGG GGCGCTGGCC CAGGGTAGGG 1260

GAGGGTGGGG CAATGGGGAG AGGCAAATGC AGTTTATTGT AATATATGGG ATTAGATTCA 1320

TCTATGGAGG GCAGAGTGGG CTGCCTGGGG ATTGGGAGGG ACAGGGCTTG GGGAGCAGGT 1380

CTCTGGCAGA GAAGGATGTC CGTTCCAGGA GCACACGGCC CTGCCCCATC CTGGGCCTTA 1440

CCTCCCCTGC CAGGGCTCGG GCGCTGTGGC TCCTGCCTTG ATGAAGCCCG TGTCCTGCCT 1500

TGATGAAGCC TGTGCCACCT GCAAGTGCCC GCCCTGCCCC TGCCCCAACC CCCACCGAAG 1560

AGCCCTGAGC TCAGGCTGAG CCCAGCCACC TCCCAAGGAC TTTCCAGTGA GGAAATGGCA 1620

ACACGTGGAG GTGAAGTCCC TGTTCTCAGC TCCGTCATCT GCGGGGCTTC TGGGTGGCTC 1680

CTGCCACTGA CCTCACCGGC ATGCTGGCCT GTGGCAGGCC TAGGACCTCA GGCGGGGAGG 1740

AGGAGCTGCC GCAAGGCCCT GTCCCAGCAG AAGAGGGAGG CTTCCTGACT GACACAGGCC 1800

AGCCCCATCT TGGTCCTGTC ACCCTGGCCC CAACTATTAA AGTGCCATTT CCTGTCAAAA 1860

AAAAAAAAAA AAAATCGGGG GGGGCCCGGA ANCCAATTTC CCCCAAAAAG GGGGGTTATA 1920

AAAATTCCCN GGCNGTGTTT TTAAAAATTC G 1951

(2) INFORMATION FOR SEQ ID NO: 28:

(i) SEQUENCE CHARACTERISTICS:
(A) LENGTH: 3989 base pairs
(B) TYPE: nucleic acid
(C) STRANDEDNESS: double
(D) TOPOLOGY: linear

(xi) SEQUENCE DESCRIPTION: SEQ ID NO: 28:

GGCACAGGCC GCAGGGNACC TATGGGCGCA TATAGGTTGT AATGAAACTG TAGTCTCAGT 60

TGGAAGCCTA GACATGAAAT GGGTCAGTGA GCAAGGCTCT ATTCCTAGTC TCCAGCCATG 120

CCTGTGGAAC CTGARCCCRC TCTCAGCACA TTGGACCCAG GCAGATGYAA AAAATTCACA 180

GAACTATGAT TTGGACTCAA GGGTTTGTAG ATTTCCTCCT TCATTCTAAT TTCAGTGTCT 240

AAAATTCTTG CATCCRTGAA CGAGCTGGGC ATTTGATGAG ACAGGGCYGA ATACTGCAGT 300

TTTCCTCCTA GAAATCATCT GGGGCATTTT CTTTGAACTG ATGGGAACAA TAAGGCATAA 360

CTGTTTGCAC AAACTTGGGA TAARTGATTT TGGGATAACG ATCTACCAGA ATGGGGATAT 420

TTCACCCTTG GTTCTGAGAT GCAAACCAAA GAATATCATG ACCAGCTTTC AGGCCTCCTG 480

AAGTATATCT CTCACATTGT CCTGTTCTCA TGCTGAGGAG CCTGAGATCC CTGTGTGGGG 540

ATTAGACAGT GGACTGTTAT GGGTGTAGGT GAATTGGCTT ATTTTGTCTG TCCCTGTCTG 600

AATGTATTGC AGGAAYTAAA AAGGACCAAG AAGAGGAAGA AGACCAAGGC CCACCATGCC 660

CCAGGCTCAG CAGGGAGCTG CTGGAGGTAG TAGAGCCTGA AGTCTTGCAG GACTCACTGG 720

ATAGATGTTA TTCAACTCCT TCCAGTTGTC TTGAACAGCC TGACTCCTGC CAGCCCTATG 780

GAAGTTCCTT TTATGCATTG GAGGAAAAAC ATGTTGGCTT TTCTCTTGAC GTGGGAGAAA 840

TTGAAAAGAA GGGGAAGGGG AAGAAAAGAA GGGGAAGAAG ATCAAAGAAG GAAAGAAGAA 900

GGGGAAGAAA AGAAGGGGAA GAAGATCAAA ACCCACCATG CCCCAGGCTC AGCAGGGAGC 960

TGCTGGATGA GAAAGRGCCT GAAGTCTTGC AGGACTCACT GGATAGATGT TATTCAACTC 1020

CTTCAGTTGT GTTGAACTGT GTGACTCATG CCAGCCCTAC AGAAGTGCCT TTTATGTATT 1080

GGAGCAACAG CATGTTGGCT TGGCTGTTGA CATGGATGAA ATTGAAAAGT ACCAAGAAGT 1140

GGAAGAAGAC CAAGACCCAT CATGCCCCAG GCTCAGCAGG GAGCTGCTGG ATGAGAAAGA 1200

GCCTGAAGTC TTGCAGGACT CACTGGATAG ATGTTATTCG ACTCCTTCAG GTTATCTTGA 1260

ACTGCCTGAC TTAGGCCAGC CCTACAGCAG TGCKGTTTAC TCATTGGAGG AMCAKTACCT 1320

TGGCTTKKCT CTTGACGTGG ASAAATTGAA AAGAAGGGGA AGGGGAARAA AAGAAGGGGA 1380

AGAAGATCAA AGAAGGAAAG AAGAAGGGGA AGAAAAGAAG GGGAAGAAGA TCAAAACCCA 1440

CCATGCCCCA GGCTCAGCAG GGAGCTGCTG GATGAGAAAG GGCCTGAAGT CTTGCAGGAC 1500

TCACTGGATA GATGTTATTC AACTCCTTCA GGTTGTCTTG AACTGACTGA CTCATGCCAG 1560

CCCTACAGAA GTGCCTTTTA YRTATTGGAG CAACAGYGTG TTGGCTTGGC TGTTGACATG 1620

GATGAAATTG AAAAGTACCA AGAAGTGGAA GAAGACCAAG ACCCATCATG CCCCAGGCTC 1680

AGCAGGGAGC TGCTGGATGA GAAAGAGCCT GAAGTCTTGC AGGACTCACT GGATAGATGT 1740

TATTCGACTC CTTCAGGTTA TCTTGAACTG CCTGACTTAG GCCAGCCCTA CAGCAGTGCT 1800

GTTTACTCAT TGGAGGAACA GTACCTTGGC TTGGCTCTTG ACGTGGACAG AATTAAAAAG 1860

GACCAAGAAG AGGAAGAAGA CCAAGGCCCA CCATGCCCCA GGCTCAGCAG GGAGCTGCTG 1920

GAGGTAGTAG AGCCTGAAGT CTTGCAGGAC TCACTGGATA GATGTTATTC AACTCCTTCC 1980

AGTTGTCTTG AACAGCCTGA CTCCTGCCAG CCCTATGGAA GTTCCTTTTA TGCATTGGAG 2040

GAAAAACATG TTGGCTTTTC TCTTGACGTG GGAGAAATTG AAAAGAAGGG GAAGGGGAAG 2100

AAAAGAAGGG GAAGAAGATC AAMGAAGRAA AGAAGAAGGG GAAGAAAAGA AGGGGAAGAA 2160

GATCAAAACC CACCATGCCC CAGGCTCAAC GGCGTGCTGA TGGAAGTGGA AGAGCSTGAA 2220

GTCTTACAGG ACTCACTGGA TAGATGTTAT TCGACTCCGT CAATGTACTT TGAACTACCT 2280

GACTCATTCC AGCACTACAG AAGTGTGTTT TACTCATTTG AGGAACAGCA CATCAGCTTC 2340

GCCCTTTACG TGGACAATAG GTTTTTTACT TTGACGGTGA CAAGTCTCCA CCTGGTGTTC 2400

CAGATGGGAG TCATATTCCC ACAATAAGCA GCCCTTASTA AKCCGAGAGA TGTCATTCCT 2460

GCAGGCAGGA CCTATAGGCA MGTGAAGATT TGAATGAAAG TACAGTTCCA TTTGGAAGCC 2520

CAGACATAGG ATGGGTCAGT GGGCATGGCT CTATTCCTAT TCTCAAACCA TGCCAGTGGC 2580

AACCTGTGCT CAGTCTGAAG ACAATGGACC CACGTTAGGT GTGACACGTT CACATAACTG 2640

TGCAGCACAT GCCGGGAGTG ATCAGTCRGA CATTTTAATT TGAACCACGT ATCTCTGGGT 2700

AGCTACAAAA TTCCTCAGGG ATTTCATTTT GCAGGCATGT CTCTGAGCTT CTATACCTGC 2760

TCAAGGTCAK TGTCATCTTT GTGTTTAGCT CATCCAAAGG TGTTACCCTG GTTTCAATGA 2820

ACCTAACCTC ATTCTTTGTG TCTTCAGTGT TGGCTTGTTT TAGCTGATCC ATCTGTAACA 2880

CAGGAGGGAT CCTTGGCTGA GGATTGTATT TCAGAACCAC CAACTGCTCT TGACAATTGT 2940

TAACCCGCTA GRCTCCTTTG GTTAGAGAAG CCACAGTCCT TCAGCCTCCA ATTGGTGTCA 3000

GTACTTAGGA AGACCACAGC TAGATGGACA AACAGCATTG GGAGGCCTTA GCCCTGCTCC 3060

TCTCRATTCC ATCCTGTAGA GAACAGGAGT CAGGAGCCGC TGGCAGGAGA CAGCATGTCA 3120

CCCAGGACTC TGCCGGTGCA GAATATGAAC AAYGCCATGT TCTTGCAGAA AACGCTTAGC 3180

CTGAGTTTCA TAGGAGGTAA TCACCAGACA ACTGCAGAAT GTRGARCACT GAGCAGGACA 3240

GCTGACCTGT CTCCTTCACA TAGTCCATRT CACCACAAAT CACACAACAA AAAGGAGARG 3300

AGATATTTTG GGTTCAAAAA AAGTAAAAAG ATAATGTAGC TGCATTTCTT TAGTTATTTT 3360

GARCCCCAAA TATTTCCTCA TCTTTTTGTT GTTGTCATKG ATGGTGGTGA CATGGACTTG 3420

TTTATAGAGG ACAGGTCAGC TGTCTGGCTC AGTGATCTAC ATTCTGAAGT TGTCTGAAAA 3480

TGTCTTCATG ATTAAATTCA GCCTAAACGT TTTGCCGGGA ACACTGCAGA GACAATGCTG 3540

TGAGTTTCCA ACCTYAGCCC ATCTGCGGGC AGAGAAGGTC TAGTTTGTCC ATCASCATTA 3600

TCATGATATC AGGACTGGTT ACTTGGTTAA GGAGGGGTCT AGGAGATCTG TCCCTTTTAG 3660

AGACACCTTA CTTATAATGA AGTATTTGGG AGGGTGGTTT TCAAAATTAG AAATGTCCTG 3720

TATTCCRATG ATCATCCTGT AAACATTTTA TCATTTATTA ATCATCCCTG CCTGTGTCTA 3780

TTATTATATT CATATCTCTA CGCTGGAAAC TTTCTGCCTC AATGTTTACT GTGCCTTTGT 3840

TTTTGCTAGT GTGTGTTGTT GAAAAAAAAA ACATTCTCTG CCTGAGTTTT AATTTTTGTC 3900

CAAAGTTATT TTAATCTATA CAATTAAAAG CTTTTGCCTA TCAAAAAAAA AAAAAAAAAA 3960

AAAAAAAAAA AAAAAGCGGA CGCGTGGGC 3989

(2) INFORMATION FOR SEQ ID NO: 29:

(i) SEQUENCE CHARACTERISTICS:
(A) LENGTH: 3735 base pairs
(B) TYPE: nucleic acid
(C) STRANDEDNESS: double
(D) TOPOLOGY: linear

(xi) SEQUENCE DESCRIPTION: SEQ ID NO: 29:

CTGCTGTTCG CTGGCTGGGC TCCGCAGCAG GCTTGGCCAG CSGCTGACGG GTCGGCGGGC 60

GGGTTTGTGT GAACAGGCAC GCAGCTGCAG ATTTTATTCT GGTAGTGCAN CCCTCTCAAA 120

GGTTGAAGGA ACTGATGTAA CAGGGATTGA AGAAGTAGTA ATTCCAAAAA AGAAAACTTG 180

GGATAAAGTA GCCGTTCTTC AGGCACTTGC ATCCACAGTA AACAGGGATA CCACAGCTGT 240

GCCTTATGTG TTTCAAGATG ATCCTTACCT TATGCCAGCA TCATCTTTGG AATCTCGTTC 300

ATTTTTACTG GCAAAGAAAT CCGGGGAGAA TGTGGCCAAG TTTATTATTA ATTCATACCC 360

CAAATATTTT CAGAAGGACA TAGCTGAACC TCATATACCG TGTTTAATGC CTGAGTACTT 420

TGAACCTCAG ATCAAAGACA TAAGTGAAGC CGCCCTGAAG GAACGAATTG AGCTCAGAAA 480

AGTCAAAGCC TCTGTGGACA TGTTTGATCA GCTTTTGCAA GCAGGAACCA CTGTGTCTCT 540

TGAAACAACA AATAGTCTCT TGGATTTWTT GTGTTACTAT GGTGACCAGG AGCCCTCAAC 600

TGATTACCAT TTTCAACAAA CTGGACAGTC AGAAGCATTG GAAGAGGAAA ATGATGAGAC 660

ATCTAGGAGG AAAGCTGGTC ATCAGTTTGG AGTTACATGG CGAGCAAAAA ACAACGCTGA 720

GAGAATCTTT TCTCTAATGC CAGAGAAAAA TGAACATTCC TATTGCACAA TGATCCGAGG 780

AATGGTGAAG CACCGAGCTT ATGAGCAGGC ATTAAACTTG TACACTGAGT TACTAAACAA 840

CAGACTCCAT GCTGATGTAT ACACATTTAA TGCATTGATT GAAGCAACAG TATGTGCGAT 900

AAATGAGAAA TTTGAGGAAA AATGGAGTAA AATACTGGAG CTGCTAAGAC ACATGGTTGC 960

ACAGAAGGTG AAACCAAATC TTCAGACTTT TAATACCATT CTGAAATGTC TCCGAAGATT 1020

TCATGTGTTT GCAAGATCGC CAGCCTTACA GGTTTTACGT GAAATGAAAG CCATTGGAAT 1080

AGAACCCTCG CTTGCAACAT ATCACCATAT TATTCGCCTG TTTGATCAAC CTGGAGACCC 1140

TTTAAAGAGA TCATCCTTCA TCATTTATGA TATAATGAAT GAATTAATGG GAAAGAGATT 1200

TTCTCCAAAG GACCCGGATG ATGATAAGTT TTTTCAGTCA GCCATGAGCA TATGCTCATC 1260

TCTCAGAGAT CTAGAACTTG CCTACCAAGT ACATGGCCTT TTAAAAACCG GAGACAACTG 1320

GAAATTCATT GGACCTGATC AACATCGTAA TTTCTATTAT TCCAAGTTCT TCGATTTGAT 1380

TTGTCTAATG GAACAAATTG ATGTTACCTT GAAGTGGTAT GAGGACCTGA TACCTTCAGC 1440

CTACTTTCCC CACTCCCAAA CAATGATACA TCTTCTCCAA GCATTGGATG TGGCCAATCG 1500

GCTAGAAGTG ATTCCTAAAA TTTGGAAAGA TAGTAAAGAA TATGGTCATA CTTTCCGCAG 1560

TGACCTGAGA GAAGAGATCC TGATGCTCAT GGCAAGGGAC AAGCACCCAC CAGAGCTTCA 1620

GGTGGCATTT GCTGACTGTG CTGCTGATAT CAAATCTGCG TATGAAAGCC AACCCATCAG 1680

ACAGACTGCT CAGGATTGGC CAGCCACCTC TCTCAACTGT ATAGCTATCC TCTTTTTAAG 1740

GGCTGGGAGA ACTCAGGAAG CCTGGAAAAT GTTGGGGCTT TTCAGGAAGC ATAATAAGAT 1800

TCCTAGAAGT GAGTTGCTGA ATGAGCTTAT GGACAGTGCA AAAGTGTCTA ACAGCCCTTC 1860

CCAGGCCATT GAAGTAGTAG AGCTGGCAAG TGCCTTCAGC TTACCTATTT GTGAGGGCCT 1920

CACCCAGAGA GTAATGAGTG ATTTTGCAAT CAACCAGGAA CAAAAGGAAG CCCTAAGTAA 1980

TCTAACTGCA TTGACCAGTG ACAGTGATAC TGACAGCAGC AGTGACAGCG ACAGTGACAC 2040

CAGTGAAGGC AAATGAAAGT GGAGATTCAG GAGCAGCAAT GGTCTCACCA TAGCTGCTGG 2100

AATCACACCT GAGAACTGAG ATATACCAAT ATTTAACATT GTTACAAAGA AGAAAAGATA 2160

CAGATTTGGT GAATTTGTTA CTGTGAGGTA CAGTCAGTAC ACAGCTGACT TATGTAGATT 2220

TAAGCTGCTA ATATGCTACT TAACCATCTA TTAATGCACC ATTAAAGGCT TAGCATTTAA 2280

GTAGCAACAT TGCGGTTTTC AGACACATGG TGAGGTCCAT GGCTCTTGTC ATCAGGATAA 2340

GCCTGCACAC CTAGAGTGTC GGTGAGCTGA CCTCACGATG CTGTCCTCGT GCGATTGCCC 2400

TCTCCTGCTG CTGGACTTCT GCCTTTGTTG GCCTGATGTG CTGCTGTGAT GCTGGTCCTT 2460

CATCTTAGGT GTTCATGCAG TTCTAACACA GTTGGGGTTG GGTCAATAGT TTCCCAATTT 2520

CAGGATATTT CGATGTCAGA AATAACGCAT CTTAGGAATG ACTAAACAAG ATAATGGCAG 2580

TTTAGGCTGC ACAACTGGTA AAATGACTGT AGATAAATGT TGTAATTAGT GTACACGTTT 2640

GTATTTTTGT TAATATAGCC GCTGCCATAG TTTTCTAACT TGAACAGCCA TGAATGTTTC 2700

ATGTCTCCCT TTTTTTTTTG TCTATAGCTG TTACCTATTT TAGTGGTTGA AATGAGAGCT 2760

AGTGATGACA GAAGGATGTG GAATGTCTTC TTGACATCAT TGTGTATTGC TGGTAATCAA 2820

GTTGGTAACG ACTACTTCTA GCAGCTCTTA CCACTATGAC TTAAGTGGTC CTGGAAGGCA 2880

GTAAGTGGAG GTTTGCAGCA TTCCTGCCTT CATGAGGGCT TCTACCACTG ACCACTTTGC 2940

ACGTACCTGG CTCCCAGATT TACTTAGGTA CCCCACGAGT CGTCCACATA AGCAGCTTCA 3000

TCTTTACCTT GCCAGAGTTG ACAATTATGG GATACTCTAG TCTACTTATA CTTGTGTTCC 3060

CATCTGTCTG CCATCCTCTG AAGGCCAGGA CCCAGTCATA CATCCTTAGA AACCAAAGTA 3120

TGGTTTTTGT TTTCTCTTGG AATGTCAGGT CTTAAGGCAT TTAATTGAGG GACAAAAAAA 3180

AAAAAAAGCC GATATAGTAG CTAGCTACTT AAGCATCCAT GGGTATTGCT CCATATCAAA 3240

GCAGATTTGC AGGACAGAAA GAGTAAATTA GCCTTCAGTC TTGGTTTACA GCTTCCAAGG 3300

AGAGCCTTGG CCACCTGAAA TGTTAACTCG GTCCCTTCCT GTCTCTAGTT CATCAGCACC 3360

TGCAGATGCC TGACTCTTGT TAGCCTTACT ATTCAATACA GTCCTTAGAT TCACGGTATG 3420

CCTCTTCCTA TCCAGGCACC TATTCTGAAT CACCATGTTG CTCTGCAGCT AGAGTTGATA 3480

GGAGAAAATC CATTTGGGTA GATGGCCTAT GAATTTGTAG TAGACTTTCA AAATGAGTGA 3540

TTTGTTAGCT TGGTACTTTT AAGTTTGTGG TACAGATCCT CCAAACCCAT ACTCTGAGCA 3600

ATTAACTGCC TTGAACATAG AGAAAATTAA GGCCTCACAG GATGAGTCTC CATTCTCTGT 3660

AAATGCTTAT TTTATCATAG TCTTTAGCCN CTACTATGAG TAAAATGTTC TCTTCNGCCG 3720

GGTGTGGTGA CTCAC 3735

(2) INFORMATION FOR SEQ ID NO: 30:

(i) SEQUENCE CHARACTERISTICS:
(A) LENGTH: 1667 base pairs
(B) TYPE: nucleic acid
(C) STRANDEDNESS: double
(D) TOPOLOGY: linear

(xi) SEQUENCE DESCRIPTION: SEQ ID NO: 30:

TAGTAATTCA TTTAACTCCT CTTACATGAG TAGCGACAAT GAGTCAGATA TCGAAGATGA 60

AGACTTAAAG TTAGAGCTGC GACGACTACG AGATAAACAT CTCAAAGAGA TTCAGGACCT 120

GCAGAGTCGC CAGAAGCATG AAATTGAATC TTTGTATACC AAACTGGGCA AGGTGCCCCC 180

TGCTGTTATT ATTCCCCCAG CTGCTCCCCT TTCAGGGAGA AGACGACGAC CCACTAAAAG 240

CAAAGGCAGC AAATCTAGTC GAAGCAGTTC CTTGGGGAAT AAAAGCCCCC AGCTTTCAGG 300

TAACCTGTCT GGTCAGAGTG CAGCTTCAGT CTTGCACCCC CAGCAGACCC TCCACCCTCC 360

TGGCAACATC CCAGAGTCCG GGCAGAATCA GCTGTTACAG CCCCTTAAGC CATCTCCCTC 420

CAGTGACAAC CTCTATTCAG CCTTCACCAG TGATGGTGCC ATTTCAGTAC CAAGCCTTTC 480

TGCTCCAGGT CAAGGAACCA GCAGCACAAA CACTGTTGGG GCAACAGTGA ACAGCCAAGC 540

CGCCCAAGCT CAGCCTCCTG CCATGACGTC CAGCAGGAAG GGCACATTCA CAGATGACTT 600

GCACAAGTTG GTAGACAATT GGGCCCGAGA TGCCATGAAT CTCTCAGGCA GGAGAGGAAG 660

CAAAGGGCAC ATGAATTATG AGGGCCCTGG AATGGCAAGG AAGTTCTCTG CACCTGGGCA 720

ACTGTGCATC TCCATGACCT CGAACCTGGG TGGCTCTGCC CCCATCTCTG CAGCATCAGC 780

TACCTCTCTA GGTCACTTCA CCAAGTCTAT GTGCCCCCCA CAGCAGTATG GCTTTCCAGC 840

TACCCCATTT GGCGCTCAAT GGAGTGGGAC GGGTGGCCCA GCACCACAGC CACTTGGCCA 900

GTTCCAACCT GTGGGAACTG CCTCCTTGCA GAATTTCAAC ATCAGCAATT TGCAGAAATC 960

CATCAGCAAC CCCCCAGGCT CCAACCTGCG GACCACTTAG ACCTAGAGAC ATTAACTGAA 1020

TAGATCTGGG GGCAGGAGAT GGAATGCTGA GGGGGTGGGT GGGGGTGGGA AGTAGCCTAT 1080

ATACTAACTA CTAGTGCTGC ATTTAACTGG TTATTTCTTG CCAGAGGGGA ATGTTTTTAA 1140

TACTGCATTG AGCCCTCAGA ATGGAGAGTC TCCCCCGCTC CAGTTATTGG AATGGGAGAG 1200

GAAGGAAAGA ACAGCTTTTT TGTCAAGGGG CAGCTTCAGA CCATGCTTTC CTGTTTATCT 1260

ATACTCAGTA ATGAGGATGA GGGCTAGGAA AGTCTTGTTC ATAAGGAAGC TGGAGAACTC 1320

AATGTAAAAT CAAACCCATC TGTAATTTCG AGTGGGTGGA GCTCTTGCTT TTGGTACATG 1380

CCCTGAATCC CTCACTCCCT CAAGAATCCG AACCACAGGA CAAAAACCAC CTACTGGGCT 1440

CTCTCCTACC CTGCCCTCCT CCCTTTTTTT TACCCCTCTC TTTTTTATTT TTTCTTTGCT 1500

CTTTAGAACC CAGTGAAAAA TACCAGGGTA CTGGGGTGCA ACTCTTTCTT ATGATAGGTC 1560

ATTAGTGCTT TAAGCAAAAG ATATTAGCAG CTTTGACTGC AGCATTAGCA ATTAGGRAAA 1620

AAAAAAANWA AAAACTCGAG GGGGGGCCCG GTTACCCAAT TCGCCCT 1667

(2) INFORMATION FOR SEQ ID NO: 31:

(i) SEQUENCE CHARACTERISTICS:
(A) LENGTH: 1408 base pairs
(B) TYPE: nucleic acid
(C) STRANDEDNESS: double
(D) TOPOLOGY: linear

(xi) SEQUENCE DESCRIPTION: SEQ ID NO: 31:

ATTACACACC TGAGCACTGT GCCTGGCAAG ACCTGTCTTA ATAGATTAGA GAACCACTGA 60

TAGATGGTCA GCTTTCTGTA GCAGTGAGAA CCCTACATTT CAAATGTGGA TAGCACCTTT 120

GCGGGGAAAC ATCACTTGGC ACATCTGCAT TCTTTTTTGA CACAGGGTCT CACTCTGTTG 180

CCCAGGCTAG AGTGCATGGC ACGATCTTAG CTCACTGCAA CCTCCACCTC CCAAGTTCAA 240

GCGATTCTTC TGCCTCAGCC TCCTGAGCAG CTGGGATCAC AGACATGCGC TACCATGCCC 300

AGCTAATTTT TTGTATTTTT TGTKTGTTTG TTTTTGTTTK TAAGTAGAGA CGGGCTTTCA 360

CCACGTTGGS CAGGCAGGTC TCGAACTCCT GAMCTCAGGT GATCCACCCA CATCTGCGTT 420

CCAATATCTT TCTCAACATA ATGATAGCCG TAATTAATAT TTTCCAGTAC ATTTTTATGC 480

CTTTACACAC GAGAGTGGTA GACAGACACA AACCCAGATC TGTCTGACTC CAAAGCCCGT 540

TTGTCATCAT TCCTTTTACG GTATCCTATA GTGGTATCCT TTACAGAAAG ACAGCTTTTA 600

CCCAACAAAG ACTTAACTTC CCAGGATGCC AGAAGGACAA AGCGGGATTG CTTTTAAGRA 660

GRAAGTTATC AAGAMCTTAT TTTATAAATG AGATTAGATA GGGAAAGGCA ATTTATCTTT 720

ATTAAAAACT GAAAAGGCCA GCATAGGGAA GGAGGTCCTT CGGTGGTCTT TTTCAGGGAA 780

ATACTTCAGT TGCTTTTATT AGAAACAGAT AGTACCTAAG GTTTTGAGGT AGGWACAGCT 840

TAAGGCATGC TAATGKTCAT GGGTCCTTCC ATAGTCATTT TKGTATTTTG GTTWACATTT 900

GAGCAATAGG CAGCCCTTCA CTGCTGCTGG AYTCATTCCT GCCAYTATTA CAGGTGACAG 960

AGGAGACAGG AGGTATGTCT TTTCTATTTT TAWACATGCT TTATATTTAA CACAAGCTCT 1020

TGGGTATCTT AGATAAACAG AAGTTGCCTA GCACTCCTTT TAGTGCATTG AACCCTTTAA 1080

CATTTAAGCA AAATAATAAA CAGTCTTTTG AGGTTCCTTA ACAATGAAAC GTGTTCGAGT 1140

GGCAGCAGCG GAATCCATGC YTCTTCTCCT GGAGTGTGCA AKAGTCCGTG GTCCTGAGTA 1200

TCTCACACAG ATGTGGCATT TTATGTGTGA TGCTCTAATT AAGGCCATTG GTACAGAACC 1260

AGATTCAGAC GTCCTCTCAG AAATAATGCA TTCTTTTGCA AAGGTGAATA TTTTTCTCTT 1320

AAAAAATATG TATAAGGTGG TATGTTCATT TATTAGTCTT GCTAAAAAAA AAAAAAAAAA 1380

ACTTNGAGGG GGGGNCCGGT ACCCAATT 1408

(2) INFORMATION FOR SEQ ID NO: 32:

(i) SEQUENCE CHARACTERISTICS:
(A) LENGTH: 2031 base pairs
(B) TYPE: nucleic acid
(C) STRANDEDNESS: double
(D) TOPOLOGY: linear

(xi) SEQUENCE DESCRIPTION: SEQ ID NO: 32:

AGGATATGCA TGATTCTTAA CCAGGCTATA TGTTAAAAAA AAATTGGAAA ATGCAATACA 60

TTTTTTACTA TACAAACTAC AGAATGAGTA TGCAAGTTTT ATTTATCAAA ATGTAATGGA 120

TTTTTAAAGG CTGAGAAATT TTCCTTATAC CTACCTTTTC AGTTATTTTA ATTATACCAA 180

ATTATCAACT AGAATAGCTT CATCCATATG AAATATAAAA TGAAGAGACA CCTAGGCTCT 240

ATCAGGCTTA GGATTCTTTG AACTTATTTC CACTTTAATT TCTCAGTGGA AGTTAAGAGG 300

GGTGAGAAAA CAAAGAAGGG GAAAAACTGA CAACTAACAA AACCAGCACC ACATCGCTAG 360

GTGGTGCTTA CTAATTACCT TCTCAGGATT TTCCTCAGAT TGAAAAGCTT ATGAGGATTT 420

CTTGGGAGTC TTAATAACCT GCCTGTTAGT ACAGAGCTTT CCTGATGATA TTTACTCTTG 480

AGCACATGTG GTTGTAAAAC CTTAACTTTC TTTCTCCAGG AGGGTGGTGA TAGAAACAGA 540

TGGTAGTATT TATGAACTGA TGTTCTCGTG AAATGTTGAG GGTGGGGAGA AAAGACTTTA 600

AGGGAGGAGA GCCATCTATT TTGTTCCTAA AGCCACCTCT CAGCAGAATC GTCATGTTTT 660

TCTGATGCAC CGCTCTGCTT CATGCCCAAG ATGACTTGCG AGGCAATCTC AGGAGCTGTG 720

GACTTAACCR TTGCAAAGCA CACTGTCTTT CTCAGCGTTC TCTGCAAGTC AGTAGGTGTT 780

AGTATGGTTG CAAAGTTCAC TGTCTCAGCA AAGTTGAACT GGGCTACCTC TCTACAGCTG 840

TTTCCTCAGA GGGAAAAATC TTGAGACCAG ATGGTGGAGC TCTGGAGTCA GAGGAAATGG 900

GTGTCTTCAG CACAAAGCTG CTGCTTTTAC TTCAGCCACT TCTGACATTT TTACATACCG 960

AGCCTGAGAT TRTGTGATTA TCTCAAATCA AATCACTTTG ATGGAGATAA ATAATCAAAA 1020

CTGTTTTATA GTCATTGATT TGGTGAGAAC AGTAATGGAA AATGGTGTTG AAGGACTTCT 1080

CATTTTTGGA GCTTTCCTTC CAGAGTCCTG GCTGATTGGT GTTCGCTGTT CATCTGAGCC 1140

CCCAAAAGCA TTATTACTGA TACTTGCACA CAGTCAAAAG CGCAGACTGG ATGGATGGTC 1200

TTTTATAAGG CATTTAAGGG TACACTACTG TGTTTCACTG ACCATACATT TTTCTTAGCC 1260

CCTCAAGTAA TATAGCACAG AGTTATGAAT GACAATTCCC CTAACCATTC CTCTTCATAT 1320

CTGCCTCTTC CCCTTACCAT CGTAATTCTC CAAACTGGTC ATAAAGGCAC TCTGTGAAGA 1380

TATTGGGGAC TGACATCTTA AGCTCTCACC TGGCTGCAGT AGGAAAGGCC AAACTGACGA 1440

CAAAAAAAAA ATTCTTTATA AAGATGATAT GGTAACATGT ATCTTTGCCC TGGGTCTGGG 1500

TGGGTCCAGT CAGTCTCAGA TTTACAAGCA TTTAGGAGCC TAGGTAAAAG CTGCTAGTAT 1560

TCTTTTAAAA GTTACATTTA TGACTTGCAA TGATAGAAAA CTCCTTCCAA TTAAATGGCA 1620

TTTTATAATA TTATGTGTGT ACTTCACAGT GTTAAAAATA CCCTCATACG TTATTGCATT 1680

TGATCTTCAC AGAAAGTGCA TTTTAACCAG TACTCTGGGT GCAATAAATA ATATGTAGAA 1740

ATTTAAGTCC TCCAATTCCA GCATATCCAG TGAGTTTTGA CAGTGTGTTT ATGTGGAATG 1800

TTTAAGGATA TACAATTGTA CTTTATATAA ATTGGTTCTT GTTCTTCTTA AATGTGACAT 1860

GAAATAATTG TGCTGCTACA TTATACTGGA AATTAACAGG GGAAAAGGGA AGAGCTCTTG 1920

GCTCCCTTGA GGTTCTGCTA GTGGTGTTAG GAGTGGTTAC AACTGAGCTT TTAGTAACCA 1980

TTTAACCGTA TGTAAACTTG GTTTCTAATT AAAAAAAAAT TTCTTTTTCC A 2031

(2) INFORMATION FOR SEQ ID NO: 33:

(i) SEQUENCE CHARACTERISTICS:
(A) LENGTH: 971 base pairs
(B) TYPE: nucleic acid
(C) STRANDEDNESS: double
(D) TOPOLOGY: linear

(xi) SEQUENCE DESCRIPTION: SEQ ID NO: 33:

CGCGTCGGAA CTCGGCCGCG GGACATCCAC GGGGCGCGAG TGACACGCGG GAGGGAGAGC 60

AGTGTTCTGC TGGAGCCGAT GCCAAAAACC ATGCATTTCT TATTCAGATT CATTGTTTTC 120

TTTTATCTGT GGGGCCTTTT TACTGCTCAG AGACAAAAGA AAGAGGAGAG CACCGAAGAA 180

GTGAAAATAG AAGTTTTGCA TCGTCCAGAA AACTGCTCTA AGACAAGCAA GAAGGGAGAC 240

CTACTAAATG CCCATTATGA CGGCTACCTG GCTAAAGACG GCTCGAAATT CTACTGCAGC 300

CGGACACAAA ATGAAGGCCA CCCCAAATGG TTTGTTCTTG GTGTTGGGCA AGTCATAAAA 360

GGCCTAGACA TTGCTATGAC AGATATGTGC CCTGGAGAAA AGCGAAAAGT AGTTATACCC 420

CCTTCATTTG CATACGGAAA GGAAGGCTAT GCAGAAGGCA AGATTCCACC GGATGCTACA 480

TTGATTTTTG AGATTGAACT TTATGCTGTG ACCAAAGGAC CACGGAGCAT TGAGACATTT 540

AAACAAATAG ACATGGACAA TGACAGGCAG CTCTCTAAAG CCGAGATAAA CCTCTACTTG 600

CAAAGGGAAT TTGAAAAAGA TGAGAAGCCA CGTGACAAGT CATATCAGGA TGCAGTTTTA 660

GAAGATATTT TTAAGAAGAA TGACCATGAT GGTGATGGCT TCATTTCTCC CAAGGAATAC 720

AATGTATACC AACACGATGA ACTATAGCAT ATTTGTATTT CTACTTTTTT TTTTTAGCTA 780

TTTACTGTAC TTTATGTATA AAACAAAGTC ACTTTTCTCC AAGTTGTATT TGCTATTTTT 840

CCCCTATGAG AAGATATTTT GATCTCCCCA ATACATTGAT TTTGGTATAA TAAATGTGAG 900

GCTGTTTTGC AAACTTAAAA AAAAAWWAAA AAAACTSGAG GGGGGCCCGT ACCCAANTCG 960

CCGNATATGA T 971

(2) INFORMATION FOR SEQ ID NO: 34:

(i) SEQUENCE CHARACTERISTICS:
(A) LENGTH: 1792 base pairs
(B) TYPE: nucleic acid
(C) STRANDEDNESS: double
(D) TOPOLOGY: linear

(xi) SEQUENCE DESCRIPTION: SEQ ID NO: 34:

GAACCCCCTT TCTCCTGGTA AAGGGTAAGG GGGGGGATAA TGTTTACCAC AGGTACGAAA 60

TAGTCACTTT AACATTGAGA CCTCTGCCTC ATTGAATTCA GGTTTTTTAA GTACTTGAAA 120

CTCTTCAGAT TCTCCTTATT TTAGTTTCTT TTTACATTTA TGAAGTAGAA AGCATTGTTT 180

TGTAAACTGT TTTGAAAATA AATAGCCTAG TCTCTTATCC TCTTTAGCGT GGATTAAAGG 240

TGAAGTTCTG CAAATGGGAG AGTGTTCACA GTAGATAGCT CAGATTGATT GAACACATTT 300

GAGGAAGAGA CTCCTGCATG AGATACCAGC ATTTTTACAA ATACTTTTTA TGTACATTCT 360

TTATTTTGTC ATTTTGTCAA CCCTCTCCCC AAGCACATCT TCTTTCCTTT TACTATGTCT 420

ATGTAGGGAA AAACAAAACA AAAAATTGCA CTTACGTTAC ACTCCCAAAA TGTGGGTAAT 480

CCGTGTCTTT CAAAAAACAT TTCTGTTTTT TGTTTTGTTT TGGTCAGTCC ATTGCATAAG 540

TGACAAGTTT GGGTGCTTGT GGCACGTATG TATGAAGCGG GAGGGGGATG ASAATTGCCT 600

GTCCTTCAGT ARGCTGTAAA AGTAATTTAC ATGTAAGTAA AAAGGGAAAA TAGAATAGAT 660

GCCAAAGTCA TTTATTCAGT CCTTAGTTTT CTTATGTGGC ATTACTGCAT CTGCTAGTTA 720

GTGAGAAAGC ACCCTCAGCT TTTACTGCTC CCCTCCCTGC CTGCCAACAC ACTTGATGTG 780

TGCAAACAGC CCTCAAGTAT CTGTCAGATG ACCTATATAA GGTATTGAAT AAGGTATTCT 840

TGTCAGTTTA GAAATGGACT GGATAAAACT TACTTGGTTG TCATTATTTT ATCTCATTTG 900

TCCTGTTACA TGCCCTATGT TAAGATAATT ATATTGCCAC TAATAATCAA GATGCTAAAT 960

GAGTATTACA ACTGGCTAAT ATCATTTTTT ATATACAAGG GTATGTGTAT ATTTGGAATT 1020

GRTATGAGAA ACTCATTTGT ACCCATTTGA GTGATATTGC ACAACAAACA CAGATAYCTA 1080

CAGACTCCGT TTTCATTTTC TCGTGTTCTT TATGATAATG ATCTTTGTAG ATTGGTTATT 1140

TCTGTACTTT ATCTGTAATA AACTTTGTAG ATCCTGTGAA CCATTACTTT GCCTAAATCA 1200

CTTGAGACTT GAGTCTTTAA TAACAAAGCA TCAATATTCA CTAAAGTCAA TCTCTTTTGA 1260

GTTTCTGTGA CTTGGCTAGA AGCTCTTGAC ACTAAGGGAT TAGTGTTAAT TTTCCCTGGG 1320

GGTGTTCCAC TAGGGCATTA CTGTATAATG ACTTGATGTT GCCACATAGA CTTCAAGATA 1380

TATAATATTT TGAGGATTTT GTTGATTGGC CTATGTTTTA TTGCATAGTG TGAAACGTGT 1440

AAAGCTTGGT TAACCTGTAT ATAGATAGCT TATTGTTGAC TAGTTATAGT GTATTTAGGG 1500

TTGCCTGTAA TATTTAAGCT TCTTTACTGA TGTGTGTGCT GGTAGGAACA TATAATTTTT 1560

GTACATTATA TTTACTGAGA TGTTGCCTTT TTTATTTTAC AAATACTTTG GAATTCCAAT 1620

GTGTTTTTTG CTTCCGTGAG GATTAATTTG GAAAGGTTTT TAATGACATT CCACTGATTT 1680

CAGATTTTGC TTGAGATTGA CTTCAATAAA TTGTCCTGTA TGTTCCAAAA AAAAATTAAA 1740

AAACTCGAGG GGGGCCCGGT ACCCAANNCG CCGGATATGA TCGTAAACAA TC 1792

(2) INFORMATION FOR SEQ ID NO: 35:

(i) SEQUENCE CHARACTERISTICS:
(A) LENGTH: 896 base pairs
(B) TYPE: nucleic acid
(C) STRANDEDNESS: double
(D) TOPOLOGY: linear

(xi) SEQUENCE DESCRIPTION: SEQ ID NO: 35:

AGTTGNANAC AACAGGACCT GAGTCCTTGG GCAGCACCAG TAGGTTGCCC CYTGCYTCYT 60

GCCAGCYTCA CYTGCCACYT TYTGCCCCTY TCGGGATGCC TTCGCAGACA GAGYTYTTCG 120

CTGCCTGTGG TGGCCAYTCT TTGCTTTTGG TTYTCTTGCC CCTTGGCCTC CCTTTTTGTC 180

CCCGGGCAGC CTTGTGTGAC CTGCCCTTTT CCCTCCCTTC CTTTCCAGGA CAAGCACGCC 240

GAGGAGGTGC GGAAAAACAA GGAGCTGAAG GAAGAGGCCT CCAGGTAAAG CCTAGAGGCC 300

AAAGAACTTT CCAGGTCAGC CGGACAGCTC CAGCAGCTCC ACGTTCCAGG CAGCCTCGMC 360

CGCCGGCTGC GCTCCCAGCA CTGGGGTTTG GGGGGAGGGG GGTGGCCAAG GGGCGTTTCC 420

TCTGCTTTTG GTGTTTGTAC ATGTTAAGAA TTGACCAGTG AAGCCATCCT ATTTGTTTCC 480

GGGGAACAAT GACGGGGTGG GARAGGGGAG AGGAGAGAGT TTGGGAAAGG GAGATGGAGA 540

AGAACTCAAG GACATTGCAA CCCTGCCCGG CGCAGATCTG ATTTTCACAT CTCTACCTGG 600

ACATTGAGCC TCCCAGGCAC CATGTTGAGG AGAGATGAAA ACCAGGGCGG TAGAACTTCA 660

GGGTGAAGGA CAGAGTCCTG GGTGGGGCAG CGGCTGCAGG GCGCACCAGA GAACCCAGCC 720

AGAGGGGGTG TGAGTACCAG TGGTGTTGCT TCCACCCTGC AGCAGGTGGG ATGAGGTCTG 780

TGTGTGTGTG TGAACCATCA TTTTTTGATC ATCATGACCA ATGAAACATT GAAAAAAAAA 840

AAAAAAACTG GAGGGGGGCC CGTACCCAAN TCGCCGNATA GTGATCGTAA ACAATC 896

(2) INFORMATION FOR SEQ ID NO: 36:

(i) SEQUENCE CHARACTERISTICS:
(A) LENGTH: 912 base pairs
(B) TYPE: nucleic acid
(C) STRANDEDNESS: double
(D) TOPOLOGY: linear

(xi) SEQUENCE DESCRIPTION: SEQ ID NO: 36:

TCGACCCACG CGTCCGGTCA GCCAGTCGCA TCCAGCCATG ACAGCCTTCT GCTCCCTGCT 60

CCTGCAAGCG CAGAGCCTCC TACCCAGGAC CATGGCAGCC CCCCAGGACA GCCTCAGACC 120

AGGGGAGGAA GACGAAGGGA TGCAGCTGCT ACAGACAAAG GACTCCATGG CCAAGGGAGC 180

TAGGCCCGGG GCCAKCCGCG GCAGGGCTCG CTGGGGTCTG GCCTACACGC TGCTGCACAA 240

CCCAACCCTG CAGGTCTTCC GCAAGACGGC CCTGTTGGGT GCCAATGGTG CCCAGCCCTG 300

ARGGCAGGGA AKGTCAACCC ACCTGCCCAT CTGTGCTGAG GCATGTTCCT GCCTACCATC 360

CTCCTCCCTC CCCGGCTCTC CTCCCAGCAT CACACCAGCC ATGCAGCCAG CAGGTCCTCC 420

GGATCACYGT GGTTKGGTGG AGGTCTGTCT GCACTGGGAG CCTCARGARG GCTCTGCTCC 480

ACCCACTTGG CTATGGGAGA GCCAGCAGGG GTTCTGGAGA AAAAAACTGG TGGGTTAGGG 540

CCTTGGTCCA GGAGCCAGTT GAGCCAGGGC AGCCACATCC AGGCGTCTCC CTACCCTGGC 600

TCTGCCATCA GCCTTGAAGG GCCTCGATGA AGCCTTCTCT GGAACCACTC CAGCCCAGCT 660

CCACCTCAGC CTTGGCCTTC ACGCTGTGGA AGCAGCCAAG GCACTTCCTC ACCCCYTCAG 720

CGCCACGGAC CTYTYTGGGG AGTGGCCGGA AAGCTCCCSG GCCTYTGGCC TGCAGGGCAG 780

CCCAAGTCAT GACTCAGACC AGGTCCCACA CTGAGCTGCC CACACTCGAG AGCCAGATAT 840

TTTTGTAGTT TTTATKCCTT TGGCTATTAT GAAAGAGGTT AGTGTGTTCC CTGCAATAAA 900

CTTGTTCCTG AG 912

(2) INFORMATION FOR SEQ ID NO: 37:

(i) SEQUENCE CHARACTERISTICS:
(A) LENGTH: 1382 base pairs
(B) TYPE: nucleic acid
(C) STRANDEDNESS: double
(D) TOPOLOGY: linear

(xi) SEQUENCE DESCRIPTION: SEQ ID NO: 37:

AATTCGGCAC GAGCGGAGGC GAGGGAAACT RAGGGCGAAA GTTGTGTGTC GTGTTGGCAG 60

GAGGGCCTAG AAGGGAAAGA CTGTCTAGTG GGACAATGTC ATATTATAAA TTTGGAATGC 120

TGAATAGAAA ATTATAGATT TTGATATTGA AGGAAATGAA GCGAAGCYTA AATGAAAATT 180

CAGCTCGAAG TACAGCAGGC TGTTTGCCTG TTCCGTTGTT CAATCAGAAA AAGAGGAACA 240

GACAGCCATT AACTTCTAAT CCACTTAAAG ATGATTCAGG TATCAGTACC CCTTCTGACA 300

ATTATGATTT TCCTCCTCTA CCTACAGATT GGGCCTGGGA AGCTGTGAAT CCAGAGTTKG 360

CTCCTGTAAT GAAAACAGTG GACACCGGGC AAATACCACA TTCAGTTTCT CGTCCTCTGA 420

GAAGTCAAGA TTCTGTCTTT AACTCTATTC AATCAAATAC TGGAAGAAGC CAGGGTGGTT 480

GGAGCTACAG AGATGGTAAC AAAAATACCA GCTTGAAAAC TTGGRATAAA AATGATTTTA 540

AGCCTCAATG TAAACGAACA AACTTAGTGG CAAATGATGG AAAAAATTCT TGTCCAATGA 600

GTTCGGGAGC TCAACAACAA AAACAATTAA GAACACCTGA ACCTCCTAAC TTATCTCGCA 660

ACAAAGAAAC CGAGCTACTC AGACAAACAC ATTCATCAAA AATATCTGGC TGCACAATGA 720

GAGGGCTAGA CAAAAACAGT GCACTACAGA CACTTAAGCC CAATTTTCAA CAAAATCAAT 780

ATAAGANACA AATGTTGGAT GATATTCCAG AAGACAACAC CCTGAAGGAA ACCTCATTGT 840

ATCAGTTACA GTTTAAGGAA AAAGCTAGTT CTTTAAGAAT TATTTCTGCA GTTATTGAAA 900

GCATGAAGTA TTGGCGTGAA CATGCACAGA AAACTGTACT TCTTTTTGAA GTATTAGCTG 960

TTCTTGATTC AGCTGTTACA CCTGGCCCAT ATTATTCGAA GACTTTTCTT ATGAGGGATG 1020

GGAAAAATAC TCTGCCTTGT GTCTTTTATG AAATCGATCG TGAACTTCCG AGACTGATTA 1080

GAGGCCGAGT TCATAGATGT GTTGGCAACT ATGACCAGAA AAAGAACATT TTCCAATGTG 1140

TTTCTGTCAG ACCGGCGTCT GTTTCTGAGC AAAAAACTTT CCAGGCATTT GTCAAAATTG 1200

CAGATGTTGA GATGCAGTAT TATATTAATG TGATGAATGA AACTTAAGTA GTGATAAAAG 1260

GAAGTTTAGC ATAAATTATA GCAGTTTTCT GTTATTGCTT AATTTACCAT CTCCATAGTT 1320

TTATAGCTAC TATTGTATTT CACTTGTTGA ATTAAAGTAT TTGAATTCTT TTAAAAAAAA 1380

AA 1382

(2) INFORMATION FOR SEQ ID NO: 38:

(i) SEQUENCE CHARACTERISTICS:
(A) LENGTH: 872 base pairs
(B) TYPE: nucleic acid
(C) STRANDEDNESS: double
(D) TOPOLOGY: linear

(xi) SEQUENCE DESCRIPTION: SEQ ID NO: 38:

GGGCTACTTC AAAGCCCTGG GCCTTATTTC TTCAGGTAAA AAAATATAAA GTCAGATCTC 60

ATCCCGGCTG GCCATGCTGT TAGACCCTTT CATCCTTCTC TTCTGCCTCT TCTCAACAGC 120

TGCCCAGTCC TGTTTGGAAT TCATATACAT ACAGTTCTAA TACTGATGTA TTTACCCTCA 180

TAAGCCACTC AACCCAGAAT CTTATTTGAA TTATAATCCA GAAACATCAG GTGACGTGTG 240

AGACTACTGT ATGAGAAAGA GACAGTTTAA GGGTCAGTCC AATGGAAAAA AGAGTTCTCA 300

GAGCTTTCTT TAGCTTATTC TCATCAAAGA GCTTTCTCTG CAGAAGGAAC CTACTGGTTC 360

CTCCTTTCCA GTCCTAGAAA TCCTGACCTA GAGTGGCTTA ATCCTGCTAG CACCTCTCTC 420

TCGCACTCTG GTGCCAAATG ACTCCAGGAA CTGGGCCATG ATGTGGTGGG AATGACCTTA 480

CCCTGAGCAT GTCACTCATG CATTGAACAA CAGCTAAGAG CAGAGCTTAG AGCTTAGAGC 540

TGGGCCCTGT AAGGTGAGAG GAATCACATC CTGCAGAAGT CTGTCCTGAG AAGCAGGTAC 600

TCCTGTCACA GCAGAGACAC AGTGGATACC TGAGTAACAA TAATACAAGA CAGGACGTGG 660

GMACAGCAAA AGATTTGGGT GTCAGAAGAR GCCGAGAACA CTTYCAGGCA GGAACATTCA 720

RARTTGTTCT TGGAGGAART AGGCMCSAAG GCTGGGCAGG ATTTCMCGGG GCAGAGATGG 780

AGCAAGCAAT TGAAATGAAA GCCATGGCAT GGGAAAAGGA GCACTGGCCA CAGGGAGTGC 840

AACGTTGTGA TGCAAGGCCA CTGTGGAGCC AT 872

(2) INFORMATION FOR SEQ ID NO: 39:

(i) SEQUENCE CHARACTERISTICS:
(A) LENGTH: 812 base pairs
(B) TYPE: nucleic acid
(C) STRANDEDNESS: double
(D) TOPOLOGY: linear

(xi) SEQUENCE DESCRIPTION: SEQ ID NO: 39:

GGCAGAGGCT CACCCCAGCA GAGATTGAGG GGGAACCGTG ATGAAATTTT TAAGTATTCT 60

GCTTGATGAT AATAATTTTY CTCTTATGTT AATGTTGGCT CCGTTTGGGT GTTTAGCTTT 120

TGAAAGGAGT ATGAAAATGC GGAATGGGGC TTTGGGGCTT GAGGAGGTGT GATCTCTAGT 180

GTTTAAAAAA TTTAATTGCA CAAATAGAAA TAATTCACCC ACATTATTGA ACCCCACTAA 240

AGCATATCCT TTTTGTCCAT ATTCCTTTCC TGCTGCCCTC GTGTGTACCA TTATTACTCA 300

GTTGTGATTT GAGCTCGTTC CACTTAAAGT CATTCATAGA TACTTTTGCG TCGTGTTKGA 360

ATATTTATTG AATTTCTATT CTGTGTTTTA CTTAATTACT TTATTATGGA ACCTTTACAC 420

AGGTCTGGTG TACTTGTTCT TTGAAAAGTC TTATGTTGAC CACCATCACT GAGCATATAG 480

CTTTTTCCTT ATTTCCTTGG GATAATTACC CGAAGTGGAA ATACCGAATC AAACTTCTGT 540

TTTCTTTCTT TGGCACTATT ATATAAATTG TTTTCCAAAC AAGGCATGTT TACAATAGAC 600

ATTTTTCAAA ATCTGGGTAT TTGTCCTATT TTGCTCTCTG TATGCAGAAT TCAGCGGGGT 660

GCCAAGTCGT TTTCTGTGTG GGTTGAGAGA CAGGCTGTGC AGCCCACTGT TGCATAGGAC 720

TAACTACTAC AAATCATGCT GAGACCGAGC TATTTTTGCT GCTTAGARGC TTTGCAGCCT 780

TGAGTAAGTT TCGNCATCTG GAAACNTTGN AA 812

(2) INFORMATION FOR SEQ ID NO: 40:

(i) SEQUENCE CHARACTERISTICS:
(A) LENGTH: 1515 base pairs
(B) TYPE: nucleic acid
(C) STRANDEDNESS: double
(D) TOPOLOGY: linear

(xi) SEQUENCE DESCRIPTION: SEQ ID NO: 40:

AATTCGGCAC GAGGGAAATT CAAGCACTTT TCCTAAAAGA AGGGGGAATG GATGCTGAAA 60

CAACACGTNT CCCACAAAGG GAGCAGACAC TGGGCTTGTG AAGCTGCCCC ATACCTTCCC 120

CACAGAACTG GGGTCCGGCC TCCCTGACAT GCAGATTTCC ACCCAGAAGA CAGAGAAGGA 180

GCCAGTGGTC ATGGAATGGG CTGGGGTCAA AGACTGGGTG CCTGGGAGCT GAGGCAGCCA 240

CCGTTTCAGC CTGGCCAGCC CTCTGGACCC CGAGGTTGGA CCCTACTGTG ACACACCTAC 300

CATGCGGACA CTCTTCAACC TCCTCTGGCT TGCCCTGGCC TGCAGCCCTG TTCACACTAC 360

CCTGTCAAAG TCAGATGCCA AAAAAGCCGC CTCAAAGACG CTGCTGGAGA AGAGTCAGTT 420

TTCAGATAAG CCGGTGCAAG ACCGGGGTTT GGTGGTGACG GACCTCAAAG CTGAGAGTGT 480

GGTTCTTGAG CATCGCAGCT ACTGCTCGGC AAAGGCCCGG GACAGACACT TTGCTGGGGA 540

TGTACTGGGC TATGTCACTC CATGGAACAG CCATGGCTAC GATGTCACCA AGGTCTTTGG 600

GAGCAAGTTC ACACAGATCT CACCCGTCTG GCTGCAGCTG AAGAGACGTG GCCGTGAGAT 660

GTTTGAGGTC ACGGGCCTCC ACGACGTGGA CCAAGGGTGG ATGCGAGCTG TCAGGAAGCA 720

TGCCAAGGGC CTGCACATAG TGCCTCGGCT CCTGTTTGAG GACTGGACTT ACGATGATTT 780

CCGGAACGTC TTAGACAGTG AGGATGAGAT AGAGGAGCTG AGCAAGACCG TGGTCCAGGT 840

GGCAAAGAAC CAGCATTTCG ATGGCTTCGT GGTGGAGGTC TGGAACCAGC TGCTAAGCCA 900

GAAGCGCGTG ACCGACCAGC TGGGCATGTT CACGCACAAG GAGTTTGAGC AGCTGGCCCC 960

CGTGCTGGAT GGTTTCAGCC TCATGACCTA CGACTACTCT ACAGCGCATC AGCCTGGCCC 1020

TAATGCACCC CTGTCCTGGG TTCGAGCCTG CGTCCAGGTC CTGGACCCGA AGTCCAAGTG 1080

GCGAAGCAAA ATCCTCCTGG GGCTCAACTT CTATGGTATG GACTACGCGA CCTCCAAGGA 1140

TGCCCGTGAG CCTGTTGTCG GGGCCAGGTA CATCCAGACA CTGAAGGACC ACAGGCCCCG 1200

GATGGTGTGG GACAGCCAGG YCTCAGAGCA CTTCTTCGAG TACAAGAAGA GCCGCAGTGG 1260

GAGGCACGTC GTCTTCTACC CAACCCTGAA GTCCCTGCAG GTGCGGCTGG AGCTGGCCCG 1320

GGAGCTGGGC GTTGGGGTCT CTATCTGGGA GCTGGGCCAG GGCCTGGACT ACTTCTACGA 1380

CCTGCTCTAG GTGGGCATTG CGGCCTCCGC GGTGGACGTG TTCTTTTCTA AGCCATGGAG 1440

TGAGTGAGCA GGTGTGAAAT ACAGGCCTTC ACTCCGTTAA AAAAAAAAAA AAAAAAAAAA 1500

AAAAAAAAAA AAAAA 1515

(2) INFORMATION FOR SEQ ID NO: 41:

(i) SEQUENCE CHARACTERISTICS:
(A) LENGTH: 704 base pairs
(B) TYPE: nucleic acid
(C) STRANDEDNESS: double
(D) TOPOLOGY: linear

(xi) SEQUENCE DESCRIPTION: SEQ ID NO: 41:

AAGATGGTGG CGCCCAGAGC TTCGCTCTAT GCTGCTCCCC TGAGAGAGGC GTTTCCATCA 60

ACCAGTTTTG CAAGGAGTTC AATGAGAGGA CAAAGGACAT CAAGGAAGGC ATTCCTCTGC 120

CTACCAAGAT TTTAGTGAAG CCTGACAGGA CATTTGAAAT TAAGATTGGA CAGCCCACTG 180

TTTCCTACTT CCTGAAGGCA GCAGCTGGGA TTGAAAAGGG GGCCCGGCAA ACAGGGAAAG 240

AGGTGGCAGG CCTGGTGACC TTGAAGCATG TGTATGAGAT TGCCCGCATC AAAGCTCAGG 300

ATGAGGCATT TGCCCTGCAG GATGTACCCC TGTCGTCTGT TGTCCGCTCC ATCATCGGGT 360

CTGCCCGTTC TCTGGGCATT CGCGTGGTGA AGGACCTCAG TTCAGAAGAG CTTGCAGCTT 420

TCCAGAAGGA ACGAGCCATC TTCCTGGCTG CTCAGAAGGA GGCAGATTTG GCTGCCCAAG 480

AAGAAGCTGC CAAGAAGTGA CCCTTGCCCC ACCAACTCCC AGATTTCAAA GGAGGTAGTT 540

GCAAAAGCTG TGCCCAAGGG GAGGAAGGAG GTCACACCAA TATGATGATG GTTTTCATGA 600

CTTTGAATGA TATATTTTTG TACATCTAGC TGTATCGAGG CATCAGGCCT GAATAAACAT 660

CCTTTCTTAA AAAAAAAAAA AAAAAAAAAA AAAAAAAAAA AAAA 704

(2) INFORMATION FOR SEQ ID NO: 42:

(i) SEQUENCE CHARACTERISTICS:
(A) LENGTH: 1094 base pairs
(B) TYPE: nucleic acid
(C) STRANDEDNESS: double
(D) TOPOLOGY: linear

(xi) SEQUENCE DESCRIPTION: SEQ ID NO: 42:

GGCAGCTTTC TTACAAACCC ATCCTTCTGA AATGTTGCTT CAAATTCATC CTCTGCTCCC 60

CAGTCCCACT ATTCCACACA TACTGTTACT GTTTCTTTAT CCTACTTTCT CAATTTTGGA 120

ACATAGTTGC AGTTACTGCA TTGAATACCT GTGGGTTTGC CTGTTGTTCT GTCTGTCTCT 180

GTGGTTCTTG TAATANTGGA TCCCAGAGAT AAAATGGACA GTTGTNATGC ACAGTTAATT 240

CAGAAACTAG ACCTTACTTG CTGTGTGAAA TACCAACTAA ATTCTCAGTG AACTCAGCTG 300

ANCTTTATCT CCTTTTGTTT CCCCAATTTA TAATTTCAGT TCAGGCCCAG AAAGATGGAA 360

TCCCAGCTAA GAAATACAAG TTACACCCTG TACTAGCAGC CCATGTGTGC ATGTTCTTTA 420

AGTGCTCTTG CAGCTATGTC ATTTATATTG ATTTCCCTGT ATTATTATAA GCAAAGCAAA 480

TTTGAGGAAA AAAACCCATA ATACCACACC TCATTTTTTT CAAGTAATAG GGTCATAAGT 540

CTCATYCTYC ATATAATATG TTGAGTATGC AGTATATTAT GTGTTAGGCT CTGGANAGGC 600

AGAGGTTAGA TCATGTWACA GATCATATCK GATTAGGCAG ATAAACAGTA TTTTAACCTT 660

TTCCTTATTA TATGTAACTT GCTTTCAGGT TTTTTAATGT TACTATTATG TCTTTAATAT 720

ATTATCTTTA TTTGTACTTT TGTATACAGA GTGATTTTCC TTTTTTAAAA AAAATTGTGT 780

CTTTAGGATG GATTCCAAAG ATGTGGAATC AGTAGGTTTA AGGAATATGG ATATTTTGGC 840

TGGCAAGGTG GCTCACACCT GTAATCCCAG CACTTTGGGA GGCTGAGGTG GGTGGATCAC 900

CTGAAGTCAG GAGTTCGAGA CCAGCCTGAC CAACATGGCG AAACCCTGTT TNTACTAAAG 960

ACACACWWAA AATTRGCCAG TGGTGGTGGC ATGTGCTTGT AGTCCCACTT AGCTACTCGA 1020

GAGGCTGAGG CAGGAGAATC GCTTGAACCC GGGAGGCAGA GGTTGCAGTG AGGCAAGATG 1080

GCACCTCTAC ACTC 1094

(2) INFORMATION FOR SEQ ID NO: 43:

(i) SEQUENCE CHARACTERISTICS:

(A) LENGTH: 1821 base pairs
(B) TYPE: nucleic acid
(C) STRANDEDNESS: double
(D) TOPOLOGY: linear

(xi) SEQUENCE DESCRIPTION: SEQ ID NO: 43:

TGGCTTAGGC CATCACCCTT CCCTTGGCTG GAACTACTGG ACAGACCCTT TTGAGATGTG 60

CCTGTGGTGC TGTGGAGATG TGTGTAGTGG TCTTAGCTCT TTGTTGAGCT TGTGTGTGTG 120

TTGTGTAGTC TTAGCTGTAT GCTGAAATTG GGCGTGTGTT GGAGGGCTTC TTAGCTCTTT 180

GGTGAGATTG TATTTCTATG TGTTTGTATC ASCTGAATGT TGCTGGAAAT AAAACCTTGG 240

TTTG[?][?]AAGG CTC[?]TTTTTG TGGGAAGTAA GTAGGGGAAA AGGTCTTTGA GGGTTCCTAG 300

GCTCCTTTGT ACAACAGGAA AATGCCTCAA AGCCTTGCTT CCCAGCAACC TGGGGCTGGT 360

TCCCAGTGCC TGGTCCTGCC CCTTCCTGGT TCTTATCTCA AGGCAGAGCT TCTGAATTTC 420

AGGCCTTCAT TCCAGAGCCC TCTTGTGGCC AGGCCTTCCT TTGCTGGAGG AAGGTACACA 480

GGGTGAAGCT GATGCTGTAC TTGGGGGATC TCCTTGGCCT GTTCCACCAA GTGAGAGAAG 540

GTACTTACTC TTGTACCTCC TGTTCAGCCA GGTGCATTAA CAGACCTCCC TACAGCTGTA 600

GGAACTACTG TCCCAGAGCT GAGGCAAGGG GATTTCTCAG GTCATTTGGA GAACAAGTGC 660

TTTAGTAGTA GTTTAAAGTA GTAACTGCTA CTGTATTTAG TGGGGTGGAA TTCAGAAGAA 720

ATTTGAAGAC CAGATCATGG GTGGTCTGCA TGTGAATGAA CAGGAATGAG CCGGACAGCC 780

TGGCTGTCAT TGCTTTCTTC CTCCCCATTT GGACCCTTCT CTGCCCTTAC ATTTTTGTTT 840

CTCCATCTAC CACCATCCAC CAGTCTATTT ATTAACTTAG CAAGAGGACA AGTAAAGGGC 900

CCTCTTGGCT TGATTTTGCT TCTTTCTTTC TGTGGAGGAT ATACTAAGTG CGACTTTGCC 960

CTATCCTATT TGGAAATCCC TAACAGAATT GAGTTTTCTA TTAAGGATCC AAAAAGAAAA 1020

ACAAAATGCT AATGAAGCCA TCAGTCAAGG GTCACATGCC AATAAACAAT AAATTTTCCA 1080

GAAGAAATGA AATCCAACTA GACAAATAAA GTAGAGCTTA TGAAATGGTT CAGTAAGGAT 1140

GAGTTTGTTG TTTTTTGTTT TGTTTTGTTT TGKTTTTTTA AAGACGGAGT CTCGCTCTGT 1200

CACTCAGGCT GGAGTGCAGT GGTATGATCT TGGCTCACTG TAACCTCCGC CTCCCGGGTT 1260

CAAGCCATTC TCCTGCCTCA GTCTCCTGAG TAGCTGGGAT TACAGGTGCG TGCCACCATG 1320

CCTGGCTAAT TTTTGTGTTT TTAGTAGAGA CAGGGTTTCA CCATGTTGGT CGGGCTGGTC 1380

TCAAACTCCT GACCTCTTGA TCCGCCTGCC TTGGCCTCCC AAAGTGATGG GATTACAGAT 1440

GTGAGCCACC CGTGCCCTAG CCAAGGATGA GATTTTTAAA GTATGTTTCA GTTCTGTGTC 1500

ATGGTTGGAA GACAGAGTAG GAAGGATATG GAAAAGGTCA TGGGGAAGCA GAGGTGATTC 1560

ATGGCTCTGT GAATTTGAGG TGAATGGTTC CTTATTGTCT AGGCCACTTG TGAAGAATAT 1620

GAGTCAGTTA TTGCCAGCCT TGGAATTTAC TTCTCTAGCT TACAATGGAC CTTTTGAACT 1680

GGAAAACACC TTGTCTGCAT TCACTTTAAA ATGTCAAAAC TAATTTTTAT AATAAATGTT 1740

TATTTTCACA TTGAAAAAAA AAAAAAATTT AAAAACYCGG GGGGGGCCCS G[?]ACCCCATT 1800

NGCCCCTAAG GGGGGGGGTT T 1821

(2) INFORMATION FOR SEQ ID NO: 44:

(i) SEQUENCE CHARACTERISTICS:
(A) LENGTH: 1024 base pairs
(B) TYPE: nucleic acid
(C) STRANDEDNESS: double
(D) TOPOLOGY: linear

(xi) SEQUENCE DESCRIPTION: SEQ ID NO: 44:

GGGGCACAGT TGAAGAAGCG ACCGAGGGAC TGGGAGTCGT TAGTGAGGAT GACGCGGCAT 60

GGCAAGAACT GCACCGCAGG GCCGTCTACA CCTACCACGA GAAGAAGAAG GACACAGCGG 120

CCTCGGGCTA TGGGACCCAG AACATTCGAC TGAGCCGGGA TGCCGTGAAG GACTTCGACT 180

GCTGTTGTCT CTCCCTGCAG CCTTGCCACG ATCCTGTTGT CACCCCAGAT GGCTACCTGT 240

ATGAGCGTGA GGCCATCCTG GAGTACATTC TGCACCAGAA GAAGGAGATT GCCCGGCAGA 300

TGAAGGCCTA CGAGAAGCAG CGGGGCACCC GGCGCGAGGA GCAGAAGGAG CTTCAGCGGG 360

CGGCCTCGCA GGACCATGTG CGGGGCTTCC TGGAGAAGGA GTCGGCTATC GTGAGCCGGC 420

CCCTCAACCC TTTCACAGCC AAGGCCCTCT CGGGCACCAG CCCAGATGAT GTCCAACCTG 480

GGCCCAGTGT GGGTCCTCCA AGTAAGGACA AGGACAAAGT GCTGCCCAGC TTCTGGATCC 540

CGTCGCTGAC GCCCGAAGCC AAGGCCACCA AGCTGGAGAA GCCGTCCCGC ACGGTGACCT 600

GCCCCATGTC AGGGAAGCCC CTGCGCATGT CGGACCTGAC GCCCGTGCAC TTCACACCGC 660

TAGACAGCTC CGTGGACCGC GTGGGGCTCA TCACCCGCAG CGAGCGCTAC GTGTGTGCCG 720

TGACCCGCGA CAGCCTGAGC AACGCCACCC CCTGCGCTGT GCTGCGGCCC TCTGGGGCTG 780

TGGTCACCCT CGAATGCGTG GAGAAGCTGA TTCGGAAGGA CATGGTGGAC CCTGTGACTG 840

GAGACAAACT CACAGACCGC GACATCATCG TGCTGCAGCG GGGCGGTACC GSTTCGCGGG 900

CTCCGGAGTG AAGCTGCAAG CGGAGAAATC ACGGCCGGTG ATGCAGGCCT GAGTGTGTGC 960

GGGAGACCAA ATAAACCGGC TTGGGTGCGC AAAAAAAAAA AAAAAAAAAA AAAAAAAAAA 1020

AAAA 1024

(2) INFORMATION FOR SEQ ID NO: 45:

(i) SEQUENCE CHARACTERISTICS:
(A) LENGTH: 983 base pairs
(B) TYPE: nucleic acid
(C) STRANDEDNESS: double
(D) TOPOLOGY: linear

(xi) SEQUENCE DESCRIPTION: SEQ ID NO: 45:

CGACACGGCT GCGAGAAGAC GACAGAAGGG CCCGACCGCG AGCCGTCCAG GTCTCAGTGC 60

TGTGCCCCCC CCAGAGCCTA GAGGATGTTT CATGGGATCC CAGCCACGCC GGGCATAGGA 120

GCCCCTGGGA ACAAGCCGGA GCTGTATGAG GAAGTGAAGT TGTACAAGAA CGCCCGGGAG 180

AGGGAGAAGT ACGACAACAT GGCAGAGCTG TTTGCGGTGG TGAAGACAAT GCAAGCCCTG 240

GAGAAGGCCT ACATCAAGGA CTGTGTCTCC CCCAGCGAGT ACACTGCAGC CTGCTCCCGG 300

CTCCTGGTCC AATACAAAGC TGCCTTCAGG CAGGTCCAGG GCTCAGAAAT CAGCTCTATT 360

GACGAATTCT GCCGCAAGTT CCGCCTGGAC TGCCCGCTGG CCATGGAGCG GATCAAGGAG 420

GACCGGCCCA TCACCATCAA GGACGACAAG GGCAACCTCA ACCGCTGCAT CGCAGACGTG 480

GTCTCGCTCT TCATCACGGT CATGGACAAG CTGCGCCTGG AGATCCGCGC CATGGATGAG 540

ATCCAGCCCG ACCTGCGAGA GCTGATGGAG ACCATGCACC GCATGAGCCA CCTCCCACCC 600

GACTTTGAGG GCCGCCAGAC GGTCAGCCAG TGGCTGCAGA CCCTGAGCGG CATGTCGGCG 660

TCAGATGAGC TGGACGACTC ACAGGTGCGT CAGATGCTGT TCGACCTGGA GTCAGCCTAC 720

AACGCCTTCA ACCGCTTCCT GCATGCCTGA GCCCGGGGCA CTAGCCCTTG CACAGAAGGG 780

CAGAGTCTGA GGCGATGGCT CCTGGTCCCC TGTCCGCCAC ACAGGCCGTG GTCATCCACA 840

CAACTCACTG TCTGCAGCTG CCTGTCTGGT GTCTGTCTTT GGTGTCAGAA CTTTTGGGCC 900

GGGCCCCTCC CCACAATAAA GATGCTCTCC GACCTTCAAA AAAAAAAAAA AAAAAAAAGR 960

KGSGGCCGGT CCCCANTCCC CCC 983

(2) INFORMATION FOR SEQ ID NO: 46:

(i) SEQUENCE CHARACTERISTICS:
(A) LENGTH: 2421 base pairs
(B) TYPE: nucleic acid
(C) STRANDEDNESS: double
(D) TOPOLOGY: linear

(xi) SEQUENCE DESCRIPTION: SEQ ID NO: 46:

CCGGCTGATC GCTGCCGCTC CGCCAATACA ATAGAGCCAK CCACTACCAG CAGCCTGGCC 60

CTCTTCCTCC TTCTCCAGAG AGACCAATCC AGCCGAACTC GGGGTTTGCC TGAGGAGAAG 120

GAGGAAGTGA CCATGGACAC AAGTGAAAAC AGACCTGAAA ATGATGTTCC AGAACCTCCC 180

ATGCCTATTG CAGACCAAGT CAGCAATGAT GACCGCCCGG AGGGCAGTGT TGAAGATGAG 240

GAGAAGAAAG AGAGCTCGCT GCCCAAATCA TTCAAGAGGA AGATCTCCGT TGTCTCAGCT 300

ACCAAGGGGG TGCCAGCTGG AAACAGTGAC ACAGAGGGGG GCCAGCCTGG TCGGAAACGA 360

CGCTGGGGAG CCAGCACAGC CACCACACAG AAGAAACCTT CCATCAGTAT CACCACTGAA 420

TCACTAAAGA GCCTCATCCC CGACATCAAA CCCCTGGCGG GGCAGGAGGC TGTTGTGGAT 480

CTTCATGCTG ATGACTCTCG CATCTCTGAG GATGAGACAG AGCGTAATGG CGATGATGGG 540

ACCCATGACA AGGGGCTGAA AATATGCCGG ACAGTCACTC AGGTAGTACC TGCAGAGGGC 600

CAGGAGAATG GGCAGAGGGA AGAAGAGGAA GAAGAGAAGG AACCTGAAGC AGAACCTCCT 660

GTACCTCCCC AGGTGTCAGT AGAGGTGGCC TTGCCCCCAC CTGCAGAGCA TGAAGTAAAG 720

AAAGTGACTT TAGGAGATAC CTTAACTCGA CGTTCCATTA GCCAGCAGAA GTCCGGAGTT 780

TCCATTACCA TTGATGACCC AGTCCGAACT GCCCAGGTGC CCTCCCCACC CCGGGGCAAG 840

ATTAGCAACA TTGTCCATAT CTCCAATTTG GTCCGTCCTT TCACTTTAGG CCAGCTAAAG 900

GAGTTGTTGG GGCGCACAGG AACCTTGGTG GAAGAGGCCT TCTGGATTGA CAAGATCAAA 960

TCTCATTGCT TTGTAACGTA CTCAACAGTA GAGGAAGCTG TTGCCACCCG CACAGCTCTG 1020

CACGGGGTCA AATGGCCCCA GTCCAATCCC AAATTCCTTT GTGCTGACTA TGCCGAGCAA 1080

GATGAGCTGG ATTATCACCG AGGCCTCTTG GTGGACCGTC CCTCTGAAAC TAAGACAGAG 1140

GAGCAGGGAA TACCACGGCC CCTGCACCCC CCACCCCCAC CCCCGGTCCA GCCACCACAG 1200

CACCCCCGGG CAGAGCAGCG GGAGCAGGAA CGGGCAGTGC GGGAACAGTG GGCAGAACGG 1260

GAACGGGAAA TGGAGCGGCG GGAGCGGACT CGATCAGAGC GTGAATGGGA TCGGGACAAA 1320

GTTCGAGAAG GGCCCCGTTC CCGATCAAGG TCCCGTRACC GCCGCCGCAA GGAACGTGCG 1380

AAGTCTAAAG AAAAGAAGAG TGAGAAGAAA GAGAAAGCCC AGGAGGAACC ACCTGCCAAG 1440

CTGCTGGATG ACCTTTTCCG AAAGACCAAG GCAGCTCCCT GCATCTATTG GCTCCCACTG 1500

ACTGACAGCC AGATCGTTCA GAAAGAGGCA GAGCGGGCCG AACGGGCCAA GGAGCGGGAG 1560

AAGCGGCGAA AGGAGCAAGA AGAAGAAGAG CAAAAGGAGC GGGAGAAGGA AGCCGAGCGG 1620

GAACGGAACC GACAGCTGGA GCGAGAGAAA CGTCGGGAGC ACAGTCGGGA GAGGGACAGG 1680

GAGAGAGAGA GAGAAAGGGA GCGGGACAGG GGGGACCGAG ATCGGGATAG GGAAAGGGAC 1740

CGAGAACGAG GCAGGGAAAG GGATCGCAGG GACACCAAGC GCCACAGCAG AAGCCGGAGT 1800

CGGAGCACAC CTGTGCGGGA CCGGGGTGGG CGCCGCTAGC TGGGAAAACA CTAGAGCTGC 1860

AGGTACCAGC CACTCGGCCC CAGGGGGTTA TGGCCACAGA GGGATAGGCA CAGTCTCCAC 1920

CACCCTGGAG CCAAGGGTCT TTCACATCAC CTATCCCTAC ATACATACCA AATGGAAAAG 1980

TGGCCATCCT TTTCCCCCCA AACACACCCC CTTAACCTAT CTCTTGGGAC TTAGCCCGAC 2040

CCTCCCTCTC ATTTCCCATT AAGTCTGAGA GGCAAGAGCT AGGTTAGGCA AGGAGGTGGT 2100

TGGCCAGAGA TGGGGAACAG CCAGGTGCCC CAGTCCTCTG ATTTTTCCTC CATCCTGCTT 2160

ACCACCTCCC TGGGTACTTA CAGCCTTCTC TTGGGAACAG CCGGGGCCAG GACTGGGTCA 2220

CCTATGAGCT GAATCAGCAT CTCCTCCTGA GTCCCAGGGC CCCTGCAGTT CCCAGTCTCT 2280

TCTGTCCTGC AGCCCTTGCC TCTTTCCCAC AGGTTCCACT TTATATCCAC CTTTTCCTTT 2340

TGTTCAATTT TTATTTTTAT TTTTTTTATT ATTAAATGAT GTGGTCTATG GAAAAAAAAA 2400

TAAAAATCTG ACTTAGTTTT A 2421

(2) INFORMATION FOR SEQ ID NO: 47:

(i) SEQUENCE CHARACTERISTICS:
(A) LENGTH: 840 base pairs
(B) TYPE: nucleic acid
(C) STRANDEDNESS: double
(D) TOPOLOGY: linear

(xi) SEQUENCE DESCRIPTION: SEQ ID NO: 47:

CTCAAACTCC TGAGCTGAAG CGATCTACCT GCCTCAGCTA GGATTACAGG TGTGAGCCAC 60

CGCACCCAAC CTCAATAAGC KTATTTGATA AAAKATATGC AAGCTCCCTT TATKCACTTT 120

TCATTCAGAA TGTTTAGTAA TTTGTATTGT TTTTCAGATT TTCAGCCCAA TATATCTCCY 180

TGCCCACTGT GTCACTGTAT TCTACCTAWA CATCATCACG TGTTTCTGCT ATTGGCTGTA 240

TGATGGAACA CTGCGGCTCA TTTTCCTGAA AACTGCCGAT AGTGCATAGA RTGCTGGGAT 300

GGAAACCAGA ARCTTTGAAT TCAAGCCTTG GTTCTGCCTT GTTTTTGCTT GGGTGGCCTT 360

GAGTCAGCCA CATACCTTTT AAAATCTCAA TTTATTAGAA ATTATTCCAA ATCAAAATCA 420

AATGAGAAGG TATATACAAA AGTGCTTTAT CCCACAATAA ACTATTCAAG AGAGAGCAAA 480

GGAGAGGACA TTTACTCAAC ACCTCCTAAA AGGCAGCCAG TGAAATTAGG CATTTTATTT 540

AATCCTCCTG GCAACTCTGA GAGTAAAGCA TTATTAATCC CATTTTGGCT GTTTAAAGAA 600

ATTATTTGCA CTAGATTCCA GCTGTAGTTT AGYTTCAGAA AAAAAAATCC TGAGATGTGA 660

ATTCACAGCT TTCTGGGTTT AAAGCCCAAG CTCTATCACA TCATGCTATT ATTGTTACAT 720

TACTGCTAGT TCTATGAAAA GAAATACTAA TTTATGAAAT ACATCTTATC CAAAAAAAAA 780

AAAAAAAAAC TGGGAGGGGG GGCCCGTACC CAAATCGCCG GATAGTGATC GTAAACAATC 840

(2) INFORMATION FOR SEQ ID NO: 48:

(i) SEQUENCE CHARACTERISTICS:
(A) LENGTH: 2432 base pairs
(B) TYPE: nucleic acid
(C) STRANDEDNESS: double
(D) TOPOLOGY: linear

(xi) SEQUENCE DESCRIPTION: SEQ ID NO: 48:

GGCACGAGGC CCGGAACGCT GAGGAAGGGC CCGTCCCGCC TTCCCCGGCG CGCCATGGAG 60

CCCCGGGCGG TTGCAGAAGC CGTGGAGACG GGTGAGGAGG ATGTGATTAT GGAAGCTCTG 120

CGGTCATACA ACCAGGAGCA CTCCCAGAGC TTCACGTTTG ATGATGCCCA ACAGGAGGAC 180

CGGAAGAGAC TGGCGGASTG CTGGTCTCCG TCCTGGAACA GGGCTTGCCA CCCTCCCACC 240

GTGTCATCTG GCTGCAGAGT GTCCGAATCC TGTCCCGGGA CCGCAACTGC CTGGACCCGT 300

TCACCAGCCG CCAGAGCCTG CAGGCAYTAG CCTGYTATGY TGACATCTCT GTCTCTGAGG 360

GGTCCGTCCC AGAGTCCGCA GACATGGATG TTGTACTGGA GTCCCTCAAG TGCCTGTGCA 420

ACCTCGTGCT CAGCAGCCCT GTGGCACAGA TGCTGGCAGC AGAGGCCCGC CTAGTGGTGA 480

AGCTCACAGA GCGTGTGGGG CTGTACCGTG AGAGGAGCTT CCCCCACGAT GTCCAGTTCT 540

TTGACTTGCG GCTCCTCTTC CTGCTAACGG CACTCCGCAC CGATGTGCGC CANAGCTGTT 600

TCAGGAGCTG AAAGGAGTGC GCCTGCTAAC TGACACACTG GAGCTGACGC TGGGGGTGAC 660

TCCTGAAGGG AACCCCCCAC CCACGCTCCT TCCTTCCCAA GAGACTGAGC GGGCCATGGA 720

GATCCTCAAA GTGCTCTTCA ACATCACCCT GGACTCCATC AAGGGGGAGG TGGACGAGGA 780

AGACGCTGCC CTTTACCGAC ACCTGGGGAC CCTTCTCCGG CACTGTGTGA TGATCGCTAC 840

TGCTGGAGAC CGCACAGAGG AGTTCCACGG CCACGCAGTA ASCCTCCTGG GGAACTTGCC 900

CCTCAAGTGT CTGGATGTTC TCCTCACCCT GGAGCCACAT GGAGACTCCA CGGAGTTCAT 960

GGGAGTGAAT ATGGATGTGA TTCGTGCCCT CCTCATCTTC CTAGAGAAGC GTTTGCACAA 1020

GACACACAGG CTGAAGGAGA GTGTAGCTCC CGTGCTGAGC GTGCTGACTG AATGTGCCCG 1080

GATGCACCGC CCAGCCAGGA AGTTCCTGAA GGCCCAGGTG CTGCCCCCTC TGCGGGATGT 1140

GAGGACACGG CCTGAGGTTG GGAGATGCT GCGGAACAAG CTTGTCCGCC TCATGACACA 1200

CCTGGACACA GATGTGAAGA GGGTGGCTGC CGAGTTCTTG TTTGTCCTGT GCTCTGAGAG 1260

TGTGCCCCGA TTCATCAAGT ACACAGGCTA TGGGAATGCT GCTGGCCTTC TGGCTGCCAG 1320

GGGCCTCATG GCAGGAGGCG GCCCGAGGGC AGTACTCAGA GGATGAGGAC ACAGACACAG 1380

ATGAGTACAA GGAAGCCAAA GCCAGCATAA ACCCTGTGAC CGGGAGGGTG GAGGAGAAGC 1440

CGCCTAACCC TATGGAGGGC ATGACAGAGG AGCAGAAGGA GCACGAGGCC ATGAAGCTGG 1500

TGACCATGTT TGACAAGCTC TCCAGGAACA GAGTCATCCA GCCAATGGGG ATGAGTCCCC 1560

GGGGTCATCT TACGTCCCTG CAGGATGCCA TGTGCGAGAC TATGGAGCAG CAGCTCTCCT 1620

CGGACCCTGA CTCGGACCCT GACTGAGGAT GGCAGCTCTT CTGCTCCCCC ATCAGGACTG 1680

GTGCTGCTTC CAGAGACTTC CTTGGGGTTG CAACCTGGGG AAGCCACATC CCACTGGATC 1740

CACACCCGCC CCCACTTCTC CATCTTAGAA ACCCCTTCTC TTGACTCCCG TTCTGTTCAT 1800

GATTTGCCTC TGGTCCAGTT TCTCATCTCT GGACTGCAAC GGTCTTCTTG TGCTAGAACT 1860

CAGGCTCAGC CTCGAATTCC ACAGACGAAG TACTTTCTTT TGTCTGCGCC AAGAGGAATG 1920

TGTTCAGAAG CTGCTGCCTG AGGGCAGGGC CTACCTGGGC ACACAGAAGA GCATATGGGA 1980

GGGCAGGGGT TTGGGTGTGG GTGCACACAA AGCAAGCACC ATCTGGGATT GGCACACTGG 2040

CAGAGCMANT GTKTTGGGGT ATGTGCTGCA CTTCCCAGGG AGAAAACCTG TCAGAACTTT 2100

CCATACGAGT ATATCAGAAC ACACCCTTCC AAGGTATGTA TGCTCTGTTG TTCCTGTCCT 2160

GTCTTCACTG AGCGCAGGGC TGGAGGCCTC TTAGACATTC TCCTTGGTCC TCGTTCAGCT 2220

GCCCACTGTA GTATCCACAG TGCCCGAGTT CTCGCTGGTT TTGGCAATTA AACCTCCTTC 2280

CTACTGGTTT AGACTACACT TACAACAAGG AAAATGCCCC TCGTGTGACC ATAGATTGAG 2340

ATTTATACCA CATACCACAC ATAGCCACAG AAACATCATC TTGAAATAAA GAAGAGTTTT 2400

GGACAAAAAA AAAAAAAAAA AAAAAAAAAA AA 2432

(2) INFORMATION FOR SEQ ID NO: 49:

(i) SEQUENCE CHARACTERISTICS:
(A) LENGTH: 1742 base pairs
(B) TYPE: nucleic acid
(C) STRANDEDNESS: double
(D) TOPOLOGY: linear

(xi) SEQUENCE DESCRIPTION: SEQ ID NO: 49:

GTCCTGCAGG AGCTGCACGC GGCCGAGGTG CGCANGAACA AGGAGCAGCG AGAAGAGATG 60

TCGGGCTAAG GGCCCGGSAC GRGSGGCGCC CATCCTGCGA CGGAACACGT TCGGGTTTTG 120

GTTTTGTTTC GTTCACCTCT GTCTAGATGC AACTTTTGTT CCTCCTCCCC CACCCCAGCC 180

CCCAGCTTCA TGCTTCTCTT CCGCACTCAG CCGCCCTGCC CTGTCCTCGT GGTGAGTCGC 240

TGACCACGGC TTCCCCTGCA GGAGCCGCCG GGCGTGRAGA CGCGGTCCCT CGGTGCAGAC 300

ACCAGGCCGG GCGCGGCTGG GTCCCCCGGG GGCCCTGTGA GAGAGGTGGY GGTGACCGTG 360

GTAAACCCAG GGCGGTGGCG TGGGATCRCG GGTCCTTACG CTGGGCTGTC TGGTCAGCAC 420

GTGCAGGTCA GGGCAGGTCC TCTGAGCCGG CGCCCCTGGC CAGCAGGCGA GGCTACAGTA 480

CCTGCTGTCT TTCCAGGGGG AAGGGGCTCC CCATGAGGRA GGGGCGACGG GGGAGGGGGG 540

TGATGGTGCC TGGGAAGCCT GCKTGTGCAN CCGGTGCTTG TTGAACTGGC AGGCGGGTGG 600

GTGGGGGCTG CAGCTTTCCT TAATGTGGTT GCACAGGGGT CCTCTRAGAC CACCTGGCGT 660

GAGGTGGACA CCCTGGGCCT TCCTGGAAGC CTGCAGTTGG GGCCTGCCCC TGAGTCTGCT 720

GGGGAGTGGG CATTCTCTGC CAGGGACCCA TGAGCAGGCT GCATGGTCTA GAGGTTGTGG 780

GCAGCATGGA CAGTCCCCCA CTCAGAAGTG CAAGAGTTCC AAAGAGCCTC TGGCCCAGGC 840

CCCTCCGTGG GACAGCCCCG CCGCCCCTCC CCACCAGGGC TTTGCAGATG TCCTTGAAAG 900

ACCCACCCTA GAGCCCTTTG GAGTGCTGGC CCCTCCTGTG CCCTCTGCCC TGGTGGAAGC 960

GGCASCACAA GTCCTCCTCA GGGAGCCCCA AGGGGGATTT TKTGGGACCG CTGCCCACAG 1020

ATCCAGGTGT TGGAAGGGCA GCGGGTAAGG TTCCCAAGCC AGCCCCAACA CCCTTCCCAC 1080

TTGGCACCCA GAGGGGGCTG TGGGTGGAGG CCTGACTCCA GGCCTCTCCT GCCCACACCC 1140

TCTGGGCTGA GTTCCTTCTT TCCCTTGGAC GCCCAGTGCT GGCCTTGGAG GACGGTCAGC 1200

TGGAGGATGG CGGTGGGGGA GGCTGTCTTT GTACCACTGC AGCATCCCCC ACTTCTCCAC 1260

GGAAGCCCCA TCCCAAAGCT GCTGCCTGGC CCCTTGCTGT AAAGTGTGAA GGGGGCGGCT 1320

GAGTTCTCTT AGGACCCAGA GCCAGGGCCC TCAACTTCCA TCCTGCGGGA GGCCTTGGCC 1380

GGGCACTGCC AGTGTCTTCC AGAGCCACAC CCAGGGACCA CGGGAGGATC CTGACCCCTG 1440

CAGGGCTCAG GGGTCAGCAG GGACCCACTG CCCCATCTCC CTCTCCCCAC CAAGACAGCC 1500

CCAGAAGGAG CAGCCAGCTG GGATGGAAAC CCAAGGCTGT CCACATCTGG CTTTTGTGGG 1560

ACTCAGAAAG GGAAGCAGAA CTGAGGGCTG GGATATTCCT CATGGTGGCA GCGCTCATAG 1620

CGAAAGCCTA CTGTAATATG CACCCATCTC ATCCACGTAG TAAAGTGAAC TTAAAAATTC 1680

AATCAAATGA ACAATTAAAT AAACACCTGT GTGTTTAAGA AAAAAAAAAA AAAAAAACTG 1740

CG 1742

(2) INFORMATION FOR SEQ ID NO: 50:

(i) SEQUENCE CHARACTERISTICS:
(A) LENGTH: 1487 base pairs
(B) TYPE: nucleic acid
(C) STRANDEDNESS: double
(D) TOPOLOGY: linear

(xi) SEQUENCE DESCRIPTION: SEQ ID NO: 50:

GGCACGAGCC TCCGCGAACT GTGGAGTCGG CGGAGGGCTG GAATCAGCGT GGGCTCCAGG 60

TCGCTGGCAG CCGGGTGGCA GAACTCTTCC GAGGCTCCTT GGGAAGAAGC TACACCCGAG 120

GGAGCCGGAT GGGCCTCGAA AACCTGGCCC GCTCTGGTTC TGTACCATTG CAAGGGGAAC 180

CGTAAACTGA GCTTTTCTAA CGTGGGTTTC TGCCAAGTAC TTTTCCAGCT GCCCCCTTCC 240

CCCCAGCACA CAGGAGAGCC TCTGTGTAGC CAGCGCTTGA CAGTCGTTAG GTAGGTTGTA 300

CTGTGTAGGG AGGAGCTCAA GATCATGAAT GGTTGTCACA GGAGAAAGCG GTTGCATCTT 360

TGCAAAACTA TATACCTGCT GTGGTTTGTG TTTTCTTTTC TGCTGAGTAA TGAAGTTGTA 420

AGTTCACACT GGCACATTCT CAGGGCTGTG CAGATTATTT GCACTTTATT TCATAGGTGR 480

ATAAGTGCTT TTTAGCTTTC TTTGTATATT GAGTTGCTTT TGAATTGCTT CCCATATTTT 540

TATTTCATAC AAACTGAACA ATTGTGGCCC CTCTATTTTA TTTATAAAGG TTCAGTGTAT 600

CTTTGCCTGC CTACATCAAT CTGCAAGGGA GTTGCAGAAA GCCTCATGTT CATCGAGCCG 660

TGAGTCACAA CCAATTTCTA AGCTGTTATA ACAAAAAAGT GTTTGCTTTT TTTCACAAGT 720

AACTTTAAAA GTGTAGTTTA GAAAGAAAAC ATTTTCAATA AAAAGACACT ACATTAATCC 780

TGGATGCTTG CAAATCCTAA AATMTATTCC TCCTCTAGCG TTGCACAGCT CTGTGTTGTA 840

TACACAGACT AGCTTTAAAA TTTGTCACAT ACCACTTTAC CTTTACTTTT ATGTATCATT 900

CCCCCGACTT CCTTACTGCA GGTGTGGGCA AGAAAACTTT TCCTTTAACA CTTTTCAACA 960

GCGGGCATAA AATTCTGCAG CTGAGGTCTT GAAGAATGCA GATGGGTACA GTATGTGTTG 1020

GAGCTCACAG TGTGTATTGA CTAACCTAGT TCCTTTTTTG CTTTTTTTGG TATTGTCTTG 1080

TTAAAAGTGA CTCCCAGGTA GCAACTCTCT TTTTTAAGGG TGGGAACGAA AGGGACGTAG 1140

GAAGAATAGA TCTAGATTAT TTAACAGTCT TCGATAGAGT TTGAAAGCTT TCTTCTTCAT 1200

TCAATTTTGG GCAAAATACT GCCTCTGCAT TTGTTCATAA CAAAAAGATT AGATTAATAA 1260

GTAGCTTTTG TTGGTGGAAA TTACCAGCTC TATAAGTCAC CCTTGGTGGT TCATGGACCT 1320

CTGATTAGCT TGGGTTTTGC AGTCTCATTG CCACATGTAT ATGTGGAGCC AATGGCCTTT 1380

TGGTGCTCAG CTGTTTACGT CTGACTCCTT GACTTCTTTG GTACAGTGAT GGAGTCAGAT 1440

CTCATTAAGT GTGATTCTCC ATGGATATAA CCAGCCCCAA AAAAANG 1487

(2) INFORMATION FOR SEQ ID NO: 51:

(i) SEQUENCE CHARACTERISTICS:
(A) LENGTH: 1328 base pairs
(B) TYPE: nucleic acid

(C) STRANDEDNESS: double
(D) TOPOLOGY: linear

(xi) SEQUENCE DESCRIPTION: SEQ ID NO: 51:

GGCACGAGCT CGTGCCGAAT TCGGCACGAG AGAAGATTTG AAGAAGCCAG ATCCAGCTTC 60

CCTGCGGGCT GCTTCTTGTG GGGAAGGGAA AAAGAGGAAG GCCTGTAAGA ACTGCACCTG 120

TGGCCTTGCC GAAGAACTGG AAAAAGAGAA GTCAAGGGAA CAGATGAGCT CCCAACCCAA 180

GTCAGCTTGT GGAAACTGCT ACCTGGGCGA TGCCTTCCGC TGTGCCAGCT GCCCCTACCT 240

TGGGATGCCA GCCTTCAAAC CTGGGGAAAA GGTGCTTCTG AGTGATAGCA ATCTTCATGA 300

TGCCTAGGAG GTTCCTGACA TGGGACCCAT CTGCTCCTCC AGCCAACTCC TGTCCCTCAC 360

ATCCCACCAT GGTGGCTCCT CCCACCTCCT CTGGATTTGT TCACTCTGAG ATCTGTTTGC 420

AGAGTGGGTG CTTAGCAGAC AGAGTGAAGC TGGCTGGGGG GCACAGTGGT GTGTAGTGCT 480

GCTGTGTATC AAAAGACCAA GGTATTATGG GACCTGGTTT CAGAATGGGA TGGGTTTCTT 540

CACCTCATGT TAAGAGAAGG GAGTGTGTCC TGAAGAAGCC CTTCTTCTGA TGTTAAAATG 600

CTGACCAGAA CGCTCTTGAG CCCAGGCATC GTTGAGCATT AACACTCTGT GACAGAGCTG 660

CAGACCCCTG CCTTGAGTCT CATCTCAGCA ATGCTGCCAC CCTCTTGTCT TTCAGAGTTG 720

TTAGTTTACT CCATTCTTTG TGACACGAGT CAAGTGGCTC ACAACCTCCT CAGGGCACCA 780

GAGGACTCAC TCACTGGTTG CTGTGATGAT ATCCAGTGTC CCTCTGCCCC CTTCCATCCC 840

CAACCACATT TGACTGTAGC ATTGCATCTG TGTCCTGTTG TCATTTATGT TAACCTTCAG 900

GTATTAAACT TGCTGCATAT CTTGACATAT CTTGAGATTC TGCATGTCTT GTAAAGAGAG 960

GGGATGTGCA TTTGTGTGTG ATGTTGGATA GTCATCCACG CTCAGTTTGG ACCATTGGAG 1020

GAACTTAGTG TCACGCACAA ATGGGGCTAT TCCTACGCTT AGAATAGGGC TTGTCTGCCC 1080

ACTTTAGAAG AGTCCCAGGT TGGTGAGCAT TTAGAGGGAA GCAGGGCAGA ACTCTGAACG 1140

ACAATACGTC TCTCTGAGCA GAGACCCCTT TGTTCTTGTT ATCCACCCAT ATGGACTTGG 1200

AATCAATCTT GCCAAATATT TGGAGAGATT GTGTGGATTT AAGAGACCTG GATTTTTATA 1260

TTTTACCAGT AAATAAAAGT TTTCATTGAT ATCTGTCCTT GAAAAAAAAA AAAAAAAAAA 1320

AAACTCGA 1328

(2) INFORMATION FOR SEQ ID NO: 52:

(i) SEQUENCE CHARACTERISTICS:
(A) LENGTH: 1856 base pairs
(B) TYPE: nucleic acid
(C) STRANDEDNESS: double

(D) TOPOLOGY: linear

(xi) SEQUENCE DESCRIPTION: SEQ ID NO: 52:

GAATTCGGCA CGAGCTCTGC AACATTGCAA ATGAACTTGT AGTCGAGGGT TCCGCTGCCC 60

CCTAGATTAA ATTCCCCGGG CTGAAATTGA GTTGCAGATT TAGAATATCA TATTTTAAAT 120

TGCTGTCTTC AATTAAACCA TTTATGACCA TAACTAATTT TCAGGATGTC GATGCATGCT 180

TTTCCAGGCC TTCCTTCTTT GTACAAAAGT AAATGTCCAT AAAGCGTTTC ACTTATATTC 240

TTCAAACATG ATGCTAATTT AAATTAATTA CTTCCTATGA TATGTTATTA TTCCTATGAT 300

TTTGCCACTG TTATTAGTTC TCTCAAAAAT ACATCTAGGG AAGAGGATTA TTTTAAGTRA 360

TTTGATTATC TTTCTATCTC TTTTATTTAT TTCTCATTTA CTTAAGAAAT TCGTTCCATT 420

GGTTGGCATT GATACAGTAA ATTTGTAAAT GAGGAGACAA TATAAAAAAT CTAAATTACT 480

TGTGCTTAAT GACTGTAGCA GAATSCCTTT TCTCTAAATC AGATTGTCTT TCTTGCAGTT 540

TAGTTTGATA GATTTGCAAG CTATGCTGCT TCCATGAAGT TAGTTGCGCT GGTAGGAACG 600

CAGGCTTCTT TGTCTCTGGT TGTAGCTTGC ATGATCGCCT CATTAGGCAG ACAACGTAGC 660

CGGAGATCAC AAATCAGGCC CTTGGTGTAG TTGCTAGTGT GTGGAGGTGC AGAGAGGTTG 720

GCAGAAACTG ACCTCACTGG GCAAGGGTGG CCATGGACCT GATTCTTTAA TGCACTCTAT 780

GTGTTCAGGA AGCCACAGGC CATATTTGAC TCTGAGAAAG AAAACAAGAG GAAAAACCCC 840

ACAAAGTATA ACAACCCCTT AAGATACATC TATTTTAAAG TGAAATTAAT TTTTCAGTTT 900

ATACCATTGG CCAATTACAA GATAAAAATG TTCAATTTCT TTAAGATCCC TTTGTTGACT 960

TGTCTTTTCA TCTCTTGCTA TTTATATTTG TCACTGTTAG TCAACAAAGT CTTATTTGCT 1020

GAGGAAGGAC TTTGCTGCAC TTACTGTACC ACATCAAACA CTGGGGAGGG TGGTGTTTAA 1080

CTTTTTAAAA AATGTTATTC TGATTATAAC AATAATATTG GCTTTTTTCA TGAAAAGAGC 1140

GCCACCTTGC AAGGTTTAGT GAGATTTATG GAAGTTGAAT ACTTAAGCAG GAATTGCTGC 1200

TAGCTCCAAA AATTTGCGAA GCAAAAGCTA GCCCCAATTG GTTTGGAAGT TTGAAACTGA 1260

TTAACAGATT TGCATTTGAA GTGACTTCAG ACATTAGGTC CAGACATTAG TTAAAAATAG 1320

AAAGAGGAAT AAAGACATCT YTTCTCTCTA GAAAAGATAA CACCRCAATT AATAATCCTT 1380

CCCACTTTCA TTGAGATCAG CTTGTCTGAT AACCTGATAT GAGTGTGATA ATGATAAACA 1440

TGATAATAGT GGTACTTTTG TAATTTTGCT GGTGCATTTA AGAAGATAGT AAAKGATGAG 1500

TTCAYCTTTT CTYCGAACAT YCCTATYCCT AGATGTAGTT TACCTCAAAT TGGGAATTAT 1560

AACTGTCCTA ATTTTTGTTG TGTACCCTGA TGCCCCTTTT GCTTTAATAC CCACAGTGTA 1620

ACAATTAAAT ATCACACTAT GACATATGAT TTAAGTAGGA TATTTAAAAG ATAAATTTTA 1680

GGGGTAAATG TTTACTTCAA AATGACTCCA TATTTCAAAT ATCTGTTTAG ACTGTGAAGG 1740

CCAAATAATT TTTAAGAAAA CATTTGAAGA GTAGTGTGTT TGCATTTGTG AATAATCTTA 1800

CTCACAGCAA GTAAACGTAA TAAAAGCCAA CATTTAAGCC AAAAAAAAAA AAAAAA 1856

(2) INFORMATION FOR SEQ ID NO: 53:

(i) SEQUENCE CHARACTERISTICS:
(A) LENGTH: 1558 base pairs
(B) TYPE: nucleic acid
(C) STRANDEDNESS: double
(D) TOPOLOGY: linear

(xi) SEQUENCE DESCRIPTION: SEQ ID NO: 53:

TGGGTATCCA TTCCTGNAAT TACTTTACTT AGGATAATGG CCTCCAGCTC CGTCCAAGTT 60

GCTGCAAAAG GTATTATTTC GTTCCTTTTT GTGGCTGAGT AGTATTCCAT GGTGTATATA 120

TACCACATTT TCTTTATCCA CTCATTGCTT GATGGGCAGT TAGGTTGGTT CCACATCTTT 180

GCAATTGTGA GTTGTGCTGC TCCAGATATC ATCTTTAACT CCTTTGCCTT CTCCACATAC 240

ATTTCCAAGT CCTGTTCATT CTACCTCCAA AATGTATCTT GTATCCATTC ATCTCTCTCC 300

ATCTTCAATC TATTTCAATG CCCCATCATC TCTTGCATGG AGGAGTGTAA TAATTGGCTA 360

ACTGGCCTGT TCTTACATTT TAAAATCAAA AGATGTGACA GGTGAAATGC CTATTTCAGT 420

GTCCATTGAT GGTTCTGCTT ACACACCACC TGGCTGCCTG GTGTCGCAGT GGCAGAGTTG 480

AGCAGTGTGA AAAAGACTGC TTGGCCCTTT ACAGGGAAAG CAGGTCCACT GTGGCCTGTG 540

AGGACGAGAG CTCTGGGCAG GCTCGGACAC TGGCAGACCC TGGTCCTGGC TGGCCAAGGC 600

AGCAGGGTAT GTGTTTCGGG TCACTCACAG GGCTCAGCAC CACTCCTCAT GGCTTCCTTA 660

CTGTTTCGGC AGAGGCTGAC CCGCGGCTGA TTGAGTCCCT CTCCCAGATG CTGTCCATGG 720

GCTTCTCTGA TGAAGGCGGC TGGCTCACCA GGCTCCTGCA GACCAAGAAC TATGACATCG 780

GAGCGGCTCT GGACACCATC CAGTATTCAA AGCATCCCCC GCCGTTGTGA CCACTTTTGC 840

CCACCTCTTC TGCGTGCCCC TCTTCTGTCT CATAGTTGTG TTAAGCTTGC GTAGAATTGC 900

AGGTCTCTGT ACGGGCCAGT TTCTCTGCCT TCTTCCAGGA TCAGGGGTTA GGGTGCAAGA 960

AGCCATTTAG GGCAGCAAAA CAAGTGACAT GAAGGGAGGG TCCCTGTGTG TGTGTGTGCT 1020

GATGTTTCCT GGGTGCCCTG GCTCCTTGCA GCAGGGCTGG GCCTGCGAGA CCCAAGGCTC 1080

ACTGCAGCGC GCTCCTGACC CCTCCCTGCA GGGGCTACGT TAGCAGCCCA GCACATAGCT 1140

TGCCTAATGG CTTTCACTTT CTCTTTTGTT TTAAATGACT CATAGGTCCC TGACATTTAG 1200

TTGATTATTT TCTGCTACAG ACCTGGTACA CTCTGATTTT AGATAAAGTA AGCCTAGGTG 1260

TTGTCAGCAG GCAGGCTGGG GAGGCCAGTG TTGTGGGCTT CCTGCTGGGA CTGAGAAGGC 1320

TCACGAAGGG CATCCGCAAT GTTGGTTTCA CTGAGAGCTG CCTCCTGGTC TCTTCACCAC 1380

TGTAGTTCTC TCATTTCCAA ACCATCAGCT GCTTTTAAAA TAAGATCTCT TTGTAGCCAT 1440

CCTGTTAAAT TTGTAAACAA TCTAATTAAA TGGCATCAGC ACTTTAACCA AAAAAAAAAA 1500

AAAAAAAAAA AAANAAAAAA AAAAGGGGGC CGCTCTAGAG GTCCAAGTTA NGACGNGG 1558

(2) INFORMATION FOR SEQ ID NO: 54:

(i) SEQUENCE CHARACTERISTICS:
(A) LENGTH: 948 base pairs
(B) TYPE: nucleic acid
(C) STRANDEDNESS: double
(D) TOPOLOGY: linear

(xi) SEQUENCE DESCRIPTION: SEQ ID NO: 54:

TAAAAATCAT GCTCTGTACC ATCCTCACCG TAGTCATCAT CATCGCCGCG CAGACCACGA 60

GAACTACTGG GATCCCTAAA AACGCCCCTG GTCCGGCCCC ACTCTGCGCC CCTCGATCTC 120

CCAGGCTCTT TCTGCAGWCA TACCGCGGAC CCAATGGGCG CCCTGCACAC CCGTTTCTGG 180

GGCCGTCAGA CTTGGATACA TCGTAAACTC CGCCTCCACG GAACGTCTCG CCTKGCGAGC 240

AAGMTCGGAA TCCAGTTCCT CAGGAACCCC TCCAAAACCC ACACCCCCAG GGACGCCGCT 300

TTCCGGGATC CCGGSCAAAC GCCGGACCCT CAGTCGCTCC AGGCCCCCTC ACCCTCAAAG 360

TGTAGCGCCC CCAACCGAGC AACCTCGGTT TGGTCCCTAA AACCCCGCCT CCTCTATAAG 420

CACCGCCCCA GCTCTGACAA AACCCCGCCT CCAGGTCGGC AGGCTCCGCT TCTTTTCTTC 480

TCCGCGGGGT GATTCAGTCC AGTGATTGGG TTTGTGGCTC CAGGCCTCGC CCACAGACGG 540

ACAGACCCCT CCCTTTCTTC CGGCAAAAGG ACCGAGCCCT GGGGTAGTAA GGSCCCCACA 600

CTCCTGTTTT TTGCAAGTAC ATTTTTGTCC YTCCTCCACC CAGGTATCTG CCTATTTTCT 660

TGCTAATCCC AGAACCTTTC CTTTTGCTTT TTTTAAGGAC ATTTGGGAAG TTCCTGGTGT 720

AGGACCCTTC TCCCTGGGAT AAGAAACCTG CCTGTAAACG CTCTGTAAAT ACTCCCTTCC 780

ACCCATCCCA GCCCCTGGGC AGCCGGGCAG AAGGGAATCC AGGCTATGGA CCTCCCAAGT 840

CCCCGCTCCC CGCTCCCCTC GGCGGCCCCG CCTTGTTCTG ATCTGTGTGT GAGTGTGTGT 900

GAACTTCTGA AAGACAATAT TAAAGAGACT TAGTTGAAAA AAAAAAAA 948

(2) INFORMATION FOR SEQ ID NO: 55:

(i) SEQUENCE CHARACTERISTICS:
(A) LENGTH: 990 base pairs
(B) TYPE: nucleic acid
(C) STRANDEDNESS: double
(D) TOPOLOGY: linear

(xi) SEQUENCE DESCRIPTION: SEQ ID NO: 55:

GGGGAACTGC AGTGACAGCA GGAGTAAGAG TGGGAGGCAG GACAGAGCTG GGACACAGGT 60

ATGGAGAGGG GGTTCAGCGA GCCTAGAGAG GGCAGACTAT CAGGGTGCCG GCGGTGAGAA 120

TCCAGGGAGA GGAGCGGAAA CAGAAGAGGG GCAGAAGACC GGGGCACTTG TGGGTTGCAG 180

AGCCCCTCAG CCATGTTGGG AGCCAAGCCA CACTGGCTAC CAGGTCCCCT ACACAGTCCC 240

GGGCTGCCCT TGGTTCTGGT GCTTCTGGCC CTGGGGGCCG GGTGGGCCCA GGAGGGGTCA 300

GAGCCCGTCC TGCTGGAGGG GGAGTGCCTG GTGGTCTGTG AGCCTGGCCG AGCTGCTGCA 360

GGGGGGCCCG GGGGAGCAGC CCTGGGAGAG GCACCCCCTG GGCGAGTGGC ATTTGYTGCG 420

GTCCGAAGCC ACCACCATGA GCCAGCAGGG GAAACCGGCA ATGGCACCAG TGGGGCCATC 480

TACTTCGACC AGGTCCTGGT GAACGAGGGC GGTGGCTTTG ACCGGGCCTC TGGCTCCTTC 540

GTAGCCCCTG TCCGGGGTGT CTACAGCTTC CGGTTCCATG TGGTGAAGGT GTACAACCGC 600

CAAACTGTCC AGGTGAGCCT GATGCTGAAC ACGTGGCCTG TCATCTCAGC CTTTGCCAAT 660

GATCCTGACG TGACCCGGGA GGCAGCCACC AGCTCTGTGC TACTGCCCTT GGACCCTGGG 720

GACCGAGTGT CTCTGCGCCT GCGTCGGGGG NAATCTACTG GGTGGTTGGA AATACTCAAG 780

TTTCTCTGGC TTCCTCATCT TCCCTCTCTG AAGGACCCAA GTCTTTCAAG CACAAGAATC 840

CAGCCCCTGA CAACTTTCTT CTGCCCTCTC TTGCCCCANA AACAGCANAA GCAGGANANA 900

NACTCCCTCT GGCTCCTATC CCACCTCTTT GCATGGGAAC CTGTGCCAAA CACCCAAGTT 960

TAAGAAAAAA ATAAAACTGT GGCATCTCCA 990

(2) INFORMATION FOR SEQ ID NO: 56:

(i) SEQUENCE CHARACTERISTICS:
(A) LENGTH: 1603 base pairs
(B) TYPE: nucleic acid
(C) STRANDEDNESS: double
(D) TOPOLOGY: linear

(xi) SEQUENCE DESCRIPTION: SEQ ID NO: 56:

GGTCGACCCA CGCGTCCGGC CCGCCGGCTC CGGAGCGGCT CTGCCTTCCC GAGCGCGGGA 60

CCGCGCCCTG GGGGAGGAGG GCGAACGACG CGGCGATGGC TCCGCGGGCA CTCCCGGGGT 120

CCGCCGTCCT AGCCGCTGCT GTCTTCGTGG GAGGCGCCGT GAGTTCGCCG CTGGTGGCTC 180

CGGACAATGG GAGCAGCCGC ACATTGCACT CCAGAACAGA GACGACCCCG TCGCCCAGCA 240

ACGATACTGG GAATGGACAC CCAGAATATA TTGCATACGC GCTTGTCCCT GTGTTCTTTA 300

TCATGGGTCT CTTTGGCGTC CTCATTTNGC CAMCTNGCTT NAAGAAGAAA GGCTATCGTT 360

GTACAACAGA AGCAGAGCAA GATATCGAAG AAGAAAAAGG TTGAAAAGWT AGRATTGAAT 420

GACAGTGTGA ATGAAAACAG TGACACTGTT GGGCAAATCG TCCACTACAT CATGAAAAAT 480

GAAGCGAATG CTGATGTYTT AAAGGCGATG GTAGCAGATA ACAGCCTGTA TGATCCTGAA 540

AGCCCCGTGA CCCCCAGCAC ACCAGGGAGC CCGCCAGTGA GTCCTGGGCT TTGTCACCAG 600

GGGGGACGCC AGGGAAGCAC GTCTGTGGCC ATCATCTGCA TACGGTGGGC GGTGTWGTCG 660

AGAGGGATGT GTGTCATCGG TGTAGGCACA AGCGGTGGCA CTTTATAAAG CCCACTAACA 720

AGTCCAGAGA GAGCAGACCA CGGCGCCAAG GCGAGGTCAC GGTCCTTTCT GTTGGCAGAT 780

TTAGAGTNAC AAAAGTGGAG CACAAGTCAA ACCAGAAGGA ACGGAGAAGC CTGATGTCTG 840

TTAGTGGGGC TGAAACCGTC AATGGGGAGG TGCCGGCAAC ACCTGTGAAG AGAGAACGCA 900

GTGGCACAGA GTAGCAGGTG AGCCGTGGTT TTGGTGACAT TGGGGGCAGA GTGGTGCAGG 960

GTGAGGAGAA GGTACTTGGA GCCTCCCAGG TGCTGTGGCA GCATAGGAAT GGTATTTGAC 1020

AGGGAAGTGG GAGAGCTTTC CTTGACCCAG GAAGACTGAG GGGGACTGAA CATGATTACT 1080

TGTCTGCCTA GAGCTTCTTG TAAAGAAGTC ACAAACTTAG TGCCTCCAGG GGCTTGGCTG 1140

TGTGATAATG AGGATAGAGG ATTACTTGTG AGGCAATGTG GCATGGTGGG GATTGTGGCA 1200

AACTAGAATT CACATCACCC ACCATATAGG GCTTGCATTA CCACGAGGCA GAAAGCACCT 1260

AGTGTTGCTG CATCTTCTTA CGCAAAAAAG ACAAAATCCA GACTTCTAAA ATGTAAAATC 1320

ACTGATTTTC GATATTGGCA GCTTACTTTT TTTTTTTAAA CAACCATGCA GGCCAAATGA 1380

CTTGTAATCT TGTCACCATT TTTAGGTAAA CTGTGACTTG AAAAAGTCTG GAGCAAACAA 1440

ACCAATGCTT TTTCCTTTTA TTCTGTTGGR AACCAGTTTT CTTTGTGTCA CAGTTYTGAA 1500

ACCTCAATAC GAATATTTCT CTTCCCACCA AATATTTTGA GGCAATTGAA AAGCCACAGT 1560

GATTTATTTC TTGATTTGGC AATTTTAATT TTGCAAGACA ATT 1603

(2) INFORMATION FOR SEQ ID NO: 57:

(i) SEQUENCE CHARACTERISTICS:
(A) LENGTH: 1052 base pairs
(B) TYPE: nucleic acid
(C) STRANDEDNESS: double
(D) TOPOLOGY: linear

(xi) SEQUENCE DESCRIPTION: SEQ ID NO: 57:

TACAGCTCAG GATGCCTGTA ACATTGTCAT CTCTGGGCTT CTGGGTCCTG CTTAGCCTGC 60

TTTTTCCCTG GAGGACTGAC CAGGGATGCG GCCCAGCAAC ATGTTACTAA ATCATACTCT 120

CCTCCCTACC TTTCCCAGAC CTCTCACTCC TGCCTGGTGT TCCAACCCGT TCTGTGGCCA 180

GAGTATACAT TTTGGAACCT CTTCGAGGCC ATCCTGCAGT TCCAGATGAA CCATAGCGTG 240

CTTCAGCAGN AAGGCCCGAG ACATGTATGC AGAGGAGCGG AAGAGGCAGC AGCTGGAGAG 300

GGACCAGGCT ACAGTGACAG AGCAGCTGCT GCGAGAGGGG CTCCAAGCCA GTGGGGACGC 360

CCAGCTCCGA AGGACACGCT TGCACAAACT CTCGGCCAGA CGGGAAGAGC GAGTCCAAGG 420

CTTCCTGCAG GCCTTGGAAC TCAAGCGAGC TGACTGGCTG GCCCGTCTGG GCACTGCATC 480

AGCCTGAATG AGGCTGGCCA CCTGCCACTT TGCCCTGCCC TCTGCCTCCA GGGCTCCMCT 540

MYCCTTCCTT TTCTTGGTGA AAGGCACCTC CTTTCCTGAT AATGAATGGT GTTCCCTTTG 600

CTTGGCTGGG GAGCCCCCCA GGCCAGGTTT GCTGGCCATA GATACCTTTG GGCTGCCTGR 660

GACAGGCTCC TGAGGAGGAT TGAGGGTGAA AGTCTCCCAC GAGTACACTA AACCTAGGTC 720

TGGTCACCAA TAGGGTTTGG AGAGCAAAGG GCCACAACTC ATCAGCTGCC TGTCTCTTAG 780

ATGCACTTTC TTTTTCCACC AGCACATCCT TCAACACACA GAATTTCAGG GAAGAGTTCT 840

CCCCAAAACC CTAGCTCTTT ACCCTTCCAT TTTAGCCTTC CACCCAGCTT CCACAAAAGA 900

TTTGGCTCTA CCTTGGATCT GCTAGTAAAT AACTAATAGG CAGGCAGTTA TTTGGGTAAG 960

GAAAAAAGGG GTGGGAGAGA CAGAAAATTT GCCCACTGCT GCTCCTCCCC TTGGSTYTCC 1020

ACCTGGGATT TGCTATTGAA TCTCTACCCT NN 1052

(2) INFORMATION FOR SEQ ID NO: 58:

(i) SEQUENCE CHARACTERISTICS:
(A) LENGTH: 814 base pairs
(B) TYPE: nucleic acid
(C) STRANDEDNESS: double
(D) TOPOLOGY: linear

(xi) SEQUENCE DESCRIPTION: SEQ ID NO: 58:

ACNCGNTGGC GGCCGCTCTA GAACTAGGGG ANCCCCCGGG CTGCAGGAAT TCGGCACGAG 60

CATAGACTTT TAAACTGGTA CGGTTCTTAG AGATGGTCCT TGGCCTTCTG TTGTTGTTGT 120

KGTTTTTTTC TTTTTCTTCT TCTCCTTCTC CTTCTTCTTC TCTTCTCCTT CTTTCTTCTT 180

TTTTTTTTCA GAGTCTTGCT CTGTCACCAA GACTGGAGTG AAGTGATGTG ATCTCGGCTT 240

ACTGCAACCT GGGAGGCAGA GGTTGCAGTG AGTCGAGATG GTGCCATTGC TCTCGTTTGG 300

GCAACAAGAG TGAAACTCTT GTCTCAAAAA AAAAAAAAAA ATGAGGTTTA AGACAGTTTT 360

GTCATTACTG GTGGGATCTG GTCACACAAG ATAGCATTAA ACGTGACATG GCACATAAAA 420

TTGGTTAAAA AATTTTGTTT TTTAATTACG TAATGTAAAA GCCCAACAAA CACTTTATGC 480

AAGATTGGAA TGTATCTTCA AATTCAGATT TAATAAACAT GTAAAGATCC TCTGTATATA 540

AAAGTTGTAT TTAATCCCTT GTGCCCCAAG AATGCTATAA AAGATCCCAA GAATGTTATC 600

TATGAAAAGA TAGCAATAGG GAATGGTGAA CAAATAATTT AATTTGCCAA TTCTAAAAAA 660

CATGGACTTA AACCCCATGA AAACTTGGTT CCATAGTTTT AACTGTTTTA TGGTTCCAAT 720

ACAAAACCAG AGTGGTTTAC ATTCCACAAT NACCAAATTT GCATCCAATN TTGGGGTAAT 780

TTTNGGTATT TGCCATGGGA TACTATTCAT TTTT 814

(2) INFORMATION FOR SEQ ID NO: 59:

(i) SEQUENCE CHARACTERISTICS:
(A) LENGTH: 1215 base pairs
(B) TYPE: nucleic acid
(C) STRANDEDNESS: double
(D) TOPOLOGY: linear

(xi) SEQUENCE DESCRIPTION: SEQ ID NO: 59:

AGAGGAAGTC TTTTGCCAAG CCTGTTCTCT GGACTAACGC CATCCAGGCT GGGAGGGGAA 60

GAGTGCTCTG CTACACTCGT CCCCCTCCTG CCTCATCTTC CTTCTCAGCC TTGGTTCCTG 120

ATGGGAACAG AATGGAGGGC CTGAGAACAT ACTTTCTAAA TGCCTTTGAC CCAGGAACCG 180

ATTATCTATA TTTGTTCCCA TTTTCCTTCA CCGTGACATT CCAGCATTGT CTGACTGTGA 240

GGTGGGCCTT TGAGAGCCTC CAGGTTCCTC AAAACAGGCC TGAGCGATGG GCATCACACC 300

CTCTGCCTAC CCACRTGCCT GCTTACCTGC CAGATAACCA AGTGNAGATG TCTGCGAGTG 360

GCTAGTTTTC ACATTCTTAC TAGTGTTTGG YTCACCTTTG GGCAAAGGCC CCCTCTAGGC 420

CTTGCCCCAC CTCCATCAAA CGCAGACACT GTAGTCAGAC CTCAGYAATA TAGGAGGCAA 480

TAATCTTTTA ACAGTGTTTT GCAAACAAAC AAAAAGAGAA AAATCCCAGC CAGGGGAACT 540

CGCCACCTGC CCACGCTAGT TCCATCCACG CTCAAGACCC GCCCTTAGAC CAGGCAGGCA 600

AAGGCCCCCA TCACACTCGG CCACTAGTGG GGTCCTGAGG CCAAGAAAGA AACCAGACCC 660

TGTATGACAA GTTGGGKTCT TTCCAGAACA CGACAGAAAC AGGGGGGGCC CCTTGTTAAT 720

GCCACTCCAT ACTCCAGAAG CATTATTCCT TATTTGGGAC AGCCAAGGGC AGATTCACAG 780

GTTATTGTAG GAATAAAGAC TAGTTTACAA AGGARAAAGA GSCCCTGGAC TTCCCMAGGA 840

AAGGTCAGGT TAGGGCTCCT GTACCCATTC TGTTCCACCA CTGTTTGATC TCTCTGGCCT 900

CCCACCAGGA ATGCCGTTTC CTTTTTATGG ATCTGTTGGG AACCAGAGAG AATCAACAGA 960

TCAATGACAT AGGATCCGAA GTGCAATGAT AGTCACTTCT AGTTTGGCAT TTCACAAACT 1020

CTGNACAGCA AGGTATTGGT AGGTTACTCA ATTTCAAAAG GGCCCCATGG CCAAATATGT 1080

TTAGGAACCG CTGTTTGNAT TTCTTTTTTT GGAGACGCAT TGTATATAAT ATATGTCAAA 1140

GGCTTTCGGA ATTCCTGCAG GAAAGAAATC AGCTTTGTTA AATCCNAAAA AAAAAAAAAA 1200

AAAAAAATAG ACTCG 1215

(2) INFORMATION FOR SEQ ID NO: 60:

(i) SEQUENCE CHARACTERISTICS:
(A) LENGTH: 478 base pairs
(B) TYPE: nucleic acid
(C) STRANDEDNESS: double
(D) TOPOLOGY: linear

(xi) SEQUENCE DESCRIPTION: SEQ ID NO: 60:

ATTTCTTATG ACATGGGGGT TTGAATTGGT TGGCAAATGT TTAATTTTAA TATCCATAAT 60

CAGTGAGGTC CTGCTGGCTG TAATCATTAA TTGTGAAATC TAAGGAGCTT AGTTCATGGC 120

TCTAGAATTT CACAGAAAAR TGYGMTATGA TACGAGCATT AAGTTTATTT CTTCTGATCT 180

TTGATGCAGC TTTGTTCAGT TTATCTGTTT TTGTATTTAT TGGTCATCTA CTTCCCATGC 240

CAAAAGGGAC TGGTCTACAT AGCTGCGCTA AACACCTGAT CAAATCACTA AAAGAAAATG 300

TGTTACCTCT AATGAATTAT CCTGATTGTA AGTTAAAAAT CAATATTTCC CCGTAGTGAG 360

GTTTGCTTTT TAAAAAGAAK KCTTAAAAAA AAAAAAAAAA AAACGAGTTN AAGAAAAGGA 420

AGCAAGCTCA GGTAAGGTGC ACACATTGGG CTAAGGAAGC TAGAGCCTGT GGAGANGC 478

(2) INFORMATION FOR SEQ ID NO: 61:

(i) SEQUENCE CHARACTERISTICS:
(A) LENGTH: 618 base pairs
(B) TYPE: nucleic acid
(C) STRANDEDNESS: double
(D) TOPOLOGY: linear

(xi) SEQUENCE DESCRIPTION: SEQ ID NO: 61:

TATGACCTTG ATAACCCCAA GTTNGAAATT AACCTTCANT AAAGGGAACA AAAGCTGGAG 60

TTCGCGCGCT TGCAGTTCGA CACTAGTGGA TCCCAAAGAA TTCGGCACGA GTCATAATGA 120

GCTACTAGGT AAGCCTTCTG GGACTTTCAG ATATTTTGGG GAAGATTGAT TTTTGTTCTT 180

ACATGCTGTG GACCCTTGGC CATCAAATGG TATGGGGAAG CTCATCCGTC TGTCTGTGAT 240

GGTCATGTCA GTCAGGCGTC TTTTTAGTAT TTACTGGGTG CTCAGTACTG TGCCAGATGC 300

TGTCGGGAGC CGTGGTGGTA TGGAGGAGGA GTGCTCCAGA GGACTCTGCT GTGTGGCAGG 360

CCAGCATAAA CAAGCCAAGG GGAAAAGGCA GGCATGGAAT AAAGGGGGAG AATACCAGTG 420

TGTGACTTAC TGCTGACTGT GTGGATTAGC CTATCAGCAG TAATCAAGCA GGGCGGAGGG 480

CATTATCTTT GAGCCAGAAG AGTGAGCACT GGSCCGAGGG TGGAGCATCA AGAGGGGGTG 540

TAGGACCNCA AGGCTTCTTN CNGGGGAGAC AACGTCAATA AGCNGTCAGT AGTCACCGAC 600

AGTTTTGGGA AGCAAGGG 618

(2) INFORMATION FOR SEQ ID NO: 62:

(i) SEQUENCE CHARACTERISTICS:
(A) LENGTH: 751 base pairs
(B) TYPE: nucleic acid
(C) STRANDEDNESS: double
(D) TOPOLOGY: linear

(xi) SEQUENCE DESCRIPTION: SEQ ID NO: 62:

TCGACCCACG CGTCCGAGGA GCTGGACTTC TGAGACAGCC ATTCTCCTTG CATAGCACTG 60

TCTGCTGCTA CAGCTCATAG AAGTCAACAA TTTTCTTCAA CACTGGTAGG CAGCCTCTAA 120

ATGGCCCTGA TCACCCTCAC CTCCTGCCAT TCACACCNNT GTAAAATTCC ACCCCTGGAC 180

CTAGTGACTC ACTTCTAACA ANGAGAATAC AGCAAAAGTA ACATCGCTTC TGAGGTGAGG 240

CTACAAGGAG ACTACGATGC CTGCCTTGGT CACCCTTCTC CTGCTCTTTC CATTGCTCCC 300

TCTGATGGAA GCCAGTTGCC ATGTGATGAG GTGCCCTATG GAGAGGCCCA CGTGACAAGG 360

TATTGTAAAA AGCCTCTGAC CAATAGCCAT CTAGAAACGG AGGCCCAGTC CAGCAGCCTC 420

TGAGATGAAT CCTGCCAACC TGAGCTTGGA GACAGATTCT CTCCCTATCC TGCCTTGGGA 480

TGATCACAGC CACCACCAAC ACCTTCACTG CCTGGTGAGA GGCCAAGCCA GTGAACCCAA 540

GGTAAACTGG ACAGAATCCT GACCCACAGA AACTGAGATA ATGTTTGTTA TTTTAAGCTG 600

CTCAGTTTGT TACAGAGCAA TAGATAACTA ACTCAAACAC CATAAAATTC TAATATTTTA 660

TTCTATCACA CAAACCAGGT AATACCAAGT AAATGCCATT ACTATACACA TATTTTTGTA 720

ACACAATTAC ATGTGATTTT TTAAGAAGGC T 751

(2) INFORMATION FOR SEQ ID NO: 63:

(i) SEQUENCE CHARACTERISTICS:
(A) LENGTH: 780 base pairs
(B) TYPE: nucleic acid
(C) STRANDEDNESS: double
(D) TOPOLOGY: linear

(xi) SEQUENCE DESCRIPTION: SEQ ID NO: 63:

CNGNCAGTCA CNGTCCCCGA TTCCCGGGTC GACCCACGCG TCCGGGTTGG CAACTCCTGA 60

GGCCTGCATG GGTGACTTCA CATTTTCCTA CCTCTCCTTC TAATCTCTTC TAGAGCACCT 120

GCTATCCCCA ACTTCTAGAC CTGCTCCAAA CTAGTGACTA GGATAGAATT TGATCCCCTA 180

ACTCACTGTC TGCGGTGCTC ATTGCTGCTA ACAGCATTGC CTGTGCTCTC CTCTCAGGGG 240

CAGCATGCTA ACGGGGCGAC GTCCTAATCC AACTGGGAGA AGCCTCAGTG GTGGAATTCC 300

AGGCACTGTG ACTGTCAAGC TGGCAAGGGC CAGGATTGGG GGAATGGAGC TGGGGCTTAG 360

CTGGGAGGTG GTCTGAAGCA GACAGGGAAT GGGAGAGGAG GATGGGAAGT AGACAGTGGC 420

TGGTATGGCT CTGAGGCTCC CTGGGGCCTG CTCAAGCTCC TCCTGCTCCT TGCTGTTTTC 480

TGATGATTTG GGGGCTTGGG AGTCCCTTTG TCCTCATCTG AGACTGAAAT GTGGGGATCC 540

AGGATGGCCT TCCTTCCTCT TACCCTTCCT CCCTCAGCCT GCAACCTCTA TCCTGGAACC 600

TGTCCTCCCT TTCTCCCCAA CTATGCATCT GTTGTCTGCT CCTCTGCAAA GGCCAGCCAG 660

CTTGGGAGCA GCAGAGAAAT AAACAGCATT TCTGATGCCA AAAAAAAAAA AAAAAAAACC 720

GCGGCCGAAA GCTTATTNCC CTTTAAGTAA GGGGTTAATT TTTAGCTTGG GCACTNGGCC 780

(2) INFORMATION FOR SEQ ID NO: 64:

(i) SEQUENCE CHARACTERISTICS:
(A) LENGTH: 588 base pairs
(B) TYPE: nucleic acid
(C) STRANDEDNESS: double
(D) TOPOLOGY: linear

(xi) SEQUENCE DESCRIPTION: SEQ ID NO: 64:

TTCCGAATTA ATCGACTCAC TATAGGAAWT GCCGTCGCCA TGACCCGCGG TAACCAGCGT 60

GAGCTCGCCC GCCAGAAGAA TATGAAAAAG CAGAGCGACT CGGTTAAGGG AAAGCGCCGA 120

GATGATGGGC TTTCTGCTGC CGCCCGCAAG CAGAGGGACT CGGAGATCAT GCAGCAGAAG 180

CAGAAAAAGG CAAACGAGAA GAAGGAGGAA CCCAAGTAGC TTTGTGGCTT CGTGTCCAAC 240

CCTCTTGCCC TTCGCCTGTG TGCCTGGAGC CAGTCCCACC ACGCTCGCGT TTCCTCCTGT 300

AGTGCTCACA GGTCCCAGCA CCGATGGCAT TCCCTTTGCC CTGAGTCTGC AGCGGGTCCC 360

TTTTGTGCTT CCTTCCCCTC AGGTAGCCTC TCTCCCCCTG GGCCACTCCC GGGGGTGAGG 420

GGGTTACCCC TTCCCAGTGT TTTTTATTCC TGTGGGGCTC ACCCCAAAGT ATTAAAAGTA 480

GCTTTGTAAT TCCAAAAAAA AAAAAAAAAA AAAAAAAAAA AAAAAAAAAA AAAAAAAAAA 540

AAAAAAAAAA AAAAAAAAAA AAAANNCGGG GGGGGGCCCC CCCCCCCC 588

(2) INFORMATION FOR SEQ ID NO: 65:

(i) SEQUENCE CHARACTERISTICS:
(A) LENGTH: 774 base pairs
(B) TYPE: nucleic acid
(C) STRANDEDNESS: double
(D) TOPOLOGY: linear

(xi) SEQUENCE DESCRIPTION: SEQ ID NO: 65:

TTTAAAGATG AAGAAATGAC AAGGGAGGGA GATGAGATGG AAAGGTGTTT GGAAGAGATA 60

AGGGGTCTRA GAAAGAAATT TAGGGCTCTG CATTCTAACC ATAGGCATTC TCGGGACCGT 120

CCTTATCCCA TTTAATTAAT TTCTCTGACA ATTCAATTAT TTTCTGTTAT TAATGTTGCC 180

ACTGCTTTCT GTTTGTCTGC ACTTTCTTGA TAAATATTTG CTATCGTTTT ACTCCAGTCA 240

TTCGATGTTG CTGAGATTTA CATATGACTC TTGTCAACAT CTCATCTTTT GACCCAATCT 300

TATTCATTTA ATAAGAGGTC TCATTCATTT GCATGGAAAA ATGCTCATTG TATATTGCAA 360

AGTGAAAATA ACGAGTTGCA AAACAGTGTA TACATATATG TGTGTATATA TGTACACTTT 420

ATTTGTACAT TTCTATGTGA CATAATGCAA AGGAAAGTGT CTGATTTTAT TATACACCAA 480

AGGTTAACAG TGAATCTCTG TGTGATCTCT TTTTTTTTCT TTTTGCCTAT CTGCATCTTC 540

TCACTTGCCA AAAAATGAAT ATATGTTTAT GTGTGTATAT TACTTGTGTC ACAAAAAACC 600

CTAAAGTAGA CAGTAAAAGA ACTTGTCAAT CGCCTTTGGA AGGCAATGAA ACACTTAATA 660

AACTCTCAAT AACAGAAGCG TAAAAATGAA ATGTAAACCT CCAATTACCT CTGGATCTCT 720

TAGCCAGAGT AATAAACTGG TAATTATTAC AGATAAAAAA AAAAAAAAAA AANA 774

(2) INFORMATION FOR SEQ ID NO: 66:

(i) SEQUENCE CHARACTERISTICS:
(A) LENGTH: 1866 base pairs
(B) TYPE: nucleic acid
(C) STRANDEDNESS: double
(D) TOPOLOGY: linear

(xi) SEQUENCE DESCRIPTION: SEQ ID NO: 66:

ACCCACGCGT CCGGTCCTCT TCTTCAGCAC ATGCCAAAGC TGTTCCTCAC GGCCTGTGAG 60

ACAAGAGCAT CTTGGATGTA GGACAATGGA AGAGTTAGAT GCCTTATTGG AGGAACTGGA 120

ACGCTCCACC CTTCAGGACA GTGATGAATA TTCCAACCCA GCTCCTCTTC CCCTGGATCA 180

GCATTCCAGA AAGGAGACTA ACCTTGATGA GACTTCGGAG ATCCTTTCTA TTCAGGATAA 240

CACAAGTCCC TTGCCGGCGC ANTCGTGTAT ACTACCAATA TCCAGGAGCT CAATGTCTAC 300

AGTGAAGCCC AAGAGCCAAA GGAATCACCA CCACCTTCTA AAACGTCAGC AGCTGCTCAG 360

TTGGATGAGC TCATGGCTCA CCTGACTGAG ATGCAGGCCA AGGTTGCAGT GAGAGCAGAT 420

GCTGGCAAGA AGCACTTACC AGACAAGCAG GATCACAAGG CCTCCCTGGA CTCAATGCTT 480

GGGGGTCTSG AGCAGGAATT GCAGGACCTT GGCATTGCCA CAGTGCCCAA GGGCCATTGT 540

GCATCCTGCC AGAAACCGAT TGCTGGGAAG GTGATCCATG CTCTAGGGCA ATCATGGCAT 600

CCTGAGCATT TTGTCTGTAC TCATTGCAAA GAAGAGATTG GCTCCAGTCC CTTCTTTGAG 660

CGGAGTGGCT TGGNCTACTG CCCCAACGAC TACCACCAAC TTTTTTCTCC ACGCTGTGCT 720

TACTGCGCTG CTCCCATCCT GGATAAAGTG CTGACAGCAA TGAACCAGAC CTGGCACCCA 780

GAGCACTTCT TCTGCTCTCA CTGCGGAGAG GTGTTTGGTG CAGAAGGCTT TCATGAGAAG 840

GACAAGAAGC CATATTGCCG AAAGGATTTC TTAGCCATGT TCTCACCCAA GTGTGGTGGC 900

TGCAATCGCC CAGTGTTGGA AAACTACCTT TCAGCCATGG ACACTGTCTG GCACCCAGAG 960

TGCTTTGTTT GTGGGGACTG CTTCACCAGT TTTTCTACTG GCTCCTTCTT TGAACTGGAT 1020

GGACGTCCAT TCTGTGAGCT CCATTACCAT CACCGCCGGG GAACGCTCTG CCATGGGTGT 1080

GGGCAGCCCA TCACTGGCCG TTGTATCAGT GCCATGGGGT ACAAGTTCCA TCCTGAGCAC 1140

TTTGTGTGTG CTTTCTGCCT GACACAGTTG TCGAAGGGCA TTTTCAGGGA GCAGAATGAC 1200

AAGACCTATT GTCAACCTTG CTTCAATAAG CTCTTCCCAC TGTAATGCCA ACTGATCCAT 1260

AGCCTCTTCA GATTCCTTAT AAAATTTAAA CCAAGAGAGG AGAGGAAAGG GTAAATTTTC 1320

TGTTACTGAC CTTCTGCTTA ATAGTCTTAT AGAAAAAGGA AAGGTGATGA GCAAATAAAG 1380

GAACTTCTAG ACTTTACATG ACTAGGCTGA TAATCTTATT TTTTAGGCTT CTATACAGTT 1440

AATTCTATAA ATTCTCTTTC TCCCTCTCTT CTCCAATCAA GCACTTGGAG TTAGATCTAG 1500

GTCCTTCTAT CTCGTCCCTC TACAGATGTA TTTTCCACTT GCATAATTCA TGCCAACACT 1560

GGTTTTCTTA GGTTTCTCCA TTTTCACCTC TAGTGATGGC CCTACTCATA TCTTCTCTAA 1620

TTTGGTCCTG ATACTTGTTT CTTTTCACGT TTTCCCATTT CCCTGTGGCT CACTGTCTTA 1680

CAATCACTGC TGTGGAATCA TGATACCACT TTTAGCTCTT TGCATCTTCC TTCAGTGTAT 1740

TTTTGTTTTT CAAGAGGAAG TAGATTTTAA CTGGACAACT TTGAGTACTG ACATCATTGA 1800

TAAATAAACT GGCTTGTGGT TTCAATAAAA AAAAAAAAAA AAAAAAAAAA AAAAAAAAAA 1860

AAAAAA 1866

(2) INFORMATION FOR SEQ ID NO: 67:

(i) SEQUENCE CHARACTERISTICS:
(A) LENGTH: 1152 base pairs
(B) TYPE: nucleic acid
(C) STRANDEDNESS: double
(D) TOPOLOGY: linear

(xi) SEQUENCE DESCRIPTION: SEQ ID NO: 67:

CTCAAGGATG TAAAGGCTCT GCAGATTTCG GGAGGCCTGT CTCCCAGCAC CTGATGGGAC 60

ACTTTTTGCC CCACTGTAAA TTCTGGGTGT ATCCTCCACT GTATGCTGTC ACCCCAAGGG 120

CAAGCACTGC ATCTGCTTAG TGAAGGATTT ATTGTTCGGA AGATACATTT TCCCCTTKAG 180

CAGAGAGTGG CGTATCCTGG CAGTCTTCGG TGAGCCAGTT GTACCAGGAT TATGAAATGC 240

AGATGTTTAC TGTGTCATTG TTGCTGTCAT TGCTACTGAG GAGTACTGAC CAGAATCATC 300

TGCAACTYTT AGTTGGCAGA GAGGACCACT ATGGCGGGTA GCTCTTTTCT TTCCTGCCAT 360

TGTGGGGATG ATTCCAGGCC AAAGATGATG GARAAGTATG GAAATCATCT GAAAGGTTGA 420

AGCTTGGCAC GTGAAGCCAT TCATGACTTT GTAAGGCAGT TTTGCTGAAG GCCAGTTCTG 480

CCCTGGGAGG GACGGAGGTG AATCCTCCTG AGTACCTGTG GTTTTCTTAC TTCCTGCTGA 540

ATTTACCTAA GTGCCTGTTG TTTGCTTGCT GTGGAGGCTT TCTGGTATTT CATTTCAGGT 600

GCAGATGCCT TCACTTTCCC ACCRAAAAAA CCCCMACCAA ACCTAAGACC TTACTGCAAC 660

TAAGTYTNCC AAGTACTTTT TAACCCAATG GGATGAACAG CCTGTGGTCT GCTCAGATCA 720

CCCTGAGTGC GTGTGAGAAG GCMTNGGCTT TGCCAGGAAA TCCAGGAAGG CAGGGCCGGG 780

CTGTGTTGGA AGCTGGCTTA GCTGGTGGGG CAGCCTTATT TCAATTAAAA GGGCATTGAC 840

TGGGAGCAGC AGTCCTGGAG TTTGTTGCAT TTCCTATTGC CCTCAAAATG AGAAACCAGG 900

AAAATAGCAG ATTGGAGCCT TCGAGAAGGC AGTAAATGGC TGTTTTTATT GACAAAAGGA 960

AAACATTTTA CTGCCATCTC ACTGATGGCA TCTCACTGAC TTAAAATGAA GGCANGTTGT 1020

AGTAAAAAAA AAAGTCTACA TTTTTCCACC GCCACGTTCT TATATCCTGT TTGTCAGCCA 1080

CTGCTCANAA GGGCATGTTG TCTTGCGGAN TANAGGCGCT CTCCTTCCCT CGTTTTCCCT 1140

ATAGGTTGGG TG 1152

(2) INFORMATION FOR SEQ ID NO: 68:

(i) SEQUENCE CHARACTERISTICS:
(A) LENGTH: 2483 base pairs
(B) TYPE: nucleic acid
(C) STRANDEDNESS: double
(D) TOPOLOGY: linear

(xi) SEQUENCE DESCRIPTION: SEQ ID NO: 68:

AGCAGGCGGT GCGCTGGGGG CGGGAGCAGC GCGKAGCCCG GCTCGGCCAC ACCGATCGCC 60

CGCCGCCATG GGCTCCTCGC AAAGCGTCGA GATCCCGGGC GGGGGCACCG AGGGCTACCA 120

CGTTCTGCGG GTACAAGAAA ATTCCCCAGG ACACAGAGCT GGTTTGGAGC CTTTCTTTGA 180

TTTTATTGTT TCTATTAATG GTTCAAGATT AAATAAAGAC AATGACACTC TTAAGGATCT 240

GCTGAAASCA AACGTTGAAA AGCCTGTAAA GATGCTTATC TATAGCAGCA AAACATTGGA 300

ACTGCGAGAG ACCTCAGTCA CACCAAGTAA CCTGTGGGGC GGCCAGGGCT TATTGGGAGT 360

GAGCATTCGT TTCTGCAGCT TTGATGGGGC AAATGAAAAT GTTTGGCATG TGCTGGAGGT 420

GGAATCAAAT TCTCCTGCAG CACTGGCAGG TCTTAGACCA CACAGTGATT ATATAATTGG 480

AGCAGATACA GTCATGAATG AGTCTGAAGA TCTATTCAGC CTTATCGAAA CACATGAAGC 540

AAAACCATTG AAACTGTATG TGTACAACAC AGACACTGAT AACTGTCGAG AAGTGATTAT 600

TACACCAAAT TCTGCATGGG GTGGAGAAGG CAGCCTAGGA TGTGGCATTG GATATGGTTA 660

TTTGCATCGA ATACCTACAC GCCCATTTGA GGAAGGAAAG AAAATTTCTC TTCCAGGACA 720

AATGGCTGGT ACACCTATTA CACGTCTTAA AGATGGGTTT ACAGAGGTCC AGCTGTCCTC 780

AGTTAATCCC CCGTCTTTGT CACCACCAGG AACTACAGGA ATTGAACAGA GTCTGACTGG 840

ACTTTCTATT AGCTCAACTC CACCAGCTGT CAGTAGTGTT CTCAGTACAG GTGTACCAAC 900

AGTACCGTTA TTGCCACCAC AAGTAAACCA GTCCCTCACT TCTGTGCCAC CAATGAATCC 960

AGCTACTACA TTACCAGGTC TGATGCCTTT ACCAGCAGGA CTGCCCAACC TCCCCAACCT 1020

CAACCTCAAC CTCCCAGCAC CACACATCAT GCCAGGGGTT GGCTTACCAG AACTTGTAAA 1080

CCCAGGTCTG CCACCTCTTC CTTCCATGCC TCCCCGAAAC TTACCTGGCA TTGCACCTCT 1140

CCCCCTGCCA TCCGAGTTCC TCCCGTCATT CCCCTTGGTT CCAGAGAGCT CTTCTGCAGC 1200

AAGCTCAGGA GAGCTGCTGT CTTCCCTCCC GCCCACCAGC AACGCACCCT CTGACCCTGC 1260

CACAACTACT GCAAAGGCAG ACGCTGCCTC CTCACTCACT GTGGATGTGA CGCCCCCCAC 1320

TGCCAAGGCC CCCACCACCG TTGAGGACAG AGTCGGCGAC TCCACCCCAG TCAGCGAGAA 1380

GCCTGTTTCT GCGGCTGTGG ATGCCAATGC TTCTGAGTCA CCTTAACTTT GAACCATTCT 1440

TTGGAATTGG CGTGGTATAT TTAACCACGG GAGCGTGTCT GGAAACGCAA ACTATCATTA 1500

ATTTCATACT AGTTTGTACC GTATCTGTAG GCATCCTGTA AATAATTCCA AGGGGAAAAC 1560

TAAACGAGGA CGTGGGTTGT ATCCTGCCAG GTTGAGTGGG GCTCACACGC TAGGGTGAGA 1620

TGTCAGAAAG CGCTTGTATT TTAAACAACC AAAAAGAATT GTAAGGGTGG CTTGCTGCCA 1680

GGCTTGCACT GCCGTTCCTG GGGGTGTGCA TCTTCGGGAA AGGTGGTGGC GGGGCGTCCA 1740

CTAGGTTTCC TGTCCCCTGC TGCTCCTTCC GTAAGAAAAT GAAATATTCT ATGCCTAATA 1800

CTCACACGCA ACATTTCTTG TACTTTGTAA GTCGTTTGCG AGAATGCAGA CCACCTCACT 1860

AAACTGTAAA CGGTAAAGAG ATTTTTACTT TTGGTCTCCG TGAGTCGCAT CTCTACTAAG 1920

GTTTACACAG GAATTCCACC TGAAGACTTG TGTTAAAGTT CTACAGCGCG CACTGTTAAC 1980

TGAACGTCTT TTTCTTCAGC CTATACGCGG ATCCTTGTTT TGAGCTCTCA GAATCACTCA 2040

GACAACATTT TGTAACTGCT GCTGTTGCTT TCTACATACA CCTTATAAAG TGACATTTCA 2100

AAAGAAATAA GGTGCCACAG TTTTAAACCA GAAGGTGGCA CTCTGTGGCT CCTTGTAGTA 2160

TTATAGCTAT ACTGGGAAAG CATAGATACA GCAATAAAGT ACAGTAATTT TACTTTTTTT 2220

CTTGTGTTAC ATCTAAATTA CAACCCTTAA TTGCCACGTG TGCACTTACT ACTCTCCAGT 2280

ATGTCTTATT ACTCTCCAGT ATGTCACGCA TCTTTAACTT TTCACGTCCT ATGTTTGCTT 2340

TCTCCCATTT TTAAGAGATG GTAAGTTAAC TGGAATTGAT TTACTGAATG AAATTAAATG 2400

CAGATATCCC TGTTTTTGAA ATAAAAAAAA AAAAAAAAAA AAAAAAAAAA AAAAAAAAAA 2460

AAAAAAAAAA AAAAAAAAAA AAA 2483

(2) INFORMATION FOR SEQ ID NO: 69:

(i) SEQUENCE CHARACTERISTICS:
(A) LENGTH: 536 base pairs
(B) TYPE: nucleic acid
(C) STRANDEDNESS: double
(D) TOPOLOGY: linear

(xi) SEQUENCE DESCRIPTION: SEQ ID NO: 69:

GAGAAATGGA GCTTTGTTAG ATAAAAATTT TTTCAACGCA AACAGTCATT TTCCAGTGAA 60

AGGAGAGCGT ATCCGCCGTA GGATGGACTT AGATCGTGTA AAAGCTGAGG CCACCGAGGA 120

TATAACCTCC GGGGTCCTTT GCCTCCTTTT CCTTAGACTC CCTCCAAACT CGTGTATCTT 180

TCCTTCAGCA GTACTGGGCT CCACGCGAAC CTAGTCCTTT GTCTTTACCC TATTACCTTT 240

CATAACATCC TAGTTGAAAA GTARTTATTC AACCGCGTTT GAAAATGAGA ACAGGTTCAC 300

AGARGCTAGG TTACTTGCGA AGGTCGTTCA ATTAGTAACC AGTAACGCCA GGACTGCCAG 360

TTTCTTGCTT CCGAATTCTC ATGGTAGCTT TCACCARGCT CCCCGTCMAA TGCTAACGTC 420

AACTACTGAA CTAGATTAGC AAAAAGGTCT TTTAACAGAA TTCCTGGTTT TCAGAGAGAG 480

TTTCTTTCAT GAAGCGCCCC ATTTCTACAG AGGAAAATAA ACTCCAAGCA GCCAGT 536

(2) INFORMATION FOR SEQ ID NO: 70:

(i) SEQUENCE CHARACTERISTICS:
(A) LENGTH: 865 base pairs
(B) TYPE: nucleic acid
(C) STRANDEDNESS: double
(D) TOPOLOGY: linear

(xi) SEQUENCE DESCRIPTION: SEQ ID NO: 70:

CCACGCGTCC GGCCTTTCTT GGCCAGAGGC GCCGGTTGGA CTCACGGGCG GGGCATGATG 60

GGTAACAGGA CCGGTGGGGT CCCCAGGAAG TCCTAGAGGG GGTCGGGGTT TGGGTGGACA 120

AGCTTTCCTC GTCCTCTCCC GACAGAGCTG ACGTGTCCTG GGTTCCACCG GGAGCGGGCA 180

TTTCCACCGG ACGGGAGGGT TCGGGGTGTC CGGGGCTGGG GAATACGTAG GGGTTGCCGC 240

GCGGTGTGGG GAGTTGGGGC GTGTGGCTGC AGTCCCGGGA GTTCTTGGAG GGGGTCGGCC 300

CACCGAGCTT CCGGACCGGC TGATCTGCCC GTAGCTTGCC GGANGGARGG CGGAGCTGAC 360

TCTCCGTCCC TTCTCCCATC CCCTCCAGTG GTGGGTACGG GCACCTCGCT GGCGCTCTCC 420

TCCCTCCTGT CCCTGCTGCT CTTTGCTGGG ATGCAGATGT ACAGCCGTCA GCTGGCCTCC 480

ACCGAGTGGC TCACCATCCA GGGCGGCCTG CTTGGTTCGG GTCTCTTCGT GTTCTCGCTC 540

ACTGCCTTCA ATAATCTGGA GAATCTTGTC TTTGGCAAAG GATTCCAAGC AAAGATCTTC 600

CCTGAGATTC TCCTGTGCCT CCTGTTGGCT CTCTTTGCAT CTGGCCTCAT CCACCGAGTC 660

TGTGTCACCA CCTGCTTCAT CTTCTCCATG GTTGGTCTGT ACTACATCAA CAAGATCTCC 720

TCCACCCTGT ACCAGGCAGC AGCTCCAGTC CTCACACCAG CCAAGGTCAC AGGCAAGAGC 780

AAGAAGAGAA ACTGACCCTG AATGTTCAAT AAAGTTGATT CTTTGTAAAA AAAAAAAAAA 840

AAAAAAAAAA AAAAAAAAAA AAAAA 865

(2) INFORMATION FOR SEQ ID NO: 71:

(i) SEQUENCE CHARACTERISTICS:
(A) LENGTH: 932 base pairs
(B) TYPE: nucleic acid
(C) STRANDEDNESS: double
(D) TOPOLOGY: linear

(xi) SEQUENCE DESCRIPTION: SEQ ID NO: 71:

TCATCATATA CAAAGTTTTT CGTCACACTG CAGGGTTGAA ACCAGAAGTT AGTTGCTTTG 60

AGAACATAAG GTCTTGTGCA AGAGGAGCCC TCGCTCTTCT GTTCCTTCTC GGCACCACCT 120

GGATCTTTGG GGTTCTCCAT GTTGTGCACG CATCAGTGGT TACAGCTTAC CTCTTCACAG 180

TCAGCAATGC TTTCCAGGGG ATGTTCATTT TTTTATTCCT GTGTGTTTTA TCTAGAAAGA 240

TTCAAGAAGA ATATTACAGA TTGTTCAAAA ATGTCCCCTG TTGTTTTGGA TGTTTAAGGT 300

AAACATAGAG AATGGTGGAT AATTACAACT GCACAAAAAT AAAAATTCCA AGCTGTGGAT 360

GACCAATGTA TAAAAATGAC TCATCAAATT ATCCAATTAT TAACTACTAG ACAAAAAGTA 420

TTTTAAATCA GTTTTTCTGT TTATGCTATA GGAACTGTAG ATAATAAGGT AAAATTATGT 480

ATCATATAGA TATACTATGT TTTTCTATGT GAAATAGTTC TGTCAAAAAT AGTATTGCAG 540

ATATTTGGAA AGTAATTGGT TTCTCAGGAG TGATATCACT GCACCCAAGG AAAGATTTTC 600

TTTCTAACAC GAGAAGTATA TGAATGTCCT GAAGGAAACC ACTGGCTTGA TATTTCTGTG 660

ACTCGTGTTG CCTTTGAAAC TAGTCCCCTA CCACCTCGGT AATGAGCTCC ATTACAGAAA 720

GTGGAACATA AGAGAATGAA GGGGCAGAAT ATCAAACAGT GAAAAGGGAA TGATAAGATG 780

TATTTTGAAT GAACTGTTTT TTCTGTAGAC TAGCTGAGAA ATTGTTGACA TAAAATAAAG 840

AATTGAAGAA ACACATTTTA CCATTTAAAA AAAAAAAAAA ACTNGAGGGG GGCCCGGTAC 900

CCAAATCGCC GCATAGTGAT CGTAAACAAT CT 932

(2) INFORMATION FOR SEQ ID NO: 72:

(i) SEQUENCE CHARACTERISTICS:
(A) LENGTH: 996 base pairs
(B) TYPE: nucleic acid
(C) STRANDEDNESS: double
(D) TOPOLOGY: linear

(xi) SEQUENCE DESCRIPTION: SEQ ID NO: 72:

CGCCTGGCAC CATGAGGACG CCTGGGCCTC TGCCTGTGCT GCTGCTGCTC CTGGCGGGAG 60

CCCCCGCCGC GCGGCCCACT CCCCCGACCT GCTACTCCCG CATGCGGGCC CTGAGCCAGG 120

AGATCACCCG CGACTTCAAC CTCCTGCAGG TCTCGGAGCC CTCGGAGCCA TGTGTGAGAT 180

ACCTGCCCAG GCTGTACCTG GACATACACA ATTACTGTGT GCTGGACAAG CTGCGGGACT 240

TTGTGGCCTC GCCCCCGTGT TGGAAAGTGG CCCAGGTAGA TTCCTTGAAG GACAAAGCAC 300

GGAAGCTGTA CACCATCATG AACTCGTTCT GCAGGAGAGA TTTGGTATTC CTGTTGGATG 360

ACTGCAATGC CTTGGAATAC CCAATCCCAG TGACTACGGT CCTGCCAGAT CGTCAGCGCT 420

AAGGGAACTG AGACCAGAGA AAGAACCCAA GAGAACTAAA GTTATGTCAG CTACCCAGAC 480

TTAATGGGCC AGAGCCATGA CCCTCACAGG TCTTGTGTTA GTTGTATCTG AAACTGTTAT 540

GTATCTCTCT ACCTTCTGGA AAACAGGGCT GGTATTCCTA CCCNGGAACC TCCTTTGAGC 600

ATAGAGTTAG CAACCATGCT TCTCATTCCC TTGACTCATG TCTTGCCAGG ATGGTTAGAT 660

ACACAGCATG TTGATTTGGT CACCTAAAAA GAAGAAAAGG ACTAACAAGC TTCACTTTTA 720

TGAACAACTA TTTTGAGAAC ATGCACAATA GTATGTTTTT ATTACTGGTT TAATGGAGTA 780

ATGGTACTTT TATTCTTTCT TGATAGAAAC CTGCTTACAT TTAACCAAGC TTCTATTATG 840

CCTTTTTCTA ACACAGACTT TCTTCACTGT CTTTCATTTA AAAAGAAATT AATGCTCTTA 900

AGATATATAT TTTAYGTAGT GCTGACAGGA CCCACTCTTT CATTGAAAGG TGATGAAAAT 960

CAAATAAAGA ATCTCTTCAC ATGARAAAAA AAAAAA 996

(2) INFORMATION FOR SEQ ID NO: 73:

(i) SEQUENCE CHARACTERISTICS:
(A) LENGTH: 785 base pairs
(B) TYPE: nucleic acid
(C) STRANDEDNESS: double
(D) TOPOLOGY: linear

(xi) SEQUENCE DESCRIPTION: SEQ ID NO: 73:

GGCACGAGGG GCTTTGCGTA CACAATAGCT GCTAGGAGTA CCCAAAGCCT GARTACARCC 60

TGCTGGTGTC ATGGCCACGT GTGAGCAGGC CAGCGTCAMA CGGCTCGCTG TGACCCGTCC 120

CGRAGACTGA AATGGGCCTG GGTCTTCTCC TKGTCCTGTG ATWAAAGTCC TCTCTTGAAA 180

GTGGAGAGCA AAGGCACACA GAGGTGCGCG CTCACAAGAA TTCCTCCCGG TGACTGGGTA 240

ATCAATGTTA CTGCTGTTTC CTTTGCAGGA AAGACCACAG CAAGATTCTT TCATTCGTCT 300

CCTCCTAGCC TGGGGGACCA GGCTCGAACT GACCCTGGAC ATCAAAGGAG GGATTATGTG 360

GCTGCTAAAG CCATCGGCCC ACAGCCCTGT TCACRTCTTG GTGCTTCTCT TTCCCAGAGG 420

CTGGTCCCAG CCAGGCACAC ACAAAAGGCA GATTCTCGTA AACSCAGCCT CCCTCCCTGG 480

AGGCTGCCTC CTGCCCTGGA TCTGGAGTGG AGCTGCTCTG AGATTTTGAG TTCTTCTGCA 540

GAGATGATTA AATATATCCA AGAGACATTG GAAAACCTGC TGAACATTTT ACATTGGTCT 600

GCTCAGCACA TGGCTGGATG CGGATATTTC TATAATTCCA GAAAGTCACA CAGCTCCTCT 660

GTATGAGACC AGTGGGCGCC ATTTAAAAGA ACAGGATGAG AATCTAAGAT ATATTATTAA 720

TAAATGTAAT GGATTTTTTT TTTGTAAAAA AAAAAAAAAA AAAAAAAAAA AAAAAAAAAA 780

AAAAA 785

(2) INFORMATION FOR SEQ ID NO: 74:

(i) SEQUENCE CHARACTERISTICS:
(A) LENGTH: 1069 base pairs
(B) TYPE: nucleic acid
(C) STRANDEDNESS: double
(D) TOPOLOGY: linear

(xi) SEQUENCE DESCRIPTION: SEQ ID NO: 74:

TCCTCACCAT TCCCCTAGGN CAGGTCCCTG CAGGTCCCAC ACTTCTCCCA GGTCCCTAAA 60

CTTGGGTCGG TCCTTTCCCT GGAGTAGCTG GNTCCTCCAG TCGAGGTCCC TGTTCAGTCG 120

GTTCTTAGGC TCCTGCACAT GAAGGTGTGT GCCTGTGGTG TGTGGGCTGC TCTAGGAGCA 180

GATACAGGCT GGTATAGAGG ATGCAGAAAG GTAGGGCAGT ATGTTTAAGT CCAGACTTGG 240

CACATGGCTA GGGATACTGC TCACTAGCTG TGGAGGTCCT CAGGAGTGGA GAGAATGAGT 300

AGGAGGGCAG AAGCTTCCAT TTTTGTCCTT CCTAAGACCC TGTTATTTGT GTTATTTCCT 360

GCCTTTCCGA GTCCTGCAGT GGGCTGCCCT GTACCCTGAA CCTCATGAGC CTCTAAGGGA 420

AAGGAGGAAC AATTAGGACG TGGCAATGAG ACCTGGCAGG GCAGARTACA AGCCCAGCAC 480

CAGTGTCCCA GCCTTACTGG GTCCTTACCC TGGGCCAAAC AGGGAGGGCT GATACCTCCT 540

TGCTCTTCCT AGATGCCCAC CTCCTACAAT CTCAGCCCAC AAGTCCTCTC CACCCTAGGG 600

GGCTTGCTGC ATGGCAATAA CTCATAATCT GATTTGGAGG TTTGCCCTTT ACAGGGGCAG 660

ATTTTCTGCT CAGTTCAACA ATGAAATGAA GAGGAACTCC CTCTTTCTAC AGCTCACTTC 720

TATCAGAGGC CCAGGTGCCT CAGAGCCACA TTGAGTTGCT TTTTCTGGGA TGAGGAAGTA 780

GGGTTAAACT CCCCAGTTTC CTGAGGGAGG CTCCTGACAG GTGCCCTTTG TCAGACCCTA 840

CCACAGCCTG GATAGGCAGC CACATTGGTC CTCGCCCTTG CTCGGNACTC CGTGGTGGTC 900

CTGCCCTTCT CCCTGCATGC CTGTGGGTCT GCTCTGGTGT GTGAAGGTCG GTGGGTTAAC 960

TGTGTGCCTA CTGAACCTGG CAAATAAACA TCACCCTGCA AAGCCAAAAA AAAAAAAAAA 1020

AAAAAAAAAA AAAAAAAAAA AAAAAAAAAA AAAAAAAAAA AAAAAAAAA 1069

(2) INFORMATION FOR SEQ ID NO: 75:

(i) SEQUENCE CHARACTERISTICS:
(A) LENGTH: 831 base pairs
(B) TYPE: nucleic acid

(C) STRANDEDNESS: double
(D) TOPOLOGY: linear

(xi) SEQUENCE DESCRIPTION: SEQ ID NO: 75:

GGACATTAGA TCACTGTGGA CCTAAAACAA ACAAACAACT ATAAGGAAAA TGGCATTAGA 60

AATGGTCTGG GGATCAGTTT ATCACTGCAG TTGTTACATC ACCCCATGGT CTAAAATACA 120

GAGCTTTAGT CTGTCTCTGT TTCAGTTCAT TTTACAGGAG GTGAACATCA CACTTCCAGA 180

AAACTCTGTC TGGTATGAAA GGTATAAATT TGATATTCCT GTCTTTCACT TGAATGGCCA 240

GTTTCTGATG ATGCATCGAG TAAACACCTC AAAACTTGAA AAACAGCTCC TGAAACTTGA 300

GCAGCAAAGT ACTGGARGCT GACTGATGCC CTCATGATTT TCCACCCTCT CTTCCCATAA 360

AGCATCTTCC TAAGGAAATG AMCATGGCCT GATACTCATT TTGTCACTTG TACAGAGCCC 420

TAAGGATGTT CTGAATTCAG TGGTGCCAAA TAAATGTTGA CATTCCCCTT TTGGTTGATG 480

GAAGTATCAG TGTGGGAACT GTTTGCTTAA TGGCATTTTA TAAAATAAKA AKAKCATATT 540

AGCAGGGAGG GAGATGATGG AGGGAGGGAG AAGTCCATTT GTCTTATTTA TCCTTTTTGT 600

ATTAATAGAG AAGCACTTCA CAGTCACTGG CAATGCCATT TATAGGAAGA AGGTTCTGCA 660

TTCCTGCTGC TCCCGGAGGG CTTAACTTTT TAATGAAAGA ATAAATGCTC TTCCACTCAG 720

TAGATAAAGT GAAATGTGAA TTGTTAATAA CTGTGCACGG TCAATAAAGC GATGTTTTAA 780

GGAATACAAA AAAAAAAAAA AAAAAAAAAA AAAAAAAAAA AAAAAACTCG A 831

(2) INFORMATION FOR SEQ ID NO: 76:

(i) SEQUENCE CHARACTERISTICS:
(A) LENGTH: 590 base pairs
(B) TYPE: nucleic acid
(C) STRANDEDNESS: double
(D) TOPOLOGY: linear

(xi) SEQUENCE DESCRIPTION: SEQ ID NO: 76:

TATATATAGA CNGTTAATAG TCGTGANTGN TGTGNACGAA CATTAACGGA AGTAGCATGT 60

AGCCAGTCGA ATAACNTATA AGGACAAAGT GGAGTCCACG CGTGCGGCCG TCTAGACTAG 120

TGGATCCCCC GGCTGCAGGA TTCGGCACGA GCTGCCAGGT GAGGAGCAGA GAGACTGTTC 180

CCTTGGGTGG AGAGGTGTGG GCATGAGAGC CACCCATTGC CAAGCAGCAA GAATGTTCGT 240

GCTTTTTTCC CTTCCAAAAT ATGCAGGGCT CAGGCTCCCA ATTCCGGGCC TGTCTGCTTT 300

GCTTGTGTTT CTCCTGTCCC TGTTCTCCCG GAGGGCCCAG GTGGAACTCA CGACAGGGAG 360

GGAGACGCTT CCCAAAAACC TGCAGGGCTA TTTCCCAGAA TTTGGTTTTC AAGTACAAAA 420

CTTTTTGTCC TGTAAGATAT ATGCAGCCTC ACAGAAGCAG CCTCTGCCTC CACTTTACCA 480

GCTACGTTTT TATCTTAAGC ACATGGGGCT CCCTTAGAAC TTACTCCACT GATTTAAAAA 540

AAAAAAAAAA AAACTCGAGG GGGGGCCCGG TACCCATTCG CCCTAAAAGT 590

(2) INFORMATION FOR SEQ ID NO: 77:

(i) SEQUENCE CHARACTERISTICS:
(A) LENGTH: 1274 base pairs
(B) TYPE: nucleic acid
(C) STRANDEDNESS: double
(D) TOPOLOGY: linear

(xi) SEQUENCE DESCRIPTION: SEQ ID NO: 77:

GAGCCACCAC ACCTGGCCTG GAAGGAACCT CTTAAAATCA GTTTACGTCT TGTATTTTGT 60

TCTGTGATGG AGGACACTGG AGAGAGTTGC TATTCCAGTC AATCATGTCG AGTCACTGGA 120

CTCTGAAAAT CCTATTGGTT CCTTTATTTT ATTTGAGTTT AGAGTTCCCT TCTGGGTTTG 180

TATTATGTCT GGCAAATGAC CTGGGTTATC ACTTTTCCTC CAGGGTTAGA TCATAGATCT 240

TGGAAACTCC TTAGAGAGCA TTTTGCTCCT ACCAAGGATC AGATACTGGA GCCCCACATA 300

ATAGATTTCA TTTCACTCTA GCCTACATAG AGCTTTCTGT TGCTGTCTCT TGCCATGCAC 360

TTGTGCGGTG ATTACACACT TGACAGTACC AGGAGACAAA TGACTTACAG ATCCCCCGAC 420

ATGCCTCTTC CCCTTGGCAA GCTCAGTTGC CCTGATAGTA GCATGTTTCT GTTTCTGATG 480

TACCTTTTTT CTCTTCTTCT TTGCATCAGC CAATTCCCAG AATTTCCCCA GGCAATTTGT 540

AGAGGACCTT TTTGGGGTCC TATATGAGCC ATGTCCTCAA AGCTTTTAAA CCTCCTTGCT 600

CTCCTACAAT ATTCAGTACA TGACCACTGT CATCCTAGAA GGCTTCTGAA AAGAGGGGCA 660

AGAGCCACTC TGCGCCACAA AGGTTGGGGT CCATCTTCTC TCCGAGGTTG TGAAAGTTTT 720

CAAATTGTAC TAATAGGSTG GGGCCCTGAC TTGGCTGTGG GCTTTGGGAG GGGTAAGCTG 780

CTTTCTAGAT CTCTCCCAGT GAGGCATGGA GGTGTTTCTG AATTTTGTCT ACCTCACAGG 840

GATGTTGTGA GGCTTGAAAA GGTCAAAAAA TGATGGCCCC TTGAGCTCTT TGTAAGAAAG 900

GTAGATGAAA TATCGGATGT AATCTGAAAA AAAGATAAAA TGTGACTTCC CCTGCTCTGT 960

GCAGCAGTCG GGCTGGATGC TCTGTGGCCT TTCTTGGGTC CTCATGCCAC CCCACAGCTC 1020

CCAGGAACCT TGAAGCCAAT CTGGGGGACT TTCAGATGTT TGACAAAGAG GTACCAGGCA 1080

AACTTCCTGC TACACATGCC CTGAATGAAT TGCTAAATTT CAAAGGAAAT GGACCCTGCT 1140

TTTAAGGATG TACAAAAGTA TGTCTGCATC GATGTCTGTA CTGTAAATTT CTAATTTATC 1200

ACTGTACAAA GAAAACCCCT TGCTATTTAA TTTTGTATTA AAGGAAAATA AAGTTTTGTT 1260

TGTTAAAAAA AAAA 1274

(2) INFORMATION FOR SEQ ID NO: 78:

(i) SEQUENCE CHARACTERISTICS:
(A) LENGTH: 1133 base pairs
(B) TYPE: nucleic acid
(C) STRANDEDNESS: double
(D) TOPOLOGY: linear

(xi) SEQUENCE DESCRIPTION: SEQ ID NO: 78:

AGGATTTTTC CTTGTTCAAC CAAAATCTGA GCATTCTTTC TATGTTGAAA ACACTGAAAA 60

ACTAATTTWA GTTAATGAAC TAGAAAGAAT ATTGATTTTW AAGAAACAGA AAAATACTAC 120

TTATTTTCCT TCTCAAATAA CGTTTCTTTC AAAAACTTCT GGCTGAAGTA TAACATGCTG 180

GTAGTTAACA TAAATCTTGT CTTTCTCTTG TTCTTTATCT TTCTTTGTTA TTTAGATGCT 240

TGTATAAATG TCTTTTGTTT TTATTAAGTG CCTAATTGAC AGAGCTTAAT TTGAAGAAGT 300

GCCCTAATTT ATTGACCACT TAAGAATTGC CTTTATTGGG GTATTTTATT TGTTCCTGCG 360

TCTTTTTGAT GTTGTTCAGT CTACTCATCC CTGTGAGTAT GTGTGGGGGA CAGCTGATAG 420

AAGGGAGGAG AGTGTGTCTA TGCTCAGGAT TGCCCTTTAG CCACTCAGCC AGAGATCCAC 480

AGGGAGCAAC AAGGACAGTT TCACATGCTT AGACTTTCTT GGAAGAAACA GTGAGGAGGA 540

GTAAGTCGTG AGTAGTGTCA AGCTGGATGT AGAATTGTCC TAAGGCAGTT GACCCCACCT 600

TCCAACATGT TTTCACTTTA TTTGCCCCTC CCTACATTTG GGTTAGGTTC CATTTGGATT 660

TGCAGCAATA ATGACTTTAT TTCTCTCTTG GTCAGGATTT GGCACATAAA ATCCTTTTAT 720

TATAGAACTA GCTATTTTAG TTACATAGTA ATGTAACTAA TGGAGAGATT TATAGAGAAT 780

TTTGKTTTTG CTGTCATATA TGTCCATTTT GGAGACAGAT ATGATAGAAC TAGAAATTAA 840

GTTGCATTTC TGCAAGTGCC ATTTGAATGA ACTTCAAGTA TCTTCTTAAT TATTAAATTT 900

TCTGATGAAG GCATTGTAAC AAATATATAG TATTATTAAA TCTAATTAAT ATTTGGAAAT 960

ATTAATAAAT AGGTATTTTA TTTACTGTAA AAAGTCAAAC TTCATTATGT AGATAAATCT 1020

TATTCTTTTC ATTCTTTCCC CTGTTTACAT CCTTTTTACA AAGCTTAGTC ACCAATTAAA 1080

GCTTTCCTAT CAAAAAAAAA AAAAAAAAAA ACTCGAGACT AGTTCTCTCT CCT 1133

(2) INFORMATION FOR SEQ ID NO: 79:

(i) SEQUENCE CHARACTERISTICS:

(A) LENGTH: 661 base pairs
(B) TYPE: nucleic acid
(C) STRANDEDNESS: double
(D) TOPOLOGY: linear

(xi) SEQUENCE DESCRIPTION: SEQ ID NO: 79:

GAATTCGGCA CGAGGGGAAA AGGATGCTGA ACGAGAGCAG AAAGCCTCTT TCCTTTGCTT 60

CACGCCTTTC CAGTCTTTAT TTTAAACTCG GGTTCCCTTT CTGTGGTCGC AGCAACCTTT 120

ACTCCACCTG CACTGCTGCT CCTGGGGGCT CCCCAGGCCT CCCTCTGCCT TTCTACCCAG 180

TGGCTGACGG GATGCCTGTC TTGCCTGGAC GCACCACTGC TCTCCTGTCC CTCACCTTGG 240

CTTTTGCTGT GCCCTGCTCT GGGGTTGAAG CTGGCCCATG TGTCCCCCGG AGTCATGGCT 300

GCTCCTCCTG GGAGGCCTCT GTGTGCGTCA CGTCTTCCAC ACCTGGGGGC AGCTGGCGAG 360

CCCGTGCTCT GTTCCCCTCG GCTGCTTGGC ACAGAGYTGC AGCCTGGGAY TCTCCGTGGA 420

CCCAGACTGG GGATTTTGCC AGGGGGGCGA TGGGAGGAGC AGGTGCTTTG CCTGGCGGCT 480

GTGTCTGCAT TTCTGGACGC CCCAGAGCAC AGAAGTTGCC GGCACTTTGA GGTCTTCCTC 540

GGCATGTGCC AGATTACATG AGTGACGGCT GGGAATATGT TTTCTTTTTT GTAATGGAGG 600

CGTGTTTCAC ATATAGTAAA GCTCACCAAA AAGTAAAAAA AAAAAAAAAA AAAAAACTCG 660

A 661

(2) INFORMATION FOR SEQ ID NO: 80:

(i) SEQUENCE CHARACTERISTICS:
(A) LENGTH: 1378 base pairs
(B) TYPE: nucleic acid
(C) STRANDEDNESS: double
(D) TOPOLOGY: linear

(xi) SEQUENCE DESCRIPTION: SEQ ID NO: 80:

ATTGGGTACC GGGCCCCCCC TCGAAGTTTT TTTTTTTTTT TTTTAATGAA AGCTCTCAAA 60

TAAGCGATTT TATTCCTATC CATGATTGCA GACATTTACA AAACCATAAC ATCTGAGTTC 120

ACCTTAAAAA ATAACTTATA TAAAGCAGTG ATATACACAG CACAAAATAG TTCAGGGAGG 180

GGGCAGGAGC AACTTGTAAT AATTAAAATG TAAACGTGAA AAAAAGGATG GAATAAAAGT 240

CCCTACTTAT TTCTACTTAA GATGTCATGT GATAATATTT TACAATGTCC TGTGGGTCAA 300

TGTATGTATG TGTATATGTC TGTATAACAT ACACATATAC AGTACATTCT CTTTCCCACA 360

CATATACATA CACACATAAT TATTTGCAGT TCAGTTTAGG GCAATTCTAA TATGCCACTC 420

CGTACAGTTG TTTGAATCAC ATTTGGACCC GCTTTCTTCA CAAAAGAGGG GAGAGAGCAG 480

GAAATAAAAA GGTTGGTTTG GTGTGACTGA GATTCCTTTG TTTAACTGTA CACTGTGATG 540

AATAATTTTC TTCCGTAGTA GTTCTGTGAA GGGCTGACTC ACTGTGGTTT TCATGAGGAG 600

ACTTGGTAAT GGATCACACG CTCATTGTCA TGCTAGGGGA GTAACTCTCA CTCTGAAAAG 660

GATTTAAGAA ATTTCCCCCC ATTTCGCCAT CATCCCTTGG AGTGCCCGGT TGATTACTCA 720

GGCTCATATT ATTGGGAGAA TTCTTGGAAA TACTGTCCAT ATCTCCTGAG CCTAAAGAGC 780

CATTCATGTG ATGTGACTCC ATTCCTCCTA ATCCACCCAT GGGACCATCT GACCCAGGRC 840

CCATTGGAAA ATTAGGTCTG TTAGGTCCAG GAGGTACTGC ATTCATTAAA GTATACATGT 900

TATCACCAGA GTTGGTTGAA TCTGCTGGAC TAGGCATGAT GGGTGTTCCT GGTGGCCCTC 960

CACCTCCTGG AGGACCTACA TAATTCCCAG GAGATGCTGA GGAGTATGGT ATTGAATTGG 1020

CATTTGTTGG GTTTGGCCAA GGTCTACCAC CACCTGGACC CATGTTCATT CCAGGCATTC 1080

CAGGGCCACC TAAAGCATTC AGTGGGGGTC TCATTGCACC TCCATAGTTC TGTGGTCCTA 1140

AGGGCACCAT TCCTCTTGGA GGAGTCATTC TCTGCATTGG CCCACCCATA TTTGGATGTC 1200

CTTGTTGTCG AGTTGGATCC ATTCCACTGG GGAGTAATGG CTGACTTCCT GGGACACCTC 1260

CAAGTGCCTG ATTAGGTATC CTCAATGGGG GCCTTGGACC TCCAGGGTAC CGAGGTGACA 1320

TAAAAGGGTA ATCATGGAAG GCTTTTGCTT CACTTGAGTG TTCACATGTT TCACGTCT 1378

(2) INFORMATION FOR SEQ ID NO: 81:

(i) SEQUENCE CHARACTERISTICS:
(A) LENGTH: 1440 base pairs
(B) TYPE: nucleic acid
(C) STRANDEDNESS: double
(D) TOPOLOGY: linear

(xi) SEQUENCE DESCRIPTION: SEQ ID NO: 81:

ACTTTGTCCA AATGTGTCTG TCACATGTAG TCAGCTGNAG NAATTTAAAA TGAATTGCCA 60

AGTGAAGAGT CTGTGGATTA ATTGGCCGTT AATTAACAGG CTTTATCAAT GTGTCCTCAA 120

GGGAGAGGCC CAACCCTAAT TAAGGAGCTA AACTTCCTGA GTGAGGGGCT GTGAGGATGG 180

AGGTGGAGGA GGCATCTGGG GCGGGTGGTG GCCGGGCCAG CAGATGGCGC CTCCCTGGCT 240

GAGCTGCCCG CACCGCCAGT TCCCTCATTT CCACTCAGGA AGGCAGAGAA GGCAGAGTGA 300

TCTCCTCAAG GAAGAGCTTC CCCAGCCTTC GGGAGCAGCT GGCAGGGCGT CCGGGAATAA 360

GCCCTACACG CCGCCGCCTG CCTCCAACTC ACTAACCCTG CGCCTCTTGT CTTTCAGATT 420

CAACGCGTTC AACAGAAGCC ATCCCCAGCC CAGCTTAAAT TATAAAGATA GACAATAACT 480

CTGTTCCAAT CTGCGTGGTG CTTCTTTAGT AAATACTGTA CAGATTTTAC CATGGAGAAC 540

TTTTTTTTTA GTTTTTACCT TTTCTTAATT ACCCTTATTC CGAATGGACG AACACTTTCT 600

ACCACTGCTG ACCATTGTAA AATACCGTGT ATATAAATCC CATTGAAATA ATGCCCTGGA 660

ATAGAACATC TCAAATGCTG CTTAATTACA GACTCAGGTC GATTACTTGT ATTTCATGTA 720

ATGTTCCTCC AAGTTAGACA TCTGGTGCAA GACCAACCGG GAGACCATGG AATTGTCAAA 780

AGTACAAACT GACAGTGTGT ATATTTAATT TAAAGACTTA TTTAAAAACT CACAAGCTCT 840

CACCTAGACT TTGGAGAGCA GTCTGTTTTC TGTAATGTCT GATACTAGAA ACTAATTTGC 900

TTATTTTAGT TGTATTCAAG ATTTGAAGAT GTATTTTATA GACAAGTTCT GTTTTTGAAC 960

TTTGTGGAAC TGTTCCAATC AATCAATTTC CCAGTTATGA TGAGTATTTA CATTATGAAT 1020

GTATAACCCA GACATGATTT GTAAAGCCGA CAGTATGTTT CTATTACACA ACACTTTTTG 1080

ATACAGCGTC TCTTGTCTTC ACTGATACTG GAGTCTCCGT TGTCTGCNNG GTCCCTTCGA 1140

GTTTCTAGTT ACAGACACAA TCATACTGTG ATTTTATTTT TAATATGGAT ATGCTATCAA 1200

ACTGTGATAC ACTTATAATT CACTGGTCCT GCATCAGGAG ATGGAGTGGG GAAAACTGTA 1260

TTTAATACAG TTTGTATCTG AATAATCTGT ATGGTTTATA CAGTTTGTGT TGTTCAGAGA 1320

TGTTTAAAGT TTGATCTTTG TTTTTCTAAA GATTAAAAAA GCACTTGCCC CACTGTAAAT 1380

ATACAGCATG TAAAATTTCT RTAGTATATA AATGGCAGCA AATCACAAAA AAAAAAAAAN 1440

(2) INFORMATION FOR SEQ ID NO: 82:

(i) SEQUENCE CHARACTERISTICS:
(A) LENGTH: 1381 base pairs
(B) TYPE: nucleic acid
(C) STRANDEDNESS: double
(D) TOPOLOGY: linear

(xi) SEQUENCE DESCRIPTION: SEQ ID NO: 82:

CCCGGGCTGC AGGAATTCGK YACGAGGCCA GCAGTTGCTC CCAGTTCAGG AGGTGCTCCT 60

GTACCCTGGC CACAGCCCAA TCCTGCCACT GCTGACATCT GGGGAGACTT TACCAAATCT 120

ACAGGATCAA CTTCCAGCCA GACCCAGCCA GGCACAGGCT GGGTCCAGTT CTGACCTGAG 180

CACGGTTTTT CCTCATGTGA CTTCTGGGAA GGCGCTCCCT CATCTGGGCC AAAGGAAGGA 240

GGACGAAGCC CTCCTCAGCT GGCCTGTGTT TGGGGCATGA ATCTCTCCTC TCCTCCTTGT 300

CTGGCTCTGT TGACAAACCG GGCATGTTTG GCAGTAAATT GGCACCGTGT CACACTGTTT 360

CCTGGGATTC AAGTATGCAA CCAGAACACA GGAGAAGAAA AGCTCCAGGA TCCCTGTCCC 420

CATCTGTCCT CTTGATGTGA GAGAGACTCT GAGACTTCTT CCATCGCAAT GACCTGTATT 480

AAACACAAGC CCCCCAAGCA AAAGAAGAGG TTGAGTTTGC TGCCAGGATT CAGATCAGCC 540

CTTCCCAGGG TCTGCAGGTG TCACATGATC ACAGTTCAGC GGGAGGCTTT CCGTACCCAC 600

ACTGGCTGTA GCACTTCAGT CCATCTGCCC TCCAGAGGAG GGTTTCTTCC TGATTTTTAG 660

CAGGTTTAGA GGCTGCAGCT TGAGCTACAA TCAGGAGGGA AATTGGAAGG ATTAGCAGCT 720

TTTAAAAATG TTTAAATATT TTGCTTTGCT AATGTGCTGA TCCGCACTAA CTCATCTTTG 780

CAAAAGGAAC TGCTCCCTCG GCGTGCCCCA GCTGGGGCCT CTGAAGGGAT TCCTCACTGT 840

GGGCAGCTGC CCTGAGCTTC AGGCAGCAGT GTTCATCTCT GGCCAGTTGT CTGGTTTCCA 900

TGTATTCTAG GCCAGGTAGG CAACACAGAG CCAAGGCGGG TGCTGGAAGC CAGACGGAAC 960

AGTGTTGGGG CAGGAAGGTG GATGCTGTTG TCATGGAGCT GTGGGAGTTG GCACTCTGTC 1020

TGCTGGTGGC CCTCTCGGCT CACATGTTCA CAGTGCAGCT CCTGGCAGAC TTGGGTTTTC 1080

TCTTTGGTGG TTTCTAAAGT GCCTTATCTG CAAACAACTT CTTTTCTCCT TCAGGAACTG 1140

TGAATGGCTA GAAGAAGGAG CTCAGTAAAC TAGAAGTCCA GGGTTGCTTG GTTTACTGGT 1200

TTATAAGAAA TCTGAAAGCA CCTCTGACAT TCCTTTTATT AACTCACCTC TCAGTTGAAA 1260

GATTTCTTCT TTGAAAGGTC AAGACCGTGA ACTGAAAAAA GTGTTGGCCT TTTTGCGGGA 1320

CCAGATTTTT AAGATAAAAT AAATATTTTT ACTTCTGTCA AAAAAAAAAA AAAAAAATNT 1380

C 1381

(2) INFORMATION FOR SEQ ID NO: 83:

(i) SEQUENCE CHARACTERISTICS:
(A) LENGTH: 1706 base pairs
(B) TYPE: nucleic acid
(C) STRANDEDNESS: double
(D) TOPOLOGY: linear

(xi) SEQUENCE DESCRIPTION: SEQ ID NO: 83:

ACTGCACCAC TGCCCAGGTC TCCCGGCTGG ATGAAGACGT GGTCCATGAG GAAGCTGGCT 60

AGCTCAGACT GGAGAGTAGC TTCAGGAAAA AAGACAAGTG GCCTAAGGAA ATCACGGCCC 120

CCAACTATCA TCTGAGGGCT AAAGATGAGA AGTAGATCAC TTAATAAGAC AAAAGCCTGT 180

AGGGGGAAAA GAAAGGATGT TTAAAAGGAC AGAATGTTTC CCAAGGTAGA AATGACACTG 240

TCAATTTCTC CTTGGAATGG GGGCAGGGAT ACTCGCCTTG TTGCTCCCAC TTGAGTCAGT 300

ACTCACCTGC TCCTGGATCT CAGTATCCAC ATCTGAGAGG CAACTCTGGC AGAGTTCACA 360

GAAGGCCACC ATTCTGTCCC TCAAACTCGA CAGCTGCTTC TGTGGGCACA GTGGCTTGAA 420

GGGGAAGAAT GAAGACACAG ACTCCTCTGT TCCCATTATC CCATCTAAGA CCCACACTCA 480

CCTGGGGAAG CATCTGATTT AGAAATGTGG GTTAGTGTCC AGAGAATGGA AAAATAGACA 540

AGAGTCAAGG CTGGCAGGAT AACCTGTAAC AACAAAGGGT TTGAAAAATG AGGTTTGGGT 600

TAGGAGAGGG AGAGACAGAT AGCCAGAAAC ACACCAGTGA AGAGGAGAGA AAATGAGTAA 660

AGGGAGAGCT AATTCCTTTT CCAGTGGAAA ATGAGTGATA TTCTGGACAT TCTTCAGAGG 720

CATCTACACG AAGTAGAAAT GTCACCGCTC CCTAATTTAC TCTACGTCTT CTAGAATCCC 780

TCAATATTAT CCTTGGCTTC CAGGAAATCC AAGAAGACCC TGGAAGTAGA GTCCACCTTC 840

TAAGAGAGGA ATGTAAGAGG TGACCCCCAC CCACCTGATC TTCCTCGCTT TGTCCACTCC 900

ACGCACTGAG ACTTGACACA CCTAGTGGCC ACCTAGAACG TAGGTCCTTA AAATYTAGCC 960

CCCCAGCCCC CAACCCATCT CTAGCCTGTC CACTCACCTG GTGAGGAACY TYTCCTGTGT 1020

CCACAGCYTT CTGCAGGAGT TGGCAACATG GCTCATAGAG CTCCCAGCGA GTCAGGTCAT 1080

GAGTGCTTTG GGGGAGAAAG GGGAATGTTA TACTGGAAAA GAACAGAGGG AACCAACTCC 1140

ACAGACACCA GTAAAAACGG GATGGGGAAG AGGAGGAAAG CCACTCACTT GTAGAAGGCA 1200

GAGAGGCGTT TCAGAGTGGC TGCCAGATTA TATACCTCAT CCTCATCTAG GAAGGACGAC 1260

TGAGAAGGAA AGAAGATCCA CAATAGCATT TCCCCCAGAA CTCATCAGTC CACATCCCCC 1320

GTCTTGCAGC CCCTCCCACC CTTGTTTGGG GTGTCCCATT GTCCAGCCCC AGCTCCTACC 1380

TGTAACAGCT CTTCAAGCTC CTGCTGGAAR CGGTCAGTCA GCAAATCTAC TAGCTGGCTG 1440

CGGGCAAAGT CCGCCCGGCT GAAGAAAGTG AATTCGGGAT TACAGAGCAG GTAAGAGCAT 1500

GCGCCCCAGC CTCAAGCACC GCTGGCTCTG CATGCTTCAC CACCACCTCC TGGAGTTGCT 1560

GCAGGAACAG CTCCAGGTGC TGAGAAGAAA AGGCAGAAGA TGGTGTGCTG TGGGGATGGG 1620

AGGAGGACAC TCTTCTGGCG GGAAGTGGAA CGGGGTTAAA AGCATTAAAC TTCAAGGATA 1680

AGATGCCTAA RAAAAAAAAA AAAAAA 1706

(2) INFORMATION FOR SEQ ID NO: 84:

(i) SEQUENCE CHARACTERISTICS:
(A) LENGTH: 573 base pairs
(B) TYPE: nucleic acid
(C) STRANDEDNESS: double
(D) TOPOLOGY: linear

(xi) SEQUENCE DESCRIPTION: SEQ ID NO: 84:

GAATTCGGCA CGAGCTTGGT AGCCTTAGAA CTGCATGAGC TGCTTTACCA CTGGGAAACA 60

CGAGCACAGC CTAGCTTGAT TTTGTATGTG GTATCAGATC TAAGGTGGAT GGAATTCAGG 120

ACTTCCTGTC TACTCTTTGA TTTTGTTTTA TTTTTAGAAA TGTTTTATTT TGTTTTATTC 180

ATTTATTCAT CTTCAGAGAC ATGGTCTGGC TCTGTTGCCC AGGATGGAGT GCATGGTGTG 240

ATCATAGGCC ACTGCAGTGT TGAGCTCCCG GGCTCAGGCG ATCCTCCTGC CTCAGCTYCC 300

TTAGTAGCTG GGACTATAGG CACATGCCCT ACCATGCCTG GCTTTGTCTA CTTTTTGAAT 360

GATGTCYCAA ACTAGAAGGT CTATTAATTT AAAAAATTAA GGATAGCATG CCATAATTAA 420

AAATAATAAC AGTGGAAAAA GGCACCTTCC AATGATTCAG ACATCAACTT GTGATTTAAA 480

AAAACGAAAA ATAAATAATA GGAAAAAAAG GGGAAAAAGT TAAATAAAAA TAAAATTAAA 540

AAAAAAAAAA AAAAACTCGA GGGGGGCCCG GTA 573

(2) INFORMATION FOR SEQ ID NO: 85:

(i) SEQUENCE CHARACTERISTICS:
(A) LENGTH: 684 base pairs
(B) TYPE: nucleic acid
(C) STRANDEDNESS: double
(D) TOPOLOGY: linear

(xi) SEQUENCE DESCRIPTION: SEQ ID NO: 85:

CTCTTTGGCT GTGTCTACCT CCTTCATCTG CTGCGCCGAC ATAAGCACCG CCCTGCCCCT 60

AGGCTCCAGC CGTCCCGCAC CAGCCCCCAG GCACCGAGAG CACGAGCATG GGCACCAAGC 120

CAGGCCTCCC AGGCTGCTCT YCACGTCCCT TATGCCACTA TCAACACCAG CTGCYGCCCA 180

GCTACTTTGG ACACAGCTCA CCCCCATGGG GGGCCGTCCT GGTGGGCGTC ACTCCCCACC 240

CACGCTGCAC ACCGGCCCCA GGGCCCTGCC GCCTGGGCCT CCACACCCAT CCCTGCACGT 300

GGCAGCTTTG TCTCTGTTGA GAATGGACTC TACGCTCAGG CAGGGGAGAR GCCTCCTCAC 360

ACTGGTCCCG GCCTCACTCT TTTCCCTGAC CCTCGGGGGC CCAGGGCCAT GGAAGGACCC 420

TTAGGAGTTC GATGAGAGAG ACCATGAGGC CACTGGGCTT TCCCCCTCCC AGGCCTCCTG 480

GGTGTCATCC CCTTACTTTA ATTCTTGGGC CTCCAATAAG TGTCCCATAG GTGTCTGGCC 540

AGGCCCACCT GCTGCGGATG TGGTCTGTGT GCGTGTGTGG GCACAGGTGT GAGTGTGTGA 600

GTGACAGTTA CCCCATTTCA GTCATTTCCT GCTGCAACTA AGTCAGCAAC ACAGTTTCTC 660

TGAAAAAAAA AAAAAAAAAA AAAC 684

(2) INFORMATION FOR SEQ ID NO: 86:

(i) SEQUENCE CHARACTERISTICS:
(A) LENGTH: 1036 base pairs

(B) TYPE: nucleic acid
(C) STRANDEDNESS: double
(D) TOPOLOGY: linear

(xi) SEQUENCE DESCRIPTION: SEQ ID NO: 86:

TGGAGGCAGA TGCACAGGAG AAAGGTTCCC GTCCGCACCC TCTCAGACCT GAGGCTGAGC 60

TTGCAGTGAG GGCTTCTCCT CGGCCCCTCG CCCGCCCCCA GAGCTGCCAT CCCTGCTGTT 120

ACAAGCCAGA GGAGCCCGGA TGTGAGGCCC CAGATCACCT CCAGGGACTT GGGGTTCCCA 180

TCTGAAATCC TTTATTTTTG TACCATGGGG TGGGCCCCGG GCTGAGAAGG AAGAAGCACC 240

CTCTCCCCGG CCTCCTCTGT CTGCACCCGT GGGGCTGTGA CTTACTCCTG CCTCCAGGGG 300

CGGGGCGGGG CCCCCTGGGA CCTCTTAAGG CCCAAGGTGG GCCCCAGGAC CTYTGGGCAG 360

AGTGGAYTGC TCATGGCAGA TGTGTGGCAA TGTCTGGCTG WGTCTTTCCG GCAMCTGCGT 420

YCCCTYTCCC GGGYTCCCCT GCTGCATGGT GGATGTGCTC CTTCCTGGCC CGGTCACATT 480

GCCTCCTTGA GCCTTAGTCC AGGGGGTCAC TYCTCCCACC CCACCTACCT CACAGGGTTG 540

TTGTGAGGGT GCACAGAGGA GCAAAGTCCC TGAAGGCCCT CAGGCAGTAT ATAGGGGCCG 600

CCCACCTTCA GCTGCCCTGG GATGGGAAGG ACCCAGCCCG ACCCCTGGGC ATAACACTGT 660

GTTTGCAAAT GGAGATTCAG GTATTGGGGA TGCAGGTTGT GGGGAGCTGG CCTGGCAGAG 720

TAGGGGTAGT TGGCTTGGCC TTCTCTTTGG TGATCCCACC CCCAGCCATT TGCATTGCTG 780

GCCCAGCGCC TGGCCTGGGG GGCGGGGAGA GGCAGCAGAA GGGGCTGGGC AGGGGCGGTG 840

GAGGACTCAG GAACTGCCCG GGGAGAGTGG GTATGGCGGC TGAGCCAGGG GCCCTCCTGT 900

GTTTGACTTC CCGGGATGGG TCCTTGCTTC TCAGCTGTGT CCGACCCCAC CATGTAATAA 960

AACCCAAAGG AACAGCAAAA AAAAAAAAAA AAAAAAAAAA AAAAAAAAAA AAAAAAAAAN 1020

CCCNGGGGGG GNCCCG 1036

(2) INFORMATION FOR SEQ ID NO: 87:

(i) SEQUENCE CHARACTERISTICS:
(A) LENGTH: 908 base pairs
(B) TYPE: nucleic acid
(C) STRANDEDNESS: double
(D) TOPOLOGY: linear

(xi) SEQUENCE DESCRIPTION: SEQ ID NO: 87:

TTAAACAAAT GGAATCATGC AATATGTGAC CTTTTGCGTC TGGCTTATTT TATTTAGCAT 60

AATGTTTTTG AGGTTCATCC AAGCTGTAGC ATGTATCAGC ACCTCATTTC TTTTTCTGGC 120

TGAATATTAT TCCATTATAT GGATTTACCA CAATTCATTT ACCTATTCAT CTTTTGTTTC 180

TGCTGTCTGG CTATTGTGAA TAATGCTTCG ATAAACATTC ATATACAAGT TTCTATGTGG 240

CTTTATGTTT TCATTTCTCT TGGCTATCTA CATGGGAGTA GAATTCTAGG TCATAATATA 300

ATTTTATGTT TAACTTCTCA AAGAATTGCC AAAAGGTTTT TCATAGTGGC TGCATCATTT 360

ACATTCCCAC CGGCAATGTA CAAGGATTTC TATTTTTCCA TATCCTTGCA CTTACCAACA 420

CTTCTTTTTK GTWATWATTT TGTTTTTTCA TTATTGCCAC CCTAGTGGAT GTGAAATGGC 480

ATCTTATTGT TTTGATTTGC ATTTCTCTAA TGACAAATGA TATCATACTT TTTTTATGTG 540

CTTACGGATC AAAGGTATTT CCTTGGAGAA ATGTCCCTTC AAGTCCTTTG CCATTTCAAA 600

ATTTGGTTAT TTGTCTTTTA TTATTCAGTT TTAAGAAATT CTGGCCAGGC GCAGTGGCTC 660

ACCTGTAATC MTAGCACTTT GGGAGGCCAA GGCGGGCAGA TCACTTGAGK TCAGGACTTC 720

GAGACCAGCC TGGCCAACAT GGTGAAACCC CATCTTACTA AAAATACAAA AATTAGCTGG 780

GCGTGGTGGC AGGTGCATGT AATCNTATCT ACTCAGGAGG CTGAGGCAGG AGAATCGCTT 840

GAACCCAGGA GGCGGAGGCT GCAGTGAGCC AAGATCACGC CATTGCACTC TAGCCTGGGT 900

GACACAGA 908

(2) INFORMATION FOR SEQ ID NO: 88:

(i) SEQUENCE CHARACTERISTICS:
(A) LENGTH: 655 base pairs
(B) TYPE: nucleic acid
(C) STRANDEDNESS: double
(D) TOPOLOGY: linear

(xi) SEQUENCE DESCRIPTION: SEQ ID NO: 88:

TGCACTGGTT CCTTCTCCCC AGCAAATACT GCCTTCTTGT TTTTCTCTGA TGTGGCAGGT 60

GACTACAAAA TCCGCCTTGG TATTCTTCAA ATGCATATAT ATTCCTTTCT TGTCAGCTCC 120

CTCTCTTCCT AGATTAGAAA ACTGCCTCAT TTTCTGCTCA CTGGATGTGC AGTCCCAGCT 180

TGTCTTCCTC TCCTCCCCCC CTGTTGCAGG TGTTCTTTTT TTTTTTCTTC TCTCCCCACT 240

GGGCAGCAAA AGTTGTTCCA CAGTGGAAAW TTAGGCATCC TCAAGTTTCY TCCCAGCTTC 300

TGCTGTGTTT TCTTAGAGTA AATTGCCAAT TTCTGTTTTT ACAGGAAATC CTTTTTTAAA 360

AATGGAATCA GTGTGGTCCC CATCTACTCT GCAAAAATTG CATTTTTCTC TATTTTCAAA 420

TGAGATTTGT TCAAGTTTCA AAACCACGTG AAATAATAAA TGTATAGTAG TTTTCTTTTC 480

CTTGGGCATT GCTWGATATG TGAAATGGGT TTATGAAAAA TAATAAAATC ATAACGCTAT 540

TTGTTTGACT TTCAATTTCA TGGGAATTTT TCTCAGCTAA ACTCTAAATG GTGATTARGC 600

AAAAAAAAAA AAAAAAAACY GRAGGGGGGC CCGGTACCAA TTCGCCCTAT AATGA 655

(2) INFORMATION FOR SEQ ID NO: 89:

(i) SEQUENCE CHARACTERISTICS:
(A) LENGTH: 1102 base pairs
(B) TYPE: nucleic acid
(C) STRANDEDNESS: double
(D) TOPOLOGY: linear

(xi) SEQUENCE DESCRIPTION: SEQ ID NO: 89:

TTTTTTTTTT ACCATTTAAA ATAAAATGAA AGTGACCTTC TGTTTATAAA AATCTTTGTC 60

TGCATCTCTG CTTATTTCCT TAGAAGAGAT TCCAAGAAGC GGTGAGTGAT TTCACGGCAG 120

CAGAGGGTTG GGACATATTA CGGGCGCGGA TCCCTCTTGG AGTGAGATGA CTCTCCGGAG 180

AGATTTAGTC GTCACCCTCG CGTGTGAGGC TGCGTCACAC CCCAGGGATG TGTCTATCAA 240

GATGGAAGAT CTTTTACACG CTCTTGATTT TGTTTGSCTY TTTTTCTATT ACTAGTGAGA 300

AKGAAACTTT TTATATGATT ATTATCCATC ATAATCCAAC ACAAATTACT GCTTCATGTT 360

CTTTTACTTT CCTGTGAAGG TTTTAGTGCC TTTTAAAAAT TGCTATATAT TAAGCTTGTT 420

AATACTTCCA TGCTGTATTT GTGGSCATCA RTTTCCCCGG GNACAGGCNT GCACATTTTG 480

CCTTCACACG CTGGGTGGTT TTTCATTTTC AMTTCTATTT CTCGTTCTTC TATCGTTTTA 540

TGTTCAGACG GGTTTCTCCG TGTAGAAAGC AGTTTATGAA GATTTACTTT CGACAGTCTT 600

CTCTCTACTT TCTACAGTGA ATTCTCTGAT GTGTCTGGGA GTTTGGGGGT CTGGGTAAGA 660

RTCCTCCTCT CACCCTATTC TCTATTACGA TCCACAGCCT CATGCTTTAT GARATTGGTG 720

GCCGGGARCG GGGGAGATTT GCGGATCCCC CAAGCCAGAC TTTATCCCCC TATCCCTGCC 780

TCTGGATCCC ACGTACAGGC CTGGGAACTC CCTGTGGGTA GGGGCCAATG GTCTCGCACT 840

CTCACCTGTA CCCCAGGGCT GGCACAGGAT GGTCAAGGAG AGAGGCTGCC CAAGCGCATC 900

CYTCTGGTGT CCCCCTGACA CGCCTCCAAA GTGAGCAGGT AGGTTTCAAC AGCCCCACGT 960

TGCAGGTGGG AGATGAAGCT CAGGGTGGAG ACCAGTATCT CACAGTTCTC TTTGCATGGC 1020

CGGGTACTTG TTAGTCAACT GATCAAGTGA AAATTCTAGC CCCAGAGGCA GGAGAATCCG 1080

GAACAAAATT AAACCAGCCA GG 1102

(2) INFORMATION FOR SEQ ID NO: 90:

(i) SEQUENCE CHARACTERISTICS:
(A) LENGTH: 1533 base pairs

(B) TYPE: nucleic acid
(C) STRANDEDNESS: double
(D) TOPOLOGY: linear

(xi) SEQUENCE DESCRIPTION: SEQ ID NO: 90:

GGCACGAGCC GNCACGGGCA GCGCCCCATA GCGCCAGGGA CCCCCTGGCA GCGGGAGCCG 60

CGGGTCGAGG TTATGGATCC AGCGGGCGGC CCCCGGGGCG TGCTCCCGCG GCCCTGCCGG 120

TGNCTGGTGC TGCTGAACCC GCGCGGCGGC AAGGGCAAGG CCTTGCAGCT CTTCCGGAGT 180

CACGTGCAGC CCCTTTTGGC TGAGGCTGAA ATCTCCTTCA CGCTGATGCT CACTGAGCGG 240

CGGAACCACG CGCGGGARCT GGTGCGGTCG GAGGAGCTGG GCCGCTGGRA CGCTCTGGTG 300

GTCATGTYTG GAGACGGGCT GATGCACGAG GTGGTGAACG GGCTTCATGG AGCGGCCTGA 360

CTGGGAGACC GCCATCCAGA AGCCCCTGTG TAGCCTCCCA GCAGGCTCTG GCAACGCSCT 420

GGCAGCTTCC TTRAACCATT ATGCTGGCTA TRAGCAGGTC ACCAATGAAG ACCTCCTGAC 480

CAACTGCACG CTATTGCTGT GCCGCCGGCT GCTGTCACCC ATGAACCTGC TGTCTCTGCA 540

CACGGCTTCG GGGCTGCGCC TCTTCTCTGT GCTCAGCCTG GCCTGGGGCT TCATTGCTGA 600

TGTGGACCTA GAGAGTGAGA AGTATCGGCG TCTGGGGGAG ATGCGCTTCA CTCTGGGCAC 660

CTTCCTGCGT CTGGCAGCCC TGCGCACCTA CCGCGGCCGA CTGGCCTACC TCCCTGTAGG 720

AAGAGTGGGT TCCAAGACAC CTGCCTCCCC CGTTGTGGTC CAGCAGGGCC CGGTAGATGC 780

ACACCTTGTG CCACTGGAGG AGCCAGTGCC CTCTCACTGG ACAGTGGTGC CCGACGAGGA 840

CTTTGTGCTA GTCCTGGCAC TGCTGCACTC GCACCTGGGC AGTGAGATGT TTGCTGCACC 900

CATGGGCCGC TGTGCAGCTG GCGTCATGCA TCTGTTCTAC GTGCGGGCGG GAGTGTCTCG 960

TGCCATGCTG CTGCGCCTCT TCCTGGCCAT GGAGAAGGGC AGGCATATGG AGTATGAATG 1020

CCCCTACTTG GTATATGTGC CCGTGGTCGC CTTCCGCTTG GAGCCCAAGG ATGGGAAAGG 1080

TGTGTTTGCA GTGGATGGGG AATTGATGGT TAGCGAGGCC GTGCAGGGCC AGGTGCACCC 1140

AAACTACTTC TGGATGGTCA GCGGTTGCGT GGAGCCCCCG CCCAGCTGGA AGCCCCAGCA 1200

GATGCCACCG CCAGAAGAGC CCTTATGACC CCTGGGCCGC GCTGTGCCTT AGTGTCTACT 1260

TGCAGGACCC TTCCTCCTTC CCTAGGGCTG CAGGGCCTGT CCACAGCTCC TGTGGGGGTG 1320

GAGGAGACTC CTCTGGAGAA GGGTGAGAAG GTGGAGGCTA TGCTTTGGGG GGACAGGCCA 1380

GAATGAAGTC CTGGGTCAGG AGCCCAGCTG GCTGGGCCCA GCTGCCTATG TAAGGCCTTC 1440

TAGTTTGTTC TGAGACCCCC ACCCCACGAA CCAAATCCAA ATAAAGTGAC ATTCCCAAAA 1500

AAAAAAAAAA AAAAAAAAAA ANCCCGNGGG GGG 1533

(2) INFORMATION FOR SEQ ID NO: 91:

(i) SEQUENCE CHARACTERISTICS:
(A) LENGTH: 575 base pairs
(B) TYPE: nucleic acid
(C) STRANDEDNESS: double
(D) TOPOLOGY: linear

(xi) SEQUENCE DESCRIPTION: SEQ ID NO: 91:

ATCCTCTGGA ATCTAGGTGG AAGCCACCAA GCCTTCTTCA CACTTGCGTT CTGAGCATCT 60

GCAGACTTAA CCCCATGTGG CAATCACCAA GGCTTATGGC TTGTGTCCTC CAGAACTGTG 120

GCCAGAGCTG TACCTGGGCC CCTTTGAGCT GAGGCTGAAG CCAGAGTCTG AAGCTCAGCA 180

GGGCAGTARG GCCCTGGGCC TGGCCCCTGA AACCATTCTT TTCTCCTAAG CCTCTGGGCC 240

TTTGATGGGA RGGGCTGTCC TCAAGATTTT TGAAATGCCT TTGGAGGGTT TTTGCCTTGT 300

CTTGGATATT GGCTTCCTTT TAGTTATGCT CATCTCTCTA GCAAGTGAAT GTTTCACAAC 360

CTGCTTGGAT TCTTTCTCTA CCACAGARCC AGGCTGCAAA TTTTACAAAC TTTTACACTC 420

TGTTTCCCTT TTAAATATAA ATTTCAATGT TAAGTCACTT CTTTGCTCCC ATATCTGATT 480

TAGGTTGCTG GAAGTAGCCA AGTCACCTCT TGAATGCTTT GCTGCTTAGA AATTTCCTCT 540

ACTAGGTAGC CTGGGTCATC ACACTTAAGT TCAAA 575

(2) INFORMATION FOR SEQ ID NO: 92:

(i) SEQUENCE CHARACTERISTICS:
(A) LENGTH: 639 base pairs
(B) TYPE: nucleic acid
(C) STRANDEDNESS: double
(D) TOPOLOGY: linear

(xi) SEQUENCE DESCRIPTION: SEQ ID NO: 92:

TCCTTTCATC TTAAGCACCA CCCGACAGGG CAGGTACTAT TACCATCTCC GTTTGACAGA 60

TNAGGAACCT GGCACAGGAA GCATTTAAGT GGATTCCCCA GGATCGCCCC ACTGTCAGGA 120

GCAGANTCAG AATGGGCCTC AGCATCAGGC TCCCAATCCT GGCTTCTAAC TGCTGCGCTC 180

TGCCCTTCYC TCWCCCCACC TCCCCACTCC AGTGCCTTTG GTCATGCCAC TGCAGCTTTC 240

AGGCCAATAC TGGATTAGCC TCTTAGTGTT CTTGTCCCTG CAGCCATTTC CCCAGGCAGC 300

AATTCCATGT GCCCTCACTG ATGTAGGTGG CTCTTGTGTC ATTTGTCACA TCCTATTGAA 360

TTGTTTATGC ATCTTGTTCA CACTCACAGC ACCCTCCCTC TCACACGTCC TCCTTATAAA 420

AATGTCCCTC AGTGTCTGCT ATGAGCCAGG TGCAGACTTA AGTGACAGGG CTGCTACGGG 480

AAATAAAAAA TTAACAAGGA GCACCTGCCT CTTAATGCAC AGTAACAAAC TATGTTAAGT 540

GTCAGGAAGG AAAGGTTAAG GATGCCAGGA AGGCTTTTAA TAAATAACCT GACTTAGATG 600

GGCAGGTGGT GCTGARGATT AAGAACGTGT TCTTCTCGA 639

(2) INFORMATION FOR SEQ ID NO: 93:

(i) SEQUENCE CHARACTERISTICS:
(A) LENGTH: 744 base pairs
(B) TYPE: nucleic acid
(C) STRANDEDNESS: double
(D) TOPOLOGY: linear

(xi) SEQUENCE DESCRIPTION: SEQ ID NO: 93:

GAATTCGGCA CGAGAGTGGC TGGAGTCTGG CTGCAGAGGG AAGACATCAG CAGGGAGGGA 60

GCCAGGGCCT GTCACATCTT TCCTCTGGCC ATTGTCCTGG TCTTTGTAAG CCCAGAATCT 120

CCCCTTCCCT GAAGGGAGGC CAGCACCCCA GGAGGGCAGC AGGTGTGCTG TGAGGGTTGG 180

AGTAGTGTGA GAGGTCAGGG TACACTAGAA TGGCCATGGA CACCATGTGG GGGTGCTCTG 240

GGCTGGGCCA CAGAACAGTG TCCTTCCTGC TGCTCCTCCC CTGCAGCTTC CCCCGACCTT 300

GTNGTTTATT TGGTTTGATA CCAATCAGCA GACCCTGCAA GGTGGAAGCT CCCAGGCTCT 360

CAGTCCCACS ACTCTCATGT GCCAGTCACC CNTACTGTAA CTGCCCAATG AGTACTTCTT 420

GCCCACTGCC AAGATAGAGC CAGTTTACCA AGACAGGGGA ATTGCAGTAG AGAAAGAGTT 480

GAATATACAT AGAGCCAGCT AAATGGGAGA GTGGAGTTTT CTTATTACTT AAATCAGCCT 540

CCCYTAAAAT TCAGAGGTGA GAATTTTTCA AGGACAGTTT GGTGGSCAGG CCTAGGGAAT 600

GGATGCTGCT GATTGGCTAG GGATGCAATC ATAGGGGTGT AGAAAAGTWC CTTGTGCACT 660

GAGTCCACTT TTGGTGAGAG CTACCAAGGA GCTGCTGGTC TGCTGGTCCC GGTAGAGCCA 720

TCTGGTGTCA GGAATGCAAA AGTG 744

(2) INFORMATION FOR SEQ ID NO: 94:

(i) SEQUENCE CHARACTERISTICS:
(A) LENGTH: 526 base pairs
(B) TYPE: nucleic acid
(C) STRANDEDNESS: double
(D) TOPOLOGY: linear

(xi) SEQUENCE DESCRIPTION: SEQ ID NO: 94:

GCAGGGGAAT TCGGCCACGG AGGGGTTTCA ACAGGGCCCG TGGGGTGAGG TGCARACACA 60

AAGCCCATAA GTGCTGGCCT GTTGGGACAA ATGAGAGAAA TCCCATAGGG TGGTGATGAC 120

AGCGCAYTCA GCCATCYTAY TCCTGGGGAA AATGAAACTT GTGCTCCTAT CAAATGCTCA 180

GTTGTAAAAC TGGAAAAAAA TTTTAGAAGA CATCTTGTCC AGCATCTGTG TTTATGTCTA 240

TAAAATGTAG AAAACTAAAG CACAGAGATG TTAAATGTTT TGTCCAAGGT CCAACAGCTG 300

GTTAGCARGC TTGGTCTGGT GACCTTTCTA CTGAACCACA GTGCCGCTGG GGGAAGTCCT 360

CAGCACAGAT GGCTGCTGCT ATAGCTGGGG TATGGGCAGT ATTAGTAGTT AACCAGTCAA 420

CCCAAGTTCC CATAGTCTAG GTTCTGCTTC AGCTGGAGGT TAGGGAAAAA CACAAGAAAA 480

TCCCTTACCA CTCTACCAGT GCTGGGGGAT GTACTAAGAG ATCCCC 526

(2) INFORMATION FOR SEQ ID NO: 95:

(i) SEQUENCE CHARACTERISTICS:
(A) LENGTH: 426 base pairs
(B) TYPE: nucleic acid
(C) STRANDEDNESS: double
(D) TOPOLOGY: linear

(xi) SEQUENCE DESCRIPTION: SEQ ID NO: 95:

GGCACAGGGC AGGAGAGACT TGGTCCATGG GGAGAAGCCT GCAGTATAGA TGGGACCTCC 60

AGGAGCCCAA GTAGCATAGA CCCTGCTGAT CCGGGGCCAT TGAGCCAGAG GATTTGGGCT 120

GAATGTCCCC AGAGACAAAA GGGAAAGGTA GATCCTTTCC CTTAAAGATG AAAGCCATCG 180

CCCGGGCTTG CTTATTGCTC TCTCTCCTGG TCCTTCCACA TGTTGTTTCT GAACATTTGT 240

TCTGGCATCA CAATCCCCGT CATCCTGTCA TCTGGCCCTT CCCACCTTTC CACCTTATCT 300

CTTGCAGTGT CTCCGCGTCG ACCTGGCACC TGGGTGAARG CTTGCTCTTG CTGGTGCCCA 360

TAGCCCCCAG TGTATGGTCT TGAMCTCCCC AGCCATATGG ARACCCACCT CAGGAGGGCC 420

CCTCGA 426

(2) INFORMATION FOR SEQ ID NO: 96:

(i) SEQUENCE CHARACTERISTICS:
(A) LENGTH: 844 base pairs
(B) TYPE: nucleic acid
(C) STRANDEDNESS: double
(D) TOPOLOGY: linear

(xi) SEQUENCE DESCRIPTION: SEQ ID NO: 96:

GGCACAGCGG CACGAGATAG GAAGCTTGGC AGGGGCAGCT CCCCCAGTGC GCATTGCCCT 60

GTAACTCGAG CGCCTGGGAG TGGGGAGAGG CTTGGAAATG GAGCAGGGTG GTGGACCTCG 120

TCTTCTCCTG CTCATCCCAG GCCTCCTCCA TAACACCTAC CTAGCACGGC CTGGGGACTT 180

CCCAGCCCAA GGAACAACTG AGAATACTGA GTGCCAGGGT AGCCCTAGCC CCATTTCACA 240

CCTGGGCAAA GTGAGGTCAC TGGATTCAAA CACTCAGATT TAAACCTCCT CTGTGTCTGC 300

AGCACCTGTA TATAACTGCC AGCCTCTGCT GCCCCTCTCC AAAAAGTCTC TGCCCTTGTC 360

TTTGGCACCT GTCTCTGTCC TCCCCATTCT CTGCTCCTCC TTTCTCCAAC TCAGANTCAC 420

CCTGTTAGTT CAGCAAATGT TCATCGAGCT CCATAATGTA GCAGGACAGG NCTGTCTAAC 480

AGATTCTGGN CTTGCAAGGG TGAGACAAGT ACTCTCCATC TTTCTCTCAT CTTCACAGAT 540

GGTCTGCTCA ACAACTTTGC ACTGAATTGT AAATAATTGA TACTGCATAA AACATTGATG 600

TTCTTTAAGG GTAGTCCAGC AAGGTGGCAA GTCTTATAAT GATAACTGCT CAAGGATCTC 660

TCAGTGAAGC ATTTGGGGST GCTAGCTCTG CCTATGGGTG AGGTCAGCTA TCTCACGCCA 720

TCTACTTCCA CNTGCCCCCC CATGCCAGGC TCACCCTGAG CTGAGATGCC TGAGCAGGTG 780

GCAGAAAGGA GCCACCTGGT TTATGCTTCG GGACCACAAA CTCCTCTATC CAGANGACAG 840

TTTT 844

(2) INFORMATION FOR SEQ ID NO: 97:

(i) SEQUENCE CHARACTERISTICS:
(A) LENGTH: 1985 base pairs
(B) TYPE: nucleic acid
(C) STRANDEDNESS: double
(D) TOPOLOGY: linear

(xi) SEQUENCE DESCRIPTION: SEQ ID NO: 97:

AGCCCTGCTG AAGTACAGGT TCTTCTATCA GTTTCTGTTG GGCAATGAAC GAGCAACAGC 60

AAAGGAGATC AGGGATGAAT ATGTGGAGAC GCTGAGCAAG ATTTACCTGT CTTACTACCG 120

CTCTTACCTG GGGCGGCTCA TGAAGGTGCA GTATGAGGAA GTCGCTGAGA AAGATGATCT 180

AATGGGTGTG GAAGATACAG CAAAGAAAGG ATTCTYCTCA AAGCCATCGC TCCGCAGCAG 240

GAACACCATT TTCACCCTAG GAACCCGCGG CTCTGTCATC TCCCCCACTG AACTTGAGGC 300

CCCCATCCTG GTGCCTCACA CAGCGCAGCG GNAGAGCAGA GGTATCCATT TGAGGCCCTC 360

TTCCGCAGCC AGCACTACGS CCTCCTAGAC AATTCCTGCC GCGAATACCT TTTCATCTGT 420

GAATTTTTTG TTGTGTCTGG CCCAGYTGCA CACGACCTGT TCCATGCTGT CATGGGCCGT 480

ACACTCAGCA TGACCCTGAA ACACCTGGAT TCTTATCTAG CTGACTGCTA CGATGCCATT 540

GCTGTTTTTC TCTGTATCCA CATTGTTCTC CGGTTCCGTA ACATTGCAGC AAAGAGGGAT 600

GTTCCTGCCC TGGACAGGTA CTGGGGAACA GGTGCTTGCC TTGCTATGGC CACGGTTTGA 660

ACTGATCCTG GAGATGAATG TTCAGAGCGT CCGAAGCACT GACCCCCAGC GCCTAGGGGG 720

GTTGGATACT CGGCCCCACT ATATCACACG CCGCTATGCA GAGTTCTCCT CCGCTCTTGT 780

CAGTATCAAC CAGACAATTC CTAATGAACG GACCATGCAA TTGCTGGGAC AGCTGCAGGT 840

GGAGGTGGAG AATTTTGTCC TCCGAGTGGC AGCTGAGTTC TCCTCAAGGA AGGAGCAGCT 900

TGTGTTTCTG ATCAACAACT ATGACATGAT GCTGGGTGTG CTGATGGAGC GGGCTGCAGA 960

TGACAGCAAA GAGGTTGAGA GCTTCCAGCA GCTGCTCAAT GCTCGGACAC AGGAATTCAT 1020

TGAAGAGTTG CTGTCTCCCC CTTTTGGGGG TTTAGTGGCA TTTGTGAAGG AGGCTGAGGC 1080

TTTGATTGAG CGTGGACAGG CTGAGCGACT TCGAGGGGAA GAAGCCCGGG TAACTCAGCT 1140

GATCCGTGGC TTTGGTAGTT CCTGGAAATC ATCAGTGGAA TCTCTGAGTC AGGATGTAAT 1200

GCGGAGTTTC ACCAACTTCA GAAATGGCAC CAGTATCATT CAGGGAGCGC TGACCCAGCT 1260

GATCCAGCTC TATCATCGCT TCCACCGGGT GCTGTCCCAG CCGCAGCTCC GAGCCCTCCC 1320

TGCCCGGGCT GAGCTCATCA ACATTCACCA CCTTATGGTG GAGCTCAAGA AGCATAAGCC 1380

CAACTTCTGA TGTGCCAGAA ACCGCCCTGA GATCTGCCGG TCATCTCCAT GGACTTCTGC 1440

ACCCCATTCC ATACCCTTCT TCACCTGGGG TACCCCTTCC AGTTTTCCCC TTGCTTCCCA 1500

GGCCCTTGAC ATGGCTTACC TGCCTTCACT CCCAGCACCT TGCCCAACAG GATAAGCTGG 1560

ATCCCCTTGG CCTTCTGAAT ATCCCAGTGT CTTCAGGTTT CCCAAGACCA CTTCCCTGTG 1620

GGCTTCCAAA ATGGCCTTTA TCATTTCTCC AGTCTGTCAC CCTCCTTTCC TGCTCCCATA 1680

CACCCAAGGC TTGTTTCTTC CCCTGTAAAA ACCACTGCCT CAATCTCTGG TTCACTCAAC 1740

TAGTCACCAT GTCCTGAGGC ATGAAGCCTC CTCAGCTCTT GGAATTGCTG GCAAGGGGTG 1800

ACTGCCTCTG AGTCATTGTG TTTTTCAAAG TGATTTCTTT TCTGTAGCTT TTTGACCTAA 1860

GATCTCAGCA ATTTGAACAC TAACCTCTCC CCTCCTGGCT CAAGAATTAC TCCGAAGTCA 1920

GTCTGCAGAA AATAAATATT TAGTATGACA TGAAAAAAAA AAAAAAAAAA AAAAAAAAAA 1980

AAAAA 1985

(2) INFORMATION FOR SEQ ID NO: 98:

(i) SEQUENCE CHARACTERISTICS:
(A) LENGTH: 1416 base pairs
(B) TYPE: nucleic acid
(C) STRANDEDNESS: double
(D) TOPOLOGY: linear

(xi) SEQUENCE DESCRIPTION: SEQ ID NO: 98:

ATATGAAGGG AAAGAATTTG ATTATGTTTT CTCAATTGAT GTCAATGAAG GTGGACCATC 60

ATATAAATTG CCATATAATA CCAGTGATGA CCCTTGGTTA ACTGCATACA ACTTCTTACA 120

GAAGAATGAT TTGAATCCTA TGTTTCTGGA TCAAGTAGCT AAATTTATTA TTGATAACAC 180

AAAAGGTCAA ATGTTGGGAC TTGGGAATCC CAGCTTTTCA GATCCATTTA CAGGTGGTGG 240

TCGGTATGTT CCGGGCTCTT CGGGATCTTC TAACACACTA CCCACAGCAG ATCCTTTTAC 300

AGGTGCTGGT CGTTATGTAC CAGGTTCTGC AAGTATGGGA ACTACCATGG CCGGAGTTGA 360

TCCATTTACA GGGAATAGTG CCTACCGATC AGCTGCATCT AAAACAATGA ATATTTATTT 420

CCCTAAAAAA GAGGCTGTCA CATTTGACCA AGCAAACCCT ACACAAATAT TAGGTAAACT 480

GAAGGAACTT AATGGAACTG CACCTGAAGA GAAGAAGTTA ACTGAGGATG ACTTGATACT 540

TCTTGAGAAG ATACTGTCTC TAATATGTAA TAGTTCTTCA GAAAAACCCA CAGTCCAGCA 600

ACTTCAGATT TTGTGGAAAG CTATTAACTG TCCTGAAGAT ATTGTCTTTC CTGCACTTGA 660

CATTCTTCGG TTGTCAATTA AACACCCCAG TGTGAATGAG AACTTCTGCA ATGAAAAGGA 720

AGGGGCTCAG TTCAGCAGTC ATCTTATCAA TCTTCTGAAC CCTAAAGGAA AGCCAGCAAA 780

CCAGCTGCTT GCTCTCAGGA CTTTTTGCAA TTGTTTTGTT GGCCAGGCAG GACAAAAACT 840

CATGATGTCC CAGAGGGAAT CACTGATGTC CCATGCAATA GAACTGAAAT CAGGGAGCAA 900

TAAGAACATT CACATTGCTC TGGCTACATT GGCCCTGAAC TATTCTGTTT GTTTTCATAA 960

AGACCATAAC ATTGAAGGGA AAGCCCAATG TTTGTCACTA ATTAGCACAA TCTTGGAAGT 1020

AGTACAAGAC CTAGAAGCCA CTTTTAGACT TCTTGTGGCT CTTGGAACAC TTATCAGTGA 1080

TGATTCAAAT GCTGTACAAT TAGCCAAGTC TTTAGGTGTT GATTCTCAAA TAAAAAAGTA 1140

TTCCTCAGTA TCAGAACCAG CTAAAGTAAG TGAATGCTGT AGATTTATCC TAAATTTGCT 1200

GTAGCAGTGG GGAAGAGGGA CGGATATTTT TAATTGATTA GTGTTTTTTT CCTCACATTT 1260

GACATGACTG ATAACAGATA ATTAAAAAAA GAGAATACGG TGGATTAAGT AAAATTTTAC 1320

ATCTTGTAAA GTGGTGGGGA GGGGAAACAG AAATAAAATT TTTGCACTGC TGAAAAAAAA 1380

AAAAAAAAAA AAAAGGAAAC TCGAGGGGGG GCCCGG 1416

(2) INFORMATION FOR SEQ ID NO: 99:

(i) SEQUENCE CHARACTERISTICS:
(A) LENGTH: 1935 base pairs
(B) TYPE: nucleic acid
(C) STRANDEDNESS: double
(D) TOPOLOGY: linear

(xi) SEQUENCE DESCRIPTION: SEQ ID NO: 99:

NTCTACCCTA ATCAAGATGG GGACATACTT CGCGACCAGG TTCTTCATGA ACATATCCAG 60

AGATTGTCTA AAGTAGTGAC TGCAAATCAC AGAGCTCTTC AGATACCAGA GGTTTATCTT 120

CGAGAAGCAC CATGGCCATC TGCACAATCA GAAATCAGGA CAATAAGTGC TTATAAAACC 180

CCCCGGGACA AAGTGCAGTG CATCCTGAGA ATGTGCTCTA CGATTATGAA CCTCCTGAGC 240

CTGGCCAATG AGGACTCTGT CCCTGGAGCG GATGACTTTG TTCCTGTGTT GGTGTTTGTG 300

TTGATAAAGG CAAATCCACC CTGTTTGCTG TCTACTGTGC AGTATATCAG TAGCTTTTAT 360

GCTAGCTGTC TGTCTGGAGA GGAGTCCTAT TGGTGGATGC AGTTCACAGC AGCAGTAGAA 420

TTCATTAAAA CCATCGATGA CCGAAAGTGA CCAAGACCAA GGCCCACCAA GGCAGCAGAC 480

TGTTAATCAG ACAAACAGAT CTCTGAGAAG GTGCATCAGC TGCTTTGAAG GCTGAAGATT 540

GTTTTGTATG ATACTGCACA GCATCAGGCA TTTTAAAGCA GATCTTTACT AAACAGGTTA 600

ATGAGCTAAC AAGCAGGTTC TCTCGTCTTT GGGCTCTTTC CTTTCTGAGT TGCATATTCT 660

ATTTTCTTGT CCCCAAGTAG AGACTAGTAC TACAAAAAGG GACCACATTT TTCAAGTATT 720

TCTAAGTATA AAAAACAAAA CAAAAATCTC TTAGGAAATG TCTAGACCTC CATTCTTGGA 780

TTCCCTTTCT TTCCTTTTAT TTTAAAAAAG AACAGTACCC CTCTTTTAAG ATGCTGTCTT 840

ACATTAATGA GCATCTAATG GAAAGAAGGT ATGAGTTGCA CTGAGGATTA GAATAGTGGT 900

GCGTTAGTGG CATTATCTAT AAATACACTC ACCTAAATTG AAAGCTAAGA AGGAAATGTA 960

AATATAATAT ATATTTATAT TTGATGTAAT ATGGACATCT GCAGATTCTA ATAAACAAGG 1020

ACTATTGCTG ATAGTAGGCT GTGACATACT GTCTTGTGAA ATGGTTTCCT TGACAAAATT 1080

TAAGCTGAGC TTAAAAGCAA AAAAACAAAA AGTACACAGA AATATTTATT AAAATGTAAT 1140

ACAGTTTATT GAACTTTCTA GGTATGGAGT TTGATGGACA GGGCTGCCTY TAATGAGTGT 1200

GAAGGTCACT AAGTCACTTA GACATCTCAC CGTGGAAGTT TGTGAGCCTG CATTAGGAGA 1260

TAGACTGATT ACCATACATG ACATAAAAAG GAACAGTGGA TAGCTCATAC TTTATGGTGG 1320

TTCTTCTCCT CCGAAATAAT ATACTGCAGA AATCCCAGAC AGAGCTCCTT ACAAACCTTT 1380

AATTGTAATA TATTTTTGAT GATTATTCAC ATTGAATGCA CAGACCAAGA ATTCAGTGAA 1440

TGTCATTTTT TAAAAAACTA ATTTGTATTG TCTGCTCTAG TGATACAAGT TTTACTAGTG 1500

ATAAACTATT TTAATCAACC ATACTATTCT TATGGAAAAA AATATCTATT TTGGCAGGTT 1560

TCTGTGCCTT TATTTCCCTC TTCTGAAAAA AAGTCTGTGT TTTCATAGTT TGGTTTGCAT 1620

TGTATATCAA TAATTAATCA GGAATGGGTT TTGGTGCCTG AAAAATTGGC CATGGAGGCA 1680

CACCAAAGCT TCAAGCACAA GTCTTGTACA TGGGCCATCA CTGTCTGGTT TCACTTCGTG 1740

TGTTTCCTAA ACACATTTAG CTGCTTTTTT AACAAACTCA GCCCCATACT TGAGTCCCTT 1800

GTTGTTGGGA GCATTTCCAG GCATCTTTTA AGGGAACTGT GACAAACAGC CTCGGGCAGA 1860

TGAACACGGA GGCTCTCTGT TGTCTGTCTC TGAGATCTTT GTGTCTGGGA ATGCCTAAAG 1920

NTTTTGNTTT TTTTT 1935

(2) INFORMATION FOR SEQ ID NO: 100:

(i) SEQUENCE CHARACTERISTICS:
(A) LENGTH: 599 base pairs
(B) TYPE: nucleic acid
(C) STRANDEDNESS: double
(D) TOPOLOGY: linear

(xi) SEQUENCE DESCRIPTION: SEQ ID NO: 100:

GAATTCGGCA CGAGCGTCCA CGCAGCCGCC GGCCGGCCAG CACCCAGGGC CCTGCATGCC 60

AGGTCGTTGG AGGTGGCAGC GAGACATGCA CCCGGCCCGG AAGCTCCTCA GCCTCCTCTT 120

CCTCATCCTG ATGGGCACTG AACTCACTCA AGACTCCGCT GCCCCCGACT CCCTGCTGAG 180

AAGTTCAAAG GGCAGCACGA GGGGGTCTTT GGCTGCTATT GTCATCTGGA GGGGGAAGAG 240

TGAGAGCCGG ATAGCCAAGA CCCCAGGCAT TTTCAGAGGT GGCGGGACCT TAGTCCTACC 300

CCCAACACAC ACCCCTGAGT GGCTCATCCT CCCTTTGGGC ATAACGCTGC CCTTGGGGGC 360

TCCAGAAACA GGCGGTGGGG ATTGTGCCGC TGAGACCTGG AAGGGCAGCC AGCGTGCCGG 420

CCAGCTGTGT GCATTGCTGG CTTAATATGC AGGGCTTGGG GGGCTGTGGC CACATGCCCG 480

GCAGGAGGTG AGTGAGGAGC CCTGTGGCGT GCTGGTGTGG GGATCGTGGG CATTTCAAAC 540

GGGCTTGTCG TACCCTGAAC AATGTATCAA TAGAGAAAAA AAAAAAAAAA AAAACTCGA 599

(2) INFORMATION FOR SEQ ID NO: 101:

(i) SEQUENCE CHARACTERISTICS:
(A) LENGTH: 784 base pairs
(B) TYPE: nucleic acid
(C) STRANDEDNESS: double
(D) TOPOLOGY: linear

(xi) SEQUENCE DESCRIPTION: SEQ ID NO: 101:

GAATTCGGCA CAGAAAAAAA AGAGAGACTG GGTCTTACTG TGTTGCCCAG ACTTGTCTTG 60

AACTCCTGCC TCAGCCTCTC AAGTACTTGG GATTATAGGC CAAGAAGCCA CCATGCCTAG 120

CTTCTTCCTG TCATTGATCC AGACTAATAC TCTGGGGTCA GCCTCATTTC TTCTCTTTCT 180

CACTTTGCAC ATCCACTTGT CACCAAATCK RGTTCATTCT GCATCCTAAG TAAGTCCTTT 240

GATTCCTCCA GTTGTTCATT AGTAATGTCT CAARTGTAAT TTTTTCTAGT AGTTTTCAGC 300

CTGTCTTTCC KGCCTTCAGT CTTAACTTCT CCAGTACATA KGCCACATTG TTGTCAGCAK 360

GATCAWATTT TATTTAAAAA TACTTTACAW AKGTTTATKG CCAAATATTA GRAAATACAG 420

ATTCATGGAA AGAAAAATCA CTGTCCCAAG GAGGTCACTG GCATGGTGAG GTTAAGGGGT 480

GATTTTAATT TTTAAAAATG TATATTTTTT CCTGTGTAGA GTAGTAACAC CCTTGAAAAC 540

ACAWTCCCTT GTAAAGTCTC TAATTCTGTA CTCCGCATCT AGSTGRTCTC TTCTTTCTCA 600

GATATTTTAC AATTTCATTT ATCACCACCT TTCTCTAGCC TTTACCCGTC TCTTCAATAT 660

TWACATATGC AGAAGTTTCT CCTAACAAAC ACCTGCCTCT GCCTCAGTTC TGCTACCACC 720

CTGTTGCTTT CTTTCCCTTC ACAATCAAAT TTAAGAGTGT CAAAAAAAAA AAAAAAAAAC 780

TCGA 784

(2) INFORMATION FOR SEQ ID NO: 102:

(i) SEQUENCE CHARACTERISTICS:
(A) LENGTH: 1035 base pairs
(B) TYPE: nucleic acid
(C) STRANDEDNESS: double
(D) TOPOLOGY: linear

(xi) SEQUENCE DESCRIPTION: SEQ ID NO: 102:

AGAGGCCTGG CTGCGTTGCC CTATCTCCGT CTCCGCCACC CACTTAGCGT TTTAGGCATC 60

AATTACCAGC AGTTTCTCCG CCACTATCTG GAAAATTACC CGATTGCTCC CGGCAGAATA 120

CAAGAGCTTG AAGAACGCCG CAGTTGCGTG GAAGCCTGCA GAGCAAGGGA AGCAGCGTTT 180

GATGCCGAAT ATCAGCGAAA TCCTCACAGG GTGGACCTCG ATATTTTAAC CTTTACGATA 240

GCTCTGACTG CCTCTGAAGT TATCAACCCT CTGATAGAAG AACTTGGTTG CGATAAGTTT 300

ATCAATAGAG AATAGTTAGG TGGTGACACT ACTTCAAGAG AACCTCTGCA TTCCAGTCAT 360

ACCAATCCTG CAACTTGATT TTCAGAAGTC AAGAGTATAT CGCGATAAGA CAGTGCACAG 420

GTGGAGGGGA AAAAAAGGGG GAGGGGGAAG CTTATCTTGA AAAAGCATCA CAGAAGTAGA 480

AAAAAATGTC GAAAGCATTA TAACTGTAAC GTTCTTTGAG TTTGTGATTG ATCCACATTT 540

TTCCCCCTGC ATTATGGAAA ATGTCTCTCA GCATTGCTTT ATTACAAAGT AAAGGATGGT 600

TTTATAAAAT TGAGACTGAT GAAACATCAA TACTAGAGCC CATGAGGATG AAAGAAATTA 660

TCAAATAGTG CTGAACAGAA TAAGATGTTA ACGCTGAGTT ATTAGGACTG GAAGGCTATG 720

AAAAGAACTT GAAATTGTCG GAATATGTGC TCTCTTCATG TCATATTCAA TAGAAGTTTC 780

TAGTTTAAGA TTGATTTTGT GTTTTCTTAG GCATTTCAAG TGACAAGCAA AGTAAATGTA 840

TATATTATGT GATAAATCAT GTTTTCAAGA ACGTCAAATT TCTGGACTTT TTTCTTTCAA 900

TTTTTAATTT TTAAAGTTTT TTTGGTATTA AAAAATCYAT TCACAAGCCA AAAAATWTWT 960

WAAATWTWCM GCGAAAAGCC AAAAAAAAAA AAAAMMAGGG GGGGCCGGGC CCCATCCCCC 1020

CAAGGGGGTC CNGNT 1035

(2) INFORMATION FOR SEQ ID NO: 103:

(i) SEQUENCE CHARACTERISTICS:
(A) LENGTH: 2218 base pairs
(B) TYPE: nucleic acid
(C) STRANDEDNESS: double
(D) TOPOLOGY: linear

(xi) SEQUENCE DESCRIPTION: SEQ ID NO: 103:

AGGTATTAGG CCCTTTTGTG GGAGCCCCAT GTTTTGTTTT TCTGAGTTGG TGGGGAGGGA 60

SGGAGGGGGA GGGCTGAATT GTTTTGCAGA GGAAGATGGC ATCTGTGCTT TAAATTTCTC 120

ATTACTGGGT TAGAAAACAA AGAGGGAKTG CCCTGCACAT TTTCTTTTGT GCTTTTAAAT 180

GTTTCTTAAG TTGGAACAGG TTTCCTCGGG CCTGTTTTGA CTGATTGCTG GAGTGCATTT 240

GATAGTTAAA AATTACTAAT TGGTTTTATT TCCCTTCACA CTCTGCCTCC CCACTTCTCC 300

CCCCGTTACT GAAAAATAAC CATTTTAGTG TCAGGCTAGA AATTGAATTG CTGAGTTTTG 360

TGTATCCTTT AAATTAAAAA CCACAAGTGT TTATTGTAGT GGTTAAACTG TAGCATCTCA 420

GCATCTGGGT GGAAGCTGCC TATATTTCTT CCCAGTTTAA CTGGGGACCA TCTGTGAAAT 480

TAATTTTCCA TCCAGACAGC TGCTGTGAGC AAATGAACAT AAATGCTCGC TGGAAATTTA 540

CTAACCAGTT TTTATATTGA CCTGCAGTGT AAAAAGCACA TTTAATTATA AACAATATAT 600

TCAAAATGGG CAAATTTTAT TTTCAAATGC AGTGTAGAGC TAGATTAAAA GCAACTCTTT 660

GCCACCTACT CTGCCCTTTT GGCAAAGTTA CCTTGAACAA AGAATCTTAA GGGTTTATTA 720

AGAACTCTTT ATTTTCTTCA TACCCTGTTC TCTGCAGTGC TTTCTAACAG CTTCTGGGTG 780

CAGATTTTCT TCGGCATCCT TTTGCACTCA GCTTATTACA GGTAGGTAGT GCTTAAGAAA 840

AGTCATGGAG GACTAAAGCC TAAGTCCTTT TCACTTTTCC TCCATCTGAA GGTAGGTGAG 900

TTCATCCTCT TCATAGTAAT GCTGTTTTAC CAAGACTTTA TAGCAGATGG ACCCAGAAAG 960

AATTTTCTGC TATTGTGTTC ACTACAACAG GATAGGGACA TCAGACAGCC CCAGAAACCC 1020

CTTCCAGATC TGATATGGGA CTATTAATTT TTATGCTGTT AATTGGTATT CATTCACAAT 1080

GCAGTTGAAG GGGGAAGGCT CCACTGCATT CTTTGGCTAA GGCCTGAATG CTTGCTCATC 1140

TGTAAGATCT ATACTCGAGG TTTTGTTTTC CTTTTAAAAT TCTTTAGGGA GAGAGGGATG 1200

GTTTCTGAGG GGTTCTGAAA GTATGATTCA ATGTGCAACA TACAGGTAGG TCTTCAGCAT 1260

AAGCTGAAAT ATATGCATGT AAAAACTTTG ACATCTTTTT TTTTAATTTT CCACTTTCTT 1320

CTTAACTTTA CTTCTCTTTT TGTCCCCCCC CCATCTTACA GAAGTTGAGG CCAAGGGAGA 1380

ATGGTAGGCA CAGAAGAAAC ATGGCAAACT GCTCTGTGCT TTCAAACCAA AGTGTTCCCC 1440

CCAACCCCAA ATTTGTCTAA GCACTGGCCA GTCTGTTGTG GGCATTGTTT TCTACAACCA 1500

AATTCTGGGT TTTTTTCTTC TTTCTTTAAA CATAGAGGTA CCACCACAAG GGATGCCCTA 1560

CTCTCTCGCA GCTCTTGAAA GCATCTGTTT GAGGGAAAGG TCTCTGGGCA AGCAAGTGGT 1620

TATTTGGATT GCTTGCTTCC CTTTTTCCAC CTGGGACATT GYAATCATAA AATAACAGTA 1680

AATTCCAAAC CTCAAAAACT ATTATGGCCT GAGCACAGCT GAAATCTAGC AGAGTTTAAC 1740

TCTTCTGCCT CCATGTCTGT CACTTATAAT TCAGGTTCTG CTGTTGGCTT CAGAACATGA 1800

GCAGAAGAAT CGTTTTATGC TAGTTATTGC ATTCATGGTT GAAACTCAAC TTAGGGAAAG 1860

GGTTCCAATG TATTAAGCAA TGGGCTGCTT CTCCCCAATC CTCCCTAACA ATTCGTTGTG 1920

TGGACTTCTC ATCTAAAAGG TTAGTGGCTT TTGCTTGGGA TCAGTGCTCT CTATTGATGT 1980

TCTTGCTGGT CTCCAGACAC ATTCCTGTTG CATTAAGACT TGAAAGACTT GTAGATGTGT 2040

GATGTTCAGG CACAGGATGC TGAAAGCTAT GTTACTATTC TTAGTTTGTA AATTGTCCTT 2100

TTGATACCAT CATCTTGTTT TCTTTTTGTA GGTATAAATA AAAACACTGT TGACAATAAA 2160

AAAAAAAAAA AAAAAAAAAA AAAAAAAAAA AAAAAAAAAA AAAAAAAAAA AAAAAAAA 2218

(2) INFORMATION FOR SEQ ID NO: 104:

(i) SEQUENCE CHARACTERISTICS:
(A) LENGTH: 1351 base pairs
(B) TYPE: nucleic acid
(C) STRANDEDNESS: double
(D) TOPOLOGY: linear

(xi) SEQUENCE DESCRIPTION: SEQ ID NO: 104:

CTTCACAGAC TGACAGAATG GTTTTGTTTT GTTTTGTTTT GTTTTGTTTT GTTTTTGAGA 60

TGGACTCTAG CTCTGTCACC CAGGCTGGAG TGCAGTGGTG CGATCTCGGC TCACTGCAAG 120

CTCCGCCTCC CGGGTTCTCA CCATTCTCCT GCCTCAGCCT CCCGAGTAGC TGGGACTACA 180

GGCGCCCACC ACCACGCCCG GCTAATTTTT TGTATTTTTT AGTAGAGACG GGGTTTCACC 240

ATGTTAGCCA GGATGGTCTC GATCTCCTGA CCTCGTGATC CGCCCGCYTC GGCCTCCCAA 300

AGTGCTGGGA TTACAGGCGT GAGCCACCGT GCCTGCCCCA GAATGGTTTT TAAAGCCACA 360

GTTGAGARGC CACCCATTGC CCGGCGCCTG GACAGTGATC ATCTTGTTCA TCTTGTTCAG 420

TCCTTTCTTG TGTGATTGGA ATTATTCATC CCCTTTGAAA GATGAGAAGG TTGAGATGCA 480

AAGAGTCTAC CTTTCCAAGT TCTCACTGCT GGAAAGARCT AGAAGCACAG TTCAAAGTTC 540

TGGNTTCTGG ACTCTGCAGT CCAGGTYTCC CTTYTCCCAC TTGCCTACCC TCAATGCCAC 600

ACTGTTTTTG AAGTGGCCCA TAACTTGAAG GRAAAGTTTA AAGACAGTTC AATTTAATCA 660

TCAGRATGCA TTCTTTTTTT TTTCGGARAC GGAKTTTCAC TCTTGCTGCC CASGCTGGAG 720

TGCAATGGTG CAATGATCTC GGCTCACTGC AACCTATGCC TCCTGGGTTC AAGNGATTAT 780

CCAGCCTCAG CCTCCCGAGT AGCTGGGATT ATGGGCGCCC ACCACCATGC CCAGCTAATT 840

TTTGTATTTT TTTTTTTAGT AGAGATGGGG TTTCGCCAGG TTGGCCAGGC TGKTCTTGTG 900

AAYTCCTGGC YTCAGGTGAT YTGCCCACYT CATCYTCCAA AAGTGCTGGG ATTACAGGCA 960

TGAGCCACTG CGCCTGGCYT CAGAATGCAT TCTTACACAT CTATCCTAGA CATTTATAAG 1020

CACTCTAATG GATAACAATC CAAGAATAAA TGATTGTAAA AGATGATGCC GAAGAGTTGA 1080

TGTCAATCTT TTTTTCCTAA GAAAAAAAGT CCGCGAGTAT TAAATATTTA GATCAATGTT 1140

TATAAAATGA TTACTTTGTA TATCTCATTA TTCCTATTTT GGAATAAAAA CTGACCTTCT 1200

TTAATCATAT ACTTGTCTTT TGTAAATAGC AGCTTTTGTG TCATTCTCCC CACTTTATTA 1260

GTTAATTTAA ATTGGAAAAA ACCCTCAAAC TAATATTCTT GTCTGTTCCA GTCTTATAAA 1320

TAAAACTTAT AATGCATGTA AAAAAAAAAA A 1351

(2) INFORMATION FOR SEQ ID NO: 105:

(i) SEQUENCE CHARACTERISTICS:
(A) LENGTH: 2066 base pairs
(B) TYPE: nucleic acid
(C) STRANDEDNESS: double
(D) TOPOLOGY: linear

(xi) SEQUENCE DESCRIPTION: SEQ ID NO: 105:

GGCACGAGGC GGCGGAGGGC CACAATCACA GCTCCGGGCA TTGGGGGAAC CCGAGCCGGC 60

TGCGCCGGGG GAATCCGTGC GGGCGCCTTC CGTCCCGGTC CCATCCTCGC CGCGCTCCAG 120

CACCTCTGAA GTTTTGCAGC GCCCAGAAAG GAGGCGAGGA AGGAGGGAGT GTGTGAGAGG 180

AGGGAGCAAA AAGCTCACCC TAAAACATTT ATTTCAAGGA GAAAAGAAAA AGGGGGGGCG 240

CAAAAATGGC TGGGGCAATT ATAGAAAACA TGAGCACCAA GAAGCTGTGC ATTGTTGGTG 300

GGATTCTGCT CGTGTTCCAA ATCATCGCCT TTCTGGTGGG AGGCTTGATT GCTCCAGGGC 360
CCACAACGGC AGTGTCCTAC ATGTCGGTGA AATGTGTGGA TGCCCGTAAG AACCATCACA 420
AGACAAAATG GTTCGTGCCT TGGGGACCCA ATCATTGTGA CAAGATCCGA GACATTGAAG 480
AGGCAATTCC AAGGGAAATT GAAGCCAATG ACATCGTGTT TTCTGTTCAC ATTCCCCTCC 540
CCCACATGGA GATGAGTCCT TGGTTCCAAT TCATGCTGTT TATCCTGCAG CTGGACATTG 600
CCTTCAAGCT AAACAACCAA ATCAGAGAAA ATGCAGAAGT CTCCATGGAC GTTTCCCTGG 660
CTTACCGTGA TGACGCATTT GCTGAGTGGA CTGAAATGGC CCATGAAAGA GTACCACGGA 720
AACTCAAATG CACCTTCACA TCTCCCAAGA CTCCAGAGCA TGAGGGCCGT TACTATGAAT 780
GTGATGTCCT TCCTTTCATG GAAATTGGGT CTGTGGCCCA TAAGTTTTAC CTTTTAAACA 840
TCCGGCTGCC TGTGAATGAG AAGAAGAAAA TCAATGTGGG AATTGGGGAG ATAAAGGATA 900
TCCGGTTGGT GGGGATCCAC CAAAATGGAG GCTTCACCAA GGTGTGGTTT GCCATGAAGA 960
CCTTCCTTAC GCCCAGCATC TTCATCATTA TGGTGTGGTA TTGGAGGAGG ATCACCATGA 1020
TGTCCCGACC CCCAGTGCTT CTGGAAAAAG TCATCTTTGC CCTTGGGATT TCCATGACCT 1080
TTATCAATAT CCCAGTGGAA TGGTTTTCCA TCGGGTTTGA CTGGACCTGG ATGCTGCTGT 1140
TTGGTGACAT CCGACAGGGC ATCTTCTATG CGATGCTTCT GTCCTTCTGG ATCATCTTCT 1200
GTGGCGAGCA CATGATGGAT CAGCACGAGC GGAACCACAT TGCAGGGTAT TGGAAGCAAG 1260
TCGGACCCAT TGCCGTTGGC TCCTTCTGCC TCTTCATATT TGACATGTGT GAGAGAGGGG 1320
TACAACTCAC GAATCCCTTC TACAGTATCT GGACTACAGA CATTGGAACA GAGCTGGCCA 1380
TGGCCTTCAT CATCGTGGCT GGAATCTGCC TCTGCCTCTA CTTCCTGTTT CTATGCTTCA 1440
TGGTATTTCA GGTGTTTCGG AACATCAGTG GGAAGCAGTC CAGCCTGCCA GCTATGAGCA 1500
AAGTCCGGCG GCTACACTAT GAGGGGCTAA TTTTTAGGTT CAAGTTCCTC ATGCTTATCA 1560
CCTTGGCCTG CGCTGCCATG ACTGTCATCT TCTTCATCGT TAGTCAGGTA ACGGAAGGCC 1620
ATTGGAAATG GGGCGGCGTC ACAGTCCAAG TGAACAGTGC CTTTTTCACA GGCATCTATG 1680
GGATGTGGAA TCTGTATGTC TTTGCTCTGA TGTTCTTGTA TGCACCATCC CATAAAAACT 1740
ATGGAGAAGA CCAGTCCAAT GGAATGCAAC TCCCATGTAA ATCGAGGGAA GATTGTGCTT 1800
TGTTTGTTTC GGAACTTTAT CAAGAATTGT TCAGCGCTTC GAAATATTCC TTCATCAATG 1860
ACAACGCAGC TTCTGGTATT TGAGTCAACA AGGCAACACA TGTTTATCAG CTTTGCATTT 1920
GCAGTTGTCA CAGTCACATT GATTGTACTT GTATACGCAC ACAAATACAC TCATTTAGCC 1980
TTTATCTCAA AATGTTAAAT ATAAGGAAAA AAGCGTCAAC AATAAATATT CTTGAGTATA 2040
AAAAAAAAAA AAAAAAAAAA AAAAAA 2066

(2) INFORMATION FOR SEQ ID NO: 106:

(i) SEQUENCE CHARACTERISTICS:
(A) LENGTH: 1705 base pairs
(B) TYPE: nucleic acid
(C) STRANDEDNESS: double
(D) TOPOLOGY: linear

(xi) SEQUENCE DESCRIPTION: SEQ ID NO: 106:

AATTCGGCAK AGGGCAGCTG TCGGCTGGAA GGAACTGGTC TGCTCACACT TGCTGGCTTG 60

CGCATCAGGA CTGGCTTTAT CTCCTGACTC ACGGTGCAAA GGTGCACTCT GCGAACGTTA 120

AGTCCGTCCC CAGCGCTTGG AATCCTACGG CCCCCACAGC CGGATCCCCT CAGCCTTCCA 180

GGTCCTCAAC TCCCGYGGAC GCTGAACAAT GGCCTCCATG GGGCTACAGG TAATGGGCAT 240

CGCGCTGGCC GTCCTGGGCT GGCTGGCCGT CATGCTGTGC TGCGCGCTGC CCATGTGGCG 300

CGTGACGGCC TTCATCGGCA GCAACATTGT CACCTCGCAG ACCATCTGGG AGGGCCTATG 360

GATGAACTGC GTGGTGCAGA GCACCGGCCA GATGCAGTGC AAGGTGTACG ACTCGCTGCT 420

GGCACTGCCG CAGGACCTGC AGGCGGCCCG CGCCCTCGTC ATCATCAGCA TCATCGTGGC 480

TGCTCTGGGC GTGCTGCTGT CCGTGGTGGG GGGCAAGTGT ACCAACTGCC TGGAGGATGA 540

AAGCGCCAAG GCCAAGACCA TGATCGTGGC GGGCGTGGTG TTCCTGTTGG CCGGCCTTAT 600

GGTGATAGTG CCGGTGTCCT GGACGGCCCA CAACATCATC CAAGACTTCT ACAATCCGCT 660

GGTGGCCTCC GGGCAGAAGC GGGAGATGGG TGCCTCGCTC TACGTCGGCT GGGCCGCCTC 720

CGGNCTGCTG CTCCTTGGCG GGGGGCTGCT TTGCTGCAAC TGTCCACCCC GCACAGACAA 780

GCCTTACTCC GCCAAGTATT CTGCTGCCCG CTCTGCTGCT GCCAGCAACT ACGTGTAAGG 840

TGCCACGGCT CCACTCTGTT CCTCTCTGCT TTGTTCTTCC CTGGACTGAG CTCAGCGCAG 900

GCTGTGACCC CAGGAGGGCC CTGCCACGGG CCACTGGCTG CTGGGGACTG GGGACTGGGC 960

AGAGACTGAG CCAGGCAGGA AGGCAGCAGC CTTCAGCCTC TCTGGCCCAC TCGGACAACT 1020

TCCCAAGGCC GCCTCCTGCT AGCAAGAACA GAGTCCACCC TCCTCTGGAT ATTGGGGAGG 1080

GACGGAAGTG ACAGGGTGTG GTGGTGGAGT GGGGAGCTGG CTTCTGCTGG CCAGGATGGC 1140

TTAACCCTGA CTTTGGGATC TGCCTGCATC GGTGTTGGCC ACTGTCCCCA TTTACATTTT 1200

CCCCACTCTG TCTGCCTGCA TCTCCTCTGT TGCGGGTAGG CCTTGATATC ACCTCTGGGA 1260

CTGTGCCTTG CTCACCGAAA CCCGCGCCCA GGAGTATGGC TGAGGCCTTG CCCACCCACC 1320

TGCCTGGGAA GTGCAGAGTG GATGGACGGG TTTAGAGGGG AGGGGCGAAG GTGCTGTAAA 1380

CAGGTTTGGG CAGTGGTGGG GGAGGGGGCC AGAGAGGCGG CTCAGGTTGC CCAGCTCTGT 1440

GGCCTCAGGA CTCTCTGCCT CACCCGCTTC AGCCCAGGGC CCCTGGAGAC TGATCCCCTC 1500

TGAGTCCTCT GCCCCTTCCA AGGACACTAA TGAGCCTGGG AGGGTGGCAG GGAGGAGGGG 1560

ACAGCTTCAC CCTTGGAAGT CCTGGGGTTT TTCCTCTTCC TTCTTTGTGG TTTCTGTTTT 1620

GTAATTTAAG AAGAGCTATT CATCACTGTA ATTATTATTA TTTTCTACAA TAAATGGGAC 1680

CTGTGCACAG GRAAAAAAAA AAAAG 1705

(2) INFORMATION FOR SEQ ID NO: 107:

(i) SEQUENCE CHARACTERISTICS:
(A) LENGTH: 1167 base pairs
(B) TYPE: nucleic acid
(C) STRANDEDNESS: double
(D) TOPOLOGY: linear

(xi) SEQUENCE DESCRIPTION: SEQ ID NO: 107:

TGCAGGAATT CGGCAGAGGT TTTCCGCTAG ACTCTGGCAG TTGGTGAGCA TCATGGCAAC 60

CGTTACAGCC ACAACCAAAG TCCCGGAGAT CCGTGATGTA ACAAGGATTG AGCGAATCGG 120

TGCCCACTCC CACATCCGGG GACTGGGGCT GGACGATGCC TTGGAGCCTC GGCAGGCTTC 180

GCAAGGCATG GTGGGTCAGC TGGCGGCACG GCGGGCGGCT GGCGTGGTGC TGGAGATGAT 240

CCGGGAAGGG AAGATTGCCG GTCGGGCAGT CCTTATTGCT GGCCAGCCGG GCACGGGGAA 300

GACGGCCATC GCCATGGGCA TGGCGCAGGC CCTGGGCCCT GACACGCCAT TCACAGCCAT 360

CGCCGGCAGT GAAATCTTCT CCCTGGAGAT GAGCAAGACC GAGGCGCTGA CGCAGGCCTT 420

CCGGCGGTCC ATCGGCGTTC GCATCAAGGA GGAGACGGAG ATCATCGAAG GGGAGGTGGT 480

GGAGATCCAG ATTGATCGAC CAGCAACAGG GACGGGCTCC AAGGTGGGCA AACTGACCCT 540

CAAGACCACA GAGATGGAGA CCATCTACGA CCTGGGCACC AAGATGATTG AKTCCCTGAC 600

CAAGGACAAG GTCCAGGCCG GGGACGTGAT CACCATCGAC AAGGCGACGG GCAAGATCTC 660

CAAGCTGGGC CGCTCCTTCA CACGCGCCCG CGAACTACGA CGCTATGGGC TCCCAGACCA 720

AGTTCGTGCA GTGCCCAGAT GGGGAGCTCC AGAAACGCAA GGAGGTGGTG CACACCGTGT 780

CCCTGCACGA GATCGACGTC ATCAACTCTC GCACCCAGGG CTTCCTGGCG CTCTTCTCAG 840

GTGACACAGG GGAGATCAAG TCAGAAGTCC GTGAGCAGAT CAATGCCAAG GTGGCTGAGT 900

GGCGCGAGGA GGGCAAGGCG GAGATCATCC CTGGAGTGCT GTTCATCGAC GAGGTCCACA 960

TGCTGGACAT CGAGAGCTTC TCCTTCCTCA ACCGGGCCCT GGAGAGTGAC ATGGCGCCTG 1020

TCCAGCAGGT CTATGGGGAT GCCGTGAGGG CTCTGGTAGC TGGTGCCCCG GATTCGCGTG 1080

ATGCCACGGT TGGTGGCCTC GTGCCGAATT CCTGCAGCCC GGGGGATCCA CTAGTTCTAG 1140

AGCGGCCGCC ACCGCGGTGG ANCTCCN 1167

(2) INFORMATION FOR SEQ ID NO: 108:

(i) SEQUENCE CHARACTERISTICS:
(A) LENGTH: 1907 base pairs
(B) TYPE: nucleic acid
(C) STRANDEDNESS: double
(D) TOPOLOGY: linear

(xi) SEQUENCE DESCRIPTION: SEQ ID NO: 108:

GGCACAGGGG AATCATCGTG TGATGTGTGT GCTGCCTTTG TGAGTGTGTG GAGTCCTGCT 60

CAGGTGTTAG GTACAGTGTG TTTGATCGTG GTGGCTTGAG GGGAACCCTT GTTCAGAGCT 120

GTGACTGCGG CTGCACTCAG AGAAGCTGCC CTTGGCTGCT CGTAGCGCCG GGCCTTCTCT 180

CCTCGTCATC ATCCAGAGCA GCCAGTGTCC GGGAGGCAGA AGGTACCGGG GCAGCTACTG 240

GAGGACTGTG CGGGCCTGCC TGGGCTGCCC CCTCCGCCGT GGGGCCCTGT TGCTGCTGTC 300

CATCTATTTC TACTACTCCC TCCCAAATGC GGTCGGCCCG CCCTTCACTT GGATGCTTGC 360

CCTCCTGGGC CTCTCGCAGG CACTGAACAT CCTCCTGGGC CTCAAGGGCC TGGCCCCAGC 420

TGAGATCTCT GCAGTGTGTG AAAAAGGGAA TTTCAACGTG GCCCATGGGC TGGCATGGTC 480

ATATTACATC GGATATCTGC GGCTGATCCT GCCAGAGCTC CAGGCCCGGA TTCGAACTTA 540

CAATCAGCAT TACAACAACC TGCTACGGGG TGCAGTGAGC CAGCGGCTGT ATATTCTCCT 600

CCCATTGGAC TGTGGGGTGC CTGATAACCT GAGTATGGCT GACCCCAACA TTCGCTTCCT 660

GGATAAACTG CCCCAGCAGA CCGGTGACCG TGCTGGCATC AAGGATCGGG TTTACAGCAA 720

CAGCATCTAT GAGCTTCTGG AGAACGGGCA GCGGGCGGGC ACCTGTGTCC TGGAGTACGC 780

CACCCCCTTG CAGACTTTGT TTGCCATGTC ACAATACAGT CAAGCTGGCT TTAGCGGGGA 840

GGATAGGCTT GAGCAGGCCA AACTCTTCTG CCGGACACTT GAGGACATCC TGGCAGATGC 900

CCCTGAGTCT CAGAACAACT GCCGCCTCAT TGCCTACCAG GAACCTGCAG ATGACAGCAG 960

CTTCTCGCTG TCCCAGGAGG TTCTCCGGCA CCTGCGGCAG GAGGAAAAGG AAGAGGTTAC 1020

TGTGGGCAGC TTGAAGACCT CAGCGGTGCC CAGTACCTCC ACGATGTCCC AAGAGCCTGA 1080

GCTCCTCATC AGTGGAATGG AAAAGCCCCT CCCTCTCCGC ACGGATTTCT CTTGAGACCC 1140

AGGGTCACCA GGCCAGAGCC TCCAGTGGTC TCCAAGCCTC TGGACTGGGG GCTCTCTTCA 1200

GTGGCTGAAT GTCCAGCAGA GCTATTTCCT TCCACAGGGG GCCTTGCAGG GAAGGGTCCA 1260

GGACTTGACA TCTTAAGATG CGTCTTGTCC CCTTGGGCCA GTCATTTCCC CTCTCTGAGC 1320

CTCGGTGTCT TCAACCTGTG AAATGGGATC ATAATCACTG CCTTACCTCC CTCACGGTTG 1380

TTGTGAGGAC TGAGTGTGTG GAAGTTTTTC ATAAACTTTG GATGCTAGTG TACTTAGGGG 1440

GTGTGCCAGG TGTCTTTCAT GGGGCCTTCC AGACCCACTC CCCACCCTTC TCCCCTTCCT 1500

TTGCCCGGGG ACGCCGAACT CTCTCAATGG TATCAACAGG CTCCTTCGCC CTCTGGCTCC 1560

TGGTCATGTT CCATTATTGG GGAGCCCCAG CAGAAGAATG GAGAGGAGGA GGAGGCTGAG 1620

TTTGGGGTAT TGAATCCCCC GGCTCCCACC CTGCAGCATC AAGGTTGCTA TGGACTCTCC 1680

TGCCGGGCAA CTCTTGCGTA ATCATGACTA TCTCTAGGAT TCTGGCACCA CTTCCTTCCC 1740

TGGCCCCTTA AGCCTAGCTG TGTATCGGCA CCCCCACCCC ACTAGAGTAC TCCCTCTCAC 1800

TTGCGGTTTC CTTATACTCC ACCCCTTTCT CAACGGTCCT TTTTTAAAGC ACATCTCAGA 1860

TTAAAAAAAA AAAAAAAAAA AAAAAAAAAA AAAAAAAGGG CGGCCGC 1907

(2) INFORMATION FOR SEQ ID NO: 109:

(i) SEQUENCE CHARACTERISTICS:
(A) LENGTH: 611 base pairs
(B) TYPE: nucleic acid
(C) STRANDEDNESS: double
(D) TOPOLOGY: linear

(xi) SEQUENCE DESCRIPTION: SEQ ID NO: 109:

ATGAATTAAC GCCAAGCTNT NAATAGGGAC TCACTATGGG GGAAAGNTGG GTAACGCCTG 60

CAGGTACCGT TCCGGAATTC CCGGGTCGAC CCACGCGTCC GATGGGGCTT TAGTAAATCA 120

GGCTTGCAGG CTCAAAGCTG CAATCTGCCC ACTCTCAGGT ACTGAGACTT TGTGGGCCTC 180

AGACACCAGG AAGAAAGTTG GGATACAGTC ATTTGAGTTA AAAAGGGAAT GACCCCTCAG 240

AAACCCGCAT TAGCAGTGTT ACTCTTGGAA GTGCCTTTAC TTTTAACGCT CTCTGTTCTG 300

AAAAAGAGGT GTTTGGTTAC GTGTGAGCCA ACATCACGTT TTGTTAGCTG TGATTTACCT 360

TTGTCCGTTT AAAAGACTTC ACGGAGCCAT TCTGTATACA AGGTGTGCTC TTTCCAATGT 420

AGAAGGGGTT ATGGAAAAGG GTGCGATCCT TTGCTGTAAA CTGGAGAGAC CAGTCCCAAA 480

CAGAGGGGAA TTTTAAGCCC TTCTCATCAC CCAATTGGAT GTTTTTGCTT ATAGCAAATT 540

CCTGCAAAAT AAATAAATAA ATATTTGCAA AACTAAAAAA AAAAAAAAAA AAAAAAAAAA 600

GGGGGGNCCN C 611

(2) INFORMATION FOR SEQ ID NO: 110:

(i) SEQUENCE CHARACTERISTICS:
(A) LENGTH: 2632 base pairs
(B) TYPE: nucleic acid
(C) STRANDEDNESS: double
(D) TOPOLOGY: linear

(xi) SEQUENCE DESCRIPTION: SEQ ID NO: 110:

TCCCAGCTCT CAGGACAAGG GCCCTGGGCG ATCTTTTAAA AAAGCCGATT GGGTGTCTTT 60

CTAAAANTAC AACCAGTACT TCATCGTCAA GTTTCTGGGA AGGGAGTCCC CTCCAGATTC 120

TCATGGAGTG ACAAATCTTG ACTCTTGCTC CTGGAATTTT TCAGGCCCAA ACTAGCGTTT 180

CTACAATGAT TTATTTGGCA AATTTGTCTT GATTATGGGT GGCTGATGAG GAACGTGCTT 240

TTGTTAGGAA CCGAAACTGG GCGGCGGTGA GGGCGTGTAC GCAATGAGTC CGGAAGAGGG 300

TGAAATGCTT TCGGTAGGCA CTCCACGGCT GTGAAGATGG CGGCGGCTGC GTGGCTTCAG 360

GTGTTGCCTG TCATTCTTCT GCTTCTGGGA GCTCACCCGT CACCACTGTC GTTTTTCAGT 420

GCGGGACCGG CAACCGTAGC TGCTGCCGAC CGGTCCAAAT GGCACATTCC GATACCGTCG 480

GGGAAAAATT ATTTTAGTTT TGGAAAGATC CTCTTCAGAA ATACCACTAT CTTCCTGAAG 540

TTTGATGGAG AACCTTGTGA CCTGTCTTTG AATATAACCT GGTATCTGAA AAGCGCTGAT 600

TGTTACAATG AAATCTATAA CTTCAAGGCA GAAGAAGTAG AGTTGTATTT GGAAAAACTT 660

AAGGAAAAAA GAGGCTTGTC TGGGAAATAT CAAACATCAT CAAAATTGTT CCAGAACTGC 720

AGTGAACTCT TTAAAACACA GACCTTTTCT GGAGATTTTA TGCATCGACT GCCTCTTTTA 780

GGAGAAAAAC AGGAGGCTAA GGAGAATGGA ACAAACCTTA CCTTTATTGG AGACAAAACC 840

GCAATGCATG AACCATTGCA AACTTGGCAA GATGCACCAT ACATTTTTAT TGTACATATT 900

GGCATTTCAT CCTCAAAGGA ATCATCAAAA GAAAATTCAC TGAGTAATCT TTTTACCATG 960

ACTGTTGAAG TGAAGGGTCC CTATGAATAC CTCACACTTG AAGACTATCC CTTGATGATT 1020

TTTTTCATGG TGATGTGTAT TGTATATGTC CTGTTTGGTG TTCTGTGGCT GGCATGGTCT 1080

GCCTGCTACT GGAGAGATCT CCTGAGAATT CAGTTTTGGA TTGGTGCTGT CATCTTCCTG 1140

GGAATGCTTG AGAAAGCTGT CTTCTATGCG GAATTTCAGA ATATCCGATA CAAAGGARAA 1200

TCTGTCCAGG GTGCTTTGAT CCTTGCAGAR CTGCTTTCAG CAGTGAAACG CTCACTGGCT 1260

CGAACCCTGG TCATCATAGT CAGTCTGGGA TATGGCATCG TCAAGCCACG CCTGGAGTCA 1320

CTCTTCATAA GGTTGTAGTA GCAGRAGCCC TCTATCTTTT GTTCTCTGGC ATGGAAGGGG 1380

TCCTCAGAGT TACTGGGGCC CAGACTGATC TTGCTTCCTT GGCCTTTATC CCCTTGGCTT 1440

TCCTAGACAC TGCCTTGTGC TGGTGGATAT TTATTAGCCT GACTCAAACA ATGAAGCTAT 1500

TAAAACTTCG GAGGAACATT GTAAAACTCT CTTTGTATCG GCATTTCACC AACACGCTTA 1560

TTTTGGCAGT GGCAGCATCC ATTGTGTTTA TCATCTGGAC AACCATGAAG TTCAGAATAG 1620

TGACATGTCA GTCGGACTGG CGGGAGCTGT GGGTAGACGA TGCCATCTGG CGCTTGCTGT 1680

TCTCCATGAT CCTCTTTGTC ATCATGGTTC TCTGGCGACC ATCTGCAAAC AACCAGAGGT 1740

TTGCCTTTTC ACCATTGTCT GAGGAAGAGG AGGAGGATGA ACAAAAGGAG CCTATGCTGA 1800

AAGAAAGCTT TGAAGGAATG AAAATGAGAA GTACCAAACA AGAACCCAAT GGAAATAGTA 1860

AAGTTAACAA AGCACAGGAA GATGATTTGA AGTGGGTAGA AGAGAATGTT CCTTCTTCTG 1920

TGACAGATGT AGCACTTCCA GCCCTTCTGG ATTCAGATGA GGAACGAATG ATCACACACT 1980

TTGAAAGGTC CAAAATGGAG TAAGGAATGG GAAGATTTGC AGTTAAAGAT GGCTACCATC 2040

AGGGAAGAGA TCAGCATCTG TGTCAGTCTT CTGTACGGCT CCATGGGATT AAAGGAAGCA 2100

ATGACATCCT GATCTGTTCC TTGATCTTTG GGCATTGGAG TTGGCGAGAG GTGTCAGAAC 2160

AAAGAGAACA TCTTACTGAA AACAAGTTCA TAAGATGAGA AAAATCTACG AGCTTCTTAT 2220

TTACAACACT GCTGCCCCCT TTCCTCCCAG ACTCTGACAT GGATGTTCAT GCAACTTAAG 2280

TGTGTTGTTC CTGAACTTTC TGTAATGTTT CATTTTTTAA ATCTGACAAA CTAAAAAGTT 2340

TAACGTCTTC TAAAAGATTG TCATCAACAC CATAATATGT AATCTCCAGG AGCAACTGCC 2400

TGTAATTTTT ATTTATTTAG GGAGTTACAT AGGTGATGGG GGAAATTGTT AACTACCTTT 2460

CATTTTCCTG GGAAGTCAAG GTTACATCTT GCAGAGGTTG TTTTGAGAAA AAAGGGCCCT 2520

TCTGAGTTAA GGAGCCATAG TTCTATCAAT GATCAAAAGA AAAAAAAAAA AACTCGATCG 2580

GCACGAGGGG GGGCCCGGTA CCCAATTCGC CCTATGGGAN TCGAATGAGA CC 2632

(2) INFORMATION FOR SEQ ID NO: 111:

(i) SEQUENCE CHARACTERISTICS:
(A) LENGTH: 2249 base pairs
(B) TYPE: nucleic acid
(C) STRANDEDNESS: double
(D) TOPOLOGY: linear

(xi) SEQUENCE DESCRIPTION: SEQ ID NO: 111:
   GAATTCGGCA CGAGCTCACC GTGCTGCGTG ACACAAGGCC AGCCTGCGCC TACGAGCCCA 60
   TGGACTTTKT RATGGCCCTC ATCTACGACA TGGTACTGSW TGTGGTCACC CTGGGGCTGG 120
   CCCTCTTCAC TCTGTGCGGC AAGTTCAAGA GGTGGAAGCT GAACGGGGCC TTCCTCCTCA 180
   TCACAGCCTT CCTCTCTGTG CTCATCTGGG TGGCCTGGAT GACCATGTAC CTCTTCGGCA 240
   ATGTCAAGCT GCAGCAGGGG GATGCCTGGA ACGACCCCAC CTTGGCCATC ACGCTGGCGG 300
   CCAGCGCTGG GTCTTCGTCA TCTTCCACGC CATCCCTGAG ATCCACTGCA CCCTTCTGCC 360
   AGCCCTGCAG GAGAACACGC CCAACTACTT CGACACGTCG CAGCCCAGGA TGCGGGAGAC 420
   GGCCTTCGAG GAGGACGTGC AGCTGCCGCG GGCCTATATG GAGAACAAGG CCTTCTCCAT 480
   GGATGAACAC AATGCAGCTC TCCGAACAGC AGGATTTCCC AACGGCAGCT TGGGAAAAAG 540
   ACCCAGTGGC AGCTTGGGGA AAAGACCCAG CGCTCCGTTT AGAAGCAACG TGTATCAGCC 600
   AACTGAGATG GCCGTCGTGC TCAACGGTGG GACCATCCCA ACTGCTCCGC CAAGTCACAC 660
   AGGAAGAMAC CTTTGGTGAA AGACTTTAAG TTCCAGAGAA TCAGAATTTC TCTTACCGAT 720
   TTGCCTCCCT GGCTGTGTCT TTCTTGAGGG AGAAATCGGT AACAGTTGCC GAACCAGGCC 780
   GCCTCACAGC CAGGAAATTT GGAAATCCTA GCCAAGGGGA TTTCGTGTAA ATGTGAACAC 840
   TGACGAACTG AAAAGCTAAC ACCGACTGCC CGCCCCTCCC CTGCCACACA CACAGACACG 900
   TAATACCAGA CCAACCTCAA TCCCCGCAAA CTAAAGCAAA GCTAATTGCA AATAGTATTA 960
   GGCTCACTGG AAAATGTGGC TGGGAAGACT GTTTCATCCT CTGGGGGTAG AACAGAACCA 1020
   AATTCACAGC TGGTGGGCCA GACTGGTGTT GGTTGGAGGT GGGGGGCTCC CACTCTTATC 1080
   ACCTCTCCCC AGCAAGTGCT GGACCCCAGG TAGCCTCTTG GAGATGACCG TTGCGTTGAG 1140
   GACAAATGGG GACTTTGCCA CCGGCTTTGC CTGGTGGTTT GCACATTTCA GGGGGGTCAG 1200
   GAGAGTTAAG GAGGTTGTGG GTGGGATTCC AAGGTGAGGC CCAACTGAAT CGTGGGGTGA 1260
   GCTTTATAGC CAGTAGAGGT GGAGGGACCC TGGCATGTGC CAAAGAAGAG GCCCTCTGGG 1320
   TGATGAAGTG ACCATCACAT TTGGAAAGTG ATCAACCACT GTTCCTTCTA TGGGGCTCTT 1380
   GCTCTAGTGT CTATGGTGAG AACACAGGCC CCGCCCCTTC CCTTGTAGAG CCATAGAAAT 1440
   ATTCTGGCTT GGGGCAGCAG TCCCTTCTTC CCTTGATCAT CTCGCCCTGT TCCTACACTT 1500
   ACGGGTGTAT CTCCAAATCC TCTCCCAATT TTATTCCCTT ATTCATTTCA AGAGCTCCAA 1560
   TGGGGTCTCC AGCTGAAANS CCCTCCGGGA GGCAGGTTGG AAGGCAGGCA CCACGGCAGG 1620
   TTTTCCGCGA TGATGTCACC TAGCAGGGCT TCAGGGGTTC CCACTAGGAT GCAGAGATGA 1680
   CCTCTCGCTG CCTCACAAGC AGTGACACCT CGGGTCCTTT CCGTTGCTAT GGTGAAAATT 1740
   CCTGGATGGA ATGGATCACA TGAGGGTTTC TTGTTGCTTT TGGAGGGTGT GGGGGATATT 1800
   TTGTTTTGGT TTTTCTGCAG GTTCCATGAA AACAGCCCTT TTCCAAGCCC ATTGTTTCTG 1860
   TCATGGTTTC CATCTGTCCT GAGCAAGTCA TTCCTTTGTT ATTTAGCATT TCGAACATCT 1920
   CGGCCATTCA AAGCCCCCAT GTTCTCTGCA CTGTTTGGCC AGCATAACCT CTAGCATCGA 1980
   TTCAAAGCAG AGTTTTAACC TGACGGCATG GAATGTATAA ATGAGGGTGG GTCCTTCTGC 2040
   AGATACTCTA ATCACTACAT TGCTTTTTCT ATAAAACTAC CCATAAGCCT TTAACCTTTA 2100
   AAGAAAAATG AAAAAGGTTA GTGTTTGGGG GCCGGGGGAG GACTGACCGC TTCATAAGCC 2160
   AGTACGTCTG AGCTGAGTAT GTTTCAATAA ACCTTTTGAT ATTTCTCAAA AAAAAAAAAA 2220
   AAAAANCCCG GGGGGGGCCC CGGACCTGG 2249

(2) INFORMATION FOR SEQ ID NO: 112:

(i) SEQUENCE CHARACTERISTICS:
(A) LENGTH: 2193 base pairs
(B) TYPE: nucleic acid
(C) STRANDEDNESS: double
(D) TOPOLOGY: linear

(xi) SEQUENCE DESCRIPTION: SEQ ID NO: 112:

GATACTATAA GGCAAGTGAC TCACGGGTGC GCCGTTAGAC TAGTGGATCC CGGGTGCAGG 60

AATTCGGCAG AGCGCCGCCG GAGCCGAAGT GCTGGCGCCC CCGCGGCCGC TGCCTCCGCG 120

GANCCCAAAA TCATGAAAGT CACCGTGAAG ACCCCGAAGA AAAGGAGGAA TTCGCCGTGC 180

CCGAGAATAG CTCCGTCCAG CAGTTTAAGG AAGAAATCTC TAAACGTTTT AAATCACATA 240

CTGACCAACT TGTGTTGATA TTTGCTGGAA AAATTTTGAA AGATCAAGAT ACCTTGAGTC 300

AGCATGGAAT TCATGATGGA CTTACTGTTC ACCTTGTCAT TAAAACACAA AACAGGCCTC 360

AGGATCATTC AGCTCAGCAA ACAAATACAG CTGGAAGCAA TGTTACTACA TCATCAACTC 420

CTAATAGTAA CTCTACATCT GGTTCTGCTA CTAGCAACCC TTTTGGTTTA GGTGGCCTTG 480

GGGGACTTGC AGGTCTGAGT AGCTTGGGTT TGAATACTAC CAACTTCTCT GAACTACAGA 540

GTCAGATGCA GCGACAACTT TTGTCTAACC CTGAAATGAT GGTCCAGATC ATGGAAAAWC 600

CCYTTGTTCA GAGCATGCTC NTCAAATCCT GACCTGATGN AGACAGTTAA TTATGGCCAA 660

TCCACAAATG CAGCAGTTGA TACAGAGAAA TCCCAGAAAT TAGTCATATG TTGAATAATC 720

CAGATATAAT GAGACAAACG TTGGAACTTG CCCAGGAATC CAGCAATGAT GCAGGAGATG 780

ATGAGGAACC AGGACCGAGC TTTGAGCAAC CTAGAAAGCA TCCCAGGGGG ATATAATGCT 840

TTAAGGCGCA TGTACACAGA TATTCAGGAA CCAATGCTGA GTGCTGCACA AGAGCAGTTT 900

GGTGGTAATC CATTTGCTTC CTTGGTGAGC AATACATCCT CTGGTGAAGG TAGTCAACCT 960

TCCCGTACAG AAAATAGAGA TCCACTACCC AATCCATGGG CTCCACAGAC TTCCCAGAGT 1020

TCATCAGCTT CCAGCGGCAC TGCCAGCACT GTGGGTGGCA CTACTGGTAG TACTGCCAGT 1080

GGCACTTCTG GGCAGAGTAC TACTGCGCCA AATTTGGTGC CTGGAGTAGG AGCTAGTATG 1140

TTCAACACAC CAGGAATGCA GAGCTTGTTG CAACAAATAA CTGAAAACCC ACAACTTATG 1200

CAAAACATGT TGTCTGCCCC CTACATGAGA AGCATGATGC AGTCACTAAG CCAGAATCCT 1260

GACCTTGCTG CACAGATGAT GCTGAATAAT CCCCTATTTG CTGGAAATCC TCAGCTTCAA 1320

GAACAAATGA GACAACAGCT CCCAACTTTC CTCCAACAAA TGCAGAATCC TGATACACTA 1380

TCAGCAATGT CAAACCCTAG AGCAATGCAG GCCTTGTTAC AGATTCAGCA GGGTTTACAG 1440

ACATTAGCAA CGGAAGCCCC GGGCCTCATC CCAGGGTTTA CTCCTGGCTT GGGGGCATTA 1500

GGAAGCACTG GAGGCTCTTC GGGAACTAAT GGATCTAACG CCACACCTAG TGAAAACACA 1560

AGTCCCACAG CAGGAACCAC TGAACCTGGA CATCAGCAGT TTATTCAGCA GATGCTGCAG 1620

GCTCTTGCTG GAGTAAATCC TCAGCTACAG AATCCAGAAG TCAGATTTCA GCAACAACTG 1680

GAACAACTCA GTGCAATGGG ATTTTTGAAC CGTGAAGCAA ACTTGCAAGC TCTAATAGCA 1740

ACAGGAGGTG ATATCAATGC AGCTATTGAA AGGTTACTGG GCTCCCAGCC ATCATAGCAG 1800

CATTTCTGTA TCTKGAAAAA ATGTAATTTA TTTTTGATAA CGGCTCTTAA ACTTTAAAAT 1860

ACCTGCTTTA TTTCATTTTG ACTCTTGGAA TTCTGTGCTG TTATAAACAA ACCCAATATG 1920

ATGCATTTTA AGGTGGAGTA CAGTAAGATG TGTGGGTTTT TCTGTATTTT TCTTTTCTGG 1980

AACAGTGGGA ATTAAGGCTA CTGCATGCAT CACTTCTGCA TTTATTGTAA TTTTTTAAAA 2040

ACATCACCTT TTATAGTTGG GTGACCAGAT TTTGTCCTGC ATCTGTCCAG TTTATTTGCT 2100

TTTTAAACAT TAGCCTATGG TAGTAATTTA TGTAGAATAA AAGCATTAAA AAGAAGCAAA 2160

AAAAAAAAAA AAAAATTCCT GCGCCCGCGA ATTCTTCT 2198

(2) INFORMATION FOR SEQ ID NO: 113:

(i) SEQUENCE CHARACTERISTICS:
(A) LENGTH: 1043 base pairs
(B) TYPE: nucleic acid
(C) STRANDEDNESS: double
(D) TOPOLOGY: linear

(xi) SEQUENCE DESCRIPTION: SEQ ID NO: 113:

CTGAAGTGTA TGTGGTGAGG AAGAAGAGGC TCCTACTGTA GACAGCCTTG TTCTACAGAT 60

CCTCCCAGAA ATCTCTGGGC CAGGTGGAAC CCAGGGTCAG AGAGGGATGG GAGAGAGGTT 120

TAATTTTCCA TGATAAATAA AAATCTATAA AATAATAAAC AAGAGAAAAG AGATTGGAAA 180

CAGCCAGGTT GGAGCAGTGA GTGAGTAAGG AAACCTGGCT GCCCTCTCCA GATTCCCCAG 240

GCTCTCAGAG AAGATCAGCA GAAAGTCTGC AAGACCCTAA GAACCATCAG CCCTCAGCTG 300

CACCTCCTCC CCTCCAAGGA TGACAAAGGC GCTACTCATC TATTTGGTCA GCAGCTTTCT 360

TGCCCTAAAT CAGGCCAGCC TCATCAGTCG CTGTGACTTG GCCCAGGTGC TGCAGCTGGA 420

RGACTTGGAT GGGTTTGAGG GTTACTCCCT GAGTGACTGG CTGTGCCTGG CTTTTGTGGA 480

AAGCAAGTTC AACATATCAA AGATWAATGA AAATGCAGAT GGAAGCTTTG ACTATGGSCT 540

CTTCCAGATC AACAGCCACT ACTGGTGCAA CRATTATAAG AGTTACTCGG AAAACCTTTG 600

CCACGTAGAC TGTCAAGATC TGCTGAATCC CAACCTTCTT GCAGGCATCC ACTGCGCAAA 660

AAGGATTGTG TCCGGAGCAC GGGGGATGAA CAACTGGGTT AGAATGGAAG KTTGCACTGT 720

TCAGGCCGGC CACTCTTCTA CTGGCTGACA GGATGCCGCC TGAGATKAAA CARGGTGCGG 780

GTGCACCGTG GARTCATTCC AAGACTCCTG TCCTCACTCA RGGATTCTTC ATTTCTTCTT 840

CCTACTGCCT CCACTTCATG TTATTTTCTT CCCTTCCCAT TTACAACTAA AACTGACCAG 900

AGCCCCAGGA ATAAATGGTT TTCTTGGCTT CCTCCTTACT CCCATCTGGA CCCAGTCCCC 960

TGGTTCCTGT CTGTTATTTG TAAACTGAGG ACCACAATAA AGAAATCTTT ATATTTATCG 1020

AAAAAAAAAA AAAAAAAACT CGA 1043

(2) INFORMATION FOR SEQ ID NO: 114:

(i) SEQUENCE CHARACTERISTICS:
(A) LENGTH: 703 base pairs
(B) TYPE: nucleic acid
(C) STRANDEDNESS: double
(D) TOPOLOGY: linear

(xi) SEQUENCE DESCRIPTION: SEQ ID NO: 114:

GAATTCGGCA CGAGTGCGCG GGCACCACGG CGGTTTTTCG ACGCTGGCGG TGGACGCAGG 60

CAGCATGGAC CACGGTTGCT GGGCGGATGG GGAGCGTCTA TGGTCAGTTG CCTTAGAAGT 120

GGTGAGATGG GAAGCTGCAG TTGGAAGACC CTGGAGGATG CCTGACAAGG GGATGTCTGA 180

CACATGATTG GAGCTCTTTT TGAAATGTTT CTTGCCCTTC CTGGAGCAGA GGAGCCATTA 240

TTTATGCAGG TACATCGAAG TCTTTTGACC TCCATACAGT GATTATGCTT GTCATCGCTG 300

GTGGTATCCT GGCGGCCTTG CTCCTGCTGA TAGTTGTCGT GCTCTGTCTT TACTTCAAAA 360

TACACAACGC GCTAAAAGCT GCAAAGGAAC CTGAAGCTGT GGCTGTAAAA AATCACAACC 420

CAGACAAGGT GTGGTGGGCC AAGAACAGCC AGGCCAAAAC CATTGCCACG GAGTCTTGTC 480

CTGCCCTGCA GTGCTGTGAA GGATATAGAA TGTGTGCCAG TTTTGATTCC CTGCCACCTT 540

GCTGTTGCGA CATAAATGAG GGCCTCTGAG TTAGGAAAGG TGGGCACAAA AATCTTCATG 600

AGCAATACTT CTTAGTAGAT TGTTTTGTTA TTCAAATCAA GTTCTAGTGT TTTTATGTGA 660

GATTATATAA TTTACAGTGT TGTTTTATAT ACTTTTGAAT AAA 703

(2) INFORMATION FOR SEQ ID NO: 115:

(i) SEQUENCE CHARACTERISTICS:
(A) LENGTH: 3684 base pairs
(B) TYPE: nucleic acid
(C) STRANDEDNESS: double
(D) TOPOLOGY: linear

(xi) SEQUENCE DESCRIPTION: SEQ ID NO: 115:

GGCAGAGGGG GCATGAGCAG GAGGAGGATT ACCGCTACGA GGTGCTCACG GCCGAGCAGA 60

TTCTACAACA CATGGTGGNA ATGTATCCGG GAGGTCAACG AGGTCATCCA GAATCCAGCA 120

ACTATCACAA GAATACTCCT TAGCCACTTC AATTGGGATA AAGAGAAGCT AATGGAAAGG 180

TACTTTGATG GAAACCTGGA GAAGCTCTTT GCTGAGTGTC ATGTAATTAA TCCAAGTAAA 240

AAGTCTCGAA CACGCCAGAT GAATACAAGG TCATCAGCAC AGGATATGCC TTGTCAGATC 300

TGCTACTTGA ACTACCCTAA CTCGTATTTC ACTGGCCTTG AATGTGGACA TAAGTTTTGT 360

ATGCAGTGCT GGAGTGAATA TTTAACTACC AAAATAATGG AAGAAGGCAT GGGTCAGACT 420

ATTTCGTGTC CTGCTCATGG TTGTGATATC TTAGTGGATG ACAACACAGT TATGCGCCTG 480

ATCACAGATT CAAAAGTTAA ATTAAAGTAT CAGCATTTAA TAACAAATAG CTTTGTAGAG 540

TGCAATCGAC TGTTAAAGTG GTGTCCTGCC CCAGATTGCC ACCATGTTGT TAAAGTCCAA 600

TATCCTGATG CTAAACCTGT TCGCTGCAAA TGTGGGCGCC AATTTTGCTT TAACTGTGGA 660

GAAAATTGGC ATGATCCTGT TAAATGTAAG TGGTTAAAGA AATGGATTAA AAAGTGTGAT 720

GATGACAGTG AAACCTCCAA TTGGATTGCA GCCAACACAA AGGAATGTCC CAAATGCCAT 780

GTCACAATTG AGAAGGATGG TGGTTGTAAT CACATGGTCT GTCGTAACCA GAATTGTAAA 840

GCAGAGTTTT GCTGGGTGTG TCTTGGCCCA TGGGAACCAC ATGGATCTGC CTGGTACAAC 900

TGTAACCGCT ATAATGAGGA TGATGCAAAG GCAGCAAGAG ATGCACAGGA GCGATCTAGG 960

GCAGCCCTGC AGAGGTACCT GTTCTACTGT AATCGCTATA TGAACCACAT GCAGAGCCTG 1020

CGCTTTGAGC ACAAACTATA TGCTCAGGTG AAACAGAAAA TGGAGGAGAT GCAGCAGCAC 1080

AACATGTCCT GGATTGAGGT GCAGTTCCTG AAGAAGGCAG TTGATGTCCT CTGCCAGTGT 1140

CGTGCCACAC TCATGTACAC TTATGTCTTC GCTTTCTACC TCAAAAAGAA TAACCAGTCC 1200

ATTATCTTTG AGAATAACCA AGCAGATCTA GAGAATGCCA CAGAGGTGCT CTCGGGCTAC 1260

CTTGAACGAG ATATTTCCCA AGATTCTCTG CAGGATATAA AGCAGAAAGT ACAAGACAAG 1320

TACAGATACT GTGAGAGTCG ACGAAGGGTT TTGTTACAGC ATGTGCATGA AGGCTATGAA 1380

AAAGATCTGT GGGAGTACAT TGAGGACTGA GAATGGCCCT GCATAAAATG AACTCTGAAA 1440

ACTTTACCAT CTAGAGTGCT CATGCAATTA AAACAAAACA AACACAAACA AGGAGGCACT 1500

AAGCCTATTC TGACACCACT GGTCTGTAGT ACCAGAATTG TTTTGTTAAT GGAAAGTTTA 1560

AGTAAATTAT ATTGTAATAA AAAGGTAGAT AAACCATTGT ACAACAGTAT TCTAGGCCGC 1620

CAACAAAAGT GTGACAGACA CACTAAAAGC CCTCCAACTT TAACTTGTAA CGTAGCTTCA 1680

TTCTCAAAGC TGACTCCTTT TTTTTCTTTT TCCTTTTCCT GAGTGTAGTA CAGTTAAAAT 1740

TTCAAACAGC TCCTTGACAC TGCTTTTCAT GTTCAAACCA GCCATTTTGT TGTACTTTGG 1800

TAAAGGACCT CTTCCCCTTC CTCCCCTACA CATACAGATA CACCCACACA CAGACTGACT 1860

CTCTTTCTCT CATACCCCAA GGTCATGAGT GAATGATGCT TAGTTCCTTG TAAAGAAAAT 1920

CTTGGGATGG GGAAAGGGGT AGGCAGCAAG AGGATTCAAC AAACGAAAAA CATAAAAACT 1980

TTGTATATGA CTTTTAAAAC AAGAGGACAA CACAGTATTT TTCAAAATTG TATATAGCGC 2040

ATATGCATGG ACAAAGCAAG CGTGGCACGT GTTTGCATAA TGTTTAATTA CAAAAAAATA 2100

TTTATTCTTT AAAAATCTTC AAGATTATGT CTATTTGCTG TGCATTTTCT TTCAGTTTGC 2160

TTATCTTTCC CGGGTTGGGG TTGGGATAAA GGTGTGTCGG TTTAGCACCT CTGGAAGACC 2220

TATCTAGAGC TCTTTCACTT TCCTGAGGTT ATTTTGCCCY TTCTGGTGTT GGTATGTCTG 2280

TTGCCGGCCA TGGGCTNCAY GCCTTGAATT CCTGCTCTTG ATCAGGGACA AGGGAGGTCA 2340

AGCTCTGACT AATGCCATGA CCTGATTAAG GGGTACAGCA GGGAGTTTTG TTGCTACAGC 2400

TCATGAATTA ACCTGTCCCA ACCTAATCCC CCTCCATGGC ATCATGCCTC TACCCAAGCC 2460

TTTGTGTGCC CATGTTATGC ACACAGCTGT AGGCATTCTT AAGTCCCCTG TCGCATCCAG 2520

TGGAAGCATT TTAAAATTTC TTTTACTTTT TGGTTTTCCC TTAATTGCTG CTTTTCAGAT 2580

TTTAGTTATG GCTCGTCTGC TCACCCCTTC TCTACATTAG GGTGTCAAAG AGAATGTTTT 2640

GCTTTAAATA TAAATAGCCA TTCATTTAGT CTCAGATTGT GAATTTAAAA TGGTGGATAC 2700

CGAAATTGCT TGTGTGTGTT GCTGTGGGTT TGGTTTGAAG GCAAACACCC CTAGAACATG 2760

ATATTCCCAT CTAGTGCATT TAAATAGAAA TCACTGAGTT TGCTGCTTTT TTATTGTCAG 2820

CAGATAGGAG AATTAATAAT GCATTTTAGC TGTGATGTCC ATTTTTATGA AATTCCTACT 2880

AAGAGCTATG TTAAAAGTAA AGGATGGTGG TGGTTGTATT AACTATATAC CTGTTTAGGC 2940

CATTCTGGCT GTGGTATTTT TCAATAGGTC AGCATCTGTA AATCTGTCAG TTTTATACAG 3000

GAGTGCAGAG TGAACTAGGC AACTAGATTA AGAGGTCTAA ATATGAAATA CCAGTTGAGG 3060

CTGAGGACCT CTTCGTCTTC CTTTAAATGT CTTTTGCCTA GGGAGTGTTT ACCATTTGTG 3120

AGGCAGCTTT GTCTGCTCTT ACACTGTACA TCCTATTACT CCATTGGGAA GTAGGTTCAC 3180

TTTCCTCTGG CCTTTTGCCT AAGTTAGGCT TTGCTGAATC AACCCTACTT TTCCTTTTAG 3240

AAAAGGTTGT TACAGGAGAT TTACTGGCAA CTGTTCTTTT CCCATCAAAA ATCAGTGAAT 3300

GTTTGCTGAG TATAAATGCT GCTTCCTTAA ACCACTTGTC GCTTTAGGAT CAACTTTACC 3360

TGTACCTTTT CTCCTTTCCT CCCTTGCCAC CTCAGGTGCA AATCTGAACT CAGTGTCTGC 3420

TTCTTCCATT TTCTCGTCTC TCTCCCCTCT TCCCCCATTA TCCATATGAC ATTATTTTAC 3480

TTCAAATGAC AGCATCAATC TTAAAAAGAT ATACATTAAA ACTAAGGAGT TTTTTTAAAG 3540

AAAGCCTGAA TAAGTTCCTT TCCCTGGTAA CTTTGAAAAG CAGTCAGAGT TGCTATATAG 3600

ATATATGTGG CTCCTTTAAA ATGCTTTGTG TATGTGTGGT GTTTAAAAAA AAAAAAAAAA 3660

TTCGGGGGGG GGCCCGGTNC CCAT 3684

(2) INFORMATION FOR SEQ ID NO: 116:

(i) SEQUENCE CHARACTERISTICS:
(A) LENGTH: 1965 base pairs
(B) TYPE: nucleic acid
(C) STRANDEDNESS: double
(D) TOPOLOGY: linear

(xi) SEQUENCE DESCRIPTION: SEQ ID NO: 116:

AAGAAAGGGT ATTAAAATTC TAGATCACAT ATGGACCCGG GAAGGTTTTT NACCCTCTGT 60

TAGTGACATC GAGTCTCCCA CTAGACAAAA TAGGTGGAAA AATCTCTCGA GGGCTCACAT 120

TGTTTTGTCA TCTTCAGGAA AAACACCACC AGGCCATACC ACAGCCTGCC CAGTGAGGCG 180

GTCTTTGCCA ACAGCACCGG GATGCTGGTG GTGGCCTTTG GGCTGCTGGT GCTCTACATC 240

CTTCTGGCTT CATCTTGGAA GCGCCCAGAG CCGGGGATCC TGACCGACAG ACAGCCCCTG 300

CTGCATGATG GGGAGTGAAG CAGCAGGAAG GGGCTCCCAA GAGCTCCTGG TGGTGCAGCC 360

TGTGCTCCCC TCAGAAGCTC TGCTCTTCCC AGGGCTCCCG GCTGGTTTCA GCAGGCGACT 420

TTCTTCCAAT GCTGGGCCCA GACTTCTTGC CTGGGTGCTG GCCTGCCCTC TCCGGNCCGC 480

TTGCTGCCTG TCTGCTTTCC TTGGTGGYTT TGCTGGGTGC TGGGCCTGCC CTCTCCGGCC 540

GCTTGCTGCC TGTCTGCTTT CCTTGGTGGC TTTGCTGGGT GCTGGGCCTG CCTTCTCTGG 600

CTGCTTGCTG CCTGTCTGCT TTCCTTGGTG GCTTTGGCTT CTGCACTCCT TGGCGTCASC 660

TCTCAGGTCC TCCATTCACA CGAGGTCCTC CTCGCTCTGG CCGCTCTTGC TGCTCCTGTC 720

TGAAGAWATC AGACTGATTT CCTCTTAAGA CTCCTAGGGA TGTGGTGAAG AGCTGGGACT 780

CAAGTGCAGT CCACGGTGTG AAACATGAGG GARGTGAGGT GTCCGTCCAC TTCCCCCATA 840

AAGGTGTGCA TTTCAGTTAG GCTGCCCCGC CACAGAGCAG GCTTCATCTG CTCTGCCATC 900

CAGCCCCATC TGGATGTGAG GTGGGGTGGA GACATCATGG GGTGATTGCA GAAAGGGGGA 960

GTGGCGGCCC ACGCAGCTTC TGCTGAGGAG CTGACCGCTC TGAGCTGTTC TGTTTCGTAT 1020

TGCTGCTCTG TGTCTGCATG TATTGTGACC GTGCGGCTCC ACCTCTTCCA GCTGCTGCTA 1080

CAGCTGAGGC CTGGATCCCG GCCTTTCCCT GTGACTTACG TGTCTGTCAC CGGCANGCAG 1140

CCCTACAAAT CCTGGTGACC TGCTCTCCCA AGAACAGAGC CTGTCCCCAG ATGTCCCAGT 1200

AGCGATGAGT AACAGAGGTG GCTGTGGACT TCCTCTACTT CTCCTTGCTG GATCAGGGCC 1260

TTCCTGCCTC CCGCTGGGCA GGTCTGGCCT TGCTCTCTTG GCAGGGCCCC AGCCCCTCTG 1320

ACCACTCTGC AGCTCACCAT GCAGCTGATG CCAAAGTTGT GGTGTCCAGT GTGCAGCAGC 1380

CCTGGGAGCC ACTGCCACCT TCAGAGGGGT TCCTTGCTGA GACCCACATT GCTTCACCTG 1440

GCCCCACCAT GGCTGCTTGC CTGGCCCAAC CTAGCGTTCT GTGCCATGCT AGAGCTTGAG 1500

CTGTTGCTCT TCTTCAGGGG AGGAAATAGG GTGGAGAGCG GGAAGGGTCT TGCTCCTAAG 1560

TGTTGCTGCT GTGGCTTTTT TGCCTTCTCC AAAGACGCAC TGCCAGGTCC CAAGCTTCAG 1620

ACTGCTGTGC TTAGTAAGCA AGTGAGAAGC CTGGGGTTTG GAGCCCACCT ACTCTCTGGC 1680

AGCATCAGCA TCCTACTCCT GGCAACATCA GGCCAACGTC CACCCCAGCC TCACATTGCC 1740

AGATGTTGGC AGAAGGGCTA ATATTGACCG TCTTGACTGG CTGGAGCCTT CAAAGCCACT 1800

GGGATGTCCT CCAGGCACCT GGGTCCCATG ACCAGCTCCC CGTCTCCATA GGGGTAGGCA 1860

TTTCACTGGT TTATGAAGCT CGAGTTTCAT TAAATATGTT AAGAATCAAA GCTGTCTTTG 1920

TTCAGGCTGC TATAACAAAA ATATAATAGC CTGGGTGGCT TAAAC 1965

(2) INFORMATION FOR SEQ ID NO: 117:

(i) SEQUENCE CHARACTERISTICS:
(A) LENGTH: 503 base pairs
(B) TYPE: nucleic acid
(C) STRANDEDNESS: double
(D) TOPOLOGY: linear

(xi) SEQUENCE DESCRIPTION: SEQ ID NO: 117:

AGTGATCCCC TTGCCTCGGC CTCCCAAAAT GCTGGAATTG TAAGCGTGGG CCTCTGCACC 60

CGGCCTGGTC CGCAATTTAA AAACGCACAG CCACCATTCC CTYTCCAGAA AGCACCCAGA 120

TGCCTTTGGG AGAACCAGCC TCCTCCATGG AGGAAAGCTT GGGATCTGCC TTCCCACCTG 180

GGGAGGAGAG GGATCTGTGG AAAATCCTTC TGACGGACTT CCCCTCAGTG CCTGATCCAT 240

ACTCAATAGT AGAAAAAGTA AGAAATATAC AAAGATAGCA GATACACGGA GACAGTTCCC 300

CAAATAGCTG AGCGAWTAGC GCAGAAGCAA TATTGAAGAC CTAATAGCTG AGACATTTCC 360

AGAACTGATA AAGTGCATCC AGCCACAGAT CAAGCAGCCC AGAAAATTCC AGGCAGCATC 420

AACAAATAAA TAGCCCCACA TGCACCCGTG AAAATGCAGA AGACCAAACA AAAAAGTCCG 480

GTCAACAGCC AGAGTTAAAG AGG 503

(2) INFORMATION FOR SEQ ID NO: 118:

(i) SEQUENCE CHARACTERISTICS:
(A) LENGTH: 1133 base pairs
(B) TYPE: nucleic acid
(C) STRANDEDNESS: double
(D) TOPOLOGY: linear

(xi) SEQUENCE DESCRIPTION: SEQ ID NO: 118:

GGCACAGCTT GGAATGAACC CCTGTGGATA AGGGGGACTA TTAGATAGAA TAAACATCAA 60

TAAATGCTTG ATGAATAAAC GCTAATCCTA CCTTCCCAGC CTGACACCTC CCAGTGGACA 120

CCACACTTCA CTTGAAGCCT TAGAAACCTT TCCCACCCAT GCTTCCAGCC CTGGCTTCAT 180

GTTGCCATTT CTCACCCCCA GAACAGGCCG CCCGCCTGAA GAAACTACAA GAGCAAGAGA 240

AACAACAGAA AGTGGAGTTT CGTAAAAGGA TGGAGAAGGA GGTGTCAGAT TTCATTCAAG 300

ACAGTGGGCA GATCAAGAAA AAGTTTCAGC CAATGAACAA GATCGAGAGG AGCATACTAC 360

ATGATGTGGT GGAAGTGGCT GGCCTGACAT CCTTCTCCTT TGGGGAAGAT GATGACTGTC 420

GCTATGTCAT GATCTTCAAA AAGGAGTTTG CACCCTCAGA TGAAGAGCTA GACTCTTACC 480

GTCGTGGAGA GGAATGGGAC CCCCAGAAGG CTGAGGAGAA GCGGAACNTG AAGGAGCTGG 540

CCCAGAGGCA ANGAGGAGGA GGCAGCCCAG CAGGGGCCTG TGGTGGTGAG CCCTGCCAGC 600

GACTACAAGG ACAAGTACAG CCACCTCATC GGCAAGGGAG CAGCCAAAGA CGCAGCCCAC 660

ATGCTACAGG CCAATAAGAC CTACGGCTGT KTGCCCGTGG CCAATAAGAG GGACACACGC 720

TCCATTGAAG AGGCTATGAA TGAGATCAGA GCCAAGAAGC GTCTGCGGCA GAGTGGGGAA 780

GAGTTGCCGC CAACCTCCTA GGCGCCCCGC CCAGCTCCCT TTGACCCCTG GGGCAGGGCA 840

GGGGGCAGGG AGAGACAAGG CTGCTGCTAT TAGAGCCCAT CCTGGAGCCC CACCTCTGAA 900

CCACCTCCTA CCAGCTGTCC CTCAGGCTGG GGGAAAACAG GTGTTTGATT TGTCACCGTT 960

GGAGCTTGGA TATGTGCGTG GCATGTGTGT GTGTGTGTGA GAGTGTGAAT GCACAGGTGG 1020

GTATTTAATC TGTATTATTC CCCGTTCTTG GAATTTTCTT CCCATGGGGC TGGGGTACTT 1080

TACATTCAAT AAATACTGTT TAACCCAAAA AAAAAAAAAA AAAAGAAAGA AGN 1133

(2) INFORMATION FOR SEQ ID NO: 119:

(i) SEQUENCE CHARACTERISTICS:
(A) LENGTH: 1101 base pairs
(B) TYPE: nucleic acid
(C) STRANDEDNESS: double
(D) TOPOLOGY: linear

(xi) SEQUENCE DESCRIPTION: SEQ ID NO: 119:

GGGCACAGCT GAAGCTGCAG ACCTCCCCAG GGGATGGCTC CTCTCCCCCA GGAGCCCCGA 60

GGCAGGGGAG GCAGAAAGCC TGGGCTCTGG GGGGTGGCCT GCGGACAGCT GTGCTGTGGG 120

CCGGGGGCTG GGCCTGTCCC ACAGGGNCGT GGAGCTCGTG GTTCTGAGCA GCCAGCTGGG 180

TGGTGTCTGG GGATAGCTGG GAGGCACAGC GGCTGCCATG TGGGACTGGG ACTGGAGTGC 240

TCCCTGGTCT TGGCCTCTGT GGCTCAGCCT TGCTCTGGTC TGCCTGAGTG CAGGGGCCAA 300

GGGGCACAGG GCCAGTGAGG CCGGCCACGC TCGGGCCCTC ACCTGTGAGA TGGGGTCGGA 360

ATTTKACACA GCCTANGGCT TGGTTCTTGG TKGTNGAMCG TGGACTYCTK AGAACGGGAG 420

TGCTGGTCCT GAAAGGCGTG GTTGGAGACC AGCTGCTTTT CTCGCTGTTT TTCTCTTAGG 480

AGATTAAACA AAAACAGAAA GCACAAGACG AACTCAGTAG CAGACCCCAG ACTCTCCCCT 540

TGCCAGACGT GGTTCCAGAC GGGGAGACGC ACCTCGTCCA GAACGGGATT CAGCTGCTCA 600

ACGGGCATGC GCCGGGGGCC GTCCCAAACC TCGCAGGGCT CCAGCAGGCC AACCGGCACC 660

ACGGACTCCT GGGTGGCGCC CTGGCGAACT TGTTTGTGAT AGTTGGGTTT GCAGCCTTTG 720

CTTACACGGT CAAGTACGTG CTGAGGAGCA TCGCGCAGGA GTGAGGCCCA GGCGCCGAGA 780

CCCAAGGCGC CACTGAGGGC ACCGCGCACC AGAGCGTGAC CTCGGCAGGC TGGACACACT 840

GCCCAGCACA GGCAGACCCA CCAGGCTCCT AGGTTTAGCT TTTAAAAACC TGAAAGGGGA 900

AGCAAAAACC AAAATGTGTG ACTGGGCTTT GGAGGAGACT GGAGCCTCAG CCCTGTCCTG 960

GCCACGGGCC GCTGGGGCTG GTGTGGGTGG GCCTTGTGTG CTGGATTTGT AGCTTATCTT 1020

CCGTGTTGTC TTTGGACCTG TTTTAGTAAA CCCGTTTTTC ATTTTAAAAA AAAAAAAAAA 1080

AAACTTTGGG GGGGGGCCCC N 1101

(2) INFORMATION FOR SEQ ID NO: 120:

(i) SEQUENCE CHARACTERISTICS:
(A) LENGTH: 282 base pairs
(B) TYPE: nucleic acid
(C) STRANDEDNESS: double
(D) TOPOLOGY: linear

(xi) SEQUENCE DESCRIPTION: SEQ ID NO: 120:

AGCTTCTCTG TCCAGTCTTG AACTCTGGGS TCTCTTGGAA CTTTCCTCAC CCCTCTCAGC 60

CTGAATATTC CTTCCATGGA TTCCACTCAA CCAGACTTTG GATCTGTGCC TACTTAATCA 120

ACCTTATCTT TGCAATATGT TCGGGCCCAC CTTCCACTCC TTGGTTCTTG TTCCTCCTTG 180

GCCTAACTTG TCCCTTCTCC ACTTCACATC CCCGGTGGGA CAGCATTCCT CCTTCCTCCC 240

AACCTCCCTC CGTCTCARAA AAAAAAAAAA AAAAAAAAAA TT 282

(2) INFORMATION FOR SEQ ID NO: 121:

(i) SEQUENCE CHARACTERISTICS:
(A) LENGTH: 2635 base pairs
(B) TYPE: nucleic acid
(C) STRANDEDNESS: double
(D) TOPOLOGY: linear

(xi) SEQUENCE DESCRIPTION: SEQ ID NO: 121:

TAAGGGGGTG TGTGCTCACC TCCTCCTGAC CCTTAACACT CCTGTCCTGC CCAGACCAAC 60

AGAGAGAGCT GTCCCTGAGA CCCCGGAGAG AAGCAGCTGC CGAAAGCTGC AGCCTTTCCG 120

CACTCTGAGA CCATGATCTT CCTCCTGCCA GGGGAGAGCC ACCCACAGGC CATGTCCAGC 180

CCCACTTCCC TCAGCCCCCA GGGYTTCCTT CTGGCCCCTC TGAGGATTCC CTAGGGCTGC 240

CCCGCAGAGG GGYTTCCCCA AGCTCTGTTT TGAAGCCTGC AATGTGGAAA AGTGAGAAGT 300

CAGAGGGAAC AGGACAGGTG CAGCCGGGCT CTGAGGCCAC ACCTCACACC TCGCTGTTCC 360

CCAACATCCC CTGAGCAGTG TGAGCTCATC TCACCAGATG AGAAGAGGCC CTGTGCATTT 420

YTTTTGTTTG TTTGTTGCTG TTTTCCCCCA CCCATCCAGT TCTCCTCAGC AAAGCAAATT 480

CCTTAACACC TTTGGTGGAG AATTTCTTAC CCAGACTTGG GGCTGTGATG CCCTTCAGTG 540

CGTGGTGAGT GCAGCGTGTG TGCGTGTGCC TGTGTGTGAA CCTGGGGGCC ATCCTGGTGG 600

CCTGGGAGCG TGAGGAGAGG CCCCCTGTGT GCTGGGTGAG TGGTGGGTGT GGGGTCAATG 660

CAGTGAGGCT CTCTGGGTGA GGCTCCCAAC CTGGCAGTCC CCAGCCTCCC AGCATCTGTG 720

AGCGTCTGTT GGACTTTACA GAAGAGCCTC ATCCYGTCTG CCCCTCACTC TGCCCTGGAA 780

TCAACATCTT CCGAGTCCTT CTTGGGGGAA ATAGCAGAGC CCCACTTAAC TCCATAAACT 840

GCTTCCCATT CCGCAGCCCA GTTCTGATTG TTGAGGTGTC GCGTCGTTCC AGGTCCCCCA 900

GTCCCCTCTT TCTCCTGTCC TCTCTCTGTC CTTCACCTCC CCACTCCAGC CCCGGCTCAG 960

TTCAGGGAAA TGCTGTTCCA YATCAGCCCT CTGCTCTCTG AGGCAGCCGC GCCTCTGACT 1020

CGGAGCTACT TGAAACTTCT GCTCTTGCTA GGATTGGAGT CTACCTATCT CTTCCATTTG 1080

TCCCAGCTGG AGTTCTGGAA CTTTCCTCCT CGGGGTGGGG GTGGGGGTTG TTAAGGATGC 1140

TGGGGGGCCT GGGGAAGGAA GGAGTTCAGA GGAAGGGTGT CCCCTGTCCT CTTGATGTCA 1200

CCCTCCGCTC CTGGGACACG TGCTCTCTCT GTCTCTGGGT CTTCTGGCTG TGCACGTTTG 1260

TGTGTCCTTG TAAATATGTT TTAGGAAGAA AGCAAAAGGG ACTGAACTAG CCTCTGGTAG 1320

GATTGCAGGG GTCCAGCCTT GCCTGTTTCC GAAGCCCCCA CACTGCCTTT CGCCCCACTG 1380

AGACTGGTCC CCTCAAAAGG TAGACAAAAC AGCAGCTCCG TGTGGAGCTG AAGGGCGGCC 1440

TCAAAGTGGC TTTTTGTTAG ACAAGGTTAA GGTTTCCTCA TGAGCAAGGT TGTAGATCGG 1500

TCCTTCCTCA GCTCCTTGAT TTGTGACCTT GACCAAGGGG CCTGCCACCC AGCCCCTCCA 1560

GTGCCCTCTC CTCGATGCCT CGCTCCTTCC TGCCCCCACT CCCCTGGTTT AGGTAGGTAG 1620

GGGAATTAGG GCCATGCTGG AAGAAGCTTA ACCATGTGTT CAAAGAAGGG TTTCTTGCTT 1680

GCTTGGTCCT GGAACTCCCC TTGGCTGCCC CAGGCCTCCT TGGCCCATGG GTGCTGGGGG 1740

AGGTGGATGT CAGATCTGGT AGGTTGCAGC AGAGAAAATA AATGTGCTTT GAGAGACCAC 1800

TCAGAGAGGG TCCAAGGGTG ATGGAGAAGG AAGCATGGCC TGGGAGCTTG GAAGGGARGG 1860

GTGGTGGGTG GCGGCATCTT GACTGCCCCC TGTTGTCCCA CACGTGGGGG GTGGTCACCC 1920

CYCTTCACTC CAGCCCGCCT GCCTTCAGCC TTCCATGAGC TTCACCTGCT TCCAACTTCA 1980

CTTTGGAGGG GGTGGGGTCC GTTGGCATCA ACACGGGGAC CCTCTGCTTC ACTAAAGCCC 2040

GAGCCCTCAG CCCCTGGGGA GAACAAATGG CTGAGCTTTG ATACCTGGGG TCGTCGAGAG 2100

GCTGCGGGCT GGCGGCAGTC CCAGGGGAGA GACACCACAG AAGGAGACCC AGACATCCCG 2160

AGGAAGTTCC CAGCAGAGCA AACTGCTTTC CAGCCTGAAG CCTGCTTAAA CTGTGTGATG 2220

TGCAATAACT GAGCTTAGAG TTAGGAATTG TGTTCAAGTG CTTGGATTTC CGTCTGTAGA 2280

TTTAACTGCT GAAATTGTAT CTCTCAGTAA TTTTAGATGT CTTTTAAAAA ATTGAAAAAC 2340

AAAGTGTTAG ACTGTGTGCG TGTGCGTTGA TGGGCACTCA AGAGTCCCGT GAGTCATCCA 2400

GCCCTGCCTT TCCCCTGCGC CCCCATCCTC TCACGTCCCG CCCYGCCTCC ACTTGGGGAC 2460

CCTGCCTCGT GTCGTCTTTA TCTGCCTATT ACTCAGCCTA AGGAAACAAG TACACTCCAC 2520

ACATGCATAA AGGAAATCAA ATGTTATTTT TAAGAAAATG GAAAATAAAA ACTTTATAAA 2580

CACCAAAAAA AAAAAAAAAA ACCCNGGGGG GGGGCCGGTA ACCCATTTCG CCTAA 2635

(2) INFORMATION FOR SEQ ID NO: 122:

(i) SEQUENCE CHARACTERISTICS:
(A) LENGTH: 994 base pairs

(B) TYPE: nucleic acid
(C) STRANDEDNESS: double
(D) TOPOLOGY: linear

(xi) SEQUENCE DESCRIPTION: SEQ ID NO: 122:

GAATTCGGCA GAGGTTCGGC GAAGATAGGG AATAAGGAAG CACAGGAGTA GGGGAGAAGG 60

AAGCACAGGA GTAGGGGAGA TATACAGCGG TCAGGATAAG GGGGAAAGGG CGGTGGTTGC 120

SCAAGAGGTG AAACAAGATG TGAGAGACAA GGGGTAGGGA AGAAATGGGG CAGCGGTTAG 180

GTTCAGAAGC GCATAGACCG TGGCGGACGG GCAATGCGAG GGGCACAGAA AGGAACTGAG 240

GGGTGGGCTA TTTTAARGGA GATGGTCCTT CAGCCCTCTT YTTTTCTGCG TAGTTCTCCT 300

CCTCCAGGCC GCGCGCGGAT ATGTCGTCCG GAAACCAGCC CAGTCTAGGC TGGATGATGA 360

CCCACCTCCT TCTACGCTGC TCAAAGACTA CCAGAATGTC CCTGGAATTG AGAAGGTTGA 420

TGATGTCGTG AAAAGACTCT TGTCTTTGGA AATGGCCAAC AAGAAGGAGA TGCTAAAAAT 480

CAAGCAAGAA CAGTTTATGA AGAAGATTGT TGCAAACCCA GAGGACACCA GATCCCTGGA 540

GGCTCGAATT ATTGCCTTGT CTGTCAAGAT CCGCAGTTAT GAAGAACACT TGGAGAAACA 600

TCGAAAGGAC AAAGCCCACA AACGCTATCT GCTAATGAGC ATTGACCAGA GGAAAAAGAT 660

GCTCAAAAAC CTCCGTAACA CCAACTATGA TGTCTTTGAG AAGATATGCT GGGGGCTGGG 720

AATTGAGTAC ACCTTCCCCC CTCTGTATTA CCGAAGAGCC CACCGCCGAT TCGTGACCAA 780

GAAGGCTCTG TGCATTCGGG TTTTCCAGGA GACTCAAAAG CTGAAGAAGC GAAGAAGAGC 840

CTTAAAGGCT GCAGCAGCAG CCCAAAAACA AGCAAAGCGG AGGAACCCAG ACAGCCCTGC 900

CAAAGCCATA CCAAAGACAC TCAAAGACAG CCAATAAATT CTGTTCAATC ATTTAAAAAA 960

AAAAAAAAAA AAAAAAAAAA AAAAAGGGGA GGGG 994

(2) INFORMATION FOR SEQ ID NO: 123:

(i) SEQUENCE CHARACTERISTICS:
(A) LENGTH: 1542 base pairs
(B) TYPE: nucleic acid
(C) STRANDEDNESS: double
(D) TOPOLOGY: linear

(xi) SEQUENCE DESCRIPTION: SEQ ID NO: 123:

GGCASAGCCA CCTCGGCCCC GGGCTCCGAA GCGGCTCGGG GGCGCCCTTT CGGTCAACAT 60

CGTAGTCCAC CCCCTCCCCA TCCCCAGCCC CCGGGGATTC AGGCTCGCCA GCGCCCAGCC 120

AGGGAGCCGG CCGGGAAGCG CGATGGGGGC CCCAGCCGCC TCGCTCCTGC TCCTGCTCCT 180

GCTGTTCGCC TGCTGCTGGG CGCCCGGCGG GGCCAACCTC TCCCAGGACG ACAGCCAGCC 240

CTGGACATCT GATGAAACAG TGGTGGCTGG TGGCACCGTG GTGCTCAAGT GCCAAGTGAA 300

AGATCACGAG GACTCATCCC TGCAATGGTC TTAACCCTGC TCAGCAGACT CTCTACTTTG 360

GGGAGAAGAG AGCCCTTCGA GATAATCGAA TTCAGCTGGT TAMCTCTACG CCCCACGAGC 420

TCAGCATCAG CATCAGCAAT GTGGCCCTGG CAGACGAGGG CGAGTACACC TGCTCAATCT 480

TCACTATGCC TGTGCGAACT GCCAAGTCCC TCGTCACTGT GCTAGGAATT CCACAGAAGC 540

CCATCATCAC TGGTTATAAA TCTTCATTAC GGGAAAAAGA CACAGCCACC CTAAACTGTC 600

AGTCTTCTGG GAGCAAGCCT GCAGCCCGGC TCACCTGGAG AAAGGGTGAC CAAGAACTCC 660

ACGGAGAACC AACCCGCATA CAGGAAGATC CCAATGGTAA AACCTTCACT GTCAGCAGCT 720

CGGTGACATT CCAGGTTACC CGGGAGGATG ATGGGGCGAG CATCGTGTGC TCTGTGAACC 780

ATGAATCTCT AAAGGGAGCT GACAGATCCA CCTCTCAACG CATTGAAGTT TTATACACAC 840

CAACTGCGAT GATTAGGCCA GACCCTCCCC ATCCTCGTGA GGGCCAGAAG CTGTTGCTAC 900

ACTGTGAGGG TCGCGGCAAT CCAGTCCCCC AGCAGTACCT ATGGGAGAAG GAGGGCAGTG 960

TGCCACCCCT GAAGATGACC CAGGAGAGTG CCCTGATCTT CCCTTTCCTC AACAAGAGTG 1020

ACAGTGGCAC CTACGGCTGC ACAGCCACCA GCAACATGGG CAGCTACAAG GCCTACTACA 1080

CCCTCAATGT TAATGACCCC AGTCCGGTGC CCTCCTCCTC CAGCACCTAC CACGCCATCA 1140

TCGGTGGGAT CGTGGCTTTC ATTGTCTTCC TGCTGCTCAT CATGCTCATC TTCCTTGGCC 1200

ACTACTTGAT CCGGCACAAA GGAACCTACC TGACACATGA GGCAAAAGGC TCCGACGATG 1260

CTCCAGACGC GGACACGGCC ATCATCAATG CAGAAGGCGG GCAGTCAGGA GGGGACGACA 1320

AGAAGGAATA TTTCATCTAG AGGCGCCTGC CCACTTCCTG CGCCCCCCAG GGCCCTGTGG 1380

GGACTTGCTG GGGCCGTCAC CAACCCGGAC TTGTACAGAG CAACCGCAGG GGCCGSCCCT 1440

CCCGNTTGTT CCCCAGCCCA CCCACCCCCT TGTTACAGAA TGTYTKGTTT GGGGTGCGGT 1500

TTTGTWATTG GTTTNGGATN GGGGAAGGGA GGGANGGCGG GG 1542

(2) INFORMATION FOR SEQ ID NO: 124:

(i) SEQUENCE CHARACTERISTICS:
(A) LENGTH: 1390 base pairs
(B) TYPE: nucleic acid
(C) STRANDEDNESS: double
(D) TOPOLOGY: linear

(xi) SEQUENCE DESCRIPTION: SEQ ID NO: 124:

CAAGCTCTAA TACGACTCAC TATAGGGAAA GCTGGTACGC CTGCAGGTAC CGGTCCGGAA 60

TTCCCGGGTC GACCCACGCG TCGGGGCCTC AGGGTGGACG CATGGTTCTG CACTGAGGCC 120

CTCGTCATGG TGGCGCCTGT GTGGTACTTG GTAGCGGCGG CTCTGCTAGT CGGCTTTATC 180

CTCTTCCTGA CTCGCAGCCG GGGCCGGGCG GCATCAGCCG GCCAAGAGCC ACTGCACAAT 240

GAGGAGCTGG CAGGAGCAGG CCGGGTGGCC CAGCCTGGGC CCCTGGAGCC TGAGGAGCCG 300

AGAGCTGGAG GCAGGCCTCG GCGCCGGAGG GACCTGGGCA GCCGCCTACA GGCCCAGCGT 360

CGAGCCCAGC GGGTGGCCTG GGCAGAAGCA GATGAGAACG AGGAGGAAGC TGTCATCCTA 420

GCCCAGGAGG AGGAAGGTGT CGAGAAGCCA GCGGAAAYTC ACCTGTCGGG GAAAATTGGA 480

GCTAAGAAAC TGCGGAANNT GGAGGAGAAA CAAGCGCGAA AGGCCCAGCK TGAGGCAGAG 540

GAGGCTGAAC GTGARGWGCG GAAACGACTC GAGTCCCAGC GCGAATGAGT GGAAGAAGGA 600

GGAGGAGCGG CTTCGCCTGG AGGAGGAGCA GAAGGAGGAG GAGGAGAGGA AGGCCCGCGA 660

GGAGCAGGCC CAGCGGGAGC ATGAGGAGTA CCTGAAACTG AAGGAGGCCT TTGTGGTGGA 720

GGAGGAAGGC GTAGGAGAGA CCATGACTGA GGAACAGTCC CAGAGCTTCC TGACAGAGTT 780

CATCAACTAC ATCAAGCAGT CCAAGGTTGT GCTCTTGGAA GACCTGGCTT CCCAGGTGGG 840

CCTACGCACT CAGGACACCA TAAATCGCAT CCAGGACCTG CTGGCTGAGG GGACTATAAC 900

AGGTGTGATT GACGACCGGG GCAAGTTCAT CTACATAACC CCAGAGGAAC TGGCCGCCGT 960

GGCCAACTTC ATCCGACAGC GGGGCCGGGT GTCCATCGCC GAGCTTGCCC AAGCCAGCAA 1020

CTCCCTCATC GCCTGGGGCC GGGAGTCCCC TGCCCAAGCC CCAGCCTGAC CCCAGTCCTT 1080

CCCTCTTGGA CTCAGAGTTG GTGTGGCCTA CCTGGCTATA CATCTTCATC CCTCCCCACC 1140

ATCCTGGGGA AGTGATGGTG TGGCCAGGCA GTTATAGATT AAAGGCCTGT GAGTACTGCT 1200

GAGCTTGGTG TGGCTTGGTG TGGCAGAAGG CCTGGCCTAG GATCCTAGAT AAGCAGGTGA 1260

AATTTAGGCT TCAGAATATA TCCGAGAGGT GGGGAGGGTC CCTTGGAAGC TGGTGAAGTC 1320

CTGTTCTTAT TATGAATCCA TTCATTCAAG AAAATAGCCT GTTGCAAAAA AAAAAAAAAA 1380

AAAAACTCGA 1390

(2) INFORMATION FOR SEQ ID NO: 125:

(i) SEQUENCE CHARACTERISTICS:
(A) LENGTH: 1288 base pairs
(B) TYPE: nucleic acid
(C) STRANDEDNESS: double
(D) TOPOLOGY: linear

(xi) SEQUENCE DESCRIPTION: SEQ ID NO: 125:

GGCGCGCGGG TGAAAGGCGC ATTGATGCAG CCTGCGGCGG CCTCGGAGCG CGGCGGASCA 60

GACGCTGACC ACGTTCCTCT CCTCGGTCTC CTCCGCCTCC AGCTCCGCGC TGCCCGGCAG 120

CCGGGAGCCA TGCGACCCCA GGGCCCCGCC GCCTCCCCGC AGCGGCTCCG CGGCCTCCTG 180

CTGCTCCTGC TGCTGCAGCT GCCCGCGCCG TCGAGCGCCT CTGAGATCCC CAAGGGGAAG 240

CAAAAGGCGC ATCCGGCAGA GGGAGGTGGT GGACCTGTAT AATGGAATGT GCTTACAAGG 300

GCCAGCAGGA GTGCCTGGTC GAGACGGGAG CCCTGGGGCC AATGGCATTC CGGGTACACC 360

TGGGATCCCA GGTCGGGATG GATTCAAAGG AGAAAAGGGG GAATGTCTGA GGGAAAGCTT 420

TGAGGAGTCC TGGACACCCA ACTACAAGCA GTGTTCATGG AGTTCATTGA ATTATGGCAT 480

AGATCTTGGG AAAATTGCGG AGTGTACATT TACAAAGATG CGTTCAAATA GTGCTCTAAG 540

AGTTTTGTTC AGTGGCTCAC TTCGGCTAAA ATGCAGAAAT GCATGCTGTC AGCGTTGGTA 600

TTTCACATTC AATGGAGCTG AATGTTCAGG ACCTCTTCCC ATTGAAGCTA TAATTTATTT 660

GGACCAAGGA AGCCCTGAAA TGAATTCAAC AATTAATATT CATCGCACTT CTTCTGTGGA 720

AGGACTTTGT GAAGGAATTG GTGCTGGATT AGTGGATGTT GCTATCTGGG TTGGCACTTG 780

TTCAGATTAC CCAAAAGGAG ATGCTTCTAC TGGATGGAAT TCAGTTTCTC GCATCATTAT 840

TGAAGAACTA CCAAAATAAA TGCTTTAATT TTCATTTGCT ACCTCTTTTT TTATTATGCC 900

TTGGAATGGT TCACTTAAAT GACATTTTAA ATAAGTTTAT GTATACATCT GAATGAAAAG 960

CAAAGCTAAA TATGTTTACA GACCAAAGTG TGATTTCACA TGTTTTTAAA TCTAGCATTA 1020

TTCATTTTGC TTCAATCAAA AGTGGTTTCA ATATTTTTTT TAGTTGGTTA GAATACTTTC 1080

TTCATAGTCA CATTCTCTCA ACCTATAATT TGGGAATATT GTTGTGGTCT TTTGTTTTTT 1140

CTCTTAGTAT AGCATTTTTA AAAAAATATA AAAGCTACCA ATCTTTGTAC AATTTGTAAA 1200

TGTTAAGAAT TTTTTTTATA TCTGTTAAAT AAAAATTATT TCCMACAACC TTAAAAAAAA 1260

AAAAAAAAAA AAAAAAAAAA AAAAANAA 1288

(2) INFORMATION FOR SEQ ID NO: 126:

(i) SEQUENCE CHARACTERISTICS:
(A) LENGTH: 1517 base pairs
(B) TYPE: nucleic acid
(C) STRANDEDNESS: double
(D) TOPOLOGY: linear

(xi) SEQUENCE DESCRIPTION: SEQ ID NO: 126:

AGTGGCTTAA AGGCATCGTT TTAGGATTTA CTGGGAAGTA TCTTCAAAGT AATACATGAG 60

AAACATTCCT TCCTAAATCC TTTATTATAT TGAATATCGT ATTAATTGGT TTTCAGAGGT 120

TAAATTAACC ATGTATTCCT GCAATAAATG TCACTTGTNT CTTGTATATA ATCTTTTTTA 180

TATATTACCG GATTGATTCA TTAGTATTTT GTTGAGGATT TTTGTGTCTA TATTCATAAG 240

AGATGCTGGT CTGCAGTTTT CTTTTTTTGT GATAATCTGG TTTTTGTATC AGTAATACAG 300

GCCCCATGAA ACGAGTTGGG AAGTGTTCAC CTCTCTTGTA TTTTTTCAAG AGTTTGTGAA 360

GAATTGCTAT TAATTCTTTA AATGTTTGGT AGAATCTACC ATTGAAATCA TGTGTCCTGG 420

GCTTTTTTTT GAGGGAAGTG TTCTGATAAC TAATTCAGTA TCTACTTTTT ATAGCTCTGT 480

TCAGATTTTG CTTCTTCCTG AGTTAGTTTT GGTAATTTGT GTATCTCTAG GARTTTGTCC 540

ATTTCATTTA TCTCATTTGT TGGCATAAAT TAAACTAAAT TTGGCCTGAG CCTACCTGTA 600

TATCTTGAGT CCCTCTGTAA GGAACTGTAG CCTAACTTGT ACATAAACAA ACTGAAATCC 660

TAAATTAGGA ATGTAGTTTT TGTAACAGCT CCTGAGTCTC AGGCAGTCAC AGCAGYCAAG 720

TCTGTCAATT GCAGGCTGCT AACTAAGCAG CCCATGSTCA AATGAGGCAA AAACCTTTGC 780

TTTTAACACA TAGTATAGCT TTGTAATCCT TTTCTTGCAC ACTCGGGTAA TTTCTTCCTT 840

TTTCATTCCC KGWATTTTCC AKGAATATGA RTCTYCCTTT TTTCCCCTCC TGTCAGTCTA 900

GCTAATGGTT TGTCAATTTT GTTGATCTTT TGAARAACAA ACCTTTGGTT CCACTTTCTT 960

GTTGCATATG CTGARTATTC TCATAATTGG AGTGGAAAGC TGATCTTTGA TTACTTATTT 1020

TACTTAGGGC TGAGGAGTTC ATGGACTTCG CAAAACCTCC TTGAATCTAA ATTGCATCTT 1080

CTTTCCTGGT TTCTGGGCTG AAACATGTTT TTTCCCATCT WANAWACCCT TGGTCTTTTC 1140

ATKGGCGATT AAGACTAGAG AAAGTTCTAG ATMCCTTGTC CTTTTATGCT GTCATTTTGT 1200

TTAAAGGCTT TCTATGTAGT AAAACTATCT ATATAGACAA AATAGAGCCT TGAGTTGTGG 1260

TCTTGAATTT GATCAACATG ATTTACCACA TTCTGTACTG GATATTTCTT CACCTGCTGC 1320

TACTGTAAAC CATTTTATTC TTGGATCTTC TGTAGAGTAT ATTATCACAG GTACTTTTTA 1380

CAGGGGTGTC TAATCTTTTG GCTTCCCTGG GCACATTGAA AGAAGAAGAA TTGTCTTGGG 1440

CCACACATCA AATACGCTAA CACTAATAAT AGTTGATGAG CTAAAAAAAA AAAAAAAAAG 1500

GCAAAAAAGN CCCAAAA 1517

(2) INFORMATION FOR SEQ ID NO: 127:

(i) SEQUENCE CHARACTERISTICS:
(A) LENGTH: 1073 base pairs
(B) TYPE: nucleic acid
(C) STRANDEDNESS: double
(D) TOPOLOGY: linear

(xi) SEQUENCE DESCRIPTION: SEQ ID NO: 127:

TGAATCTATT CTTTGAACAT TCTACAACAA GAATTACATT ATACTGTTAT ACCAGAGTAC 60

TTCTGCAGTG TGAAATAGAT TGGTTTGGAA AATGAACCTG GCTTTGCTAT AAATTACATT 120

CACAGGCCTT TTTGCAAATG TGTAACTTGC CTATCAAAGT AGTTTGTAGG GCAAATGCAG 180

AATATATGTC TCCATCTGGT AAAGTACCTT WTAYTCATGT GGGAAATCAA GTAGTATCAG 240

AACTTGGTCC AATAGTCCAA TTTGTTAAAG CCAAGGGCCA TTCTCTTAGT GATGGGCTGG 300

AGGAAGTCCA AAAAGCAGAA ATGAAAGCTT ACATGGAATT AGTCAACAAT ATGCTGTTGA 360

CTGCAGAGCT GTATCTTCAG TGGTGTGATG AAGCTACAGT AGGGRMGATC ACTCATGMTA 420

GGTATGGWTC TCCTTACCCT TGGCCTCTGW WTCATATTTT GGCCTATCAA AAACAGTGGG 480

AAGTCAAACG TAAGNTGAAA GCTATTGGAT GGGGAAAGAA GACTCTGGAC CAGGTCTTAG 540

AGGATGTAGA CCAGTGCTGT CAAGCTCTCT CTCAAAGACT GGGAACACAA CCGTATTTCT 600

TCAATAAGCA GCCTACTGAA CTTGACGCAC TGGTATTTGG CCATCTATAC ACCATTCTTA 660

CCACACAATT GACAAATGAT GAACTTTCTG AGAAGGTGAA AAACTATAGC AACCTCCTTG 720

CTTTCTGTAG GAGAATTGAA CAGCACTATT TTGAAGATCG TGGTAAAGGC AGGCTGTCAT 780

AGAGTTATGT GTTAGTCTCA GGAGTCTTAA CTTTTGAAAT ATGTTTTACT TGAATGTTAC 840

ATTAGATATT GGTGTCAGAA TTTTAAAACC AAATTACTGC TTTTTGAAAC CTCAAATTAT 900

ATAATGTATC TTATGTATGT GCTTTATATT GTTATTTGTG TATACATTAA AATAATTCTG 960

AATTATTTAA TCTGATATGT TGTATTCTGT ATCTTGAAAT TTTTGTTTCC TTGAAACATG 1020

CATGCATTTA AAAATAAAGC TTAAACAACT GTAAAAAAAA AAAAAAAAAA CTC 1073

(2) INFORMATION FOR SEQ ID NO: 128:

(i) SEQUENCE CHARACTERISTICS:
(A) LENGTH: 300 base pairs
(B) TYPE: nucleic acid
(C) STRANDEDNESS: double
(D) TOPOLOGY: linear

(xi) SEQUENCE DESCRIPTION: SEQ ID NO: 128:

CAACCCCTGC CTTTTTTTTG TTTTCCATTT GCTTGGTAGA TCTTCCTCCA TCCCTTTATT 60

TTGAGCCTAT GTGTGTCTCT GCCCGTGAGA TGAGTCTCCT GAATACAGCA CACTTACTGG 120

TCTTGACTCT GTATCCAATT TGCCAGTCTG TGTCTTTCAT TTGGAGCATT TAGCCCATTT 180

ACATTTAAGG TKAATATTGT TATGTGTGAA TTTRATCYTR TCATTATGWT GTTAGCTGGT 240

TATTTTGCTT GTTAGTTGAT GCAGTTTCTT CCNGGCATCA ATGGTCTTTA CAANTTGGCA 300

(2) INFORMATION FOR SEQ ID NO: 129:

(i) SEQUENCE CHARACTERISTICS:
(A) LENGTH: 1275 base pairs
(B) TYPE: nucleic acid
(C) STRANDEDNESS: double
(D) TOPOLOGY: linear

(xi) SEQUENCE DESCRIPTION: SEQ ID NO: 129:

GGCAGAGCCT GTCCCTGCTG CCCCTGCAAA AAAAACCCCC TCTGGTGTGA GCAGGATGGT 60

TGGAGGTTAT GTGAGCTCCT TCTCCTTTCC TCCAGTTTCC TCTTCCCTTC TCCTCCCTGC 120

CTCTTTTGCT TTTCCCTTTC TTCCTGGTAC CCCCTGCCCA TTCCTGTATT TTCTCCCATC 180

GCCATTCTCC CCTCTCCCAC TGTCCCTAAC CCGTTCAAAC TCTTTCCTCT TAAATGGTTG 240

AGATTTTCTC TCACCAAGCA CACCCCAGTA TTAATTAAAC TAGCTGCAAA CAGGCAGCAA 300

GTGGTCTACC ATGACAGATG GGTTTTGTGT GTGTGTGTGT GTGTGTAATT GTAATAAAAC 360

ATATTGARTC ACTCAATAAA CACAGAGTGT CTACTACATG TATCARGCAC TATCATAGAT 420

GCTAATTAAC GAAACTGAAA TGGCCAGGCC CTCACAGTGG CTCATGCCTA TAATCCCAGC 480

ACTTTGGGAG GATGAGGCAG GAGGATCACT TGAGGCCGGG AGTTCAAGAC CAGCCTGGGC 540

AACATAGTAA GACTCCATCT CTACAAAAAA AAAATTTTTT TTATTATACT TTAAGTTTTG 600

GGTTACATGT GCAGAACGTG TAGTTTTGTT ACATAGGTAT ATACGTGCCC TGGTAGTTTG 660

CTGCACCCAT CAACCCATCA CCTACATTAG GTATTTCTCC TAATGTTACC CCTCTCCTAG 720

CCCCCCACCC CGTGACAGGC CCTGGTGTGT GATGTTCCCC TCCCTGTGTC CATGTGTTCT 780

CATTGGTCAA CTCTCACCTA TGGAGTGAGA ACATGTGGTA TTTGGTTTTC TGATCTTGTG 840

ATAGCTTGCT GAGAATGTKG GTTTCCAGCT TTATCCACGT CCCTGCAAAG GGCATAAACT 900

CATCCCTTTT TATGGCTGCA TAGTGTTCCA TGGTGTATAC GTGCCACATT TTCTTAATCT 960

ATCATTGATG GACAAGTTTT GCTATTGTGA ATAGTGCCAC AATAAACATA CGTGTGCGTG 1020

TGTCTTTATA GCAGCATGAT TTATAATCCT TTGGGTATAT ACCCAGTAAT GGGATCACTG 1080

AGTCAAATGG TATTTCTCGT TCTAGATCCG TAAGGAATTG CCACACTGTC TTCCACAATG 1140

TTTGAACTAA TNTACACTCC CACCAACAGT GTAAAAGTGT TTCTATTTTT CCACAACCTC 1200

TCCAACATCT GTTATTTCCT GACTTTTTAA TGAACGTCAT TCTAACTGGC GTGAGATGGT 1260

ATCTCATTGT GGTTT 1275

(2) INFORMATION FOR SEQ ID NO: 130:

(i) SEQUENCE CHARACTERISTICS:
(A) LENGTH: 472 base pairs
(B) TYPE: nucleic acid
(C) STRANDEDNESS: double
(D) TOPOLOGY: linear

(xi) SEQUENCE DESCRIPTION: SEQ ID NO: 130:

CNGAAACCCC GTGAACCCTC CCCGGGTTAA AAAGCCCCCC CTAAATGGGG GGAACGCYTC 60

ACACGTTATA AAAAAGCACT AGAATGTTTT GAAAGCGAGA AACAACAGCT GTGTAGGGTA 120

GCTAGCAGTT AGTGTTGTAC AGAAGACAGA TATTTGTGCA TTTYTGCATT TTCTAAGTTT 180

GCTGCAATGA GCATGTATTA CTTTCATAGT TATAAAACAC ATGCAAAATG CCCTTTTAAA 240

ATGAAAAAAA ATCCATGAGT GTAAGTGATA TATATGCTTT GGAAAGCCTG GGACGGTCAT 300

TGTTTACTCT CAATAGTATG TGTTTGCCTT TGTCTTTTTG AGACATTTTG TTTTAATCTG 360

TTGATGACAA TAACCTGTTG ATAATATAAC TTGATAACAA ATAAAATGAC TTATGATTGA 420

AWMAAAAAAA AAAAAAAAAA AAAAAAAAAA AAAAAAAAAA AAAAAAAAAA NN 472

(2) INFORMATION FOR SEQ ID NO: 131:

(i) SEQUENCE CHARACTERISTICS:
(A) LENGTH: 1950 base pairs
(B) TYPE: nucleic acid
(C) STRANDEDNESS: double
(D) TOPOLOGY: linear

(xi) SEQUENCE DESCRIPTION: SEQ ID NO: 131:

ACCTCTCAGA ATCTTCTCTC AGCAACCTGA GTCTTCGCCG TTCCTCAGAG CGCCTCAGTG 60

ACACCCCTGG ATCCTTCCAG TCACCTTCCC TGGAAATTCT GCTGTCCAGC TGCTCCCTGT 120

GCCGTGCCTG TNATTCGCTG GTGTATGATG AGGAAATCAT GGCTGGCTGG GCACCTGATG 180

ACTCTAACCT CAACACAACC TGCCCCTTCT GCGCCTGCCC CTTTNTGCCC CTGCTCAGTG 240

TCCAGACCNT TGATTCCCGG CCCAGTGTCC CCAGCCCCAA ATCTGCTGGT GCCAGTGGCA 300

GCAAAGATGC TCCTGTCCCT GGTGGTCCTG GCCCTGTGCT CAGTGACCGA AGCTCTGCCT 360

TGCTCTGGAT GAGCCCCAGC TCTGCAACGG GCACATGGGG GGAGCCTCCC GGCGGGTTGA 420

GAGTGGGGCA TGGGCATACC TGAGCCCCCT GGTGCTGCGT AAGGAGCTGG AGTCGCTGGT 480

AGAGAACGAG GGCAGTGAGG TGCTGGCGTT GCCTGAACTG CCCTCTGCCC ACCCCATCAT 540

CTTCTGGAAC CTTTTGTGGT ATTTCCAACG GCTACGNCTG CCCAGTATTC TACCAGGCCT 600

GGTGCTGGCC TCCTGTGATG GGCCTTCGMA CTCCCAGGCC CCATCTCCTT GGCTAACCCC 660

TGATCCAGCC TCTGTTCAGG TACGGCTGCT GTGGGATGTA CTGACCCCTG ACCCCAATAG 720

CTGCCCACCT CTCTATGTGC TCTGGAGGGT CCACAGCCAG ATCCCCCAGC GGGTGGTATG 780

GCCAGGCCCT GTACCTGCAT CCCTTAGTTT GGCACTGTTG GAGTCAGTGC TGCGCCATGT 840

TGGACTCAAT GAAGTGCACA AGGCTGTGGG GCTCCTGCTG GAAACTCTAG GGCCCCCACC 900

CACTGGCCTG CACCTGCAGA GGGGAATCTA CCGTGAGATA TTATTCCTGA CAATGGCTGC 960

TCTGGGCAAG GACCACGTGG ACATAGTGGC CTTCGATAAG AAGTACAAGT CTGCCTTTAA 1020

CAAGCTGGCC AGCAGCATGG GCAAGGAGGA GCTGAGGCAC CGGCGGGCGC AGATGCCCAC 1080

TCCCAAGGCC ATTGACTGCC GAAAATGTTT TGGAGCACCT CCAGAATGCT AGAGACCTTA 1140

AGCTTCCCTC TCCAGCCTAG GGTGGGGAAG TGAGGAAGAA GGGATTCTAG AGTTAAACTG 1200

CTTCCCTGTT GCCTTCATGG AGTTGGGAAC AGGCTGGGAA GGATGCCCAG TCAAAGGCTC 1260

CAAGCGAGGA CAACAGGAAG AGGGATCCAC TGTTACCAAA AGTCCTGATT CCCCCATCAC 1320

CAACCTACCC AGTTTGTTCG TGCTGATGTT GGGGGAGATC TGGGGGGAGT TGGTACAGCT 1380

CTGTTCTTCC CTTGTCCTAT ACCGGGAACT CCCCTCCAGG GTACCCACAG ATCTGCATTG 1440

CCCTGGTCAT TTTAGAAGTT TTTGTTTTAA AAAACAACTG GAAAGATGCA GAGCTACTGA 1500

GCCTTTGCCC TGAATGGGAG GTAGGGATGT CATTCTCCAC CAATAATGGT CCCTCTTCCC 1560

TGACGTTGCT GAAGGAGCCC AAGGCTCTCC ATGCCTTTCT ACCTAAGTGT TTGTATTTTA 1620

TTTTAAATTA TTTATTCTGG AGCCACAGCC CCCTTGCTTA TGAGGTTCTT ATGGAGAGTG 1680

AGAAAGGGAA GGGAAATAGG GCACCATGGT CCGGTGGTTT GTAGTTCCTT CAAAGTCAGG 1740

CACTGGGAGC TAGAGGAGTC TCAAGCTCCC CTTAGGAAGA ACTGGTGCCC CCTCCAGTCC 1800

TAATTTTTCT TGCCTGCCCC GCCTTGGGGA ATGCCTCACC CACCCAGGTC CTGACCTGTG 1860

CAATAAGGAT TGTTCCCTGC GAAGTTTTGT TGGATGTAAA TATAGTAAAA GCTGCTTCTG 1920

TCTTTTTCAA AANAAAAAAA AAAAAAAACT 1950

(2) INFORMATION FOR SEQ ID NO: 132:

(i) SEQUENCE CHARACTERISTICS:
(A) LENGTH: 990 base pairs
(B) TYPE: nucleic acid
(C) STRANDEDNESS: double
(D) TOPOLOGY: linear

(xi) SEQUENCE DESCRIPTION: SEQ ID NO: 132:

TGGAAGATTT AAAATAGGTT TCATATTTCT CTTGAATATG AATATATAAG CTTGAATAAG 60

CTTGAGTCCT TATTATTATG AAATTTTCCT TATTATTTCT ACCAATGCTT CTTATATTAA 120

AGCCTGATCT TTTTCATATT AGTATATGTA CATTAGCTGC CTGTGGATTA ACATTTCCAT 180

GAAATGTATT TTTGCATTGT TTGATCTTAA ACTTTTTGTG TCTTTATATA AGGTATGCTY 240

CTTTTAAGCA TGATATTTTT AACCACAATA GTTGAAAGAC AATCTYCACC TTTTACTTGT 300

ATATTTACAT GTAATGTAAT TTTTGATGCA TATTACGTCT TATTATTTAA CCAACCTATT 360

TTATTTTATC TAGGCATTTT TTCAGAAAGC CTTATTTTCT TGTATTAATC AAATATTTTT 420

AYCATTGTAT TTTCCYTTAT TAGTTAGKAA TACGKTACYC YAAATATATA TTGTGGSTAT 480

TTTCAGAATT GCAATATGCC TCCTTAATTT ATTAGAGGCT AACCTAAATT ATTACTTTTA 540

CCACTTACTT GAAAATTCTG GAACTTTAGA ACATTTATTG TTTTATGCAT TTTAATTCTA 600

CTTGTATTTT TACTACTCCT AAACATTATT ATTGTTTTAG ACAAGCCAAA ATATATNTTG 660

TTATTATCTT ATYCTCCATT TCTTTCTGTA TTTTTATGCC ACTATGTATG CTCAATTTCC 720

TTCTATGTGA TGAACCTAAT TCAGTACTTT TGTTTTTTAA TCTGTGCAGG TAGCCTGGCC 780

ATTAAATTTT TATTTTTGGT TTGCTGAAAA AATTGTGTTT ATTTCTATAT GCATACTTAT 840

GCATATAGAA TNCTAGGTNG ACATATTTTT AGTATTTATA AATGTAAAGT CATTWATTKG 900

GCTTCTATCA TTTCKGTKGA GAAATCAATT GTCAGCCCAA TAGTTTTTCA TTTTAAATTA 960

CNGAATTTTT TCATGTCTCT GGTTTTAGGA 990

(2) INFORMATION FOR SEQ ID NO: 133:

(i) SEQUENCE CHARACTERISTICS:
(A) LENGTH: 1720 base pairs
(B) TYPE: nucleic acid
(C) STRANDEDNESS: double
(D) TOPOLOGY: linear

(xi) SEQUENCE DESCRIPTION: SEQ ID NO: 133:

GTCTGATAAG CGACTGTGGT TATTCCCCTA AAGTTTACTT CAGCACTAAC ACTAGTGCTT 60

CCGCTGGAGT TTGCAGTTTT CCAGCTTTAT ACAGGATTTT CCTTTGACTG GAAGAGTCAA 120

GGATATAGAG ACTCAATAGT GACATTTATT GTACAACATC AAGGGGAATA GGATACTCAT 180

CAAACTGGGA TTATTCTTAT CAAAACATGG TCTTCTTTGA ATAAGAAAAA TACATAGTTG 240

GTTATTATGG ACTTAAAACT GTGTTAAATG GATATTCTGA TAAAATATTT GCTGCTCTGT 300

AGAGTGTGGA AAATCTGAGA ATATTAGCTT TACTCATCTT GAGCTTTGAG GATGTTCTCT 360

GTACGCCGAT GGTTTCATAT TAACTAAAAA AGCTGGGTAT TGTAAAATCT CATTTATAAA 420

AACTCAGATG AGAAGAAAAT TTTCTTTGAT GGTGAGACTG TTGTCTTAGT TCAGGAAATT 480

ATTTAATAAT CCTTTGTTAC CTGTGAATGA AGGAACTTTG TAATTCTGAT TTATCGTAAA 540

ACATGAGCCT TTCCAGAGTC AGCTTAGACA CTGTTGTCGC AAATAGCCAT GCTTTGCCTT 600

ATGCCAAGGA GGCCCAGAGG GAGGGCCTAG TCTTCCTCTG TTGCTGTACA TATATTGAAA 660

TGCTTTTTTT TTTTATTTTG CATTTGTTAT CTATAATGAG CTTTCTGAGC CCTGATATTA 720

TGTGAGACAA ACAGGAGTTA TTGATGTTAT ACACTCCCTT CCATTCAGGA TTTTCTGCTT 780

GGAGGGAAAT ATGTTGACCT TAGAGAATTG TGAATATTGT TGCAATTCTT GAATATATTA 840

CCATGTGAAT AATAGAGACT GTGTTGCTCT CTAGTATAAG CTATATTTAT TTTTGATTCA 900

TTTGAATTAC TAGTTATAAC TGGAGAAATT TTGTTACCTC TATCCTGGCT TGCCTGACTG 960

GCTGTATAAT AGCAGCAGCC TCTTTTAGAG CATCTTAATG AAAACATGGA TGAAAGGAAT 1020

TAATGATGAT ATCTGCAGAC TGCGTAGAAA ATGGCTTTTG TTCCCAGCGT TAACATTTTC 1080

TTCTCAATCA CATTTCAATG TTTGTGGAGA GTGGCAGATT CACACCAGAA ACACTAGGTG 1140

TTCATATCCA TAGCATGGAT GCAGAATAAG CAGTTGGGAG AGAAGCTTCT TCCTACCTGG 1200

TACTCCTCCC ATTCACCTCA GCCCAGCCCC AGACAGGCGT TAGCATTCAG TGTGGGCCCT 1260

CAGGCAGCCC TGAAGCCTGG CTGGGTCATC AGATGGGGGC AGCCTGTGAC GGGCACCAGC 1320

GGCCTGATTC CAGGGAAGAG TTCCTGGAGG GTGTTGGCTG TTTTTGTTAG CTCAGTTTTT 1380

TTCTGGGCTC CACCATTCCT AACTCCAGGT AGACAAGATA GATGTCACAC ACAACAATTT 1440

TAAAGTATTT TGCTTAGTGC ATTTTGTTTA TGATTGCAGT GTTTGTTTCT TATTTAATAG 1500

GCTTTTTACT TCATTCTATT AAATTTTAGT GTTTAGAAGA GGCGGGTACT GTCACTGTGT 1560

AAAATATGTA ATATTTTATA TGTTATACCA TGTCATATAT ACTTGCAATA TCAGACCTTG 1620

CATTCAATAT ACAATGCAAT TGACTCTTTG CAGACCTGCA TTTTTCAGTG AACAATAAAA 1680

AGATTGTCTG GCACTCCAAA AAAAAAAAAA AAAAAAAAAA 1720

(2) INFORMATION FOR SEQ ID NO: 134:

(i) SEQUENCE CHARACTERISTICS:
(A) LENGTH: 705 base pairs
(B) TYPE: nucleic acid
(C) STRANDEDNESS: double
(D) TOPOLOGY: linear

(xi) SEQUENCE DESCRIPTION: SEQ ID NO: 134:

GGCACGAGGC CATCTGGGCT CATTCAGCAG GAAATAATGG AAAAAGCTGC AATATCCAGG 60

TGTTTACTAC AATCTGGAGG CAAGATCTTT CCTCAGTATG TGCTGATGTT TGGGTTGCTT 120

GTGGAATCAC AGACACTCCT AGAGGAGAAT GCTGTTCAAG GAACAGAACG TACTCTTGGA 180

TTAAATATAG CACCTTTTAT TAACCAGTTT CAGGTACCTA TACGTGTATT TTTGGACCTA 240

TCCTCATTGC CCTGTATACC TTTAAGCAAG CCAGTGGAAC TCTTAAGACT AGATTTAATG 300

ACTCCGTATT TGAACACCTC TAACAGAGAA GTAAAGGTAT ACGTTTGTNA AATCTGGGAA 360

GACTTGACTG CTATTCCATT TTGGGTATCA TATGTACCTT GATGAAGANG ATTAGGTTGG 420

GATACTTCAA GTGAAGCCTC CCACTGGAAA CAAGCTGCAG TTGTTTTAGA TAATCCCATC 480

CAGGTTGAAA TGGGAGAGGA ACTTGTACTC AGCATTCAGC ATCACAAAAG CAATGTCAGC 540

ATCACAGTAA AGCAATGAAG AGCAGTTTTC CAATGAAAAC TGTGTAAATA GAGCATCAAC 600

AAGTACAAAA TTCTTGTCTT AATTAGTGGG GGTATATAAA AATTCCTTGT AATGGTCAAA 660

TATTTTTTAA AATTGACATT AATAAAGCAT ATTTTAAAAG TTTCT 705

(2) INFORMATION FOR SEQ ID NO: 135:

(i) SEQUENCE CHARACTERISTICS:
(A) LENGTH: 323 base pairs
(B) TYPE: nucleic acid
(C) STRANDEDNESS: double
(D) TOPOLOGY: linear

(xi) SEQUENCE DESCRIPTION: SEQ ID NO: 135:

AGCACACACC TCCTTTAGTT GCTCCTAAGG TCATGTTCAA CATTCGTGGA GTGCATTTTC 60

TGCTCAGGGA GCTTTCCCAG ACCCGGAATG TTTGGTGCTC ACAGACYCTG GCAAGGATCG 120

GTATTGCTGT TCCTCAGTTT TGCCTGGGGA AATGGAGGST CAGTGACGTT CAGTGACGTG 180

CCCAGAGTCA TGCCATTGGC GGGTGGCCCA GKGMTCCAGG TCTCCAGCAC CCCTCGGCCC 240

CCTCCTCACC AGGTCACATC ATCTCCTGGA TTAGAATCTG CTCACATAGT CTGTCCTGAA 300

AGGAAAAAAA AAAAAAAAAA AAC 323

(2) INFORMATION FOR SEQ ID NO: 136:

(i) SEQUENCE CHARACTERISTICS:
(A) LENGTH: 582 base pairs
(B) TYPE: nucleic acid
(C) STRANDEDNESS: double
(D) TOPOLOGY: linear

(xi) SEQUENCE DESCRIPTION: SEQ ID NO: 136:

GGACGGAATG GTGCAACCCT CCTWAMTTTT CTKGKGCTGT TGACAACAGA GGGAGGGAGG 60

GAAAACATTT TTYGTGGGAG AATCCTACYT CTGCAGSGGA GCCCTTAAGC GATKGATTTT 120

GAATCTKGAC CCTTTACCAA CTAATTTTGA AGGAAGATAC CTTGGAAATA TTTGGCATTC 180

AGTGGGTTAC TGAAACAGCA TTAGTGAATT CATCTAGAGA ACTCTTTCAT TTATTCAGGC 240

AACAACTGTA CAACTTGGAA ACCTTGTTAC AGTCCAGTTG TGATTTTGGG AARGTATCAA 300

CTCTACACTG CAAAGCAGAC AATATTAGGC AGCAGTGTGT ACTATTTCTC CATTATGTTA 360

AAGTTTTCAT CTTCAGGTAT CTGAAAGTAC AGAATGCTGA GAGTCATGTT CCTGTCCATC 420

CTTATGAGGC TTTGGAGGCT CAGCTTCCCT CAGTGTTGAT TGATGAGCTT CATGGATTAC 480

TCTTGTATAT TGGACACCTA TCTGAACTTC CCAGTGTTAA TATAGGAGCA TTTGTAAATC 540

AAAACCAGAT TAAGGTTTGA CTGGTTTCAT TTGATTTTTA AG 582

(2) INFORMATION FOR SEQ ID NO: 137:

(i) SEQUENCE CHARACTERISTICS:
(A) LENGTH: 1021 base pairs
(B) TYPE: nucleic acid
(C) STRANDEDNESS: double
(D) TOPOLOGY: linear

(xi) SEQUENCE DESCRIPTION: SEQ ID NO: 137:

TTCGGCAGAG CCCTTGCGCG CTCTTGAATA CCTGCKTTCT GTAGCGCTAG TTCTCTTCAA 60

GATTTGCTTA GTGTCATTTC ATTTCGGTTT CTTTTCTCGC CATGTTTTTC TGTCGGAATT 120

ACGGTTCGTT TTGGTTCTAT GTACTCTCTA AAATGTTATC GTTTTTCATT TGTCTACTAA 180

TTTTCGTGCA TTTGTTACTA CTGAGTTTCT TAATATCTGA CTGGCCTCCG CCCACGGGCT 240

CTGCAGANCA TAAAATACTC AGGCTGATGG TAGTGCAGAG ACTCTCCCTC CTTGATCAGC 300

GCAAACGTTG GTCTGAGGCT TGAGGGATGG AGCAACATTT TCTTGGCTGT GTGAAGCGGG 360

CTTGGGATTC CGCAGAGGTG GCGCCAGAGC CCCAGCCTCC ACCTATTGTG AGTTCAGAAG 420

ATCGTGGGCC GTGGCCTCTT CCTTTGTATC CAGTACTAGG AGAGTACTCA CTGGACAGCT 480

GTGATTTGGG ACTGCTTTCC AGCCCTTGCT GGCGGCTGCC CGGAGTCTAC TGGCAAAACG 540

GACTCTCTCC TGGAGTCCAG AGCACCTTGG AACCAAGTAC AGCGAAGCCC ACTGAGTTCA 600

GTTGGCCGGG GACACAGAAG CAGCAAGARG CACCCGTAGA AKARGTGGGG CAGGCAGARG 660

AACCCGACAG ACTCAGGCTC CRGCAGCTTC CCTGGAGCAG TCCTCTCCAT CCYTGGGACA 720

GACAGCAGGA CACCGAGGTC TGTGACAGCG GGTGCCTTTT GGAACGCCGC CATCCTCCTG 780

CCCTCCAGCC GTGGCGCCAC CTCCCGGGTT TCTCAGACTG CCTGGAGTGG ATTCTTCGCG 840

TTGGTTTTGC CGCGTTCTCT GTACTCTGGG CGTGCTGTTC ACGGATCTGT GGAGCTAAGC 900

AGCCTTAGAT AGCAGCAGAA GGCTTTTTGG ATTCTCCTCC TTGAAAAGAT TCTCAGTTAC 960

CAAACGTCTC CACCTAGAAA ATAAAAATAC ATTAAGATGT TGANAAAAAA AAANAAAAAA 1020

A 1021

(2) INFORMATION FOR SEQ ID NO: 138:

(i) SEQUENCE CHARACTERISTICS:
(A) LENGTH: 1777 base pairs
(B) TYPE: nucleic acid
(C) STRANDEDNESS: double
(D) TOPOLOGY: linear

(xi) SEQUENCE DESCRIPTION: SEQ ID NO: 138:

CGGAAGATGA TGGCTTCAAC AGATCCATTC ATGAAGTGAT ACTAAAAAAT ATTACTTGGT 60

ATTCAGAACG AGTTTTAACT GAAATCTCCT TGGGGAGTCT CCTGATCCTG GTGGTAATAA 120

GAACCATTCA ATACAACATG ACTAGGACAC GAGACAAGTA CCTTCACACA AATTGTTTGG 180

CAGCTTTAGC AAATATGTCG GCACAGTTTC GTTCTCTCCA TCAGTATGCT GCCCAGAGGA 240

TCATCAGTTT ATTTTCTTTG CTGTCTAAAA AACACAACAA AGTTCTGGAA CAAGCCACAC 300

AGTCCTTGAG AGGTTCGCTG AGTTCTAATG ATGTTCCTCT ACCAGATTAT GCACAAGACC 360

TAAATGTCAT TGAAGAAGTG ATTCGAATGA TGTTAGAGAT CATCAACTCC TGCCTGACAA 420

ATTCCCTTCA CCACAACCCA AACTTGGTAT ACGCCCTGCT TTACAAACGC GATCTCTTTG 480

AACAATTTCG AACTCATCCT TCATTTCAGG ATATAATGCA AAATATTGAT CTGGTGATCT 540

CCTTCTTTAG CTCAAGGTTG CTGCAAGCTG GGAGCTGAGC TGTCAGTGGA ACGGGTCCTG 600

GAAATCATTA AGCAAGGCGT CGTTGCGCTG CCCAAAGACA GACTGAAGAA ATTTCCAGAA 660

TTGAAATTCA AATATGTGGA AGAGGAGCAG CCCGAGGAGT TTTTTATCCC CTATGTCTGG 720

TCTCTTGTCT ACAACTCAGC AGTCGGCCTG TACTGGAATC CACAGGACAT CCAGCTGTTC 780

ACCATGGATT CCGACTGAGG GCAGGATGCT CTCCCACCCG GACCCCTCCA GCCAAGCAGC 840

CCTTCAAGTT CTTTTATTTC TGGGTAACAG AAGTAGACAG ACAGGTTACT TGGTGTATCT 900

TCTGTTAAAG AGGATTGCAC GAGTGTGTTT TCCTCACACA CTTTGATTTG GAGAATTGGT 960

GCTAGTTGGC AATAGATAAC TCAGCGTAGA TAGTATTGCA AAAAGGGGAG GAAATACACA 1020

ACAATAATAA ATGTAAAAAC CTGCTATTCA ACATGCAGTT TTATTTCGAR GCCAAAAATC 1080

TAGAGCTTTC CCAAGATCCT GTTGCCTTAG GCACATNCAC ACTTCAACAG TGCACACTAT 1140

CCAACAGTGC ACACTATTCA ACAGTGCACA CTATTCAAAA GCGTAGACTA TTTTTTTGCA 1200

TGTTCAAGAT ATTTGTTTTG GTCTTATGTG TGTGTGAGAG AGAGAGATTC CTTTGACATT 1260

AAGGAGCATC AATGAGAAAA GATGATGAGG CAGGAATTAA TAAAGAAATG AAGTCGTGTG 1320

TGTTTGGTTG CCTGTCAGAG GGCACACAAT TTCATAAACA CCATGCCTGG ACAATTTGAT 1380

ATTAATATTT AACACCTCTG CATCTTTTTC TTAAAAAAGA ATATGGGCCA GATACAGTGG 1440

CTCACATTTG TAATCCCAGC ACTTTGGGGA GCCAAGTTAG CAGAATCCCT TGAGCACAGG 1500

AATCTGAAAC CAGCTTGGGC AACATAGTGA GATCCCATCT NTACAAAAAA CTTAAAAATT 1560

AGCCAGGCAT GATGGCACAT TCCTGTAGTC CTAGCTACTC AGGAGGCTAA GGTAGGAGGA 1620

TTGCCTGAGC CCAGGAGTTC AAGGCTGCAG TGAGCTAAGN ACGTGCCAGT ACACTCCAGC 1680

CTGAGCCACA AAGTGAGACC CTGTCTCGCA AAAAAAAAAN TTAAAAAGTC GGGGGGGGGC 1740

CCGGTACCCA AATCGCCGGA TATGATCGTA AACAATC 1777

(2) INFORMATION FOR SEQ ID NO: 139:

(i) SEQUENCE CHARACTERISTICS:
(A) LENGTH: 643 base pairs
(B) TYPE: nucleic acid
(C) STRANDEDNESS: double
(D) TOPOLOGY: linear

(xi) SEQUENCE DESCRIPTION: SEQ ID NO: 139:

TTTTTTTTTT TTTTTTTTTT TTTTTTTTTT TTTTTTTGGG AATGAGAAAA TAACTTTATT 60

TTCATTGTGG GGAGCGGGCC GATGTCCAGC CTCAGAACTT CTGGAACTGC TTCTTGGTGC 120

CGGCAGCCTT GGTGACCTTG AGCACGTTGA AGCGCACTGT CTTGCTCAGA GGCCGGCACT 180

CGCCCACTGT GACGATGTCA CCGATCTGGA CGTCCCTGAA GCAGGGGGAC AGGTGTACAG 240

ACATGTTCTT GTGGCGCTTC TCGAAGCGGT TGTACTTGCG GATGTAGTGC AGATAGTCTC 300

GGCGGATGAC AATGGTCCTC TGCATCTTCA TCTTGGGTCA CCACGCCAGA GAGGATCCGC 360

CCTCGAATGG ACACATTACC AGTGAAGGGG CATTTCTTGT CAATGTAGGT GCCCCTCAAT 420

AGCCTCCTTG GGGTGTCTTT GAAGCCCAGA CCGATGTTCT TGTTAGTAAC CCGCGGGAGC 480

TTCTCCTTGC CAGTTTCTCC CAGCAGGACC CTCTTCTTGT TTTGAAAGAT GGTCGGCTGC 540

TTTTGGTAGG CACGCTCAGT CTGAATGTCC GCCATCTTCT CGTGCCGMAY TCCTGCAGCC 600

CGGGGGATCC ACTAGTTCTA GAGCGGCCGC ACCGCGGTGG AGC 643

(2) INFORMATION FOR SEQ ID NO: 140:

(i) SEQUENCE CHARACTERISTICS:
(A) LENGTH: 1220 base pairs
(B) TYPE: nucleic acid
(C) STRANDEDNESS: double
(D) TOPOLOGY: linear

(xi) SEQUENCE DESCRIPTION: SEQ ID NO: 140:

GGCACGAGGA TGATAGACCT ACTGGAGGAA TACATGGTTT ACAGGAAGCA TACCTACATR 60

AGGCTTGATG GCTCATCCAA GATCTCGGAG AGGCGAGACA TGGTTGCTGA TTTTCAGAAC 120

AGGAATGACA TCTTTGTGTT CCTGTTAAGC ACACGAGCTG GAGGACTGGG TATCAATCTC 180

ACTGCTGMAG ACACAGTGCA TTTTCTATGA TAGCGACTGG AACCCCACTG TGGACCAGCA 240

GGCCATGGAC AGGGCCCACC GCTTAGGGCA GACAAAGCAG GTTACTGTGT ACCGGCTCAT 300

CTGTAAAGGC ACCATTGAAG AACGCATTCT GCAAAGAGCC AAGGAGAAGA GTGAGATTCA 360

GCGGATGGTG ATTTCAGGTG GGAACTTCAA ACCAGATACC TTGAAACCCA AAGAGGTGGT 420

TAGTCTTCTT CTAGACGACG AAGAGTTGGA GAAGAAACGT ATGTACTCTA AACCTCTATA 480

CACTCCCCTC ACGTATCTGA GAATGGAAGA GGTACTTGGS TGTGTGCCAA GGGTTAGGCA 540

AAGCCAGAGG CTGTATTTAG GGAAAGTATT TTTGTGCTCA TATTTTATAT AAAAACCCAA 600

ACAAGAATGT GTTTGTAGGC CAGGCGTGGT GGCTCGCGCC TCTAGTCTCA GCATTTCGGG 660

ARGCCAAAGT GGGCAGATCA CCTGARGTCA GGARTTTGAG TTTGARACCA GCCTGGCCMA 720

CGTTGTGAAA CCCCACCTCT ACTARGARTA CSGAAAATTG GTTGGGCATG GTGGCGGGCA 780

CCTGTAATTC CAGCACTTTG GGAGGCTGGG GCAGAANAAT TGCTTGAGCC CAGGAGGTGG 840

AGATTGCGGT GAGCCGAGAT YGTGCCATTG CAMTCCAGCC SGGGCAATAA GAGTGAAAYT 900

CCATCTTTTA AAAACAAACA AAAACAAAAA ACACAAGACG GCTCACACCT GTAATCCCAG 960

CACTTTGGGA RGCCGARGCA GGTGGATCAC GARGTCAGGA GTTCCAAGAC TAGCCTGGCC 1020

AACCTGGTGA AGCCCCGTCT CTACTAAAAA TACMAATATT AGTCGGGCGT GGTGGTGGGC 1080

ACGTGTAATC CCAGCTACTC GGGAGGCTGA GGCAGGAGAA TCCCTTGAAG CTAGGAGGCA 1140

GAGGTTGCAG TGAGCCAGGA TCGTGCCATT GCACTCCAGC CTGGACAACA AGAGCAAGAT 1200

TCCATCTCAA AAAAAAAAAA 1220

(2) INFORMATION FOR SEQ ID NO: 141:

(i) SEQUENCE CHARACTERISTICS:
(A) LENGTH: 721 base pairs
(B) TYPE: nucleic acid
(C) STRANDEDNESS: double
(D) TOPOLOGY: linear

(xi) SEQUENCE DESCRIPTION: SEQ ID NO: 141:

AATTCGGCAC GAGCCAGGTT AGCCGGAAGG GCAGCTCTCC AGGCCCTGCC CACCCCACAG 60

GGGGCTCCTT ATGCACAGCG GGGCGTCTCC TTGTGGCCAT AGAAACGGAA CTGGCTCTTT 120

TCAACAGTGC TGCAAGAGGA TGGTTATTTA ACGCTGGCCC CCAAGGAGGA AAGGCACAGA 180

CYTTCCTCCC TCCTGGAACA TCCAAGGGCA CTGGATCCTC TGTGTCCCTC TGAGATGGGG 240

TGCCACTCCA GCAAGAGCAC CACGGTGGCA GCTGAGTCCC AGAAGCTTGA AGAAGAGYGC 300

GAGGGAAGAG AGCCAGGTCT GGAGACCGGC ACCCAGGCAG CAGACTGCAA GGATGCCCCG 360

CTGAAGGATG GAACCCCTGA GCCAAAGAGC TGAAATGCCT CTCTCCAGAG TCGGACCCTC 420

ACCTCYTTCC TGGAACTGCC TTTGGCCCCA GAACCATGAG ACAATCCCCA CCCTGAGAAG 480

CTCCGATCAC TGGGAGGAGA GAGAAAGCCT CCAGCTTTGG GATTCAGGCT TCAGAAGTTT 540

TTAGCAGCCT TTGCTCATTG GAGAGGTGGG GAAAGGATAA AGTTCTTATA AGGAAATCCC 600

TAATTTCCCC CAGCTCCTCC CCNCCNGAAG AAGGAACNAA AGAAAGTTCC TTCCACACGT 660

TTTGTTGGAA ACTTTTCCCT TGCCAACTTT CCTTGGATTG CCAGAACAAA GCCCTCCAGA 720

A 721

(2) INFORMATION FOR SEQ ID NO: 142:

(i) SEQUENCE CHARACTERISTICS:
(A) LENGTH: 1468 base pairs
(B) TYPE: nucleic acid
(C) STRANDEDNESS: double
(D) TOPOLOGY: linear

(xi) SEQUENCE DESCRIPTION: SEQ ID NO: 142:

ATGAATTAAT GTTTATAAAT GACTGTACTG AATTTAAAAC CGTACAGTTT CATTTGCATT 60

TTGACATTAC TTTATTATAC ATTTTGCATT TAAAAGGCTG CACCAGTTGG CTTTTCTTCT 120

GTTTTATTCT CAAAATATAG AGATTCTGTG ATTTATTTGC CCTGTTTATG GATTAAAAAG 180

AAAATTCTAA TATAAAGCAT TTCAATAGGA TGCATAGGTA TATTACGTTT TTTAAATGCT 240

TTAGATCTGT GATTCTTGAC TTACTATTTA TTTTATCCCC TTTAAGTCAG GGATGCTTTA 300

TTCTATTTTA AAGCACTTAT GAGTTACATG TTGTAATCAA GTTTGCACAA TATATTTATC 360

TATATGAGGA ACCCATAAAT GAATAGCTAA TTTTTAAAAT GCCATTAAAA TGCATGAAAT 420

KCTTATTAAA ACCTTACTAT ACTATTTCTT CAAGGCAAGT AAATTGACCA TGRGRAAAGR 480

ACACAGTTAT TAAACACTGT TGACAGGAAA ATTCTCCTTG ATAACATAGG ACAATTAATG 540

GAAAAAAAAA TTCTCATTAT TTGCAAAGAA TGAACAAGTT AATGAACAAA CAAACTAGAT 600

TTGGTATGTT TTCAGCTTTT GTATCATGTT TAATTGTTTA ATTTGGTTGA AAAACTGCAG 660

TTGAGAAATC AGATAGCAAT ATAGACATTC ACAGCAGCTC TGTGGATACC ATGTAATTGT 720

CAGGTAATTT CAGAATGTTG AAAATTATTC AGTGCAGCCC TCATAGTATC ATACTTGAAG 780

AAATTGATTA CAGTTCCACT AAATTGTTGA AGATAAATTA TTTTTAAAGG TTATGAAAAC 840

TAAGTTATAT TAATTCATAT GTTTGATTTT TAAATCCCAC CTCCTCAAGC TATCCAATTT 900

NCTGACTTTG AAAATAACCA TGAGAGATGC CACATTTCTC TCTGGGAAAC TACCACTCAA 960

AGAATAATTG TTAAAAATTA AGCTTTTAGG TATTAGAAGC TGTTATAAAG TATAAAATTA 1020

AGATATAAGC AGATCACATG TAAATCATTC CTAAAGCACA AGAAAAGAAT GTGCCTTGAT 1080

GTACATATAT TACTAAGTTG CCTCTCCCAG TTTACTTTAA AAATGGCTTT AAGGATAAAG 1140

AATAAATGTG ATAGCTGTGC ATGCATTATA TATTTGCATT TGCAAATTTC CCATTGTTTT 1200

AACAGCTGTG TGGCTGACTT TCAATTTTAA GACGTGAATT GACATACAGC CCATAACTTT 1260

ATAATGGCTG CTCATTTATC TTATCTTTCA GTTAGTGGAA AAACATTTCA ACCTGACTAA 1320

AATTTGGAAT TGTGTCTTTT ATGTTCCATC CTCTGTTGTT ACTAGATTTA GTTTAAAAAT 1380

TGTGTATGAC CATTAATGTA TGTCATAAAC ATGTAAATAA AAGATGTTGA ATCTTGTTGA 1440

AAAGCAWRAA AAAAAAAAAA AAACTCGA 1468

(2) INFORMATION FOR SEQ ID NO: 143:

(i) SEQUENCE CHARACTERISTICS:
(A) LENGTH: 300 base pairs
(B) TYPE: nucleic acid
(C) STRANDEDNESS: double
(D) TOPOLOGY: linear

(xi) SEQUENCE DESCRIPTION: SEQ ID NO: 143:

TGAATTTTTT GCCAAACTTA GTAACTCTGT TAAATATTTG GAGGATTTAA AGAACATCCC 60

AGTTTGAATT CATTTCAAAC TTTTTAAATT TTTTTGTACT ATGTTTGGTT TTATTTTCCT 120

TCTGTTAATC TTTTGTATTC RCTTATGCTC TCGTACATTG AGTACTTTTA TTCCAAAACT 180

AGTGGGTTTT CTCTACTGGA AATTTTCAAT AAACCTGTCA TTATTGCTTA CTTTGATTAA 240

AAAAAAAAAA AAAAAAAAAA AAACCCCNAG GGGGGGGCCG GGTNCCCAAT CCCCCCCAAA 300

(2) INFORMATION FOR SEQ ID NO: 144:

(i) SEQUENCE CHARACTERISTICS:
(A) LENGTH: 2243 base pairs
(B) TYPE: nucleic acid
(C) STRANDEDNESS: double
(D) TOPOLOGY: linear

(xi) SEQUENCE DESCRIPTION: SEQ ID NO: 144:

TGCCTCCCTT CCTGCAGATT GTGGACAGTA GTTCCTCAGC CTGCACCCTG GATTCCTTCT 60

TCCCCTTCCT AGCTCCATGG GACTCGCCCC AAGACTGTGG CTTCAAGGAC CACCAGCCCC 120

TTACTCTTCA AGCCCTGACT GTGGAGTTGG TAGATGCCTC TGATCCTCAG TATTCTCTCT 180

GGCAATGTTC CACGGCTTCT CCTTCCTGGG AGCTGGCTCC ATAACTTGAT TTTCCCCAAA 240

CGTGTTGCAA TCCCTGCTGC CCCTTAGCCA CCCAGGGTCT TGTGTGGGTA TGAGTGTAGA 300

GGATGGGGGT ATGCCAGGCC TGGGCCGTCC CAGGCAGGCC CGCTGGACCC TGATGCTACT 360

CCTATCCACT GCCATGTACG GTGCCCATGC CCCATTGCTG GCACTGTGCC ATGTGGACGG 420

CCGAGTGCCC TTYCGGCCCT CCTCAGCCGT GCTGCTGACT GAGCTGACCA AGCTACTGTT 480

ATGCGCCTTC TCCCTTCTGG TAGGCTGGCA AGCATGGCCC CAGGGGCCCC CACCCTGGCG 540

CCAGGCTGCT CCCTTCGCAC TATCAGCCCT GCTCTATGGC GCTAACAACA ACCTGGTGAT 600

CTATCTTCAG CGTTACATGG ACCCCAGCAC CTACCAGGTG CTGAGTAATC TCAAGATTGG 660

AAGCACAGCT GTGCTCTACT GCCTCTGCCT CCGGCACCGC CTCTCTGTGC GTCAGGGGTT 720

AGCGCTGCTG CTGCTGATGG CTGCGGGAGC CTGCTATGCA GCAGGGGGCC TTCAAGTTCC 780

CGGGAACACC CTTCCCAGTC CCCCTCCAGC AGCTGCTGCC AGCCCCATGC CCCTGCATAT 840

CACTCCGCTA GGCCTGCTGC TCCTCATTCT GTACTGCCTC ATCTCAGGCT TGTCGTCAGT 900

GTACACAGAG CTGCTCATGA AGCGACAGNG GCTGCCCCTG GCACTTCAGA ACCTCTTCCT 960

CTACACTTTT GGTGTGCTTC TGAATCTAGG TCTGCATGCT GGCGGCGGCT CTGGCCCAGG 1020

SCTCCTGGAA GGTTTCTCAG GATGGGCAGC ACTCGTGGTG CTGAGCCAGG CACTAAATGG 1080

ACTGCTCATG TCTGCTGTCA TGAAGCATGG CAGCAGCATC ACACGCCTCT TTGTGGTGTC 1140

CTGCTCGCTG GTGGTCAACG CCGTGCTCTC AGCAGTCCTG CTACGGCTGC AGCTCACAGC 1200

CGCCTTCTTC CTGGCCACAT TGCTCATTGG CCTGGCCATG CGCCTGTACT ATGGCAGCCG 1260

CTAGTCCCTG ACAACTTCCA CCCTGATTCC GGACCCTGTA GATTGGGCGC CACCACCAGA 1320

TCCCCCTCCC AGGCCTTCCT CCCTCTCCCA TCAGCAGCCC TGTAACAAGT GCCTTGTGAG 1380

AAAAGCTGGA GAAGTGAGGG CAGCCAGGTT ATTCTCTGGA GGTTGGTGGA TGAAGGGGTA 1440

CCCCTAGGAG ATGTGAAGTG TGGGTTTGGT TAAGGAAATG CTTACCATCC CCCACCCCCA 1500

ACCAAGTTCT TCCAGACTAA AGAATTAAGG TAACATCAAT ACCTAGGCCT GAGAAATAAC 1560

CCCATCCTTG TTGGGCAGCT CCCTGCTTTG TCCTGCATGA ACAGAGTTGA TGAAAGTGGG 1620

GTGTGGGCAA CAAGTGGCTT TCCTTGCCTA CTTTAGTCAC CCAGCAGAGC CACTGGAGCT 1680

GGCTAGTCCA GCCCAGCCAT GGTGCATGAC TCTTCCATAA GGGATCCTCA CCCTTCCACT 1740

TTCATGCAAG AAGGCCCAGT TGCCACAGAT TATACAACCA TTACCCAAAC CACTCTGACA 1800

GTCTCCTCCA GTTCCAGCAA TGCCTAGAGA CATGCTCCCT GCCCTCTCCA CAGTGCTGCT 1860

CCCCACACCT AGCCTTTGTT CTGGAAACCC CAGAGAGGGC TGGGCTTGAC TCATCTCAGG 1920

GAATGTAGCC CCTGGGCCCT GGCTTAAGCC GACACTCCTG ACCTCTCTGT TCACCCTGAG 1980

GGCTGTCTTG AAGCCCGCTA CCCACTCTGA GGCTCCTAGG AGGTACCATG CTTCCCACTC 2040

TGGGGCCTGC CCCTGCCTAG CAGTCTCCCA GCTCCCAACA GCCTGGGGAA GCTCTGCACA 2100

GAGTGACCTG AGACCAGGTA CAGGAAACCT GTAGCTCAAT CAGTGTCTCT WTAACTGCAT 2160

AAGCAATAAG ATCTTAATAA AGTCTTCTAG GCTGTAGGGT GGTTCCTACA ACCACAGCCA 2220

AAAAAAAAAA AAAAAAACTC GAG 2243

(2) INFORMATION FOR SEQ ID NO: 145:

(i) SEQUENCE CHARACTERISTICS:
(A) LENGTH: 1082 base pairs
(B) TYPE: nucleic acid
(C) STRANDEDNESS: double
(D) TOPOLOGY: linear

(xi) SEQUENCE DESCRIPTION: SEQ ID NO: 145:

GCCAAGCTCT AATACGACTC ACTATAGGGA AAGCTGGTAC GCCTGCAGKT ACCGGTTCCG 60

GGAATTCCCG GGTCGACCCA CGCGTCCGCT TCCGTGTGTC AAAATCCTCA CCTCCTTCAT 120

AACCATCTCC CACAATTAAT TCTTGACTAT ATAAATTTAT GGTTTGATAA TATTATCAAT 180

TTGTAATCAA TTGAGATTTC TTTAGTGCTT GCTTTTCTGT GACTCAACTG CCCAGACACC 240

TCATTGTACT TGAAAACTGG AACANCTTGG GAATGCCATG GGGTTTGATA ATCTGCCAGG 300

GACATGAAGA GGCTCAGCTT CCTGGGACCA TGACTTTGGC TCAGCTGATC CTGNACATGG 360

GAGAACAACC ACATTTTTCT TTGTGTGTGC TTCTAGCAGC TGTTCGGGAG GACCKTGACC 420

CAAYAGTGTT CCCATGCTGT TTCTTGTGAA ATGCTCTCGG CTATGTAGCA GCTTTTGATT 480

CCCTGCATAC CCTAGGCTGC TGCCCCTATC CTGTCCCTTG TTTATAACAT TGAGAGGTTT 540

TCTAGGGCAC ATACTGAGTG AGAGCAGTGT TGAGAAGTCG GGGAAAATGG TGACTACTTT 600

TAGAGCAAGG CTGGGCATCA GCACCTGTCC AGCTCTACTT GTGTGATGTT TCAGGAACTC 660

AGCCCCTTTT TCTGCCTAGG ATAAGGAGCT GAAAGATTAA CTTGGATCTY CTAATGGTCC 720

AAATCTTTTG GTCACAATAA AGAGTCTCCA AATTAGAGAC TGCATGTTAG TTCTGGATGG 780

ATTTGGTGGC CTGACATGAT ACCCTGCCAG CTGTGAGGGG ACCCCGTTTT TAAGATGCAT 840

GGCCAAGCTC TCTGCAAATG GAAATGCTTA CACTGGGTGT TGGGGATGTT TGCTACCTCC 900

TGCTATTTTT GTGGTTTTGG TTCTCCCACT ATGGTAGGAC CCCTGGCCAG CATTGTGGCT 960

TGTCATGTCA GCCCCATTGA CTACCTTCTC ATGCTCTGAG GTACTACTGC CTCTGCAGCA 1020

CAAATTTCTA TTTCTGTCAA TAAAAGGAGA TGAAAATAAA AAANAAAAAA AAAAAACTCG 1080

NG 1082

(2) INFORMATION FOR SEQ ID NO: 146:

(i) SEQUENCE CHARACTERISTICS:
(A) LENGTH: 4313 base pairs
(B) TYPE: nucleic acid
(C) STRANDEDNESS: double
(D) TOPOLOGY: linear

(xi) SEQUENCE DESCRIPTION: SEQ ID NO: 146:

CAAGCTGGTT TGAAACTAGG GGTCGGGCTC GGCCGTCGTC GTTGTTTGTC GCCGCATCCC 60

CGCTTCCGGG TTAGGCCGTT CCTGCCCGCC CCCTCCTCTC CTCCCTTCGG ACCCATAGAT 120

CTCAGGCTCG GCTCCCCGCC CGCCGCAGCC CACTGTTGAC CCGGCCCGTA CTGCGGCCCC 180

GTGGCCACCA TGTCCCTGCA CGGCAAACGG AAGGAGATCT ACAAGTATGA AGCGCCCTGG 240

ACAGTCTACG CGATGAACTG GAGTGTGCGG CCCGATAAGC GCTTTCGCTT GGCGCTGGGC 300

AGCTTCGTGG AGGAGTACAA CAACAAGGTT CAGCTTGTTG GTTTAGATGA GGAGAGTTCA 360

GAGTTTATTT GCAGAAACAC CTTTGACCAC CCATACCCCA CCACAAAGCT CATGTGGATC 420

CCTGACACAA AAGGCGTCTA TCCAGACCTA CTGGCAACAA GCGGTGACTA TCTCCGTGTG 480

TGGAGGGTTG GTGAAACAGA GACCAGGCTG GAGTGTTTGC TAAACAATAA TAAGAACTCT 540

GATTTCTGTG CTCCCCTGAC CTCCTTTGAC TGGAATGAGG TGGATCCTTA TCTTTTAGGT 600

ACCTCAAGCA TTGATACGAC ATGCACCATC TGGGGGCTGG AGACAGGGCA GGTGTTAGGG 660

CGAGTGAATC TCGTGTCTGG CCACGTGAAG ACCCAGCTGA TCGCCCATGA CAAAGAGGTC 720

TATGATATTG CATTTAGCCG GGCCGGGGGT GGCAGGGACA TGTTTGCCTC TGTGGGTGCT 780

GATGGCTCGG TGCGGATGTT TGACCTCCGC CATCTAGAAC ACAGCACCAT CATTTACGAA 840

GACCCACAGC ATCACCCACT GCTTCGCCTC TGCTGGAACA AGCAGGACCC TAACTACCTG 900

GCCACCATGG CCATGGATGG AATGGAGGTG GTGATTCTAG ATGTCCGGGT TCCTGCACAC 960

CTGTSGCCAG GTTAAACAAC CATCGAGCAT GTGTCAATGG CATTGCTTGG GCCCCACATT 1020

CATCCTGCCA CATCTGCACT GCAGCGGATG ACCACCAGGC TCTCATCTGG GACATCCAGC 1080

AAATGCCCCG AGCCATTGAG GACCCTATCC TGGCCTACAC AGCTGNAAGG WGAGATCAAC 1140

AATGTGCAGT GGGCATCAAC TCAGCCCGAA YTGTCGCCAT CTGCTACAAC AACTGCCTGG 1200

AGATACTCAG AGTGTAGTGT TGGTGGCGCT GTGCCCACGA GGCAGGGGCT TTTGTATTTC 1260

CTGCCTCTGC CCCACCCCCA AAGTAAGAAG AAACATGTTT CCAGTGGCCA GTATGTCTTT 1320

CATTGCTTTG CACCCACTGT TACCAGAAGC TGCTCTAGGA GTTCCTGGCC AGTCACCCCA 1380

TCGCCCTCTG TGGCAGACTC AGTGCTGTGT GGCGCCTCCT CAGCCCAGGG CTGAGTTTTA 1440

AGATTTTCTC TCCTTTCCTC TTCTCCTTTG GTTCCTCAAT TAAAAAATGT GTGTATATTT 1500

GTTTGTCAGG CGTTGTGTTG AGGAGCAGTT CACGCACTGG CTGTGTCTAT TCCTCTGCCC 1560

AGGTGTCTCT GTTTGCTGCC CAAKGYWKKT TTTCATGTCT CGTCCATGTC CATGTTCGTG 1620

TTAGCACTWA CGTGGGAACA AATACCAATT TGTCTTTTCT CCTAGTATCA GTGTGTTTAA 1680

CAAATTTTAA CTTTGTATAT TTGTTATCTA TCAGGCTAAT TTTTTTATGA AAAGAATTTT 1740

ACTCTCCTGC TTCATTTCTT TGTCTTATAG TCCTCCCTCT TTGCACCTTC TTCTCTTCCC 1800

TCAGTGCCTG GAGCTGGTAC TGGGCCCCTG GCCCCATGAG CAGTTTGCCT TCTTGAGTCA 1860

CTGCCTGTGT AGTACATACC TGACCGGGAG TCCAAACCAC CTTGGTGCTC TGAAGTCCAC 1920

TGACTCATCA CACCTTTCTT AGCCTGGCTC CTCTCAAGGG CATTCTGGGC TTGTAAACAG 1980

ACATAGGAAG CCTCTGTTTA CCCTGAAGCA CCACTGTCCA GCCCATTGGT TCCCACTGGC 2040

AGCATGGTAG AGCTGAGAGA AACAGGCTCT CAGGGTACCT GACTTGAGGG GAATCGTTTC 2100

ATGAAGCTGA ACTTCAAGCA TATTTCCAGT ACATTCTTTC AGAGTCTGTT TTTCCATCCA 2160

AATATAAGCC CCAGGCCATT CCACTTAGTG TCTTTTCAAT GATAGGCAAG AATGATATCT 2220

GAGTTGAACT TCGGTGCTTC TGTTGTTTGA GTTTACTGTG CCTGGTGGTA TATTGGGCAT 2280

TCTTTGGATT GAGTGTTCTG AGGTGAGAGA GTCTTCCCGA GGCATCCTGT CTGTGCTTCC 2340

AACCCTGAAC AAGACCTTAC ATGAGAGATG GACTGATGGA CTGCGGCAAT CCTGGGCTGT 2400

CAAGTGGATA GATAGTTAAA AAGCATTATA CTGTGGGTAA TGAAAAGGGA GGAAAAAAAA 2460

AGAAGGAAAA GGAATTATAG ACCCCCAGGG TCAGCCAGTT AAGAGCTCTA CCCACACCTG 2520

TCAACCCCTC TCTCCCCCAG TTTAGGTTCT GAGCAGTATT GGACTTGTAG CCTGCAGTTG 2580

TCTTTTGACT TGCAGGCCGC AGTGTCTTTC TGTTATGTGA ATGAGTTCCA TGGAGGGGCA 2640

TATGTGTGAT TCCACCGTTA GATGAGCCCT TGGGGCAGGC AGTTTGGGAT GTGCTCTTGG 2700

GGGAAAGTTG GCTGTTTCCT TGCGCTCTGC TCCTACCCGA AGTTTTTAAG TCCCTCTGAA 2760

TTGCTCATCT GAGATTAGTA GAGTAGCAGG CCTGAAGGAT GATGGTTTTG TCCTCTTTGG 2820

TTCTCACCTG CTTGAGAAGT AAAACAGTAA CTTTGTTCTT CTGGGCCCTT AAGCTTTTTT 2880

GGTTAAGTCT TCCTTTTCAG AAGTAGATGT CATTATATGC CAAAAGTCTA GCTCTTTGCT 2940

TTACCATACA GGGACCTGTC CCAAAGAAAA AGGCTCTTTT TTTAGCCAGC ATATTTCCCC 3000

TTCTACCCTT TTACTTTGTT GTTCTGATTT TAGGACTCTG GCTGGCCATG TGCTTGTGGT 3060

TGCCTCTCCT GCATTTGCCA CTGGATTTGC ACTGCATCGT TTGGAGATAC AAAGCGAGCA 3120

GTTCTTGGTC AGAACCCTCC TCTGCTTTTC ATTGTGTTTG ATAATGGTTA CTGGGTCCTT 3180

CTCTCAAGGG TAGCAAGGCC AAGCTGATGG CTGCTTGTTT AGGAGGCCAT CAGTTCCTTC 3240

CTGTGGAGAA GGGTCTGAAA TGGAAGTCAG TGGTAGAAGG GGCTGGTCTG CTGGGCAGGG 3300

CTTACATCCA CTGAGTTCTA AGATTCCTTT CCTGATCTGC ACCTACGCCT GGTCTGTATG 3360

GTGGAATTTG TCAGCTGGAA CTCAGAAACA ACAACTTGAA AAAAAAATAA TAATTAGAAC 3420

ATATTTGCAT AAGATAGCTA TTTACTCTGG AAACCAACAA CTTTTGAGAT TTCCCTTGCC 3480

CTGTGGACGC CCAGCTCCTG TCATCCTTCC TTAGGTCCTG CAGTACAGTC TTCCCCTGAA 3540

TGCCACCGGG GACCCAGGGG GACTCCACCC CCCTAAGCAA GCACACACAT ACTCACAGTT 3600

GATGAGTTGC TGGTCTTTGA GTCCCAGCTC TCTTACCCTC CCTTTACTCC ACCAGCCCGA 3660

CGACCCATGA CTGAGGAGGG GATTTCTACA GTCTCAGGAT TTAGAAAGTC TGTAAGCCAT 3720

CCATGCTCCA GAAAGCACCG ATCTGTTGTA GTTGCAAAAA CAACTCTGTA ATTTGTTGAG 3780

GTTCTCAAAC TGACAGCCAG CGAGACTGGG TGGGAGGCCC TGGATCTGTT CTCCCTGACT 3840

GCGGGAGGAG CAGCCACTAG GACTTTAGCA GGAAGCCCAC ATGGAGGCTC CGCCAGGCTG 3900

TGGCCCAGCT GGTGATGGCC CTTTTGCTCC TGGCAGCCTG AGGCACAGCT GCCTGTATTG 3960

TCCTCATCTG TTCTGACTGA AGGATGGAGG TGCTGAATAA ATTAGGCCTC AGGCNTCTAC 4020

CACCAGAGAG CTGGAGAATG GGTCCACGTC ATTCAAGGAC CTGAATTTTT TATGCTCAGG 4080

AGCATTGGAA TCCTCTTCTT CCAGGGAGGA ATTAGCCTGC AAGGTTAGGA CTTGAAGAGG 4140

GAAGGTATTT AATAACTGGG CGAGGATGGG TGTGGTGGCT CACACCTGTA ATCCCAGCAT 4200

TTTGGGAGGC TGAGGTGGCC AGATCCCAAG GTCAGAAGAT CGAGACCATC CTGGCTAACA 4260

TGGTGAAACC CCATCTCTAC TAAAAATACA AAATTAAATT GGCCGGGCGT GAA 4313

(2) INFORMATION FOR SEQ ID NO: 147:

(i) SEQUENCE CHARACTERISTICS:
(A) LENGTH: 1183 base pairs
(B) TYPE: nucleic acid

(C) STRANDEDNESS: double
(D) TOPOLOGY: linear

(xi) SEQUENCE DESCRIPTION: SEQ ID NO: 147:

GGCAGAGCCT CAAGCTGACT TGGATTATGT GGTCCCTCAA ATCTACCGAC ACATGCAGGA 60

GGAGTTCCGG GGCCGGTTAG AGAGGACCAA ATCTCAGGGT CCCCTGACTG TGGCTGCTTA 120

TCAKWYGGGG AGTGTCTACT CAGCTGCTAT GGTCACAGCC CTCACCCTGT TGGCCTTCCC 180

ACTTCTGCTG TTGCATGCGG AGCGCATCAG CCTTGTGTTC CTGCTTCTGT TTCTGCAGAG 240

CTTCCTTCTC CTACATCTGC TTGCTGCTGG GATACCCGTC ACCACCCCTG GTCCTTTTAC 300

TGTGCCATGG CAGGCAGTCT CGGCTTGGGC CCTCATGGCC ACACAGACCT TCTACTCCAC 360

AGGCCACCAG CCTGTCTTTC CAGCCATCCA TTGGCATGCA GCCTTCGTGG GATTCCCAGA 420

GGGTCATGGC TCCTGTACTT GGCTGCCTGC TTTGCTAGTG GGAGCCAACA CCTTTGCCTC 480

CCACCTCCTC TTTGCAGTAG GTTGCCCACT GCTCCTGCTC TGGCCTTTCC TGTGTGAGAG 540

TCAAGGGCTG CGGAAGAGAC AGCAGCCCCC AGGGAATGAA GCTGATGCCA GAGTCAGACC 600

CGAGGAGGAA GAGGAGCCAC TGATGGAGAT GCGGCTCCGG GATGCGCCTC AGCACTTCTA 660

TGCAGCACTG CTGCAGCTGG GCCTCAAGTA CCTCTTTATC CTTGGTATTC AGATTCTGGC 720

CTGTGCCTTG GCAGCCTCCA TCCTTCGCAG GCATCTCATG GTCTGGAAAG TGTTTGCCCC 780

TAAGTTCATA TTTGAGGCTG TGGGCTTCAT TGTGAGCAGC GTGGGACTTC TCCTGGGCAT 840

AGCTTTGGTG ATGAGAGTGG ATGGTGCTGT GAGCTCCTGG TTCAGGCAGC TATTTCTGGC 900

CCAGCAGAGG TAGCCTAGTC TGTGATTACT GGCACTTGGC TACAGAGAGT GCTGGAGAAC 960

AGTGTAGCCT GGCCTGTACA GGTACTGGAT GATCTGCAAG ACAGGCTCAG CCATACTCTT 1020

ACTATCATGC AGCCAGGGGC CGCTGACATC TANGACTTCA TTATTCWATR ATTCAGGACC 1080

ACAGTGGAGT ATGATCCCTA ACTCCTGATT TGGATGCATC TGAGGGACAA GGGGGKCGGT 1140

STCCGAAGTG GAATAAAATA GGCGGGCGTG GTGACTTGCA CCT 1183

(2) INFORMATION FOR SEQ ID NO: 148:

(i) SEQUENCE CHARACTERISTICS:
(A) LENGTH: 734 base pairs
(B) TYPE: nucleic acid
(C) STRANDEDNESS: double
(D) TOPOLOGY: linear

(xi) SEQUENCE DESCRIPTION: SEQ ID NO: 148:

GAATTCGGCA GAGTGAAGCA TTAGAATGAT TCCAACACTG CTCTTCTGCA CCATGAGACC 60

AACCCAGGGC AAGATCCCAT CCCATCACAT CAGCCTACCT CCCTCCTGGC TGCTGGCCAK 120

GATGTCGCCA GCATTACCTT CCACTGCCTT TCTCCCTGGG AAGCAGCACA GCTGAGACTG 180

GGCACCAGGC CACCTCTGTT GGGACCCACA GGAAAGAGTG TGGCAGCAAC TGCMTGGCTG 240

ACCTTTCTAT CTTCTCTAGG CTCAGGTACT GCTCCTCCAT GCCCATGGYT GGGCCGTGGG 300

GAGAAGAAGC TCTCATACGC CTTCCCACTC CCTCTGGTTT ATAGGACTTC ACTCCCTAGC 360

CAACAGGAGA GGAGGCCTCC TGGGGTTTCC CCRRGGCAGT AGGTCAAACG ACCTCATCAC 420

AGTCTTCCTT CCTCTTCAAG CGTTTCATGT TGAACACAGC TCTCTCCRCT CCCTTGTGAT 480

TTCTGAGGGT CACCACTGCC ARCCTCAGGC AACATAGAGA GCCTCCTGTT CTTTCTATGC 540

TTGGTCTGAC TGAGCCTAAA GTTGAGAAAA TGGGTGCCAA GGCCAGTGCC AGTGTCTTGG 600

GGCCCCTTTG GCTCTCCCTC ACTCTCTGAG GCTCCAGCTG GTCCTGGGAC ATGCAGCCAG 660

GACTGTGAGT CTGGGCASGT CCAAGGCCTG CACCTTCAAG AAGTGGAATA AATGTGGCCT 720

TTGCTTCTAT TTAA 734

(2) INFORMATION FOR SEQ ID NO: 149:

(i) SEQUENCE CHARACTERISTICS:
(A) LENGTH: 1405 base pairs
(B) TYPE: nucleic acid
(C) STRANDEDNESS: double
(D) TOPOLOGY: linear

(xi) SEQUENCE DESCRIPTION: SEQ ID NO: 149:

GGCACAGTGG ACCCCAGACT CCCTCTCCGC CTTTCTCTGC CTGGGGAGAC CCACTGTGTG 60

CATGGCATCA CTGACTCCCA TACCTCTGGC TATCAAAGGT TTCTGCCATG GCCACCCTGG 120

AAGSAAACCA GAGGGAGGTA GACAGGGAGA TCAGGTCCCT TCTACTCTGG TTCCTGCTCT 180

GTGAAATTGT CTCAGGCTGG CTGTGTCCAG ARGGTCCCTG GTTCTCTCAR GGATGCCAAA 240

TCTACAAGAA TCTCTCCTCT TCCAGTTCCT ATAACCTCTC CTTCCTTTTG TCTCTTTAGA 300

CCTTGGAGTA GTAGCAGCCA GGTTCTTTCT ATCTCTGGGT TAGTGCATTA TCTCTGGTGG 360

CTCCCTTACC CAGGACTTTG GGAATGGTCT TTTTGTAATA CATTCTCCTC AAATAATTCA 420

ATTTTGAGTG TTCTGTATGT ATCCTGCTGG GAGGTTGTTA TATACAAATC ACTGTGCCCG 480

TTTAGCAGAG AAGGAGACTG AAGCTCAGGG AGGTTAAGTG TCTTTCTCTA GGTCGTATTG 540

TGGAGAAAGT GGCTGACTGG GGACTTGAAT GAGGTCCCTA GTTTCATGCT CGGAGGGCAA 600

AGANGAATGT CCAATTGGCC TGAGATAAGC CTCTGGTAAA ATGTACTGTA CATAATAGGT 660

AATCAATAAA TGTTGGCTGA TGACAAACAT GTTTTCTTTG TTCATTAGTT ATAGTGATTA 720

TGTTCTAAAT AACTCCMACA AGGAARTCAG CACATTTGGA ATATCAWTAT CTTTCCATGA 780

TAATATCTTT CCMYGGAAAG AWAATGATAT TCCMAACTGG GAGTGTCCCW AGCARATCTG 840

ANTCTGTGTA TTGGCCCTGG GGTGGGCCAG CCCCTTAGAC TCTATGGTCT CATTCTCTTT 900

GTTTACAAAA TTGAGATAAG GCCTTATTCT CTCCCCACCC CACCCATCCA TATTGTTTTG 960

AGAATAAAAT GAGAGGATGT GTGTCAAGGG TGTATTTTGG CAATAGTCTC TGAGCCATTT 1020

TCTGAGCACC TCCATACTGT TGACACTCAA GTAATATTTC ATCAGCATTC CATTCAGGNT 1080

CCTCCCTTAA TGAGGTGTGC GATGTACAAG AGTYGTGAGG TGGCAAAGGA TGGGCTCCTG 1140

AGGAAACACT TAGGAAACTG GGCTTTCTGC CATTAAAAGA GACAAACCTT TGTGGTGACC 1200

TAATTAAAGT TTTTAAAATT CAATTTGGAA AGTTAGCAAG CTAGCTCCTK TCCAGGWAAA 1260

ATAAGGAGTC AGTGCATGAC CTAACCGGTC CCGGGCTGCT TGCCATTCCA AACAACTGCA 1320

GTAAGTTTAT CACNTTCTTT CAGGGACTGA GGTTTCCAGG CACAGACTTG GATAAGGAAG 1380

GATGTCCTAT GGGGTCACAT TGATG 1405

(2) INFORMATION FOR SEQ ID NO: 150:

(i) SEQUENCE CHARACTERISTICS:
(A) LENGTH: 2890 base pairs
(B) TYPE: nucleic acid
(C) STRANDEDNESS: double
(D) TOPOLOGY: linear

(xi) SEQUENCE DESCRIPTION: SEQ ID NO: 150:

TTATATGCTA CAGCTACAGT AATTTCTTCT CCAAGCACAG AGGANCTTTC CCAGGATCAG 60

GGGGATCGCG CGTCACTTGA TGCTGCTGAC AGTGGTCGTG GGAGCTGGAC GTCATGCTCA 120

AGTGGCTCCC ATGATAATAT ACAGACGATC CAGCACCAGA GAAGCTGGGA GACTCTTCCA 180

TTCGGGCATA CTCACTTTGA TTATTCAGGG GATCCTGCAG GTTTATGGGC ATCAAGCAGC 240

CATATGGACC AAATTATGTT TTCTGATCAT AGCACAAAGT ATAACAGGCA AAATCAAAGT 300

AGAGAGAGCC TTGAACAAGC CCAGTCCCGA GCAAGCTGGG CGTCTTCCAC AGGTTACTGG 360

GGAGAAGACT CAGAAGGTGA CACAGGCACA ATAAAGCGGA GGGGTGGAAA GGATGTTTCC 420

ATTGAAGCCG AAAGCAGTAG CCTAACGTCT GTGACTACGG AAGAAACCAA GCCTGTCCCC 480

ATGCCTGCCC ACATAGCTGT GGCATCAAGT ACTACAAAGG GGCTCATTGC ACGAAAGGAG 540

GGCAGGTATC GAGAGCCCCC GCCCACCCCT CCCGGCTACA TTGGAATTCC CATTACTGAC 600

TTTCCAGAAG GGCACTCCCA TCCAGCCAGG AAACCGCCGG ACTACAACGT GGCCCTTCAG 660

AGATCGCGGA TGGTCGCACG ATCCTCCGAC ACAGCTGGGC CTTCATCCGT ACAGCAGCCA 720

CATGGGCATC CCACCAGCAG CAGGCCTGTG AACAAACCTC AGTGGCATAA AYCGAACGAG 780

TCTGACCCGC GCCTCGCCCC YTATCAGTCC CAAGGGTTTT CCACCGAGGA GGATGAAGAT 840

GAACAAGTTT CTGCTGTTTG AGGCACAGAC TTTTCTGGAA GCAGAGCGAG CCACCTGAAA 900

GGAGAGCACA AGAAGACGTC CTGAGCATTG GAGCCTTGGA ACTCACATTC TGAGGACGGT 960

GGACCAGTTT GCCTCCTTCC CTGCCTTAAA AGCAGCATGG GGSTTCTTCT CCCCTTCTTC 1020

CTTTCCCCTT TGCATGTGAA ATACTGTGAA GAAATTGCCC TGGCACTTTT CAGACTTTGT 1080

TGCTTGAAAT GCACAGTGCA GCAATCTTCG AGCTCCCACT GTTGCTGCCT GCCACATCAC 1140

ACAGTATCAT TCCAAATTCC AAGATCATCA CAACAAGATG ATTCACTCTG GCTGCACTTC 1200

TCAATGCCTG GAAGGATTTT TTTTAATCTT CCTTTTAGAT TTCAATCCAG TCCTAGCACT 1260

TGATCTCATT GGGATAATGA GAAAAGCTAG CCATTGAACT ACTTGGGGCC TTTAACCCAC 1320

CAAGGAAGAC AAAGAAAAAC AATGAAATCC TTTGAGTACA GTGCTTGTCC ACTTGTTTAC 1380

AATGTCCTCC TTTTAAAAAA AAAAAAATGA GTTTAAAGAT TTTGTTCAGA GAGTAAATAT 1440

ATATCCATTT AATGATTACA GTATTATTTT AAACCTTAAG TAGGGTTGCC AGCCTGGTTT 1500

CTGAAAAACC AAATATGCCG GACAGGGTGT GGCCACACCA AGAAGACGGG AAGACCTGGC 1560

TTGTGACCCT GGCTTCCCAT GTCCTTCTGG TCTCACCCGC GAAGTGCCCT ATCCTGGAAG 1620

TATGAAATGT TAGCCAATTA ATACCAAGAC ACCTCATCTG CTCCTTCCCC AGTGGATGGG 1680

GTTCTTCTGT AAAACTGTTT GCACATGGCC AGGGGAGGGA ACTAGGACCC TTGTGTCCTG 1740

TCTGAGCCTT ATGGAGGCAG GACGGTGTCA TTGGCGGATG TGTCCTGCTC CATTGAGATG 1800

GATGGCAAAC CCCATTTTTA AGTTATATTT CTTTGATTTT TGTTAATTTA GAGGTGTAGG 1860

TTTTGTTTTT TGTTTTTTTG TTTTTTTTTA AGAGAAACAT TTATAACTGG ATAGCATTGC 1920

AGTGAAAGCA GCTTGGGATG TTGGAGCTAA TGCCAGCTGT TTATACTGCT CTTTCAAGAC 1980

AGCCTCCCTT TATTGAATTG GCATTAGGGA ATAAACAAGC CTTTAAACGT GATAAAAGAT 2040

CAAAAACCTG GTTAGACATG CCAGCCTTTG CAAGGCAGGT TAGTCACCAA AGACTAACCT 2100

CCAAGTGGCT TTATGGACGC TGCATATAGA GAAGGCCTAA GTGTAGCAAC CATCTGCTCA 2160

CAGCTGCTAT TAACCCTATA ATGACTGAAA TGACCCCTCC ACTCTATTTT TGTGTTGTTT 2220

TGCACAGACT CCGGAAAAGT GAAGGCTGCC AATCTGAGTA GTACTCAAAT GTGAGGAACT 2280

GCTGGTCTTG GATTTTTTTT CCATTAAATT CAGCTGATCA TATTGATCAG TAGATAAACG 2340

TAAATAGCTT CAAATTTTAA AAGTGGAATT GCAGTGTTTT TTCACTGTAT CAAACAATGT 2400

CAGTGCTTTA TTTAATAATT CTCTTCTGTA TCATGGCATT TGTCTACTTG CTTATTACAT 2460

TGTCAATTAT GCATTTGTAA TTTTACATGT AATATGCATT ATTTGCCAGT TTTATTATAT 2520

AGGCTATGGA CCTCATGTGC ATATAGAAAG ACAGAAATCT AGCTCTACCA CAAGTTGCAC 2580

AAATGTTATC TAAGCATTAA GTAATTGTAG AACATAGGAC TGCTAATCTC AGTTCGCTCT 2640

GTGATGTCAA GTGCAGAATG TACAATTAAC TGGTGATTTC CTCATACTTT TGATACTACT 2700

TGTACCTGTA TGTCTTTTAG AAAGACATTG GTGGAGTCTG TATCCCTTTT GTATTTTTAA 2760

TACAATAATT GTACATATTG GTTATATTTT TGTTGAAGAT GGTAGAAATG TACTATGTTT 2820

ATGCTTCTAC ATCCAGTTTG TACAAGCTGG AAAATAAATA AATATAACAT AAAAAAAAAA 2880

AAAAAAAAAA 2890

(2) INFORMATION FOR SEQ ID NO: 151:

(i) SEQUENCE CHARACTERISTICS:
(A) LENGTH: 2399 base pairs
(B) TYPE: nucleic acid
(C) STRANDEDNESS: double
(D) TOPOLOGY: linear

(xi) SEQUENCE DESCRIPTION: SEQ ID NO: 151:

GAACTTTTCC ATCTGGCAAA CCGGAAACTC CATCCCCATT AAACCAACTC CCCCTTTTGG 60

TTTCCCCCCC AGNGGAATAG AATTTGGACN CCCATATAAA TCCAGGAAAC CACCTAAATT 120

CTTTAGTNGT TTGTGTTTGC AAGATCTAAG GTCATGGTAA ACATTAAGTT CTTAAAATTT 180

TTGGGAGGGA CCAGTGCACC TCTCCCTCTG AATTGTTCNC CAATTTAAAA TTGGAGTAAG 240

GTTTTAAAAT GTCTNATTCC ATTGGAAGGG TNTGTTATTT CATTTTGAGC CCAGAGGGGA 300

GAGGCACATT TTAAATATCA GAATTAGATT AGCTTTGAGT TTGTACAATT GGGAACATAA 360

TAGATTTTCA TAAATTATGT GTGCCTTGTT GGAAGTGTCA ACTGTCTTTA TGTCTGCTTG 420

TAAAAGTTTC AAAATATGTT TTCCCTCAAA AAGGCAACGT TACTTCATTT GCTTGAATAT 480

TATGATAGGA ATGCTTACTG ATATTACTTG ATAGTCATAT ATAGCCTAGG AAATTTAACA 540

TATATATAAC TATAGCAGTA TTAATAATGA TAGTTGTACT TCTTTAAAAC ATTAAATTTG 600

AGGAAACTTT AATGCTGTCT CGTGTACATT GCTTTACTAC AGTGAGGGGG AATATCCTTT 660

AGATTGAGCC TCAATTTACT GGTTAGTAGT ATGTGAACTC TGGTATAAAA ACGTAAACTA 720

GACAGTAGAG CCGATGAATT AAAATTGTAA ATTGCTACAT TGGCATTTTC TACCTCCTTT 780

TCTGTCAGAG TATTACTTTT TCCAGCATTT ATTCTTATTT GTGAGTAAAG AGGAAATGGG 840

AACCTGAGGT TAAAATTGAC ATTTTTGTTT CATTGAGAAT TTAAGCAGTA GGTACAGGAG 900

AAGTGACTTG TCACATTAAT TTGGTGCCTA AATCTGTAAC TACAAGTTGT GATCGACATG 960

TACAAAATGT CTAAGAAAGG TCATATGCTG AATATTTTAC TTTTCCTGTA TAGTCTGCAT 1020

GATTTGTTTC ATAAACCCAG CTTATTTCCT CCAAAAAGCA AAATGGTCCT GTAATTTTTA 1080

AAGTAAAATA AACGTGCCAT TTTGTCTGCA ATCTATAATT TCAGGAAGTT ATTGRAAGTT 1140

CTGACTCAGG GCTTTTTAAC AGTTCAAGCA ATTGTCAGTT ATATTTTGGA AACTCCATCT 1200

GTGTAATTCT CCAGTGCCTT GAAAGAATTA TTAACTTGGC AACACTATTA AAACTTTATA 1260

AAAGATGGTC TTTAGTGCAC GTGTATCATT ATATACACGT TTTAAAGTCA TATTGCTTAG 1320

CTTGTTAATA ATGATTCTGC ATGTGTGCTG GGTTTGGGTA ATTCTTTAAA GGAAGTTTTC 1380

TAGATTTGCA CTTGATGTTT GTTTTTTAAA AACTGATTAT TTATGGCCGT GACACTGTTA 1440

CCAGAAAAGT AATTCTAATT AAGTTATTAT GCAAAGTCAT CTATAAGTAG CATCTGGGAA 1500

GAGGAGATSG AGGCCACAGT TTGCTATTTT AGTATGAAAG GAGGATCTGT TTGGGAAACA 1560

TAGATTGTCT TCCCCTCAAA TGAGGGGAAA AAAAAAGACC CTTTGTTCAA ATGGATTCTG 1620

TTGTAAAAAA TTATTTTTAA AGGAAATCAC AAATTGTATG TCATTCTTAA TGCTAGTCTT 1680

ATAGAATAAA TCCATAAAAT TGTTTTTATG TTCAGTATGT TTATGTCATT CTAAATGCAG 1740

CAAATTCAAT GATAGCAGTT CAATTGACTC ATAGCAGTGT TTTGTATTTT TTCTAATTCT 1800

TTAGCTTTCA ATATTGGATT AAAGTCTTGT TTGTGAATAT AGTTTCCGTA TGGCAAATGA 1860

TTTCTTGCTT ATTAGCTTTT GTTAAAGAAT GCTTAGTAAG AGCTAAGCTT TTAAAAGTAA 1920

TGCAAACATT TATCGTTAAT AAAACCTATG GTGTAATATC ATATAATGCT TTTCTTTGAT 1980

CTTTGGAGAA TTATTCTTTT ATAGTAGTAT ACATGAATTT TGATTTTTAA AGCATTTAAA 2040

AACAAATCTC AATACATTAA AAAACCTGTT ATTGTTAAAA RGGAAATTAC CATGCCTTTA 2100

AGAAACAAGG ATGTACATCT TCAATTCAGC ATRAGTGTCC ACATCTAGAA GGCTCTCATT 2160

GCAGTTGTTT ACAGTTAAGG TACCTCTATC TAAAGGGCCA AAGAAGCATT TCATAYTTTA 2220

ACACCTCACA TTCTTTCAGG ATTAAGACAT ATGAAAATAG TCTGAATAGG ATAAATTTGG 2280

ATAGGAAGTA ACTTAACCAG TCTGGGAAGA TTCAGGCTTT TTCTATKAAA AAGCTTATTC 2340

CTCTTCACAA CTCNGGTGGT AGGNTTTCAT TTTTCAAGAG GGTAGATATT TTAAAGCCA 2399

(2) INFORMATION FOR SEQ ID NO: 152:

(i) SEQUENCE CHARACTERISTICS:
(A) LENGTH: 802 base pairs
(B) TYPE: nucleic acid
(C) STRANDEDNESS: double
(D) TOPOLOGY: linear

(xi) SEQUENCE DESCRIPTION: SEQ ID NO: 152:

CGTGCCTGTA GTAAGCTCAT CCCTGCCTTT GAGATGGTGA TGCGTGCCAA GGACAATGTT 60

TACCACCTGG ACTGCTTTGC ATGTCAGCTT TGTAATCAGA GATTNTGTGT TGGAGACAAA 120

TTTTTCCTAA AGAATAACWT GAYCCTTTGC CARACGGACT ACGAGGAAGG TTTAATGAAA 180

GAAGGTTATG CACCCCMGGT TCGCTGATCT ATCAACATCA CCCCATTAAG AATACAAAGC 240

ACTACATTCT TTTATCTTTT TTGCTCCACA TGTACATAAG AATTGACACA GGAACCTACT 300

GAATAGCGTA GATATAGGAA GGCAGGATGG TTATATGGAA TAAAAGGCGG ACTGCATCTG 360

TATGTAGTGA AATTGCCCCA GTTCAGAGTT GAATGTTTAT TATTAAAGAA AAAAGTAATG 420

TACATATGGC TGGATTTTTT TGCTTGCTAT TCGTTTTTGT GTCACTTGGC ATGAGATGTT 480

TATTTTGGAC TATTGTATAT AATGTATTGT AATATTTGAA GCACAAATGT AATACAGTTT 540

TATTGTGTTA CCATTTGTGT TCCATTTGCT YCTTTGTATT GTTGCATTTA GTACAATCAG 600

TGTTTAAACT TACTGTATAT TTATGCTTTC TGTATTTACC AGCTATTTTA AATGAGCTGT 660

AACTTTCTAG TAAAGAATTG AAAAGCAAAT CCTCACTAAA GGATACACAG GATAGGATAA 720

AGCCAAGTCN CATCAACATT AAAAAATACT AAAANANAAA ACACAAAAAA AAAAAANCCC 780

GGGGGGGGCC CGGAACCCAT TC 802

(2) INFORMATION FOR SEQ ID NO: 153:

(i) SEQUENCE CHARACTERISTICS:
(A) LENGTH:-461 base pairs
(B) TYPE: nucleic acid
(C) STRANDEDNESS: double
(D) TOPOLOGY: linear

(xi) SEQUENCE DESCRIPTION: SEQ ID NO: 153:

CTAGGAGCAC CGAGCAGCTT GGCTAAAAGT AAGGGTGTCG TGCTGATGGC CCTGTGCGCA 60

CTGACCCGCG CTCTGCNCTC TCTGAACCTG GCGCCCCCGA CCGTCGCCGC CCCTGCCCCG 120

AGTCTGTTCC CCGCCGCCCA GATGATGAAC AATGGCCTCC TCCAACAGCC CTCTGCCTTG 180

ATGTTGCTCC CCTGCCGCCC AGTTCTTACT TCTGTGGCCC TTAATGCCAA CTTTGTGTCC 240

TGGAAGAGTC GTACCAAGTA CACCATTACA CCAGTGAAGA TGAGGAAGTC TGGGGGCCGA 300

GACCACACAG GTGGGAACAA GGACAGGGGG ATTTAAGCAG TCAAAAGGAA AAACATGTTA 360

AGACCCTAGA CTTGTATATT GACACACTTG TACCTTGTAA GGCAGAGGAA TGTAATTAAA 420

AAGCACTTAT TTGGCWNAAA AAAAAAAAAA AAAAAAAAAA C 461

(2) INFORMATION FOR SEQ ID NO: 154:

(i) SEQUENCE CHARACTERISTICS:
(A) LENGTH: 2388 base pairs
(B) TYPE: nucleic acid
(C) STRANDEDNESS: double
(D) TOPOLOGY: linear

(xi) SEQUENCE DESCRIPTION: SEQ ID NO: 154:

GCCCACGCGT CCGAAAGCGG AGAACGCTGG TGGGCCTGTT GTGGAGTACG CTTTGGACTG 60

AGAAGCATCG AGGCTATAGG ACGCAGCTGT TGCCATGACG GCCCAGGGGG GCTGGTGGCT 120

AACCGAGGCC GGCGCTTCAA GTGGGCCATT GAGCTAAGCG GGCCTGGAGG AGGCAGCAGG 180

GGTCGAAGTG ACCGGGGCAG TGGCCAGGGA GACTCGCTCT ACCCAGTCGG TTACTTGGAC 240

AAGCAAGTGC CTGATACCAG CGTGCAAGAG ACAGACCGGA TCCTGGTGGA GAAGCGCTGC 300

TGGGACATCG CCTTGGGTCC CCTCAAACAG ATTCCCATGA ATCTCTTCAT CATGTACATG 360

GCAGGCAATA CTATCTCCAT CTTCCCTACT ATGATGGTGT GTATGATGGC CTGGCGACCC 420

ATTCAGGCAC TTATGGCCAT TTCAGCCACT TTCAAGATGT TAGAAAGTTC AAGCCAGAAG 480

TTTCTTCAGG GTTTGGTCTA TCTCATTGGG AACCTGATGG GTTTGGCATT GGCTGTTTAC 540

AAGTGCCAGT CCATGGGACT GTTACCTACA CATGCATCGG ATTGGTTAGC CTTCATTGAG 600

CCCCCTGAGA GAATGGAGTT CAGTGGTGGA GGACTGCTTT TGTGAACATG AGAAAGCAGC 660

GCCTGGTCCC TATGTATTTG GGTCTTATTT ACATCCTTCT TTAAGCCCAG TGGCTCCTCA 720

GCATACTCTT AAACTAATCA CTTATGTTAA AAAGAACCAA AAGACTCTTT TCTCCATGGT 780

GGGGTGACAG GTCCTAGAAG GACAATGTGC ATATTACGAC AAACACAAAG AAACTATACC 840

ATAACCCAAG GCTGAAAATA ATGTAGAAAA CTTTATTTTT GTTTCCAGTA CAGAGCAAAA 900

CAACAACAAA AAAACATAAC TATGTAAACA AGAGAATAAC TGCTGCTAAA TCAAGAACTG 960

TTGCAGCATC TCCTTTCAAT AAATTAAATG GTTGAGAACA ATGCATAAAA AAAGTTGCAC 1020

AAGTTCCTTA TTTTCCTTAA TATTTCACTT CTATTTAATA CAAGCTGGGA CATAAAAATT 1080

CTGTTGGGGA TACCTGGGGG AAGATGTGAG AAACTAATGC TGAATTCAGC TTATACATGA 1140

TGAAAAGAAA AACCAGACAA AAGGAGCACA TAAATATGCA TACAGTGTAA CTGTTATTAT 1200

TTTAATACCC ACGATAAGGG ATTTTTGTTA GCATGTTTAG GGGGAACGAG GATTGGTGGG 1260

ATCCTTGGGG CCACAGGAAT CTGAGGCAAC GGAAGATATA TAGAGTGATC GTCCCCCTGC 1320

CGAAGGAACC TGGCAYCTGT CAAGCAGATG CTGCAGTTCA AACTTCAGCT TTTAAGATAG 1380

ATAGCTATTG AAGGCAGAGG GTCAGCAGGA GGATGTGTAT TTCTAATCTA CCCTGGTAAA 1440

GTCATAGGTA AGACTCAAAA GCGGGATCTT ATTCAAAAGG CAGGTATTTC CTTTGTTTTC 1500

TGTCTTGAAA TAGCCCCTTC CCCTAAGGTG CATTCTCTCA AGTTTTCAGT ATTGCTTTAT 1560

TTGCAGTGAT TAAAAGAGAT GAGAGACTTT GGAGACAGAC AACGTAAGCA ACACATACAC 1620

ACATGAAATA CTCTAGACAG AGATGAATAT AAATCTGGCC TAATAACCAG TTTTCCATGT 1680

AACAGTGATT TTGTGTTTCG GGCTGAAGCA GTGGTTATAT TAAAAGCCAC TAATTCCCTT 1740

ATCCCTTTAA AAGATTTTTA CAATTCTCCA ACCACAAACA GCACTTCTAA AACTAACTTT 1800

ACTTTCTGCC CATAATTTGT TCTACATGGA AAAAAAAAAT ATTACTTTGG CCAGGGGTGT 1860

GTGTAAATGT GGCAGAATTC CTAGGCAGGC TGACCTTTAC AGTATGGGCC TTTAAGATAC 1920

TGGATCCTGG TTGGGCAACA AGTGTCACGC CTGAAGTTTC TGAAAACAAA TTAGAAGACT 1980

GTTGGCTTGG CTAATCTCGT AGTTCAGGGC CAAGTTTCTG TAGTCAGAAT GAAGAATAAA 2040

ATTGAAAGAA AAAGGGGGAA ATGCTTATAC TTGGCATTAA GTTGAATGCC TCAAGTCTTA 2100

ACTATGGCTT TGTAGATGAG GCAAAAGATT TCTTAGTGGT AAAATTTCTT CAACAGGTCA 2160

ATGCCAATCT GTATGCCATT TTAGTAAAGT AGGTAAGGAG AGTAGCCGCT CAGTAACTTT 2220

GGCACTAAAG AAAGAGTGTG GCTCTAGAAC TTCCAATCCC ATTGCTAGAT GTGCCCTTTA 2280

AAAGATGGTC CAGTGCTTTC AGGGAAGGAT GTTTAGCCAG TTTTCCTAGT ATTTGTTCCT 2340

TAAGATTTTT TGACCTGTGC TTAATAAGAC GGACGCGTGG GTCGACCC 2388

(2) INFORMATION FOR SEQ ID NO: 155:

(i) SEQUENCE CHARACTERISTICS:
(A) LENGTH: 642 base pairs
(B) TYPE: nucleic acid
(C) STRANDEDNESS: double
(D) TOPOLOGY: linear

(xi) SEQUENCE DESCRIPTION: SEQ ID NO: 155:

AAAACAGACC ATTTAAAAAC TCAGACAAGA TTATATTTAA TATATTAATT ACTAAAAAGG 60

CACAAGATTA CACTGAACAT ATTAGCTACT AAAAAGGCAC TGCTAAGACA TTCAAGCAAA 120

TAGCTATTAC ACACTACTGC AGATTTTACA GGTTTCTAAT TCTAACATAT GTTTGAAAAA 180

TCCGTGAGTA TTCCAAAATA TATTTAATAA TGGAATATCT GCATTAATAT ACCATCCATG 240

TGTTTTTACC ATTTGCCTTA ATATTGAATA TACTGTTTAC CTCACACTAA AAAGAAAACC 300

AGAAGCCTTA TTTGTGATTT TGGGAGTGGA AGCTTCCATT TTTGTGTCAA AAATGAATCC 360

TGATTCTTAT GGAAATCTCT GTTATTAAGA TATTTCAAGA TGAGACAACA CTGAAGATCA 420

AATTGTGTTT AGTATCACTA TCTTCTCTCC TCGTTTCTCT CTTACTCCTC ATCCTCCCAG 480

AATCTACCAG TTTATGGTAG AAAGATGGGA ACCTTATTTG AATGTGTTTT TTTTTTTCCA 540

TGATGTCCAA TTTTGTTGTG GGAAAGGATT TGGATAAAAT TTTTGTTTAA ATTTTGGTAG 600

ATTTTTATCT ATACAAATTT AAATAAAATT ATGTTTTGTA AG 642

(2) INFORMATION FOR SEQ ID NO: 156:

(i) SEQUENCE CHARACTERISTICS:
(A) LENGTH: 1251 base pairs
(B) TYPE: nucleic acid
(C) STRANDEDNESS: double
(D) TOPOLOGY: linear

(xi) SEQUENCE DESCRIPTION: SEQ ID NO: 156:

GCCGCTGCCC CTCCACGGAG TTGCTGATCA TCTGGGCTGT GATCCACAAA CCCGGTTCTT 60

TGTCCCTCCT AATATCAAAC AGTGGATTGC CTTGCTGCAG AGGGGAAACT GCACGTTTAA 120

AGAGAAAATA TCACGGGCCG CTTTCCACAA TGCAGTTGCT GTAGTCATCT ACAATAATAA 180

ATCCAAAGAG GAGCCAGTTA CCATGACTCA TCCAGGCACT GAGCATATTA TTGCTGTCAT 240

GATAACAGAA TTGAGGGGTA AGGATATTTT GAGTTATCTG GAGAAAAACA TCTCTGTACA 300

AATGACAATA GCTGTTGGAA CTCGAATGCC ACCGAAGAAC TTCAGCCGTG GCTCTCTAGT 360

CTTCGTGTCA ATATCCTTTA TTGTTTTGAT GATTATTTCT TCAGCATGGC TCATATTCTA 420

CTTCATTCAG AAGATCAGGT ACACAAATGC ACGCGACAGG AACCAGCGTC GTCTCGGAGA 480

TGCAGCCAAG AAAGCCATCA GTAAATTGAC AACCAGGACA GTAAAGAAGG GTGACAAGGA 540

AACTGACCCA GACTTTGATC ATTGTGCAGT CTGCATAGAG AGCTATAAGC AGAATGATGT 600

CGTCCGAATT CTCCCCTGCA AGCATGTTTT CCACAAATCC TGCGTGGATC CCTGGCTTAG 660

TGAACATTGT ACCTGTCCTA TGTGCAAACT TAATATATTG AAGGCCCTGG GAATTGTGCC 720

GAATTTGCCA TGTACTGATA ACGTAGCATT CGATATGGAA AGGCTCACCA GAACCCAAGC 780

TGTTAACCGA AGATCAGCCC TCGGCGACCT CGCCGGCGAC AACTCCCTTG GCCTTGAGCC 840

ACTTCGAACT TCGGGGATCT CACCTCTTCC TCAGGATGGG GAGCTCACTC CGAGAACAGG 900

AGAAATCAAC ATTGCAGTAA CAAAAGAATG GTTTATTATT GCCAGTTTTG GCCTCCTCAG 960

TGCCCTCACA CTCTGCTACA TGATCATCAG AGCCACAGCT AGCTTGAATG CTAATGAGGT 1020

AGAATGGTTT TGAAGAAGAA AAAACCTGCT TTCTGACTGA TTTTGCCTTG AAGGAAAAAA 1080

GAACCTATTT TTGTGCATCA TTTACCAATC ATGCCACACA AGCATTTATT TTTAGTACAT 1140

TTTATTTTTT CATAAAATTG CTAATGCCAA AGCTTTGTAT TAAAAGAAAT AAATAATAAA 1200

ATAAAAAAAA AAAAACCCCG GGGGGGGCCC GGTCCCCAAT TGGCCCTATG G 1251

(2) INFORMATION FOR SEQ ID NO: 157:

(i) SEQUENCE CHARACTERISTICS:
(A) LENGTH: 2127 base pairs
(B) TYPE: nucleic acid
(C) STRANDEDNESS: double
(D) TOPOLOGY: linear

(xi) SEQUENCE DESCRIPTION: SEQ ID NO: 157:

CCGGCGGGAG AGGGAAGCTG CAGCGAGAGG CGCGGATCTC AGCGCGGGAG CAGTGCTTCT 60

GCGGCAGGCC CCTGAGGGAG GGAGCTGTCA GCCAGGGAAA ACCGAGAACA CCATCACCAT 120

GACAACCAGT CACCAGCCTC AGGACAGATA CAAAGCTGTC TGGCTTATCT TCTTCATGCT 180

GGGTCTGGGA ACGCTGCTCC CGTGGAATTT TTTCATGACG GCCACTCAGT ATTTCACAAA 240

CCGCCTGGAC ATGTCCCAGA ATGTGTCCTT GGTCACTGCT GAACTGAGCA AGGACGCCCA 300

GGCGTCAGCG CNCCCTGCAG CACCCTTGCC TGAGCGGAAC TCTCTCAGTG CCATCTTCAA 360

CAATGTCATG ACCCTATGTG CCATGCTGCC CCTGCTGTTA TTCACCTACC TCAACTCCTT 420

CCTGCATCAG AGGATCCCCC AGTCCGTACG GATCCTGGGC AGCCTGGTGG CCATCCTGCT 480

GGTGTTTCTG ATCACTGCCA TCCTGGTGAA GGTGCAGCTG GATGCTCTGC CCTTCTTTGT 540

CATCACCATG ATCAAGATCG TGCTCATTAA TTCATTTGGT GCCATCCTGC AGGGCAGCCT 600

GTTTGGTCTG GCTGGCCTTC TGCCTGCCAG CTRACACGGC CCCCATCATG AGTGGCCAGG 660

GCCTAGCAGG CTTCTTTGCC TCCGTGGCCA TGATCTGCGC TATTGCCAGT GGCTCGGAGC 720

TATCAGAAAG TGCCTTCGGC TACTTTATCA CAGCCTGTGC TGTKATCATT TTGACCATCA 780

TCTGTTACCT GGGCCTGCCC CGCCTGGAAT TCTACCGCTA CTACCAGCAG CTCAAGCTTG 840

AAGGACCCGG GGAGCAGGAG ACCAAGTTGG ACCTCATTAG CAAAGGAGAG GAGCCAAGAG 900

CAGGCAAAGA GGAATCTGGA GTTTCAGTCT CCAACTCTCA GCCCACCAAT GAAAGCCACT 960

CTATCAAAGC CATCCTGAAA AATATCTCAG TCCTGGCTTT CTCTGTCTGC TTCATCTTCA 1020

CTATCACCAT TGGGATGTTT CCAGCCGTGA CTGTTGAGGT CAAGTCCAGC ATCGCAGGCA 1080

GCAGCACCTG GGAACGTTAC TTCATTCCTG TGTCCTGTTT CTTGACTTTC AATATCTTTG 1140

ACTGGTTGGG CCGGAGCCTC ACAGCTGTAT TCATGTGGCC TGGGAAGGAC AGCCGCTGGC 1200

TGCCAAGCTG GNTGCTGGCC CGGCTGGTGT TTGTGCCACT GCTGCTGCTG TGCAACATTA 1260

AGCCCCGCCG CTACCTGACT GTGGTCTTCG AGCACGATGC CTGGTTCATC TTCTTCATGG 1320

CTGCCTTTGC CTTCTCCAAC GGCTACCTCG CCAGCCTCTG CATGTGCTTC GGGCCCAAGA 1380

AAGTGAAGCC AGCTGAGGCA GAGACCGCAG AGCCATCATG GCCTTCTTCC TGTGTCTGGG 1440

TCTGGCACTG GGGGCTGTTT TCTCCTTCCT GTTCCGGGCA ATTGTGTGAC AAAGGATGGA 1500

CAGAAGGACT GCCTGCCTCC CTCCCTGTCT GCCTCCTGCC CCTTCCTTCT GCCAGGGGTG 1560

ATCCTGAGTG GTCTGGCGGT TTTTTCTTCT AACTGACTTC TGCTTTCCAC GGCGTGTGCT 1620

GGGCCCGGAT CTCCAGGCCC TGGGGAGGGA GCCTCTGGAC GGACAGTGGG GACATTGTGG 1680

GTTTGGGGCT CAGAGTCGAG GGACGGGGTG TAGCCTCGGC ATTTGCTTGA GTTTCTCCAC 1740

TCTTGGCTCT GACTGATCCC TGCTTGTGCA GGCCAGTGGA GGCTCTTGGG CTTGGAGAAC 1800

ACGTGTGTCT CTGTGTATGT GTCTGTGTGT CTGCGTCCGT GTCTGTCAGA CTGTCTGCCT 1860

GTCCTGGGGT GGCTAGGAGC TGGGTCTGAC CGTTGTATGG TTTGACCTGA TATACTCCAT 1920

TCTCCCCTGC GCCTCCTCCT CTGTGTTCTC TCCATGTCCC CCTCCCAACT CCCCATGCCC 1980

AGTTCTTACC CATCATGCAC CCTGTACAGT TGCCACGTTA CTGCCTTTTT TAAAAATATA 2040

TTTGACAGAA ACCAGGTGCC TTCAGAGGCT CTCTGATTTA AATAAACCTT TCTTGTTTTT 2100

TTCTCCATGG AAAAAAAAAA AAAAAAA 2127

(2) INFORMATION FOR SEQ ID NO: 158:

(i) SEQUENCE CHARACTERISTICS:
(A) LENGTH: 1625 base pairs
(B) TYPE: nucleic acid
(C) STRANDEDNESS: double
(D) TOPOLOGY: linear

(xi) SEQUENCE DESCRIPTION: SEQ ID NO: 158:

CAAAAGATCT ATAATCAGGA CATTGTTTAT GTAAGTTGGA CAANAAAAAT TCTTCCCCTT 60

TATGTCCACC CTTCCTATGA TTGCAAGACA AAATTTCCCT CCTTTACCTC ATCCCTATAA 120

CATGGGAGGC TGAGAAAAAT GAGGGGAGAT GGAACCAGAT ACAAGGAGAT CCAATAAGAG 180

AAGCTTATTT AAATATTGTG AAATAAAGGA AGAMCCAAAG CATTTTTTTA AGTGGGGAAT 240

CCTTTTGAAC AGTTATTATT TATCCATATT ATTAAYAACA TCTTTTCTGA CAAAATCCAT 300

CAGATGAAGT GTAAATGGAT AATCTTTTAA TGGATCTAAA CCTAGAAAGT TTCACTTACT 360

GTTCATGTCC GTGTTCCAGA ATTGTGAAAT GGTGTGTGGT TTTGCTTTCC AAGTTCTTCT 420

CTGCCTCCTC TTAATTCTCT AATTCCATGT CTTACAGAAG AATGAGAAAT TTCTTTCTTA 480

CTTGAGTATC ATGCTCTAAA AAACTTGGCT TCAGTCACAG AAACGCTGGC TCTCCTGTGC 540

TTATATTGAA GCCAACTGCC TTTAATTCTT GGGCCCTCTT ATATTTTTAA GGTGCAAAAT 600

TTGAAGTCTC AGTCACCAGA CACAGGTTCT ATACAATTAA TGATGAGCTG GAGAAGTAAT 660

ATGTAGCTAA TTTTTCAAAA GCATTGAATA TACTTTCCGG AAAGAAAACA GAAATTAAAT 720

ATTGCCACAT CTTGCCAGAA TCCCATCTGA CACCTTAACT TTGTCAGGTT TCCTACAACT 780

TGCTAATCAA GTTTTATACA TTCTAAATCT CCCCAGTTTC TTTGGGGCTG GAAGATGCAA 840

CTTCCATTTA ATAGAAACTT TGAAATCTTG GGGTAAGGGA GCAGTGGGGG GACTAGGGAG 900

AAGGATAAGA AATAGAATTA TTGAAAAGCC CCCACCAGGG ACCTTCCTGG CCAGAATATG 960

CAGAGTAATT CCTGCTGGCT TCACCTTTGA AAGTCCCTCG AAACTATGCA GATGAAACTG 1020

AGTCTGTTTT TGATATTGTC AGATGTATTC TACCTTGGAA GTCCCNACAC CTAAACTGGA 1080

ATTCTTGTAT TTACATCTCC TCCACTGTCC CCCACACCAC CCCTCAATTC CTGCTGCCCC 1140

TGCTAATGTT AAGCATTTTT CTCTTGTTAT CATCAGGTTC ACATTAAAAM CAGRTACTTA 1200

CAAACTGACT TGAAGCACAG ATACTTTTAC GAATGTGATA AAATATTTTC TTAAGAAAAG 1260

GAAAGAGGAT GTGGGTCAAA TAAAACACCG CATGGATGTT GATTGGTGAA TACTGGTGTA 1320

AGAAAAGGGA GCTCAGGAAT TTTTATTACT GTATTTGTAA ATGAGTTTGA AGGAATTTGT 1380

AAATGCCACT GGTACATTTT TAAGGTGACA CATTTGCTCC TTATAAAGTT ATTAAAAATT 1440

ACAGGGTAAG CTTAAATGAC GTTTGCCAGT AGTTTTACTT TATATAATCA ATATTGATAT 1500

TGTTGCTGAA CTATGTAACT TTATGATGCA TTTTTCAGTC CCTTTTCAGA GCAAATGCTT 1560

TTGCAATGGT AGTAATGTTT AGTTTAAATT GACTTAATAA ATTMTTACCT GAGCAAAAAA 1620

AAAAA 1625

(2) INFORMATION FOR SEQ ID NO: 159:

(i) SEQUENCE CHARACTERISTICS:
(A) LENGTH: 1687 base pairs
(B) TYPE: nucleic acid
(C) STRANDEDNESS: double
(D) TOPOLOGY: linear

(xi) SEQUENCE DESCRIPTION: SEQ ID NO: 159:

CGGGGTCACC AGTTATTAGA GGAAGTAACA CAAGGGGATA TGAGTGCAGC AGACACATTT 60

CTGTCCGATC TGCCAAGGGA TGATATCTAT GTGTCAGATG TTGAGGACGA CGGTGATGAC 120

ACATCTCTGG ATAGTGACCT GGATCCAGAG GAGCTGGCAG GAGTCAGGGG ACATCAGGGT 180

CTAAGGGACC AAAAGCGTAT GCGACTTACT GAAGTGCAAG ATGATAAAGA GGAGGAGGAG 240

GAGGAGAATC CACTGCTGGT ACCACTGGAG GAAAAGGCAG TACTGCAGGA AGAACAAGCC 300

AACCTGTGGT TCTCAAAGGG CAGCTTTGCT GGGNATCGAG GACGATGCCG ATGAAGGCCC 360

TGGAGATCAG TCAGGCCCAG CTGTTATTTG AGAACCGGYG GAAGGGACGG CAGCAGCAGC 420

AGAAGCAGCA GCTGCCACAG ACACCCCCTT CCTGTTTGAA GACTGAGATA ATGTCTCCCC 480

TGTACCAAGA TGAAGCCCCT AAGGNAACAG AGGCTTCTTC GGGGACAGAA GCTGCCACTG 540

GCCTTGAAGG GGAAGAAAAG GATGGCATCT CAGACAGTGA TAGCAGTACT AGCAKTGAGG 600

AAGAAGAGAG CTGGGAACCC TCCGTGGTAA GAAGCGAASC GTGGGCCTAA AGTCAGATGA 660

TGACGGGTTT GAGATAGTGC CTATTGAGGA CCCAGCGAAA CATCGGATAC TGGACCCCGA 720

AGGCCTTGCT CTAGGTGCTG TTATTGCCTC TTCCAAAAAG GCCAAGAGAG ACCTCATAGA 780

TAACTCCTTC AACCGGTACA CATTTAATGA GGATGAGGGG GAGCTTCCGG AGTGGTTTGT 840

GCAAGAGGAA AAGCAGCACC GGATACGACA GTTGCCTGTT GGTAAGAAGG AGGTGGAGCA 900

TTACCGGAAA CGCTGGCGGG AAATCAATGC ACGTCCCATC AAGAAGGTGG CTGAGGCTAA 960

GGCTAGAAAG AAAAGGAGGA TGCTGAAGAG GCTGGAGCAG ACCAGGAAGA AGGCAGAAGC 1020

CGTGGTGAAC ACAGTGGACA TCTNCAGAAC GAGAGAAAGT GGCACAGCTG CGAAGTCTCT 1080

ACAAGAAGGC TGGGCTTGGC AAGGAGAAAC GCCATGTCAC CTACGTTGTA GCCAAAAAAG 1140

GTGTGGGCCG CAAAGTGCGC CGGCCAGCTG GAGTCAGAGG TCATTTCAAG GTGGTGGACT 1200

CAAGGATGAA GAAGGACCAA AGAGCACAGC AACGTAAGGA ACAAAAGAAA AAACACAAAC 1260

GGAAGTAAGC AGAGCTGCCA GGCTCCCAGG AGAGCATGGG GACTAGGAGG AAGGGTGTGG 1320

CATGGCTCAG TCTGGCCCCC TTGATTACCG GCCTAGCCCC TGCTCACATC ACAGCTGTCT 1380

GAAGAACAGT GAGGTGGAGT GCCTAGAACT CCCGTGGTGG TCCTGAGCAG AGAGGAGGAT 1440

GTCCTCCTGC CTGCCTGAAG GTCTCCCATG AAAACACTGC TGAACTGTGT TGACACTCAT 1500

GACCCTTTTT TTAAACCGTT AAAGGGAAGT TCGGTGTTGG AGCGATACTC AATGTAGTCA 1560

GTCTACACCT GGACGTGTGG GCCACTTAAG CCCTCCCCAC CCCCATCCTA TTCCTRAATA 1620

AAACCAGGAT AATGGAARAA AAAAAAAAAA AAAAAAAAAG GGGGGGCCCN TAAAGGGNCC 1680

CANNTTT 1687

(2) INFORMATION FOR SEQ ID NO: 160:

(i) SEQUENCE CHARACTERISTICS:
(A) LENGTH: 1842 base pairs
(B) TYPE: nucleic acid
(C) STRANDEDNESS: double
(D) TOPOLOGY: linear

(xi) SEQUENCE DESCRIPTION: SEQ ID NO: 160:

GGATGACAGA TTGCGACANA GATTTGTGAC CCTTCCTGCT GAACTTCAGA GGGAGCTGAA 60

ANCAGCGTAT GATCAAAGAC AAAGGCAGGG CGAGAACAGC ACTCACCAGC AGTCAGCCAG 120

CGCATCTGTG CCCCGAGAAT CCTTTACTTC ATCTAAAGGC AGCAGTGAAA GAAAAGAAAA 180

GAAACAAGAA GAAAAAAACC ATTGGTTCAC CAAAAAGGAT TCAGAGTCCT TTGAATAACA 240

AGCTGCTTAA CAGTCCTGCA AAAACTCTGC CAGGGGCCTG TGGCAGTCCC CAGAAGTTAA 300

TTGATGGGTT TCTAAAACAT GAAGGACCTC CTGCAGAGAA ACCCCTGGAA GAACTCTCTG 360

CTTCTACTTC AGGTGTGCCA GGCCTTTCTA GTTTGCAGTC TGACCCAGCT GGCTGTGTGA 420

GACCTCCAGC ACCCAATCTA GCTGGAGCTG TTGAATTCAA TGATGTGAAG ACCTTGCTCA 480

GAGAATGGAT AACTACAATT TCAGATCCAA TGGAAGAAGA CATTCTCCAA GTTGTGAAAT 540

ACTGTACTGA TCTAATAGAA GAAAAAGATT TGGAAAAACT GGATCTAGTT ATAAAATACA 600

TGAAAAGGCT GATGCAGCAA TCGGTGGAAT CGGTTTGGAA TATGGCATTT GACTTTATTC 660

TTGACAATGT CCAGGTGGTT TTACAACAAA CTTATGGAAG CACATTAAAA GTTACATAAA 720

TATTACCAGA GAGCCTGATG CTCTCTGATA GCTGTGCCAT AAGTGCTTGT GAGGTATTTG 780

CAAAGTGCAT GATAGTAATG CTCGGAGTTT TTATAATTTT AAATTTCTTT TAAAGCAAGT 840

GTTTTGTACA TTTCTTTTCA AAAAGTGCCA AATTTGTCAG TATTGCATGT AAATAATTGT 900

GTTAATTATT TTACTGTAGC ATAGATTCTA TTTACAAAAT GTTTGTTTAT AAAGTTTTAT 960

GGATTTTTAC AGTGAAGTGT TTACAGTTGT TTAATAAAGA ACTGTATGTA TATTTGGTAC 1020

RGGCTCCTTT TKGTGAAYCC TTAAAAACTC AACTCTAGGA RGCAACTACT GTTTATTATA 1080

CTAAARGGCT GAAAAMCCTC CAGGCCAGAC TGCTAAGCTC TGAAATYCCT GAGAGGTCTC 1140

AGACCGGGAT TCTACTTGTT CCAAGAAAGG GTAAAGCTTC TAAACCATCT TATTCTTGTC 1200

TCCAAGCATG AACACAGGAG CATGTYAAGA AAATCTTTAC TACTTTCTYC CATGCGGAGA 1260

AATCTACATA TTTTGAATTA GAAACACCCT CACACCCACT TGAAGATTTT TTTCCTGGGA 1320

ACATTATGTC CCGTAGATCA GAGGTGGTGT TGTCTTTTTG CTTCTACTGG CCATTGAGAA 1380

ACTTTGATGA TAAAAAAGAA CGGTATAGAT TTTTCAAACG TATATAAAAT ATTTTTATGT 1440

TATATGTTAT GCCATAACTT TAAAATAAAA ATAGTTTAAA ATTCTATGCT AGTGGATATT 1500

TGGAACTTTT TCCTCAAACA AACACCCCAC ACTGACTTCA GCAAAACCCT AAAACTAGCT 1560

ACAGATTACT ACTACGAATG AATCATYAAG TTTTGTGTCT GCAACAATTT AGAAGCACTA 1620

AGCCCAAATA TCAGGAAATG TGTGTATGAT GGAATTTTCT AGGACAAAAC AGATCAAGAT 1680

TAAAACAGGA TCAAGGATTA ATGGTATAAA AATGGTCTAC TAAAACAGGA TCAAGGATTA 1740

AAACAGGATC AAGGATTAAT GGTATAAAAA TCTCTACTGG TTACCGGGTG GCNGGGCCAT 1800

ACAGGGTAGT GGTGGATGGA TAGTTTAGTT TGGNAAGGGT AA 1842

(2) INFORMATION FOR SEQ ID NO: 161:

(i) SEQUENCE CHARACTERISTICS:
(A) LENGTH: 770 base pairs
(B) TYPE: nucleic acid
(C) STRANDEDNESS: double
(D) TOPOLOGY: linear

(xi) SEQUENCE DESCRIPTION: SEQ ID NO: 161:

GGCACGAGCC CTATGCTGTT CTTGTGATAA TGAGTGAGTC TCACAAGATC TGGTGGTGTT 60

ATAGGCATCT GGCATTTCCC CTGCTGACGC TCATTCTCTA TCCTGCCACC CTGGGAAGAA 120

GTGTCTTCTG TCATGATTGT AAGTTTCCTG AGGCCTCCCC AGCTATGTAG AACTGTGAGC 180

CAATTAAACC TCTTTTCTCT ATAAATTATC CAGTCTTATA TATTTCTTCA TAGCAGTGTG 240

AGAACAGATA ATACCGTAAA TTGGTATCAC AGAGAGTGGG GTGTTGCTAT AAACACATCT 300

GAAAATGTTA AAGCAAATTT GGAACTGGGT AACAGGCAAA GGCTGGAACA GTTKGAAGAA 360

CAGTTAAGAA GAAGACAGGA AAATATGAGA AATCTTGAAA CTTCCTAGAG TCTTAAAGGT 420

CTCAGAAGAC ATGAAGATGT GGGAAGCTTT GGAACTTCCT AGAGACTTGT TTGAATGGCT 480

TTGACCAAAA TGCTGATAGT GATATGGACA ATGAAGTCCA GGCTGAGCTT ATCCAGACAG 540

ACATAAGAAG CTCGCTGGGA ACTTGAGTAA AGATCACTCT TGCTAGGCAA AGAGACTGGT 600

GGCCTTTTTT CCTCTGCCCT AGAGATCTGT GGAAATCTGA ACCTGAGAGA GATGATTTAG 660

GGTATCTGGC AGAAGAAATA TCTAAGCGGC AAAACCTTCM AGAGGAAGCA GAGCATAAAC 720

GTTTGAAAAA TTTGCAGCCT GACNATGGGA GACCAAAGTT AAACCCAATT 770

(2) INFORMATION FOR SEQ ID NO: 162:

(i) SEQUENCE CHARACTERISTICS:
(A) LENGTH: 519 base pairs
(B) TYPE: nucleic acid
(C) STRANDEDNESS: double
(D) TOPOLOGY: linear

(xi) SEQUENCE DESCRIPTION: SEQ ID NO: 162:

GAATTCGGCA CGAGCTGAGA GGCACAGGAG CAACAGCCAG TGCCCCCTGC AGAGGACCAC 60

TGGGGTCACA GACTTCARAC CTGATGACCT GGGCTCAGAT CCCAGCTCTG CACCTACCAG 120

CCGTGTGACA AGGTGTCCTC TCTGAGCCTC AGTCACACAC TGCCTTAACG GTTGGGCCTC 180

ATGGAGCTGT TTGTGAAGGT TAAATGGGAA GACATAAAGC ACTTAGCCCA GAGCCAAGGA 240

CATGCTGAAT AGGATAATGG TGGCCTCCTT TGGCGCTGTG CTGGTGCAGG TGTGCCGAGG 300

AAYTGGGCAG GGGTGACAGA TACCTCTTCT AACCTAGTTC CTTTCCAAGA ACCTAATTGG 360

TGTCTCTCCC TCCCCCAGGC AATTGGAAGG AGGAGGCTGG GCCCCAGCCC CAGAATACGG 420

GAGGTTTCTC ACCGTGGTAG GGAAATTGCT GGGTTGGGGG TGTGGGCAAC CACAGTGATC 480

GTCTCTCTGC AGGACGGATG AGGCTTTGCT GACAGAGGC 519

(2) INFORMATION FOR SEQ ID NO: 163:

(i) SEQUENCE CHARACTERISTICS:
(A) LENGTH: 753 base pairs
(B) TYPE: nucleic acid
(C) STRANDEDNESS: double
(D) TOPOLOGY: linear

(xi) SEQUENCE DESCRIPTION: SEQ ID NO: 163:

GGCACGAGCG GCACGAGCAG CCAGTTGCTG ACTGGCACAT GGCCTCCAGC GTCCCGGCTG 60

GTGGGCACAC TAGAGCCGGA GGGATCTTCT TAATTGGTAA ATTGGATCTT GAAGCTTCAC 120

TGTTTAAATC TTTTCAGTGG CTTCCCTTTG TACTTAGAAA AAAATGCAAC TTCTTCTGCT 180

GGGACTCATC CGCTCACAGC CTTCCCCTCC ACCCTCTCTC TGCCTCATGC TCTGCCCCTG 240

CCTGCCATGC CTCCGATACT CACCTTTTGT ACCCCAGCAC CCGTGCCCTC TGCCCCTCGA 300

TCTTTGCCTG GCTGGTTGCT CCTCACTCAG TGTTCAGGAC AAATGCTCCT GGCCCTACCC 360

CATCTAGCCA GTCTAGCCCG GTCTTCCCTG TCTTCCCTGT TTCATTCATG GCTCTTATTG 420

TTTGTTWACT TGTGTGCTGT TGACTTTTAA CTCTCTCAGT CCCCACTGGA ATGCAAGCGA 480

TCTCCCAAGC TCCTAGAATT GTTCCTGCCT CTTCACAGGC CCTTACGCTG TGTGTGCTCG 540

TGCCGAATTC GGCACGAGGG TATGTGCACT TGCTGGTATG TATGTAGGTG TTTGCTAACA 600

CATACGTGCA CACGCAGAAT GCTTCCAGGG GACTGCACAG CCTCTAGTTC GCAGCCCCCA 660

CCCCTCCCTT TGSCCCTGCA CTCTCCCCTC TCTGAGCTGC ATTCGCATGA AAGGGTGCAN 720

GGTTCCTGAN CCCGCNAGCG NCACCTCCTG GGA 753

(2) INFORMATION FOR SEQ ID NO: 164:

(i) SEQUENCE CHARACTERISTICS:
(A) LENGTH: 1400 base pairs
(B) TYPE: nucleic acid
(C) STRANDEDNESS: double

(D) TOPOLOGY: linear

(xi) SEQUENCE DESCRIPTION: SEQ ID NO: 164:

GGCACAGTTT ATTAATACCT ATTATGGGAA AGTCACTTTG GTTGGCATTG AAAATTACAT 60

CATCTTTAAA GCAGTATTTG TCCCCAGATG GACTCATCAC TAGCAAAGAC TAGGTTCATT 120

GGAAGGCATA GGGTGAGAGA ATGGGAAGAT GRAGTGGAGG CGGGTTGTTA AAGTGCTGTC 180

AGTGAGTGAT TTTGTCTACT TGAATAATGG TCCATGTTTG GGGGCATATT GTGTTTCATA 240

AGAAGTGAAA GGTATTTGCA AAGTAAGCTA CAAATGACCC ATAAATCTGT TAACAACAGT 300

CCTTAATATG CAAAGATGAA AAACAAGCAT TACTGCTACC CAAAGGGAAC TGGTGCTTGG 360

TGATGTGCAG ATGGGGCTGT TGGTTAAGAG AGCTATTACA GGTTTTCTCT CTTAGGTTTC 420

ATAGGAGGTA GTTACTGAGA TGAGATTGTT TTATCTTTTT GAATACAGAT CTCTTGTCTT 480

GAGTTAGTTC TGAGGATGGG AGTAATAAAG GAGTTTTTTG TTTTTTTGTT TGTTTGTTTG 540

TTTTGGCTCC TTAGTAATAC TCCTCTGACA TTTATTTCTA TTATTCTTCA AAGAAAGGAA 600

ACCAACTGAA ATGTTTGCTT TAACAAACAT TTTAATAAGT TCTCTGGGTT TTTTTTTCCC 660

CTTTTAAAAA AATTAGCATA TACCATAGCA ATAAAAGAAC TAATGTTAAC TATTGTATGC 720

TACAACTTAA GTGATTTTTC TAAAGAAGCA CAATGTCATT GRAAGTATTA TTGAAAAGGA 780

TCATAGTCAC ATTGAATTTG TGAAGGCCAA AGAAATTGAA GGGAGTGATA TTTTCATTTT 840

ATGATATTCA CATATTTAGT AAATTTTGTG TACAAGAATA CCAGGCAGAG TGTTTTACCC 900

ATGGAAACAG GTTTCAGATT ACTTTGTTTT TACTGTTAGA GTCTCAAGTT TAGAAATGCT 960

AACACTTAAA TCAGTTTTTT TCTCACTATA CTTGAAGATT GTTAATATTT TGATATCTTC 1020

CTAGCTTGAT GGAATTTAAA CATATCTTCA GATCTGTGAC AGTGACAGCC AATAGGACTG 1080

ATAATATTAG CTTCAAACCA ATAATATCCA GGGTTAAAAT AAAAATCATA GTGAAAGTAC 1140

GATTGTAAAA TTATGCTATA TTAACTTTTA AGTCTGTAAT AACTTGACAT CAAAATGTTA 1200

TGTAATTACC ATAAATAATG GCTAGCGAGA ACATCTTTGG AAATTCTCAA ATTACCTTTC 1260

TTACTACACT GTTTGCAGAA TGAATGTAGA AATGATCCTG TTAGCTTTCT GAATGTTCTG 1320

TGGTTGAATG TGTTTTTGCT TAAATAAAGC TTTTGGTATT TGTTTAAATW ACAAAAAAAA 1380

AAAAAAAAAA AAAAACTCGA 1400

(2) INFORMATION FOR SEQ ID NO: 165:

(i) SEQUENCE CHARACTERISTICS:
(A) LENGTH: 2153 base pairs
(B) TYPE: nucleic acid

(C) STRANDEDNESS: double
(D) TOPOLOGY: linear

(xi) SEQUENCE DESCRIPTION: SEQ ID NO: 165:

CAGGCCTCAG GGCCTCTGGT GGCTCTGGCC CAGACAGTAT TTGCAGTTCT TGTGCTATGG 60

GTGGGAGTCT TCTTCCTCAA GTTTCGGCAG CTGTGCTGTG NCTGGATGGG CTGCTCCTCC 120

CAGGGCTCAA GGGCTGTGGT CCGCTCAGGG TCTCATTTCC CCAGGCCAAG TTCAAGGCAG 180

CAGCCCTTTG TGAGGCGCTC TTGGCCCTGG GCTGGAGGGA GAACTTTAAG CTTTTTTGCT 240

CACAGGGACG TGGTATGGGC CCTGGGTGCA GGTGCCCACA TTCTGCTAAT GAGAGCTTTG 300

TCTGATCAGT CCTGGGTCCA TCAGTTTGTC CATGTGTCCG GCTGCCAGCC CGTCCCTTGG 360

GATCCTTCCC CTGGGGTGTA GCCTTGTTCA TTAGTATATA CTCATTCCTT CATGCTTTCC 420

TCAGCAGAAC ACTTCCACTT CTGAGGTGAG CTTTTGCCCC RTGCCCTTCC TCCACAGGTG 480

TTGCCTTTTT ATAAAGACCT GATAGCAGAA TAAATTGGTG TTTCCCTGTT GACCCAGCAC 540

CATTTCTGTG GGCCTAGAAT ATGGCCCTCA ACCCTTAGAG TGGGGCAGTG AGGGCTTGAG 600

GAGTGACCCT TCCTTTCTCA TGGTTTTAGT CATTTTGGCT GCCAGCCCTT AATGGCACAG 660

ATCTGCTGCT TCTAACAGAT GGCCAGGAGG TGACACCGAT TTCAGCCATT GCCAAGGTTA 720

GCACCCTCTC CTTTGAGCCT AGGGCCACAC TGTTCATTGT CACTTTAGGC AAGTGCCTGT 780

TTGGCTTTAA AGGTAAGCCT GCCAGCTGTG AGAAGCCTTG GTAACTGATG GACTCATTTC 840

CTGGTCCTTA AAGATGCAGC CTCTTAAGGG CTCCTTGATG GATGCCATCT CTCCTAGCCC 900

CCAGCCCTGG TGCCACTGGT GGGCAGGTTC CCATTCTTTG GGGCTGGGAG GGACAGCTTG 960

CCTGTTTCTG GTCACAAATT ACAGTCTTCT CTCCTGTACC ATTCTGTGGC TTCAGCATGG 1020

GGGCAGTAGC CTTTCATTAG TGTAGATAGT CATTCCCTGG TAGGGTGGAG GGTAAGACAT 1080

AGGGTCTGGA ACTGTTTGGG ACCTTTTGGG GATGTCCTGT GCCTCCCAGA TTCCTMGATT 1140

CTGGGAGGAG AGGCTGCCGC ATTCTGCTGC TCCTCACAGC GAGCAAAGCT GCACCCACTT 1200

ACATTCAGTA TTTTCCTGGC ACTACAAAGA GTGGGAAGGC CTGGGATTTG CTGCTGCTCC 1260

CTTAGAGCAG GGCCCCTYTT TTCAGCACTT TGGACACCTG GAGACCCAGC CCTGTTATTT 1320

AATGGTAGTG GGCAAGTGTG TGTGCATACT GTCTGCCACT GCTTTCTCCC TGCCCCATGC 1380

CAGAGAGCCC TGTCCCTGCC AGGCCCAGCC TTCTTAGCCC CAACTTGGGA ACAAAGTGCA 1440

ACATGGGATC ATGGGTTGGG GTGCTCAGGT GAGCCCTCTC TATAGTGCTT CCCTGGGCCA 1500

AGCTGACACC AGCCCCTGAG GGTGGGGTGG GACGGGTGGT GCTTAAAAGA GGAAGGGGAC 1560

CAGTGTAGCA ACTTGCCAGG GACCCCACCC CTCCCTCTCT GGGCCTGTGC AGTGAGCATG 1620

GGGATTCCCA TCAAGGGGCC TGGCACCTGT GCTAGTTACG TAGCCGCTGN TCACGCGCTC 1680

ACTCCTGACC ACATGCACGT TCCCTAGATG CAGACTGCTT TGAACTTTAA AGCTGTACAA 1740

TTTGGTTATG TTTGTGCTGA CTTAAAATAT ATTTTAATGA GGAAAAAATA ATGGAGAACC 1800

CTGGGAAGGA CCTGGTTCTT TTGCTTCTCG GGGAACTGTA AGCCCTCGCG TTCTGGGAAT 1860

CGCTCTCTGC TGCTCTTTCC TGGAAGCTAA GCCTGTCTCC ACCGCCCGAG GCCTGCGCCG 1920

GTGCTCCCGC CGCAGTTGCG TTTGCTTTGG ACCTTGCGTG CGGGGGAGGG GGTGCTCGGT 1980

CCGAGCCCGC TCCTTTCTGT ACACCTAGCG CTGCCCGCCC CGCTTGTGTC TGAGGTCGTG 2040

TATGTCAAAA ATAAAGCCGC TAGAAACGGA AAAAAAAAAA AAAAAAAAAA AAAAAAAAAA 2100

AAACTCGAGG GGGGGCCCGT ACCCAATTAA CCCNNTATGA TCTATAAAGC GTC 2153

(2) INFORMATION FOR SEQ ID NO: 166:

(i) SEQUENCE CHARACTERISTICS:
(A) LENGTH: 1251 base pairs
(B) TYPE: nucleic acid
(C) STRANDEDNESS: double
(D) TOPOLOGY: linear

(xi) SEQUENCE DESCRIPTION: SEQ ID NO: 166:

GCCCACGCGT CCGCCCACGC GTCCGGCGGT GCGGAGTATG GGGCGCTGAT GGCCATGGAG 60

GGCTACTGGC GCTTCCTGGC GCTGCTGGGG TCGGCACTGC TCGTCGGCTT CCTGTCGGTG 120

ATCTTCGCCC TCGTCTGGGT CCTCCACTAC CGAGAGGGGC TTGGCTGGGA TGGGAGCGCA 180

CTAGAGTTTA ACTGGCACCC AGTGCTCATG GTCACCGGCT TCGTCTTCAT CCAGGGCATC 240

GCCATCATCG TCTACAGACT GCCGTGGACC TGGAAATGCA GCAAGCTCCT GATGAAATCC 300

ATCCATGCAG GGTTAAATGC AGTTGCTGCC ATTCTTGCAA TTATCTCTGT GGTGGCCGTG 360

TTTGAGAACC ACAATGTTAA CAATATAGCC AATATGTACA GTCTGCACAG CTGGGTTGGA 420

CTGATAGCTG TCATATGCTA TTTGTTACAG CTTCTTTCAG GTTTTTCAGT CTTTCTGCTT 480

CCATGGGCTC CGCTTTCTCT CCGAGCATTT CTCATGCCCA TACATGTTTA TTCTGGAATT 540

GTCATCTTTG GAACAGTGAT TGCAACAGCA CTTATGGGAT TGACAGAGAA ACTGATTTTT 600

TCCCTGAGAG ATCCTGCATA CAGTACATTC CCGCCAGAAG GTGTTTTCGT AAATACGCTT 660

GGCCTTCTGA TCCTGGTGTT CGGGGCCCTC ATTTTTTGGA TAGTCACCAG ACCGCAATGG 720

AAACGTCCTA AGGAGCCAAA TTCTACCATT CTTCATCCAA ATGGAGGCAC TGAACAGGGA 780

GCAAGAGGTT CCATGCCAGC CTACTCTGGC AACAACATGG ACAAATCAGA TTCAGAGTTA 840

AACAGTGAAG TAGCAGCAAG GAAAAGAAAC TTAGCTCTGG ATGAGGCTGG GCAGAGATCT 900

ACCATGTAAA ATGTTGTAGA GATAGAGCCA TATAACGTCA CGTTTCAAAA CTAGCTCTAC 960

AGTTTTGCTT CTCCTATTAG CCATATGATA ATTGGGCTAT GTAGTATCAA TATTTACTTT 1020

AATCACAAAG GATGGTTTCT TGAAATAATT TGTATTGATT GAGGCCTATG AACTGACCTG 1080

AATTGGAAAG GATGTGATTA ATATAAATAA TAGCAGATAT AAATTGTGGT TATGTTACCT 1140

TTATCTTGTT GAGGACCACA ACATTAGCAC GGTGCCTTGT GCAKAATAGA TACTCAATAT 1200

GTGAATATGT GTCTACTAGT AGTTAATTGG ATAAACTGGC AGCATCCCTG A 1251

(2) INFORMATION FOR SEQ ID NO: 167:

(i) SEQUENCE CHARACTERISTICS:
(A) LENGTH: 882 base pairs
(B) TYPE: nucleic acid
(C) STRANDEDNESS: double
(D) TOPOLOGY: linear

(xi) SEQUENCE DESCRIPTION: SEQ ID NO: 167:

GACSMTCTAG AACTATGGTC CCCCGGGACT GCAGGAATTC GGCACAGCGG CTGCGGGCGC 60

GAGGTGAGGG GCGCGAGGTT CCCAGCAGGA TGCCCCGGCT CTGCAGGAAG CTGAAGTGAG 120

AGGCCCGGAG AGGGCCCAGC CCGCCCGGGG CAGGATGACC AAGGCCCGGC TGTTCCGGCT 180

GTGGCTGGTG CTGGGGTCGG TGTTCATGAT CCTGCTGATC ATCGTGTACT GGGACAGCGC 240

AGGCGCCGCG CACTTCTACT TGCACACGTC CTTCTCTAGG CCGCACACGG GGCCGCCGCT 300

GCCCACGCCC GGGCCGGACA GGGACAGGGA GCTCACGGCC GAYTCCGATG TCGACGAKTT 360

TCTGGACAAK TTTCTCAGTG CTGGCGTGAA GCAGAGTGAC YTTCCCAGAA AGGAGACGGA 420

GCAGCCGCCT GCGCCGGGGA GCATGGAGGA GAGCGTGAGA RGCTACGACT GGTCCCCGCG 480

CGAMGCCCGG CGCACCCAGA CCAGGGCCGG CAGCARGCGG ANCGGAGGAR CGTGCTGCGG 540

GGCTTCTGCG CCAAYTCCAG CCTGGCCTTC CCCACCAAGG AGCGCGCATT CRACGACATC 600

CCCAACTCGG AGCTGAGCCA CCTGATCGTG GACGACCGGC ACGGGGCCAT CTACTGCTAC 660

GTGCCCAAGG TGGCCTGCAC CAACTGGAAG CGCGTRATGA TCGTGCTGAG CGGAAGCTGT 720

GCACCGCGTG CGCCTACCGC GACCCGYTGC GNTCCCGCGC GAGCACGTGC ACAACGCCAG 780

CGCGCACTGA CTTCAACAAT TCTGGCGCCG CTACGGGAAG TCTCCCCCAC CTCATGAAGT 840

CAAGCTCAAG AATACACCAA TTCTTTCTGC GCGACCCTTC TG 882

(2) INFORMATION FOR SEQ ID NO: 168:

(i) SEQUENCE CHARACTERISTICS:
(A) LENGTH: 1208 base pairs
(B) TYPE: nucleic acid
(C) STRANDEDNESS: double
(D) TOPOLOGY: linear

(xi) SEQUENCE DESCRIPTION: SEQ ID NO: 168:

GGGAAACTCA AAAGGATGAT GGAATGGTTG ATGGAGCCAG AGCCTAGAAG TRAAGGGATA 60

CAGAGTGAAG ATAGAGGTAT TTACGTATAT TTWAATATTA GCTTTGGAAT TACGTAGGGA 120

TTCTTAAGAA AAGATCATGA CAGGACAGCC ACATTTGGTA AAATGTCAGG GCAGCCAGTG 180

CATGGTCCTC CTGGGGCTCC TCAGTTGACG GGTTTAAATC ATTTCCTGAT CCCCCTGCCC 240

TGGTTTGAGG AATGCATACA GTACGTGAAA TGCCTGTGGT ATGAGTTGCA ATGGGCAATC 300

AACCTGGGTA AATCCAAGAT TAATGATTAG TTCTAAAGAT CCAGTTGAAG TTCTAGAGTG 360

GGAATTTTCC GTCAAGCARC TCAGCACAGC TTTATGCCTG TTCCTCTAAT AACGATAGGT 420

AACAAATAGC TGTGTKTWCA CAGCTAGGAR GATAACCAAA TCTAGAGTTC TTGARTCTCA 480

TTTAATAAAT AAKTATTATG AGTACCAACT GCATATTTCA GGCACTGCAT TTGACTCTGT 540

TAAATACTGA TYCCTTAKGA CMSCCACWTC AGAWAACMTT AATCTGTCTG ATCAATAAAC 600

AGCTTGACTT AGAGRGGTAA AATAGCTTGC CACAGGTWAC CCAATTAGTA GGTAACAGCG 660

ACAGAATAAC AGTGCAGTTA AAATCTTAGA CTGGAGACTA ATTGCATAAG TTTGAATTTC 720

AGTTCTGCTA TGTAAATTTG GGTGAGTACC TTAATTYACC TGAGTCTCGG TCTTTATATC 780

TGTAGAATGG AGCTAATGAT ATTACTTAAT TTGCTTTATG TGAGATTAAA TGTACTAATA 840

TATGTAAATC ACTTACAACA GCATTTGACA TATTTGACAT ACTTAATATA TTTGCTACTA 900

ATACTATTAG CAACAGCATT CTGATTTTCC AAGTTGAAAT TCAGTGTTTT CTTTTTTACT 960

TTGCCATAAT TTACAATGTT GTGCTCTGTA AACCATAAAT TTCCCTGAGG TGTTGTCAGG 1020

TTAAAAAAAA ATCACTATGG CCCCCARNMA CTTGGAAAAT AGAAATGAGA CCAGCTTCAT 1080

CTATATTCTT TACTGCAAAT AACTTAGAAT TGTAATAGGC TAATATGTAC TGGGACTTCC 1140

AATTTGGGAA TATGACAAAA ATAATACTAT TTAGCTAAAA CATATACAGA ACTTATTTTT 1200

CCTCTGAA 1208

(2) INFORMATION FOR SEQ ID NO: 169:

(i) SEQUENCE CHARACTERISTICS:
(A) LENGTH: 1307 base pairs
(B) TYPE: nucleic acid
(C) STRANDEDNESS: double
(D) TOPOLOGY: linear

(xi) SEQUENCE DESCRIPTION: SEQ ID NO: 169:

GGCACGAGAG AAAAGAGGTT GAGAATGTTT TCTAGCAGGC AGAATGTGCA TACATGTTTT 60

CATGARTGTC CTTTGGGTGC TGTTTCTTTT AAATCCTCTG TGCACAGGGC TCTGGCCTTT 120

ARTAAACTGT TTTTCTGTCT TACGTCATGC TGACTGGGTG CTAGGGGCTG ATTACAAAGG 180

GGAAGAGTTG AACAGACATC AGGGGCCGAT GAAACCAAAG GACTAGGAGT CAGGAGAACA 240

AGTCAGGGAT TAGGAGACAG CGGTTTGGTT TATTGTTATC CAGCTGGAGG ACTCCTAGGG 300

GCAGCAGCAG GAGGAATACC AGGGCCACGG AGGGGCAGGA GTCTCACAGT GGAGGGCAGA 360

CTCTAACAGA TGCCAGCTGA ACGCTCGCTG GCCCTGGATG TCATACGAGT TGGGGACCAG 420

AAATCTGGGC TCAGAGAACC CGTCCAGGGA GATTTGAAGC CATGGGTTAT CTTCTAGAGT 480

TGATACTGAT AATATATTTT AATTTTTATT GATGTTTAAT ACCTTCTGAA ACAGGAGGGT 540

AAGATCAGAT GGGAAGCCCY TCTGTTGAAG GATCTTGGGA ACCTTGGTGG TTTTTTTTTT 600

TTGGTTTTTT TTTTTTTGAT CGAGCTGTGG ACATCCTTCT TAATTCGATT NTGAGGATTT 660

GTTTAACTAA AAAGTTCCCA AACACAGAAA GGGCCTCCCC ACCTGCTTTG GGGAGCTGTC 720

TGTSCTGGGA GTGCCAGGCA TCCSATGGGA CCCATCACTG CCAGTGTCTG TGCCTCCCAG 780

AGGTCAGCCC TGTGTCTGCC CTGGCTCTGT CTCCTCTGTG ACAGGGCAGA GCATTTCTGG 840

TCAGTTTCTC CATGGTGCCT CCCACCCCTT TGTAAAGTGG ATGGACATGA TGGAATTCAG 900

TTGTCTCACC CTGATAGCCT GGGTGTTGAT ATTCACTTTA CCCGCACTCA GACACAGGCG 960

ACCTTGAAGC AGTTCTCGGT GTGTAGAGTC CACGTGACAG TCCCCACAGC CTCCCCAGAT 1020

AGCTGTGTGC CTGTGCGCTA CTGCTGTGCC ATTTTCCCAA CTTNGGCGTT TCACTAAATG 1080

CAGCTGATCT CTCTCTCTGT GCACTCGTGA TCCATGTTGA ACAATACATG TAGGTTCTTT 1140

TTCCACGCAA TGTAAGAACA TGATATACTG TACGTTGGAA AGCATTTACC TTATTTATAT 1200

ACCTGAATGT TCCTACTACA CAAATAAACA TATATTAAAT WCTAAAAAAA AAAAAAAAAA 1260

CTGGAGGGGG GGCCCGGTAC CCAAATCGCC GGATAGTGAT CGTAAAC 1307

(2) INFORMATION FOR SEQ ID NO: 170:

(i) SEQUENCE CHARACTERISTICS:
(A) LENGTH: 1624 base pairs
(B) TYPE: nucleic acid
(C) STRANDEDNESS: double
(D) TOPOLOGY: linear

(xi) SEQUENCE DESCRIPTION: SEQ ID NO: 170:

GGCACGAGGT CGCCGCCGCG GCCGCCTGGA ATTGTGGGAG TTGTGTCTGC CACTCGGCTG 60

CCGGAGGCGA AGGTCCCTGA CTATGGCTCC CCAGAGCCTG CCTTCATCTA GGATGGCTCC 120

TCTGGGCATG CTGCTTGGGC TGCTGATGGC CGCCTGCTTC ACCTTCTGCC TCAGTCATCA 180

GAACCTGAAG GAGTTTGCCC TGACCAACCC AGAGAAGAGC AGCACCAAAG AAACRGAGAG 240

AAAAGAAACC AAAGCCGAGG AGGAGCTGGA TGCCGAAGTC CTGGAGGTGT TCCACCCGAC 300

GCATGAGTGG CAGGCCCTTC AGCCAGGGCA GGCTGTCCCT GCAGGATCCC ACGTACGGCT 360

GAATCTTCAG ACTGGGGAAA GAGAGGCAAA ACTCCAATAT GAGGACAAGT TCCGAAATAA 420

TTTGAAAGGC AAAAGGCTGG ATATCAACAC CAACACCTAC ACATCTCAGG ATCTCAAGAG 480

TGCACTGGCA AAATTCAAGG AGGGGGCAGA GATGGAGAGT TCAAAGGAAG ACAAGGCAAG 540

GCAGGCTGAG GTAAAGCGGC TCTTCCGCCC CATTGAGGAA CTGAAGAAAG ACTTTGATGA 600

GCTGAATGTT GTCATTGAGA CTGACATGCA GATCATGGTA CGGCTGATCA ACAAGTTCAA 660

TAGTTCCAGC TCCAGTTTGG AAGAGAAGAT TGCTGCGCTC TTTGATCTTG AATATTATGT 720

CCATCAGATG GACAATGCGC AGGACCTGCT TTCCTTTGGT GGTCTTCAAG TGGTGATCAA 780

TGGGCTGAAC AGCACAGAGC CCCTCGTGAA GGAGTATGCT GCGTTTGTGC TGGGCGCTGC 840

CTTTTCCAGC AACCCCAAGG TCCAGGTGGA GGCCATCGAA GGGGGAGCCC TGCAGAAGCT 900

GCTGGTCATC CTGGCCACGG AGCAGCCGCT CACTGCAAAG AAGAAGGTCC TGTTTGCACT 960

GTGCTCCCTG CTGCGCCACT TCCCCTATGC CCAGCGGCAG TTCCTGAAGC TCGGGGGGCT 1020

GCAGGTCCTG AGGACCCTGG TGCAGGAGAA GGGCACGGAG GTGCTCGCCG TGCGCGTGGT 1080

CACACTGCTC TACGACCTGG TCACGGAGAA GATGTTCGCC GAGGAGGAGG CTGAGCTGAC 1140

CCAGGAGATG TCCCCAGAGA AGCTGCAGCA GTATCGCCAG GTACACCTCC TGCCAGGCCT 1200

GTGGGAACAG GGCTGGTGCG AGATCACGGC CCACCTCCTG GCGCTGCCCG AGCATGATGC 1260

CCGTGAGAAG GTGCTGCAGA CACTGGGCGT CCTCCTGACC ACCTGCCGGG ACCGCTACCG 1320

TCAGGACCCC CAGCTCGGCA GGACACTGGC CAGCCTGCAG GCTGAGTACC AGGTGCTGGC 1380

CAGCCTGGAG CTGCAGGATG GTGAGGACGA GGGCTACTTC CAGGAGCTGC TGGGCTCTGT 1440

CAACAGCTTG CTGAAGGAGC TGAGATGAGG CCCCACACCA GGACTGGACT GGGATGCCGC 1500

TAGTGAGGCT GAGGGGTGCC AGCGTGGGTG GGCTTCTCAG GCAGGAGGAC ATCTTGGCAG 1560

TGCTGGCTTG GCCATTAAAT GGAAACCTGA AGGCCAAAAA AAAAAAAAAA AAAAAAAAAA 1620

AAAA 1624

(2) INFORMATION FOR SEQ ID NO: 171:

(i) SEQUENCE CHARACTERISTICS:
(A) LENGTH: 2003 base pairs
(B) TYPE: nucleic acid
(C) STRANDEDNESS: double
(D) TOPOLOGY: linear

(xi) SEQUENCE DESCRIPTION: SEQ ID NO: 171:

GGCACGAGCC AGCTTGCAGG AGGAATCGGT GAGGTCCTGT CCTGAGGCTG CTGTCCGGGG 60

CCGGTGGCTG CCCTCAAGGT CCCTTCCCTA GCTGCTGCGG TTGCCATTGC TTCTTGCCTG 120

TTCTGGCATC AGGCACCTGG ATTGAGTTGC ACAGCTTTGC TTTATCCGGG CTTGTGTGCA 180

GGGCCCGGCT GGGCTCCCCA TCTGCACATC CTGAGGACAG AAAAAGCTGG GTCTTGCTGT 240

GCCCTCCCAG GCTTAGTGTT CCCTCCCTCA AAGACTGACA GCCATCGTTC TGCACGGGGC 300

TTTCTGCATG TGACGCCAGC TAAGCATAGT AAGAAGTCCA GCCTAGGAAG GGAAGGATTT 360

TGGAGGTAGG TGGCTTTGGT GACACACTCA CTTCTTTCTC AGCCTCCAGG ACACTATGGC 420

CTGTTTTAAG AGACATCTTA TTTTTCTAAA GGTGAATTCT CAGATGATAG GTGAACCTGA 480

GTTGCAGATA TACCAACTTC TGCTTGTATT TCTTAAATGA CAAAGATTAC CTAGCTAAGA 540

AACTTCCTAG GGAACTAGGG AACCTATGTG TTCCCTCAGT GTGGTTTCCT GAAGCCAGTG 600

ATATGGGGGT TAGGATAGGA AGAACTTTCT CGGTAATGAT AAGGAGAATC TCTTGTTTCC 660

TCCCACCTGT GTTGTAAAGA TAAACTGACG ATATACAGGC ACATTATGTA AACATACACA 720

CGCAATGAAA CCGAAGCTTG GCGGCCTGGG CGTGGTCTTG CAAAATGCTT CCAAAGCCAC 780

CTTAGCCTGT TCTATTCAGC GGCAACCCCA AAGCACCTGT TAAGACTCCT GACCCCCAAG 840

TGGCATGCAG CCCCCATGCC CACCGGGACC TGGTCAGCAC AGATCTTGAT GACTTCCCTT 900

TCTAGGGCAG ACTGGGAGGG TATCCAGGAA TCGGCCCCTG CCCCACGGGC GTTTTCATGC 960

TGTACAGTGA CCTAAAGTTG GTAAGATGTC ATAATGGACC AGTCCATGTG ATTTCAGTAT 1020

ATACAACTCC ACCAGACCCC TCCAACCCAT ATAACACCCC ACCCCTGTTC GCTTCCTGTA 1080

TGGTGATATC ATATGTAACA TTTACTCCTG TTTCTGCTGA TTGTTTTTTT AATGTTTTGG 1140

TTTGTTTTTG ACATCAGCTG TAATCATTCC TGTGCTGTGT TTTTTATTAC CCTTGGTAGG 1200

TATTAGACTT GCACTTTTTT AAAAAAAGGT TTCTGCATCG TGGAAGCATT TGACCCAGAG 1260

TGGAACGCGT GGCCTATGCA GGTGGATTCC TTCAGGTCTT TCCTTTGGTT CTTTGAGCAT 1320

CTTTGCTTTC ATTCGTCTCC CGTCTTTGGT TCTCCAGTTC AAATTATTGC AAAGTAAAGG 1380

ATCTTTGAGT AGGTTCGGTC TGAAAGGTGT GGCCTTTATA TTTGATCCAC ACACGTTGGT 1440

CTTTTAACCG TGCTGAGCAG AAAACAAAAC AGGTTAAGAA GAGCCGGGTG GCAGCTGACA 1500

GAGGAAGCCG CTCAAATACC TTCACAATAA ATAGTGGCAA TATATATATA GTTTAAGAAG 1560

GCTCTCCATT TGGCATCGTT TAATTTATAT GTTATGTTCT AAGCACAGCT CTCTTCTCCT 1620

ATTTTCATCC TGCAAGCAAC TCAAAATATT TAAAATAAAG TTTACATTGT AGTTATTTTC 1680

AAATCTTTGC TTGATAAGTA TTAAGAAATA TTGGACTTGC TGCCGTAATT TAAAGCTCTG 1740

TTGATTTTGT TTCCGTTTGG ATTTTTGGGG GAGGGGAGCA CTGTGTTTAT GCTGGAATAT 1800

GAAGTCTGAG ACCTTCCGGT GCTGGGAACA CACAAGAGTT GTTGAAAGTT GACAAGCAGA 1860

CTGCGCATGT CTCTGATGCT TTGTATCATT CTTGAGCAAT CGCTCGGTCC GTGGACAATA 1920

AACAGTATTA TCAAAGAGAA AAAAAAAAAA AAAAAACTCG NGGGGGGGCC CGGTACCCAA 1980

TTCGCCCTAT AGTGAGCCNA TTC 2003

(2) INFORMATION FOR SEQ ID NO: 172:

(i) SEQUENCE CHARACTERISTICS:
(A) LENGTH: 786 base pairs
(B) TYPE: nucleic acid
(C) STRANDEDNESS: double
(D) TOPOLOGY: linear

(xi) SEQUENCE DESCRIPTION: SEQ ID NO: 172:

GGCACAGCGG CACGAGAAGA CTTTGGTGTT TAAGAGATTA ATGTGTTAGC CAGAACAACT 60

CATTTCTCTA CCMGTGTGTA GTCCATTTAT CTTTAAAGAT TTTCTATTGG AATAATTTTG 120

AAATTACTTT CTTAGTTTTC TTCATTAAAA ACTAAGAAAA TGCTTTGTTT ATTATGAATT 180

GCTATTTCTC TTGATTATTA TTCTTGGAGA AAGTCTATCA GACGTAATTC TTCTGATTTG 240

CTTCTAGGCT AGAGGAAAAT GTGAAAGATG ACAAATGAAA ATTTCAAAGG TTGTCAGTAG 300

TATGACTTCT TTTATCGTTT GTCATTATCA CAAATATATC AACATAGGAC TTTTAAAAGA 360

TATTTTGTAC ATATTGGGCC TTAGTAGGAT TTTGCATGAA TTTTTTTTTT CTTTTATGCC 420

CAGAGAGAAA GAGCAAAGAA ATAACCAAGG GTGATGTACT CGTATTGAAG GTTTACCAAA 480

TAAGGACTGC TTTTATTATG AACTATAGTC TATATTCTAA GTAAATCAAT TTTTCTATTA 540

TGTGTTTTTT GTTCCTGCAG GCAAGATCTC TGAACTTTAT GCAGAGGGTT CTTTTAAAAA 600

AACAAAGTTG AATTTTTTTA TTTCTTGGAA TATTTTTTTT CATTGATTTC TCCCAAGTAG 660

AGCAGATTCA AATCTCCTTT GTACCCTATG TCTTTTTTGT TTTGCTATTA GCTCAGTATT 720

CCGTTTCTAC ATTTTCCTTT CCTAGAACCA GTCAATAAAT GACAAAAAAA AAAAAAAAAA 780

ACTCGA 786

(2) INFORMATION FOR SEQ ID NO: 173:

(i) SEQUENCE CHARACTERISTICS:
(A) LENGTH: 1758 base pairs
(B) TYPE: nucleic acid
(C) STRANDEDNESS: double
(D) TOPOLOGY: linear

(xi) SEQUENCE DESCRIPTION: SEQ ID NO: 173:

GGGACGAGCC CTGCCCACCT CCTGCAGCCT CCTGCGCCCC GCCGAGCTGG CGGATGGAGC 60

TGCGCACGGG GAGCGTGGGC AGCCAGGCGG TGGCGCGGAG GATGGATGGG GACAGCCGAG 120

ATGGCGGCGG CGGCAAGGAC GCCACCGGGT CGGAGGACTA CGAGAACCTG CCGACTAGCG 180

CCTCCGTGTC CACCCACATG ACAGCAGGAG CGATGGCCGG GATCCTGGAG CACTCGGTCA 240

TGTACCCGGT GGACTCGGTG AAGACACGAA TGCAGAGTTT GAGTCCAGAT CCCAAAGCCC 300

AGTACACAAG TATCTACGGA GCCCTCAAGA AAATCATGCG GACCGAAGCT TCTGGAGGCC 360

CTTGCGAGGC GTCAACGTCA TGATCATGGG TGCAGGGCCR GCCCATGCCA TGTATTTTGC 420

CTGCTATGAA AACATGAAAA GGACTTTAAA TGACGTTTTC CACCACCAAG GAAACAGCCA 480

CCTAGCCAAC GGTATTTTGA AAGCGTTTGT CTGGAGTTAG AAAGTTCTCT TCTTCAACAC 540

GTCCCTCCCC AGGGTGTTCC TCCCTGTGAC CCAGCCGCCT CGACTTCGGC CCGCTTGCTC 600

ACGAATAAAG AACTCAGAGT TGTGTGTGCA ATGCACACCC AGACACACGC ACGCACACAC 660

ACGCGCGCGC ACACACATGC TTTTTTCTGT TCCCCTCCGC TTTCTGAAGC CTGGGGAGAA 720

ATCAGTGACA GAGGTGTTTT GGTTTTATTG TTATGTGGGT TTTCTTTTGT ATTTTTTTTG 780

TTTGTTTTGT TTTTAAACAT TCAAAAGCAA TTAATGATCA GACATAGGAG AAACCCTGAA 840

TAGAAACAAA ACTTTTGAAT GCTGGATTCA AAAAAAAAAA AAAGTTATCT GGACAGCTTC 900

TTTGAGACTA TTTAAAAACT GGTACAACAG GTCTCTACAA CGCCAAGATC TAACTAAGCT 960

TTAAAAGGTC AAGAAGTTTT ATGGCTGACA AAGGACTCGC GCAACGCAGA AGGCCTTTCC 1020

CACCTTAAGC TTCCGGGGAT CTGGGAATTT TACCCCCATT CTCTTCTGTT TGTCTGAGTC 1080

TCATCTCTCT GCAAGCAAGG GCTGAAATCA TTTTGTTTGG TTGTTTTGAG GGAGAGAGGC 1140

GGGGTGGGGG GGTGCAAATC TGCCAGCAGC TCTTACGTAA GGCATGTTTT ATTGGGGAGG 1200

GCTGAGCTTT TATTTTCTCC TCTCCAGTGG GGTTGGCTTT TATTGTTTCT TGTTTGGGTT 1260

TGGAATGGAA ATATGGATAG CAGCATAAAG TACTTTTATT TTGACAAAAT TCATTTTTTT 1320

CAACAATGGA GACATAGATT TGACCCACAA TAACTTCTCC CCCTCTCTTT TTACTCTGCT 1380

CAAAAAGCAT CTCTCCTCCC ATTACCCAAC CTTGGTCATA AGTGTGCCTG GCTGGTTTGC 1440

AGATATTTGT TCTGCTTTGT AAAAATTGGC CATTAGTGCA TTTATTGAGA TGATCTCTAA 1500

AGAGCTATGC CCTGACCTAC CCCTGATTCT ATGACATTGG GGCCCTTCTT TTGCTGAAAC 1560

TGCCTTACGT AATGGTTTTA CTCCTTGAAA GAGATTTGAC GGAATCCATT TTATGCCAAG 1620

TGCTGCCCTG CACTGTTTCT GCAATATGTG GTGTATGCTG TGGTGATCTT GCTGGGAATG 1680

ATTATAAGTG TGTGTGTGGT GGGGGAGTGG GTATTACATG CATTGCTGAA GAGTCAAAAA 1740

AAAAAAAAAA AAACTCGA 1758

(2) INFORMATION FOR SEQ ID NO: 174:

(i) SEQUENCE CHARACTERISTICS:
(A) LENGTH: 888 base pairs
(B) TYPE: nucleic acid
(C) STRANDEDNESS: double
(D) TOPOLOGY: linear

(xi) SEQUENCE DESCRIPTION: SEQ ID NO: 174:

CTGTTAGAAT GCCCAGTTTA CCTGGATGGC AACCCAACAG TGCTCCTGCC CACCTGCCCC 60

TCAATCCTCC TAGAATTCAG CCCCCAATTG CCCAGTTACC AATAAAAACT TGTACACCAG 120

CCCCAGGGAC AGTCTCAAAT GCAAATCCAC AGAGTGASMC ACCACCTCGG GTAGAATTTG 180

ATGACAACAA TCCCTTTAGT GAAAGTTTTC AAGAACGGGA ACGTAAGGAA CGTTTACGAG 240

AACAGCAAGA GAGACAACGG ATCCAACTCA TGCAGGAGGT AGATAGACAA AGAGCTTTGC 300

AGCAGAGGAT GGAAATGGAG CAGCATGGTA TGGTGGGCTC TGAGATAAGT AGTAGTAGGA 360

CATCTGTGTC CCAGATTCCC TTCTACAGTT CCGACTTACC TTGTGATTTT ATGCAACCTC 420

TAGGACCCCT TCAGCAGTCT CCACAACACC AACAGCAAAT GGGGCAGGTT TTACAGCAGC 480

AGAATATACA ACAAGGATCA ATTAATTCAC CCTCCACCCA AACTTTCATG CAGACTAATG 540

AGCGAGGCAG GTAGGCCCTC CTTCATTTGT TCCTGATTCA CCATCAATCC CTGTTGGAAG 600

CCCAAATTTT TCTTCTGTGA AGCAGGGACA TGGAAATCTT TCTGGGACCA GCTTCCAGCA 660

GTCCCCAGTG AGGCCTTCTT TTACACCTGC TTTACCAGCA GCACCTCCAG TAGCTAATAG 720

CAGTCTCCCA TGTGGCCAAG ATTCTACTAT AACCCATGGA CACAGTTATC CGGGATCAAC 780

CCAATCGCTC ATTCAGTTGT ATTCTGATAT AATCCCAGAG GAAAAAGGGN AAAAAAAARA 840

AMAARAAARA ARAAAGGAGA TGATGATGCA GAATTCCACC AAGGCTCC 888

(2) INFORMATION FOR SEQ ID NO: 175:

(i) SEQUENCE CHARACTERISTICS:

(A) LENGTH: 2379 base pairs
(B) TYPE: nucleic acid
(C) STRANDEDNESS: double
(D) TOPOLOGY: linear

(xi) SEQUENCE DESCRIPTION: SEQ ID NO: 175:

GGCAGAGCTA GTGTGGACTC CATCCCCCTG GAGTGGGATC ACGNCTATGA CCTCAGTCGG 60

GACCTGGAGT CTGCAATGTC CAGAGCTCTG CCCTCTGAGG ATGAAGAAGG TCAGGATGAC 120

AAAGATTTCT ACCTCCGGGG AGCTGTTGSC TTATCAGGGG ACCACAGTGC CCTAGAGTCA 180

CAGATCCGAC AACTGGGCAA AGCCTGGATG ATAGCCGCTT TCAGATACAG CAAACCGAAA 240

ATATCATTCG CAGCAAAACT CCCACGGGGC CGGAGCTAGA CACCAGCTAC AAAGGCTACA 300

TGAAACTGCT GGGCGAATGC AGTAGCAGTA TAGACTCCGT GAAGAGACTG GAGCACAAAC 360

TGAAGGAGGA AGAGGAGAGC CTTCCTGGCT TTGTTAACCT GCATAGTACC GAAACCCAAA 420

CGGCTGGTGT GATTGACCGA TGGGAGCTTC TCCAGGCCCA GGCATTGAGC AAGGAGTTGA 480

GGATGAAGCA GAACCTCCAG AAGTGGCAGC AGTTTAACTC AGACTTGAAC AGCATCTGGG 540

CCTGGCTGGG GGACACGGAG GAGGAGTTGG AACAGCTCCA GCGTCTGGAA CTCAGCACTG 600

ACATCCAGAC CATCGAGCTC CAGATCAAAA AGCTCAAGGA GCTCCAGAAA GCTGTGGACC 660

ACCGCAAAGC CATCATCCTC TCCATCAATC TCTGCAGCCC TGAGTTCACC CAGGCTGACA 720

GCAAGGAGAG CCGGGACCTG CAGGATCGCT TGTSGCAGAT GAATGGGCGC TGGGACCGAG 780

TGTGCTCTCT GCTGGAGGAG TGGCGGGGCC TGCTGCAGGA TGCCCTGATG CAGTGCCAGG 840

GTTTCCATGA AATGAGCCAT GGTTTGCTTC TTATGCTGGA GAACATTGAC AGAAGGAAAA 900

ATGAAATTGT CCCTATTGAT TCTAACCTTG ATGCAGAGAT ACTTCAGGAC CATCACAAAC 960

AGCTTATGCA AATAAAGCAT GAGCTGTTGG AATCCCAACT CAGAGTAGCC TCTTTGCAAG 1020

ACATGTCTTG CCAACTACTG GTGAATGCTG AAGGAACAGA CTGTTTAGAA GCCAAAGAAA 1080

AAGTCCATGT TATTGGAAAT CGGCTCAAAC TTCTCTTGAA GGAGGTCAGT CGTCATATCA 1140

AGGAACTGGA GAAGTTATTA GACGTGTCAA GTAGTCAGCA GGATTTGTCT TCCTGGTCTT 1200

CTGCTGATGA ACTGGACACC TCAGGGTCTG TGAGTCCCAY ATCAGGAAGG AGCACCCCAA 1260

ACAGACAGAA AACGCCACGA GGCAAGTGTA GTCTCTCACA GCCTGGACCC TCTGTCAGCA 1320

GTCCACATAG CAGGTCCACA AAAGGTGGCT CCGATTCCTC CCTTTCTGAG CCARGGCCAG 1380

GTCGGTCCGG CCGCGGCTTC CTGTTCAGAG TCCTCCGAGC AGCTCTTCCC CTTCAGCTTC 1440

TCCTGCTCCT CCTCATCGGG CTTGCCTGCC TTGTACCAAT GTCAGAGGAA GACTACAGCT 1500

GTGCCCTCTC CAACAACTTT GCCCGGTCAT TCCACCCCAT GCTCAGATAC ACGAATGGCC 1560

CTCCTCCACT CTGAACTAAG CAGATGCCAT CTGCAGAAGT GCTGGTAGCA TAAGGAGGAT 1620

CGGGTCATAA GCAATCCCAA ACTACCAACA AGAGGACCTT GATCTTGGCG AAAGCCMTCG 1680

GTGTGGCAGC TTTAGCCTCC TCCAGATCAC ATGTGTGCAA ATTATGGCTT CAGAGGTGGA 1740

AGATAAACAG TGACGGGGGA ACAAACAGAC AACAAGAAGG TTTGGAAGAA ATCTGGTTTG 1800

AGACTCTGAA CCTTAGCACT AAGGAGATTG AGTAAGGACC TCCAAAGTTC CCCGGACTCA 1860

TGAATTCTGG GCCCTTGGCC NATTCTGTGC ACAGCCAAGG ACTTCAGTAG ACCATCTGGG 1920

CAGCTTTCCC ATGGTGCTGC TCCAACCATC AGATAAATGA CCCTCCCAAG CACCATGTCA 1980

GTGTCGTACA ATCTACCAAC CAACCAGTGC TGAAGAGATT TTAGAACCTT GTAACATACA 2040

ATTTTTAAGA GCTTATATGG CAGCTTCCTT TTTACCTTGT TTTCCTTTGG GGCATGATGT 2100

TTTAACCTTT GCTTTAGAAG CACAAGCTGT AAATCTAAAA GGCACTTTTT TTTAGAGGTA 2160

TAAAGAAAAA CTAGATGTAA TAAATAAGAT CATGGAAGGC TTTATGTGAA AAAAGTTGAA 2220

TGTTATAGTA AAAAAAAAAG ATATTTATGT ATGTACAGTT TGCTAAAGCC AAGTTTTGTT 2280

TGTATTGATT TCTTTGCATT TATTATAGAT ATTATAAAAT AAAAAAAAAA AAAAAAAAAC 2340

TCGAGGGGGG GCCCGGTACC CAATTCGCCC TATAGTGAG 2379

(2) INFORMATION FOR SEQ ID NO: 176:

(i) SEQUENCE CHARACTERISTICS:
(A) LENGTH: 1348 base pairs
(B) TYPE: nucleic acid
(C) STRANDEDNESS: double
(D) TOPOLOGY: linear

(xi) SEQUENCE DESCRIPTION: SEQ ID NO: 176:

GCGCCTTCAC GATGCCGGCG GTCAGTGGTC CAGGTCCCTT ATTCTGCCTT CTCCTCCTGC 60

TCCTGGACCC CCACAGCCCT GAGACGGGGT GTCCTCCTCT ACGCAGGTTT GAGTACAAGC 120

TCAGCTTCAA AGGCCCAAGG CTGGCATTGC CTGGGGCTGG AATACCCTTC TGGAGCCATC 180

ATGGAGGTGA GGGGCAGGGG TGGGGACCGC TATGCCCAGG GTCCCTCAAA GTGCTGGAGG 240

GGCTGTRACT TGGTGGGGAG TGGGTCTGTC ACAGCCATCC TCTGTCCAGG GTGGGGCAAG 300

GCCTGGGACA GTGCCAGGCA CCCCAGGACC CCTTCCAGGC TTGTCTCCTG CTCCACCGCC 360

TCAACACCCC CCACCCCTGC CCAAGCTGTT TCTCCTCTGC CTCTCTNNTT CCCTGCCCCA 420

GGACTTCTCT CTTCTCCTCT GCCTCTCCTT GGACCCCTGC CCTTCCTCTA CCTCTGACCT 480

GTGAACACAC AGACACATGC TCACACACTA AGTCCCARGC ACACMSAAAG GCAATGTGGA 540

CCAGCACAAA CCTCCACTCT CCCGGCTCCA TCCCARCGGG CCTGTGGCTG GCCATGAAAA 600

CTGGGGGCTA CCTGGAGGGA AGCATCCTCA TCCCAGGTGA GTGGGCACCA GCCCTTCCCT 660

GTATGTGTGT TGTGGGTGGA AGCAGGCATG AGAGCATCTT AGCCCATAGG TTTGTATTCA 720

GGGACTTCCA AACCCAGACC TACAAAGAGT GTGTCTTCTA CCAGATCTTG TTCAAAAAAG 780

GGTTTGTGAT GATGGAACTA CACGATAGAG GGAGTGAGCA AGAACAATGA GGATTAGAGT 840

GGAGCGTGAA ATAGTCTAGG AGCATGGCTT CCAAAACATA TGCTGTGAGG TCTGTCCACC 900

TGAGAGTTGG GCCATGGATT TAATTCTGAG CCTCTTAGCA GGCAAAGCAA AGACAGAAAG 960

CAGATCGGCT GTGGATTTCT GTCTATAAAA TGTGAGTTCT TGGCCGGGTG CGGTGGCTCA 1020

CGCCTGTAAT CCCGGCGCTT TGGGAGGCCA GGGCGGATGG GTCGCGAGGT CAGGAGGTTG 1080

GAAACCATCC TGGCCGGAAT GGTGAAGCCC TGACTCTACT AGAAGTGCAA AGATTGGCTG 1140

GGTGTGGTGG CGTGCGCCTG TGGTCCCAGC TTCTCGGGAG GCTGAGGCGG GAGAGTTGCT 1200

TGGGCCTGGG AGGCCGAGGT TGCGGTGAGC TGAGATCCTG CCATTGCACT TCAGCCTGGG 1260

CACAGAGCCA GACTCTGGCT CAAAAAAAAA AAAAAAAAAA ACTCGAGGGG GGCCCGTACC 1320

CAATTCGCCG NATATGATCG TAAACAAT 1348

(2) INFORMATION FOR SEQ ID NO: 177:

(i) SEQUENCE CHARACTERISTICS:
(A) LENGTH: 1502 base pairs
(B) TYPE: nucleic acid
(C) STRANDEDNESS: double
(D) TOPOLOGY: linear

(xi) SEQUENCE DESCRIPTION: SEQ ID NO: 177:

CTCAAAATAA ATAAATAAAT AAAAATTTGT ATTCCATTGA TTTGGGTAGA CACCAGGAAT 60

GTGCATTTCT AACAAGCTTT CCAGGCGATC CTATAGTAAG TCATCTGTGG ACTACTTTAA 120

GAAACTCTTC TATAGAGAAT GGAGTTGGAT TAATAATAGG TGATTTTTTA CACTGGACTG 180

ATTCACAAGA ACCTAAACAG TAGTCCATGA AGCTGCTCAT CTGTGGTAAC TATTTGGCCC 240

CGTCTCACTC TGAAAGCAGC AGGAGATGTT GTTTACTTTG TTTCTATCCC CTTTGTCTGG 300

AGATTAATTT TGGAATGAAA GTTTTTCTCT CTATGCCATT CCTGGTTCTT TTCCAAAGCC 360

TCATACAAGA GGATTAGGTC ACAATGCATG CATTACCTTT TAAAAGAATG CGATATTGAT 420

ACCGATGCTT ACTTTTTTTT TTTTTNACTA CTTGTTTTAT TCCTTCCAGN AAAGTATAGC 480

[…]

ATTGCACTAA ATGTGTCTTC CTTGGTATAG TGGAGGATTT GAGGATTGGA ATATAGAGTA 960

GAGTGCTTGC TTAAGCCTGG GAGCCCATCT TTATAGCTAT TTGATGTAAG AAAAGAGACA 1020

TGGNCCATTT CTAAACTATA TAAGGTGAGT GTGTCTATTC CCAGCAGATA TAAAGGAAAA 1080

AGGAAACTTT TTTGATTCCC ACCTTCCCAG CCTCACCTAG CCATCTTCCA GCCTCAAATA 1140

TAGAGATGTT AGTGCAAGGT CCTGGGCTCT AGGTGATCAT TTCATAAGTC CTTTACAGAT 1200

AAAGAAAAAG TAGTGTTTGT ATGTTTGTTT TTAAGTAACC CCAAAACAAA TTTATATTGT 1260

ATTCAGCAAA ATTGGAATTC AGGTGTTTAA TTTTAGAACA TGAAGTGCCT GCTGTTTTAA 1320

GCATTGACTT GTATAAAAAG AATTGCATGT CTCCAGTAAG CTTATGGGTT TTCTCATTTT 1380

TAGGTATATG GCTTTTAATC ATGTAAAGTG AAACATTAGT TTTCTTGCAT TTTATTACAG 1440

GTTCTTTGTT GCAATAAAGA TGCTGCTGAA ATTAATTGAA AAAAAAAAAA AAAAAAACTC 1500

GA 1502

(2) INFORMATION FOR SEQ ID NO: 178:

(i) SEQUENCE CHARACTERISTICS:
(A) LENGTH: 1637 base pairs
(B) TYPE: nucleic acid
(C) STRANDEDNESS: double
(D) TOPOLOGY: linear

(xi) SEQUENCE DESCRIPTION: SEQ ID NO: 178:

ATTTTCTAGC CCACAAGGAC TGAAGTTCAG ATCCAAAAGT TCACTTGCTA ATTATCTTCA 60

CAAAAATGGA GAGACTTCTC TTAAGCCAGA AGATTTTGAT TTTACTGTAC TTTCTAAAAG 120

GGGTATCAAG TCAAGATATA AAGACTGCAG CATGGCAGCC CTGACATCCC ATCTACAAAA 180

CCAAAGTAAC AATTCAAACT GGAACCTCAG GACCCGAAGC AAGTGCAAAA AGGATGTGTT 240

TATGCCGCCA AGTAGTAGTT CAGAGTTGCA GGAGAGCAGA GGACTCTCTA ACTTTACTTC 300

CACTCATTTG CTTTTGAAAG AAGATGAGGG TGTTGATGAT GTTAACTTCA GAAAGGTTAG 360

AAAGCCCAAA GGAAAGGTGA CTATTTTGAA AGGAATCCCA ATTAAGAAAA CTAAAAAAGG 420

ATGTAGGAAG AGCTGTTCAG GTTTTGTTCM AAGTGATAGC AAAAGAGAAT CTGTGTGTAA 480

TAAAGCAGAT GCTGAAAGTG AACCTGTTGC ACAAAAAAGT CAGCTTGATA GAACTGTCTG 540

CATTTCTGAT GCTGGAGCAT GTGGTGAGAC CCTCAGTGTG ACCAGTGAAG AAAACAGCCT 600

TGTAAAAAAA AAAGAAAGAT CATTGAGTTC AGGATCAAAT TTTTGTTCTG AACAAAAAAC 660

TTCTGGCATC ATAAACAAAT TTTGTTCAGC CAAAGACTCA GAACACAACG AGAAGTATGA 720

GGATACCTTT TTAGAATCTG AAGAAATCGG AACAAAAGTA GAAGTTGTGG AAAGGAAAGA 780

ACATTTGCAT ACTGACATTT TAAAACGTGG CTCTGAAATG GACAACAACT GCTCACCAAC 840

CAGGAAAGAC TTCACTGAAG ATACCATCCC ACGGAACACA GATAGAAAGA AGGAAAACAA 900

GCCTGTATTT TTCCAGCAAA TATAACAAAG AAGCTCTTAG CCCCCCACGA CGTAAAGCCT 960

TTAAGAAATG GACACCTCCT CGGTCACCTT TTAATCTCGT TCAAGAAACA CTTTTTCATG 1020

ATCCATGGAA GCTTCTCATC GCTACTATAT TTCTCAATCG GACCTCAGGC AAAATGGCAA 1080

TACCTGTGCT TTGGAAGTTT CTGGAGAAGT ATCCTTCAGC TGAGGTAGCA AGAACCGCAG 1140

ACTGGAGAGA TGTGTCAGAA CTTCTTAAAC CTCTTGGTCT CTACGATCTT CGGGCAAAAA 1200

CCATTGTCAA GTTCTCAGAT GAATACCTGA CAAAGCAGTG GAAGTATCCA ATTGAGCTTC 1260

ATGGGATTGG TGCACCCTGA AGACCACAAA TTAAATAAAT ATCATGACTG GCTTTGGGAA 1320

AATCATGAAA AATTAAGTCT ATCTTAAACT CTGCAGCTTT CAAGCTCATC TGTTATGCAT 1380

AGCTTTGCAC TTCAAAAAAG CTTAATTAAG TACAACCAAC CACCTTTCCA GCCATAGAGA 1440

TTTTAATTAG CCCAACTAGA AGCCTAGTGT GTGTGCTTTC TTAATGTGTG TGCCAATGGT 1500

GGATCTTTGC TACTGAATGT GTTTGAACAT GTTTTGAGAT TTTTTTAAAA TAAATTATTA 1560

TTTGACAACA ATCCAAAAAA AAAAAAAAAA AAAAAAAAAA AAAAAAAAAA AAAAAAAAAA 1620

AAAAAAAAAA AAAAAAA 1637

(2) INFORMATION FOR SEQ ID NO: 179:

(i) SEQUENCE CHARACTERISTICS:
(A) LENGTH: 2911 base pairs
(B) TYPE: nucleic acid
(C) STRANDEDNESS: double
(D) TOPOLOGY: linear

(xi) SEQUENCE DESCRIPTION: SEQ ID NO: 179:

GGTGGTTTTT GTTCTGCAAT AGGCGGCTTA GAGGGAGGGG CTTTTTCGCC TATACCTACT 60

GTAGCTTCTC CACGTATGGA CCCTAAAGGC TACTGCTGCT ACTACGGGGC TAGACAGTTA 120

CTGTCTCAGC TCTAGGATGT GCGTTCTTCC ACTAGAAGCT CTTCTGAGGG AGGTAATTAA 180

AAAACAGTGG AATGGAAAAA CAGTGCTGTA GTCATCCTGT AATATGCTCC TTGTCAACAA 240

TGTATACATT CCTGCTAGGT GCCATATTCA TTGCTTTAAG CTCAAGTCGC ATCTTACTAG 300

TGAAGTATTC TGCCAATGAA GAAAACAAGT ATGATTATCT TCCAACTACT GTGAATGTGT 360

GCTCAGAACT GGTGAAGCTA GTTTTCTGTG TGCTTGTGTC ATTCTGTGTT ATAAAGAAAG 420

ATCATCAAAG TAGAAATTTG AAATATGCTT CCTGGAAGGA ATTCTCTGAT TTCATGAAGT 480

GGTCCATTCC TGCCTTTCTT TATTTCCTGG ATAACTTGAT TGTCTTCTAT GTCCTGTCCT 540

ATCTTCAACC AGCCATGGCT GTTATCTTCT CAAATTTTAG CATTATAACA ACAGCTCTTC 600

TATTCAGGAT AGTGCTGAAG ANGCGTCTAA ACTGGATCCA GTGGGCTTCC CTCCTGACTT 660

TATTTTTGTC TATTGTGGCC TTGACTGCCG GGACTAAAAC TTTACAGCAC AACTTGGCAG 720

GACGTGGATT TCATCACGAT GCCTTTTTCA GCCCTTCCAA TTCCTGCCTT CTTTTCAGAA 780

ATGAGTGTCC CAGAAAAGAC AATTGTACAG CAAAGGAATG GACTTTTCCT GAAGCTAAAT 840

GGAACACCAC AGCCAGAGTT TTCAGTCACA TCCGTCTTGG CATGGGCCAT GTTCTTATTA 900

TAGTCCAGTG TTTTATTTCT TCAATGGCTA ATATCTATAA TGAAAAGATA CTGAAGGAAG 960

GGAACCAGCT CACTGAARGC ATCTTCATAC AGAACAGCAA ACTCTATTTC TTTGGCATTC 1020

TGTTTAATGG GCTGACTCTG GGCCTTCAGA GGAGTAACCG TGATCAGATT AAGAACTGTG 1080

GATTTTTTTA TGGCCACAGT GCATTTTCAG TAGCCCTTAT TTTTGTAACT GCATTCCAGG 1140

GCCTTTCAGT GGCTTTCATT CTGAAGTTCC TGGATAACAT GTTCCATGTC TTGATGGCCC 1200

AGGTTACCAC TGTCATTATC ACAACAGTGT CTGTCCTGGT CTTTGACTTC AGGCCCTCCC 1260

TGGAATTTTT CTTGGAAGCC CCATCAGTCC TTCTCTCTAT ATTTATTTAT AATGCCAGCA 1320

AGCCTCAAGT TCCGGAATAC GCACCTAGGC AAGAAAGGAT CCGAGATCTA AGTGGCAATC 1380

TTTGGGAGCG TTCCAGTGGG GATGGAGAAG AACTAGAAAG ACTTACCAAA CCCAAGAGTG 1440

ATGAGTCAGA TGAAGATACT TTCTAACTGG TACCCACATA GTTTGCAGCT CTCTTGAACC 1500

TTATTTTCAC ATTTTCAGTG TTTGTAATAT TTATCTTTTC ACTTTGATAA ACCAGAAATG 1560

TTTCTAAATC CTAATATTCT TTGCATATAT CTAGCTACTC CCTAAATGGT TCCATCCAAG 1620

GCTTAGAGTA CCCAAAGGCT AAGAAATTCT AAAGAACTGA TACAGGAGTA ACAATATGAA 1680

GAATTCATTA ATATCTCAGT ACTTGATAAA TCAGAAAGTT ATATGTGCAG ATTATTTTCC 1740

TTGGCCTTCA AGCTTCCAAA AAACTTGTAA TAATCATGTT AGCTATAGCT TGTATATACA 1800

CATAGAGATC AATTTGCCAA ATATTCACAA TCATGTAGTT CTAGTTTACA TGCCAAAGTC 1860

TTCCCTTTTT AACATTATAA AAGCTAGGTT GTCTCTTGAA TTTTGAGGCC CTAGAGATAG 1920

TCATTTTGCA AGTAAAGAGC AACGGGACCC TTTCTAAAAA CGTTGGTTGA AGGACCTAAA 1980

TACCTGGCCA TACCATAGAT TTGGGATGAT GTAGTCTGTG CTAAATATTT TGCTGAAGAA 2040

GCAGTTTCTC AGACACAACA TCTCAGAATT TTAATTTTTA GAAATTCATG GGAAATTGGA 2100

TTTTTGTAAT AATCTTTTGA TGTTTTAAAC ATTGGTTCCC TAGTCACCAT AGTTACCACT 2160

TGTATTTTAA GTCATTTAAA CAAGCCACGG TGGGGCTTTT TTCTCCTCAG TTTGAGGAGA 2220

AAAATCTTGA TGTCATTACT CCTGAATTAT TACATTTTGG AGAATAAGAG GGCATTTTAT 2280

TTTATTAGTT ACTAATTCAA GCTGTGACTA TTGTATATCT TTCCAAGAGT TGAAATGCTG 2340

GCTTCAGAAT CATACCAGAT TGTCAGTGAA GCTGATGCCT AGGAACTTTT AAAGGGATCC 2400

TTTCAAAAGG ATCACTTAGC AAACACATGT TGACTTTTAA CTGATGTATG AATATTAATA 2460

CTCTAAAAAT AGAAAGACCA GTAATATATA AGTCACTTTA CAGTGCTACT TCACACTTAA 2520

AAGTGCATGG TATTTTTCAT GGTATTTTGC ATGCAGCCAG TTAACTCTCG TAGATAGAGA 2580

AGTCAGGTGA TAGATGATAT TAAAAATTAG CAAACAAAAG TGACTTGCTC AGGGTCATGC 2640

AGCTGGGTGA TGATAGAAGA GTGGGCTTTA ACTGGCAGGC CTGTATGTTT ACAGACTACC 2700

ATACTGTAAA TATGAGCTTT ATGGTGTCAT TCTCAGAAAC TTATACATTT CTGCTCTCCT 2760

TTCTCCTAAG TTTCATGCAG ATGAATATAA GGTAATATAC TATTATATAA TTCATTTGTG 2820

ATATCCACAA TAATATGACT GGCAAGAATT GGTGGAAATT TGTAATTAAA ATAATTATTA 2880

AACCTAAAAA AAAAAAAAAA AAAAACTCGA G 2911

(2) INFORMATION FOR SEQ ID NO: 180:

(i) SEQUENCE CHARACTERISTICS:
(A) LENGTH: 519 base pairs
(B) TYPE: nucleic acid
(C) STRANDEDNESS: double
(D) TOPOLOGY: linear

(xi) SEQUENCE DESCRIPTION: SEQ ID NO: 180:

GGCACGAGCC CCAGGCCAGC CAGGGCCAGG CCTACTTTGG CCACCCTTAA ATTAGAATGT 60

GGGGTCAGGG GTCACAGAAA AGCCATTTCT CTGACCTAGT GTTTGGCGTC CGGGAACTCT 120

GTGCCCAACC TTCAGACCCT GGCAGTCCTC ACTGAGGCCA TTGGCCCAGA GCCCGCCATC 180

CCCCGARACC CCCGGGAGCC GCCTGTTGCC ACGTCCACAC CTGCCACACC CTCTGCCGGG 240

CCCCAGCCCC TCCCAACCGG GACCGTGCTG GTCCCTGGGG GTCCTGCCCC ACCTTGCCTT 300

GGGGAGGCAT GGGCCCTCCT CCTCCCACCC TGCCGGCCGT CACTCACCTC TTGCTTCTGG 360

TCCCCCAGGC CTAGCCCTTG GAAGGAGACA GGAGTCTAGG GAGGCTGAAG CCCACTCCCG 420

GGGAGGCCCG TGCTCCTCCA GCCCCAGGGA CAGCAAGGAA AAGAGAAGAG AGCAGAGCAT 480

TTCATGGCTC TAATAAAAAA AAAAAAAAAA AAAACTCGA 519

(2) INFORMATION FOR SEQ ID NO: 181:

(i) SEQUENCE CHARACTERISTICS:
(A) LENGTH: 968 base pairs
(B) TYPE: nucleic acid
(C) STRANDEDNESS: double
(D) TOPOLOGY: linear

(xi) SEQUENCE DESCRIPTION: SEQ ID NO: 181:

TCCCCTTGGG GCCGGAAAAA GCGGGGTTGG CCTGNCCATT GGTTNTCCAT GCCGCCCGCC 60

CATGCCCCAG TACTAGCCTG CAGTCCCAAT GTAGCCCCTC CCTCYTCCMA GAGCCCYTCM 120

AACCGCCCCG STCANTTGTG ATTTCAGGAG GATTTGATGA AGATGTTAAA GCGAAAGTGG 180

AGAACCTTCT CGGGATTTCC AGCCTGGAAA AAACGGACCC TGTTAGGCAA GCACCCTGCA 240

GCCCTCCCTG TCCCCTTCTT CCCCTCCCCT TCYCCCGCCC GTGGAGACAG CTGTTYTCAG 300

CAGGGCTCTC CGCAGGGAGG GGGCCGGCTC CTTCCCTGGC AGCAACATCC TTGCCCTTGT 360

CACACAAGTC AGCCTCCATC TGCGCAGCTC TGTGGATGCG CTGCTGGAGG GCAACAGGTA 420

TGTCACTGGC TGGTTCAGCC CCTACCACCG CCAGCGGAAG CTCATCCACC CGGTCATGGT 480

TCAGCACATC CAGCCCGCAG CGCTCAGCCT CCTGGCACAG TGGAGCACCC TCGTGCAGGA 540

GCTGGAGGCT GCCCTGCAGC TGGCTTTCTA CCCGGATGCC GTGGAGGAGT GGCTGGAGGA 600

AAACGTGCAC CCCAGCCTGC AGCGGCTGCA ARCTCTGCTG CAGGACCTCA GCGAGGTGTC 660

TGCCCCCCCG CTGCCACCCA CCAGCCCTGG CAGGGACGTT GCTCAGGACC CCTGAGGGGA 720

GAGCTCATGC CAGGGGGCTC CTGCTGGAGG CTGGGGGGGC TCTGCWYTKY CWWWTGGCCT 780

GGGCAATACG GCCCACGTGG GCGTCGTGCC CTCTGGCCCA GCAGTGTCTT GCCCACACTC 840

AGTTCCTGAG GGCCCTGGGC AGCCCCTGGG GGAGAGACTA GAAAACACAG AAGGAAGCAG 900

CACAGGGAGA CCCGCTTTGT GATCTGCATG TGTGACACTG ATTCTTTGGA AATAAAGAGT 960

GGAAGCTG 968

(2) INFORMATION FOR SEQ ID NO: 182:

(i) SEQUENCE CHARACTERISTICS:
(A) LENGTH: 1128 base pairs
(B) TYPE: nucleic acid
(C) STRANDEDNESS: double
(D) TOPOLOGY: linear

(xi) SEQUENCE DESCRIPTION: SEQ ID NO: 182:

TGTAAAAGTT ATCAGTAATC CTAATTCTTT TCCTGGGTTT TCCTTTTGTC ACTTATTAAT 60

CAGTTTTTGA AAGGACGAAT GAATTTAGAG ATGTACTCTG GAGCAGTATC ATGTTAAACC 120

AGGGGTATAT TAGAAAAATC ATCCTCATAA TCATTCTGGG AAGTTTTTCC TCCCCAAAAA 180

AAGCCATCCT GATGGGTTTT CAAAACCAGA AAAAAGCTCT TAATGAGGAA CAGACCACTG 240

GAGTACCCAT GAGCATCTCA GGAAAACTGA GACCCTCGAG AAGCCTTGAT TTCGTGCAAC 300

CCCCAAGGTT TCAGAGCCAG CAGCCCAGTG CTGTGGTTGA CAGACGTGGT TTTKTGGRGA 360

AAGCAGCCAG AGGCCAGGAA TTTTCAGAGT CGTGAGTCAC GRTYTCCCAC CCAAGATTAG 420

AGCAMAGATT AGCCATACTG AGATTTGGTA AAATCATTCT GTCTAAGCAA TGGAGGTGTG 480

TGCAMACGTG CAGTGCCTGT TCACAGGGGA TGCAGGCAGA TCSYGGGTTT AGGATGGGGR 540

AGGCCACCGC ACCCCCYTTC AYTGCTCTGC ACCTGCTCCC TCACGTGGAC ACTGTCCACA 600

ACTGTGGCTC TCACAGGACA GTTGCCCAAG GAGCTCATAT CTTATTGGAG ATAGGGGGTC 660

GTACAGGTGA CATTCATGAG CAGTGTGAGC CGGGTGACAT GGGGGTGTCA ACCCAGCATC 720

TGTCCAGGAG CTCCTCCTGC AGCGGCTCTG GCAGGTGGCC TGAGGCTCCT TTTTGAGAGA 780

GAACTGTTTG GCCTTCCTGT CTCCTCTCCT CTGATCTGTT CTTTCTTGGA ACACCACCCA 840

AGAACGTCAC CTCCTCCATC AGATTGTGAG CTCCTGGAGG GCAGGAGCTG TGTCCTTCTA 900

TTCATCTTCC TATCCCCAGA ACCTTGCACA GATCCTGGAA TGTGGTAGGT GCTCAGTAAA 960

TGTGTGTTGA ATAAATGAAT GAATGAATGA ACAAATGAAT GAATTTGCTT ACTTCAAGGC 1020

AAAAGAACCA TGAAACTGTA TTTTGAGTTT CTATGTTATA GCAGTCAGCA AATCCTATTA 1080

AATACTTTGT GTTTCCAAGC AAAAAAAAAA AAAAAAAAAA AAACTCGA 1128

(2) INFORMATION FOR SEQ ID NO: 183:

(i) SEQUENCE CHARACTERISTICS:
(A) LENGTH: 2276 base pairs
(B) TYPE: nucleic acid
(C) STRANDEDNESS: double
(D) TOPOLOGY: linear

(xi) SEQUENCE DESCRIPTION: SEQ ID NO: 183:

CCGCGGCGTC TGACCTCATG GCGTAGAGCC TAGCAACAGC GCAGGCTCCC AGCCGAGTCC 60

GTTATGGCCG CTGCCGTCCC GAAGAGGATG AGGGGGCCAG CACAAGCGAA ACTGCTGCCC 120

GGGTCGGCCA TCCAAGCCCT TGTGGGGTTG GCGCGGCCGC TGGTCTTGGC GCTCCTGCTT 180

GTGTCCGCCG CTCTATCCAG TGTTGTATCA CGGACTGATT CACCGAGCCC AACCGTACTC 240

AACTCACATA TTTCTACCCC AAATGTGAAT GCTTTAACAC ATGAAAACCA AACCAAACCT 300

TCTATTTCCC AAATCAGCAC CACCCTCCCT CCCACGACGA GTACCAAGAA AAGTGGAGGA 360

GCATCTGTGG TCCCTCATCC CTCGCCTACT CCTCTGTCTC AAGAGGAAGC TGATAACAAT 420

GAAGATCCTA GTATAGAGGA GGAGGATCTT CTCATGCTGA ACAGTTCTCC ATCCACAGCC 480

AAAGACACTC TAGACAATGG CGATTATGGA GAACCAGACT ATGACTGGAC CACGGGCCCC 540

AGGGACGACG ACGAGTCTGA TGACACCTTG GAAGAAAACA GGGGTTACAT GGAAATTGAA 600

CAGTCAGTGA AATCTTTTAA GATGCCATCC TCAAATATAG AAGAGGAAGA CAGCCATTTC 660

TTTTTTCATC TTATTATTTT TGCTTTTTGC ATTGCTGTTG TTTACATTAC ATATCACAAC 720

AAAAGGAAGA TTTTTCTTCT GGTTCAAAGC AGGAAATGGC GTGATGGCCT TTGTTCCAAA 780

ACAGTGGAAT ACCATCGCCT AGATCAGAAT GTTAATGAGG CAATGCCTTC TTTGAAGATT 840

ACCAATGATT ATATTTTTTA AAGCACTGTG ATTTGAATTT GCTTATGTAA TTTTATTTGC 900

TTGACTTTTT ATATGATATT GTGCAAATGT TTGCCATAGG CAATTGGTAC TTAAATGAGA 960

GGTGAGTCTC TCTTTTGCCT TGGTGCTTTG GAAATTAAAT GTCACAAACG AGTATATAAT 1020

TTTTTATCTG TACTTTTAGA GCTGAGTTTA ATCAGGTGTC CAAAATGTGA GTTAAACATT 1080

ACCTTATATT TACACTGTTA GTTTTTATTG TTTTAGATTT ATTATGCTTC TTCTGGAAGT 1140

ATTAGTGATG CTACTTTTAA AAGATCCCAA ACTTGTAACT AAATTCTGAC ATATCTGTTA 1200

CTGCTGACTC ACATTCATTC TCCGCCATTC AAATACTATT TTTTATCCAC ATTTTTTTTT 1260

GTTCCCAAAC TGTAATGTAC AAGGATATGT GTGATAATGC TTTGGATTTG AGTAATATTT 1320

TTTTTTCTTC CAAGAAAACT GCTTTGGATA TTTTTAGATA ATTTAAACAT AATTTAGGAT 1380

AATGATATTG CTCAATCTGA CCACAATTTT AGGTAAAACA TTAAATGTGT CAGAAATCTT 1440

GGCAACAGAG ACTCTGCAGC TTGCAGTGGA CATAGATAAA ATGTTACAGA GATACTATTT 1500

TTTTGGTTGG AATTACTATA TTAAATTTAG AAGCAGAAAC TGGTAAAATG TTAAATACAT 1560

GTACAATTGC TTTTAGTTAG CAATTGATTG TAGCATGGGT TCCTCCAAGG TTTCAAGCAA 1620

TGGGCAGAGT TTAAAATTAT ATCAGATTCG TTTACTTCGT TTATTATTTT ACAGTAAATT 1680

TGAATAAATC TTAGGGGTCA TTATCACTTA AATAATACTG TACCTAGGTC TTTCAAATTA 1740

AAATTATACC TGAATGAAGT TGTTTGTATA CATAAAGGAT ATTTGTGTAC AATTACCTTT 1800

TTTCCCCCAC ACTTGTTTTC TTTGTTTTTG TTTTTTATGG CAACTGGAAA GTATTTACTA 1860

TGGGATTCAT TTATGTCTGT CTTTCTATCA TAAAGAATTG ATCAATATGT AAATATGTGA 1920

TTTGAACCAT GGTTGACTTA CAAGTGTCAC TACAGCTTTT TAGAAAACAT AGCCCTAATA 1980

TATGTTAAGC AGGACCCGGG TGAGCCAGTG GGCTTGCGCT TTATGTAGAG CTGGAAGAAG 2040

GCCGTCCATC CTGTCTCTTG GGCGGACAGT GTACTTTCCT AATAGGGAAG GGAAGCACAA 2100

TGGAAATACC CCTGAACCGT TTTATTGCAG TAATTTTTTT CATATCTGAA ACTATTATTT 2160

AATATTTTGA ATAAGATTTT AAAAAATAAA TGGCAAAGAT ATAAATCTAA AAAAAAAAAA 2220

AAAAAAAAAA AAAAAAAAAA AAAAAAAAAA AAAAAAAAAA AAAAAAAAAA AAAAAA 2276

(2) INFORMATION FOR SEQ ID NO: 184:

(i) SEQUENCE CHARACTERISTICS:
(A) LENGTH: 2500 base pairs
(B) TYPE: nucleic acid
(C) STRANDEDNESS: double
(D) TOPOLOGY: linear

(xi) SEQUENCE DESCRIPTION: SEQ ID NO: 184:

TCCAAGCTAC GCCACTCGGG CTGGGGCGTT GGGAGCGGGA GTGCAGAGCG TGGTCGTGGC 60

GGCGGCGGTG AGAAGAGCGA GGCGKAGGAG GGGGTGCCAT GGCCGGGCAG CAGTTCCAGT 120

ACGATGACAG TGGGAACACC TTCTTCTACT TCCTCACCTC CTTCGTGGGG CTCATCGTGA 180

TCCCGGCGAC ATACTACCTC TGGCCCCGAG ATCAGAATGC CGAGCAAATT CGATTAAAGA 240

ATATCAGAAA AGTATATGGA AGGTGTATGT GGTACGTTTA CGGTTATTAA AACCCCAGCC 300

AAATATTATT CCTACAGTAA AGAAAATAGT TCTGCTTGCA GGATGGGCAT TGTTCTTATT 360

CCTTGCATAT AAAGTTTCCA AAACAGACCG AGAATACCAA GAATACAATC CTTATGAAGT 420

ATTAAATTTG GATCCTGGAG CCACAGTAGC AGAAATTAAA AAACAATATC GTTTGCTGTC 480

ACTTAAATAT CATCCAGATA AAGGAGGTGA TGAGGTTATG TTCATGAGGA TAGCAAAAGC 540

TTATGCTGCT TTAACGGATG AAGAGTCCCG GAAAAATTGG GAAGAATTTG GAAATCCAGA 600

TGGGCCTCAA GCCACAAGCT TTGGAATTGC CCTGCCAGCT TGGATAGTTG ACCAGAAAAA 660

TTCAATTCTG GTTTTACTTG TATATGGATT GGCATTTATG GTTATCCTTC CAGTTGTTGT 720

GGGCTCTTGG TGGTATCGCT CAATACGCTA TAGTGGAGAC CAGATTCTAA TACGSACAAC 780

ACAGATTTAT ACATACTTTG TTTATAAAAC CCGAAATATG GATATGAAAC GTCTTATCAT 840

GGTTTTGGST GGAGCTTCTG AATTTGATCC TCAGTATAAT AAAGATGCCA CAAGCAGACC 900

AACGGATAAT ATTCTAATAC CACAGCTAAT CAGAGAAATT GGCAGCATTA ATTTAAAGAA 960

GAATGAGCCT CCACTTACCT GCCCATATAG CCTGAAGGCC AGAGTTCTTT TACTGTCTCA 1020

TCTTGCTAGA ATGAAAATTC CTGAGACCCT TGAAGAAGAT CAGCAATTCA TGCTAAAAAA 1080

GTGTCCTGCC CTACTTCAAG AAATGGTTAA TGTAATCTGC CAACTAATAG TAATGGCCCG 1140

GAACCGTGAA GAAAGGGAGT TTCGTGCTCC AACTTTGGCA TCCCTAGAAA ACTGCATGAA 1200

GCTTTCTCAG ATGGCCGTTC AGGGACTTCA GCAATTTAAG TCTCCCCTTC TGCAGCTCCC 1260

TCATATTGAA GAGGACAATC TTAGACGGGT TTCTAATCAT AAGAAGTATA AAATTAAAAC 1320

TATCCAGGAT TTGGTGAGTT TAAAAGAATC AGATCGTCAC ACTCTACTGC ACTTCCTTGA 1380

AGATGAAAAA TATGAAGAGG TTATGGCTGT CCTTGGGAGT TTTCCATATG TGACCATGGA 1440

TATAAAATCA CAGGTGTTAG ATGATGAAGA TAGCAACAAC ATCACAGTAG GATCCTTAGT 1500

TACAGTGTTG GTTAAGTTGA CAAGGCAAAC AATGGCTGAA GTATTTGAAA AGGAGCAGTC 1560

CATCTGTGCT GCAGAGGAAC AGCCAGCAGA AGATGGGCAG GGTGAAACTA ACAAGAACAG 1620

GACAAAAGGA GGATGGCAAC AGAAGAGTAA AGGACCCAAG AAAACTGCTA AATCAAAAAA 1680

AAAGAAACCT TTAAAAAAAA AACCTACACC TGTGCTATTA CCACAGTCAA AGCAACAGAA 1740

ACAAAAGCAG GCAAATGGAG TCGTTGGGAA TGAAGCTGCA GTAAAGGAAG ATGAAGAAGA 1800

AGTTTCAGAT AAGGGCAGTG ATTCTGAAGA AGAAGAAACC AATAGAGATT CCCAAAGTGA 1860

GAAAGATGAT GGTAGTGACA GAGACTCTGA TAGAGAGCAA GATGAAAAAC AAAACAAAGA 1920

TGATGAAGCA GAGTGGCAAG AATTACAACA AAGCATACAG CGAAAAGAGA GAGCTCTATT 1980

GGAAACCAAA TCAAAAATAA CACATCCTGT GTATAGCCTT TACTTTCCTG AGGAAAAACA 2040

AGAATGGTGG TGGCTTTACA TTGCAGATAG GAAGGAGCAG ACATTAATAT CCATGCCATA 2100

TCATGTGTGT ACGCTGAAAG ATACAGAGGA GGTAGAGCTG AAGTTTCCTG CACCAGGCAA 2160

GCCTGGAAAT TATCAGTATA CTGTGTTTCT GAGATCAGAC TCCTATATGG GTTTGGATCA 2220

GATTAAACCA TTGGAAGTTK GGAAGTTCAT GAGGCTGAAG CCTGTGCCAG AAAATCACCC 2280

ACAGTGGGAT ACAGCAATAG AGGGGGATGA AGACCAGGAG GACAGTGAGG GCTTTGAAGA 2340

TAGCTTTGAG GGAGGAAGAG GGAGGGAGGA AGGAAGGTGG TGGACTTAAG GCAGTTACTC 2400

TGGAATGGGA CCCACAGTGT TTTGCACCAT ATTTTGGCAA TTTTTTTTGC CCGTTTTTNG 2460

GAAGTGTTTT CCNTNAANCC CAGGAACCAT TACAGAACCG 2500

(2) INFORMATION FOR SEQ ID NO: 185:

(i) SEQUENCE CHARACTERISTICS:
(A) LENGTH: 1337 base pairs
(B) TYPE: nucleic acid
(C) STRANDEDNESS: double
(D) TOPOLOGY: linear

(xi) SEQUENCE DESCRIPTION: SEQ ID NO: 185:

CTTCCGGTTC TCCGGGCAGC TGCCACTGCT GTAGCTTCTG CCACCTGCCA CGACCGGGCC 60

TCTCCCTGGC GTTTGGTCAC CTCTGCTTCA TTCTCCACCG CGCCTATGGT CCCTCTTGGA 120

GCCAGCGTGG CGGGCCTGGC GGCTCCCGGG TGGTGAGAGA GCGGTCCGGG AACGATGAAG 180

GCCTCGCAGT GCTGCTGCTG TCTCAGCCAC CTCTTGGCTT CCGTCCTCCT CCTGCTGTTG 240

CTGCCTGAAC TAAGCGGGYC CCTGGMAGTC CTGCTGCAGG CAGCCGAGGC CGCGCCAGGT 300

CTTGGGCCTC CTGACCCTAG ACCACGGACA TTACCGCCGC TGCCACCGGG CCCTACCCCT 360

GCCCAGCAGC CGGGCCGTGG TCTGGCTGAA GCTGCGGGGC CGCGGGGCTC CGAGGGAGGC 420

AATGGCAGCA ACCCTGTGGC CGGGCTTGAG ACGGACGATC ACGGAGGGAA GGCCGGGGAA 480

GGCTCGGTGG GTGGCGGCCT TGCTGTGAGC CCCAACCCTG GCGACAAGCC CATGACCCAG 540

CGGGCCCTGA CCGTGTTGAT GGTGGTGAGC GGCGCGGTGC TGGTGTACTT CGTGGTCAGG 600

ACGGTCAGGA TGAGAAGAAG AAACCGAAAG ACTAGGAGAT ATGGAGTTTT GGACACTAAC 660

ATAGAAAATA TGGAATTGAC ACCTTTAGAA CAGGATGATG AGGATGATGA CAACACGTTG 720

TTTGATGCCA ATCATCCTCG AAGATAAGAA TGTGCCTTTT GATGAAAGAA CTTTATCTTT 780

CTACAATGAA GAGTGGAATT TCTATGTTTA AGGAATAAGA AGCCACTATA TCAATGTTGG 840

GGGGGTATTT AAGTTACATA TATTTTAACA ACCTTTAATT TGCTGTTGCA ATAAATACCG 900

TATCCTTTTA TTATATCTTT ATATGTATAG AAGTACTCTR TTAATGGGCT CAGAGATGTT 960

GGGGATAAAG TATACTGTAA TAATTTATCT GTTTGAAAAT TACTATAAAA CGGTGTTTTC 1020

TGATCGGTTT TTGTTTCCTG CTTACCATAT GATTGTAAAT TGTTTTATGT ATTAATCAGT 1080

TAATGCTAAT TATTTTTGCT GATGTCATAT GTTAAAGAGC TATAAATTCC AACAACCAAC 1140

TGGTGTGTAA AAATAATTTA AAATTTCCTT TACTGAAAGG TATTTCCCAT TTTTGTGGGG 1200

AAAAGAAGCC AAATTTATTA CTTTGTGTTG GGGTTTTTAA AATATTAAGA AATGTCTAAG 1260

TTATTGTTTG CAAAACAATA AATATGATTT TAAATTCTCT TAAAAAAAAA AAAAAAAACC 1320

CCGGGGGGGG GCCCGGN 1337

(2) INFORMATION FOR SEQ ID NO: 186:

(i) SEQUENCE CHARACTERISTICS:
(A) LENGTH: 941 base pairs
(B) TYPE: nucleic acid
(C) STRANDEDNESS: double
(D) TOPOLOGY: linear

(xi) SEQUENCE DESCRIPTION: SEQ ID NO: 186:

GGCACGAGCC TGGACGCAGC AGCCACCGCC GCGTCCCTCT CTCCACGAGG CTGCCGGCTT 60

AGGACCCCCA GCTCCGACAT GTCGCCCTCT GGTCGCCTGT GTCTTCTCAC CATCGTTGGC 120

CTGATTCTCC CCACCAGAGG ACAGACGTTG AAAGATACCA CGTCCAGTTC TTCAGCAGAC 180

TCAACTATCA TGGACATTCA GGTCCCGACA CGAGCCCCAG ATGCAGTCTA CACAGAACTC 240

CAGCCCACCT CTCCAACCCC AACCTGGCCT GCTGATGAAA CACCACAACC CCAGACCCAG 300

ACCCAGCAAC TGGAAGGAAC GGATGGGCCT CTAGTGACAG ATCCAGAGAC ACACAAGAGC 360

ACCAAAGCAG CTCATCCCAC TGATGACACC ACGACGCTCT CTGAGAGACC ATCCCCAAGC 420

ACAGACGTCC AGACAGACCC CCAGACCCTC AAGCCATCTG GTTTTCATGA GGATGACCCC 480

TTCTTCTATG ATGAACACAC CCTCCGGAAA CGGGGGCTGT TGGTCGCAGC TGTGCTGTTC 540

ATCACAGGCA TCATCATCCT CACCAGTGGC AAGTGCAGGC AGCTGTCCCG GTTATGCCGG 600

AATCATTGCA GGTGAGTCCA TCAGAAACAG GAGCTGACAA CCYGCTGGGC ACCCGAAGAC 660

CAAGCCCCCT GCCAGCTCAC CGTGCCCAGC CTCCTGCATC CCCTCGAAGA GCCTGGCCAG 720

AGAGGGAAGA CACAGATGAT GAAGCTGGAG CCAGGGCTGC CGGTCCGAGT CTCCTACCTC 780

CCCCAACCCT GCCCGCCCCT GAAGGCTACC TGGCGCCTTG GGGGCTGTCC CTCAAGTTAT 840

CTCCTCTGYT AAGACAAAAA GTAAAGCACT GTGGTCTTTG CAAAAAAAAA AAAAAAAAAA 900

AAAAAAAAAA AAAAAAAAAA AAAAAAAAAA AAAAAACTCG A 941

(2) INFORMATION FOR SEQ ID NO: 187:

(i) SEQUENCE CHARACTERISTICS:
(A) LENGTH: 654 base pairs
(B) TYPE: nucleic acid
(C) STRANDEDNESS: double
(D) TOPOLOGY: linear

(xi) SEQUENCE DESCRIPTION: SEQ ID NO: 187:

GAATTCGGCA CGAGGCAGCT TGTGCTTTAA AGGAGGTGTT CAAAGCATGT CTGAGCAGAG 60

ACTTTTGGGC TCTGTTTTAA TTAATACTTT AAAATAATTC ATATTTAAAA TATCARATGT 120

TTCCATAAAG AGGAGGATGT TTAAATGCCT CCAGACTACA TTCCTTTTTA TTSCTTGATT 180

TTACCTGGGA GTCCAAAGTT CAATTCCCAT AAAGCAAGCG TTTTATTTGT CACTTTCAAT 240

ATACATCCGA TTGCCATGCT TAAGATGCAA TATGGGCTGC GGAAATAGGT TAACCCACAG 300

GCTCCCAGGG CCCAGTGTAG AAGGTGAGAG ATTCGTGTAA AATGATTCAA ATAAAAGGAA 360

GACCCTGGCC GGGTGCCGTA RCTCACGCCT GTAATCCCAG CACTTTGGGA GGCCGAAGCG 420

AGTGGATGAC GAGGTTAGGA GTTGGAGACC AGCCTGGCCA ACATCGTGAA ACCCCGTCTC 480

TACTAAAAAT ACAAAAATTA GCCGGGCATG GTGGCAGGCA CCTGTAATCC TAGCTAGTTG 540

GGAGGCTGAG GCAGGAGAAT CGTTTGAATC TGGGAGTTGG AGGTTGTCAG TGAGCTGAGA 600

TCGCGCCACA GCACTCCAGC CTGGGTGACA GGGTGAGACT CTGTCTCAAA NAGA 654

(2) INFORMATION FOR SEQ ID NO: 188:

(i) SEQUENCE CHARACTERISTICS:
(A) LENGTH: 1848 base pairs
(B) TYPE: nucleic acid
(C) STRANDEDNESS: double
(D) TOPOLOGY: linear

(xi) SEQUENCE DESCRIPTION: SEQ ID NO: 188:

GAAACTGGAC CGGAGAACCG GAGCGAAGCG AAGCGGAAGC CCGGAATGAG GCCGGACTGG 60

AAAGCCGGAG CGGGGCCAGG CGGGCCTCCC CAAAAGCCTG CCCCTTCATC CCAGCGGAAA 120

CCGCCGGCCC GGCCGAGCGC GGCGGCCGCT GCGATTGCAG TCGCGGCGGC GGAGGAAGAG 180

AGACGGCTCC GGCAGCGGAA CCGCCTGAGG CTGGAGGAGG ACAAACCGGC CGTGGAGCGG 240

TGCTTGGAGG AGCTGGTCTT CGGCGACGTC GAGAACGACG AGGACGCGTT GCTGCGGCGT 300

CTGCGAGGCC CGAGGGTTCA AGAACATGAA GACTCGGGTG ACTCAGAAGT GGAGAATGAA 360

GCAAAAGGTA ATTTTCCACC TCAAAAGAAG CCAGTTTGGG TGGATGAAGA AGATGAAGAT 420

GAGGAAATGG TTGACATGAT GAACAATCGG TTTCGGAAGG ATATGATGAA AAATGCTAGT 480

GAAAGTAAAC TTTCGAAAGA CAACCTTAAA AAGAGACTTA AAGAAGAATT CCAACATGCC 540

ATGGGAGGAG TACCTGCCTG GGCAGAGACT ACTAAGCGGA AAACATCTTC AGATGATGAA 600

AGTGAAGAGG ATGAAGATGA TTTGTTGCAA AGGACTGGGA ATTTCATATC CACATCAACT 660

TCTCTTCCAA GAGGCATCTT GAAGATGAAG AACTGCCAGC ATGCGAATGC TGAACGTCCT 720

ACTGTTGCTC GGATCTCCAT CTGTGCAGTT CCATCCCGGT GCACAGATTG TGATGGTTGC 780

TGGGATTAGA TAATGCTGTA TCACTATTTC AGGTTGATGG GAAAACAAAT CCTAAAATTC 840

AGAGCATCTA TTTGGAAAGG TTTCCAATCT TTAAGGCTTG TTTTAGTGCT AATGGGGAAG 900

AAGTTTTAGC CACGAGTACC CACAGCAAGG TTCTTTATGT CTATGACATG CTGGCTGGAA 960

AGTTAATTCC TGTGCATCAA GTGAGAGGTT TGAAAGAGAA GATAGTGAGG AGCTTTGAAG 1020

TCTCCCCAGA TGGGTCCTTC TTGCTCATAA ATGGCATTGC TGGATATTTG CATTTGCTAG 1080

CAATGAAGAC CAAAGAACTG ATTGGAAGCA TGAAAATTAA TGGAAGGGTT GCAGCATCCA 1140

CATTCTCTTC AGATAGTAAG AAAGTATACG CCTCTTCGGG GGATGGAGAA GTTTATGTTT 1200

GGGATGTGAA CTCAAGGAAG TGCCTTAACA GATTTGTTGA TGAAGGCAGT TTATATGGAT 1260

TAAGCATTGC CACATCTAGG AATGGACAGT ATGTTGCTTG TGGTTCTAAT TGTGGAGTGG 1320

TAAATATATA CAATCAAGAT TCTTGTCTCC AAGAAACAAA CCCAAAGCCA ATAAAAGCTA 1380

TAATGAACTT GGTTACAGGT GTTACTTCTC TGACCTTCAA TCCTACTACA GAAATCTTGG 1440

CAATTGCTTC AGAAAAAATG AAAGAAGCAG TCAGATTGGT TCATCTTCCT TCCTGTACAG 1500

TATTTTCAAA CTTCCCAGTC ATTAAAAATA AGAATATTTC TCATGTTCAT ACCATGGATT 1560

TTTCTCCGAG AAGTGGATAC TTTGCCTTGG GGAATGAAAA GGGCAAGGCC CTGATGTATA 1620

GGTTGCACCA TTACTCAGAC TTCTAAAGAG ACTATTTGAA GTCCAGTTGA GTCACAAGAG 1680

AAGCCTGTCT TGATATATCA TCTCAGAAAC TTTCCTGAAT ATGTGATAAT ATATGGAAAA 1740

TGATTTATAG ATCCAGCTGT GCTTAAGAGC CAGTAATGTC TTAATAAACA TGTGGCAGCT 1800

TTTGTTTGAA AAAAAAAAAA AAAAAAAAAA AAAAAAAAAA AAACTCGA 1848

(2) INFORMATION FOR SEQ ID NO: 189:

(i) SEQUENCE CHARACTERISTICS:
(A) LENGTH: 1146 base pairs
(B) TYPE: nucleic acid
(C) STRANDEDNESS: double
(D) TOPOLOGY: linear

(xi) SEQUENCE DESCRIPTION: SEQ ID NO: 189:

AAAAAAAACC CAGGGGAACN TTGGGGGCCG CTTTNNNTTC CCCCTCCAGG CCATTGGGGA 60

ATTCTTCAAG TTAATCCTGC TTTGCTCTTG GCCAACAGGG CTTGTAGGGG GGAGAGACCC 120

AGGATCATCA AGGGGTTCGA GTGCAAGCCT CACTCCCAGC CCTGGCAGGC AGCCCTGTTC 180

GAGAAGACGC GGCTACTCTG TGGGGCGACG CTCATCGCCC CCAGATGGCT CCTGACAGCA 240

GCCCACTGCC TCAAGCCCCG CTACATAGTT CACCTGGGGC AGCACAACCT CCAGAAGGAG 300

GAGGGCTGTG AGCAGACCCG GACAGCCACT GAGTCCTTCC CCCACCCCGG CTTCAACAAC 360

AGCCTCCCCA ACAAAGACCA CCGCAATGAC ATCATGCTGG TGAAGATGGC ATCGCCAGTC 420

TCCATCACCT GGGCTGTGCG ACCCCTCACC CTCTCCTCAC GCTGTGTCAC TGCTGGCACC 480

AGCTGYCTCA TTTCCGGCTG GGGCAGMACG TCCAGCCCCC AGTTACGCCT GCCTCACACC 540

TTGSGATGCG CCAACATCAC CATCATTGAG CACCAGAAGT GTGAGAACGC CTACCCCGGC 600

AACATCACAG ACACCATGGT GTGTGCCAGC GTGCAGGAAG GGGGCAAGGA CTCCTGCCAG 660

GGTGACTCCG GGGGCCCTCT GGTCTGTAAC CAGTCTCTTC AAGGCATTAT CTCCTGGGGC 720

CAGGATCCGT GTGCGATCAC CCGAAAGCCT GGTGTCTACA CGAAAGTCTG CAAATATGTG 780

GACTGGATCC AGGAGACGAT GAAGAACAAT TAGACTGGAC CCACCCACCA CAGCCCATCA 840

CCCTCCATTT CCACTTGGTG TTTGGTTCCT GTTCACTCTG TTAATAAGAA ACCCTAAGCC 900

AAGACCCTCT ACGAACATTC TTTGGGCCTC CTGGACTACA GGAGATGCTG TCACTTAATA 960

ATCAACCTGG GGTTCGAAAT CAGTGAGACC TGGATTCAAA TTCTGCCTTG AAATATTGTG 1020

ACTCTGGGAA TGACAACACC TGGTTTGTTC TCTGTTGTAT CCCCAGCCCC AAAGACAGCT 1080

CCTGGCCATA TATCAAGGTT TCAATAAATA TTTGCTAAAT GAAAAARAAA AAAAAAAAAA 1140

ACTCGA 1146

(2) INFORMATION FOR SEQ ID NO: 190:

(i) SEQUENCE CHARACTERISTICS:
(A) LENGTH: 906 base pairs
(B) TYPE: nucleic acid
(C) STRANDEDNESS: double
(D) TOPOLOGY: linear

(xi) SEQUENCE DESCRIPTION: SEQ ID NO: 190:

ACTCCCTCAC CCAGGTCCCA GCCCTGGGAA CCACCTACCG TGAGCCCTTT TGCAGATATA 60

GACTCATTTC ATCCTCAGAT GGTCCTTCAA GGTAGGTACT TTAGTCCCAT TTTAGAGATG 120

AGACGATTGA GGCCAGAGGG GTGNNGTAAC TTGCCTGGGG GCTCACGAGC ACAAAAGGAG 180

CCGAGGCAGG ATCTGACCCT TGTTCTCTGG CCTCACTGCC CTCACTTTGC CATGACCCGA 240

AGTTATGTCC CTACAAAGCA ATGCATGGTC CAAGGYTCTT TTTATTGTAT TTTTATTTTT 300

AAGGGTCCTG TTCAAAACTG GTGTGAGCTC TGAGGAGTCC TGAACCCTGG GTGCAGCATC 360

CTAGCATCCT GGGAGTCCTT TTCTGCCCAC ACTGAGCTGG GCTCCTCGAG GGGTGGGGCT 420

GCTGTCCCTG GAAGCCTGGC AGCAGCACTG TATCGGGTTG GCTGAAGCTG ARCGCCGTGG 480

GGTGCAGGGC TCCMGGAATC CCCGTTTGGC TGAAGGGGTT CCCTGTAGCC MGGGATGTTT 540

ATGAGGTCTC TCTGATGCCC CAGGCGCAGG ACATGTGTGC GGGTGGAGAA AAGCAGGCCC 600

TTTCAGTGCC AGCTCCACTC AATTTCTATG TGGACCAAGA ACGATAAACT TAAAAAATTT 660

TTTTTCCTAA GGTATCTTCA GAATATGGTG TATTTTTATG TGGAAAAGAA AAGTTATGAA 720

GGCAGCTGTT ACTTTAAGAG AAAATTCATT AAAAGTCCTC GAGGTATGAA GATGACGGCG 780

TGCTTCTCAA TCATTTTGGC ATAACTTGAT TGTGGCTGTA ATTTTTTTTT TTTTTTTTGT 840

CAAGCATGTC AGACAATAAA GTCTTTGTAA AAAGRGAAAA AAAAAAAAAA AAAAAAAAAA 900

ACTCGA 906

(2) INFORMATION FOR SEQ ID NO: 191:

(i) SEQUENCE CHARACTERISTICS:
(A) LENGTH: 1941 base pairs
(B) TYPE: nucleic acid
(C) STRANDEDNESS: double
(D) TOPOLOGY: linear

(xi) SEQUENCE DESCRIPTION: SEQ ID NO: 191:

CTTCAGCTGA AGCCCAGGGA CCCCTTTTCC ACCCTGGGCC CCAATGCCGT CCTTTCCCCG 60

CAGAGACTGG TCTTGGAAAC CCTCAGCAAA CTCAGCATCC AGGACAACAA TGTGGACCTG 120

ATTCTGGCCA CACCCCCCTT CAGCCGCCTG GAGAAGTTGT ATAGCACTAT GGTGCGCTTC 180

CTCAGTGACC GAAAGAACCC GGTGTGCCGG AGATGGCTGT GGTACTGCTG GCCAACCTGG 240

CTCAGGGGGA CAGCCTGGCA GCTCGTGCCA TTGCAGTGCA GAAGGGCAGT ATCGGCAACC 300

TCCTGGGCTT CCTAGAGGAC AGCCTTGCCG CCACACAGTT CCAGCAGAGC CAGGCCAGCC 360

TCCTCCACAT GCAGAACCCA CCCTTTGAGC CAAYTAGTGT GGACATGATG CGGCGGGCTG 420

CCCGCGCGCT GCTTGCCTTG GCCAAGGTGG ACGAGAACCA CTCAGAGTTT ACTCTGTACG 480

AATCACGGCT GTTGGACATC TCGGTATCAC CGTTGATGAA CTCAKTGGTT TCACAAGTCA 540

TTTGTGATGT ACTGTTTTTG NATTGGCCAG TCATGACAGC CGTGGGACAC CTCCCCCCCC 600

CGTGTGTGTG TGCGTGTGTG GAGAACTTAG AAACTGACTG TTGCCCTTTA TTTATGCAAA 660

ACCACCTCAG AATCCAGTTT ACCCTGTGCT GTCCAGCTTC TCCCTTGGGA AAAAGTCTCT 720

CCTGTTTCTC TCTCCTCCTT CCACCTCCCC TCCCTCCATC ACCTCACGCC TTTCTGTTCC 780

TTGTCCTCAC CTTACTCCCC TCAGGACCCT ACCCCACCCT CTTTGAAAAG ACAAAGCTCT 840

GCCTACATAG AAGACTTTTT TTATTTTAAC CAAAGTTACT GTTGTTTACA GTGAGTTTGG 900

GGAAAAAAAA TAAAATAAAA ATGGCTTTCC CAGTCCTTGC ATCAACGGGA TGCCACATTT 960

CATAACTGTT TTTAATGGTA AAAAAAAAAA AAAAAAATAC AAAAAAAAAT TCTGAAGGAC 1020

AAAAAAGGTG ACTGCTGAAC TGTGTGTGGT TTATTGTTGT ACATTCACAA TCTTGCAGGA 1080

GCCAAGAAGT TCGCAGTTGT GAACAGACCC TGTTCACTGG AGAGGCCTGT GCAGTAGAGT 1140

GTAGACCCTT TCATGTACTG TACTGTACAC CTGATACTGT AAACATACTG TAATAATAAT 1200

GTCTCACATG GAAACAGAAA ACGCTGGGTC AGCAGCAAGC TGTAGTTTTT AAAAATGTTT 1260

TTAGTTAAAC GTTGAGGAGA AAAAAAAAAA AGGCTTTTCC CCCAAAGTAT CATGTGTGAA 1320

CCTACAACAC CCTGACCTCT TTCTCTCCTC CTTGATTGTA TGAATAACCC TGAGATCACC 1380

TCTTAGAACT GGTTTTAACC TTTAGCTGCA GCGNCTACGT CNAWCGNTGT GTATATATAT 1440

GACGTKGTAC ATTGCACATA CCCTTGGATC CCCACAGTTK GGTCCTCCTC CCAGCTACCC 1500

CTTTATAGTA TGACGAGTTA ACAAGTTGGT GACCTGCACA AAGCGAGACA CAGCTATTTA 1560

ATCTCTTGCC CAGATATCGC CCCTCTTGGT GCGATGCTGT ACAGGTCTCT GTAAAAAGTC 1620

CTTGCTGTCT CAGCAGCCAA TCAACTTATA GTTTATTTTT TTCTGGGTTT TTGTTTTGTT 1680

TTGTTTTCTT TCTAATCGAG GTGTGAAAAA GTTCTAGGTT CAGTTGAAGT TCTGATGAAG 1740

AAACACAATT GAGATTTTTT CAGTGATAAA ATCTGCATAT TTGTATTTCA ACAATGTAGC 1800

TAAAACTTGA TGTAAATTCC TCCTTTTTTT CCTTTTTTGG CTTAATGAAT ATCATTTATT 1860

CAGTATGAAA TCTTTATACT ATATGTTCCA CGTGTTAAGA ATAAATGTAC ATTAAATCTT 1920

GGTAAGACTT TAAAAAAAAA A 1941

(2) INFORMATION FOR SEQ ID NO: 192:

(i) SEQUENCE CHARACTERISTICS:
(A) LENGTH: 2118 base pairs
(B) TYPE: nucleic acid
(C) STRANDEDNESS: double
(D) TOPOLOGY: linear

(xi) SEQUENCE DESCRIPTION: SEQ ID NO: 192:

AAATAATAAT AANAATAAAT AAAAATWAAG TGCTTAKTGT AACTCAGCGG ACAGGGCTCC 60

CAGCTGCTCT GGCACGTGGG ACACCYTCCA CCCTGCACAC AACAGGCATG CAAAGAGGAC 120

TGGATATGGT GGGGTAGAGT GCTTCTGGTG TGTTCACTTT AAGAAAACAT CTGCCAAGAG 180

AGAAGAGTGC CCAGGAAAGA CCAGGAAAAT ACAAGTACAT GGCTGCTTCA TACCATATAC 240

CCCAATTCTT TAAAGCAGCA AAAGGCACTT TTTTTTTCAG GCCAGAGTGA ATCTAAAACA 300

AACCTGGCTT TGCTTACAGG GAAGCTGTCC CAGAAGGACT GAGTGATGCC TCTTGTTCCC 360

TAAGGTCTGG AGAGTCTTTG CAAGTTTCCA ACGACATTTC CAACCAGGTG GGAGAGACCA 420

GCAGTTGACG AGACAAGTCA GACCCAAAAA ACGACGCCAA GGTAGTGAGT GGGTGCCTAT 480

TTGGGAGTAG GATGATTTGA GGAAAACAGG AAGAAAAACC GGTCAGAAAG TGGCACTTTG 540

GAAGTGGAAA GCTGTTTGCA AATAGCAACT CTGGCTAAAG CGAAAATGTT AATCAAGTAG 600

AAAGTAAAAT TCAGGATCTT AGAAGCTCAT CCTTCTGATG AGAACTATTT TTTTTTCCGT 660

GAAGGAACTA TTATTACTTT AAAAGTGAGG GTAATTTACA TATGGGGTGT ATATATTCTA 720

AAAATAGTAA TAAAAGTACC TTTTATAAGC AATGTTGTGT GGCTTGTAGA AGAAAGCAGG 780

GAGGAAAAAA AGGCAGGCAA AACTAGTCTA GGTCTAGGCC CTAAAAATGA GCTTCCTTCC 840

CACTTGACTG GAAACGCCCA TGTGATTTCT AGGCTGAAAA TAGGTAGGAT TTAACGAGTA 900

ACCTAGTTCC CTTCTGTCTC TGATTTCTGA TCAGCTGATG GAGCTGCTAG TAAGAGGGGC 960

CGATCATGCT CCCAGACGAG TCCTTTGGCC TCTTGCTCTC CATCCCAAGC CTGACTCCTT 1020

CAGCAGCAGC CCCCTCCTTC TGTGTCCATC TGATGCAGGC AAGCAGGAGC AGTAAGAGGG 1080

CATCCCATGT TCCAGTTCAC CTTCTATGGG GTGACTARGA GGTTCCCGGT AACTAGGGCA 1140

GCCCARGCCC AGCAGGTTGC AAAAGCAGCT GCAAGCTTCA GAAACCCACT TCCTCCAACA 1200

CCAGGGAGGT GGCAGAGAGC CCATCCAAAA GCCCACTGGG AGAGGCATAA GATTCTGTGC 1260

CAGGCCCCCA GGTCCCCTCT GTGTCAGGTA GGCTCTGCTA CTGGCCTCTG AAGTAAAGGC 1320

AAANACAAAC GGGCAGGGCA GGGTGGCAGG AATAAAAAAC TCTGGACAGA AACCCTTTTA 1380

ATAAAGGAAA TTCCACCCCT CCCAATCCTT CCATGGAAGG GTGAGACCTT AATGTGATGT 1440

AAGAGGAAGG TCTTCTCTGG CTTTCAGGGA AACAGCTGCA GCTGAAACTT AGGGGCCCAT 1500

TCCAGGGCAC TTTTCACCAC AGCCAGTGCA GCCGCTCCAA GTGCCACTGT CAGCCCCATC 1560

ACTGCCAATT TCACAAAGCG GTTGGTCCTT GGCTTGGTCA GGACATCTTT TGTTCGATCT 1620

TCAGGCCGCA GAAGTCCCCG AANACCGCTG CCGCAGCACC ATATCAGGCC TCTGCTGGGC 1680

TGATGCCAGC TCAAAGTCTT TGAAAGTAGA GGCTGCCGTC CTCTCAGCTT GCTGTTGGGC 1740

AGCGGCCTCC CGAGCAAGTT CGGATGGGGG AAACTGAACA AAAAGGTCTC CTSTCTGCTG 1800

ATCAGTGTCT CATAGGGCAA GTCCTGAGGG ATCTGGGACA ACAGGTGGTG GACCGAGGCC 1860

ATGTCACAGT CACAGTCCAG GACTTCCTGC TCGCGATACA ACACAATCAC GGCTGCAAAG 1920

TAAATCGGCA TCAGTGGGTG GCAGGCCAGG AAGAAGTCAT ATAACCGCAC GACGTGCCTG 1980

AAGTCAGACA GGACATGCCC AAACCAGGTG ATGAGCCAGC TGAGGGCAAA GATGGTCCCT 2040

ACCTCAGCAC TCTGCATGAA GTCATGGAGC TCTGGATTCA CCTGGTCAAT GATGGGCATC 2100

AGATAGTTTA ATATATGC 2118

(2) INFORMATION FOR SEQ ID NO: 193:

(i) SEQUENCE CHARACTERISTICS:
(A) LENGTH: 1538 base pairs
(B) TYPE: nucleic acid
(C) STRANDEDNESS: double
(D) TOPOLOGY: linear

(xi) SEQUENCE DESCRIPTION: SEQ ID NO: 193:

CCGGGTTCGG CTCTGTGTCA GCAGCCGGGC GGCGCTCGGG CGGGACATGG CAGCCTGTAC 60

AGCCCGGCGG CCTGGCCGTG GGCAGCCGCT GGTGGTCCCG GTCGCTGACT GNGGCCCGGT 120

GGCCAAGGCC GCTCTGTGCG CGGCCGNAGC TGGAGCCTTC TCGCCAGCGT CGACCACGAC 180

GACGCGGAGG CACCTCTCGT CCCGAAACCG ACCAGAGGGC AAAGTGTTGG AGACAGTTGG 240

TGTGTTTGAG GTGCCAAAAC AGAATGGAAA ATATGAGACC GGGCAGCTTT TCCTTCATAG 300

CATTTTTGGC TACCGAGGTG TCGTCCTGTT TCCCTGGCAG GCCAGACTGT RTGACCGGGA 360

TGTGGCTTCT GCAGCTCCAG AAAAAGCAGA GAACCCTGCT GGCCATGGCT CCAAGGAGGT 420

GAAAGGCAAA ACTCACACTT ACTATCAGGT GCTGATTGAT GCTCGTGACT GCCCACATAT 480

ATCTCAGAGA TCTCAGACAG AAGCTGTGAC CTTCTTGGCT AACCATGATG ACAGTCGGGC 540

CCTCTATGCC ATCCCAGGCT TGGACTATGT CAGCCATGAA GACATCCTCC CCTACACCTC 600

CACTGATCAG GTTCCCATCC AACATGAACT CTTTGAAAGA TTTCTTCTGT ATGACCAGAC 660

AAAAGCACCT CCTTTTGTGG CTCGGGAGAC GCTAAGGGCC TGGCAAGAGA AGAATCACCC 720

CTGGCTGGAG CTCTCCGATG TTCATCGGGA AACAACTGAG AACATACGTG TCACTGTCAT 780

CCCCTTCTAC ATGGGCATGA GGGAAGCCCA GAATTCCCAC GTGTACTGGT GGCGCTACTG 840

TATCCGTTTG GAGAACCTTG ACAGTGATGT GGTACAGCTC CGGGAGCGGC ACTGGAGGAT 900

ATTCAGTCTC TCTGGCACCT TGGAGACAGT GCGAGGCCGA GGGGTAGTGG GCAGGGAACC 960

AGTGTTATCC AAGGAGCAGC CTGCGTTCCA GTATAGCAGC CACGTCTCGC TGCAGGCTTC 1020

CAGTGGGCAC ATGTGGGGCA CGTTCCGCTT TGAAAGACCT GATGGCTCCC ACTTTGATGT 1080

TCGGATTCCT CCCTTCTCCC TGGAAAGCAA TAAAGATGAG AAGACACCAC CCTCAGGCCT 1140

TCACTGGTAG GCCAGCTGAG GCCCCAAGTG CCCAGGCTTG GTCACCGGGA AGAACAACTC 1200

TCATCCCACA ATTGCTGCAG AACTCTTCTC TCCCCATCAT GGGCCACAGT GGGTCTCTTA 1260

ATTTGATTGT GGGGTTCTTT TTGTGGGGAG GGGTGGTATA ACTTTTCTTC AGAAGACCCA 1320

TGTGGGACAC CTCCAAGGCT GGCCTCCTCA TAAGCCCTGC CTACACCATG TTCCAGTAAA 1380

CCTCTCCACC AAGGAACTGT GTTCAGCTGC CACAGGCCTG GAGGAGTTTC CTGGCCTGTC 1440

ACGTGAGGTT TGATCAGTAA ACCAGTGCAS GYTTGGCCAA AAAAAAAAAA AAAAAAAAAA 1500

AAAAAAAAAA AAAAAAAAAA AAAAAAAAAA AAACTCGA 1538

(2) INFORMATION FOR SEQ ID NO: 194:

(i) SEQUENCE CHARACTERISTICS:
(A) LENGTH: 1098 base pairs
(B) TYPE: nucleic acid
(C) STRANDEDNESS: double
(D) TOPOLOGY: linear

(xi) SEQUENCE DESCRIPTION: SEQ ID NO: 194:

AGACCCTGTC TCAAATAATA ATAATAATAA TAATCTTATT TTGGAGAATA AAGAGACCTS 60

TGGATTTGAG GTGCCATTTG GGTAGAAAGA AAAGACGTTT ACACCGAGAA ATAGTCTGTG 120

TTGCCCTGAA GGAGCAGAGG GATGCATCGC TGGAGGTGAC CTACAGTTGA AGAAGACTCA 180

TTATGACAGA CCTTGTCCTT CTTCCTTGTG GAAAGTGTTT CCTCTGCTGC TACTGCTCAT 240

GAGACTCTTC CCCCTCCCTG TCCCAGGGAA CCAAAGGGCT TTNCTACCAC ACCCTTTCTT 300

NGCCCCCCGC CTCCCATGTC TGCTGTGCCT TTGTACTCAG CAATTCTTNG TTTGCTCCCA 360

TTATCTTCCA GCCGGATACA GAGTGAATAG TTAACCACAC TTAGGTCAAA TAGGATCTAA 420

ATTTTTGTTC CTGCTCCNGT GTAAAGAGGC CAGTGTTTGT GTGTTGCAAG CAGCCTTGGA 480

ATAGTAACTC TTCTCATTTG TTTGGGATCT GGCCAMCAAG TTCCAGAATG ATACACGGAT 540

CAGTGCAGAA GTTCATCAGG CTCTCGGACC TTAGGGCTGT TGGAGAAGGC TTCAGCAGCA 600

GAACTGATGG TKAWKGYTCG TGTTCTCCAT CCTCAACTTT CTTTGCTTCG ATCATACACA 660

AGAATACATT TGGAAGGGCA AAAAATGAAC ACTGTTGTTC ATTGCAGCCG TGTTTTGTGA 720

CACAGATGCA CAGTCTGCTG TGAAGACCTT CTCTCAAGTG GSATYTGGGA GTCCATGCCA 780

GATCATGGTG CTTCATGAGA GACTGACAGC TATCAGGGGT TGTGGCACTT AGTGAGGACT 840

CTCCTCCCCC AGTGTGTGCT GATGACACAT ACACACCTGA CAATAGCTTG AGTCTTCTCT 900

GTTCCTTTTA CTCTGTAGCC AACATACACA TGATTTAAAA CCCTTTCTAA ATATCTATCA 960

TGGTTCATCC TTGTCCAAAT GCAGAGTCAG AGCTATTTGT ACTTCATTAT TATTTCCAAG 1020

GCGAATAGTT GGCTTTCTTT TTGCAAAAAT AATTAAAGTT TTTGTATGTT GCAAAAAAAA 1080

AAAAAAAAAA CTACGTAG 1098

(2) INFORMATION FOR SEQ ID NO: 195:

(i) SEQUENCE CHARACTERISTICS:
(A) LENGTH: 1001 base pairs
(B) TYPE: nucleic acid
(C) STRANDEDNESS: double
(D) TOPOLOGY: linear

(xi) SEQUENCE DESCRIPTION: SEQ ID NO: 195:

GAATTCGGCA CGAGATAGCT TGCATCTCAT CCCAGTAAAA CCACTTATTT ATAACATATC 60

AACGTATTGA CAAGGTTGAA GAGCAAGATT GTTCTGAGGT GAGATGCAAA TTTCAAAGGG 120

GTGAGCACTA ATTGTTCCAG TGATTGTTTA TTTATTGGCT AGGACATAAT TACTCTCTTT 180

GAGGTTACAC ATCTGCCTCC AGGTTCCTGT GTGCTTGTGC CCTTGGGATC AGGCCAGGGC 240

AGACTGTGAT CACTGAGATT CAAACTCCCA GARTAATCAG CAAGAGCTTT CTAGAGACCA 300

AGGCCAGGCC TGATCCCTGA GGGATGCATG AGAAGGCTTG GAATCTCATT CTGCTATGGT 360

GGCTCTCTCT TGATCTTCTT GGAGTAGCAA AAACAGCAAT GTGGGCCCAA TGGTGTGGCC 420

TAAATGATCA CAAAGGTAAA TGAGTAAAGG GCTCAGCAGA TGAGTAAGGA GCCTTGTCCT 480

GAGAAATTAG CACTGGGCTC TGCATTCAGA AACATGTGAT AAGCATTGCC CATTGCACAT 540

TGCCTTTATT GTGTAAGGAC ATGAAATTCC AGTTTTGCAT AGCTAGTGAT GAATACCTGA 600

AGGGAATTGC AGACATATTT TATTTTATTT TTAATTGACA GATGGAATTG TATATATTTA 660

TCATGTACAT AATCATGCTT TAAAATATGT ACATTATGGA ATGGCTAAAT CAAACTAACC 720

TAGGCATTAT CTCATATAAT TGTCATTTTT GTGGCGAGAA GACTAAAAAT CTACCCTTTC 780

AGCATTTTTA AAGAATACAA TGTGTTTTAT TAACAACAGT CACCATTTGG TACACTAGAT 840

CTCTTGAACT TCTTCCTCTT ATCTAACTGA GATCTTGTAA CCTTTGATAA CAGCTCCCAA 900

GCCCTTCCCC AACCACTGCT CCACCCGTGG TAACCACCAT TCTATTCTCA ACTTCCTGGT 960

AATCACCATT CTAGACACAG GGAAGACTCT CTACCCTCTG A 1001

(2) INFORMATION FOR SEQ ID NO: 196:

(i) SEQUENCE CHARACTERISTICS:
(A) LENGTH: 1443 base pairs
(B) TYPE: nucleic acid
(C) STRANDEDNESS: double
(D) TOPOLOGY: linear

(xi) SEQUENCE DESCRIPTION: SEQ ID NO: 196:

ATAAACTGAA ATAGGTCATG CAAATATAAA ATATTATTTT TAAATTATTT GTCATAAGAA 60

ACGATGGTGG CCATATTTTG CTTTAATAAT GGAAAAAATG TGGTTAGCAT TCTKTGGAAG 120

GTGGTCATCA GATAGTAGAC ATTTTCTAGG ATTTATTTCT ACCTGCATAT GTGGAAATGT 180

GTACTACTTT AGATTTATWT AATGGCAGCT AACTCAGAGG CATCAAAATG TGCTAATGGT 240

GTAATATGGC CTTTGTCTTG CTGTYCTGTT TTGTARGCCT TCAATCAAGC ARGGGCAGGG 300

CCGTACAGTG AACTTGTCCT TTGSCAGACG CCAGCGTCTG CCCCTGACCC CGTCTCCACT 360

CTCTGTGTCC TGGAGGAGGA GCCCCTTGAT GCYTACCCTG ATTCACCTTC TGCGTGCCTT 420

GTACTGAACT GGGAAGAGCC GTGCAATAAC GGATCTGAAA TCCTTGCTTA CACCATTGAT 480

CTAGGAGACA CTAGCATTAC CGTGGGCAAC ACCACCATGC ATGTTATGAA AGATCTCCTT 540

CCAGAAACCA CCTACCGGTG AGTGCAAGGG AGTAGAAATC TGCATCAGCA CATCAGCACT 600

TGGGGATCTA AGTAAACCTC TCGGGGAAAA TGACCAAGTG GATGTCATCT CCCAGCTGTT 660

TCTAAGAGCC CAGATGTCCA GAGTATTGTC TCACCTTGAT CCCTCAGGCC AGAAGACCTG 720

TGAAAAAGCC ACACTGGTTC AGGGACTCAC TGGACGGTTT TGTGTCCACT YTAACNTGCA 780

CCGTCTCTAC CCCAGAGTGG ACTCARATCC TCAAGTCATC CTCTGAACAT TGRRGTCAGA 840

AATTATAAAA GGGCTTTGGC AATATGTTAG CCCAAGRATT TGGCTTCTTC CAGAAATTGT 900

GCCGACNTTA ACAGTGGCTT AAATGATGGT AAAACTTTTA AGATTTCTAA AAGGRTGGCA 960

TTGGAGATAC GTTGACTTTT ATTAAACMAC CTATAGTTGT TTAATGATTT CTAAAAAAAT 1020

ATCTGGAGCT CAGGGGTTCA ACTGAGGGAA CACATGTTGA GRATCATTGT TTACTAATTA 1080

AATGCCAGGT AACCCGTTGA AATTATCAAA AACATCTTCC ACGTACCAGA AAGCACCTCA 1140

GAGGATAGTT CTGTTATGGA GAAGATGAAA TGGTTTAGTA GTGTAGGAAC TATGGAAAGG 1200

TGAGCTTAGA TTTGGATAGT AAAACCTCAA GACCCTATTT AAAAAGTATT TTATGAATGC 1260

AGCATAAATA ATTTAATTCA GTGTTAANAT GCCAAGGCTA GTATATTGAG CTGAATGTGA 1320

AAAGAAACTC ACATTGGGAG AATGCCACCT TTTCCTTATA AGATAGCTTT GAAGATACCA 1380

TTTTAGACAG ATGGAAATTG AATAGCTTTA GAAAAGGCAA ATGTTTGATC TTGGGGAAAA 1440

AAA 1443

(2) INFORMATION FOR SEQ ID NO: 197:

(i) SEQUENCE CHARACTERISTICS:
(A) LENGTH: 1282 base pairs
(B) TYPE: nucleic acid
(C) STRANDEDNESS: double
(D) TOPOLOGY: linear

(xi) SEQUENCE DESCRIPTION: SEQ ID NO: 197:

GAAAAAAAAA AGTATGACCC AGTAGCTAGG CACCTGTGGC CCCGCCAAGT TGACACATAA 60

AATTAACTGT CACAGTATCA TCTTAGAAGT GAAAGAAGCC CCTTTATCCT GCAGTGCCCC 120

TCTACCACCA CCTACTGACA AAGAACATGG TGCTATCTGG CATGGGAGAA ATGTTCAGTT 180

TGCTATGGCT TGTATGTGTC CCCTCAAATT CAAGTGTTGC CAATGTGACA GCATCAAGAG 240

GTGGGGTCTT TAAGAGATCA CTAGGCCATG AGGGATTCTC TTAGGACTGG GATGAAGGCC 300

CATAATAAAA GAGGTTTCAG GGAGCATCCT GCTAGCTTGC CTTCTGTATG TGAGAACACA 360

GCAAGAAAGC CCTAGTCAAC AAGTGCCAGC TCCTTGATCT TAGACTTCCC ATCCTCCAGA 420

ACTGTGAGAA ATACATTTCT GTTCCTTACA AATTACCCAG TCTCCTGTAT TCTGTTATAG 480

CAGCACAAAA TGAAGATACC ATACCTGAAC ACCTGAACAT TCTTCACAAG GTAGTAAATG 540

CACTGCTTTA TTCTGGTCTC AGTATTGTGT GCTTAATAAG GAAATGAGAA AGGGTGGATC 600

AGGGCATAGG ATGAACAAGT TACTGCTAGA CCTCTCACAA TGCCACTAAT GGATAAGATT 660

GTATTTTCAT CATTNCTTGT CTCTTCGGAA GCTAACACCA TGCTATAATA GGCATTAAAT 720

AGATGTCTAA AAACACCTTA AGTATTTGTC TAGAAATCTG GTGCATTGTC CAGAAAGAAC 780

CAAAATTCMA AATAATTTCA AAGGGCCTAA AGCACTAKTT AATCMAAATT CATTAGTTTT 840

TAATGGTACT ACCACTCTCA AATTTAAAAT GTCATCTTAC GTTCCTCTTC CTCGCATTGG 900

ATTTATTGCT AAAACCTGGT AAACACTTTA ATCCYTTTCA ATTCCATTAC CACTGCTCTT 960

GTCCAGAATT ACTCGCAGAC TAATAGTCAC CTGACTTCTC CCCCTGCATC CCGATTTGCT 1020

GTCTAATTCT GGTTACAAAT AAGTAACTGC CAAACTAATC TTTCTAAAAA GCAAGACTGA 1080

TCTCGTCACT CCTTTGCTCA ACAATGTAAA AGCTCCCATT GTCTCCCAAA TAAAACCAGC 1140

TTTCCACTGT GTATACAATA CATCCATGAT CTGTATCCAG CATCATTTTG TATTTGCTCA 1200

CTTTATACAC CACCCCCCAT GCCACATCAA ATTAAATTAT CCTGATAAAT GCAACTGCAA 1260

AAAAAAAAAA AAAAAAACTC GA 1282

(2) INFORMATION FOR SEQ ID NO: 198:

(i) SEQUENCE CHARACTERISTICS:
(A) LENGTH: 951 base pairs
(B) TYPE: nucleic acid
(C) STRANDEDNESS: double
(D) TOPOLOGY: linear

(xi) SEQUENCE DESCRIPTION: SEQ ID NO: 198:
   ATTTCGGAAC GAGGACTGAA GTGGGAGCGG CGGCAGGGTA GAAGACAGAA GGGGGATCTA 60
   TGTGGTAACT AAAGAATGTT TCTGTTTTGT TAATTATTGT GTGTGTGTGG TTTTATTGTT 120
   TGCTTAAGAG AATCAAAAAC TGAAAAAAAT GAGAATACAG GAAATGGCTC TTGTTTATTT 180
   TTTTGCTGTG TTTACAGCTT GTTAATGCTC TACTGTCTTT GTTTCAAGAG AGATTTGTTC 240
   ACTGCCCAGC TCGTTTTGTG TCCTGAGCCC TATGCCCAGC CCACCTTATA AATCATGCCT 300
   GTTTAGATGT TTGATTTTGT TCTGTTTGCT ATTGTTATCT TAAAGGTGTA TAACTCTGAC 360
   ATGCCAGACA TCAAATTAAG CTCAAATTAA GCTCTCGTTT AAATGTTTAA ACACCTAATT 420
   TATATTCTAA TTGATCCCAG CCACTGATGC ATGTACTTTA GCTACTTCTG CTAAATAAGC 480
   ATATTAATTT TCCACATCAG GCCATCAGAT CTTGAGAACC AACAGTTATC TAGAATTCCG 540
   TGTCTACTAA TGTTTCACCT GCATGCAGCC TTCATTAATT TTGTAGCAAA ATATAAAGTG 600
   ATCATTATGT AGTTTCTGGA TTAAAAAAAT TTGTGTGTGA AGTTGCTTTG TAAAGTGCAT 660
   GTGGAATTAA TGGGACAGTG TGCCCTTTGT GTTAGATGTT AGAGCAAAAG AAAGGGCTTA 720
   TAGTGTTAGT ATTGGAGCAC TTTGAAGATA GATATTTTCA GAAAAGATGT AGGATTTAAA 780
   AGTTAAATTT TAAATTTTAG AAAAAGATAT GATGGCAATT GGAAATAGTC ACAATGAAGT 840
   TCTTCATCCA GTAGGTGTTT AACAGTGTTA TTTTGCCACT GGTAATGTGT AAACTGTGAG 900
   TGATTTACAA TAAATGATTA TGAATTCAAA AAAAAAAAAA AAAAAACTCG A 951

(2) INFORMATION FOR SEQ ID NO: 199:

(i) SEQUENCE CHARACTERISTICS:
(A) LENGTH: 1740 base pairs
(B) TYPE: nucleic acid
(C) STRANDEDNESS: double
(D) TOPOLOGY: linear

(xi) SEQUENCE DESCRIPTION: SEQ ID NO: 199:

TTATTATAAT AATGATGATG ATTCCAAGGA AAAAACCTAC AGCGAATGTT CCATTTCTAC 60

CCCGCACGCA GACACTCTCC CTAACACTGA TAACCTGAGC CCCCAGCACT GGACGGAAGA 120

ATGCTGGCGT CTCCGTGTGT ACTGGTTCAG GGTTCTGGCC CCAGCCTTGT CAGGACCCCC 180

TGGTGTCCAG AGCCCCCACC CCTCCCGCAA CAAGCAGCTG ATGCCCCAGT GATTCTCTAT 240

ACATTTTTCA CCTCGGCCAA TATGTCCAGG AAAACTGCTT ACTTCTCTTT TCTTGCCTGG 300

AGCCTTCATT GTTCACCCTT ACGTTGCAAT ATAGGAATTA ATGCTACAAA ATAAAAGTAA 360

AGCTTACCTG AAAAGTGCAT AGTTTGGGGC AATGGTATCT ACATCTCCCA CTGTGGGAAA 420

ACCAGCAAAG CATCAAAACT CTCAATTCTC CTGTTACCRA ATGCAGATCT GAATTATAAG 480

ATGTTTATGT TTGACCATTG TTTCAACAAT GGGATTTTGT TACGAATTAT CCCTTTAACT 540

GAAACCCTCA GTTTTACTGT TTACATTATT AGGAAAACAG GGATATCTTT TGAATCTAAA 600

AATTTGATGT ACAGCATGTG ATTTTTGAAG TTTACATGTA AAGTCACAGT ATAGGTGAAA 660

TAACGTTTGT CATATTTTGA GACGTATCCT GCAGCCATGT TTTTACGTGA GTGTTTTAGT 720

CAAAGTACAT GGTAGACAGT CTTTCACAAT AAAAGGAAAA GGATTTTTTT TCCTCCAAAT 780

GTACATTTAT CAACCTAATG ATTGATTTTT TTAAAAAGAG ATTTCGCCCC AGTCTGGTTT 840

ATGAAAGTTC ATTGCCCTAA ACTGTGCTGA TTGTTTTTAA TCAAGTTATA AATTTCCAAC 900

CTAGATCATG TATCTACCAA CTCTCCTGCA TTTTCCAAAA GGCATTGAGC TTAAATATTA 960

GTCTTGCTTA GAGTAGGTTA TCCACTTACA TGCTGCGCTA AAGCCATGCC TTTGAAACTC 1020

CTTGTTTAAA ACATGATATG ATTTTTGTGG GCAGTTTCAG AAAAGAAAAC AAACAAACAA 1080

AAATCGACCC TTTAATTATT ACTTGCAACT CAACAGATCT CCCTGCCGTA CTGCCTTTTC 1140

CAGGAACTTT ACTTCAGGGC TGTCCAGATT GCAGTTGTGC CCCGTGTATG TGGATCTAGT 1200

TCACAGAGTC TTTGGAAGCC AGCAGTCGTG CCCTCCGTAT ACTGTCCACT CATTTTATGT 1260

AGATTTGGTA TCCTCAGCAG CCAGTGTTAA CACCACTGTC ACGTAGTTAN CAGATTCATC 1320

TTTTATGTAT TTAAAGTAAT CCATACTATG ATTTGGTTTT TCCCTGCACC ATTAATTCTG 1380

GCATCAGATC AGTTTTTGTG TTGTGAAGTT CTACTGTGGT TTGACCCAAG ACCACAACCA 1440

TGAGACCCTG AAGTAAAGAT AAGGTACACA TACATTATTT GAGTAACTGT TTCCTTGGGG 1500

GCCAATCTGT GTATGCTTTT AGAAGTTTAC AGAATGCTTT TATTTTTGTC TATAACAAAC 1560

AGTCTGTCAT TTATTTCTGT TGATAAACCA TTTGGACAGA GTGAGGACGT TTGCCCTGTT 1620

ATCTCCTAGT GCTAACAATA CACTCCAGTC ATGAGCCGGG CTTTACAAAT AAAGCACTTT 1680

TGATGACTCA MAAAAAAAAA AAAAAAAAMC YCGGGGGGGG GCCGGTAACC CATTTNNCCC 1740

(2) INFORMATION FOR SEQ ID NO: 200:

(i) SEQUENCE CHARACTERISTICS:
(A) LENGTH: 1707 base pairs
(B) TYPE: nucleic acid
(C) STRANDEDNESS: double
(D) TOPOLOGY: linear

(xi) SEQUENCE DESCRIPTION: SEQ ID NO: 200:

GCTTATAGAA GGGAGAGGAG CGAACATGGC AGCGCGTTGG CGGTTTTGGT GTGTCTCTGT 60

GACCATGGTG GTGGCGCTGC TCATCGTTTG CGACGTTCCC TCAGCCTCTG CCCAAAGAAA 120

GAAGGAGATG GTGTTATCTG AAAAGGTTAG TCAGCTGATG GAATGGACTA ACAAAAGACC 180

TGTAATAAGA ATGAATGGAG ACAAGTTCCG TCGCCTTGTG AAAGCCCCAC CGAGAAATTA 240

CTCCGTTATC GTCATGTTCA CTGCTCTCCA ACTGCATAGA CAGTGTGTCG TTTGCAAGCA 300

AGCTGATGAA GAATTCCAGA TCCTGGCAAA CTCCTGGCGA TACTCCAGTG CATTCACCAA 360

CAGGATATTT TTTGCCATGG TGGATTTTGA TGAAGGCTCT GATGTATTTC AGATGCTAAA 420

CATGAATTCA GCTCCAACTT TCATCAACTT TCCTGCAAAA GGGAAACCCA AACGGGGTGA 480

TACATATGAG TTACAGGTGC GGGGTTTTTC AGCTGAGCAG ATTGCCCGGT GGATCGCCGA 540

CAGAACTGAT GTCAATATTA GAGTGATTAG ACCCCCAAAT TATGCTGGTC CCCTTATGTT 600

GGGATTGCTT TTGGCTGTTA TTGGTGGACT TGTGTATCTT CGAAGAGTAA TATGGAATTT 660

CTCTTTAATA AAACTGGATG GGCTTTTGCA GCTTTGTGTT TTGTGCTTGC TATGACATCT 720

GGTCAAATGT GGAACCATAT AAGAGGACCA CCATATGCCC ATAAGAATCC CCACACGGGA 780

CATGTGAATT ATATCCATGG AAGCAGTCAA GCCCAGTTTG TAGCTGAAAC ACACATTGTT 840

CTTCTGTTTA ATGGTGGAGT TACCTTAGGA ATGGTGCTTT TATGTGAAGC TGCTACCTCT 900

GACATGGATA TTGGAAAGCG AAAGATAATG TGTGTGGCTG GTATTGGACT TGTTGTATTA 960

TTCTTCAGTT GGATGCTCTC TATTTTTAGA TCTAAATATC ATGGCTACCC ATACAGCTTT 1020

CTGATGAGTT AAAAAGGTCC CAGAGATATA TAGACACTGG AGTACTGGAA ATTGAAAAAC 1080

GAAAATCGTG TGTGTTTGAA AAGAAGAATG CAACTTGTAT ATTTTGTATT ACCTCTTTTT 1140

TTCAAGTGAT TTAAATAGTT AATCATTTAA CCAAAGAAGA TGTGTAGTGC CTTAACAAGC 1200

AATCCTCTGT CAAAATCTGA GGTATTTGAA AATAATTATC CTCTTAACCT TCTCTTCCCA 1260

GTGAACTTTA TGGAACATTT AATTTAGTAC AATTAAGTAT ATTATAAAAA TTGTAAAACT 1320

ACTACTTTGT TTTAGTTAGA ACAAAGCTCA AAACTACTTT AGTTAACTTG GTCATCTGAT 1380

TTTATATTGC CTTATCCAAA GATGGGGAAA GTAAGTCCTG ACCAGGTGTT CCCACATATG 1440

CCTGTTACAG ATAACTACAT TAGGAATTCA TTCTTAGCTT CTTCATCTTT GTGTGGATGT 1500

GTATACTTTA CGCATCTTTC CTTTTGAGTA GAGAAATTAT GTGTGTCATG TGGTCTTCTG 1560

AAAATGGAAC ACCATTCTTC AGAGCACACG TCTAGCCCTC AGCAAGACAG TTGTTTCTCC 1620

TCCTCCTTGC ATATTTCCTA CTGAAATACA GTGCTGTCTA TGATTGTTTT TGTTTTGTTG 1680

TTTTTTYGAG ATCACGYTAC TGGGCTC 1707

(2) INFORMATION FOR SEQ ID NO: 201:

(i) SEQUENCE CHARACTERISTICS:
(A) LENGTH: 779 base pairs
(B) TYPE: nucleic acid
(C) STRANDEDNESS: double
(D) TOPOLOGY: linear

(xi) SEQUENCE DESCRIPTION: SEQ ID NO: 201:

CTGTCCCCAG TGTTTCCAGG TAATGACTTG GCACTCCAGA GAAAGTTTCA TRCTGTTGCG 60

TGTGGTGGCT CCAAGCCAAG CACCTGGCAT GCAGGTCAGC CCTTCCCAGC GGGCGTGGCG 120

TCGTCCTCTT CACAGATGCC ACGTTGCAGC CCCAAGGCCT CACCATTTTG CGTTTTTTAG 180

AAACCCATTT TCTTGGTCAT TTATAAAGCT GCTTTATAGA TATCTTTGAT CCTGGCATGC 240

CTTGGTTTCC TCTCCCTTCC CTCTTTCCAA TCCTGGTTTC CTAACCTCCT CTTGTAGTAA 300

TTCTCAACTC AACTCAAAGT CCCAAGAATT TGGAATGGTA GGATGCTGTG CGGGGAGCTC 360

GAGGCTGAGG CATAATCACT GCTTCGGTTC TGCTCATCAG GGGACACGCT CCCTTACTCA 420

TGGCAGCCAT GTTTGATTGT CACAGAGCCC CCCGAATACT CTGTCTATAG TGACACACTG 480

TAGGTGTCAT AAATTTTAAG AAACCTGCTT TTAAGTACTA TTTATAGGTT TTTCTGTTAT 540

ACTTGCAACC TAGTTTTAAA ATACATGAGG ATTTTATGAA AGCTTTATAC AGACATTTAT 600

AGGAAACTCA TTCTTTGATT TTAGGTGCCA TTTAAATTGA TAACACTTAC TTTATAAAAA 660

GATGCTTTTT GTCTGGATAG AGCCTTATAG TTTAAAATAT CTTCATATAT TGCCATTTGA 720

TCAAATAAAT TTCTTACTTA GAAAAAAAAA AAAAAAAAAA AAAAAAAAAA AAAACTCGA 779

(2) INFORMATION FOR SEQ ID NO: 202:

(i) SEQUENCE CHARACTERISTICS:
(A) LENGTH: 1617 base pairs
(B) TYPE: nucleic acid
(C) STRANDEDNESS: double
(D) TOPOLOGY: linear

(xi) SEQUENCE DESCRIPTION: SEQ ID NO: 202:

GGCACAGCTT TCTGTCTCTT CCTCGCTCCC TCTCTTTCTC TCCTCCCTCT GCCTTCCCAG 60

TGCATAAAGT CTCTGTCGCT CCCGGAACTT GTTGGCAATG CCTATTTTTT GGCTTTCCCC 120

CGCGTTCTCT AAACTAACTA TTTAAAGGTC TGCGGTCGCA AATGGTTTGA CTAAACGTAG 180

GATGGGACTT AAGTTGAACG GCAGATATAT TTCACTGATC CTCGCGGTGC AAATAGCGTA 240

TCTGGTGCAG GCCGTGAGAG CAGCGGGCAA GTGCGATGCG GTCTTCAAGG GCTTTTCGGA 300

CTGTTTGCTC AAGCTGGGCG ACACATGGCC AACTACCCGC AGCCTGGGAC GACAAGACGA 360

ACATCAAGAC CGTGTGCACA TACTGGGAGG ATTTCCACAG CTGCACGGTC ACAGCCCTTA 420

CGGATTGCCA GGAAGGGGCG AAAGATATGT GGGATAAACT GAGAAAAGAA TCCAAAAACC 480

TCAACATCCA AGGCAGCTTA TTCGAACTCT GCGGCAGCGG CAACGGGGCG GCGGGGTCCC 540

TGCTCCCGGC GTTCCCGGTG CTCCTGGTGT CTCTCTCGGC AGCTTTAGCG ACCTGGCTTT 600

CCTTCTGAGC GTGGGGCCAG CTCCCCCCGC GCGCCCACCC ACACTCACTC CATGCTCCCG 660

GAAATCGAGA GGAAGATCCA TTAGTTCTTT GGGGACGTTG TGATTCTCTG TGATGCTGAA 720

AACACTCATA TAGGATTGTG GGAAATCCTG ATTCTCTTTT TTATTTCGTT TGATTTCTTG 780

TGTTTTATTT GCCAAATGTT ACCAATCAGT GAGCAAGCAA GCACAGCCAA AATCGGACCT 840

CAGCTTTAGT CCGTCTTCAC ACACAAATAA GAAAACGGCA AACCCACCCC ATTTTTTAAT 900

TTTATTATTA TTAATTTTTT TTGTTGGCAA AAGAATCTCA GGAACGGCCC TGGGCACCTA 960

CTATATTAAT CATGCTAGTA ACATGAAAAA TGATGGGCTC CTCCTAATAG GAAGGCGAGG 1020

AGAGGAGAAG GCCAGGGGAA TGAATTCAAG AGAGATGTCC ACGGACGAAA CATACGGTGA 1080

ATAATTCACG CTCACGTCGT TCTTCCACAG TATCTTGTTT TGATCATTTC CACTGCACAT 1140

TTCTCCTCAA GAAAAGCGAA AGGACAGACT GTTGGCTTTG TGTTTGGAGG ATAGGAGGGA 1200

GAGAGGGAAG GGGCTGAGGA AATCTCTGGG GTAAGAGTAA AGGCTTCCAG AAGACATGCT 1260

GCTATGGTCA CTGAGGGGTT AGCTTTATCT GCTGTTGTTG ATGCATCCGT CCAAGTTCAC 1320

TGCCTTTATT TTCCCTCCTC CCTCTTGTTT TAGCTGTTAC ACACACAGTA ATACCTGAAT 1380

ATCCAACGGT ATAGATCACA AGGGGGGGAT GTTAAATGTT AATCTAAAAT ATAGCTAAAA 1440

AAAGATTTTG ACATAAAAGA GCCTTGATTT TAAAAAAAAA AGAGAGAGAG ATGTAATTTA 1500

AAAAGTTTAT TATAAATTAA ATTCAGCAAA AAAAGATTTG CTACAAAGTA TAGAGAAGTA 1560

TAAAATAAAA GTTATTGTTT GAAAAAAAAA AAAAAAAAAW CTCGACCGCA AGGGAAT 1617

(2) INFORMATION FOR SEQ ID NO: 203:

(i) SEQUENCE CHARACTERISTICS:
(A) LENGTH: 1974 base pairs
(B) TYPE: nucleic acid
(C) STRANDEDNESS: double
(D) TOPOLOGY: linear

(xi) SEQUENCE DESCRIPTION: SEQ ID NO: 203:

GAATTCGGCA CGAGGCTGAG GGAGCTGCAG CGCAGCAGAG TATCTGACGG CGCCAGGTTG 60

CGTAGGTGCG GCACGAGGAG TTTTCCCGGC AGCGAGGAGG TCCTGAGCAG CATGGCCCGG 120

AGGAGCGCCT TCCCTGCCGC CGCGCTCTGG CTCTGGAGCA TCCTCCTGTG CCTGCTGGCA 180

CTGCGGGCGG AGGCCGGGCC GCCGCAGGAG GAGAGCCTGT ACCTATGGAT CGATGCTCAC 240

CAGGCAAGAG TACTCATAGG ATTTGAAGAA GATATCCTGA TTGTTTCAGA GGGGAAAATG 300

GCACCTTTTA CACATGATTT CAGAAAAGCG CAACAGAGAA TGCCAGCTAT TCCTGTCAAT 360

ATCCATTCCA TGAATTTTAC CTGGCAAGCT GCAGGGCAGG CAGAATACTT CTATGAATTC 420

CTGTCCTTGC GCTCCCTGGA TAAAGGCATC ATGGCAGATC CAACCGTCAA TGTCCCTCTG 480

CTGGGAACAG TGCCTCACAA GGCATCAGTT GTTCAAGTTG GTTTCCCATG TCTTGGAAAA 540

CAGGATGGGG TGGCAGCATT TGAAGTGGAT GTGATTGTTA TGAATTCTGA AGGCAACACC 600

ATTCTCCAAA CACCTCAAAA TGCTATCTTC TTTAAAACAT GTCAACAAGC TGAGTGCCCA 660

GGCGGGTGCC GAAATGGAGG CTTTTGTAAT GAAAGACGCA TCTGCGAGTG TCCTGATGGG 720

TTCCACGGAC CTCACTGTGA GAAAGCCCTT TGTACCCCAC GATGTATGAA TGGTGGACTT 780

TGTGTGACTC CTGGTTTCTG CATCTGCCCA CCTGGATTCT ATGGAGTGAA CTGTGACAAA 840

GCAAACTGCT CAACCACCTG CTTTAATGGA GGGACCTGTT TCTACCCTGG AAAATGTATT 900

TSCCCTCCAG GACTAGAGGG AGAGCAGTGT GAAATCAGCA AATGCCCACA ACCCTGTCGA 960

AATGGAGGTA AATGCATTGG TAAAAGCAAA TGTAAGTKTT CCAAAGGTTA CCAGGGAGAC 1020

CTCTGTTCAA AGCCTGTCTG CGAGCCTGGC TGTGGTGCAC ATGGAACCTG CCATGAACCC 1080

AACAAATGCC AATGTCAAGA AGGTTGGCAT GGAAGACACT GCAATAAAAG GTACGAAGCC 1140

AGCCTCATAC ATGCCCTGAG GCCAGCAGGC GCCCAGCTCA GGCAGCACAC GCCTTCACTT 1200

AAAAAGGCCG AGGAGCGGCG GGATCCACCT GAATCCAATT ACATCTGGTG AACTCCGACA 1260

TCTGAAACGT TTTAAGTTAC ACCAAGTTCA TAGCCTTTGT TAACCTTTCA TGTGTTGAAT 1320

GTTCAAATAA TGTTCATTAC ACTTAAGAAT ACTGGCCTGA ATTTTATTAG CTTCATTATA 1380

AATCACTGAG CTGATATTTA CTCTTCCTTT TAAGTTTTCT AAGTACGTCT GTAGCATGAT 1440

GGTATAGATT TTCTTGTTTC AGTGCTTTGG GACAGATTTT ATATTATGTC AATTGATCAG 1500

GTTAAAATTT TCAGTGTGTA GTTGGCAGAT ATTTTCAAAA TTACAATGCA TTTATGGTGT 1560

CTGGGGGCAG GGGAACATCA GAAAGGTTAA ATTGGGCAAA AATGCGTAAG TCACAAGAAT 1620

TTGGATGGTG CAGTTAATGT TGAAGTTACA GCATTTCAGA TTTTATTGTC AGATATTTAG 1680

ATGTTTGTTA CATTTTTAAA AATTGCTCTT AATTTTTAAA CTCTCAATAC AATATATTTT 1740

GACCTTACCA TTATTCCAGA GATTCAGTAT TAAAAAAAAA AAAATTACAC TGTGGTAGTG 1800

GCATTTAAAC AATATAATAT ATTCTAAACA CAATGAAATA GGGAATATAA TGTATGAACT 1860

TTTTGCATTG GCTTGAAGCA ATATAATATA TTGTAAACAA AACACAGCTC TTACCTAATA 1920

AACATTTTAT ACTGTTTGTA TGTATAAAAT AAAGGTGCTG CTTTAGTTTT CTGA 1974

(2) INFORMATION FOR SEQ ID NO: 204:

(i) SEQUENCE CHARACTERISTICS:
(A) LENGTH: 1057 base pairs
(B) TYPE: nucleic acid
(C) STRANDEDNESS: double
(D) TOPOLOGY: linear

(xi) SEQUENCE DESCRIPTION: SEQ ID NO: 204:

CGGCCTTCCG GGGCAACCGT TCGTCCCAAC NCGGGAAAGG GTCCTGGAGN CGGGAACTAG 60

GAGCCTCGGA AGTCCAAGGG CGGAGCGCCC TTTGCTAATA AGCCAATCAG AACGTGAGAC 120

GCTCCGGTGG GNCGGTGCCG TCGAGCGCGG GGTGGAGTCT GGGTGACTTG GCTGGCGGGA 180

TCAAGTGCAG CTGCTTCAGG CTGAGGTGGC AGATAGTGAG CGCTGGTGGC GGAGTTAAAG 240

TYAAAGCAGG AGAGTAATWA TGAATAGCGC AGCGGGATTC TCACACCTAG ACCGTCGCGA 300

GCGGGTTCTC AAGTTAGGGG AGAGTTTCGA GAAGCAGCCG CGCTGCGCTT CCACACTGTG 360

CGCTATGACT TCAAACCTGC TTCTATTGAC ACTTCTTCTG AAGGATACCT TGAGKTTGGC 420

GAAGKTGAAC AGKTGACCAT WACTCTGCCM AATATAGAAA GTTGAAGGAA GCAGTAAAAT 480

TCAGTATCGT AAAGAACAAC AGCAACAACA ATGTGGAATT CASCCAGGAC TCCCAATCTT 540

GTAAAACATT CTCCATCTGA AGATAAGATG TCCCCAGCAT CTCCAATAGA TGATATCGAA 600

AGAGAACTGA AGGCAGAAGC TAGTCTAATG GACCAGATGA GTAGTTGTGA TAGTTCATCA 660

GATTCCAAAA GTTCATCATC TTCAAGTAGT GAGGATAGTT CTAGTGACTC AGAAGATGAA 720

GATTGCAAAT CCTCTACTTC TGATACAGGG NAATTGTGTC TCAGGACATC CTACCATGAC 780

ACAGTACAGG ATTCCTGATA TAGATGCCAG TCATAATAGA TTTCGAGACA ACAGTGGCCT 840

TCTGATGAAT ACTTTAAGAA ATGATTTGCA GCTGAGTGAA TCAGGAAGTG ACAGTGATGA 900

CTGAAGAAAT ATTTAGCTAT AAATAAAAAT TTATACAGCA TGTATAATTT ATTTTGTATT 960

AACAATAAAA ATTCCTAAGA CTGAGGGAAA TATGTCTTAA CTTTTGATGA TAAAAGAAAT 1020

TAAATTTGAT TCAGAAAAAA AAAAAAAAAA AACTCGA 1057

(2) INFORMATION FOR SEQ ID NO: 205:

(i) SEQUENCE CHARACTERISTICS:
(A) LENGTH: 721 base pairs
(B) TYPE: nucleic acid
(C) STRANDEDNESS: double
(D) TOPOLOGY: linear

(xi) SEQUENCE DESCRIPTION: SEQ ID NO: 205:

GAATTCGGCA CGAGTCATCC CTCTCCCTCT TTCACTCCCT TACTCTTACT CTGTTTTTTG 60

TGCTCCAGAC AGACAGACCC TACCTCTTTT GCTTCTTTTT TGTTTGTTTG TTTTGAGATG 120

GAGTGTCGCT CTTGTTGCCC AGGCTGGAGT GCAGTGGCGC AATCTCGGCT CACCACAACC 180

TCTGCCTCCC GGGTTCAAGC AATTCTCCTG CCTCAGCCTC CCGAGAAGCT GGGGATTACA 240

GGCATGCGCC ACCACACCCA GCTNAATTTT ATATTTTTAG TAGAGATGGT GTTTCTCCAT 300

GTTGGTCAGG CTGGCCTCAA ACTCCCAACC TCAGGTGATN CCGCCTGCTT TGGCCTCCCC 360

AAAGTGCTGG GATTACAGGC GTGAGCCACT GCGCCCAGCC TCTTTTGCTC CTTTATACTC 420

ATTAACTCAC GCCTGTAATC CCTGTTTTGG GAGGCCAAAG TGAGAAGGTT GCTTGAGGCC 480

AAGAGTTTGA GACTAGCCTG GGCAACACAG CAAGATGCCA TCTTTATAAT AAAAATAAAA 540

ATAAAAATCA ATTAGCTGGG CATGGTGGAA CGCACCTGTA GTCCCAGCCA ATTGAGAGGC 600

TGAAGTGGGA GGATCATTGA GCCCAGGAGT TGAGGTTGCA GTGAGCCATG ATCATGTCAC 660

TACACTCAGC CTGGGCAATA GAGGGACATG TTGTCTCTAA AAAAAAAAAA AAAAAACTCG 720

A 721

(2) INFORMATION FOR SEQ ID NO: 206:

(i) SEQUENCE CHARACTERISTICS:
(A) LENGTH: 2465 base pairs
(B) TYPE: nucleic acid
(C) STRANDEDNESS: double
(D) TOPOLOGY: linear

(xi) SEQUENCE DESCRIPTION: SEQ ID NO: 206:

CCACCATTTA TCCAACTGAA GAGGAGTTAC AGGCAGTTCA GAAAATTGTT TCTATTACTG 60

AACGTGCTTT AAAACTCGTT TCAGACAGTT TGTCTGAACA TGAGAAGAAC AAGAACAAAG 120

AGGGAGATGA TAAGAAAGAG GGAGGTAAAG ACAGAGCTTT GAAAGGAGTT TTGCGAGTGG 180

GAGTATTGGC AAAAGGATTA CTTCTCCGAG GAGATAGAAA TGTCAACCTT GTTTTGCTGT 240

GCTCAGAGAA ACCTTCAAAG ACATTATTAA GCCGTATTGC AGAAAACCTA CCCAAACAGC 300

TTGCTGTTAT AAGCCCTGAG AAGTATGACA TAAAATGTGC TGTATCTGAA GCGGCAATAA 360

TTTTGAATTC ATGTGTGGAA CCCAAAATGC AAGTCACTAT CACACTGACA TCTCCAATTA 420

TTCGAGAAGA GAACATGAGG GAAGGAGATG TAACCTCGGG TATGGTGAAA GACCCACCGG 480

ACGTCTTGGA CAGGCAAAAA TGCCTTGACG CTCTGGCTGC TCTACGCCAC GCTAAGTGGT 540

TCCAGGCTAG AGCTAATGGT CTGCAGTCCT GTGTGATTAT CATACGCATT CTTCGAGACC 600

TCTGTCAGCG AGTTCCAACT TGGTCTGATT TTCCAAGCTG GGCTATGGAG TTACTAGTAG 660

AGAAAGCAAT CAGCAGTGCT TCTAGCCCTC AGAGCCCTGG GGATGCACTG AGAAGAGTTT 720

TTGAATGCAT TTCTTCAGGG ATTATTCTTA AAGGTAGTCC TGGACTTCTG GATCCTTGTG 780

AAAAGGATCC CTTTGATACC TTGGCAACAA TGACTGACCA GCAGCGTGAA GACATCACAT 840

CCAGTGCACA GTTTGCATTG AGACTCCTTG CATTCCGCCA GATACACAAA GTTCTAGGCA 900

TGGATCCATT ACCGCAAATG AGCCAACGTT TTAACATCCA CAACAACAGG AAACGAAGAA 960

GAGATAGTGA TGGAGTTGAT GGATTTGAAG CTGAGGGGAA AAAAGACAAA AAAGATTATG 1020

ATAACTTTTA AAAAGTGTCT GTAAATCTTC AGTGTTAAAA AAACAGATGC CCATTTGTTG 1080

GCTGTTTTTC ATTCATAATA ATGTCTACAT TGAAAAATTT ATCAAGAATT TAAAGGATTT 1140

CATGGAAGAA CCAAGTTTTT CTATGATATT AAAAAATGTA CAGTGTTAGG TATTATTTGA 1200

ATGGAAAGAC ACCCAAAAAA AAAAATGTGC TCCGACTAGG GGGAAAACAG TAGTTCCGAT 1260

TTTTTCCCAT TATTTTTATT TTATTTTCTG GTTGCCCTAG CTTCCCCCCC TATTTTTGTG 1320

TCTTTTATTA ACTAGTGCAT TGTCTTATTA AATCTTCACT GTATTTAATG CAGGATGTGT 1380

GCTTCAGTTG CTCTGTGTAT TTTGATATTT TAATTTAGAG GTTTTGTTTG CTTTTTGACA 1440

CTAGTTGTAA GTTACTTTGT TATAGATGGT ATCCTTTACC CCTTCTTAAT ATTTTACAGC 1500

AGTACGTTTT TTTGTAACGT GAGACTGCAG AGTTTGTTTT TCTATATGTG AAGGATTACA 1560

ACACAAAAAG TTATCCTGCC ATTCGAGTGC TCAGAACTGA ATGTTTCTGC AGATCTTGTG 1620

GCATTTGTCT CTAGTGTGAT ATATAAAGGT GTAATTAAGA CAGAGTTCTG TTAATCTAAT 1680

CAAGTTTGCT GTTAGTTGTG CATTAGCAGT ATAAAAGCTA ATATATACTA TATGGTCTTG 1740

CAACAGTTTT AAAGCCTCTG CATAATTGAT AATAAAAATG CATGACATTC TTGTTTTTAA 1800

TAGACTTTTA AAATCATAAT TTTAGGTTTA ACACGTAGAT CTTTGTACAG TTGACTTTTT 1860

GACATAGCAA GGCCAAAAAT AACTTTCTGA ATATTTTTTT CTTGTGTATA AGTGGAAAGG 1920

GCATTTTTCA CATATAAGTG GGCTAACCAA TATTTTCAAA AGAACTTCAT CATTGTACAA 1980

CTAACAACAG TAACTAGCCC TTAATTATGG TGACAGTTCC TTATTGGTGT GTGTGAGATT 2040

ACTCTAGCAA CTATTACAGT ATAACACAGA TGATCTTCTC CACACACCCC ATCACCCAGA 2100

TAATTTACAG TTCTGTTAAC AGTGAGGTTG ATAAAGTATT ACTGATAAAA AATTATCTAA 2160

GGAAAAAAAC AGAAAATTAT TTGGTGTGGC CATCTTACCT GCTTATGTCT CCTACACAAA 2220

GCTAAATATT CTAGCAGTGA TGTAATGAAA AATTACATCT TACTGTTGAT ATATGTATGC 2280

TCTGGTACAC AGATGTCATT TTGTTGTCAC AGCACTACAG TGAAATACAC AAAAAATGAA 2340

ATTCATATAA TGACTTAAAT GTATTATATG TTAGAATTGA CAACATAAAC TACTTTTGCT 2400

TTGAAATGAT GTATGCTTCA GTAAAATCAT ATTCAAATTT AAAAAAAAAA AAAAAAAAAA 2460

CTCGA 2465

(2) INFORMATION FOR SEQ ID NO: 207:

(i) SEQUENCE CHARACTERISTICS:
(A) LENGTH: 1480 base pairs
(B) TYPE: nucleic acid
(C) STRANDEDNESS: double
(D) TOPOLOGY: linear

(xi) SEQUENCE DESCRIPTION: SEQ ID NO: 207:

GAATTCGGCA CGAGCTCAAG CTGGCAGGTG GTCGGGGGAG CGGCCGGAGA GGAGCTGCCG 60

GGAGTTCGTG CCCTGCAGGA CATGACACCA GTGGCATATC ACGGCCATGG GGTCTCAGCA 120

TTCCGCTGCT GCTCGCCCCT CCTCCTGCAG GCGAAAGCAA GAAGATGACA GGGACGGTTT 180

GCTGGCTGAA CGAGAGCAGG AAGAAGCCAT TGCTCAGTTC CCATATGTGG AATTCACCGG 240

GAGAGATAGC ATCACCTGTC TCACGTGCCA GGGGACAGGC TACATTCCAA CAGAGCAAGT 300

AAATGAGTTG GTGGCTTTGA TCCCACACAG TGATCAGAGA TTGCGCCCTC AGCGAACTAA 360

GCAATATGTC CTCCTGTCCA TCCTGCTTTG TCTCCTGGCA TCTGGTTTGG TGGTTTTCTT 420

CCTGTTTCCG CATTCAGTCC TTGTGGATGA TGACGGCATC AAAGTGGTGA AAGTCACATT 480

TAATAAGCAA GACTCCCTTG TAATTCTCAC CATCATGGCC ACCCTGAAAA TCAGGAACTC 540

CAACTTCTAC ACGGTGGCAG TGACCAGCCT GTCCAGCCAG ATTCAGTACA TGAACACAGT 600

GGTGAATTTT ACCGGGAAGG CCGAGATGGG AGGACCGTTT TCCTATGTGT ACTTCTTCTG 660

CACGGTACCT GAGATCCTGG TGCACAACAT AGTGATCTTC ATGCGAACTT CAGTGAAGAT 720

TTCATACATT GGCCTCATGA CCCAGAGCTC CTTGGAGACA CATCACTATG TGGATTGTGG 780

AGGAAATTCC ACAGCTATTT AACAACTGCT ATTGGTTCTT CCACACAGCG CCTGTAGAAG 840

AGAGCACAGC ATATGTTCCC AAGGCCTGAG TTCTGGACCT ACCCCCACGT GGTGTAAGCA 900

GAGGAGGAAT TGGTTCACTT AACTCCCAGC AAACATCCTC CTGCCACTTA GGAGGAAACA 960

CCTCCCTATG GTACCATTTA TGTTTCTCAG AACCAGCAGA ATCAGTGCCT AGCCTGTGCC 1020

CAGCAAATAG TTGGCACTCA ATAAAGATTT GCAGAATTTA ATACAGATCT TTTCAGCTGT 1080

TCTTAGGGCA TTATAAATGG AAATCATAAC GTGGTTCTAG GTTATCAAAC CATGGAGTGA 1140

TGTGGAGCTA GGATTGTGAG TGACCTGCAG GCCATTATCA GTGCCTCATC TGTGCAGAAG 1200

TCGCAGCAGA GAGGGACCAT CCAAATACCT AAGAGAAAAC AGACCTAGTC AGGATATGAA 1260

TTTGTTTCAG CTGTTCCCAA AGGCCTGGGA GCTTTTTGAA AAGAAAGAAA AAAGTGTGTT 1320

GGCTTTTTTT TTTTTTAGAA AGTTAGAATT GTTTTTACCA AGAGTCTATG TGGGGCTTGA 1380

TTCACCCTTC ATCCATTGGC TGGAACATGG ATTGGGGATT TGATAGAAAA ATAAACCCTG 1440

CTTTTGATTC AAAAAAAAAA AAAAAAWAAA AAAAACTCGA 1480

(2) INFORMATION FOR SEQ ID NO: 208:

(i) SEQUENCE CHARACTERISTICS:
(A) LENGTH: 872 base pairs
(B) TYPE: nucleic acid
(C) STRANDEDNESS: double
(D) TOPOLOGY: linear

(xi) SEQUENCE DESCRIPTION: SEQ ID NO: 208:

CAGTATTTCC CTCAGTACTG TAAGCAAAAG TGGTATGTTT TTCTTTCTTT ATGTCTACTC 60

TGTCCTCTGT GGCCTTCTGG TGTACCCCTC TCTTCCTAGC CATTCAGTCT CTCTAGTCAC 120

CTCCCTAGTA GCTAGTGCTC TCTAAGTTTT TATTTAATTA GAACAACTCC ATTTCCATTT 180

CAAGGTAGGT CAATGGGGGG AAAAGCCTCA TGATTTAAAC TGAAGTTAAC AACACAGCTT 240

TTAAAATGAA AACTCATACT CCAACTTCTA AAGTATATTT GAGCTGATTT GTTTCCAAAA 300

CAAAGATATG CTGTACCTAA AACTGCTAAA ACAAAAATAT AAAGACAAGG ACTAGGTGAT 360

TAAGGGGAGA GAAAAATCAT YTCTTTTCCA GGAAACCTTT GCTAAAATAA GCAAAACTTG 420

ANTCTATGCT TCATGGAAAC TGACACAAAG AAAAGAAACT GATGGATTGC ACAGGCCTTG 480

TTATAGAAAT AGATCTATAA AAAGATCTGT CCACAGGAAA TATACACCTT CTCCTGGTTC 540

TGAACTTCAA TGGGGATTTG TCACCTAGGT CTCCATCTAT AGGAATACCT TCACATACCT 600

ATCTATTCAT GCACATATTC TGAAAACAGG TACATACAAA ATTACAACAA AGGAAAAAAA 660

TTCTATTGAA CACTTAAAAA TAGAAACAGG CCAGGCACGG TGGCTCATGC TGTAATCCCA 720

ACAATTTGGG AGGCTGAGGC TGGTGGATCA CCTGAGGTCA GGAGTGTGAG ACCAGCTTGG 780

CCAACATGGT GAAACCCCGT CACTACTAAA AATACAAAAA AAATTAGCCT GTGTGGTGGC 840

ACACTCNTAC AATCCNGGCT GACTCGGGAA AN 872

(2) INFORMATION FOR SEQ ID NO: 209:

(i) SEQUENCE CHARACTERISTICS:
(A) LENGTH: 1779 base pairs
(B) TYPE: nucleic acid
(C) STRANDEDNESS: double
(D) TOPOLOGY: linear

(xi) SEQUENCE DESCRIPTION: SEQ ID NO: 209:

AATTGCCAAG ACTGCACAAA ATTACAGTGC TAATGTATAT GGTTGCAGTT CACATAAAGA 60

CAAAAGCATC TGTTATGAAA TGAGTAGTAA TATTGGGTGG TTGATTTGTT CTTAGCAGAC 120

TTGGCTTCAT WTTGGTCTTG AGATAAAATG GCCAGCATAA ATGCTGTTTA TATTCACGTT 180

TTCCTAGGTG TGTGTGTGCA GGCCACAGCA GCATGCCCTT GGTGTAGTCA GTGCCGAAAS 240

GGGTCTGTTC CTTCTTGAGC CTGCCTGCAG GGATGGTCTC CTTTTAAAGC AGGTTGTGTG 300

CAGCATTCAG TACACTGAAG GTAAGCTAAA CCATCAACAT CTCTGGTGTT TTAAGATGTT 360

ATTTTATTGG AACAACTGAC AAATGAGGGA TGTTAGCTTT GTGGCAGAAT TCCCTGCATG 420

TGTGATAACT GATCTTGTTT TATTTTTTGG CATTGCAACT GTGGCATAGT TACAATTTCT 480

GTTTGKTCAT CACATTTAAA ATTGGRAGAG AACGCGCTTG AKGGATAGAG CGCCTTCAGK 540

GTACTGTTTC TTATTAACTT TACTTTTTTT AAATCAACTT GCTATAGACT TTATATACAT 600

TTTGTTAAAT ATAGTTCCTA GTGACATAGA AACGATGCGT AGTTTTCATT TACTAATTAC 660

AAATGTTGAG GCCTAATTCT GAAAGTCCTC ATATTTAAAG GCTAGACAAC GTAATGAAAT 720

TTTTAACTAT TTGTATGTCA TTTTGAAAGT GTACTGCTTT ATGGTAAAAG TGTTTTTCAT 780

TTGTTCATTG TTTTCATTAT TTGTGATCAT GTTGTCTTTC AATACAGGCA TAAACCTTCC 840

ACTCTTGAAC AAAGCAGCTG CTTTTTAAAA GCGGTAATTG CTTCTTTACC TTTTATTTCT 900

TTTGTAAATG AAGCTTTTCT TTAAGAATGT GACTTTAAAG TGTTGTCTAT TGCATAAAAC 960

AGTTGACACT CACTTATTGT AAAGTGAAGA TTGTTCTACT GCATGTGAAG TGGACCATGC 1020

AGATTTCTGT ATGTTCTCAG TATGCATCAC TAGATAATAA AGTCTTTTGT GAACAAGGCA 1080

TTTGTAGCCA TTTTTAAAAG TTTTTGTCTT CAGTGCTGGT AAGTCAGGTA AACCATAAAT 1140

AGTTAAAAGC AACCTTTTGT TTTTTTCCTG AAAGTTTTTA ATTGAAAGTA TTATTAGTTA 1200

AAGATGTAAA CCTAGCCAAA ATTACCAGTT TATTAATAAT TAGGATCCTA ATTATTTCAA 1260

AAAATCCTAC AAATATTGTC AGCTTTCAGT GTAGTGAGAT TATTCCTGTA GGTTATGGGG 1320

TATAATTCAG GATTTAACTA ATGTTTCTGC TATTTTCTCA CTTTTCCTTT TGATGGTGCG 1380

GAAAGAGAAA AAGGAAAACG GGGCACAGGC CATTCGACGC CTTCTCCAAG GGGTCTGATT 1440

TGCTGAGACA CCAGCTTCAC CTTCTTAACA AGGCACCTAA TTACAACAAG CATGCACATT 1500

TTGGTGCATT CAAGAATGGA AAATCAGAAT AGCAGCATTG ATTCTTCTGG TGCAGCTCAG 1560

TGGAAGATGA TGACAACCAG AAGACATGAG CTAAGGGTAA GGGACTGTTC TGAAGAACCT 1620

TTCCATTTAG TGATCAAGAT ATGGAAGCTG ATTTCTGAAA ATGCTCAGTG TGTACTCTAA 1680

TTATTTATGG TACCATTTGA ATTGTAACTT GCATTTTAGC AGTGCATGTT TCTAATTGAC 1740

TTACTGGGAA ACTGAATAAA ATATGCCTCT TATTATCAA 1779

(2) INFORMATION FOR SEQ ID NO: 210:

(i) SEQUENCE CHARACTERISTICS:
(A) LENGTH: 2110 base pairs
(B) TYPE: nucleic acid
(C) STRANDEDNESS: double
(D) TOPOLOGY: linear

(xi) SEQUENCE DESCRIPTION: SEQ ID NO: 210:

GCGGCCGCTG CAGCCCGGAG CTGAGCTAGC CGTCCGAGCC GAGCCGTCCG AGCCGGGGAA 60

GCCGGCGCGT GCTGCCGCTC GTGGCGGCCA GAGGAGAGGA GAGGCAGCAG CATGGCGAGT 120

GTCCTGTCCC GACGCCTTGG AAAGCGGTCC CTCCTGGGAG CCCGGGTGTT GGGACCCAGT 180

GCCTCGGAGG GGCCTCGGCT GCCCCACCCT CGGAGCCACT GCTAGAAGGG GCCGCTCCCC 240

AGCCTTTCAC CACCTCTGAT GACACCCCCT GCCAGGAGCA GCCCAAGGAA GTCCTTAAGG 300

CTCCCAGCAC CTCGGGCCTT CAGCAGGTGG CCTTTMAGCC TGGGCAGAAG GTTTATGTGT 360

GGTACGGGGG TCAAGAGTGC ACAGGACTGG TGGWGCAGCA CAGCTGGATG GAGGGTCAGG 420

TGACCGTCTG GCTGCTGGAG CAGAAGCTGC AGGTCTGCTG CAGGGTGGAG GAGGTGTGGC 480

TGGCAGAGCT GCAGGGCCCC TGTCCCCAGG CACCACCCCT GGAGCCCGGA GCCCAGGCCC 540

TGGCCTACAG GCCCGTCTCC AGGAACATCG ATGTCCCAAA GAGGAAGTCG GACGCATGGA 600

AATGGATGAG ATGATGGCGG CCATGGTGCT GACGTCCCTG TCCTGCAGCC CTGTTGTACA 660

GAGTCCTCCC GGGACCGAGG CCAACTTCTC TGCTTCCCGT GCGGCCTGCG ACCCATGGAA 720

GGAGAGTGGT GACATCTCGG ACAGCGGCAN CAGCACTACC AGCGGTCACT GGAGTGGGAG 780

CAGTGGTGTC TCCACCCCCT CGCCCCCCCA CCCCCAGGCC AGCCCCAAGT ATTTGGGGGA 840

TGCTTTTGGT TCTCCCCAAA CTGATCATGG CTTTGAGACC GATCCTGACC CTTTCCTGCT 900

GGACGAACCA GCTCCACGAA AAAGAAAGAA CTCTGTGAAG GTGATGTACA AGTGCCTGTG 960

GCCAAACTGT GGCAAAGTTC TGCGCTCCAT TGTGGGCATC AAACGACACG TCAAAGCCCT 1020

CCATCTGGGG GACACAGTGG ACTCTGATCA GTTCAAGCGG GAGGAGGATT TCTACTACAC 1080

AGAGGTGCAG CTGAAGGAGG AATCTGCTGC TGCTGCTGCT GCTGCTGCCG CAGACCCCCA 1140

GTCCCTGGGA CTCCCACCTC CGAGCCAGCT CCCACCCCCA GCATGACTGG CCTGCCTCTG 1200

TCTGCTCTTC CACCACCTCT GCACAAAGCC CAGTCCTCCG GCCCAGAACA TCCTGGCCCG 1260

GAGTCCTCCC TGCCCTCAGG GGCTCTCAGC AAGTCAGCTC CTGGGTCCTT CTGGCACATT 1320

CAGGCAGATC ATGCATACCA GGCTCTGCCA TCCTTCCAGA TCCCAGTCTC ACCACACATC 1380

TACACCAGTG TCAGCTGGGC TGCTGCCCCC TCCGCCGCCT GCTCTCTMTC TCCGGTCCGG 1440

AGCCGGTCGC TAAGCTTCAG CGAAGCCCCA GCAGCCAGCA CCTGCGATGA AATCTCATCT 1500

GATCGTCACT TCTCCACCCC GGGCCCAGAG TGGTGCCAGG AAAGCCCGAG GGGAGGCTAA 1560

GAAGTGCCGC AAGTGTATGG CATCGAGCAC CGGGACCAGT GGTGCACGGC CTGCCGGTGG 1620

AAGAAGGCCT GCCAGCGCTT TCTGGACTGA GCTGTGCTGC AGGTTCTACT CTGTTCCTGG 1680

CCCTGCCGGC AGCCACTGAC AAGAGGCCAG TGTGTCACCA GCCCTCAGCA GAAACCGAAA 1740

GAGAAAGAAC GGAAACACGG AGTTTGGGCT CTGTTGGCTA AGGTGTAACA CTTAAAGCAA 1800

TTTTCTCCCA TTGTGCGAAC ATTTTATTTT TTAAAAAAAA GAAACAAAAA TATTTTTCCC 1860

CCTAAAATAG GAGAGAGCCA AAACTGACCA AGGCTATTCA GCAGTGAACC AGTGACCAAA 1920

GAATTAATTA CCCTCCGTTT CCCACATCCC CACTCTCTAG GGGATTAGCT TGTGCGTGTC 1980

AAAAGAAGGA ACAGCTCGTT CTGCTTCCTG CTGAGTCGGT GAATTCTTTG CTTTCTAAAC 2040

TCTTCCAGAA AGGACTGTGA GCAAGATGAA TTTACTTTTC TTAAAAAAAA AAAAAAAAAA 2100

AAAAACTCGA 2110

(2) INFORMATION FOR SEQ ID NO: 211:

(i) SEQUENCE CHARACTERISTICS:
(A) LENGTH: 938 base pairs
(B) TYPE: nucleic acid
(C) STRANDEDNESS: double
(D) TOPOLOGY: linear

(xi) SEQUENCE DESCRIPTION: SEQ ID NO: 211:

GGCACAGGAA AAAAAAGAAA AAAGAAAAAA GAAAAAAGTT TTTGTACCCA CAGATTAGCA 60

TTTTCTTGAT GTTTGAAAAA AGTTTAAGCT ATGTCCTAAT TTAAAAATGA GCACAAACTA 120

CTTAACAGAT GTCTGTTCCC TCTTCTCTTA CTTAAATTAT CTTTATTTTC ACCATCACCT 180

CCCAGTGCCG AACACCTGAN CTCTGTGTTT TGTGGTTGGA TCCTGGGTTG CCAAGTTCCT 240

ATTTGGTCAG TCCCTGGCCT GTGGGGCGGT CTCAGGAAGT GGCATGCTCT TCAMGRAGGA 300

TCGTTCATYT CCAGTATAAC CAWTTTGTTA ATAATAGTTG ATAATTCCCA GCTTTTACCA 360

GATGARTTTT GACTTATTTT TCCTCCTTTG ACCTGTTCAA AGCTAACATA TCTCGGTCAG 420

TTCGGAGAGG GTGGGGGATT TGAGAATGTG AGGAGGAGTG GGGTTAGAAT GGGTTTGCCT 480

ATCTGGGCAA GGAAAGAGTT CCTAGTCGAT TGGGCACAAT GACAAAATGA TTCCATGGAT 540

AGAATCGTCC CATGTTGCTG GAACACCTCA CGTGTTGTGA ACGCCTTAAA TTCCTGCCAT 600

CCCTTCTCTG ATTCCCCACC TCCCTGTAGT TTCCACAGGA TTTATCTCTC TGTACCCCCG 660

TCCTCCAACT CTACTCTGTC AGCCTCTCCT CCATCCCTTA CTTCCCTTCT AAATTCCAGG 720

AGATGACCTC ACTTTGCAAA GCAAATTGGA GCCACCAAAT TGTAGCTCTC CTCGGTGGAA 780

ACTGCATCTG TGCTCATCCC TGCACCTTCT TGCAGAAAGC CGCCCCCTCA GGCCAAGATG 840

AGTGCCTGGC CCCCATGGGA GACTCAGACA CTTTGACCCC TTGTGACTTC AGCATCTCCC 900

TCTTTAAAGA TTCTCTCCCA ACATTCAGTC GTGCTCGA 938

(2) INFORMATION FOR SEQ ID NO: 212:

(i) SEQUENCE CHARACTERISTICS:
(A) LENGTH: 1551 base pairs
(B) TYPE: nucleic acid
(C) STRANDEDNESS: double

(D) TOPOLOGY: linear

(xi) SEQUENCE DESCRIPTION: SEQ ID NO: 212:

AGGCTGGACT AAGCATAGAG AACCAGGAGA GAAAGAAAGA TTTAAGAGAC TGAGTAATAT 60

TTTTTGACAG ATCATTTAAG AAACTGAGTA ATTTTTTTTT TCTCCAAAAG GGCATGGGTT 120

TTTTTTTTGT TTTGTTTTTT CTCTATTTGG CACTTTCTAG GGATTGGTCT ATAAATTTTT 180

TGAAAGATCA TAGGATAAAT TTCTTTGTAG CAACTTCCTA TTTTAGTGTT TATGTTAGGG 240

GARCCCCARG TGTCCCTGCT GATACGCCAT TAGGGCCACT TCTCAGCCTC TGGCTACATC 300

ATAATGCTTT TTTTTCTATC TTGCCAAAGT TTCCMGAAAA TTKAKGTTTT CTAATTTTAA 360

AAAAATTGGT TGTGGAGATG GGATGGGACC TCTTTATAAG CCCTGAAAAT AAGTGATTTN 420

TTTTAAGTGC TATTCTGCTA TAAACCTGAT TCTCACTTTT TTCTGTAGAC AACAGTTTTT 480

TATAATATAT CTATTTTGTG TGGACATTAT TTCCTTTTAA CCAATACTGA AATTCCATAG 540

TGTAWACTTT CTCCACATTT TCTTTGATTA ATACTTYCTT AAAATAGACA CTTGGATTGG 600

CACCAGCTGT CACCAATAAA GCTGCCCTGA ACATTGTCAA TCAATCCTGT TAACCAATTT 660

GAGAATTTTT CTGGAATGCT TAGTTAGGGA TGAAATTGCT GGGTTATAGG TATGAGTATG 720

CTTGATATAC TTTTCTCCAG AATGTCTACA CCTGTGTGTA CACCACATCT CCAGAGATAG 780

GGGAATCTTA TGTCCCTGCT AACTGCTCTC GTTATTTAAT TTTCTGACAT TTGCCGCCGC 840

CGCCGCCCCC TGCCCCCAAC ACACACATGG TATAAAGTGG TAGTTTCTTG TTTTAAATTG 900

AACTTTTGAA TGATTTGAAT TTGGGCATTT CTTTGTATCC TGAGTTATTT TGGTTTCCCG 960

TTATGTGAAT ATCCTTTTCC TATGCTTTAA CTACTTTTCT AATTTGTCCC TTTTTTNGGT 1020

TATCAAATTC CAGGCCATTG TCTATTCCAT CGTCACTTTT GGGTATTGGA AACATCTTTC 1080

CATTCTGTAG CCTGTCTGTT GAACATAAAT CTTGATTTTT ATGTAATCAG ATTTTTCTCC 1140

TTACGGTTAT GTTCTTGGAA TTTTATTTAA GAAATCTTTT TCTATCCTGA GACCACAAAA 1200

ATGTCCCCAC CATTTTCTTC TGTTTCATAG TTTTGCCTTG TATGTTTAAT CCTTTAAGGC 1260

ATGTGTAGTT CATTTTATAT GGTGTGAAAT AGTTCTTATT CATTTATTCA ACACATATTG 1320

GTGGAGTGCC TGCTGATGGT AGTACTCTTC AGAGTACTTT GTATATATTT GTGAACACAT 1380

ATTCTTGCCC TGGAAGCTTA TGTTGTCNTT CAAGGTAGAT CCNTACTCGG TTTCCACCTG 1440

TTTTCTTCAG CCCTCAGGAT GAATTCCACA ATTTTACACA TAGCACCAGT TAAGGAATAG 1500

GCTTTATTGG AGAAAAGGAA GGCTTATTAG ACCAGCATCA GCAAAAAAAA A 1551

(2) INFORMATION FOR SEQ ID NO: 213:

(i) SEQUENCE CHARACTERISTICS:
(A) LENGTH: 997 base pairs
(B) TYPE: nucleic acid
(C) STRANDEDNESS: double
(D) TOPOLOGY: linear

(xi) SEQUENCE DESCRIPTION: SEQ ID NO: 213:

AGAGAGTCCT CAACAGAACC TAATCATGCT GGCACCCTAA TCTCATACTT CTAGCCTCCA 60

GAACTGAGAG AACATAAACT CCAGTTGTTT AAGCTACCCA GTCTATGGTA TTTGTTATTA 120

TAGCCCAAGC TAAGTCAGGT GGAAAGGCAG AAATATTTTG AGAAGATTCA TTTCTACAAA 180

AACAGAGTTG TTCTAAATGA AATGGCCAGA TATTTCATCT TCTTCATACT AGTATTTATG 240

AAAGTTTCAT TAAACACCAC TTGGCCAGCA CCCAGGCCTG CCACTCTCAG AACGGCAAAC 300

AAAAGCAAAT GATTTGAGGA ACAAAAGAGT GGACACAGAG CTTCTCAGAA GATGGCTCCA 360

TCTTCTGAGA TGATCTTCTG AGATCATCAA TTTTCTGCAC CTGATGTCCT ACTCCAATTG 420

TAGTAGATAA GAGCAAAGAC ACTTCCTGAT CCTGTGGAAA ATGCTGGAGC CCTGCTGATG 480

GAGAGGCTGA CACTGGGACC AACAGAAGGC CGGACATTTA TTTGCTGCAG CCCTTCTGCA 540

CCTGGGCCCT CTTCAGGCCT TGTACCTTGC ACTCCCCATG CCACTGTAGC ACCTGGTAAG 600

CTGAAGTTAG GTATTTGAAG AGATAATTTG CCCCCAACAA AGAATTACTT AAAAGAAAAA 660

GGAAACCACT AAATTCCACT TGACAAACCA GTTTGTTCAG TTTTGACTTT TGCAAATTTG 720

AAACTTTCTC TTTGGCACCA TATGATTCTG TTACATTAGG GCTCATCAAT GCTAAGATAC 780

ACAGCTAGGT CTACCAGCTG CCAGTGGTCA AGAATGAAAG AACCTCTCAG AGAGAGATCA 840

GTTTCTAATA ACCTAACAGT TTTCCTTGGS TATTACMAAA AAAAAAAAAA TTAGAATAAA 900

ATGTCAGTGC CATGCAGGCA AGTACAGATA TGGAAATGAA AGCTCTGTCT ACAACTGCAA 960

GATTTGTTTG TTAATAAAAT TGATTGGGAT CACTCGA 997

(2) INFORMATION FOR SEQ ID NO: 214:

(i) SEQUENCE CHARACTERISTICS:
(A) LENGTH: 1496 base pairs
(B) TYPE: nucleic acid
(C) STRANDEDNESS: double
(D) TOPOLOGY: linear

(xi) SEQUENCE DESCRIPTION: SEQ ID NO: 214:

GAATTCGGCA CGAGTGACCA CAGATATCTT TGGCTTTCAG CCTCACCACA ATGCTGTCCA 60

CTATGTTTTT TTTAATCGAT TGACATCTCA TGAATCCACA AATTTAGCCG CTTTTCCATC 120

TTTTCCATCT TTGTCATAGC TTCATCACGC ACGATGGAGG TCACTTCAGC ACTATCCGGA 180

GCGGCCTCAC GGACAGATCR GTGAATTTCC TTTTCCTTTT TCTTGATGTA CCGGATTGTC 240

GACTCGTTAA CATTGAGCTC ATGGCCAACA GCACTGTAAC TCATGCCTGA TTGGAGCTTA 300

TCCAACACGC GGAMTTTCTC CGTAAGGSAM ATCAMGGTCT TCTTTCGCTT AGGAACACTG 360

GGCARARCTT AARCACTACG CTTGGGGGCC ATTTTAGAAA GCAAAACCAC CCACAAAAAG 420

CAGAAAAAAA AGTGTCAGTA AACAGACTGN NGANAGGACT CTTTGTTTAC AGCACAGGAG 480

CTGCGACTAG AAGGCGGCGC TTCTCCCCAG TTCAAACTTC AGCTGGGAAC CTTACCTCCG 540

CCAACTCCAA ATTTTCACCC TCTGCGCATG CCCGGGAAAS AAACCCCCAG AACAGTACCG 600

TGATGATTGA TTTTAGGGTT ACAAATACAT TTTAGCAAGT AAGTGAATTT GGCATTACGA 660

ATTAATGATT AATGAAGGTC ACCTGTATTT CCATAGATAT GTAATTTTAT TTAAGCAGGT 720

TTATTATATT AAGGCGGSGA GGCAGCGCCG AAGACTACAA GTTCCAGCAT GCACCGCGTC 780

CGGGCGGGTT CGGGCTCCCA GCGAGGGCTT CAGGGACGCC AGCCCGGAGG CATCGGCCGG 840

AAGTGTCGTA GGGCAACCAC GTAGTACTCT CTGCGCATGT GCAAAGCGCT GTCGGGGGCC 900

GCCCTAGCTG CCGTCGCCGC CGCCGGGGCT CTATGGTCTC TCCCTAGAGC TTTGCCGTTG 960

GAGGCGGCTG CTGCGGTCTT GTGAGTTTGA CCAGCGTCGA GCGGCAGCAA CATGGAGGAA 1020

TTCGACTCCG AAGACTTCTC TACGTCGGAG GAGGACGAGG ACTACGTGCC GTCGGGTGAG 1080

CGATTCCGCC TGAGGCGAGA AGCGAATTGC CCCGCCCCAC GCCTCACGTG AGGCGCGCTC 1140

TGCCCCCGCG GGCGTCTGCC CTGTGGCCCA GGTGGTCCAG GGGGGCTCCT GTTCTCGAGC 1200

GTCCGCTCCC TCAGGCCCCT CATCCTCGGC CGCTCCGGCC CGAGGCGTGT GCGCGTGGCG 1260

GTTCTGTGCT CCCCTCCCGT TGGGCAGCTC CGGCCGCCGC CCCCTCTTGC AGCGCGGGAA 1320

CGGCACATGG ACACGGCCCC TTGTCGCTAG GGACGCTCGT CGGTCAGCCC CGAACGACAA 1380

CGCTGCTTCA GAAGTCGGGG CGGCAGTTCG AGCCTTGGAA GTTTTTTTCA GCCCTGGCCC 1440

GAGAGAGCTG CTGGCCAACA ACCCGTCCAA GATAGAGCTG TCCGNTCTCC GNCTGG 1496

(2) INFORMATION FOR SEQ ID NO: 215:

(i) SEQUENCE CHARACTERISTICS:
(A) LENGTH: 1308 base pairs
(B) TYPE: nucleic acid
(C) STRANDEDNESS: double
(D) TOPOLOGY: linear

(xi) SEQUENCE DESCRIPTION: SEQ ID NO: 215:

TTGGCANCNG GGAGAGGGAA AGAGGAGGAA ATGGGGTTTG AGGACCATGG CTTACCTTTC 60

CTGCCTTTGA CCCATCACAC CCCATTTCCT CCTCTTTCCC TCTCCCCGCT GCCAAAAAAA 120

AAAAAAAAGG AAACGTTTAT CATGAATCAA CAGGGTTTCA GTCCTTATCA AAGAGAGATG 180

TGGAAAGAGC TAAAGAAACC ACCCTTTGTT CCCAACTCCA CTTTACCCAT ATTTTATGCA 240

ACACAAACAC TGTCCTTTTG GGTCCCTTTC TTACAGATGG ACCTCTTGAG AAGAATTATC 300

GTATTCCACG TTTTTAGCCC TCAGGTTACC AAGATAAATA TATGTATATA TAACCTTTAT 360

TATTGCTATA TCTTTGTGGA TAATACATTC AGGTGGTGCT GGGTGATTTA TTATAATCTG 420

AACCTAGGTA TATCCTTTGG TCTTCCACAG TCATGTTGAG GTGGGCTCCC TGGTATGGTA 480

AAAAGCCAGG TATAATGTAA CTTCACCCCA GCCTTTGTAC TAAGCTCTTG ATAGTGGATA 540

TACTCTTTTA AGTTTAGCCC CAATATAGGG TAATGGAAAT TTCCTGCCCT CTGGGTTCCC 600

CATTTTTACT ATTAAGAAGA CCAGTGATAA TTTAATAATG CCACCAACTC TGGCTTAGTT 660

AAGTGAGAGT GTGAACTGTG TGGCAAGAGA GCCTCACACC TCACTAGGTG CAGAGAGCCC 720

AGGCCTTATG TTAAAATCAT GCACTTGAAA AGCAAACCTT AATCTGCAAA GACAGCAGCA 780

AGCATTATAC GGTCATCTTG AATGATCCCT TTGAAATTTT TTTTTTGTTT GTTTGTTTAA 840

ATCAAGCCTG AGGCTGGTGA ACAGTAGCTA CACACCCATA TTGTGTGTTC TGTGAATGCT 900

AGCTCTCTTG AATTTGGATA TTGGTTATTT TTTATAGAGT GTAAACCAAG TTTTATATTC 960

TGCAATGCGA ACAGGTACCT ATCTGTTTCT AAATAAAACT GTTTACATTC ATTATGGGGT 1020

ATGTATGACC TTCATTTTCC AAGAAATAGA ACTCTAGCTT AGAATTATGG ATGCTCTAAA 1080

ATGTCAGAAT GGGAACTCTC CTCGAAGTTC TCCCAAACTC AGAGACAGCA CTGCCTTCTC 1140

CTAAATGATT ATTCTTTTCT CCCTGTTTTC TGGTATTTTC TAGGCATCCT TCTCACCACA 1200

GCCATAACCC TTTTTTACTT CCATTAGGCC GTATAACTGG NGGGACNGCT GGTCGGTATA 1260

TAATACTGGT WCCAACAMAG GGGTTCTGGA TGTACACMAG GTTATCTT 1308

(2) INFORMATION FOR SEQ ID NO: 216:

(i) SEQUENCE CHARACTERISTICS:
(A) LENGTH: 1705 base pairs
(B) TYPE: nucleic acid
(C) STRANDEDNESS: double
(D) TOPOLOGY: linear

(xi) SEQUENCE DESCRIPTION: SEQ ID NO: 216:

TGGCCATGGA AGCGCTAGAA GGTTTAGATT TTGAAACAGC AAAGAAGGAT TTCCTTGGAT 60

CTGGAGACCC CAAAGAAACA AAGATGCTAA TCACCAAACA GGCTGACTGG GCCAGAAATA 120

TCAAGGAGCC CAAAGCCGCC GTGGAGATGT ACATCTCAGC AGGAGAGCAC GTCAAGGCCA 180

TCGAGATCTG TGGTGACCAT GGCTGGGTTG ACATGTTGAT CGACATCGCC CGCAAACTGG 240

ACAAGGCTGA GCGCGAGCCC CTGCTGCTGT GCGCTACCTA CCTCAAGAAG CTGGACAGCC 300

CTGGCTATGC TGCTGAGACC TACCTGAAGA TGGGTGACCT CAAGTCCCTG GTGCAGCTGC 360

AGTGGAGACC CAGCGCTGGG ATGAGGCCTT TGCTTTGGGT GAGAAGCATC CTGAGTTTAA 420

GGATGACATC TACATGCCGT ATGCTCAGTG GCTAGCAGAG AACGATCGCT TTGAGGAAGC 480

CCAGAAAGCG TTCCACAAGG CTGGGCGACA GAGAGAAGCG GTCCAGGTGC TGGAGCAGCT 540

CACAAACAAT GCCGTGGCGG AGAGCAGGTT TAATGATGCT GCCTATTATT ACTGGATGCT 600

GTCCATGCAG TGCCTCGATA TAGCTCAAGA TCCTGCCCAG AAGGACACAA TGCTTGGCAA 660

GTTCTACCAC TTCCAGCGTT TGGCAGAGCT GTACCATGGT TACCATGCCA TCCATCGCCA 720

CACGGAAGAT CCGTTCAGTG TCCATCGTCC TGAAACTCTT TTCAACATCT CCAGGTTCCT 780

GCTGCACAGC CTGCCCAAGG ACACCCCCTC GGGCATCTCT AAAGTGAAAA TACTCTTCAC 840

CTTGGCCAAG CAGAGCAAGG CCCTCGGTGC CTACAGGCTG GCCCGGCACG CCTATGACAA 900

GCTGCGTGGC CTGTACATCC CTGCCAGATT CCAAAAGTCC ATTGAGCTGG GTACCCTGAC 960

CATCCGCGCC AAGCCCTTCC ACGACAGTGA GGAGTTGGTG CCCTTGTGCT ACCGCTGCTC 1020

CACCAACAAC CCGCTGCTCA ACAACCTGGG CAACGTCTGC ATCAACTGCC GCCAGCCCTT 1080

CATCTTCTCC GCCTCTTCCT ACGACGTGCT ACACCTGGTT GAGTTCTACC TGGAGGAAGG 1140

GATCACTGAT GAAGAAGCCA TCTCCCTCAT CGACCTGGAG GTGCTGAGAC CCAAGCGGGA 1200

TGACAGACAG CTAGAGATTT GCAAACAACA GCTCCCAGAT TCTTGCGGCT AGTGGGAGAC 1260

CAAGGGACTC CATCGGAGAT NAGGACCCGT TCACAGCTAA GCTRAGCTTT GAGCAAGGTG 1320

GCTCARAGTT CGTGCCAGTG GTGGTGAGCC GGCTGGTGCT GCGCTCCATG AGCCGCCGGG 1380

ATGTCCTCAT CAAGCGATGG CCCCCACCCC TGAGGTGGCA ATACTTCCGC TCACTGCTGC 1440

CTGACGCCTC CATTACCATG TGCCCCTCCT GCTTCCAGAT GTTCCATTCT GAGGACTATG 1500

AGTTGCTGGT GCTTCAGCAT GGCTGCTGCC CCTACTGCCG CAGGTGCAAG GATGACCCTG 1560

GCCCATGACC AGCATCCTGG GGACGGCCTG CACCCTCTGC CCGCCTTGGG GTCTGCTGGG 1620

CTGTGAAGGA GAATAAAGAG TTAAACTGTC AAAAAAAAAA AAAAAAAAAA AAAAAAAAAA 1680

AAAAAAAAAA AAAAAAAAAA AAANA 1705

(2) INFORMATION FOR SEQ ID NO: 217:

(i) SEQUENCE CHARACTERISTICS:

(A) LENGTH: 999 base pairs
(B) TYPE: nucleic acid
(C) STRANDEDNESS: double
(D) TOPOLOGY: linear

(xi) SEQUENCE DESCRIPTION: SEQ ID NO: 217:

AGCAAATCAC CTTAACGATC TGGAATGAAA CTGTGACCAG TGCCGCCCTG GGTGGTTCTG 60

GAGAGACTGC CGTCTTCTTG TTTGGCCATA GGTGCTGGGG CCCCGGCTTC AGTCACTGTC 120

TCAGACAGKA GTCCCGATAA GCAGATCACC AGTCCTCCAC TGTCCTTCCT GTCGGCCTTG 180

CTGCATGAGA AGATAGCTGC TTCCTCCCTC TTTTCCTACA CTGTAAATTA TTGTTTTACA 240

ATTGAGTGYC TTAATAATAG TYTACAAATA CTATGTATTT ATGCAAAACT GTTAAAGTTC 300

TCATCTGTTA TGATTGGATA CTTGGTCTTG TCAGTAGTGG TCAGCATTGG GTTGTGAGCT 360

TGTCCTACTC CATACGTGTT TATCCTGCTA TGCATTTTAC ATTGTGTGTT CACATCTATT 420

CCAAGGAGCC TTGCTAGAAA CAACACTGGC GGTTCCTGCA GGCCAGGCAG GCATTGGCCC 480

ATGCTGTGTC CCATAGGAGC CAATGGAAAG AACGTAGCTT GGTCTGCTAG CCAGCCGTGG 540

GGTGGCGCAG GCCAGGCAGC CTCTGCACCA GAGTCCAGCA CCTGCCCATT CCCCAGTCAC 600

ACAATCATAC TCTTCTTTCA TAGAGATTTT ATTACCACCT AGACCACCCT AGTTTTCCTC 660

TCTGTTAGTG TCCTGAGCTC TTTTGCAACA AAATGTAGGT ACAGACCAAT CCCTGTCCCT 720

TCCCCAATCA GGAGCTCCAC ACCATGAGTT GTTTGGTTTT CCAGAAGCTG CCAGTGGGTT 780

CCCGTGAATT GCGTTAAGAT ATCGATGATK TTTTTTATTG TTTTTCTTCT TGTTTTTTTA 840

AATAATATAT TTAAAGGCAG TATCTTTTGT ACTGTGAATT TGCAGTAGAA GATGCAGAAT 900

GCACTTTTTT TTTACTTCTG TTGGTGTGTA TTGTATATAG TGTGTGTGCT TCTTGTGATG 960

AAAATAAACT TTTTCTTTAT AAAAAAAAAA AAAAAAAAC 999

(2) INFORMATION FOR SEQ ID NO: 218:

(i) SEQUENCE CHARACTERISTICS:
(A) LENGTH: 941 base pairs
(B) TYPE: nucleic acid
(C) STRANDEDNESS: double
(D) TOPOLOGY: linear

(xi) SEQUENCE DESCRIPTION: SEQ ID NO: 218:

GGCACGAGTA GCATTTCATT TAATCTGCAG GTATATTCTC CCAACAGTTT ATTGTCATGT 60

GATGTCCTCA GCCAAGATTG TRAGGCAGAG AGGAGCTGTC CCAACCTACT ATACCACCGA 120

GGCTGGAGAG ATCATATTTT TGGTATTAAA CTGGAGTCTC TCCATCCTTC ACATTGTTGA 180

TGTCCTCTGT AGCAAACCGG AAAAGTCAGT GACAGAAGAT GCCGCTAGCG GTTTGAGCCA 240

GAGAATGACA GCTCTGGTTT GGAGAAAAGG GCCGGATGGT GGCTCTAGAA AGCCCATCCT 300

TCTGCTCTTC TTTTTTCTCC CCCTTATATT GTGCTTTCAT TCATTCATTC ATTCATCAAA 360

CATTTGTTGA GCACCTATTA TGTGTCAAGC TCTGTGCTAG CCTCTGGAAA ACCTGCCCTC 420

ATGTAGCTCA CTGTGGAGTA GGAGAAACAA TGACTACACT ATGATAAGCA CGGGTTGTCA 480

GGGTCTCACA GAGCAGTGGC CCCTCATCCA GACCGATGAG GTCAAAGAAG GCATCCAGGC 540

GAGGATGGTG TCAGAGCTAA CTGAAGAATG AGAGGGAGCT GCACCASCAG GGGTTGGAAC 600

TGAAGGTGGC AGTGCCTGGA GTCTTGATTC CAGCAGAGGG AGAGCAGTCT GTGAAAAGGC 660

ACCAAGGGTG GGAGAGGGCA GAGCACATGG AGGAACTTCA GGTAGTTCTG GATGGCSCTG 720

GGGCAAAGCT AGAGAGGTAA GAAGAATCTA CAAATGTTCC TCGAGTTACA TGAACTTCCA 780

TCCCAATAAA CCCATTGGAA ACGAAAAATT TAAGTCAGAA GTGCATTTAA GGCTGGTCCG 840

AGTAGAATGA TTTTTACAAC GAATTGATCA CAACCAGTTA CAGATGTCTT TGTTCCTTCT 900

CCACTCCCAC TGCTTCACCT GACTAGCCTT TAAAAAAAAA A 941

(2) INFORMATION FOR SEQ ID NO: 219:

(i) SEQUENCE CHARACTERISTICS:
(A) LENGTH: 575 base pairs
(B) TYPE: nucleic acid
(C) STRANDEDNESS: double
(D) TOPOLOGY: linear

(xi) SEQUENCE DESCRIPTION: SEQ ID NO: 219:

TAAGTGGAAT CCCCCGGGGT TGCAGGGAAT TCGGCACGAG GCATTCTGAG AAGCTTAAGA 60

CATACTTTGA AGACAACCCT AGGGACCTCC AGCTGCTGCG GCATGACCTA CCTTTGCACC 120

CCGCAGTGGT GAAGCCCCAC CTGGGCCATG TTCCTGACTA CCTGGTTCCT CCTGCTCTCC 180

GTGGCCTGGT RCGCCCTCAC AAGAAGCGGA AGAAGCTGTC TTCCTCTTGT AGGAAGGCCA 240

AGAGAGCAAA GTCCCAGAAC CCACTGCGCA GCTTCAAGCA CAAAGGAAAG AAATTCAGAC 300

CCACAGCCAA GCCCTCCTGA GGTTGTTGGG CCTCTCTGGA GCTGAGCACA TTGTGGAGCA 360

CAGGCTTACA CCCTTCGTGG ACAGGCGAGG CTCTGGTGCT TACTGCACAG CCTGAACAGA 420

CAGTTCTGGG GCCGGCAGTG CTGGGCCCTT TAGCTCCTTG GCACTTCCAA GCTGGCATCT 480

TGCCCCTTGA CAACAGAATA AAAATTTTAG CTGCCCCAAA AAAAAAAAAA AAAAAAAAAA 540

CTCGAGGGGG GGCCCGTACC CAATTCGCCC TATAA 575

(2) INFORMATION FOR SEQ ID NO: 220:

(i) SEQUENCE CHARACTERISTICS:
(A) LENGTH: 3018 base pairs
(B) TYPE: nucleic acid
(C) STRANDEDNESS: double
(D) TOPOLOGY: linear

(xi) SEQUENCE DESCRIPTION: SEQ ID NO: 220:

GCCAGCCTTA CAGGTTTTAC GTGAAATGAA AGCCATTGGA ATAGAACCCT CGCTTGCAAC 60

ATATCACCAT ATTATTCGCC TGTTTGATCA ACCTGGAGAC CCTTTAAAGA GATCATCCTT 120

CATCATTTAT GATATAATGA ATGAATTAAT GGGAAAGAGA TTTTCTCCAA AGGACCCGGA 180

TGATGATAAG TTTTTTCAGT CAGCCATGAG CATATGCTCA TCTCTCAGAG ATCTAGAACT 240

TGCCTACCAA GTACATGGCC TTTTAAAAAC CGGAGACAAC TGGAAATTCA TTGGACCTGA 300

TCAACATCGT AATTTCTATT ATTCCAAGTT CTTCGATTTG ATTTGTCTAA TGGAACAAAT 360

TGATGTTACC TTGAAGTGGT ATGAGGACCT GATACCTTCA GCCTACTTTC CCCACTCCCA 420

AACAATGATA CATCTTCTCC AAGCATTGGA TGTGGCCAAT CGGCTAGAAG TGATTCCTAA 480

AATTTGGGAA AGATAGTAAA GAATATGGTC ATACTTTCCG CAGTGACCTG AGAGAAGAGA 540

TCCTGATGCT CATGGCAAGG GACAAGCACC CACCAGAGCT TCAGGTGGCA TTTGCTGACT 600

GTGCTGCTGA TATCAAATCT GCGTATGAAA GCCAACCCAT CAGACAGACT GCTCAGGATT 660

GGCCAGCCAC CTCTCTCAAC TGTATAGCTA TCCTCTTTTT AAGGGCTGGG AGAACTCAGG 720

AAGCCTGGAA AATGTTGGGG CTTTTCAGGA AGCATAATAA GATTCCTAGA AGTGAGTTGC 780

TGAATGAGCT TATGGACAGT GCAAAAGTGT CTAACAGCCC TTCCCAGGCC ATTGAAGTAG 840

TAGAGCTGGC AAGTGCCTTC AGCTTACCTA TTTGTGAGGG CCTCACCCAG AGAGTAATGA 900

GTGATTTTGC AATCAACCAG GAACAAAAGG AAGCCCTAAG TAATCTAACT GCATTGACCA 960

GTGACAGTGA TACTGACAGC AGCAGTGACA GCGACAGTGA CACCAGTGAA GGCAAATGAA 1020

AGTGGAGATT CAGGAGCAGC AATGGTCTCA CCATAGCTGC TGGAATCACA CCTGAGAACT 1080

GAGATATACC AATATTTAAC ATTGTTACAA AGAAGAAAAG ATACAGATTT GGTGAATTTG 1140

TTACTGTGAG GTACAGTCAG TACACAGCTG ACTTATGTAG ATTTAAGCTG CTAATATGCT 1200

ACTTAACCAT CTATTAATGC ACCATTAAAG GCTTAGCATT TAAGTAGCAA CATTGCGGTT 1260

TTCAGACACA TGGTGAGGTC CATGGCTCTT GTCATCAGGA TAAGCCTGCA CACCTAGAGT 1320

GTCGGTGAGC TGACCTCACG ATGCTGTCCT CGTGCGATTG CCCTCTCCTG CTGCTGGACT 1380

TCTGCCTTTG TTGGCCTGAT GTGCTGCTGT GATGCTGGTC CTTCATCTTA GGTGTTCATG 1440

CAGTTCTAAC ACAGTTGGGG TTGGGTCAAT AGTTTCCCAA TTTCAGGATA TTTCGATGTC 1500

AGAAATAACG CATCTTAGGA ATGACTAAAC AAGATAATGG CAGTTTAGGC TGCACAACTG 1560

GTAAAATGAC TGTAGATAAA TGTTGTAATT AGTGTACACG TTTGTATTTT TGTTAATATA 1620

GCCGCTGCCA TAGTTTTCTA ACTTGAACAG CCATGAATGT TTCATGTCTC CCTTTTTTTT 1680

TTGTCTATAG CTGTTACCTA TTTTAGTGGT TGAAATGAGA GCTAGTGATG ACAGAAGGAT 1740

GTGGAATGTC TTCTTGACAT CATTGTGTAT TGCTGGTAAT CAAGTTGGTA ACGACTACTT 1800

CTAGCAGCTC TTACCACTAT GACTTAAGTG GTCCTGGAAG GCAGTAAGTG GAGGTTTGCA 1860

GCATTCCTGC CTTCATGAGG GCTTCTACCA CTGACCACTT TGCACGTACC TGGCTCCCAG 1920

ATTTACTTAG GTACCCCACG AGTCGTCCAC ATAAGCAGCT TCATCTTTAC CTTGCCAGAG 1980

TTGACAATTA TGGGATACTC TAGTCTACTT ATACTTGTGT TCCCATCTGT CTGCCATCCT 2040

CTGAAGGCCA GGACCCAGTC ATACATCCTT AGAAACCAAA GTATGGTTTT TGTTTTCTCT 2100

TGGAATGTCA GGTCTTAAGG CATTTAATTG AGGGACAAAA AAAAAAAAAA GCCGATATAG 2160

TAGCTAGCTA CTTAAGCATC CATGGGTATT GCTCCATATC AAAGCAGATT TGCAGGACAG 2220

AAAGAGTAAA TTAGCCTTCA GTCTTGGTTT ACAGCTTCCA AGGAGAGCCT TGGSCACCTG 2280

AAATGTTAAC TCGGTCCCTT CCTGTCTCTA GTTCATCAGC ACCTGCAGAT GCCTGACTCT 2340

TGTTAGCCTT ACTATTCAAT ACAGTCCTTA GATTCACGGT ATGCCTCTTC CTATCCAGGC 2400

ACCTATTCTG AATCACCATG TTGCTCTGCA GCTAGAGTTG ATAGGAGAAA ATCCATTTGG 2460

GTAGATGGCC TATGAATTTG TAGTAGACTT TCAAAATGAG TGATTTGTTA GCTTGGTACT 2520

TTTAAGTTTG TGGTACAGAT CCTCCAAACC CATACTCTGA GCAATTAACT GCCTTGAACA 2580

TAGAGAAAAA TTAAGGCCTC ACAGGATGAG TCTCCATTCT CTGTAAATGC TTATTTTATC 2640

ATAGTCTTTA GCCTCTAACT ATGAGTAAAA TGTTCTCTTC GGCCGGGTGT GGTGACTCAC 2700

ACCTGTAACC TCAGCACTTT GGGAGGCAGA GGTGGGAGGA TCACTTAGGT CCAGGAGTTC 2760

GAGACTAGCC TGGGCAACAT AGTGAGACAC CGGATCTACA AAAAAATAAA AAGCCAGACT 2820

GGTGGTATGT ATCTGTGTCC CAGCTAATTG GGAGGGTGAG ATGGGAGGAT TGTTTGAGCC 2880

TAGGAGAGGG AGGTTGCAGT GAGCCGTGAT CGCACCACTG CACTCCAGCC TGGGCAACAG 2940

AGCAAGACCC TGTCTTGGAG AAACCAGAAT TTTGGAAGAG CAAATGGGGC TGAGTGCAGT 3000

GGCTCATGCC TGTAATCC 3018

(2) INFORMATION FOR SEQ ID NO: 221:

(i) SEQUENCE CHARACTERISTICS:

(A) LENGTH: 968 base pairs
(B) TYPE: nucleic acid
(C) STRANDEDNESS: double
(D) TOPOLOGY: linear

(xi) SEQUENCE DESCRIPTION: SEQ ID NO: 221:

GGCACGAGGG CCGCGGGACA TCCACGGGGC GCGAGTGACA CGCGGGAGGG AGAGCAGTGT 60

TCTGCTGGAG CCGATGCCAA AAACCATGCA TTTCTTATTC AGATTCATTG TTTTCTTTTA 120

TCTGTGGGGC CTTTTTACTG CTCAGAGACA AAAGAAAGAG GAGAGCACCG AAGAAGTGAA 180

AATAGAAGTT TTGCATCGTC CAGAAAACTG CTCTAAGACA AGCAAGAAGG GAGACCTACT 240

NAAATGCCCA TTATGACGGC TACCTGGCTA AAGACGGCTC GAAATTCTAC TGCAGCCGGA 300

CACAAAATGA AGGCCACCCC AAATGGTTTG TTCTTGGTGT TGGGCAAGTC ATAAAAGGCC 360

TAGACATTGC TATGACAGAT ATGTGCCCTG GAGAAAAGCG AAAAGTAGTT ATACCCCCTT 420

CATTTGCATA CGGAAAGGAA GGCTATGCAG AAGGCAAGAT TCCACCGGAT GCTACATTGA 480

TTTTTGAGAT TGAACTTTAT GCTGTGACCA AAGGACCACG GAGCATTGAG ACATTTAAAC 540

AAATAGACAT GGACAATGAC AGGCAGCTCT CTAAAGCCGA GATAAACCTC TACTTGCAAA 600

GGGAATTTGA AAAAGATGAG AAGCCACGTG ACAAGTCATA TCAGGATGCA GTTTTAGAAG 660

ATATTTTTAA GAAGAATGAC CATGATGGTG ATGGCTTCAT TTCTCCCAAG GAATACAATG 720

TATACCAACA CGATGAACTA TAGCATATTT GTATTTCTAC TTTTTTTTTT TAGCTATTTA 780

CTGTACTTTA TGTATWAAAC AAAGTCMCTT TTCTCCMAGT TGKATTTGCT ATTTTTCCCC 840

TATGAGAAGA TATTTTGATC TCCCCAATAC ATTGATTTTG GTATAATAAA TGTGAGGCTG 900

TTTTGCAAAC TTAAAAAAAA ATTTAAAAAA ACTGGAGGGG GGCCCGTACC CAANTCGCCG 960

NATATGAT 968

(2) INFORMATION FOR SEQ ID NO: 222:

(i) SEQUENCE CHARACTERISTICS:
(A) LENGTH: 1404 base pairs
(B) TYPE: nucleic acid
(C) STRANDEDNESS: double
(D) TOPOLOGY: linear

(xi) SEQUENCE DESCRIPTION: SEQ ID NO: 222:

CGTTTTCCGG CCGTGCGTTT GTGGCCGTCC GGCCTCCCTG ACATGCAGCC CTCTGGACCC 60

CGAGGTTGGA CCCTACTGTG ACACACCTAC CATGCGGACA CTCTTCAACC TCCTCTGGCT 120

TGCCCTGGCC TGCAGCCCTG TTCACACTAC CCTGTCAAAG TCAGATGCCA AAAAAGCCGC 180

CTCAAAGACG CTGCTGGAGA AGAGTCAGTT TTCAGATAAG CCGGTGCAAG ACCGGGGTTT 240

GGTGGTGACG GACCTCAAAG CTGAGAGTGT GGTTCTTGAG CATCGCAGCT ACTGCTCGGC 300

AAAGGCCCGG GACAGACACT TTGCTGGGGA TGTACTGGGC TATGTCACTC CATGGAACAG 360

CCATGGCTAC GATGTCACCA AGGTCTTTGG GAGCAAGTTC ACACAGATCT CACCCGTCTG 420

GCTGCAGCTG AAGAGACGTG GCCGTGAGAT GTTTGAGGTC ACGGGCCTCC ACGACGTGGA 480

CCAAGGGTGG ATGCGAGCTG TCAGGAAGCA TGCCAAGGGC CTGCACATAG TGCCTCGGCT 540

CCTGTTTGAG GACTGGACTT ACGATGATTT CCGGAACGTC TTAGACAGTG AGGATGAGAT 600

AGAGGAGCTG AGCAAGACCG TGGTCCAGGT GGCAAAGAAC CAGCATTTCG ATGGCTTCGT 660

GGTGGAGGTC TGGAACCAGC TGCTAAGCCA GAAGCGCGTG GGCCTCATCC ACATGCTCAC 720

CCACTTGGCC GAGGCTCTGC ACCAGGCCCG GCTGCTGGCC CTCCTGGTCA TCCCGCCTGC 780

CATCACCCCC GGGACCGACC AGCTGGGCAT GTTCACGCAC AAGGAGTTTG AGCAGCTGGC 840

CCCCGTGCTG GATGGTTTCA GCCTCATGAC CTACGACTAC TCTACAGCGC ATCAGCCTGG 900

CCCTAATGCA CCCCTGTCCT GGGTTCGAGC CTGCGTCCAG GTCCTGGACC CGAAGTCCAA 960

GTGGCGAAGC AAAATCCTCC TGGGGCTCAA CTTCTATGGT ATGGACTACG CGACCTCCAA 1020

GGATGCCCGT GAGCCTGTTG TCGGGGCCAG GTACATCCAG ACACTGAAGG ACCACAGGCC 1080

CCGGATGGTG TGGGACAGCC AGGYCTCAGA GCACTTCTTC GAGTACAAGA AGAGCCGCAG 1140

TGGGAGGCAC GTCGTCTTCT ACCCAACCCT GAAGTCCCTG CAGGTGCGGC TGGAGCTGGC 1200

CCGGGAGCTG GGCGTTGGGG TCTCTATCTG GGAGCTGGCC AGGGCCTGGA CTACTTCTAC 1260

GACCTGCTCT AGGTGGGCAT TGCGGCCTCC GCGGTGGACG TGTTCTTTTC TAAGCCATGG 1320

AGTGAGTGAG CAGGTGTGAA ATACAGGCCT NCACTCCGTT TGCTGTGAAA AAAAAAAAAA 1380

AAAAAAAAAA AAAAAAAAAA AAAA 1404

(2) INFORMATION FOR SEQ ID NO: 223:

(i) SEQUENCE CHARACTERISTICS:
(A) LENGTH: 707 base pairs
(B) TYPE: nucleic acid
(C) STRANDEDNESS: double
(D) TOPOLOGY: linear

(xi) SEQUENCE DESCRIPTION: SEQ ID NO: 223:
   NGCGCGCCTG CAGTCGACAC TAGTGGATCC AAAGAATTCG GCACGAGGGC AGGTCCAGGG 60
   CTCAGAAATC AGCTCTATTG ACGAATTCTG CCGCAAGTTC CGCCTGGACT GCCCGCTGGC 120
   CATGGAGCGG ATCAAGGAGG ACCGGCCCAT CACCATCAAG GACGACAAGG GCAACCTCAA 180
   CCGCTGCATC GCAGACGTGG TCTCGCTCTT CATCACGGTC ATGGACAAGC TGCGCCTGGA 240
   GATCCGCGCC ATGGATGAGA TCCAGCCCGA CCTGCGAGAG CTGATGGAGA CCATGCACCG 300
   CATGAGCCAC CTCCCACCCG ACTTTGAGGG CCGCCAGACG GTCAGCCAGT GGCTGCAGAC 360
   CCTGAGCGGC ATGTCGGCGT CAGATGAGCT GGACGACTCA CAGGTGCGTC AGATGCTGTT 420
   CGACCTGGAG TCAGCCTACA ACGCCTTCAA CCGCTTCCTG CATGCCTGAG CCTGGGGCAC 480
   TAGCCCTTGC ACAGAAGGGC AGAGTCTGAG GCGATGGCTC CTGGTCCCCT GTCCGCCACA 540
   CAGGCCGTGG TCATCCACAC AACTCACTGT CTGCAGCTGC CTGTCTGGTG TCTGTCTTTG 600
   GTGTCAGAAC TTTTGGGCCG GGCCCCTCCC CACAATAAAG ATGCTCTCCG ACCTTCAAAA 660
   AAAAAAAAAA AAAAACTCRG GGGGGGCCCG GTCCCAATCC CCCCNNN 707

(2) INFORMATION FOR SEQ ID NO: 224:

(i) SEQUENCE CHARACTERISTICS:
(A) LENGTH: 1384 base pairs
(B) TYPE: nucleic acid
(C) STRANDEDNESS: double
(D) TOPOLOGY: linear

(xi) SEQUENCE DESCRIPTION: SEQ ID NO: 224:

GGGGAACTGC AGTGACAGCA GGAGTAAGAG TGGGAGGCAG GACAGAGCTG GGACACAGGT 60

ATGGAGAGGG GGTTCAGCGA GCCTAGAGAG GGCAGACTAT CAGGGTGCCG GCGGTGAGAA 120

TCCAGGGAGA GGAGCGGAAA CAGAAGAGGG GCAGAAGACC GGGGCACTTG TGGGTTGCAG 180

AGCCCCTCAG CCATGTTGGG AGCCAAGCCA CACTGGCTAC CAGGTCCCCT ACACAGTCCC 240

GGGCTGCCCT TGGTTCTGGT GCTTCTGGCC CTGGGGGCCG GGTGGGCCCA GGAGGGGTCA 300

GAGCCCGTCC TGCTGGAGGG GGAGTGCCTG GTGGTCTGTG AGCCTGGCCG AGTTGCTGCA 360

GGGGGGCCCG GGGGAGCAGC CCTGGGAGAG GCACCCCCTG GGCGAGTGGC ATTTGCTGCG 420

GTCCGAAGCC AMCACCATGA GCCAGCAGGG GAAACCGGCA ATGGCACCAK TGGGGCCATC 480

TACTTCGACC AGGTCCTGGT GAACGAGGGC GGTGGCTTTG ACCGGGCCTC TGGCTCCTTC 540

GTAGCCCCTG TCCGGGGTGT CTACAGCTTC CGGTTCCATG TGGTGAAGGT GTACAACCGC 600

CAAACTGTCC AGGTGAGCCT GATGCTGAAC ACGTGGCCTG TCATCTCAGC CTTTGCCAAT 660

GATCCTGACG TGACCCGGGA GGCAGCCACC AGCTCTGTGC TACTGCCCTT GGACCCTGGG 720

GACCGAGTGT CTCTGCGCCT GCGTCGGGGG AATCTACTGG GTGGTTGGAA ATACTCAAGT 780

TTCTCTGGCT TCCTCATCTT CCCTCTCTGA GGACCCAAGT YTTTCAAGCA CAAGAATCCA 840

GCCCCTGACA ACTTTCTTCT GCCCTCTCTT GCCCCAGAAA CAGCAGAGGC AGGAGAGAGA 900

CTCCCTCTGG YTCCTATCCC ACYTCTTTGC ATGGGAMCCT GTGCCAAACA CCCAAGTTTA 960

AGARAARARY ARARCTGWGG CAGGTATACA GAGCTGGAAG TGGACCATGG AAAACATSGA 1020

TAACCATGCA TCYTCTTGCT TGGCCACCTC CTGAAACTGT CCACCTTTGA AGTTTGAACT 1080

TTAGTCCCTC CAMACTCTGA CTGCTGCCTC CTTCCTCCCA GCTCTCTCAC TGAGTTATYT 1140

TCACTGTACC TGTTCCAGCA TATCCCCACT ATCTCTCTTT CTCCTGATCT GTGCTGTCTT 1200

ATTCTCCTCC TTAGGCTTCC TATTACCTGG GATTCCATGA TTCATTCCTT CAGACCCTCT 1260

CCTGCCAGTA TGCTAAACCC TCCCTCTCTC TTTCTTATCC CGCTGTCCCA TTGGCCCAGC 1320

CTGGATGAAT CTATCAATAA AACAACTAGA GAATGGTGGT CAAAAAAAAA AAAAAAAAAC 1380

TCGA 1384

(2) INFORMATION FOR SEQ ID NO: 225:

(i) SEQUENCE CHARACTERISTICS:
(A) LENGTH: 760 base pairs
(B) TYPE: nucleic acid
(C) STRANDEDNESS: double
(D) TOPOLOGY: linear

(xi) SEQUENCE DESCRIPTION: SEQ ID NO: 225:

GGGTCGACCC ACGCGTCCGC TGACCAGTCC GTTATAGATA CTTCTTCCTA TACCAAAACT 60

GTTTAAACAG GTGCCACCAC AAGGGATGTC GTCCTTACTC TCTGCGGGTC TTCAAGCATC 120

CCTTTGTGGG AAARGTCTCT GGGCAAGCAC GTGGTATTTG GTCTGCTGCT TGCTTCCCTT 180

TTTCCACCAG GGATGTTGTG ATCATAAGTC AAAACAACAG TATATTCCAA ATCTCAAAAG 240

CTATTGTGGC CTGAGCACAA TTGAAATCTA GCAGAGTTTT TCCTATGTAG CTTTAGAGTA 300

ACTCTTCTGC TTCTCTGTCA CTTACAATTC AGGTTCTGCC TTTGCCTAAG AGCATGAGCA 360

GAAGAGTCCT CATGTGACGC TTAGTTCTAT TGCAGTCCTG GGTGAAACTA TTTAAGCWAT 420

GGGGCTGCTK CTCCCCANWT CCTCCCTAAC AATTCGTTGT GTGGACTTCT CATCTAAAAG 480

GTTAGTGGCT TTTGCTTGGG ATCAGTGCTC TCTATTGATG TTCTTGCTGG TCTCCAGACA 540

CATTCCTGTT GCATTAAGAC TTGAAAGACT TGTAGATGTG TGATGTTCAG GCACAGGATG 600

CTGAAAGCTA TGTTACTATT CTTAGTTTGT AAATTGTCCT TTTGATACCA TCATCTTGTT 660

TTCTTTTTGT AGGTATAAAT AAAAACACTG TTGACAATAA AAAAAAAAAA AAAAAAAAAA 720

AAAAAAAAAA AAAAAAAAAA NAAAAAAAAA AAAAAAAAAA 760

(2) INFORMATION FOR SEQ ID NO: 226:

(i) SEQUENCE CHARACTERISTICS:
(A) LENGTH: 2057 base pairs
(B) TYPE: nucleic acid
(C) STRANDEDNESS: double
(D) TOPOLOGY: linear

(xi) SEQUENCE DESCRIPTION: SEQ ID NO: 226:

CCGAGCCGGC TGCGCCGGGG GAATCCGTGC GGGCGCCTTC CGTCCCRGTC CCATCCTCGC 60

CGCGCTCCAG CACCTCTGAA GTTTTGCAGC GCCCAGAAAG GAGGCGAGGA AGGAGGGAGT 120

GTGTGAGAGG AGGGAGCAAA AAGCTCACCC TAAAACATTT ATTTCAAGGA GAAAAGAAAA 180

AGGGGGGGCG CAAAAATGGC TGGGGCAATT ATAGAAAACA TGAGCACCAA GAAGCTGTGC 240

ATTGTTGGTG GGATTCTGCT CGTGTTCCAA ATCATCGCCT TTCTGGTGGG AGGCTTGATT 300

GCTCCAGGGC CCACAACGGC AGTGTCCTAC ATGTCGGTGA AATGTGTGGA TGCCCGTAAG 360

AACCATCACA AGACAAAATG GTTCGTGCCT TGGGGACCCA ATCATTGTGA CAAGATCCGA 420

GACATTGAAG AGGCAATTCC AAGGGAAATT GAAGCCAATG ACATCGTGTT TTCTGTTCAC 480

ATTCCCCTCC CCCACATGGA GATGAGTCCT TGGTTCCAAT TCATGMTGTT TATCCTGCAG 540

CTGGACATTG CCTTCAAGCT AAACAACCAA ATCAGRGAAA ATGCAGAAGT CTCCATGGAC 600

GTTTCCCTGG CTTACCGTGA TGACGCGTTT GCTGAGTGGA CTGAAATGGC CCATGAAAGA 660

GTACCACGGA AACTCAAATG CACCTTCACA TCTCCCAAGA CTCCAGAGCA TGGAGGGCCG 720

GTTACTATGA ATGTGATGTC CTTCCTTTCA TGGAAATTGG GTCTGTGGCC CATGAAGTTT 780

TACCTTTTAA ACATCCGGCT GCCTGTGAAT GAGAAGAAGA AAATCAATGT GGGAATTGGG 840

GAGATAAAGG ATATCCGGTT GGTGGGGATC CACCAAAATG GAGGCTTCAC CAAGGTGTGG 900

TTTGCCATGA AGACCTTCCT TACGCCCAGC ATCTTCATCA TTATGGTGTG GTATTGGAGG 960

AGGATCACCA TGATGTCCCG ACCCCCAGTG CTTCTGGAAA AAGTCATCTT TGCCCTTGGG 1020

ATTTCCATGA CCTTTATCAA TATCCCAGTG GAATGGTTTT CCATCGGGTT TGACTGGACC 1080

TGGATGCTGC TGTTTGGTGA CATCCGACAG GCATCTTCTA TGCRATGCTT CTKTCCTTCT 1140

GGATCATCTT CTGTGGCGAG CACATGATGG ATCAGCACGA GCGGAACCAC ATCGCAGGGT 1200

ATTGGAAGCA AGTCGGACCC ATTGCCGTTG GTCCTTCTGC CTCTTCATAT TTGACATGTG 1260

TGAGAGAGGG GTACAACTCA CGAATCCCTT CTACAGTATC TGGACTACAG ACATTGGGAA 1320

CAGAGCTGGC CATGGCTTTC ATCATCGTGG CTGGAATCTG CCTCTGCCTC TAACTTCCTG 1380

TTTCTATGCT TCATGGTATT TCAGGTGTTT CGGAACATCA GTGGGAAGCA GTCCAGCCTG 1440

CCAGCTATGA GCAAAGTCCG GCGGCTACAC TATGAGGGGC TAATTTTTAG GTTCAAGTTC 1500

CTCATGCTTA TCACCTTGGC TTGCGCTGCC ATGACTGTCA TCTTCTTCAT CGTTAGTCAG 1560

GTAACGGAAG GCTATTGGGA AATGGGGCGG CGTCACAGTC CCAAGTGAAC AGTGCCTTTT 1620

TCACAGGCAT CTATGGGATG TGGAATCTGT ATGTCTTTGC TCTGATGTTC TTGTATGCAC 1680

CATCCCATAA AAACTATGGA GAAGACCAGT CCAATGGAAT GCAACTCCCA TGTAAATCGA 1740

GGGAAGATTG TGCTTTGTTT GTTTCGGAAC TTTATCAAGA ATTGTTCAGC GCTTCGAAAT 1800

ATTCCTTCAT CAATGACAAC GTAGCTTCTG GTATTTGAGT CAACAAGGCA ACACATGTTT 1860

ATCAGCTTTG CATTTGCAGT TGTCACAGTC ACATTGATTG TACTTGTATA CGCACACAAA 1920

TACACTCATT TAGCCTTTAT CTCAAAATGT TAAATATAAG GAAAAAAGCG TCAACAATAA 1980

ATATTCTTTG AGTATTGTCT TACTTCTCTT AAAAAAAAAA AAAAAAACTC GTGCCGAATT 2040

CGGCACGAGC GGCACGA 2057

(2) INFORMATION FOR SEQ ID NO: 227:

(i) SEQUENCE CHARACTERISTICS:
(A) LENGTH: 2084 base pairs
(B) TYPE: nucleic acid
(C) STRANDEDNESS: double
(D) TOPOLOGY: linear

(xi) SEQUENCE DESCRIPTION: SEQ ID NO: 227:

GGCAGAGGGC CATTTCCTGC AAAGAGCCAA ACCCCCATTC CTCTGTGCCC CTCCTCTCCC 60

ACCAAGTGCT TTATAAAAAT AGCTCTTGTT ACCGGAAATA ACTGTTCATT TTTCACTCCT 120

CCCTCCTAGG TCACACTTTT CAGAAAAAGA ATCTGCATCC TGGAAACCAG AAGAAAAATA 180

TGAGACGGGG AATCATCGTG TGATGTGTGT SCTGCCTTTG GCTGAGTGTG TGGAGTCCTG 240

CTCAGGTGTT AGGTACAGTG TGTTTGATCG TGGTGGCTTG AGGGGAACCG CTTGTTCAGA 300

GCTGTGACTG CGGCTGCACT GCAGAGAAGC TGCCCTTGGC TGCTCGTAGC GCCGGGCCTT 360

CTCTCCTCGT CATCATCCAG AGCAGCCAGT GTCCGGGAGG CAGAAGGTAC CGGGGCAGCT 420

ACTGGAGGAC TGTGCGGGCC TGCCTGGGCT GCCCCCTCCG CCGTGGGGCC CTGTTGCTGC 480

TGTCCATCTA TTTCTACTAC TCCCTCCCAA ATGCGGTCGG CCCGCCCTTC ACTTGGATGC 540

TTGCCCTCCT GGGCCTTCTC GCAGGCACTG AACATCCTCC TGGGCCTCAA GGGCCTGGCC 600

CCAGCTGAGA TCTCTGCAGT GTGTGAAAAA GGGAATTTCA ACGTGGCCCA TGGGCTGGCA 660

TGGTCATATT ACATCGGATA TCTGCGGCTG ATCCTGCCAG AGCTCCAGGC CCGGATTCGA 720

ACTTACAATC AGCATTACAA CAACCTGCTA CGGGGTGCAG TGAGCCAGCG GTGTNATATT 780

CTCCTCCCAT TGGACTGTGG GGTGCCTGAT AACCTGAGTA TGGCTGACCC CAACATTCGC 840

TTCCTGGATA AACTGCCCCA GCAGACCGGT GACCGTGCTG GCATCAAGGA TCGGGTTTAC 900

AGCAACAGCA TCTATGAGCT TCTGGAGAAC GGGCAGCGGG CGGGCACCTG TGTCCTGGAG 960

TACGCCACCC CCTTGCAGAC TTTGTTTGCC ATGTCACAAT ACAGTCAAGC TGGCTTTAGC 1020

GGGGAGGATA GGCTTGAGCA GGCCAAACTC TTCTGCCGGA CACTTGAGGA CATCCTGGCA 1080

GATGCCCCTG AGTCTCAGAA CAACTGCCGC CTCATTGCCT ACCAGGAACC TGCAGATGAC 1140

AGCAGCTTCT CGCTGTCCCA GGAGGTTCTC CGGCACCTGC GGCAGGAGGA AAAGGAAGAG 1200

GTTACTGTGG GCAGCTTGAA GACCTCAGCG GTGCCCAGTA CCTCCACGAT GTCCCAAGAG 1260

CCTGAGCTCC TCATCAGTGG AATGGAAAAG CCCCTCCCTC TCCGCACGGA TTTCTCTTGA 1320

GACCCAGGGT CACCAGGCCA GAGCCTCCAG TGGTCTCCAA GCCTCTGGAC TGGGGGCTCT 1380

CTTCAGTGGC TGAATGTCCA GCAGAGCTAT TTCCTTCCAC AGGGGGCCTT GCAGGGAAGG 1440

GTCCAGGACT TGACATCTTA AGATGCGTCT TGTCCCCTTG GGCCAGTCAT TTCCCCTCTC 1500

TGAGCCTCGG TGTCTTCAAC CTGTGAAATG GGATCATAAT CACTGCCTTA CCTCCCTCAC 1560

GGTTGTTGTG AGGACTGAGT GTGTGGAAGT TTTTCATAAA CTTTGGATGC TAGTGTACTT 1620

AGGGGGTGTG CCAGGTGTCT TTCATGGGGC CTTCCAGACC CACTCCCCAC CCTTCTCCCC 1680

TTCCTTTGCC CGGGGACGCC GAACTCTCTC AATGGTATCA ACAGGCTCCT TCGCCCTCTG 1740

GCTCCTGGTC ATGTTCCATT ATTGGGGAGC CCCAGCAGAA GAATGGAGAG GAGGAGGAGG 1800

CTGAGTTTGG GGTATTGAAT CCCCCGGCTC CCACCCTGCA GCATCAAGGT TGCTATGGAC 1860

TCTCCTGCCG GGCAACTCTT GCGTAATCAT GACTATCTCT AGGATTCTGG CACCACTTCC 1920

TTCCCTGGCC CCTTAAGCCT AGCTGTGTAT CGGCACCCCC ACCCCACTAG AGTACTCCCT 1980

CTCACTTGCG GTTTCCTTAT ACTCCACCCC TTTCTCAACG GTCCTTTTTT AAAGCACATC 2040

TCAGATTAAA AAAAAAAAAA AAAAAAAAAA AGGGGGGGCN GCNT 2084

(2) INFORMATION FOR SEQ ID NO: 228:

(i) SEQUENCE CHARACTERISTICS:
(A) LENGTH: 2143 base pairs
(B) TYPE: nucleic acid
(C) STRANDEDNESS: double
(D) TOPOLOGY: linear

(xi) SEQUENCE DESCRIPTION: SEQ ID NO: 228:

TCGACCCACG CGTCCGGTTG AATTCCTTGA CCTGCAAACA CATATTTATT AGCCTGACTC 60

AAACAATGAA GCTATTAAAA CTTCGGAGGA ACATTGTAAA ACTCTCTTTG TATCGGCATT 120

TCACCAACAC GCTTATTTTG GCAGTGGCAG CATCCATTGT GTTTATCATC TGGACAACCA 180

TGAAGTTCAG AATAGTGACA TGTCAGTCGG ACTGGCGGGA GCTGTGGGTA GACGATGCCA 240

TCTGGCGCTT GCTGTTCTCC ATGATCCTCT TTGTCATCAT GGTTCTCTGG CGACCATCTG 300

CAAACAACCA GAGGTTTGCC TTTTCACCAT TGTCTGAGGA AGAGGAGGAG GATGAACAAA 360

AGGAGCCTAT GCTGAAAGAA AGCTTTGAAG GAATGAAAAT GAGAAGTACC AAACAAGAAC 420

CCAATGGAAA TAGTAAAGTT AACAAAGCAC AGGAAGATGA TTTGAAGTGG GTAGAAGAGA 480

ATGTTCCTTC TTCTGTGACA GATGTAGCAC TTCCAGCCCT TCTGGATTCA GATGAGGAAC 540

GAATGATCAC ACACTTTGAA AGGTCCAAAA TGGAGTAAGG AATGGGAAGA TTTGCAGTTA 600

AAGATGGCTA CCATCAGGGA AGAGATCAGC ATCTGTGTCA GTCTTCTGTA CGGCTCCATG 660

GGATTAAAGG AAGCAATGAC ATCCTGATCT GTTCCTTGAT CTTTGGGCAT TGGAGTTGGC 720

GAGAGGTGTC AGAACAAAGA GAACATCTTA CTGAAAACAA GTTCATAAGA TGAGAAAAAT 780

CTACGAGCTT CTTATTTACA ACACTGCTGC CCCCTTTCCT CCCAGACTCT GACATGGATG 840

TTCATGCAAC TTAAGTGTGT TGTTCCTGAA CTTTCTGTAA TGTTTCATTT TTTAAATCTG 900

ACAAACTAAA AAGTTTAACG TCTTCTAAAA GATTGTCATC AACACCATAA TATGTAATCT 960

CCAGGAGCAA CTGCCTGTAA TTTTTATTTA TTTAGGGAGT TACATAGGTG ATGGGGGAAA 1020

TTGTTAACTA CCTTTCATTT TCCTGGGAAG TCAAGGTTAC ATCTTGCAGA GGTTGTTTTG 1080

AGAAAAAAGG GCCCTTCTGA GTTAAGGAGC CATAGTTCTA TCAATGATCA AAAGAAAAAA 1140

AAAAAAAAGA GAAACTGTTA CAGTATGATT CAGATCATTT AAAAAAGCAA AATCAAGTGC 1200

AATTTTGTTT ACAAATGGTG TATATTAAAG ATTTTTCTAT TTCAGATGTA CTTTAAAGAG 1260

AAATATTAGC TTAACTCTTT TGACATCTGC TATTGTGACA CATCCCATTG CTGGCAATGT 1320

GGTGCACACT CCGAAACTTT TAACTACTGT TTTGTAAGCC TCCAAGGGTG GCATTGCAGG 1380

GTCCTTAGGC AATGTTTTGT TTGCCTTTAT GCAGAGAGGT GCTCCAAGTG CTGTGATTGA 1440

GCACCGTGCT AGAGGAACTG TAATGCTTCA GAAGTTGTAG CTTATACAAA GGAAACAGGT 1500

CCTGCTGGCT TAATTTAAAC AGTTATTGCA TGAAGTAGCG TGGAGGCCCT GGACTGCTGC 1560

TCGTTCTTTA GGATGGACTG TTCTGGTATC TGGTATTGGT TTAGAGACTG TTAATAAGGG 1620

ACATCACAAG GTGATGGGAT TCATTTGAAG CACTCTATTT CTGTTTTAAT GGTTTTATCC 1680

AATTTTGCCT TCCCAAGATT TTTGTTCTAC ATAAAAAGTT CATGCCACTT TTTAATATAA 1740

AAAAATTTAA CAAAATTAAT GTATTTTTCT CATTTTTTTC AAACTTTTTC TAAAGACTCT 1800

TTCTGTCAAA CTCATGAAAA ATTTCTTTCT ATGGCTTTTA TTCTAGATTG TCTTATTTTC 1860

TGTTAAAACC AATGACCACA TGACCACAAT CTTCACTAAC TCATACTGCA GTGAAAGTGT 1920

TAACCCTTAG GTAGTTTCTC TACAACTCTT TGCTATGGTG ATTTTTAAAA AAGTTTCCTA 1980

GGGAAGTATC TCTGAGGGAA CAGGCAATCT GAAGGAACTG ACTATATTCT CCATGGCTAA 2040

GTCCATTAGG CCAAAAGNCT GGGTGGGTAT TGGTTGTCAN GCTGTCTATT GGCATATTAA 2100

AAACGTAGGC CGGANGGAAT AATTAGGTTG TNATGCCGGC GGG 2143

(2) INFORMATION FOR SEQ ID NO: 229:

(i) SEQUENCE CHARACTERISTICS:
(A) LENGTH: 1025 base pairs
(B) TYPE: nucleic acid
(C) STRANDEDNESS: double
(D) TOPOLOGY: linear

(xi) SEQUENCE DESCRIPTION: SEQ ID NO: 229:

CCTGGCCCAC ATTGCTTCAT TGGCCTGGCC ATGCGCCTGT ACTATGGCAG CCGCTAGTCC 60

CTGACAACTT CCACCCTGAT TCCGGACCCT GTAGATTGGG CGCCACCACC AGATCCCCCT 120

CCCAGGCCTT CCTCCCTCTC CCATCAGCAG CCCTGTAACA AGTGCCTTGT GAGAAAAGCT 180

GGAGAAGTGA GGGCAGCCAG GTTATTCTCT GGAGGTTGGT GGATGAAGGG GTACCCTAGG 240

AGATGTGAAG TGTGGGTTTG GTTAAGGAAA TGCTTACCAT CCCCCACCCC CAACCAAGTT 300

CTTCCAGACT AAAGAATTAA GGTAACATCA ATACCTAGGC CTGAGAAATA ACCCCATCCT 360

TGTTGGGCAG CTCCCTGCTT TGTCCTGCAT GAACAGAGTT GATGAAAGTG GGGTGTGGGC 420

AACAAGTGGC TTTCCTTGCC TACTTTAGTC ACCCAGCAGA GCCACTGGAG CTGGCTAGTC 480

CAGCCCAGCC ATGGTGCATG ACTCTTCCAT AAGGGATCCT CACCCTTCCA CTTTCATGCA 540

AGAAGGCCCA GTTGCCACAG ATTATACAAC CATTACCCAA ACCACTCTGA CAGTCTCCTC 600

CAGTTCCAGC AATGCCTAGA GACATGCTCC CTGCCCTCTC CACAGTGCTG CTCCCCACAC 660

CTAGCCTTTG TTCTGGAAAC CCCAGAGAGG GCTGGGCTTG ACTCATCTCA GGGAATGTAG 720

CCCCTGGGCC CTGGCTTAAG CCGACACTCC TGACCTCTCT GTTCACCCTG AGGGCTGTCT 780

TGAAGCCCGC TACCCACTCT GAGGCTCCTA GGAGGTACCA TGCTTCCCAC TCTGGGGCCT 840

GCCCCTGCCT AGCAGTCTCC CAGCTCCCAA CAGCCTGGGG AAGCTCTGCA CAGAGTGACC 900

TGAGACCAGG TACAGGAAAC CTGTAGCTCA ATCAGTGTCT CTTTAACTGC ATAAGCAATA 960

AGATCTTAAT AAAGTCTTCT AGGCTGTAGG GTGGTTCCTA CAACCACAGC CAAAAAAAAA 1020

AAAAA 1025

(2) INFORMATION FOR SEQ ID NO: 230:

(i) SEQUENCE CHARACTERISTICS:
(A) LENGTH: 1250 base pairs
(B) TYPE: nucleic acid
(C) STRANDEDNESS: double
(D) TOPOLOGY: linear

(xi) SEQUENCE DESCRIPTION: SEQ ID NO: 230:

GCCCACGCGT CCGCCCACGC GTCCGGCGGT GCGGAGTATG GGGCGCTGAT GGCCATGGAG 60

GGCTACTGGC GCTTCCTGGC GCYGCTGGGG TCGGCACTGC TCGTCGGCTT CCTGTCGGTG 120

ATSTTCGCCC TCGTCTGGGT CCTCCACTAC CGAGAGGGGC TTGGCTGGGA TGGGAGCGCA 180

CTAGAGTTTA ACTGGCACCC AGTGCTSATG GTCACCGGCT TCGTCTTCAT CCAGGGCATC 240

GCATCATCGT CTACAGACTG CCGTGGACCT GGAAATGCAG CAAGCTCCTG ATGAAATCCA 300

TCCATGCAGG GTTAAATGCA GTTGCTGCCA TTCTTGCAAT TATCTCTGTG GTGGCCGTGT 360

TTGAGAACCA CAATGTTAAC AATATAGCCA ATATGTACAG TCTGCACAGC TGGGTTGGAC 420

TGATAGCTGT CATATGCTAT TTGTTACAGC TTCTTTCAGG TTTTTCAGTC TTTCTGCTTC 480

CATGGGCTCC GCTTTCTCTC CGAGCATTTC TCATGCCCAT ACATGTTTAT TCTGGAATTG 540

TCATCTTTGG AACAGTGATT GCAACAGCAC TTATGGGATT GACAGAGAAA CTGATTTTTT 600

CCCTGAGAGA TCCTGCATAC AGTACATTCC CGCCAGAAGG TGTTTTCGTA AATACGCTTG 660

GCCTTCTGAT CCTGGTGTTC GGGGCCCTCA TTTTTTGGAT AGTCACCAGA CCGCAATGGA 720

AACGTCCTAA GGAGCCAAAT TCTACCATTC TTCATCCAAA TGGAGGCACT GAACAGGGAG 780

CAAGAGGTTC CATGCCAGCC TACTCTGGCA ACAACATGGA CAAATCAGAT TCAGAGTTAA 840

ACARTGAAGT AGCAGCAAGG AAAAGAAACT TAGCTCTGGA TGAGGCTGGG CAGAGATCTA 900

CCATGTAAAA TGTTGTAGAG ATAGAGCCAT ATAACGTCAC GTTTCAAAAC TAGCTCTACA 960

GTTTTGCTTC TCCTATTAGC CATATGATAA TTGGGCTATG TAGTATCAAT ATTTACTTTA 1020

ATCACAAAGG ATGGTTTCTT GAAATAATTT GTATTGATTG AGGCCTATGA ACTGACCTGA 1080

ATTGGAAAGG ATGTGATTAA TATAAATAAT AGCAGATATA AATTGTGGTT ATGTTACCTT 1140

TATCTTGTTG AGGACCACAA CATTAGCACG GTGCCTTGTG CAKAATAGAT ACTCAATATG 1200

TGAATATGTG TCTACTAGTA GTTAATTGGA TAAACTGGCA GCATCCCTGA 1250

(2) INFORMATION FOR SEQ ID NO: 231:

(i) SEQUENCE CHARACTERISTICS:

(A) LENGTH: 1811 base pairs
(B) TYPE: nucleic acid
(C) STRANDEDNESS: double
(D) TOPOLOGY: linear

(xi) SEQUENCE DESCRIPTION: SEQ ID NO: 231:

CNGNCAGTAC CGGTCNGATT CCCGGGTCGA CCCACGCGTC CGCTGCATTC CAGGGCCTTT 60

CAGTGGCTTT CATTCTGAAG TTCCTGGATA ACATGTTCCA TGTCTTGATG GCCCAGGTTA 120

CCASTGTCAT TATCACAACA GTGTCTGTCC TGGTCTTTGA CTTCAGGCCC TCCCTGGAAT 180

TTTTCTTGGA AGCCSCATCA GTCSTYCTCT CTATATTTAT TTATAATGCC AGCAAGCCTC 240

AAGTTCCGGA ATACGCACCT AGGCAAGAAA GGATCCGAGA TCTAAGTGGC AATCTTTGGG 300

AGCGTTCCAG TGGGGATGGA GAAGAACTAG AAAGACTTAC CAAACCCAAG AGTGATGAGT 360

CAGATGAAGA TACTTTCTAA CTGGTACCCA CATAGTTTGC AGCTCTCTTG AACCTTATTT 420

TCACATTTTC AGTGTTTGTA ATATTTATCT TTTCACTTTG ATAAACCAGA AATGTTTCTA 480

AATCCTAATA TTCTTTGCAT ATATCTAGCT ACTCCCTAAA TGGTTCCATC CAAGGCTTAG 540

AGTACCCAAA GGCTAAGAAA TTCTAAAGAA CTGATACAGG AGTAACAATA TGAAGAATTC 600

ATTAATATCT CAGTACTTGA TAAATCAGAA AGTTATATGT GCAGATTATT TTCCTTGGCC 660

TTCAAGCTTC CAAAAAACTT GTAATAATCA TGTTAGCTAT AGCTTGTATA TACACATAGA 720

GATCAATTTG CCAAATATTC ACAATCATGT AGTTCTAGTT TACATGCCAA AGTCTTCCCT 780

TTTTAACATT ATAAAAGCTA GGTTGTCTCT TGAATTTTGA GGCCCTAGAG ATAGTCATTT 840

TGCAAGTAAA GAGCAACGGG ACCCTTTCTA AAAACGTTGG TTGAAGGACC TAAATACCTG 900

GCCATACCAT AGATTTGGGA TGATGTAGTC TGTGCTAAAT ATTTTGCTGA AGAAGCAGTT 960

TCTCAGACAC AACATCTCAG AATTTTAATT TTTAGAAATT CATGGGAAAT TGGATTTTTG 1020

TAATAATCTT TTGATGTTTT AAACATTGGT TCCCTAGTCA CCATAGTTAC CACTTGTATT 1080

TTAAGTCATT TAAACAAGCC ACGGTGGGGC TTTTTTCTCC TCAGTTTGAG GAGAAAAATC 1140

TTGATGTCAT TACTCCTGAA TTATTACATT TTGGAGAATA AGAGGGCATT TTATTTTATT 1200

AGTTACTAAT TCAAGCTGTG ACTATTGTAT ATCTTTCCAA GAGTTGAAAT GCTGGCTTCA 1260

GAATCATACC AGATTGTCAG TGAAGCTGAT GCCTAGGAAC TTTTAAAGGG ATCCTTTCAA 1320

AAGGATCACT TAGCAAACAC ATGTTGACTT TTAACTGATG TATGAATATT AATACTCTAA 1380

AAATAGAAAG ACCAGTAATA TATAAGTCAC TTTACAGTGC TACTTCACAC TTAAAAGTGC 1440

ATGGTATTTT TCATGGTATT TTGCATGCAG CCAGTTAACT CTCGTAGATA GAGAAGTCAG 1500

GTGATAGATG ATATTAAAAA TTAGCAAACA AAAGTGACTT GCTCAGGGTC ATGCAGCTGG 1560

GTGATGATAG AAGAGTGGGC TTTAACTGGC AGGCCTGTAT GTTTACAGAC TACCATACTG 1620

TAAATATGAG CTTTATGGTG TCATTCTCAG AAACTTATAC ATTTCTGCTC TCCTTTCTCC 1680

TAAGTTTCAT GCAGATGAAT ATAAGGTAAT ATACTATTAT ATAATTCATT TGTGATATCC 1740

ACAATAATAT GACTGGCAAG AATTGGTGGA AATTTGTAAT TAAAATAATT ATTAAACCTA 1800

AAAAAAAAAN N 1811

(2) INFORMATION FOR SEQ ID NO: 232:

(i) SEQUENCE CHARACTERISTICS:
(A) LENGTH: 2271 base pairs
(B) TYPE: nucleic acid
(C) STRANDEDNESS: double
(D) TOPOLOGY: linear

(xi) SEQUENCE DESCRIPTION: SEQ ID NO: 232:

CTGACCTCAT GGCGTAGAGC CTAGCAACAG CGCAGGCTCC CAGCCGAGTC CGTTATGGCC 60

GCTGCCGTCC CGAAGAGGAT GAGGGGGCCA GCACAAGCGA AACTGCTGCC CGGGTCGGCC 120

ATCCAAGCCC TTGTGGGGTT GGCGCGGCCG CTGGTCTTGG CGCTCCTGCT TGTGTCCGCC 180

GCTCTATCCA GTGTTGTATC ACGGACTGAT TCACCGAGCC CAACCGTACT CAACTCACAT 240

ATTTCTACCC CAAATGTGAA TGCTTTAACA CATGAAAACC AAACCAAACC TTCTATTTCC 300

CAAATCAGCA CCACCCTCCC TCCCACGACG AGTACCAAGA AAAGTGGAGG AGCATCTGTG 360

GTCCCTCATC CCTCGCCTAC TCCTCTGTCT CAAGAGGAAG CTGATAACAA TGAAGATCCT 420

AGTATAGAGG AGGAGGATCT TCTGATGCTG AACAGTTCTC CATCCACAGC CAAAGACACT 480

CTAGACAATG GCGATTATGG AGAACCAGAC TATGACTGGA CCACGGGCCC CAGGGACGAC 540

GACGAGTCTG ATNGACACCT TGGAAGAAAA CAGGGGTTAC ATGGAAATTG AACAGTCAGT 600

GAAATCTTTT AAGATGCCAT CCTCAAATAT AGAAGAGGAA GACAGCCATT TCTTTTTTCA 660

TCTTATTATT TTTGCTTTTT GCATTGCTGT TGTTTACATT ACATATCACA ACAAAAGGAA 720

GATTTTTCTT CTGGTTCAAA GCAGGAAATG GCGTGATGGC CTTTGTTCCA AAACAGTGGA 780

ATACCATCGC CTAGATCAGA ATGTTAATGA GGCAATGCCT TCTTTGAAGA TTACCAATGA 840

TTATATTTTT TAAAGCACTG TGATTTGAAT TTGCTTATGT AATTTTATTT GCTTGACTTT 900

TTATATGATA TTGTGCAAAT GTTTGCCATA GGCAATTGGT ACTTAAATGA GAGGTGAGTC 960

TCTCTTTTGC CTTGGTGCTT TGGAAATTAA ATGTCACAAA CGAGTATATA ATTTTTTATC 1020

TGTACTTTTA GAGCTGAGTT TAATCAGGTG TCCAAAATGT GAGTTAAACA TTACCTTATA 1080

TTTACACTGT TAGTTTTTAT TGTTTTAGAT TTATTATGCT TCTTCTGGAA GTATTAGTGA 1140

TGCTACTTTT AAAAGATCCC AAACTTGTAA CTAAATTCTG ACATATCTGT TACTGCTGAC 1200

TCACATTCAT TCTCCGCCAT TCAAATACTA TTTTTTATCC ACATTTTTTT TTGTTCCCAA 1260

ACTGTAATGT ACAAGGATAT GTGTGATAAT GCTTTGGATT TGAGTAATAT TTTTTTTTCT 1320

TCCAAGAAAA CTGCTTTGGA TATTTTTAGA TAATTTAAAC ATAATTTAGG ATAATGATAT 1380

TGCTCAATCT GACCACAATT TTAGGTAAAA CATTAAATGT GTCAAGAAAT CTTGGCAACA 1440

GAGACTCTGC AGCTTGCAGT GGACATAGAT AAAATGTTAC AGAGATACTA TTTTTTTGGT 1500

TGGAATTACT ATATTAAATT TAGAAGCAGA AACTGGTAAA ATGTTAAATA CATGTACAAT 1560

TGCTTTTAGT TAGCAATTGA TTGTAGCATG GGTTCCTCCA AGGTTTCAAG CAATGGGCAG 1620

AGTTTAAAAT TATATCAGAT TCGTTTACTT CGTTTATTAT TTTACAGTAA ATTTGAATAA 1680

ATCTTAGGGG TCATTATCAC TTAAATAATA CTGTACCTAG GTCTTTCAAA TTAAAATTAT 1740

ACCTGAATGA AGTTGTTTGT ATACATAAAG GATATTTGTG TACAATTACC TTTTTTCCCC 1800

CACACTTGTT TTCTTTGTTT TTGTTTTTTA TGGCAACTGG AAAGTATTTA CTATGGGATT 1860

CATTTATGTC TGTCTTTCTA TCATAAAGAA TTGATCAATA TGTAAATATG TGATTTGAAC 1920

CATGGTTGAC TTACAAGTGT CACTACAGCT TTTTAGAAAA CATAGCCCTA ATATATGTTA 1980

AGCAGGACCC GGGTGAGCCA GTGGGCTTGC GCTTTATGTA GAGCTGGAAG AAGGCCGTCC 2040

ATCCTGTCTC TTGGGCGGAC AGTGTACTTT CCTAATAGGG AAGGGAAGCA CAATGGAAAT 2100

ACCCCTGAAC CGTTTTATTG CAGTAATTTT TTTCATATCT GAAACTATTA TTTAATATTT 2160

TGAATAAGAT TTTAAAAAAT AAATGGCAAA GATATAAATC TAAAAAAAAA AAAAAAAAAA 2220

AAAAAAAAAA AAAAAAAAAA AAAAAAAAAA AAAAAAAAAA AAAAAANANA N 2271

(2) INFORMATION FOR SEQ ID NO: 233:

(i) SEQUENCE CHARACTERISTICS:
(A) LENGTH: 1338 base pairs
(B) TYPE: nucleic acid
(C) STRANDEDNESS: double
(D) TOPOLOGY: linear

(xi) SEQUENCE DESCRIPTION: SEQ ID NO: 233:

CTTCCGGTTC TCCGGGCAGC TGCCACTGCT GTAGCTTCTG CCACCTGCCA CGACCGGGCC 60

TCTCCCTGGC GTTTGGTCAC CTCTGCTTCA TTCTCCACCG CGCCTATGGT CCCTCTTGGA 120

GCCAGCGTGG CGNGCCTGGC GGCTCCCGGG TGGTGAGAGA GCGGTCCGGG AACGATGAAG 180

GCCTCGCAGT GCTGCTGCTG TCTCAGCCAC CTCTTGGCTT CCGTCCTCCT CCTGCTGTTG 240

CTGCCTGAAC TAAGCGGGYC CCTGGMAGTC CTGCTGCAGG CAGCCGAGGC CGCGCCAGGT 300

YTTGGGCCTC CTGACCCTAG ACCAGGACAT TACCGCCGCT GCCACCGGGC CCTWACCCCT 360

GCCCAGCAGC CGGGCCGTGG TCTGGCTGAA GCTGCGGGGG CCGCGGGGCT CCGAGGGAGG 420

CAATGGCAGC AACCCTGTGG CCGGGCTTGA GACGGACGAT CACGGAGGGA AGGCCGGGGA 480

ARGCTCGGTG GGTGGCGGCC TTGCTGTGAG CCCCAACCCT GGCGACAAGC CCATGACCCA 540

GCGGGCCCTG ACCGTGTTGA TGGTGGTGAG CGGCGCGGTG CTGGTGTACT TCGTGGTCAG 600

GACGGTCAGG ATGAGAAGAA GAAACCGAAA GACTAGGAGA TATGGAGTTT TGGACACTAA 660

CATAGAAAAT ATGGAATTGA CACCTTTAGA ACAGGATGAT GAGGATGATG ACAACACGTT 720

GTTTGATGCC AATCATCCTC GAAGATAAGA ATGTGCCTTT TGATGAAAGA ACTTTATCTT 780

TCTACAATGA AGAGTGGAAT TTCTATGTTT AAGGAATAAG AAGCCACTAT ATCAATGTTG 840

GGGGGGTATT TAAGTTACAT ATATTTNAAC AACCTTTAAT TTGCTGTTGC AATAAATACC 900

GTATCCTTTT ATTATATCTT TATATGTATA GAAGTACTCT GTTAATGGGC TCAGAGATGT 960

TGGGGATAAA GTATACTGTA ATAATTTATC TGTTTGAAAA TTACTATAAA ACGGTGTTTT 1020

CTGRTCGGTT TTTGTTTCCT GCTTACCATA TGATTGTAAA TTGTTTTATG TATTAATCAG 1080

TTAATGCTAA TTATTTTTGC TGATGTCATA TGTTAAAGAG CTATAAATTC CAACAACCAA 1140

CTGGTGTGTA AAAATAATTT AAAATYTCCT TTACTGAAAG GTATTTCCCA TTTTTGTGGG 1200

GAAAAGAAGC CAAATTTATT ACTTTGTGTT GGGGTTTTTA AAATATTAAG AAATGTCTAA 1260

GTTATTGTTT GCAAAACAAT AAATATGATT TTAAATTCTC TTAAAAAAAA AAAAAAAAAC 1320

CCCGGGGGGG GGCCCGGN 1338

(2) INFORMATION FOR SEQ ID NO: 234:

(i) SEQUENCE CHARACTERISTICS:
(A) LENGTH: 31 amino acids
(B) TYPE: amino acid
(D) TOPOLOGY: linear
(xi) SEQUENCE DESCRIPTION: SEQ ID NO: 234:

Met Leu Ser Thr Gly Ile Glu Val Ala Arg Pro Pro Ala Thr Leu Leu
1               5                   10                  15

Gly Leu Met Phe Val Leu Thr Gly Met Pro Arg Gly Leu Arg Xaa
            20                  25                  30

(2) INFORMATION FOR SEQ ID NO: 235:

(i) SEQUENCE CHARACTERISTICS:
(A) LENGTH: 116 amino acids

(B) TYPE: amino acid
(D) TOPOLOGY: linear
(xi) SEQUENCE DESCRIPTION: SEQ ID NO: 235:

Met Asn Val Val Ile Val Ile Ile Leu Phe Ser Phe Asp Ser Val Gly
1               5                   10                  15

Thr Met Phe Ser Cys Asn Arg Ile Pro Lys Ile Thr Val Leu Asn Lys
            20                  25                  30

Leu Lys Phe Xaa Cys Glu Val Leu Leu Arg Ile Gln Thr Ile Gln Gly
        35                  40                  45

Phe Tyr Arg Cys Thr Arg Ile Ser Arg Tyr Lys Gly Ile Phe Pro Asp
    50                  55                  60

Phe Cys Gln Ser Gln Cys Met Gly Cys Asn Pro Glu Ser Xaa Met Ala
65                  70                  75                  80

Val Pro Ala Leu Val Thr Pro Ile Leu Ala His Arg Lys Lys Glu Lys
                85                  90                  95

Gly Met Cys Leu Phe Thr Leu Ile Ile Ala Pro Thr Arg Cys Thr His
            100                 105                 110

Tyr Phe Cys Xaa
        115

(2) INFORMATION FOR SEQ ID NO: 236:

(i) SEQUENCE CHARACTERISTICS:
(A) LENGTH: 103 amino acids
(B) TYPE: amino acid
(D) TOPOLOGY: linear
(xi) SEQUENCE DESCRIPTION: SEQ ID NO: 236:

Met Ser Ser Ala Lys Ile Val Arg Gln Arg Gly Ala Val Pro Thr Tyr
1               5                   10                  15

Tyr Thr Thr Glu Ala Gly Glu Ile Ile Phe Leu Val Leu Asn Trp Ser
            20                  25                  30

Leu Ser Ile Leu His Ile Val Asp Val Leu Cys Ser Lys Pro Glu Lys
        35                  40                  45

Ser Val Thr Glu Asp Ala Ala Ser Gly Leu Ser Gln Arg Met Thr Ala
    50                  55                  60

Leu Val Trp Arg Lys Gly Pro Asp Gly Gly Ser Arg Lys Pro Ile Leu
65                  70                  75                  80

Leu Leu Phe Phe Phe Leu Pro Leu Ile Leu Cys Phe His Ser Phe Ile
                85                  90                  95

His Ser Ser Asn Ile Cys Xaa
            100

(2) INFORMATION FOR SEQ ID NO: 237:

(i) SEQUENCE CHARACTERISTICS:
(A) LENGTH: 42 amino acids
(B) TYPE: amino acid
(D) TOPOLOGY: linear
(xi) SEQUENCE DESCRIPTION: SEQ ID NO: 237:

Met Ile Leu Phe Pro Gln Xaa Ala Leu Arg Leu Gly Xaa Trp Pro Arg
1               5                   10                  15

Thr Trp Ser Ile Leu Xaa Lys Tyr Ser Val Asn Phe Phe Ser Ala Tyr
            20                  25                  30

Ser Pro Met Gly Ala Val Gly Thr Glu Phe
        35                  40

(2) INFORMATION FOR SEQ ID NO: 238:

(i) SEQUENCE CHARACTERISTICS:
(A) LENGTH: 37 amino acids
(B) TYPE: amino acid
(D) TOPOLOGY: linear
(xi) SEQUENCE DESCRIPTION: SEQ ID NO: 238:

Met Ile Ile Leu Leu Leu Phe Met Leu Leu Asn Asn Val Val Leu Val
1               5                   10                  15

Gln Glu Asp Asn Cys Gln Arg Lys Asn Thr Val Gln Glu Arg Arg Xaa
            20                  25                  30

Trp Ser Gln Trp Xaa
        35

(2) INFORMATION FOR SEQ ID NO: 239:

(i) SEQUENCE CHARACTERISTICS:
(A) LENGTH: 128 amino acids
(B) TYPE: amino acid
(D) TOPOLOGY: linear
(xi) SEQUENCE DESCRIPTION: SEQ ID NO: 239:

Met Ala Ala Xaa Pro Pro Gly Cys Thr Pro Pro Xaa Leu Leu Asp Ile
1               5                   10                  15

Ser Trp Leu Thr Glu Ser Leu Gly Ala Gly Gln Pro Val Pro Val Glu
            20                  25                  30

Cys Arg His Arg Leu Glu Val Ala Gly Pro Arg Lys Gly Pro Leu Ser
        35                  40                  45

Pro Ala Trp Met Pro Ala Tyr Ala Cys Gln Arg Pro Thr Pro Leu Thr
    50                  55                  60

His His Asn Thr Gly Leu Ser Glu Leu Leu Glu His Gly Val Cys Glu

65                  70                  75                  80

Glu Val Glu Arg Val Arg Arg Ser Glu Arg Tyr Gln Thr Met Lys Val
                85                  90                  95

Arg Arg Ala Gly Leu Gly Pro Thr Pro Gly Met Ser Cys Pro Gly Asn
            100                 105                 110

Asp Asn Thr Val His Thr Met His Gly Glu Ala Asn Arg Gly Ser Xaa
        115                 120                 125

(2) INFORMATION FOR SEQ ID NO: 240:

(i) SEQUENCE CHARACTERISTICS:
(A) LENGTH: 67 amino acids
(B) TYPE: amino acid
(D) TOPOLOGY: linear
(xi) SEQUENCE DESCRIPTION: SEQ ID NO: 240:

Met Ser Ile Leu Cys Cys Pro Xaa Leu Cys Leu Phe Phe Ser Phe Cys
 1               5                  10                  15

Ile Ser Ser Gly Ser Cys Pro Phe Ser His Val Ser Gln Leu Ser Phe
            20                  25                  30

Ile Ala Thr Phe Ser Gln Ser Ser Pro Val Leu Leu Val Pro Ala Tyr
        35                  40                  45

Asn Thr Tyr Leu Ser Phe Leu Ala Phe Leu Asp Cys Ala Ser Leu Thr
    50                  55                  60

Ser Thr Xaa
65

(2) INFORMATION FOR SEQ ID NO: 241:

(i) SEQUENCE CHARACTERISTICS:
(A) LENGTH: 69 amino acids
(B) TYPE: amino acid
(D) TOPOLOGY: linear
(xi) SEQUENCE DESCRIPTION: SEQ ID NO: 241:

Met Ser Thr Phe Gln Leu Leu Leu Leu Ile Leu Ala Gln Ser Thr Tyr
 1               5                  10                  15

Lys Ile Lys Ser Lys Pro Leu His Met Thr Asn His Thr Leu Leu Asn
            20                  25                  30

Ser Pro Gly Leu Asn Pro Ser Ser Pro Thr Leu Asn Phe Lys Thr Gln
        35                  40                  45

Gln His Glu Ser Val Ser Tyr Ala Cys Cys His Met Arg Ser Leu His
    50                  55                  60

His Ala Phe Ala Xaa
65

(2) INFORMATION FOR SEQ ID NO: 242:

(i) SEQUENCE CHARACTERISTICS:
(A) LENGTH: 44 amino acids
(B) TYPE: amino acid
(D) TOPOLOGY: linear
(xi) SEQUENCE DESCRIPTION: SEQ ID NO: 242:

Met Val Ser Val Val Leu Ile Phe Ser Phe Leu Ser Leu Thr Ile Ser
 1               5                  10                  15

Thr Thr Ala Ser Ala Tyr Asn Gly Asn Asp Thr Gln Gly Trp Asn Asp
            20                  25                  30

Lys Phe His Xaa Xaa Ser Val Lys Thr Gln Thr Xaa
        35                  40

(2) INFORMATION FOR SEQ ID NO: 243:

(i) SEQUENCE CHARACTERISTICS:
(A) LENGTH: 51 amino acids
(B) TYPE: amino acid
(D) TOPOLOGY: linear
(xi) SEQUENCE DESCRIPTION: SEQ ID NO: 243:

Met Ile Ser Asp Ala Gly Ala Gly Phe Gly Val Phe Leu Leu Val Pro
 1               5                  10                  15

Arg Ala Gly His Cys Trp Gly Ala Gly Lys Pro Leu Pro Ser Cys Pro
            20                  25                  30

Ser Val Ala Ser Ile Pro Ser Trp Val Leu Pro Ser Phe Leu Glu Arg
        35                  40                  45

Gly Arg Xaa
    50

(2) INFORMATION FOR SEQ ID NO: 244:

(i) SEQUENCE CHARACTERISTICS:
(A) LENGTH: 43 amino acids
(B) TYPE: amino acid
(D) TOPOLOGY: linear
(xi) SEQUENCE DESCRIPTION: SEQ ID NO: 244:

Met Val Gln Thr Ile Gln Asp Phe Leu Ser Leu Phe Ser Thr Pro Ile
 1               5                  10                  15

Phe Leu Leu Leu Leu Met Phe Glu Thr Leu Ser Leu Ala Pro Ala Trp
            20                  25                  30

Leu Lys Pro Leu Arg Val Thr Ser His Ser Xaa
        35                  40

(2) INFORMATION FOR SEQ ID NO: 245:

(i) SEQUENCE CHARACTERISTICS:
(A) LENGTH: 61 amino acids
(B) TYPE: amino acid
(D) TOPOLOGY: linear
(xi) SEQUENCE DESCRIPTION: SEQ ID NO: 245:

Met Ile Leu Met Pro Gly Leu Gly Thr Ser Arg Gln Arg Ser Val Pro
1               5                   10                  15

Phe Val Pro Thr Leu Asn Ala Ser Thr Pro Gly Ala Met Thr Gly Pro
            20                  25                  30

Thr Ala Thr Leu Thr Ser Cys Gln Trp Thr Thr Ala Cys Arg Val Ser
        35                  40                  45

Trp Ala Asn Gly Trp Thr Ser Leu Arg Thr Phe Arg Xaa
    50                  55                  60

(2) INFORMATION FOR SEQ ID NO: 246:

(i) SEQUENCE CHARACTERISTICS:
(A) LENGTH: 36 amino acids
(B) TYPE: amino acid
(D) TOPOLOGY: linear
(xi) SEQUENCE DESCRIPTION: SEQ ID NO: 246:

Met Ser His His Ala Gln Pro Arg Phe Leu Leu Ile Thr Met Leu Leu
1               5                   10                  15

Gln Glu Ala Lys Pro Val Ser Asn Ile Pro His Leu Leu Glu Ser Trp
            20                  25                  30

Tyr Phe Gly Xaa
        35

(2) INFORMATION FOR SEQ ID NO: 247:

(i) SEQUENCE CHARACTERISTICS:
(A) LENGTH: 3[?] amino acids
(B) TYPE: amino acid
(D) TOPOLOGY: linear
(xi) SEQUENCE DESCRIPTION: SEQ ID NO: 247:

Met Asn Ser Leu Phe Trp Met Ile Leu Leu Pro Val Ser Gln Asp Gln
1               5                   10                  15

Val Val Glu Gly Leu Gln Gly Gly Phe Ser Gln Ile His Met Arg Ile
            20                  25                  30

Leu Arg Lys His Leu Xaa
        35

(2) INFORMATION FOR SEQ ID NO: 248:

        (i) SEQUENCE CHARACTERISTICS:
              (A) LENGTH: 211 amino acids
              (B) TYPE: amino acid
              (D) TOPOLOGY: linear
        (xi) SEQUENCE DESCRIPTION: SEQ ID NO: 248:

Met Ser Arg Ser Xaa Asp Val Thr Asn Thr Thr Phe Leu Leu Met Ala
  1               5                  10                  15

Ala Ser Ile Tyr Leu His Asp Gln Asn Pro Asp Ala Ala Leu Arg Ala
             20                  25                  30

Leu His Gln Gly Asp Ser Leu Glu Cys Thr Ala Met Thr Val Gln Ile
        35                  40                  45

Leu Leu Lys Leu Asp Arg Leu Asp Leu Ala Arg Lys Glu Leu Lys Arg
    50                  55                  60

Met Gln Asp Leu Asp Glu Asp Ala Thr Leu Thr Gln Leu Ala Thr Ala
 65                  70                  75                  80

Trp Val Ser Leu Ala Thr Gly Gly Glu Lys Leu Gln Asp Ala Tyr Tyr
                 85                  90                  95

Ile Phe Gln Glu Met Ala Asp Lys Cys Ser Pro Thr Leu Leu Leu Leu
            100                 105                 110

Asn Gly Gln Ala Ala Cys His Met Ala Gln Gly Arg Trp Glu Ala Ala
        115                 120                 125

Glu Gly Leu Leu Gln Glu Ala Leu Asp Lys Asp Ser Gly Tyr Pro Glu
    130                 135                 140

Thr Leu Val Asn Leu Ile Val Leu Ser Gln His Leu Gly Lys Pro Pro
145                 150                 155                 160

Glu Val Thr Asn Arg Tyr Leu Ser Gln Leu Lys Asp Ala His Arg Ser
                165                 170                 175

His Pro Phe Ile Lys Glu Tyr Gln Ala Lys Glu Asn Asp Phe Asp Arg
            180                 185                 190

Leu Val Leu Gln Tyr Ala Pro Ser Ala Glu Ala Gly Pro Glu Leu Ser
        195                 200                 205

Gly Pro Xaa
    210

(2) INFORMATION FOR SEQ ID NO: 249:

(i) SEQUENCE CHARACTERISTICS:
(A) LENGTH: 548 amino acids
(B) TYPE: amino acid
(D) TOPOLOGY: linear
(xi) SEQUENCE DESCRIPTION: SEQ ID NO: 249:

Met Glu Asp Ser Glu Ala Leu Gly Phe Glu His Met Gly Leu Asp Pro
1               5                   10                  15

Arg Leu Leu Gln Ala Val Thr Asp Leu Gly Trp Ser Arg Pro Thr Leu
            20                  25                  30

Ile Gln Glu Lys Ala Ile Pro Leu Ala Leu Glu Gly Lys Asp Leu Leu
        35                  40                  45

Ala Arg Ala Arg Thr Gly Ser Gly Lys Thr Ala Ala Tyr Ala Ile Pro
    50                  55                  60

Met Leu Gln Leu Leu Leu His Arg Lys Ala Thr Gly Pro Val Val Glu
65                  70                  75                  80

Gln Ala Val Arg Gly Leu Val Leu Val Pro Thr Lys Glu Leu Ala Arg
                85                  90                  95

Gln Ala Gln Ser Met Ile Gln Gln Leu Ala Thr Tyr Cys Ala Arg Asp
            100                 105                 110

Val Arg Val Ala Asn Val Ser Ala Ala Glu Asp Ser Val Ser Gln Arg
        115                 120                 125

Ala Val Leu Met Glu Lys Pro Asp Val Val Val Gly Thr Pro Ser Arg
    130                 135                 140

Ile Leu Ser His Leu Gln Gln Asp Ser Leu Lys Leu Arg Asp Ser Leu
145                 150                 155                 160

Glu Leu Leu Val Val Asp Glu Ala Asp Leu Leu Phe Ser Phe Gly Phe
                165                 170                 175

Glu Glu Glu Leu Lys Ser Leu Leu Cys His Leu Pro Arg Ile Tyr Gln
            180                 185                 190

Ala Phe Leu Met Ser Ala Thr Phe Asn Glu Asp Val Gln Ala Leu Lys
        195                 200                 205

Glu Leu Ile Leu His Asn Pro Val Thr Leu Lys Leu Gln Glu Ser Gln
    210                 215                 220

Leu Pro Gly Pro Asp Gln Leu Gln Gln Phe Gln Val Val Cys Glu Thr
225                 230                 235                 240

Glu Glu Asp Lys Phe Leu Leu Leu Tyr Ala Leu Leu Lys Leu Ser Leu
                245                 250                 255

Ile Arg Gly Lys Ser Leu Leu Phe Val Asn Thr Leu Glu Arg Ser Tyr
            260                 265                 270

Arg Leu Arg Leu Phe Leu Glu Gln Phe Ser Ile Pro Thr Cys Val Leu
        275                 280                 285

Asn Gly Glu Leu Pro Leu Arg Ser Arg Cys His Ile Ile Ser Gln Phe
    290                 295                 300

Asn Gln Gly Phe Tyr Asp Cys Val Ile Ala Thr Asp Ala Glu Val Leu
305                 310                 315                 320

Gly Ala Pro Val Lys Gly Lys Arg Arg Gly Arg Gly Pro Lys Gly Asp
                325                 330                 335

Lys Ala Ser Asp Pro Glu Ala Gly Val Ala Arg Gly Ile Asp Phe His
            340                 345                 350

His Val Ser Ala Val Leu Asn Phe Asp Leu Pro Pro Thr Pro Glu Ala
        355                 360                 365

Tyr Ile His Arg Ala Gly Arg Thr Ala Arg Ala Asn Asn Pro Gly Ile
    370                 375                 380

Val Leu Thr Phe Val Leu Pro Thr Glu Gln Phe His Leu Gly Lys Ile
385                 390                 395                 400

Glu Glu Leu Leu Ser Gly Glu Asn Arg Gly Pro Ile Leu Leu Pro Tyr
                405                 410                 415

Gln Phe Arg Met Glu Glu Ile Glu Gly Phe Arg Tyr Arg Cys Arg Asp
            420                 425                 430

Ala Met Arg Ser Val Thr Lys Gln Ala Ile Arg Glu Ala Arg Leu Lys
        435                 440                 445

Glu Ile Lys Glu Glu Leu Leu His Ser Glu Lys Leu Lys Thr Tyr Phe
    450                 455                 460

Glu Asp Asn Pro Arg Asp Leu Gln Leu Leu Arg His Asp Leu Pro Leu
465                 470                 475                 480

His Pro Ala Val Val Lys Pro His Leu Gly His Val Pro Asp Tyr Leu
                485                 490                 495

Val Pro Pro Ala Leu Arg Gly Leu Val Arg Pro His Lys Lys Arg Lys
            500                 505                 510

Lys Leu Ser Ser Ser Cys Arg Lys Ala Lys Arg Ala Lys Ser Gln Asn
        515                 520                 525

Pro Leu Arg Ser Phe Lys His Lys Gly Lys Lys Phe Arg Pro Thr Ala
    530                 535                 540

Lys Pro Ser Xaa
545

(2) INFORMATION FOR SEQ ID NO: 250:

    (i) SEQUENCE CHARACTERISTICS:
        (A) LENGTH: 299 amino acids
        (B) TYPE: amino acid
        (D) TOPOLOGY: linear
    (xi) SEQUENCE DESCRIPTION: SEQ ID NO: 250:

Met Thr Thr Val Pro Pro Ser Pro Arg Pro Met Ser Arg Pro Ser Glu
1               5                   10                  15

Arg Asn Met Arg Arg Pro Arg Gly Pro Ser Pro Leu Pro Ala Ser Pro
            20                  25                  30

Arg Asn Ser Thr Pro Asp Glu Pro Asp Val His Phe Ser Lys Lys Phe
        35                  40                  45

Leu Asn Val Phe Met Ser Gly Arg Ser Arg Ser Ser Ser Ala Glu Ser
    50                  55                  60

Phe Gly Leu Phe Ser Cys Ile Ile Asn Gly Glu Glu Gln Glu Gln Thr
65                  70                  75                  80

His Arg Ala Ile Phe Arg Phe Val Pro Arg His Glu Asp Glu Leu Glu
                85                  90                  95

Leu Glu Val Asp Asp Pro Leu Leu Val Glu Leu Gln Ala Glu Asp Tyr
            100                 105                 110

Trp Tyr Glu Ala Tyr Asn Met Arg Thr Gly Ala Arg Gly Val Phe Pro
        115                 120                 125

Ala Tyr Tyr Ala Ile Glu Val Thr Lys Glu Pro Glu His Met Ala Ala
    130                 135                 140

Leu Ala Lys Asn Ser Asp Trp Val Asp Gln Phe Arg Val Lys Phe Leu
145                 150                 155                 160

Gly Ser Val Gln Val Pro Tyr His Lys Gly Asn Asp Val Leu Cys Ala
                165                 170                 175

Ala Met Gln Lys Ile Ala Thr Thr Arg Arg Leu Thr Val His Phe Asn
            180                 185                 190

Pro Pro Ser Ser Cys Val Leu Glu Ile Ser Val Arg Gly Val Lys Ile
        195                 200                 205

Gly Val Lys Ala Asp Asp Ser Gln Glu Ala Lys Gly Asn Lys Cys Ser
    210                 215                 220

His Phe Phe Gln Leu Lys Asn Ile Ser Phe Cys Gly Tyr His Pro Lys
225                 230                 235                 240

Asn Asn Lys Tyr Phe Gly Phe Ile Thr Lys His Pro Ala Asp His Arg
                245                 250                 255

Phe Ala Cys His Val Phe Val Ser Glu Asp Ser Thr Lys Ala Leu Ala
            260                 265                 270

Glu Ser Val Gly Arg Ala Phe Gln Gln Phe Tyr Lys Gln Phe Val Glu
        275                 280                 285

Tyr Thr Cys Pro Thr Glu Asp Ile Tyr Leu Glu
    290                 295

(2) INFORMATION FOR SEQ ID NO: 251:

(i) SEQUENCE CHARACTERISTICS:
(A) LENGTH: 40 amino acids
(B) TYPE: amino acid
(D) TOPOLOGY: linear
(xi) SEQUENCE DESCRIPTION: SEQ ID NO: 251:

Leu Leu Tyr Leu Leu Lys Val Xaa Val Ile Phe Val Phe Ser Ser Ser
1               5                   10                  15

Lys Gly Val Thr Leu Val Ser Met Asn Leu Thr Ser Phe Phe Val Ser
            20                  25                  30

Ser Val Leu Ala Cys Phe Ser Xaa
        35                  40

(2) INFORMATION FOR SEQ ID NO: 252:

(i) SEQUENCE CHARACTERISTICS:
(A) LENGTH: 594 amino acids
(B) TYPE: amino acid
(D) TOPOLOGY: linear
(xi) SEQUENCE DESCRIPTION: SEQ ID NO: 252:

Met Pro Ala Ser Ser Leu Glu Ser Arg Ser Phe Leu Leu Ala Lys Lys
1               5                   10                  15

Ser Gly Glu Asn Val Ala Lys Phe Ile Ile Asn Ser Tyr Pro Lys Tyr
            20                  25                  30

Phe Gln Lys Asp Ile Ala Glu Pro His Ile Pro Cys Leu Met Pro Glu
        35                  40                  45

Tyr Phe Glu Pro Gln Ile Lys Asp Ile Ser Glu Ala Ala Leu Lys Glu
    50                  55                  60

Arg Ile Glu Leu Arg Lys Val Lys Ala Ser Val Asp Met Phe Asp Gln
65                  70                  75                  80

Leu Leu Gln Ala Gly Thr Thr Val Ser Leu Glu Thr Thr Asn Ser Leu
                85                  90                  95

Leu Asp Xaa Leu Cys Tyr Tyr Gly Asp Gln Glu Pro Ser Thr Asp Tyr
            100                 105                 110

His Phe Gln Gln Thr Gly Gln Ser Glu Ala Leu Glu Glu Glu Asn Asp
        115                 120                 125

Glu Thr Ser Arg Arg Lys Ala Gly His Gln Phe Gly Val Thr Trp Arg
    130                 135                 140

Ala Lys Asn Asn Ala Glu Arg Ile Phe Ser Leu Met Pro Glu Lys Asn
145                 150                 155                 160

Glu His Ser Tyr Cys Thr Met Ile Arg Gly Met Val Lys His Arg Ala
                165                 170                 175

Tyr Glu Gln Ala Leu Asn Leu Tyr Thr Glu Leu Leu Asn Asn Arg Leu
            180                 185                 190

His Ala Asp Val Tyr Thr Phe Asn Ala Leu Ile Glu Ala Thr Val Cys
        195                 200                 205

Ala Ile Asn Glu Lys Phe Glu Glu Lys Trp Ser Lys Ile Leu Glu Leu
    210                 215                 220

Leu Arg His Met Val Ala Gln Lys Val Lys Pro Asn Leu Gln Thr Phe
225                 230                 235                 240

Asn Thr Ile Leu Lys Cys Leu Arg Arg Phe His Val Phe Ala Arg Ser
                245                 250                 255

Pro Ala Leu Gln Val Leu Arg Glu Met Lys Ala Ile Gly Ile Glu Pro
            260                 265                 270

Ser Leu Ala Thr Tyr His His Ile Ile Arg Leu Phe Asp Gln Pro Gly
        275                 280                 285

Asp Pro Leu Lys Arg Ser Ser Phe Ile Ile Tyr Asp Ile Met Asn Glu
    290                 295                 300

Leu Met Gly Lys Arg Phe Ser Pro Lys Asp Pro Asp Asp Asp Lys Phe
305                 310                 315                 320

Phe Gln Ser Ala Met Ser Ile Cys Ser Ser Leu Arg Asp Leu Glu Leu
                325                 330                 335

Ala Tyr Gln Val His Gly Leu Leu Lys Thr Gly Asp Asn Trp Lys Phe
            340                 345                 350

Ile Gly Pro Asp Gln His Arg Asn Phe Tyr Tyr Ser Lys Phe Phe Asp
        355                 360                 365

Leu Ile Cys Leu Met Glu Gln Ile Asp Val Thr Leu Lys Trp Tyr Glu
    370                 375                 380

Asp Leu Ile Pro Ser Ala Tyr Phe Pro His Ser Gln Thr Met Ile His
385                 390                 395                 400

Leu Leu Gln Ala Leu Asp Val Ala Asn Arg Leu Glu Val Ile Pro Lys
                405                 410                 415

Ile Trp Lys Asp Ser Lys Glu Tyr Gly His Thr Phe Arg Ser Asp Leu
            420                 425                 430

Arg Glu Glu Ile Leu Met Leu Met Ala Arg Asp Lys His Pro Pro Glu
        435                 440                 445

Leu Gln Val Ala Phe Ala Asp Cys Ala Ala Asp Ile Lys Ser Ala Tyr
    450                 455                 460

Glu Ser Gln Pro Ile Arg Gln Thr Ala Gln Asp Trp Pro Ala Thr Ser
465                 470                 475                 480

Leu Asn Cys Ile Ala Ile Leu Phe Leu Arg Ala Gly Arg Thr Gln Glu
                485                 490                 495

Ala Trp Lys Met Leu Gly Leu Phe Arg Lys His Asn Lys Ile Pro Arg
            500                 505                 510

Ser Glu Leu Leu Asn Glu Leu Met Asp Ser Ala Lys Val Ser Asn Ser
        515                 520                 525

Pro Ser Gln Ala Ile Glu Val Val Glu Leu Ala Ser Ala Phe Ser Leu
    530                 535                 540

Pro Ile Cys Glu Gly Leu Thr Gln Arg Val Met Ser Asp Phe Ala Ile
545                 550                 555                 560

Asn Gln Glu Gln Lys Glu Ala Leu Ser Asn Leu Thr Ala Leu Thr Ser
                565                 570                 575

Asp Ser Asp Thr Asp Ser Ser Ser Asp Ser Asp Ser Asp Thr Ser Glu
            580                 585                 590

Gly Lys

(2) INFORMATION FOR SEQ ID NO: 253:

(i) SEQUENCE CHARACTERISTICS:
(A) LENGTH: 131 amino acids
(B) TYPE: amino acid
(D) TOPOLOGY: linear
(xi) SEQUENCE DESCRIPTION: SEQ ID NO: 253:

Met Lys Leu Asn Leu Cys Ile Pro Asn Trp Ala Arg Cys Pro Leu Leu
1               5                   10                  15

Leu Leu Phe Pro Gln Leu Leu Pro Phe Gln Gly Glu Asp Asp Asp Pro
            20                  25                  30

Leu Lys Ala Lys Ala Ala Asn Leu Val Glu Ala Val Pro Trp Gly Ile
        35                  40                  45

Lys Ala Pro Ser Phe Gln Val Thr Cys Leu Val Arg Val Gln Leu Gln
    50                  55                  60

Ser Cys Thr Pro Ser Arg Pro Ser Thr Leu Leu Ala Thr Ser Gln Ser
65                  70                  75                  80

Pro Gly Arg Ile Ser Cys Tyr Ser Pro Leu Ser His Leu Pro Pro Val
                85                  90                  95

Thr Thr Ser Ile Gln Pro Ser Pro Val Met Val Pro Phe Gln Tyr Gln
            100                 105                 110

Ala Phe Leu Leu Gln Val Lys Glu Pro Ala Ala Gln Thr Leu Leu Gly
        115                 120                 125

Gln Gln Xaa
    130

(2) INFORMATION FOR SEQ ID NO: 254:

(i) SEQUENCE CHARACTERISTICS:
(A) LENGTH: 21 amino acids
(B) TYPE: amino acid
(D) TOPOLOGY: linear
(xi) SEQUENCE DESCRIPTION: SEQ ID NO: 254:

Met Arg Tyr His Ala Gln Leu Ile Phe Cys Ile Phe Cys Xaa Phe Val
1               5                   10                  15

Phe Val Xaa Lys Xaa
            20

(2) INFORMATION FOR SEQ ID NO: 255:

(i) SEQUENCE CHARACTERISTICS:
(A) LENGTH: 31 amino acids
(B) TYPE: amino acid
(D) TOPOLOGY: linear
(xi) SEQUENCE DESCRIPTION: SEQ ID NO: 255:

Met Asn Asp Asn Ser Pro Asn His Ser Ser Ser Tyr Leu Pro Leu Pro
1               5                   10                  15

Leu Thr Ile Val Ile Leu Gln Thr Gly His Lys Gly Thr Leu Xaa
            20                  25                  30

(2) INFORMATION FOR SEQ ID NO: 256:

(i) SEQUENCE CHARACTERISTICS:
(A) LENGTH: 219 amino acids
(B) TYPE: amino acid
(D) TOPOLOGY: linear
(xi) SEQUENCE DESCRIPTION: SEQ ID NO: 256:

Met His Phe Leu Phe Arg Phe Ile Val Phe Phe Tyr Leu Trp Gly Leu
1               5                   10                  15

Phe Thr Ala Gln Arg Gln Lys Lys Glu Glu Ser Thr Glu Glu Val Lys
            20                  25                  30

Ile Glu Val Leu His Arg Pro Glu Asn Cys Ser Lys Thr Ser Lys Lys
        35                  40                  45

Gly Asp Leu Leu Asn Ala His Tyr Asp Gly Tyr Leu Ala Lys Asp Gly
    50                  55                  60

Ser Lys Phe Tyr Cys Ser Arg Thr Gln Asn Glu Gly His Pro Lys Trp
65                  70                  75                  80

Phe Val Leu Gly Val Gly Gln Val Ile Lys Gly Leu Asp Ile Ala Met
                85                  90                  95

Thr Asp Met Cys Pro Gly Glu Lys Arg Lys Val Val Ile Pro Pro Ser
            100                 105                 110

Phe Ala Tyr Gly Lys Glu Gly Tyr Ala Glu Gly Lys Ile Pro Pro Asp
        115                 120                 125

Ala Thr Leu Ile Phe Glu Ile Glu Leu Tyr Ala Val Thr Lys Gly Pro
    130                 135                 140

Arg Ser Ile Glu Thr Phe Lys Gln Ile Asp Met Asp Asn Asp Arg Gln
145                 150                 155                 160

Leu Ser Lys Ala Glu Ile Asn Leu Tyr Leu Gln Arg Glu Phe Glu Lys
                165                 170                 175

Asp Glu Lys Pro Arg Asp Lys Ser Tyr Gln Asp Ala Val Leu Glu Asp
            180                 185                 190

Ile Phe Lys Lys Asn Asp His Asp Gly Asp Gly Phe Ile Ser Pro Lys
        195                 200                 205

Glu Tyr Asn Val Tyr Gln His Asp Glu Leu Xaa
    210                 215

(2) INFORMATION FOR SEQ ID NO: 257:

        (i) SEQUENCE CHARACTERISTICS:
                (A) LENGTH: 50 amino acids
                (B) TYPE: amino acid
                (D) TOPOLOGY: linear
        (xi) SEQUENCE DESCRIPTION: SEQ ID NO: 257:

Met Trp Val Ile Arg Val Phe Gln Lys Thr Phe Leu Phe Phe Val Leu
  1               5                  10                  15

Phe Trp Ser Val His Cys Ile Ser Asp Lys Phe Gly Cys Leu Trp His
             20                  25                  30

Val Cys Met Lys Arg Glu Gly Asp Xaa Asn Cys Leu Ser Phe Ser Xaa
         35                  40                  45

Leu Xaa
     50

(2) INFORMATION FOR SEQ ID NO: 258:

        (i) SEQUENCE CHARACTERISTICS:
                (A) LENGTH: 122 amino acids
                (B) TYPE: amino acid
                (D) TOPOLOGY: linear
        (xi) SEQUENCE DESCRIPTION: SEQ ID NO: 258:
   Met Pro Ser Gln Thr Glu Xaa Phe Ala Ala Cys Gly Gly His Ser Leu
     1               5                  10                  15
   Leu Leu Val Xaa Leu Pro Leu Gly Leu Pro Phe Cys Pro Arg Ala Ala
               20                  25                  30
   Leu Cys Asp Leu Pro Phe Ser Leu Pro Ser Phe Pro Gly Gln Ala Arg
   35 40 45
   Arg Gly Gly Ala Glu Lys Gln Gly Ala Glu Gly Arg Gly Leu Gln Val
   50 55 60
   Lys Pro Arg Gly Gln Arg Thr Phe Gln Val Ser Arg Thr Ala Pro Ala
   65 70 75 80
   Ala Pro Arg Ser Arg Gln Pro Arg Pro Pro Ala Ala Leu Pro Ala Leu
   85 90 95
   Gly Phe Gly Gly Arg Gly Val Ala Lys Gly Arg Phe Leu Cys Phe Trp
   100 105 110
   Cys Leu Tyr Met Leu Arg Ile Asp Gln Xaa
   115 120

(2) INFORMATION FOR SEQ ID NO: 259:

(i) SEQUENCE CHARACTERISTICS:
(A) LENGTH: 88 amino acids
(B) TYPE: amino acid
(D) TOPOLOGY: linear
(xi) SEQUENCE DESCRIPTION: SEQ ID NO: 259:

Met Thr Ala Phe Cys Ser Leu Leu Leu Gln Ala Gln Ser Leu Leu Pro
1               5                   10                  15

Arg Thr Met Ala Ala Pro Gln Asp Ser Leu Arg Pro Gly Glu Glu Asp
            20                  25                  30

Glu Gly Met Gln Leu Leu Gln Thr Lys Asp Ser Met Ala Lys Gly Ala
        35                  40                  45

Arg Pro Gly Ala Xaa Arg Gly Arg Ala Arg Trp Gly Leu Ala Tyr Thr
    50                  55                  60

Leu Leu His Asn Pro Thr Leu Gln Val Phe Arg Lys Thr Ala Leu Leu
65                  70                  75                  80

Gly Ala Asn Gly Ala Gln Pro Xaa
                85

(2) INFORMATION FOR SEQ ID NO: 260:

(i) SEQUENCE CHARACTERISTICS:
(A) LENGTH: 26 amino acids
(B) TYPE: amino acid
(D) TOPOLOGY: linear
(xi) SEQUENCE DESCRIPTION: SEQ ID NO: 260:

Met Ile Gln Val Ser Val Pro Leu Leu Thr Ile Met Ile Phe Leu Leu
1               5                   10                  15

Tyr Leu Gln Ile Gly Pro Gly Lys Leu Xaa

20                  25

(2) INFORMATION FOR SEQ ID NO: 261:

(i) SEQUENCE CHARACTERISTICS:
(A) LENGTH: 29 amino acids
(B) TYPE: amino acid
(D) TOPOLOGY: linear
(xi) SEQUENCE DESCRIPTION: SEQ ID NO: 261:

Met Leu Leu Asp Pro Phe Ile Leu Leu Phe Cys Leu Phe Ser Thr Ala
1               5                   10                  15

Ala Gln Ser Cys Leu Glu Phe Ile Tyr Ile Gln Phe Xaa
            20                  25

(2) INFORMATION FOR SEQ ID NO: 262:

(i) SEQUENCE CHARACTERISTICS:
(A) LENGTH: 44 amino acids
(B) TYPE: amino acid
(D) TOPOLOGY: linear
(xi) SEQUENCE DESCRIPTION: SEQ ID NO: 262:

Met Lys Phe Leu Ser Ile Leu Leu Asp Asp Asn Asn Phe Xaa Leu Met
1               5                   10                  15

Leu Met Leu Ala Pro Phe Gly Cys Leu Ala Phe Glu Arg Ser Met Lys
            20                  25                  30

Met Arg Asn Gly Ala Leu Gly Leu Glu Glu Val Xaa
        35                  40

(2) INFORMATION FOR SEQ ID NO: 263:

(i) SEQUENCE CHARACTERISTICS:
(A) LENGTH: 363 amino acids
(B) TYPE: amino acid
(D) TOPOLOGY: linear
(xi) SEQUENCE DESCRIPTION: SEQ ID NO: 263:

Met Arg Thr Leu Phe Asn Leu Leu Trp Leu Ala Leu Ala Cys Ser Pro
1               5                   10                  15

Val His Thr Thr Leu Ser Lys Ser Asp Ala Lys Lys Ala Ala Ser Lys
            20                  25                  30

Thr Leu Leu Glu Lys Ser Gln Phe Ser Asp Lys Pro Val Gln Asp Arg
        35                  40                  45

Gly Leu Val Val Thr Asp Leu Lys Ala Glu Ser Val Val Leu Glu His
    50                  55                  60

Arg Ser Tyr Cys Ser Ala Lys Ala Arg Asp Arg His Phe Ala Gly Asp

65                  70                  75                  80

Val Leu Gly Tyr Val Thr Pro Trp Asn Ser His Gly Tyr Asp Val Thr
                85                  90                  95

Lys Val Phe Gly Ser Lys Phe Thr Gln Ile Ser Pro Val Trp Leu Gln
            100                 105                 110

Leu Lys Arg Arg Gly Arg Glu Met Phe Glu Val Thr Gly Leu His Asp
        115                 120                 125

Val Asp Gln Gly Trp Met Arg Ala Val Arg Lys His Ala Lys Gly Leu
    130                 135                 140

His Ile Val Pro Arg Leu Leu Phe Glu Asp Trp Thr Tyr Asp Asp Phe
145                 150                 155                 160

Arg Asn Val Leu Asp Ser Glu Asp Glu Ile Glu Glu Leu Ser Lys Thr
                165                 170                 175

Val Val Gln Val Ala Lys Asn Gln His Phe Asp Gly Phe Val Val Glu
            180                 185                 190

Val Trp Asn Gln Leu Leu Ser Gln Lys Arg Val Thr Asp Gln Leu Gly
        195                 200                 205

Met Phe Thr His Lys Glu Phe Glu Gln Leu Ala Pro Val Leu Asp Gly
    210                 215                 220

Phe Ser Leu Met Thr Tyr Asp Tyr Ser Thr Ala His Gln Pro Gly Pro
225                 230                 235                 240

Asn Ala Pro Leu Ser Trp Val Arg Ala Cys Val Gln Val Leu Asp Pro
                245                 250                 255

Lys Ser Lys Trp Arg Ser Lys Ile Leu Leu Gly Leu Asn Phe Tyr Gly
            260                 265                 270

Met Asp Tyr Ala Thr Ser Lys Asp Ala Arg Glu Pro Val Val Gly Ala
        275                 280                 285

Arg Tyr Ile Gln Thr Leu Lys Asp His Arg Pro Arg Met Val Trp Asp
    290                 295                 300

Ser Gln Xaa Ser Glu His Phe Phe Glu Tyr Lys Lys Ser Arg Ser Gly
305                 310                 315                 320

Arg His Val Val Phe Tyr Pro Thr Leu Lys Ser Leu Gln Val Arg Leu
                325                 330                 335

Glu Leu Ala Arg Glu Leu Gly Val Gly Val Ser Ile Trp Glu Leu Gly
            340                 345                 350

Gln Gly Leu Asp Tyr Phe Tyr Asp Leu Leu Xaa
        355                 360

(2) INFORMATION FOR SEQ ID NO: 264:

(i) SEQUENCE CHARACTERISTICS:
(A) LENGTH: 128 amino acids
(B) TYPE: amino acid
(D) TOPOLOGY: linear
(xi) SEQUENCE DESCRIPTION: SEQ ID NO: 264:

Leu Pro Thr Lys Ile Leu Val Lys Pro Asp Arg Thr Phe Glu Ile Lys
1               5                   10                  15

Ile Gly Gln Pro Thr Val Ser Tyr Phe Leu Lys Ala Ala Ala Gly Ile
            20                  25                  30

Glu Lys Gly Ala Arg Gln Thr Gly Lys Glu Val Ala Gly Leu Val Thr
        35                  40                  45

Leu Lys His Val Tyr Glu Ile Ala Arg Ile Lys Ala Gln Asp Glu Ala
    50                  55                  60

Phe Ala Leu Gln Asp Val Pro Leu Ser Ser Val Val Arg Ser Ile Ile
65                  70                  75                  80

Gly Ser Ala Arg Ser Leu Gly Ile Arg Val Val Lys Asp Leu Ser Ser
                85                  90                  95

Glu Glu Leu Ala Ala Phe Gln Lys Glu Arg Ala Ile Phe Leu Ala Ala
            100                 105                 110

Gln Lys Glu Ala Asp Leu Ala Ala Gln Glu Glu Ala Ala Lys Lys Xaa
        115                 120                 125

(2) INFORMATION FOR SEQ ID NO: 265:

(i) SEQUENCE CHARACTERISTICS:
(A) LENGTH: 54 amino acids
(B) TYPE: amino acid
(D) TOPOLOGY: linear
(xi) SEQUENCE DESCRIPTION: SEQ ID NO: 265:

Met Leu Leu Gln Ile His Pro Leu Leu Pro Ser Pro Thr Ile Pro His
1               5                   10                  15

Ile Leu Leu Leu Phe Leu Tyr Pro Thr Phe Ser Ile Leu Glu His Ser
            20                  25                  30

Cys Ser Tyr Cys Ile Glu Tyr Leu Trp Val Cys Leu Leu Phe Cys Leu
        35                  40                  45

Ser Leu Trp Phe Leu Xaa
    50

(2) INFORMATION FOR SEQ ID NO: 266:

(i) SEQUENCE CHARACTERISTICS:

(A) LENGTH: 29 amino acids
(B) TYPE: amino acid
(D) TOPOLOGY: linear
(xi) SEQUENCE DESCRIPTION: SEQ ID NO: 266:

Met Cys Leu Trp Cys Cys Gly Asp Val Cys Ser Gly Leu Ser Ser Leu
1               5                   10                  15

Leu Ser Leu Cys Val Cys Cys Val Val Leu Ala Val Cys
            20                  25

(2) INFORMATION FOR SEQ ID NO: 267:

(i) SEQUENCE CHARACTERISTICS:
(A) LENGTH: 26 amino acids
(B) TYPE: amino acid
(D) TOPOLOGY: linear
(xi) SEQUENCE DESCRIPTION: SEQ ID NO: 267:

Glu Gly Leu Arg Leu Leu Leu Ser Leu Pro Ala Ala Leu Pro Arg Ser
1               5                   10                  15

Cys Cys His Pro Arg Trp Leu Pro Val Xaa
            20                  25

(2) INFORMATION FOR SEQ ID NO: 268:

(i) SEQUENCE CHARACTERISTICS:
(A) LENGTH: 221 amino acids
(B) TYPE: amino acid
(D) TOPOLOGY: linear
(xi) SEQUENCE DESCRIPTION: SEQ ID NO: 268:

Met Phe His Gly Ile Pro Ala Thr Pro Gly Ile Gly Ala Pro Gly Asn
1               5                   10                  15

Lys Pro Glu Leu Tyr Glu Glu Val Lys Leu Tyr Lys Asn Ala Arg Glu
            20                  25                  30

Arg Glu Lys Tyr Asp Asn Met Ala Glu Leu Phe Ala Val Val Lys Thr
        35                  40                  45

Met Gln Ala Leu Glu Lys Ala Tyr Ile Lys Asp Cys Val Ser Pro Ser
    50                  55                  60

Glu Tyr Thr Ala Ala Cys Ser Arg Leu Leu Val Gln Tyr Lys Ala Ala
65                  70                  75                  80

Phe Arg Gln Val Gln Gly Ser Glu Ile Ser Ser Ile Asp Glu Phe Cys
                85                  90                  95

Arg Lys Phe Arg Leu Asp Cys Pro Leu Ala Met Glu Arg Ile Lys Glu
            100                 105                 110

Asp Arg Pro Ile Thr Ile Lys Asp Asp Lys Gly Asn Leu Asn Arg Cys
        115                 120                 125

Ile Ala Asp Val Val Ser Leu Phe Ile Thr Val Met Asp Lys Leu Arg
    130                 135                 140

Leu Glu Ile Arg Ala Met Asp Glu Ile Gln Pro Asp Leu Arg Glu Leu
145                 150                 155                 160

Met Glu Thr Met His Arg Met Ser His Leu Pro Pro Asp Phe Glu Gly
                165                 170                 175

Arg Gln Thr Val Ser Gln Trp Leu Gln Thr Leu Ser Gly Met Ser Ala
            180                 185                 190

Ser Asp Glu Leu Asp Asp Ser Gln Val Arg Gln Met Leu Phe Asp Leu
        195                 200                 205

Glu Ser Ala Tyr Asn Ala Phe Asn Arg Phe Leu His Ala
    210                 215                 220

(2) INFORMATION FOR SEQ ID NO: 269:

        (i) SEQUENCE CHARACTERISTICS:
                (A) LENGTH: 3 amino acids
                (B) TYPE: amino acid
                (D) TOPOLOGY: linear
        (xi) SEQUENCE DESCRIPTION: SEQ ID NO: 269:

Met Lys Xaa
1

(2) INFORMATION FOR SEQ ID NO: 270:

        (i) SEQUENCE CHARACTERISTICS:
                (A) LENGTH: 49 amino acids
                (B) TYPE: amino acid
                (D) TOPOLOGY: linear
        (xi) SEQUENCE DESCRIPTION: SEQ ID NO: 270:

Met Gln Ala Pro Phe Xaa His Phe Ser Phe Arg Met Phe Ser Asn Leu
 1               5                  10                  15

Tyr Cys Phe Ser Asp Phe Gln Pro Asn Ile Ser Pro Cys Pro Leu Cys
            20                  25                  30

His Cys Ile Leu Pro Xaa His His His Val Phe Leu Leu Leu Ala Val
        35                  40                  45

Xaa

(2) INFORMATION FOR SEQ ID NO: 271:

        (i) SEQUENCE CHARACTERISTICS:
                (A) LENGTH: 52 amino acids

(B) TYPE: amino acid
(D) TOPOLOGY: linear
(xi) SEQUENCE DESCRIPTION: SEQ ID NO: 271:

Met Lys Leu Val Thr Met Phe Asp Lys Leu Ser Arg Asn Arg Val Ile
1               5                   10                  15

Gln Pro Met Gly Met Ser Pro Arg Gly His Leu Thr Ser Leu Gln Asp
            20                  25                  30

Ala Met Cys Glu Thr Met Glu Gln Gln Leu Ser Ser Asp Pro Asp Ser
        35                  40                  45

Asp Pro Asp Xaa
    50

(2) INFORMATION FOR SEQ ID NO: 272:

(i) SEQUENCE CHARACTERISTICS:
(A) LENGTH: 32 amino acids
(B) TYPE: amino acid
(D) TOPOLOGY: linear
(xi) SEQUENCE DESCRIPTION: SEQ ID NO: 272:

Met Ala Val Gly Glu Ala Val Phe Val Pro Leu Gln His Pro Pro Leu
1               5                   10                  15

Leu His Gly Ser Pro Ile Pro Lys Leu Leu Pro Gly Pro Leu Leu Xaa
            20                  25                  30

(2) INFORMATION FOR SEQ ID NO: 273:

(i) SEQUENCE CHARACTERISTICS:
(A) LENGTH: 57 amino acids
(B) TYPE: amino acid
(D) TOPOLOGY: linear
(xi) SEQUENCE DESCRIPTION: SEQ ID NO: 273:

Met Asn Gly Cys His Arg Arg Lys Arg Leu His Leu Cys Lys Thr Ile
1               5                   10                  15

Tyr Leu Leu Trp Phe Val Phe Ser Phe Leu Leu Ser Asn Glu Val Val
            20                  25                  30

Ser Ser His Trp His Ile Leu Arg Ala Val Gln Ile Ile Cys Thr Leu
        35                  40                  45

Phe His Arg Xaa Ile Ser Ala Phe Xaa
    50                  55

(2) INFORMATION FOR SEQ ID NO: 274:

(i) SEQUENCE CHARACTERISTICS:
(A) LENGTH: 22 amino acids
(B) TYPE: amino acid
(D) TOPOLOGY: linear
(xi) SEQUENCE DESCRIPTION: SEQ ID NO: 274:

Met Gly Trp Val Ser Ser Pro His Val Lys Arg Arg Glu Cys Val Leu
1               5                   10                  15

Lys Lys Pro Phe Phe Xaa
            20

(2) INFORMATION FOR SEQ ID NO: 275:

(i) SEQUENCE CHARACTERISTICS:
(A) LENGTH: 51 amino acids
(B) TYPE: amino acid
(D) TOPOLOGY: linear
(xi) SEQUENCE DESCRIPTION: SEQ ID NO: 275:

Met Phe Asn Phe Phe Lys Asn Pro Leu Leu Thr Cys Leu Phe Ile Ser
1               5                   10                  15

Cys Tyr Leu Tyr Leu Ser Leu Leu Val Asn Lys Val Leu Phe Ala Glu
            20                  25                  30

Glu Gly Leu Cys Cys Thr Tyr Cys Thr Thr Ser Asn Thr Gly Glu Gly
        35                  40                  45

Gly Val Xaa
    50

(2) INFORMATION FOR SEQ ID NO: 276:

(i) SEQUENCE CHARACTERISTICS:
(A) LENGTH: 2 amino acids
(B) TYPE: amino acid
(D) TOPOLOGY: linear
(xi) SEQUENCE DESCRIPTION: SEQ ID NO: 276:

Met Xaa
1

(2) INFORMATION FOR SEQ ID NO: 277:

(i) SEQUENCE CHARACTERISTICS:
(A) LENGTH: 66 amino acids
(B) TYPE: amino acid
(D) TOPOLOGY: linear
(xi) SEQUENCE DESCRIPTION: SEQ ID NO: 277:

Met Leu Cys Thr Ile Leu Thr Val Val Ile Ile Ile Ala Ala Gln Thr
1               5                   10                  15

Thr Arg Thr Thr Gly Ile Pro Lys Asn Ala Pro Gly Pro Ala Pro Leu
            20                  25                  30

Cys Ala Pro Arg Ser Pro Arg Leu Phe Leu Gln Xaa Tyr Arg Gly Pro
        35                  40                  45

Asn Gly Arg Pro Ala His Pro Phe Leu Gly Pro Ser Asp Leu Asp Thr
    50                  55                  60

Ser Xaa
65

(2) INFORMATION FOR SEQ ID NO: 278:

        (i) SEQUENCE CHARACTERISTICS:
              (A) LENGTH: 257 amino acids
              (B) TYPE: amino acid
              (D) TOPOLOGY: linear
        (xi) SEQUENCE DESCRIPTION: SEQ ID NO: 278:

Met Leu Gly Ala Lys Pro His Trp Leu Pro Gly Pro Leu His Ser Pro
  1               5                  10                  15

Gly Leu Pro Leu Val Leu Val Leu Leu Ala Leu Gly Ala Gly Trp Ala
            20                  25                  30

Gln Glu Gly Ser Glu Pro Val Leu Leu Glu Gly Glu Cys Leu Val Val
        35                  40                  45

Cys Glu Pro Gly Arg Ala Ala Ala Gly Gly Pro Gly Gly Ala Ala Leu
    50                  55                  60

Gly Glu Ala Pro Pro Gly Arg Val Ala Phe Xaa Ala Val Arg Ser His
65                  70                  75                  80

His His Glu Pro Ala Gly Glu Thr Gly Asn Gly Thr Ser Gly Ala Ile
                85                  90                  95

Tyr Phe Asp Gln Val Leu Val Asn Glu Gly Gly Gly Phe Asp Arg Ala
            100                 105                 110

Ser Gly Ser Phe Val Ala Pro Val Arg Gly Val Tyr Ser Phe Arg Phe
        115                 120                 125

His Val Val Lys Val Tyr Asn Arg Gln Thr Val Gln Val Ser Leu Met
    130                 135                 140

Leu Asn Thr Trp Pro Val Ile Ser Ala Phe Ala Asn Asp Pro Asp Val
145                 150                 155                 160

Thr Arg Glu Ala Ala Thr Ser Ser Val Leu Leu Pro Leu Asp Pro Gly
                165                 170                 175

Asp Arg Val Ser Leu Arg Leu Arg Arg Gly Xaa Ser Thr Gly Trp Leu
            180                 185                 190

Glu Ile Leu Lys Phe Leu Trp Leu Pro His Leu Pro Ser Leu Lys Asp

        195                 200                 205

Pro Ser Leu Ser Ser Thr Arg Ile Gln Pro Leu Thr Thr Phe Phe Cys
    210                 215                 220

Pro Leu Leu Pro Xaa Lys Gln Xaa Lys Gln Xaa Xaa Xaa Ser Leu Trp
225                 230                 235                 240

Leu Leu Ser His Leu Phe Ala Trp Glu Pro Val Pro Asn Thr Gln Val
                245                 250                 255

Xaa

(2) INFORMATION FOR SEQ ID NO: 279:

(i) SEQUENCE CHARACTERISTICS:
(A) LENGTH: 103 amino acids
(B) TYPE: amino acid
(D) TOPOLOGY: linear
(xi) SEQUENCE DESCRIPTION: SEQ ID NO: 279:

Met Ala Pro Arg Ala Leu Pro Gly Ser Ala Val Leu Ala Ala Ala Val
1               5                   10                  15

Phe Val Gly Gly Ala Val Ser Ser Pro Leu Val Ala Pro Asp Asn Gly
            20                  25                  30

Ser Ser Arg Thr Leu His Ser Arg Thr Glu Thr Thr Pro Ser Pro Ser
        35                  40                  45

Asn Asp Thr Gly Asn Gly His Pro Glu Tyr Ile Ala Tyr Ala Leu Val
    50                  55                  60

Pro Val Phe Phe Ile Met Gly Leu Phe Gly Val Leu Ile Xaa Pro Xaa
65                  70                  75                  80

Xaa Xaa Lys Lys Lys Gly Tyr Arg Cys Thr Thr Glu Ala Glu Gln Asp
                85                  90                  95

Ile Glu Glu Glu Lys Gly Xaa
            100

(2) INFORMATION FOR SEQ ID NO: 280:

(i) SEQUENCE CHARACTERISTICS:
(A) LENGTH: 33 amino acids
(B) TYPE: amino acid
(D) TOPOLOGY: linear
(xi) SEQUENCE DESCRIPTION: SEQ ID NO: 280:

Met Pro Val Thr Leu Ser Ser Leu Gly Phe Trp Val Leu Leu Ser Leu
1               5                   10                  15

Leu Phe Pro Trp Arg Thr Asp Gln Gly Cys Gly Pro Ala Thr Cys Tyr
            20                  25                  30

Xaa

(2) INFORMATION FOR SEQ ID NO: 281:

(i) SEQUENCE CHARACTERISTICS:
(A) LENGTH: 43 amino acids
(B) TYPE: amino acid
(D) TOPOLOGY: linear
(xi) SEQUENCE DESCRIPTION: SEQ ID NO: 281:

Met Val Leu Gly Leu Leu Leu Leu Leu Xaa Phe Phe Ser Phe Ser Ser
1               5                   10                  15

Ser Pro Ser Pro Ser Ser Ser Leu Leu Leu Leu Ser Ser Phe Phe Phe
            20                  25                  30

Gln Ser Leu Ala Leu Ser Pro Arg Leu Glu Xaa
        35                  40

(2) INFORMATION FOR SEQ ID NO: 282:

(i) SEQUENCE CHARACTERISTICS:
(A) LENGTH: 21 amino acids
(B) TYPE: amino acid
(D) TOPOLOGY: linear
(xi) SEQUENCE DESCRIPTION: SEQ ID NO: 282:

Glu Trp Leu Val Phe Thr Phe Leu Leu Val Phe Gly Ser Pro Leu Gly
1               5                   10                  15

Lys Gly Pro Leu Xaa
            20

(2) INFORMATION FOR SEQ ID NO: 283:

(i) SEQUENCE CHARACTERISTICS:
(A) LENGTH: 70 amino acids
(B) TYPE: amino acid
(D) TOPOLOGY: linear
(xi) SEQUENCE DESCRIPTION: SEQ ID NO: 283:

Met Ile Arg Ala Leu Ser Leu Phe Leu Leu Ile Phe Asp Ala Ala Leu
1               5                   10                  15

Phe Ser Leu Ser Val Phe Val Phe Ile Gly His Leu Leu Pro Met Pro
            20                  25                  30

Lys Gly Thr Gly Leu His Ser Cys Ala Lys His Leu Ile Lys Ser Leu
        35                  40                  45

Lys Glu Asn Val Leu Pro Leu Met Asn Tyr Pro Asp Cys Lys Leu Lys
    50                  55                  60

Ile Asn Ile Ser Pro Xaa
65                    70

(2) INFORMATION FOR SEQ ID NO: 284:

(i) SEQUENCE CHARACTERISTICS:
(A) LENGTH: 75 amino acids
(B) TYPE: amino acid
(D) TOPOLOGY: linear
(xi) SEQUENCE DESCRIPTION: SEQ ID NO: 284:

Met Gly Lys Leu Ile Arg Leu Ser Val Met Val Met Ser Val Arg Arg
1               5                   10                  15

Leu Phe Ser Ile Tyr Trp Val Leu Ser Thr Val Pro Asp Ala Val Gly
            20                  25                  30

Ser Arg Gly Gly Met Glu Glu Glu Cys Ser Arg Gly Leu Cys Cys Val
        35                  40                  45

Ala Gly Gln His Lys Gln Ala Lys Gly Lys Arg Gln Ala Trp Asn Lys
    50                  55                  60

Gly Gly Glu Tyr Gln Cys Val Thr Tyr Cys Xaa
65                  70                  75

(2) INFORMATION FOR SEQ ID NO: 285:

(i) SEQUENCE CHARACTERISTICS:
(A) LENGTH: 33 amino acids
(B) TYPE: amino acid
(D) TOPOLOGY: linear
(xi) SEQUENCE DESCRIPTION: SEQ ID NO: 285:

Met Pro Ala Leu Val Thr Leu Leu Leu Leu Phe Pro Leu Leu Pro Leu
1               5                   10                  15

Met Glu Ala Ser Cys His Val Met Arg Cys Pro Met Glu Arg Pro Thr
            20                  25                  30

Xaa

(2) INFORMATION FOR SEQ ID NO: 286:

(i) SEQUENCE CHARACTERISTICS:
(A) LENGTH: 17 amino acids
(B) TYPE: amino acid
(D) TOPOLOGY: linear
(xi) SEQUENCE DESCRIPTION: SEQ ID NO: 286:

Glu Ala Pro Trp Gly Leu Leu Lys Leu Leu Leu Leu Leu Ala Val Phe
1               5                   10                  15

Xaa

(2) INFORMATION FOR SEQ ID NO: 287:

(i) SEQUENCE CHARACTERISTICS:
(A) LENGTH: 17 amino acids
(B) TYPE: amino acid
(D) TOPOLOGY: linear
(xi) SEQUENCE DESCRIPTION: SEQ ID NO: 287:

Met Gln Gln Lys Gln Lys Lys Ala Asn Glu Lys Lys Glu Glu Pro Lys
1               5                   10                  15

Xaa

(2) INFORMATION FOR SEQ ID NO: 288:

(i) SEQUENCE CHARACTERISTICS:
(A) LENGTH: 38 amino acids
(B) TYPE: amino acid
(D) TOPOLOGY: linear
(xi) SEQUENCE DESCRIPTION: SEQ ID NO: 288:

Met Gln Arg Lys Val Ser Asp Phe Ile Ile His Gln Arg Leu Thr Val
1               5                   10                  15

Asn Leu Cys Val Ile Ser Phe Phe Phe Phe Leu Pro Ile Cys Ile Phe
            20                  25                  30

Ser Leu Ala Lys Lys Xaa
        35

(2) INFORMATION FOR SEQ ID NO: 289:

(i) SEQUENCE CHARACTERISTICS:
(A) LENGTH: 12 amino acids
(B) TYPE: amino acid
(D) TOPOLOGY: linear
(xi) SEQUENCE DESCRIPTION: SEQ ID NO: 289:

Met Ala Leu Leu Ile Ser Ser Leu Ile Trp Ser Xaa
1               5                   10

(2) INFORMATION FOR SEQ ID NO: 290:

(i) SEQUENCE CHARACTERISTICS:
(A) LENGTH: 35 amino acids
(B) TYPE: amino acid
(D) TOPOLOGY: linear

(xi) SEQUENCE DESCRIPTION: SEQ ID NO: 290:

Met Gln Met Phe Thr Val Ser Leu Leu Leu Ser Leu Leu Leu Arg Ser
1               5                   10                  15

Thr Asp Gln Asn His Leu Gln Leu Leu Val Gly Arg Glu Asp His Tyr
            20                  25                  30

Gly Gly Xaa
        35

(2) INFORMATION FOR SEQ ID NO: 291:

(i) SEQUENCE CHARACTERISTICS:
(A) LENGTH: 15 amino acids
(B) TYPE: amino acid
(D) TOPOLOGY: linear
(xi) SEQUENCE DESCRIPTION: SEQ ID NO: 291:

Met Ser Glu Ser Ala Cys Ile Leu Asn Asn Gln Lys Glu Leu Xaa
1               5                   10                  15

(2) INFORMATION FOR SEQ ID NO: 292:

(i) SEQUENCE CHARACTERISTICS:
(A) LENGTH: 44 amino acids
(B) TYPE: amino acid
(D) TOPOLOGY: linear
(xi) SEQUENCE DESCRIPTION: SEQ ID NO: 292:

Met Asp Leu Asp Arg Val Lys Ala Glu Ala Thr Glu Asp Ile Thr Ser
1               5                   10                  15

Gly Val Leu Cys Leu Leu Phe Leu Arg Leu Pro Pro Asn Ser Cys Ile
            20                  25                  30

Phe Pro Ser Ala Val Leu Gly Ser Thr Arg Thr Xaa
        35                  40

(2) INFORMATION FOR SEQ ID NO: 293:

(i) SEQUENCE CHARACTERISTICS:
(A) LENGTH: 136 amino acids
(B) TYPE: amino acid
(D) TOPOLOGY: linear
(xi) SEQUENCE DESCRIPTION: SEQ ID NO: 293:

Val Val Gly Thr Gly Thr Ser Leu Ala Leu Ser Ser Leu Leu Ser Leu
1               5                   10                  15

Leu Leu Phe Ala Gly Met Gln Met Tyr Ser Arg Gln Leu Ala Ser Thr
            20                  25                  30

Glu Trp Leu Thr Ile Gln Gly Gly Leu Leu Gly Ser Gly Leu Phe Val

35                  40                  45

Phe Ser Leu Thr Ala Phe Asn Asn Leu Glu Asn Leu Val Phe Gly Lys
    50                  55                  60

Gly Phe Gln Ala Lys Ile Phe Pro Glu Ile Leu Leu Cys Leu Leu Leu
65                  70                  75                  80

Ala Leu Phe Ala Ser Gly Leu Ile His Arg Val Cys Val Thr Thr Cys
                85                  90                  95

Phe Ile Phe Ser Met Val Gly Leu Tyr Tyr Ile Asn Lys Ile Ser Ser
            100                 105                 110

Thr Leu Tyr Gln Ala Ala Ala Pro Val Leu Thr Pro Ala Lys Val Thr
        115                 120                 125

Gly Lys Ser Lys Lys Arg Asn Xaa
    130                 135

(2) INFORMATION FOR SEQ ID NO: 294:

(i) SEQUENCE CHARACTERISTICS:
(A) LENGTH: 34 amino acids
(B) TYPE: amino acid
(D) TOPOLOGY: linear
(xi) SEQUENCE DESCRIPTION: SEQ ID NO: 294:

Met Phe Ile Phe Leu Phe Leu Cys Val Leu Ser Arg Lys Ile Gln Glu
1               5                   10                  15

Glu Tyr Tyr Arg Leu Phe Lys Asn Val Pro Cys Cys Phe Gly Cys Leu
            20                  25                  30

Arg Xaa

(2) INFORMATION FOR SEQ ID NO: 295:

(i) SEQUENCE CHARACTERISTICS:
(A) LENGTH: 137 amino acids
(B) TYPE: amino acid
(D) TOPOLOGY: linear
(xi) SEQUENCE DESCRIPTION: SEQ ID NO: 295:

Met Arg Thr Pro Gly Pro Leu Pro Val Leu Leu Leu Leu Leu Ala Gly
1               5                   10                  15

Ala Pro Ala Ala Arg Pro Thr Pro Pro Thr Cys Tyr Ser Arg Met Arg
            20                  25                  30

Ala Leu Ser Gln Glu Ile Thr Arg Asp Phe Asn Leu Leu Gln Val Ser
        35                  40                  45

Glu Pro Ser Glu Pro Cys Val Arg Tyr Leu Pro Arg Leu Tyr Leu Asp
    50                  55                  60

Ile His Asn Tyr Cys Val Leu Asp Lys Leu Arg Asp Phe Val Ala Ser
65                  70                  75                  80

Pro Pro Cys Trp Lys Val Ala Gln Val Asp Ser Leu Lys Asp Lys Ala
                85                  90                  95

Arg Lys Leu Tyr Thr Ile Met Asn Ser Phe Cys Arg Arg Asp Leu Val
            100                 105                 110

Phe Leu Leu Asp Asp Cys Asn Ala Leu Glu Tyr Pro Ile Pro Val Thr
        115                 120                 125

Thr Val Leu Pro Asp Arg Gln Arg Xaa
    130                 135

(2) INFORMATION FOR SEQ ID NO: 296:

(i) SEQUENCE CHARACTERISTICS:
(A) LENGTH: 58 amino acids
(B) TYPE: amino acid
(D) TOPOLOGY: linear
(xi) SEQUENCE DESCRIPTION: SEQ ID NO: 296:

Met Trp Leu Leu Lys Pro Ser Ala His Ser Pro Val His Xaa Leu Val
1               5                   10                  15

Leu Leu Phe Pro Arg Gly Trp Ser Gln Pro Gly Thr His Lys Arg Gln
            20                  25                  30

Ile Leu Val Asn Xaa Ala Ser Leu Pro Gly Gly Cys Leu Leu Pro Trp
        35                  40                  45

Ile Trp Ser Gly Ala Ala Leu Arg Phe Xaa
    50                  55

(2) INFORMATION FOR SEQ ID NO: 297:

(i) SEQUENCE CHARACTERISTICS:
(A) LENGTH: 35 amino acids
(B) TYPE: amino acid
(D) TOPOLOGY: linear
(xi) SEQUENCE DESCRIPTION: SEQ ID NO: 297:

Met Ser Arg Arg Ala Glu Ala Ser Ile Phe Val Leu Pro Lys Thr Leu
1               5                   10                  15

Leu Phe Val Leu Phe Pro Ala Phe Pro Ser Pro Ala Val Gly Cys Pro
            20                  25                  30

Val Pro Xaa
        35

(2) INFORMATION FOR SEQ ID NO: 298:

(i) SEQUENCE CHARACTERISTICS:
(A) LENGTH: 78 amino acids
(B) TYPE: amino acid
(D) TOPOLOGY: linear
(xi) SEQUENCE DESCRIPTION: SEQ ID NO: 298:

Ser Cys Tyr Ile Thr Pro Trp Ser Lys Ile Gln Ser Phe Ser Leu Ser
1               5                   10                  15

Leu Phe Gln Phe Ile Leu Gln Glu Val Asn Ile Thr Leu Pro Glu Asn
            20                  25                  30

Ser Val Trp Tyr Glu Arg Tyr Lys Phe Asp Ile Pro Val Phe His Leu
        35                  40                  45

Asn Gly Gln Phe Leu Met Met His Arg Val Asn Thr Ser Lys Leu Glu
    50                  55                  60

Lys Gln Leu Leu Lys Leu Glu Gln Gln Ser Thr Gly Xaa Xaa
65                  70                  75

(2) INFORMATION FOR SEQ ID NO: 299:

(i) SEQUENCE CHARACTERISTICS:
(A) LENGTH: 95 amino acids
(B) TYPE: amino acid
(D) TOPOLOGY: linear
(xi) SEQUENCE DESCRIPTION: SEQ ID NO: 299:

Met Phe Val Leu Phe Ser Leu Pro Lys Tyr Ala Gly Leu Arg Leu Pro
1               5                   10                  15

Ile Pro Gly Leu Ser Ala Leu Leu Val Phe Leu Leu Ser Leu Phe Ser
            20                  25                  30

Arg Arg Ala Gln Val Glu Leu Thr Thr Gly Arg Glu Thr Leu Pro Lys
        35                  40                  45

Asn Leu Gln Gly Tyr Phe Pro Glu Phe Gly Phe Gln Val Gln Asn Phe
    50                  55                  60

Leu Ser Cys Lys Ile Tyr Ala Ala Ser Gln Lys Gln Pro Leu Pro Pro
65                  70                  75                  80

Leu Tyr Gln Leu Arg Phe Tyr Leu Lys His Met Gly Leu Pro Xaa
                85                  90                  95

(2) INFORMATION FOR SEQ ID NO: 300:

(i) SEQUENCE CHARACTERISTICS:
(A) LENGTH: 44 amino acids
(B) TYPE: amino acid
(D) TOPOLOGY: linear
(xi) SEQUENCE DESCRIPTION: SEQ ID NO: 300:

Met Ser Ser His Trp Thr Leu Lys Ile Leu Leu Val Pro Leu Phe Tyr
1               5                   10                  15

Leu Ser Leu Glu Phe Pro Ser Gly Phe Val Leu Cys Leu Ala Asn Asp
            20                  25                  30

Leu Gly Tyr His Phe Ser Ser Arg Val Arg Ser Xaa
        35                  40

(2) INFORMATION FOR SEQ ID NO: 301:

(i) SEQUENCE CHARACTERISTICS:
(A) LENGTH: 31 amino acids
(B) TYPE: amino acid
(D) TOPOLOGY: linear
(xi) SEQUENCE DESCRIPTION: SEQ ID NO: 301:

Met Leu Val Val Asn Ile Asn Leu Val Phe Leu Leu Phe Phe Ile Phe
1               5                   10                  15

Leu Cys Tyr Leu Asp Ala Cys Ile Asn Val Phe Cys Phe Tyr Xaa
            20                  25                  30

(2) INFORMATION FOR SEQ ID NO: 302:

(i) SEQUENCE CHARACTERISTICS:
(A) LENGTH: 113 amino acids
(B) TYPE: amino acid
(D) TOPOLOGY: linear
(xi) SEQUENCE DESCRIPTION: SEQ ID NO: 302:

Met Pro Val Leu Pro Gly Arg Thr Thr Ala Leu Leu Ser Leu Thr Leu
1               5                   10                  15

Ala Phe Ala Val Pro Cys Ser Gly Val Glu Ala Gly Pro Cys Val Pro
            20                  25                  30

Arg Ser His Gly Cys Ser Ser Trp Glu Ala Ser Val Cys Val Thr Ser
        35                  40                  45

Ser Thr Pro Gly Gly Ser Trp Arg Ala Arg Ala Leu Phe Pro Ser Ala
    50                  55                  60

Ala Trp His Arg Xaa Ala Ala Trp Asp Ser Pro Trp Thr Gln Thr Gly
65                  70                  75                  80

Asp Phe Ala Arg Gly Ala Met Gly Gly Ala Gly Ala Leu Pro Gly Gly
                85                  90                  95

Cys Val Cys Ile Ser Gly Arg Pro Arg Ala Gln Lys Leu Pro Ala Leu
            100                 105                 110

Xaa

(2) INFORMATION FOR SEQ ID NO: 303:

(i) SEQUENCE CHARACTERISTICS:
(A) LENGTH: 14 amino acids
(B) TYPE: amino acid
(D) TOPOLOGY: linear
(xi) SEQUENCE DESCRIPTION: SEQ ID NO: 303:

Thr His Ile His Thr His Ile Ile Ile Cys Ser Ser Val Xaa
1               5                   10

(2) INFORMATION FOR SEQ ID NO: 304:

(i) SEQUENCE CHARACTERISTICS:
(A) LENGTH: 35 amino acids
(B) TYPE: amino acid
(D) TOPOLOGY: linear
(xi) SEQUENCE DESCRIPTION: SEQ ID NO: 304:

Met Glu Asn Phe Phe Phe Ser Phe Tyr Leu Phe Leu Ile Thr Leu Ile
1               5                   10                  15

Pro Asn Gly Arg Thr Leu Ser Thr Thr Ala Asp His Cys Lys Ile Pro
            20                  25                  30

Cys Ile Xaa
        35

(2) INFORMATION FOR SEQ ID NO: 305:

(i) SEQUENCE CHARACTERISTICS:
(A) LENGTH: 35 amino acids
(B) TYPE: amino acid
(D) TOPOLOGY: linear
(xi) SEQUENCE DESCRIPTION: SEQ ID NO: 305:

Met Glu Leu Trp Glu Leu Ala Leu Cys Leu Leu Val Ala Leu Ser Ala
1               5                   10                  15

His Met Phe Thr Val Gln Leu Leu Ala Asp Leu Gly Phe Leu Phe Gly
            20                  25                  30

Gly Phe Xaa
        35

(2) INFORMATION FOR SEQ ID NO: 306:

(i) SEQUENCE CHARACTERISTICS:
(A) LENGTH: 82 amino acids
(B) TYPE: amino acid
(D) TOPOLOGY: linear
(xi) SEQUENCE DESCRIPTION: SEQ ID NO: 306:

Met Gly Ala Gly Ile Leu Ala Leu Leu Leu Pro Leu Glu Ser Val Leu
1               5                   10                  15

Thr Cys Ser Trp Ile Ser Val Ser Thr Ser Glu Arg Gln Leu Trp Gln
            20                  25                  30

Ser Ser Gln Lys Ala Thr Ile Leu Ser Leu Lys Leu Asp Ser Cys Phe
        35                  40                  45

Cys Gly His Ser Gly Leu Lys Gly Lys Asn Glu Asp Thr Asp Ser Ser
    50                  55                  60

Val Pro Ile Ile Pro Ser Lys Thr His Thr His Leu Gly Lys His Leu
65                  70                  75                  80

Ile Xaa

(2) INFORMATION FOR SEQ ID NO: 307:

(i) SEQUENCE CHARACTERISTICS:
(A) LENGTH: 72 amino acids
(B) TYPE: amino acid
(D) TOPOLOGY: linear
(xi) SEQUENCE DESCRIPTION: SEQ ID NO: 307:

Met Phe Tyr Phe Val Leu Phe Ile Tyr Ser Ser Ser Glu Thr Trp Ser
1               5                   10                  15

Gly Ser Val Ala Gln Asp Gly Val His Gly Val Ile Ile Gly His Cys
            20                  25                  30

Ser Val Glu Leu Pro Gly Ser Gly Asp Pro Pro Ala Ser Ala Xaa Leu
        35                  40                  45

Val Ala Gly Thr Ile Gly Thr Cys Pro Thr Met Pro Gly Phe Val Tyr
    50                  55                  60

Phe Leu Asn Asp Val Xaa Asn Xaa
65                  70

(2) INFORMATION FOR SEQ ID NO: 308:

(i) SEQUENCE CHARACTERISTICS:
(A) LENGTH: 34 amino acids
(B) TYPE: amino acid
(D) TOPOLOGY: linear
(xi) SEQUENCE DESCRIPTION: SEQ ID NO: 308:

Met Asp Ser Thr Leu Arg Gln Gly Arg Xaa Leu Leu Thr Leu Val Pro
1               5                   10                  15

Ala Ser Leu Phe Ser Leu Thr Leu Gly Gly Pro Gly Pro Trp Lys Asp
            20                  25                  30

Pro Xaa

(2) INFORMATION FOR SEQ ID NO: 309:

(i) SEQUENCE CHARACTERISTICS:
(A) LENGTH: 115 amino acids
(B) TYPE: amino acid
(D) TOPOLOGY: linear
(xi) SEQUENCE DESCRIPTION: SEQ ID NO: 309:

Met Gln Val Val Gly Ser Trp Pro Gly Arg Val Gly Val Val Gly Leu
1               5                   10                  15

Ala Phe Ser Leu Val Ile Pro Pro Pro Ala Ile Cys Ile Ala Gly Pro
            20                  25                  30

Ala Pro Gly Leu Gly Gly Gly Glu Arg Gln Gln Lys Gly Leu Gly Arg
        35                  40                  45

Gly Gly Gly Gly Leu Arg Asn Cys Pro Gly Arg Val Gly Met Ala Ala
    50                  55                  60

Glu Pro Gly Ala Leu Leu Cys Leu Thr Ser Arg Asp Gly Ser Leu Leu
65                  70                  75                  80

Leu Ser Cys Val Arg Pro His His Val Ile Lys Pro Lys Gly Thr Ala
                85                  90                  95

Lys Lys Lys Lys Lys Lys Lys Lys Lys Lys Lys Lys Lys Lys Xaa Xaa
            100                 105                 110

Gly Gly Xaa
        115

(2) INFORMATION FOR SEQ ID NO: 310:

(i) SEQUENCE CHARACTERISTICS:
(A) LENGTH: 108 amino acids
(B) TYPE: amino acid
(D) TOPOLOGY: linear
(xi) SEQUENCE DESCRIPTION: SEQ ID NO: 310:

Met Asp Leu Pro Gln Phe Ile Tyr Leu Phe Ile Phe Cys Phe Cys Cys
1               5                   10                  15

Leu Ala Ile Val Asn Asn Ala Ser Ile Asn Ile His Ile Gln Val Ser
            20                  25                  30

Met Trp Leu Tyr Val Phe Ile Ser Leu Gly Tyr Leu His Gly Ser Arg
        35                  40                  45

Ile Leu Gly His Asn Ile Ile Leu Cys Leu Thr Ser Gln Arg Ile Ala
    50                  55                  60

Lys Arg Phe Phe Ile Val Ala Ala Ser Phe Thr Phe Pro Pro Ala Met
65                  70                  75                  80

Tyr Lys Asp Phe Tyr Phe Ser Ile Ser Leu His Leu Pro Thr Leu Leu
                85                  90                  95

Phe Xaa Xaa Xaa Phe Val Phe Ser Leu Leu Pro Pro
            100                 105

(2) INFORMATION FOR SEQ ID NO: 311:

        (i) SEQUENCE CHARACTERISTICS:
                (A) LENGTH: 65 amino acids
                (B) TYPE: amino acid
                (D) TOPOLOGY: linear
        (xi) SEQUENCE DESCRIPTION: SEQ ID NO: 311:

Met Cys Ser Pro Ser Leu Ser Ser Ser Pro Pro Pro Leu Leu Gln Val
  1               5                  10                  15

Phe Phe Phe Phe Phe Phe Ser Pro His Trp Ala Ala Lys Val Val Pro
            20                  25                  30

Gln Trp Lys Xaa Arg His Pro Gln Val Ser Ser Gln Leu Leu Leu Cys
        35                  40                  45

Phe Leu Arg Val Asn Cys Gln Phe Leu Phe Leu Gln Glu Ile Leu Phe
    50                  55                  60

Xaa
65

(2) INFORMATION FOR SEQ ID NO: 312:

        (i) SEQUENCE CHARACTERISTICS:
                (A) LENGTH: 50 amino acids
                (B) TYPE: amino acid
                (D) TOPOLOGY: linear
        (xi) SEQUENCE DESCRIPTION: SEQ ID NO: 312:

Met Cys Leu Ser Arg Trp Lys Ile Phe Tyr Thr Leu Leu Ile Leu Phe
  1               5                  10                  15

Xaa Xaa Phe Ser Ile Thr Ser Glu Xaa Glu Thr Phe Tyr Met Ile Ile
            20                  25                  30

Ile His His Asn Pro Thr Gln Ile Thr Ala Ser Cys Ser Phe Thr Phe
        35                  40                  45

Leu Xaa
    50

(2) INFORMATION FOR SEQ ID NO: 313:

        (i) SEQUENCE CHARACTERISTICS:
                (A) LENGTH: 293 amino acids

(B) TYPE: amino acid
(D) TOPOLOGY: linear
(xi) SEQUENCE DESCRIPTION: SEQ ID NO: 313:

Met Glu Arg Pro Asp Trp Glu Thr Ala Ile Gln Lys Pro Leu Cys Ser
1               5                   10                  15

Leu Pro Ala Gly Ser Gly Asn Ala Leu Ala Ala Ser Leu Asn His Tyr
            20                  25                  30

Ala Gly Tyr Xaa Gln Val Thr Asn Glu Asp Leu Leu Thr Asn Cys Thr
        35                  40                  45

Leu Leu Leu Cys Arg Arg Leu Leu Ser Pro Met Asn Leu Leu Ser Leu
    50                  55                  60

His Thr Ala Ser Gly Leu Arg Leu Phe Ser Val Leu Ser Leu Ala Trp
65                  70                  75                  80

Gly Phe Ile Ala Asp Val Asp Leu Glu Ser Glu Lys Tyr Arg Arg Leu
                85                  90                  95

Gly Glu Met Arg Phe Thr Leu Gly Thr Phe Leu Arg Leu Ala Ala Leu
            100                 105                 110

Arg Thr Tyr Arg Gly Arg Leu Ala Tyr Leu Pro Val Gly Arg Val Gly
        115                 120                 125

Ser Lys Thr Pro Ala Ser Pro Val Val Val Gln Gln Gly Pro Val Asp
    130                 135                 140

Ala His Leu Val Pro Leu Glu Glu Pro Val Pro Ser His Trp Thr Val
145                 150                 155                 160

Val Pro Asp Glu Asp Phe Val Leu Val Leu Ala Leu Leu His Ser His
                165                 170                 175

Leu Gly Ser Glu Met Phe Ala Ala Pro Met Gly Arg Cys Ala Ala Gly
            180                 185                 190

Val Met His Leu Phe Tyr Val Arg Ala Gly Val Ser Arg Ala Met Leu
        195                 200                 205

Leu Arg Leu Phe Leu Ala Met Glu Lys Gly Arg His Met Glu Tyr Glu
    210                 215                 220

Cys Pro Tyr Leu Val Tyr Val Pro Val Val Ala Phe Arg Leu Glu Pro
225                 230                 235                 240

Lys Asp Gly Lys Gly Val Phe Ala Val Asp Gly Glu Leu Met Val Ser
                245                 250                 255

Glu Ala Val Gln Gly Gln Val His Pro Asn Tyr Phe Trp Met Val Ser
            260                 265                 270

Gly Cys Val Glu Pro Pro Pro Ser Trp Lys Pro Gln Gln Met Pro Pro
        275                 280                 285

Pro Glu Glu Pro Leu
    290

(2) INFORMATION FOR SEQ ID NO: 314:

(i) SEQUENCE CHARACTERISTICS:
(A) LENGTH: 68 amino acids
(B) TYPE: amino acid
(D) TOPOLOGY: linear
(xi) SEQUENCE DESCRIPTION: SEQ ID NO: 314:

Met Pro Leu Glu Gly Phe Cys Leu Val Leu Asp Ile Gly Phe Leu Leu
1               5                   10                  15

Val Met Leu Ile Ser Leu Ala Ser Glu Cys Phe Thr Thr Cys Leu Asp
            20                  25                  30

Ser Phe Ser Thr Thr Glu Pro Gly Cys Lys Phe Tyr Lys Leu Leu His
        35                  40                  45

Ser Val Ser Leu Leu Asn Ile Asn Phe Asn Val Lys Ser Leu Leu Cys
    50                  55                  60

Ser His Ile Xaa
65

(2) INFORMATION FOR SEQ ID NO: 315:

(i) SEQUENCE CHARACTERISTICS:
(A) LENGTH: 105 amino acids
(B) TYPE: amino acid
(D) TOPOLOGY: linear
(xi) SEQUENCE DESCRIPTION: SEQ ID NO: 315:

Met Pro Leu Gln Leu Ser Gly Gln Tyr Trp Ile Ser Leu Leu Val Phe
1               5                   10                  15

Leu Ser Leu Gln Pro Phe Pro Gln Ala Ala Ile Pro Cys Ala Leu Thr
            20                  25                  30

Asp Val Gly Gly Ser Cys Val Ile Cys His Ile Leu Leu Asn Cys Leu
        35                  40                  45

Cys Ile Leu Phe Thr Leu Thr Ala Pro Ser Leu Ser His Val Leu Leu
    50                  55                  60

Ile Lys Met Ser Leu Ser Val Cys Tyr Glu Pro Gly Ala Asp Leu Ser
65                  70                  75                  80

Asp Arg Ala Ala Thr Gly Asn Lys Lys Leu Thr Arg Ser Thr Cys Leu
                85                  90                  95

Leu Met His Ser Asn Lys Leu Cys Xaa
            100                 105

(2) INFORMATION FOR SEQ ID NO: 316:

(i) SEQUENCE CHARACTERISTICS:
(A) LENGTH: 71 amino acids
(B) TYPE: amino acid
(D) TOPOLOGY: linear
(xi) SEQUENCE DESCRIPTION: SEQ ID NO: 316:

Met Trp Gly Cys Ser Gly Leu Gly His Arg Thr Val Ser Phe Leu Leu
1               5                   10                  15

Leu Leu Pro Cys Ser Phe Pro Arg Pro Cys Xaa Leu Phe Gly Leu Ile
            20                  25                  30

Pro Ile Ser Arg Pro Cys Lys Val Glu Ala Pro Arg Leu Ser Val Pro
        35                  40                  45

Xaa Leu Ser Cys Ala Ser His Pro Tyr Cys Asn Cys Pro Met Ser Thr
    50                  55                  60

Ser Cys Pro Leu Pro Arg Xaa
65                  70

(2) INFORMATION FOR SEQ ID NO: 317:

(i) SEQUENCE CHARACTERISTICS:
(A) LENGTH: 39 amino acids
(B) TYPE: amino acid
(D) TOPOLOGY: linear
(xi) SEQUENCE DESCRIPTION: SEQ ID NO: 317:

Met Leu Asn Val Leu Ser Lys Val Gln Gln Leu Val Ser Xaa Leu Gly
1               5                   10                  15

Leu Val Thr Phe Leu Leu Asn His Ser Ala Ala Gly Gly Ser Pro Gln
            20                  25                  30

His Arg Trp Leu Leu Leu Xaa
        35

(2) INFORMATION FOR SEQ ID NO: 318:

(i) SEQUENCE CHARACTERISTICS:
(A) LENGTH: 72 amino acids
(B) TYPE: amino acid
(D) TOPOLOGY: linear
(xi) SEQUENCE DESCRIPTION: SEQ ID NO: 318:

Met Lys Ala Ile Ala Arg Ala Cys Leu Leu Leu Ser Leu Leu Val Leu
1               5                   10                  15

Pro His Val Val Ser Glu His Leu Phe Trp His His Asn Pro Arg His
            20                  25                  30

Pro Val Ile Trp Pro Phe Pro Pro Phe His Leu Ile Ser Cys Ser Val
        35                  40                  45

Ser Ala Ser Thr Trp His Leu Gly Glu Xaa Leu Leu Leu Leu Val Pro
50                  55                  60

Ile Ala Pro Ser Val Trp Ser Xaa
65                  70

(2) INFORMATION FOR SEQ ID NO: 319:

(i) SEQUENCE CHARACTERISTICS:
(A) LENGTH: 62 amino acids
(B) TYPE: amino acid
(D) TOPOLOGY: linear
(xi) SEQUENCE DESCRIPTION: SEQ ID NO: 319:

Met Glu Gln Gly Gly Gly Pro Arg Leu Leu Leu Leu Ile Pro Gly Leu
1               5                   10                  15

Leu His Asn Thr Tyr Leu Ala Arg Pro Gly Asp Phe Pro Ala Gln Gly
            20                  25                  30

Thr Thr Glu Asn Thr Glu Cys Gln Gly Ser Pro Ser Pro Ile Ser His
        35                  40                  45

Leu Gly Lys Val Arg Ser Leu Asp Ser Asn Thr Gln Ile Xaa
    50                  55                  60

(2) INFORMATION FOR SEQ ID NO: 320:

(i) SEQUENCE CHARACTERISTICS:
(A) LENGTH: 286 amino acids
(B) TYPE: amino acid
(D) TOPOLOGY: linear
(xi) SEQUENCE DESCRIPTION: SEQ ID NO: 320:

Met Pro Leu Leu Phe Phe Ser Val Ser Thr Leu Phe Ser Gly Ser Val
1               5                   10                  15

Thr Leu Gln Gln Arg Gly Met Phe Leu Pro Trp Thr Gly Thr Gly Glu
            20                  25                  30

Gln Val Leu Ala Leu Leu Trp Pro Arg Phe Glu Leu Ile Leu Glu Met
        35                  40                  45

Asn Val Gln Ser Val Arg Ser Thr Asp Pro Gln Arg Leu Gly Gly Leu
    50                  55                  60

Asp Thr Arg Pro His Tyr Ile Thr Arg Arg Tyr Ala Glu Phe Ser Ser
65                  70                  75                  80

Ala Leu Val Ser Ile Asn Gln Thr Ile Pro Asn Glu Arg Thr Met Gln
                85                  90                  95

Leu Leu Gly Gln Leu Gln Val Glu Val Glu Asn Phe Val Leu Arg Val
            100                 105                 110

Ala Ala Glu Phe Ser Ser Arg Lys Glu Gln Leu Val Phe Leu Ile Asn

        115                 120                 125

Asn Tyr Asp Met Met Leu Gly Val Leu Met Glu Arg Ala Ala Asp Asp
    130                 135                 140

Ser Lys Glu Val Glu Ser Phe Gln Gln Leu Leu Asn Ala Arg Thr Gln
145                 150                 155                 160

Glu Phe Ile Glu Glu Leu Leu Ser Pro Pro Phe Gly Gly Leu Val Ala
                165                 170                 175

Phe Val Lys Glu Ala Glu Ala Leu Ile Glu Arg Gly Gln Ala Glu Arg
            180                 185                 190

Leu Arg Gly Glu Glu Ala Arg Val Thr Gln Leu Ile Arg Gly Phe Gly
        195                 200                 205

Ser Ser Trp Lys Ser Ser Val Glu Ser Leu Ser Gln Asp Val Met Arg
    210                 215                 220

Ser Phe Thr Asn Phe Arg Asn Gly Thr Ser Ile Ile Gln Gly Ala Leu
225                 230                 235                 240

Thr Gln Leu Ile Gln Leu Tyr His Arg Phe His Arg Val Leu Ser Gln
                245                 250                 255

Pro Gln Leu Arg Ala Leu Pro Ala Arg Ala Glu Leu Ile Asn Ile His
            260                 265                 270

His Leu Met Val Glu Leu Lys Lys His Lys Pro Asn Phe Xaa
        275                 280                 285

(2) INFORMATION FOR SEQ ID NO: 321:

(i) SEQUENCE CHARACTERISTICS:
(A) LENGTH: 55 amino acids
(B) TYPE: amino acid
(D) TOPOLOGY: linear
(xi) SEQUENCE DESCRIPTION: SEQ ID NO: 321:

Met Phe Arg Ala Leu Arg Asp Leu Leu Thr His Tyr Pro Gln Gln Ile
1               5                   10                  15

Leu Leu Gln Val Leu Val Val Met Tyr Gln Val Leu Gln Val Trp Glu
            20                  25                  30

Leu Pro Trp Pro Glu Leu Ile His Leu Gln Gly Ile Val Pro Thr Asp
        35                  40                  45

Gln Leu His Leu Lys Gln Xaa
    50                  55

(2) INFORMATION FOR SEQ ID NO: 322:

(i) SEQUENCE CHARACTERISTICS:
(A) LENGTH: 59 amino acids

(B) TYPE: amino acid
(D) TOPOLOGY: linear
(xi) SEQUENCE DESCRIPTION: SEQ ID NO: 322:

Asp Phe Val Pro Val Leu Val Phe Val Leu Ile Lys Ala Asn Pro Pro
1               5                   10                  15

Cys Leu Leu Ser Thr Val Gln Tyr Ile Ser Ser Phe Tyr Ala Ser Cys
            20                  25                  30

Leu Ser Gly Glu Glu Ser Tyr Trp Trp Met Gln Phe Thr Ala Ala Val
        35                  40                  45

Glu Phe Ile Lys Thr Ile Asp Asp Arg Lys Xaa
    50                  55

(2) INFORMATION FOR SEQ ID NO: 323:

(i) SEQUENCE CHARACTERISTICS:
(A) LENGTH: 120 amino acids
(B) TYPE: amino acid
(D) TOPOLOGY: linear
(xi) SEQUENCE DESCRIPTION: SEQ ID NO: 323:

Met His Pro Ala Arg Lys Leu Leu Ser Leu Leu Phe Leu Ile Leu Met
1               5                   10                  15

Gly Thr Glu Leu Thr Gln Asp Ser Ala Ala Pro Asp Ser Leu Leu Arg
            20                  25                  30

Ser Ser Lys Gly Ser Thr Arg Gly Ser Leu Ala Ala Ile Val Ile Trp
        35                  40                  45

Arg Gly Lys Ser Glu Ser Arg Ile Ala Lys Thr Pro Gly Ile Phe Arg
    50                  55                  60

Gly Gly Gly Thr Leu Val Leu Pro Pro Thr His Thr Pro Glu Trp Leu
65                  70                  75                  80

Ile Leu Pro Leu Gly Ile Thr Leu Pro Leu Gly Ala Pro Glu Thr Gly
                85                  90                  95

Gly Gly Asp Cys Ala Ala Glu Thr Trp Lys Gly Ser Gln Arg Ala Gly
            100                 105                 110

Gln Leu Cys Ala Leu Leu Ala Xaa
        115                 120

(2) INFORMATION FOR SEQ ID NO: 324:

(i) SEQUENCE CHARACTERISTICS:
(A) LENGTH: 44 amino acids
(B) TYPE: amino acid
(D) TOPOLOGY: linear
(xi) SEQUENCE DESCRIPTION: SEQ ID NO: 324:

Phe Phe Leu Val Val Phe Ser Leu Ser Phe Xaa Pro Ser Val Leu Thr
1               5                   10                  15

Ser Pro Val His Xaa Pro His Cys Cys Gln Xaa Asp Xaa Ile Leu Phe
            20                  25                  30

Lys Asn Thr Leu Xaa Xaa Phe Xaa Ala Lys Tyr Xaa
        35                  40

(2) INFORMATION FOR SEQ ID NO: 325:

(i) SEQUENCE CHARACTERISTICS:
(A) LENGTH: 59 amino acids
(B) TYPE: amino acid
(D) TOPOLOGY: linear
(xi) SEQUENCE DESCRIPTION: SEQ ID NO: 325:

Met Phe Ser Arg Thr Ser Asn Phe Trp Thr Phe Phe Phe Gln Phe Leu
1               5                   10                  15

Ile Phe Lys Val Phe Leu Val Leu Lys Asn Xaa Phe Thr Ser Gln Lys
            20                  25                  30

Ile Xaa Xaa Ile Xaa Xaa Glu Lys Pro Lys Lys Lys Lys Xaa Arg Gly
        35                  40                  45

Gly Arg Ala Pro Ser Pro Gln Gly Gly Pro Xaa
    50                  55

(2) INFORMATION FOR SEQ ID NO: 326:

(i) SEQUENCE CHARACTERISTICS:
(A) LENGTH: 18 amino acids
(B) TYPE: amino acid
(D) TOPOLOGY: linear
(xi) SEQUENCE DESCRIPTION: SEQ ID NO: 326:

Met Gly Leu Leu Ile Phe Met Leu Leu Ile Gly Ile His Ser Gln Cys
1               5                   10                  15

Ser Xaa

(2) INFORMATION FOR SEQ ID NO: 327:

(i) SEQUENCE CHARACTERISTICS:
(A) LENGTH: 87 amino acids
(B) TYPE: amino acid
(D) TOPOLOGY: linear
(xi) SEQUENCE DESCRIPTION: SEQ ID NO: 327:

Met Val Leu Phe Cys Phe Val Leu Phe Cys Phe Val Phe Glu Met Asp
1               5                   10                  15

Ser Ser Ser Val Thr Gln Ala Gly Val Gln Trp Cys Asp Leu Gly Ser
            20                  25                  30

Leu Gln Ala Pro Pro Pro Gly Phe Ser Pro Phe Ser Cys Leu Ser Leu
        35                  40                  45

Pro Ser Ser Trp Asp Tyr Arg Arg Pro Pro Pro Arg Pro Ala Asn Phe
    50                  55                  60

Leu Tyr Phe Leu Val Glu Thr Gly Phe His His Val Ser Gln Asp Gly
65                  70                  75                  80

Leu Asp Leu Leu Thr Ser Xaa
                85

(2) INFORMATION FOR SEQ ID NO: 328:

      (i) SEQUENCE CHARACTERISTICS:
            (A) LENGTH: 538 amino acids
            (B) TYPE: amino acid
            (D) TOPOLOGY: linear
      (xi) SEQUENCE DESCRIPTION: SEQ ID NO: 328:

Met Ser Thr Lys Lys Leu Cys Ile Val Gly Gly Ile Leu Leu Val Phe
  1               5                  10                  15

Gln Ile Ile Ala Phe Leu Val Gly Gly Leu Ile Ala Pro Gly Pro Thr
            20                  25                  30

Thr Ala Val Ser Tyr Met Ser Val Lys Cys Val Asp Ala Arg Lys Asn
        35                  40                  45

His His Lys Thr Lys Trp Phe Val Pro Trp Gly Pro Asn His Cys Asp
    50                  55                  60

Lys Ile Arg Asp Ile Glu Glu Ala Ile Pro Arg Glu Ile Glu Ala Asn
65                  70                  75                  80

Asp Ile Val Phe Ser Val His Ile Pro Leu Pro His Met Glu Met Ser
                85                  90                  95

Pro Trp Phe Gln Phe Met Leu Phe Ile Leu Gln Leu Asp Ile Ala Phe
            100                 105                 110

Lys Leu Asn Asn Gln Ile Arg Glu Asn Ala Glu Val Ser Met Asp Val
        115                 120                 125

Ser Leu Ala Tyr Arg Asp Asp Ala Phe Ala Glu Trp Thr Glu Met Ala
    130                 135                 140

His Glu Arg Val Pro Arg Lys Leu Lys Cys Thr Phe Thr Ser Pro Lys
145                 150                 155                 160

Thr Pro Glu His Glu Gly Arg Tyr Tyr Glu Cys Asp Val Leu Pro Phe
                165                 170                 175

Met Glu Ile Gly Ser Val Ala His Lys Phe Tyr Leu Leu Asn Ile Arg
            180                 185                 190

Leu Pro Val Asn Glu Lys Lys Lys Ile Asn Val Gly Ile Gly Glu Ile
        195                 200                 205

Lys Asp Ile Arg Leu Val Gly Ile His Gln Asn Gly Gly Phe Thr Lys
    210                 215                 220

Val Trp Phe Ala Met Lys Thr Phe Leu Thr Pro Ser Ile Phe Ile Ile
225                 230                 235                 240

Met Val Trp Tyr Trp Arg Arg Ile Thr Met Met Ser Arg Pro Pro Val
                245                 250                 255

Leu Leu Glu Lys Val Ile Phe Ala Leu Gly Ile Ser Met Thr Phe Ile
            260                 265                 270

Asn Ile Pro Val Glu Trp Phe Ser Ile Gly Phe Asp Trp Thr Trp Met
        275                 280                 285

Leu Leu Phe Gly Asp Ile Arg Gln Gly Ile Phe Tyr Ala Met Leu Leu
    290                 295                 300

Ser Phe Trp Ile Ile Phe Cys Gly Glu His Met Met Asp Gln His Glu
305                 310                 315                 320

Arg Asn His Ile Ala Gly Tyr Trp Lys Gln Val Gly Pro Ile Ala Val
                325                 330                 335

Gly Ser Phe Cys Leu Phe Ile Phe Asp Met Cys Glu Arg Gly Val Gln
            340                 345                 350

Leu Thr Asn Pro Phe Tyr Ser Ile Trp Thr Thr Asp Ile Gly Thr Glu
        355                 360                 365

Leu Ala Met Ala Phe Ile Ile Val Ala Gly Ile Cys Leu Cys Leu Tyr
    370                 375                 380

Phe Leu Phe Leu Cys Phe Met Val Phe Gln Val Phe Arg Asn Ile Ser
385                 390                 395                 400

Gly Lys Gln Ser Ser Leu Pro Ala Met Ser Lys Val Arg Arg Leu His
                405                 410                 415

Tyr Glu Gly Leu Ile Phe Arg Phe Lys Phe Leu Met Leu Ile Thr Leu
            420                 425                 430

Ala Cys Ala Ala Met Thr Val Ile Phe Phe Ile Val Ser Gln Val Thr
        435                 440                 445

Glu Gly His Trp Lys Trp Gly Gly Val Thr Val Gln Val Asn Ser Ala
    450                 455                 460

Phe Phe Thr Gly Ile Tyr Gly Met Trp Asn Leu Tyr Val Phe Ala Leu
465                 470                 475                 480

Met Phe Leu Tyr Ala Pro Ser His Lys Asn Tyr Gly Glu Asp Gln Ser
                485                 490                 495

Asn Gly Met Gln Leu Pro Cys Lys Ser Arg Glu Asp Cys Ala Leu Phe
            500                 505                 510

Val Ser Glu Leu Tyr Gln Glu Leu Phe Ser Ala Ser Lys Tyr Ser Phe
        515                 520                 525

Ile Asn Asp Asn Ala Ala Ser Gly Ile Xaa
    530                 535

(2) INFORMATION FOR SEQ ID NO: 329:

(i) SEQUENCE CHARACTERISTICS:
(A) LENGTH: 202 amino acids
(B) TYPE: amino acid
(D) TOPOLOGY: linear
(xi) SEQUENCE DESCRIPTION: SEQ ID NO: 329:

Met Gly Ile Ala Leu Ala Val Leu Gly Trp Leu Ala Val Met Leu Cys
1               5                   10                  15

Cys Ala Leu Pro Met Trp Arg Val Thr Ala Phe Ile Gly Ser Asn Ile
            20                  25                  30

Val Thr Ser Gln Thr Ile Trp Glu Gly Leu Trp Met Asn Cys Val Val
        35                  40                  45

Gln Ser Thr Gly Gln Met Gln Cys Lys Val Tyr Asp Ser Leu Leu Ala
    50                  55                  60

Leu Pro Gln Asp Leu Gln Ala Ala Arg Ala Leu Val Ile Ile Ser Ile
65                  70                  75                  80

Ile Val Ala Ala Leu Gly Val Leu Leu Ser Val Val Gly Gly Lys Cys
                85                  90                  95

Thr Asn Cys Leu Glu Asp Glu Ser Ala Lys Ala Lys Thr Met Ile Val
            100                 105                 110

Ala Gly Val Val Phe Leu Leu Ala Gly Leu Met Val Ile Val Pro Val
        115                 120                 125

Ser Trp Thr Ala His Asn Ile Ile Gln Asp Phe Tyr Asn Pro Leu Val
    130                 135                 140

Ala Ser Gly Gln Lys Arg Glu Met Gly Ala Ser Leu Tyr Val Gly Trp
145                 150                 155                 160

Ala Ala Ser Gly Leu Leu Leu Leu Gly Gly Gly Leu Leu Cys Cys Asn
                165                 170                 175

Cys Pro Pro Arg Thr Asp Lys Pro Tyr Ser Ala Lys Tyr Ser Ala Ala
            180                 185                 190

Arg Ser Ala Ala Ala Ser Asn Tyr Val Xaa
        195                 200

(2) INFORMATION FOR SEQ ID NO: 330:

(i) SEQUENCE CHARACTERISTICS:
(A) LENGTH: 263 amino acids
(B) TYPE: amino acid
(D) TOPOLOGY: linear
(xi) SEQUENCE DESCRIPTION: SEQ ID NO: 330:

Met Ala Thr Val Thr Ala Thr Thr Lys Val Pro Glu Ile Arg Asp Val
1               5                   10                  15

Thr Arg Ile Glu Arg Ile Gly Ala His Ser His Ile Arg Gly Leu Gly
            20                  25                  30

Leu Asp Asp Ala Leu Glu Pro Arg Gln Ala Ser Gln Gly Met Val Gly
        35                  40                  45

Gln Leu Ala Ala Arg Arg Ala Ala Gly Val Val Leu Glu Met Ile Arg
    50                  55                  60

Glu Gly Lys Ile Ala Gly Arg Ala Val Leu Ile Ala Gly Gln Pro Gly
65                  70                  75                  80

Thr Gly Lys Thr Ala Ile Ala Met Gly Met Ala Gln Ala Leu Gly Pro
                85                  90                  95

Asp Thr Pro Phe Thr Ala Ile Ala Gly Ser Glu Ile Phe Ser Leu Glu
            100                 105                 110

Met Ser Lys Thr Glu Ala Leu Thr Gln Ala Phe Arg Arg Ser Ile Gly
        115                 120                 125

Val Arg Ile Lys Glu Glu Thr Glu Ile Ile Glu Gly Glu Val Val Glu
    130                 135                 140

Ile Gln Ile Asp Arg Pro Ala Thr Gly Thr Gly Ser Lys Val Gly Lys
145                 150                 155                 160

Leu Thr Leu Lys Thr Thr Glu Met Glu Thr Ile Tyr Asp Leu Gly Thr
                165                 170                 175

Lys Met Ile Xaa Ser Leu Thr Lys Asp Lys Val Gln Ala Gly Asp Val
            180                 185                 190

Ile Thr Ile Asp Lys Ala Thr Gly Lys Ile Ser Lys Leu Gly Arg Ser
        195                 200                 205

Phe Thr Arg Ala Arg Glu Leu Arg Arg Tyr Gly Leu Pro Asp Gln Val
    210                 215                 220

Arg Ala Val Pro Arg Trp Gly Ala Pro Glu Thr Gln Gly Gly Gly Ala
225                 230                 235                 240

His Arg Val Pro Ala Arg Asp Arg Arg His Gln Leu Ser His Pro Gly
                245                 250                 255

Leu Pro Gly Ala Leu Leu Arg
            260

(2) INFORMATION FOR SEQ ID NO: 331:

(i) SEQUENCE CHARACTERISTICS:
(A) LENGTH: 260 amino acids
(B) TYPE: amino acid
(D) TOPOLOGY: linear
(xi) SEQUENCE DESCRIPTION: SEQ ID NO: 331:

Met Leu Ala Leu Leu Gly Leu Ser Gln Ala Leu Asn Ile Leu Leu Gly
1               5                   10                  15

Leu Lys Gly Leu Ala Pro Ala Glu Ile Ser Ala Val Cys Glu Lys Gly
            20                  25                  30

Asn Phe Asn Val Ala His Gly Leu Ala Trp Ser Tyr Tyr Ile Gly Tyr
        35                  40                  45

Leu Arg Leu Ile Leu Pro Glu Leu Gln Ala Arg Ile Arg Thr Tyr Asn
    50                  55                  60

Gln His Tyr Asn Asn Leu Leu Arg Gly Ala Val Ser Gln Arg Leu Tyr
65                  70                  75                  80

Ile Leu Leu Pro Leu Asp Cys Gly Val Pro Asp Asn Leu Ser Met Ala
                85                  90                  95

Asp Pro Asn Ile Arg Phe Leu Asp Lys Leu Pro Gln Gln Thr Gly Asp
            100                 105                 110

Arg Ala Gly Ile Lys Asp Arg Val Tyr Ser Asn Ser Ile Tyr Glu Leu
        115                 120                 125

Leu Glu Asn Gly Gln Arg Ala Gly Thr Cys Val Leu Glu Tyr Ala Thr
    130                 135                 140

Pro Leu Gln Thr Leu Phe Ala Met Ser Gln Tyr Ser Gln Ala Gly Phe
145                 150                 155                 160

Ser Gly Glu Asp Arg Leu Glu Gln Ala Lys Leu Phe Cys Arg Thr Leu
                165                 170                 175

Glu Asp Ile Leu Ala Asp Ala Pro Glu Ser Gln Asn Asn Cys Arg Leu
            180                 185                 190

Ile Ala Tyr Gln Glu Pro Ala Asp Asp Ser Ser Phe Ser Leu Ser Gln
        195                 200                 205

Glu Val Leu Arg His Leu Arg Gln Glu Glu Lys Glu Glu Val Thr Val
    210                 215                 220

Gly Ser Leu Lys Thr Ser Ala Val Pro Ser Thr Ser Thr Met Ser Gln
225                 230                 235                 240

Glu Pro Glu Leu Leu Ile Ser Gly Met Glu Lys Pro Leu Pro Leu Arg
                245                 250                 255

Thr Asp Phe Ser
            260

(2) INFORMATION FOR SEQ ID NO: 332:

(i) SEQUENCE CHARACTERISTICS:
(A) LENGTH: 48 amino acids
(B) TYPE: amino acid
(D) TOPOLOGY: linear
(xi) SEQUENCE DESCRIPTION: SEQ ID NO: 332:

Met Thr Pro Gln Lys Pro Ala Leu Ala Val Leu Leu Leu Glu Val Pro
1               5                   10                  15

Leu Leu Leu Thr Leu Ser Val Leu Lys Lys Arg Cys Leu Val Thr Cys
            20                  25                  30

Glu Pro Thr Ser Arg Phe Val Ser Cys Asp Leu Pro Leu Ser Val Xaa
        35                  40                  45

(2) INFORMATION FOR SEQ ID NO: 333:

(i) SEQUENCE CHARACTERISTICS:
(A) LENGTH: 334 amino acids
(B) TYPE: amino acid
(D) TOPOLOGY: linear
(xi) SEQUENCE DESCRIPTION: SEQ ID NO: 333:

Met Ala Ala Ala Ala Trp Leu Gln Val Leu Pro Val Ile Leu Leu Leu
1               5                   10                  15

Leu Gly Ala His Pro Ser Pro Leu Ser Phe Phe Ser Ala Gly Pro Ala
            20                  25                  30

Thr Val Ala Ala Ala Asp Arg Ser Lys Trp His Ile Pro Ile Pro Ser
        35                  40                  45

Gly Lys Asn Tyr Phe Ser Phe Gly Lys Ile Leu Phe Arg Asn Thr Thr
    50                  55                  60

Ile Phe Leu Lys Phe Asp Gly Glu Pro Cys Asp Leu Ser Leu Asn Ile
65                  70                  75                  80

Thr Trp Tyr Leu Lys Ser Ala Asp Cys Tyr Asn Glu Ile Tyr Asn Phe
                85                  90                  95

Lys Ala Glu Glu Val Glu Leu Tyr Leu Glu Lys Leu Lys Glu Lys Arg
            100                 105                 110

Gly Leu Ser Gly Lys Tyr Gln Thr Ser Ser Lys Leu Phe Gln Asn Cys
        115                 120                 125

Ser Glu Leu Phe Lys Thr Gln Thr Phe Ser Gly Asp Phe Met His Arg
    130                 135                 140

Leu Pro Leu Leu Gly Glu Lys Gln Glu Ala Lys Glu Asn Gly Thr Asn
145                 150                 155                 160

Leu Thr Phe Ile Gly Asp Lys Thr Ala Met His Glu Pro Leu Gln Thr
                165                 170                 175

Trp Gln Asp Ala Pro Tyr Ile Phe Ile Val His Ile Gly Ile Ser Ser
            180                 185                 190

Ser Lys Glu Ser Ser Lys Glu Asn Ser Leu Ser Asn Leu Phe Thr Met
        195                 200                 205

Thr Val Glu Val Lys Gly Pro Tyr Glu Tyr Leu Thr Leu Glu Asp Tyr
    210                 215                 220

Pro Leu Met Ile Phe Phe Met Val Met Cys Ile Val Tyr Val Leu Phe
225                 230                 235                 240

Gly Val Leu Trp Leu Ala Trp Ser Ala Cys Tyr Trp Arg Asp Leu Leu
                245                 250                 255

Arg Ile Gln Phe Trp Ile Gly Ala Val Ile Phe Leu Gly Met Leu Glu
            260                 265                 270

Lys Ala Val Phe Tyr Ala Glu Phe Gln Asn Ile Arg Tyr Lys Gly Xaa
        275                 280                 285

Ser Val Gln Gly Ala Leu Ile Leu Ala Glu Leu Leu Ser Ala Val Lys
    290                 295                 300

Arg Ser Leu Ala Arg Thr Leu Val Ile Ile Val Ser Leu Gly Tyr Gly
305                 310                 315                 320

Ile Val Lys Pro Arg Leu Glu Ser Leu Phe Ile Arg Leu Xaa
                325                 330

(2) INFORMATION FOR SEQ ID NO: 334:

(i) SEQUENCE CHARACTERISTICS:
(A) LENGTH: 200 amino acids
(B) TYPE: amino acid
(D) TOPOLOGY: linear
(xi) SEQUENCE DESCRIPTION: SEQ ID NO: 334:

Met Val Leu Xaa Val Val Thr Leu Gly Leu Ala Leu Phe Thr Leu Cys
1               5                   10                  15

Gly Lys Phe Lys Arg Trp Lys Leu Asn Gly Ala Phe Leu Leu Ile Thr
            20                  25                  30

Ala Phe Leu Ser Val Leu Ile Trp Val Ala Trp Met Thr Met Tyr Leu
        35                  40                  45

Phe Gly Asn Val Lys Leu Gln Gln Gly Asp Ala Trp Asn Asp Pro Thr
    50                  55                  60

Leu Ala Ile Thr Leu Ala Ala Ser Ala Gly Ser Ser Ser Ser Ser Thr
65                  70                  75                  80

Pro Ser Leu Arg Ser Thr Ala Pro Phe Cys Gln Pro Cys Arg Arg Thr
                85                  90                  95

Arg Pro Thr Thr Ser Thr Arg Arg Ser Pro Gly Cys Gly Arg Arg Pro
            100                 105                 110

Ser Arg Arg Thr Cys Ser Cys Arg Gly Pro Ile Trp Arg Thr Arg Pro
        115                 120                 125

Ser Pro Trp Met Asn Thr Met Gln Leu Ser Glu Gln Gln Asp Phe Pro
    130                 135                 140

Thr Ala Ala Trp Glu Lys Asp Pro Val Ala Ala Trp Gly Lys Asp Pro
145                 150                 155                 160

Ala Leu Arg Leu Glu Ala Thr Cys Ile Ser Gln Leu Arg Trp Pro Ser
                165                 170                 175

Cys Ser Thr Val Gly Pro Ser Gln Leu Leu Arg Gln Val Thr Gln Glu
            180                 185                 190

Xaa Thr Phe Gly Glu Arg Leu Xaa
        195                 200

(2) INFORMATION FOR SEQ ID NO: 335:

(i) SEQUENCE CHARACTERISTICS:
(A) LENGTH: 24 amino acids
(B) TYPE: amino acid
(D) TOPOLOGY: linear
(xi) SEQUENCE DESCRIPTION: SEQ ID NO: 335:

Met Leu Leu His His Gln Leu Leu Ile Val Thr Leu His Leu Val Leu
1               5                   10                  15

Leu Leu Ala Thr Leu Leu Val Xaa
            20

(2) INFORMATION FOR SEQ ID NO: 336:

(i) SEQUENCE CHARACTERISTICS:
(A) LENGTH: 143 amino acids
(B) TYPE: amino acid
(D) TOPOLOGY: linear
(xi) SEQUENCE DESCRIPTION: SEQ ID NO: 336:

Met Thr Lys Ala Leu Leu Ile Tyr Leu Val Ser Ser Phe Leu Ala Leu
1               5                   10                  15

Asn Gln Ala Ser Leu Ile Ser Arg Cys Asp Leu Ala Gln Val Leu Gln
            20                  25                  30

Leu Glu Asp Leu Asp Gly Phe Glu Gly Tyr Ser Leu Ser Asp Trp Leu
        35                  40                  45

Cys Leu Ala Phe Val Glu Ser Lys Phe Asn Ile Ser Lys Ile Asn Glu
    50                  55                  60

Asn Ala Asp Gly Ser Phe Asp Tyr Gly Leu Phe Gln Ile Asn Ser His
65                  70                  75                  80

Tyr Trp Cys Asn Xaa Tyr Lys Ser Tyr Ser Glu Asn Leu Cys His Val
                85                  90                  95

Asp Cys Gln Asp Leu Leu Asn Pro Asn Leu Leu Ala Gly Ile His Cys
            100                 105                 110

Ala Lys Arg Ile Val Ser Gly Ala Arg Gly Met Asn Asn Trp Val Arg
        115                 120                 125

Met Glu Xaa Cys Thr Val Gln Ala Gly His Ser Ser Thr Gly Xaa
    130                 135                 140

(2) INFORMATION FOR SEQ ID NO: 337:

(i) SEQUENCE CHARACTERISTICS:
(A) LENGTH: 95 amino acids
(B) TYPE: amino acid
(D) TOPOLOGY: linear
(xi) SEQUENCE DESCRIPTION: SEQ ID NO: 337:

Met Leu Val Ile Ala Gly Gly Ile Leu Ala Ala Leu Leu Leu Leu Ile
1               5                   10                  15

Val Val Val Leu Cys Leu Tyr Phe Lys Ile His Asn Ala Leu Lys Ala
            20                  25                  30

Ala Lys Glu Pro Glu Ala Val Ala Val Lys Asn His Asn Pro Asp Lys
        35                  40                  45

Val Trp Trp Ala Lys Asn Ser Gln Ala Lys Thr Ile Ala Thr Glu Ser
    50                  55                  60

Cys Pro Ala Leu Gln Cys Cys Glu Gly Tyr Arg Met Cys Ala Ser Phe
65                  70                  75                  80

Asp Ser Leu Pro Pro Cys Cys Cys Asp Ile Asn Glu Gly Leu Xaa
                85                  90                  95

(2) INFORMATION FOR SEQ ID NO: 338:

(i) SEQUENCE CHARACTERISTICS:
(A) LENGTH: 38 amino acids
(B) TYPE: amino acid
(D) TOPOLOGY: linear
(xi) SEQUENCE DESCRIPTION: SEQ ID NO: 338:

Met Leu Leu Lys Ser Asn Ile Leu Met Leu Asn Leu Phe Ala Ala Asn
1               5                   10                  15

Val Gly Ala Asn Phe Ala Leu Thr Val Glu Lys Ile Gly Met Ile Leu
            20                  25                  30

Leu Asn Val Ser Gly Xaa

        35

(2) INFORMATION FOR SEQ ID NO: 339:

(i) SEQUENCE CHARACTERISTICS:
(A) LENGTH: 39 amino acids
(B) TYPE: amino acid
(D) TOPOLOGY: linear
(xi) SEQUENCE DESCRIPTION: SEQ ID NO: 339:

Met Leu Val Val Ala Phe Gly Leu Leu Val Leu Tyr Ile Leu Leu Ala
1               5                   10                  15

Ser Ser Trp Lys Arg Pro Glu Pro Gly Ile Leu Thr Asp Arg Gln Pro
            20                  25                  30

Leu Leu His Asp Gly Glu Xaa
        35

(2) INFORMATION FOR SEQ ID NO: 340:

(i) SEQUENCE CHARACTERISTICS:
(A) LENGTH: 71 amino acids
(B) TYPE: amino acid
(D) TOPOLOGY: linear
(xi) SEQUENCE DESCRIPTION: SEQ ID NO: 340:

Ser Asp Pro Leu Ala Ser Ala Ser Gln Asn Ala Gly Ile Val Ser Val
1               5                   10                  15

Gly Leu Cys Thr Arg Pro Gly Pro Gln Phe Lys Asn Ala Gln Pro Pro
            20                  25                  30

Phe Pro Xaa Gln Lys Ala Pro Arg Cys Leu Trp Glu Asn Gln Pro Pro
        35                  40                  45

Pro Trp Arg Lys Ala Trp Asp Leu Pro Ser His Leu Gly Arg Arg Gly
    50                  55                  60

Ile Cys Gly Lys Ser Phe Xaa
65                  70

(2) INFORMATION FOR SEQ ID NO: 341:

(i) SEQUENCE CHARACTERISTICS:
(A) LENGTH: 85 amino acids
(B) TYPE: amino acid
(D) TOPOLOGY: linear
(xi) SEQUENCE DESCRIPTION: SEQ ID NO: 341:

Tyr Val Met Ile Phe Lys Lys Glu Phe Ala Pro Ser Asp Glu Glu Leu
1               5                   10                  15

Asp Ser Tyr Arg Arg Gly Glu Glu Trp Asp Pro Gln Lys Ala Glu Glu

20                  25                  30

Lys Arg Asn Xaa Lys Glu Leu Ala Gln Arg Gln Xaa Gly Gly Gly Ser
        35                  40                  45

Pro Ala Gly Ala Cys Gly Gly Glu Pro Cys Gln Arg Leu Gln Gly Gln
    50                  55                  60

Val Gln Pro Pro His Arg Gln Gly Ser Ser Gln Arg Arg Ser Pro His
65                  70                  75                  80

Ala Thr Gly Gln Xaa
                85

(2) INFORMATION FOR SEQ ID NO: 342:

(i) SEQUENCE CHARACTERISTICS:
(A) LENGTH: 90 amino acids
(B) TYPE: amino acid
(D) TOPOLOGY: linear
(xi) SEQUENCE DESCRIPTION: SEQ ID NO: 342:

Met Trp Asp Trp Asp Trp Ser Ala Pro Trp Ser Trp Pro Leu Trp Leu
 1               5                  10                  15

Ser Leu Ala Leu Val Cys Leu Ser Ala Gly Ala Lys Gly His Arg Ala
            20                  25                  30

Ser Glu Ala Gly His Ala Arg Ala Leu Thr Cys Glu Met Gly Ser Glu
        35                  40                  45

Phe Xaa Thr Ala Xaa Gly Leu Val Leu Gly Xaa Xaa Xaa Trp Thr Xaa
    50                  55                  60

Xaa Asn Gly Ser Ala Gly Pro Glu Arg Arg Gly Trp Arg Pro Ala Ala
65                  70                  75                  80

Phe Leu Ala Val Phe Leu Leu Gly Asp Xaa
                85                  90

(2) INFORMATION FOR SEQ ID NO: 343:

(i) SEQUENCE CHARACTERISTICS:
(A) LENGTH: 48 amino acids
(B) TYPE: amino acid
(D) TOPOLOGY: linear
(xi) SEQUENCE DESCRIPTION: SEQ ID NO: 343:

Met Phe Gly Pro Thr Phe His Ser Leu Val Leu Val Pro Pro Trp Pro
 1               5                  10                  15

Asn Leu Ser Leu Leu His Phe Thr Ser Pro Val Gly Gln His Ser Ser
            20                  25                  30

Phe Leu Pro Thr Ser Leu Arg Leu Xaa Lys Lys Lys Lys Lys Lys Lys
        35                  40                  45

(2) INFORMATION FOR SEQ ID NO: 344:

(i) SEQUENCE CHARACTERISTICS:
(A) LENGTH: 56 amino acids
(B) TYPE: amino acid
(D) TOPOLOGY: linear
(xi) SEQUENCE DESCRIPTION: SEQ ID NO: 344:

Met Cys Ser Lys Asn Gly Phe Leu Leu Ala Trp Ser Trp Asn Ser Pro
1               5                   10                  15

Trp Leu Pro Gln Ala Ser Leu Ala His Gly Cys Trp Gly Arg Trp Met
            20                  25                  30

Ser Asp Leu Val Gly Cys Ser Arg Glu Asn Lys Cys Ala Leu Arg Asp
        35                  40                  45

His Ser Glu Arg Val Gln Gly Xaa
    50                  55

(2) INFORMATION FOR SEQ ID NO: 345:

(i) SEQUENCE CHARACTERISTICS:
(A) LENGTH: 222 amino acids
(B) TYPE: amino acid
(D) TOPOLOGY: linear
(xi) SEQUENCE DESCRIPTION: SEQ ID NO: 345:

Ser Pro Leu Xaa Phe Cys Val Val Leu Leu Leu Gln Ala Ala Arg Gly
1               5                   10                  15

Tyr Val Val Arg Lys Pro Ala Gln Ser Arg Leu Asp Asp Asp Pro Pro
            20                  25                  30

Pro Ser Thr Leu Leu Lys Asp Tyr Gln Asn Val Pro Gly Ile Glu Lys
        35                  40                  45

Val Asp Asp Val Val Lys Arg Leu Leu Ser Leu Glu Met Ala Asn Lys
    50                  55                  60

Lys Glu Met Leu Lys Ile Lys Gln Glu Gln Phe Met Lys Lys Ile Val
65                  70                  75                  80

Ala Asn Pro Glu Asp Thr Arg Ser Leu Glu Ala Arg Ile Ile Ala Leu
                85                  90                  95

Ser Val Lys Ile Arg Ser Tyr Glu Glu His Leu Glu Lys His Arg Lys
            100                 105                 110

Asp Lys Ala His Lys Arg Tyr Leu Leu Met Ser Ile Asp Gln Arg Lys
        115                 120                 125

Lys Met Leu Lys Asn Leu Arg Asn Thr Asn Tyr Asp Val Phe Glu Lys
    130                 135                 140

Ile Cys Trp Gly Leu Gly Ile Glu Tyr Thr Phe Pro Pro Leu Tyr Tyr
145                 150                 155                 160

Arg Arg Ala His Arg Arg Phe Val Thr Lys Lys Ala Leu Cys Ile Arg
                165                 170                 175

Val Phe Gln Glu Thr Gln Lys Leu Lys Lys Arg Arg Arg Ala Leu Lys
            180                 185                 190

Ala Ala Ala Ala Ala Gln Lys Gln Ala Lys Arg Arg Asn Pro Asp Ser
        195                 200                 205

Pro Ala Lys Ala Ile Pro Lys Thr Leu Lys Asp Ser Gln Xaa
    210                 215                 220

(2) INFORMATION FOR SEQ ID NO: 346:

(i) SEQUENCE CHARACTERISTICS:
(A) LENGTH: 64 amino acids
(B) TYPE: amino acid
(D) TOPOLOGY: linear
(xi) SEQUENCE DESCRIPTION: SEQ ID NO: 346:

Met Gly Ala Pro Ala Ala Ser Leu Leu Leu Leu Leu Leu Leu Phe Ala
  1               5                  10                  15

Cys Cys Trp Ala Pro Gly Gly Ala Asn Leu Ser Gln Asp Asp Ser Gln
            20                  25                  30

Pro Trp Thr Ser Asp Glu Thr Val Val Ala Gly Gly Thr Val Val Leu
        35                  40                  45

Lys Cys Gln Val Lys Asp His Glu Asp Ser Ser Leu Gln Trp Ser Xaa
    50                  55                  60

(2) INFORMATION FOR SEQ ID NO: 347:

(i) SEQUENCE CHARACTERISTICS:
(A) LENGTH: 154 amino acids
(B) TYPE: amino acid
(D) TOPOLOGY: linear
(xi) SEQUENCE DESCRIPTION: SEQ ID NO: 347:

Met Val Ala Pro Val Trp Tyr Leu Val Ala Ala Ala Leu Leu Val Gly
  1               5                  10                  15

Phe Ile Leu Phe Leu Thr Arg Ser Arg Gly Arg Ala Ala Ser Ala Gly
            20                  25                  30

Gln Glu Pro Leu His Asn Glu Glu Leu Ala Gly Ala Gly Arg Val Ala

        35                  40                  45

Gln Pro Gly Pro Leu Glu Pro Glu Glu Pro Arg Ala Gly Gly Arg Pro
    50                  55                  60

Arg Arg Arg Arg Asp Leu Gly Ser Arg Leu Gln Ala Gln Arg Arg Ala
65                  70                  75                  80

Gln Arg Val Ala Trp Ala Glu Ala Asp Glu Asn Glu Glu Glu Ala Val
                85                  90                  95

Ile Leu Ala Gln Glu Glu Glu Gly Val Glu Lys Pro Ala Glu Xaa His
            100                 105                 110

Leu Ser Gly Lys Ile Gly Ala Lys Lys Leu Arg Xaa Xaa Glu Glu Lys
        115                 120                 125

Gln Ala Arg Lys Ala Gln Xaa Glu Ala Glu Glu Ala Glu Arg Glu Xaa
    130                 135                 140

Arg Lys Arg Leu Glu Ser Gln Arg Glu Xaa
145                 150

(2) INFORMATION FOR SEQ ID NO: 348:

(i) SEQUENCE CHARACTERISTICS:
(A) LENGTH: 17 amino acids
(B) TYPE: amino acid
(D) TOPOLOGY: linear
(xi) SEQUENCE DESCRIPTION: SEQ ID NO: 348:

Met Gln Lys Cys Met Leu Ser Ala Leu Val Phe His Ile Gln Trp Ser
1               5                   10                  15

Xaa

(2) INFORMATION FOR SEQ ID NO: 349:

(i) SEQUENCE CHARACTERISTICS:
(A) LENGTH: 10 amino acids
(B) TYPE: amino acid
(D) TOPOLOGY: linear
(xi) SEQUENCE DESCRIPTION: SEQ ID NO: 349:

Met Leu Val Cys Ser Phe Leu Phe Leu Xaa
1               5                   10

(2) INFORMATION FOR SEQ ID NO: 350:

(i) SEQUENCE CHARACTERISTICS:
(A) LENGTH: 14 amino acids
(B) TYPE: amino acid
(D) TOPOLOGY: linear

(xi) SEQUENCE DESCRIPTION: SEQ ID NO: 350:

Val Ile Glu Leu Cys Val Ser Leu Arg Ser Leu Asn Phe Xaa
1               5                   10

(2) INFORMATION FOR SEQ ID NO: 351:

(i) SEQUENCE CHARACTERISTICS:
(A) LENGTH: 18 amino acids
(B) TYPE: amino acid
(D) TOPOLOGY: linear
(xi) SEQUENCE DESCRIPTION: SEQ ID NO: 351:

Met Cys Glu Phe Xaa Xaa Xaa Ile Met Xaa Leu Ala Gly Tyr Phe Ala
1               5                   10                  15

Cys Xaa

(2) INFORMATION FOR SEQ ID NO: 352:

(i) SEQUENCE CHARACTERISTICS:
(A) LENGTH: 62 amino acids
(B) TYPE: amino acid
(D) TOPOLOGY: linear
(xi) SEQUENCE DESCRIPTION: SEQ ID NO: 352:

Met Val Gly Gly Tyr Val Ser Ser Phe Ser Phe Pro Pro Val Ser Ser
1               5                   10                  15

Ser Leu Leu Leu Pro Ala Ser Phe Ala Phe Pro Phe Leu Pro Gly Thr
            20                  25                  30

Pro Cys Pro Phe Leu Tyr Phe Leu Pro Ser Pro Phe Ser Pro Leu Pro
        35                  40                  45

Leu Ser Leu Thr Arg Ser Asn Ser Phe Leu Leu Asn Gly Xaa
    50                  55                  60

(2) INFORMATION FOR SEQ ID NO: 353:

(i) SEQUENCE CHARACTERISTICS:
(A) LENGTH: 33 amino acids
(B) TYPE: amino acid
(D) TOPOLOGY: linear
(xi) SEQUENCE DESCRIPTION: SEQ ID NO: 353:

Glu Lys Lys Ser Met Ser Val Ser Asp Ile Tyr Ala Leu Glu Ser Leu
1               5                   10                  15

Gly Arg Ser Leu Phe Thr Leu Asn Ser Met Cys Leu Pro Leu Ser Phe
            20                  25                  30

Xaa

(2) INFORMATION FOR SEQ ID NO: 354:

(i) SEQUENCE CHARACTERISTICS:
(A) LENGTH: 245 amino acids
(B) TYPE: amino acid
(D) TOPOLOGY: linear
(xi) SEQUENCE DESCRIPTION: SEQ ID NO: 354:

Met Gly Gly Ala Ser Arg Arg Val Glu Ser Gly Ala Trp Ala Tyr Leu
1               5                   10                  15

Ser Pro Leu Val Leu Arg Lys Glu Leu Glu Ser Leu Val Glu Asn Glu
            20                  25                  30

Gly Ser Glu Val Leu Ala Leu Pro Glu Leu Pro Ser Ala His Pro Ile
        35                  40                  45

Ile Phe Trp Asn Leu Leu Trp Tyr Phe Gln Arg Leu Arg Leu Pro Ser
    50                  55                  60

Ile Leu Pro Gly Leu Val Leu Ala Ser Cys Asp Gly Pro Ser Xaa Ser
65                  70                  75                  80

Gln Ala Pro Ser Pro Trp Leu Thr Pro Asp Pro Ala Ser Val Gln Val
                85                  90                  95

Arg Leu Leu Trp Asp Val Leu Thr Pro Asp Pro Asn Ser Cys Pro Pro
            100                 105                 110

Leu Tyr Val Leu Trp Arg Val His Ser Gln Ile Pro Gln Arg Val Val
        115                 120                 125

Trp Pro Gly Pro Val Pro Ala Ser Leu Ser Leu Ala Leu Leu Glu Ser
    130                 135                 140

Val Leu Arg His Val Gly Leu Asn Glu Val His Lys Ala Val Gly Leu
145                 150                 155                 160

Leu Leu Glu Thr Leu Gly Pro Pro Pro Thr Gly Leu His Leu Gln Arg
                165                 170                 175

Gly Ile Tyr Arg Glu Ile Leu Phe Leu Thr Met Ala Ala Leu Gly Lys
            180                 185                 190

Asp His Val Asp Ile Val Ala Phe Asp Lys Lys Tyr Lys Ser Ala Phe
        195                 200                 205

Asn Lys Leu Ala Ser Ser Met Gly Lys Glu Glu Leu Arg His Arg Arg
    210                 215                 220

Ala Gln Met Pro Thr Pro Lys Ala Ile Asp Cys Arg Lys Cys Phe Gly
225                 230                 235                 240

Ala Pro Pro Glu Cys
                245

(2) INFORMATION FOR SEQ ID NO: 355:

(i) SEQUENCE CHARACTERISTICS:
(A) LENGTH: 35 amino acids
(B) TYPE: amino acid
(D) TOPOLOGY: linear
(xi) SEQUENCE DESCRIPTION: SEQ ID NO: 355:

Met Lys Phe Ser Leu Leu Phe Leu Pro Met Leu Leu Ile Leu Lys Pro
1               5                   10                  15

Asp Leu Phe His Ile Ser Ile Cys Thr Leu Ala Ala Cys Gly Leu Thr
            20                  25                  30

Phe Pro Xaa
        35

(2) INFORMATION FOR SEQ ID NO: 356:

(i) SEQUENCE CHARACTERISTICS:
(A) LENGTH: 22 amino acids
(B) TYPE: amino acid
(D) TOPOLOGY: linear
(xi) SEQUENCE DESCRIPTION: SEQ ID NO: 356:

Met Leu Phe Phe Phe Ile Leu His Leu Leu Ser Ile Met Ser Phe Leu
1               5                   10                  15

Ser Pro Asp Ile Met Xaa
            20

(2) INFORMATION FOR SEQ ID NO: 357:

(i) SEQUENCE CHARACTERISTICS:
(A) LENGTH: 98 amino acids
(B) TYPE: amino acid
(D) TOPOLOGY: linear
(xi) SEQUENCE DESCRIPTION: SEQ ID NO: 357:

Met Phe Gly Leu Leu Val Glu Ser Gln Thr Leu Leu Glu Glu Asn Ala
1               5                   10                  15

Val Gln Gly Thr Glu Arg Thr Leu Gly Leu Asn Ile Ala Pro Phe Ile
            20                  25                  30

Asn Gln Phe Gln Val Pro Ile Arg Val Phe Leu Asp Leu Ser Ser Leu
        35                  40                  45

Pro Cys Ile Pro Leu Ser Lys Pro Val Glu Leu Leu Arg Leu Asp Leu
    50                  55                  60

Met Thr Pro Tyr Leu Asn Thr Ser Asn Arg Glu Val Lys Val Tyr Val
65                  70                  75                  80

Cys Xaa Ile Trp Glu Asp Leu Thr Ala Ile Pro Phe Trp Val Ser Tyr
                85                  90                  95

Val Pro

(2) INFORMATION FOR SEQ ID NO: 358:

        (i) SEQUENCE CHARACTERISTICS:
                (A) LENGTH: 78 amino acids
                (B) TYPE: amino acid
                (D) TOPOLOGY: linear
        (xi) SEQUENCE DESCRIPTION: SEQ ID NO: 358:

Met Phe Gly Ala His Arg Xaa Trp Gln Gly Ser Val Leu Leu Phe Leu
  1               5                  10                  15

Ser Phe Ala Trp Gly Asn Gly Gly Ser Val Thr Phe Ser Asp Val Pro
            20                  25                  30

Arg Val Met Pro Leu Ala Gly Gly Pro Xaa Xaa Gln Val Ser Ser Thr
        35                  40                  45

Pro Arg Pro Pro Pro His Gln Val Thr Ser Ser Pro Gly Leu Glu Ser
    50                  55                  60

Ala His Ile Val Cys Pro Glu Arg Lys Lys Lys Lys Lys Lys
 65                  70                  75

(2) INFORMATION FOR SEQ ID NO: 359:

        (i) SEQUENCE CHARACTERISTICS:
                (A) LENGTH: 31 amino acids
                (B) TYPE: amino acid
                (D) TOPOLOGY: linear
        (xi) SEQUENCE DESCRIPTION: SEQ ID NO: 359:

Thr Leu Leu Xaa Phe Leu Xaa Leu Leu Thr Thr Glu Gly Gly Arg Glu
  1               5                  10                  15

Asn Ile Phe Xaa Gly Arg Ile Leu Xaa Leu Gln Xaa Ser Pro Xaa
            20                  25                  30

(2) INFORMATION FOR SEQ ID NO: 360:

        (i) SEQUENCE CHARACTERISTICS:
                (A) LENGTH: 57 amino acids
                (B) TYPE: amino acid
                (D) TOPOLOGY: linear
        (xi) SEQUENCE DESCRIPTION: SEQ ID NO: 360:

Met Leu Ser Phe Phe Ile Cys Leu Leu Ile Phe Val His Leu Leu Leu
  1               5                  10                  15

Leu Ser Phe Leu Ile Ser Asp Trp Pro Pro Pro Thr Gly Ser Ala Xaa
            20                  25                  30

His Lys Ile Leu Arg Leu Met Val Val Gln Arg Leu Ser Leu Leu Asp
        35                  40                  45

Gln Arg Lys Arg Trp Ser Glu Ala Xaa
    50                  55

(2) INFORMATION FOR SEQ ID NO: 361:

(i) SEQUENCE CHARACTERISTICS:
(A) LENGTH: 3 amino acids
(B) TYPE: amino acid
(D) TOPOLOGY: linear
(xi) SEQUENCE DESCRIPTION: SEQ ID NO: 361:

Lys Tyr Xaa
1

(2) INFORMATION FOR SEQ ID NO: 362:

(i) SEQUENCE CHARACTERISTICS:
(A) LENGTH: 32 amino acids
(B) TYPE: amino acid
(D) TOPOLOGY: linear
(xi) SEQUENCE DESCRIPTION: SEQ ID NO: 362:

Trp Ser Ser Ala Ser Ser Ser Trp Val Thr Thr Pro Glu Arg Ile Arg
1               5                   10                  15

Pro Arg Met Asp Thr Leu Pro Val Lys Gly His Phe Leu Ser Met Xaa
            20                  25                  30

(2) INFORMATION FOR SEQ ID NO: 363:

(i) SEQUENCE CHARACTERISTICS:
(A) LENGTH: 28 amino acids
(B) TYPE: amino acid
(D) TOPOLOGY: linear
(xi) SEQUENCE DESCRIPTION: SEQ ID NO: 363:

Asp Ile Phe Val Phe Leu Leu Ser Thr Arg Ala Gly Gly Leu Gly Ile
1               5                   10                  15

Asn Leu Thr Ala Xaa Asp Thr Val His Phe Leu Xaa
            20                  25

(2) INFORMATION FOR SEQ ID NO: 364:

(i) SEQUENCE CHARACTERISTICS:
(A) LENGTH: 15 amino acids
(B) TYPE: amino acid
(D) TOPOLOGY: linear
(xi) SEQUENCE DESCRIPTION: SEQ ID NO: 364:

Thr Leu Thr Ser Phe Leu Glu Leu Pro Leu Ala Pro Glu Pro Xaa
1               5                   10                  15

(2) INFORMATION FOR SEQ ID NO: 365:

(i) SEQUENCE CHARACTERISTICS:
(A) LENGTH: 34 amino acids
(B) TYPE: amino acid
(D) TOPOLOGY: linear
(xi) SEQUENCE DESCRIPTION: SEQ ID NO: 365:

Met His Arg Tyr Ile Thr Phe Phe Lys Cys Phe Arg Ser Val Ile Leu
1               5                   10                  15

Asp Leu Leu Phe Ile Leu Ser Pro Leu Ser Gln Gly Cys Phe Ile Leu
            20                  25                  30

Phe Xaa

(2) INFORMATION FOR SEQ ID NO: 366:

(i) SEQUENCE CHARACTERISTICS:
(A) LENGTH: 66 amino acids
(B) TYPE: amino acid
(D) TOPOLOGY: linear
(xi) SEQUENCE DESCRIPTION: SEQ ID NO: 366:

Met Phe Gly Phe Ile Phe Leu Leu Leu Ile Phe Cys Ile Xaa Leu Cys
1               5                   10                  15

Ser Arg Thr Leu Ser Thr Phe Ile Pro Lys Leu Val Gly Phe Leu Tyr
            20                  25                  30

Trp Lys Phe Ser Ile Asn Leu Ser Leu Leu Leu Thr Leu Ile Lys Lys
        35                  40                  45

Lys Lys Lys Lys Lys Lys Thr Pro Arg Gly Gly Pro Gly Xaa Gln Ser
    50                  55                  60

Pro Pro
65

(2) INFORMATION FOR SEQ ID NO: 367:

(i) SEQUENCE CHARACTERISTICS:
(A) LENGTH: 317 amino acids

(B) TYPE: amino acid
(D) TOPOLOGY: linear
(xi) SEQUENCE DESCRIPTION: SEQ ID NO: 367:

Met Pro Gly Leu Gly Arg Pro Arg Gln Ala Arg Trp Thr Leu Met Leu
1               5                   10                  15

Leu Leu Ser Thr Ala Met Tyr Gly Ala His Ala Pro Leu Leu Ala Leu
            20                  25                  30

Cys His Val Asp Gly Arg Val Pro Phe Arg Pro Ser Ser Ala Val Leu
        35                  40                  45

Leu Thr Glu Leu Thr Lys Leu Leu Leu Cys Ala Phe Ser Leu Leu Val
    50                  55                  60

Gly Trp Gln Ala Trp Pro Gln Gly Pro Pro Pro Trp Arg Gln Ala Ala
65                  70                  75                  80

Pro Phe Ala Leu Ser Ala Leu Leu Tyr Gly Ala Asn Asn Asn Leu Val
                85                  90                  95

Ile Tyr Leu Gln Arg Tyr Met Asp Pro Ser Thr Tyr Gln Val Leu Ser
            100                 105                 110

Asn Leu Lys Ile Gly Ser Thr Ala Val Leu Tyr Cys Leu Cys Leu Arg
        115                 120                 125

His Arg Leu Ser Val Arg Gln Gly Leu Ala Leu Leu Leu Leu Met Ala
    130                 135                 140

Ala Gly Ala Cys Tyr Ala Ala Gly Gly Leu Gln Val Pro Gly Asn Thr
145                 150                 155                 160

Leu Pro Ser Pro Pro Pro Ala Ala Ala Ala Ser Pro Met Pro Leu His
                165                 170                 175

Ile Thr Pro Leu Gly Leu Leu Leu Leu Ile Leu Tyr Cys Leu Ile Ser
            180                 185                 190

Gly Leu Ser Ser Val Tyr Thr Glu Leu Leu Met Lys Arg Gln Xaa Leu
        195                 200                 205

Pro Leu Ala Leu Gln Asn Leu Phe Leu Tyr Thr Phe Gly Val Leu Leu
    210                 215                 220

Asn Leu Gly Leu His Ala Gly Gly Gly Ser Gly Pro Gly Leu Leu Glu
225                 230                 235                 240

Gly Phe Ser Gly Trp Ala Ala Leu Val Val Leu Ser Gln Ala Leu Asn
                245                 250                 255

Gly Leu Leu Met Ser Ala Val Met Lys His Gly Ser Ser Ile Thr Arg
            260                 265                 270

Leu Phe Val Val Ser Cys Ser Leu Val Val Asn Ala Val Leu Ser Ala
        275                 280                 285

Val Leu Leu Arg Leu Gln Leu Thr Ala Ala Phe Phe Leu Ala Thr Leu
    290                 295                 300

Leu Ile Gly Leu Ala Met Arg Leu Tyr Tyr Gly Ser Arg
305                 310                 315

(2) INFORMATION FOR SEQ ID NO: 368:

(i) SEQUENCE CHARACTERISTICS:
(A) LENGTH: 31 amino acids
(B) TYPE: amino acid
(D) TOPOLOGY: linear
(xi) SEQUENCE DESCRIPTION: SEQ ID NO: 368:

Met Gly Glu Gln Pro His Phe Ser Leu Cys Val Leu Leu Ala Ala Val
1               5                   10                  15

Arg Glu Asp Xaa Asp Pro Xaa Val Phe Pro Cys Cys Phe Leu Xaa
            20                  25                  30

(2) INFORMATION FOR SEQ ID NO: 369:

(i) SEQUENCE CHARACTERISTICS:
(A) LENGTH: 43 amino acids
(B) TYPE: amino acid
(D) TOPOLOGY: linear
(xi) SEQUENCE DESCRIPTION: SEQ ID NO: 369:

Met Ser Phe Ile Ala Leu His Pro Leu Leu Pro Glu Ala Ala Leu Gly
1               5                   10                  15

Val Pro Gly Gln Ser Pro His Arg Pro Leu Trp Gln Thr Gln Cys Cys
            20                  25                  30

Val Ala Pro Pro Gln Pro Arg Ala Glu Phe Xaa
        35                  40

(2) INFORMATION FOR SEQ ID NO: 370:

(i) SEQUENCE CHARACTERISTICS:
(A) LENGTH: 255 amino acids
(B) TYPE: amino acid
(D) TOPOLOGY: linear
(xi) SEQUENCE DESCRIPTION: SEQ ID NO: 370:

Met Val Thr Ala Leu Thr Leu Leu Ala Phe Pro Leu Leu Leu Leu His
1               5                   10                  15

Ala Glu Arg Ile Ser Leu Val Phe Leu Leu Leu Phe Leu Gln Ser Phe
            20                  25                  30

Leu Leu Leu His Leu Leu Ala Ala Gly Ile Pro Val Thr Thr Pro Gly
        35                  40                  45

Pro Phe Thr Val Pro Trp Gln Ala Val Ser Ala Trp Ala Leu Met Ala
    50                  55                  60

Thr Gln Thr Phe Tyr Ser Thr Gly His Gln Pro Val Phe Pro Ala Ile
65                  70                  75                  80

His Trp His Ala Ala Phe Val Gly Phe Pro Glu Gly His Gly Ser Cys
                85                  90                  95

Thr Trp Leu Pro Ala Leu Leu Val Gly Ala Asn Thr Phe Ala Ser His
            100                 105                 110

Leu Leu Phe Ala Val Gly Cys Pro Leu Leu Leu Leu Trp Pro Phe Leu
        115                 120                 125

Cys Glu Ser Gln Gly Leu Arg Lys Arg Gln Gln Pro Pro Gly Asn Glu
    130                 135                 140

Ala Asp Ala Arg Val Arg Pro Glu Glu Glu Glu Glu Pro Leu Met Glu
145                 150                 155                 160

Met Arg Leu Arg Asp Ala Pro Gln His Phe Tyr Ala Ala Leu Leu Gln
                165                 170                 175

Leu Gly Leu Lys Tyr Leu Phe Ile Leu Gly Ile Gln Ile Leu Ala Cys
            180                 185                 190

Ala Leu Ala Ala Ser Ile Leu Arg Arg His Leu Met Val Trp Lys Val
        195                 200                 205

Phe Ala Pro Lys Phe Ile Phe Glu Ala Val Gly Phe Ile Val Ser Ser
    210                 215                 220

Val Gly Leu Leu Leu Gly Ile Ala Leu Val Met Arg Val Asp Gly Ala
225                 230                 235                 240

Val Ser Ser Trp Phe Arg Gln Leu Phe Leu Ala Gln Gln Arg Xaa
                245                 250                 255

(2) INFORMATION FOR SEQ ID NO: 371:

(i) SEQUENCE CHARACTERISTICS:
(A) LENGTH: 20 amino acids
(B) TYPE: amino acid
(D) TOPOLOGY: linear
(xi) SEQUENCE DESCRIPTION: SEQ ID NO: 371:

Met Xaa Gly Pro Trp Gly Glu Glu Ala Leu Ile Arg Leu Pro Thr Pro
1               5                   10                  15

Ser Gly Leu Xaa
            20

(2) INFORMATION FOR SEQ ID NO: 372:

(i) SEQUENCE CHARACTERISTICS:
(A) LENGTH: 64 amino acids
(B) TYPE: amino acid

(D) TOPOLOGY: linear
(xi) SEQUENCE DESCRIPTION: SEQ ID NO: 372:

Met Ala Thr Leu Glu Xaa Asn Gln Arg Glu Val Asp Arg Glu Ile Arg
1               5                   10                  15

Ser Leu Leu Leu Trp Phe Leu Leu Cys Glu Ile Val Ser Gly Trp Leu
            20                  25                  30

Cys Pro Glu Gly Pro Trp Phe Ser Gln Gly Cys Gln Ile Tyr Lys Asn
        35                  40                  45

Leu Ser Ser Ser Ser Ser Tyr Asn Leu Ser Phe Leu Leu Ser Leu Xaa
    50                  55                  60

(2) INFORMATION FOR SEQ ID NO: 373:

(i) SEQUENCE CHARACTERISTICS:
(A) LENGTH: 40 amino acids
(B) TYPE: amino acid
(D) TOPOLOGY: linear
(xi) SEQUENCE DESCRIPTION: SEQ ID NO: 373:

Met Ile His Ser Gly Cys Thr Ser Gln Cys Leu Glu Gly Phe Phe Leu
1               5                   10                  15

Ile Phe Leu Leu Asp Phe Asn Pro Val Leu Ala Leu Asp Leu Ile Gly
            20                  25                  30

Ile Met Arg Lys Ala Ser His Xaa
        35                  40

(2) INFORMATION FOR SEQ ID NO: 374:

(i) SEQUENCE CHARACTERISTICS:
(A) LENGTH: 35 amino acids
(B) TYPE: amino acid
(D) TOPOLOGY: linear
(xi) SEQUENCE DESCRIPTION: SEQ ID NO: 374:

Met Val Phe Ser Ala Arg Val Ser Leu Tyr Thr Arg Phe Lys Val Ile
1               5                   10                  15

Leu Leu Ser Leu Leu Ile Met Ile Leu His Val Cys Trp Val Trp Val
            20                  25                  30

Ile Leu Xaa
        35

(2) INFORMATION FOR SEQ ID NO: 375:

(i) SEQUENCE CHARACTERISTICS:
(A) LENGTH: 11 amino acids
(B) TYPE: amino acid
(D) TOPOLOGY: linear
(xi) SEQUENCE DESCRIPTION: SEQ ID NO: 375:

Gly Leu Leu Tyr Ile Met Tyr Cys Asn Ile Xaa
1               5                   10

(2) INFORMATION FOR SEQ ID NO: 376:

(i) SEQUENCE CHARACTERISTICS:
(A) LENGTH: 64 amino acids
(B) TYPE: amino acid
(D) TOPOLOGY: linear
(xi) SEQUENCE DESCRIPTION: SEQ ID NO: 376:

Met Asn Asn Gly Leu Leu Gln Gln Pro Ser Ala Leu Met Leu Leu Pro
1               5                   10                  15

Cys Arg Pro Val Leu Thr Ser Val Ala Leu Asn Ala Asn Phe Val Ser
            20                  25                  30

Trp Lys Ser Arg Thr Lys Tyr Thr Ile Thr Pro Val Lys Met Arg Lys
        35                  40                  45

Ser Gly Gly Arg Asp His Thr Gly Gly Asn Lys Asp Arg Gly Ile Xaa
    50                  55                  60

(2) INFORMATION FOR SEQ ID NO: 377:

(i) SEQUENCE CHARACTERISTICS:
(A) LENGTH: 19 amino acids
(B) TYPE: amino acid
(D) TOPOLOGY: linear
(xi) SEQUENCE DESCRIPTION: SEQ ID NO: 377:

Met Arg Lys Gln Arg Leu Val Pro Met Tyr Leu Gly Leu Ile Tyr Ile
1               5                   10                  15

Leu Leu Xaa

(2) INFORMATION FOR SEQ ID NO: 378:

(i) SEQUENCE CHARACTERISTICS:
(A) LENGTH: 5 amino acids
(B) TYPE: amino acid
(D) TOPOLOGY: linear
(xi) SEQUENCE DESCRIPTION: SEQ ID NO: 378:

Met Arg Gln His Xaa
1               5

(2) INFORMATION FOR SEQ ID NO: 379:

(i) SEQUENCE CHARACTERISTICS:
(A) LENGTH: 17 amino acids
(B) TYPE: amino acid
(D) TOPOLOGY: linear
(xi) SEQUENCE DESCRIPTION: SEQ ID NO: 379:

Leu Leu Leu Pro Val Leu Ala Ser Ser Val Pro Ser His Ser Ala Thr
1               5                   10                  15

Xaa

(2) INFORMATION FOR SEQ ID NO: 380:

(i) SEQUENCE CHARACTERISTICS:
(A) LENGTH: 84 amino acids
(B) TYPE: amino acid
(D) TOPOLOGY: linear
(xi) SEQUENCE DESCRIPTION: SEQ ID NO: 380:

Met Leu Pro Leu Leu Leu Phe Thr Tyr Leu Asn Ser Phe Leu His Gln
1               5                   10                  15

Arg Ile Pro Gln Ser Val Arg Ile Leu Gly Ser Leu Val Ala Ile Leu
            20                  25                  30

Leu Val Phe Leu Ile Thr Ala Ile Leu Val Lys Val Gln Leu Asp Ala
        35                  40                  45

Leu Pro Phe Phe Val Ile Thr Met Ile Lys Ile Val Leu Ile Asn Ser
    50                  55                  60

Phe Gly Ala Ile Leu Gln Gly Ser Leu Phe Gly Leu Ala Gly Leu Leu
65                  70                  75                  80

Pro Ala Ser Xaa

(2) INFORMATION FOR SEQ ID NO: 381:

(i) SEQUENCE CHARACTERISTICS:
(A) LENGTH: 21 amino acids
(B) TYPE: amino acid
(D) TOPOLOGY: linear
(xi) SEQUENCE DESCRIPTION: SEQ ID NO: 381:

Met Lys Leu Ser Leu Phe Leu Ile Leu Ser Asp Val Phe Tyr Leu Gly
1               5                   10                  15

Ser Pro Xaa Thr Xaa
            20

(2) INFORMATION FOR SEQ ID NO: 382:

        (i) SEQUENCE CHARACTERISTICS:
                (A) LENGTH: 29 amino acids
                (B) TYPE: amino acid
                (D) TOPOLOGY: linear
        (xi) SEQUENCE DESCRIPTION: SEQ ID NO: 382:

Met Gly Thr Arg Arg Lys Gly Val Ala Trp Leu Ser Leu Ala Pro Leu
  1               5                  10                  15

Ile Thr Gly Leu Ala Pro Ala His Ile Thr Ala Val Xaa
            20                  25

(2) INFORMATION FOR SEQ ID NO: 383:

        (i) SEQUENCE CHARACTERISTICS:
                (A) LENGTH: 34 amino acids
                (B) TYPE: amino acid
                (D) TOPOLOGY: linear
        (xi) SEQUENCE DESCRIPTION: SEQ ID NO: 383:

Met Lys Asp Leu Leu Gln Arg Asn Pro Trp Lys Asn Ser Leu Leu Leu
  1               5                  10                  15

Leu Gln Val Cys Gln Ala Phe Leu Val Cys Ser Leu Thr Gln Leu Ala
            20                  25                  30

Val Xaa

(2) INFORMATION FOR SEQ ID NO: 384:

        (i) SEQUENCE CHARACTERISTICS:
                (A) LENGTH: 47 amino acids
                (B) TYPE: amino acid
                (D) TOPOLOGY: linear
        (xi) SEQUENCE DESCRIPTION: SEQ ID NO: 384:

Met Ser Glu Ser His Lys Ile Trp Trp Cys Tyr Arg His Leu Ala Phe
  1               5                  10                  15

Pro Leu Leu Thr Leu Ile Leu Tyr Pro Ala Thr Leu Gly Arg Ser Val
            20                  25                  30

Phe Cys His Asp Cys Lys Phe Pro Glu Ala Ser Pro Ala Met Xaa
        35                  40                  45

(2) INFORMATION FOR SEQ ID NO: 385:

(i) SEQUENCE CHARACTERISTICS:
(A) LENGTH: 25 amino acids
(B) TYPE: amino acid
(D) TOPOLOGY: linear
(xi) SEQUENCE DESCRIPTION: SEQ ID NO: 385:

Met Leu Asn Arg Ile Met Val Ala Ser Phe Gly Ala Val Leu Val Gln
1               5                   10                  15

Val Cys Arg Gly Xaa Gly Gln Gly Xaa
            20                  25

(2) INFORMATION FOR SEQ ID NO: 386:

(i) SEQUENCE CHARACTERISTICS:
(A) LENGTH: 68 amino acids
(B) TYPE: amino acid
(D) TOPOLOGY: linear
(xi) SEQUENCE DESCRIPTION: SEQ ID NO: 386:

Met Gln Leu Leu Leu Leu Gly Leu Ile Arg Ser Gln Pro Ser Pro Pro
1               5                   10                  15

Pro Ser Leu Cys Leu Met Leu Cys Pro Cys Leu Pro Cys Leu Arg Tyr
            20                  25                  30

Ser Pro Phe Val Pro Gln His Pro Cys Pro Leu Pro Leu Asp Leu Cys
        35                  40                  45

Leu Ala Gly Cys Ser Ser Leu Ser Val Gln Asp Lys Cys Ser Trp Pro
    50                  55                  60

Tyr Pro Ile Xaa
65

(2) INFORMATION FOR SEQ ID NO: 387:

(i) SEQUENCE CHARACTERISTICS:
(A) LENGTH: 34 amino acids
(B) TYPE: amino acid
(D) TOPOLOGY: linear
(xi) SEQUENCE DESCRIPTION: SEQ ID NO: 387:

Lys Glu Phe Phe Val Phe Leu Phe Val Cys Leu Phe Trp Leu Leu Ser
1               5                   10                  15

Asn Thr Pro Leu Thr Phe Ile Ser Ile Ile Leu Gln Arg Lys Glu Thr
            20                  25                  30

Asn Xaa

(2) INFORMATION FOR SEQ ID NO: 388:

(i) SEQUENCE CHARACTERISTICS:
(A) LENGTH: 14 amino acids
(B) TYPE: amino acid
(D) TOPOLOGY: linear
(xi) SEQUENCE DESCRIPTION: SEQ ID NO: 388:

Ser Phe Leu Met Val Leu Val Ile Leu Ala Ala Ser Pro Xaa
1               5                   10

(2) INFORMATION FOR SEQ ID NO: 389:

(i) SEQUENCE CHARACTERISTICS:
(A) LENGTH:  amino acids
(B) TYPE: amino acid
(D) TOPOLOGY: linear
(xi) SEQUENCE DESCRIPTION: SEQ ID NO: 389:

1               5                   10

(2) INFORMATION FOR SEQ ID NO: 390:

(i) SEQUENCE CHARACTERISTICS:
(A) LENGTH: 154 amino acids
(B) TYPE: amino acid
(D) TOPOLOGY: linear
(xi) SEQUENCE DESCRIPTION: SEQ ID NO: 390:

Met Thr Lys Ala Arg Leu Phe Arg Leu Trp Leu Val Leu Gly Ser Val
1               5                   10                  15

Phe Met Ile Leu Leu Ile Ile Val Tyr Trp Asp Ser Ala Gly Ala Ala
            20                  25                  30

His Phe Tyr Leu His Thr Ser Phe Ser Arg Pro His Thr Gly Pro Pro
        35                  40                  45

Leu Pro Thr Pro Gly Pro Asp Arg Asp Arg Glu Leu Thr Ala Asp Ser
    50                  55                  60

Asp Val Asp Xaa Phe Leu Asp Xaa Phe Leu Ser Ala Gly Val Lys Gln
65                  70                  75                  80

Ser Asp Xaa Pro Arg Lys Glu Thr Glu Gln Pro Pro Ala Pro Gly Ser
                85                  90                  95

Met Glu Glu Ser Val Arg Xaa Tyr Asp Trp Ser Pro Arg Xaa Ala Arg
            100                 105                 110

Arg Thr Gln Thr Arg Ala Gly Ser Xaa Arg Xaa Gly Gly Xaa Cys Cys
        115                 120                 125

Gly Ala Ser Ala Pro Xaa Pro Ala Trp Pro Ser Pro Pro Arg Ser Ala
    130                 135                 140

His Ser Thr Thr Ser Pro Thr Arg Ser Xaa
145                 150

(2) INFORMATION FOR SEQ ID NO: 391:

        (i) SEQUENCE CHARACTERISTICS:
                (A) LENGTH: 9 amino acids
                (B) TYPE: amino acid
                (D) TOPOLOGY: linear
        (xi) SEQUENCE DESCRIPTION: SEQ ID NO: 391:

Met Val Leu Leu Gly Leu Leu Ser Xaa
  1               5

(2) INFORMATION FOR SEQ ID NO: 392:

        (i) SEQUENCE CHARACTERISTICS:
                (A) LENGTH: 61 amino acids
                (B) TYPE: amino acid
                (D) TOPOLOGY: linear
        (xi) SEQUENCE DESCRIPTION: SEQ ID NO: 392:

Met Cys Ile His Val Phe Met Xaa Val Leu Trp Val Leu Phe Leu Leu
  1               5                  10                  15

Asn Pro Leu Cys Thr Gly Leu Trp Pro Leu Xaa Asn Cys Phe Ser Val
             20                  25                  30

Leu Arg His Ala Asp Trp Val Leu Gly Ala Asp Tyr Lys Gly Glu Glu
         35                  40                  45

Leu Asn Arg His Gln Gly Pro Met Lys Pro Lys Asp Xaa
     50                  55                  60

(2) INFORMATION FOR SEQ ID NO: 393:

        (i) SEQUENCE CHARACTERISTICS:
                (A) LENGTH: 447 amino acids
                (B) TYPE: amino acid
                (D) TOPOLOGY: linear
        (xi) SEQUENCE DESCRIPTION: SEQ ID NO: 393:

Met Leu Leu Gly Leu Leu Met Ala Ala Cys Phe Thr Phe Cys Leu Ser
  1               5                  10                  15

His Gln Asn Leu Lys Glu Phe Ala Leu Thr Asn Pro Glu Lys Ser Ser
             20                  25                  30

Thr Lys Glu Thr Glu Arg Lys Glu Thr Lys Ala Glu Glu Glu Leu Asp
         35                  40                  45

Ala Glu Val Leu Glu Val Phe His Pro Thr His Glu Trp Gln Ala Leu
     50                  55                  60

Gln Pro Gly Gln Ala Val Pro Ala Gly Ser His Val Arg Leu Asn Leu
65                  70                  75                  80

Gln Thr Gly Glu Arg Glu Ala Lys Leu Gln Tyr Glu Asp Lys Phe Arg
                85                  90                  95

Asn Asn Leu Lys Gly Lys Arg Leu Asp Ile Asn Thr Asn Thr Tyr Thr
            100                 105                 110

Ser Gln Asp Leu Lys Ser Ala Leu Ala Lys Phe Lys Glu Gly Ala Glu
        115                 120                 125

Met Glu Ser Ser Lys Glu Asp Lys Ala Arg Gln Ala Glu Val Lys Arg
    130                 135                 140

Leu Phe Arg Pro Ile Glu Glu Leu Lys Lys Asp Phe Asp Glu Leu Asn
145                 150                 155                 160

Val Val Ile Glu Thr Asp Met Gln Ile Met Val Arg Leu Ile Asn Lys
                165                 170                 175

Phe Asn Ser Ser Ser Ser Ser Leu Glu Glu Lys Ile Ala Ala Leu Phe
            180                 185                 190

Asp Leu Glu Tyr Tyr Val His Gln Met Asp Asn Ala Gln Asp Leu Leu
        195                 200                 205

Ser Phe Gly Gly Leu Gln Val Val Ile Asn Gly Leu Asn Ser Thr Glu
    210                 215                 220

Pro Leu Val Lys Glu Tyr Ala Ala Phe Val Leu Gly Ala Ala Phe Ser
225                 230                 235                 240

Ser Asn Pro Lys Val Gln Val Glu Ala Ile Glu Gly Gly Ala Leu Gln
                245                 250                 255

Lys Leu Leu Val Ile Leu Ala Thr Glu Gln Pro Leu Thr Ala Lys Lys
            260                 265                 270

Lys Val Leu Phe Ala Leu Cys Ser Leu Leu Arg His Phe Pro Tyr Ala
        275                 280                 285

Gln Arg Gln Phe Leu Lys Leu Gly Gly Leu Gln Val Leu Arg Thr Leu
    290                 295                 300

Val Gln Glu Lys Gly Thr Glu Val Leu Ala Val Arg Val Val Thr Leu
305                 310                 315                 320

Leu Tyr Asp Leu Val Thr Glu Lys Met Phe Ala Glu Glu Glu Ala Glu
                325                 330                 335

Leu Thr Gln Glu Met Ser Pro Glu Lys Leu Gln Gln Tyr Arg Gln Val
            340                 345                 350

His Leu Leu Pro Gly Leu Trp Glu Gln Gly Trp Cys Glu Ile Thr Ala
        355                 360                 365

His Leu Leu Ala Leu Pro Glu His Asp Ala Arg Glu Lys Val Leu Gln
    370                 375                 380

Thr Leu Gly Val Leu Leu Thr Thr Cys Arg Asp Arg Tyr Arg Gln Asp
385                 390                 395                 400

Pro Gln Leu Gly Arg Thr Leu Ala Ser Leu Gln Ala Glu Tyr Gln Val
                405                 410                 415

Leu Ala Ser Leu Glu Leu Gln Asp Gly Glu Asp Glu Gly Tyr Phe Gln
            420                 425                 430

Glu Leu Leu Gly Ser Val Asn Ser Leu Leu Lys Glu Leu Arg Xaa
        435                 440                 445

(2) INFORMATION FOR SEQ ID NO: 394:

(i) SEQUENCE CHARACTERISTICS:
(A) LENGTH: 24 amino acids
(B) TYPE: amino acid
(D) TOPOLOGY: linear
(xi) SEQUENCE DESCRIPTION: SEQ ID NO: 394:

Met Val Ile Ser Tyr Val Thr Phe Thr Pro Val Ser Ala Asp Cys Phe
  1               5                  10                  15

Phe Asn Val Leu Val Cys Phe Xaa
            20

(2) INFORMATION FOR SEQ ID NO: 395:

(i) SEQUENCE CHARACTERISTICS:
(A) LENGTH: 24 amino acids
(B) TYPE: amino acid
(D) TOPOLOGY: linear
(xi) SEQUENCE DESCRIPTION: SEQ ID NO: 395:

Glu Leu Leu Phe Leu Leu Ile Ile Ile Leu Gly Glu Ser Leu Ser Asp
  1               5                  10                  15

Val Ile Leu Leu Ile Cys Phe Xaa
            20

(2) INFORMATION FOR SEQ ID NO: 396:

(i) SEQUENCE CHARACTERISTICS:
(A) LENGTH: 35 amino acids
(B) TYPE: amino acid
(D) TOPOLOGY: linear
(xi) SEQUENCE DESCRIPTION: SEQ ID NO: 396:

Met Phe Tyr Trp Gly Gly Leu Ser Phe Tyr Phe Leu Leu Ser Ser Gly
  1               5                  10                  15

Val Gly Phe Tyr Cys Phe Leu Phe Gly Phe Gly Met Glu Ile Trp Ile
            20                  25                  30

Ala Ala Xaa
        35

(2) INFORMATION FOR SEQ ID NO: 397:

(i) SEQUENCE CHARACTERISTICS:
(A) LENGTH: 3 amino acids
(B) TYPE: amino acid
(D) TOPOLOGY: linear
(xi) SEQUENCE DESCRIPTION: SEQ ID NO: 397:

Gly Arg Xaa
  1

(2) INFORMATION FOR SEQ ID NO: 398:

(i) SEQUENCE CHARACTERISTICS:
(A) LENGTH: 25 amino acids
(B) TYPE: amino acid
(D) TOPOLOGY: linear
(xi) SEQUENCE DESCRIPTION: SEQ ID NO: 398:

Met Lys Leu Ser Leu Leu Ile Leu Thr Leu Met Gln Arg Tyr Phe Arg
  1               5                  10                  15

Thr Ile Thr Asn Ser Leu Cys Lys Xaa
            20                  25

(2) INFORMATION FOR SEQ ID NO: 399:

(i) SEQUENCE CHARACTERISTICS:
(A) LENGTH: 79 amino acids
(B) TYPE: amino acid
(D) TOPOLOGY: linear
(xi) SEQUENCE DESCRIPTION: SEQ ID NO: 399:

Met Pro Ala Val Ser Gly Pro Gly Pro Leu Phe Cys Leu Leu Leu Leu
  1               5                  10                  15

Leu Leu Asp Pro His Ser Pro Glu Thr Gly Cys Pro Pro Leu Arg Arg
            20                  25                  30

Phe Glu Tyr Lys Leu Ser Phe Lys Gly Pro Arg Leu Ala Leu Pro Gly
        35                  40                  45

Ala Gly Ile Pro Phe Trp Ser His His Gly Gly Glu Gly Gln Gly Trp
    50                  55                  60

Gly Pro Leu Cys Pro Gly Ser Leu Lys Val Leu Glu Gly Leu Xaa
 65                  70                  75

(2) INFORMATION FOR SEQ ID NO: 400:

(i) SEQUENCE CHARACTERISTICS:
(A) LENGTH: 21 amino acids
(B) TYPE: amino acid
(D) TOPOLOGY: linear
(xi) SEQUENCE DESCRIPTION: SEQ ID NO: 400:

Met Lys Val Phe Leu Ser Met Pro Phe Leu Val Leu Phe Gln Ser Leu
1               5                   10                  15

Ile Gln Glu Asp Xaa
            20

(2) INFORMATION FOR SEQ ID NO: 401:

(i) SEQUENCE CHARACTERISTICS:
(A) LENGTH: 257 amino acids
(B) TYPE: amino acid
(D) TOPOLOGY: linear
(xi) SEQUENCE DESCRIPTION: SEQ ID NO: 401:

Met Ala Ala Leu Thr Ser His Leu Gln Asn Gln Ser Asn Asn Ser Asn
1               5                   10                  15

Trp Asn Leu Arg Thr Arg Ser Lys Cys Lys Lys Asp Val Phe Met Pro
            20                  25                  30

Pro Ser Ser Ser Ser Glu Leu Gln Glu Ser Arg Gly Leu Ser Asn Phe
        35                  40                  45

Thr Ser Thr His Leu Leu Leu Lys Glu Asp Glu Gly Val Asp Asp Val
    50                  55                  60

Asn Phe Arg Lys Val Arg Lys Pro Lys Gly Lys Val Thr Ile Leu Lys
65                  70                  75                  80

Gly Ile Pro Ile Lys Lys Thr Lys Lys Gly Cys Arg Lys Ser Cys Ser
                85                  90                  95

Gly Phe Val Xaa Ser Asp Ser Lys Arg Glu Ser Val Cys Asn Lys Ala
            100                 105                 110

Asp Ala Glu Ser Glu Pro Val Ala Gln Lys Ser Gln Leu Asp Arg Thr
        115                 120                 125

Val Cys Ile Ser Asp Ala Gly Ala Cys Gly Glu Thr Leu Ser Val Thr
    130                 135                 140

Ser Glu Glu Asn Ser Leu Val Lys Lys Lys Glu Arg Ser Leu Ser Ser
145                 150                 155                 160

Gly Ser Asn Phe Cys Ser Glu Gln Lys Thr Ser Gly Ile Ile Asn Lys
                165                 170                 175

Phe Cys Ser Ala Lys Asp Ser Glu His Asn Glu Lys Tyr Glu Asp Thr
            180                 185                 190

Phe Leu Glu Ser Glu Glu Ile Gly Thr Lys Val Glu Val Val Glu Arg

        195                 200                 205

Lys Glu His Leu His Thr Asp Ile Leu Lys Arg Gly Ser Glu Met Asp
    210                 215                 220

Asn Asn Cys Ser Pro Thr Arg Lys Asp Phe Thr Glu Asp Thr Ile Pro
225                 230                 235                 240

Arg Asn Thr Asp Arg Lys Lys Glu Asn Lys Pro Val Phe Phe Gln Gln
                245                 250                 255

Ile

(2) INFORMATION FOR SEQ ID NO: 402:

(i) SEQUENCE CHARACTERISTICS:
(A) LENGTH: 424 amino acids
(B) TYPE: amino acid
(D) TOPOLOGY: linear
(xi) SEQUENCE DESCRIPTION: SEQ ID NO: 402:

Met Glu Lys Gln Cys Cys Ser His Pro Val Ile Cys Ser Leu Ser Thr
1               5                   10                  15

Met Tyr Thr Phe Leu Leu Gly Ala Ile Phe Ile Ala Leu Ser Ser Ser
            20                  25                  30

Arg Ile Leu Leu Val Lys Tyr Ser Ala Asn Glu Glu Asn Lys Tyr Asp
        35                  40                  45

Tyr Leu Pro Thr Thr Val Asn Val Cys Ser Glu Leu Val Lys Leu Val
    50                  55                  60

Phe Cys Val Leu Val Ser Phe Cys Val Ile Lys Lys Asp His Gln Ser
65                  70                  75                  80

Arg Asn Leu Lys Tyr Ala Ser Trp Lys Glu Phe Ser Asp Phe Met Lys
                85                  90                  95

Trp Ser Ile Pro Ala Phe Leu Tyr Phe Leu Asp Asn Leu Ile Val Phe
            100                 105                 110

Tyr Val Leu Ser Tyr Leu Gln Pro Ala Met Ala Val Ile Phe Ser Asn
        115                 120                 125

Phe Ser Ile Ile Thr Thr Ala Leu Leu Phe Arg Ile Val Leu Lys Xaa
    130                 135                 140

Arg Leu Asn Trp Ile Gln Trp Ala Ser Leu Leu Thr Leu Phe Leu Ser
145                 150                 155                 160

Ile Val Ala Leu Thr Ala Gly Thr Lys Thr Leu Gln His Asn Leu Ala
                165                 170                 175

Gly Arg Gly Phe His His Asp Ala Phe Phe Ser Pro Ser Asn Ser Cys
            180                 185                 190

Leu Leu Phe Arg Asn Glu Cys Pro Arg Lys Asp Asn Cys Thr Ala Lys
        195                 200                 205

Glu Trp Thr Phe Pro Glu Ala Lys Trp Asn Thr Thr Ala Arg Val Phe
    210                 215                 220

Ser His Ile Arg Leu Gly Met Gly His Val Leu Ile Ile Val Gln Cys
225                 230                 235                 240

Phe Ile Ser Ser Met Ala Asn Ile Tyr Asn Glu Lys Ile Leu Lys Glu
                245                 250                 255

Gly Asn Gln Leu Thr Glu Xaa Ile Phe Ile Gln Asn Ser Lys Leu Tyr
            260                 265                 270

Phe Phe Gly Ile Leu Phe Asn Gly Leu Thr Leu Gly Leu Gln Arg Ser
        275                 280                 285

Asn Arg Asp Gln Ile Lys Asn Cys Gly Phe Phe Tyr Gly His Ser Ala
    290                 295                 300

Phe Ser Val Ala Leu Ile Phe Val Thr Ala Phe Gln Gly Leu Ser Val
305                 310                 315                 320

Ala Phe Ile Leu Lys Phe Leu Asp Asn Met Phe His Val Leu Met Ala
                325                 330                 335

Gln Val Thr Thr Val Ile Ile Thr Thr Val Ser Val Leu Val Phe Asp
            340                 345                 350

Phe Arg Pro Ser Leu Glu Phe Phe Leu Glu Ala Pro Ser Val Leu Leu
        355                 360                 365

Ser Ile Phe Ile Tyr Asn Ala Ser Lys Pro Gln Val Pro Glu Tyr Ala
    370                 375                 380

Pro Arg Gln Glu Arg Ile Arg Asp Leu Ser Gly Asn Leu Trp Glu Arg
385                 390                 395                 400

Ser Ser Gly Asp Gly Glu Glu Leu Glu Arg Leu Thr Lys Pro Lys Ser
                405                 410                 415

Asp Glu Ser Asp Glu Asp Thr Phe
            420

(2) INFORMATION FOR SEQ ID NO: 403:

(i) SEQUENCE CHARACTERISTICS:
(A) LENGTH: 33 amino acids
(B) TYPE: amino acid
(D) TOPOLOGY: linear
(xi) SEQUENCE DESCRIPTION: SEQ ID NO: 403:

Met Trp Gly Gln Gly Ser Gln Lys Ser His Phe Ser Asp Leu Val Phe
1               5                   10                  15

Gly Val Arg Glu Leu Cys Ala Gln Pro Ser Asp Pro Gly Ser Pro His
            20                  25                  30

Xaa

(2) INFORMATION FOR SEQ ID NO: 404:

(i) SEQUENCE CHARACTERISTICS:
(A) LENGTH: 80 amino acids
(B) TYPE: amino acid
(D) TOPOLOGY: linear
(xi) SEQUENCE DESCRIPTION: SEQ ID NO: 404:

Met Val Gln His Ile Gln Pro Ala Ala Leu Ser Leu Leu Ala Gln Trp
1               5                   10                  15

Ser Thr Leu Val Gln Glu Leu Glu Ala Ala Leu Gln Leu Ala Phe Tyr
            20                  25                  30

Pro Asp Ala Val Glu Glu Trp Leu Glu Glu Asn Val His Pro Ser Leu
        35                  40                  45

Gln Arg Leu Gln Xaa Leu Leu Gln Asp Leu Ser Glu Val Ser Ala Pro
    50                  55                  60

Pro Leu Pro Pro Thr Ser Pro Gly Arg Asp Val Ala Gln Asp Pro Xaa
65                  70                  75                  80

(2) INFORMATION FOR SEQ ID NO: 405:

(i) SEQUENCE CHARACTERISTICS:
(A) LENGTH: 95 amino acids
(B) TYPE: amino acid
(D) TOPOLOGY: linear
(xi) SEQUENCE DESCRIPTION: SEQ ID NO: 405:

Met Leu Asn Gln Gly Tyr Ile Arg Lys Ile Ile Leu Ile Ile Ile Leu
1               5                   10                  15

Gly Ser Phe Ser Ser Pro Lys Lys Ala Ile Leu Met Gly Phe Gln Asn
            20                  25                  30

Gln Lys Lys Ala Leu Asn Glu Glu Gln Thr Thr Gly Val Pro Met Ser
        35                  40                  45

Ile Ser Gly Lys Leu Arg Pro Ser Arg Ser Leu Asp Phe Val Gln Pro
    50                  55                  60

Pro Arg Phe Gln Ser Gln Gln Pro Ser Ala Val Val Asp Arg Arg Gly
65                  70                  75                  80

Phe Xaa Xaa Lys Ala Ala Arg Gly Gln Glu Phe Ser Glu Ser Xaa
                85                  90                  95

(2) INFORMATION FOR SEQ ID NO: 406:

(i) SEQUENCE CHARACTERISTICS:
(A) LENGTH: 257 amino acids
(B) TYPE: amino acid
(D) TOPOLOGY: linear
(xi) SEQUENCE DESCRIPTION: SEQ ID NO: 406:

Met Arg Gly Pro Ala Gln Ala Lys Leu Leu Pro Gly Ser Ala Ile Gln
1               5                   10                  15

Ala Leu Val Gly Leu Ala Arg Pro Leu Val Leu Ala Leu Leu Leu Val
            20                  25                  30

Ser Ala Ala Leu Ser Ser Val Val Ser Arg Thr Asp Ser Pro Ser Pro
        35                  40                  45

Thr Val Leu Asn Ser His Ile Ser Thr Pro Asn Val Asn Ala Leu Thr
    50                  55                  60

His Glu Asn Gln Thr Lys Pro Ser Ile Ser Gln Ile Ser Thr Thr Leu
65                  70                  75                  80

Pro Pro Thr Thr Ser Thr Lys Lys Ser Gly Gly Ala Ser Val Val Pro
                85                  90                  95

His Pro Ser Pro Thr Pro Leu Ser Gln Glu Glu Ala Asp Asn Asn Glu
            100                 105                 110

Asp Pro Ser Ile Glu Glu Glu Asp Leu Leu Met Leu Asn Ser Ser Pro
        115                 120                 125

Ser Thr Ala Lys Asp Thr Leu Asp Asn Gly Asp Tyr Gly Glu Pro Asp
    130                 135                 140

Tyr Asp Trp Thr Thr Gly Pro Arg Asp Asp Asp Glu Ser Asp Asp Thr
145                 150                 155                 160

Leu Glu Glu Asn Arg Gly Tyr Met Glu Ile Glu Gln Ser Val Lys Ser
                165                 170                 175

Phe Lys Met Pro Ser Ser Asn Ile Glu Glu Glu Asp Ser His Phe Phe
            180                 185                 190

Phe His Leu Ile Ile Phe Ala Phe Cys Ile Ala Val Val Tyr Ile Thr
        195                 200                 205

Tyr His Asn Lys Arg Lys Ile Phe Leu Leu Val Gln Ser Arg Lys Trp
    210                 215                 220

Arg Asp Gly Leu Cys Ser Lys Thr Val Glu Tyr His Arg Leu Asp Gln
225                 230                 235                 240

Asn Val Asn Glu Ala Met Pro Ser Leu Lys Ile Thr Asn Asp Tyr Ile
                245                 250                 255

Phe

(2) INFORMATION FOR SEQ ID NO: 407:

(i) SEQUENCE CHARACTERISTICS:
(A) LENGTH: 623 amino acids
(B) TYPE: amino acid
(D) TOPOLOGY: linear
(xi) SEQUENCE DESCRIPTION: SEQ ID NO: 407:

Met Phe Met Arg Ile Ala Lys Ala Tyr Ala Ala Leu Thr Asp Glu Glu
1               5                   10                  15

Ser Arg Lys Asn Trp Glu Glu Phe Gly Asn Pro Asp Gly Pro Gln Ala
            20                  25                  30

Thr Ser Phe Gly Ile Ala Leu Pro Ala Trp Ile Val Asp Gln Lys Asn
        35                  40                  45

Ser Ile Leu Val Leu Leu Val Tyr Gly Leu Ala Phe Met Val Ile Leu
    50                  55                  60

Pro Val Val Val Gly Ser Trp Trp Tyr Arg Ser Ile Arg Tyr Ser Gly
65                  70                  75                  80

Asp Gln Ile Leu Ile Arg Thr Thr Gln Ile Tyr Thr Tyr Phe Val Tyr
                85                  90                  95

Lys Thr Arg Asn Met Asp Met Lys Arg Leu Ile Met Val Leu Xaa Gly
            100                 105                 110

Ala Ser Glu Phe Asp Pro Gln Tyr Asn Lys Asp Ala Thr Ser Arg Pro
        115                 120                 125

Thr Asp Asn Ile Leu Ile Pro Gln Leu Ile Arg Glu Ile Gly Ser Ile
    130                 135                 140

Asn Leu Lys Lys Asn Glu Pro Pro Leu Thr Cys Pro Tyr Ser Leu Lys
145                 150                 155                 160

Ala Arg Val Leu Leu Leu Ser His Leu Ala Arg Met Lys Ile Pro Glu
                165                 170                 175

Thr Leu Glu Glu Asp Gln Gln Phe Met Leu Lys Lys Cys Pro Ala Leu
            180                 185                 190

Leu Gln Glu Met Val Asn Val Ile Cys Gln Leu Ile Val Met Ala Arg
        195                 200                 205

Asn Arg Glu Glu Arg Glu Phe Arg Ala Pro Thr Leu Ala Ser Leu Glu
    210                 215                 220

Asn Cys Met Lys Leu Ser Gln Met Ala Val Gln Gly Leu Gln Gln Phe
225                 230                 235                 240

Lys Ser Pro Leu Leu Gln Leu Pro His Ile Glu Glu Asp Asn Leu Arg
                245                 250                 255

Arg Val Ser Asn His Lys Lys Tyr Lys Ile Lys Thr Ile Gln Asp Leu

            260                 265                 270

Val Ser Leu Lys Glu Ser Asp Arg His Thr Leu Leu His Phe Leu Glu
        275                 280                 285

Asp Glu Lys Tyr Glu Glu Val Met Ala Val Leu Gly Ser Phe Pro Tyr
    290                 295                 300

Val Thr Met Asp Ile Lys Ser Gln Val Leu Asp Asp Glu Asp Ser Asn
305                 310                 315                 320

Asn Ile Thr Val Gly Ser Leu Val Thr Val Leu Val Lys Leu Thr Arg
                325                 330                 335

Gln Thr Met Ala Glu Val Phe Glu Lys Glu Gln Ser Ile Cys Ala Ala
            340                 345                 350

Glu Glu Gln Pro Ala Glu Asp Gly Gln Gly Glu Thr Asn Lys Asn Arg
        355                 360                 365

Thr Lys Gly Gly Trp Gln Gln Lys Ser Lys Gly Pro Lys Lys Thr Ala
    370                 375                 380

Lys Ser Lys Lys Lys Lys Pro Leu Lys Lys Lys Pro Thr Pro Val Leu
385                 390                 395                 400

Leu Pro Gln Ser Lys Gln Gln Lys Gln Lys Gln Ala Asn Gly Val Val
                405                 410                 415

Gly Asn Glu Ala Ala Val Lys Glu Asp Glu Glu Glu Val Ser Asp Lys
            420                 425                 430

Gly Ser Asp Ser Glu Glu Glu Glu Thr Asn Arg Asp Ser Gln Ser Glu
        435                 440                 445

Lys Asp Asp Gly Ser Asp Arg Asp Ser Asp Arg Glu Gln Asp Glu Lys
    450                 455                 460

Gln Asn Lys Asp Asp Glu Ala Glu Trp Gln Glu Leu Gln Gln Ser Ile
465                 470                 475                 480

Gln Arg Lys Glu Arg Ala Leu Leu Glu Thr Lys Ser Lys Ile Thr His
                485                 490                 495

Pro Val Tyr Ser Leu Tyr Phe Pro Glu Glu Lys Gln Glu Trp Trp Trp
            500                 505                 510

Leu Tyr Ile Ala Asp Arg Lys Glu Gln Thr Leu Ile Ser Met Pro Tyr
        515                 520                 525

His Val Cys Thr Leu Lys Asp Thr Glu Glu Val Glu Leu Lys Phe Pro
    530                 535                 540

Ala Pro Gly Lys Pro Gly Asn Tyr Gln Tyr Thr Val Phe Leu Arg Ser
545                 550                 555                 560

Asp Ser Tyr Met Gly Leu Asp Gln Ile Lys Pro Leu Glu Val Xaa Lys
                565                 570                 575

Phe Met Arg Leu Lys Pro Val Pro Glu Asn His Pro Gln Trp Asp Thr

580                    585                    590

Ala Ile Glu Gly Asp Glu Asp Gln Glu Asp Ser Glu Gly Phe Glu Asp
        595                    600                    605

Ser Phe Glu Gly Gly Arg Gly Arg Glu Glu Gly Arg Trp Trp Thr
    610                    615                    620

(2) INFORMATION FOR SEQ ID NO: 408:

        (i) SEQUENCE CHARACTERISTICS:
                (A) LENGTH: 190 amino acids
                (B) TYPE: amino acid
                (D) TOPOLOGY: linear
        (xi) SEQUENCE DESCRIPTION: SEQ ID NO: 408:

Met Lys Ala Ser Gln Cys Cys Cys Cys Leu Ser His Leu Leu Ala Ser
  1               5                  10                  15

Val Leu Leu Leu Leu Leu Leu Pro Glu Leu Ser Gly Xaa Leu Xaa Val
             20                  25                  30

Leu Leu Gln Ala Ala Glu Ala Ala Pro Gly Leu Gly Pro Pro Asp Pro
         35                  40                  45

Arg Pro Arg Thr Leu Pro Pro Leu Pro Pro Gly Pro Thr Pro Ala Gln
     50                  55                  60

Gln Pro Gly Arg Gly Leu Ala Glu Ala Ala Gly Pro Arg Gly Ser Glu
 65                  70                  75                  80

Gly Gly Asn Gly Ser Asn Pro Val Ala Gly Leu Glu Thr Asp Asp His
                 85                  90                  95

Gly Gly Lys Ala Gly Glu Gly Ser Val Gly Gly Gly Leu Ala Val Ser
            100                 105                 110

Pro Asn Pro Gly Asp Lys Pro Met Thr Gln Arg Ala Leu Thr Val Leu
        115                 120                 125

Met Val Val Ser Gly Ala Val Leu Val Tyr Phe Val Val Arg Thr Val
    130                 135                 140

Arg Met Arg Arg Arg Asn Arg Lys Thr Arg Arg Tyr Gly Val Leu Asp
145                 150                 155                 160

Thr Asn Ile Glu Asn Met Glu Leu Thr Pro Leu Glu Gln Asp Asp Glu
                165                 170                 175

Asp Asp Asp Asn Thr Leu Phe Asp Ala Asn His Pro Arg Arg
            180                 185                 190

(2) INFORMATION FOR SEQ ID NO: 409:

        (i) SEQUENCE CHARACTERISTICS:
                (A) LENGTH: 179 amino acids

(B) TYPE: amino acid
(D) TOPOLOGY: linear
(xi) SEQUENCE DESCRIPTION: SEQ ID NO: 409:

Met Ser Pro Ser Gly Arg Leu Cys Leu Leu Thr Ile Val Gly Leu Ile
1               5                   10                  15

Leu Pro Thr Arg Gly Gln Thr Leu Lys Asp Thr Thr Ser Ser Ser Ser
            20                  25                  30

Ala Asp Ser Thr Ile Met Asp Ile Gln Val Pro Thr Arg Ala Pro Asp
        35                  40                  45

Ala Val Tyr Thr Glu Leu Gln Pro Thr Ser Pro Thr Pro Thr Trp Pro
    50                  55                  60

Ala Asp Glu Thr Pro Gln Pro Gln Thr Gln Thr Gln Gln Leu Glu Gly
65                  70                  75                  80

Thr Asp Gly Pro Leu Val Thr Asp Pro Glu Thr His Lys Ser Thr Lys
                85                  90                  95

Ala Ala His Pro Thr Asp Asp Thr Thr Thr Leu Ser Glu Arg Pro Ser
            100                 105                 110

Pro Ser Thr Asp Val Gln Thr Asp Pro Gln Thr Leu Lys Pro Ser Gly
        115                 120                 125

Phe His Glu Asp Asp Pro Phe Phe Tyr Asp Glu His Thr Leu Arg Lys
    130                 135                 140

Arg Gly Leu Leu Val Ala Ala Val Leu Phe Ile Thr Gly Ile Ile Ile
145                 150                 155                 160

Leu Thr Ser Gly Lys Cys Arg Gln Leu Ser Arg Leu Cys Arg Asn His
                165                 170                 175

Cys Arg Xaa

(2) INFORMATION FOR SEQ ID NO: 410:

(i) SEQUENCE CHARACTERISTICS:
(A) LENGTH: 14 amino acids
(B) TYPE: amino acid
(D) TOPOLOGY: linear
(xi) SEQUENCE DESCRIPTION: SEQ ID NO: 410:

Met Phe Lys Cys Leu Gln Thr Thr Phe Leu Phe Ile Xaa Xaa
1               5                   10

(2) INFORMATION FOR SEQ ID NO: 411:

(i) SEQUENCE CHARACTERISTICS:
(A) LENGTH: 232 amino acids
(B) TYPE: amino acid

(D) TOPOLOGY: linear
(xi) SEQUENCE DESCRIPTION: SEQ ID NO: 411:

Met Leu Ala Gly Lys Leu Ile Pro Val His Gln Val Arg Gly Leu Lys
1               5                   10                  15

Glu Lys Ile Val Arg Ser Phe Glu Val Ser Pro Asp Gly Ser Phe Leu
            20                  25                  30

Leu Ile Asn Gly Ile Ala Gly Tyr Leu His Leu Leu Ala Met Lys Thr
        35                  40                  45

Lys Glu Leu Ile Gly Ser Met Lys Ile Asn Gly Arg Val Ala Ala Ser
    50                  55                  60

Thr Phe Ser Ser Asp Ser Lys Lys Val Tyr Ala Ser Ser Gly Asp Gly
65                  70                  75                  80

Glu Val Tyr Val Trp Asp Val Asn Ser Arg Lys Cys Leu Asn Arg Phe
                85                  90                  95

Val Asp Glu Gly Ser Leu Tyr Gly Leu Ser Ile Ala Thr Ser Arg Asn
            100                 105                 110

Gly Gln Tyr Val Ala Cys Gly Ser Asn Cys Gly Val Val Asn Ile Tyr
        115                 120                 125

Asn Gln Asp Ser Cys Leu Gln Glu Thr Asn Pro Lys Pro Ile Lys Ala
    130                 135                 140

Ile Met Asn Leu Val Thr Gly Val Thr Ser Leu Thr Phe Asn Pro Thr
145                 150                 155                 160

Thr Glu Ile Leu Ala Ile Ala Ser Glu Lys Met Lys Glu Ala Val Arg
                165                 170                 175

Leu Val His Leu Pro Ser Cys Thr Val Phe Ser Asn Phe Pro Val Ile
            180                 185                 190

Lys Asn Lys Asn Ile Ser His Val His Thr Met Asp Phe Ser Pro Arg
        195                 200                 205

Ser Gly Tyr Phe Ala Leu Gly Asn Glu Lys Gly Lys Ala Leu Met Tyr
    210                 215                 220

Arg Leu His His Tyr Ser Asp Phe
225                 230

(2) INFORMATION FOR SEQ ID NO: 412:

(i) SEQUENCE CHARACTERISTICS:
(A) LENGTH: 54 amino acids
(B) TYPE: amino acid
(D) TOPOLOGY: linear
(xi) SEQUENCE DESCRIPTION: SEQ ID NO: 412:

Ile Leu Leu Cys Ser Trp Pro Thr Gly Leu Val Gly Gly Arg Asp Pro
1               5                   10                  15

Gly Ser Ser Arg Gly Ser Ser Ala Ser Leu Thr Pro Ser Pro Gly Arg
            20                  25                  30

Gln Pro Cys Ser Arg Arg Arg Gly Tyr Ser Val Gly Arg Arg Ser Ser
        35                  40                  45

Pro Pro Asp Gly Ser Xaa
    50

(2) INFORMATION FOR SEQ ID NO: 413:

        (i) SEQUENCE CHARACTERISTICS:
                (A) LENGTH: 33 amino acids
                (B) TYPE: amino acid
                (D) TOPOLOGY: linear
        (xi) SEQUENCE DESCRIPTION: SEQ ID NO: 413:

Met Ser Leu Gln Ser Asn Ala Trp Ser Lys Xaa Leu Phe Ile Val Phe
  1               5                  10                  15

Leu Phe Leu Arg Val Leu Phe Lys Thr Gly Val Ser Ser Glu Glu Ser
            20                  25                  30

Xaa

(2) INFORMATION FOR SEQ ID NO: 414:

        (i) SEQUENCE CHARACTERISTICS:
                (A) LENGTH: 219 amino acids
                (B) TYPE: amino acid
                (D) TOPOLOGY: linear
        (xi) SEQUENCE DESCRIPTION: SEQ ID NO: 414:

Met Ala Val Val Leu Leu Ala Asn Leu Ala Gln Gly Asp Ser Leu Ala
  1               5                  10                  15

Ala Arg Ala Ile Ala Val Gln Lys Gly Ser Ile Gly Asn Leu Leu Gly
            20                  25                  30

Phe Leu Glu Asp Ser Leu Ala Ala Thr Gln Phe Gln Gln Ser Gln Ala
        35                  40                  45

Ser Leu Leu His Met Gln Asn Pro Pro Phe Glu Pro Xaa Ser Val Asp
    50                  55                  60

Met Met Arg Arg Ala Ala Arg Ala Leu Leu Ala Leu Ala Lys Val Asp
 65                  70                  75                  80

Glu Asn His Ser Glu Phe Thr Leu Tyr Glu Ser Arg Leu Leu Asp Ile
                 85                  90                  95

Ser Val Ser Pro Leu Met Asn Ser Xaa Val Ser Gln Val Ile Cys Asp
            100                 105                 110

Val Leu Phe Leu Xaa Trp Pro Val Met Thr Ala Val Gly His Leu Pro
        115                 120                 125

Pro Pro Cys Val Cys Ala Cys Val Glu Asn Leu Glu Thr Asp Cys Cys
    130                 135                 140

Pro Leu Phe Met Gln Asn His Leu Arg Ile Gln Phe Thr Leu Cys Cys
145                 150                 155                 160

Pro Ala Ser Pro Leu Gly Lys Ser Leu Ser Cys Phe Ser Leu Leu Leu
                165                 170                 175

Pro Pro Pro Leu Pro Pro Ser Pro His Ala Phe Leu Phe Leu Val Leu
            180                 185                 190

Thr Leu Leu Pro Ser Gly Pro Tyr Pro Thr Leu Phe Glu Lys Thr Lys
        195                 200                 205

Leu Cys Leu His Arg Arg Leu Phe Leu Phe Xaa
    210                 215

(2) INFORMATION FOR SEQ ID NO: 415:

(i) SEQUENCE CHARACTERISTICS:
(A) LENGTH: 51 amino acids
(B) TYPE: amino acid
(D) TOPOLOGY: linear
(xi) SEQUENCE DESCRIPTION: SEQ ID NO: 415:

Met Leu Pro Asp Glu Ser Phe Gly Leu Leu Leu Ser Ile Pro Ser Leu
  1               5                  10                  15

Thr Pro Ser Ala Ala Ala Pro Ser Phe Cys Val His Leu Met Gln Ala
            20                  25                  30

Ser Arg Ser Ser Lys Arg Ala Ser His Val Pro Val His Leu Leu Trp
        35                  40                  45

Gly Asp Xaa
    50

(2) INFORMATION FOR SEQ ID NO: 416:

(i) SEQUENCE CHARACTERISTICS:
(A) LENGTH: 50 amino acids
(B) TYPE: amino acid
(D) TOPOLOGY: linear
(xi) SEQUENCE DESCRIPTION: SEQ ID NO: 416:

Met Arg Pro Gly Ser Phe Ser Phe Ile Ala Phe Leu Ala Thr Glu Val
  1               5                  10                  15

Ser Ser Cys Phe Pro Gly Arg Pro Asp Cys Xaa Thr Gly Met Trp Leu
            20                  25                  30

Leu Gln Leu Gln Lys Lys Gln Arg Thr Leu Leu Ala Met Ala Pro Arg

        35                  40                  45

Arg Xaa
    50

(2) INFORMATION FOR SEQ ID NO: 417:

(i) SEQUENCE CHARACTERISTICS:
(A) LENGTH: 70 amino acids
(B) TYPE: amino acid
(D) TOPOLOGY: linear
(xi) SEQUENCE DESCRIPTION: SEQ ID NO: 417:

Asp Arg Pro Cys Pro Ser Ser Leu Trp Lys Val Phe Pro Leu Leu Leu
1               5                   10                  15

Leu Leu Met Arg Leu Phe Pro Leu Pro Val Pro Gly Asn Gln Arg Ala
            20                  25                  30

Xaa Leu Pro His Pro Phe Xaa Ala Pro Arg Leu Pro Cys Leu Leu Cys
        35                  40                  45

Leu Cys Thr Gln Gln Phe Xaa Val Cys Ser His Tyr Leu Pro Ala Gly
    50                  55                  60

Tyr Arg Val Asn Ser Xaa
65                  70

(2) INFORMATION FOR SEQ ID NO: 418:

(i) SEQUENCE CHARACTERISTICS:
(A) LENGTH: 40 amino acids
(B) TYPE: amino acid
(D) TOPOLOGY: linear
(xi) SEQUENCE DESCRIPTION: SEQ ID NO: 418:

Met His Glu Lys Ala Trp Asn Leu Ile Leu Leu Trp Trp Leu Ser Leu
1               5                   10                  15

Asp Leu Leu Gly Val Ala Lys Thr Ala Met Trp Ala Gln Trp Cys Gly
            20                  25                  30

Leu Asn Asp His Lys Gly Lys Xaa
        35                  40

(2) INFORMATION FOR SEQ ID NO: 419:

(i) SEQUENCE CHARACTERISTICS:
(A) LENGTH: 22 amino acids
(B) TYPE: amino acid
(D) TOPOLOGY: linear
(xi) SEQUENCE DESCRIPTION: SEQ ID NO: 419:

Met Ala Phe Val Leu Leu Xaa Cys Phe Val Xaa Leu Gln Ser Ser Xaa

1                   5                   10                  15

Gly Arg Ala Val Gln Xaa
            20

(2) INFORMATION FOR SEQ ID NO: 420:

(i) SEQUENCE CHARACTERISTICS:
(A) LENGTH: 38 amino acids
(B) TYPE: amino acid
(D) TOPOLOGY: linear
(xi) SEQUENCE DESCRIPTION: SEQ ID NO: 420:

Met Phe Ser Leu Leu Trp Leu Val Cys Val Pro Ser Asn Ser Ser Val
1               5                   10                  15

Ala Asn Val Thr Ala Ser Arg Gly Gly Val Phe Lys Arg Ser Leu Gly
            20                  25                  30

His Glu Gly Phe Ser Xaa
        35

(2) INFORMATION FOR SEQ ID NO: 421:

(i) SEQUENCE CHARACTERISTICS:
(A) LENGTH: 35 amino acids
(B) TYPE: amino acid
(D) TOPOLOGY: linear
(xi) SEQUENCE DESCRIPTION: SEQ ID NO: 421:

Lys Trp Leu Leu Phe Ile Phe Leu Leu Cys Leu Gln Leu Val Asn Ala
1               5                   10                  15

Leu Leu Ser Leu Phe Gln Glu Arg Phe Val His Cys Pro Ala Arg Phe
            20                  25                  30

Val Ser Xaa
        35

(2) INFORMATION FOR SEQ ID NO: 422:

(i) SEQUENCE CHARACTERISTICS:
(A) LENGTH: 32 amino acids
(B) TYPE: amino acid
(D) TOPOLOGY: linear
(xi) SEQUENCE DESCRIPTION: SEQ ID NO: 422:

Met Leu Leu Phe Leu Ser Ile Thr Asn Ser Leu Ser Phe Ile Ser Val
1               5                   10                  15

Asp Lys Pro Phe Gly Gln Ser Glu Asp Val Cys Pro Val Ile Ser Xaa
            20                  25                  30

(2) INFORMATION FOR SEQ ID NO: 423:

(i) SEQUENCE CHARACTERISTICS:
(A) LENGTH: 127 amino acids
(B) TYPE: amino acid
(D) TOPOLOGY: linear
(xi) SEQUENCE DESCRIPTION: SEQ ID NO: 423:

Met Glu Phe Leu Phe Asn Lys Thr Gly Trp Ala Phe Ala Ala Leu Cys
1               5                   10                  15

Phe Val Leu Ala Met Thr Ser Gly Gln Met Trp Asn His Ile Arg Gly
            20                  25                  30

Pro Pro Tyr Ala His Lys Asn Pro His Thr Gly His Val Asn Tyr Ile
        35                  40                  45

His Gly Ser Ser Gln Ala Gln Phe Val Ala Glu Thr His Ile Val Leu
    50                  55                  60

Leu Phe Asn Gly Gly Val Thr Leu Gly Met Val Leu Leu Cys Glu Ala
65                  70                  75                  80

Ala Thr Ser Asp Met Asp Ile Gly Lys Arg Lys Ile Met Cys Val Ala
                85                  90                  95

Gly Ile Gly Leu Val Val Leu Phe Phe Ser Trp Met Leu Ser Ile Phe
            100                 105                 110

Arg Ser Lys Tyr His Gly Tyr Pro Tyr Ser Phe Leu Met Ser Xaa
        115                 120                 125

(2) INFORMATION FOR SEQ ID NO: 424:

(i) SEQUENCE CHARACTERISTICS:
(A) LENGTH: 69 amino acids
(B) TYPE: amino acid
(D) TOPOLOGY: linear
(xi) SEQUENCE DESCRIPTION: SEQ ID NO: 424:

Met Thr Trp His Ser Arg Glu Ser Phe Xaa Leu Leu Arg Val Val Ala
1               5                   10                  15

Pro Ser Gln Ala Pro Gly Met Gln Val Ser Pro Ser Gln Arg Ala Trp
            20                  25                  30

Arg Arg Pro Leu His Arg Cys His Val Ala Ala Pro Arg Pro His His
        35                  40                  45

Phe Ala Phe Phe Arg Asn Pro Phe Ser Trp Ser Phe Ile Lys Leu Leu
    50                  55                  60

Tyr Arg Tyr Leu Xaa
65

(2) INFORMATION FOR SEQ ID NO: 425:

(i) SEQUENCE CHARACTERISTICS:
(A) LENGTH: 92 amino acids
(B) TYPE: amino acid
(D) TOPOLOGY: linear
(xi) SEQUENCE DESCRIPTION: SEQ ID NO: 425:

Met Gly Leu Lys Leu Asn Gly Arg Tyr Ile Ser Leu Ile Leu Ala Val
1               5                   10                  15

Gln Ile Ala Tyr Leu Val Gln Ala Val Arg Ala Ala Gly Lys Cys Asp
            20                  25                  30

Ala Val Phe Lys Gly Phe Ser Asp Cys Leu Leu Lys Leu Gly Asp Thr
        35                  40                  45

Trp Pro Thr Thr Arg Ser Leu Gly Arg Gln Asp Glu His Gln Asp Arg
    50                  55                  60

Val His Ile Leu Gly Gly Phe Pro Gln Leu His Gly His Ser Pro Tyr
65                  70                  75                  80

Gly Leu Pro Gly Arg Gly Glu Arg Tyr Val Gly Xaa
                85                  90

(2) INFORMATION FOR SEQ ID NO: 426:

(i) SEQUENCE CHARACTERISTICS:
(A) LENGTH: 380 amino acids
(B) TYPE: amino acid
(D) TOPOLOGY: linear
(xi) SEQUENCE DESCRIPTION: SEQ ID NO: 426:

Met Ala Arg Arg Ser Ala Phe Pro Ala Ala Ala Leu Trp Leu Trp Ser
1               5                   10                  15

Ile Leu Leu Cys Leu Leu Ala Leu Arg Ala Glu Ala Gly Pro Pro Gln
            20                  25                  30

Glu Glu Ser Leu Tyr Leu Trp Ile Asp Ala His Gln Ala Arg Val Leu
        35                  40                  45

Ile Gly Phe Glu Glu Asp Ile Leu Ile Val Ser Glu Gly Lys Met Ala
    50                  55                  60

Pro Phe Thr His Asp Phe Arg Lys Ala Gln Gln Arg Met Pro Ala Ile
65                  70                  75                  80

Pro Val Asn Ile His Ser Met Asn Phe Thr Trp Gln Ala Ala Gly Gln
                85                  90                  95

Ala Glu Tyr Phe Tyr Glu Phe Leu Ser Leu Arg Ser Leu Asp Lys Gly
            100                 105                 110

Ile Met Ala Asp Pro Thr Val Asn Val Pro Leu Leu Gly Thr Val Pro
        115                 120                 125

His Lys Ala Ser Val Val Gln Val Gly Phe Pro Cys Leu Gly Lys Gln
    130                 135                 140

Asp Gly Val Ala Ala Phe Glu Val Asp Val Ile Val Met Asn Ser Glu
145                 150                 155                 160

Gly Asn Thr Ile Leu Gln Thr Pro Gln Asn Ala Ile Phe Phe Lys Thr
                165                 170                 175

Cys Gln Gln Ala Glu Cys Pro Gly Gly Cys Arg Asn Gly Gly Phe Cys
            180                 185                 190

Asn Glu Arg Arg Ile Cys Glu Cys Pro Asp Gly Phe His Gly Pro His
        195                 200                 205

Cys Glu Lys Ala Leu Cys Thr Pro Arg Cys Met Asn Gly Gly Leu Cys
    210                 215                 220

Val Thr Pro Gly Phe Cys Ile Cys Pro Pro Gly Phe Tyr Gly Val Asn
225                 230                 235                 240

Cys Asp Lys Ala Asn Cys Ser Thr Thr Cys Phe Asn Gly Gly Thr Cys
                245                 250                 255

Phe Tyr Pro Gly Lys Cys Ile Xaa Pro Pro Gly Leu Glu Gly Glu Gln
            260                 265                 270

Cys Glu Ile Ser Lys Cys Pro Gln Pro Cys Arg Asn Gly Gly Lys Cys
        275                 280                 285

Ile Gly Lys Ser Lys Cys Lys Xaa Ser Lys Gly Tyr Gln Gly Asp Leu
    290                 295                 300

Cys Ser Lys Pro Val Cys Glu Pro Gly Cys Gly Ala His Gly Thr Cys
305                 310                 315                 320

His Glu Pro Asn Lys Cys Gln Cys Gln Glu Gly Trp His Gly Arg His
                325                 330                 335

Cys Asn Lys Arg Tyr Glu Ala Ser Leu Ile His Ala Leu Arg Pro Ala
            340                 345                 350

Gly Ala Gln Leu Arg Gln His Thr Pro Ser Leu Lys Lys Ala Glu Glu
        355                 360                 365

Arg Arg Asp Pro Pro Glu Ser Asn Tyr Ile Trp Xaa
    370                 375                 380

(2) INFORMATION FOR SEQ ID NO: 427:

(i) SEQUENCE CHARACTERISTICS:
(A) LENGTH: 24 amino acids
(B) TYPE: amino acid
(D) TOPOLOGY: linear
(xi) SEQUENCE DESCRIPTION: SEQ ID NO: 427:

Met Thr Ser Asn Leu Leu Leu Leu Thr Leu Leu Leu Lys Asp Thr Leu
1               5                   10                  15

Xaa Leu Ala Lys Xaa Asn Xaa Xaa
            20

(2) INFORMATION FOR SEQ ID NO: 428:

(i) SEQUENCE CHARACTERISTICS:
(A) LENGTH: 47 amino acids
(B) TYPE: amino acid
(D) TOPOLOGY: linear
(xi) SEQUENCE DESCRIPTION: SEQ ID NO: 428:

Met Arg His His Thr Gln Leu Asn Phe Ile Phe Leu Val Glu Met Val
1               5                   10                  15

Phe Leu His Val Gly Gln Ala Gly Leu Lys Leu Pro Thr Ser Gly Asp
            20                  25                  30

Xaa Ala Cys Phe Gly Leu Pro Lys Val Leu Gly Leu Gln Ala Xaa
        35                  40                  45

(2) INFORMATION FOR SEQ ID NO: 429:

(i) SEQUENCE CHARACTERISTICS:
(A) LENGTH: 5 amino acids
(B) TYPE: amino acid
(D) TOPOLOGY: linear
(xi) SEQUENCE DESCRIPTION: SEQ ID NO: 429:

Met Cys Ser Asp Xaa
1               5

(2) INFORMATION FOR SEQ ID NO: 430:

(i) SEQUENCE CHARACTERISTICS:
(A) LENGTH: 144 amino acids
(B) TYPE: amino acid
(D) TOPOLOGY: linear
(xi) SEQUENCE DESCRIPTION: SEQ ID NO: 430:

Leu Leu Ser Ile Leu Leu Cys Leu Leu Ala Ser Gly Leu Val Val Phe
1               5                   10                  15

Phe Leu Phe Pro His Ser Val Leu Val Asp Asp Asp Gly Ile Lys Val
            20                  25                  30

Val Lys Val Thr Phe Asn Lys Gln Asp Ser Leu Val Ile Leu Thr Ile
        35                  40                  45

Met Ala Thr Leu Lys Ile Arg Asn Ser Asn Phe Tyr Thr Val Ala Val
    50                  55                  60

Thr Ser Leu Ser Ser Gln Ile Gln Tyr Met Asn Thr Val Val Asn Phe
65                  70                  75                  80

Thr Gly Lys Ala Glu Met Gly Gly Pro Phe Ser Tyr Val Tyr Phe Phe
                85                  90                  95

Cys Thr Val Pro Glu Ile Leu Val His Asn Ile Val Ile Phe Met Arg
            100                 105                 110

Thr Ser Val Lys Ile Ser Tyr Ile Gly Leu Met Thr Gln Ser Ser Leu
        115                 120                 125

Glu Thr His His Tyr Val Asp Cys Gly Gly Asn Ser Thr Ala Ile Xaa
    130                 135                 140

(2) INFORMATION FOR SEQ ID NO: 431:

(i) SEQUENCE CHARACTERISTICS:
(A) LENGTH: 37 amino acids
(B) TYPE: amino acid
(D) TOPOLOGY: linear
(xi) SEQUENCE DESCRIPTION: SEQ ID NO: 431:

Met Phe Phe Phe Leu Tyr Val Tyr Ser Val Leu Cys Gly Leu Leu Val
1               5                   10                  15

Tyr Pro Ser Leu Pro Ser His Ser Val Ser Leu Val Thr Ser Leu Val
            20                  25                  30

Ala Ser Ala Leu Xaa
        35

(2) INFORMATION FOR SEQ ID NO: 432:

(i) SEQUENCE CHARACTERISTICS:
(A) LENGTH: 37 amino acids
(B) TYPE: amino acid
(D) TOPOLOGY: linear
(xi) SEQUENCE DESCRIPTION: SEQ ID NO: 432:

Met Ala Ser Ile Asn Ala Val Tyr Ile His Val Phe Leu Gly Val Cys
1               5                   10                  15

Val Gln Ala Thr Ala Ala Cys Pro Trp Cys Ser Gln Cys Arg Xaa Gly
            20                  25                  30

Ser Val Pro Ser Xaa
        35

(2) INFORMATION FOR SEQ ID NO: 433:

(i) SEQUENCE CHARACTERISTICS:
(A) LENGTH: 192 amino acids
(B) TYPE: amino acid
(D) TOPOLOGY: linear
(xi) SEQUENCE DESCRIPTION: SEQ ID NO: 433:

Met Met Ala Ala Met Val Leu Thr Ser Leu Ser Cys Ser Pro Val Val
1               5                   10                  15

Gln Ser Pro Pro Gly Thr Glu Ala Asn Phe Ser Ala Ser Arg Ala Ala
            20                  25                  30

Cys Asp Pro Trp Lys Glu Ser Gly Asp Ile Ser Asp Ser Gly Xaa Ser
        35                  40                  45

Thr Thr Ser Gly His Trp Ser Gly Ser Ser Gly Val Ser Thr Pro Ser
    50                  55                  60

Pro Pro His Pro Gln Ala Ser Pro Lys Tyr Leu Gly Asp Ala Phe Gly
65                  70                  75                  80

Ser Pro Gln Thr Asp His Gly Phe Glu Thr Asp Pro Asp Pro Phe Leu
                85                  90                  95

Leu Asp Glu Pro Ala Pro Arg Lys Arg Lys Asn Ser Val Lys Val Met
            100                 105                 110

Tyr Lys Cys Leu Trp Pro Asn Cys Gly Lys Val Leu Arg Ser Ile Val
        115                 120                 125

Gly Ile Lys Arg His Val Lys Ala Leu His Leu Gly Asp Thr Val Asp
    130                 135                 140

Ser Asp Gln Phe Lys Arg Glu Glu Asp Phe Tyr Tyr Thr Glu Val Gln
145                 150                 155                 160

Leu Lys Glu Glu Ser Ala Ala Ala Ala Ala Ala Ala Ala Ala Asp Pro
                165                 170                 175

Gln Ser Leu Gly Leu Pro Pro Pro Ser Gln Leu Pro Pro Pro Ala Xaa
            180                 185                 190

(2) INFORMATION FOR SEQ ID NO: 434:

(i) SEQUENCE CHARACTERISTICS:
(A) LENGTH: 31 amino acids
(B) TYPE: amino acid
(D) TOPOLOGY: linear
(xi) SEQUENCE DESCRIPTION: SEQ ID NO: 434:

Met Ser Thr Asn Tyr Leu Thr Asp Val Cys Ser Leu Phe Ser Tyr Leu
1               5                   10                  15

Asn Tyr Leu Tyr Phe His His His Leu Pro Val Pro Asn Thr Xaa

            20                  25                  30

(2) INFORMATION FOR SEQ ID NO: 435:

(i) SEQUENCE CHARACTERISTICS:
(A) LENGTH: 101 amino acids
(B) TYPE: amino acid
(D) TOPOLOGY: linear
(xi) SEQUENCE DESCRIPTION: SEQ ID NO: 435:

Met Gly Phe Phe Phe Val Leu Phe Phe Leu Tyr Leu Ala Leu Ser Arg
1               5                   10                  15

Asp Trp Ser Ile Asn Phe Leu Lys Asp His Arg Ile Asn Phe Phe Val
            20                  25                  30

Ala Thr Ser Tyr Phe Ser Val Tyr Val Arg Gly Xaa Pro Xaa Val Pro
        35                  40                  45

Ala Asp Thr Pro Leu Gly Pro Leu Leu Ser Leu Trp Leu His His Asn
    50                  55                  60

Ala Phe Phe Ser Ile Leu Pro Lys Phe Pro Glu Asn Xaa Xaa Phe Leu
65                  70                  75                  80

Ile Leu Lys Lys Leu Val Val Glu Met Gly Trp Asp Leu Phe Ile Ser
                85                  90                  95

Pro Glu Asn Lys Xaa
            100

(2) INFORMATION FOR SEQ ID NO: 436:

(i) SEQUENCE CHARACTERISTICS:
(A) LENGTH: 37 amino acids
(B) TYPE: amino acid
(D) TOPOLOGY: linear
(xi) SEQUENCE DESCRIPTION: SEQ ID NO: 436:

Met Ala Arg Tyr Phe Ile Phe Phe Ile Leu Val Phe Met Lys Val Ser
1               5                   10                  15

Leu Asn Thr Thr Trp Pro Ala Pro Arg Pro Ala Thr Leu Arg Thr Ala
            20                  25                  30

Asn Lys Ser Lys Xaa
        35

(2) INFORMATION FOR SEQ ID NO: 437:

(i) SEQUENCE CHARACTERISTICS:
(A) LENGTH: 42 amino acids
(B) TYPE: amino acid
(D) TOPOLOGY: linear

(xi) SEQUENCE DESCRIPTION: SEQ ID NO: 437:

Phe Ser Thr Ile Arg Ser Gly Leu Thr Asp Arg Ser Val Asn Phe Leu
1               5                   10                  15

Phe Leu Phe Leu Asp Val Pro Asp Cys Arg Leu Val Asn Ile Glu Leu
            20                  25                  30

Met Ala Asn Ser Thr Val Thr His Ala Xaa
        35                  40

(2) INFORMATION FOR SEQ ID NO: 438:

(i) SEQUENCE CHARACTERISTICS:
(A) LENGTH: 1 amino acids
(B) TYPE: amino acid
(D) TOPOLOGY: linear
(xi) SEQUENCE DESCRIPTION: SEQ ID NO: 438:

Leu
1

(2) INFORMATION FOR SEQ ID NO: 439:

(i) SEQUENCE CHARACTERISTICS:
(A) LENGTH: 25 amino acids
(B) TYPE: amino acid
(D) TOPOLOGY: linear
(xi) SEQUENCE DESCRIPTION: SEQ ID NO: 439:

Met Pro Trp Arg Arg Ala Gly Leu Met Met Leu Pro Ile Ile Thr Gly
1               5                   10                  15

Cys Cys Pro Cys Ser Ala Ser Ile Xaa
            20                  25

(2) INFORMATION FOR SEQ ID NO: 440:

(i) SEQUENCE CHARACTERISTICS:
(A) LENGTH: 54 amino acids
(B) TYPE: amino acid
(D) TOPOLOGY: linear
(xi) SEQUENCE DESCRIPTION: SEQ ID NO: 440:

(2) INFORMATION FOR SEQ ID NO: 441:

(i) SEQUENCE CHARACTERISTICS:
(A) LENGTH: 42 amino acids
(B) TYPE: amino acid
(D) TOPOLOGY: linear
(xi) SEQUENCE DESCRIPTION: SEQ ID NO: 441:

Met Thr Ala Leu Val Trp Arg Lys Gly Pro Asp Gly Gly Ser Arg Lys
1               5                   10                  15

Pro Ile Leu Leu Leu Phe Phe Phe Leu Pro Leu Ile Leu Cys Phe His
            20                  25                  30

Ser Phe Ile His Ser Ser Asn Ile Cys Xaa
        35                  40

(2) INFORMATION FOR SEQ ID NO: 442:

(i) SEQUENCE CHARACTERISTICS:
(A) LENGTH: 66 amino acids
(B) TYPE: amino acid
(D) TOPOLOGY: linear
(xi) SEQUENCE DESCRIPTION: SEQ ID NO: 442:

Met Phe Leu Thr Thr Trp Phe Leu Leu Leu Ser Val Ala Trp Xaa Ala
1               5                   10                  15

Leu Thr Arg Ser Gly Arg Ser Cys Leu Pro Leu Val Gly Arg Pro Arg
            20                  25                  30

Glu Gln Ser Pro Arg Thr His Cys Ala Ala Ser Ser Thr Lys Glu Arg
        35                  40                  45

Asn Ser Asp Pro Gln Pro Ser Pro Pro Glu Val Val Gly Pro Leu Trp
    50                  55                  60

Ser Xaa
65

(2) INFORMATION FOR SEQ ID NO: 443:

(i) SEQUENCE CHARACTERISTICS:
(A) LENGTH: 156 amino acids
(B) TYPE: amino acid
(D) TOPOLOGY: linear
(xi) SEQUENCE DESCRIPTION: SEQ ID NO: 443:

Met Lys Ala Ile Gly Ile Glu Pro Ser Leu Ala Thr Tyr His His Ile
1               5                   10                  15

Ile Arg Leu Phe Asp Gln Pro Gly Asp Pro Leu Lys Arg Ser Ser Phe

            20                  25                  30

Ile Ile Tyr Asp Ile Met Asn Glu Leu Met Gly Lys Arg Phe Ser Pro
        35                  40                  45

Lys Asp Pro Asp Asp Asp Lys Phe Phe Gln Ser Ala Met Ser Ile Cys
    50                  55                  60

Ser Ser Leu Arg Asp Leu Glu Leu Ala Tyr Gln Val His Gly Leu Leu
65                  70                  75                  80

Lys Thr Gly Asp Asn Trp Lys Phe Ile Gly Pro Asp Gln His Arg Asn
                85                  90                  95

Phe Tyr Tyr Ser Lys Phe Phe Asp Leu Ile Cys Leu Met Glu Gln Ile
            100                 105                 110

Asp Val Thr Leu Lys Trp Tyr Glu Asp Leu Ile Pro Ser Ala Tyr Phe
        115                 120                 125

Pro His Ser Gln Thr Met Ile His Leu Leu Gln Ala Leu Asp Val Ala
    130                 135                 140

Asn Arg Leu Glu Val Ile Pro Lys Ile Trp Glu Arg
145                 150                 155

(2) INFORMATION FOR SEQ ID NO: 444:

(i) SEQUENCE CHARACTERISTICS:
(A) LENGTH: 57 amino acids
(B) TYPE: amino acid
(D) TOPOLOGY: linear
(xi) SEQUENCE DESCRIPTION: SEQ ID NO: 444:

Met His Phe Leu Phe Arg Phe Ile Val Phe Phe Tyr Leu Trp Gly Leu
1               5                   10                  15

Phe Thr Ala Gln Arg Gln Lys Lys Glu Glu Ser Thr Glu Glu Val Lys
            20                  25                  30

Ile Glu Val Leu His Arg Pro Glu Asn Cys Ser Lys Thr Ser Lys Lys
        35                  40                  45

Gly Asp Leu Leu Lys Cys Pro Leu Xaa
    50                  55

(2) INFORMATION FOR SEQ ID NO: 445:

(i) SEQUENCE CHARACTERISTICS:
(A) LENGTH: 416 amino acids
(B) TYPE: amino acid
(D) TOPOLOGY: linear
(xi) SEQUENCE DESCRIPTION: SEQ ID NO: 445:

Met Arg Thr Leu Phe Asn Leu Leu Trp Leu Ala Leu Ala Cys Ser Pro
1               5                   10                  15

Val His Thr Thr Leu Ser Lys Ser Asp Ala Lys Lys Ala Ala Ser Lys
            20                  25                  30

Thr Leu Leu Glu Lys Ser Gln Phe Ser Asp Lys Pro Val Gln Asp Arg
        35                  40                  45

Gly Leu Val Val Thr Asp Leu Lys Ala Glu Ser Val Val Leu Glu His
    50                  55                  60

Arg Ser Tyr Cys Ser Ala Lys Ala Arg Asp Arg His Phe Ala Gly Asp
65                  70                  75                  80

Val Leu Gly Tyr Val Thr Pro Trp Asn Ser His Gly Tyr Asp Val Thr
                85                  90                  95

Lys Val Phe Gly Ser Lys Phe Thr Gln Ile Ser Pro Val Trp Leu Gln
            100                 105                 110

Leu Lys Arg Arg Gly Arg Glu Met Phe Glu Val Thr Gly Leu His Asp
        115                 120                 125

Val Asp Gln Gly Trp Met Arg Ala Val Arg Lys His Ala Lys Gly Leu
    130                 135                 140

His Ile Val Pro Arg Leu Leu Phe Glu Asp Trp Thr Tyr Asp Asp Phe
145                 150                 155                 160

Arg Asn Val Leu Asp Ser Glu Asp Glu Ile Glu Glu Leu Ser Lys Thr
                165                 170                 175

Val Val Gln Val Ala Lys Asn Gln His Phe Asp Gly Phe Val Val Glu
            180                 185                 190

Val Trp Asn Gln Leu Leu Ser Gln Lys Arg Val Gly Leu Ile His Met
        195                 200                 205

Leu Thr His Leu Ala Glu Ala Leu His Gln Ala Arg Leu Leu Ala Leu
    210                 215                 220

Leu Val Ile Pro Pro Ala Ile Thr Pro Gly Thr Asp Gln Leu Gly Met
225                 230                 235                 240

Phe Thr His Lys Glu Phe Glu Gln Leu Ala Pro Val Leu Asp Gly Phe
                245                 250                 255

Ser Leu Met Thr Tyr Asp Tyr Ser Thr Ala His Gln Pro Gly Pro Asn
            260                 265                 270

Ala Pro Leu Ser Trp Val Arg Ala Cys Val Gln Val Leu Asp Pro Lys
        275                 280                 285

Ser Lys Trp Arg Ser Lys Ile Leu Leu Gly Leu Asn Phe Tyr Gly Met
    290                 295                 300

Asp Tyr Ala Thr Ser Lys Asp Ala Arg Glu Pro Val Val Gly Ala Arg
305                 310                 315                 320

Tyr Ile Gln Thr Leu Lys Asp His Arg Pro Arg Met Val Trp Asp Ser
                325                 330                 335

Gln Xaa Ser Glu His Phe Phe Glu Tyr Lys Lys Ser Arg Ser Gly Arg
            340                 345                 350

His Val Val Phe Tyr Pro Thr Leu Lys Ser Leu Gln Val Arg Leu Glu
        355                 360                 365

Leu Ala Arg Glu Leu Gly Val Gly Val Ser Ile Trp Glu Leu Ala Arg
    370                 375                 380

Ala Trp Thr Thr Ser Thr Thr Cys Ser Arg Trp Ala Leu Arg Pro Pro
385                 390                 395                 400

Arg Trp Thr Cys Ser Phe Leu Ser His Gly Val Ser Glu Gln Val Xaa
                405                 410                 415

(2) INFORMATION FOR SEQ ID NO: 446:

        (i) SEQUENCE CHARACTERISTICS:
                (A) LENGTH: 64 amino acids
                (B) TYPE: amino acid
                (D) TOPOLOGY: linear
        (xi) SEQUENCE DESCRIPTION: SEQ ID NO: 446:

Met Ala Pro Gly Pro Leu Ser Ala Thr Gln Ala Val Val Ile His Thr
  1               5                  10                  15

Thr His Cys Leu Gln Leu Pro Val Trp Cys Leu Ser Leu Val Ser Glu
            20                  25                  30

Leu Leu Gly Arg Ala Pro Pro His Asn Lys Asp Ala Leu Arg Pro Ser
        35                  40                  45

Lys Lys Lys Lys Lys Lys Leu Xaa Gly Gly Pro Val Pro Ile Pro Pro
    50                  55                  60

(2) INFORMATION FOR SEQ ID NO: 447:

        (i) SEQUENCE CHARACTERISTICS:
                (A) LENGTH: 206 amino acids
                (B) TYPE: amino acid
                (D) TOPOLOGY: linear
        (xi) SEQUENCE DESCRIPTION: SEQ ID NO: 447:

Met Leu Gly Ala Lys Pro His Trp Leu Pro Gly Pro Leu His Ser Pro
  1               5                  10                  15

Gly Leu Pro Leu Val Leu Val Leu Leu Ala Leu Gly Ala Gly Trp Ala
            20                  25                  30

Gln Glu Gly Ser Glu Pro Val Leu Leu Glu Gly Glu Cys Leu Val Val
        35                  40                  45

Cys Glu Pro Gly Arg Ala Ala Ala Gly Gly Pro Gly Gly Ala Ala Leu
    50                  55                  60

Gly Glu Ala Pro Pro Gly Arg Val Ala Phe Ala Ala Val Arg Ser Xaa
65                  70                  75                  80

His His Glu Pro Ala Gly Glu Thr Gly Asn Gly Thr Xaa Gly Ala Ile
                85                  90                  95

Tyr Phe Asp Gln Val Leu Val Asn Glu Gly Gly Gly Phe Asp Arg Ala
            100                 105                 110

Ser Gly Ser Phe Val Ala Pro Val Arg Gly Val Tyr Ser Phe Arg Phe
        115                 120                 125

His Val Val Lys Val Tyr Asn Arg Gln Thr Val Gln Val Ser Leu Met
    130                 135                 140

Leu Asn Thr Trp Pro Val Ile Ser Ala Phe Ala Asn Asp Pro Asp Val
145                 150                 155                 160

Thr Arg Glu Ala Ala Thr Ser Ser Val Leu Leu Pro Leu Asp Pro Gly
                165                 170                 175

Asp Arg Val Ser Leu Arg Leu Arg Arg Gly Asn Leu Leu Gly Gly Trp
            180                 185                 190

Lys Tyr Ser Ser Phe Ser Gly Phe Leu Ile Phe Pro Leu Xaa
        195                 200                 205

(2) INFORMATION FOR SEQ ID NO: 448:

(i) SEQUENCE CHARACTERISTICS:
(A) LENGTH: 62 amino acids
(B) TYPE: amino acid
(D) TOPOLOGY: linear
(xi) SEQUENCE DESCRIPTION: SEQ ID NO: 448:

Met Ser Ser Leu Leu Ser Ala Gly Leu Gln Ala Ser Leu Cys Gly Lys
1               5                   10                  15

Xaa Leu Trp Ala Ser Thr Trp Tyr Leu Val Cys Cys Leu Leu Pro Phe
            20                  25                  30

Phe His Gln Gly Cys Cys Asp His Lys Ser Lys Gln Gln Tyr Ile Pro
        35                  40                  45

Asn Leu Lys Ser Tyr Cys Gly Leu Ser Thr Ile Glu Ile Xaa
    50                  55                  60

(2) INFORMATION FOR SEQ ID NO: 449:

(i) SEQUENCE CHARACTERISTICS:

(A) LENGTH: 316 amino acids
(B) TYPE: amino acid
(D) TOPOLOGY: linear
(xi) SEQUENCE DESCRIPTION: SEQ ID NO: 449:

Met Ser Thr Lys Lys Leu Cys Ile Val Gly Gly Ile Leu Leu Val Phe
1               5                   10                  15

Gln Ile Ile Ala Phe Leu Val Gly Gly Leu Ile Ala Pro Gly Pro Thr
            20                  25                  30

Thr Ala Val Ser Tyr Met Ser Val Lys Cys Val Asp Ala Arg Lys Asn
        35                  40                  45

His His Lys Thr Lys Trp Phe Val Pro Trp Gly Pro Asn His Cys Asp
    50                  55                  60

Lys Ile Arg Asp Ile Glu Glu Ala Ile Pro Arg Glu Ile Glu Ala Asn
65                  70                  75                  80

Asp Ile Val Phe Ser Val His Ile Pro Leu Pro His Met Glu Met Ser
                85                  90                  95

Pro Trp Phe Gln Phe Met Xaa Phe Ile Leu Gln Leu Asp Ile Ala Phe
            100                 105                 110

Lys Leu Asn Asn Gln Ile Arg Glu Asn Ala Glu Val Ser Met Asp Val
        115                 120                 125

Ser Leu Ala Tyr Arg Asp Asp Ala Phe Ala Glu Trp Thr Glu Met Ala
    130                 135                 140

His Glu Arg Val Pro Arg Lys Leu Lys Cys Thr Phe Thr Ser Pro Lys
145                 150                 155                 160

Thr Pro Glu His Gly Gly Pro Val Thr Met Asn Val Met Ser Phe Leu
                165                 170                 175

Ser Trp Lys Leu Gly Leu Trp Pro Met Lys Phe Tyr Leu Leu Asn Ile
            180                 185                 190

Arg Leu Pro Val Asn Glu Lys Lys Lys Ile Asn Val Gly Ile Gly Glu
        195                 200                 205

Ile Lys Asp Ile Arg Leu Val Gly Ile His Gln Asn Gly Gly Phe Thr
    210                 215                 220

Lys Val Trp Phe Ala Met Lys Thr Phe Leu Thr Pro Ser Ile Phe Ile
225                 230                 235                 240

Ile Met Val Trp Tyr Trp Arg Arg Ile Thr Met Met Ser Arg Pro Pro
                245                 250                 255

Val Leu Leu Glu Lys Val Ile Phe Ala Leu Gly Ile Ser Met Thr Phe
            260                 265                 270

Ile Asn Ile Pro Val Glu Trp Phe Ser Ile Gly Phe Asp Trp Thr Trp
        275                 280                 285

Met Leu Leu Phe Gly Asp Ile Arg Gln Ala Ser Ser Met Xaa Cys Phe

290                    295                    300

Xaa Pro Ser Gly Ser Ser Ser Val Ala Ser Thr Xaa
305                    310                    315

(2) INFORMATION FOR SEQ ID NO: 450:

        (i) SEQUENCE CHARACTERISTICS:
                (A) LENGTH: 24 amino acids
                (B) TYPE: amino acid
                (D) TOPOLOGY: linear
        (xi) SEQUENCE DESCRIPTION: SEQ ID NO: 450:

Met Leu Ala Leu Leu Gly Leu Leu Ala Gly Thr Glu His Pro Pro Gly
  1               5                  10                  15

Pro Gln Gly Pro Gly Pro Ser Xaa
            20

(2) INFORMATION FOR SEQ ID NO: 451:

        (i) SEQUENCE CHARACTERISTICS:
                (A) LENGTH: 10 amino acids
                (B) TYPE: amino acid
                (D) TOPOLOGY: linear
        (xi) SEQUENCE DESCRIPTION: SEQ ID NO: 451:

Met Pro Ser Gly Ala Cys Cys Ser Pro Xaa
  1               5                  10

(2) INFORMATION FOR SEQ IQ NO: 452:

        (i) SEQUENCE CHARACTERISTICS:
                (A) LENGTH: 26 amino acids
                (B) TYPE: amino acid
                (D) TOPOLOGY: linear
        (xi) SEQUENCE DESCRIPTION: SEQ ID NO: 452:

Met Leu Pro Ala Leu Ser Thr Val Leu Leu Pro Thr Pro Ser Leu Cys
  1               5                  10                  15

Ser Gly Asn Pro Arg Glu Gly Trp Ala Xaa
            20                  25

(2) INFORMATION FOR SEQ ID NO: 453:

        (i) SEQUENCE CHARACTERISTICS:
                (A) LENGTH: 172 amino acids
                (B) TYPE: amino acid
                (D) TOPOLOGY: linear
        (xi) SEQUENCE DESCRIPTION: SEQ ID NO: 453:

Met Tyr Ser Leu His Ser Trp Val Gly Leu Ile Ala Val Ile Cys Tyr
1               5                   10                  15

Leu Leu Gln Leu Leu Ser Gly Phe Ser Val Phe Leu Leu Pro Trp Ala
            20                  25                  30

Pro Leu Ser Leu Arg Ala Phe Leu Met Pro Ile His Val Tyr Ser Gly
        35                  40                  45

Ile Val Ile Phe Gly Thr Val Ile Ala Thr Ala Leu Met Gly Leu Thr
    50                  55                  60

Glu Lys Leu Ile Phe Ser Leu Arg Asp Pro Ala Tyr Ser Thr Phe Pro
65                  70                  75                  80

Pro Glu Gly Val Phe Val Asn Thr Leu Gly Leu Leu Ile Leu Val Phe
                85                  90                  95

Gly Ala Leu Ile Phe Trp Ile Val Thr Arg Pro Gln Trp Lys Arg Pro
            100                 105                 110

Lys Glu Pro Asn Ser Thr Ile Leu His Pro Asn Gly Gly Thr Glu Gln
        115                 120                 125

Gly Ala Arg Gly Ser Met Pro Ala Tyr Ser Gly Asn Asn Met Asp Lys
    130                 135                 140

Ser Asp Ser Glu Leu Asn Xaa Glu Val Ala Ala Arg Lys Arg Asn Leu
145                 150                 155                 160

Ala Leu Asp Glu Ala Gly Gln Arg Ser Thr Met Xaa
                165                 170

(2) INFORMATION FOR SEQ ID NO: 454:

(i) SEQUENCE CHARACTERISTICS:
(A) LENGTH: 96 amino acids
(B) TYPE: amino acid
(D) TOPOLOGY: linear
(xi) SEQUENCE DESCRIPTION: SEQ ID NO: 454:

Met Phe His Val Leu Met Ala Gln Val Thr Xaa Val Ile Ile Thr Thr
1               5                   10                  15

Val Ser Val Leu Val Phe Asp Phe Arg Pro Ser Leu Glu Phe Phe Leu
            20                  25                  30

Glu Ala Xaa Ser Val Xaa Leu Ser Ile Phe Ile Tyr Asn Ala Ser Lys
        35                  40                  45

Pro Gln Val Pro Glu Tyr Ala Pro Arg Gln Glu Arg Ile Arg Asp Leu
    50                  55                  60

Ser Gly Asn Leu Trp Glu Arg Ser Ser Gly Asp Gly Glu Glu Leu Glu
65                  70                  75                  80

Arg Leu Thr Lys Pro Lys Ser Asp Glu Ser Asp Glu Asp Thr Phe Xaa
                85                  90                  95

(2) INFORMATION FOR SEQ ID NO: 455:

(i) SEQUENCE CHARACTERISTICS:
(A) LENGTH: 171 amino acids
(B) TYPE: amino acid
(D) TOPOLOGY: linear
(xi) SEQUENCE DESCRIPTION: SEQ ID NO: 455:

Met Arg Gly Pro Ala Gln Ala Lys Leu Leu Pro Gly Ser Ala Ile Gln
1               5                   10                  15

Ala Leu Val Gly Leu Ala Arg Pro Leu Val Leu Ala Leu Leu Leu Val
            20                  25                  30

Ser Ala Ala Leu Ser Ser Val Val Ser Arg Thr Asp Ser Pro Ser Pro
        35                  40                  45

Thr Val Leu Asn Ser His Ile Ser Thr Pro Asn Val Asn Ala Leu Thr
    50                  55                  60

His Glu Asn Gln Thr Lys Pro Ser Ile Ser Gln Ile Ser Thr Thr Leu
65                  70                  75                  80

Pro Pro Thr Thr Ser Thr Lys Lys Ser Gly Gly Ala Ser Val Val Pro
                85                  90                  95

His Pro Ser Pro Thr Pro Leu Ser Gln Glu Glu Ala Asp Asn Asn Glu
            100                 105                 110

Asp Pro Ser Ile Glu Glu Glu Asp Leu Leu Met Leu Asn Ser Ser Pro
        115                 120                 125

Ser Thr Ala Lys Asp Thr Leu Asp Asn Gly Asp Tyr Gly Glu Pro Asp
    130                 135                 140

Tyr Asp Trp Thr Thr Gly Pro Arg Asp Asp Asp Glu Ser Asp Xaa His
145                 150                 155                 160

Leu Gly Arg Lys Gln Gly Leu His Gly Asn Xaa
                165                 170

(2) INFORMATION FOR SEQ ID NO: 456:

(i) SEQUENCE CHARACTERISTICS:
(A) LENGTH: 92 amino acids
(B) TYPE: amino acid
(D) TOPOLOGY: linear
(xi) SEQUENCE DESCRIPTION: SEQ ID NO: 456:

Met Lys Ala Ser Gln Cys Cys Cys Cys Leu Ser His Leu Leu Ala Ser
1               5                   10                  15

Val Leu Leu Leu Leu Leu Leu Pro Glu Leu Ser Gly Xaa Leu Xaa Val
            20                  25                  30

Leu Leu Gln Ala Ala Glu Ala Ala Pro Gly Xaa Gly Pro Pro Asp Pro
        35                  40                  45

Arg Pro Gly His Tyr Arg Arg Cys His Arg Ala Leu Thr Pro Ala Gln
    50                  55                  60

Gln Pro Gly Arg Gly Leu Ala Glu Ala Ala Gly Ala Ala Gly Leu Arg
65                  70                  75                  80

Gly Arg Gln Trp Gln Gln Pro Cys Gly Arg Ala Xaa
                85                  90

(2) INFORMATION FOR SEQ ID NO: 457:

(i) SEQUENCE CHARACTERISTICS:
(A) LENGTH: 206 amino acids
(B) TYPE: amino acid
(D) TOPOLOGY: linear
(xi) SEQUENCE DESCRIPTION: SEQ ID NO: 457:

Ile Ser Val Leu Xaa Tyr Pro His Cys Val Val His Glu Leu Pro Glu
1               5                   10                  15

Leu Thr Ala Glu Ser Leu Glu Ala Gly Asp Ser Asn Gln Phe Cys Trp
            20                  25                  30

Arg Asn Leu Phe Ser Cys Ile Asn Leu Leu Arg Ile Leu Asn Lys Leu
        35                  40                  45

Thr Lys Trp Lys His Ser Arg Thr Met Met Leu Val Val Phe Lys Ser
    50                  55                  60

Ala Pro Ile Leu Lys Arg Ala Leu Lys Val Lys Gln Ala Met Met Gln
65                  70                  75                  80

Leu Tyr Val Leu Lys Leu Leu Lys Val Gln Thr Lys Tyr Leu Gly Arg
                85                  90                  95

Gln Trp Arg Lys Ser Asn Met Lys Thr Met Ser Ala Ile Tyr Gln Lys
            100                 105                 110

Val Arg His Arg Leu Asn Asp Asp Trp Ala Tyr Gly Asn Asp Leu Asp
        115                 120                 125

Ala Arg Pro Trp Asp Phe Gln Ala Glu Glu Cys Ala Leu Arg Ala Asn
    130                 135                 140

Ile Glu Arg Phe Asn Ala Arg Arg Tyr Asp Arg Ala His Ser Asn Pro
145                 150                 155                 160

Asp Phe Leu Pro Val Asp Asn Cys Leu Gln Ser Val Leu Gly Gln Arg
                165                 170                 175

Val Asp Leu Pro Glu Asp Phe Gln Met Asn Tyr Asp Leu Trp Leu Glu
            180                 185                 190

Arg Glu Val Phe Ser Lys Pro Ile Ser Trp Glu Glu Leu Leu
        195                 200                 205

(2) INFORMATION FOR SEQ ID NO: 458:

        (i) SEQUENCE CHARACTERISTICS:
              (A) LENGTH: 317 amino acids
              (B) TYPE: amino acid
              (D) TOPOLOGY: linear
        (xi) SEQUENCE DESCRIPTION: SEQ ID NO: 458:

Met Ala Pro Pro Ala Pro Gly Pro Ala Ser Gly Gly Ser Gly Glu Val
  1               5                  10                  15

Asp Glu Leu Phe Asp Val Lys Asn Ala Phe Tyr Ile Gly Ser Tyr Gln
             20                  25                  30

Gln Cys Ile Asn Glu Ala Xaa Xaa Val Lys Leu Ser Ser Pro Glu Arg
         35                  40                  45

Asp Val Glu Arg Asp Val Phe Leu Tyr Arg Ala Tyr Leu Ala Gln Arg
     50                  55                  60

Lys Phe Gly Val Val Leu Asp Glu Ile Lys Pro Ser Ser Ala Pro Glu
 65                  70                  75                  80

Leu Gln Ala Val Arg Met Phe Ala Asp Tyr Leu Ala His Glu Ser Arg
                 85                  90                  95

Arg Asp Ser Ile Val Ala Glu Leu Asp Arg Glu Met Ser Arg Ser Xaa
            100                 105                 110

Asp Val Thr Asn Thr Thr Phe Leu Leu Met Ala Ala Ser Ile Tyr Leu
        115                 120                 125

His Asp Gln Asn Pro Asp Ala Ala Leu Arg Ala Leu His Gln Gly Asp
    130                 135                 140

Ser Leu Glu Cys Thr Ala Met Thr Val Gln Ile Leu Leu Lys Leu Asp
145                 150                 155                 160

Arg Leu Asp Leu Ala Arg Lys Glu Leu Lys Arg Met Gln Asp Leu Asp
                165                 170                 175

Glu Asp Ala Thr Leu Thr Gln Leu Ala Thr Ala Trp Val Ser Leu Ala
            180                 185                 190

Thr Gly Gly Glu Lys Leu Gln Asp Ala Tyr Tyr Ile Phe Gln Glu Met
        195                 200                 205

Ala Asp Lys Cys Ser Pro Thr Leu Leu Leu Leu Asn Gly Gln Ala Ala
    210                 215                 220

Cys His Met Ala Gln Gly Arg Trp Glu Ala Ala Glu Gly Leu Leu Gln
225                 230                 235                 240

Glu Ala Leu Asp Lys Asp Ser Gly Tyr Pro Glu Thr Leu Val Asn Leu

                245                 250                 255

Ile Val Leu Ser Gln His Leu Gly Lys Pro Pro Glu Val Thr Asn Arg
            260                 265                 270

Tyr Leu Ser Gln Leu Lys Asp Ala His Arg Ser His Pro Phe Ile Lys
        275                 280                 285

Glu Tyr Gln Ala Lys Glu Asn Asp Phe Asp Arg Leu Val Leu Gln Tyr
    290                 295                 300

Ala Pro Ser Ala Glu Ala Gly Pro Glu Leu Ser Gly Pro
305                 310                 315

(2) INFORMATION FOR SEQ ID NO: 459:

(i) SEQUENCE CHARACTERISTICS:
(A) LENGTH: 261 amino acids
(B) TYPE: amino acid
(D) TOPOLOGY: linear
(xi) SEQUENCE DESCRIPTION: SEQ ID NO: 459:

Arg Asp Val Glu Arg Asp Val Phe Leu Tyr Arg Ala Tyr Leu Ala Gln
1               5                   10                  15

Arg Lys Phe Gly Val Val Leu Asp Glu Ile Lys Pro Ser Ser Ala Pro
            20                  25                  30

Glu Leu Gln Ala Val Arg Met Phe Ala Asp Tyr Leu Ala His Glu Ser
        35                  40                  45

Arg Arg Asp Ser Ile Val Ala Glu Leu Asp Arg Glu Met Ser Arg Ser
    50                  55                  60

Xaa Asp Val Thr Asn Thr Thr Phe Leu Leu Met Ala Ala Ser Ile Tyr
65                  70                  75                  80

Leu His Asp Gln Asn Pro Asp Ala Ala Leu Arg Ala Leu His Gln Gly
                85                  90                  95

Asp Ser Leu Glu Cys Thr Ala Met Thr Val Gln Ile Leu Leu Lys Leu
            100                 105                 110

Asp Arg Leu Asp Leu Ala Arg Lys Glu Leu Lys Arg Met Gln Asp Leu
        115                 120                 125

Asp Glu Asp Ala Thr Leu Thr Gln Leu Ala Thr Ala Trp Val Ser Leu
    130                 135                 140

Ala Thr Gly Gly Glu Lys Leu Gln Asp Ala Tyr Tyr Ile Phe Gln Glu
145                 150                 155                 160

Met Ala Asp Lys Cys Ser Pro Thr Leu Leu Leu Leu Asn Gly Gln Ala
                165                 170                 175

Ala Cys His Met Ala Gln Gly Arg Trp Glu Ala Ala Glu Gly Leu Leu
            180                 185                 190

Gln Glu Ala Leu Asp Lys Asp Ser Gly Tyr Pro Glu Thr Leu Val Asn
        195                 200                 205

Leu Ile Val Leu Ser Gln His Leu Gly Lys Pro Pro Glu Val Thr Asn
    210                 215                 220

Arg Tyr Leu Ser Gln Leu Lys Asp Ala His Arg Ser His Pro Phe Ile
225                 230                 235                 240

Lys Glu Tyr Gln Ala Lys Glu Asn Asp Phe Asp Arg Leu Val Leu Gln
                245                 250                 255

Tyr Ala Pro Ser Ala
            260

(2) INFORMATION FOR SEQ ID NO: 460:

(i) SEQUENCE CHARACTERISTICS:
(A) LENGTH: 156 amino acids
(B) TYPE: amino acid
(D) TOPOLOGY: linear
(xi) SEQUENCE DESCRIPTION: SEQ ID NO: 460:

Met Lys Ala Ile Gly Ile Glu Pro Ser Leu Ala Thr Tyr His His Ile
1               5                   10                  15

Ile Arg Leu Phe Asp Gln Pro Gly Asp Pro Leu Lys Arg Ser Ser Phe
            20                  25                  30

Ile Ile Tyr Asp Ile Met Asn Glu Leu Met Gly Lys Arg Phe Ser Pro
        35                  40                  45

Lys Asp Pro Asp Asp Asp Lys Phe Phe Gln Ser Ala Met Ser Ile Cys
    50                  55                  60

Ser Ser Leu Arg Asp Leu Glu Leu Ala Tyr Gln Val His Gly Leu Leu
65                  70                  75                  80

Lys Thr Gly Asp Asn Trp Lys Phe Ile Gly Pro Asp Gln His Arg Asn
                85                  90                  95

Phe Tyr Tyr Ser Lys Phe Phe Asp Leu Ile Cys Leu Met Glu Gln Ile
            100                 105                 110

Asp Val Thr Leu Lys Trp Tyr Glu Asp Leu Ile Pro Ser Ala Tyr Phe
        115                 120                 125

Pro His Ser Gln Thr Met Ile His Leu Leu Gln Ala Leu Asp Val Ala
    130                 135                 140

Asn Arg Leu Glu Val Ile Pro Lys Ile Trp Glu Arg
145                 150                 155

(2) INFORMATION FOR SEQ ID NO: 461:

(i) SEQUENCE CHARACTERISTICS:

(A) LENGTH: 176 amino acids
(B) TYPE: amino acid
(D) TOPOLOGY: linear
(xi) SEQUENCE DESCRIPTION: SEQ ID NO: 461:

Lys Asp Ser Lys Glu Tyr Gly His Thr Phe Arg Ser Asp Leu Arg Glu
1               5                   10                  15

Glu Ile Leu Met Leu Met Ala Arg Asp Lys His Pro Pro Glu Leu Gln
            20                  25                  30

Val Ala Phe Ala Asp Cys Ala Ala Asp Ile Lys Ser Ala Tyr Glu Ser
        35                  40                  45

Gln Pro Ile Arg Gln Thr Ala Gln Asp Trp Pro Ala Thr Ser Leu Asn
    50                  55                  60

Cys Ile Ala Ile Leu Phe Leu Arg Ala Gly Arg Thr Gln Glu Ala Trp
65                  70                  75                  80

Lys Met Leu Gly Leu Phe Arg Lys His Asn Lys Ile Pro Arg Ser Glu
                85                  90                  95

Leu Leu Asn Glu Leu Met Asp Ser Ala Lys Val Ser Asn Ser Pro Ser
            100                 105                 110

Gln Ala Ile Glu Val Val Glu Leu Ala Ser Ala Phe Ser Leu Pro Ile
        115                 120                 125

Cys Glu Gly Leu Thr Gln Arg Val Met Ser Asp Phe Ala Ile Asn Gln
    130                 135                 140

Glu Gln Lys Glu Ala Leu Ser Asn Leu Thr Ala Leu Thr Ser Asp Ser
145                 150                 155                 160

Asp Thr Asp Ser Ser Ser Asp Ser Asp Ser Asp Thr Ser Glu Gly Lys
                165                 170                 175

(2) INFORMATION FOR SEQ ID NO: 462:

(i) SEQUENCE CHARACTERISTICS:
(A) LENGTH: 324 amino acids
(B) TYPE: amino acid
(D) TOPOLOGY: linear
(xi) SEQUENCE DESCRIPTION: SEQ ID NO: 462:

(2) INFORMATION FOR SEQ ID NO: 463:

    (i) SEQUENCE CHARACTERISTICS:
        (A) LENGTH: 133 amino acids

(B) TYPE: amino acid
(D) TOPOLOGY: linear
(xi) SEQUENCE DESCRIPTION: SEQ ID NO: 463:

Ile Gln Asp Leu Gln Ser Arg Gln Lys His Glu Ile Glu Ser Leu Tyr
1               5                   10                  15

Thr Lys Leu Gly Lys Val Pro Pro Ala Val Ile Ile Pro Pro Ala Ala
            20                  25                  30

Pro Leu Ser Gly Arg Arg Arg Arg Pro Thr Lys Ser Lys Gly Ser Lys
        35                  40                  45

Ser Ser Arg Ser Ser Ser Leu Gly Asn Lys Ser Pro Gln Leu Ser Gly
    50                  55                  60

Asn Leu Ser Gly Gln Ser Ala Ala Ser Val Leu His Pro Gln Gln Thr
65                  70                  75                  80

Leu His Pro Pro Gly Asn Ile Pro Glu Ser Gly Gln Asn Gln Leu Leu
                85                  90                  95

Gln Pro Leu Lys Pro Ser Pro Ser Ser Asp Asn Leu Tyr Ser Ala Phe
            100                 105                 110

Thr Ser Asp Gly Ala Ile Ser Val Pro Ser Leu Ser Ala Pro Gly Gln
        115                 120                 125

Gly Thr Ser Ser Thr
    130

(2) INFORMATION FOR SEQ ID NO: 464:

(i) SEQUENCE CHARACTERISTICS:
(A) LENGTH: 53 amino acids
(B) TYPE: amino acid
(D) TOPOLOGY: linear
(xi) SEQUENCE DESCRIPTION: SEQ ID NO: 464:

Thr Ser Asp Gly Ala Ile Ser Val Pro Ser Leu Ser Ala Pro Gly Gln
1               5                   10                  15

Gly Thr Ser Ser Thr Asn Thr Val Gly Ala Thr Val Asn Ser Gln Ala
            20                  25                  30

Ala Gln Ala Gln Pro Pro Ala Met Thr Ser Ser Arg Lys Gly Thr Phe
        35                  40                  45

Thr Asp Asp Leu His
    50

(2) INFORMATION FOR SEQ ID NO: 465:

(i) SEQUENCE CHARACTERISTICS:
(A) LENGTH: 48 amino acids
(B) TYPE: amino acid

(D) TOPOLOGY: linear
(xi) SEQUENCE DESCRIPTION: SEQ ID NO: 465:

Lys Gly His Met Asn Tyr Glu Gly Pro Gly Met Ala Arg Lys Phe Ser
1               5                   10                  15

Ala Pro Gly Gln Leu Cys Ile Ser Met Thr Ser Asn Leu Gly Gly Ser
            20                  25                  30

Ala Pro Ile Ser Ala Ala Ser Ala Thr Ser Leu Gly His Phe Thr Lys
        35                  40                  45

(2) INFORMATION FOR SEQ ID NO: 466:

(i) SEQUENCE CHARACTERISTICS:
(A) LENGTH: 31 amino acids
(B) TYPE: amino acid
(D) TOPOLOGY: linear
(xi) SEQUENCE DESCRIPTION: SEQ ID NO: 466:

Gln Pro Leu Lys Pro Ser Pro Ser Ser Asp Asn Leu Tyr Ser Ala Phe
1               5                   10                  15

Thr Ser Asp Gly Ala Ile Ser Val Pro Ser Leu Ser Ala Pro Gly
            20                  25                  30

(2) INFORMATION FOR SEQ ID NO: 467:

(i) SEQUENCE CHARACTERISTICS:
(A) LENGTH: 57 amino acids
(B) TYPE: amino acid
(D) TOPOLOGY: linear
(xi) SEQUENCE DESCRIPTION: SEQ ID NO: 467:

Val Arg Val Ala Ala Ala Glu Ser Met Xaa Leu Leu Leu Glu Cys Ala
1               5                   10                  15

Xaa Val Arg Gly Pro Glu Tyr Leu Thr Gln Met Trp His Phe Met Cys
            20                  25                  30

Asp Ala Leu Ile Lys Ala Ile Gly Thr Glu Pro Asp Ser Asp Val Leu
        35                  40                  45

Ser Glu Ile Met His Ser Phe Ala Lys
    50                  55

(2) INFORMATION FOR SEQ ID NO: 468:

(i) SEQUENCE CHARACTERISTICS:
(A) LENGTH: 85 amino acids
(B) TYPE: amino acid

(D) TOPOLOGY: linear
(xi) SEQUENCE DESCRIPTION: SEQ ID NO: 468:

(2) INFORMATION FOR SEQ ID NO: 469:

(i) SEQUENCE CHARACTERISTICS:
(A) LENGTH: 20 amino acids
(B) TYPE: amino acid
(D) TOPOLOGY: linear
(xi) SEQUENCE DESCRIPTION: SEQ ID NO: 469:

Gln Asp Lys His Ala Glu Glu Val Arg Lys Asn Lys Glu Leu Lys Glu
1               5                   10                  15

Glu Ala Ser Arg
            20

(2) INFORMATION FOR SEQ ID NO: 470:

(i) SEQUENCE CHARACTERISTICS:
(A) LENGTH: 92 amino acids
(B) TYPE: amino acid
(D) TOPOLOGY: linear
(xi) SEQUENCE DESCRIPTION: SEQ ID NO: 470:

Gln Gln Asp Leu Ser Pro Trp Ala Ala Pro Val Gly Cys Pro Leu Xaa
1               5                   10                  15

Xaa Ala Ser Xaa Thr Cys His Xaa Leu Pro Leu Ser Gly Cys Leu Arg
            20                  25                  30

Arg Gln Ser Xaa Ser Leu Pro Val Val Ala Xaa Leu Cys Phe Trp Phe
        35                  40                  45

Ser Cys Pro Leu Ala Ser Leu Phe Val Pro Gly Gln Pro Cys Val Thr
    50                  55                  60

Cys Pro Phe Pro Ser Leu Pro Phe Gln Asp Lys His Ala Glu Glu Val

65                  70                  75                  80

Arg Lys Asn Lys Glu Leu Lys Glu Glu Ala Ser Arg
                85                  90

(2) INFORMATION FOR SEQ ID NO: 471:

      (i) SEQUENCE CHARACTERISTICS:
            (A) LENGTH: 37 amino acids
            (B) TYPE: amino acid
            (D) TOPOLOGY: linear
      (xi) SEQUENCE DESCRIPTION: SEQ ID NO: 471:

Pro Thr Arg Cys Cys Thr Thr Gln Pro Cys Arg Ser Ser Ala Arg Arg
1               5                   10                  15

Pro Cys Trp Val Pro Met Val Pro Ser Pro Glu Gly Arg Glu Xaa Gln
            20                  25                  30

Pro Thr Cys Pro Ser
        35

(2) INFORMATION FOR SEQ ID NO: 472:

      (i) SEQUENCE CHARACTERISTICS:
            (A) LENGTH: 363 amino acids
            (B) TYPE: amino acid
            (D) TOPOLOGY: linear
      (xi) SEQUENCE DESCRIPTION: SEQ ID NO: 472:

Met Lys Arg Ser Leu Asn Glu Asn Ser Ala Arg Ser Thr Ala Gly Cys
1               5                   10                  15

Leu Pro Val Pro Leu Phe Asn Gln Lys Lys Arg Asn Arg Gln Pro Leu
            20                  25                  30

Thr Ser Asn Pro Leu Lys Asp Asp Ser Gly Ile Ser Thr Pro Ser Asp
        35                  40                  45

Asn Tyr Asp Phe Pro Pro Leu Pro Thr Asp Trp Ala Trp Glu Ala Val
    50                  55                  60

Asn Pro Glu Xaa Ala Pro Val Met Lys Thr Val Asp Thr Gly Gln Ile
65                  70                  75                  80

Pro His Ser Val Ser Arg Pro Leu Arg Ser Gln Asp Ser Val Phe Asn
                85                  90                  95

Ser Ile Gln Ser Asn Thr Gly Arg Ser Gln Gly Gly Trp Ser Tyr Arg
            100                 105                 110

Asp Gly Asn Lys Asn Thr Ser Leu Lys Thr Trp Xaa Lys Asn Asp Phe
        115                 120                 125

Lys Pro Gln Cys Lys Arg Thr Asn Leu Val Ala Asn Asp Gly Lys Asn
    130                 135                 140

Ser Cys Pro Met Ser Ser Gly Ala Gln Gln Gln Lys Gln Leu Arg Thr
145                 150                 155                 160

Pro Glu Pro Pro Asn Leu Ser Arg Asn Lys Glu Thr Glu Leu Leu Arg
                165                 170                 175

Gln Thr His Ser Ser Lys Ile Ser Gly Cys Thr Met Arg Gly Leu Asp
            180                 185                 190

Lys Asn Ser Ala Leu Gln Thr Leu Lys Pro Asn Phe Gln Gln Asn Gln
        195                 200                 205

Tyr Lys Xaa Gln Met Leu Asp Asp Ile Pro Glu Asp Asn Thr Leu Lys
    210                 215                 220

Glu Thr Ser Leu Tyr Gln Leu Gln Phe Lys Glu Lys Ala Ser Ser Leu
225                 230                 235                 240

Arg Ile Ile Ser Ala Val Ile Glu Ser Met Lys Tyr Trp Arg Glu His
                245                 250                 255

Ala Gln Lys Thr Val Leu Leu Phe Glu Val Leu Ala Val Leu Asp Ser
            260                 265                 270

Ala Val Thr Pro Gly Pro Tyr Tyr Ser Lys Thr Phe Leu Met Arg Asp
        275                 280                 285

Gly Lys Asn Thr Leu Pro Cys Val Phe Tyr Glu Ile Asp Arg Glu Leu
    290                 295                 300

Pro Arg Leu Ile Arg Gly Arg Val His Arg Cys Val Gly Asn Tyr Asp
305                 310                 315                 320

Gln Lys Lys Asn Ile Phe Gln Cys Val Ser Val Arg Pro Ala Ser Val
                325                 330                 335

Ser Glu Gln Lys Thr Phe Gln Ala Phe Val Lys Ile Ala Asp Val Glu
            340                 345                 350

Met Gln Tyr Tyr Ile Asn Val Met Asn Glu Thr
        355                 360

(2) INFORMATION FOR SEQ ID NO: 473:

(i) SEQUENCE CHARACTERISTICS:
(A) LENGTH: 45 amino acids
(B) TYPE: amino acid
(D) TOPOLOGY: linear
(xi) SEQUENCE DESCRIPTION: SEQ ID NO: 473:

Ser Gln Asp Ser Val Phe Asn Ser Ile Gln Ser Asn Thr Gly Arg Ser
1               5                   10                  15

Gln Gly Gly Trp Ser Tyr Arg Asp Gly Asn Lys Asn Thr Ser Leu Lys
            20                  25                  30

Thr Trp Xaa Lys Asn Asp Phe Lys Pro Gln Cys Lys Arg

        35                  40                  45

(2) INFORMATION FOR SEQ ID NO: 474:

(i) SEQUENCE CHARACTERISTICS:
(A) LENGTH: 36 amino acids
(B) TYPE: amino acid
(D) TOPOLOGY: linear
(xi) SEQUENCE DESCRIPTION: SEQ ID NO: 474:

Asn Lys Glu Thr Glu Leu Leu Arg Gln Thr His Ser Ser Lys Ile Ser
1               5                   10                  15

Gly Cys Thr Met Arg Gly Leu Asp Lys Asn Ser Ala Leu Gln Thr Leu
            20                  25                  30

Lys Pro Asn Phe
        35

(2) INFORMATION FOR SEQ ID NO: 475:

(i) SEQUENCE CHARACTERISTICS:
(A) LENGTH: 49 amino acids
(B) TYPE: amino acid
(D) TOPOLOGY: linear
(xi) SEQUENCE DESCRIPTION: SEQ ID NO: 475:

Ser Ser Leu Arg Ile Ile Ser Ala Val Ile Glu Ser Met Lys Tyr Trp
1               5                   10                  15

Arg Glu His Ala Gln Lys Thr Val Leu Leu Phe Glu Val Leu Ala Val
            20                  25                  30

Leu Asp Ser Ala Val Thr Pro Gly Pro Tyr Tyr Ser Lys Thr Phe Leu
        35                  40                  45

Met

(2) INFORMATION FOR SEQ ID NO: 476:

(i) SEQUENCE CHARACTERISTICS:
(A) LENGTH: 42 amino acids
(B) TYPE: amino acid
(D) TOPOLOGY: linear
(xi) SEQUENCE DESCRIPTION: SEQ ID NO: 476:

Pro Arg Leu Ile Arg Gly Arg Val His Arg Cys Val Gly Asn Tyr Asp
1               5                   10                  15

Gln Lys Lys Asn Ile Phe Gln Cys Val Ser Val Arg Pro Ala Ser Val
            20                  25                  30

Ser Glu Gln Lys Thr Phe Gln Ala Phe Val

35                    40

(2) INFORMATION FOR SEQ ID NO: 477:

(i) SEQUENCE CHARACTERISTICS:
(A) LENGTH: 370 amino acids
(B) TYPE: amino acid
(D) TOPOLOGY: linear
(xi) SEQUENCE DESCRIPTION: SEQ ID NO: 477:

Gly Val Phe Arg Pro Cys Val Cys Gly Arg Pro Ala Ser Leu Thr Cys
1               5                   10                  15

Ser Pro Leu Asp Pro Glu Val Gly Pro Tyr Cys Asp Thr Pro Thr Met
            20                  25                  30

Arg Thr Leu Phe Asn Leu Leu Trp Leu Ala Leu Ala Cys Ser Pro Val
        35                  40                  45

His Thr Thr Leu Ser Lys Ser Asp Ala Lys Lys Ala Ala Ser Lys Thr
    50                  55                  60

Leu Leu Glu Lys Ser Gln Phe Ser Asp Lys Pro Val Gln Asp Arg Gly
65                  70                  75                  80

Leu Val Val Thr Asp Leu Lys Ala Glu Ser Val Val Leu Glu His Arg
                85                  90                  95

Ser Tyr Cys Ser Ala Lys Ala Arg Asp Arg His Phe Ala Gly Asp Val
            100                 105                 110

Leu Gly Tyr Val Thr Pro Trp Asn Ser His Gly Tyr Asp Val Thr Lys
        115                 120                 125

Val Phe Gly Ser Lys Phe Thr Gln Ile Ser Pro Val Trp Leu Gln Leu
    130                 135                 140

Lys Arg Arg Gly Arg Glu Met Phe Glu Val Thr Gly Leu His Asp Val
145                 150                 155                 160

Asp Gln Gly Trp Met Arg Ala Val Arg Lys His Ala Lys Gly Leu His
                165                 170                 175

Ile Val Pro Arg Leu Leu Phe Glu Asp Trp Thr Tyr Asp Asp Phe Arg
            180                 185                 190

Asn Val Leu Asp Ser Glu Asp Glu Ile Glu Glu Leu Ser Lys Thr Val
        195                 200                 205

Val Gln Val Ala Lys Asn Gln His Phe Asp Gly Phe Val Val Glu Val
    210                 215                 220

Trp Asn Gln Leu Leu Ser Gln Lys Arg Val Gly Leu Ile His Met Leu
225                 230                 235                 240

Thr His Leu Ala Glu Ala Leu His Gln Ala Arg Leu Leu Ala Leu Leu
                245                 250                 255

Val Ile Pro Pro Ala Ile Thr Pro Gly Thr Asp Gln Leu Gly Met Phe
            260                 265                 270

Thr His Lys Glu Phe Glu Gln Leu Ala Pro Val Leu Asp Gly Phe Ser
        275                 280                 285

Leu Met Thr Tyr Asp Tyr Ser Thr Ala His Gln Pro Gly Pro Asn Ala
    290                 295                 300

Pro Leu Ser Trp Val Arg Ala Cys Val Gln Val Leu Asp Pro Lys Xaa
305                 310                 315                 320

Lys Trp Arg Thr Lys Ser Ser Trp Gly Ser Thr Ser Met Xaa Trp Thr
                325                 330                 335

Xaa Arg Xaa Pro Xaa Asp Ala Arg Xaa Pro Val Val Gly Xaa Arg Xaa
            340                 345                 350

Ile Gln Xaa Leu Lys Asp His Xaa Pro Arg Met Val Leu Asp Ser Lys
        355                 360                 365

Pro Gln
    370

(2) INFORMATION FOR SEQ ID NO: 478:

(i) SEQUENCE CHARACTERISTICS:
(A) LENGTH: 39 amino acids
(B) TYPE: amino acid
(D) TOPOLOGY: linear
(xi) SEQUENCE DESCRIPTION: SEQ ID NO: 478:

Thr Cys Ser Pro Leu Asp Pro Glu Val Gly Pro Tyr Cys Asp Thr Pro
1               5                   10                  15

Thr Met Arg Thr Leu Phe Asn Leu Leu Trp Leu Ala Leu Ala Cys Ser
            20                  25                  30

Pro Val His Thr Thr Leu Ser
        35

(2) INFORMATION FOR SEQ ID NO: 479:

(i) SEQUENCE CHARACTERISTICS:
(A) LENGTH: 54 amino acids
(B) TYPE: amino acid
(D) TOPOLOGY: linear
(xi) SEQUENCE DESCRIPTION: SEQ ID NO: 479:

Leu Val Val Thr Asp Leu Lys Ala Glu Ser Val Val Leu Glu His Arg
1               5                   10                  15

Ser Tyr Cys Ser Ala Lys Ala Arg Asp Arg His Phe Ala Gly Asp Val
            20                  25                  30

Leu Gly Tyr Val Thr Pro Trp Asn Ser His Gly Tyr Asp Val Thr Lys

        35                  40                  45

Val Phe Gly Ser Lys Phe
    50

(2) INFORMATION FOR SEQ ID NO: 480:

(i) SEQUENCE CHARACTERISTICS:
(A) LENGTH: 52 amino acids
(B) TYPE: amino acid
(D) TOPOLOGY: linear
(xi) SEQUENCE DESCRIPTION: SEQ ID NO: 480:

Arg Glu Met Phe Glu Val Thr Gly Leu His Asp Val Asp Gln Gly Trp
1               5                   10                  15

Met Arg Ala Val Arg Lys His Ala Lys Gly Leu His Ile Val Pro Arg
            20                  25                  30

Leu Leu Phe Glu Asp Trp Thr Tyr Asp Asp Phe Arg Asn Val Leu Asp
        35                  40                  45

Ser Glu Asp Glu
    50

(2) INFORMATION FOR SEQ ID NO: 481:

(i) SEQUENCE CHARACTERISTICS:
(A) LENGTH: 56 amino acids
(B) TYPE: amino acid
(D) TOPOLOGY: linear
(xi) SEQUENCE DESCRIPTION: SEQ ID NO: 481:

His Phe Asp Gly Phe Val Val Glu Val Trp Asn Gln Leu Leu Ser Gln
1               5                   10                  15

Lys Arg Val Gly Leu Ile His Met Leu Thr His Leu Ala Glu Ala Leu
            20                  25                  30

His Gln Ala Arg Leu Leu Ala Leu Leu Val Ile Pro Pro Ala Ile Thr
        35                  40                  45

Pro Gly Thr Asp Gln Leu Gly Met
    50                  55

(2) INFORMATION FOR SEQ ID NO: 482:

(i) SEQUENCE CHARACTERISTICS:
(A) LENGTH: 47 amino acids
(B) TYPE: amino acid
(D) TOPOLOGY: linear
(xi) SEQUENCE DESCRIPTION: SEQ ID NO: 482:

Asp Gly Phe Ser Leu Met Thr Tyr Asp Tyr Ser Thr Ala His Gln Pro

1                   5                   10                  15

Gly Pro Asn Ala Pro Leu Ser Trp Val Arg Ala Cys Val Gln Val Leu
            20                  25                  30

Asp Pro Lys Xaa Lys Trp Arg Thr Lys Ser Ser Trp Gly Ser Thr
        35                  40                  45

(2) INFORMATION FOR SEQ ID NO: 483:

(i) SEQUENCE CHARACTERISTICS:
(A) LENGTH: 152 amino acids
(B) TYPE: amino acid
(D) TOPOLOGY: linear
(xi) SEQUENCE DESCRIPTION: SEQ ID NO: 483:

Glu Arg Gly Val Ser Ile Asn Gln Phe Cys Lys Glu Phe Asn Glu Arg
1               5                   10                  15

Thr Lys Asp Ile Lys Glu Gly Ile Pro Leu Pro Thr Lys Ile Leu Val
            20                  25                  30

Lys Pro Asp Arg Thr Phe Glu Ile Lys Ile Gly Gln Pro Thr Val Ser
        35                  40                  45

Tyr Phe Leu Lys Ala Ala Ala Gly Ile Glu Lys Gly Ala Arg Gln Thr
    50                  55                  60

Gly Lys Glu Val Ala Gly Leu Val Thr Leu Lys His Val Tyr Glu Ile
65                  70                  75                  80

Ala Arg Ile Lys Ala Gln Asp Glu Ala Phe Ala Leu Gln Asp Val Pro
                85                  90                  95

Leu Ser Ser Val Val Arg Ser Ile Ile Gly Ser Ala Arg Ser Leu Gly
            100                 105                 110

Ile Arg Val Val Lys Asp Leu Ser Ser Glu Glu Leu Ala Ala Phe Gln
        115                 120                 125

Lys Glu Arg Ala Ile Phe Leu Ala Ala Gln Lys Glu Ala Asp Leu Ala
    130                 135                 140

Ala Gln Glu Glu Ala Ala Lys Lys
145                 150

(2) INFORMATION FOR SEQ ID NO: 484:

(i) SEQUENCE CHARACTERISTICS:
(A) LENGTH: 270 amino acids
(B) TYPE: amino acid
(D) TOPOLOGY: linear
(xi) SEQUENCE DESCRIPTION: SEQ ID NO: 484:

Ala Val Tyr Thr Tyr His Glu Lys Lys Lys Asp Thr Ala Ala Ser Gly
1               5                   10                  15

Tyr Gly Thr Gln Asn Ile Arg Leu Ser Arg Asp Ala Val Lys Asp Phe
                20                  25                  30

Asp Cys Cys Cys Leu Ser Leu Gln Pro Cys His Asp Pro Val Val Thr
            35                  40                  45

Pro Asp Gly Tyr Leu Tyr Glu Arg Glu Ala Ile Leu Glu Tyr Ile Leu
    50                  55                  60

His Gln Lys Lys Glu Ile Ala Arg Gln Met Lys Ala Tyr Glu Lys Gln
65                  70                  75                  80

Arg Gly Thr Arg Arg Glu Glu Gln Lys Glu Leu Gln Arg Ala Ala Ser
                85                  90                  95

Gln Asp His Val Arg Gly Phe Leu Glu Lys Glu Ser Ala Ile Val Ser
            100                 105                 110

Arg Pro Leu Asn Pro Phe Thr Ala Lys Ala Leu Ser Gly Thr Ser Pro
        115                 120                 125

Asp Asp Val Gln Pro Gly Pro Ser Val Gly Pro Pro Ser Lys Asp Lys
    130                 135                 140

Asp Lys Val Leu Pro Ser Phe Trp Ile Pro Ser Leu Thr Pro Glu Ala
145                 150                 155                 160

Lys Ala Thr Lys Leu Glu Lys Pro Ser Arg Thr Val Thr Cys Pro Met
                165                 170                 175

Ser Gly Lys Pro Leu Arg Met Ser Asp Leu Thr Pro Val His Phe Thr
            180                 185                 190

Pro Leu Asp Ser Ser Val Asp Arg Val Gly Leu Ile Thr Arg Ser Glu
        195                 200                 205

Arg Tyr Val Cys Ala Val Thr Arg Asp Ser Leu Ser Asn Ala Thr Pro
    210                 215                 220

Cys Ala Val Leu Arg Pro Ser Gly Ala Val Val Thr Leu Glu Cys Val
225                 230                 235                 240

Glu Lys Leu Ile Arg Lys Asp Met Val Asp Pro Val Thr Gly Asp Lys
                245                 250                 255

Leu Thr Asp Arg Asp Ile Ile Val Leu Gln Arg Gly Gly Thr
            260                 265                 270

(2) INFORMATION FOR SEQ ID NO: 485:

     (i) SEQUENCE CHARACTERISTICS:
          (A) LENGTH: 54 amino acids
          (B) TYPE: amino acid
          (D) TOPOLOGY: linear
     (xi) SEQUENCE DESCRIPTION: SEQ ID NO: 485:

Tyr Leu Tyr Glu Arg Glu Ala Ile Leu Glu Tyr Ile Leu His Gln Lys

1                   5                   10                  15

Lys Glu Ile Ala Arg Gln Met Lys Ala Tyr Glu Lys Gln Arg Gly Thr
            20                  25                  30

Arg Arg Glu Glu Gln Lys Glu Leu Gln Arg Ala Ala Ser Gln Asp His
        35                  40                  45

Val Arg Gly Phe Leu Glu
    50

(2) INFORMATION FOR SEQ ID NO: 486:

(i) SEQUENCE CHARACTERISTICS:
(A) LENGTH: 64 amino acids
(B) TYPE: amino acid
(D) TOPOLOGY: linear
(xi) SEQUENCE DESCRIPTION: SEQ ID NO: 486:

Phe Thr Ala Lys Ala Leu Ser Gly Thr Ser Pro Asp Asp Val Gln Pro
1                   5                   10                  15

Gly Pro Ser Val Gly Pro Pro Ser Lys Asp Lys Asp Lys Val Leu Pro
            20                  25                  30

Ser Phe Trp Ile Pro Ser Leu Thr Pro Glu Ala Lys Ala Thr Lys Leu
        35                  40                  45

Glu Lys Pro Ser Arg Thr Val Thr Cys Pro Met Ser Gly Lys Pro Leu
    50                  55                  60

(2) INFORMATION FOR SEQ ID NO: 487:

(i) SEQUENCE CHARACTERISTICS:
(A) LENGTH: 56 amino acids
(B) TYPE: amino acid
(D) TOPOLOGY: linear
(xi) SEQUENCE DESCRIPTION: SEQ ID NO: 487:

Val His Phe Thr Pro Leu Asp Ser Ser Val Asp Arg Val Gly Leu Ile
1                   5                   10                  15

Thr Arg Ser Glu Arg Tyr Val Cys Ala Val Thr Arg Asp Ser Leu Ser
            20                  25                  30

Asn Ala Thr Pro Cys Ala Val Leu Arg Pro Ser Gly Ala Val Val Thr
        35                  40                  45

Leu Glu Cys Val Glu Lys Leu Ile
    50                  55

(2) INFORMATION FOR SEQ ID NO: 488:

(i) SEQUENCE CHARACTERISTICS:
(A) LENGTH: 567 amino acids
(B) TYPE: amino acid
(D) TOPOLOGY: linear
(xi) SEQUENCE DESCRIPTION: SEQ ID NO: 488:

Met Asp Thr Ser Glu Asn Arg Pro Glu Asn Asp Val Pro Glu Pro Pro
1               5                   10                  15

Met Pro Ile Ala Asp Gln Val Ser Asn Asp Asp Arg Pro Glu Gly Ser
            20                  25                  30

Val Glu Asp Glu Glu Lys Lys Glu Ser Ser Leu Pro Lys Ser Phe Lys
        35                  40                  45

Arg Lys Ile Ser Val Val Ser Ala Thr Lys Gly Val Pro Ala Gly Asn
    50                  55                  60

Ser Asp Thr Glu Gly Gly Gln Pro Gly Arg Lys Arg Arg Trp Gly Ala
65                  70                  75                  80

Ser Thr Ala Thr Thr Gln Lys Lys Pro Ser Ile Ser Ile Thr Thr Glu
                85                  90                  95

Ser Leu Lys Ser Leu Ile Pro Asp Ile Lys Pro Leu Ala Gly Gln Glu
            100                 105                 110

Ala Val Val Asp Leu His Ala Asp Asp Ser Arg Ile Ser Glu Asp Glu
        115                 120                 125

Thr Glu Arg Asn Gly Asp Asp Gly Thr His Asp Lys Gly Leu Lys Ile
    130                 135                 140

Cys Arg Thr Val Thr Gln Val Val Pro Ala Glu Gly Gln Glu Asn Gly
145                 150                 155                 160

Gln Arg Glu Glu Glu Glu Glu Glu Lys Glu Pro Glu Ala Glu Pro Pro
                165                 170                 175

Val Pro Pro Gln Val Ser Val Glu Val Ala Leu Pro Pro Pro Ala Glu
            180                 185                 190

His Glu Val Lys Lys Val Thr Leu Gly Asp Thr Leu Thr Arg Arg Ser
        195                 200                 205

Ile Ser Gln Gln Lys Ser Gly Val Ser Ile Thr Ile Asp Asp Pro Val
    210                 215                 220

Arg Thr Ala Gln Val Pro Ser Pro Pro Arg Gly Lys Ile Ser Asn Ile
225                 230                 235                 240

Val His Ile Ser Asn Leu Val Arg Pro Phe Thr Leu Gly Gln Leu Lys
                245                 250                 255

Glu Leu Leu Gly Arg Thr Gly Thr Leu Val Glu Glu Ala Phe Trp Ile
            260                 265                 270

Asp Lys Ile Lys Ser His Cys Phe Val Thr Tyr Ser Thr Val Glu Glu

        275                 280                 285

Ala Val Ala Thr Arg Thr Ala Leu His Gly Val Lys Trp Pro Gln Ser
    290                 295                 300

Asn Pro Lys Phe Leu Cys Ala Asp Tyr Ala Glu Gln Asp Glu Leu Asp
305                 310                 315                 320

Tyr His Arg Gly Leu Leu Val Asp Arg Pro Ser Glu Thr Lys Thr Glu
                325                 330                 335

Glu Gln Gly Ile Pro Arg Pro Leu His Pro Pro Pro Pro Pro Pro Val
            340                 345                 350

Gln Pro Pro Gln His Pro Arg Ala Glu Gln Arg Glu Gln Glu Arg Ala
        355                 360                 365

Val Arg Glu Gln Trp Ala Glu Arg Glu Arg Glu Met Glu Arg Arg Glu
    370                 375                 380

Arg Thr Arg Ser Glu Arg Glu Trp Asp Arg Asp Lys Val Arg Glu Gly
385                 390                 395                 400

Pro Arg Ser Arg Ser Arg Ser Arg Xaa Arg Arg Arg Lys Glu Arg Ala
                405                 410                 415

Lys Ser Lys Glu Lys Lys Ser Glu Lys Lys Glu Lys Ala Gln Glu Glu
            420                 425                 430

Pro Pro Ala Lys Leu Leu Asp Asp Leu Phe Arg Lys Thr Lys Ala Ala
        435                 440                 445

Pro Cys Ile Tyr Trp Leu Pro Leu Thr Asp Ser Gln Ile Val Gln Lys
    450                 455                 460

Glu Ala Glu Arg Ala Glu Arg Ala Lys Glu Arg Glu Lys Arg Arg Lys
465                 470                 475                 480

Glu Gln Glu Glu Glu Glu Gln Lys Glu Arg Glu Lys Glu Ala Glu Arg
                485                 490                 495

Glu Arg Asn Arg Gln Leu Glu Arg Glu Lys Arg Arg Glu His Ser Arg
            500                 505                 510

Glu Arg Asp Arg Glu Arg Glu Arg Glu Arg Glu Arg Asp Arg Gly Asp
        515                 520                 525

Arg Asp Arg Asp Arg Glu Arg Asp Arg Glu Arg Gly Arg Glu Arg Asp
    530                 535                 540

Arg Arg Asp Thr Lys Arg His Ser Arg Ser Arg Ser Arg Ser Thr Pro
545                 550                 555                 560

Val Arg Asp Arg Gly Gly Arg
                565

(2) INFORMATION FOR SEQ ID NO: 489:

(i) SEQUENCE CHARACTERISTICS:
(A) LENGTH: 51 amino acids
(B) TYPE: amino acid
(D) TOPOLOGY: linear
(xi) SEQUENCE DESCRIPTION: SEQ ID NO: 489:

Gly Cys Asp Ser Cys Pro Pro His Leu Pro Arg Glu Ala Phe Ala Gln
1               5                   10                  15

Asp Thr Gln Ala Glu Gly Glu Cys Ser Ser Arg Ala Glu Arg Ala Asp
            20                  25                  30

Met Cys Pro Asp Ala Pro Pro Ser Gln Glu Val Pro Glu Gly Pro Gly
        35                  40                  45

Ala Ala Pro
    50

(2) INFORMATION FOR SEQ ID NO: 490:

(i) SEQUENCE CHARACTERISTICS:
(A) LENGTH: 50 amino acids
(B) TYPE: amino acid
(D) TOPOLOGY: linear
(xi) SEQUENCE DESCRIPTION: SEQ ID NO: 490:

Pro Gln Leu Pro Ser Cys Gly Arg Pro Trp Pro Gly Thr Ala Ser Val
1               5                   10                  15

Phe Gln Ser His Thr Gln Gly Pro Arg Glu Asp Pro Asp Pro Cys Arg
            20                  25                  30

Ala Gln Gly Ser Ala Gly Thr His Cys Pro Ile Ser Leu Ser Pro Pro
        35                  40                  45

Arg Gln
    50

(2) INFORMATION FOR SEQ ID NO: 491:

(i) SEQUENCE CHARACTERISTICS:
(A) LENGTH: 42 amino acids
(B) TYPE: amino acid
(D) TOPOLOGY: linear
(xi) SEQUENCE DESCRIPTION: SEQ ID NO: 491:

Pro Gly Phe Arg Gly Pro Ser Gly Ser Leu Gly Cys Ser Phe Phe Pro
1               5                   10                  15

Arg Ser Leu Gly Arg Val Leu Pro Pro Gly Cys Gln Arg Pro Gly Ala
            20                  25                  30

His Ala Asp Ser Ser Pro Pro Pro Thr Pro
        35                  40

(2) INFORMATION FOR SEQ ID NO: 492:

(i) SEQUENCE CHARACTERISTICS:
(A) LENGTH: 84 amino acids
(B) TYPE: amino acid
(D) TOPOLOGY: linear
(xi) SEQUENCE DESCRIPTION: SEQ ID NO: 492:

Glu Asp Leu Lys Lys Pro Asp Pro Ala Ser Leu Arg Ala Ala Ser Cys
1               5                   10                  15

Gly Glu Gly Lys Lys Arg Lys Ala Cys Lys Asn Cys Thr Cys Gly Leu
            20                  25                  30

Ala Glu Glu Leu Glu Lys Glu Lys Ser Arg Glu Gln Met Ser Ser Gln
        35                  40                  45

Pro Lys Ser Ala Cys Gly Asn Cys Tyr Leu Gly Asp Ala Phe Arg Cys
    50                  55                  60

Ala Ser Cys Pro Tyr Leu Gly Met Pro Ala Phe Lys Pro Gly Glu Lys
65                  70                  75                  80

Val Leu Leu Ser

(2) INFORMATION FOR SEQ ID NO: 493:

(i) SEQUENCE CHARACTERISTICS:
(A) LENGTH: 90 amino acids
(B) TYPE: amino acid
(D) TOPOLOGY: linear
(xi) SEQUENCE DESCRIPTION: SEQ ID NO: 493:

Glu Asp Leu Lys Lys Pro Asp Pro Ala Ser Leu Arg Ala Ala Ser Cys
1               5                   10                  15

Gly Glu Gly Lys Lys Arg Lys Ala Cys Lys Asn Cys Thr Cys Gly Leu
            20                  25                  30

Ala Glu Glu Leu Glu Lys Glu Lys Ser Arg Glu Gln Met Ser Ser Gln
        35                  40                  45

Pro Lys Ser Ala Cys Gly Asn Cys Tyr Leu Gly Asp Ala Phe Arg Cys
    50                  55                  60

Ala Ser Cys Pro Tyr Leu Gly Met Pro Ala Phe Lys Pro Gly Glu Lys
65                  70                  75                  80

Val Leu Leu Ser Asp Ser Asn Leu His Asp
                85                  90

(2) INFORMATION FOR SEQ ID NO: 494:

(i) SEQUENCE CHARACTERISTICS:

(A) LENGTH: 34 amino acids
(B) TYPE: amino acid
(D) TOPOLOGY: linear
(xi) SEQUENCE DESCRIPTION: SEQ ID NO: 494:

Cys Gly Asn Cys Tyr Leu Gly Asp Ala Phe Arg Cys Ala Ser Cys Pro
1               5                   10                  15

Tyr Leu Gly Met Pro Ala Phe Lys Pro Gly Glu Lys Val Leu Leu Ser
            20                  25                  30

Asp Ser

(2) INFORMATION FOR SEQ ID NO: 495:

(i) SEQUENCE CHARACTERISTICS:
(A) LENGTH: 25 amino acids
(B) TYPE: amino acid
(D) TOPOLOGY: linear
(xi) SEQUENCE DESCRIPTION: SEQ ID NO: 495:

Ser Cys Gly Glu Gly Lys Lys Arg Lys Ala Cys Lys Asn Cys Thr Cys
1               5                   10                  15

Gly Leu Ala Glu Glu Leu Glu Lys Glu
            20                  25

(2) INFORMATION FOR SEQ ID NO: 496:

(i) SEQUENCE CHARACTERISTICS:
(A) LENGTH: 21 amino acids
(B) TYPE: amino acid
(D) TOPOLOGY: linear
(xi) SEQUENCE DESCRIPTION: SEQ ID NO: 496:

Ser Gln Pro Lys Ser Ala Cys Gly Asn Cys Tyr Leu Gly Asp Ala Phe
1               5                   10                  15

Arg Cys Ala Ser Cys
            20

(2) INFORMATION FOR SEQ ID NO: 497:

(i) SEQUENCE CHARACTERISTICS:
(A) LENGTH: 17 amino acids
(B) TYPE: amino acid
(D) TOPOLOGY: linear
(xi) SEQUENCE DESCRIPTION: SEQ ID NO: 497:

Arg Glu Ala Gly Gln Asn Ser Glu Arg Gln Tyr Val Ser Leu Ser Arg
1               5                   10                  15

Asp

(2) INFORMATION FOR SEQ ID NO: 498:

(i) SEQUENCE CHARACTERISTICS:
(A) LENGTH: 90 amino acids
(B) TYPE: amino acid
(D) TOPOLOGY: linear
(xi) SEQUENCE DESCRIPTION: SEQ ID NO: 498:

Glu Ser Ser Gly Gln Ala Arg Thr Leu Ala Asp Pro Gly Pro Gly Trp
1               5                   10                  15

Pro Arg Gln Gln Gly Met Cys Phe Gly Ser Leu Thr Gly Leu Ser Thr
            20                  25                  30

Thr Pro His Gly Phe Leu Thr Val Ser Ala Glu Ala Asp Pro Arg Leu
        35                  40                  45

Ile Glu Ser Leu Ser Gln Met Leu Ser Met Gly Phe Ser Asp Glu Gly
    50                  55                  60

Gly Trp Leu Thr Arg Leu Leu Gln Thr Lys Asn Tyr Asp Ile Gly Ala
65                  70                  75                  80

Ala Leu Asp Thr Ile Gln Tyr Ser Lys His
                85                  90

(2) INFORMATION FOR SEQ ID NO: 499:

(i) SEQUENCE CHARACTERISTICS:
(A) LENGTH: 159 amino acids
(B) TYPE: amino acid
(D) TOPOLOGY: linear
(xi) SEQUENCE DESCRIPTION: SEQ ID NO: 499:

Gln Glu Gly Ser Glu Pro Val Leu Leu Glu Gly Glu Cys Leu Val Val
1               5                   10                  15

Cys Glu Pro Gly Arg Ala Ala Ala Gly Gly Pro Gly Gly Ala Ala Leu
            20                  25                  30

Gly Glu Ala Pro Pro Gly Arg Val Ala Phe Xaa Ala Val Arg Ser His
        35                  40                  45

His His Glu Pro Ala Gly Glu Thr Gly Asn Gly Thr Ser Gly Ala Ile
    50                  55                  60

Tyr Phe Asp Gln Val Leu Val Asn Glu Gly Gly Gly Phe Asp Arg Ala
65                  70                  75                  80

Ser Gly Ser Phe Val Ala Pro Val Arg Gly Val Tyr Ser Phe Arg Phe
                85                  90                  95

His Val Val Lys Val Tyr Asn Arg Gln Thr Val Gln Val Ser Leu Met
            100                 105                 110

Leu Asn Thr Trp Pro Val Ile Ser Ala Phe Ala Asn Asp Pro Asp Val
        115                 120                 125

Thr Arg Glu Ala Ala Thr Ser Ser Val Leu Leu Pro Leu Asp Pro Gly
    130                 135                 140

Asp Arg Val Ser Leu Arg Leu Arg Arg Gly Xaa Ser Thr Gly Trp
145                 150                 155

(2) INFORMATION FOR SEQ ID NO: 500:

        (i) SEQUENCE CHARACTERISTICS:
                (A) LENGTH: 32 amino acids
                (B) TYPE: amino acid
                (D) TOPOLOGY: linear
        (xi) SEQUENCE DESCRIPTION: SEQ ID NO: 500:

Pro Arg Ser Arg Pro Ala Leu Arg Pro Gly Arg Gln Arg Pro Pro Ser
  1               5                  10                  15

His Ser Ala Thr Ser Gly Val Leu Arg Pro Arg Lys Lys Pro Asp Pro
             20                  25                  30

(2) INFORMATION FOR SEQ ID NO: 501:

        (i) SEQUENCE CHARACTERISTICS:
                (A) LENGTH: 31 amino acids
                (B) TYPE: amino acid
                (D) TOPOLOGY: linear
        (xi) SEQUENCE DESCRIPTION: SEQ ID NO: 501:

Met Thr Leu Ile Thr Pro Ser Xaa Lys Leu Thr Phe Xaa Lys Gly Asn
  1               5                  10                  15

Lys Ser Trp Ser Ser Arg Ala Cys Ser Ser Thr Leu Val Asp Pro
             20                  25                  30

(2) INFORMATION FOR SEQ ID NO: 502:

        (i) SEQUENCE CHARACTERISTICS:
                (A) LENGTH: 51 amino acids
                (B) TYPE: amino acid
                (D) TOPOLOGY: linear
        (xi) SEQUENCE DESCRIPTION: SEQ ID NO: 502:

Gly His Pro Ser Pro Ala Leu Ser Ile Ala Pro Ser Asp Gly Ser Gln
  1               5                  10                  15

Leu Pro Cys Asp Glu Val Pro Tyr Gly Glu Ala His Val Thr Arg Tyr
             20                  25                  30

Cys Lys Lys Pro Leu Thr Asn Ser His Leu Glu Thr Glu Ala Gln Ser
        35                  40                  45

Ser Ser Leu
    50

(2) INFORMATION FOR SEQ ID NO: 503:

        (i) SEQUENCE CHARACTERISTICS:
                (A) LENGTH: 263 base pairs
                (B) TYPE: nucleic acid
                (C) STRANDEDNESS: double
                (D) TOPOLOGY: linear

        (xi) SEQUENCE DESCRIPTION: SEQ ID NO: 503:

GCTTCGTGTC CAACCCTCTT GCCCTTCGCC TGTGTGCCTG GAGCCAGTCC CACCACGCTC        60

GCGTTTCCTC CTGTAGTGCT CACAGGTCCC AGCACCGATG GCATTCCCTT TGCCCTGAGT       120

CTGCAGCGGG TCCCTTTTGT GCTTCCTTCC CCTCAGGTAG CCTCTCTCCC CCTGGGCCAC       180

TCCCGGGGGT GAGGGGGTTA CCCCTTCCCA GTGTTTTTTA TTCCTGTGGG GCTCACCCCA       240

AAGTATTAAA AGTAGCTTTG TAA                                               263

(2) INFORMATION FOR SEQ ID NO: 504:

        (i) SEQUENCE CHARACTERISTICS:
                (A) LENGTH: 263 base pairs
                (B) TYPE: nucleic acid
                (C) STRANDEDNESS: double
                (D) TOPOLOGY: linear

        (xi) SEQUENCE DESCRIPTION: SEQ ID NO: 504:

GCTTCGTGTC CAACCCTCTT GCCCTTCGCC TGTGTGCCTG GAGCCAGTCC CACCACGCTC        60

GCGTTTCCTC CTGTAGTGCT CACAGGTCCC AGCACCGATG GCATTCCCTT TGCCCTGAGT       120

CTGCAGCGGG TCCCTTTTGT GCTTCCTTCC CCTCAGGTAG CCTCTCTCCC CCTGGGCCAC       180

TCCCGGGGGT GAGGGGGTTA CCCCTTCCCA GTGTTTTTTA TTCCTGTGGG GCTCACCCCA       240

AAGTATTAAA AGTAGCTTTG TAA                                               263

(2) INFORMATION FOR SEQ ID NO: 505:

        (i) SEQUENCE CHARACTERISTICS:
                (A) LENGTH: 263 base pairs
                (B) TYPE: nucleic acid

(C) STRANDEDNESS: double
(D) TOPOLOGY: linear

(xi) SEQUENCE DESCRIPTION: SEQ ID NO: 505:

GCTTCGTGTC CAACCCTCTT GCCCTTCGCC TGTGTGCCTG GAGCCAGTCC CACCACGCTC 60

GCGTTTCCTC CTGTAGTGCT CACAGGTCCC AGCACCGATG GCATTCCCTT TGCCCTGAGT 120

CTGCAGCGGG TCCCTTTTGT GCTTCCTTCC CCTCAGGTAG CCTCTCTCCC CCTGGGCCAC 180

TCCCGGGGGT GAGGGGGTTA CCCCTTCCCA GTGTTTTTTA TTCCTGTGGG GCTCACCCCA 240

AAGTATTAAA AGTAGCTTTG TAA 263

(2) INFORMATION FOR SEQ ID NO: 506:

(i) SEQUENCE CHARACTERISTICS:
(A) LENGTH: 160 base pairs
(B) TYPE: nucleic acid
(C) STRANDEDNESS: double
(D) TOPOLOGY: linear

(xi) SEQUENCE DESCRIPTION: SEQ ID NO: 506:

TGGCTCACTG TCTTACAATC ACTGCTGTGG AATCATGATA CCACTTTTAG CTCTTTGCAT 60

CTTCCTTCAG TGTATTTTTG TTTTTCAAGA GGAAGTAGAT TTTAACTGGA CAACTTTGAG 120

TACTGACATC ATTGATAAAT AAACTGGCTT GTGGTTTCAA 160

(2) INFORMATION FOR SEQ ID NO: 507:

(i) SEQUENCE CHARACTERISTICS:
(A) LENGTH: 292 amino acids
(B) TYPE: amino acid
(D) TOPOLOGY: linear
(xi) SEQUENCE DESCRIPTION: SEQ ID NO: 507:

Leu Asp Glu Leu Met Ala His Leu Thr Glu Met Gln Ala Lys Val Ala
1               5                   10                  15

Val Arg Ala Asp Ala Gly Lys Lys His Leu Pro Asp Lys Gln Asp His
            20                  25                  30

Lys Ala Ser Leu Asp Ser Met Leu Gly Gly Leu Glu Gln Glu Leu Gln
        35                  40                  45

Asp Leu Gly Ile Ala Thr Val Pro Lys Gly His Cys Ala Ser Cys Gln
    50                  55                  60

Lys Pro Ile Ala Gly Lys Val Ile His Ala Leu Gly Gln Ser Trp His
65                  70                  75                  80

Pro Glu His Phe Val Cys Thr His Cys Lys Glu Glu Ile Gly Ser Ser
                85                  90                  95

Pro Phe Phe Glu Arg Ser Gly Leu Xaa Tyr Cys Pro Asn Asp Tyr His
            100                 105                 110

Gln Leu Phe Ser Pro Arg Cys Ala Tyr Cys Ala Ala Pro Ile Leu Asp
        115                 120                 125

Lys Val Leu Thr Ala Met Asn Gln Thr Trp His Pro Glu His Phe Phe
    130                 135                 140

Cys Ser His Cys Gly Glu Val Phe Gly Ala Glu Gly Phe His Glu Lys
145                 150                 155                 160

Asp Lys Lys Pro Tyr Cys Arg Lys Asp Phe Leu Ala Met Phe Ser Pro
                165                 170                 175

Lys Cys Gly Gly Cys Asn Arg Pro Val Leu Glu Asn Tyr Leu Ser Ala
            180                 185                 190

Met Asp Thr Val Trp His Pro Glu Cys Phe Val Cys Gly Asp Cys Phe
        195                 200                 205

Thr Ser Phe Ser Thr Gly Ser Phe Phe Glu Leu Asp Gly Arg Pro Phe
    210                 215                 220

Cys Glu Leu His Tyr His His Arg Arg Gly Thr Leu Cys His Gly Cys
225                 230                 235                 240

Gly Gln Pro Ile Thr Gly Arg Cys Ile Ser Ala Met Gly Tyr Lys Phe
                245                 250                 255

His Pro Glu His Phe Val Cys Ala Phe Cys Leu Thr Gln Leu Ser Lys
            260                 265                 270

Gly Ile Phe Arg Glu Gln Asn Asp Lys Thr Tyr Cys Gln Pro Cys Phe
        275                 280                 285

Asn Lys Leu Phe
    290

(2) INFORMATION FOR SEQ ID NO: 508:

        (i) SEQUENCE CHARACTERISTICS:
                (A) LENGTH: 43 amino acids
                (B) TYPE: amino acid
                (D) TOPOLOGY: linear
        (xi) SEQUENCE DESCRIPTION: SEQ ID NO: 508:

Lys Ala Ser Leu Asp Ser Met Leu Gly Gly Leu Glu Gln Glu Leu Gln
1               5                   10                  15

Asp Leu Gly Ile Ala Thr Val Pro Lys Gly His Cys Ala Ser Cys Gln
            20                  25                  30

Lys Pro Ile Ala Gly Lys Val Ile His Ala Leu
        35                  40

(2) INFORMATION FOR SEQ ID NO: 509:

(i) SEQUENCE CHARACTERISTICS:
(A) LENGTH: 50 amino acids
(B) TYPE: amino acid
(D) TOPOLOGY: linear
(xi) SEQUENCE DESCRIPTION: SEQ ID NO: 509:

Cys Pro Asn Asp Tyr His Gln Leu Phe Ser Pro Arg Cys Ala Tyr Cys
1               5                   10                  15

Ala Ala Pro Ile Leu Asp Lys Val Leu Thr Ala Met Asn Gln Thr Trp
            20                  25                  30

His Pro Glu His Phe Phe Cys Ser His Cys Gly Glu Val Phe Gly Ala
        35                  40                  45

Glu Gly
    50

(2) INFORMATION FOR SEQ ID NO: 510:

(i) SEQUENCE CHARACTERISTICS:
(A) LENGTH: 67 amino acids
(B) TYPE: amino acid
(D) TOPOLOGY: linear
(xi) SEQUENCE DESCRIPTION: SEQ ID NO: 510:

Asp Lys Lys Pro Tyr Cys Arg Lys Asp Phe Leu Ala Met Phe Ser Pro
1               5                   10                  15

Lys Cys Gly Gly Cys Asn Arg Pro Val Leu Glu Asn Tyr Leu Ser Ala
            20                  25                  30

Met Asp Thr Val Trp His Pro Glu Cys Phe Val Cys Gly Asp Cys Phe
        35                  40                  45

Thr Ser Phe Ser Thr Gly Ser Phe Phe Glu Leu Asp Gly Arg Pro Phe
    50                  55                  60

Cys Glu Leu
65

(2) INFORMATION FOR SEQ ID NO: 511:

(i) SEQUENCE CHARACTERISTICS:
(A) LENGTH: 46 amino acids
(B) TYPE: amino acid
(D) TOPOLOGY: linear
(xi) SEQUENCE DESCRIPTION: SEQ ID NO: 511:

Cys Gly Gln Pro Ile Thr Gly Arg Cys Ile Ser Ala Met Gly Tyr Lys
1               5                   10                  15

Phe His Pro Glu His Phe Val Cys Ala Phe Cys Leu Thr Gln Leu Ser
            20                  25                  30

Lys Gly Ile Phe Arg Glu Gln Asn Asp Lys Thr Tyr Cys Gln
        35                  40                  45

(2) INFORMATION FOR SEQ ID NO: 512:

      (i) SEQUENCE CHARACTERISTICS:
            (A) LENGTH: 452 amino acids
            (B) TYPE: amino acid
            (D) TOPOLOGY: linear
      (xi) SEQUENCE DESCRIPTION: SEQ ID NO: 512:

Met Gly Ser Ser Gln Ser Val Glu Ile Pro Gly Gly Gly Thr Glu Gly
  1               5                  10                  15

Tyr His Val Leu Arg Val Gln Glu Asn Ser Pro Gly His Arg Ala Gly
            20                  25                  30

Leu Glu Pro Phe Phe Asp Phe Ile Val Ser Ile Asn Gly Ser Arg Leu
        35                  40                  45

Asn Lys Asp Asn Asp Thr Leu Lys Asp Leu Leu Lys Xaa Asn Val Glu
    50                  55                  60

Lys Pro Val Lys Met Leu Ile Tyr Ser Ser Lys Thr Leu Glu Leu Arg
 65                  70                  75                  80

Glu Thr Ser Val Thr Pro Ser Asn Leu Trp Gly Gly Gln Gly Leu Leu
                 85                  90                  95

Gly Val Ser Ile Arg Phe Cys Ser Phe Asp Gly Ala Asn Glu Asn Val
            100                 105                 110

Trp His Val Leu Glu Val Glu Ser Asn Ser Pro Ala Ala Leu Ala Gly
        115                 120                 125

Leu Arg Pro His Ser Asp Tyr Ile Ile Gly Ala Asp Thr Val Met Asn
    130                 135                 140

Glu Ser Glu Asp Leu Phe Ser Leu Ile Glu Thr His Glu Ala Lys Pro
145                 150                 155                 160

Leu Lys Leu Tyr Val Tyr Asn Thr Asp Thr Asp Asn Cys Arg Glu Val
                165                 170                 175

Ile Ile Thr Pro Asn Ser Ala Trp Gly Gly Glu Gly Ser Leu Gly Cys
            180                 185                 190

Gly Ile Gly Tyr Gly Tyr Leu His Arg Ile Pro Thr Arg Pro Phe Glu
        195                 200                 205

Glu Gly Lys Lys Ile Ser Leu Pro Gly Gln Met Ala Gly Thr Pro Ile
    210                 215                 220

Thr Pro Leu Lys Asp Gly Phe Thr Glu Val Gln Leu Ser Ser Val Asn

225                 230                 235                 240

Pro Pro Ser Leu Ser Pro Pro Gly Thr Thr Gly Ile Glu Gln Ser Leu
                245                 250                 255

Thr Gly Leu Ser Ile Ser Ser Thr Pro Pro Ala Val Ser Ser Val Leu
            260                 265                 270

Ser Thr Gly Val Pro Thr Val Pro Leu Leu Pro Pro Gln Val Asn Gln
        275                 280                 285

Ser Leu Thr Ser Val Pro Pro Met Asn Pro Ala Thr Thr Leu Pro Gly
    290                 295                 300

Leu Met Pro Leu Pro Ala Gly Leu Pro Asn Leu Pro Asn Leu Asn Leu
305                 310                 315                 320

Asn Leu Pro Ala Pro His Ile Met Pro Gly Val Gly Leu Pro Glu Leu
                325                 330                 335

Val Asn Pro Gly Leu Pro Pro Leu Pro Ser Met Pro Pro Arg Asn Leu
            340                 345                 350

Pro Gly Ile Ala Pro Leu Pro Leu Pro Ser Glu Phe Leu Pro Ser Phe
        355                 360                 365

Pro Leu Val Pro Glu Ser Ser Ser Ala Ala Ser Ser Gly Glu Leu Leu
    370                 375                 380

Ser Ser Leu Pro Pro Thr Ser Asn Ala Pro Ser Asp Pro Ala Thr Thr
385                 390                 395                 400

Thr Ala Lys Ala Asp Ala Ala Ser Ser Leu Thr Val Asp Val Thr Pro
                405                 410                 415

Pro Thr Ala Lys Ala Pro Thr Thr Val Glu Asp Arg Val Gly Asp Ser
            420                 425                 430

Thr Pro Val Ser Glu Lys Pro Val Ser Ala Ala Val Asp Ala Asn Ala
        435                 440                 445

Ser Glu Ser Pro
    450

(2) INFORMATION FOR SEQ ID NO: 513:

(i) SEQUENCE CHARACTERISTICS:
(A) LENGTH: 109 amino acids
(B) TYPE: amino acid
(D) TOPOLOGY: linear
(xi) SEQUENCE DESCRIPTION: SEQ ID NO: 513:

Ser Val Glu Ile Pro Gly Gly Gly Thr Glu Gly Tyr His Val Leu Arg
 1               5                  10                  15

Val Gln Glu Asn Ser Pro Gly His Arg Ala Gly Leu Glu Pro Phe Phe
            20                  25                  30

Asp Phe Ile Val Ser Ile Asn Gly Ser Arg Leu Asn Lys Asp Asn Asp
        35                  40                  45

Thr Leu Lys Asp Leu Leu Lys Xaa Asn Val Glu Lys Pro Val Lys Met
    50                  55                  60

Leu Ile Tyr Ser Ser Lys Thr Leu Glu Leu Arg Glu Thr Ser Val Thr
65                  70                  75                  80

Pro Ser Asn Leu Trp Gly Gly Gln Gly Leu Leu Gly Val Ser Ile Arg
                85                  90                  95

Phe Cys Ser Phe Asp Gly Ala Asn Glu Asn Val Trp His
            100                 105

(2) INFORMATION FOR SEQ ID NO: 514:

(i) SEQUENCE CHARACTERISTICS:
(A) LENGTH: 145 amino acids
(B) TYPE: amino acid
(D) TOPOLOGY: linear
(xi) SEQUENCE DESCRIPTION: SEQ ID NO: 514:

Glu Ser Asn Ser Pro Ala Ala Leu Ala Gly Leu Arg Pro His Ser Asp
1               5                   10                  15

Tyr Ile Ile Gly Ala Asp Thr Val Met Asn Glu Ser Glu Asp Leu Phe
            20                  25                  30

Ser Leu Ile Glu Thr His Glu Ala Lys Pro Leu Lys Leu Tyr Val Tyr
        35                  40                  45

Asn Thr Asp Thr Asp Asn Cys Arg Glu Val Ile Ile Thr Pro Asn Ser
    50                  55                  60

Ala Trp Gly Gly Glu Gly Ser Leu Gly Cys Gly Ile Gly Tyr Gly Tyr
65                  70                  75                  80

Leu His Arg Ile Pro Thr Arg Pro Phe Glu Glu Gly Lys Lys Ile Ser
                85                  90                  95

Leu Pro Gly Gln Met Ala Gly Thr Pro Ile Thr Pro Leu Lys Asp Gly
            100                 105                 110

Phe Thr Glu Val Gln Leu Ser Ser Val Asn Pro Pro Ser Leu Ser Pro
        115                 120                 125

Pro Gly Thr Thr Gly Ile Glu Gln Ser Leu Thr Gly Leu Ser Ile Ser
    130                 135                 140

Ser
145

(2) INFORMATION FOR SEQ ID NO: 515:

(i) SEQUENCE CHARACTERISTICS:

(A) LENGTH: 145 amino acids
(B) TYPE: amino acid
(D) TOPOLOGY: linear
(xi) SEQUENCE DESCRIPTION: SEQ ID NO: 515:

Glu Ser Asn Ser Pro Ala Ala Leu Ala Gly Leu Arg Pro His Ser Asp
1               5                   10                  15

Tyr Ile Ile Gly Ala Asp Thr Val Met Asn Glu Ser Glu Asp Leu Phe
            20                  25                  30

Ser Leu Ile Glu Thr His Glu Ala Lys Pro Leu Lys Leu Tyr Val Tyr
        35                  40                  45

Asn Thr Asp Thr Asp Asn Cys Arg Glu Val Ile Ile Thr Pro Asn Ser
    50                  55                  60

Ala Trp Gly Gly Glu Gly Ser Leu Gly Cys Gly Ile Gly Tyr Gly Tyr
65                  70                  75                  80

Leu His Arg Ile Pro Thr Arg Pro Phe Glu Glu Gly Lys Lys Ile Ser
                85                  90                  95

Leu Pro Gly Gln Met Ala Gly Thr Pro Ile Thr Pro Leu Lys Asp Gly
            100                 105                 110

Phe Thr Glu Val Gln Leu Ser Ser Val Asn Pro Pro Ser Leu Ser Pro
        115                 120                 125

Pro Gly Thr Thr Gly Ile Glu Gln Ser Leu Thr Gly Leu Ser Ile Ser
    130                 135                 140

Ser
145

(2) INFORMATION FOR SEQ ID NO: 516:

(i) SEQUENCE CHARACTERISTICS:
(A) LENGTH: 151 amino acids
(B) TYPE: amino acid
(D) TOPOLOGY: linear
(xi) SEQUENCE DESCRIPTION: SEQ ID NO: 516:

Arg Ile Pro Thr Arg Pro Phe Glu Glu Gly Lys Lys Ile Ser Leu Pro
1               5                   10                  15

Gly Gln Met Ala Gly Thr Pro Ile Thr Pro Leu Lys Asp Gly Phe Thr
            20                  25                  30

Glu Val Gln Leu Ser Ser Val Asn Pro Pro Ser Leu Ser Pro Pro Gly
        35                  40                  45

Thr Thr Gly Ile Glu Gln Ser Leu Thr Gly Leu Ser Ile Ser Ser Thr
    50                  55                  60

Pro Pro Ala Val Ser Ser Val Leu Ser Thr Gly Val Pro Thr Val Pro
65                  70                  75                  80

Leu Leu Pro Pro Gln Val Asn Gln Ser Leu Thr Ser Val Pro Pro Met
                85                  90                  95

Asn Pro Ala Thr Thr Leu Pro Gly Leu Met Pro Leu Pro Ala Gly Leu
            100                 105                 110

Pro Asn Leu Pro Asn Leu Asn Leu Asn Leu Pro Ala Pro His Ile Met
        115                 120                 125

Pro Gly Val Gly Leu Pro Glu Leu Val Asn Pro Gly Leu Pro Pro Leu
    130                 135                 140

Pro Ser Met Pro Pro Arg Asn
145                 150

(2) INFORMATION FOR SEQ ID NO: 517:

    (i) SEQUENCE CHARACTERISTICS:
        (A) LENGTH: 109 amino acids
        (B) TYPE: amino acid
        (D) TOPOLOGY: linear
    (xi) SEQUENCE DESCRIPTION: SEQ ID NO: 517:

Pro Gly Leu Pro Pro Leu Pro Ser Met Pro Pro Arg Asn Leu Pro Gly
 1               5                  10                  15

Ile Ala Pro Leu Pro Leu Pro Ser Glu Phe Leu Pro Ser Phe Pro Leu
            20                  25                  30

Val Pro Glu Ser Ser Ser Ala Ala Ser Ser Gly Glu Leu Leu Ser Ser
        35                  40                  45

Leu Pro Pro Thr Ser Asn Ala Pro Ser Asp Pro Ala Thr Thr Thr Ala
    50                  55                  60

Lys Ala Asp Ala Ala Ser Ser Leu Thr Val Asp Val Thr Pro Pro Thr
65                  70                  75                  80

Ala Lys Ala Pro Thr Thr Val Glu Asp Arg Val Gly Asp Ser Thr Pro
                85                  90                  95

Val Ser Glu Lys Pro Val Ser Ala Ala Val Asp Ala Asn
            100                 105

(2) INFORMATION FOR SEQ ID NO: 518:

    (i) SEQUENCE CHARACTERISTICS:
        (A) LENGTH: 93 amino acids
        (B) TYPE: amino acid
        (D) TOPOLOGY: linear
    (xi) SEQUENCE DESCRIPTION: SEQ ID NO: 518:

Ile Tyr Lys Val Phe Arg His Thr Ala Gly Leu Lys Pro Glu Val Ser
 1               5                  10                  15

Cys Phe Glu Asn Ile Arg Ser Cys Ala Arg Xaa Xaa Xaa Xaa Xaa Xaa

20                  25                  30

Xaa Xaa Xaa Xaa Xaa Xaa Trp Ile Phe Gly Val Leu His Val Val His
        35                  40                  45

Ala Ser Val Val Thr Ala Tyr Leu Phe Thr Val Ser Asn Ala Phe Gln
    50                  55                  60

Gly Met Phe Ile Phe Leu Phe Leu Cys Val Leu Ser Arg Lys Ile Gln
65                  70                  75                  80

Glu Glu Tyr Tyr Arg Leu Phe Lys Asn Val Pro Cys Cys
                85                  90


(2) INFORMATION FOR SEQ ID NO: 519:

        (i) SEQUENCE CHARACTERISTICS:
                (A) LENGTH: 55 amino acids
                (B) TYPE: amino acid
                (D) TOPOLOGY: linear
        (xi) SEQUENCE DESCRIPTION: SEQ ID NO: 519:

Trp Ile Phe Gly Val Leu His Val Val His Ala Ser Val Val Thr Ala
 1               5                  10                  15

Tyr Leu Phe Thr Val Ser Asn Ala Phe Gln Gly Met Phe Ile Phe Leu
            20                  25                  30

Phe Leu Cys Val Leu Ser Arg Lys Ile Gln Glu Glu Tyr Tyr Arg Leu
        35                  40                  45

Phe Lys Asn Val Pro Cys Cys
    50                  55


(2) INFORMATION FOR SEQ ID NO: 520:

        (i) SEQUENCE CHARACTERISTICS:
                (A) LENGTH: 50 amino acids
                (B) TYPE: amino acid
                (D) TOPOLOGY: linear
        (xi) SEQUENCE DESCRIPTION: SEQ ID NO: 520:

Ala Leu Thr Arg Ile Pro Pro Gly Asp Trp Val Ile Asn Val Thr Ala
 1               5                  10                  15

Val Ser Phe Ala Gly Lys Thr Thr Ala Arg Phe Phe Xaa His Ser Ser
            20                  25                  30

Pro Pro Ser Leu Gly Asp Gln Ala Arg Thr Asp Pro Gly His Gln Arg
        35                  40                  45

Arg Asp
    50

(2) INFORMATION FOR SEQ ID NO: 521:

(i) SEQUENCE CHARACTERISTICS:
(A) LENGTH: 27 amino acids
(B) TYPE: amino acid
(D) TOPOLOGY: linear
(xi) SEQUENCE DESCRIPTION: SEQ ID NO: 521:

Leu Gln Glu Val Asn Ile Thr Leu Pro Glu Asn Ser Val Trp Tyr Glu
1               5                   10                  15

Arg Tyr Lys Phe Asp Ile Pro Val Phe His Leu
            20                  25

(2) INFORMATION FOR SEQ ID NO: 522:

(i) SEQUENCE CHARACTERISTICS:
(A) LENGTH: 110 amino acids
(B) TYPE: amino acid
(D) TOPOLOGY: linear
(xi) SEQUENCE DESCRIPTION: SEQ ID NO: 522:

Met Gln Gly Ser Gly Ser Gln Phe Arg Ala Cys Leu Leu Cys Leu Cys
1               5                   10                  15

Phe Ser Cys Pro Cys Ser Pro Gly Gly Pro Arg Trp Asn Ser Arg Gln
            20                  25                  30

Gly Gly Arg Arg Phe Pro Lys Thr Cys Arg Ala Ile Ser Gln Asn Leu
        35                  40                  45

Val Phe Lys Tyr Lys Thr Phe Cys Pro Val Arg Tyr Met Gln Pro His
    50                  55                  60

Arg Ser Ser Leu Cys Leu His Phe Thr Ser Tyr Val Phe Ile Leu Ser
65                  70                  75                  80

Thr Trp Gly Ser Leu Arg Thr Tyr Ser Thr Asp Leu Lys Lys Lys Lys
                85                  90                  95

Lys Asn Ser Arg Gly Gly Pro Val Pro Ile Arg Pro Lys Ser
            100                 105                 110

(2) INFORMATION FOR SEQ ID NO: 523:

(i) SEQUENCE CHARACTERISTICS:
(A) LENGTH: 99 base pairs
(B) TYPE: nucleic acid
(C) STRANDEDNESS: double
(D) TOPOLOGY: linear

(xi) SEQUENCE DESCRIPTION: SEQ ID NO: 523:

TAGCATGTAG CCAGTCGAAT AACNTATAAG GACAAAGTGG AGTCCACGCG TGCGGCCGTC     60

TAGACTAGTG GATCCCCCGG CTGCAGGATT CGGCACGAG     99

(2) INFORMATION FOR SEQ ID NO: 524:

(i) SEQUENCE CHARACTERISTICS:
(A) LENGTH: 51 amino acids
(B) TYPE: amino acid
(D) TOPOLOGY: linear
(xi) SEQUENCE DESCRIPTION: SEQ ID NO: 524:

Met Gln Gly Ser Gly Ser Gln Phe Arg Ala Cys Leu Leu Cys Leu Cys
1               5                   10                  15

Phe Ser Cys Pro Cys Ser Pro Gly Gly Pro Arg Trp Asn Ser Arg Gln
            20                  25                  30

Gly Gly Arg Arg Phe Pro Lys Thr Cys Arg Ala Ile Ser Gln Asn Leu
        35                  40                  45

Val Phe Lys
    50

(2) INFORMATION FOR SEQ ID NO: 525:

(i) SEQUENCE CHARACTERISTICS:
(A) LENGTH: 54 amino acids
(B) TYPE: amino acid
(D) TOPOLOGY: linear
(xi) SEQUENCE DESCRIPTION: SEQ ID NO: 525:

Pro Val Arg Tyr Met Gln Pro His Arg Ser Ser Leu Cys Leu His Phe
1               5                   10                  15

Thr Ser Tyr Val Phe Ile Leu Ser Thr Trp Gly Ser Leu Arg Thr Tyr
            20                  25                  30

Ser Thr Asp Leu Lys Lys Lys Lys Lys Asn Ser Arg Gly Gly Pro Val
        35                  40                  45

Pro Ile Arg Pro Lys Ser
    50

(2) INFORMATION FOR SEQ ID NO: 526:

(i) SEQUENCE CHARACTERISTICS:
(A) LENGTH: 38 amino acids
(B) TYPE: amino acid
(D) TOPOLOGY: linear
(xi) SEQUENCE DESCRIPTION: SEQ ID NO: 526:

(2) INFORMATION FOR SEQ ID NO: 527:

(i) SEQUENCE CHARACTERISTICS:
(A) LENGTH: 161 amino acids
(B) TYPE: amino acid
(D) TOPOLOGY: linear
(xi) SEQUENCE DESCRIPTION: SEQ ID NO: 527:

Met Pro Arg Lys Thr Ser Lys Cys Arg Gln Leu Leu Cys Ser Gly Ala
1               5                   10                  15

Ser Arg Asn Ala Asp Thr Ala Ala Arg Gln Ser Thr Cys Ser Ser His
            20                  25                  30

Arg Pro Pro Gly Lys Ile Pro Ser Leu Gly Pro Arg Arg Xaa Pro Gly
        35                  40                  45

Cys Xaa Ser Val Pro Ser Ser Arg Gly Glu Gln Ser Thr Gly Ser Pro
    50                  55                  60

Ala Ala Pro Arg Cys Gly Arg Arg Asp Ala His Arg Gly Leu Pro Gly
65                  70                  75                  80

Gly Ala Ala Met Thr Pro Gly Asp Thr Trp Ala Ser Phe Asn Pro Arg
                85                  90                  95

Ala Gly His Ser Lys Ser Gln Gly Glu Gly Gln Glu Ser Ser Gly Ala
            100                 105                 110

Ser Arg Gln Asp Arg His Pro Val Ser His Trp Val Glu Arg Gln Arg
        115                 120                 125

Glu Ala Trp Gly Ala Pro Arg Ser Ser Ser Ala Gly Gly Val Lys Val
    130                 135                 140

Ala Ala Thr Thr Glu Arg Glu Pro Glu Phe Lys Ile Lys Thr Gly Lys
145                 150                 155                 160

Ala

(2) INFORMATION FOR SEQ ID NO: 528:

(i) SEQUENCE CHARACTERISTICS:
(A) LENGTH: 88 amino acids
(B) TYPE: amino acid
(D) TOPOLOGY: linear
(xi) SEQUENCE DESCRIPTION: SEQ ID NO: 528:

Cys Ser Gly Ala Ser Arg Asn Ala Asp Thr Ala Ala Arg Gln Ser Thr
1               5                   10                  15

Cys Ser Ser His Arg Pro Pro Gly Lys Ile Pro Ser Leu Gly Pro Arg
            20                  25                  30

Arg Xaa Pro Gly Cys Xaa Ser Val Pro Ser Ser Arg Gly Glu Gln Ser
        35                  40                  45

Thr Gly Ser Pro Ala Ala Pro Arg Cys Gly Arg Arg Asp Ala His Arg
    50                  55                  60

Gly Leu Pro Gly Gly Ala Ala Met Thr Pro Gly Asp Thr Trp Ala Ser
65                  70                  75                  80

Phe Asn Pro Arg Ala Gly His Ser
                85

(2) INFORMATION FOR SEQ ID NO: 529:

(i) SEQUENCE CHARACTERISTICS:
(A) LENGTH: 59 amino acids
(B) TYPE: amino acid
(D) TOPOLOGY: linear
(xi) SEQUENCE DESCRIPTION: SEQ ID NO: 529:

Gln Gly Glu Gly Gln Glu Ser Ser Gly Ala Ser Arg Gln Asp Arg His
1               5                   10                  15

Pro Val Ser His Trp Val Glu Arg Gln Arg Glu Ala Trp Gly Ala Pro
            20                  25                  30

Arg Ser Ser Ser Ala Gly Gly Val Lys Val Ala Ala Thr Thr Glu Arg
        35                  40                  45

Glu Pro Glu Phe Lys Ile Lys Thr Gly Lys Ala
    50                  55

(2) INFORMATION FOR SEQ ID NO: 530:

(i) SEQUENCE CHARACTERISTICS:
(A) LENGTH: 235 amino acids
(B) TYPE: amino acid
(D) TOPOLOGY: linear
(xi) SEQUENCE DESCRIPTION: SEQ ID NO: 530:

Met Ser Pro Arg Tyr Pro Gly Gly Pro Arg Pro Pro Leu Arg Ile Pro
1               5                   10                  15

Asn Gln Ala Leu Gly Gly Val Pro Gly Ser Gln Pro Leu Leu Pro Ser
            20                  25                  30

Gly Met Asp Pro Thr Arg Gln Gln Gly His Pro Asn Met Gly Gly Pro
        35                  40                  45

Met Gln Arg Met Thr Pro Pro Arg Gly Met Val Pro Leu Gly Pro Gln
    50                  55                  60

Asn Tyr Gly Gly Ala Met Arg Pro Pro Leu Asn Ala Leu Gly Gly Pro
65                  70                  75                  80

Gly Met Pro Gly Met Asn Met Gly Pro Gly Gly Gly Arg Pro Trp Pro
                85                  90                  95

Asn Pro Thr Asn Ala Asn Ser Ile Pro Tyr Ser Ser Ala Ser Pro Gly
            100                 105                 110

Asn Tyr Val Gly Pro Pro Gly Gly Gly Gly Pro Pro Gly Thr Pro Ile
        115                 120                 125

Met Pro Ser Pro Ala Asp Ser Thr Asn Ser Gly Asp Asn Met Tyr Thr
    130                 135                 140

Leu Met Asn Ala Val Pro Pro Gly Pro Asn Arg Pro Asn Phe Pro Met
145                 150                 155                 160

Gly Pro Gly Ser Asp Gly Pro Met Gly Gly Leu Gly Gly Met Glu Ser
                165                 170                 175

His His Met Asn Gly Ser Leu Gly Ser Gly Asp Met Asp Ser Ile Ser
            180                 185                 190

Lys Asn Ser Pro Asn Asn Met Ser Leu Ser Asn Gln Pro Gly Thr Pro
        195                 200                 205

Arg Asp Asp Gly Glu Met Gly Gly Asn Phe Leu Asn Pro Phe Gln Ser
    210                 215                 220

Glu Ser Tyr Ser Pro Ser Met Thr Met Ser Val
225                 230                 235

(2) INFORMATION FOR SEQ ID NO: 531:

(i) SEQUENCE CHARACTERISTICS:
(A) LENGTH: 114 amino acids
(B) TYPE: amino acid
(D) TOPOLOGY: linear
(xi) SEQUENCE DESCRIPTION: SEQ ID NO: 531:

Met Ser Pro Arg Tyr Pro Gly Gly Pro Arg Pro Pro Leu Arg Ile Pro
1               5                   10                  15

Asn Gln Ala Leu Gly Gly Val Pro Gly Ser Gln Pro Leu Leu Pro Ser
            20                  25                  30

Gly Met Asp Pro Thr Arg Gln Gln Gly His Pro Asn Met Gly Gly Pro
        35                  40                  45

Met Gln Arg Met Thr Pro Pro Arg Gly Met Val Pro Leu Gly Pro Gln
    50                  55                  60

Asn Tyr Gly Gly Ala Met Arg Pro Pro Leu Asn Ala Leu Gly Gly Pro
65                  70                  75                  80

Gly Met Pro Gly Met Asn Met Gly Pro Gly Gly Gly Arg Pro Trp Pro
                85                  90                  95

Asn Pro Thr Asn Ala Asn Ser Ile Pro Tyr Ser Ser Ala Ser Pro Gly
            100                 105                 110

Asn Tyr

(2) INFORMATION FOR SEQ ID NO: 532:

(i) SEQUENCE CHARACTERISTICS:
(A) LENGTH: 81 amino acids
(B) TYPE: amino acid
(D) TOPOLOGY: linear
(xi) SEQUENCE DESCRIPTION: SEQ ID NO: 532:

Leu Asn Ala Leu Gly Gly Pro Gly Met Pro Gly Met Asn Met Gly Pro
1               5                   10                  15

Gly Gly Gly Arg Pro Trp Pro Asn Pro Thr Asn Ala Asn Ser Ile Pro
            20                  25                  30

Tyr Ser Ser Ala Ser Pro Gly Asn Tyr Val Gly Pro Pro Gly Gly Gly
        35                  40                  45

Gly Pro Pro Gly Thr Pro Ile Met Pro Ser Pro Ala Asp Ser Thr Asn
    50                  55                  60

Ser Gly Asp Asn Met Tyr Thr Leu Met Asn Ala Val Pro Pro Gly Pro
65                  70                  75                  80

Asn

(2) INFORMATION FOR SEQ ID NO: 533:

(i) SEQUENCE CHARACTERISTICS:
(A) LENGTH: 70 amino acids
(B) TYPE: amino acid
(D) TOPOLOGY: linear
(xi) SEQUENCE DESCRIPTION: SEQ ID NO: 533:

Gly Pro Met Gly Gly Leu Gly Gly Met Glu Ser His His Met Asn Gly
1               5                   10                  15

Ser Leu Gly Ser Gly Asp Met Asp Ser Ile Ser Lys Asn Ser Pro Asn
            20                  25                  30

Asn Met Ser Leu Ser Asn Gln Pro Gly Thr Pro Arg Asp Asp Gly Glu
        35                  40                  45

Met Gly Gly Asn Phe Leu Asn Pro Phe Gln Ser Glu Ser Tyr Ser Pro
    50                  55                  60

Ser Met Thr Met Ser Val
65                  70

(2) INFORMATION FOR SEQ ID NO: 534:

(i) SEQUENCE CHARACTERISTICS:
(A) LENGTH: 14 amino acids
(B) TYPE: amino acid
(D) TOPOLOGY: linear
(xi) SEQUENCE DESCRIPTION: SEQ ID NO: 534:

Thr Cys Glu His Ser Ser Glu Ala Lys Ala Phe His Asp Tyr
1               5                   10

(2) INFORMATION FOR SEQ ID NO: 535:

(i) SEQUENCE CHARACTERISTICS:
(A) LENGTH: 59 amino acids
(B) TYPE: amino acid
(D) TOPOLOGY: linear
(xi) SEQUENCE DESCRIPTION: SEQ ID NO: 535:

Gln Ala Phe Val Leu Leu Ser Asp Leu Leu Leu Ile Phe Ser Pro Gln
1               5                   10                  15

Met Ile Val Gly Gly Arg Asp Phe Leu Arg Pro Leu Val Phe Phe Pro
            20                  25                  30

Glu Ala Thr Leu Gln Ser Glu Leu Ala Ser Phe Leu Met Asp His Val
        35                  40                  45

Phe Ile Gln Pro Gly Asp Leu Gly Ser Gly Ala
    50                  55

(2) INFORMATION FOR SEQ ID NO: 536:

(i) SEQUENCE CHARACTERISTICS:
(A) LENGTH: 43 amino acids
(B) TYPE: amino acid
(D) TOPOLOGY: linear
(xi) SEQUENCE DESCRIPTION: SEQ ID NO: 536:

Ala Cys Ser Tyr Leu Leu Cys Asn Pro Glu Phe Thr Phe Phe Ser Arg
1               5                   10                  15

Ala Asp Phe Ala Arg Ser Gln Leu Val Asp Leu Leu Thr Asp Arg Phe
            20                  25                  30

Gln Gln Glu Leu Glu Glu Leu Leu Gln Val Gly
        35                  40

(2) INFORMATION FOR SEQ ID NO: 537:

(i) SEQUENCE CHARACTERISTICS:
(A) LENGTH: 35 amino acids

(B) TYPE: amino acid
(D) TOPOLOGY: linear
(xi) SEQUENCE DESCRIPTION: SEQ ID NO: 537:

Gln Lys Gln Leu Ser Ser Leu Arg Asp Arg Met Val Ala Phe Cys Glu
1               5                   10                  15

Leu Cys Gln Ser Cys Leu Ser Asp Val Asp Thr Glu Ile Gln Glu Gln
            20                  25                  30

Val Ser Thr
        35

(2) INFORMATION FOR SEQ ID NO: 538:

(i) SEQUENCE CHARACTERISTICS:
(A) LENGTH: 27 amino acids
(B) TYPE: amino acid
(D) TOPOLOGY: linear
(xi) SEQUENCE DESCRIPTION: SEQ ID NO: 538:

Gln Val Ile Leu Pro Ala Leu Thr Leu Val Tyr Phe Ser Ile Leu Trp
1               5                   10                  15

Thr Leu Thr His Ile Ser Lys Ser Asp Ala Ser
            20                  25

(2) INFORMATION FOR SEQ ID NO: 539:

(i) SEQUENCE CHARACTERISTICS:
(A) LENGTH: 31 amino acids
(B) TYPE: amino acid
(D) TOPOLOGY: linear
(xi) SEQUENCE DESCRIPTION: SEQ ID NO: 539:

Ser Thr His Asp Leu Thr Arg Trp Glu Leu Tyr Glu Pro Cys Cys Gln
1               5                   10                  15

Leu Leu Gln Lys Ala Val Asp Thr Gly Xaa Val Pro His Gln Val
            20                  25                  30

(2) INFORMATION FOR SEQ ID NO: 540:

(i) SEQUENCE CHARACTERISTICS:
(A) LENGTH: 106 amino acids
(B) TYPE: amino acid
(D) TOPOLOGY: linear
(xi) SEQUENCE DESCRIPTION: SEQ ID NO: 540:

Leu Ala Val Ser Thr Ser Phe Ile Cys Cys Ala Asp Ile Ser Thr Ala
1               5                   10                  15

Leu Pro Leu Gly Ser Ser Arg Pro Ala Pro Ala Pro Arg His Arg Glu
            20                  25                  30

His Glu His Gly His Gln Ala Arg Pro Pro Arg Leu Leu Xaa Thr Ser
        35                  40                  45

Leu Met Pro Leu Ser Thr Pro Ala Ala Ala Gln Leu Leu Trp Thr Gln
    50                  55                  60

Leu Thr Pro Met Gly Gly Arg Pro Gly Gly Arg His Ser Pro Pro Thr
65                  70                  75                  80

Leu His Thr Gly Pro Arg Ala Leu Pro Pro Gly Pro Pro His Pro Ser
                85                  90                  95

Leu His Val Ala Ala Leu Ser Leu Leu Arg
            100                 105

(2) INFORMATION FOR SEQ ID NO: 541:

    (i) SEQUENCE CHARACTERISTICS:
        (A) LENGTH: 207 amino acids
        (B) TYPE: amino acid
        (D) TOPOLOGY: linear
    (xi) SEQUENCE DESCRIPTION: SEQ ID NO: 541:

Glu Gln Val Leu Ala Leu Leu Trp Pro Arg Phe Glu Leu Ile Leu Glu
1               5                   10                  15

Met Asn Val Gln Ser Val Arg Ser Thr Asp Pro Gln Arg Leu Gly Gly
            20                  25                  30

Leu Asp Thr Arg Pro His Tyr Ile Thr Arg Arg Tyr Ala Glu Phe Ser
        35                  40                  45

Ser Ala Leu Val Ser Ile Asn Gln Thr Ile Pro Asn Glu Arg Thr Met
    50                  55                  60

Gln Leu Leu Gly Gln Leu Gln Val Glu Val Glu Asn Phe Val Leu Arg
65                  70                  75                  80

Val Ala Ala Glu Phe Ser Ser Arg Lys Glu Gln Leu Val Phe Leu Ile
                85                  90                  95

Asn Asn Tyr Asp Met Met Leu Gly Val Leu Met Glu Arg Ala Ala Asp
            100                 105                 110

Asp Ser Lys Glu Val Glu Ser Phe Gln Gln Leu Leu Asn Ala Arg Thr
        115                 120                 125

Gln Glu Phe Ile Glu Glu Leu Leu Ser Pro Pro Phe Gly Gly Leu Val
    130                 135                 140

Ala Phe Val Lys Glu Ala Glu Ala Leu Ile Glu Arg Gly Gln Ala Glu
145                 150                 155                 160

Arg Leu Arg Gly Glu Glu Ala Arg Val Thr Gln Leu Ile Arg Gly Phe
                165                 170                 175

Gly Ser Ser Trp Lys Ser Ser Val Glu Ser Leu Ser Gln Asp Val Met

            180                 185                 190

Arg Ser Phe Thr Asn Phe Arg Asn Gly Thr Ser Ile Ile Gln Gly
        195                 200                 205

(2) INFORMATION FOR SEQ ID NO: 542:

(i) SEQUENCE CHARACTERISTICS:
(A) LENGTH: 110 amino acids
(B) TYPE: amino acid
(D) TOPOLOGY: linear
(xi) SEQUENCE DESCRIPTION: SEQ ID NO: 542:

Ala Leu Leu Lys Tyr Arg Phe Phe Tyr Gln Phe Leu Leu Gly Asn Glu
1               5                   10                  15

Arg Ala Thr Ala Lys Glu Ile Arg Asp Glu Tyr Val Glu Thr Leu Ser
            20                  25                  30

Lys Ile Tyr Leu Ser Tyr Tyr Arg Ser Tyr Leu Gly Arg Leu Met Lys
        35                  40                  45

Val Gln Tyr Glu Glu Val Ala Glu Lys Asp Asp Leu Met Gly Val Glu
    50                  55                  60

Asp Thr Ala Lys Lys Gly Phe Xaa Ser Lys Pro Ser Leu Arg Ser Arg
65                  70                  75                  80

Asn Thr Ile Phe Thr Leu Gly Thr Arg Gly Ser Val Ile Ser Pro Thr
                85                  90                  95

Glu Leu Glu Ala Pro Ile Leu Val Pro His Thr Ala Gln Arg
            100                 105                 110

(2) INFORMATION FOR SEQ ID NO: 543:

(i) SEQUENCE CHARACTERISTICS:
(A) LENGTH: 97 amino acids
(B) TYPE: amino acid
(D) TOPOLOGY: linear
(xi) SEQUENCE DESCRIPTION: SEQ ID NO: 543:

Glu Gln Arg Tyr Pro Phe Glu Ala Leu Phe Arg Ser Gln His Tyr Xaa
1               5                   10                  15

Leu Leu Asp Asn Ser Cys Arg Glu Tyr Leu Phe Ile Cys Glu Phe Phe
            20                  25                  30

Val Val Ser Gly Pro Xaa Ala His Asp Leu Phe His Ala Val Met Gly
        35                  40                  45

Arg Thr Leu Ser Met Thr Leu Lys His Leu Asp Ser Tyr Leu Ala Asp
    50                  55                  60

Cys Tyr Asp Ala Ile Ala Val Phe Leu Cys Ile His Ile Val Leu Arg
65                  70                  75                  80

Phe Arg Asn Ile Ala Ala Lys Arg Asp Val Pro Ala Leu Asp Arg Tyr
                85                  90                  95

Trp

(2) INFORMATION FOR SEQ ID NO: 544:

        (i) SEQUENCE CHARACTERISTICS:
                (A) LENGTH: 26 amino acids
                (B) TYPE: amino acid
                (D) TOPOLOGY: linear
        (xi) SEQUENCE DESCRIPTION: SEQ ID NO: 544:

Gly Gly Leu Asp Thr Arg Pro His Tyr Ile Thr Arg Arg Tyr Ala Glu
  1               5                  10                  15

Phe Ser Ser Ala Leu Val Ser Ile Asn Gln
            20                  25

(2) INFORMATION FOR SEQ ID NO: 545:

        (i) SEQUENCE CHARACTERISTICS:
                (A) LENGTH: 20 amino acids
                (B) TYPE: amino acid
                (D) TOPOLOGY: linear
        (xi) SEQUENCE DESCRIPTION: SEQ ID NO: 545:

Ser Arg Lys Glu Gln Leu Val Phe Leu Ile Asn Asn Tyr Asp Met Met
  1               5                  10                  15

Leu Gly Val Leu
            20

(2) INFORMATION FOR SEQ ID NO: 546:

        (i) SEQUENCE CHARACTERISTICS:
                (A) LENGTH: 411 amino acids
                (B) TYPE: amino acid
                (D) TOPOLOGY: linear
        (xi) SEQUENCE DESCRIPTION: SEQ ID NO: 546:

Ala Leu Leu Lys Tyr Arg Phe Phe Tyr Gln Phe Leu Leu Gly Asn Glu
  1               5                  10                  15

Arg Ala Thr Ala Lys Glu Ile Arg Asp Glu Tyr Val Glu Thr Leu Ser
            20                  25                  30

Lys Ile Tyr Leu Ser Tyr Tyr Arg Ser Tyr Leu Gly Arg Leu Met Lys
        35                  40                  45

Val Gln Tyr Glu Glu Val Ala Glu Lys Asp Asp Leu Met Gly Val Glu
    50                  55                  60

Asp Thr Ala Lys Lys Gly Phe Xaa Ser Lys Pro Ser Leu Arg Ser Arg
65                  70                  75                  80

Asn Thr Ile Phe Thr Leu Gly Thr Arg Gly Ser Val Ile Ser Pro Thr
                85                  90                  95

Glu Leu Glu Ala Pro Ile Leu Val Pro His Thr Ala Gln Arg Xaa Glu
            100                 105                 110

Gln Arg Tyr Pro Phe Glu Ala Leu Phe Arg Ser Gln His Tyr Xaa Leu
        115                 120                 125

Leu Asp Asn Ser Cys Arg Glu Tyr Leu Phe Ile Cys Glu Phe Phe Val
    130                 135                 140

Val Ser Gly Pro Xaa Ala His Asp Leu Phe His Ala Val Met Gly Arg
145                 150                 155                 160

Thr Leu Ser Met Thr Leu Lys His Leu Asp Ser Tyr Leu Ala Asp Cys
                165                 170                 175

Tyr Asp Ala Ile Ala Val Phe Leu Cys Ile His Ile Val Leu Arg Phe
            180                 185                 190

Arg Asn Ile Ala Ala Lys Arg Asp Val Pro Ala Leu Asp Arg Tyr Trp
        195                 200                 205

Glu Gln Val Leu Ala Leu Leu Trp Pro Arg Phe Glu Leu Ile Leu Glu
    210                 215                 220

Met Asn Val Gln Ser Val Arg Ser Thr Asp Pro Gln Arg Leu Gly Gly
225                 230                 235                 240

Leu Asp Thr Arg Pro His Tyr Ile Thr Arg Arg Tyr Ala Glu Phe Ser
                245                 250                 255

Ser Ala Leu Val Ser Ile Asn Gln Thr Ile Pro Asn Glu Arg Thr Met
            260                 265                 270

Gln Leu Leu Gly Gln Leu Gln Val Glu Val Glu Asn Phe Val Leu Arg
        275                 280                 285

Val Ala Ala Glu Phe Ser Ser Arg Lys Glu Gln Leu Val Phe Leu Ile
    290                 295                 300

Asn Asn Tyr Asp Met Met Leu Gly Val Leu Met Glu Arg Ala Ala Asp
305                 310                 315                 320

Asp Ser Lys Glu Val Glu Ser Phe Gln Gln Leu Leu Asn Ala Arg Thr
                325                 330                 335

Gln Glu Phe Ile Glu Glu Leu Leu Ser Pro Pro Phe Gly Gly Leu Val
            340                 345                 350

Ala Phe Val Lys Glu Ala Glu Ala Leu Ile Glu Arg Gly Gln Ala Glu
        355                 360                 365

Arg Leu Arg Gly Glu Glu Ala Arg Val Thr Gln Leu Ile Arg Gly Phe
    370                 375                 380

Gly Ser Ser Trp Lys Ser Ser Val Glu Ser Leu Ser Gln Asp Val Met
385                 390                 395                 400

Arg Ser Phe Thr Asn Phe Arg Asn Gly Thr Ser
                405                 410

(2) INFORMATION FOR SEQ ID NO: 547:

        (i) SEQUENCE CHARACTERISTICS:
                (A) LENGTH: 303 amino acids
                (B) TYPE: amino acid
                (D) TOPOLOGY: linear
        (xi) SEQUENCE DESCRIPTION: SEQ ID NO: 547:

Tyr Glu Gly Lys Glu Phe Asp Tyr Val Phe Ser Ile Asp Val Asn Glu
 1               5                  10                  15

Gly Gly Pro Ser Tyr Lys Leu Pro Tyr Asn Thr Ser Asp Asp Pro Trp
            20                  25                  30

Leu Thr Ala Tyr Asn Phe Leu Gln Lys Asn Asp Leu Asn Pro Met Phe
        35                  40                  45

Leu Asp Gln Val Ala Lys Phe Ile Ile Asp Asn Thr Lys Gly Gln Met
    50                  55                  60

Leu Gly Leu Gly Asn Pro Ser Phe Ser Asp Pro Phe Thr Gly Gly Gly
65                  70                  75                  80

Arg Tyr Val Pro Gly Ser Ser Gly Ser Ser Asn Thr Leu Pro Thr Ala
                85                  90                  95

Asp Pro Phe Thr Gly Ala Gly Arg Tyr Val Pro Gly Ser Ala Ser Met
            100                 105                 110

Gly Thr Thr Met Ala Gly Val Asp Pro Phe Thr Gly Asn Ser Ala Tyr
        115                 120                 125

Arg Ser Ala Ala Ser Lys Thr Met Asn Ile Tyr Phe Pro Lys Lys Glu
    130                 135                 140

Ala Val Thr Phe Asp Gln Ala Asn Pro Thr Gln Ile Leu Gly Lys Leu
145                 150                 155                 160

Lys Glu Leu Asn Gly Thr Ala Pro Glu Glu Lys Lys Leu Thr Glu Asp
                165                 170                 175

Asp Leu Ile Leu Leu Glu Lys Ile Leu Ser Leu Ile Cys Asn Ser Ser
            180                 185                 190

Ser Glu Lys Pro Thr Val Gln Gln Leu Gln Ile Leu Trp Lys Ala Ile
        195                 200                 205

Asn Cys Pro Glu Asp Ile Val Phe Pro Ala Leu Asp Ile Leu Arg Leu
    210                 215                 220

Ser Ile Lys His Pro Ser Val Asn Glu Asn Phe Cys Asn Glu Lys Glu

225                 230                 235                 240

Gly Ala Gln Phe Ser Ser His Leu Ile Asn Leu Leu Asn Pro Lys Gly
                245                 250                 255

Lys Pro Ala Asn Gln Leu Leu Ala Leu Arg Thr Phe Cys Asn Cys Phe
            260                 265                 270

Val Gly Gln Ala Gly Gln Lys Leu Met Met Ser Gln Arg Glu Ser Leu
        275                 280                 285

Met Ser His Ala Ile Glu Leu Lys Ser Gly Ser Asn Lys Asn Ile
    290                 295                 300

(2) INFORMATION FOR SEQ ID NO: 548:

(i) SEQUENCE CHARACTERISTICS:
(A) LENGTH: 18 amino acids
(B) TYPE: amino acid
(D) TOPOLOGY: linear
(xi) SEQUENCE DESCRIPTION: SEQ ID NO: 548:

His Ile Ala Leu Ala Thr Leu Ala Leu Asn Tyr Ser Val Cys Phe His
  1               5                  10                  15

Lys Asp

(2) INFORMATION FOR SEQ ID NO: 549:

(i) SEQUENCE CHARACTERISTICS:
(A) LENGTH: 49 amino acids
(B) TYPE: amino acid
(D) TOPOLOGY: linear
(xi) SEQUENCE DESCRIPTION: SEQ ID NO: 549:

His Asn Ile Glu Gly Lys Ala Gln Cys Leu Ser Leu Ile Ser Thr Ile
  1               5                  10                  15

Leu Glu Val Val Gln Asp Leu Glu Ala Thr Phe Arg Leu Leu Val Ala
             20                  25                  30

Leu Gly Thr Leu Ile Ser Asp Asp Ser Asn Ala Val Gln Leu Ala Lys
         35                  40                  45

Ser

(2) INFORMATION FOR SEQ ID NO: 550:

(i) SEQUENCE CHARACTERISTICS:
(A) LENGTH: 30 amino acids
(B) TYPE: amino acid
(D) TOPOLOGY: linear

(xi) SEQUENCE DESCRIPTION: SEQ ID NO: 550:

Leu Gly Val Asp Ser Gln Ile Lys Lys Tyr Ser Ser Val Ser Glu Pro
1               5                   10                  15

Ala Lys Val Ser Glu Cys Cys Arg Phe Ile Leu Asn Leu Leu
            20                  25                  30

(2) INFORMATION FOR SEQ ID NO: 551:

(i) SEQUENCE CHARACTERISTICS:
(A) LENGTH: 400 amino acids
(B) TYPE: amino acid
(D) TOPOLOGY: linear
(xi) SEQUENCE DESCRIPTION: SEQ ID NO: 551:

Tyr Glu Gly Lys Glu Phe Asp Tyr Val Phe Ser Ile Asp Val Asn Glu
1               5                   10                  15

Gly Gly Pro Ser Tyr Lys Leu Pro Tyr Asn Thr Ser Asp Asp Pro Trp
            20                  25                  30

Leu Thr Ala Tyr Asn Phe Leu Gln Lys Asn Asp Leu Asn Pro Met Phe
        35                  40                  45

Leu Asp Gln Val Ala Lys Phe Ile Ile Asp Asn Thr Lys Gly Gln Met
    50                  55                  60

Leu Gly Leu Gly Asn Pro Ser Phe Ser Asp Pro Phe Thr Gly Gly Gly
65                  70                  75                  80

Arg Tyr Val Pro Gly Ser Ser Gly Ser Ser Asn Thr Leu Pro Thr Ala
                85                  90                  95

Asp Pro Phe Thr Gly Ala Gly Arg Tyr Val Pro Gly Ser Ala Ser Met
            100                 105                 110

Gly Thr Thr Met Ala Gly Val Asp Pro Phe Thr Gly Asn Ser Ala Tyr
        115                 120                 125

Arg Ser Ala Ala Ser Lys Thr Met Asn Ile Tyr Phe Pro Lys Lys Glu
    130                 135                 140

Ala Val Thr Phe Asp Gln Ala Asn Pro Thr Gln Ile Leu Gly Lys Leu
145                 150                 155                 160

Lys Glu Leu Asn Gly Thr Ala Pro Glu Glu Lys Lys Leu Thr Glu Asp
                165                 170                 175

Asp Leu Ile Leu Leu Glu Lys Ile Leu Ser Leu Ile Cys Asn Ser Ser
            180                 185                 190

Ser Glu Lys Pro Thr Val Gln Gln Leu Gln Ile Leu Trp Lys Ala Ile
        195                 200                 205

Asn Cys Pro Glu Asp Ile Val Phe Pro Ala Leu Asp Ile Leu Arg Leu
    210                 215                 220

Ser Ile Lys His Pro Ser Val Asn Glu Asn Phe Cys Asn Glu Lys Glu
225                 230                 235                 240

Gly Ala Gln Phe Ser Ser His Leu Ile Asn Leu Leu Asn Pro Lys Gly
                245                 250                 255

Lys Pro Ala Asn Gln Leu Leu Ala Leu Arg Thr Phe Cys Asn Cys Phe
            260                 265                 270

Val Gly Gln Ala Gly Gln Lys Leu Met Met Ser Gln Arg Glu Ser Leu
        275                 280                 285

Met Ser His Ala Ile Glu Leu Lys Ser Gly Ser Asn Lys Asn Ile His
    290                 295                 300

Ile Ala Leu Ala Thr Leu Ala Leu Asn Tyr Ser Val Cys Phe His Lys
305                 310                 315                 320

Asp His Asn Ile Glu Gly Lys Ala Gln Cys Leu Ser Leu Ile Ser Thr
                325                 330                 335

Ile Leu Glu Val Val Gln Asp Leu Glu Ala Thr Phe Arg Leu Leu Val
            340                 345                 350

Ala Leu Gly Thr Leu Ile Ser Asp Asp Ser Asn Ala Val Gln Leu Ala
        355                 360                 365

Lys Ser Leu Gly Val Asp Ser Gln Ile Lys Lys Tyr Ser Ser Val Ser
    370                 375                 380

Glu Pro Ala Lys Val Ser Glu Cys Cys Arg Phe Ile Leu Asn Leu Leu
385                 390                 395                 400

(2) INFORMATION FOR SEQ ID NO: 552:

(i) SEQUENCE CHARACTERISTICS:
(A) LENGTH: 139 amino acids
(B) TYPE: amino acid
(D) TOPOLOGY: linear
(xi) SEQUENCE DESCRIPTION: SEQ ID NO: 552:

Tyr Pro Asn Gln Asp Gly Asp Ile Leu Arg Asp Gln Val Leu His Glu
  1               5                  10                  15

His Ile Gln Arg Leu Ser Lys Val Val Thr Ala Asn His Arg Ala Leu
             20                  25                  30

Gln Ile Pro Glu Val Tyr Leu Arg Glu Ala Pro Trp Pro Ser Ala Gln
         35                  40                  45

Ser Glu Ile Arg Thr Ile Ser Ala Tyr Lys Thr Pro Arg Asp Lys Val
     50                  55                  60

Gln Cys Ile Leu Arg Met Cys Ser Thr Ile Met Asn Leu Leu Ser Leu
 65                  70                  75                  80

Ala Asn Glu Asp Ser Val Pro Gly Ala Asp Asp Phe Val Pro Val Leu
                85                  90                  95

Val Phe Val Leu Ile Lys Ala Asn Pro Pro Cys Leu Leu Ser Thr Val
            100                 105                 110

Gln Tyr Ile Ser Ser Phe Tyr Ala Ser Cys Leu Ser Gly Glu Glu Ser
        115                 120                 125

Tyr Trp Trp Met Gln Phe Thr Ala Ala Val Glu
    130                 135

(2) INFORMATION FOR SEQ ID NO: 553:

       (i) SEQUENCE CHARACTERISTICS:
              (A) LENGTH: 144 amino acids
              (B) TYPE: amino acid
              (D) TOPOLOGY: linear
       (xi) SEQUENCE DESCRIPTION: SEQ ID NO: 553:

Tyr Pro Asn Gln Asp Gly Asp Ile Leu Arg Asp Gln Val Leu His Glu
  1               5                  10                  15

His Ile Gln Arg Leu Ser Lys Val Val Thr Ala Asn His Arg Ala Leu
            20                  25                  30

Gln Ile Pro Glu Val Tyr Leu Arg Glu Ala Pro Trp Pro Ser Ala Gln
        35                  40                  45

Ser Glu Ile Arg Thr Ile Ser Ala Tyr Lys Thr Pro Arg Asp Lys Val
    50                  55                  60

Gln Cys Ile Leu Arg Met Cys Ser Thr Ile Met Asn Leu Leu Ser Leu
 65                  70                  75                  80

Ala Asn Glu Asp Ser Val Pro Gly Ala Asp Asp Phe Val Pro Val Leu
                85                  90                  95

Val Phe Val Leu Ile Lys Ala Asn Pro Pro Cys Leu Leu Ser Thr Val
            100                 105                 110

Gln Tyr Ile Ser Ser Phe Tyr Ala Ser Cys Leu Ser Gly Glu Glu Ser
        115                 120                 125

Tyr Trp Trp Met Gln Phe Thr Ala Ala Val Glu Phe Ile Lys Thr Ile
    130                 135                 140

(2) INFORMATION FOR SEQ ID NO: 554:

       (i) SEQUENCE CHARACTERISTICS:
              (A) LENGTH: 14 amino acids
              (B) TYPE: amino acid

(D) TOPOLOGY: linear
(xi) SEQUENCE DESCRIPTION: SEQ ID NO: 554:

Tyr Pro Asn Gln Asp Gly Asp Ile Leu Arg Asp Gln Val Leu
1               5                   10

(2) INFORMATION FOR SEQ ID NO: 555:

(i) SEQUENCE CHARACTERISTICS:
(A) LENGTH: 11 amino acids
(B) TYPE: amino acid
(D) TOPOLOGY: linear
(xi) SEQUENCE DESCRIPTION: SEQ ID NO: 555:

Glu Ala Pro Trp Pro Ser Ala Gln Ser Glu Ile
1               5                   10

(2) INFORMATION FOR SEQ ID NO: 556:

(i) SEQUENCE CHARACTERISTICS:
(A) LENGTH: 21 amino acids
(B) TYPE: amino acid
(D) TOPOLOGY: linear
(xi) SEQUENCE DESCRIPTION: SEQ ID NO: 556:

Ser Gly Glu Glu Ser Tyr Trp Trp Met Gln Phe Thr Ala Ala Val Glu
1               5                   10                  15

Phe Ile Lys Thr Ile
            20

(2) INFORMATION FOR SEQ ID NO: 557:

(i) SEQUENCE CHARACTERISTICS:
(A) LENGTH: 18 amino acids
(B) TYPE: amino acid
(D) TOPOLOGY: linear
(xi) SEQUENCE DESCRIPTION: SEQ ID NO: 557:

Ala Asp Asp Phe Val Pro Val Leu Val Phe Val Leu Ile Lys Ala Asn
1               5                   10                  15

Pro Pro

(2) INFORMATION FOR SEQ ID NO: 558:

(i) SEQUENCE CHARACTERISTICS:
(A) LENGTH: 12 amino acids
(B) TYPE: amino acid
(D) TOPOLOGY: linear
(xi) SEQUENCE DESCRIPTION: SEQ ID NO: 558:

Tyr Lys Thr Pro Arg Asp Lys Val Gln Cys Ile Leu
1               5                   10

(2) INFORMATION FOR SEQ ID NO: 559:

(i) SEQUENCE CHARACTERISTICS:
(A) LENGTH: 15 amino acids
(B) TYPE: amino acid
(D) TOPOLOGY: linear
(xi) SEQUENCE DESCRIPTION: SEQ ID NO: 559:

Gly Ala Asp Asp Phe Val Pro Val Leu Val Phe Val Leu Ile Lys
1               5                   10                  15

(2) INFORMATION FOR SEQ ID NO: 560:

(i) SEQUENCE CHARACTERISTICS:
(A) LENGTH: 12 amino acids
(B) TYPE: amino acid
(D) TOPOLOGY: linear
(xi) SEQUENCE DESCRIPTION: SEQ ID NO: 560:

Pro Val Leu Val Phe Val Leu Ile Lys Ala Asn Pro
1               5                   10

(2) INFORMATION FOR SEQ ID NO: 561:

(i) SEQUENCE CHARACTERISTICS:
(A) LENGTH: 17 amino acids
(B) TYPE: amino acid
(D) TOPOLOGY: linear
(xi) SEQUENCE DESCRIPTION: SEQ ID NO: 561:

Ser Ala Arg Ala Ser Thr Gln Pro Pro Ala Gly Gln His Pro Gly Pro
1               5                   10                  15

Cys

(2) INFORMATION FOR SEQ ID NO: 562:

(i) SEQUENCE CHARACTERISTICS:
(A) LENGTH: 33 amino acids
(B) TYPE: amino acid
(D) TOPOLOGY: linear
(xi) SEQUENCE DESCRIPTION: SEQ ID NO: 562:

Met Pro Gly Arg Trp Arg Trp Gln Arg Asp Met His Pro Ala Arg Lys
1               5                   10                  15

Leu Leu Ser Leu Leu Phe Leu Ile Leu Met Gly Thr Glu Leu Thr Gln

20                  25                  30

Asp

(2) INFORMATION FOR SEQ ID NO: 563:

(i) SEQUENCE CHARACTERISTICS:
(A) LENGTH: 19 amino acids
(B) TYPE: amino acid
(D) TOPOLOGY: linear
(xi) SEQUENCE DESCRIPTION: SEQ ID NO: 563:

Ser Ala Ala Pro Asp Ser Leu Leu Arg Ser Ser Lys Gly Ser Thr Arg
1               5                   10                  15

Gly Ser Leu

(2) INFORMATION FOR SEQ ID NO: 564:

(i) SEQUENCE CHARACTERISTICS:
(A) LENGTH: 20 amino acids
(B) TYPE: amino acid
(D) TOPOLOGY: linear
(xi) SEQUENCE DESCRIPTION: SEQ ID NO: 564:

Ala Ala Ile Val Ile Trp Arg Gly Lys Ser Glu Ser Arg Ile Ala Lys
1               5                   10                  15

Thr Pro Gly Ile
            20

(2) INFORMATION FOR SEQ ID NO: 565:

(i) SEQUENCE CHARACTERISTICS:
(A) LENGTH: 17 amino acids
(B) TYPE: amino acid
(D) TOPOLOGY: linear
(xi) SEQUENCE DESCRIPTION: SEQ ID NO: 565:

Pro Leu Gly Ile Thr Leu Pro Leu Gly Ala Pro Glu Thr Gly Gly Gly
1               5                   10                  15

Asp

(2) INFORMATION FOR SEQ ID NO: 566:

(i) SEQUENCE CHARACTERISTICS:
(A) LENGTH: 20 amino acids
(B) TYPE: amino acid

(D) TOPOLOGY: linear
(xi) SEQUENCE DESCRIPTION: SEQ ID NO: 566:

Cys Ala Ala Glu Thr Trp Lys Gly Ser Gln Arg Ala Gly Gln Leu Cys
1               5                   10                  15

Ala Leu Leu Ala
            20

(2) INFORMATION FOR SEQ ID NO: 567:

(i) SEQUENCE CHARACTERISTICS:
(A) LENGTH: 20 amino acids
(B) TYPE: amino acid
(D) TOPOLOGY: linear
(xi) SEQUENCE DESCRIPTION: SEQ ID NO: 567:

Phe Arg Gly Gly Gly Thr Leu Val Leu Pro Pro Thr His Thr Pro Glu
1               5                   10                  15

Trp Leu Ile Leu
            20

(2) INFORMATION FOR SEQ ID NO: 568:

(i) SEQUENCE CHARACTERISTICS:
(A) LENGTH: 22 amino acids
(B) TYPE: amino acid
(D) TOPOLOGY: linear
(xi) SEQUENCE DESCRIPTION: SEQ ID NO: 568:

Met Arg Ser Ala Arg Pro Ser Leu Gly Cys Leu Pro Ser Trp Ala Phe
1               5                   10                  15

Ser Gln Ala Leu Asn Ile
            20

(2) INFORMATION FOR SEQ ID NO: 569:

(i) SEQUENCE CHARACTERISTICS:
(A) LENGTH: 22 amino acids
(B) TYPE: amino acid
(D) TOPOLOGY: linear
(xi) SEQUENCE DESCRIPTION: SEQ ID NO: 569:

Leu Leu Gly Leu Lys Gly Leu Ala Pro Ala Glu Ile Ser Ala Val Cys
1               5                   10                  15

Glu Lys Gly Asn Phe Asn
            20

(2) INFORMATION FOR SEQ ID NO: 570:

(i) SEQUENCE CHARACTERISTICS:
(A) LENGTH: 26 amino acids
(B) TYPE: amino acid
(D) TOPOLOGY: linear
(xi) SEQUENCE DESCRIPTION: SEQ ID NO: 570:

Val Ala His Gly Leu Ala Trp Ser Tyr Tyr Ile Gly Tyr Leu Arg Leu
1               5                   10                  15

Ile Leu Pro Glu Leu Gln Ala Arg Ile Arg
            20                  25

(2) INFORMATION FOR SEQ ID NO: 571:

(i) SEQUENCE CHARACTERISTICS:
(A) LENGTH: 18 amino acids
(B) TYPE: amino acid
(D) TOPOLOGY: linear
(xi) SEQUENCE DESCRIPTION: SEQ ID NO: 571:

Thr Tyr Asn Gln His Tyr Asn Asn Leu Leu Arg Gly Ala Val Ser Gln
1               5                   10                  15

Arg Cys

(2) INFORMATION FOR SEQ ID NO: 572:

(i) SEQUENCE CHARACTERISTICS:
(A) LENGTH: 43 amino acids
(B) TYPE: amino acid
(D) TOPOLOGY: linear
(xi) SEQUENCE DESCRIPTION: SEQ ID NO: 572:

Ile Leu Leu Pro Leu Asp Cys Gly Val Pro Asp Asn Leu Ser Met Ala
1               5                   10                  15

Asp Pro Asn Ile Arg Phe Leu Asp Lys Leu Pro Gln Gln Thr Gly Asp
            20                  25                  30

Arg Ala Gly Ile Lys Asp Arg Val Tyr Ser Asn
        35                  40

(2) INFORMATION FOR SEQ ID NO: 573:

(i) SEQUENCE CHARACTERISTICS:
(A) LENGTH: 45 amino acids
(B) TYPE: amino acid
(D) TOPOLOGY: linear
(xi) SEQUENCE DESCRIPTION: SEQ ID NO: 573:

Ser Ile Tyr Glu Leu Leu Glu Asn Gly Gln Arg Ala Gly Thr Cys Val
1               5                   10                  15

Leu Glu Tyr Ala Thr Pro Leu Gln Thr Leu Phe Ala Met Ser Gln Tyr
            20                  25                  30

Ser Gln Ala Gly Phe Ser Gly Glu Asp Arg Leu Glu Gln
        35                  40                  45

(2) INFORMATION FOR SEQ ID NO: 574:

(i) SEQUENCE CHARACTERISTICS:
(A) LENGTH: 92 amino acids
(B) TYPE: amino acid
(D) TOPOLOGY: linear
(xi) SEQUENCE DESCRIPTION: SEQ ID NO: 574:

Ala Lys Leu Phe Cys Arg Thr Leu Glu Asp Ile Leu Ala Asp Ala Pro
 1               5                  10                  15

Glu Ser Gln Asn Asn Cys Arg Leu Ile Ala Tyr Gln Glu Pro Ala Asp
            20                  25                  30

Asp Ser Ser Phe Ser Leu Ser Gln Glu Val Leu Arg His Leu Arg Gln
        35                  40                  45

Glu Glu Lys Glu Glu Val Thr Val Gly Ser Leu Lys Thr Ser Ala Val
    50                  55                  60

Pro Ser Thr Ser Thr Met Ser Gln Glu Pro Glu Leu Leu Ile Ser Gly
65                  70                  75                  80

Met Glu Lys Pro Leu Pro Leu Arg Thr Asp Phe Ser
                85                  90

(2) INFORMATION FOR SEQ ID NO: 575:

(i) SEQUENCE CHARACTERISTICS:
(A) LENGTH: 43 amino acids
(B) TYPE: amino acid
(D) TOPOLOGY: linear
(xi) SEQUENCE DESCRIPTION: SEQ ID NO: 575:

Leu Leu Gly Leu Lys Gly Leu Ala Pro Ala Glu Ile Ser Ala Val Cys
 1               5                  10                  15

Glu Lys Gly Asn Phe Asn Val Ala His Gly Leu Ala Trp Ser Tyr Tyr
            20                  25                  30

Ile Gly Tyr Leu Arg Leu Ile Leu Pro Glu Leu
        35                  40

(2) INFORMATION FOR SEQ ID NO: 576:

(i) SEQUENCE CHARACTERISTICS:
(A) LENGTH: 22 amino acids

(B) TYPE: amino acid
(D) TOPOLOGY: linear
(xi) SEQUENCE DESCRIPTION: SEQ ID NO: 576:

Thr Met Lys Leu Leu Lys Leu Arg Arg Asn Ile Val Lys Leu Ser Leu
1               5                   10                  15

Tyr Arg His Phe Thr Asn
            20

(2) INFORMATION FOR SEQ ID NO: 577:

(i) SEQUENCE CHARACTERISTICS:
(A) LENGTH: 22 amino acids
(B) TYPE: amino acid
(D) TOPOLOGY: linear
(xi) SEQUENCE DESCRIPTION: SEQ ID NO: 577:

Thr Leu Ile Leu Ala Val Ala Ala Ser Ile Val Phe Ile Ile Trp Thr
1               5                   10                  15

Thr Met Lys Phe Arg Ile
            20

(2) INFORMATION FOR SEQ ID NO: 578:

(i) SEQUENCE CHARACTERISTICS:
(A) LENGTH: 28 amino acids
(B) TYPE: amino acid
(D) TOPOLOGY: linear
(xi) SEQUENCE DESCRIPTION: SEQ ID NO: 578:

Val Thr Cys Gln Ser Asp Trp Arg Glu Leu Trp Val Asp Asp Ala Ile
1               5                   10                  15

Trp Arg Leu Leu Phe Ser Met Ile Leu Phe Val Ile
            20                  25

(2) INFORMATION FOR SEQ ID NO: 579:

(i) SEQUENCE CHARACTERISTICS:
(A) LENGTH: 27 amino acids
(B) TYPE: amino acid
(D) TOPOLOGY: linear
(xi) SEQUENCE DESCRIPTION: SEQ ID NO: 579:

Met Val Leu Trp Arg Pro Ser Ala Asn Asn Gln Arg Phe Ala Phe Ser
1               5                   10                  15

Pro Leu Ser Glu Glu Glu Glu Glu Asp Glu Gln
            20                  25

(2) INFORMATION FOR SEQ ID NO: 580:

(i) SEQUENCE CHARACTERISTICS:
(A) LENGTH: 27 amino acids
(B) TYPE: amino acid
(D) TOPOLOGY: linear
(xi) SEQUENCE DESCRIPTION: SEQ ID NO: 580:

Met Val Leu Trp Arg Pro Ser Ala Asn Asn Gln Arg Phe Ala Phe Ser
1               5                   10                  15

Pro Leu Ser Glu Glu Glu Glu Glu Asp Glu Gln
            20                  25

(2) INFORMATION FOR SEQ ID NO: 581:

(i) SEQUENCE CHARACTERISTICS:
(A) LENGTH: 35 amino acids
(B) TYPE: amino acid
(D) TOPOLOGY: linear
(xi) SEQUENCE DESCRIPTION: SEQ ID NO: 581:

Lys Glu Pro Met Leu Lys Glu Ser Phe Glu Gly Met Lys Met Arg Ser
1               5                   10                  15

Thr Lys Gln Glu Pro Asn Gly Asn Ser Lys Val Asn Lys Ala Gln Glu
            20                  25                  30

Asp Asp Leu
        35

(2) INFORMATION FOR SEQ ID NO: 582:

(i) SEQUENCE CHARACTERISTICS:
(A) LENGTH: 37 amino acids
(B) TYPE: amino acid
(D) TOPOLOGY: linear
(xi) SEQUENCE DESCRIPTION: SEQ ID NO: 582:

Lys Trp Val Glu Glu Asn Val Pro Ser Ser Val Thr Asp Val Ala Leu
1               5                   10                  15

Pro Ala Leu Leu Asp Ser Asp Glu Glu Arg Met Ile Thr His Phe Glu
            20                  25                  30

Arg Ser Lys Met Glu
        35

(2) INFORMATION FOR SEQ ID NO: 583:

(i) SEQUENCE CHARACTERISTICS:
(A) LENGTH: 20 amino acids
(B) TYPE: amino acid
(D) TOPOLOGY: linear

(xi) SEQUENCE DESCRIPTION: SEQ ID NO: 583:

Asp Pro Arg Val Arg Leu Asn Ser Leu Thr Cys Lys His Ile Phe Ile
1               5                   10                  15

Ser Leu Thr Gln
            20

(2) INFORMATION FOR SEQ ID NO: 584:

(i) SEQUENCE CHARACTERISTICS:
(A) LENGTH: 13 amino acids
(B) TYPE: amino acid
(D) TOPOLOGY: linear
(xi) SEQUENCE DESCRIPTION: SEQ ID NO: 584:

Tyr Glu Pro Met Asp Phe Xaa Met Ala Leu Ile Tyr Asp
1               5                   10

(2) INFORMATION FOR SEQ ID NO: 585:

(i) SEQUENCE CHARACTERISTICS:
(A) LENGTH: 16 amino acids
(B) TYPE: amino acid
(D) TOPOLOGY: linear
(xi) SEQUENCE DESCRIPTION: SEQ ID NO: 585:

Ile Arg His Glu Leu Thr Val Leu Arg Asp Thr Arg Pro Ala Cys Ala
1               5                   10                  15

(2) INFORMATION FOR SEQ ID NO: 586:

(i) SEQUENCE CHARACTERISTICS:
(A) LENGTH: 10 amino acids
(B) TYPE: amino acid
(D) TOPOLOGY: linear
(xi) SEQUENCE DESCRIPTION: SEQ ID NO: 586:

Met Asp Phe Xaa Met Ala Leu Ile Tyr Asp
1               5                   10

(2) INFORMATION FOR SEQ ID NO: 587:

(i) SEQUENCE CHARACTERISTICS:
(A) LENGTH: 24 amino acids
(B) TYPE: amino acid
(D) TOPOLOGY: linear
(xi) SEQUENCE DESCRIPTION: SEQ ID NO: 587:

Met Gln Glu Met Met Arg Asn Gln Asp Arg Ala Leu Ser Asn Leu Glu
1               5                   10                  15

Ser Ile Pro Gly Gly Tyr Asn Ala
            20

(2) INFORMATION FOR SEQ ID NO: 588:

(i) SEQUENCE CHARACTERISTICS:
(A) LENGTH: 25 amino acids
(B) TYPE: amino acid
(D) TOPOLOGY: linear
(xi) SEQUENCE DESCRIPTION: SEQ ID NO: 588:

Leu Arg Arg Met Tyr Thr Asp Ile Gln Glu Pro Met Leu Ser Ala Ala
1               5                   10                  15

Gln Glu Gln Phe Gly Gly Asn Pro Phe
            20                  25

(2) INFORMATION FOR SEQ ID NO: 589:

(i) SEQUENCE CHARACTERISTICS:
(A) LENGTH: 32 amino acids
(B) TYPE: amino acid
(D) TOPOLOGY: linear
(xi) SEQUENCE DESCRIPTION: SEQ ID NO: 589:

Ala Ser Leu Val Ser Asn Thr Ser Ser Gly Glu Gly Ser Gln Pro Ser
1               5                   10                  15

Arg Thr Glu Asn Arg Asp Pro Leu Pro Asn Pro Trp Ala Pro Gln Thr
            20                  25                  30

(2) INFORMATION FOR SEQ ID NO: 590:

(i) SEQUENCE CHARACTERISTICS:
(A) LENGTH: 71 amino acids
(B) TYPE: amino acid
(D) TOPOLOGY: linear
(xi) SEQUENCE DESCRIPTION: SEQ ID NO: 590:

Ser Gln Ser Ser Ser Ala Ser Ser Gly Thr Ala Ser Thr Val Gly Gly
1               5                   10                  15

Thr Thr Gly Ser Thr Ala Ser Gly Thr Ser Gly Gln Ser Thr Thr Ala
            20                  25                  30

Pro Asn Leu Val Pro Gly Val Gly Ala Ser Met Phe Asn Thr Pro Gly
        35                  40                  45

Met Gln Ser Leu Leu Gln Gln Ile Thr Glu Asn Pro Gln Leu Met Gln
50                  55                  60

Asn Met Leu Ser Ala Pro Tyr
65                  70

(2) INFORMATION FOR SEQ ID NO: 591:

(i) SEQUENCE CHARACTERISTICS:
(A) LENGTH: 45 amino acids
(B) TYPE: amino acid
(D) TOPOLOGY: linear
(xi) SEQUENCE DESCRIPTION: SEQ ID NO: 591:

Met Arg Ser Met Met Gln Ser Leu Ser Gln Asn Pro Asp Leu Ala Ala
1               5                   10                  15

Gln Met Met Leu Asn Asn Pro Leu Phe Ala Gly Asn Pro Gln Leu Gln
            20                  25                  30

Glu Gln Met Arg Gln Gln Leu Pro Thr Phe Leu Gln Gln
        35                  40                  45

(2) INFORMATION FOR SEQ ID NO: 592:

(i) SEQUENCE CHARACTERISTICS:
(A) LENGTH: 73 amino acids
(B) TYPE: amino acid
(D) TOPOLOGY: linear
(xi) SEQUENCE DESCRIPTION: SEQ ID NO: 592:

Met Gln Asn Pro Asp Thr Leu Ser Ala Met Ser Asn Pro Arg Ala Met
1               5                   10                  15

Gln Ala Leu Leu Gln Ile Gln Gln Gly Leu Gln Thr Leu Ala Thr Glu
            20                  25                  30

Ala Pro Gly Leu Ile Pro Gly Phe Thr Pro Gly Leu Gly Ala Leu Gly
        35                  40                  45

Ser Thr Gly Gly Ser Ser Gly Thr Asn Gly Ser Asn Ala Thr Pro Ser
    50                  55                  60

Glu Asn Thr Ser Pro Thr Ala Gly Thr
65                  70

(2) INFORMATION FOR SEQ ID NO: 593:

(i) SEQUENCE CHARACTERISTICS:
(A) LENGTH: 72 amino acids
(B) TYPE: amino acid
(D) TOPOLOGY: linear
(xi) SEQUENCE DESCRIPTION: SEQ ID NO: 593:

Thr Glu Pro Gly His Gln Gln Phe Ile Gln Gln Met Leu Gln Ala Leu
1               5                   10                  15

Ala Gly Val Asn Pro Gln Leu Gln Asn Pro Glu Val Arg Phe Gln Gln
            20                  25                  30

Gln Leu Glu Gln Leu Ser Ala Met Gly Phe Leu Asn Arg Glu Ala Asn
        35                  40                  45

Leu Gln Ala Leu Ile Ala Thr Gly Gly Asp Ile Asn Ala Ala Ile Glu
    50                  55                  60

Arg Leu Leu Gly Ser Gln Pro Ser
65                  70

(2) INFORMATION FOR SEQ ID NO: 594:

(i) SEQUENCE CHARACTERISTICS:
(A) LENGTH: 45 amino acids
(B) TYPE: amino acid
(D) TOPOLOGY: linear
(xi) SEQUENCE DESCRIPTION: SEQ ID NO: 594:

Arg Asn Pro Ala Met Met Gln Glu Met Met Arg Asn Gln Asp Arg Ala
1               5                   10                  15

Leu Ser Asn Leu Glu Ser Ile Pro Gly Gly Tyr Asn Ala Leu Arg Arg
            20                  25                  30

Met Tyr Thr Asp Ile Gln Glu Pro Met Leu Ser Ala Ala
        35                  40                  45

(2) INFORMATION FOR SEQ ID NO: 595:

(i) SEQUENCE CHARACTERISTICS:
(A) LENGTH: 13 amino acids
(B) TYPE: amino acid
(D) TOPOLOGY: linear
(xi) SEQUENCE DESCRIPTION: SEQ ID NO: 595:

Gly Asn Pro Phe Ala Ser Leu Val Ser Asn Thr Ser Ser
1               5                   10

(2) INFORMATION FOR SEQ ID NO: 596:

(i) SEQUENCE CHARACTERISTICS:
(A) LENGTH: 11 amino acids
(B) TYPE: amino acid
(D) TOPOLOGY: linear
(xi) SEQUENCE DESCRIPTION: SEQ ID NO: 596:

Glu Asn Arg Asp Pro Leu Pro Asn Pro Trp Ala
1               5                   10

(2) INFORMATION FOR SEQ ID NO: 597:

(i) SEQUENCE CHARACTERISTICS:
(A) LENGTH: 17 amino acids
(B) TYPE: amino acid
(D) TOPOLOGY: linear
(xi) SEQUENCE DESCRIPTION: SEQ ID NO: 597:

Gly Lys Ile Leu Lys Asp Gln Asp Thr Leu Ser Gln His Gly Ile His
1               5                   10                  15

Asp

(2) INFORMATION FOR SEQ ID NO: 598:

(i) SEQUENCE CHARACTERISTICS:
(A) LENGTH: 14 amino acids
(B) TYPE: amino acid
(D) TOPOLOGY: linear
(xi) SEQUENCE DESCRIPTION: SEQ ID NO: 598:

Gly Leu Thr Val His Leu Val Ile Lys Thr Gln Asn Arg Pro
1               5                   10

(2) INFORMATION FOR SEQ ID NO: 599:

(i) SEQUENCE CHARACTERISTICS:
(A) LENGTH: 18 amino acids
(B) TYPE: amino acid
(D) TOPOLOGY: linear
(xi) SEQUENCE DESCRIPTION: SEQ ID NO: 599:

Ser Glu Leu Gln Ser Gln Met Gln Arg Gln Leu Leu Ser Asn Pro Glu
1               5                   10                  15

Met Met

(2) INFORMATION FOR SEQ ID NO: 600:

(i) SEQUENCE CHARACTERISTICS:
(A) LENGTH: 14 amino acids
(B) TYPE: amino acid
(D) TOPOLOGY: linear
(xi) SEQUENCE DESCRIPTION: SEQ ID NO: 600:

Pro Glu Ile Ser His Met Leu Asn Asn Pro Asp Ile Met Arg
1               5                   10

(2) INFORMATION FOR SEQ ID NO: 601:

(i) SEQUENCE CHARACTERISTICS:
(A) LENGTH: 18 amino acids
(B) TYPE: amino acid
(D) TOPOLOGY: linear
(xi) SEQUENCE DESCRIPTION: SEQ ID NO: 601:

Arg Gln Leu Ile Met Ala Asn Pro Gln Met Gln Gln Leu Ile Gln Arg
1               5                   10                  15

Asn Pro

(2) INFORMATION FOR SEQ ID NO: 602:

(i) SEQUENCE CHARACTERISTICS:
(A) LENGTH: 27 amino acids
(B) TYPE: amino acid
(D) TOPOLOGY: linear
(xi) SEQUENCE DESCRIPTION: SEQ ID NO: 602:

Asn Leu Cys His Val Asp Cys Gln Asp Leu Leu Asn Pro Asn Leu Leu
1               5                   10                  15

Ala Gly Ile His Cys Ala Lys Arg Ile Val Ser
            20                  25

(2) INFORMATION FOR SEQ ID NO: 603:

(i) SEQUENCE CHARACTERISTICS:
(A) LENGTH: 23 amino acids
(B) TYPE: amino acid
(D) TOPOLOGY: linear
(xi) SEQUENCE DESCRIPTION: SEQ ID NO: 603:

Leu Asp Gly Phe Glu Gly Tyr Ser Leu Ser Asp Trp Leu Cys Leu Ala
1               5                   10                  15

Phe Val Glu Ser Lys Phe Asn
            20

(2) INFORMATION FOR SEQ ID NO: 604:

(i) SEQUENCE CHARACTERISTICS:
(A) LENGTH: 22 amino acids
(B) TYPE: amino acid
(D) TOPOLOGY: linear
(xi) SEQUENCE DESCRIPTION: SEQ ID NO: 604:

Asn Glu Asn Ala Asp Gly Ser Phe Asp Tyr Gly Leu Phe Gln Ile Asn
1               5                   10                  15

Ser His Tyr Trp Cys Asn

            20

(2) INFORMATION FOR SEQ ID NO: 605:

(i) SEQUENCE CHARACTERISTICS:
(A) LENGTH: 27 amino acids
(B) TYPE: amino acid
(D) TOPOLOGY: linear
(xi) SEQUENCE DESCRIPTION: SEQ ID NO: 605:

Asn Leu Cys His Val Asp Cys Gln Asp Leu Leu Asn Pro Asn Leu Leu
1               5                   10                  15

Ala Gly Ile His Cys Ala Lys Arg Ile Val Ser
            20                  25

(2) INFORMATION FOR SEQ ID NO: 606:

(i) SEQUENCE CHARACTERISTICS:
(A) LENGTH: 13 amino acids
(B) TYPE: amino acid
(D) TOPOLOGY: linear
(xi) SEQUENCE DESCRIPTION: SEQ ID NO: 606:

Ile Arg Glu Val Asn Glu Val Ile Gln Asn Pro Ala Thr
1               5                   10

(2) INFORMATION FOR SEQ ID NO: 607:

(i) SEQUENCE CHARACTERISTICS:
(A) LENGTH:-30 amino acids
(B) TYPE: amino acid
(D) TOPOLOGY: linear
(xi) SEQUENCE DESCRIPTION: SEQ ID NO: 607:

Ile Thr Arg Ile Leu Leu Ser His Phe Asn Trp Asp Lys Glu Lys Leu
1               5                   10                  15

Met Glu Arg Tyr Phe Asp Gly Asn Leu Glu Lys Leu Phe Ala
            20                  25                  30

(2) INFORMATION FOR SEQ ID NO: 608:

(i) SEQUENCE CHARACTERISTICS:
(A) LENGTH: 23 amino acids
(B) TYPE: amino acid
(D) TOPOLOGY: linear
(xi) SEQUENCE DESCRIPTION: SEQ ID NO: 608:

Asn Thr Arg Ser Ser Ala Gln Asp Met Pro Cys Gln Ile Cys Tyr Leu
1               5                   10                  15

Asn Tyr Pro Asn Ser Tyr Phe
            20

(2) INFORMATION FOR SEQ ID NO: 609:

      (i) SEQUENCE CHARACTERISTICS:
            (A) LENGTH: 60 amino acids
            (B) TYPE: amino acid
            (D) TOPOLOGY: linear
      (xi) SEQUENCE DESCRIPTION: SEQ ID NO: 609:

Cys Asp Ile Leu Val Asp Asp Asn Thr Val Met Arg Leu Ile Thr Asp
 1               5                  10                  15

Ser Lys Val Lys Leu Lys Tyr Gln His Leu Ile Thr Asn Ser Phe Val
            20                  25                  30

Glu Cys Asn Arg Leu Leu Lys Trp Cys Pro Ala Pro Asp Cys His His
        35                  40                  45

Val Val Lys Val Gln Tyr Pro Asp Ala Lys Pro Val
    50                  55                  60

(2) INFORMATION FOR SEQ ID NO: 610:

      (i) SEQUENCE CHARACTERISTICS:
            (A) LENGTH: 52 amino acids
            (B) TYPE: amino acid
            (D) TOPOLOGY: linear
      (xi) SEQUENCE DESCRIPTION: SEQ ID NO: 610:

Cys Asp Ile Leu Val Asp Asp Asn Thr Val Met Arg Leu Ile Thr Asp
 1               5                  10                  15

Ser Lys Val Lys Leu Lys Tyr Gln His Leu Ile Thr Asn Ser Phe Val
            20                  25                  30

Glu Cys Asn Arg Leu Leu Lys Trp Cys Pro Ala Pro Asp Cys His His
        35                  40                  45

Val Val Lys Val
    50

(2) INFORMATION FOR SEQ ID NO: 611:

      (i) SEQUENCE CHARACTERISTICS:
            (A) LENGTH: 60 amino acids
            (B) TYPE: amino acid
            (D) TOPOLOGY: linear
      (xi) SEQUENCE DESCRIPTION: SEQ ID NO: 611:

Gly Cys Asn His Met Val Cys Arg Asn Gln Asn Cys Lys Ala Glu Phe
 1               5                  10                  15

Cys Trp Val Cys Leu Gly Pro Trp Glu Pro His Gly Ser Ala Trp Tyr
            20                  25                  30

Asn Cys Asn Arg Tyr Asn Glu Asp Asp Ala Lys Ala Ala Arg Asp Ala
        35                  40                  45

Gln Glu Arg Ser Arg Ala Ala Leu Gln Arg Tyr Leu
    50                  55                  60

(2) INFORMATION FOR SEQ ID NO: 612:

(i) SEQUENCE CHARACTERISTICS:
(A) LENGTH: 60 amino acids
(B) TYPE: amino acid
(D) TOPOLOGY: linear
(xi) SEQUENCE DESCRIPTION: SEQ ID NO: 612:

Phe Tyr Cys Asn Arg Tyr Met Asn His Met Gln Ser Leu Arg Phe Glu
1               5                   10                  15

His Lys Leu Tyr Ala Gln Val Lys Gln Lys Met Glu Glu Met Gln Gln
            20                  25                  30

His Asn Met Ser Trp Ile Glu Val Gln Phe Leu Lys Lys Ala Val Asp
        35                  40                  45

Val Leu Cys Gln Cys Arg Ala Thr Leu Met Tyr Thr
    50                  55                  60

(2) INFORMATION FOR SEQ ID NO: 613:

(i) SEQUENCE CHARACTERISTICS:
(A) LENGTH: 60 amino acids
(B) TYPE: amino acid
(D) TOPOLOGY: linear
(xi) SEQUENCE DESCRIPTION: SEQ ID NO: 613:

Tyr Val Phe Ala Phe Tyr Leu Lys Lys Asn Asn Gln Ser Ile Ile Phe
1               5                   10                  15

Glu Asn Asn Gln Ala Asp Leu Glu Asn Ala Thr Glu Val Leu Ser Gly
            20                  25                  30

Tyr Leu Glu Arg Asp Ile Ser Gln Asp Ser Leu Gln Asp Ile Lys Gln
        35                  40                  45

Lys Val Gln Asp Lys Tyr Arg Tyr Cys Glu Ser Arg
    50                  55                  60

(2) INFORMATION FOR SEQ ID NO: 614:

(i) SEQUENCE CHARACTERISTICS:
(A) LENGTH: 37 amino acids
(B) TYPE: amino acid

(D) TOPOLOGY: linear
(xi) SEQUENCE DESCRIPTION: SEQ ID NO: 614:

Thr Gly Leu Glu Cys Gly His Lys Phe Cys Met Gln Cys Trp Ser Glu
1               5                   10                  15

Tyr Leu Thr Thr Lys Ile Met Glu Glu Gly Met Gly Gln Thr Ile Ser
            20                  25                  30

Cys Pro Ala His Gly
        35

(2) INFORMATION FOR SEQ ID NO: 615:

(i) SEQUENCE CHARACTERISTICS:
(A) LENGTH: 21 amino acids
(B) TYPE: amino acid
(D) TOPOLOGY: linear
(xi) SEQUENCE DESCRIPTION: SEQ ID NO: 615:

Met Trp Gly Tyr Leu Phe Val Asp Ala Ala Trp Asn Phe Leu Gly Cys
1               5                   10                  15

Leu Ile Cys Gly Trp
            20

(2) INFORMATION FOR SEQ ID NO: 616:

(i) SEQUENCE CHARACTERISTICS:
(A) LENGTH: 46 amino acids
(B) TYPE: amino acid
(D) TOPOLOGY: linear
(xi) SEQUENCE DESCRIPTION: SEQ ID NO: 616:

Met His Phe Ile Ser Ser Gly Asn Val Ser Ala Ile Arg Ser Ser Ile
1               5                   10                  15

Leu Leu Leu Arg Xaa Ser Leu Ser Tyr Leu Gly Asn Cys Leu Arg Val
            20                  25                  30

Ser Ala Ile Phe Val Tyr Phe Leu Leu Phe Leu Leu Leu Ser
        35                  40                  45

(2) INFORMATION FOR SEQ ID NO: 617:

(i) SEQUENCE CHARACTERISTICS:
(A) LENGTH: 80 amino acids
(B) TYPE: amino acid
(D) TOPOLOGY: linear
(xi) SEQUENCE DESCRIPTION: SEQ ID NO: 617:

(2) INFORMATION FOR SEQ ID NO: 618:

(i) SEQUENCE CHARACTERISTICS:
(A) LENGTH: 131 amino acids
(B) TYPE: amino acid
(D) TOPOLOGY: linear
(xi) SEQUENCE DESCRIPTION: SEQ ID NO: 618:

Met Leu Pro Ala Leu Ala Ser Cys Cys His Phe Ser Pro Pro Glu Gln
1               5                   10                  15

Ala Ala Arg Leu Lys Lys Leu Gln Glu Gln Glu Lys Gln Gln Lys Val
            20                  25                  30

Glu Phe Arg Lys Arg Met Glu Lys Glu Val Ser Asp Phe Ile Gln Asp
        35                  40                  45

Ser Gly Gln Ile Lys Lys Lys Phe Gln Pro Met Asn Lys Ile Glu Arg
    50                  55                  60

Ser Ile Leu His Asp Val Val Glu Val Ala Gly Leu Thr Ser Phe Ser
65                  70                  75                  80

Phe Gly Glu Asp Asp Asp Cys Arg Tyr Val Met Ile Phe Lys Lys Glu
                85                  90                  95

Phe Ala Pro Ser Asp Glu Glu Leu Asp Ser Tyr Arg Arg Gly Glu Glu
            100                 105                 110

Trp Asp Pro Gln Lys Ala Glu Glu Lys Arg Asn Xaa Lys Glu Leu Ala
        115                 120                 125

Gln Arg Gln
    130

(2) INFORMATION FOR SEQ ID NO: 619:

(i) SEQUENCE CHARACTERISTICS:
(A) LENGTH: 76 amino acids
(B) TYPE: amino acid
(D) TOPOLOGY: linear

(xi) SEQUENCE DESCRIPTION: SEQ ID NO: 619:

Glu Glu Glu Ala Ala Gln Gln Gly Pro Val Val Val Ser Pro Ala Ser
1               5                   10                  15

Asp Tyr Lys Asp Lys Tyr Ser His Leu Ile Gly Lys Gly Ala Ala Lys
            20                  25                  30

Asp Ala Ala His Met Leu Gln Ala Asn Lys Thr Tyr Gly Cys Xaa Pro
        35                  40                  45

Val Ala Asn Lys Arg Asp Thr Arg Ser Ile Glu Glu Ala Met Asn Glu
    50                  55                  60

Ile Arg Ala Lys Lys Arg Leu Arg Gln Ser Gly Glu
65                  70                  75

(2) INFORMATION FOR SEQ ID NO: 620:

(i) SEQUENCE CHARACTERISTICS:
(A) LENGTH: 40 amino acids
(B) TYPE: amino acid
(D) TOPOLOGY: linear
(xi) SEQUENCE DESCRIPTION: SEQ ID NO: 620:

Pro Pro Arg Arg Pro Ala Gln Leu Pro Leu Thr Pro Gly Ala Gly Gln
1               5                   10                  15

Gly Ala Gly Arg Asp Lys Ala Ala Ala Ile Arg Ala His Pro Gly Ala
            20                  25                  30

Pro Pro Leu Asn His Leu Leu Pro
        35                  40

(2) INFORMATION FOR SEQ ID NO: 621:

(i) SEQUENCE CHARACTERISTICS:
(A) LENGTH: 28 amino acids
(B) TYPE: amino acid
(D) TOPOLOGY: linear
(xi) SEQUENCE DESCRIPTION: SEQ ID NO: 621:

Ala Val Pro Gln Ala Gly Gly Lys Gln Val Phe Asp Leu Ser Pro Leu
1               5                   10                  15

Glu Leu Gly Tyr Val Arg Gly Met Cys Val Cys Val
            20                  25

(2) INFORMATION FOR SEQ ID NO: 622:

(i) SEQUENCE CHARACTERISTICS:
(A) LENGTH: 207 amino acids
(B) TYPE: amino acid
(D) TOPOLOGY: linear

(xi) SEQUENCE DESCRIPTION: SEQ ID NO: 622:

Met Leu Pro Ala Leu Ala Ser Cys Cys His Phe Ser Pro Pro Glu Gln
1               5                   10                  15

Ala Ala Arg Leu Lys Lys Leu Gln Glu Gln Glu Lys Gln Gln Lys Val
            20                  25                  30

Glu Phe Arg Lys Arg Met Glu Lys Glu Val Ser Asp Phe Ile Gln Asp
        35                  40                  45

Ser Gly Gln Ile Lys Lys Lys Phe Gln Pro Met Asn Lys Ile Glu Arg
    50                  55                  60

Ser Ile Leu His Asp Val Val Glu Val Ala Gly Leu Thr Ser Phe Ser
65                  70                  75                  80

Phe Gly Glu Asp Asp Asp Cys Arg Tyr Val Met Ile Phe Lys Lys Glu
                85                  90                  95

Phe Ala Pro Ser Asp Glu Glu Leu Asp Ser Tyr Arg Arg Gly Glu Glu
            100                 105                 110

Trp Asp Pro Gln Lys Ala Glu Glu Lys Arg Asn Xaa Lys Glu Leu Ala
        115                 120                 125

Gln Arg Gln Glu Glu Glu Ala Ala Gln Gln Gly Pro Val Val Val Ser
    130                 135                 140

Pro Ala Ser Asp Tyr Lys Asp Lys Tyr Ser His Leu Ile Gly Lys Gly
145                 150                 155                 160

Ala Ala Lys Asp Ala Ala His Met Leu Gln Ala Asn Lys Thr Tyr Gly
                165                 170                 175

Cys Xaa Pro Val Ala Asn Lys Arg Asp Thr Arg Ser Ile Glu Glu Ala
            180                 185                 190

Met Asn Glu Ile Arg Ala Lys Lys Arg Leu Arg Gln Ser Gly Glu
        195                 200                 205

(2) INFORMATION FOR SEQ ID NO: 623:

(i) SEQUENCE CHARACTERISTICS:
(A) LENGTH: 34 amino acids
(B) TYPE: amino acid
(D) TOPOLOGY: linear
(xi) SEQUENCE DESCRIPTION: SEQ ID NO: 623:

Leu Leu Cys Pro Val Leu Asn Ser Gly Xaa Ser Trp Asn Phe Pro His
1               5                   10                  15

Pro Ser Gln Pro Glu Tyr Ser Phe His Gly Phe His Ser Thr Arg Leu
            20                  25                  30

Trp Ile

(2) INFORMATION FOR SEQ ID NO: 624:

(i) SEQUENCE CHARACTERISTICS:
(A) LENGTH: 28 amino acids
(B) TYPE: amino acid
(D) TOPOLOGY: linear
(xi) SEQUENCE DESCRIPTION: SEQ ID NO: 624:

Pro Ser Thr Pro Trp Phe Leu Phe Leu Leu Gly Leu Thr Cys Pro Phe
1               5                   10                  15

Ser Thr Ser His Pro Arg Trp Asp Ser Ile Pro Pro
            20                  25

(2) INFORMATION FOR SEQ ID NO: 625:

(i) SEQUENCE CHARACTERISTICS:
(A) LENGTH: 227 amino acids
(B) TYPE: amino acid
(D) TOPOLOGY: linear
(xi) SEQUENCE DESCRIPTION: SEQ ID NO: 625:

Glu Leu Ser Ile Ser Ile Ser Asn Val Ala Leu Ala Asp Glu Gly Glu
1               5                   10                  15

Tyr Thr Cys Ser Ile Phe Thr Met Pro Val Arg Thr Ala Lys Ser Leu
            20                  25                  30

Val Thr Val Leu Gly Ile Pro Gln Lys Pro Ile Ile Thr Gly Tyr Lys
        35                  40                  45

Ser Ser Leu Arg Glu Lys Asp Thr Ala Thr Leu Asn Cys Gln Ser Ser
    50                  55                  60

Gly Ser Lys Pro Ala Ala Arg Leu Thr Trp Arg Lys Gly Asp Gln Glu
65                  70                  75                  80

Leu His Gly Glu Pro Thr Arg Ile Gln Glu Asp Pro Asn Gly Lys Thr
                85                  90                  95

Phe Thr Val Ser Ser Ser Val Thr Phe Gln Val Thr Arg Glu Asp Asp
            100                 105                 110

Gly Ala Ser Ile Val Cys Ser Val Asn His Glu Ser Leu Lys Gly Ala
        115                 120                 125

Asp Arg Ser Thr Ser Gln Arg Ile Glu Val Leu Tyr Thr Pro Thr Ala
    130                 135                 140

Met Ile Arg Pro Asp Pro Pro His Pro Arg Glu Gly Gln Lys Leu Leu
145                 150                 155                 160

Leu His Cys Glu Gly Arg Gly Asn Pro Val Pro Gln Gln Tyr Leu Trp
                165                 170                 175

Glu Lys Glu Gly Ser Val Pro Pro Leu Lys Met Thr Gln Glu Ser Ala

180                 185                 190

Leu Ile Phe Pro Phe Leu Asn Lys Ser Asp Ser Gly Thr Tyr Gly Cys
        195                 200                 205

Thr Ala Thr Ser Asn Met Gly Ser Tyr Lys Ala Tyr Tyr Thr Leu Asn
    210                 215                 220

Val Asn Asp
225

(2) INFORMATION FOR SEQ ID NO: 626:

(i) SEQUENCE CHARACTERISTICS:
(A) LENGTH: 64 amino acids
(B) TYPE: amino acid
(D) TOPOLOGY: linear
(xi) SEQUENCE DESCRIPTION: SEQ ID NO: 626:

Glu Leu Ser Ile Ser Ile Ser Asn Val Ala Leu Ala Asp Glu Gly Glu
1               5                   10                  15

Tyr Thr Cys Ser Ile Phe Thr Met Pro Val Arg Thr Ala Lys Ser Leu
            20                  25                  30

Val Thr Val Leu Gly Ile Pro Gln Lys Pro Ile Ile Thr Gly Tyr Lys
        35                  40                  45

Ser Ser Leu Arg Glu Lys Asp Thr Ala Thr Leu Asn Cys Gln Ser Ser
    50                  55                  60

(2) INFORMATION FOR SEQ ID NO: 627:

(i) SEQUENCE CHARACTERISTICS:
(A) LENGTH: 65 amino acids
(B) TYPE: amino acid
(D) TOPOLOGY: linear
(xi) SEQUENCE DESCRIPTION: SEQ ID NO: 627:

Cys Gln Ser Ser Gly Ser Lys Pro Ala Ala Arg Leu Thr Trp Arg Lys
1               5                   10                  15

Gly Asp Gln Glu Leu His Gly Glu Pro Thr Arg Ile Gln Glu Asp Pro
            20                  25                  30

Asn Gly Lys Thr Phe Thr Val Ser Ser Ser Val Thr Phe Gln Val Thr
        35                  40                  45

Arg Glu Asp Asp Gly Ala Ser Ile Val Cys Ser Val Asn His Glu Ser
    50                  55                  60

Leu
65

(2) INFORMATION FOR SEQ ID NO: 628:

(i) SEQUENCE CHARACTERISTICS:
(A) LENGTH: 58 amino acids
(B) TYPE: amino acid
(D) TOPOLOGY: linear
(xi) SEQUENCE DESCRIPTION: SEQ ID NO: 628:

His Glu Ser Leu Lys Gly Ala Asp Arg Ser Thr Ser Gln Arg Ile Glu
1               5                   10                  15

Val Leu Tyr Thr Pro Thr Ala Met Ile Arg Pro Asp Pro Pro His Pro
            20                  25                  30

Arg Glu Gly Gln Lys Leu Leu Leu His Cys Glu Gly Arg Gly Asn Pro
        35                  40                  45

Val Pro Gln Gln Tyr Leu Trp Glu Lys Glu
    50                  55

(2) INFORMATION FOR SEQ ID NO: 629:

(i) SEQUENCE CHARACTERISTICS:
(A) LENGTH: 52 amino acids
(B) TYPE: amino acid
(D) TOPOLOGY: linear
(xi) SEQUENCE DESCRIPTION: SEQ ID NO: 629:

Trp Glu Lys Glu Gly Ser Val Pro Pro Leu Lys Met Thr Gln Glu Ser
1               5                   10                  15

Ala Leu Ile Phe Pro Phe Leu Asn Lys Ser Asp Ser Gly Thr Tyr Gly
            20                  25                  30

Cys Thr Ala Thr Ser Asn Met Gly Ser Tyr Lys Ala Tyr Tyr Thr Leu
        35                  40                  45

Asn Val Asn Asp
    50

(2) INFORMATION FOR SEQ ID NO: 630:

(i) SEQUENCE CHARACTERISTICS:
(A) LENGTH: 123 amino acids
(B) TYPE: amino acid
(D) TOPOLOGY: linear
(xi) SEQUENCE DESCRIPTION: SEQ ID NO: 630:

Val Pro Glu Leu Pro Asp Arg Val His Gln Leu His Gln Ala Val Gln
1               5                   10                  15

Gly Cys Ala Leu Gly Arg Pro Gly Phe Pro Gly Gly Pro Thr His Ser
            20                  25                  30

Gly His His Lys Ser His Pro Gly Pro Ala Gly Gly Asp Tyr Asn Arg
        35                  40                  45

Cys Asp Arg Pro Gly Gln Val His Leu His Asn Pro Arg Gly Thr Gly
    50                  55                  60

Arg Arg Gly Gln Leu His Pro Thr Ala Gly Pro Gly Val His Arg Arg
65                  70                  75                  80

Ala Cys Pro Ser Gln Gln Leu Pro His Arg Leu Gly Pro Gly Val Pro
                85                  90                  95

Cys Pro Ser Pro Ser Leu Thr Pro Val Leu Pro Ser Trp Thr Gln Ser
            100                 105                 110

Trp Cys Gly Leu Pro Gly Tyr Thr Ser Ser Ser
        115                 120

(2) INFORMATION FOR SEQ ID NO: 631:

(i) SEQUENCE CHARACTERISTICS:
(A) LENGTH: 22 amino acids
(B) TYPE: amino acid
(D) TOPOLOGY: linear
(xi) SEQUENCE DESCRIPTION: SEQ ID NO: 631:

Val His Gln Leu His Gln Ala Val Gln Gly Cys Ala Leu Gly Arg Pro
1               5                   10                  15

Gly Phe Pro Gly Gly Pro
            20

(2) INFORMATION FOR SEQ ID NO: 632:

(i) SEQUENCE CHARACTERISTICS:
(A) LENGTH: 42 amino acids
(B) TYPE: amino acid
(D) TOPOLOGY: linear
(xi) SEQUENCE DESCRIPTION: SEQ ID NO: 632:

Pro Thr His Ser Gly His His Lys Ser His Pro Gly Pro Ala Gly Gly
1               5                   10                  15

Asp Tyr Asn Arg Cys Asp Arg Pro Gly Gln Val His Leu His Asn Pro
            20                  25                  30

Arg Gly Thr Gly Arg Arg Gly Gln Leu His
        35                  40

(2) INFORMATION FOR SEQ ID NO: 633:

(i) SEQUENCE CHARACTERISTICS:
(A) LENGTH: 55 amino acids

(B) TYPE: amino acid
(D) TOPOLOGY: linear
(xi) SEQUENCE DESCRIPTION: SEQ ID NO: 633:

Leu His Pro Thr Ala Gly Pro Gly Val His Arg Arg Ala Cys Pro Ser
1               5                   10                  15

Gln Gln Leu Pro His Arg Leu Gly Pro Gly Val Pro Cys Pro Ser Pro
            20                  25                  30

Ser Leu Thr Pro Val Leu Pro Ser Trp Thr Gln Ser Trp Cys Gly Leu
        35                  40                  45

Pro Gly Tyr Thr Ser Ser Ser
    50                  55

(2) INFORMATION FOR SEQ ID NO: 634:

(i) SEQUENCE CHARACTERISTICS:
(A) LENGTH: 276 amino acids
(B) TYPE: amino acid
(D) TOPOLOGY: linear
(xi) SEQUENCE DESCRIPTION: SEQ ID NO: 634:

Ser Leu Arg Arg Pro Arg Ser Ala Ala Xaa Gln Thr Leu Thr Thr Phe
1               5                   10                  15

Leu Ser Ser Val Ser Ser Ala Ser Ser Ser Ala Leu Pro Gly Ser Arg
            20                  25                  30

Glu Pro Cys Asp Pro Arg Ala Pro Pro Pro Pro Arg Ser Gly Ser Ala
        35                  40                  45

Ala Ser Cys Cys Ser Cys Cys Cys Ser Cys Pro Arg Arg Arg Ala Pro
    50                  55                  60

Leu Arg Ser Pro Arg Gly Ser Lys Arg Arg Ile Arg Gln Arg Glu Val
65                  70                  75                  80

Val Asp Leu Tyr Asn Gly Met Cys Leu Gln Gly Pro Ala Gly Val Pro
                85                  90                  95

Gly Arg Asp Gly Ser Pro Gly Ala Asn Gly Ile Pro Gly Thr Pro Gly
            100                 105                 110

Ile Pro Gly Arg Asp Gly Phe Lys Gly Glu Lys Gly Glu Cys Leu Arg
        115                 120                 125

Glu Ser Phe Glu Glu Ser Trp Thr Pro Asn Tyr Lys Gln Cys Ser Trp
    130                 135                 140

Ser Ser Leu Asn Tyr Gly Ile Asp Leu Gly Lys Ile Ala Glu Cys Thr
145                 150                 155                 160

Phe Thr Lys Met Arg Ser Asn Ser Ala Leu Arg Val Leu Phe Ser Gly
                165                 170                 175

Ser Leu Arg Leu Lys Cys Arg Asn Ala Cys Cys Gln Arg Trp Tyr Phe

            180                 185                 190

Thr Phe Asn Gly Ala Glu Cys Ser Gly Pro Leu Pro Ile Glu Ala Ile
        195                 200                 205

Ile Tyr Leu Asp Gln Gly Ser Pro Glu Met Asn Ser Thr Ile Asn Ile
    210                 215                 220

His Arg Thr Ser Ser Val Glu Gly Leu Cys Glu Gly Ile Gly Ala Gly
225                 230                 235                 240

Leu Val Asp Val Ala Ile Trp Val Gly Thr Cys Ser Asp Tyr Pro Lys
                245                 250                 255

Gly Asp Ala Ser Thr Gly Trp Asn Ser Val Ser Arg Ile Ile Ile Glu
            260                 265                 270

Glu Leu Pro Lys
        275

(2) INFORMATION FOR SEQ ID NO: 635:

(i) SEQUENCE CHARACTERISTICS:
(A) LENGTH: 61 amino acids
(B) TYPE: amino acid
(D) TOPOLOGY: linear
(xi) SEQUENCE DESCRIPTION: SEQ ID NO: 635:

Ser Leu Arg Arg Pro Arg Ser Ala Ala Xaa Gln Thr Leu Thr Thr Phe
1               5                   10                  15

Leu Ser Ser Val Ser Ser Ala Ser Ser Ser Ala Leu Pro Gly Ser Arg
            20                  25                  30

Glu Pro Cys Asp Pro Arg Ala Pro Pro Pro Pro Arg Ser Gly Ser Ala
        35                  40                  45

Ala Ser Cys Cys Ser Cys Cys Cys Ser Cys Pro Arg Arg
    50                  55                  60

(2) INFORMATION FOR SEQ ID NO: 636:

(i) SEQUENCE CHARACTERISTICS:
(A) LENGTH: 52 amino acids
(B) TYPE: amino acid
(D) TOPOLOGY: linear
(xi) SEQUENCE DESCRIPTION: SEQ ID NO: 636:

Arg Ala Pro Leu Arg Ser Pro Arg Gly Ser Lys Arg Arg Ile Arg Gln
1               5                   10                  15

Arg Glu Val Val Asp Leu Tyr Asn Gly Met Cys Leu Gln Gly Pro Ala
            20                  25                  30

Gly Val Pro Gly Arg Asp Gly Ser Pro Gly Ala Asn Gly Ile Pro Gly
        35                  40                  45

Thr Pro Gly Ile
     50

(2) INFORMATION FOR SEQ ID NO: 637:

        (i) SEQUENCE CHARACTERISTICS:
              (A) LENGTH: 52 amino acids
              (B) TYPE: amino acid
              (D) TOPOLOGY: linear
        (xi) SEQUENCE DESCRIPTION: SEQ ID NO: 637:

Thr Pro Gly Ile Pro Gly Arg Asp Gly Phe Lys Gly Glu Lys Gly Glu
  1               5                  10                  15

Cys Leu Arg Glu Ser Phe Glu Glu Ser Trp Thr Pro Asn Tyr Lys Gln
             20                  25                  30

Cys Ser Trp Ser Ser Leu Asn Tyr Gly Ile Asp Leu Gly Lys Ile Ala
         35                  40                  45

Glu Cys Thr Phe
     50

(2) INFORMATION FOR SEQ ID NO: 638:

        (i) SEQUENCE CHARACTERISTICS:
              (A) LENGTH: 66 amino acids
              (B) TYPE: amino acid
              (D) TOPOLOGY: linear
        (xi) SEQUENCE DESCRIPTION: SEQ ID NO: 638:

Phe Thr Lys Met Arg Ser Asn Ser Ala Leu Arg Val Leu Phe Ser Gly
  1               5                  10                  15

Ser Leu Arg Leu Lys Cys Arg Asn Ala Cys Cys Gln Arg Trp Tyr Phe
             20                  25                  30

Thr Phe Asn Gly Ala Glu Cys Ser Gly Pro Leu Pro Ile Glu Ala Ile
         35                  40                  45

Ile Tyr Leu Asp Gln Gly Ser Pro Glu Met Asn Ser Thr Ile Asn Ile
     50                  55                  60

His Arg
65

(2) INFORMATION FOR SEQ ID NO: 639:

        (i) SEQUENCE CHARACTERISTICS:
              (A) LENGTH: 51 amino acids
              (B) TYPE: amino acid
              (D) TOPOLOGY: linear
        (xi) SEQUENCE DESCRIPTION: SEQ ID NO: 639:

Arg Thr Ser Ser Val Glu Gly Leu Cys Glu Gly Ile Gly Ala Gly Leu
1               5                   10                  15

Val Asp Val Ala Ile Trp Val Gly Thr Cys Ser Asp Tyr Pro Lys Gly
            20                  25                  30

Asp Ala Ser Thr Gly Trp Asn Ser Val Ser Arg Ile Ile Ile Glu Glu
        35                  40                  45

Leu Pro Lys
    50

(2) INFORMATION FOR SEQ ID NO: 640:

(i) SEQUENCE CHARACTERISTICS:
(A) LENGTH: 26 amino acids
(B) TYPE: amino acid
(D) TOPOLOGY: linear
(xi) SEQUENCE DESCRIPTION: SEQ ID NO: 640:

Thr Lys Lys Glu Asn Cys Arg Pro Ala Ser Leu Met Asn Ile Asp Thr
1               5                   10                  15

Lys Ile Leu Asn Lys Ile Leu Met Asn Gln
            20                  25

(2) INFORMATION FOR SEQ ID NO: 641:

(i) SEQUENCE CHARACTERISTICS:
(A) LENGTH: 214 amino acids
(B) TYPE: amino acid
(D) TOPOLOGY: linear
(xi) SEQUENCE DESCRIPTION: SEQ ID NO: 641:

Met Cys Asn Leu Pro Ile Lys Val Val Cys Arg Ala Asn Ala Glu Tyr
1               5                   10                  15

Met Ser Pro Ser Gly Lys Val Pro Xaa Xaa His Val Gly Asn Gln Val
            20                  25                  30

Val Ser Glu Leu Gly Pro Ile Val Gln Phe Val Lys Ala Lys Gly His
        35                  40                  45

Ser Leu Ser Asp Gly Leu Glu Glu Val Gln Lys Ala Glu Met Lys Ala
    50                  55                  60

Tyr Met Glu Leu Val Asn Asn Met Leu Leu Thr Ala Glu Leu Tyr Leu
65                  70                  75                  80

Gln Trp Cys Asp Glu Ala Thr Val Gly Xaa Ile Thr His Xaa Arg Tyr
                85                  90                  95

Gly Ser Pro Tyr Pro Trp Pro Leu Xaa His Ile Leu Ala Tyr Gln Lys
            100                 105                 110

Gln Trp Glu Val Lys Arg Lys Xaa Lys Ala Ile Gly Trp Gly Lys Lys
        115                 120                 125

Thr Leu Asp Gln Val Leu Glu Asp Val Asp Gln Cys Cys Gln Ala Leu
    130                 135                 140

Ser Gln Arg Leu Gly Thr Gln Pro Tyr Phe Phe Asn Lys Gln Pro Thr
145                 150                 155                 160

Glu Leu Asp Ala Leu Val Phe Gly His Leu Tyr Thr Ile Leu Thr Thr
                165                 170                 175

Gln Leu Thr Asn Asp Glu Leu Ser Glu Lys Val Lys Asn Tyr Ser Asn
            180                 185                 190

Leu Leu Ala Phe Cys Arg Arg Ile Glu Gln His Tyr Phe Glu Asp Arg
        195                 200                 205

Gly Lys Gly Arg Leu Ser
    210

(2) INFORMATION FOR SEQ ID NO: 642:

(i) SEQUENCE CHARACTERISTICS:
(A) LENGTH: 44 amino acids
(B) TYPE: amino acid
(D) TOPOLOGY: linear
(xi) SEQUENCE DESCRIPTION: SEQ ID NO: 642:

Met Cys Asn Leu Pro Ile Lys Val Val Cys Arg Ala Asn Ala Glu Tyr
1               5                   10                  15

Met Ser Pro Ser Gly Lys Val Pro Xaa Xaa His Val Gly Asn Gln Val
            20                  25                  30

Val Ser Glu Leu Gly Pro Ile Val Gln Phe Val Lys
        35                  40

(2) INFORMATION FOR SEQ ID NO: 643:

(i) SEQUENCE CHARACTERISTICS:
(A) LENGTH: 44 amino acids
(B) TYPE: amino acid
(D) TOPOLOGY: linear
(xi) SEQUENCE DESCRIPTION: SEQ ID NO: 643:

Phe Val Lys Ala Lys Gly His Ser Leu Ser Asp Gly Leu Glu Glu Val
1               5                   10                  15

Gln Lys Ala Glu Met Lys Ala Tyr Met Glu Leu Val Asn Asn Met Leu
            20                  25                  30

Leu Thr Ala Glu Leu Tyr Leu Gln Trp Cys Asp Glu
        35                  40

(2) INFORMATION FOR SEQ ID NO: 644:

(i) SEQUENCE CHARACTERISTICS:
(A) LENGTH: 51 amino acids
(B) TYPE: amino acid
(D) TOPOLOGY: linear
(xi) SEQUENCE DESCRIPTION: SEQ ID NO: 644:

Leu Gln Trp Cys Asp Glu Ala Thr Val Gly Xaa Ile Thr His Xaa Arg
1               5                   10                  15

Tyr Gly Ser Pro Tyr Pro Trp Pro Leu Xaa His Ile Leu Ala Tyr Gln
            20                  25                  30

Lys Gln Trp Glu Val Lys Arg Lys Xaa Lys Ala Ile Gly Trp Gly Lys
        35                  40                  45

Lys Thr Leu
    50

(2) INFORMATION FOR SEQ ID NO: 645:

(i) SEQUENCE CHARACTERISTICS:
(A) LENGTH: 43 amino acids
(B) TYPE: amino acid
(D) TOPOLOGY: linear
(xi) SEQUENCE DESCRIPTION: SEQ ID NO: 645:

Asp Gln Val Leu Glu Asp Val Asp Gln Cys Cys Gln Ala Leu Ser Gln
1               5                   10                  15

Arg Leu Gly Thr Gln Pro Tyr Phe Phe Asn Lys Gln Pro Thr Glu Leu
            20                  25                  30

Asp Ala Leu Val Phe Gly His Leu Tyr Thr Ile
        35                  40

(2) INFORMATION FOR SEQ ID NO: 646:

(i) SEQUENCE CHARACTERISTICS:
(A) LENGTH: 41 amino acids
(B) TYPE: amino acid
(D) TOPOLOGY: linear
(xi) SEQUENCE DESCRIPTION: SEQ ID NO: 646:

Leu Thr Thr Gln Leu Thr Asn Asp Glu Leu Ser Glu Lys Val Lys Asn
1               5                   10                  15

Tyr Ser Asn Leu Leu Ala Phe Cys Arg Arg Ile Glu Gln His Tyr Phe
            20                  25                  30

Glu Asp Arg Gly Lys Gly Arg Leu Ser
        35                  40

(2) INFORMATION FOR SEQ ID NO: 647:

(i) SEQUENCE CHARACTERISTICS:
(A) LENGTH: 70 amino acids
(B) TYPE: amino acid
(D) TOPOLOGY: linear
(xi) SEQUENCE DESCRIPTION: SEQ ID NO: 647:

Met Xaa Xaa Xaa Asn Ser His Ile Thr Ile Phe Thr Leu Asn Val Asn
1               5                   10                  15

Gly Leu Asn Ala Pro Asn Glu Arg His Arg Leu Ala Asn Trp Ile Gln
            20                  25                  30

Ser Gln Asp Gln Val Cys Cys Ile Gln Glu Thr His Leu Thr Gly Arg
        35                  40                  45

Asp Thr His Arg Leu Lys Ile Lys Gly Trp Arg Lys Ile Tyr Gln Ala
    50                  55                  60

Asn Gly Lys Gln Lys Lys
65                  70

(2) INFORMATION FOR SEQ ID NO: 648:

(i) SEQUENCE CHARACTERISTICS:
(A) LENGTH: 28 amino acids
(B) TYPE: amino acid
(D) TOPOLOGY: linear
(xi) SEQUENCE DESCRIPTION: SEQ ID NO: 648:

Phe Thr Leu Asn Val Asn Gly Leu Asn Ala Pro Asn Glu Arg His Arg
1               5                   10                  15

Leu Ala Asn Trp Ile Gln Ser Gln Asp Gln Val Cys
            20                  25

(2) INFORMATION FOR SEQ ID NO: 649:

(i) SEQUENCE CHARACTERISTICS:
(A) LENGTH: 17 amino acids
(B) TYPE: amino acid
(D) TOPOLOGY: linear
(xi) SEQUENCE DESCRIPTION: SEQ ID NO: 649:

Thr His Leu Thr Gly Arg Asp Thr His Arg Leu Lys Ile Lys Gly Trp
1               5                   10                  15

Arg

(2) INFORMATION FOR SEQ ID NO: 650:

(i) SEQUENCE CHARACTERISTICS:
(A) LENGTH: 14 amino acids
(B) TYPE: amino acid
(D) TOPOLOGY: linear
(xi) SEQUENCE DESCRIPTION: SEQ ID NO: 650:

Gly Trp Arg Lys Ile Tyr Gln Ala Asn Gly Lys Gln Lys Lys
1               5                   10

(2) INFORMATION FOR SEQ ID NO: 651:

(i) SEQUENCE CHARACTERISTICS:
(A) LENGTH: 54 amino acids
(B) TYPE: amino acid
(D) TOPOLOGY: linear
(xi) SEQUENCE DESCRIPTION: SEQ ID NO: 651:

Ile Tyr His Leu His Ser Trp Ile Phe Phe His Phe Lys Arg Ala Phe
1               5                   10                  15

Cys Met Cys Phe Ile Thr Met Lys Val Ile His Ala His Cys Ser Lys
            20                  25                  30

Leu Arg Lys Cys Xaa Asn Ala Gln Ile Ser Val Phe Cys Thr Thr Leu
        35                  40                  45

Thr Ala Ser Tyr Pro Thr
    50

(2) INFORMATION FOR SEQ ID NO: 652:

(i) SEQUENCE CHARACTERISTICS:
(A) LENGTH: 23 amino acids
(B) TYPE: amino acid
(D) TOPOLOGY: linear
(xi) SEQUENCE DESCRIPTION: SEQ ID NO: 652:

Ile Tyr His Leu His Ser Trp Ile Phe Phe His Phe Lys Arg Ala Phe
1               5                   10                  15

Cys Met Cys Phe Ile Thr Met
            20

(2) INFORMATION FOR SEQ ID NO: 653:

(i) SEQUENCE CHARACTERISTICS:
(A) LENGTH: 31 amino acids
(B) TYPE: amino acid
(D) TOPOLOGY: linear
(xi) SEQUENCE DESCRIPTION: SEQ ID NO: 653:

Lys Val Ile His Ala His Cys Ser Lys Leu Arg Lys Cys Xaa Asn Ala
1               5                   10                  15

Gln Ile Ser Val Phe Cys Thr Thr Leu Thr Ala Ser Tyr Pro Thr
            20                  25                  30

(2) INFORMATION FOR SEQ ID NO: 654:

      (i) SEQUENCE CHARACTERISTICS:
            (A) LENGTH: 58 amino acids
            (B) TYPE: amino acid
            (D) TOPOLOGY: linear
      (xi) SEQUENCE DESCRIPTION: SEQ ID NO: 654:

Trp Asn Leu Leu Trp Tyr Phe Gln Arg Leu Arg Leu Pro Ser Ile Leu
  1               5                  10                  15

Pro Gly Leu Val Leu Ala Ser Cys Asp Gly Pro Ser Xaa Ser Gln Ala
            20                  25                  30

Pro Ser Pro Trp Leu Thr Pro Asp Pro Ala Ser Val Gln Val Arg Leu
        35                  40                  45

Leu Trp Asp Val Leu Thr Pro Asp Pro Asn
    50                  55

(2) INFORMATION FOR SEQ ID NO: 655:

      (i) SEQUENCE CHARACTERISTICS:
            (A) LENGTH: 54 amino acids
            (B) TYPE: amino acid
            (D) TOPOLOGY: linear
      (xi) SEQUENCE DESCRIPTION: SEQ ID NO: 655:

Gln Arg Gly Ile Tyr Arg Glu Ile Leu Phe Leu Thr Met Ala Ala Leu
  1               5                  10                  15

Gly Lys Asp His Val Asp Ile Val Ala Phe Asp Lys Lys Tyr Lys Ser
            20                  25                  30

Ala Phe Asn Lys Leu Ala Ser Ser Met Gly Lys Glu Glu Leu Arg His
        35                  40                  45

Arg Arg Ala Gln Met Pro
    50

(2) INFORMATION FOR SEQ ID NO: 656:

      (i) SEQUENCE CHARACTERISTICS:
            (A) LENGTH: 23 amino acids
            (B) TYPE: amino acid
            (D) TOPOLOGY: linear
      (xi) SEQUENCE DESCRIPTION: SEQ ID NO: 656:

Trp Asn Leu Leu Trp Tyr Phe Gln Arg Leu Arg Leu Pro Ser Ile Leu
  1               5                  10                  15

Pro Gly Leu Val Leu Ala Ser
            20

(2) INFORMATION FOR SEQ ID NO: 657:

      (i) SEQUENCE CHARACTERISTICS:
            (A) LENGTH: 191 amino acids
            (B) TYPE: amino acid
            (D) TOPOLOGY: linear
      (xi) SEQUENCE DESCRIPTION: SEQ ID NO: 657:

Glu Asp Asp Gly Phe Asn Arg Ser Ile His Glu Val Ile Leu Lys Asn
 1               5                  10                  15

Ile Thr Trp Tyr Ser Glu Arg Val Leu Thr Glu Ile Ser Leu Gly Ser
            20                  25                  30

Leu Leu Ile Leu Val Val Ile Arg Thr Ile Gln Tyr Asn Met Thr Arg
        35                  40                  45

Thr Arg Asp Lys Tyr Leu His Thr Asn Cys Leu Ala Ala Leu Ala Asn
    50                  55                  60

Met Ser Ala Gln Phe Arg Ser Leu His Gln Tyr Ala Ala Gln Arg Ile
65                  70                  75                  80

Ile Ser Leu Phe Ser Leu Leu Ser Lys Lys His Asn Lys Val Leu Glu
                85                  90                  95

Gln Ala Thr Gln Ser Leu Arg Gly Ser Leu Ser Ser Asn Asp Val Pro
            100                 105                 110

Leu Pro Asp Tyr Ala Gln Asp Leu Asn Val Ile Glu Glu Val Ile Arg
        115                 120                 125

Met Met Leu Glu Ile Ile Asn Ser Cys Leu Thr Asn Ser Leu His His
    130                 135                 140

Asn Pro Asn Leu Val Tyr Ala Leu Leu Tyr Lys Arg Asp Leu Phe Glu
145                 150                 155                 160

Gln Phe Arg Thr His Pro Ser Phe Gln Asp Ile Met Gln Asn Ile Asp
                165                 170                 175

Leu Val Ile Ser Phe Phe Ser Ser Arg Leu Leu Gln Ala Gly Ser
            180                 185                 190

(2) INFORMATION FOR SEQ ID NO: 658:

      (i) SEQUENCE CHARACTERISTICS:
            (A) LENGTH: 38 amino acids
            (B) TYPE: amino acid
            (D) TOPOLOGY: linear
      (xi) SEQUENCE DESCRIPTION: SEQ ID NO: 658:

Glu Asp Asp Gly Phe Asn Arg Ser Ile His Glu Val Ile Leu Lys Asn

1               5                   10                  15

Ile Thr Trp Tyr Ser Glu Arg Val Leu Thr Glu Ile Ser Leu Gly Ser
            20                  25                  30

Leu Leu Ile Leu Val Val
        35

(2) INFORMATION FOR SEQ ID NO: 659:

(i) SEQUENCE CHARACTERISTICS:
(A) LENGTH: 53 amino acids
(B) TYPE: amino acid
(D) TOPOLOGY: linear
(xi) SEQUENCE DESCRIPTION: SEQ ID NO: 659:

Arg Thr Ile Gln Tyr Asn Met Thr Arg Thr Arg Asp Lys Tyr Leu His
1               5                   10                  15

Thr Asn Cys Leu Ala Ala Leu Ala Asn Met Ser Ala Gln Phe Arg Ser
            20                  25                  30

Leu His Gln Tyr Ala Ala Gln Arg Ile Ile Ser Leu Phe Ser Leu Leu
        35                  40                  45

Ser Lys Lys His Asn
    50

(2) INFORMATION FOR SEQ ID NO: 660:

(i) SEQUENCE CHARACTERISTICS:
(A) LENGTH: 56 amino acids
(B) TYPE: amino acid
(D) TOPOLOGY: linear
(xi) SEQUENCE DESCRIPTION: SEQ ID NO: 660:

Ser Cys Leu Thr Asn Ser Leu His His Asn Pro Asn Leu Val Tyr Ala
1               5                   10                  15

Leu Leu Tyr Lys Arg Asp Leu Phe Glu Gln Phe Arg Thr His Pro Ser
            20                  25                  30

Phe Gln Asp Ile Met Gln Asn Ile Asp Leu Val Ile Ser Phe Phe Ser
        35                  40                  45

Ser Arg Leu Leu Gln Ala Gly Ser
    50                  55

(2) INFORMATION FOR SEQ ID NO: 661:

(i) SEQUENCE CHARACTERISTICS:
(A) LENGTH: 31 amino acids
(B) TYPE: amino acid
(D) TOPOLOGY: linear

(xi) SEQUENCE DESCRIPTION: SEQ ID NO: 661:

Lys Lys His Asn Lys Val Leu Glu Gln Ala Thr Gln Ser Leu Arg Gly
1               5                   10                  15

Ser Leu Ser Ser Asn Asp Val Pro Leu Pro Asp Tyr Ala Gln Asp
            20                  25                  30

(2) INFORMATION FOR SEQ ID NO: 662:

(i) SEQUENCE CHARACTERISTICS:
(A) LENGTH: 125 amino acids
(B) TYPE: amino acid
(D) TOPOLOGY: linear
(xi) SEQUENCE DESCRIPTION: SEQ ID NO: 662:

(2) INFORMATION FOR SEQ ID NO: 663:

(i) SEQUENCE CHARACTERISTICS:
(A) LENGTH: 79 amino acids
(B) TYPE: amino acid
(D) TOPOLOGY: linear
(xi) SEQUENCE DESCRIPTION: SEQ ID NO: 663:

Met Lys Met Gln Arg Thr Ile Val Ile Arg Arg Asp Tyr Leu His Tyr
1               5                   10                  15

Ile Arg Lys Tyr Asn Arg Phe Glu Lys Arg His Lys Asn Met Ser Val
            20                  25                  30

His Leu Ser Pro Cys Phe Arg Asp Val Gln Ile Gly Asp Ile Val Thr
        35                  40                  45

Val Gly Glu Cys Arg Pro Leu Ser Lys Thr Val Arg Phe Asn Val Leu
    50                  55                  60

Lys Val Thr Lys Ala Ala Gly Thr Lys Lys Gln Phe Gln Lys Phe
65                  70                  75

(2) INFORMATION FOR SEQ ID NO: 664:

(i) SEQUENCE CHARACTERISTICS:
(A) LENGTH: 30 amino acids
(B) TYPE: amino acid
(D) TOPOLOGY: linear
(xi) SEQUENCE DESCRIPTION: SEQ ID NO: 664:

Met Ala Asp Ile Gln Thr Glu Arg Ala Tyr Gln Lys Gln Pro Thr Ile
1               5                   10                  15

Phe Gln Asn Lys Lys Arg Val Leu Leu Gly Glu Thr Gly Lys
            20                  25                  30

(2) INFORMATION FOR SEQ ID NO: 665:

(i) SEQUENCE CHARACTERISTICS:
(A) LENGTH: 58 amino acids
(B) TYPE: amino acid
(D) TOPOLOGY: linear
(xi) SEQUENCE DESCRIPTION: SEQ ID NO: 665:

Lys Leu Pro Arg Val Thr Asn Lys Asn Ile Gly Leu Gly Phe Lys Asp
1               5                   10                  15

Thr Pro Arg Arg Leu Leu Arg Gly Thr Tyr Ile Asp Lys Lys Cys Pro
            20                  25                  30

Phe Thr Gly Asn Val Ser Ile Arg Gly Arg Ile Leu Ser Gly Val Val
        35                  40                  45

Thr Gln Asp Glu Asp Ala Glu Asp His Cys
    50                  55

(2) INFORMATION FOR SEQ ID NO: 666:

(i) SEQUENCE CHARACTERISTICS:
(A) LENGTH: 38 amino acids
(B) TYPE: amino acid
(D) TOPOLOGY: linear
(xi) SEQUENCE DESCRIPTION: SEQ ID NO: 666:

His Cys His Pro Pro Arg Leu Ser Ala Leu His Pro Gln Val Gln Pro
1               5                   10                  15

Leu Arg Glu Ala Pro Gln Glu His Val Cys Thr Pro Val Pro Leu Leu
            20                  25                  30

Gln Gly Arg Pro Asp Arg
        35

(2) INFORMATION FOR SEQ ID NO: 667:

        (i) SEQUENCE CHARACTERISTICS:
                (A) LENGTH: 36 amino acids
                (B) TYPE: amino acid
                (D) TOPOLOGY: linear
        (xi) SEQUENCE DESCRIPTION: SEQ ID NO: 667:

Met Lys Met Gln Arg Thr Ile Val Ile Arg Arg Asp Tyr Leu His Tyr
  1               5                  10                  15

Ile Arg Lys Tyr Asn Arg Phe Glu Lys Arg His Lys Asn Met Ser Val
            20                  25                  30

His Leu Ser Pro
        35

(2) INFORMATION FOR SEQ ID NO: 668:

        (i) SEQUENCE CHARACTERISTICS:
                (A) LENGTH: 43 amino acids
                (B) TYPE: amino acid
                (D) TOPOLOGY: linear
        (xi) SEQUENCE DESCRIPTION: SEQ ID NO: 668:

Cys Phe Arg Asp Val Gln Ile Gly Asp Ile Val Thr Val Gly Glu Cys
  1               5                  10                  15

Arg Pro Leu Ser Lys Thr Val Arg Phe Asn Val Leu Lys Val Thr Lys
            20                  25                  30

Ala Ala Gly Thr Lys Lys Gln Phe Gln Lys Phe
        35                  40

(2) INFORMATION FOR SEQ ID NO: 669:

        (i) SEQUENCE CHARACTERISTICS:
                (A) LENGTH: 33 amino acids
                (B) TYPE: amino acid
                (D) TOPOLOGY: linear
        (xi) SEQUENCE DESCRIPTION: SEQ ID NO: 669:

Pro Arg Arg Leu Leu Arg Gly Thr Tyr Ile Asp Lys Lys Cys Pro Phe
  1               5                  10                  15

Thr Gly Asn Val Ser Ile Arg Gly Arg Ile Leu Ser Gly Val Val Thr
            20                  25                  30

Gln

(2) INFORMATION FOR SEQ ID NO: 670:

(i) SEQUENCE CHARACTERISTICS:
(A) LENGTH: 60 amino acids
(B) TYPE: amino acid
(D) TOPOLOGY: linear
(xi) SEQUENCE DESCRIPTION: SEQ ID NO: 670:

Ile Phe Tyr Asp Ser Asp Trp Asn Pro Thr Val Asp Gln Gln Ala Met
1               5                   10                  15

Asp Arg Ala His Arg Leu Gly Gln Thr Lys Gln Val Thr Val Tyr Arg
            20                  25                  30

Leu Ile Cys Lys Gly Thr Ile Glu Glu Arg Ile Leu Gln Arg Ala Lys
        35                  40                  45

Glu Lys Ser Glu Ile Gln Arg Met Val Ile Ser Gly
    50                  55                  60

(2) INFORMATION FOR SEQ ID NO: 671:

(i) SEQUENCE CHARACTERISTICS:
(A) LENGTH: 67 amino acids
(B) TYPE: amino acid
(D) TOPOLOGY: linear
(xi) SEQUENCE DESCRIPTION: SEQ ID NO: 671:

Thr Arg Met Ile Asp Leu Leu Glu Glu Tyr Met Val Tyr Arg Lys His
1               5                   10                  15

Thr Tyr Xaa Arg Leu Asp Gly Ser Ser Lys Ile Ser Glu Arg Arg Asp
            20                  25                  30

Met Val Ala Asp Phe Gln Asn Arg Asn Asp Ile Phe Val Phe Leu Leu
        35                  40                  45

Ser Thr Arg Ala Gly Gly Leu Gly Ile Asn Leu Thr Ala Xaa Asp Thr
    50                  55                  60

Val His Phe
65

(2) INFORMATION FOR SEQ ID NO: 672:

(i) SEQUENCE CHARACTERISTICS:
(A) LENGTH: 32 amino acids
(B) TYPE: amino acid
(D) TOPOLOGY: linear
(xi) SEQUENCE DESCRIPTION: SEQ ID NO: 672:

Ile Phe Tyr Asp Ser Asp Trp Asn Pro Thr Val Asp Gln Gln Ala Met
1               5                   10                  15

Asp Arg Ala His Arg Leu Gly Gln Thr Lys Gln Val Thr Val Tyr Arg
            20                  25                  30

(2) INFORMATION FOR SEQ ID NO: 673:

      (i) SEQUENCE CHARACTERISTICS:
            (A) LENGTH: 31 amino acids
            (B) TYPE: amino acid
            (D) TOPOLOGY: linear
      (xi) SEQUENCE DESCRIPTION: SEQ ID NO: 673:

Val Tyr Arg Leu Ile Cys Lys Gly Thr Ile Glu Glu Arg Ile Leu Gln
  1               5                  10                  15

Arg Ala Lys Glu Lys Ser Glu Ile Gln Arg Met Val Ile Ser Gly
            20                  25                  30

(2) INFORMATION FOR SEQ ID NO: 674:

      (i) SEQUENCE CHARACTERISTICS:
            (A) LENGTH: 33 amino acids
            (B) TYPE: amino acid
            (D) TOPOLOGY: linear
      (xi) SEQUENCE DESCRIPTION: SEQ ID NO: 674:

Thr Arg Met Ile Asp Leu Leu Glu Glu Tyr Met Val Tyr Arg Lys His
  1               5                  10                  15

Thr Tyr Xaa Arg Leu Asp Gly Ser Ser Lys Ile Ser Glu Arg Arg Asp
            20                  25                  30

Met

(2) INFORMATION FOR SEQ ID NO: 675:

      (i) SEQUENCE CHARACTERISTICS:
            (A) LENGTH: 38 amino acids
            (B) TYPE: amino acid
            (D) TOPOLOGY: linear
      (xi) SEQUENCE DESCRIPTION: SEQ ID NO: 675:

Arg Arg Asp Met Val Ala Asp Phe Gln Asn Arg Asn Asp Ile Phe Val
  1               5                  10                  15

Phe Leu Leu Ser Thr Arg Ala Gly Gly Leu Gly Ile Asn Leu Thr Ala
            20                  25                  30

Xaa Asp Thr Val His Phe
        35

(2) INFORMATION FOR SEQ ID NO: 676:

(i) SEQUENCE CHARACTERISTICS:
(A) LENGTH: 37 amino acids
(B) TYPE: amino acid
(D) TOPOLOGY: linear
(xi) SEQUENCE DESCRIPTION: SEQ ID NO: 676:

Ile Phe Tyr Asp Ser Asp Trp Asn Pro Thr Val Asp Gln Gln Ala Met
1               5                   10                  15

Asp Arg Ala His Arg Leu Gly Gln Thr Lys Gln Val Thr Val Tyr Arg
            20                  25                  30

Leu Ile Cys Lys Gly
        35

(2) INFORMATION FOR SEQ ID NO: 677:

(i) SEQUENCE CHARACTERISTICS:
(A) LENGTH: 37 amino acids
(B) TYPE: amino acid
(D) TOPOLOGY: linear
(xi) SEQUENCE DESCRIPTION: SEQ ID NO: 677:

Ile Phe Tyr Asp Ser Asp Trp Asn Pro Thr Val Asp Gln Gln Ala Met
1               5                   10                  15

Asp Arg Ala His Arg Leu Gly Gln Thr Lys Gln Val Thr Val Tyr Arg
            20                  25                  30

Leu Ile Cys Lys Gly
        35

(2) INFORMATION FOR SEQ ID NO: 678:

(i) SEQUENCE CHARACTERISTICS:
(A) LENGTH: 29 amino acids
(B) TYPE: amino acid
(D) TOPOLOGY: linear
(xi) SEQUENCE DESCRIPTION: SEQ ID NO: 678:

Arg Leu Ile Cys Lys Gly Thr Ile Glu Glu Arg Ile Leu Gln Arg Ala
1               5                   10                  15

Lys Glu Lys Ser Glu Ile Gln Arg Met Val Ile Ser Gly
            20                  25

(2) INFORMATION FOR SEQ ID NO: 679:

(i) SEQUENCE CHARACTERISTICS:

(A) LENGTH: 364 amino acids
(B) TYPE: amino acid
(D) TOPOLOGY: linear
(xi) SEQUENCE DESCRIPTION: SEQ ID NO: 679:

Met Ser Leu His Gly Lys Arg Lys Glu Ile Tyr Lys Tyr Glu Ala Pro
1               5                   10                  15

Trp Thr Val Tyr Ala Met Asn Trp Ser Val Arg Pro Asp Lys Arg Phe
            20                  25                  30

Arg Leu Ala Leu Gly Ser Phe Val Glu Glu Tyr Asn Asn Lys Val Gln
        35                  40                  45

Leu Val Gly Leu Asp Glu Glu Ser Ser Glu Phe Ile Cys Arg Asn Thr
    50                  55                  60

Phe Asp His Pro Tyr Pro Thr Thr Lys Leu Met Trp Ile Pro Asp Thr
65                  70                  75                  80

Lys Gly Val Tyr Pro Asp Leu Leu Ala Thr Ser Gly Asp Tyr Leu Arg
                85                  90                  95

Val Trp Arg Val Gly Glu Thr Glu Thr Arg Leu Glu Cys Leu Leu Asn
            100                 105                 110

Asn Asn Lys Asn Ser Asp Phe Cys Ala Pro Leu Thr Ser Phe Asp Trp
        115                 120                 125

Asn Glu Val Asp Pro Tyr Leu Leu Gly Thr Ser Ser Ile Asp Thr Thr
    130                 135                 140

Cys Thr Ile Trp Gly Leu Glu Thr Gly Gln Val Leu Gly Arg Val Asn
145                 150                 155                 160

Leu Val Ser Gly His Val Lys Thr Gln Leu Ile Ala His Asp Lys Glu
                165                 170                 175

Val Tyr Asp Ile Ala Phe Ser Arg Ala Gly Gly Gly Arg Asp Met Phe
            180                 185                 190

Ala Ser Val Gly Ala Asp Gly Ser Val Arg Met Phe Asp Leu Arg His
        195                 200                 205

Leu Glu His Ser Thr Ile Ile Tyr Glu Asp Pro Gln His His Pro Leu
    210                 215                 220

Leu Arg Leu Cys Trp Asn Lys Gln Asp Pro Asn Tyr Leu Ala Thr Met
225                 230                 235                 240

Ala Met Asp Gly Met Glu Val Val Ile Leu Asp Val Arg Val Pro Ala
                245                 250                 255

His Leu Xaa Pro Gly Thr Thr Ile Glu His Val Ser Met Ala Leu Leu
            260                 265                 270

Gly Pro His Ile His Pro Ala Thr Ser Ala Leu Gln Arg Met Thr Thr
        275                 280                 285

Arg Leu Ser Ser Gly Thr Ser Ser Lys Cys Pro Glu Pro Leu Arg Thr

290                 295                 300

Leu Ser Trp Pro Thr Gln Leu Xaa Gly Glu Ile Asn Asn Val Gln Trp
305                 310                 315                 320

Ala Ser Thr Gln Pro Glu Leu Ser Pro Ser Ala Thr Thr Thr Ala Trp
                325                 330                 335

Arg Tyr Ser Glu Cys Ser Val Gly Gly Ala Val Pro Thr Arg Gln Gly
            340                 345                 350

Leu Leu Tyr Phe Leu Pro Leu Pro His Pro Gln Ser
        355                 360

(2) INFORMATION FOR SEQ ID NO: 680:

(i) SEQUENCE CHARACTERISTICS:
(A) LENGTH: 136 amino acids
(B) TYPE: amino acid
(D) TOPOLOGY: linear
(xi) SEQUENCE DESCRIPTION: SEQ ID NO: 680:

Met Ser Leu His Gly Lys Arg Lys Glu Ile Tyr Lys Tyr Glu Ala Pro
1               5                   10                  15

Trp Thr Val Tyr Ala Met Asn Trp Ser Val Arg Pro Asp Lys Arg Phe
            20                  25                  30

Arg Leu Ala Leu Gly Ser Phe Val Glu Glu Tyr Asn Asn Lys Val Gln
        35                  40                  45

Leu Val Gly Leu Asp Glu Glu Ser Ser Glu Phe Ile Cys Arg Asn Thr
    50                  55                  60

Phe Asp His Pro Tyr Pro Thr Thr Lys Leu Met Trp Ile Pro Asp Thr
65                  70                  75                  80

Lys Gly Val Tyr Pro Asp Leu Leu Ala Thr Ser Gly Asp Tyr Leu Arg
                85                  90                  95

Val Trp Arg Val Gly Glu Thr Glu Thr Arg Leu Glu Cys Leu Leu Asn
            100                 105                 110

Asn Asn Lys Asn Ser Asp Phe Cys Ala Pro Leu Thr Ser Phe Asp Trp
        115                 120                 125

Asn Glu Val Asp Pro Tyr Leu Leu
    130                 135

(2) INFORMATION FOR SEQ ID NO: 681:

(i) SEQUENCE CHARACTERISTICS:
(A) LENGTH: 140 amino acids
(B) TYPE: amino acid
(D) TOPOLOGY: linear
(xi) SEQUENCE DESCRIPTION: SEQ ID NO: 681:

Ser Phe Asp Trp Asn Glu Val Asp Pro Tyr Leu Leu Gly Thr Ser Ser
1               5                   10                  15

Ile Asp Thr Thr Cys Thr Ile Trp Gly Leu Glu Thr Gly Gln Val Leu
            20                  25                  30

Gly Arg Val Asn Leu Val Ser Gly His Val Lys Thr Gln Leu Ile Ala
        35                  40                  45

His Asp Lys Glu Val Tyr Asp Ile Ala Phe Ser Arg Ala Gly Gly Gly
    50                  55                  60

Arg Asp Met Phe Ala Ser Val Gly Ala Asp Gly Ser Val Arg Met Phe
65                  70                  75                  80

Asp Leu Arg His Leu Glu His Ser Thr Ile Ile Tyr Glu Asp Pro Gln
                85                  90                  95

His His Pro Leu Leu Arg Leu Cys Trp Asn Lys Gln Asp Pro Asn Tyr
            100                 105                 110

Leu Ala Thr Met Ala Met Asp Gly Met Glu Val Val Ile Leu Asp Val
        115                 120                 125

Arg Val Pro Ala His Leu Xaa Pro Gly Thr Thr Ile
    130                 135                 140

(2) INFORMATION FOR SEQ ID NO: 682:

(i) SEQUENCE CHARACTERISTICS:
(A) LENGTH: 170 amino acids
(B) TYPE: amino acid
(D) TOPOLOGY: linear
(xi) SEQUENCE DESCRIPTION: SEQ ID NO: 682:

Val Gly Ala Asp Gly Ser Val Arg Met Phe Asp Leu Arg His Leu Glu
1               5                   10                  15

His Ser Thr Ile Ile Tyr Glu Asp Pro Gln His His Pro Leu Leu Arg
            20                  25                  30

Leu Cys Trp Asn Lys Gln Asp Pro Asn Tyr Leu Ala Thr Met Ala Met
        35                  40                  45

Asp Gly Met Glu Val Val Ile Leu Asp Val Arg Val Pro Ala His Leu
    50                  55                  60

Xaa Pro Gly Thr Thr Ile Glu His Val Ser Met Ala Leu Leu Gly Pro
65                  70                  75                  80

His Ile His Pro Ala Thr Ser Ala Leu Gln Arg Met Thr Thr Arg Leu
                85                  90                  95

Ser Ser Gly Thr Ser Ser Lys Cys Pro Glu Pro Leu Arg Thr Leu Ser
            100                 105                 110

Trp Pro Thr Gln Leu Xaa Gly Glu Ile Asn Asn Val Gln Trp Ala Ser

115                 120                 125

Thr Gln Pro Glu Leu Ser Pro Ser Ala Thr Thr Thr Ala Trp Arg Tyr
    130                 135                 140

Ser Glu Cys Ser Val Gly Gly Ala Val Pro Thr Arg Gln Gly Leu Leu
145                 150                 155                 160

Tyr Phe Leu Pro Leu Pro His Pro Gln Ser
                165                 170

(2) INFORMATION FOR SEQ ID NO: 683:

(i) SEQUENCE CHARACTERISTICS:
(A) LENGTH: 286 amino acids
(B) TYPE: amino acid
(D) TOPOLOGY: linear
(xi) SEQUENCE DESCRIPTION: SEQ ID NO: 683:

Leu Tyr Ala Thr Ala Thr Val Ile Ser Ser Pro Ser Thr Glu Xaa Leu
1               5                   10                  15

Ser Gln Asp Gln Gly Asp Arg Ala Ser Leu Asp Ala Ala Asp Ser Gly
            20                  25                  30

Arg Gly Ser Trp Thr Ser Cys Ser Ser Gly Ser His Asp Asn Ile Gln
        35                  40                  45

Thr Ile Gln His Gln Arg Ser Trp Glu Thr Leu Pro Phe Gly His Thr
    50                  55                  60

His Phe Asp Tyr Ser Gly Asp Pro Ala Gly Leu Trp Ala Ser Ser Ser
65                  70                  75                  80

His Met Asp Gln Ile Met Phe Ser Asp His Ser Thr Lys Tyr Asn Arg
                85                  90                  95

Gln Asn Gln Ser Arg Glu Ser Leu Glu Gln Ala Gln Ser Arg Ala Ser
            100                 105                 110

Trp Ala Ser Ser Thr Gly Tyr Trp Gly Glu Asp Ser Glu Gly Asp Thr
        115                 120                 125

Gly Thr Ile Lys Arg Arg Gly Gly Lys Asp Val Ser Ile Glu Ala Glu
    130                 135                 140

Ser Ser Ser Leu Thr Ser Val Thr Thr Glu Glu Thr Lys Pro Val Pro
145                 150                 155                 160

Met Pro Ala His Ile Ala Val Ala Ser Ser Thr Thr Lys Gly Leu Ile
                165                 170                 175

Ala Arg Lys Glu Gly Arg Tyr Arg Glu Pro Pro Pro Thr Pro Pro Gly
            180                 185                 190

Tyr Ile Gly Ile Pro Ile Thr Asp Phe Pro Glu Gly His Ser His Pro
        195                 200                 205

Ala Arg Lys Pro Pro Asp Tyr Asn Val Ala Leu Gln Arg Ser Arg Met
    210                 215                 220

Val Ala Arg Ser Ser Asp Thr Ala Gly Pro Ser Ser Val Gln Gln Pro
225                 230                 235                 240

His Gly His Pro Thr Ser Ser Arg Pro Val Asn Lys Pro Gln Trp His
                245                 250                 255

Lys Xaa Asn Glu Ser Asp Pro Arg Leu Ala Pro Tyr Gln Ser Gln Gly
            260                 265                 270

Phe Ser Thr Glu Glu Asp Glu Asp Glu Gln Val Ser Ala Val
        275                 280                 285

(2) INFORMATION FOR SEQ ID NO: 684:

(i) SEQUENCE CHARACTERISTICS:
(A) LENGTH: 42 amino acids
(B) TYPE: amino acid
(D) TOPOLOGY: linear
(xi) SEQUENCE DESCRIPTION: SEQ ID NO: 684:

His Met Asp Gln Ile Met Phe Ser Asp His Ser Thr Lys Tyr Asn Arg
1               5                   10                  15

Gln Asn Gln Ser Arg Glu Ser Leu Glu Gln Ala Gln Ser Arg Ala Ser
            20                  25                  30

Trp Ala Ser Ser Thr Gly Tyr Trp Gly Glu
        35                  40

(2) INFORMATION FOR SEQ ID NO: 685:

(i) SEQUENCE CHARACTERISTICS:
(A) LENGTH: 51 amino acids
(B) TYPE: amino acid
(D) TOPOLOGY: linear
(xi) SEQUENCE DESCRIPTION: SEQ ID NO: 685:

Ser Val Thr Thr Glu Glu Thr Lys Pro Val Pro Met Pro Ala His Ile
1               5                   10                  15

Ala Val Ala Ser Ser Thr Thr Lys Gly Leu Ile Ala Arg Lys Glu Gly
            20                  25                  30

Arg Tyr Arg Glu Pro Pro Pro Thr Pro Pro Gly Tyr Ile Gly Ile Pro
        35                  40                  45

Ile Thr Asp
    50

(2) INFORMATION FOR SEQ ID NO: 686:

(i) SEQUENCE CHARACTERISTICS:
(A) LENGTH: 57 amino acids
(B) TYPE: amino acid
(D) TOPOLOGY: linear
(xi) SEQUENCE DESCRIPTION: SEQ ID NO: 686:

Val Ala Leu Gln Arg Ser Arg Met Val Ala Arg Ser Ser Asp Thr Ala
1               5                   10                  15

Gly Pro Ser Ser Val Gln Gln Pro His Gly His Pro Thr Ser Ser Arg
            20                  25                  30

Pro Val Asn Lys Pro Gln Trp His Lys Xaa Asn Glu Ser Asp Pro Arg
        35                  40                  45

Leu Ala Pro Tyr Gln Ser Gln Gly Phe
    50                  55

(2) INFORMATION FOR SEQ ID NO: 687:

(i) SEQUENCE CHARACTERISTICS:
(A) LENGTH: 41 amino acids
(B) TYPE: amino acid
(D) TOPOLOGY: linear
(xi) SEQUENCE DESCRIPTION: SEQ ID NO: 687:

Cys Leu Leu Phe Val Phe Val Ser Leu Gly Met Arg Cys Leu Phe Trp
1               5                   10                  15

Thr Ile Val Tyr Asn Val Leu Tyr Leu Lys His Lys Cys Asn Thr Val
            20                  25                  30

Leu Leu Cys Tyr His Leu Cys Ser Ile
        35                  40

(2) INFORMATION FOR SEQ ID NO: 688:

(i) SEQUENCE CHARACTERISTICS:
(A) LENGTH: 67 amino acids
(B) TYPE: amino acid
(D) TOPOLOGY: linear
(xi) SEQUENCE DESCRIPTION: SEQ ID NO: 688:

Ala Cys Ser Lys Leu Ile Pro Ala Phe Glu Met Val Met Arg Ala Lys
1               5                   10                  15

Asp Asn Val Tyr His Leu Asp Cys Phe Ala Cys Gln Leu Cys Asn Gln
            20                  25                  30

Arg Xaa Cys Val Gly Asp Lys Phe Phe Leu Lys Asn Asn Xaa Xaa Leu
        35                  40                  45

Cys Gln Thr Asp Tyr Glu Glu Gly Leu Met Lys Glu Gly Tyr Ala Pro
    50                  55                  60

Xaa Val Arg

65

(2) INFORMATION FOR SEQ ID NO: 689:

(i) SEQUENCE CHARACTERISTICS:
(A) LENGTH: 45 amino acids
(B) TYPE: amino acid
(D) TOPOLOGY: linear
(xi) SEQUENCE DESCRIPTION: SEQ ID NO: 689:

Ser Ala Leu Ser Glu Pro Gly Ala Pro Asp Arg Arg Arg Pro Cys Pro
1               5                   10                  15

Glu Ser Val Pro Arg Arg Pro Asp Asp Glu Gln Trp Pro Pro Pro Thr
            20                  25                  30

Ala Leu Cys Leu Asp Val Ala Pro Leu Pro Pro Ser Ser
        35                  40                  45

(2) INFORMATION FOR SEQ ID NO: 690:

(i) SEQUENCE CHARACTERISTICS:
(A) LENGTH: 43 amino acids
(B) TYPE: amino acid
(D) TOPOLOGY: linear
(xi) SEQUENCE DESCRIPTION: SEQ ID NO: 690:

Pro Val Gly Tyr Leu Asp Lys Gln Val Pro Asp Thr Ser Val Gln Glu
1               5                   10                  15

Thr Asp Arg Ile Leu Val Glu Lys Arg Cys Trp Asp Ile Ala Leu Gly
            20                  25                  30

Pro Leu Lys Gln Ile Pro Met Asn Leu Phe Ile
        35                  40

(2) INFORMATION FOR SEQ ID NO: 691:

(i) SEQUENCE CHARACTERISTICS:
(A) LENGTH: 214 amino acids
(B) TYPE: amino acid
(D) TOPOLOGY: linear
(xi) SEQUENCE DESCRIPTION: SEQ ID NO: 691:

Ala His Ala Ser Glu Ser Gly Glu Arg Trp Trp Ala Cys Cys Gly Val
1               5                   10                  15

Arg Phe Gly Leu Arg Ser Ile Glu Ala Ile Gly Arg Ser Cys Cys His
            20                  25                  30

Asp Gly Pro Gly Gly Leu Val Ala Asn Arg Gly Arg Arg Phe Lys Trp
        35                  40                  45

Ala Ile Glu Leu Ser Gly Pro Gly Gly Gly Ser Arg Gly Arg Ser Asp

50                  55                  60

Arg Gly Ser Gly Gln Gly Asp Ser Leu Tyr Pro Val Gly Tyr Leu Asp
65                  70                  75                  80

Lys Gln Val Pro Asp Thr Ser Val Gln Glu Thr Asp Arg Ile Leu Val
                85                  90                  95

Glu Lys Arg Cys Trp Asp Ile Ala Leu Gly Pro Leu Lys Gln Ile Pro
            100                 105                 110

Met Asn Leu Phe Ile Met Tyr Met Ala Gly Asn Thr Ile Ser Ile Phe
        115                 120                 125

Pro Thr Met Met Val Cys Met Met Ala Trp Arg Pro Ile Gln Ala Leu
    130                 135                 140

Met Ala Ile Ser Ala Thr Phe Lys Met Leu Glu Ser Ser Ser Gln Lys
145                 150                 155                 160

Phe Leu Gln Gly Leu Val Tyr Leu Ile Gly Asn Leu Met Gly Leu Ala
                165                 170                 175

Leu Ala Val Tyr Lys Cys Gln Ser Met Gly Leu Leu Pro Thr His Ala
            180                 185                 190

Ser Asp Trp Leu Ala Phe Ile Glu Pro Pro Glu Arg Met Glu Phe Ser
        195                 200                 205

Gly Gly Gly Leu Leu Leu
    210

(2) INFORMATION FOR SEQ ID NO: 692:

(i) SEQUENCE CHARACTERISTICS:
(A) LENGTH: 46 amino acids
(B) TYPE: amino acid
(D) TOPOLOGY: linear
(xi) SEQUENCE DESCRIPTION: SEQ ID NO: 692:

Ala Thr Phe Lys Met Leu Glu Ser Ser Ser Gln Lys Phe Leu Gln Gly
1               5                   10                  15

Leu Val Tyr Leu Ile Gly Asn Leu Met Gly Leu Ala Leu Ala Val Tyr
            20                  25                  30

Lys Cys Gln Ser Met Gly Leu Leu Pro Thr His Ala Ser Asp
        35                  40                  45

(2) INFORMATION FOR SEQ ID NO: 693:

(i) SEQUENCE CHARACTERISTICS:
(A) LENGTH: 43 amino acids
(B) TYPE: amino acid
(D) TOPOLOGY: linear
(xi) SEQUENCE DESCRIPTION: SEQ ID NO: 693:

Pro Val Gly Tyr Leu Asp Lys Gln Val Pro Asp Thr Ser Val Gln Glu
1               5                   10                  15

Thr Asp Arg Ile Leu Val Glu Lys Arg Cys Trp Asp Ile Ala Leu Gly
            20                  25                  30

Pro Leu Lys Gln Ile Pro Met Asn Leu Phe Ile
        35                  40

(2) INFORMATION FOR SEQ ID NO: 694:

(i) SEQUENCE CHARACTERISTICS:
(A) LENGTH: 48 amino acids
(B) TYPE: amino acid
(D) TOPOLOGY: linear
(xi) SEQUENCE DESCRIPTION: SEQ ID NO: 694:

Pro Thr Thr Lys Leu Asp Ile Met Glu Lys Lys Lys His Ile Gln Ile
1               5                   10                  15

Arg Phe Pro Ser Phe Tyr His Lys Leu Val Asp Ser Gly Arg Met Arg
            20                  25                  30

Ser Lys Arg Glu Thr Arg Arg Glu Asp Ser Asp Thr Lys His Asn Leu
        35                  40                  45

(2) INFORMATION FOR SEQ ID NO: 695:

(i) SEQUENCE CHARACTERISTICS:
(A) LENGTH: 167 amino acids
(B) TYPE: amino acid
(D) TOPOLOGY: linear
(xi) SEQUENCE DESCRIPTION: SEQ ID NO: 695:

Thr Glu His Ile Ile Ala Val Met Ile Thr Glu Leu Arg Gly Lys Asp
1               5                   10                  15

Ile Leu Ser Tyr Leu Glu Lys Asn Ile Ser Val Gln Met Thr Ile Ala
            20                  25                  30

Val Gly Thr Arg Met Pro Pro Lys Asn Phe Ser Arg Gly Ser Leu Val
        35                  40                  45

Phe Val Ser Ile Ser Phe Ile Val Leu Met Ile Ile Ser Ser Ala Trp
    50                  55                  60

Leu Ile Phe Tyr Phe Ile Gln Lys Ile Arg Tyr Thr Asn Ala Arg Asp
65                  70                  75                  80

Arg Asn Gln Arg Arg Leu Gly Asp Ala Ala Lys Lys Ala Ile Ser Lys
                85                  90                  95

Leu Thr Thr Arg Thr Val Lys Lys Gly Asp Lys Glu Thr Asp Pro Asp
            100                 105                 110

Phe Asp His Cys Ala Val Cys Ile Glu Ser Tyr Lys Gln Asn Asp Val
        115                 120                 125

Val Arg Ile Leu Pro Cys Lys His Val Phe His Lys Ser Cys Val Asp
    130                 135                 140

Pro Trp Leu Ser Glu His Cys Thr Cys Pro Met Cys Lys Leu Asn Ile
145                 150                 155                 160

Leu Lys Ala Leu Gly Ile Val
                165

(2) INFORMATION FOR SEQ ID NO: 696:

(i) SEQUENCE CHARACTERISTICS:
(A) LENGTH: 276 amino acids
(B) TYPE: amino acid
(D) TOPOLOGY: linear
(xi) SEQUENCE DESCRIPTION: SEQ ID NO: 696:

Met Thr His Pro Gly Thr Glu His Ile Ile Ala Val Met Ile Thr Glu
1               5                   10                  15

Leu Arg Gly Lys Asp Ile Leu Ser Tyr Leu Glu Lys Asn Ile Ser Val
            20                  25                  30

Gln Met Thr Ile Ala Val Gly Thr Arg Met Pro Pro Lys Asn Phe Ser
        35                  40                  45

Arg Gly Ser Leu Val Phe Val Ser Ile Ser Phe Ile Val Leu Met Ile
    50                  55                  60

Ile Ser Ser Ala Trp Leu Ile Phe Tyr Phe Ile Gln Lys Ile Arg Tyr
65                  70                  75                  80

Thr Asn Ala Arg Asp Arg Asn Gln Arg Arg Leu Gly Asp Ala Ala Lys
                85                  90                  95

Lys Ala Ile Ser Lys Leu Thr Thr Arg Thr Val Lys Lys Gly Asp Lys
            100                 105                 110

Glu Thr Asp Pro Asp Phe Asp His Cys Ala Val Cys Ile Glu Ser Tyr
        115                 120                 125

Lys Gln Asn Asp Val Val Arg Ile Leu Pro Cys Lys His Val Phe His
    130                 135                 140

Lys Ser Cys Val Asp Pro Trp Leu Ser Glu His Cys Thr Cys Pro Met
145                 150                 155                 160

Cys Lys Leu Asn Ile Leu Lys Ala Leu Gly Ile Val Pro Asn Leu Pro
                165                 170                 175

Cys Thr Asp Asn Val Ala Phe Asp Met Glu Arg Leu Thr Arg Thr Gln
            180                 185                 190

Ala Val Asn Arg Arg Ser Ala Leu Gly Asp Leu Ala Gly Asp Asn Ser
        195                 200                 205

Leu Gly Leu Glu Pro Leu Arg Thr Ser Gly Ile Ser Pro Leu Pro Gln
    210                 215                 220

Asp Gly Glu Leu Thr Pro Arg Thr Gly Glu Ile Asn Ile Ala Val Thr
225                 230                 235                 240

Lys Glu Trp Phe Ile Ile Ala Ser Phe Gly Leu Leu Ser Ala Leu Thr
                245                 250                 255

Leu Cys Tyr Met Ile Ile Arg Ala Thr Ala Ser Leu Asn Ala Asn Glu
            260                 265                 270

Val Glu Trp Phe
        275

(2) INFORMATION FOR SEQ ID NO: 697:

(i) SEQUENCE CHARACTERISTICS:
(A) LENGTH: 69 amino acids
(B) TYPE: amino acid
(D) TOPOLOGY: linear
(xi) SEQUENCE DESCRIPTION: SEQ ID NO: 697:

Thr Glu His Ile Ile Ala Val Met Ile Thr Glu Leu Arg Gly Lys Asp
1               5                   10                  15

Ile Leu Ser Tyr Leu Glu Lys Asn Ile Ser Val Gln Met Thr Ile Ala
            20                  25                  30

Val Gly Thr Arg Met Pro Pro Lys Asn Phe Ser Arg Gly Ser Leu Val
        35                  40                  45

Phe Val Ser Ile Ser Phe Ile Val Leu Met Ile Ile Ser Ser Ala Trp
    50                  55                  60

Leu Ile Phe Tyr Phe
65

(2) INFORMATION FOR SEQ ID NO: 698:

(i) SEQUENCE CHARACTERISTICS:
(A) LENGTH: 58 amino acids
(B) TYPE: amino acid
(D) TOPOLOGY: linear
(xi) SEQUENCE DESCRIPTION: SEQ ID NO: 698:

Ser Ile Ser Phe Ile Val Leu Met Ile Ile Ser Ser Ala Trp Leu Ile
1               5                   10                  15

Phe Tyr Phe Ile Gln Lys Ile Arg Tyr Thr Asn Ala Arg Asp Arg Asn
            20                  25                  30

Gln Arg Arg Leu Gly Asp Ala Ala Lys Lys Ala Ile Ser Lys Leu Thr
        35                  40                  45

Thr Arg Thr Val Lys Lys Gly Asp Lys Glu
    50                  55

(2) INFORMATION FOR SEQ ID NO: 699:

        (i) SEQUENCE CHARACTERISTICS:
                (A) LENGTH: 66 amino acids
                (B) TYPE: amino acid
                (D) TOPOLOGY: linear
        (xi) SEQUENCE DESCRIPTION: SEQ ID NO: 699:

Val Lys Lys Gly Asp Lys Glu Thr Asp Pro Asp Phe Asp His Cys Ala
  1               5                  10                  15

Val Cys Ile Glu Ser Tyr Lys Gln Asn Asp Val Val Arg Ile Leu Pro
            20                  25                  30

Cys Lys His Val Phe His Lys Ser Cys Val Asp Pro Trp Leu Ser Glu
        35                  40                  45

His Cys Thr Cys Pro Met Cys Lys Leu Asn Ile Leu Lys Ala Leu Gly
    50                  55                  60

Ile Val
65

(2) INFORMATION FOR SEQ ID NO: 700:

        (i) SEQUENCE CHARACTERISTICS:
                (A) LENGTH: 106 amino acids
                (B) TYPE: amino acid
                (D) TOPOLOGY: linear
        (xi) SEQUENCE DESCRIPTION: SEQ ID NO: 700:

Met Thr His Pro Gly Thr Glu His Ile Ile Ala Val Met Ile Thr Glu
  1               5                  10                  15

Leu Arg Gly Lys Asp Ile Leu Ser Tyr Leu Glu Lys Asn Ile Ser Val
            20                  25                  30

Gln Met Thr Ile Ala Val Gly Thr Arg Met Pro Pro Lys Asn Phe Ser
        35                  40                  45

Arg Gly Ser Leu Val Phe Val Ser Ile Ser Phe Ile Val Leu Met Ile
    50                  55                  60

Ile Ser Ser Ala Trp Leu Ile Phe Tyr Phe Ile Gln Lys Ile Arg Tyr
 65                  70                  75                  80

Thr Asn Ala Arg Asp Arg Asn Gln Arg Arg Leu Gly Asp Ala Ala Lys
                85                  90                  95

Lys Ala Ile Ser Lys Leu Thr Thr Arg Thr

100                    105

(2) INFORMATION FOR SEQ ID NO: 701:

(i) SEQUENCE CHARACTERISTICS:
(A) LENGTH: 84 amino acids
(B) TYPE: amino acid
(D) TOPOLOGY: linear
(xi) SEQUENCE DESCRIPTION: SEQ ID NO: 701:

Ala Ala Lys Lys Ala Ile Ser Lys Leu Thr Thr Arg Thr Val Lys Lys
1               5                   10                  15

Gly Asp Lys Glu Thr Asp Pro Asp Phe Asp His Cys Ala Val Cys Ile
            20                  25                  30

Glu Ser Tyr Lys Gln Asn Asp Val Val Arg Ile Leu Pro Cys Lys His
        35                  40                  45

Val Phe His Lys Ser Cys Val Asp Pro Trp Leu Ser Glu His Cys Thr
    50                  55                  60

Cys Pro Met Cys Lys Leu Asn Ile Leu Lys Ala Leu Gly Ile Val Pro
65                  70                  75                  80

Asn Leu Pro Cys

(2) INFORMATION FOR SEQ ID NO: 702:

(i) SEQUENCE CHARACTERISTICS:
(A) LENGTH: 86 amino acids
(B) TYPE: amino acid
(D) TOPOLOGY: linear
(xi) SEQUENCE DESCRIPTION: SEQ ID NO: 702:

Thr Gln Ala Val Asn Arg Arg Ser Ala Leu Gly Asp Leu Ala Gly Asp
1               5                   10                  15

Asn Ser Leu Gly Leu Glu Pro Leu Arg Thr Ser Gly Ile Ser Pro Leu
            20                  25                  30

Pro Gln Asp Gly Glu Leu Thr Pro Arg Thr Gly Glu Ile Asn Ile Ala
        35                  40                  45

Val Thr Lys Glu Trp Phe Ile Ile Ala Ser Phe Gly Leu Leu Ser Ala
    50                  55                  60

Leu Thr Leu Cys Tyr Met Ile Ile Arg Ala Thr Ala Ser Leu Asn Ala
65                  70                  75                  80

Asn Glu Val Glu Trp Phe
                85

(2) INFORMATION FOR SEQ ID NO: 703:

(i) SEQUENCE CHARACTERISTICS:
(A) LENGTH: 341 amino acids
(B) TYPE: amino acid
(D) TOPOLOGY: linear
(xi) SEQUENCE DESCRIPTION: SEQ ID NO: 703:

Pro Leu His Gly Val Ala Asp His Leu Gly Cys Asp Pro Gln Thr Arg
1               5                   10                  15

Phe Phe Val Pro Pro Asn Ile Lys Gln Trp Ile Ala Leu Leu Gln Arg
            20                  25                  30

Gly Asn Cys Thr Phe Lys Glu Lys Ile Ser Arg Ala Ala Phe His Asn
        35                  40                  45

Ala Val Ala Val Val Ile Tyr Asn Asn Lys Ser Lys Glu Glu Pro Val
    50                  55                  60

Thr Met Thr His Pro Gly Thr Glu His Ile Ile Ala Val Met Ile Thr
65                  70                  75                  80

Glu Leu Arg Gly Lys Asp Ile Leu Ser Tyr Leu Glu Lys Asn Ile Ser
                85                  90                  95

Val Gln Met Thr Ile Ala Val Gly Thr Arg Met Pro Pro Lys Asn Phe
            100                 105                 110

Ser Arg Gly Ser Leu Val Phe Val Ser Ile Ser Phe Ile Val Leu Met
        115                 120                 125

Ile Ile Ser Ser Ala Trp Leu Ile Phe Tyr Phe Ile Gln Lys Ile Arg
    130                 135                 140

Tyr Thr Asn Ala Arg Asp Arg Asn Gln Arg Arg Leu Gly Asp Ala Ala
145                 150                 155                 160

Lys Lys Ala Ile Ser Lys Leu Thr Thr Arg Thr Val Lys Lys Gly Asp
                165                 170                 175

Lys Glu Thr Asp Pro Asp Phe Asp His Cys Ala Val Cys Ile Glu Ser
            180                 185                 190

Tyr Lys Gln Asn Asp Val Val Arg Ile Leu Pro Cys Lys His Val Phe
        195                 200                 205

His Lys Ser Cys Val Asp Pro Trp Leu Ser Glu His Cys Thr Cys Pro
    210                 215                 220

Met Cys Lys Leu Asn Ile Leu Lys Ala Leu Gly Ile Val Pro Asn Leu
225                 230                 235                 240

Pro Cys Thr Asp Asn Val Ala Phe Asp Met Glu Arg Leu Thr Arg Thr
                245                 250                 255

Gln Ala Val Asn Arg Arg Ser Ala Leu Gly Asp Leu Ala Gly Asp Asn
            260                 265                 270

Ser Leu Gly Leu Glu Pro Leu Arg Thr Ser Gly Ile Ser Pro Leu Pro

275                 280                 285

Gln Asp Gly Glu Leu Thr Pro Arg Thr Gly Glu Ile Asn Ile Ala Val
    290                 295                 300

Thr Lys Glu Trp Phe Ile Ile Ala Ser Phe Gly Leu Leu Ser Ala Leu
305                 310                 315                 320

Thr Leu Cys Tyr Met Ile Ile Arg Ala Thr Ala Ser Leu Asn Ala Asn
                325                 330                 335

Glu Val Glu Trp Phe
            340

(2) INFORMATION FOR SEQ ID NO: 704:

(i) SEQUENCE CHARACTERISTICS:
(A) LENGTH: 60 amino acids
(B) TYPE: amino acid
(D) TOPOLOGY: linear
(xi) SEQUENCE DESCRIPTION: SEQ ID NO: 704:

His Gly Val Ala Asp His Leu Gly Cys Asp Pro Gln Thr Arg Phe Phe
  1               5                  10                  15

Val Pro Pro Asn Ile Lys Gln Trp Ile Ala Leu Leu Gln Arg Gly Asn
             20                  25                  30

Cys Thr Phe Lys Glu Lys Ile Ser Arg Ala Ala Phe His Asn Ala Val
         35                  40                  45

Ala Val Val Ile Tyr Asn Asn Lys Ser Lys Glu Glu
     50                  55                  60

(2) INFORMATION FOR SEQ ID NO: 705:

(i) SEQUENCE CHARACTERISTICS:
(A) LENGTH: 314 amino acids
(B) TYPE: amino acid
(D) TOPOLOGY: linear
(xi) SEQUENCE DESCRIPTION: SEQ ID NO: 705:

Met Ser Gly Gln Gly Leu Ala Gly Phe Phe Ala Ser Val Ala Met Ile
  1               5                  10                  15

Cys Ala Ile Ala Ser Gly Ser Glu Leu Ser Glu Ser Ala Phe Gly Tyr
             20                  25                  30

Phe Ile Thr Ala Cys Ala Val Ile Ile Leu Thr Ile Ile Cys Tyr Leu
         35                  40                  45

Gly Leu Pro Arg Leu Glu Phe Tyr Arg Tyr Tyr Gln Gln Leu Lys Leu
     50                  55                  60

Glu Gly Pro Gly Glu Gln Glu Thr Lys Leu Asp Leu Ile Ser Lys Gly
 65                  70                  75                  80

Glu Glu Pro Arg Ala Gly Lys Glu Glu Ser Gly Val Ser Val Ser Asn
                85                  90                  95

Ser Gln Pro Thr Asn Glu Ser His Ser Ile Lys Ala Ile Leu Lys Asn
            100                 105                 110

Ile Ser Val Leu Ala Phe Ser Val Cys Phe Ile Phe Thr Ile Thr Ile
        115                 120                 125

Gly Met Phe Pro Ala Val Thr Val Glu Val Lys Ser Ser Ile Ala Gly
    130                 135                 140

Ser Ser Thr Trp Glu Arg Tyr Phe Ile Pro Val Ser Cys Phe Leu Thr
145                 150                 155                 160

Phe Asn Ile Phe Asp Trp Leu Gly Arg Ser Leu Thr Ala Val Phe Met
                165                 170                 175

Trp Pro Gly Lys Asp Ser Arg Trp Leu Pro Ser Trp Xaa Leu Ala Arg
            180                 185                 190

Leu Val Phe Val Pro Leu Leu Leu Leu Cys Asn Ile Lys Pro Arg Arg
        195                 200                 205

Tyr Leu Thr Val Val Phe Glu His Asp Ala Trp Phe Ile Phe Phe Met
    210                 215                 220

Ala Ala Phe Ala Phe Ser Asn Gly Tyr Leu Ala Ser Leu Cys Met Cys
225                 230                 235                 240

Phe Gly Pro Lys Lys Val Lys Pro Ala Glu Ala Glu Thr Ala Glu Pro
                245                 250                 255

Ser Trp Pro Ser Ser Cys Val Trp Val Trp His Trp Gly Leu Phe Ser
            260                 265                 270

Pro Ser Cys Ser Gly Gln Leu Cys Asp Lys Gly Trp Thr Glu Gly Leu
        275                 280                 285

Pro Ala Ser Leu Pro Val Cys Leu Leu Pro Leu Pro Ser Ala Arg Gly
    290                 295                 300

Asp Pro Glu Trp Ser Gly Gly Phe Phe Phe
305                 310


(2) INFORMATION FOR SEQ ID NO: 706:

        (i) SEQUENCE CHARACTERISTICS:
                (A) LENGTH: 106 amino acids
                (B) TYPE: amino acid
                (D) TOPOLOGY: linear
        (xi) SEQUENCE DESCRIPTION: SEQ ID NO: 706:

Met Ser Gly Gln Gly Leu Ala Gly Phe Phe Ala Ser Val Ala Met Ile
  1               5                  10                  15

Cys Ala Ile Ala Ser Gly Ser Glu Leu Ser Glu Ser Ala Phe Gly Tyr

20                  25                  30

Phe Ile Thr Ala Cys Ala Val Ile Ile Leu Thr Ile Ile Cys Tyr Leu
        35                  40                  45

Gly Leu Pro Arg Leu Glu Phe Tyr Arg Tyr Tyr Gln Gln Leu Lys Leu
    50                  55                  60

Glu Gly Pro Gly Glu Gln Glu Thr Lys Leu Asp Leu Ile Ser Lys Gly
65                  70                  75                  80

Glu Glu Pro Arg Ala Gly Lys Glu Glu Ser Gly Val Ser Val Ser Asn
                85                  90                  95

Ser Gln Pro Thr Asn Glu Ser His Ser Ile
            100                 105

(2) INFORMATION FOR SEQ ID NO: 707:

(i) SEQUENCE CHARACTERISTICS:
(A) LENGTH: 81 amino acids
(B) TYPE: amino acid
(D) TOPOLOGY: linear
(xi) SEQUENCE DESCRIPTION: SEQ ID NO: 707:

Ser Gly Val Ser Val Ser Asn Ser Gln Pro Thr Asn Glu Ser His Ser
1               5                   10                  15

Ile Lys Ala Ile Leu Lys Asn Ile Ser Val Leu Ala Phe Ser Val Cys
            20                  25                  30

Phe Ile Phe Thr Ile Thr Ile Gly Met Phe Pro Ala Val Thr Val Glu
        35                  40                  45

Val Lys Ser Ser Ile Ala Gly Ser Ser Thr Trp Glu Arg Tyr Phe Ile
    50                  55                  60

Pro Val Ser Cys Phe Leu Thr Phe Asn Ile Phe Asp Trp Leu Gly Arg
65                  70                  75                  80

Ser

(2) INFORMATION FOR SEQ ID NO: 708:

(i) SEQUENCE CHARACTERISTICS:
(A) LENGTH: 92 amino acids
(B) TYPE: amino acid
(D) TOPOLOGY: linear
(xi) SEQUENCE DESCRIPTION: SEQ ID NO: 708:

Thr Ile Gly Met Phe Pro Ala Val Thr Val Glu Val Lys Ser Ser Ile
1               5                   10                  15

Ala Gly Ser Ser Thr Trp Glu Arg Tyr Phe Ile Pro Val Ser Cys Phe
            20                  25                  30

Leu Thr Phe Asn Ile Phe Asp Trp Leu Gly Arg Ser Leu Thr Ala Val
        35                  40                  45

Phe Met Trp Pro Gly Lys Asp Ser Arg Trp Leu Pro Ser Trp Xaa Leu
    50                  55                  60

Ala Arg Leu Val Phe Val Pro Leu Leu Leu Leu Cys Asn Ile Lys Pro
65                  70                  75                  80

Arg Arg Tyr Leu Thr Val Val Phe Glu His Asp Ala
                85                  90

(2) INFORMATION FOR SEQ ID NO: 709:

        (i) SEQUENCE CHARACTERISTICS:
                (A) LENGTH: 74 amino acids
                (B) TYPE: amino acid
                (D) TOPOLOGY: linear
        (xi) SEQUENCE DESCRIPTION: SEQ ID NO: 709:

Phe Gly Pro Lys Lys Val Lys Pro Ala Glu Ala Glu Thr Ala Glu Pro
 1               5                  10                  15

Ser Trp Pro Ser Ser Cys Val Trp Val Trp His Trp Gly Leu Phe Ser
            20                  25                  30

Pro Ser Cys Ser Gly Gln Leu Cys Asp Lys Gly Trp Thr Glu Gly Leu
        35                  40                  45

Pro Ala Ser Leu Pro Val Cys Leu Leu Pro Leu Pro Ser Ala Arg Gly
    50                  55                  60

Asp Pro Glu Trp Ser Gly Gly Phe Phe Phe
65                  70

(2) INFORMATION FOR SEQ ID NO: 710:

        (i) SEQUENCE CHARACTERISTICS:
                (A) LENGTH: 135 amino acids
                (B) TYPE: amino acid
                (D) TOPOLOGY: linear
        (xi) SEQUENCE DESCRIPTION: SEQ ID NO: 710:

Asp Asp Asp Gly Phe Glu Ile Val Pro Ile Glu Asp Pro Ala Lys His
 1               5                  10                  15

Arg Ile Leu Asp Pro Glu Gly Leu Ala Leu Gly Ala Val Ile Ala Ser
            20                  25                  30

Ser Lys Lys Ala Lys Arg Asp Leu Ile Asp Asn Ser Phe Asn Arg Tyr
        35                  40                  45

Thr Phe Asn Glu Asp Glu Gly Glu Leu Pro Glu Trp Phe Val Gln Glu
    50                  55                  60

Glu Lys Gln His Arg Ile Arg Gln Leu Pro Val Gly Lys Lys Glu Val
65                  70                  75                  80

Glu His Tyr Arg Lys Arg Trp Arg Glu Ile Asn Ala Arg Pro Ile Xaa
                85                  90                  95

Xaa Xaa Xaa Xaa Xaa Xaa Xaa Xaa Xaa Xaa Xaa Xaa Xaa Xaa Xaa Xaa
            100                 105                 110

Leu Glu Gln Thr Arg Lys Lys Ala Glu Ala Val Val Asn Thr Val Asp
        115                 120                 125

Ile Xaa Arg Thr Arg Glu Ser
    130                 135

(2) INFORMATION FOR SEQ ID NO: 711:

(i) SEQUENCE CHARACTERISTICS:
(A) LENGTH: 50 amino acids
(B) TYPE: amino acid
(D) TOPOLOGY: linear
(xi) SEQUENCE DESCRIPTION: SEQ ID NO: 711:

Asp Asp Asp Gly Phe Glu Ile Val Pro Ile Glu Asp Pro Ala Lys His
1               5                   10                  15

Arg Ile Leu Asp Pro Glu Gly Leu Ala Leu Gly Ala Val Ile Ala Ser
            20                  25                  30

Ser Lys Lys Ala Lys Arg Asp Leu Ile Asp Asn Ser Phe Asn Arg Tyr
        35                  40                  45

Thr Phe
    50

(2) INFORMATION FOR SEQ ID NO: 712:

(i) SEQUENCE CHARACTERISTICS:
(A) LENGTH: 51 amino acids
(B) TYPE: amino acid
(D) TOPOLOGY: linear
(xi) SEQUENCE DESCRIPTION: SEQ ID NO: 712:

Lys Arg Trp Arg Glu Ile Asn Ala Arg Pro Ile Xaa Xaa Xaa Xaa Xaa
1               5                   10                  15

Xaa Xaa Xaa Xaa Xaa Xaa Xaa Xaa Xaa Xaa Xaa Xaa Leu Glu Gln Thr
            20                  25                  30

Arg Lys Lys Ala Glu Ala Val Val Asn Thr Val Asp Ile Xaa Arg Thr
        35                  40                  45

Arg Glu Ser
    50

(2) INFORMATION FOR SEQ ID NO: 713:

(i) SEQUENCE CHARACTERISTICS:
(A) LENGTH: 216 amino acids
(B) TYPE: amino acid
(D) TOPOLOGY: linear
(xi) SEQUENCE DESCRIPTION: SEQ ID NO: 713:

Met Ile Lys Asp Lys Gly Arg Ala Arg Thr Ala Leu Thr Ser Ser Gln
1               5                   10                  15

Pro Ala His Leu Cys Pro Glu Asn Pro Leu Leu His Leu Lys Ala Ala
            20                  25                  30

Val Lys Glu Lys Lys Arg Asn Lys Lys Lys Lys Thr Ile Gly Ser Pro
        35                  40                  45

Lys Arg Ile Gln Ser Pro Leu Asn Asn Lys Leu Leu Asn Ser Pro Ala
    50                  55                  60

Lys Thr Leu Pro Gly Ala Cys Gly Ser Pro Gln Lys Leu Ile Asp Gly
65                  70                  75                  80

Phe Leu Lys His Glu Gly Pro Pro Ala Glu Lys Pro Leu Glu Glu Leu
                85                  90                  95

Ser Ala Ser Thr Ser Gly Val Pro Gly Leu Ser Ser Leu Gln Ser Asp
            100                 105                 110

Pro Ala Gly Cys Val Arg Pro Pro Ala Pro Asn Leu Ala Gly Ala Val
        115                 120                 125

Glu Phe Asn Asp Val Lys Thr Leu Leu Arg Glu Trp Ile Thr Thr Ile
    130                 135                 140

Ser Asp Pro Met Glu Glu Asp Ile Leu Gln Val Val Lys Tyr Cys Thr
145                 150                 155                 160

Asp Leu Ile Glu Glu Lys Asp Leu Glu Lys Leu Asp Leu Val Ile Lys
                165                 170                 175

Tyr Met Lys Arg Leu Met Gln Gln Ser Val Glu Ser Val Trp Asn Met
            180                 185                 190

Ala Phe Asp Phe Ile Leu Asp Asn Val Gln Val Val Leu Gln Gln Thr
        195                 200                 205

Tyr Gly Ser Thr Leu Lys Val Thr
    210                 215

(2) INFORMATION FOR SEQ ID NO: 714:

(i) SEQUENCE CHARACTERISTICS:
(A) LENGTH: 52 amino acids
(B) TYPE: amino acid
(D) TOPOLOGY: linear
(xi) SEQUENCE DESCRIPTION: SEQ ID NO: 714:

Met Ile Lys Asp Lys Gly Arg Ala Arg Thr Ala Leu Thr Ser Ser Gln
1               5                   10                  15

Pro Ala His Leu Cys Pro Glu Asn Pro Leu Leu His Leu Lys Ala Ala
            20                  25                  30

Val Lys Glu Lys Lys Arg Asn Lys Lys Lys Lys Thr Ile Gly Ser Pro
        35                  40                  45

Lys Arg Ile Gln
    50

(2) INFORMATION FOR SEQ ID NO: 715:

(i) SEQUENCE CHARACTERISTICS:
(A) LENGTH: 100 amino acids
(B) TYPE: amino acid
(D) TOPOLOGY: linear
(xi) SEQUENCE DESCRIPTION: SEQ ID NO: 715:

Lys Arg Ile Gln Ser Pro Leu Asn Asn Lys Leu Leu Asn Ser Pro Ala
1               5                   10                  15

Lys Thr Leu Pro Gly Ala Cys Gly Ser Pro Gln Lys Leu Ile Asp Gly
            20                  25                  30

Phe Leu Lys His Glu Gly Pro Pro Ala Glu Lys Pro Leu Glu Glu Leu
        35                  40                  45

Ser Ala Ser Thr Ser Gly Val Pro Gly Leu Ser Ser Leu Gln Ser Asp
    50                  55                  60

Pro Ala Gly Cys Val Arg Pro Pro Ala Pro Asn Leu Ala Gly Ala Val
65                  70                  75                  80

Glu Phe Asn Asp Val Lys Thr Leu Leu Arg Glu Trp Ile Thr Thr Ile
                85                  90                  95

Ser Asp Pro Met
            100

(2) INFORMATION FOR SEQ ID NO: 716:

(i) SEQUENCE CHARACTERISTICS:
(A) LENGTH: 74 amino acids
(B) TYPE: amino acid
(D) TOPOLOGY: linear
(xi) SEQUENCE DESCRIPTION: SEQ ID NO: 716:

Thr Ile Ser Asp Pro Met Glu Glu Asp Ile Leu Gln Val Val Lys Tyr
1               5                   10                  15

Cys Thr Asp Leu Ile Glu Glu Lys Asp Leu Glu Lys Leu Asp Leu Val
            20                  25                  30

Ile Lys Tyr Met Lys Arg Leu Met Gln Gln Ser Val Glu Ser Val Trp
        35                  40                  45

Asn Met Ala Phe Asp Phe Ile Leu Asp Asn Val Gln Val Val Leu Gln
    50                  55                  60

Gln Thr Tyr Gly Ser Thr Leu Lys Val Thr
65                  70

(2) INFORMATION FOR SEQ ID NO: 717:

        (i) SEQUENCE CHARACTERISTICS:
                (A) LENGTH: 18 amino acids
                (B) TYPE: amino acid
                (D) TOPOLOGY: linear
        (xi) SEQUENCE DESCRIPTION: SEQ ID NO: 717:

Phe Cys His Asp Cys Lys Phe Pro Glu Ala Ser Pro Ala Met Asn Cys
 1               5                  10                  15

Glu Pro

(2) INFORMATION FOR SEQ ID NO: 718:

        (i) SEQUENCE CHARACTERISTICS:
                (A) LENGTH: 18 amino acids
                (B) TYPE: amino acid
                (D) TOPOLOGY: linear
        (xi) SEQUENCE DESCRIPTION: SEQ ID NO: 718:

Phe Cys His Asp Cys Lys Phe Pro Glu Ala Ser Pro Ala Met Asn Cys
 1               5                  10                  15

Glu Pro

(2) INFORMATION FOR SEQ ID NO: 719:

        (i) SEQUENCE CHARACTERISTICS:
                (A) LENGTH: 27 amino acids
                (B) TYPE: amino acid
                (D) TOPOLOGY: linear
        (xi) SEQUENCE DESCRIPTION: SEQ ID NO: 719:

Pro Gln Pro Ser Asn Phe Pro Thr Thr Val Arg Asn Leu Pro Tyr Ser
 1               5                  10                  15

Gly Ala Gly Ala Gln Pro Pro Pro Ser Asn Cys
            20                  25

(2) INFORMATION FOR SEQ ID NO: 720:

(i) SEQUENCE CHARACTERISTICS:
(A) LENGTH: 134 amino acids
(B) TYPE: amino acid
(D) TOPOLOGY: linear
(xi) SEQUENCE DESCRIPTION: SEQ ID NO: 720:

Met Ala Ser Ser Val Pro Ala Gly Gly His Thr Arg Ala Gly Gly Ile
1               5                   10                  15

Phe Leu Ile Gly Lys Leu Asp Leu Glu Ala Ser Leu Phe Lys Ser Phe
            20                  25                  30

Gln Trp Leu Pro Phe Val Leu Arg Lys Lys Cys Asn Phe Phe Cys Trp
        35                  40                  45

Asp Ser Ser Ala His Ser Leu Pro Leu His Pro Leu Ser Ala Ser Cys
    50                  55                  60

Ser Ala Pro Ala Cys His Ala Ser Asp Thr His Leu Leu Tyr Pro Ser
65                  70                  75                  80

Thr Arg Ala Leu Cys Pro Ser Ile Phe Ala Trp Leu Val Ala Pro His
                85                  90                  95

Ser Val Phe Arg Thr Asn Ala Pro Gly Pro Thr Pro Ser Ser Gln Ser
            100                 105                 110

Ser Pro Val Phe Pro Val Phe Pro Val Ser Phe Met Ala Leu Ile Val
        115                 120                 125

Cys Xaa Leu Val Cys Cys
    130

(2) INFORMATION FOR SEQ ID NO: 721:

(i) SEQUENCE CHARACTERISTICS:
(A) LENGTH: 71 amino acids
(B) TYPE: amino acid
(D) TOPOLOGY: linear
(xi) SEQUENCE DESCRIPTION: SEQ ID NO: 721:

Met Ala Ser Ser Val Pro Ala Gly Gly His Thr Arg Ala Gly Gly Ile
1               5                   10                  15

Phe Leu Ile Gly Lys Leu Asp Leu Glu Ala Ser Leu Phe Lys Ser Phe
            20                  25                  30

Gln Trp Leu Pro Phe Val Leu Arg Lys Lys Cys Asn Phe Phe Cys Trp
        35                  40                  45

Asp Ser Ser Ala His Ser Leu Pro Leu His Pro Leu Ser Ala Ser Cys
    50                  55                  60

Ser Ala Pro Ala Cys His Ala
65                  70

(2) INFORMATION FOR SEQ ID NO: 722:

(i) SEQUENCE CHARACTERISTICS:
(A) LENGTH: 46 amino acids
(B) TYPE: amino acid
(D) TOPOLOGY: linear
(xi) SEQUENCE DESCRIPTION: SEQ ID NO: 722:

Phe Ala Trp Leu Val Ala Pro His Ser Val Phe Arg Thr Asn Ala Pro
1               5                   10                  15

Gly Pro Thr Pro Ser Ser Gln Ser Ser Pro Val Phe Pro Val Phe Pro
            20                  25                  30

Val Ser Phe Met Ala Leu Ile Val Cys Xaa Leu Val Cys Cys
        35                  40                  45

(2) INFORMATION FOR SEQ ID NO: 723:

(i) SEQUENCE CHARACTERISTICS:
(A) LENGTH: 134 amino acids
(B) TYPE: amino acid
(D) TOPOLOGY: linear
(xi) SEQUENCE DESCRIPTION: SEQ ID NO: 723:

Met Ala Ser Ser Val Pro Ala Gly Gly His Thr Arg Ala Gly Gly Ile
1               5                   10                  15

Phe Leu Ile Gly Lys Leu Asp Leu Glu Ala Ser Leu Phe Lys Ser Phe
            20                  25                  30

Gln Trp Leu Pro Phe Val Leu Arg Lys Lys Cys Asn Phe Phe Cys Trp
        35                  40                  45

Asp Ser Ser Ala His Ser Leu Pro Leu His Pro Leu Ser Ala Ser Cys
    50                  55                  60

Ser Ala Pro Ala Cys His Ala Ser Asp Thr His Leu Leu Tyr Pro Ser
65                  70                  75                  80

Thr Arg Ala Leu Cys Pro Ser Ile Phe Ala Trp Leu Val Ala Pro His
                85                  90                  95

Ser Val Phe Arg Thr Asn Ala Pro Gly Pro Thr Pro Ser Ser Gln Ser
            100                 105                 110

Ser Pro Val Phe Pro Val Phe Pro Val Ser Phe Met Ala Leu Ile Val
        115                 120                 125

Cys Xaa Leu Val Cys Cys
    130

(2) INFORMATION FOR SEQ ID NO: 724:

(i) SEQUENCE CHARACTERISTICS:

(A) LENGTH: 286 amino acids
(B) TYPE: amino acid
(D) TOPOLOGY: linear
(xi) SEQUENCE DESCRIPTION: SEQ ID NO: 724:

Met Ala Met Glu Gly Tyr Trp Arg Phe Leu Ala Leu Leu Gly Ser Ala
1               5                   10                  15

Leu Leu Val Gly Phe Leu Ser Val Ile Phe Ala Leu Val Trp Val Leu
            20                  25                  30

His Tyr Arg Glu Gly Leu Gly Trp Asp Gly Ser Ala Leu Glu Phe Asn
        35                  40                  45

Trp His Pro Val Leu Met Val Thr Gly Phe Val Phe Ile Gln Gly Ile
    50                  55                  60

Ala Ile Ile Val Tyr Arg Leu Pro Trp Thr Trp Lys Cys Ser Lys Leu
65                  70                  75                  80

Leu Met Lys Ser Ile His Ala Gly Leu Asn Ala Val Ala Ala Ile Leu
                85                  90                  95

Ala Ile Ile Ser Val Val Ala Val Phe Glu Asn His Asn Val Asn Asn
            100                 105                 110

Ile Ala Asn Met Tyr Ser Leu His Ser Trp Val Gly Leu Ile Ala Val
        115                 120                 125

Ile Cys Tyr Leu Leu Gln Leu Leu Ser Gly Phe Ser Val Phe Leu Leu
    130                 135                 140

Pro Trp Ala Pro Leu Ser Leu Arg Ala Phe Leu Met Pro Ile His Val
145                 150                 155                 160

Tyr Ser Gly Ile Val Ile Phe Gly Thr Val Ile Ala Thr Ala Leu Met
                165                 170                 175

Gly Leu Thr Glu Lys Leu Ile Phe Ser Leu Arg Asp Pro Ala Tyr Ser
            180                 185                 190

Thr Phe Pro Pro Glu Gly Val Phe Val Asn Thr Leu Gly Leu Leu Ile
        195                 200                 205

Leu Val Phe Gly Ala Leu Ile Phe Trp Ile Val Thr Arg Pro Gln Trp
    210                 215                 220

Lys Arg Pro Lys Glu Pro Asn Ser Thr Ile Leu His Pro Asn Gly Gly
225                 230                 235                 240

Thr Glu Gln Gly Ala Arg Gly Ser Met Pro Ala Tyr Ser Gly Asn Asn
                245                 250                 255

Met Asp Lys Ser Asp Ser Glu Leu Asn Ser Glu Val Ala Ala Arg Lys
            260                 265                 270

Arg Asn Leu Ala Leu Asp Glu Ala Gly Gln Arg Ser Thr Met
        275                 280                 285

(2) INFORMATION FOR SEQ ID NO: 725:

(i) SEQUENCE CHARACTERISTICS:
(A) LENGTH: 43 amino acids
(B) TYPE: amino acid
(D) TOPOLOGY: linear
(xi) SEQUENCE DESCRIPTION: SEQ ID NO: 725:

(2) INFORMATION FOR SEQ ID NO: 726:

(i) SEQUENCE CHARACTERISTICS:
(A) LENGTH: 424 amino acids
(B) TYPE: amino acid
(D) TOPOLOGY: linear
(xi) SEQUENCE DESCRIPTION: SEQ ID NO: 726:

Met Lys Leu Leu Gly Glu Cys Ser Ser Ser Ile Asp Ser Val Lys Arg
1               5                   10                  15

Leu Glu His Lys Leu Lys Glu Glu Glu Glu Ser Leu Pro Gly Phe Val
            20                  25                  30

Asn Leu His Ser Thr Glu Thr Gln Thr Ala Gly Val Ile Asp Arg Trp
        35                  40                  45

Glu Leu Leu Gln Ala Gln Ala Leu Ser Lys Glu Leu Arg Met Lys Gln
    50                  55                  60

Asn Leu Gln Lys Trp Gln Gln Phe Asn Ser Asp Leu Asn Ser Ile Trp
65                  70                  75                  80

Ala Trp Leu Gly Asp Thr Glu Glu Glu Leu Glu Gln Leu Gln Arg Leu
                85                  90                  95

Glu Leu Ser Thr Asp Ile Gln Thr Ile Glu Leu Gln Ile Lys Lys Leu
            100                 105                 110

Lys Glu Leu Gln Lys Ala Val Asp His Arg Lys Ala Ile Ile Leu Ser
        115                 120                 125

Ile Asn Leu Cys Ser Pro Glu Phe Thr Gln Ala Asp Ser Lys Glu Ser
    130                 135                 140

Arg Asp Leu Gln Asp Arg Leu Xaa Gln Met Asn Gly Arg Trp Asp Arg
145                 150                 155                 160

Val Cys Ser Leu Leu Glu Glu Trp Arg Gly Leu Leu Gln Asp Ala Leu
                165                 170                 175

Met Gln Cys Gln Gly Phe His Glu Met Ser His Gly Leu Leu Leu Met
            180                 185                 190

Leu Glu Asn Ile Asp Arg Arg Lys Asn Glu Ile Val Pro Ile Asp Ser
        195                 200                 205

Asn Leu Asp Ala Glu Ile Leu Gln Asp His His Lys Gln Leu Met Gln
    210                 215                 220

Ile Lys His Glu Leu Leu Glu Ser Gln Leu Arg Val Ala Ser Leu Gln
225                 230                 235                 240

Asp Met Ser Cys Gln Leu Leu Val Asn Ala Glu Gly Thr Asp Cys Leu
                245                 250                 255

Glu Ala Lys Glu Lys Val His Val Ile Gly Asn Arg Leu Lys Leu Leu
            260                 265                 270

Leu Lys Glu Val Ser Arg His Ile Lys Glu Leu Glu Lys Leu Leu Asp
        275                 280                 285

Val Ser Ser Ser Gln Gln Asp Leu Ser Ser Trp Ser Ser Ala Asp Glu
    290                 295                 300

Leu Asp Thr Ser Gly Ser Val Ser Pro Xaa Ser Gly Arg Ser Thr Pro
305                 310                 315                 320

Asn Arg Gln Lys Thr Pro Arg Gly Lys Cys Ser Leu Ser Gln Pro Gly
                325                 330                 335

Pro Ser Val Ser Ser Pro His Ser Arg Ser Thr Lys Gly Gly Ser Asp
            340                 345                 350

Ser Ser Leu Ser Glu Pro Xaa Pro Gly Arg Ser Gly Arg Gly Phe Leu
        355                 360                 365

Phe Arg Val Leu Arg Ala Ala Leu Pro Leu Gln Leu Leu Leu Leu Leu
    370                 375                 380

Leu Ile Gly Leu Ala Cys Leu Val Pro Met Ser Glu Glu Asp Tyr Ser
385                 390                 395                 400

Cys Ala Leu Ser Asn Asn Phe Ala Arg Ser Phe His Pro Met Leu Arg
                405                 410                 415

Tyr Thr Asn Gly Pro Pro Pro Leu
            420

(2) INFORMATION FOR SEQ ID NO: 727:

(i) SEQUENCE CHARACTERISTICS:
(A) LENGTH: 110 amino acids
(B) TYPE: amino acid
(D) TOPOLOGY: linear
(xi) SEQUENCE DESCRIPTION: SEQ ID NO: 727:

Met Lys Leu Leu Gly Glu Cys Ser Ser Ser Ile Asp Ser Val Lys Arg

1                   5                   10                  15

Leu Glu His Lys Leu Lys Glu Glu Glu Glu Ser Leu Pro Gly Phe Val
            20                  25                  30

Asn Leu His Ser Thr Glu Thr Gln Thr Ala Gly Val Ile Asp Arg Trp
        35                  40                  45

Glu Leu Leu Gln Ala Gln Ala Leu Ser Lys Glu Leu Arg Met Lys Gln
    50                  55                  60

Asn Leu Gln Lys Trp Gln Gln Phe Asn Ser Asp Leu Asn Ser Ile Trp
65                  70                  75                  80

Ala Trp Leu Gly Asp Thr Glu Glu Glu Leu Glu Gln Leu Gln Arg Leu
                85                  90                  95

Glu Leu Ser Thr Asp Ile Gln Thr Ile Glu Leu Gln Ile Lys
            100                 105                 110

(2) INFORMATION FOR SEQ ID NO: 728:

(i) SEQUENCE CHARACTERISTICS:
(A) LENGTH: 136 amino acids
(B) TYPE: amino acid
(D) TOPOLOGY: linear
(xi) SEQUENCE DESCRIPTION: SEQ ID NO: 728:

Lys Leu Lys Glu Leu Gln Lys Ala Val Asp His Arg Lys Ala Ile Ile
1               5                   10                  15

Leu Ser Ile Asn Leu Cys Ser Pro Glu Phe Thr Gln Ala Asp Ser Lys
            20                  25                  30

Glu Ser Arg Asp Leu Gln Asp Arg Leu Xaa Gln Met Asn Gly Arg Trp
        35                  40                  45

Asp Arg Val Cys Ser Leu Leu Glu Glu Trp Arg Gly Leu Leu Gln Asp
    50                  55                  60

Ala Leu Met Gln Cys Gln Gly Phe His Glu Met Ser His Gly Leu Leu
65                  70                  75                  80

Leu Met Leu Glu Asn Ile Asp Arg Arg Lys Asn Glu Ile Val Pro Ile
                85                  90                  95

Asp Ser Asn Leu Asp Ala Glu Ile Leu Gln Asp His His Lys Gln Leu
            100                 105                 110

Met Gln Ile Lys His Glu Leu Leu Glu Ser Gln Leu Arg Val Ala Ser
        115                 120                 125

Leu Gln Asp Met Ser Cys Gln Leu
    130                 135

(2) INFORMATION FOR SEQ ID NO: 729:

(i) SEQUENCE CHARACTERISTICS:
(A) LENGTH: 105 amino acids
(B) TYPE: amino acid
(D) TOPOLOGY: linear
(xi) SEQUENCE DESCRIPTION: SEQ ID NO: 729:

Gln Asp Met Ser Cys Gln Leu Leu Val Asn Ala Glu Gly Thr Asp Cys
1               5                   10                  15

Leu Glu Ala Lys Glu Lys Val His Val Ile Gly Asn Arg Leu Lys Leu
            20                  25                  30

Leu Leu Lys Glu Val Ser Arg His Ile Lys Glu Leu Glu Lys Leu Leu
        35                  40                  45

Asp Val Ser Ser Ser Gln Gln Asp Leu Ser Ser Trp Ser Ser Ala Asp
    50                  55                  60

Glu Leu Asp Thr Ser Gly Ser Val Ser Pro Xaa Ser Gly Arg Ser Thr
65                  70                  75                  80

Pro Asn Arg Gln Lys Thr Pro Arg Gly Lys Cys Ser Leu Ser Gln Pro
                85                  90                  95

Gly Pro Ser Val Ser Ser Pro His Ser
            100                 105

(2) INFORMATION FOR SEQ ID NO: 730:

(i) SEQUENCE CHARACTERISTICS:
(A) LENGTH: 73 amino acids
(B) TYPE: amino acid
(D) TOPOLOGY: linear
(xi) SEQUENCE DESCRIPTION: SEQ ID NO: 730:

Asp Ser Ser Leu Ser Glu Pro Xaa Pro Gly Arg Ser Gly Arg Gly Phe
1               5                   10                  15

Leu Phe Arg Val Leu Arg Ala Ala Leu Pro Leu Gln Leu Leu Leu Leu
            20                  25                  30

Leu Leu Ile Gly Leu Ala Cys Leu Val Pro Met Ser Glu Glu Asp Tyr
        35                  40                  45

Ser Cys Ala Leu Ser Asn Asn Phe Ala Arg Ser Phe His Pro Met Leu
    50                  55                  60

Arg Tyr Thr Asn Gly Pro Pro Pro Leu
65                  70

(2) INFORMATION FOR SEQ ID NO: 731:

(i) SEQUENCE CHARACTERISTICS:
(A) LENGTH: 56 amino acids
(B) TYPE: amino acid

(D) TOPOLOGY: linear
(xi) SEQUENCE DESCRIPTION: SEQ ID NO: 731:

Met Lys Leu Leu Ile Cys Gly Asn Tyr Leu Ala Pro Ser His Ser Glu
1               5                   10                  15

Ser Ser Arg Arg Cys Cys Leu Leu Cys Phe Tyr Pro Leu Cys Leu Glu
            20                  25                  30

Ile Asn Phe Gly Met Lys Val Phe Leu Ser Met Pro Phe Leu Val Leu
        35                  40                  45

Phe Gln Ser Leu Ile Gln Glu Asp
    50                  55

(2) INFORMATION FOR SEQ ID NO: 732:

(i) SEQUENCE CHARACTERISTICS:
(A) LENGTH: 271 amino acids
(B) TYPE: amino acid
(D) TOPOLOGY: linear
(xi) SEQUENCE DESCRIPTION: SEQ ID NO: 732:

Arg Ile Leu Leu Val Lys Tyr Ser Ala Asn Glu Glu Asn Lys Tyr Asp
1               5                   10                  15

Tyr Leu Pro Thr Thr Val Asn Val Cys Ser Glu Leu Val Lys Leu Val
            20                  25                  30

Phe Cys Val Leu Val Ser Phe Cys Val Ile Lys Lys Asp His Gln Ser
        35                  40                  45

Arg Asn Leu Lys Tyr Ala Ser Trp Lys Glu Phe Ser Asp Phe Met Lys
    50                  55                  60

Trp Ser Ile Pro Ala Phe Leu Tyr Phe Leu Asp Asn Leu Ile Val Phe
65                  70                  75                  80

Tyr Val Leu Ser Tyr Leu Gln Pro Ala Met Ala Val Ile Phe Ser Asn
                85                  90                  95

Phe Ser Ile Ile Thr Thr Ala Leu Leu Phe Arg Ile Val Leu Lys Xaa
            100                 105                 110

Arg Leu Asn Trp Ile Gln Trp Ala Ser Leu Leu Thr Leu Phe Leu Ser
        115                 120                 125

Ile Val Ala Leu Thr Ala Gly Thr Lys Thr Leu Gln His Asn Leu Ala
    130                 135                 140

Gly Arg Gly Phe His His Asp Ala Phe Phe Ser Pro Ser Asn Ser Cys
145                 150                 155                 160

Leu Leu Phe Arg Asn Glu Cys Pro Arg Lys Asp Asn Cys Thr Ala Lys
                165                 170                 175

Glu Trp Thr Phe Pro Glu Ala Lys Trp Asn Thr Thr Ala Arg Val Phe
            180                 185                 190

Ser His Ile Arg Leu Gly Met Gly His Val Leu Ile Ile Val Gln Cys
        195                 200                 205

Phe Ile Ser Ser Met Ala Asn Ile Tyr Asn Glu Lys Ile Leu Lys Glu
    210                 215                 220

Gly Asn Gln Leu Thr Glu Xaa Ile Phe Ile Gln Asn Ser Lys Leu Tyr
225                 230                 235                 240

Phe Phe Gly Ile Leu Phe Asn Gly Leu Thr Leu Gly Leu Gln Arg Ser
                245                 250                 255

Asn Arg Asp Gln Ile Lys Asn Cys Gly Phe Phe Tyr Gly His Ser
            260                 265                 270


(2) INFORMATION FOR SEQ ID NO: 733:

        (i) SEQUENCE CHARACTERISTICS:
                (A) LENGTH: 94 amino acids
                (B) TYPE: amino acid
                (D) TOPOLOGY: linear
        (xi) SEQUENCE DESCRIPTION: SEQ ID NO: 733:

Asn Ser Val Pro Asn Leu Gln Thr Leu Ala Val Leu Thr Glu Ala Ile
  1               5                  10                  15

Gly Pro Glu Pro Ala Ile Pro Arg Xaa Pro Arg Glu Pro Pro Val Ala
            20                  25                  30

Thr Ser Thr Pro Ala Thr Pro Ser Ala Gly Pro Gln Pro Leu Pro Thr
        35                  40                  45

Gly Thr Val Leu Val Pro Gly Gly Pro Ala Pro Pro Cys Leu Gly Glu
    50                  55                  60

Ala Trp Ala Leu Leu Leu Pro Pro Cys Arg Pro Ser Leu Thr Ser Cys
 65                  70                  75                  80

Phe Trp Ser Pro Arg Pro Ser Pro Trp Lys Glu Thr Gly Val
                85                  90


(2) INFORMATION FOR SEQ ID NO: 734:

        (i) SEQUENCE CHARACTERISTICS:
                (A) LENGTH: 40 amino acids
                (B) TYPE: amino acid
                (D) TOPOLOGY: linear
        (xi) SEQUENCE DESCRIPTION: SEQ ID NO: 734:

Ala Leu Gln Leu Ala Phe Tyr Pro Asp Ala Val Glu Glu Trp Leu Glu
  1               5                  10                  15

Glu Asn Val His Pro Ser Leu Gln Arg Leu Gln Xaa Leu Leu Gln Asp
            20                  25                  30

Leu Ser Glu Val Ser Ala Pro Pro
        35                  40

(2) INFORMATION FOR SEQ ID NO: 735:

        (i) SEQUENCE CHARACTERISTICS:
                (A) LENGTH: 30 amino acids
                (B) TYPE: amino acid
                (D) TOPOLOGY: linear
        (xi) SEQUENCE DESCRIPTION: SEQ ID NO: 735:

Cys His Pro Pro Ala Leu Ala Gly Thr Leu Leu Arg Thr Pro Glu Gly
  1               5                  10                  15

Arg Ala His Ala Arg Gly Leu Leu Leu Glu Ala Gly Gly Ala
             20                  25                  30

(2) INFORMATION FOR SEQ ID NO: 736:

        (i) SEQUENCE CHARACTERISTICS:
                (A) LENGTH: 59 amino acids
                (B) TYPE: amino acid
                (D) TOPOLOGY: linear
        (xi) SEQUENCE DESCRIPTION: SEQ ID NO: 736:

Gly Ser Ser Ser Thr Arg Ser Trp Phe Ser Thr Ser Ser Pro Gln Arg
  1               5                  10                  15

Ser Ala Ser Trp His Ser Gly Ala Pro Ser Cys Arg Ser Trp Arg Leu
             20                  25                  30

Pro Cys Ser Trp Leu Ser Thr Arg Met Pro Trp Arg Ser Gly Trp Arg
        35                  40                  45

Lys Thr Cys Thr Pro Ala Cys Ser Gly Cys Lys
    50                  55

(2) INFORMATION FOR SEQ ID NO: 737:

        (i) SEQUENCE CHARACTERISTICS:
                (A) LENGTH: 247 amino acids
                (B) TYPE: amino acid
                (D) TOPOLOGY: linear
        (xi) SEQUENCE DESCRIPTION: SEQ ID NO: 737:

Met Arg Pro Asp Trp Lys Ala Gly Ala Gly Pro Gly Gly Pro Pro Gln
  1               5                  10                  15

Lys Pro Ala Pro Ser Ser Gln Arg Lys Pro Pro Ala Arg Pro Ser Ala
             20                  25                  30

Ala Ala Ala Ala Ile Ala Val Ala Ala Ala Glu Glu Glu Arg Arg Leu
        35                  40                  45

Arg Gln Arg Asn Arg Leu Arg Leu Glu Glu Asp Lys Pro Ala Val Glu
    50                  55                  60

Arg Cys Leu Glu Glu Leu Val Phe Gly Asp Val Glu Asn Asp Glu Asp
65                  70                  75                  80

Ala Leu Leu Arg Arg Leu Arg Gly Pro Arg Val Gln Glu His Glu Asp
                85                  90                  95

Ser Gly Asp Ser Glu Val Glu Asn Glu Ala Lys Gly Asn Phe Pro Pro
            100                 105                 110

Gln Lys Lys Pro Val Trp Val Asp Glu Glu Asp Glu Asp Glu Glu Met
        115                 120                 125

Val Asp Met Met Asn Asn Arg Phe Arg Lys Asp Met Met Lys Asn Ala
    130                 135                 140

Ser Glu Ser Lys Leu Ser Lys Asp Asn Leu Lys Lys Arg Leu Lys Glu
145                 150                 155                 160

Glu Phe Gln His Ala Met Gly Gly Val Pro Ala Trp Ala Glu Thr Thr
                165                 170                 175

Lys Arg Lys Thr Ser Ser Asp Asp Glu Ser Glu Glu Asp Glu Asp Asp
            180                 185                 190

Leu Leu Gln Arg Thr Gly Asn Phe Ile Ser Thr Ser Thr Ser Leu Pro
        195                 200                 205

Arg Gly Ile Leu Lys Met Lys Asn Cys Gln His Ala Asn Ala Glu Arg
    210                 215                 220

Pro Thr Val Ala Arg Ile Ser Ile Cys Ala Val Pro Ser Arg Cys Thr
225                 230                 235                 240

Asp Cys Asp Gly Cys Trp Asp
                245

(2) INFORMATION FOR SEQ ID NO: 738:

(i) SEQUENCE CHARACTERISTICS:
(A) LENGTH: 180 amino acids
(B) TYPE: amino acid
(D) TOPOLOGY: linear
(xi) SEQUENCE DESCRIPTION: SEQ ID NO: 738:

Cys Leu Glu Glu Leu Val Phe Gly Asp Val Glu Asn Asp Glu Asp Ala
1               5                   10                  15

Leu Leu Arg Arg Leu Arg Gly Pro Arg Val Gln Glu His Glu Asp Ser
            20                  25                  30

Gly Asp Ser Glu Val Glu Asn Glu Ala Lys Gly Asn Phe Pro Pro Gln
        35                  40                  45

Lys Lys Pro Val Trp Val Asp Glu Glu Asp Glu Asp Glu Glu Met Val
    50                  55                  60

Asp Met Met Asn Asn Arg Phe Arg Lys Asp Met Met Lys Asn Ala Ser
65                  70                  75                  80

Glu Ser Lys Leu Ser Lys Asp Asn Leu Lys Lys Arg Leu Lys Glu Glu
                85                  90                  95

Phe Gln His Ala Met Gly Gly Val Pro Ala Trp Ala Glu Thr Thr Lys
            100                 105                 110

Arg Lys Thr Ser Ser Asp Asp Glu Ser Glu Glu Asp Glu Asp Asp Leu
        115                 120                 125

Leu Gln Arg Thr Gly Asn Phe Ile Ser Thr Ser Thr Ser Leu Pro Arg
    130                 135                 140

Gly Ile Leu Lys Met Lys Asn Cys Gln His Ala Asn Ala Glu Arg Pro
145                 150                 155                 160

Thr Val Ala Arg Ile Ser Ile Cys Ala Val Pro Ser Arg Cys Thr Asp
                165                 170                 175

Cys Asp Gly Cys
            180

(2) INFORMATION FOR SEQ ID NO: 739:

(i) SEQUENCE CHARACTERISTICS:
(A) LENGTH: 218 amino acids
(B) TYPE: amino acid
(D) TOPOLOGY: linear
(xi) SEQUENCE DESCRIPTION: SEQ ID NO: 739:

Leu Lys Glu Lys Ile Val Arg Ser Phe Glu Val Ser Pro Asp Gly Ser
1               5                   10                  15

Phe Leu Leu Ile Asn Gly Ile Ala Gly Tyr Leu His Leu Leu Ala Met
            20                  25                  30

Lys Thr Lys Glu Leu Ile Gly Ser Met Lys Ile Asn Gly Arg Val Ala
        35                  40                  45

Ala Ser Thr Phe Ser Ser Asp Ser Lys Lys Val Tyr Ala Ser Ser Gly
    50                  55                  60

Asp Gly Glu Val Tyr Val Trp Asp Val Asn Ser Arg Lys Cys Leu Asn
65                  70                  75                  80

Arg Phe Val Asp Glu Gly Ser Leu Tyr Gly Leu Ser Ile Ala Thr Ser
                85                  90                  95

Arg Asn Gly Gln Tyr Val Ala Cys Gly Ser Asn Cys Gly Val Val Asn
            100                 105                 110

Ile Tyr Asn Gln Asp Ser Cys Leu Gln Glu Thr Asn Pro Lys Pro Ile
        115                 120                 125

Lys Ala Ile Met Asn Leu Val Thr Gly Val Thr Ser Leu Thr Phe Asn

130                    135                    140

Pro Thr Thr Glu Ile Leu Ala Ile Ala Ser Glu Lys Met Lys Glu Ala
145                 150                 155                 160

Val Arg Leu Val His Leu Pro Ser Cys Thr Val Phe Ser Asn Phe Pro
                165                 170                 175

Val Ile Lys Asn Lys Asn Ile Ser His Val His Thr Met Asp Phe Ser
            180                 185                 190

Pro Arg Ser Gly Tyr Phe Ala Leu Gly Asn Glu Lys Gly Lys Ala Leu
        195                 200                 205

Met Tyr Arg Leu His His Tyr Ser Asp Phe
    210                 215

(2) INFORMATION FOR SEQ ID NO: 740:

(i) SEQUENCE CHARACTERISTICS:
(A) LENGTH: 167 amino acids
(B) TYPE: amino acid
(D) TOPOLOGY: linear
(xi) SEQUENCE DESCRIPTION: SEQ ID NO: 740:

Lys Ile Asn Gly Arg Val Ala Ala Ser Thr Phe Ser Ser Asp Ser Lys
  1               5                  10                  15

Lys Val Tyr Ala Ser Ser Gly Asp Gly Glu Val Tyr Val Trp Asp Val
             20                  25                  30

Asn Ser Arg Lys Cys Leu Asn Arg Phe Val Asp Glu Gly Ser Leu Tyr
         35                  40                  45

Gly Leu Ser Ile Ala Thr Ser Arg Asn Gly Gln Tyr Val Ala Cys Gly
     50                  55                  60

Ser Asn Cys Gly Val Val Asn Ile Tyr Asn Gln Asp Ser Cys Leu Gln
 65                  70                  75                  80

Glu Thr Asn Pro Lys Pro Ile Lys Ala Ile Met Asn Leu Val Thr Gly
                 85                  90                  95

Val Thr Ser Leu Thr Phe Asn Pro Thr Thr Glu Ile Leu Ala Ile Ala
            100                 105                 110

Ser Glu Lys Met Lys Glu Ala Val Arg Leu Val His Leu Pro Ser Cys
        115                 120                 125

Thr Val Phe Ser Asn Phe Pro Val Ile Lys Asn Lys Asn Ile Ser His
    130                 135                 140

Val His Thr Met Asp Phe Ser Pro Arg Ser Gly Tyr Phe Ala Leu Gly
145                 150                 155                 160

Asn Glu Lys Gly Lys Ala Leu
                165

(2) INFORMATION FOR SEQ ID NO: 741:

(i) SEQUENCE CHARACTERISTICS:
(A) LENGTH: 246 amino acids
(B) TYPE: amino acid
(D) TOPOLOGY: linear
(xi) SEQUENCE DESCRIPTION: SEQ ID NO: 741:

Met Arg Ile Leu Gln Leu Ile Leu Leu Ala Leu Ala Thr Gly Leu Val
1               5                   10                  15

Gly Gly Glu Thr Arg Ile Ile Lys Gly Phe Glu Cys Lys Leu His Ser
            20                  25                  30

Gln Pro Trp Gln Ala Ala Leu Phe Glu Lys Thr Arg Leu Leu Cys Gly
        35                  40                  45

Ala Thr Leu Ile Ala Pro Arg Trp Leu Leu Thr Ala Ala His Cys Leu
    50                  55                  60

Lys Pro Arg Tyr Ile Val His Leu Gly Gln His Asn Leu Gln Lys Glu
65                  70                  75                  80

Glu Gly Cys Glu Gln Thr Arg Thr Ala Thr Glu Ser Phe Pro His Pro
                85                  90                  95

Gly Phe Asn Asn Ser Leu Pro Asn Lys Asp His Arg Asn Asp Ile Met
            100                 105                 110

Leu Val Lys Met Ala Ser Pro Val Ser Ile Thr Trp Ala Val Arg Pro
        115                 120                 125

Leu Thr Leu Ser Ser Arg Cys Val Thr Ala Gly Thr Ser Cys Ser Phe
    130                 135                 140

Pro Ala Gly Ala Ala Arg Pro Asp Pro Ser Tyr Ala Cys Leu Thr Pro
145                 150                 155                 160

Cys Asp Ala Pro Thr Ser Pro Ser Leu Ser Thr Arg Ser Val Arg Thr
                165                 170                 175

Pro Thr Pro Ala Thr Ser Gln Thr Pro Trp Cys Val Pro Ala Cys Arg
            180                 185                 190

Lys Gly Ala Arg Thr Pro Ala Arg Val Thr Pro Gly Ala Leu Trp Ser
        195                 200                 205

Val Thr Ser Leu Phe Lys Ala Leu Ser Pro Gly Ala Arg Ile Arg Val
    210                 215                 220

Arg Ser Pro Glu Ser Leu Val Ser Thr Arg Lys Ser Ala Asn Met Trp
225                 230                 235                 240

Thr Gly Ser Arg Arg Arg
                245

(2) INFORMATION FOR SEQ ID NO: 742:

(i) SEQUENCE CHARACTERISTICS:
(A) LENGTH: 228 amino acids
(B) TYPE: amino acid
(D) TOPOLOGY: linear
(xi) SEQUENCE DESCRIPTION: SEQ ID NO: 742:

Glu Thr Arg Ile Ile Lys Gly Phe Glu Cys Lys Leu His Ser Gln Pro
1               5                   10                  15

Trp Gln Ala Ala Leu Phe Glu Lys Thr Arg Leu Leu Cys Gly Ala Thr
            20                  25                  30

Leu Ile Ala Pro Arg Trp Leu Leu Thr Ala Ala His Cys Leu Lys Pro
        35                  40                  45

Arg Tyr Ile Val His Leu Gly Gln His Asn Leu Gln Lys Glu Glu Gly
    50                  55                  60

Cys Glu Gln Thr Arg Thr Ala Thr Glu Ser Phe Pro His Pro Gly Phe
65                  70                  75                  80

Asn Asn Ser Leu Pro Asn Lys Asp His Arg Asn Asp Ile Met Leu Val
                85                  90                  95

Lys Met Ala Ser Pro Val Ser Ile Thr Trp Ala Val Arg Pro Leu Thr
            100                 105                 110

Leu Ser Ser Arg Cys Val Thr Ala Gly Thr Ser Cys Ser Phe Pro Ala
        115                 120                 125

Gly Ala Ala Arg Pro Asp Pro Ser Tyr Ala Cys Leu Thr Pro Cys Asp
    130                 135                 140

Ala Pro Thr Ser Pro Ser Leu Ser Thr Arg Ser Val Arg Thr Pro Thr
145                 150                 155                 160

Pro Ala Thr Ser Gln Thr Pro Trp Cys Val Pro Ala Cys Arg Lys Gly
                165                 170                 175

Ala Arg Thr Pro Ala Arg Val Thr Pro Gly Ala Leu Trp Ser Val Thr
            180                 185                 190

Ser Leu Phe Lys Ala Leu Ser Pro Gly Ala Arg Ile Arg Val Arg Ser
        195                 200                 205

Pro Glu Ser Leu Val Ser Thr Arg Lys Ser Ala Asn Met Trp Thr Gly
    210                 215                 220

Ser Arg Arg Arg
225

(2) INFORMATION FOR SEQ ID NO: 743:

(i) SEQUENCE CHARACTERISTICS:
(A) LENGTH: 74 amino acids
(B) TYPE: amino acid

(D) TOPOLOGY: linear
(xi) SEQUENCE DESCRIPTION: SEQ ID NO: 743:

Cys Lys Leu His Ser Gln Pro Trp Gln Ala Ala Leu Phe Glu Lys Thr
1               5                   10                  15

Arg Leu Leu Cys Gly Ala Thr Leu Ile Ala Pro Arg Trp Leu Leu Thr
            20                  25                  30

Ala Ala His Cys Leu Lys Pro Arg Tyr Ile Val His Leu Gly Gln His
        35                  40                  45

Asn Leu Gln Lys Glu Glu Gly Cys Glu Gln Thr Arg Thr Ala Thr Glu
    50                  55                  60

Ser Phe Pro His Pro Gly Phe Asn Asn Ser
65                  70

(2) INFORMATION FOR SEQ ID NO: 744:

(i) SEQUENCE CHARACTERISTICS:
(A) LENGTH: 81 amino acids
(B) TYPE: amino acid
(D) TOPOLOGY: linear
(xi) SEQUENCE DESCRIPTION: SEQ ID NO: 744:

Val Leu Gln Gly Arg Tyr Phe Ser Pro Ile Leu Glu Met Arg Arg Leu
1               5                   10                  15

Arg Pro Glu Gly Xaa Xaa Asn Leu Pro Gly Gly Ser Arg Ala Gln Lys
            20                  25                  30

Glu Pro Arg Gln Asp Leu Thr Leu Val Leu Trp Pro His Cys Pro His
        35                  40                  45

Phe Ala Met Thr Arg Ser Tyr Val Pro Thr Lys Gln Cys Met Val Gln
    50                  55                  60

Gly Ser Phe Tyr Cys Ile Phe Ile Phe Lys Gly Pro Val Gln Asn Trp
65                  70                  75                  80

Cys

(2) INFORMATION FOR SEQ ID NO: 745:

(i) SEQUENCE CHARACTERISTICS:
(A) LENGTH: 211 amino acids
(B) TYPE: amino acid
(D) TOPOLOGY: linear
(xi) SEQUENCE DESCRIPTION: SEQ ID NO: 745:

Met Pro Ile Ile Asp Gln Val Asn Pro Glu Leu His Asp Phe Met Gln
1               5                   10                  15

Ser Ala Glu Val Gly Thr Ile Phe Ala Leu Ser Trp Leu Ile Thr Trp

20                  25                  30

Phe Gly His Val Leu Ser Asp Phe Arg His Val Val Arg Leu Tyr Asp
        35                  40                  45

Phe Phe Leu Ala Cys His Pro Leu Met Pro Ile Tyr Phe Ala Ala Val
    50                  55                  60

Ile Val Leu Tyr Arg Glu Gln Glu Val Leu Asp Cys Asp Cys Asp Met
65                  70                  75                  80

Ala Ser Val His His Leu Leu Ser Gln Ile Pro Gln Asp Leu Pro Tyr
                85                  90                  95

Glu Thr Leu Ile Ser Arg Xaa Glu Thr Phe Leu Phe Ser Phe Pro His
            100                 105                 110

Pro Asn Leu Leu Gly Arg Pro Leu Pro Asn Ser Lys Leu Arg Gly Arg
        115                 120                 125

Gln Pro Leu Leu Ser Lys Thr Leu Ser Trp His Gln Pro Ser Arg Gly
    130                 135                 140

Leu Ile Trp Cys Cys Gly Ser Gly Xaa Arg Gly Leu Leu Arg Pro Glu
145                 150                 155                 160

Asp Arg Thr Lys Asp Val Leu Thr Lys Pro Arg Thr Asn Arg Phe Val
                165                 170                 175

Lys Leu Ala Val Met Gly Leu Thr Val Ala Leu Gly Ala Ala Ala Leu
            180                 185                 190

Ala Val Val Lys Ser Ala Leu Glu Trp Ala Pro Lys Phe Gln Leu Gln
        195                 200                 205

Leu Phe Pro
    210

(2) INFORMATION FOR SEQ ID NO: 746:

     (i) SEQUENCE CHARACTERISTICS:
          (A) LENGTH: 70 amino acids
          (B) TYPE: amino acid
          (D) TOPOLOGY: linear
     (xi) SEQUENCE DESCRIPTION: SEQ ID NO: 746:

Cys Pro Glu Phe Phe Ile Pro Ala Thr Leu Pro Cys Pro Phe Val Phe
1               5                   10                  15

Ala Phe Thr Ser Glu Ala Ser Ser Arg Ala Tyr Leu Thr Gln Arg Gly
            20                  25                  30

Pro Gly Gly Leu Ala Gln Asn Leu Met Pro Leu Pro Val Gly Phe Trp
        35                  40                  45

Met Gly Ser Leu Pro Pro Pro Trp Cys Trp Arg Lys Trp Val Ser Glu
    50                  55                  60

Ala Cys Ser Cys Phe Cys
65                  70

(2) INFORMATION FOR SEQ ID NO: 747:

(i) SEQUENCE CHARACTERISTICS:
(A) LENGTH: 20 amino acids
(B) TYPE: amino acid
(D) TOPOLOGY: linear
(xi) SEQUENCE DESCRIPTION: SEQ ID NO: 747:

Gly Phe Gly Ser Val Ser Ala Ala Gly Arg Arg Ser Gly Gly Thr Trp
1               5                   10                  15

Gln Pro Val Gln
            20

(2) INFORMATION FOR SEQ ID NO: 748:

(i) SEQUENCE CHARACTERISTICS:
(A) LENGTH: 16 amino acids
(B) TYPE: amino acid
(D) TOPOLOGY: linear
(xi) SEQUENCE DESCRIPTION: SEQ ID NO: 748:

Pro Gly Gly Leu Ala Val Gly Ser Arg Trp Trp Ser Arg Ser Leu Thr
1               5                   10                  15

(2) INFORMATION FOR SEQ ID NO: 749:

(i) SEQUENCE CHARACTERISTICS:
(A) LENGTH: 30 amino acids
(B) TYPE: amino acid
(D) TOPOLOGY: linear
(xi) SEQUENCE DESCRIPTION: SEQ ID NO: 749:

Leu Glu Pro Ser Arg Gln Arg Arg Pro Arg Arg Arg Gly Gly Thr Ser
1               5                   10                  15

Arg Pro Glu Thr Asp Gln Arg Ala Lys Cys Trp Arg Gln Leu
            20                  25                  30

(2) INFORMATION FOR SEQ ID NO: 750:

(i) SEQUENCE CHARACTERISTICS:
(A) LENGTH: 11 amino acids
(B) TYPE: amino acid
(D) TOPOLOGY: linear
(xi) SEQUENCE DESCRIPTION: SEQ ID NO: 750:

Val Cys Leu Arg Cys Gln Asn Arg Met Glu Asn
1               5                   10

(2) INFORMATION FOR SEQ ID NO: 751:

(i) SEQUENCE CHARACTERISTICS:
(A) LENGTH: 367 amino acids
(B) TYPE: amino acid
(D) TOPOLOGY: linear
(xi) SEQUENCE DESCRIPTION: SEQ ID NO: 751:

Met Ala Ala Cys Thr Ala Arg Arg Pro Gly Arg Gly Gln Pro Leu Val
1               5                   10                  15

Val Pro Val Ala Asp Xaa Gly Pro Val Ala Lys Ala Ala Leu Cys Ala
            20                  25                  30

Ala Xaa Ala Gly Ala Phe Ser Pro Ala Ser Thr Thr Thr Thr Arg Arg
        35                  40                  45

His Leu Ser Ser Arg Asn Arg Pro Glu Gly Lys Val Leu Glu Thr Val
    50                  55                  60

Gly Val Phe Glu Val Pro Lys Gln Asn Gly Lys Tyr Glu Thr Gly Gln
65                  70                  75                  80

Leu Phe Leu His Ser Ile Phe Gly Tyr Arg Gly Val Val Leu Phe Pro
                85                  90                  95

Trp Gln Ala Arg Leu Xaa Asp Arg Asp Val Ala Ser Ala Ala Pro Glu
            100                 105                 110

Lys Ala Glu Asn Pro Ala Gly His Gly Ser Lys Glu Val Lys Gly Lys
        115                 120                 125

Thr His Thr Tyr Tyr Gln Val Leu Ile Asp Ala Arg Asp Cys Pro His
    130                 135                 140

Ile Ser Gln Arg Ser Gln Thr Glu Ala Val Thr Phe Leu Ala Asn His
145                 150                 155                 160

Asp Asp Ser Arg Ala Leu Tyr Ala Ile Pro Gly Leu Asp Tyr Val Ser
                165                 170                 175

His Glu Asp Ile Leu Pro Tyr Thr Ser Thr Asp Gln Val Pro Ile Gln
            180                 185                 190

His Glu Leu Phe Glu Arg Phe Leu Leu Tyr Asp Gln Thr Lys Ala Pro
        195                 200                 205

Pro Phe Val Ala Arg Glu Thr Leu Arg Ala Trp Gln Glu Lys Asn His
    210                 215                 220

Pro Trp Leu Glu Leu Ser Asp Val His Arg Glu Thr Thr Glu Asn Ile
225                 230                 235                 240

Arg Val Thr Val Ile Pro Phe Tyr Met Gly Met Arg Glu Ala Gln Asn

                245                 250                 255

Ser His Val Tyr Trp Trp Arg Tyr Cys Ile Arg Leu Glu Asn Leu Asp
            260                 265                 270

Ser Asp Val Val Gln Leu Arg Glu Arg His Trp Arg Ile Phe Ser Leu
        275                 280                 285

Ser Gly Thr Leu Glu Thr Val Arg Gly Arg Gly Val Val Gly Arg Glu
    290                 295                 300

Pro Val Leu Ser Lys Glu Gln Pro Ala Phe Gln Tyr Ser Ser His Val
305                 310                 315                 320

Ser Leu Gln Ala Ser Ser Gly His Met Trp Gly Thr Phe Arg Phe Glu
                325                 330                 335

Arg Pro Asp Gly Ser His Phe Asp Val Arg Ile Pro Pro Phe Ser Leu
            340                 345                 350

Glu Ser Asn Lys Asp Glu Lys Thr Pro Pro Ser Gly Leu His Trp
        355                 360                 365

(2) INFORMATION FOR SEQ ID NO: 752:

(i) SEQUENCE CHARACTERISTICS:
(A) LENGTH: 33 amino acids
(B) TYPE: amino acid
(D) TOPOLOGY: linear
(xi) SEQUENCE DESCRIPTION: SEQ ID NO: 752:

Met Ala Ala Cys Thr Ala Arg Arg Pro Gly Arg Gly Gln Pro Leu Val
1               5                   10                  15

Val Pro Val Ala Asp Xaa Gly Pro Val Ala Lys Ala Ala Leu Cys Ala
            20                  25                  30

Ala

(2) INFORMATION FOR SEQ ID NO: 753:

(i) SEQUENCE CHARACTERISTICS:
(A) LENGTH: 33 amino acids
(B) TYPE: amino acid
(D) TOPOLOGY: linear
(xi) SEQUENCE DESCRIPTION: SEQ ID NO: 753:

Met Ala Ala Cys Thr Ala Arg Arg Pro Gly Arg Gly Gln Pro Leu Val
1               5                   10                  15

Val Pro Val Ala Asp Xaa Gly Pro Val Ala Lys Ala Ala Leu Cys Ala
            20                  25                  30

Ala

(2) INFORMATION FOR SEQ ID NO: 754:

(i) SEQUENCE CHARACTERISTICS:
(A) LENGTH: 33 amino acids
(B) TYPE: amino acid
(D) TOPOLOGY: linear
(xi) SEQUENCE DESCRIPTION: SEQ ID NO: 754:

Met Ala Ala Cys Thr Ala Arg Arg Pro Gly Arg Gly Gln Pro Leu Val
1               5                   10                  15

Val Pro Val Ala Asp Xaa Gly Pro Val Ala Lys Ala Ala Leu Cys Ala
            20                  25                  30

Ala

(2) INFORMATION FOR SEQ ID NO: 755:

(i) SEQUENCE CHARACTERISTICS:
(A) LENGTH: 33 amino acids
(B) TYPE: amino acid
(D) TOPOLOGY: linear
(xi) SEQUENCE DESCRIPTION: SEQ ID NO: 755:

Met Ala Ala Cys Thr Ala Arg Arg Pro Gly Arg Gly Gln Pro Leu Val
1               5                   10                  15

Val Pro Val Ala Asp Xaa Gly Pro Val Ala Lys Ala Ala Leu Cys Ala
            20                  25                  30

Ala

(2) INFORMATION FOR SEQ ID NO: 756:

(i) SEQUENCE CHARACTERISTICS:
(A) LENGTH: 33 amino acids
(B) TYPE: amino acid
(D) TOPOLOGY: linear
(xi) SEQUENCE DESCRIPTION: SEQ ID NO: 756:

Met Ala Ala Cys Thr Ala Arg Arg Pro Gly Arg Gly Gln Pro Leu Val
1               5                   10                  15

Val Pro Val Ala Asp Xaa Gly Pro Val Ala Lys Ala Ala Leu Cys Ala
            20                  25                  30

Ala

(2) INFORMATION FOR SEQ ID NO: 757:

(i) SEQUENCE CHARACTERISTICS:
(A) LENGTH: 35 amino acids
(B) TYPE: amino acid
(D) TOPOLOGY: linear
(xi) SEQUENCE DESCRIPTION: SEQ ID NO: 757:

Val Leu Glu Thr Val Gly Val Phe Glu Val Pro Lys Gln Asn Gly Lys
1               5                   10                  15

Tyr Glu Thr Gly Gln Leu Phe Leu His Ser Ile Phe Gly Tyr Arg Gly
            20                  25                  30

Val Val Leu
        35

(2) INFORMATION FOR SEQ ID NO: 758:

(i) SEQUENCE CHARACTERISTICS:
(A) LENGTH: 16 amino acids
(B) TYPE: amino acid
(D) TOPOLOGY: linear
(xi) SEQUENCE DESCRIPTION: SEQ ID NO: 758:

Gly Leu Asp Tyr Val Ser His Glu Asp Ile Leu Pro Tyr Thr Ser Thr
1               5                   10                  15

(2) INFORMATION FOR SEQ ID NO: 759:

(i) SEQUENCE CHARACTERISTICS:
(A) LENGTH: 19 amino acids
(B) TYPE: amino acid
(D) TOPOLOGY: linear
(xi) SEQUENCE DESCRIPTION: SEQ ID NO: 759:

Asp Val His Arg Glu Thr Thr Glu Asn Ile Arg Val Thr Val Ile Pro
1               5                   10                  15

Phe Tyr Met

(2) INFORMATION FOR SEQ ID NO: 760:

(i) SEQUENCE CHARACTERISTICS:
(A) LENGTH: 21 amino acids
(B) TYPE: amino acid
(D) TOPOLOGY: linear
(xi) SEQUENCE DESCRIPTION: SEQ ID NO: 760:

Trp Trp Arg Tyr Cys Ile Arg Leu Glu Asn Leu Asp Ser Asp Val Val

1                 5                   10                  15

Gln Leu Arg Glu Arg
            20

(2) INFORMATION FOR SEQ ID NO: 761:

      (i) SEQUENCE CHARACTERISTICS:
            (A) LENGTH: 26 amino acids
            (B) TYPE: amino acid
            (D) TOPOLOGY: linear
      (xi) SEQUENCE DESCRIPTION: SEQ ID NO: 761:

Pro Ala Phe Gln Tyr Ser Ser His Val Ser Leu Gln Ala Ser Ser Gly
 1               5                  10                  15

His Met Trp Gly Thr Phe Arg Phe Glu Arg
            20                  25

(2) INFORMATION FOR SEQ ID NO: 762:

      (i) SEQUENCE CHARACTERISTICS:
            (A) LENGTH: 11 amino acids
            (B) TYPE: amino acid
            (D) TOPOLOGY: linear
      (xi) SEQUENCE DESCRIPTION: SEQ ID NO: 762:

Ser Leu Cys Cys Pro Glu Gly Ala Glu Gly Cys
 1               5                  10

(2) INFORMATION FOR SEQ ID NO: 763:

      (i) SEQUENCE CHARACTERISTICS:
            (A) LENGTH: 12 amino acids
            (B) TYPE: amino acid
            (D) TOPOLOGY: linear
      (xi) SEQUENCE DESCRIPTION: SEQ ID NO: 763:

Gln Leu Lys Lys Thr His Tyr Asp Arg Pro Cys Pro
 1               5                  10

(2) INFORMATION FOR SEQ ID NO: 764:

      (i) SEQUENCE CHARACTERISTICS:
            (A) LENGTH: 12 amino acids
            (B) TYPE: amino acid
            (D) TOPOLOGY: linear
      (xi) SEQUENCE DESCRIPTION: SEQ ID NO: 764:

Gln Leu Lys Lys Thr His Tyr Asp Arg Pro Cys Pro
 1               5                  10

(2) INFORMATION FOR SEQ ID NO: 765:

(i) SEQUENCE CHARACTERISTICS:
(A) LENGTH: 170 amino acids
(B) TYPE: amino acid
(D) TOPOLOGY: linear
(xi) SEQUENCE DESCRIPTION: SEQ ID NO: 765:

Ala Gln Arg Lys Lys Glu Met Val Leu Ser Glu Lys Val Ser Gln Leu
1               5                   10                  15

Met Glu Trp Thr Asn Lys Arg Pro Val Ile Arg Met Asn Gly Asp Lys
            20                  25                  30

Phe Arg Arg Leu Val Lys Ala Pro Pro Arg Asn Tyr Ser Val Ile Val
        35                  40                  45

Met Phe Thr Ala Leu Gln Leu His Arg Gln Cys Val Val Cys Lys Gln
    50                  55                  60

Ala Asp Glu Glu Phe Gln Ile Leu Ala Asn Ser Trp Arg Tyr Ser Ser
65                  70                  75                  80

Ala Phe Thr Asn Arg Ile Phe Phe Ala Met Val Asp Phe Asp Glu Gly
                85                  90                  95

Ser Asp Val Phe Gln Met Leu Asn Met Asn Ser Ala Pro Thr Phe Ile
            100                 105                 110

Asn Phe Pro Ala Lys Gly Lys Pro Lys Arg Gly Asp Thr Tyr Glu Leu
        115                 120                 125

Gln Val Arg Gly Phe Ser Ala Glu Gln Ile Ala Arg Trp Ile Ala Asp
    130                 135                 140

Arg Thr Asp Val Asn Ile Arg Val Ile Arg Pro Pro Asn Met Ala Ala
145                 150                 155                 160

Arg Trp Arg Phe Trp Cys Val Ser Val Thr
                165                 170

(2) INFORMATION FOR SEQ ID NO: 766:

(i) SEQUENCE CHARACTERISTICS:
(A) LENGTH: 15 amino acids
(B) TYPE: amino acid
(D) TOPOLOGY: linear
(xi) SEQUENCE DESCRIPTION: SEQ ID NO: 766:

Met Val Val Ala Leu Leu Ile Val Cys Asp Val Pro Ser Ala Ser
1               5                   10                  15

(2) INFORMATION FOR SEQ ID NO: 767:

(i) SEQUENCE CHARACTERISTICS:
(A) LENGTH: 16 amino acids
(B) TYPE: amino acid
(D) TOPOLOGY: linear
(xi) SEQUENCE DESCRIPTION: SEQ ID NO: 767:

Ala Gln Arg Lys Lys Glu Met Val Leu Ser Glu Lys Val Ser Gln Leu
1               5                   10                  15

(2) INFORMATION FOR SEQ ID NO: 768:

(i) SEQUENCE CHARACTERISTICS:
(A) LENGTH: 17 amino acids
(B) TYPE: amino acid
(D) TOPOLOGY: linear
(xi) SEQUENCE DESCRIPTION: SEQ ID NO: 768:

Met Glu Trp Thr Asn Lys Arg Pro Val Ile Arg Met Asn Gly Asp Lys
1               5                   10                  15

Phe

(2) INFORMATION FOR SEQ ID NO: 769:

(i) SEQUENCE CHARACTERISTICS:
(A) LENGTH: 56 amino acids
(B) TYPE: amino acid
(D) TOPOLOGY: linear
(xi) SEQUENCE DESCRIPTION: SEQ ID NO: 769:

Arg Arg Leu Val Lys Ala Pro Pro Arg Asn Tyr Ser Val Ile Val Met
1               5                   10                  15

Phe Thr Ala Leu Gln Leu His Arg Gln Cys Val Val Cys Lys Gln Ala
            20                  25                  30

Asp Glu Glu Phe Gln Ile Leu Ala Asn Ser Trp Arg Tyr Ser Ser Ala
        35                  40                  45

Phe Thr Asn Arg Ile Phe Phe Ala
    50                  55

(2) INFORMATION FOR SEQ ID NO: 770:

(i) SEQUENCE CHARACTERISTICS:
(A) LENGTH: 31 amino acids
(B) TYPE: amino acid
(D) TOPOLOGY: linear
(xi) SEQUENCE DESCRIPTION: SEQ ID NO: 770:

Met Val Asp Phe Asp Glu Gly Ser Asp Val Phe Gln Met Leu Asn Met
1               5                   10                  15

Asn Ser Ala Pro Thr Phe Ile Asn Phe Pro Ala Lys Gly Lys Pro
            20                  25                  30

(2) INFORMATION FOR SEQ ID NO: 771:

(i) SEQUENCE CHARACTERISTICS:
(A) LENGTH: 37 amino acids
(B) TYPE: amino acid
(D) TOPOLOGY: linear
(xi) SEQUENCE DESCRIPTION: SEQ ID NO: 771:

Lys Arg Gly Asp Thr Tyr Glu Leu Gln Val Arg Gly Phe Ser Ala Glu
1               5                   10                  15

Gln Ile Ala Arg Trp Ile Ala Asp Arg Thr Asp Val Asn Ile Arg Val
            20                  25                  30

Ile Arg Pro Pro Asn
        35

(2) INFORMATION FOR SEQ ID NO: 772:

(i) SEQUENCE CHARACTERISTICS:
(A) LENGTH: 44 amino acids
(B) TYPE: amino acid
(D) TOPOLOGY: linear
(xi) SEQUENCE DESCRIPTION: SEQ ID NO: 772:

Tyr Ala Gly Pro Leu Met Leu Gly Leu Leu Leu Ala Val Ile Gly Gly
1               5                   10                  15

Leu Val Tyr Leu Arg Arg Val Ile Trp Asn Phe Ser Leu Ile Lys Leu
            20                  25                  30

Asp Gly Leu Leu Gln Leu Cys Val Leu Cys Leu Leu
        35                  40

(2) INFORMATION FOR SEQ ID NO: 773:

(i) SEQUENCE CHARACTERISTICS:
(A) LENGTH: 17 amino acids
(B) TYPE: amino acid
(D) TOPOLOGY: linear
(xi) SEQUENCE DESCRIPTION: SEQ ID NO: 773:

Asp Ala Val Phe Lys Gly Phe Ser Asp Cys Leu Leu Lys Leu Gly Asp
1               5                   10                  15

Ser

(2) INFORMATION FOR SEQ ID NO: 774:

(i) SEQUENCE CHARACTERISTICS:
(A) LENGTH: 20 amino acids
(B) TYPE: amino acid
(D) TOPOLOGY: linear
(xi) SEQUENCE DESCRIPTION: SEQ ID NO: 774:

Cys Gln Glu Gly Ala Lys Asp Met Trp Asp Lys Leu Arg Lys Glu Ser
1               5                   10                  15

Lys Asn Leu Asn
            20

(2) INFORMATION FOR SEQ ID NO: 775:

(i) SEQUENCE CHARACTERISTICS:
(A) LENGTH: 16 amino acids
(B) TYPE: amino acid
(D) TOPOLOGY: linear
(xi) SEQUENCE DESCRIPTION: SEQ ID NO: 775:

Val Leu Leu Val Ser Leu Ser Ala Ala Leu Ala Thr Trp Leu Ser Phe
1               5                   10                  15

(2) INFORMATION FOR SEQ ID NO: 776:

(i) SEQUENCE CHARACTERISTICS:
(A) LENGTH: 48 amino acids
(B) TYPE: amino acid
(D) TOPOLOGY: linear
(xi) SEQUENCE DESCRIPTION: SEQ ID NO: 776:

Met Gly Leu Lys Leu Asn Gly Arg Tyr Ile Ser Leu Ile Leu Ala Val
1               5                   10                  15

Gln Ile Ala Tyr Leu Val Gln Ala Val Arg Ala Ala Gly Lys Cys Asp
            20                  25                  30

Ala Val Phe Lys Gly Phe Ser Asp Cys Leu Leu Lys Leu Gly Asp Ser
        35                  40                  45

(2) INFORMATION FOR SEQ ID NO: 777:

(i) SEQUENCE CHARACTERISTICS:
(A) LENGTH: 90 amino acids

(B) TYPE: amino acid
(D) TOPOLOGY: linear
(xi) SEQUENCE DESCRIPTION: SEQ ID NO: 777:

Pro Ala Ala Trp Asp Asp Lys Thr Asn Ile Lys Thr Val Cys Thr Tyr
1               5                   10                  15

Trp Glu Asp Phe His Ser Cys Thr Val Thr Ala Leu Thr Asp Cys Gln
            20                  25                  30

Glu Gly Ala Lys Asp Met Trp Asp Lys Leu Arg Lys Glu Ser Lys Asn
        35                  40                  45

Leu Asn Ile Gln Gly Ser Leu Phe Glu Leu Cys Gly Ser Gly Asn Gly
    50                  55                  60

Ala Ala Gly Ser Leu Leu Pro Ala Phe Pro Val Leu Leu Val Ser Leu
65                  70                  75                  80

Ser Ala Ala Leu Ala Thr Trp Leu Ser Phe
                85                  90

(2) INFORMATION FOR SEQ ID NO: 778:

(i) SEQUENCE CHARACTERISTICS:
(A) LENGTH: 143 amino acids
(B) TYPE: amino acid
(D) TOPOLOGY: linear
(xi) SEQUENCE DESCRIPTION: SEQ ID NO: 778:

Met Gly Leu Lys Leu Asn Gly Arg Tyr Ile Ser Leu Ile Leu Ala Val
1               5                   10                  15

Gln Ile Ala Tyr Leu Val Gln Ala Val Arg Ala Ala Gly Lys Cys Asp
            20                  25                  30

Ala Val Phe Lys Gly Phe Ser Asp Cys Leu Leu Lys Leu Gly Asp Ser
        35                  40                  45

Xaa Xaa Xaa Xaa Xaa Pro Ala Ala Trp Asp Asp Lys Thr Asn Ile Lys
    50                  55                  60

Thr Val Cys Thr Tyr Trp Glu Asp Phe His Ser Cys Thr Val Thr Ala
65                  70                  75                  80

Leu Thr Asp Cys Gln Glu Gly Ala Lys Asp Met Trp Asp Lys Leu Arg
                85                  90                  95

Lys Glu Ser Lys Asn Leu Asn Ile Gln Gly Ser Leu Phe Glu Leu Cys
            100                 105                 110

Gly Ser Gly Asn Gly Ala Ala Gly Ser Leu Leu Pro Ala Phe Pro Val
        115                 120                 125

Leu Leu Val Ser Leu Ser Ala Ala Leu Ala Thr Trp Leu Ser Phe
    130                 135                 140

(2) INFORMATION FOR SEQ ID NO: 779:

(i) SEQUENCE CHARACTERISTICS:
(A) LENGTH: 34 amino acids
(B) TYPE: amino acid
(D) TOPOLOGY: linear
(xi) SEQUENCE DESCRIPTION: SEQ ID NO: 779:

Met Asn Ser Ala Ala Gly Phe Ser His Leu Asp Arg Arg Glu Arg Val
1               5                   10                  15

Leu Lys Leu Gly Glu Ser Phe Glu Lys Gln Pro Arg Cys Ala Ser Thr
            20                  25                  30

Leu Cys

(2) INFORMATION FOR SEQ ID NO: 780:

(i) SEQUENCE CHARACTERISTICS:
(A) LENGTH: 28 amino acids
(B) TYPE: amino acid
(D) TOPOLOGY: linear
(xi) SEQUENCE DESCRIPTION: SEQ ID NO: 780:

Thr Ile Tyr Pro Thr Glu Glu Glu Leu Gln Ala Val Gln Lys Ile Val
1               5                   10                  15

Ser Ile Thr Glu Arg Ala Leu Lys Leu Val Ser Asp
            20                  25

(2) INFORMATION FOR SEQ ID NO: 781:

(i) SEQUENCE CHARACTERISTICS:
(A) LENGTH: 30 amino acids
(B) TYPE: amino acid
(D) TOPOLOGY: linear
(xi) SEQUENCE DESCRIPTION: SEQ ID NO: 781:

Arg Ala Leu Lys Gly Val Leu Arg Val Gly Val Leu Ala Lys Gly Leu
1               5                   10                  15

Leu Leu Arg Gly Asp Arg Asn Val Asn Leu Val Leu Leu Cys
            20                  25                  30

(2) INFORMATION FOR SEQ ID NO: 782:

(i) SEQUENCE CHARACTERISTICS:
(A) LENGTH: 39 amino acids
(B) TYPE: amino acid
(D) TOPOLOGY: linear
(xi) SEQUENCE DESCRIPTION: SEQ ID NO: 782:

Ala Leu Ala Ala Leu Arg His Ala Lys Trp Phe Gln Ala Arg Ala Asn
1               5                   10                  15

Gly Leu Gln Ser Cys Val Ile Ile Ile Arg Ile Leu Arg Asp Leu Cys
            20                  25                  30

Gln Arg Val Pro Thr Trp Ser
        35

(2) INFORMATION FOR SEQ ID NO: 783:

(i) SEQUENCE CHARACTERISTICS:
(A) LENGTH: 17 amino acids
(B) TYPE: amino acid
(D) TOPOLOGY: linear
(xi) SEQUENCE DESCRIPTION: SEQ ID NO: 783:

Gly Asp Ala Leu Arg Arg Val Phe Glu Cys Ile Ser Ser Gly Ile Ile
1               5                   10                  15

Leu

(2) INFORMATION FOR SEQ ID NO: 784:

(i) SEQUENCE CHARACTERISTICS:
(A) LENGTH: 16 amino acids
(B) TYPE: amino acid
(D) TOPOLOGY: linear
(xi) SEQUENCE DESCRIPTION: SEQ ID NO: 784:

Leu Ala Phe Arg Gln Ile His Lys Val Leu Gly Met Asp Pro Leu Pro
1               5                   10                  15

(2) INFORMATION FOR SEQ ID NO: 785:

(i) SEQUENCE CHARACTERISTICS:
(A) LENGTH: 342 amino acids
(B) TYPE: amino acid
(D) TOPOLOGY: linear
(xi) SEQUENCE DESCRIPTION: SEQ ID NO: 785:

Thr Ile Tyr Pro Thr Glu Glu Glu Leu Gln Ala Val Gln Lys Ile Val
1               5                   10                  15

Ser Ile Thr Glu Arg Ala Leu Lys Leu Val Ser Asp Ser Leu Ser Glu
            20                  25                  30

His Glu Lys Asn Lys Asn Lys Glu Gly Asp Asp Lys Lys Glu Gly Gly
        35                  40                  45

Lys Asp Arg Ala Leu Lys Gly Val Leu Arg Val Gly Val Leu Ala Lys
    50                  55                  60

Gly Leu Leu Leu Arg Gly Asp Arg Asn Val Asn Leu Val Leu Leu Cys
65                  70                  75                  80

Ser Glu Lys Pro Ser Lys Thr Leu Leu Ser Arg Ile Ala Glu Asn Leu
                85                  90                  95

Pro Lys Gln Leu Ala Val Ile Ser Pro Glu Lys Tyr Asp Ile Lys Cys
            100                 105                 110

Ala Val Ser Glu Ala Ala Ile Ile Leu Asn Ser Cys Val Glu Pro Lys
        115                 120                 125

Met Gln Val Thr Ile Thr Leu Thr Ser Pro Ile Ile Arg Glu Glu Asn
    130                 135                 140

Met Arg Glu Gly Asp Val Thr Ser Gly Met Val Lys Asp Pro Pro Asp
145                 150                 155                 160

Val Leu Asp Arg Gln Lys Cys Leu Asp Ala Leu Ala Ala Leu Arg His
                165                 170                 175

Ala Lys Trp Phe Gln Ala Arg Ala Asn Gly Leu Gln Ser Cys Val Ile
            180                 185                 190

Ile Ile Arg Ile Leu Arg Asp Leu Cys Gln Arg Val Pro Thr Trp Ser
        195                 200                 205

Asp Phe Pro Ser Trp Ala Met Glu Leu Leu Val Glu Lys Ala Ile Ser
    210                 215                 220

Ser Ala Ser Ser Pro Gln Ser Pro Gly Asp Ala Leu Arg Arg Val Phe
225                 230                 235                 240

Glu Cys Ile Ser Ser Gly Ile Ile Leu Lys Gly Ser Pro Gly Leu Leu
                245                 250                 255

Asp Pro Cys Glu Lys Asp Pro Phe Asp Thr Leu Ala Thr Met Thr Asp
            260                 265                 270

Gln Gln Arg Glu Asp Ile Thr Ser Ser Ala Gln Phe Ala Leu Arg Leu
        275                 280                 285

Leu Ala Phe Arg Gln Ile His Lys Val Leu Gly Met Asp Pro Leu Pro
    290                 295                 300

Gln Met Ser Gln Arg Phe Asn Ile His Asn Asn Arg Lys Arg Arg Arg
305                 310                 315                 320

Asp Ser Asp Gly Val Asp Gly Phe Glu Ala Glu Gly Lys Lys Asp Lys
                325                 330                 335

Lys Asp Tyr Asp Asn Phe
            340

(2) INFORMATION FOR SEQ ID NO: 786:

(i) SEQUENCE CHARACTERISTICS:
(A) LENGTH: 24 amino acids
(B) TYPE: amino acid
(D) TOPOLOGY: linear
(xi) SEQUENCE DESCRIPTION: SEQ ID NO: 786:

Met Gly Ser Gln His Ser Ala Ala Ala Arg Pro Ser Ser Cys Arg Arg
1               5                   10                  15

Lys Gln Glu Asp Asp Arg Asp Gly
            20

(2) INFORMATION FOR SEQ ID NO: 787:

(i) SEQUENCE CHARACTERISTICS:
(A) LENGTH: 30 amino acids
(B) TYPE: amino acid
(D) TOPOLOGY: linear
(xi) SEQUENCE DESCRIPTION: SEQ ID NO: 787:

Leu Leu Ala Glu Arg Glu Gln Glu Glu Ala Ile Ala Gln Phe Pro Tyr
1               5                   10                  15

Val Glu Phe Thr Gly Arg Asp Ser Ile Thr Cys Leu Thr Cys
            20                  25                  30

(2) INFORMATION FOR SEQ ID NO: 788:

(i) SEQUENCE CHARACTERISTICS:
(A) LENGTH: 34 amino acids
(B) TYPE: amino acid
(D) TOPOLOGY: linear
(xi) SEQUENCE DESCRIPTION: SEQ ID NO: 788:

Gln Gly Thr Gly Tyr Ile Pro Thr Glu Gln Val Asn Glu Leu Val Ala
1               5                   10                  15

Leu Ile Pro His Ser Asp Gln Arg Leu Arg Pro Gln Arg Thr Lys Gln
            20                  25                  30

Tyr Val

(2) INFORMATION FOR SEQ ID NO: 789:

(i) SEQUENCE CHARACTERISTICS:
(A) LENGTH: 55 amino acids
(B) TYPE: amino acid
(D) TOPOLOGY: linear
(xi) SEQUENCE DESCRIPTION: SEQ ID NO: 789:

Ala Arg Leu Asn Val Gly Arg Glu Ser Leu Lys Arg Glu Met Leu Lys
1               5                   10                  15

Ser Gln Gly Val Lys Val Ser Glu Ser Pro Met Gly Ala Arg His Ser
            20                  25                  30

Ser Trp Pro Glu Gly Ala Ala Phe Cys Lys Lys Val Gln Gly Ala Gln
        35                  40                  45

Met Gln Phe Pro Pro Arg Arg
    50                  55

(2) INFORMATION FOR SEQ ID NO: 790:

(i) SEQUENCE CHARACTERISTICS:
(A) LENGTH: 15 amino acids
(B) TYPE: amino acid
(D) TOPOLOGY: linear
(xi) SEQUENCE DESCRIPTION: SEQ ID NO: 790:

Ala Arg Leu Asn Val Gly Arg Glu Ser Leu Lys Arg Glu Met Leu
1               5                   10                  15

(2) INFORMATION FOR SEQ ID NO: 791:

(i) SEQUENCE CHARACTERISTICS:
(A) LENGTH: 20 amino acids
(B) TYPE: amino acid
(D) TOPOLOGY: linear
(xi) SEQUENCE DESCRIPTION: SEQ ID NO: 791:

Leu Lys Ser Gln Gly Val Lys Val Ser Glu Ser Pro Met Gly Ala Arg
1               5                   10                  15

His Ser Ser Trp
            20

(2) INFORMATION FOR SEQ ID NO: 792:

(i) SEQUENCE CHARACTERISTICS:
(A) LENGTH: 17 amino acids
(B) TYPE: amino acid
(D) TOPOLOGY: linear
(xi) SEQUENCE DESCRIPTION: SEQ ID NO: 792:

Ala Phe Cys Lys Lys Val Gln Gly Ala Gln Met Gln Phe Pro Pro Arg
1               5                   10                  15

Arg

(2) INFORMATION FOR SEQ ID NO: 793:

(i) SEQUENCE CHARACTERISTICS:

(A) LENGTH: 17 amino acids
(B) TYPE: amino acid
(D) TOPOLOGY: linear
(xi) SEQUENCE DESCRIPTION: SEQ ID NO: 793:

Ala Phe Cys Lys Lys Val Gln Gly Ala Gln Met Gln Phe Pro Pro Arg
1               5                   10                  15

Arg

(2) INFORMATION FOR SEQ ID NO: 794:

(i) SEQUENCE CHARACTERISTICS:
(A) LENGTH: 37 amino acids
(B) TYPE: amino acid
(D) TOPOLOGY: linear
(xi) SEQUENCE DESCRIPTION: SEQ ID NO: 794:

Val Gln Val Leu Glu Gln Leu Thr Asn Asn Ala Val Ala Glu Ser Arg
1               5                   10                  15

Phe Asn Asp Ala Ala Tyr Tyr Tyr Trp Met Leu Ser Met Gln Cys Leu
            20                  25                  30

Asp Ile Ala Gln Asp
        35

(2) INFORMATION FOR SEQ ID NO: 795:

(i) SEQUENCE CHARACTERISTICS:
(A) LENGTH: 34 amino acids
(B) TYPE: amino acid
(D) TOPOLOGY: linear
(xi) SEQUENCE DESCRIPTION: SEQ ID NO: 795:

Pro Ala Gln Lys Asp Thr Met Leu Gly Lys Phe Tyr His Phe Gln Arg
1               5                   10                  15

Leu Ala Glu Leu Tyr His Gly Tyr His Ala Ile His Arg His Thr Glu
            20                  25                  30

Asp Pro

(2) INFORMATION FOR SEQ ID NO: 796:

(i) SEQUENCE CHARACTERISTICS:
(A) LENGTH: 27 amino acids
(B) TYPE: amino acid
(D) TOPOLOGY: linear
(xi) SEQUENCE DESCRIPTION: SEQ ID NO: 796:

Leu Ala Lys Gln Ser Lys Ala Leu Gly Ala Tyr Arg Leu Ala Arg His

1                   5                   10                  15

Ala Tyr Asp Lys Leu Arg Gly Leu Tyr Ile Pro
            20                  25

(2) INFORMATION FOR SEQ ID NO: 797:

      (i) SEQUENCE CHARACTERISTICS:
            (A) LENGTH: 36 amino acids
            (B) TYPE: amino acid
            (D) TOPOLOGY: linear
      (xi) SEQUENCE DESCRIPTION: SEQ ID NO: 797:

Ala Arg Phe Gln Lys Ser Ile Glu Leu Gly Thr Leu Thr Ile Arg Ala
 1               5                  10                  15

Lys Pro Phe His Asp Ser Glu Glu Leu Val Pro Leu Cys Tyr Arg Cys
            20                  25                  30

Ser Thr Asn Asn
        35

(2) INFORMATION FOR SEQ ID NO: 798:

      (i) SEQUENCE CHARACTERISTICS:
            (A) LENGTH: 73 amino acids
            (B) TYPE: amino acid
            (D) TOPOLOGY: linear
      (xi) SEQUENCE DESCRIPTION: SEQ ID NO: 798:

Pro Leu Leu Asn Asn Leu Gly Asn Val Cys Ile Asn Cys Arg Gln Pro
 1               5                  10                  15

Phe Ile Phe Ser Ala Ser Ser Tyr Asp Val Leu His Leu Val Glu Phe
            20                  25                  30

Tyr Leu Glu Glu Gly Ile Thr Asp Glu Glu Ala Ile Ser Leu Ile Asp
        35                  40                  45

Leu Glu Val Leu Arg Pro Lys Arg Asp Asp Arg Gln Leu Glu Ile Cys
    50                  55                  60

Lys Gln Gln Leu Pro Asp Ser Cys Gly
65                  70

(2) INFORMATION FOR SEQ ID NO: 799:

      (i) SEQUENCE CHARACTERISTICS:
            (A) LENGTH: 29 amino acids
            (B) TYPE: amino acid
            (D) TOPOLOGY: linear
      (xi) SEQUENCE DESCRIPTION: SEQ ID NO: 799:

Met Pro Tyr Ala Gln Trp Leu Ala Glu Asn Asp Arg Phe Glu Glu Ala

1                 5                   10                  15

Gln Lys Ala Phe His Lys Ala Gly Arg Gln Arg Glu Ala
            20                  25

(2) INFORMATION FOR SEQ ID NO: 800:

      (i) SEQUENCE CHARACTERISTICS:
            (A) LENGTH: 36 amino acids
            (B) TYPE: amino acid
            (D) TOPOLOGY: linear
      (xi) SEQUENCE DESCRIPTION: SEQ ID NO: 800:

Phe Ser Val His Arg Pro Glu Thr Leu Phe Asn Ile Ser Arg Phe Leu
 1               5                  10                  15

Leu His Ser Leu Pro Lys Asp Thr Pro Ser Gly Ile Ser Lys Val Lys
            20                  25                  30

Ile Leu Phe Thr
        35

INDICATIONS RELATING TO A DEPOSITED MICROORGANISM

(PCT Rule 13bis)

A. The indications made below relate to the microorganism referred to in the description
on page 161 , line N/A .

B. IDENTIFICATION OF DEPOSIT | Further deposits are identified on an additional sheet [ ]

Name of depositary institution
American Type Culture Collection

Address of depositary institution (including postal code and country)

10801 University Boulevard
Manassas, Virginia 20110-2209
United States of America

Date of deposit March 27, 1997 | Accession Number 97979

C. ADDITIONAL INDICATIONS (leave blank if not applicable) | This information is continued on an additional sheet [ ]

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications. e.g.. "Accession Number of Deposit")

For receiving Office use only | For International Bureau use only

[X] This sheet was received with the international application | [ ] This sheet was received by the International Bureau on:

Authorized officer | Authorized officer

INDICATIONS RELATING TO A DEPOSITED MICROORGANISM

(PCT Rule 13bis)

A. The indications made below relate to the microorganism referred to in the description
on page 162 , line N/A .

B. IDENTIFICATION OF DEPOSIT Further deposits are identified on an additional sheet [?]

Name of depositary institution
American Type Culture Collection

Address of depositary institution (including postal code and country)

10801 University Boulevard
Manassas, Virginia 20110-2209
United States of America

Date of deposit April 4, 1997 | Accession Number 97974

C. ADDITIONAL INDICATIONS (leave blank if not applicable) This information is continued on an additional sheet [?]

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications. e.g., "Accession Number of Deposit")

For receiving Office use only | For International Bureau use only

[X] This sheet was received with the international application | [ ] This sheet was received by the International Bureau on:
[?] [?] Specialist
[?] [?] Operations
[?]

Authorized officer | Authorized officer
[?] [?]

INDICATIONS RELATING TO A DEPOSITED MICROORGANISM

(PCT Rule 13bis)

A. The indications made below relate to the microorganism referred to in the description
on page 162 , line N/A .

B. IDENTIFICATION OF DEPOSIT | Further deposits are identified on an additional sheet ☐

Name of depositary institution
American Type Culture Collection

Address of depositary institution (including postal code and country)

10801 University Boulevard
Manassas, Virginia 20110-2209
United States of America

Date of deposit May 29, 1997 | Accession Number 209080

C. ADDITIONAL INDICATIONS (leave blank if not applicable) | This information is continued on an additional sheet ☐

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications, e.g., "Accession Number of Deposit")

For receiving Office use only | For International Bureau use only

☒ This sheet was received with the international application | ☐ This sheet was received by the International Bureau on:
[?]
Paralega[?] [?]pecialist
[?]PD-PCT [?]perations

Authorized officer (703) [?] | Authorized officer

INDICATIONS RELATING TO A DEPOSITED MICROORGANISM

(PCT Rule 13bis)

A. The indications made below relate to the microorganism referred to in the description
on page 164 , line N/A .

B. IDENTIFICATION OF DEPOSIT | Further deposits are identified on an additional sheet [?]

Name of depositary institution
American Type Culture Collection

Address of depositary institution (including postal code and country)

10801 University Boulevard
Manassas, Virginia 20110-2209
United States of America

Date of deposit December 3, 1997 | Accession Number 209511

C. ADDITIONAL INDICATIONS (leave blank if not applicable) | This information is continued on an additional sheet [?]

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications, e.g., "Accession Number of Deposit")

For receiving Office use only | For International Bureau use only

[X] This sheet was received with the international application | [ ] This sheet was received by the International Bureau on:
[?]. Anton Smith
P[?]r[?]l[?]gal Specialist
[?]EP PCT [?]perations
(703) [?][?][?]-[?][?][?][?]

Authorized officer | Authorized officer

04 JUN 1998

INDICATIONS RELATING TO A DEPOSITED MICROORGANISM

(PCT Rule 13bis)

A. The indications made below relate to the microorganism referred to in the description
on page 167 . line N/A .

B. IDENTIFICATION OF DEPOSIT | Further deposits are identified on an additional sheet [?]

Name of depositary institution
American Type Culture Collection

Address of depositary institution (including postal code and country)

10801 University Boulevard
Manassas, Virginia 20110-2209
United States of America

Date of deposit April 4, 1997 | Accession Number 97975

C. ADDITIONAL INDICATIONS (leave blank if not applicable) | This information is continued on an additional sheet [?]

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications, e.g., "Accession Number of Deposit")

For receiving Office use only | For International Bureau use only

[X] This sheet was received with the international application | [ ] This sheet was received by the International Bureau on:

Authorized officer | Authorized officer

04 JUN 1998

INDICATIONS RELATING TO A DEPOSITED MICROORGANISM

(PCT Rule 13bis)

A. The indications made below relate to the microorganism referred to in the description
on page 167 , line N/A .

B. IDENTIFICATION OF DEPOSIT | Further deposits are identified on an additional sheet [ ]

Name of depositary institution
American Type Culture Collection

Address of depositary institution (including postal code and country)

10801 University Boulevard
Manassas, Virginia 20110-2209
United States of America

Date of deposit May 29, 1997 | Accession Number 209081

C. ADDITIONAL INDICATIONS (leave blank if not applicable) | This information is continued on an additional sheet [ ]

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications, e.g., "Accession Number of Deposit")

For receiving Office use only | For International Bureau use only

[X] This sheet was received with the international application | [ ] This sheet was received by the International Bureau on:

[?]
[?]

Authorized officer [?] | Authorized officer
[?]
04 JUN 1998

INDICATIONS RELATING TO A DEPOSITED MICROORGANISM

(PCT Rule 13bis)

A. The indications made below relate to the microorganism referred to in the description
on page 171 , line N/A .

B. IDENTIFICATION OF DEPOSIT | Further deposits are identified on an additional sheet [?]

Name of depositary institution
American Type Culture Collection

Address of depositary institution (including postal code and country)

10801 University Boulevard
Manassas, Virginia 20110-2209
United States of America

Date of deposit April 4, 1997 | Accession Number 97976

C. ADDITIONAL INDICATIONS (leave blank if not applicable) | This information is continued on an additional sheet [?]

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications, e.g., "Accession Number of Deposit")

For receiving Office use only | For International Bureau use only

[X] This sheet was received with the international application | [ ] This sheet was received by the International Bureau on:
[?][?][?][?][?][?]cialist
[?][?][?][?][?][?]erations

Authorized officer | Authorized officer
04 JUN 199[?]

INDICATIONS RELATING TO A DEPOSITED MICROORGANISM

(PCT Rule 13bis)

A. The indications made below relate to the microorganism referred to in the description
on page 172 , line N/A .

B. IDENTIFICATION OF DEPOSIT Further deposits are identified on an additional sheet

Name of depositary institution
American Type Culture Collection

Address of depositary institution (including postal code and country)

10801 University Boulevard
Manassas, Virginia 20110-2209
United States of America

Date of deposit April 4, 1997 | Accession Number 97977

C. ADDITIONAL INDICATIONS (leave blank if not applicable) This information is continued on an additional sheet

D. DESIGNATED STATES FOR WHICH INDICATIONS ARE MADE (if the indications are not for all designated States)

E. SEPARATE FURNISHING OF INDICATIONS (leave blank if not applicable)
The indications listed below will be submitted to the International Bureau later (specify the general nature of the indications. e.g., "Accession Number of Deposit")

For receiving Office use only | For International Bureau use only

[X] This sheet was received with the international application | [ ] This sheet was received by the International Bureau on:
[?]. [?]
P[?] Specialist
[?] Operations

Authorized officer | Authorized officer
04 JUN 1998

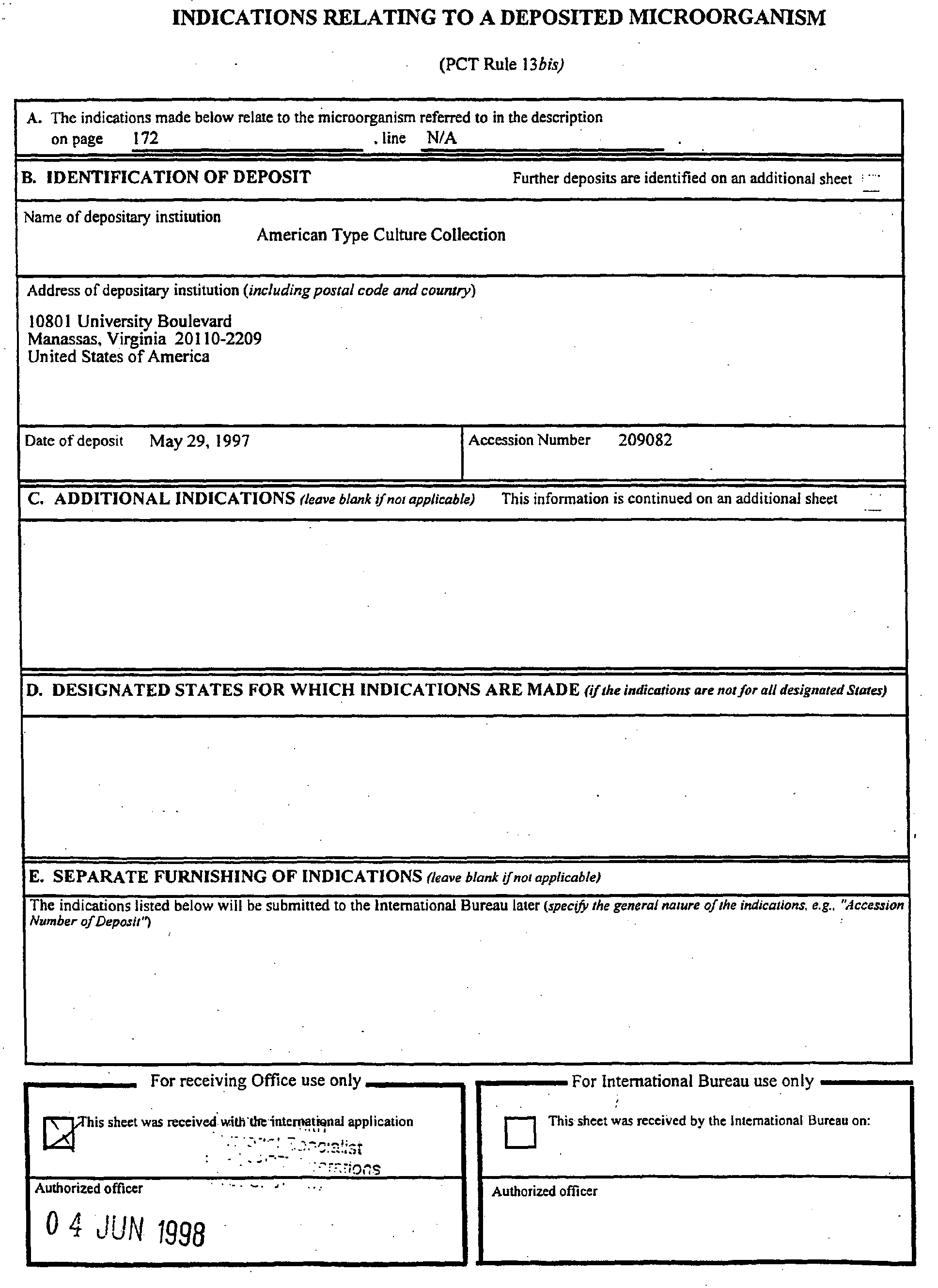
INDICATIONS RELATING TO A DEPOSITED MICROORGANISM

(PCT Rule 13bis)

A. The indications made below relate to the microorganism referred to in the description
on page 172 , line N/A .

B. IDENTIFICATION OF DEPOSIT | Further deposits are identified on an additional sheet [?]

Name of depositary institution
American Type Culture Collection

Address of depositary institution (including postal code and country)

10801 University Boulevard
Manassas, Virginia 20110-2209
United States of America

Date of deposit May 29, 1997 | Accession Number 209082

C. ADDITIONAL INDICATIONS (leave blank if not applicable) | This information is continued on an additional sheet [?]

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications, e.g., "Accession Number of Deposit")

For receiving Office use only | For International Bureau use only

 This sheet was received with the international application | This sheet was received by the International Bureau on:
[?][?][?][?][?] [?]pecialist
[?][?][?][?][?][?][?] [?][?]rations

Authorized officer | Authorized officer
[?][?][?][?] [?][?] [?][?][?]
0 4 JUN 1998

INDICATIONS RELATING TO A DEPOSITED MICROORGANISM

(PCT Rule 13bis)

A. The indications made below relate to the microorganism referred to in the description
on page 176 , line N/A .

B. IDENTIFICATION OF DEPOSIT | Further deposits are identified on an additional sheet [ ]

Name of depositary institution
American Type Culture Collection

Address of depositary institution (including postal code and country)

10801 University Boulevard
Manassas, Virginia 20110-2209
United States of America

Date of deposit April 28, 1997 | Accession Number 209007

C. ADDITIONAL INDICATIONS (leave blank if not applicable) | This information is continued on an additional sheet [ ]

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications, e.g., "Accession Number of Deposit")

For receiving Office use only | For International Bureau use only

[X] This sheet was received with the international application | [ ] This sheet was received by the International Bureau on:

[?]

Authorized officer | Authorized officer
04 JUN 1998

INDICATIONS RELATING TO A DEPOSITED MICROORGANISM

(PCT Rule 13bis)

A. The indications made below relate to the microorganism referred to in the description
on page 176 , line N/A .

B. IDENTIFICATION OF DEPOSIT | Further deposits are identified on an additional sheet [ ]

Name of depositary institution
American Type Culture Collection

Address of depositary institution (including postal code and country)

10801 University Boulevard
Manassas, Virginia 20110-2209
United States of America

Date of deposit May 29, 1997 | Accession Number 209083

C. ADDITIONAL INDICATIONS (leave blank if not applicable) | This information is continued on an additional sheet [ ]

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications, e.g., "Accession Number of Deposit")

For receiving Office use only | For International Bureau use only

[ ] This sheet was received with the international application | [ ] This sheet was received by the International Bureau on:
[?][?][?][?][?][?][?] [?][?]ecialist
[?][?][?] [?][?][?]erations

Authorized officer [?][?][?][?] [?][?][?]-[?][?][?][?] | Authorized officer
04 JUN 1998

INDICATIONS RELATING TO A DEPOSITED MICROORGANISM

(PCT Rule 13bis)

A. The indications made below relate to the microorganism referred to in the description
on page 179 , line N/A .

B. IDENTIFICATION OF DEPOSIT | Further deposits are identified on an additional sheet

Name of depositary institution
American Type Culture Collection

Address of depositary institution (including postal code and country)

10801 University Boulevard
Manassas, Virginia 20110-2209
United States of America

Date of deposit April 28, 1997 | Accession Number 209008

C. ADDITIONAL INDICATIONS (leave blank if not applicable) | This information is continued on an additional sheet

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications. e.g.. "Accession Number of Deposit")

For receiving Office use only | For International Bureau use only

This sheet was received with the international application | This sheet was received by the International Bureau on:

Authorized officer | Authorized officer

[?][?][?] 398

INDICATIONS RELATING TO A DEPOSITED MICROORGANISM

(PCT Rule 13bis)

A. The indications made below relate to the microorganism referred to in the description
on page 179 , line N/A .

B. IDENTIFICATION OF DEPOSIT | Further deposits are identified on an additional sheet [?]

Name of depositary institution
American Type Culture Collection

Address of depositary institution (including postal code and country)

10801 University Boulevard
Manassas, Virginia 20110-2209
United States of America

Date of deposit May 29, 1997 | Accession Number 209084

C. ADDITIONAL INDICATIONS (leave blank if not applicable) | This information is continued on an additional sheet [?]

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications. e.g., "Accession Number of Deposit")

For receiving Office use only | For International Bureau use only

This sheet was received with the international application | This sheet was received by the International Bureau on:
[?] Specialist
[?] Operations

Authorized officer | Authorized officer
04 JUN 1998

INDICATIONS RELATING TO A DEPOSITED MICROORGANISM

(PCT Rule 13bis)

A. The indications made below relate to the microorganism referred to in the description
on page 180 , line N/A .

B. IDENTIFICATION OF DEPOSIT | Further deposits are identified on an additional sheet

Name of depositary institution
American Type Culture Collection

Address of depositary institution (including postal code and country)
10801 University Boulevard
Manassas, Virginia 20110-2209
United States of America

Date of deposit April 28, 1997 | Accession Number 209010

C. ADDITIONAL INDICATIONS (leave blank if not applicable) | This information is continued on an additional sheet

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications. e.g., "Accession Number of Deposit")

For receiving Office use only | For International Bureau use only

This sheet was received with the international application | This sheet was received by the International Bureau on:

Authorized officer | Authorized officer
[?] [?] [?][?][?] [?]998

INDICATIONS RELATING TO A DEPOSITED MICROORGANISM

(PCT Rule 13bis)

A. The indications made below relate to the microorganism referred to in the description
on page 180 , line N/A .

B. IDENTIFICATION OF DEPOSIT | Further deposits are identified on an additional sheet

Name of depositary institution
American Type Culture Collection

Address of depositary institution (including postal code and country)

10801 University Boulevard
Manassas, Virginia 20110-2209
United States of America

Date of deposit May 29, 1997 | Accession Number 209085

C. ADDITIONAL INDICATIONS (leave blank if not applicable) | This information is continued on an additional sheet

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications, e.g., "Accession Number of Deposit")

For receiving Office use only | For International Bureau use only

This sheet was received with the international application | This sheet was received by the International Bureau on:

[?]. [?][?][?][?] Smith
[?][?]ecialist
[?][?] [?]perations

Authorized officer | Authorized officer
04 JUN 1998

INDICATIONS RELATING TO A DEPOSITED MICROORGANISM

(PCT Rule 13bis)

A. The indications made below relate to the microorganism referred to in the description
on page 182 , line N/A .

B. IDENTIFICATION OF DEPOSIT | Further deposits are identified on an additional sheet [ ]

Name of depositary institution
American Type Culture Collection

Address of depositary institution (including postal code and country)

10801 University Boulevard
Manassas, Virginia 20110-2209
United States of America

Date of deposit April 28, 1997 | Accession Number 209009

C. ADDITIONAL INDICATIONS (leave blank if not applicable) | This information is continued on an additional sheet [ ]

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications, e.g., "Accession Number of Deposit")

For receiving Office use only | For International Bureau use only

[ ] This sheet was received with the international application | [ ] This sheet was received by the International Bureau on:

Authorized officer | Authorized officer

INDICATIONS RELATING TO A DEPOSITED MICROORGANISM

(PCT Rule 13bis)

A. The indications made below relate to the microorganism referred to in the description
on page 186 , line N/A .

B. IDENTIFICATION OF DEPOSIT | Further deposits are identified on an additional sheet [ ]

Name of depositary institution
American Type Culture Collection

Address of depositary institution (including postal code and country)
10801 University Boulevard
Manassas, Virginia 20110-2209
United States of America

Date of deposit April 28, 1997 | Accession Number 209011

C. ADDITIONAL INDICATIONS (leave blank if not applicable) | This information is continued on an additional sheet [ ]

D. DESIGNATED STATES FOR WHICH INDICATIONS ARE MADE (if the indications are not for all designated States)

E. SEPARATE FURNISHING OF INDICATIONS (leave blank if not applicable)
The indications listed below will be submitted to the International Bureau later (specify the general nature of the indications, e.g., "Accession Number of Deposit")

For receiving Office use only | For International Bureau use only

[X] This sheet was received with the international application | [ ] This sheet was received by the International Bureau on:
[?][?] [?][?][?] Specialist
[?][?][?]D-PCT Operations
Authorized officer [?][?][?][?] [?][?][?]-[?][?][?][?] | Authorized officer
04 JUN 1998

INDICATIONS RELATING TO A DEPOSITED MICROORGANISM

(PCT Rule 13bis)

A. The indications made below relate to the microorganism referred to in the description
on page 174 , line N/A .

B. IDENTIFICATION OF DEPOSIT | Further deposits are identified on an additional sheet ☐

Name of depositary institution
American Type Culture Collection

Address of depositary institution (including postal code and country)

10801 University Boulevard
Manassas, Virginia 20110-2209
United States of America

Date of deposit April 7, 1998 | Accession Number 209746

C. ADDITIONAL INDICATIONS (leave blank if not applicable) | This information is continued on an additional sheet ☐

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications, e.g., "Accession Number of Deposit")

For receiving Office use only | For International Bureau use only

☒ This sheet was received with the international application | ☐ This sheet was received by the International Bureau on:

Authorized officer | Authorized officer

04 JUN 1998

## Claims

1. A nucleic acid molecule comprising a polynucleotide having a nucleotide sequence selected from the group consisting of:
(a) a polynucleotide having the entire nucleotide sequence of SEQ ID NO:224;
(b) a polynucleotide having the entire cDNA sequence included in ATCC Deposit No. 97974 or 209080, which is hybridizable to SEQ ID NO:224;
(c) a polynucleotide fragment of SEQ ID NO:224;
(d) a polynucleotide fragment of the cDNA sequence included in ATCC Deposit No. 97974 or 209080, which is hybridizable to SEQ ID NO:224;
(e) a polynucleotide encoding a polypeptide fragment of SEQ ID NO:447;
(f) a polynucleotide encoding a polypeptide fragment encoded by the cDNA sequence included in ATCC Deposit No. 97974 or 209080, which is hybridizable to SEQ ID NO:224;
(g) a polynucleotide encoding a polypeptide fragment of SEQ ID NO:447 or a polypeptide fragment encoded by the cDNA sequence included in ATCC Deposit No. 97974 or 209080, having biological activity;
(h) a polynucleotide encoding a polypeptide fragment of SEQ ID NO:447 or a polypeptide fragment encoded by the cDNA sequence included in ATCC Deposit No. 97974 or 209080, which is secreted;
(i) a polynucleotide encoding a full length polypeptide of SEQ ID NO:447 or being encoded by the cDNA sequence included in ATCC Deposit No. 97974 or 209080;
(j) a polynucleotide encoding a polypeptide domain of SEQ ID NO:447;
(k) a polynucleotide encoding a polypeptide domain encoded by the cDNA sequence included in ATCC Deposit No. 97974 or 209080, which is hybridizable to SEQ ID NO:224;
(l) a polynucleotide encoding a polypeptide epitope of SEQ ID NO:447;
(m) a polynucleotide encoding a polypeptide epitope encoded by the cDNA hybridizable to SEQ ID NO:224;
(l) a polynucleotide encoding a polypeptide epitope of SEQ ID NO:447;
(m) a polynucleotide encoding a polypeptide epitope encoded by the cDNA sequence included in ATCC Deposit No. 97974 or 209080, which is hybridizable to SEQ ID NO:224;
(n) a polynucleotide which is at least 90% identical to a polynucleotide as defined in any one of (a) to (m);
(o) a polynucleotide encoding a polypeptide having an amino acid sequence at least 95% identical to the amino acid sequence of a polypeptide encoded by a polynucleotide of any one of (a) to (n);
(p) the polynucleotide of any one of (a) to (o), wherein the polynucleotide fragment comprises a nucleotide sequence encoding a secreted protein; and
(q) a polynucleotide capable of hybridizing under stringent conditions to any one of the polynucleotides specified in (a) to (p), wherein said polynucleotide does not hybridize under stringent conditions to a nucleic acid molecule having a nucleotide sequence of only A residues or of only T residues;
or the complementary strand of polynucleotide (a) to (q).

2. The nucleic acid molecule of claim 1, wherein the nucleotide sequence comprises sequential nucleotide deletions from either the 5' terminus or the 3' terminus.

3. The nucleic acid molecule of claim 1 or 2 which is DNA or RNA.

4. A gene corresponding to the cDNA sequence of SEQ ID NO:224 or encoding the polypeptide having the amino acid sequence of SEQ ID NO:447.

5. A vector comprising the nucleic acid molecule of any one of claims 1 to 3 or the gene of claim 4.

6. A method of making a recombinant host cell comprising introducing the nucleic acid molecule of any one of claims 1 to 3, the gene of claim 4 or the vector of claim 5.

7. A recombinant host cell containing the nucleic acid molecule of claims 1 to 3, the gene of claim 4 or the vector of claim 5 or produced by the method of claim 6.

8. The recombinant host cell of claim 7 which expresses a polypeptide encoded by the nucleic acid molecule of any one of claims 1 to 3 or the gene of claim 4.

9. A method of making a polypeptide encoded by the nucleic acid molecule of any one of claims 1 to 3 or the gene of claim 4 comprising:
(a) culturing the recombinant host cell of claim 8 under conditions such that said polypeptide is expressed; and
(b) recovering said polypeptide.

10. A polypeptide encoded by the nucleic acid molecule of any one of claims 1 to 3, the gene of claim 4 or obtainable by the method of claim 9.

11. An antibody that binds specifically to the polypeptide of claim 10.

12. An antagonist of the polypeptide of claim 10 comprising a molecule closely related to the natural ligand of said polypeptide or a polypeptide related to the natural receptor of the polypeptide of claim 10 or a fragment thereof.

13. An agonist of the polypeptide of claim 10 comprising a molecule closely related to the natural ligand of said polypeptide or a polypeptide related to the natural receptor of the polypeptide of claim 10 or a fragment thereof.

14. A method for identifying antagonists or agonists of the polypeptide of claim 10 comprising:
(a) producing cells which express said polypeptide either as a secreted protein or on the cell membrane;
(b) contacting the polypeptide produced in step (a) with a test sample potentially containing an antagonist or agonist; and
(c) identifying an antagonist or agonist by observing binding and inhibition or stimulation of activity of said polypeptide.

15. An in vitro method of diagnosing a pathological condition or a susceptibility to a pathological condition in a subject comprising:
(a) determining the presence or absence of a mutation in the nucleic acid molecule of any one of claims 1 to 3 or the gene of claim 4; and
(b) diagnosing a pathological condition or a susceptibility to a pathological condition based on the presence or absence of said mutation.

16. An in vitro method of diagnosing a pathological condition or a susceptibility to a pathological condition in a subject comprising:
(a) determining the presence or amount of expression of the polypeptide of claim 10 in a biological sample; and
(b) diagnosing a pathological condition or a susceptibility to a pathological condition based on the presence or amount of expression of the polypeptide.

17. A method for identifying a binding partner to the polypeptide of claim 10 comprising:
(a) contacting the polypeptide of claim 10 with a binding partner; and
(b) determining whether the binding partner effects an activity of the polypeptide.

18. A method of identifying an activity in a biological assay, wherein the method comprises:
(a) expressing the nucleic acid molecule of any one of claims 1 to 3 or the gene of claim 4 in a cell;
(b) isolating the supernatant;
(c) detecting an activity in a biological assay; and
(d) identifying the protein in the supernatant having the activity.

19. The product produced by the method of any one of claims 14, 17 and 18.

20. A pharmaceutical composition comprising the nucleic acid molecule of any one of claims 1 to 3, the gene of claim 4, the polypeptide of claim 10, a DNA encoding and capable of expressing said polypeptide in vivo, the antibody of claim 11, the antagonist of claim 12, the agonist of claim 13 or the product of claim 19 and optionally a pharmaceutically acceptable carrier.

21. Use of the nucleic acid molecule of any one of claims 1 to 3, the polypeptide of claim 10, the gene of claim 4, a DNA encoding and capable of expressing said polypeptide in vivo the antibody of claim 11, antagonist of claim 12, the agonist of claim 13 or the product of claim 19 for the preparation of a pharmaceutical composition for preventing, treating, or ameliorating a medical condition.

22. A diagnostic composition comprising the nucleic acid molecule of any one of claims 1 to 3, the gene of claim 4 or the antibody of claim 11.

23. A method for the production of a pharmaceutical composition comprising the steps of the method of any one of claim 14, 17 or 18 and the further step of formulating the compound identified in a pharmaceutically acceptable form.
